(19) Europäisches Patentamt
European Patent Office
Office européen des brevets

(11) EP 4 656 642 A1

(12) **EUROPEAN PATENT APPLICATION**
published in accordance with Art. 153(4) EPC

(43) Date of publication:
**03.12.2025 Bulletin 2025/49**

(21) Application number: **24747001.6**

(22) Date of filing: **29.01.2024**

(51) International Patent Classification (IPC):
***C07D 491/20*** *(2006.01)* ***C07D 491/107*** *(2006.01)*
***C07D 491/113*** *(2006.01)* ***C07D 487/04*** *(2006.01)*
***C07D 487/00*** *(2006.01)* ***A61K 31/438*** *(2006.01)*
***A61K 31/397*** *(2006.01)* ***A61K 31/4035*** *(2006.01)*
***A61P 35/00*** *(2006.01)*

(52) Cooperative Patent Classification (CPC):
**A61K 31/397; A61K 31/4035; A61K 31/438; A61P 35/00; C07D 487/00; C07D 487/04; C07D 491/107; C07D 491/113; C07D 491/20**

(86) International application number:
**PCT/CN2024/074559**

(87) International publication number:
**WO 2024/156294 (02.08.2024 Gazette 2024/31)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **29.01.2023 CN 202310044209**
**29.01.2023 CN 202310044194**
**28.07.2023 CN 202310939490**

(71) Applicant: **GAN & LEE PHARM CO., LTD**
**Beijing 101109 (CN)**

(72) Inventors:
- **XU, Fuming**
**Beijing 101109 (CN)**
- **CHEN, Wenmin**
**Beijing 101109 (CN)**
- **ZHOU, Guan**
**Beijing 101109 (CN)**
- **WANG, Yuanli**
**Beijing 101109 (CN)**
- **WANG, Kanghua**
**Beijing 101109 (CN)**
- **LI, Xiaoguang**
**Beijing 101109 (CN)**

(74) Representative: **Kraus & Lederer PartGmbB**
**Thomas-Wimmer-Ring 15**
**80539 München (DE)**

# (54) ESTROGEN RECEPTOR PROTEOLYSIS TARGETING CHIMERA COMPOUND AND USE THEREOF

(57) Provided are a novel estrogen receptor proteolysis targeting chimera compound and a use thereof in medicine. The compound provided by the present invention can be used as an estrogen receptor degrader for treating estrogen-dependent diseases.

EP 4 656 642 A1

## Description

### Technical Field

The present invention relates to the field of chemical-pharmaceutical technology, particularly to a compound serving as an estrogen receptor degrader and a pharmaceutically acceptable salt thereof, which can be used as a drug in the treatment or prevention of conditions treated by degradation of estrogen receptor proteins.

### Background Art

The concept of Proteolysis Targeting Chimeras (PROTACs) technology was proposed in 2001 (Proc. Natl. Acad. Sci. USA, 2001, 98, 8584). Early PROTACs recruited E3 ligases through peptide-based ligands, exhibiting poor molecular membrane permeability and limited activity. In 2008, small-molecule PROTACs based on MDM2 E3 ligase appeared, but exhibited inadequate potency. Until 2010-2012, now commonly used ligand for E3 ligase based on cereblon (CRBN) and VHL (von Hippel-Lindau) emerged, achieving micromolar-affinity binding to E3 ligases and establishing the foundation for subsequent PROTACs development. PROTACs molecules are bifunctional molecules comprising a ligand binding to E3 ubiquitin ligase on one end, and a ligand binding to a target protein on the other end, and the two portions are connected by a linker unit. The linker unit brings the E3 ligase and the target protein in close proximity to each other, thereby causing polyubiquitination of the target protein and degradation of the proteasome. PROTACs employ a completely different mechanism of action from that of small molecule inhibitors. First, the ligand of E3 ubiquitin ligase recruits the E3 ubiquitin ligase to the vicinity of the target protein, which in turn ubiquitinates and labels the target protein by drawing it closer to the target protein. The labeled target protein will be degraded by the proteasome system in vivo, which in turn leads to the inhibition of the corresponding protein pathway (Cell Biochem Funct. 2019,37,21-30). Compared with traditional small-molecule drugs, PROTACs can achieve irreversible degradation of target proteins by only requiring transient binding to target proteins to complete the process of ubiquitin transfer due to the altered binding mechanism. Therefore, PROTACs have the following advantages: 1) stronger degradation and more prolonged pharmacodynamic efficacy; 2) higher selectivity for target proteins; and 3) they can overcome the drug-resistant effects of traditional small-molecule inhibitors due to target protein variation (Cell Chem. Biol. 2018, 25, 67-77).

The estrogen receptor (ER) is a member of the nuclear hormone receptor family and acts as a ligand-activated transcription factor involved in the up-regulation and down-regulation of gene expression. The natural hormones of the estrogen receptor are estradiol (E2) and closely related metabolites. Binding of estradiol to the estrogen receptor causes dimerization of the receptor, which in turn binds to the estrogen response element (ERE) on DNA. The ER-DNA complex recruits other transcription factors responsible for transcribing the DNA downstream of the ERE into mRNA, and said mRNA is ultimately converted into protein. Alternatively, the interaction of ER with DNA can occur indirectly through the intermediacy of other transcription factors, the most obvious said transcription factors mentioned are fos and jun. Since the expression of a large number of genes is regulated by the estrogen receptor, and the estrogen receptor is expressed in many cell types, regulation of the estrogen receptor through the incorporation of natural hormones or synthetic ER ligands can have a profound effect on biological physiology and pathophysiology with profound effects.

CRBN is a protein encoded by the CRBN gene in the human body. Homologs of CRBN are highly conserved from plants to humans. Cereblon forms an E3 ubiquitin ligase complex with damaged DNA binding protein I (DDBI), cullin-4A (CUL4A), and cullin regulator I (ROCI). This complex ubiquitinates many other proteins. By a mechanism that has not been fully elucidated, cereblon ubiquitination of target proteins leads to increased levels of fibroblast growth factor 8 (FGF8) and fibroblast growth factor 10 (FGF10), which in turn regulate a number of developmental processes, such as limb and auditory vesicle formation. Thus, this ubiquitin ligase complex is important for limb growth in the embryo. In the absence of CRBN, DDBI forms a complex with DDB2, said DDB2 acting as a DNA damage binding protein.

The process of discovery about CRBN type of E3 ligase ligand is related to the study of thalidomide's mechanism of action. In 2010, scientists discovered that cereblon is a thalidomide-binding protein during the study of thalidomide's toxicity (Science 2010,327,1345). Cereblon is part of the E3 ubiquitin ligase protein complex, which acts as a substrate receptor selectively acting on ubiquitinated proteins. This study suggests that thalidomide-cereblon binding in vivo may be responsible for the teratogenicity of thalidomide. Subsequent studies have revealed that this compound and related structures can be used as anti-inflammatory, anti-angiogenic, and anti-cancer agents. Further modification of thalidomide's structure yielded lenalidomide and pomalidomide with greatly improved safety and significantly reduced teratogenicity, and lenalidomide was approved for marketing by the FDA in 2006. Two seminal papers published in Science in 2014 indicated that lenalidomide acts by degrading two specific B-cell transcription factors, the Ikaros family zinc finger structural proteins 1 and 3 (IKZF1 and IKZF3), which further revealed that the thalidomide structure may play a further role in degrading the target proteins by binding to the E3 ubiquitin ligase protein complex of the cereblon and thus in degrading the target proteins (Science, 2014, 343, 301; Science, 2014, 343, 305). On this basis, CRBN ligands have been widely used in protein degradation, and a series of CRBN ligand-based PROTACs molecules have been developed. Due to the

impact of CRBN ligands themselves on target proteins, off-target degradation of zinc-finger structural domain proteins may occur. Therefore, the design and synthesis of novel, highly selective CRBN ligands are also particularly important in the synthesis of PROTACs molecules.

## Summary

The present disclosure provides a novel estrogen receptor protein proteolysis targeting chimera PROTAC compounds. These molecules exhibit significant activity as estrogen receptor degraders for the treatment of estrogen receptor-mediated or estrogen receptor-dependent diseases.

In one aspect, the present disclosure provides a compound, wherein the compound is a compound having the following chemical structure:

CLM-L-PTM,

or an isomer, an isotopic derivative, a polymorph, a prodrug, or a pharmaceutically acceptable salt or a solvate thereof, wherein:

PTM is a binding moiety that targets the estrogen receptor protein, and has the following structure:

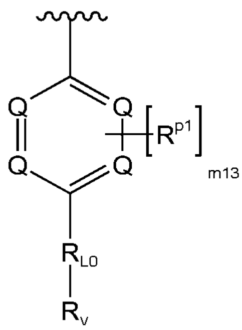

;

$R_{L0}$ is selected from a single bond, -O-, alkylene, and -C(=O)-; preferably, $R_{L0}$ is selected from a single bond, -O-, $C_{1-3}$ alkylene, and -C(=O)-;

$R_v$ is selected from

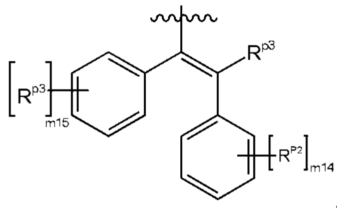
,
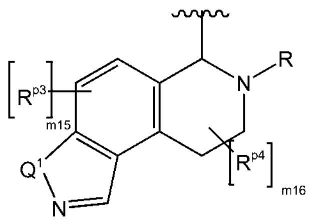
,
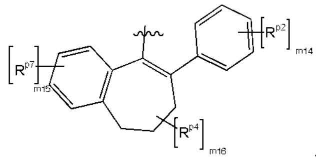
,
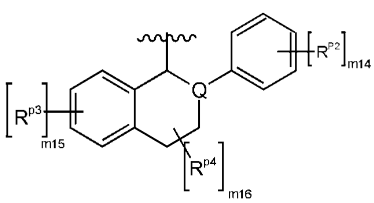
,
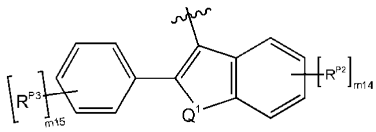
,
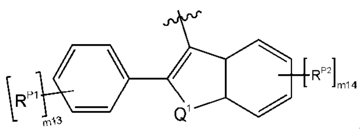
,
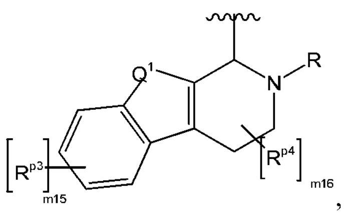
,
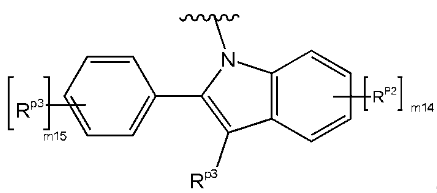
,
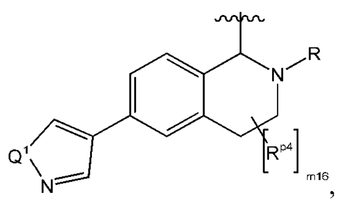
,

and

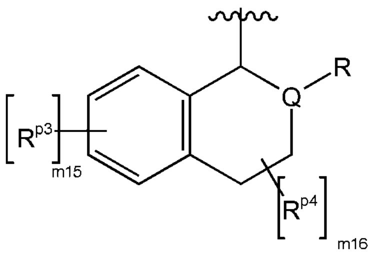;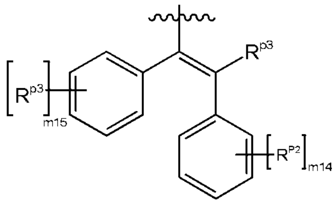,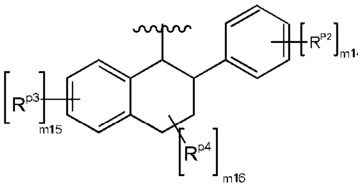,

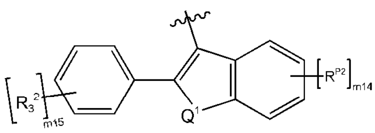,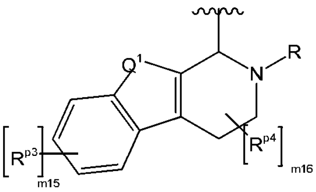,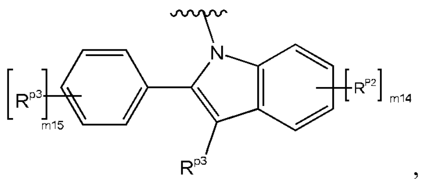,

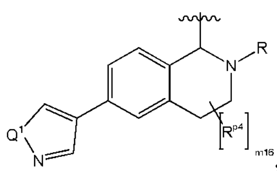, and 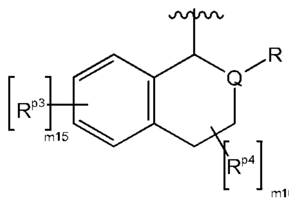;

R is

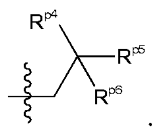;

Each occurrence of Q is independently selected from $CR^x$ and N;
Each occurrence of $Q^1$ is O, S, or $NR^x$.
Each occurrence of $R^{P1}$, $R^{P2}$, $R^{P3}$, $R^{P4}$, $R^{P5}$, $R^{P6}$, $R^x$ and $R^{P7}$ are each independently selected from H, carboxyl, deuterium atom, halogen, alkyl, deuterated alkyl, heteroalkyl, alkenyl, alkynyl, alkoxy, haloalkyl, haloalkoxy,hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkoxy, haloalkyl,hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl;
Preferably, each occurrence of $R^{P1}$, $R^{P2}$, $R^{P3}$, $R^{P4}$, $R^{P5}$, $R^{P6}$, $R^x$, and $R^{P7}$ are each independently selected from H, carboxyl, deuterium atom, halogen, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy,hydroxyl, $C_1$-$C_6$ hydroxyalkyl, nitro, cyano, amino, $C_1$-$C_6$ alkylamino, $C_1$-$C_6$ alkylcarbonyl, $C_1$-$C_6$ alkoxycarbonyl, $C_1$-$C_6$ alkylaminocarbonyl, $C_3$-$C_8$ cycloalkyl, 4-10-membered heterocyclyl, $C_5$-$C_{10}$ heterocyclyl, $C_6$-$C_{10}$ aryl, $C_5$-$C_{10}$ heteroaryl, and 5-10-membered heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, 4-10-membered heterocyclyl, $C_5$-$C_{10}$ heterocyclyl, $C_6$-$C_{10}$ aryl, $C_5$-$C_{10}$ heteroaryl, and 5-10-membered heteroaryl are each independently optionally substituted with one or more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_5$-$C_{10}$ heterocyclyl, 4-10-membered heterocyclyl, $C_2$-$C_6$ alkenyl, $C_2$-$C_6$ alkynyl, $C_1$-$C_6$ alkylamino, $C_1$-$C_6$ alkylcarbonyl, $C_1$-$C_6$ alkoxycarbonyl, $C_1$-$C_6$ alkylaminocarbonyl, $C_6$-$C_{10}$ aryl, $C_5$-$C_{10}$ heteroaryl, and 5-10-membered heteroaryl ;
Preferably, each occurrence of $R^{P1}$, $R^{P2}$, $R^{P3}$, $R^{P4}$, $R^{P5}$, $R^{P6}$, $R^X$, and $R^{P7}$ are each independently selected from H, carboxyl, deuterium atom, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$

alkoxy, hydroxyl, nitro, cyano, amino, $C_3$-$C_8$ cycloalkyl, 4-10-membered heterocyclyl, $C_5$-$C_{10}$ heterocyclyl, $C_6$-$C_{10}$ aryl, $C_5$-$C_{10}$ heteroaryl, and 5-10-membered heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, 4-10-membered heterocyclyl, $C_5$-$C_{10}$ heterocyclyl, $C_6$-$C_{10}$ aryl, $C_5$-$C_{10}$ heteroaryl, and 5-10-membered heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_5$-$C_{10}$ heterocyclyl, 4-10-membered heterocyclyl, $C_6$-$C_{10}$ aryl, $C_5$-$C_{10}$ heteroaryl, and 5-10-membered heteroaryl; Preferably, each occurrence of $R^{P1}$, $R^{P2}$, $R^{P3}$, $R^{P4}$, $R^{P5}$, $R^{P6}$, $R^X$, and $R^{P7}$ is independently selected from H, carboxyl, hydroxyl, deuterium atom, F, Cl, Br, I, $C_1$-$C_3$ alkyl, and $C_1$-$C_3$ alkoxy;

m13 is 0, 1, 2, 3, 4 or 5;
m14 is 0, 1, 2, 3, 4 or 5;
m15 is 0, 1, 2, 3, 4 or 5;
m16 is 0, 1, 2, 3, 4 or 5;
Preferably, the PTM is:

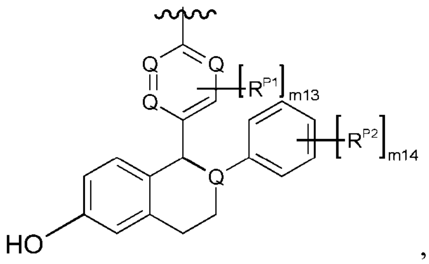
,
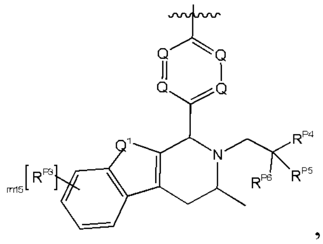
,
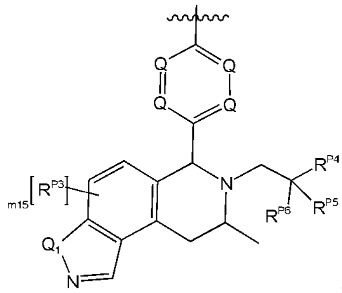
,
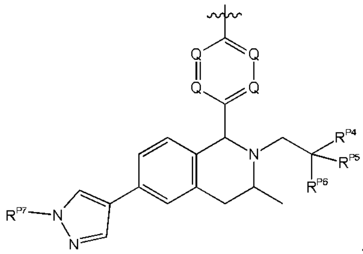
,
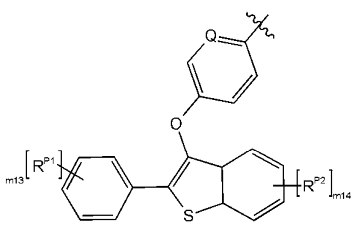
,
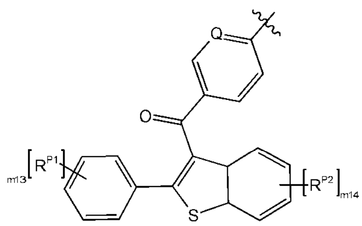
,
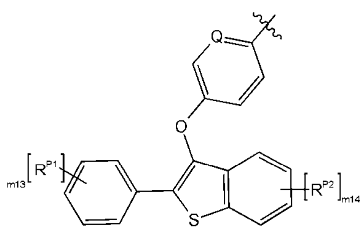
,
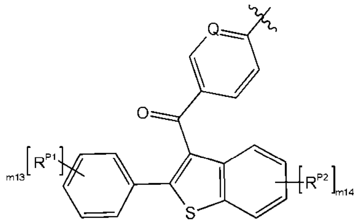
,
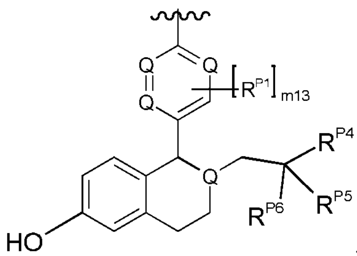
,
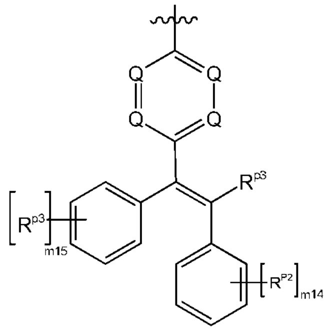
or
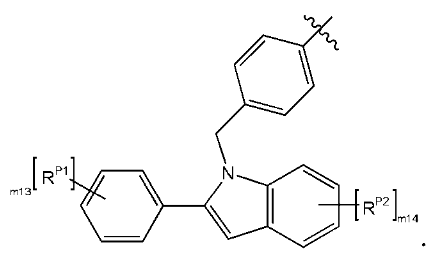
;

L is a bond or a chemical linker moiety that covalently connects CLM and the PTM, and the CLM is a cereblon E3 ubiquitin ligase binding moiety selected from the following structures:

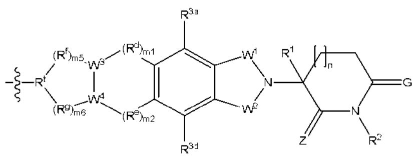,
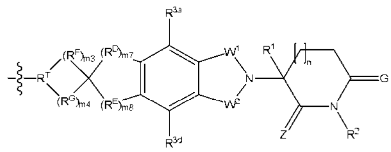,
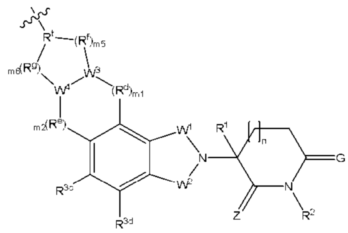,
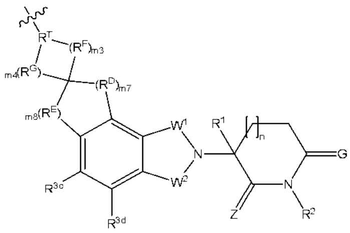,
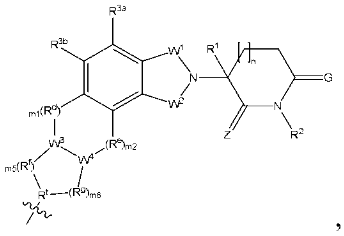, and
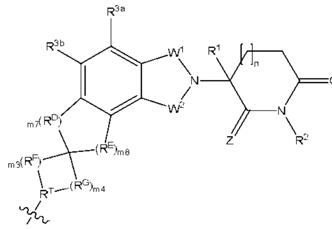;

wherein:

W$^1$ and W$^2$ are identical or different and are each independently CR$^a$R$^b$ or C(=O), and at least one of W$^1$ and W$^2$ is C(=O);

G and Z are identical or different and are each independently selected from O, S, and Se;

R$^{3a}$, R$^{3b}$, R$^{3c}$, and R$^{3d}$ are each independently selected from H, deuterium atom, halogen, alkyl, deuterated alkyl, heteroalkyl, alkenyl, alkynyl, alkoxy, haloalkyl, haloalkoxy,hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkoxy, haloalkyl,hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl;

Each occurrence of R$^d$, R$^e$, R$^f$, R$^g$, R$^D$, R$^E$, R$^F$, and R$^G$ are each independently C(R$^m$)$_2$, NR$^m$, C(=O), O, or S;

Each occurrence of W$^3$ and W$^4$ are each independently CR$^m$ or N;

Each occurrence of and R$^T$ are each independently N or CR$^{2h}$, and when R$^D$, R$^E$, R$^F$, and R$^G$ are all C(R$^m$)$_2$, R$^T$ is CR$^{2h}$;

Each occurrence of m1 and m2 are each independently an integer of 0, 1,2, 3, 4, 5 or 6, and m1 + m2 $\leq$ 6;

Each occurrence of m3 is an integer of 0, 1,2, 3, 4, 5, 6 or 7, and m4 is an integer of 1,2, 3, 4, 5, 6, 7 or 8; and m3 + m4 $\leq$ 8;

Each occurrence of m5 and m6 are each independently an integer of 0, 1,2, 3, 4, 5, 6 or 7, and m5 + m6 $\leq$ 7;

Each occurrence of m7 and m8 are each independently an integer of 0, 1,2, 3, 4, 5, 6 or 7, and m7 + m8 $\leq$ 7;

Each occurrence of R$^m$ is independently selected from H, deuterium atom, halogen, alkyl, deuterated alkyl, heteroalkyl, alkenyl, alkynyl, alkoxy, haloalkyl, haloalkoxy,hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkoxy, haloalkyl,hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl;

R$^{2h}$ is selected from H, deuterium atom, halogen, alkyl, deuterated alkyl, heteroalkyl, alkenyl, alkynyl, alkoxy, haloalkyl, haloalkoxy,hydroxyl, hydroxyalkyl, nitro, cyano, amino, alkylamino, alkylcarbonyl, alkoxycarbonyl, alkylaminocarbonyl, cycloalkyl, heterocyclyl, aryl, heteroaryl, alkenyl, and alkynyl, wherein the alkyl, heteroalkyl, alkoxy, cycloalkyl, heterocyclyl, aryl, and heteroaryl are each independently optionally substituted with one or more substituents selected from halogen, alkyl, heteroalkyl, alkenyl, alkynyl, alkoxy, haloalkyl,hydroxyl, hydroxyalkyl, cyano, amino, nitro, cycloalkyl, heterocyclyl, alkylamino, alkylcarbonyl, alkoxycarbonyl, alkylaminocarbonyl, aryl, and heteroaryl;

R$^1$ is selected from H, halogen, deuterium atom, $C_1$-$C_6$ alkyl, $C_1$-$C_6$ alkoxy,hydroxyl, cycloalkyl, $C_1$-$C_6$ haloalkyl, and hydroxyalkyl;

R$^2$, R$^a$, and R$^b$ are each independently selected from H, $C_1$-$C_3$ alkyl, $C_3$-$C_6$ cycloalkyl, and $C_1$-$C_6$ alkoxy; and

n is 0, 1, 2 or 3;

Preferably, the compound is not:

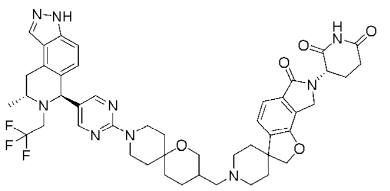,
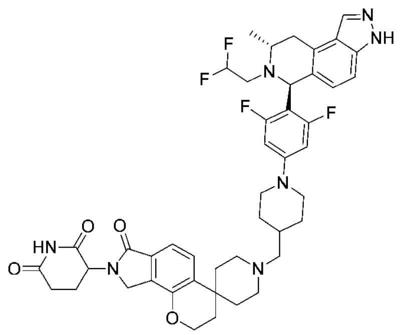,
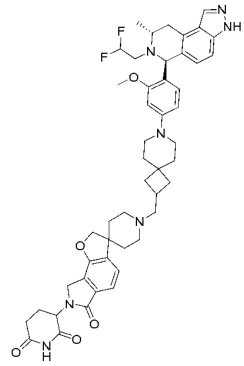,
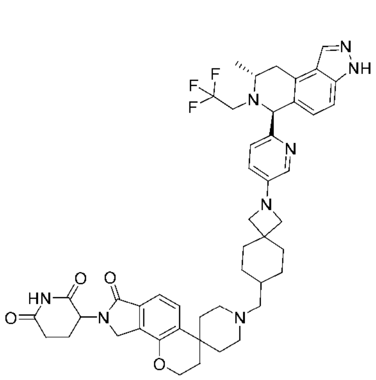, or
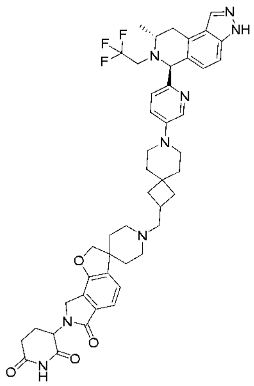.

.

In another embodiment, the compound has the following chemical structure:

CLM-L-PTM,

or an isomer, an isotopic derivative, a polymorph, a prodrug, or a pharmaceutically acceptable salt or a solvate thereof, wherein,

PTM is a binding moiety that targets the estrogen receptor protein, , and has the following structure:

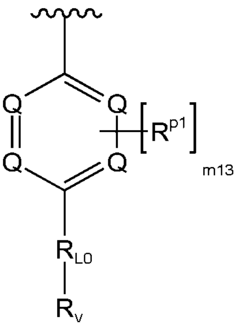;

$R_{L0}$ is selected from a single bond, -O-, alkylene, and -C(=O)-; preferably, $R_{L0}$ is selected from a single bond, - O-, $C_{1-3}$ alkylene, and -C(=O)-;

$R_v$ is selected from

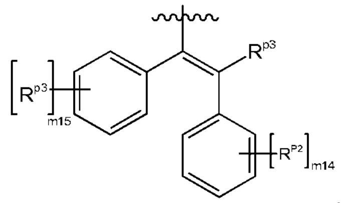,
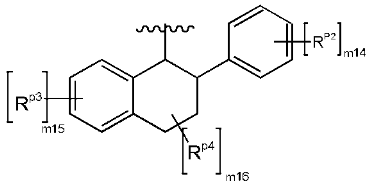,

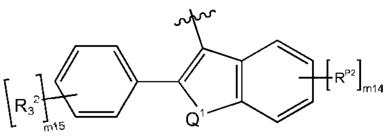,
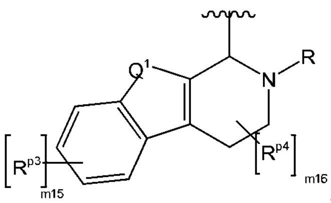,
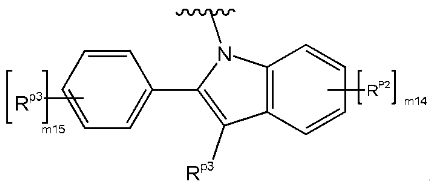,

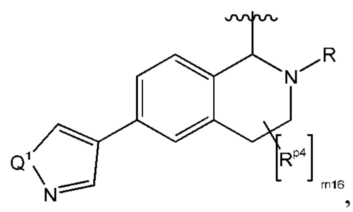, and
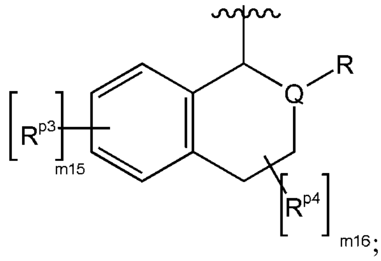;

R is

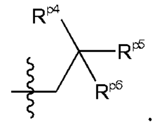;

Each occurrence of Q is each independently selected from $CR^x$ and N;

$Q^1$ is O, S or $NR^x$;

Each occurrence of $R^{P1}$, $R^{P2}$, $R^{P3}$, $R^{P4}$, $R^{P5}$, $R^{P6}$ and $R^x$ are each independently selected from H, deuterium atom, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl, wherein the alkyl, heteroalkyl, alkenyl, alkynyl, alkoxy, cycloalkyl, heterocyclyl, aryl, and heteroaryl are each optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl;

m13 is 0, 1, 2, 3, 4 or 5;

m14 is 0, 1, 2, 3, 4 or 5;

m15 is 0, 1, 2, 3, 4 or 5;

m16 is 0, 1, 2, 3, 4 or 5;

Preferably, the PTM is:

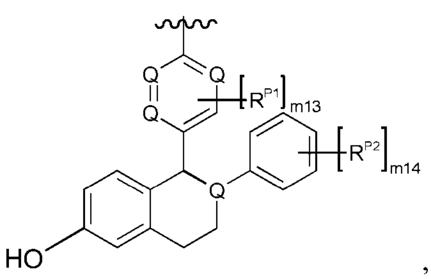,
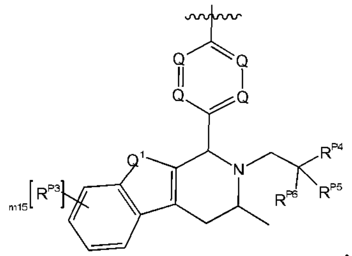,
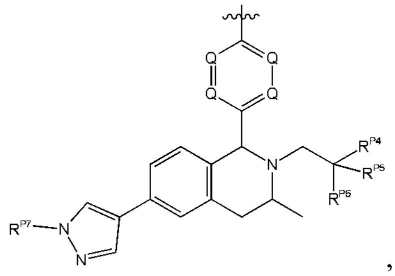,

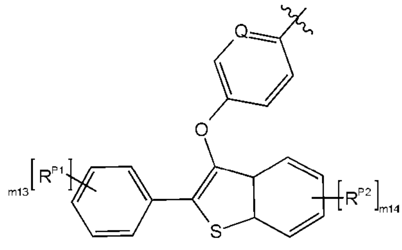, 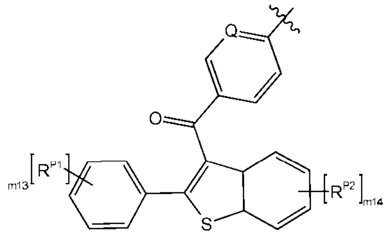, 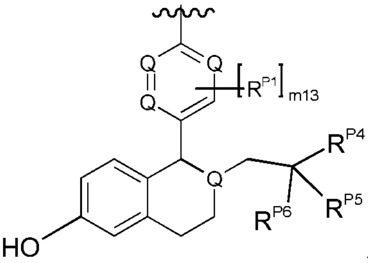,

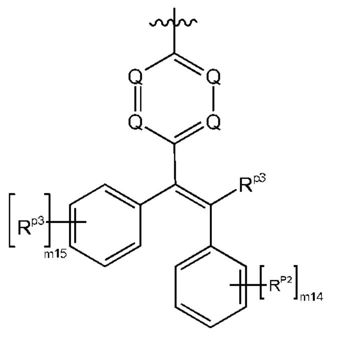 or 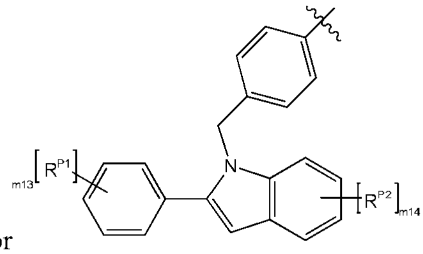;

L is a bond or a chemical linker moiety that covalently connects the CLM and the PTM, and
the CLM is a cereblon E3 ubiquitin ligase binding moiety selected from the following structures:

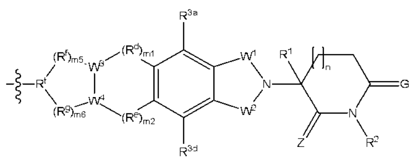, 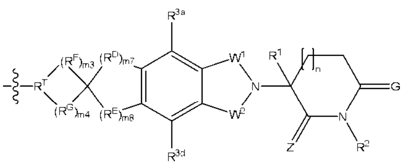, 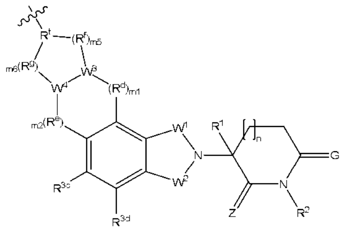,

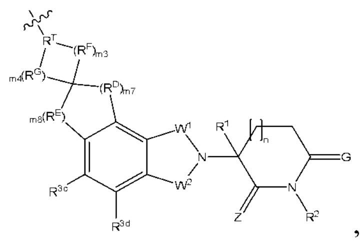, 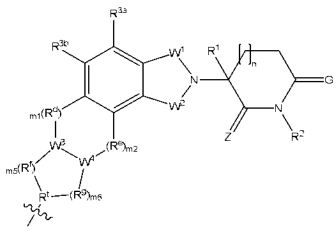, and 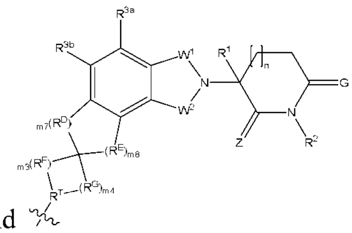;

wherein:

$W^1$ and $W^2$ are identical or different and are each independently $CR^aR^b$ or C(=O), and at least one of $W_1$ and $W^2$ is C(=O);

G and Z are identical or different and are each independently selected from O, S, and Se;

$R^{3a}$, $R^{3b}$, $R^{3c}$, and $R^{3d}$ are each independently selected from H, deuterium atom, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl;

Each occurrence of $R^d$, $R^e$, $R^f$, $R^g$, $R^D$, $R^E$, $R^F$, and $R^G$ are each independently $C(R^m)_2$, $NR^m$, C(=O), O, or S;

Each occurrence of $W^3$ and $W^4$ are each independently $CR^m$ or N;

Each occurrence of $R^t$ and $R^T$ are each independently N or $CR^{2h}$, and when $R^D$, $R^E$, $R^F$, and $R^G$ are all $C(R^m)_2$, $R^T$

is $CR^{2h}$;

Each occurrence of m1 and m2 are each independently an integer of 0, 1, 2, 3, 4, 5, or 6, and m1 + m2 ≤ 6;

Each occurrence of m3 is independently an integer of 0, 1, 2, 3, 4, 5, 6, or 7; m4 is an integer of 1, 2, 3, 4, 5, 6, 7, or 8, and m3 + m4 ≤ 8;

Each occurrence of m5 and m6 are each independently an integer of 0, 1, 2, 3, 4, 5, 6, or 7, and m5 + m6 ≤ 7;

Each occurrence of m7 and m8 are each independently an integer of 0, 1, 2, 3, 4, 5, 6, or 7, and m7 + m8 ≤ 7;

Each occurrence of $R^m$ is independently selected from H, deuterium atom, halogen, alkyl, deuterated alkyl, heteroalkyl, alkenyl, alkynyl, alkoxy, haloalkyl, haloalkoxy,hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkoxy, haloalkyl,hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl;

$R^{2h}$ is selected from H, deuterium atom, halogen, alkyl, deuterated alkyl, heteroalkyl, alkenyl, alkynyl, alkoxy, haloalkyl, haloalkoxy,hydroxyl, hydroxyalkyl, nitro, cyano, amino, alkylamino, alkylcarbonyl, alkoxycarbonyl, alkylaminocarbonyl, cycloalkyl, heterocyclyl, aryl, heteroaryl, alkenyl and alkynyl, wherein the alkyl, heteroalkyl, alkoxy, cycloalkyl, heterocyclyl, aryl, and heteroaryl are each independently optionally substituted with one or more substituents selected from halogen, alkyl, heteroalkyl, alkoxy, haloalkyl,hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl;

$R^1$ is selected from H, halogen, deuterium atom, $C_1$-$C_6$ alkyl, $C_1$-$C_6$ alkoxy,hydroxyl, cycloalkyl, $C_1$-$C_6$ haloalkyl, and hydroxyalkyl;

$R^2$, $R^a$, and $R^b$ are each independently selected from H, $C_1$-$C_3$ alkyl, $C_3$-$C_6$ cycloalkyl, and $C_1$-$C_6$ alkoxy; and n is 0, 1, 2, or 3.

In one of the embodiments, the compound has the following chemical structure:

CLM-L-PTM,

or an isomer, an isotopic derivative, a polymorph, a prodrug, or a pharmaceutically acceptable salt or a solvate thereof, wherein:

PTM is a binding moiety that targets to the estrogen receptor protein, selected from the following structures:

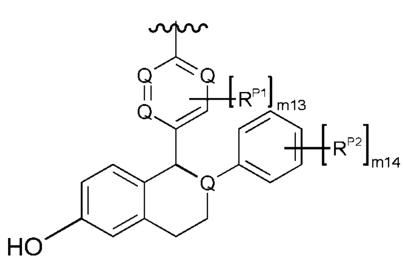
,
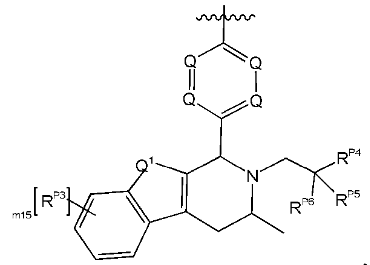
,
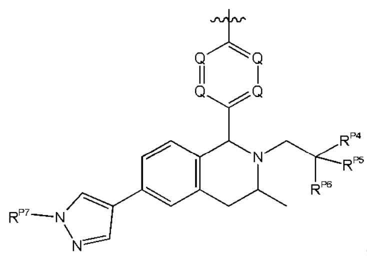
,
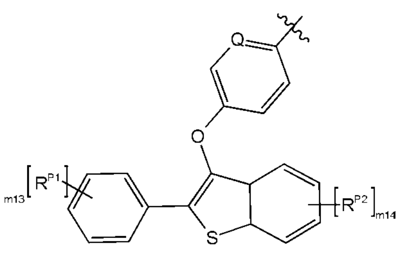
and
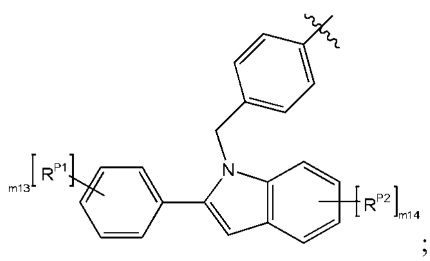
;

L is a bond or a chemical linker moiety that covalently connects CLM and the PTM; and
the CLM is a cereblon E3 ubiquitin ligase binding moiety selected from the following structures:

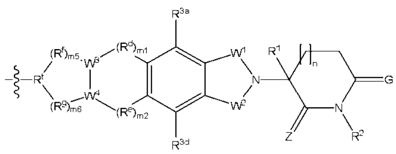, 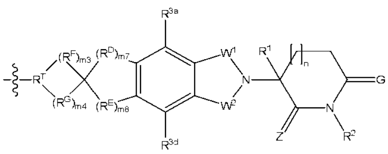, 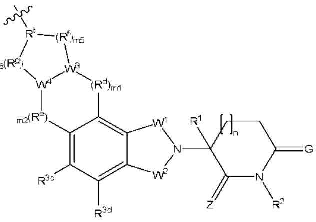,

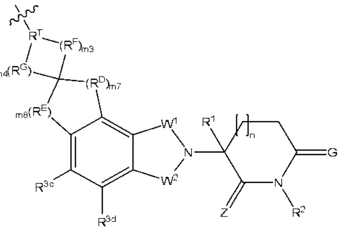, 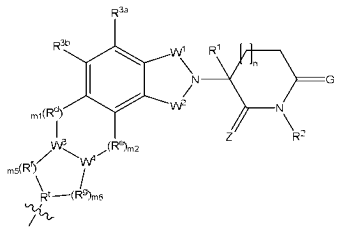, and 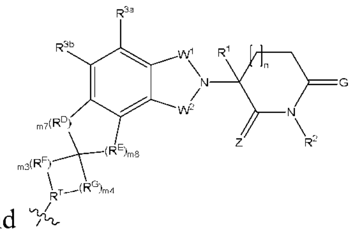;

wherein:

each occurrence of Q is each independently selected from $CR^x$ and N;

$Q^1$ is O, S, or NH;

each occurrence of $R^{P1}$, $R^{P2}$, $R^{P3}$, $R^{P4}$, $R^{P5}$, $R^{P6}$, and $R^x$ are each independently selected from H, deuterium atom, halogen, alkyl, deuterated alkyl, heteroalkyl, alkenyl, alkynyl, alkoxy, haloalkyl, haloalkoxy,hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkoxy, haloalkyl,hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl;

m13 is 0, 1, 2, 3, 4, or 5;

m14 is 0, 1, 2, 3, 4, or 5;

m15 is 0, 1, 2, 3, 4, or 5;

$W^1$ and $W^2$ are identical or different and are each independently $CR^aR^b$ or C(=O), and at least one of $W^1$ and $W^2$ is C(=O);

G and Z are identical or different and are each independently selected from O, S, and Se;

$R^{3a}$, $R^{3b}$, $R^{3c}$, and $R^{3d}$ are each independently selected from H, deuterium atom, halogen, alkyl, deuterated alkyl, heteroalkyl, alkenyl, alkynyl, alkoxy, haloalkyl, haloalkoxy,hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl;

Each occurrence of $R^d$, $R^e$, $R^f$, $R^g$, $R^D$, $R^E$, $R^F$, and $R^G$ are each independently $C(R^m)_2$, $NR^m$, C(=O), O, or S;

Each occurrence of $W^3$ and $W^4$ are each independently $CR^m$ or N;

Each occurrence of $R^t$ and $R^T$ are each independently N or $CR^{2h}$, and when $R^D$, $R^E$, $R^F$, and $R^G$ are all $C(R^m)_2$, $R^T$ is $CR^{2h}$;

Each occurrence of m1 and m2 are each independently an integer of 0, 1,2, 3, 4, 5 or 6, and $m1 + m2 \leq 6$;

Each occurrence of m3 is an integer of 0, 1,2, 3, 4, 5, 6 or 7, and m4 is an integer of 1,2, 3, 4, 5, 6, 7 or 8; and $m3 + m4 \leq 8$;

Each occurrence of m5 and m6 are each independently an integer of 0, 1,2, 3, 4, 5, 6 or 7, and $m5 + m6 \leq 7$;

Each occurrence of m7 and m8 are each independently an integer of 0, 1,2, 3, 4, 5, 6 or 7, and $m7 + m8 \leq 7$;

Each occurrence of $R^m$ is independently selected from H, deuterium atom, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkoxy, haloalkyl,hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl;

$R^{2h}$ is selected from H, deuterium atom, halogen, alkyl, deuterated alkyl, heteroalkyl, alkenyl, alkynyl, alkoxy, haloalkyl, haloalkoxy,hydroxyl, hydroxyalkyl, nitro, cyano, amino, alkylamino, alkylcarbonyl, alkoxycarbonyl, alkylaminocarbonyl, cycloalkyl, heterocyclyl, aryl, heteroaryl, alkenyl, and alkynyl, wherein the alkyl, heteroalkyl, alkoxy, cycloalkyl, heterocyclyl, aryl, and heteroaryl are each independently optionally substituted with one or more substituents selected from halogen, alkyl, heteroalkyl, alkenyl, alkynyl, alkoxy, haloalkyl,hydroxyl, hydroxyalkyl, cyano, amino, nitro, cycloalkyl, heterocyclyl, alkylamino, alkylcarbonyl, alkoxycarbonyl, alkylaminocarbonyl, aryl, and heteroaryl;

$R^1$ is selected from H, halogen, deuterium atom, $C_1$-$C_6$ alkyl, $C_1$-$C_6$ alkoxy, hydroxyl, cycloalkyl, $C_1$-$C_6$ haloalkyl, and hydroxyalkyl;

$R^2$, $R^a$, and $R^b$ are each independently selected from H, $C_1$-$C_3$ alkyl, $C_3$-$C_6$ cycloalkyl, and $C_1$-$C_6$ alkoxy; and n is 0, 1, 2, or 3.

In one embodiment, $W^1$ and $W^2$ are identical or different and are each independently $CH_2$ or C(=O), and at least one of $W^1$ and $W^2$ is C(=O); and/or

$R^{P1}$, $R^{P2}$, $R^{P3}$, $R^{P4}$, $R^{P5}$, $R^{P6}$, $R^x$, $R^{3a}$, $R^{3b}$, $R^{3c}$, and $R^{3d}$ are each independently selected from H, deuterium atom, halogen, $C_1$-$C_6$ alkyl, heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy,hydroxyl, $C_1$-$C_6$ hydroxyalkyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted with one or more substituents selected from halogen, $C_1$-$C_6$ alkyl, $C_1$-$C_6$ heteroalkyl, $C_1$-$C_6$ alkoxy, $C_1$-$C_6$ haloalkyl,hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; preferably, $R^{P1}$, $R^{P2}$, $R^{P3}$, $R^{P4}$, $R^{P5}$, $R^{P6}$, $R^x$, $R^{3a}$, $R^{3b}$, $R^{3c}$, and $R^{3d}$ are each independently selected from H, deuterium atom, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy,hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl,hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; preferably, $R^{P1}$, $R^{P2}$, $R^{P3}$, $R^{P4}$, $R^{P6}$, $R^{P6}$, $R^x$, $R^{3a}$, $R^{3b}$, $R^{3c}$, and $R^{3d}$ are each independently selected from H,hydroxyl, deuterium atom, halogen, $C_1$-$C_3$ alkyl, and $C_1$-$C_3$ alkoxy; preferably, $R^{P1}$, $R^{P2}$, $R^{P3}$, $R^{P4}$, $R^{P6}$, $R^{P6}$, $R^x$, $R^{3a}$, $R^{3b}$, $R^{3c}$, and $R^{3d}$ are each independently selected from H, deuterium atom, halogen, $C_1$-$C_3$ alkyl, and $C_1$-$C_3$ alkoxy; preferably, $R^{P1}$, $R^{P2}$, $R^{P3}$, $R^{P4}$, $R^{P5}$, $R^{P6}$, $R^x$, $R^{3a}$, $R^{3b}$, $R^{3c}$, and $R^{3d}$ are each independently selected from H, deuterium atom, F, Cl, Br, I, $C_1$-$C_3$ alkyl, and $C_1$-$C_3$ alkoxy; and/or

Each occurrence of $R^d$, $R^e$, $R^f$ and $R^g$ are each independently $C(R^m)_2$ or O; and/or

Each occurrence of $R^D$, $R^E$, $R^F$, and $R^G$ are each independently $C(R^m)_2$ or O; and/or

$W^3$ and $W^4$ are CH; and/or

$R^{2h}$ is selected from H, deuterium atom, halogen, $C_1$-$C_6$ alkyl, $C_1$-$C_6$ deuterated alkyl, $C_1$-$C_6$ heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, $C_5$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted with one or more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; preferably, $R^{2h}$ is selected from H, deuterium atom, F, Cl, Br, I, $C_1$-$C_6$ alkyl, $C_1$-$C_6$ deuterated alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; preferably, $R^{2h}$ is selected from H, deuterium atom, halogen, $C_1$-$C_3$ alkyl, and $C_1$-$C_3$ alkoxy; preferably, $R^{2h}$ is selected from H, deuterium atom, F, Cl, Br, I, $C_1$-$C_3$ alkyl, $C_1$-$C_3$ haloalkyl, $C_1$-$C_3$ alkoxy, and $C_1$-$C_3$ haloalkoxy; and/or

Each occurrence of m1 and m2 are each independently an integer of 0, 1,2 or 3, and $m1 + m2 \leq 3$; preferably $m1 + m2 = 1$ or $m1 + m2 = 2$; and/or

Each occurrence of m3 is an integer of 0, 1,2, 3 or 4, and m4 is an integer of 1,2, 3, 4, or 5; and $m3 + m4 \leq 5$; preferably $m3 + m4 = 2$, $m3 + m4 = 3$, or $m3 + m4 = 4$; and/or

Each occurrence of m5 and m6 is are each independently an integer of 0, 1,2, 3, or 4, and $m5 + m6 \leq 4$, preferably $m5 + m6 = 2$ or $m5 + m6 = 3$; and/or

Each occurrence of m7 and m8 are each independently an integer of 0, 1,2, 3 or 4, and m7 + m8 ≤ 4; preferably m7 + m8 =2 or m7 + m8 = 3; and/or

Each occurrence of $R^m$ is each independently selected from H, deuterium atom, halogen, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, aryl, and heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted with one or more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_a$ cycloalkyl, $C_4$-$C_{10}$ heterocyclyl, $C_6$-$C_{10}$ aryl, and $C_5$-$C_{10}$ heteroaryl; preferably, each occurrence of $R^m$ is each independently selected from H, deuterium atom, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; preferably, each occurrence of $R^m$ is independently selected from H, deuterium atom, halogen, $C_1$-$C_3$ alkyl, and $C_1$-$C_3$ alkoxy; and/or

$R^1$ is selected from H, halogen, $C_1$-$C_3$ alkyl, and hydroxyl; preferably $R^1$ is selected from H, F, Cl, Br, I, $C_1$-$C_3$ alkyl, and hydroxyl; and/or

$R^2$ is selected from H and $C_1$-$C_3$ alkyl; and/or

n is 0 or 1.

In one embodiment, the CLM is selected from the following structures:

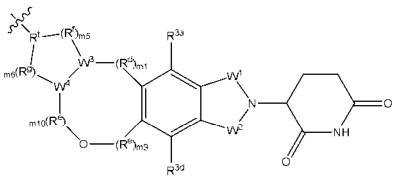
,
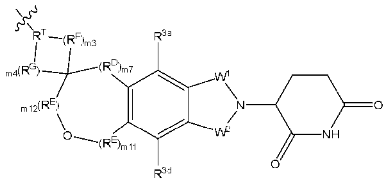
,
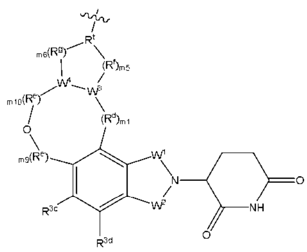
,
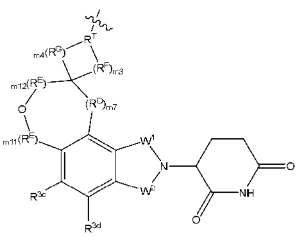
,
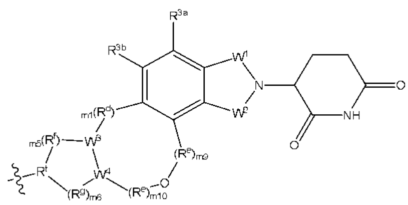
, and
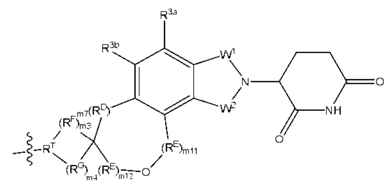
;

$W^1$, $W^2$, $W^3$, $W^4$, $R^{3a}$, $R^{3b}$, $R^{3c}$, $R^{3d}$, $R^d$, $R^e$, $R^f$, $R^t$, $R^g$, $R^D$, $R^E$, $R^F$, $R^T$, $R^G$, m3, m4, m5, and m6 are as defined in claim 1, 2, 3, or 4;

preferably,

$R^{3a}$, $R^{3b}$, $R^{3c}$, and $R^{3d}$ are each independently selected from H, deuterium atom, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy,hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl;

Each occurrence of m1, m9, and m10 are each independently an integer of 0, 1, 2, 3, 4, or 5, and m1 + m9 + m10 ≤ 5; preferably, each occurrence of m1, m9, and m10 are each independently an integer of 0, 1, or 2, and m1 + m9 + m10 ≤ 2; preferably, m1 + m9 + m10 =1 or m1 + m9 + m10 =0; and

Each occurrence of m7, m11, and m12 are each independently an integer of 0, 1, 2, 3, 4, 5, or 6, and m7 + m11 + m12 ≤ 6; preferably, m7, m11, and m12 are each independently an integer of 0, 1, 2, or 3, and m7 + m11 + m12 ≤ 3; preferably,

m7 + m11 + m12=2 or m7 + m11 + m12=1.

In one embodiment, the CLM is selected from the following structures:

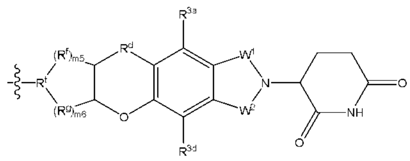,
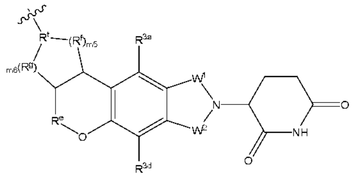,
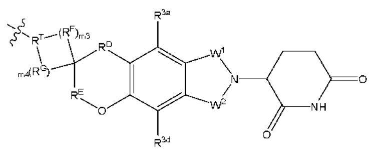,
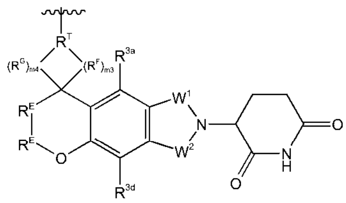,
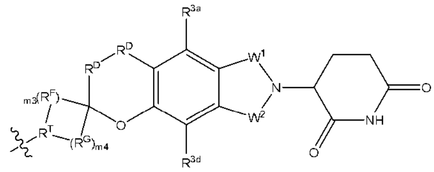,
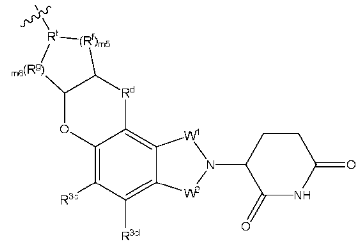,
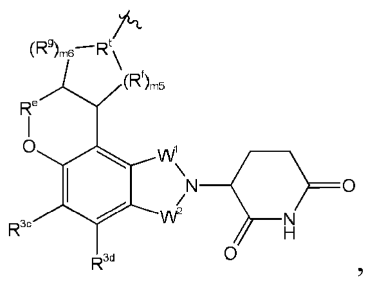,
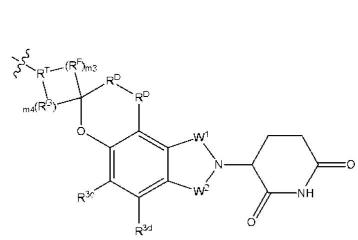,
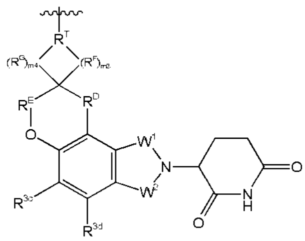,
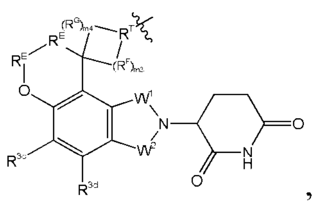,
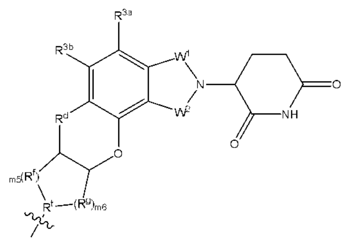,
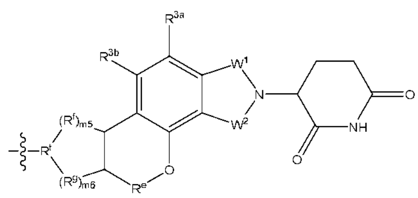,
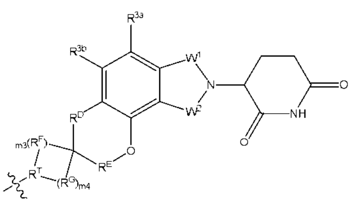,
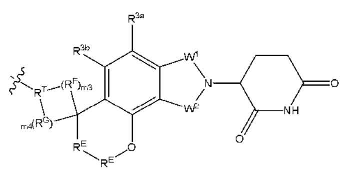,
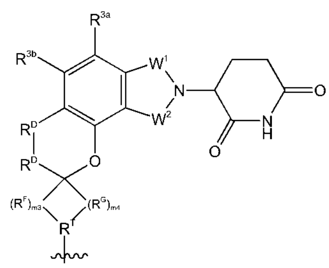,
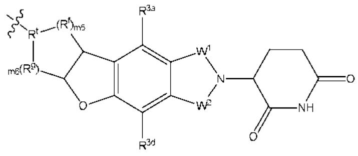,
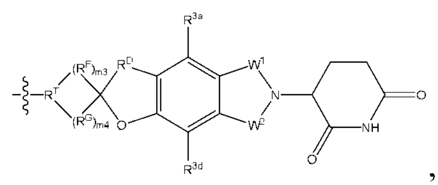,
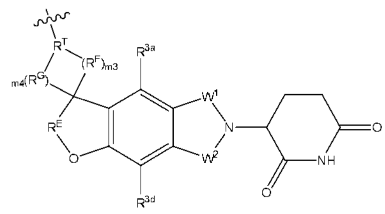,

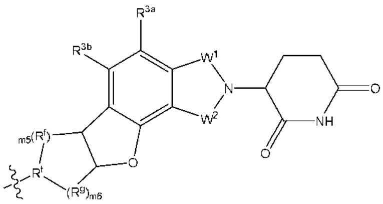 , 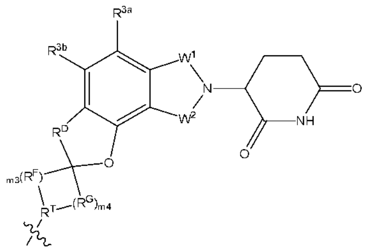 , 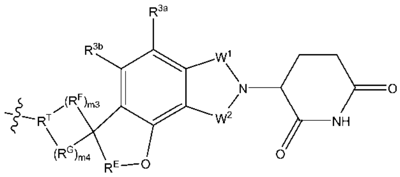 ,

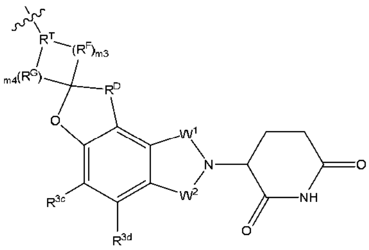 , 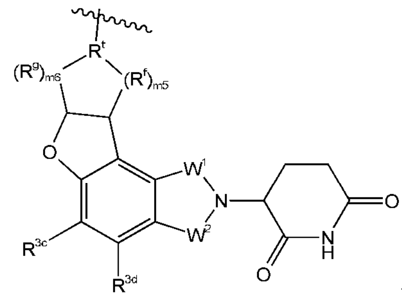 and 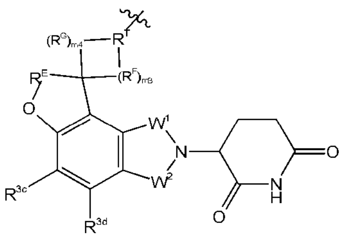 ;

wherein:

$W^1$, $W^2$, $R^{3a}$ $R^{3b}$, $R^{3c}$ $R^{3a}$ $R^d$, $R^e$ $R^f$, $R^t$, $R^g$, $R^D$, $R^F$, $R^F$, $R^T$, $R^G$, m3, m4, m5, and m6 are as defined in claim 1, 2, 3, or 4; Preferably:

$R^{3a}$, $R^{3b}$, $R^{3c}$, and $R^{3d}$ are each independently selected from H, deuterium atom, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy,hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; and/or

Each occurrence of m3 is each independently an integer of 0, 1, 2, 3, or 4, and m4 is an integer of 1, 2, 3, 4, or 5, and $m3 + m4 \leq 5$, preferably m3 + m4 = 2, m3 + m4 = 3, or m3 + m4 = 4; and/or

Each occurrence of m5 and m6 are each independently an integer of 0, 1, 2, 3, or 4, and $m5 + m6 \leq 4$; preferably m5 + m6 = 2 or m5 + m6 = 3; and/or

$W^1$ and $W^2$ are identical or different and are each independently $CH_2$ or C(=O), and at least one of $W^1$ and $W^2$ is C(=O); and/or

Each occurrence of $R^d$, $R^e$, $R^f$, $R^g$, $R^D$, $R^E$, $R^F$, and $R^G$ are each independently $C(R^m)_2$ or O, each occurrence of $R^m$ is each independently selected from H, deuterium atom, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy,hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_5$-$C_{10}$ aryl, and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; and/or

Each occurrence of $R^t$ and $R^T$ are each independently N or $CR^{2h}$, wherein $R^{2h}$ is selected from H, deuterium atom, F, Cl, Br, I, $C_1$-$C_6$ alkyl, $C_1$-$C_6$ deuterated alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl.

In one embodiment, the CLM is selected from the following structures:

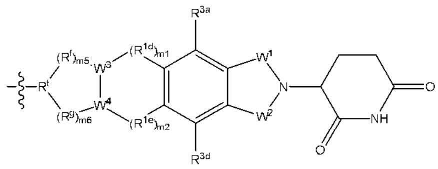, 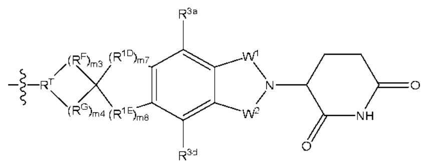, 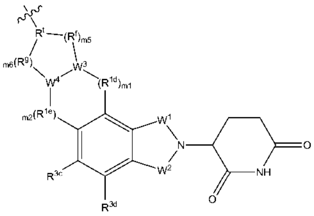,

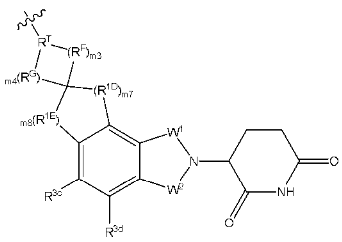, 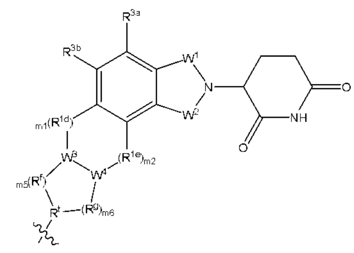, and 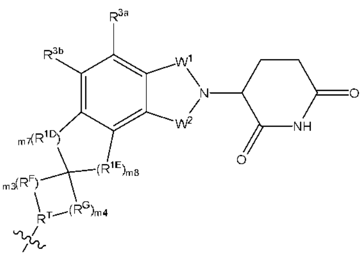;

Wherein:

$W^1$, $W^2$, $W^3$, $W^4$, $R^{3a}$, $R^{3b}$, $R^{3c}$, $R^{3d}$, $R^f$, $R^t$, $R^g$, $R^F$, $R^G$, m1, m2, m3, m4, m5, m6, m7, and m8 are as defined in claim 1, 2, 3, or 4;

Preferably,

$R^{3a}$, $R^{3b}$, $R^{3c}$, and $R^{3d}$ are each independently selected from H, deuterium atom, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy,hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl;

Each occurrence of $R^{14}$, $R^{1e}$, $R^{1D}$, and $R^{1E}$ are each independently $C(R^m)_2$;

$R^T$ is N or $CR^{2h}$, and when both $R^F$ and $R^G$ are $C(R^m)_2$, $R^T$ is $CR^{2h}$; and

$R^{2h}$ and $R^m$ are as defined in claim 1, 2, 3, or 4.

In one embodiment, the CLM is selected from the following structures:

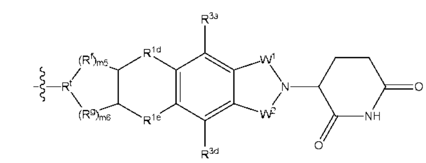, 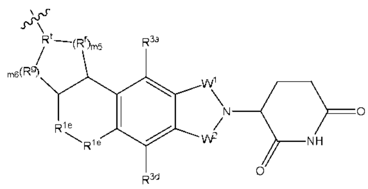, 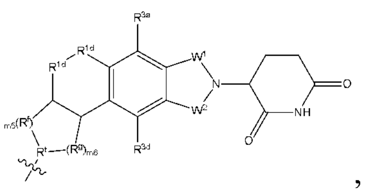,

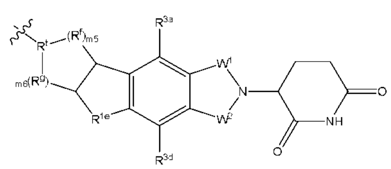, 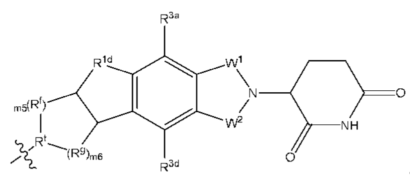, 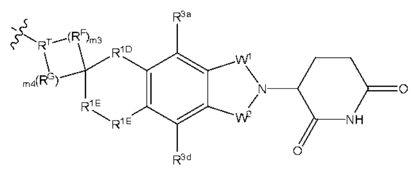,

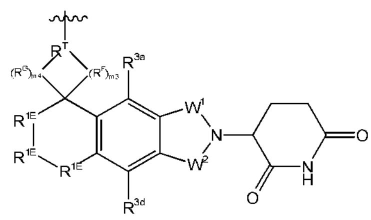 , 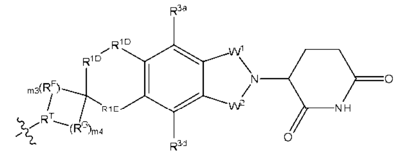 , 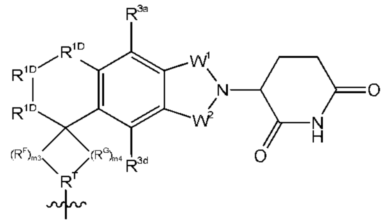 ,

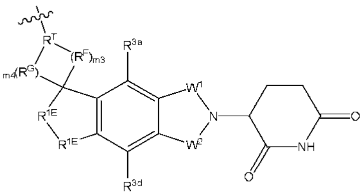 , 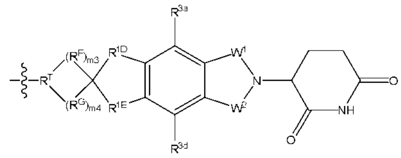 , 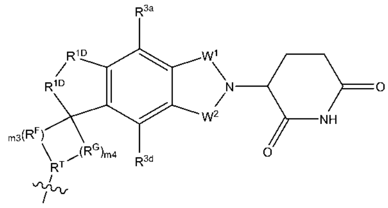 ,

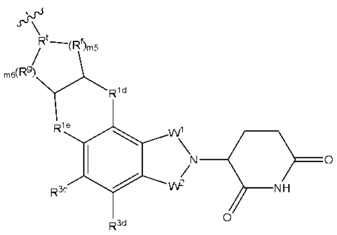 , 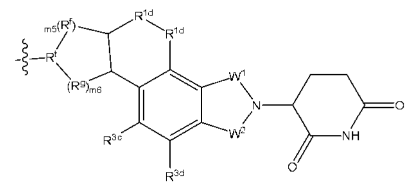 , 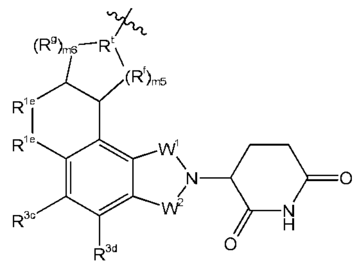 ,

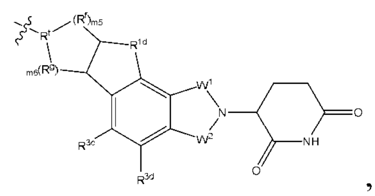 , 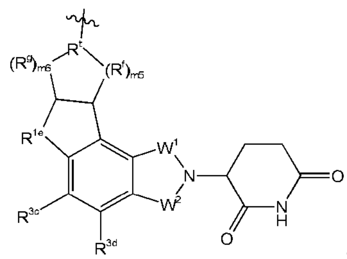 , 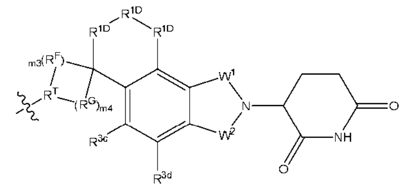 ,

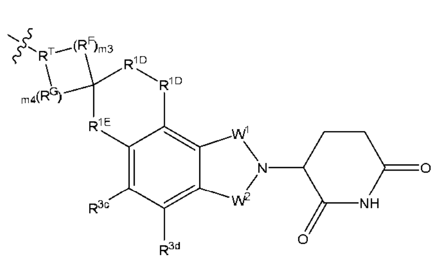 , 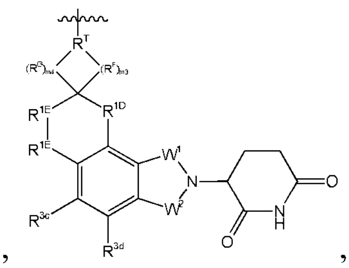 , 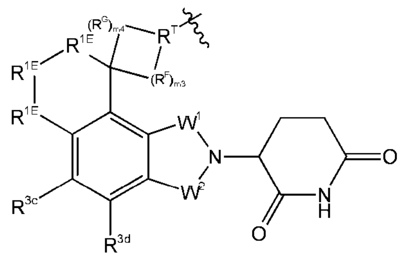 ,

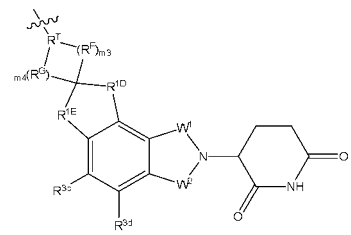 , 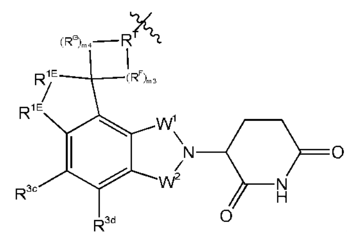 , 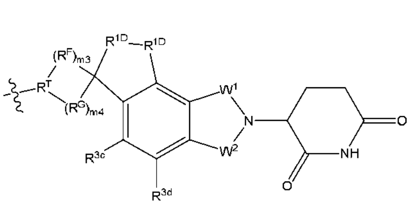 ,

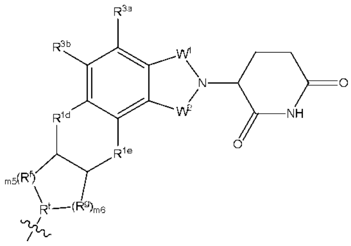, 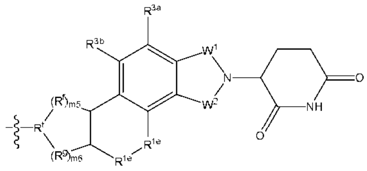, 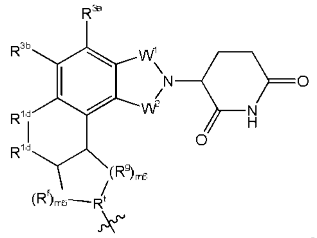,

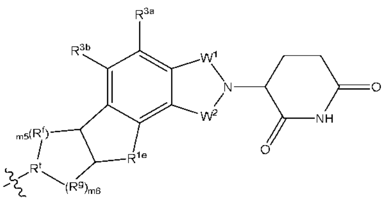, 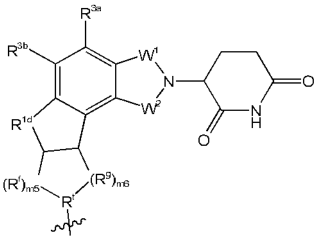, 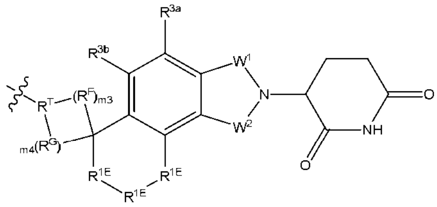,

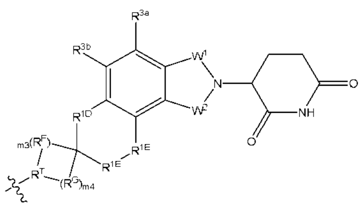, 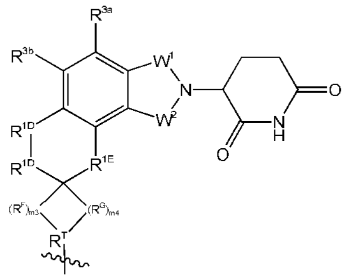, 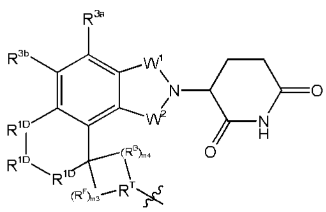,

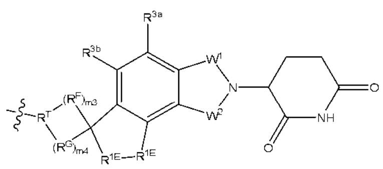, 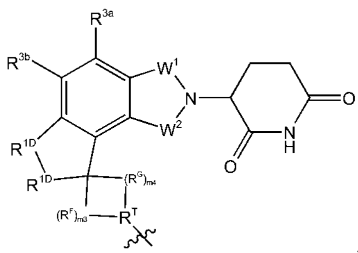 and 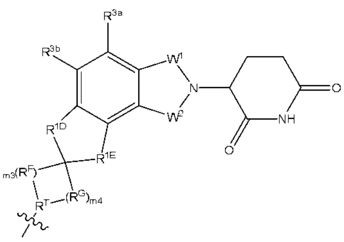,

wherein, $W^1$, $W^2$, $R^{3a}$, $R^{3b}$, $R^{3c}$, $R^{3a}$, $R^f$, $R^t$, $R^g$, $R^F$, $R^G$, m3, m4, m5, and m6 are as defined in claim 1, 2, 3, or 4; each occurrence of $R^{1d}$, $R^{1e}$, $R^{1D}$, and $R^{1E}$ are each independently $C(R^m)_2$; $R^T$ is N or $CR^{2h}$, and when both $R^F$ and $R^G$ are $C(R^m)_2$, $R^T$ is $CR^{2h}$; and $R^{2h}$ and $R^m$ are as defined in claims 1, 2, 3, or 4;

Preferably,

$R^{3a}$, $R^{3b}$, $R^{3c}$, $R^{3d}$ are each independently selected from H, deuterium atom, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy,hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; and/or

Each occurrence of m3 is independently an integer of 0, 1, 2, 3, or 4, m4 is an integer of 1, 2, 3, 4, or 5; and $m3 + m4 \leq 5$; preferably, m3 + m4 = 2, m3 + m4 =3, or m3 + m4 =4; and/or

Each occurrence of m5 and m6 are each independently 0, 1, 2, 3, or 4, and $m5 + m6 \leq 4$, preferably m5 + m6 = 2 or m5 + m6 =3; and/or

$W^1$ and $W^2$ are identical or different and are each independently $CH_2$ or C(=O), and at least one of $W^1$ and $W^2$ is C(=O); and/or

Each occurrence of $R^{1d}$, $R^{1e}$, $R^{1D}$, $R^{1E}$, $R^f$, $R^g$, $R^F$, and $R^G$ are each independently $C(R^m)_2$, and each occurrence of $R^m$ is independently selected from H, deuterium atom, F, Cl, Br, I, $C_1$-$C_6$ alkyl, $C_1$-$C_6$ heteroalkyl, $C_2$-$C_6$ alkenyl, $C_2$-$C_6$

alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_5$-$C_{10}$ aryl, and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; and/or

Each occurrence of $R^t$ is independently N or $CR^{2h}$, each occurrence of $R^T$ is independently $CR^{2h}$, and $R^{1h}$, $R^{2h}$, and $R^{3h}$ are each independently selected from H, deuterium atom, F, Cl, Br, I, $C_1$-$C_6$ alkyl, $C_1$-$C_6$ deuterated alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl.

In one embodiment, the CLM is selected from the following structures:

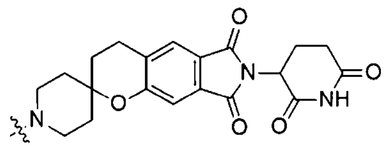,
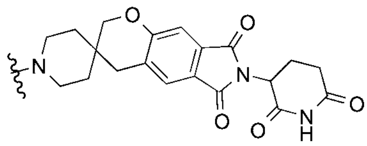,
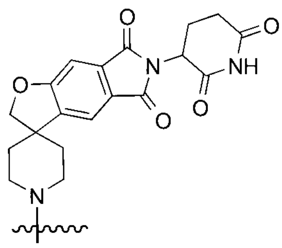,

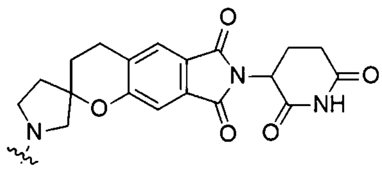,
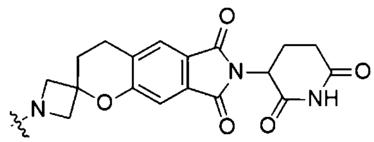,
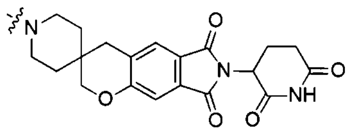,

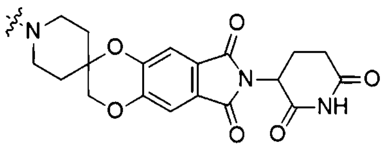,
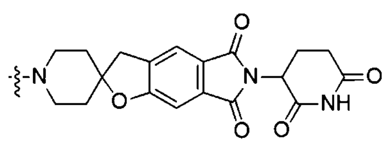,
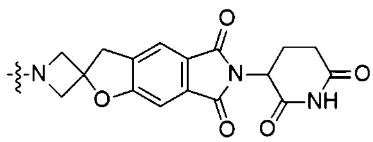,

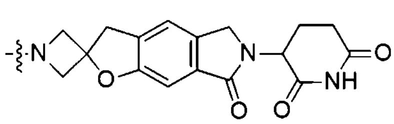,
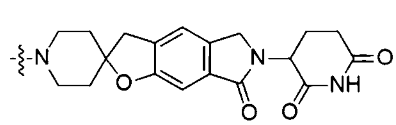,
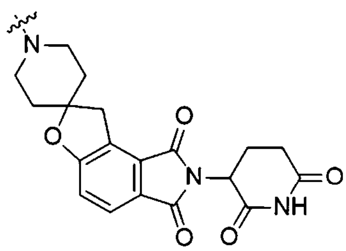,

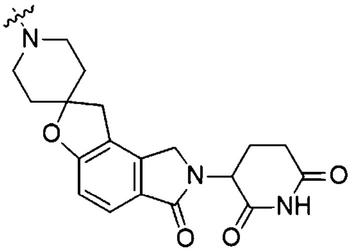 , 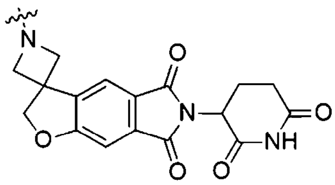 , 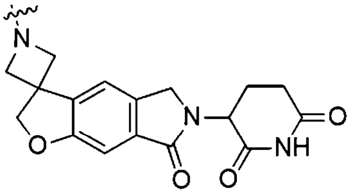 ,

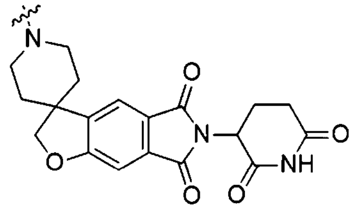 , 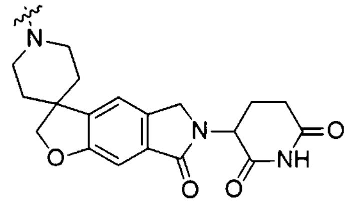 , 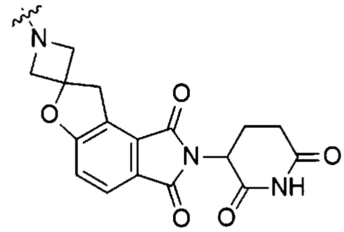 ,

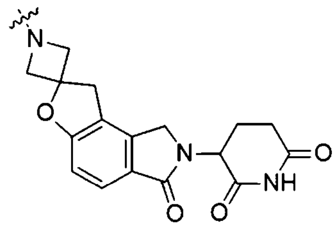 , 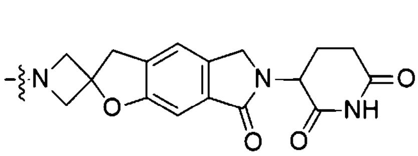 , 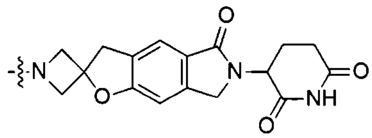 ,

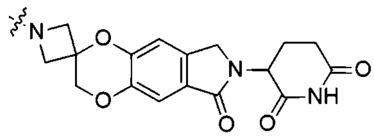 , 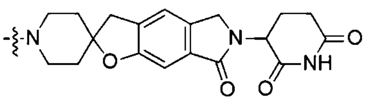 , 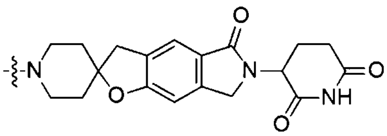 ,

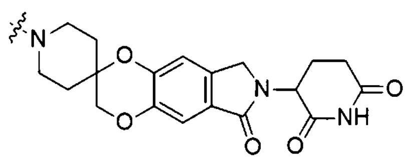 , 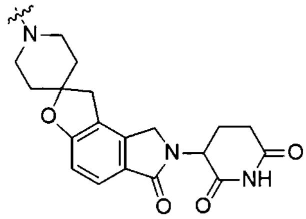 , 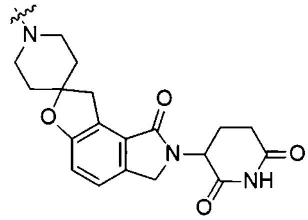 ,

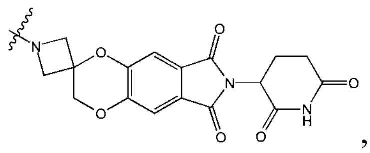 , 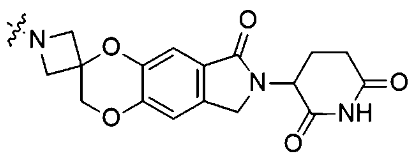 , 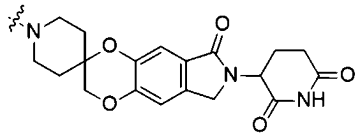 ,

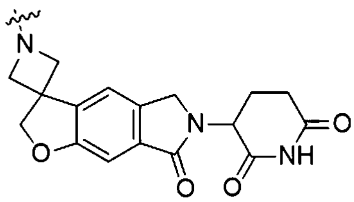 , 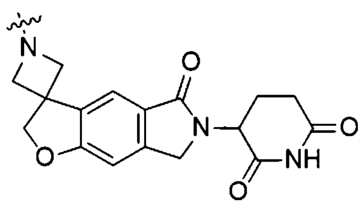 , 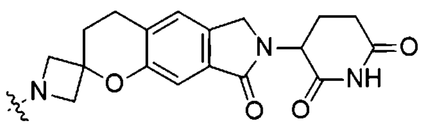 ,

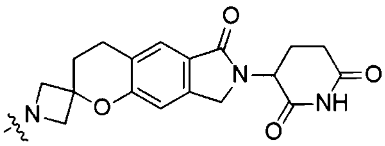,
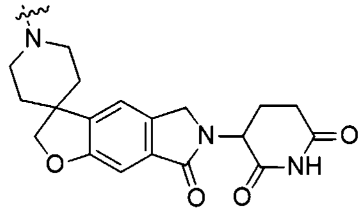,
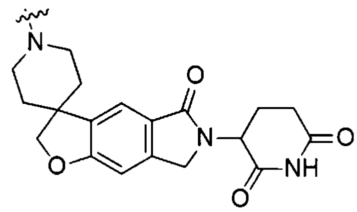,

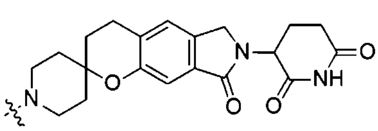,
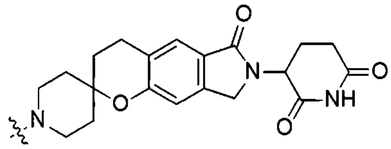,
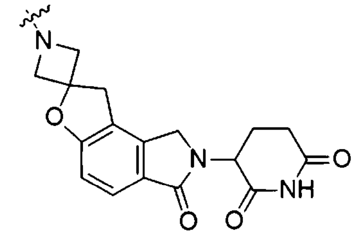,

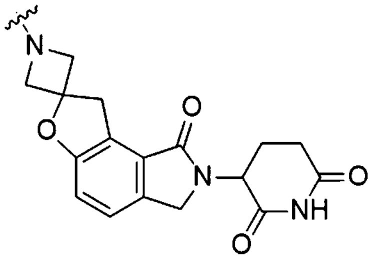,
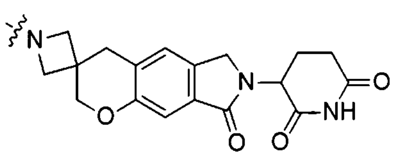,
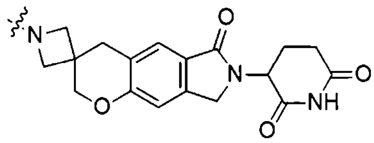,

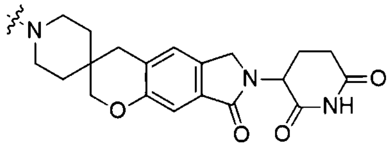,
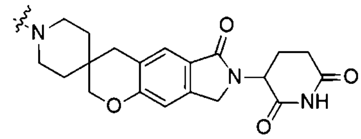,
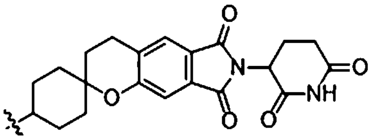,

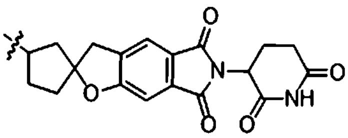,
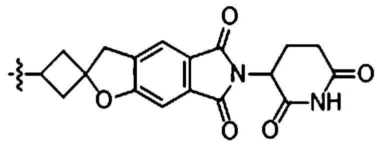,
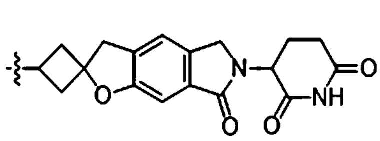,

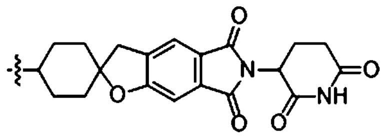,
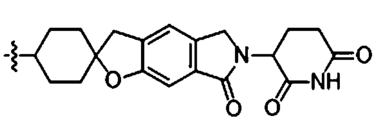,
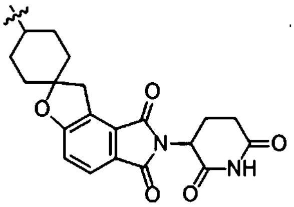,

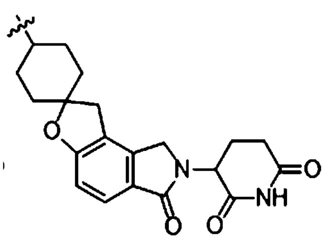,
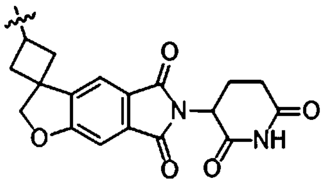,
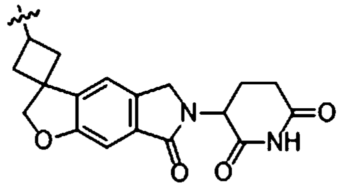,

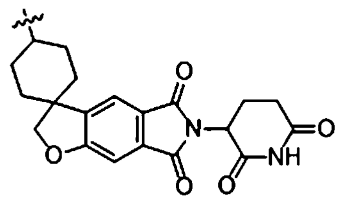,
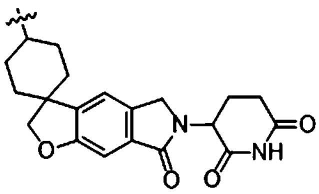,
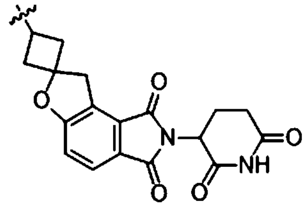,

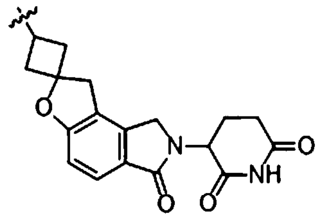,
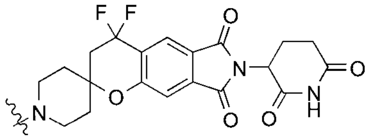,
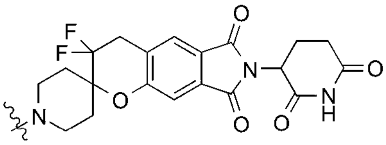,

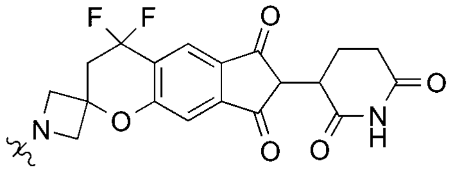,
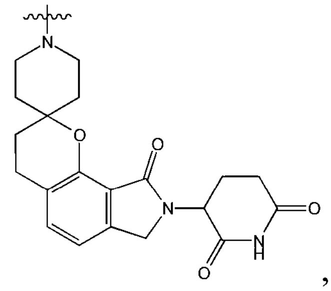,
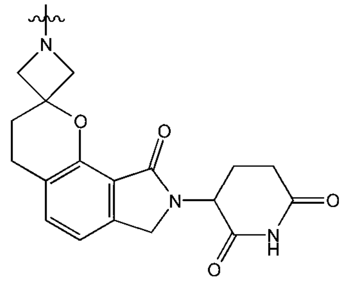,

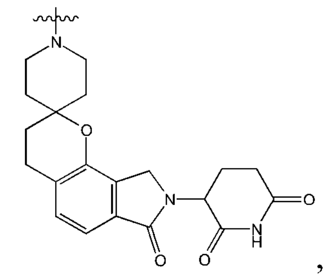,
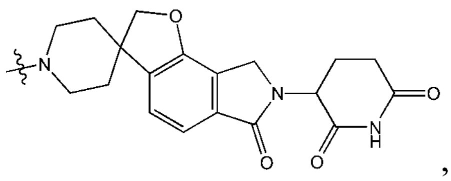,
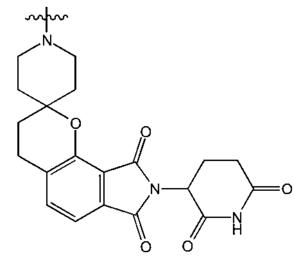,

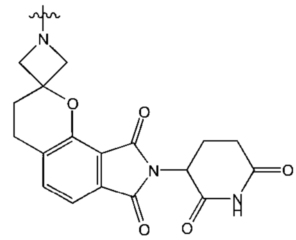,
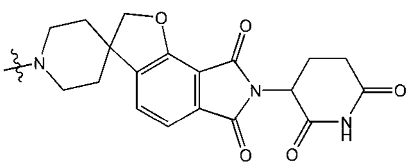,
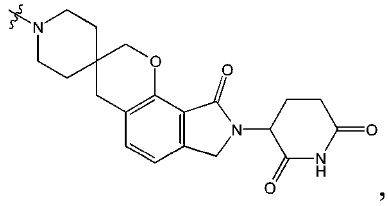,

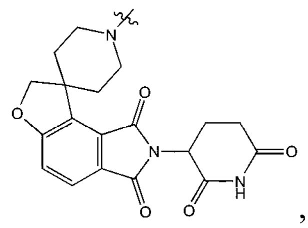,
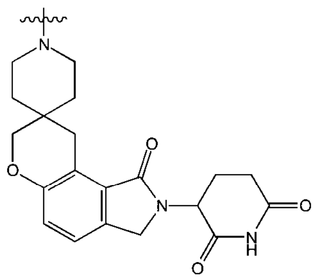,
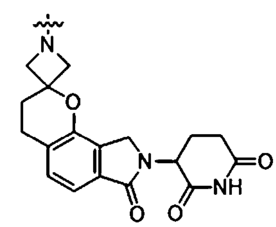,
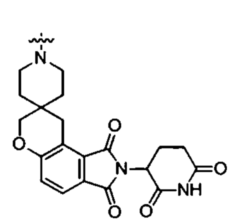,

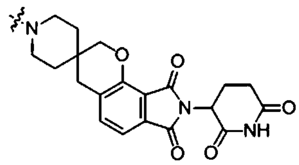, 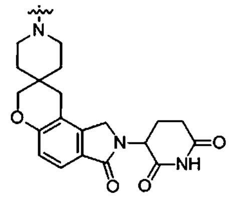, 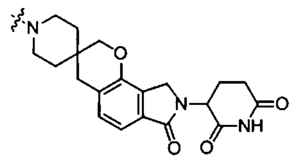, 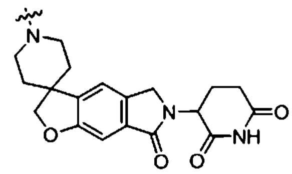,

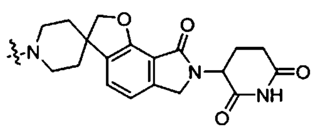, 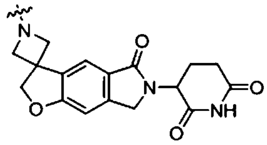, 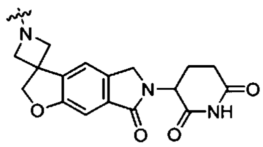, 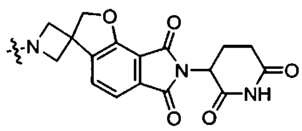,

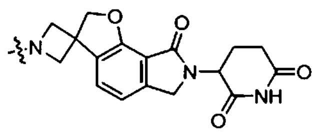, 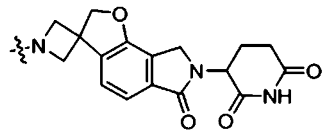, 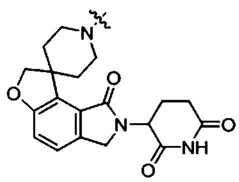, 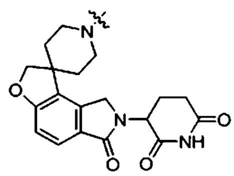,

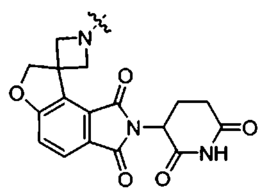, 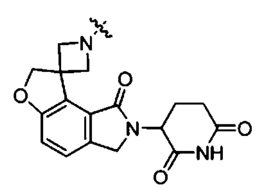, 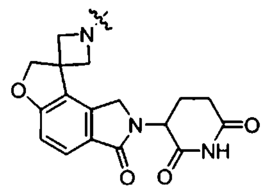, 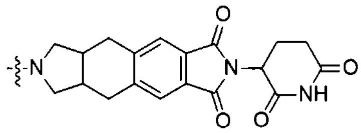,

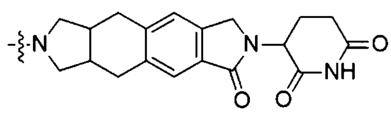, 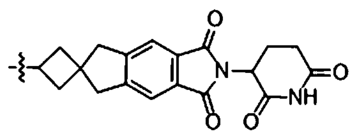, 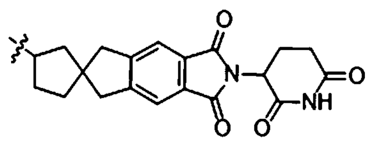,

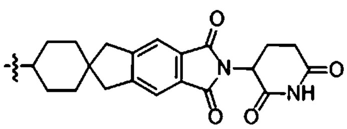, 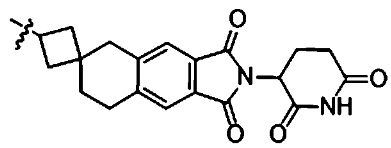, 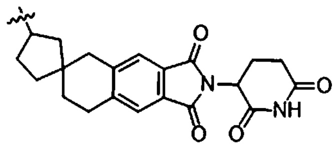,

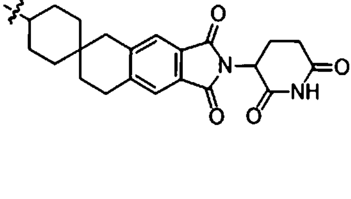, 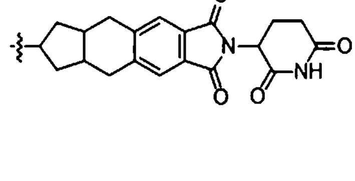, 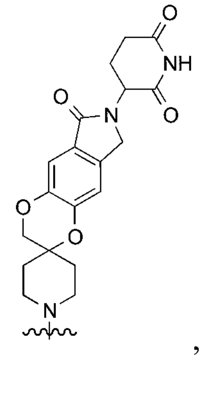,

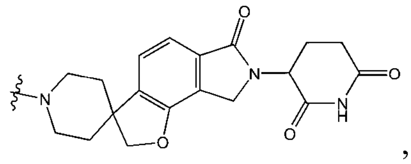 , 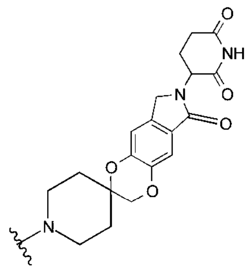 , 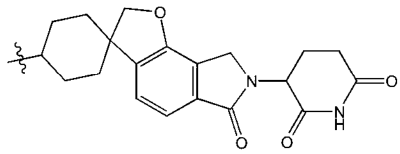 ,

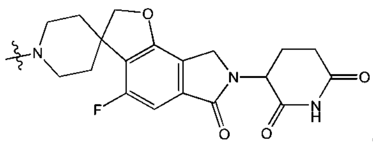 , and 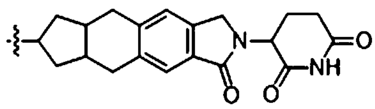 。

In one embodiment, L is a bond or $-(B^L)_q-$: each occurrence of $B^L$ is identical or different, and is each independently selected from: $CR^{L1}R^{L2}$, O, S, SO, $SO_2$, $NR^{L3}$, $SO_2NR^{L3}$, $SONR^{L3}$, $CONR^{L3}$, $NR^{L3}CONR^{L4}$, $NR^{L3}SO_2NR^{L4}$, CO, $CR^{L1}=CR^{L2}$, C≡C, $SiR^{L1}R^{L2}$, $P(O)R^{L1}$, $P(O)OR^{L1}$, $NR^{L3}C(=NCN)NR^{L4}$, $NR^{L3}C(=NCN)$, $NR^{L3}C(=CNO_2)NR^{L4}$, cycloalkylene, heterocyclylene, arylene, or heteroarylene, wherein the cycloalkylene, heterocyclylene, arylene, and heteroarylene are optionally substituted with 0-6 $R^{L1}$ and/or $R^{L2}$ groups;

Preferably, each occurrence of $B^L$ is independently selected from: $CR^{L1}R^{L1}$, O, S, SO, $SO_2$, $NR^{L3}$, CO, C≡C, 3-16-membered cycloalkylene , 3-16-membered heterocyclylene, 6-10-membered arylene, or 5-10-membered heteroarylene, wherein the 3-16-membered cycloalkylene , 3-16-membered heterocyclylene, 6-10-membered arylene, or 5-10-membered heteroarylene optionally substituted with 0, 1, 2, or 3 $R^{L1}$ and/or $R^{L2}$ groups;

Each occurrence of $R^{L1}$, $R^{L2}$, $R^{L3}$, and $R^{L4}$ are each independently selected from: H, halogen, $C_{1-8}$ alkyl, O-$C_{1-8}$ alkyl, S-$C_{1-8}$ alkyl, NH-$C_{1-8}$ alkyl, N($C_{1-8}$ alkyl)$_2$, $C_{3-11}$ cycloalkyl, aryl, heteroaryl, $C_{3-11}$ heterocyclyl, O-$C_{3-8}$ cycloalkyl, O-$C_{3-11}$ heterocyclyl, CO-$C_{3-8}$ cycloalkyl, CO-$C_{3-11}$ heterocyclyl, O-aryl, O-heteroaryl, S-$C_{3-8}$ cycloalkyl, NH-$C_{3-8}$ cycloalkyl, N($C_{3-8}$ cycloalkyl)$_2$, N($C_{3-8}$ cycloalkyl)($C_{1-8}$ alkyl), N($C_{1-8}$ alkylene)($C_{3-8}$ cycloalkyl), NH-$C_{3-8}$ heterocyclyl, N($C_{3-8}$ heterocyclyl)$_2$, N($C_{3-8}$ heterocyclyl)($C_{1-8}$ alkyl), NH-aryl, N(aryl)($C_{1-8}$ alkyl), NH-heteroaryl, N(heteroaryl)($C_{1-8}$ alkyl), OH, $NH_2$, SH, $SO_2$ P(O)(O-$C_{1-8}$ alkyl)($C_{1-8}$ alkyl), P(O)(O-$C_{1-8}$ alkyl)$_2$, C≡C-$C_{1-8}$ alkyl, C≡CH, CH=CH-($C_{1-8}$ alkyl), C($C_{1-8}$ alkyl)=CH-($C_{1-8}$ alkyl), C($C_{1-8}$ alkyl)=C($C_{1-8}$ alkyl)$_2$, $Si(OH)_3$, Si($C_{1-8}$ alkyl)$_3$, Si(OH)($C_{1-8}$ alkyl)$_2$, CO-$C_{1-8}$ alkyl, COO-$C_{1-8}$ alkyl, $CO_2H$, CN, $CF_3$, $CHF_2$, $CH_2F$, $NO_2$, $SF_5$, $SO_2NH$-$C_{1-8}$ alkyl, $SO_2N$($C_{1-8}$ alkyl)$_2$, SONH-$C_{1-8}$ alkyl, SON($C_{1-8}$ alkyl)$_2$, CONH-$C_{1-8}$ alkyl, CONH-$C_{3-8}$ cycloalkyl, CONH-$C_{3-11}$ heterocyclyl, CON($C_{1-8}$ alkyl)$_2$, N($C_{1-8}$ alkyl)CONH($C_{1-8}$ alkyl), N($C_{1-8}$ alkyl)CON($C_{1-8}$ alkyl)$_2$, NHCONH($C_{1-8}$ alkyl), NHCON($C_{1-8}$ alkyl)$_2$, $NHCONH_2$, N($C_{1-8}$ alkyl)$SO_2NH$($C_{1-8}$ alkyl), N($C_{1-8}$ alkyl)$SO_2N$($C_{1-8}$ alkyl)$_2$, $NHSO_2NH$($C_{1-8}$ alkyl), $NHSO_2N$($C_{1-8}$ alkyl)$_2$, and $NHSO_2NH_2$, optionally, the $C_{1-8}$ alkyl, $C_{3-11}$ cycloalkyl, $C_{3-8}$ cycloalkyl, $C_{3-11}$ heterocyclyl, $C_{6-10}$ aryl, and 5-10 membered heteroaryl are each independently substituted with one or more substituents selected from: halogen, alkyl, heteroalkyl, alkenyl, alkynyl, alkoxy, hydroxyl, haloalkyl, hydroxyalkyl, cyano, amino, nitro, cycloalkyl, heterocyclyl, halocycloalkyl, haloheteroalkyl, alkylamino, aryl, heteroaryl, haloaryl, and haloheteroaryl; preferably, the $C_{1-8}$ alkyl, $C_{3-11}$ cycloalkyl, $C_{3-8}$ cycloalkyl, $C_{3-11}$ heterocyclyl, $C_{6-10}$ aryl, and 5-10 membered heteroaryl are each independently substituted with one or more substituents selected from F, Cl, Br, I, $C_{1-6}$ alkyl, methoxy, ethoxy; and

q is an integer greater than or equal to 1; preferably, q is 1, 2, 3, 4, 5, 6, 7, 8, 9, 10, 11, 12, 13, 14, 15, 16, 17, 18, 19, or 20;

Preferably:

each occurrence of $B^L$ is independently identical or different and is selected from: $CR^{L1}R^{L2}$, O, S, SO, $SO_2$, $NR^{L3}$, $SO_2NR^{L3}$, $SONR^{L3}$, $CONR^{L3}$, $NR^{L3}CONR^{L4}$, $NR^{L3}SO_2NR^{L4}$, CO, $CR^{L1}=CR^{L2}$, C≡C, $SiR^{L1}R^{L2}$, $P(O)R^{L1}$, $P(O)OR^{L1}$, $NR^{L3}C(=NCN)NR^{L4}$, $NR^{L3}C(=NCN)$, $NR^{L3}C(=CNO_2)NR^{L4}$, cycloalkylene, heterocyclylene, arylene, or heteroarylene, wherein the cycloalkylene, heterocyclylene, arylene, and heteroarylene are optionally substituted with 0-6 $R^{L1}$ and/or $R^{L2}$ groups;

each occurrence of $R^{L1}$, $R^{L2}$, $R^{L3}$, and $R^{L4}$ are independently selected from: H, halogen, $C_{1-8}$ alkyl, O-$C_{1-8}$ alkyl, S-$C_{1-8}$ alkyl, NH-$C_{1-8}$ alkyl, N($C_{1-8}$ alkyl)$_2$, $C_{3-11}$ cycloalkyl, aryl, heteroaryl, $C_{3-11}$ heterocyclyl, O-$C_{3-8}$ cycloalkyl, O-$C_{3-11}$ heterocyclyl, O-aryl, O-heteroaryl, S-$C_{3-8}$ cycloalkyl, NH-$C_{3-8}$ cycloalkyl, N($C_{3-8}$ cycloalkyl)$_2$, N($C_{3-8}$ cycloalkyl)($C_{1-8}$ alkyl), NH-$C_{3-8}$ heterocyclyl, N($C_{3-8}$ heterocyclyl)$_2$, N($C_{3-8}$ heterocyclyl)($C_{1-8}$ alkyl), NH-aryl, N(aryl)($C_{1-8}$ alkyl), NH-heteroaryl, N(heteroaryl)($C_{1-8}$ alkyl), OH, $NH_2$, SH, $SO_2$, P(O)(O-$C_{1-8}$ alkyl)($C_{1-8}$ alkyl), P(O)(O-$C_{1-8}$ alkyl)$_2$, C≡C-$C_{1-8}$ alkyl, C≡CH, CH=CH-($C_{1-8}$ alkyl), C($C_{1-8}$ alkyl)=CH-($C_{1-8}$ alkyl), C($C_{1-8}$ alkyl) =C($C_{1-8}$ alkyl)$_2$, $Si(OH)_3$, Si($C_{1-8}$ alkyl)$_3$, Si(OH)($C_{1-8}$ alkyl)$_2$, CO-$C_{1-8}$ alkyl, $CO_2H$, CN, $CF_3$, $CHF_2$, $CH_2F$, $NO_2$,

$SF_5$, $SO_2NH-C_{1-8}$ alkyl, $SO_2N(C_{1-8}$ alkyl$)_2$, $SONH-C_{1-8}$ alkyl, $SON(C_{1-8}$ alkyl$)_2$, $CONH-C_{1-8}$ alkyl, $CON(C_{1-8}$ alkyl$)_2$, $N(C_{1-8}$ alkyl)$CONH(C_{1-8}$ alkyl), $N(C_{1-8}$ alkyl)$CON(C_{1-8}$ alkyl$)_2$, $NHCONH(C_{1-8}$ alkyl), $NHCON(C_{1-8}$ alkyl$)_2$, $NHCONH_2$, $N(C_{1-8}$ alkyl)$SO_2NH(C_{1-8}$ alkyl), $N(C_{1-8}$ alkyl)$SO_2N(C_{1-8}$ alkyl$)_2$, $NHSO_2NH(C_{1-8}$ alkyl), $NHSO_2N(C_{1-8}$ alkyl$)_2$, and $NHSO_2NH_2$; Optionally, the $C_{1-8}$ alkyl, $C_{3-11}$ cycloalkyl, $C_{3-11}$ heterocyclyl, $C_{6-10}$ aryl, and $C_{5-10}$ heteroaryl are each independently substituted with one or more substituents selected from: halogen, alkyl, heteroalkyl, alkenyl, alkynyl, alkoxy, hydroxyl, haloalkyl, hydroxyalkyl, cyano, amino, nitro, cycloalkyl, heterocyclyl, halocycloalkyl, haloheteroalkyl, alkylamino, aryl, heteroaryl, haloaryl, and haloheteroaryl; and q is an integer greater than or equal to 1, preferably q is 1, 2, 3, 4, 5, 6, 7, 8, 9, 10, 11, 12, 13, 14, 15, 16, 17, 18, 19, or 20.

In one embodiment, $B^L$ is selected from one or more of the following structures: -O-, -S-, -SO-, $-SO_2-$, $-CH_2-$, -CO-, -NH-, $-CH(CH_3)-$, $-CH(CH_2CH_3)-$, $-C(CH_3)_2-$, $-N(CH_3)-$, $-N(CH_2CH_3)-$,

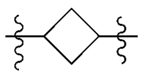, 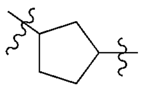,

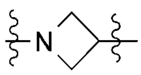, 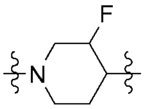, 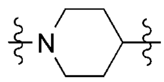, 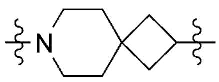, 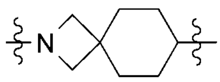,

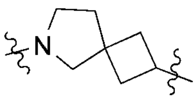, 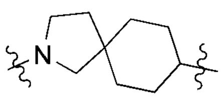, 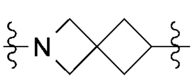, 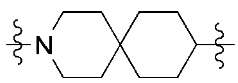, 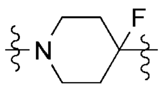,

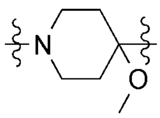, 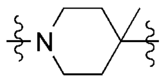, 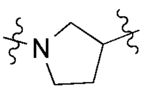, 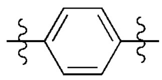, 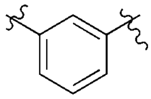, 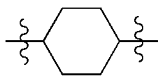,

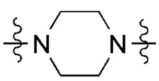, 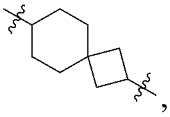, 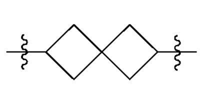, 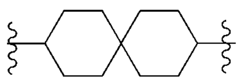, 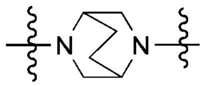,

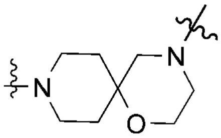, 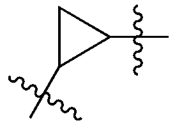, 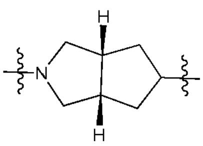, 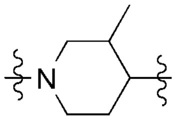, 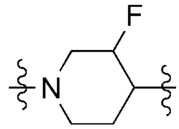,

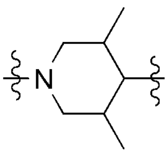, 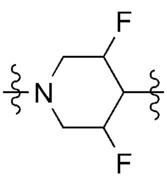, 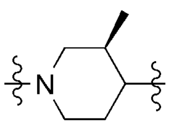, 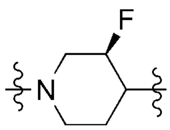, 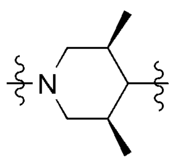, 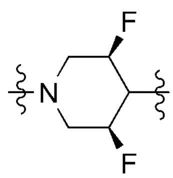,

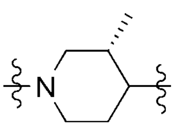, 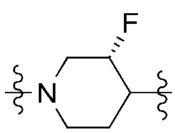, 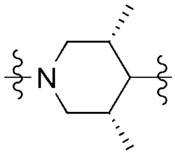, 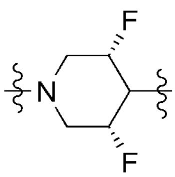, 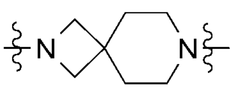,

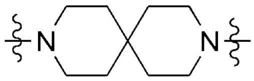, 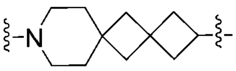 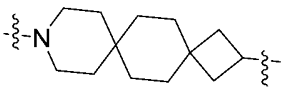, 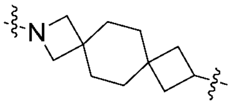, 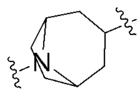,

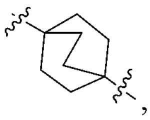, 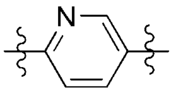, 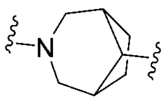, 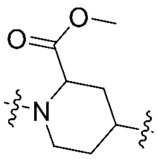, 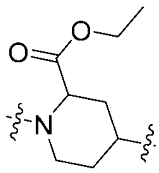, 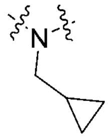, 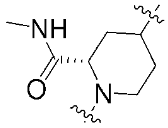,

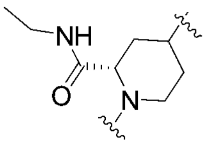, 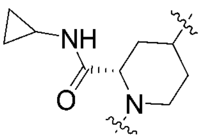, 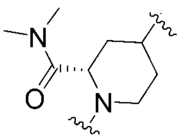, 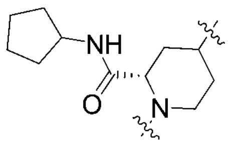, 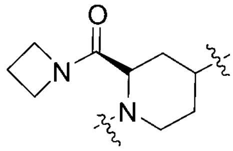,

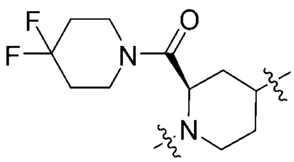, 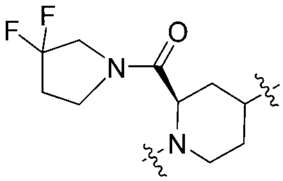, 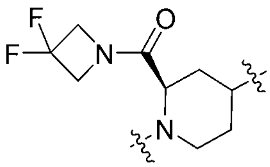, 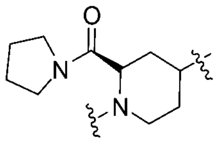,

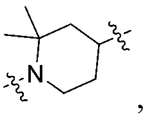, 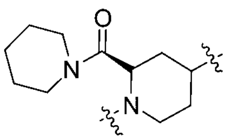, 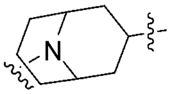, 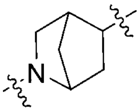, 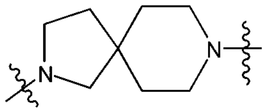,

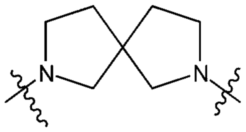, 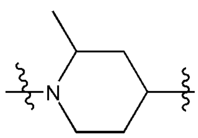, 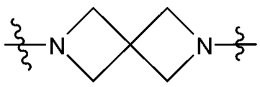, 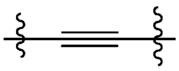, 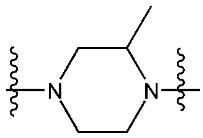,

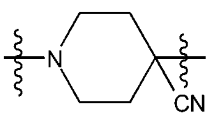, 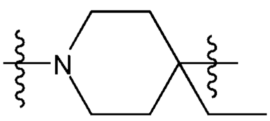 and 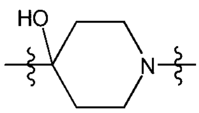,

wherein

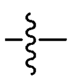

is the the point of attachment.

In one embodiment, L is selected from the following structures:

a covalent bond, $-(CH_2)_j-$, $-(CH_2)_p-NH-(CH_2)_s-$, $-(CH_2)_y-NH-(CH_2)_j-NH-(CH_2)_s-$, $-(CH_2)_p-CO-(CH_2)_s-$, $-(CH_2)_p-O-(CH_2)_s-$, $-(CH_2)_y-CO-(CH_2)_j-CO-(CH_2)_s-$, $-(CH_2)_y-O-(CH_2)_j-O-(CH_2)_s-$, $-(CH_2)_y-O-(CH_2)_j-CO-(CH_2)_s-$, $-(CH_2)_p-NH-(CH_2)_y-O-(CH_2)_j-CO-(CH_2)_s-$,

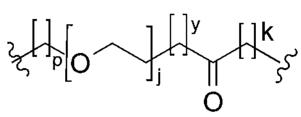 , 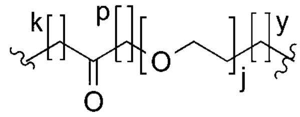 ,

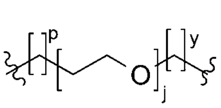 , 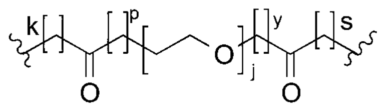 , 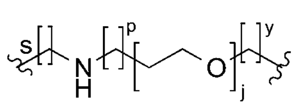 ,

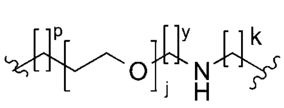 , 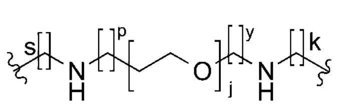 , 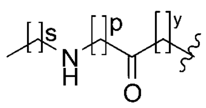 , 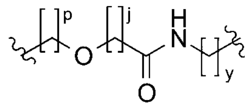 ,

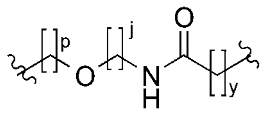 , 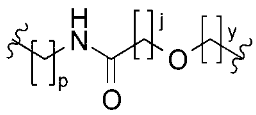 , 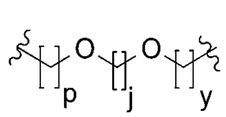 , 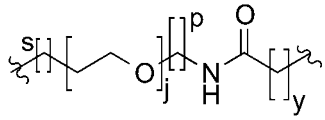 ,

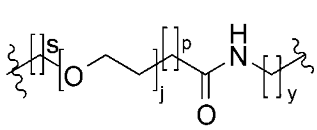 , 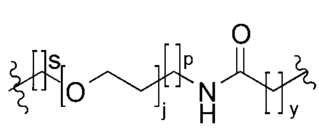 , 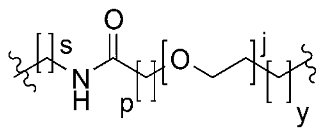 ,

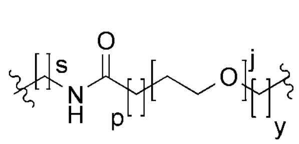 , 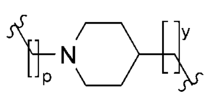 , 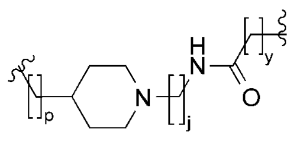 , 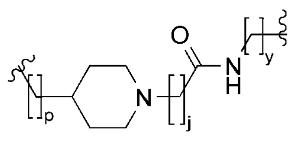 ,

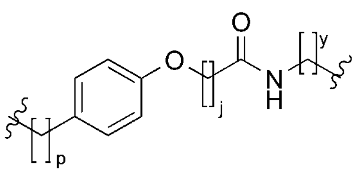 , 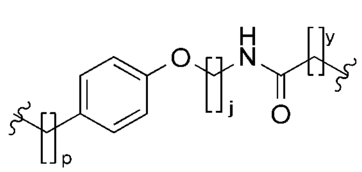 , 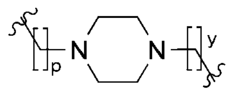 ,

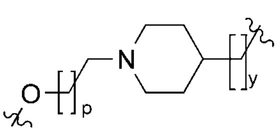 , 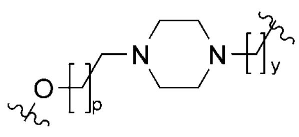 , 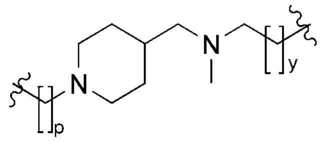 ,

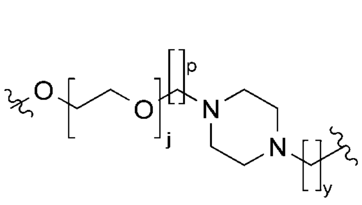 , 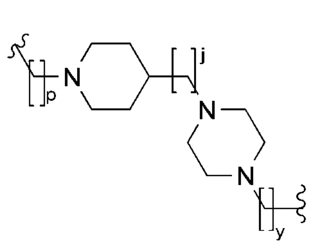 , 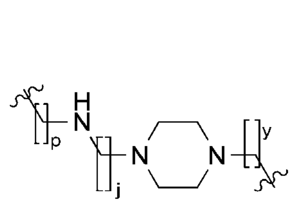 , ,

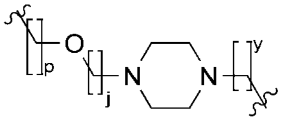, 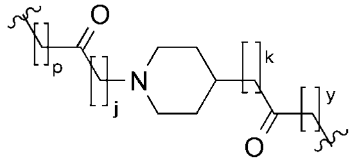, 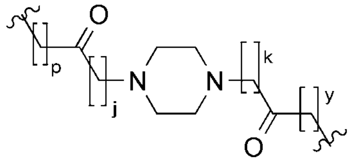,

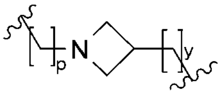, 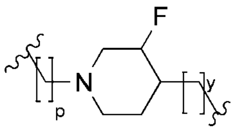, 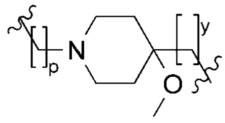, 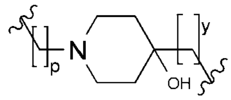,

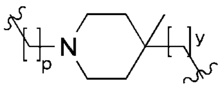, 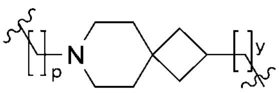, 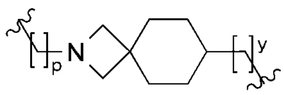, 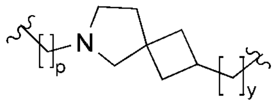,

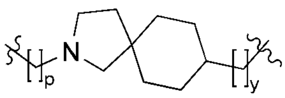, 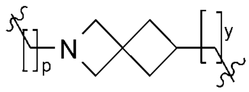, 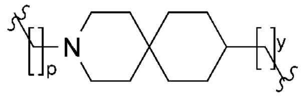, 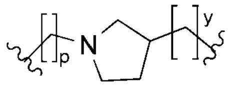,

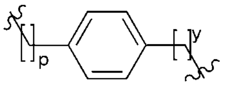, 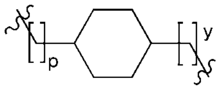, 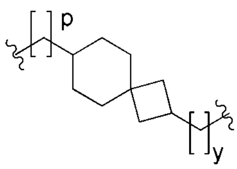, 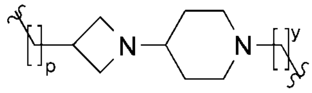,

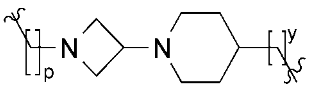, 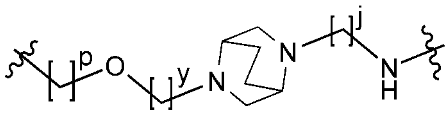,

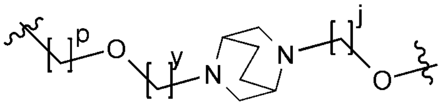, 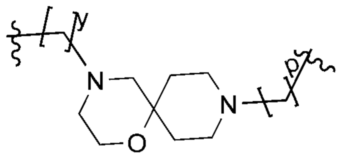, 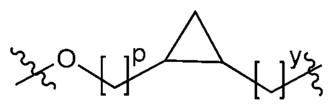,

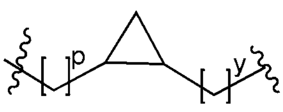, 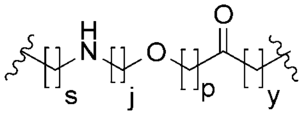, 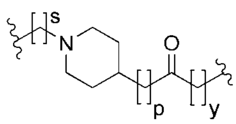, 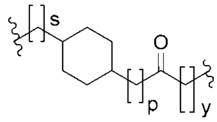,

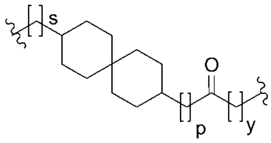, 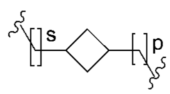, 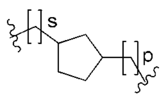, 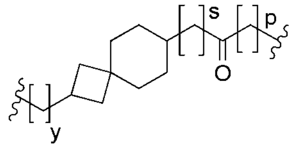,

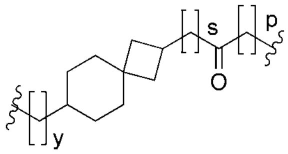, 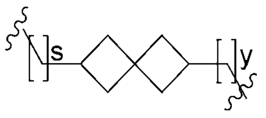, 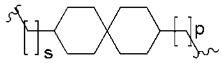, 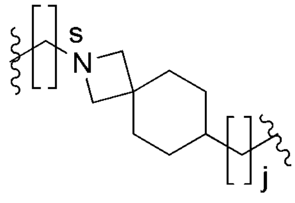,

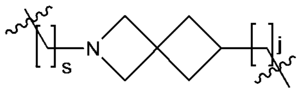, 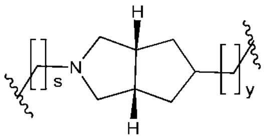, 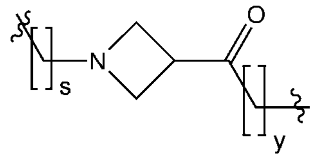,

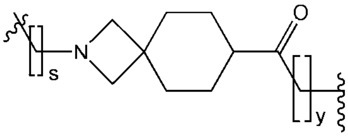, 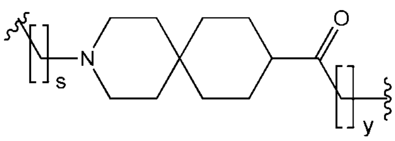, 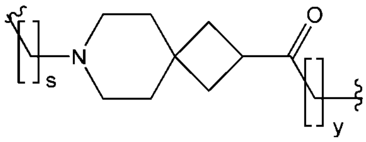,

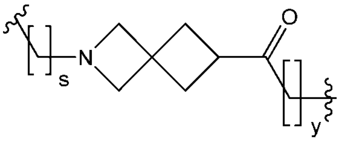, 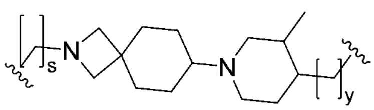 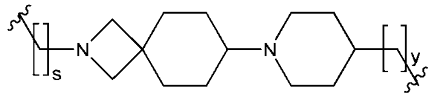,

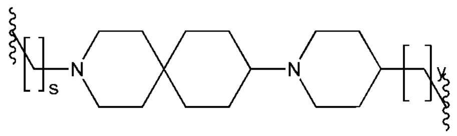, 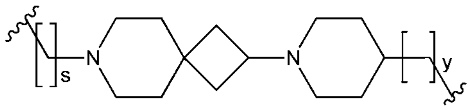,

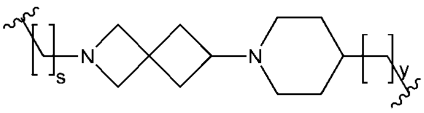, , 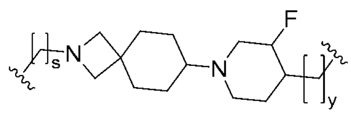, 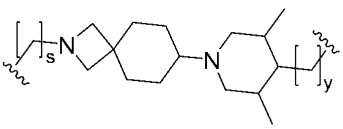,

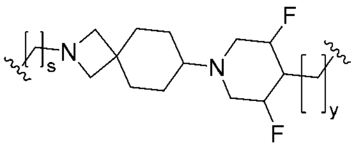, 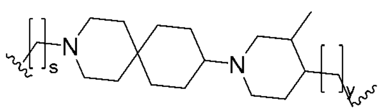, 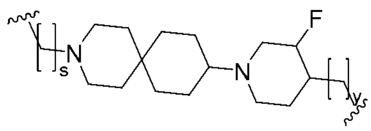,

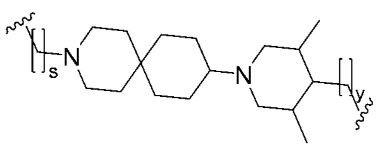, 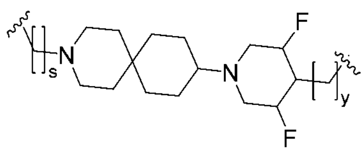, 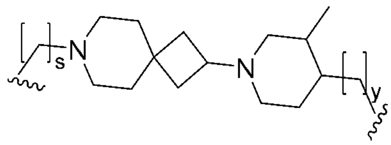,

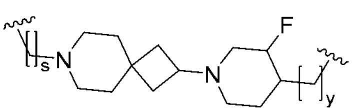, 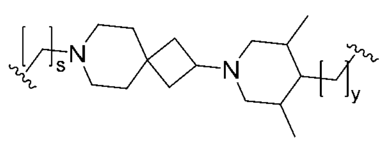, 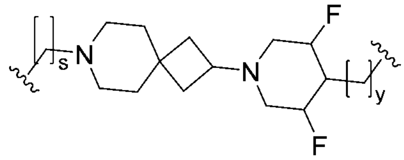,

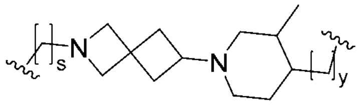, 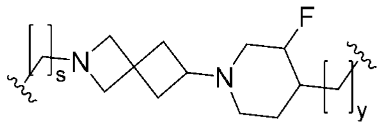, 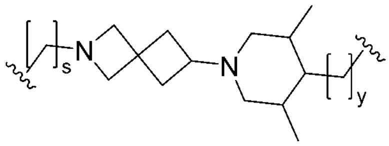,

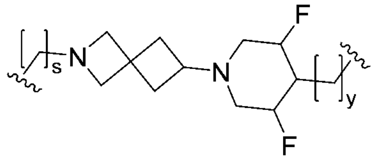, 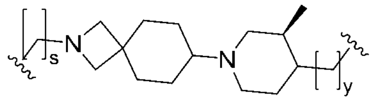, 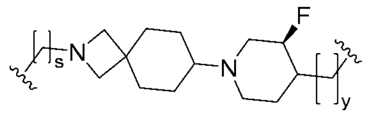,

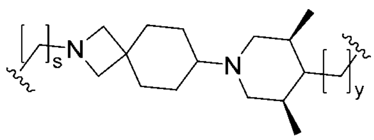, 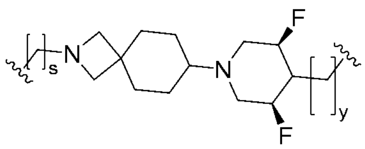, 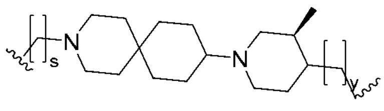,

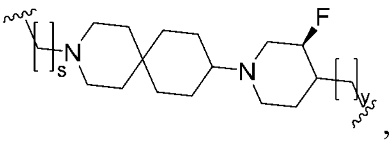, 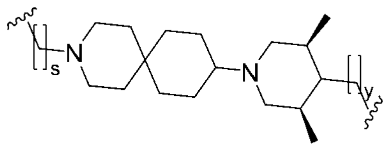, 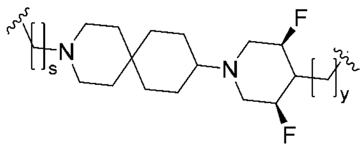,

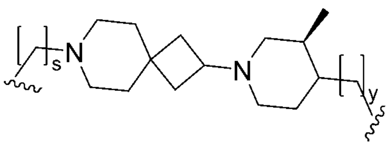, 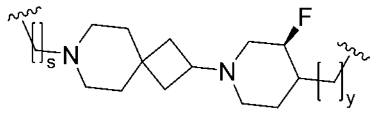, 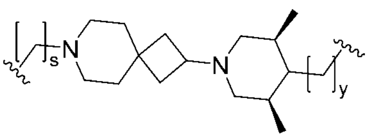,

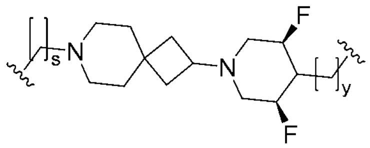, 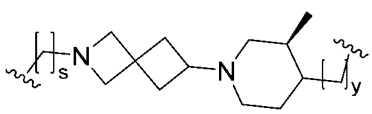, 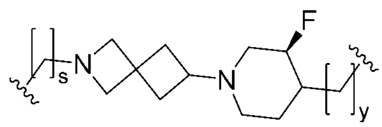,

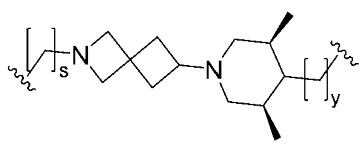, 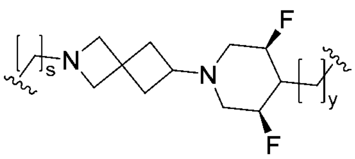, 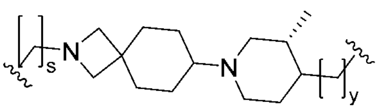,

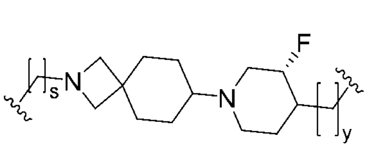, 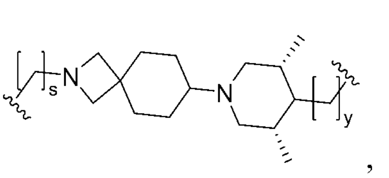, 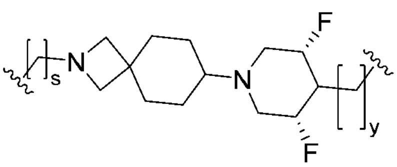,

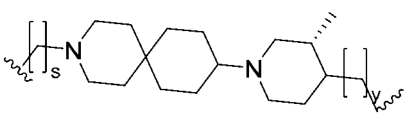, 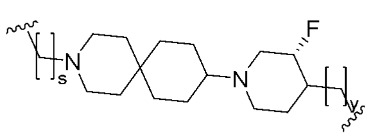, 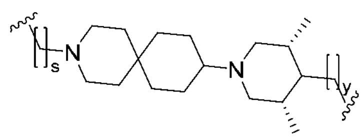,

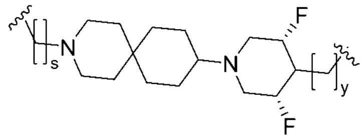, 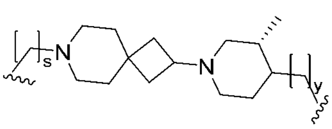, 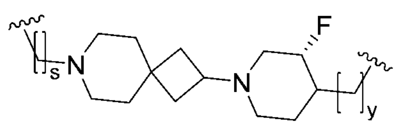,

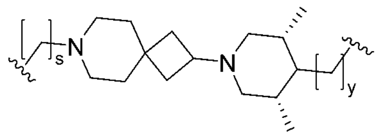, 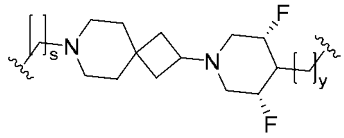, 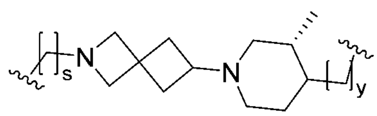,

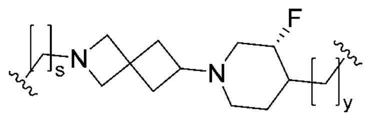, 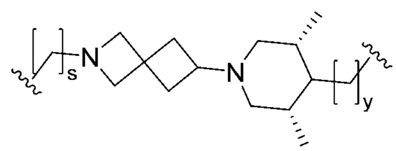, 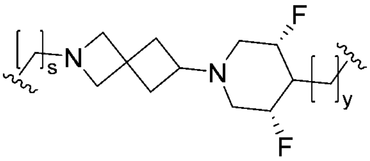,

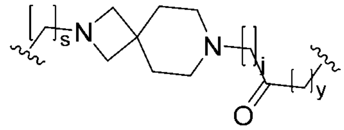, 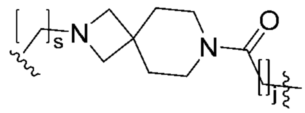, 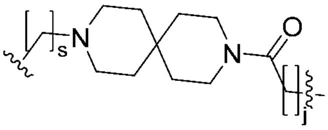,

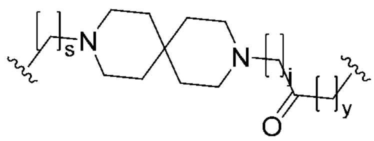, 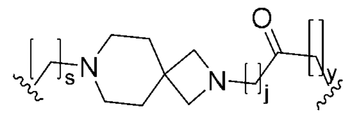, 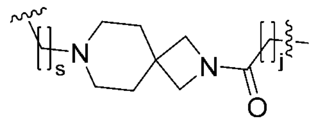,

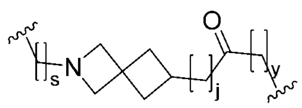, 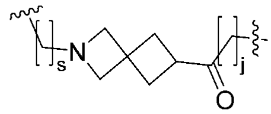, 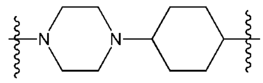, 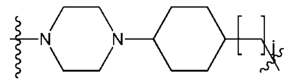,

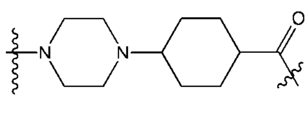, 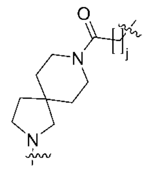, 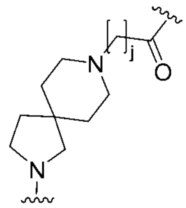, 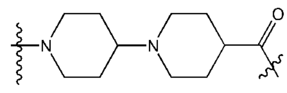,

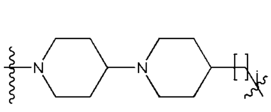, 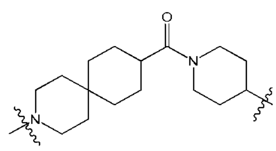, 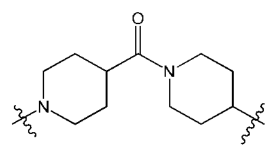, 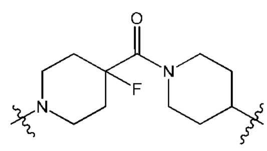,

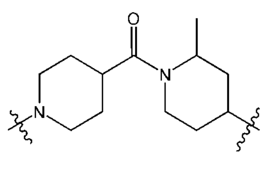, 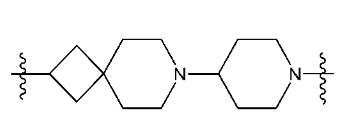, 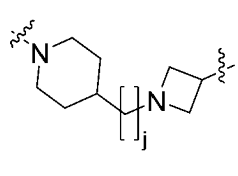, 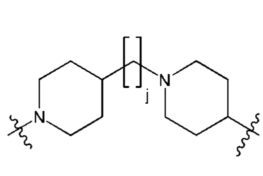,

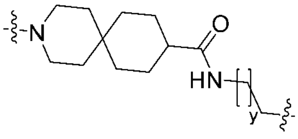, 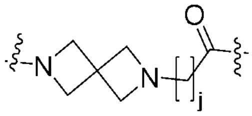, 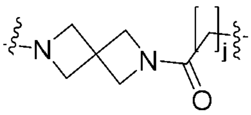, 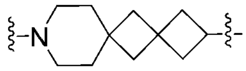

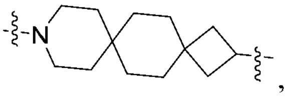, 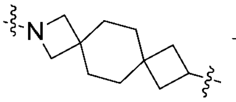 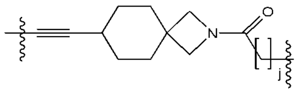, 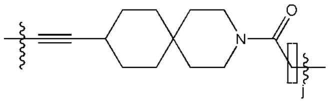,

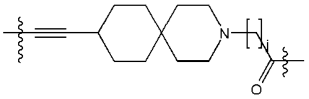, 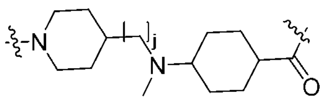, 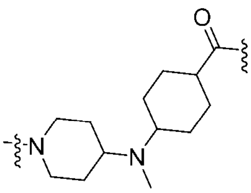, 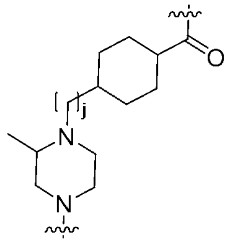,

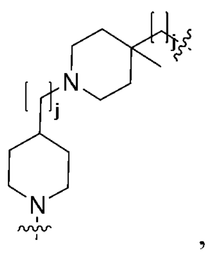, 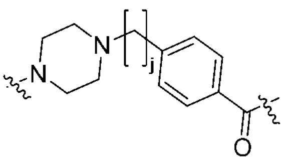, 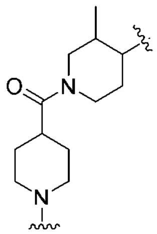, 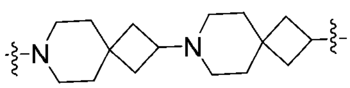,

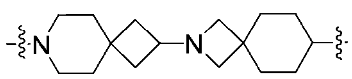, 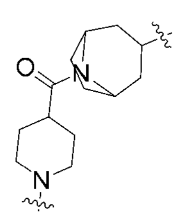, 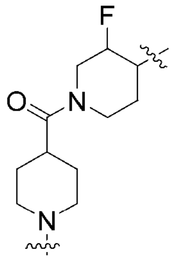, 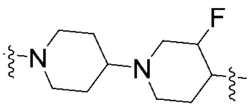,

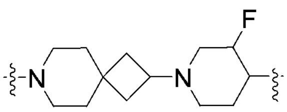, 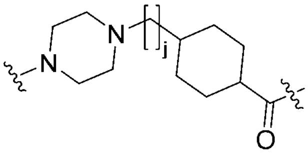, 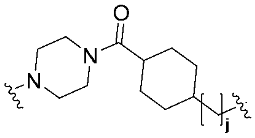, 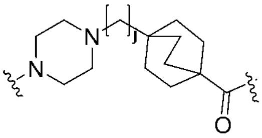,

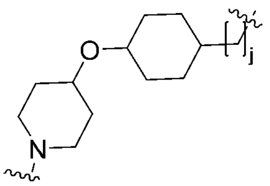, 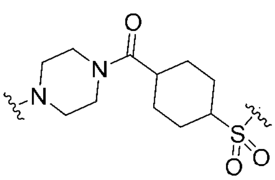, 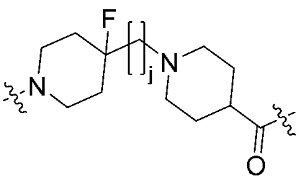, 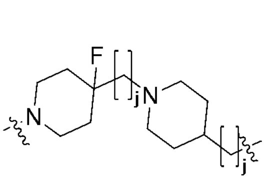,

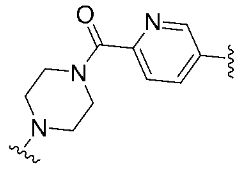, 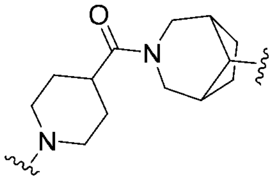, 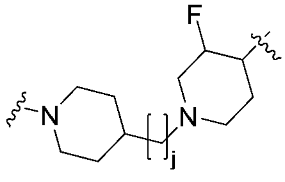, 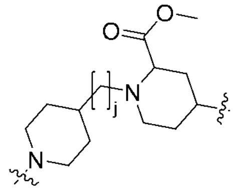,

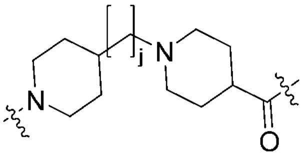, 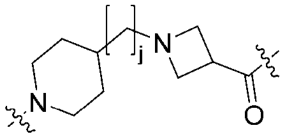, 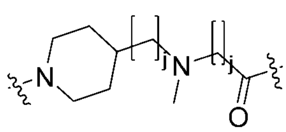, 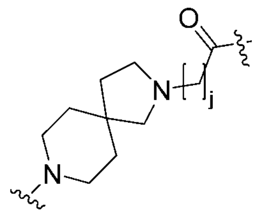

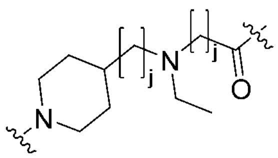 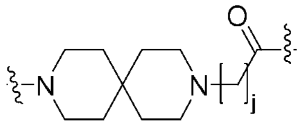 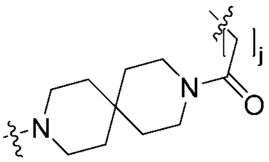 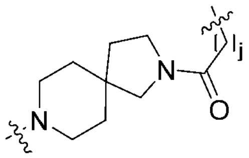

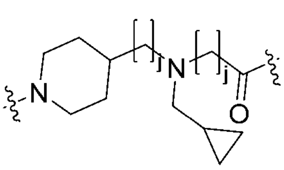 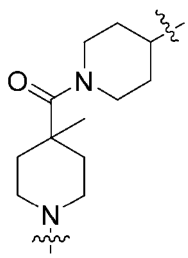 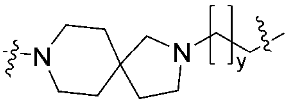, 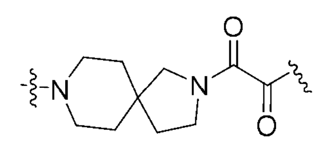,

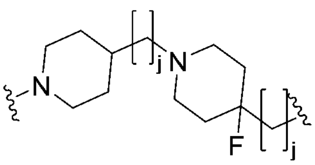, 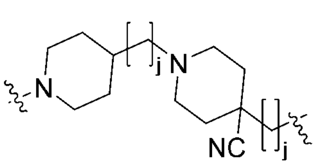, 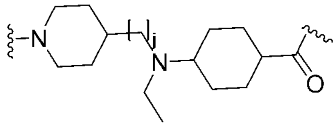,

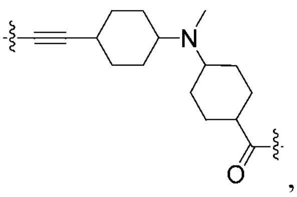, 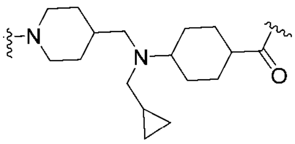, 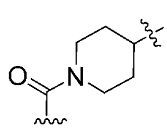, 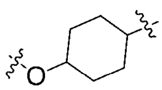, 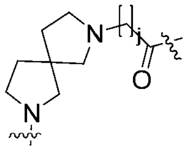,

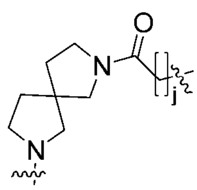, 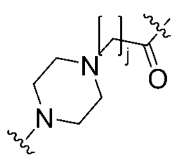, 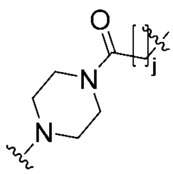, 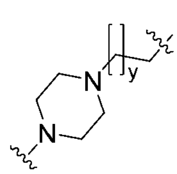, 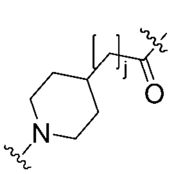, 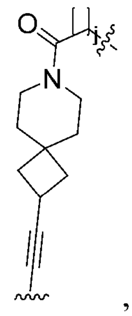,

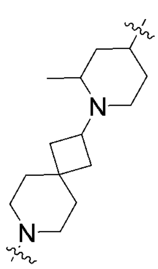, 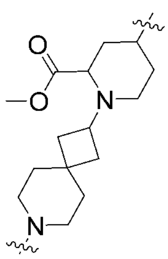, 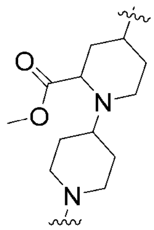, 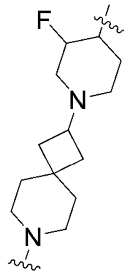, 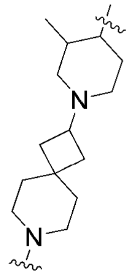, 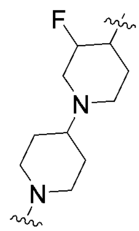, 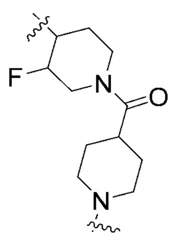,

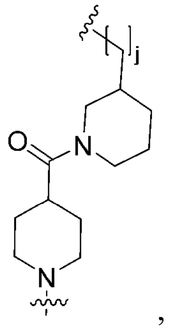, 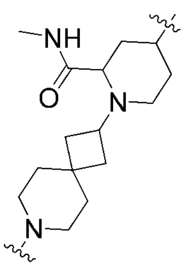, 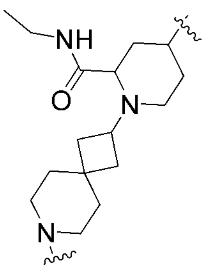, 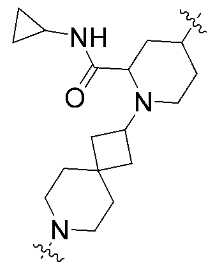, 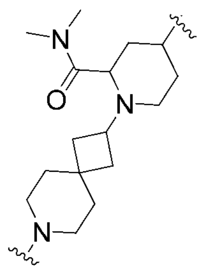,

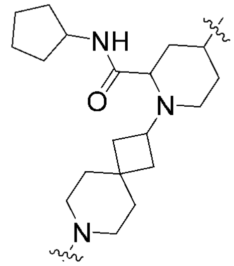, 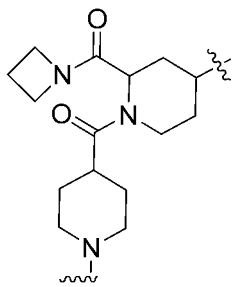, 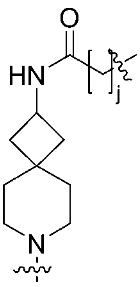, 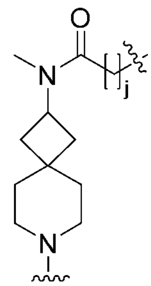, 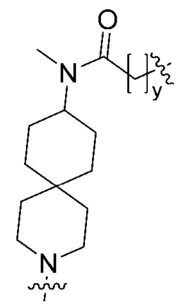, 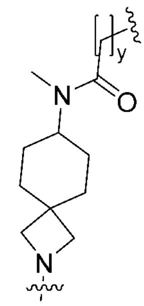,

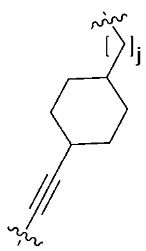, 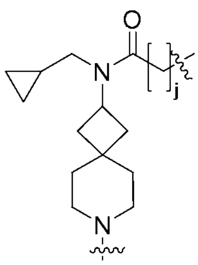, 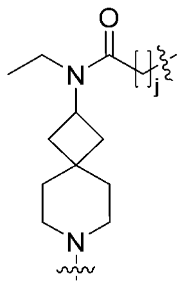, 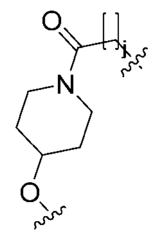, 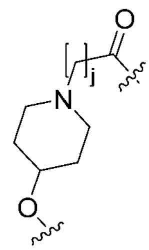, 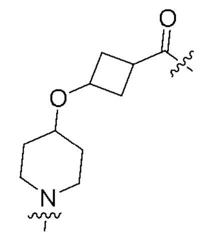,

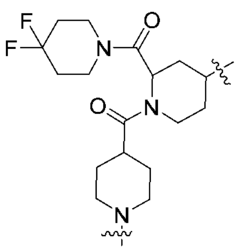, 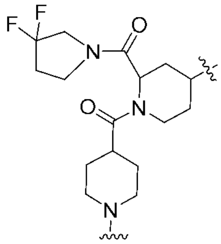, 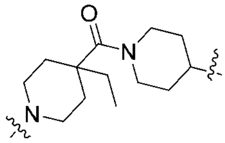, 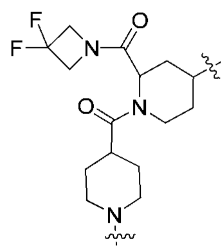,

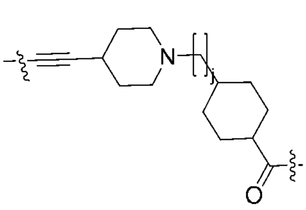,
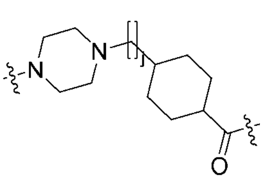,
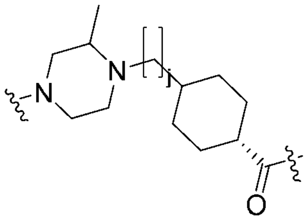
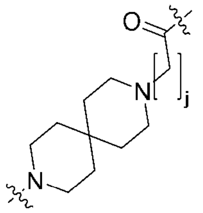

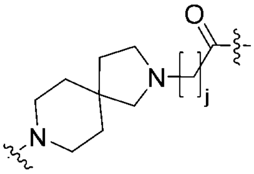
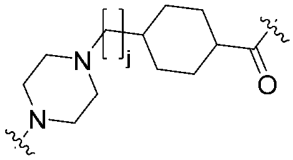
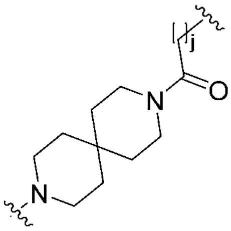
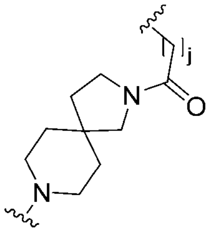
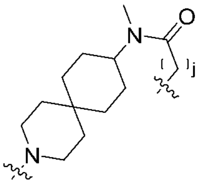

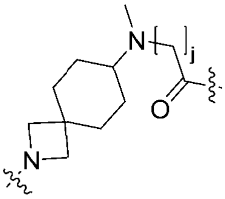
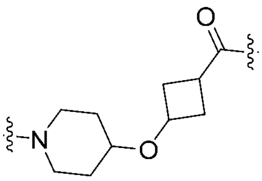,
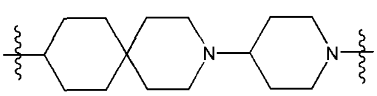,
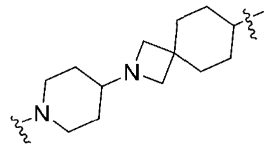,

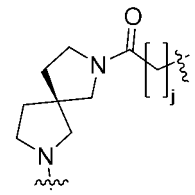,
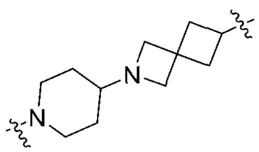,
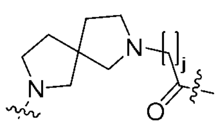,
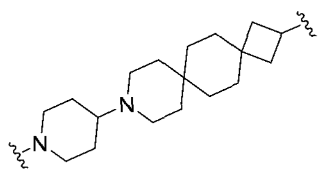,

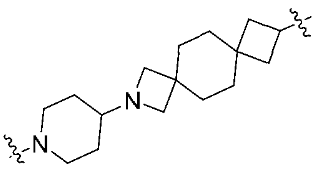,
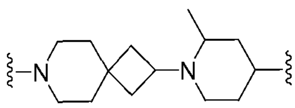,
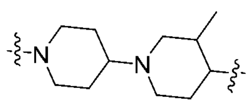
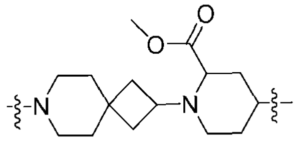

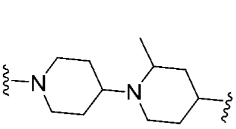
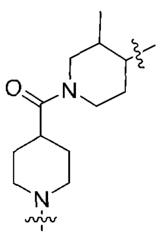
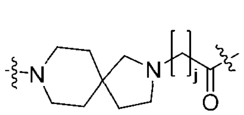
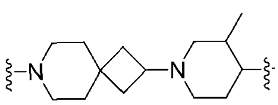
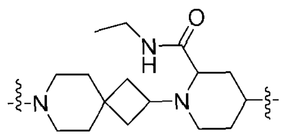

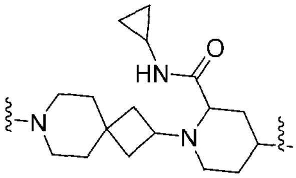
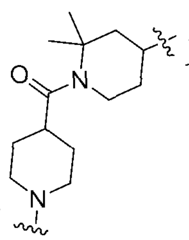
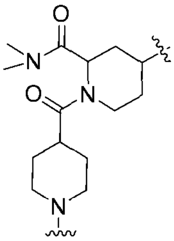
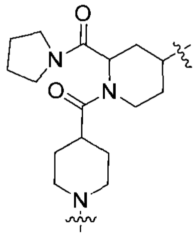
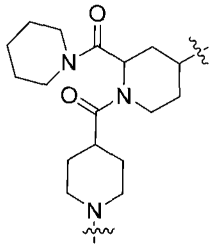

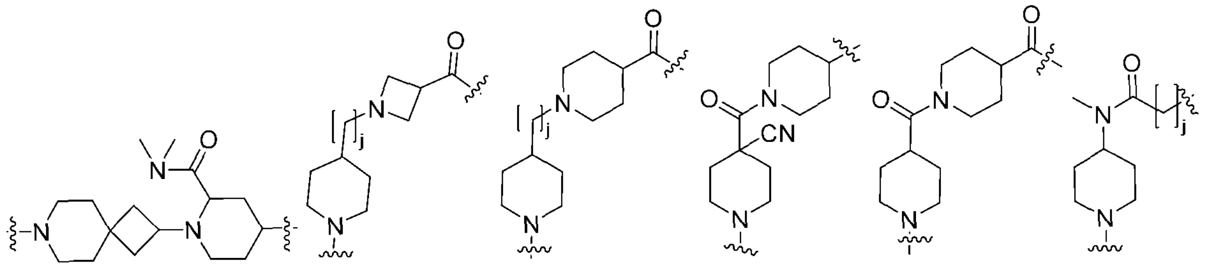

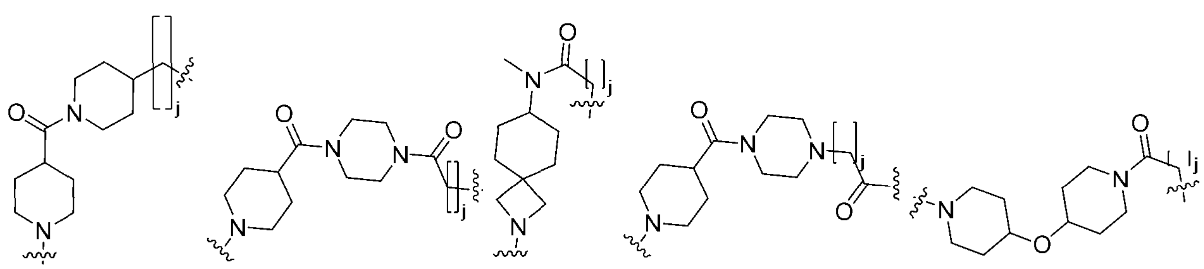

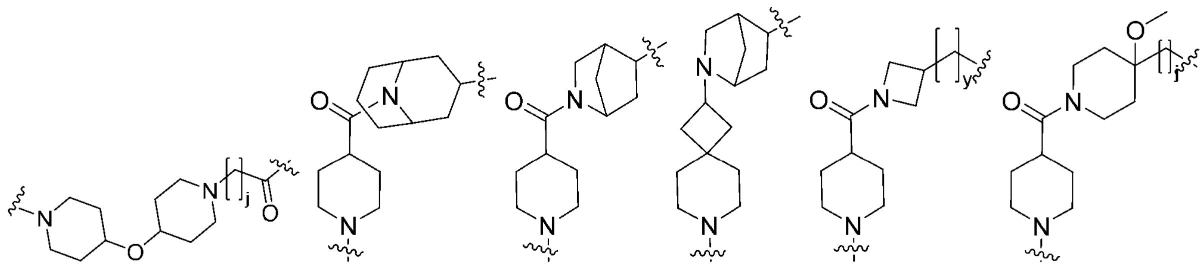

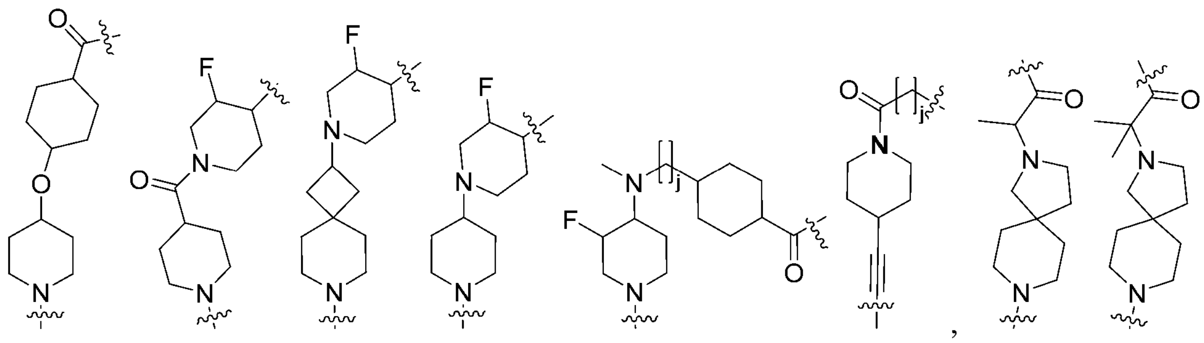

,

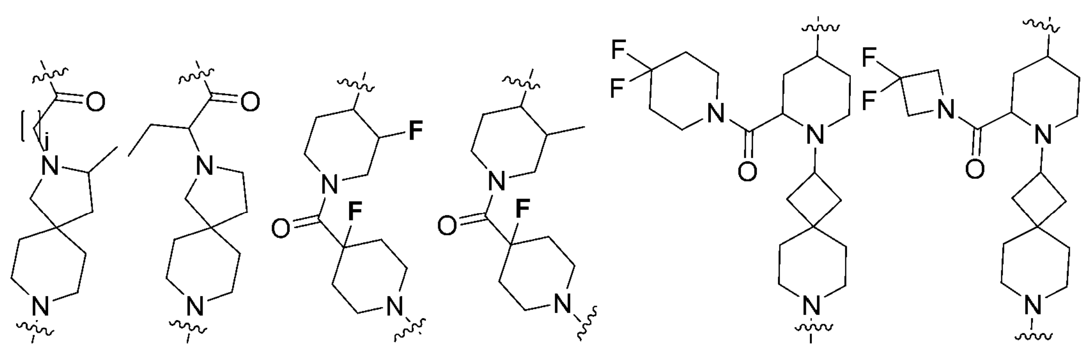

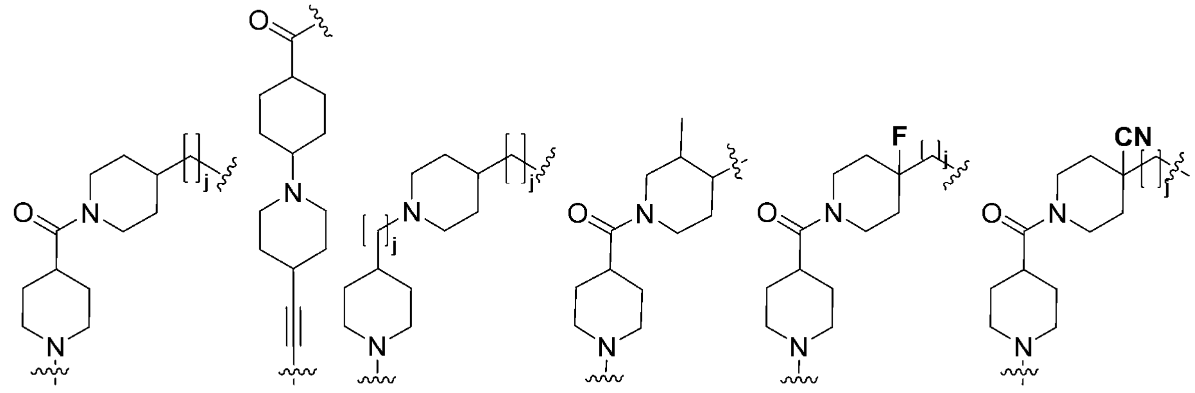

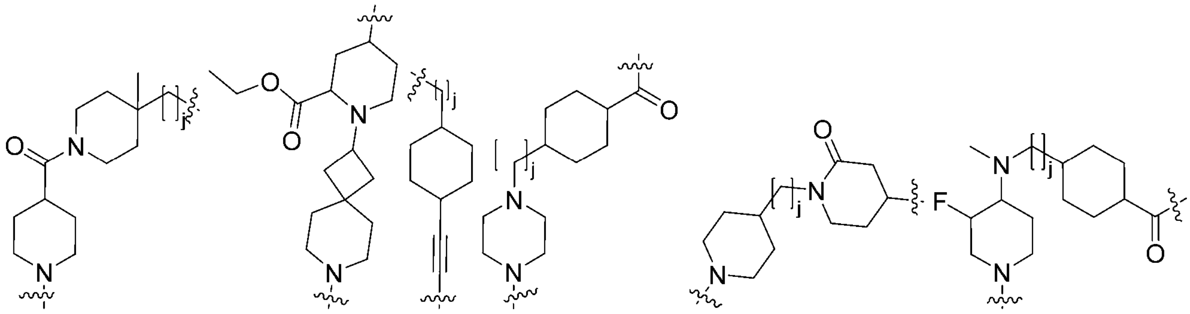

,

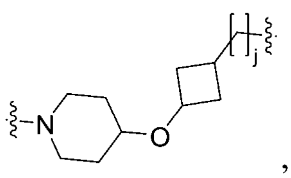

,

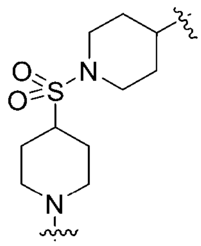

,

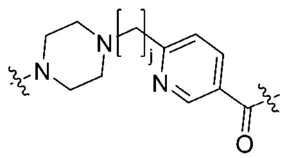

,

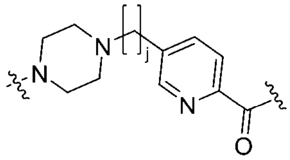

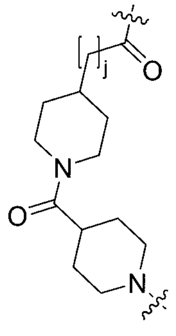

,

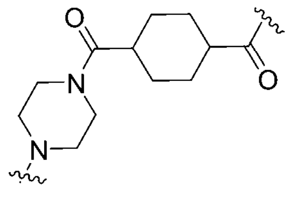

,

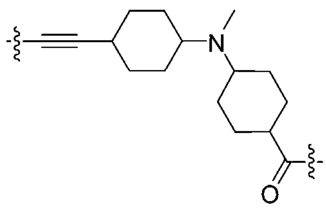

, ,

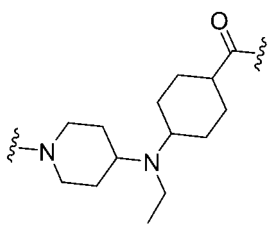

,

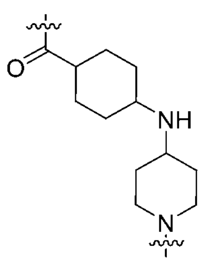

,

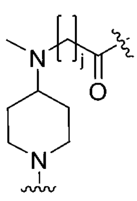

and

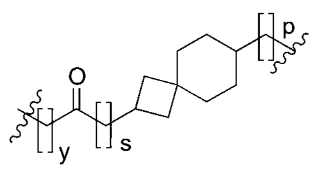

;

wherein each occurrence of j is independently 1, 2, 3, 4, 5, 6, 7, 8, 9, or 10;
k, s, p, and y are each independently 0, 1, 2, 3, 4, 5, 6, 7, 8, 9, or 10;

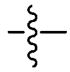

is a point of attachment to CLM or PTM;

L is preferably selected from a covalent bond, $-CH_2-$, $-(CH_2)_2-$, $-(CH_2)_3-$, $-(CH_2)_4-$, $-(CH_2)_5-$, $-(CH_2)_6-$, $-(CH_2)_7-$, $-(CH_2)_8-$, $-NH-CH_2-$, $-NH-(CH_2)_2-$, $-NH-(CH_2)_3-$, $-NH-(CH_2)_4-$, $-NH-(CH_2)_5-$, $-NH-(CH_2)_6-$, $-NH-(CH_2)_7-$, $-NH-(CH_2)_8-$, $-CO-NH-CH_2-$, $-CO-NH-(CH_2)_2-$, $-CO-NH-(CH_2)_3-$, $-CO-NH-(CH_2)_4-$, $-CO-NH-(CH_2)_5-$, $-CO-NH-(CH_2)_6-$, $-CO-NH-(CH_2)_7-$, $-CO-NH-(CH_2)_8-$, $-CH_2-NH-$, $-(CH_2)_2-NH-$, $-(CH_2)_3-NH-$, $-(CH_2)_4-NH-$, $-(CH_2)_5-NH-$, $-(CH_2)_6-NH-$, $-(CH_2)_7-NH-$, $-(CH_2)_8-NH-$, $-NH-CH_2-NH-$, $-NH-(CH_2)_2-NH-$, $-NH-(CH_2)_3-NH-$, $-NH-(CH_2)_4-NH-$, $-NH-(CH_2)_5-NH-$, $-NH-(CH_2)_6-NH-$, $-NH-(CH_2)_7-NH-$, $-NH-(CH_2)_8-NH-$, $-(CH_2-CH_2-O)-CH_2-CH_2-$, $-(CH_2-CH_2-O)_2-CH_2-CH_2-$, $-(CH_2-CH_2-O)_3-CH_2-CH_2-$, $-NH-(CH_2-CH_2-O)-CH_2-CH_2-$, $-NH-(CH_2-CH_2-O)_2-CH_2-CH_2-$, $-NH-(CH_2-CH_2-O)_3-CH_2-CH_2-$, $-CO-NH-(CH_2-CH_2-O)-CH_2-CH_2-$, $-CO-NH-(CH_2-CH_2-O)_2-CH_2-CH_2-$, $-CO-NH-(CH_2-CH_2-O)_3-CH_2-CH_2-$, $-(CH_2-CH_2-O)-CH_2-CH_2-NH-$, $-(CH_2-CH_2-O)_2-CH_2-CH_2-NH-$, $-(CH_2-CH_2-O)_3-CH_2-CH_2-NH-$, $-NH-(CH_2-CH_2-O)-CH_2-CH_2-NH-$, $-NH-(CH_2-CH_2-O)_2-CH_2-CH_2-NH-$, $-NH-(CH_2-CH_2-O)_3-CH_2-CH_2-NH-$, $-CO-NH-(CH_2-CH_2-O)-CH_2-CH_2-NH-$, $-CO-NH-(CH_2-CH_2-O)_2-CH_2-CH_2-NH-$, $-CO-NH-(CH_2-CH_2-O)_3-CH_2-CH_2-NH-$, $-CH_2-CH_2-(O-CH_2-CH_2)-$, $-CH_2-CH_2-(O-CH_2-CH_2)_2-$, $-CH_2-CH_2-(O-CH_2-CH_2)_3-$, $-NH-CH_2-CH_2-(O-CH_2-CH_2)-$, $-NH-CH_2-CH_2-(O-CH_2-CH_2)_2-$, $-NH-CH_2-CH_2-(O-CH_2-CH_2)_3-$, $-CO-NH-CH_2-CH_2-(O-CH_2-CH_2)-$, $-CO-NH-CH_2-CH_2-(O-CH_2-CH_2)_2-$, $-CO-NH-CH_2-CH_2-(O-CH_2-CH_2)_3-$, $-CH_2-CH_2-(O-CH_2-CH_2)-NH-$, $-CH_2-CH_2-(O-CH_2-CH_2)_2-NH-$, $-CH_2-CH_2-(O-CH_2-CH_2)_3-NH-$, $-NH-CH_2-CH_2-(O-CH_2-CH_2)-NH-$, $-NH-CH_2-CH_2-(O-CH_2-CH_2)_2-NH-$, $-NH-CH_2-CH_2-(O-CH_2-CH_2)_3-NH-$, $-NH-CH_2-CH_2-O-CH_2-CH_2-CO-$, $-CO-CH_2-CH_2-O-CH_2-CH_2-NH-$, $-NH-(CH_2)_4-CO-$, $-NH-(CH_2)_5-CO-$, $-NH-(CH_2)_6-CO-$, $-CO-(CH_2)_4-NH-$, $-CO-(CH_2)_5-NH-$, $-CO-(CH_2)_6-NH-$, $-NH-(CH_2-CH_2-O)-(CH_2)_3-$, $-NH-(CH_2-CH_2-O)-(CH_2)_4-$, $-NH-(CH_2-CH_2-O)-(CH_2)_5-$, $-NH-(CH_2-CH_2-O)-(CH_2)_6-$, $-(CH_2)_3-(O-CH_2-CH_2)-NH-$, $-(CH_2)_4-(O-CH_2-CH_2)-NH-$, $-(CH_2)_5-(O-CH_2-CH_2)-NH-$, $-(CH_2)_6-(O-CH_2-CH_2)-NH-$, $-CH_2-CH_2-O-(CH_2)_2-CO-$, $-CH_2-CH_2-O-(CH_2)_3-CO-$, $-CH_2-CH_2-O-(CH_2)_4-CO-$, $-CO-(CH_2)_2-O-CH_2-CH_2-$, $-CO-(CH_2)_3-O-CH_2-CH_2-$, $-CO-(CH_2)_4-O-CH_2-CH_2-$, $-CO-(CH_2)_2-$, $-CO-(CH_2)_3-$, $-CO-(CH_2)_4-$, $-CO-(CH_2)_5-$, $-CO-(CH_2)_6-$, $-(CH_2)_2-CO-$, $-(CH_2)_3-CO-$, $-(CH_2)_4-CO-$, $-(CH_2)_5-CO-$, $-(CH_2)_6-CO-$, $-CO-(CH_2)_2-CO-$, $-CO-(CH_2)_3-CO-$, $-CO-(CH_2)_4-CO-$, $-CO-(CH_2)_5-CO-$, $-CO-(CH_2)_6-CO-$, $-CH_2-CO-CH_2-$, $-CH_2-CO-(CH_2)_2-$, $-CH_2-CO-(CH_2)_3-$, $-CH_2-CO-(CH_2)_4-$, $-(CH_2)_2-CO-CH_2-$, $-(CH_2)_2-CO-(CH_2)_2-$, $-(CH_2)_2-CO-(CH_2)_3-$, $-(CH_2)_2-CO-(CH_2)_4-$, $-(CH_2)_3-CO-CH_2-$, $-(CH_2)_3-CO-(CH_2)_2-$, $-(CH_2)_3-CO-(CH_2)_3-$, $-(CH_2)_3-CO-(CH_2)_4-$, $-(CH_2)_4-CO-CH_2-$, $-(CH_2)_4-CO-(CH_2)_2-$, $-(CH_2)_4-CO-(CH_2)_3-$, $-(CH_2)_4-CO-(CH_2)_4-$, $-CH_2-O-CH_2-$, $-CH_2-O-(CH_2)_2-$, $-CH_2-O-(CH_2)_3-$, $-CH_2-O-(CH_2)_4-$, $-(CH_2)_2-O-CH_2-$, $-(CH_2)_2-O-(CH_2)_2-$, $-(CH_2)_2-O-(CH_2)_3-$, $-(CH_2)_2-O-(CH_2)_4-$, $-(CH_2)_3-O-CH_2-$, $-(CH_2)_3-O-(CH_2)_2-$, $-(CH_2)_3-O-(CH_2)_3-$, $-(CH_2)_3-O-(CH_2)_4-$, $-(CH_2)_4-O-CH_2-$, $-(CH_2)_4-O-(CH_2)_2-$, $-(CH_2)_4-O-(CH_2)_3-$, $-(CH_2)_4-O-(CH_2)_4-$,

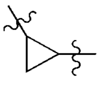, 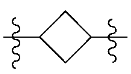, 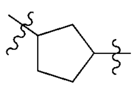, 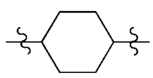, 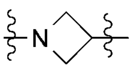, 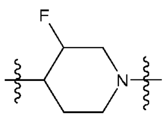, 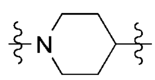,

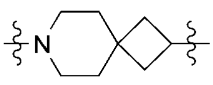, 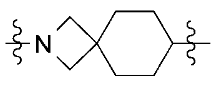, 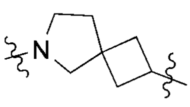, 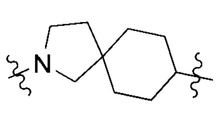, 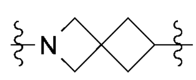,

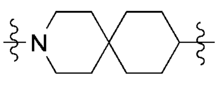, 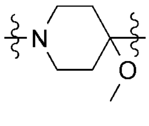, 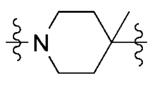, 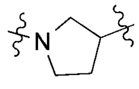, 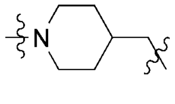, 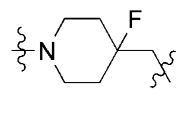,

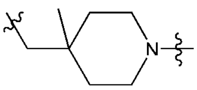, 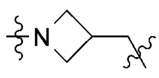, 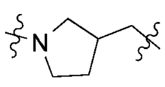, 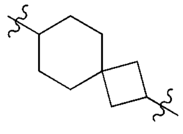, 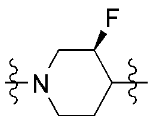, 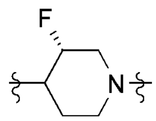,

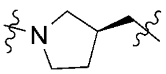, 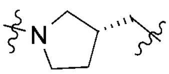, 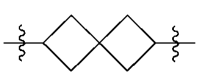, 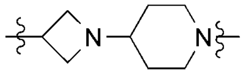, 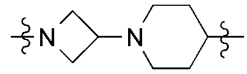,

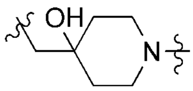, 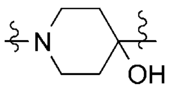, 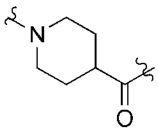, 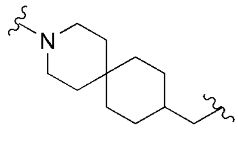, 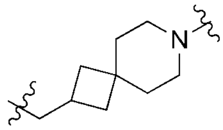,

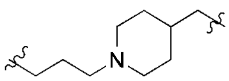, 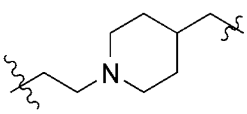, 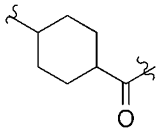, 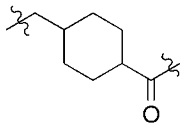, 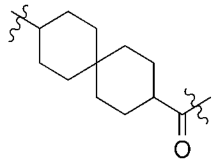,

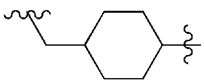, 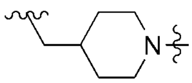, 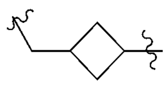, 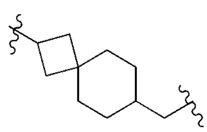, 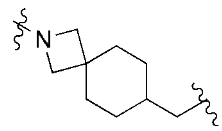,

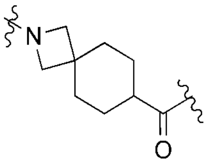, 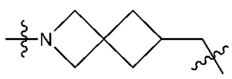, 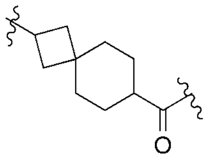, 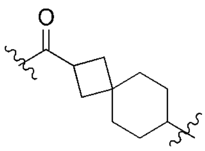, 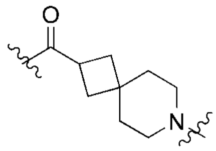,

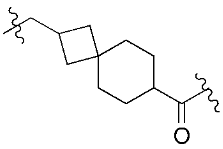, 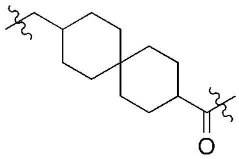, 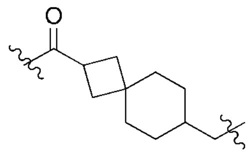, 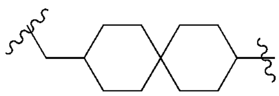,

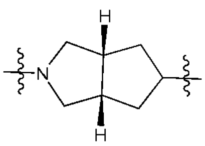, 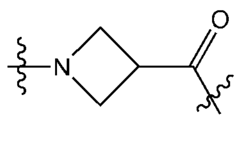, 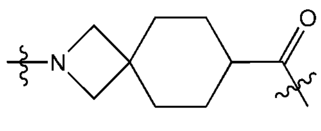, 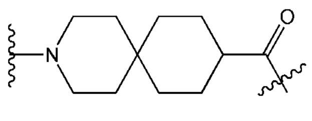,

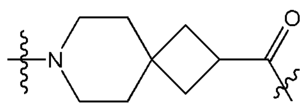, 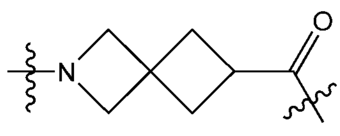, 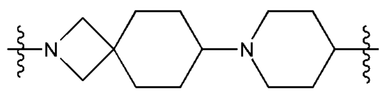,

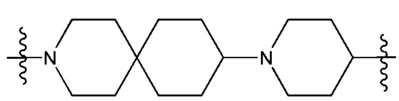, 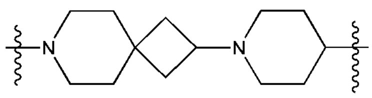, 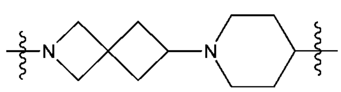,

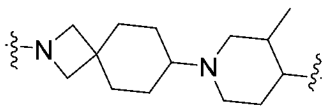, 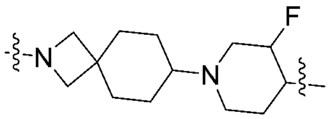, 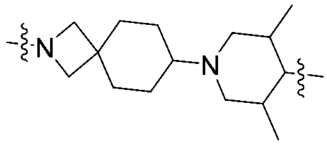,

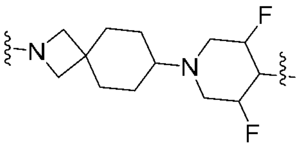, 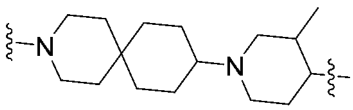, 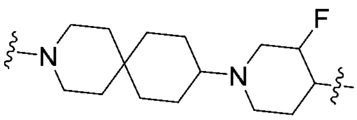,

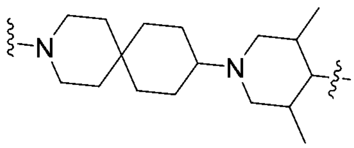, 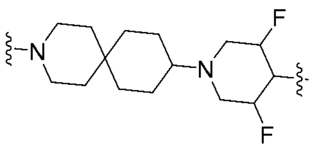, 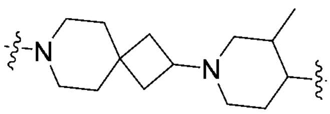,

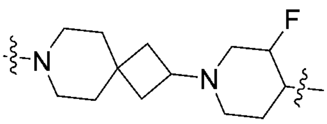, 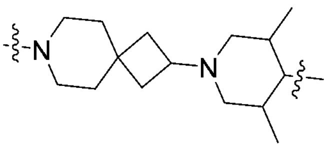, 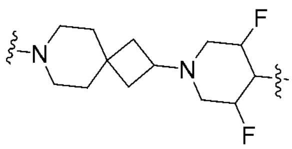,

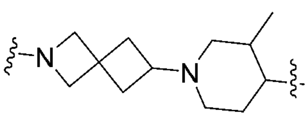, 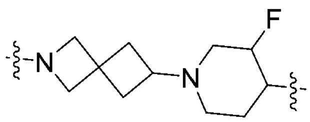, 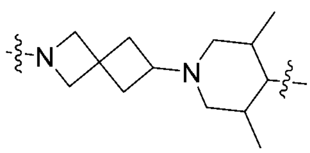,

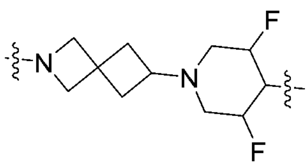, 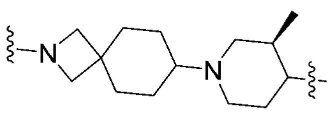, 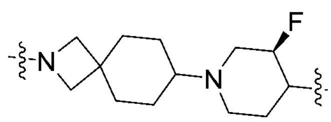,

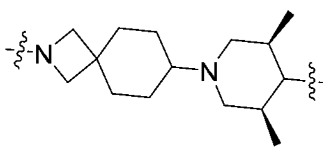, 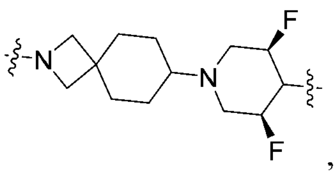, 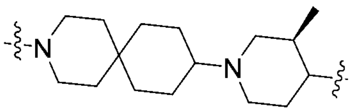,

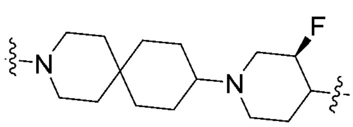, 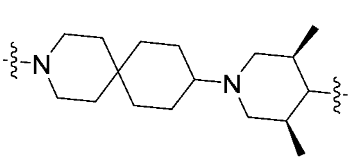, 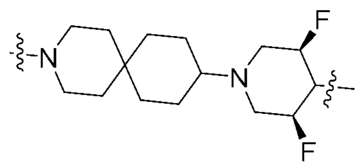,

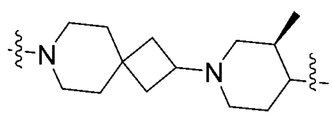, 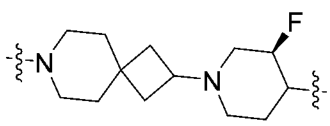, 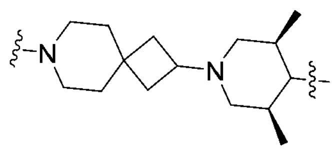,

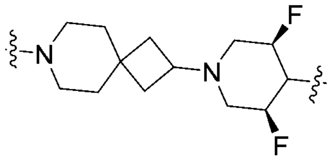, 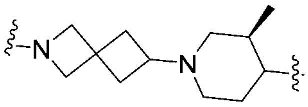, 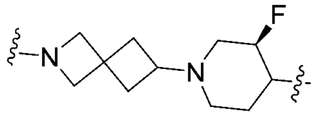,

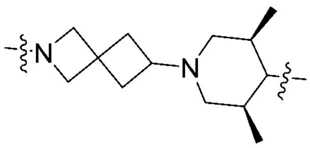, 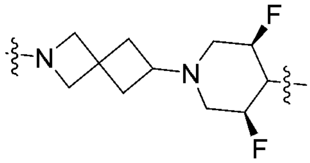, 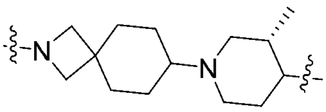,

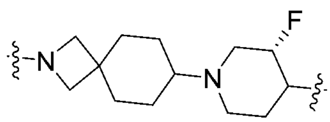, 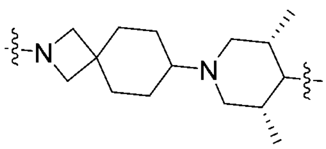, 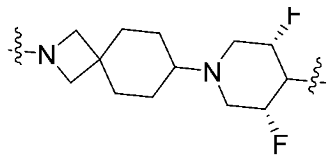,

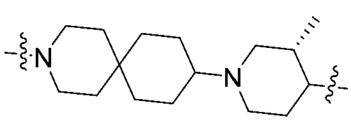, 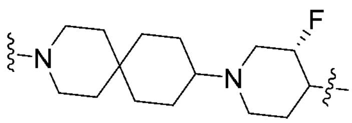, 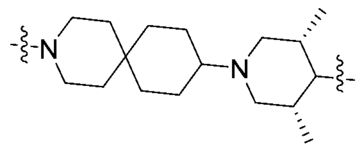,

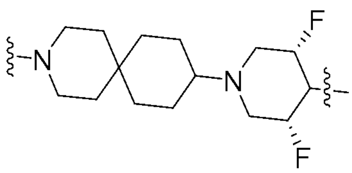, 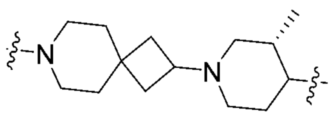, 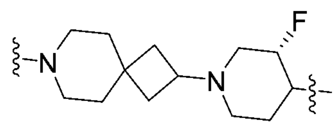,

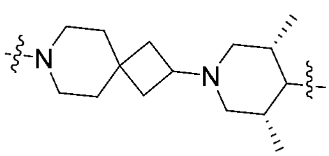, 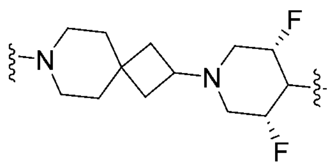, 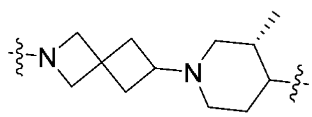,

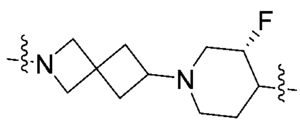, 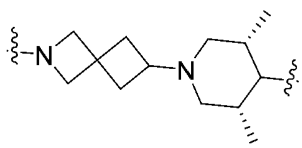, 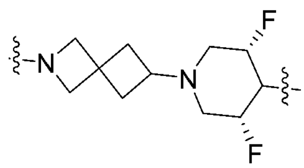, 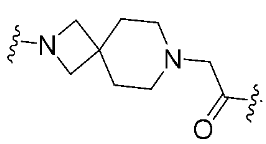,

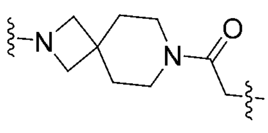, 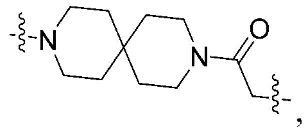, 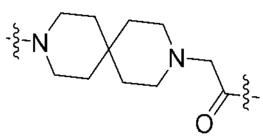, 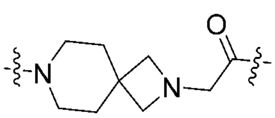,

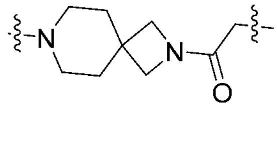, 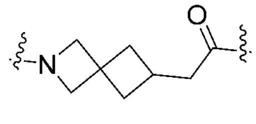, 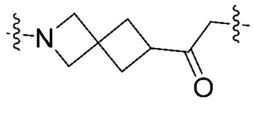, 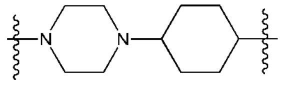,

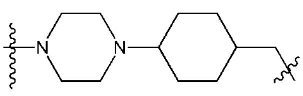, 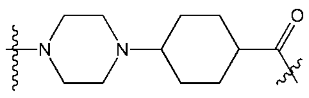, 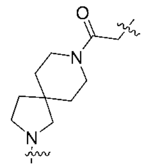, 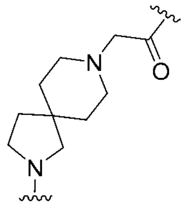,

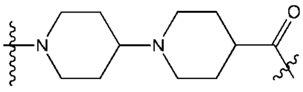, 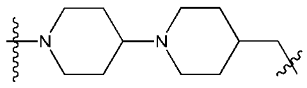, 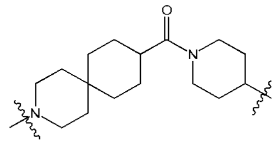, 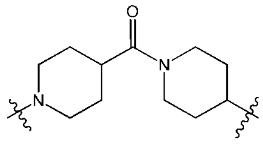,

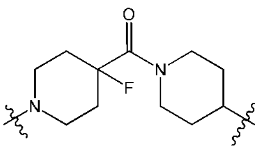, 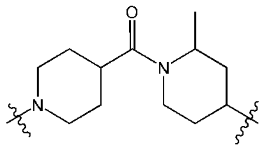, 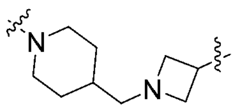, 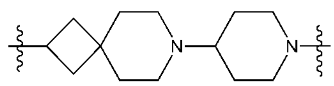,

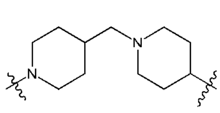, 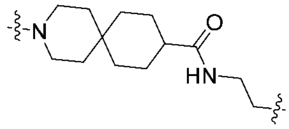, 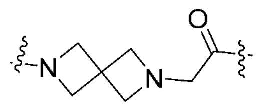, 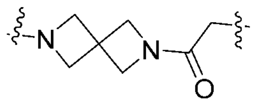,

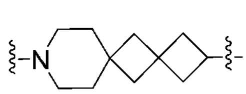 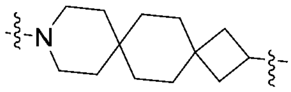, 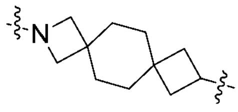 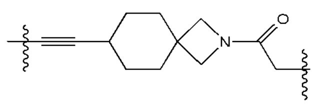,

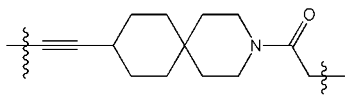, 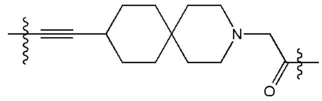, 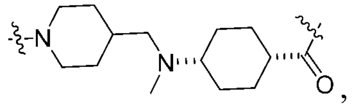,

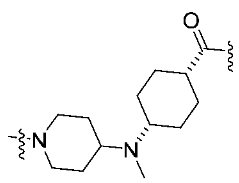, 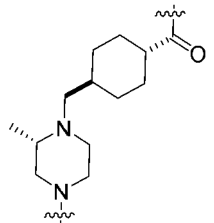, 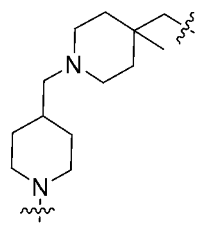, 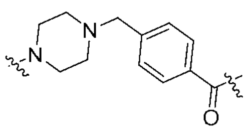, 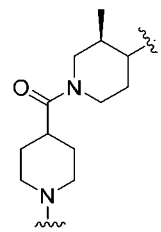,

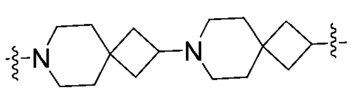, 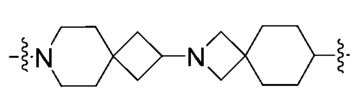, 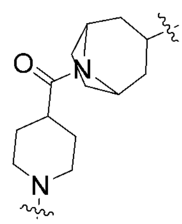, 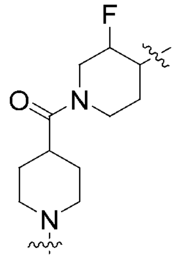,

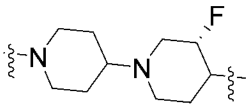, 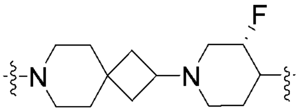, 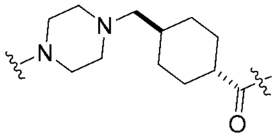, 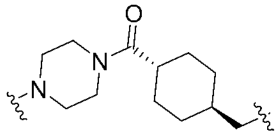,

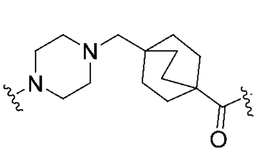, 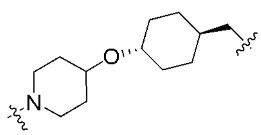, 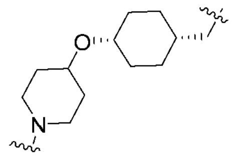, 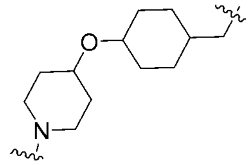,

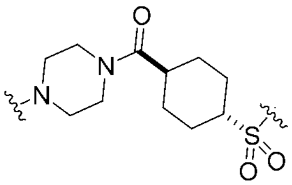, 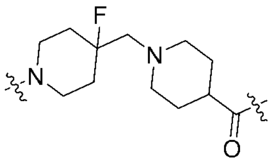, 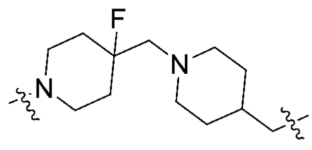, 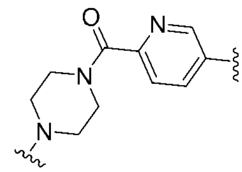,

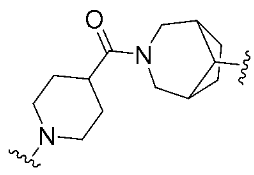, 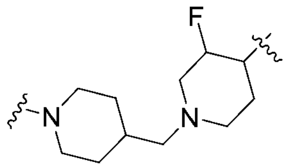, 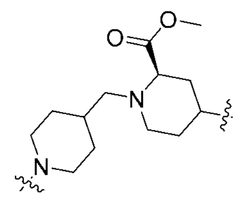, 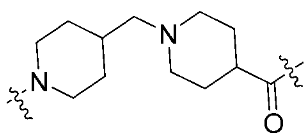,

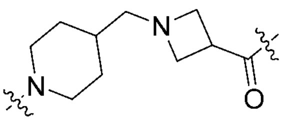, 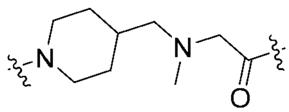, 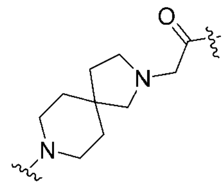 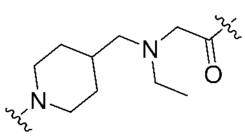

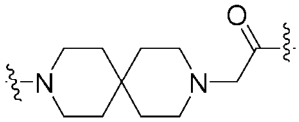 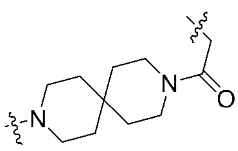 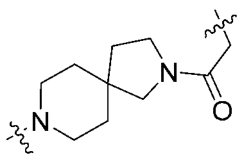 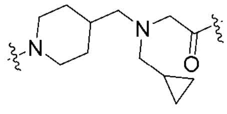, 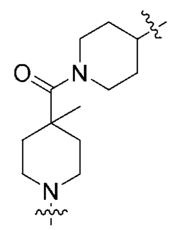,

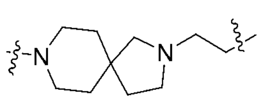, 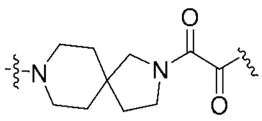, 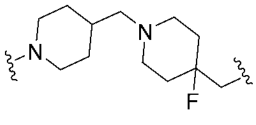, 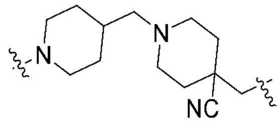,

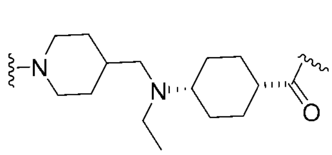 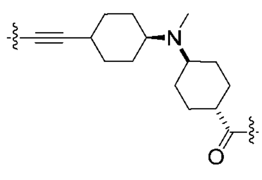, 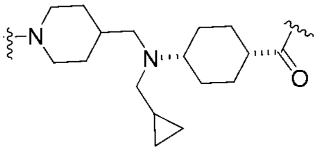, 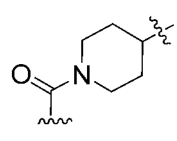,

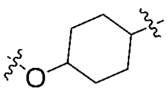 , 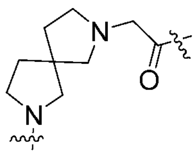 , 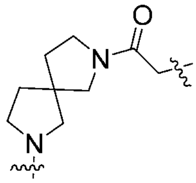 , 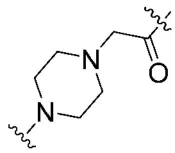 , 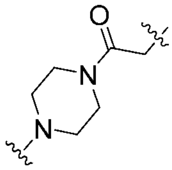 , 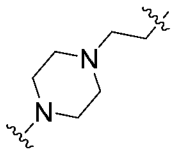 ,

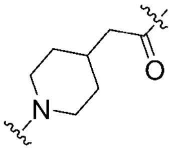 , 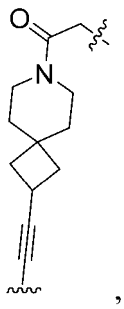 , 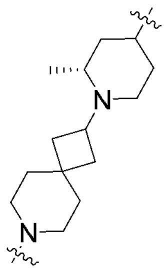 , 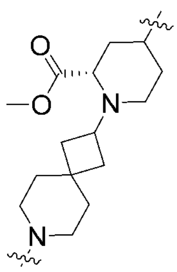 , 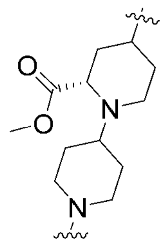 , 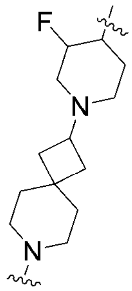 , 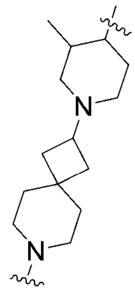 ,

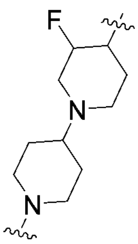 , 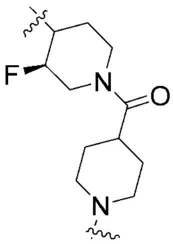 , 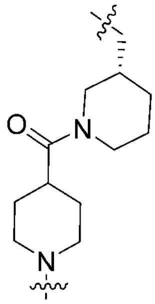 , 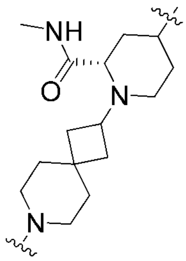 , 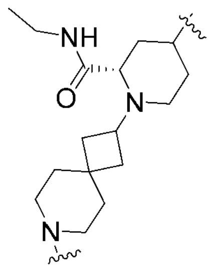 , 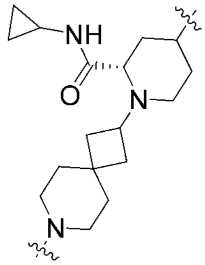 ,

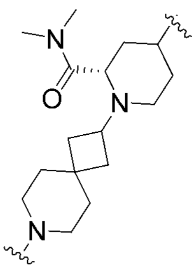 , 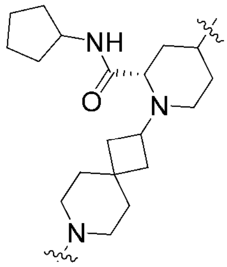 , 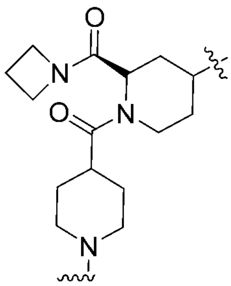 , 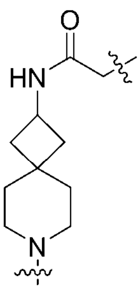 , 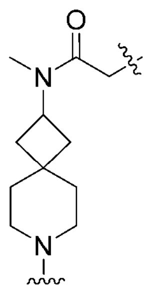 , 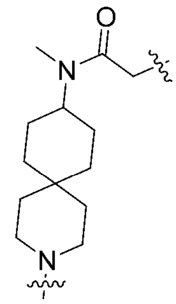 ,

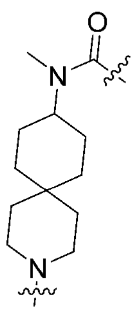 , 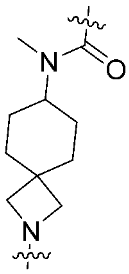 , 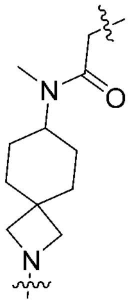 , 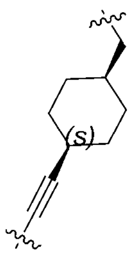 , 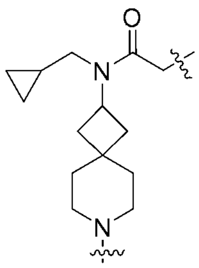 , 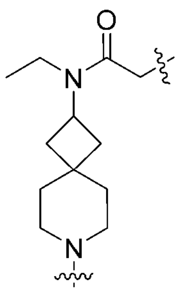 , 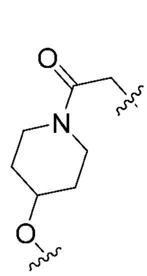 ,

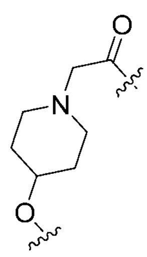, 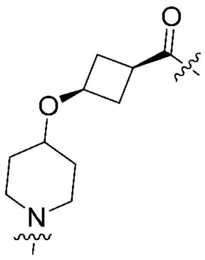, 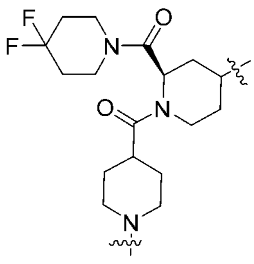, 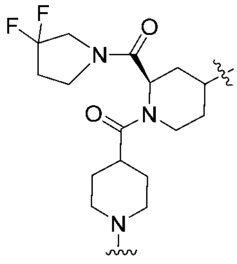, 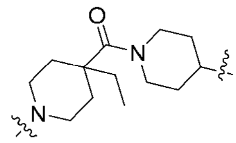,

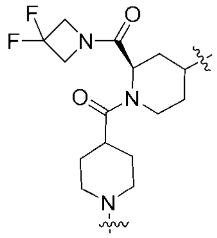, 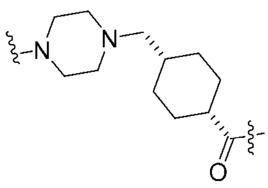, 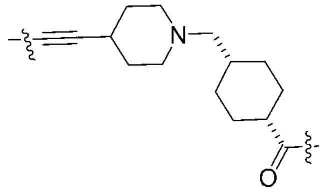, 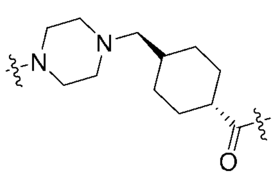,

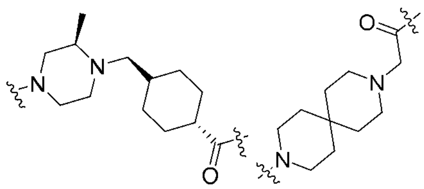 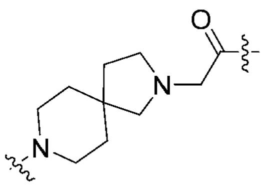 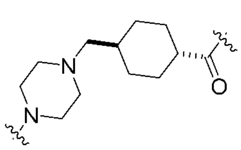 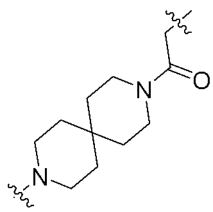

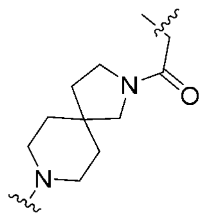 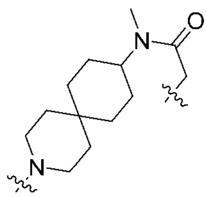 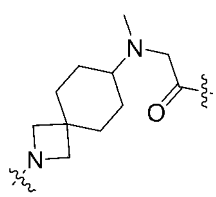, 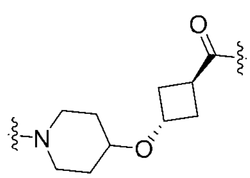, 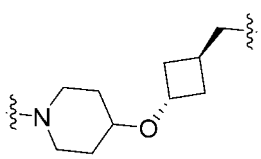,

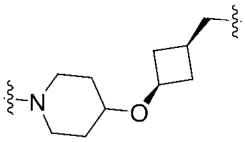, 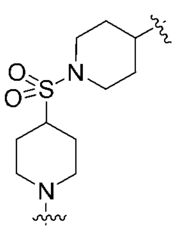, 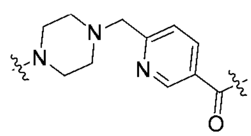, 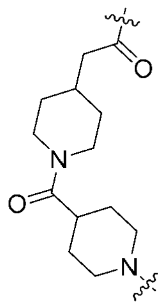 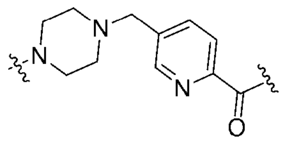,

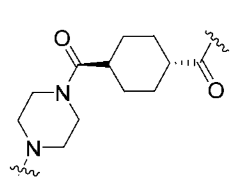, 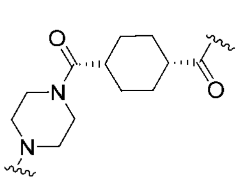, 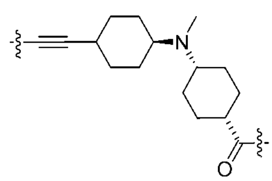, 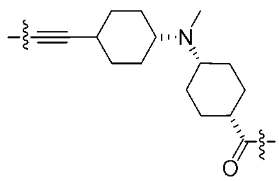,

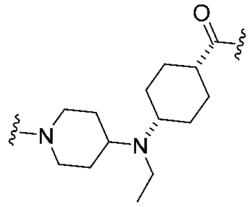, 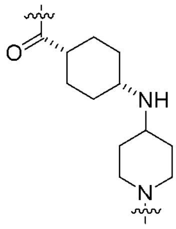, 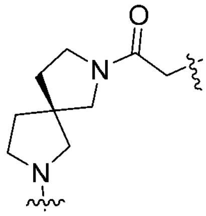, 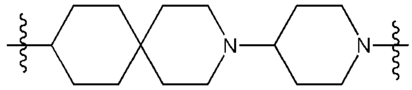,

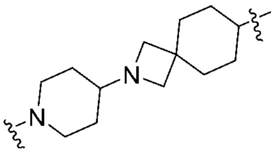, , 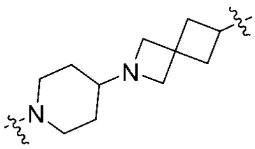, 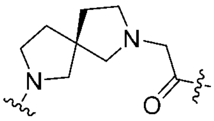, 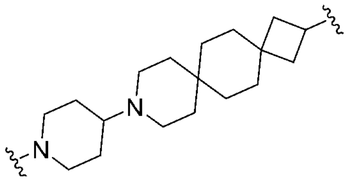,

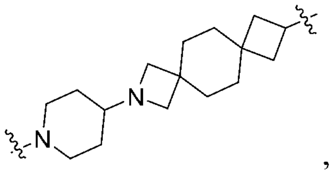, 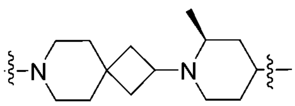, 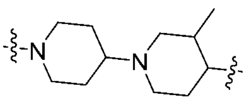, 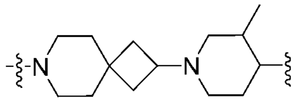,

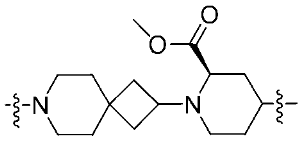, 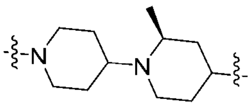, 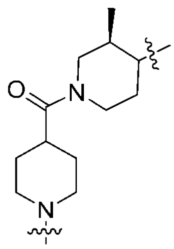, 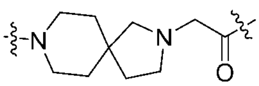,

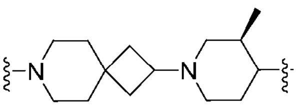, 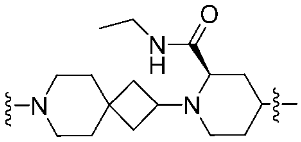, 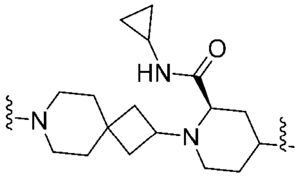, 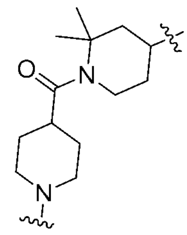,

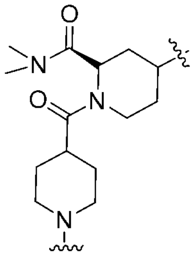, 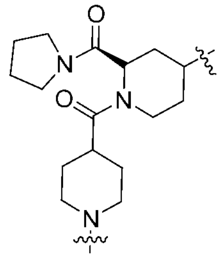, 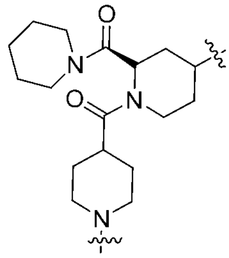, 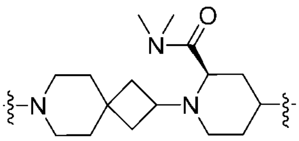, 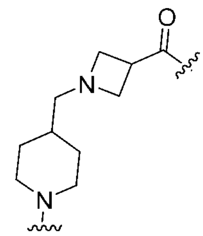,

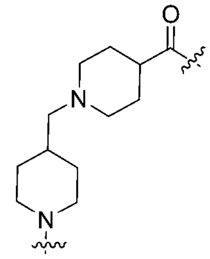, 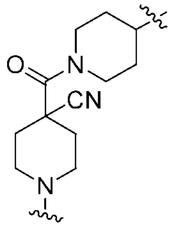, 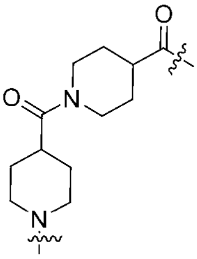, 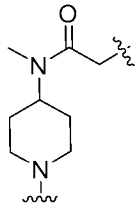, 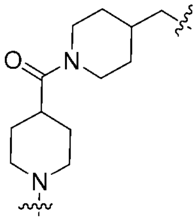,

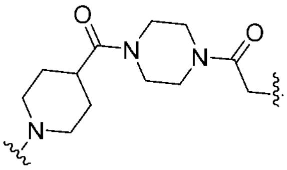, 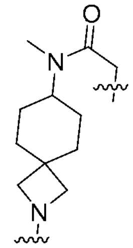, 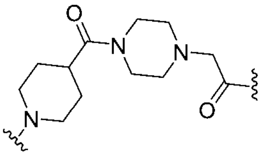, 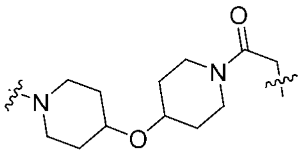,

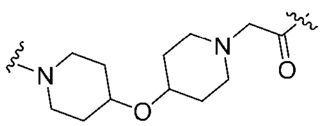, 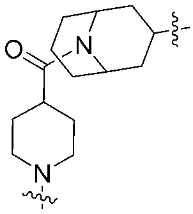, 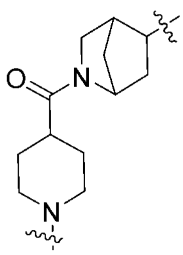, 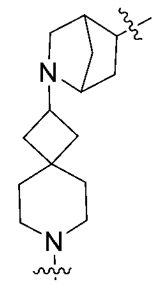, 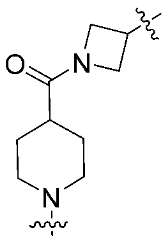,

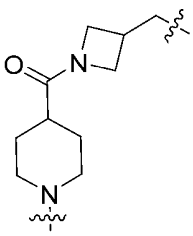, 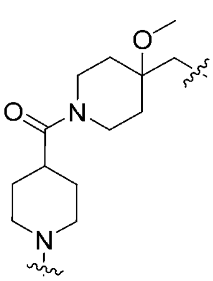, 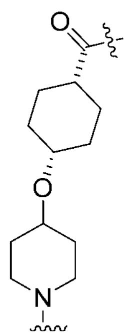, 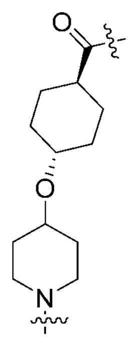, 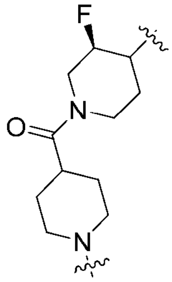, 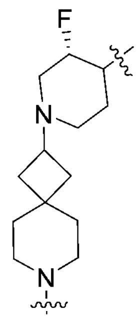, 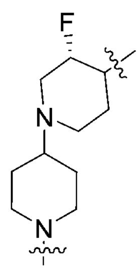,

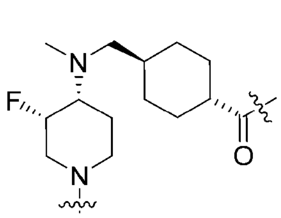, 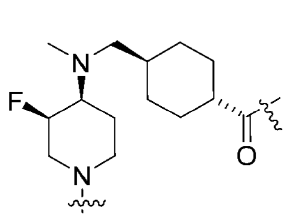, 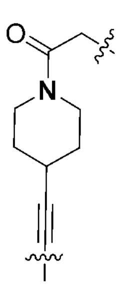, 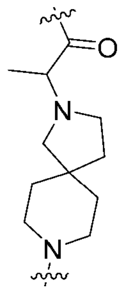, 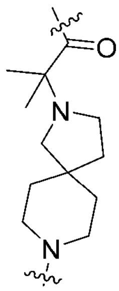, 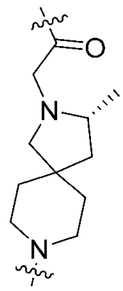,

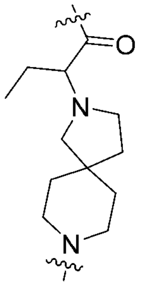, 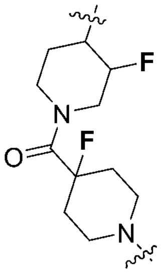, 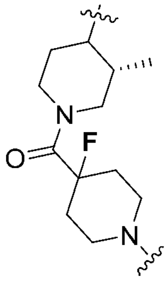, 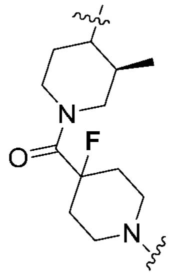, 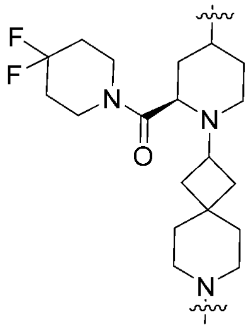, 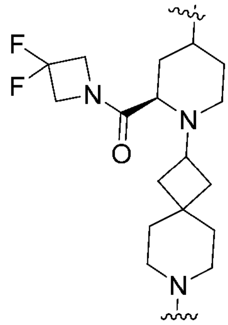,

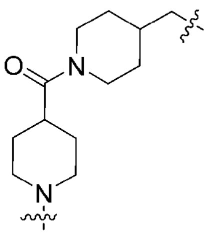, 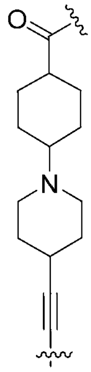, 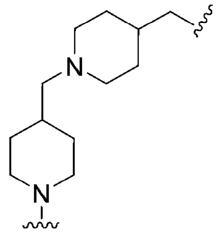, 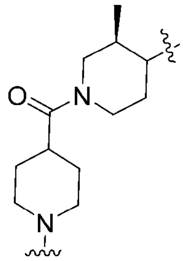, 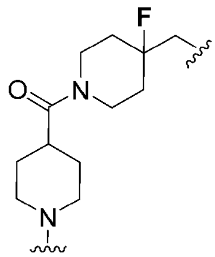, 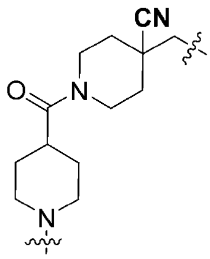,

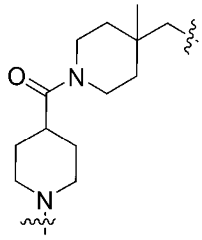, 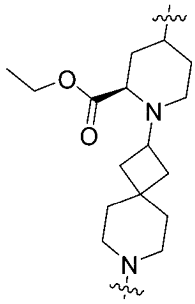, 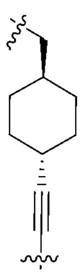, 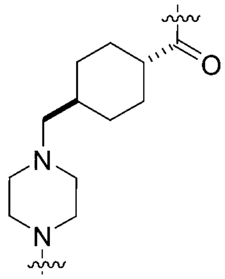, 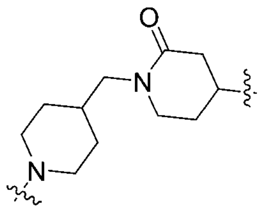,

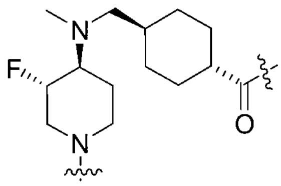, 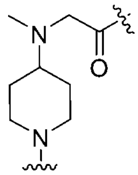 and 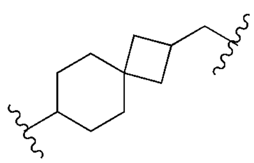.

In one embodiment, the PTM is selected from the following structures:

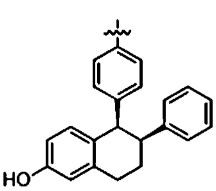 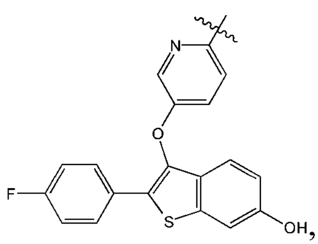 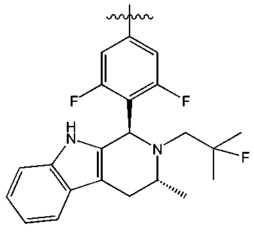, 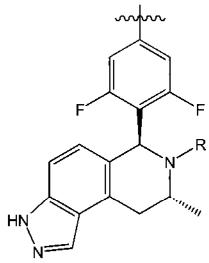,

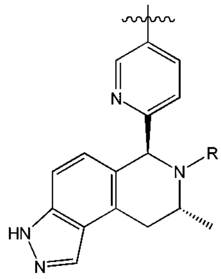, 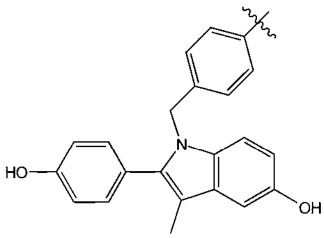, 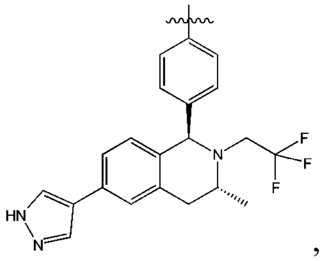, 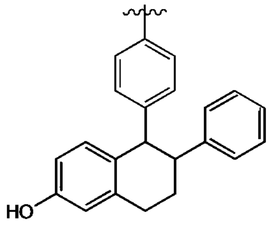,

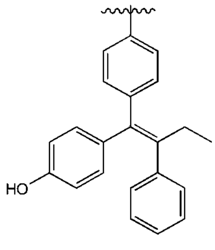, 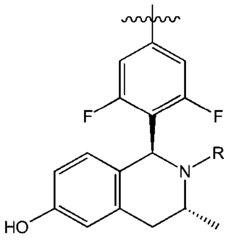, 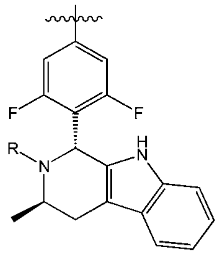, 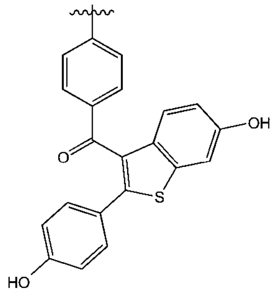, 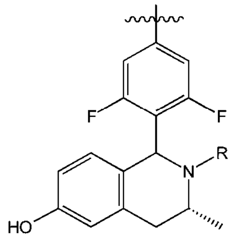,

and

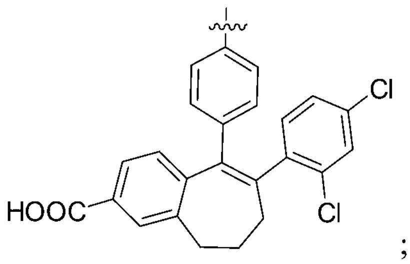;

R is selected from

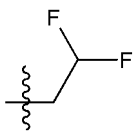, 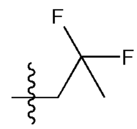, 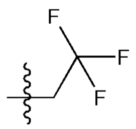 and 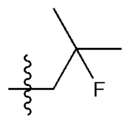.

In a second aspect, the present disclosure provides a pharmaceutical composition comprising the aforementioned compound or an isomer, an isotopic derivative, a polymorph, a prodrug, a pharmaceutically acceptable salt, or a solvate thereof, and one or more pharmaceutically acceptable carriers, diluents, or excipients.

In a third aspect, the present disclosure provides a use of the aforementioned compound or an isomer, an isotopic derivative, a polymorph, a prodrug, a pharmaceutically acceptable salt, or a solvate thereof, or the pharmaceutical composition described above in the preparation of a medicament for the treatment or prevention of a disorder treatable by degradation of estrogen receptor protein.

## Detailed Description

### Terms and Definitions

The term "alkyl" as used herein refers to a saturated aliphatic hydrocarbon group which is a straight or branched chain group containing 1 to 20 carbon atoms, preferably an alkyl group containing 1 to 12 (e.g., 1, 2, 3, 4, 5, 6, 7, 8, 9, 10, 11, and 12) carbon atoms, and more preferably an alkyl group containing 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl,

4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched-chain isomers thereof. More preferred are lower alkyl groups containing 1 to 6 carbon atoms, non-limiting examples of which include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, and 2,3-dimethylbutyl, etc. The alkyl group may be substituted or unsubstituted, and when substituted, the substituent group may be attached at any available position and are preferably independently optionally selected from one or more of the group consisting of: H atom, D atom, halogen, alkyl, alkoxy, haloalkyl, hydroxy, hydroxyalkyl, cyano, amino, nitro, cycloalkyl, heterocyclyl, aryl, and heteroaryl.

The term "heteroalkyl" refers to an alkyl group in which one or more $-CH_2-$ are replaced bya heteroatom selected from NH, O, and S, or in which one or more -CH- are replaced by a N atom; wherein the alkyl group is as defined herein; the heteroalkyl group may be substituted or unsubstituted., and when substituted, the substituent group may be attached at any available position. The substituent is preferably independently optionally selected from one or more of the group consisting of H atom, D atom, halogen, alkyl, alkoxy, haloalkyl, hydroxy, hydroxyalkyl, cyano, amino, nitro, cycloalkyl, heterocyclyl, aryl, and heteroaryl.

The term "alkoxy" refers to -O-(alkyl) and -O- (unsubstituted cycloalkyl), wherein alkyl or cycloalkyl is as defined herein. Non-limiting examples of alkoxy include methoxy, ethoxy, propoxy, butoxy, cyclopropoxy, cyclobutoxy, cyclopentyloxy, and cyclohexyloxy. The alkoxy group may be optionally substituted or unsubstituted, and when substituted, the substituent is preferably one or more of the following groups independently selected from H atom, D atom, halogen, alkyl, alkoxy, haloalkyl, hydroxyl, hydroxyalkyl, cyano, amino, nitro, cycloalkyl, heterocyclyl, aryl, and heteroaryl.

The term "alkenyl" refers to an alkyl compound containing a carbon-carbon double bond in the molecule, wherein alkyl is defined herein. Alkenyl may be substituted or unsubstituted, and when substituted, the substituent is preferably one or more of the following groups independently selected from H atom, alkyl, alkoxy, halogen, haloalkyl, hydroxy, hydroxyalkyl, cyano, amino, nitro, cycloalkyl, heterocyclyl, aryl, and heteroaryl.

The term "alkynyl" refers to an alkyl compound containing a carbon-carbon triple bond in the molecule, wherein alkyl is defined herein. The alkynyl may be substituted or unsubstituted, and when substituted, the substituent is preferably one or more of the following groups independently selected from: H atom, alkyl, alkoxy, halogen, haloalkyl, hydroxy, hydroxyalkyl, cyano, amino, nitro, cycloalkyl, heterocyclyl, aryl, and heteroaryl.

The term "cycloalkyl" refers to a saturated or partially unsaturated monocyclic cyclic hydrocarbon substituent, the cycloalkyl ring containing 3 to 20 carbon atoms, preferably from 3 to 12 carbon atoms, more preferably from 3 to 8 (e.g., 3, 4, 5, 6, 7, and 8) carbon atoms, more preferably from 4 to 7 carbon atoms. Non-limiting examples of monocyclic cycloalkyl groups include but not limited to cyclopropyl, cyclobutyl, cyclopentyl, cyclopentenyl, cyclohexyl, cyclohexenyl, cyclohexadienyl, cycloheptyl, cycloheptatrienyl, and cyclooctyl.

The cycloalkyl group may be substituted or unsubstituted, and when substituted, the substituent may be attached at any available position, the substituent preferably being one or more of the following groups independently optionally selected from H atom, halogen, alkyl, alkoxyl, haloalkyl, hydroxyl, hydroxyalkyl, cyano, amino, nitro, cycloalkyl, heterocyclyl, aryl, and heteroaryl.

The term "heterocyclyl" refers to a saturated or partially unsaturated monocyclic cyclic hydrocarbon substituent containing 3 to 20 ring atoms, wherein one or more of the ring atoms is a heteroatom selected from nitrogen, oxygen, or S(O)m (wherein m is an integer from 0 to 2), but does not contain a ring portion of -O-O-, -O-S-, or -S-S-, and wherein the remaining ring atoms are carbon. are carbon. Preferably, 3 to 12 (e.g., 3, 4, 5, 6, 7, 8, 9, 10, 11, and 12) ring atoms are included, of which 1 to 4 (e.g., 1, 2, 3, and 4) are heteroatoms; more preferably, 3 to 8 ring atoms are included, of which 1-3 are heteroatoms; more preferably, 3 to 6 ring atoms are included, of which 1-3 are heteroatoms; and most preferably, 5 or 6 ring atoms are included, of which 1-3 are heteroatoms. Non-limiting examples of monocyclic heterocyclic groups include but not limited to pyrrolidinyl, tetrahydropyranyl, 1,2,3,6-tetrahydropyridinyl, piperidinyl, piperazinyl, morpholinyl, thiomorpholinyl, and homopiperazinyl.

The heterocyclyl group may be substituted or unsubstituted, and when substituted, the substituent may be attached at any available position, the substituent preferably being one or more of the following groups independently optionally selected from: H atom, halogen, alkyl, alkoxy, alkyl, hydroxy, hydroxy, cyano, amino, nitro, cycloalkyl, heterocyclyl, aryl, and heteroaryl.

The term "aryl" refers to a 6 to 14-membered all-carbon monocyclic or polycyclic fused ring (i.e., a ring sharing adjacent pairs of carbon atoms) groups having a conjugated $\pi$-electronic system, preferably a 6 to 10-membered aryl, e.g., phenyl and naphthyl. Said aryl ring contains an aryl ring as described above fused to a heteroaryl, heterocyclyl or cycloalkyl ring, wherein the ring attached to the parent structure is an aryl ring. The aryl group may be substituted or unsubstituted,

and when substituted, the substituent group may be attached at any available position, the substituent preferably being one or more of the following groups independently optionally selected from H atom, halogen, alkyl, alkoxyl, haloalkyl, hydroxyl, hydroxyalkyl, cyano, amino, nitro, cycloalkyl, heterocyclyl, aryl, and heteroaryl. The term "heteroaryl" refers to a heteroaromatic system containing 1 to 4 (e.g., 1, 2, 3 and 4) heteroatoms, 5 to 14 ring atoms, wherein the heteroatoms are selected from oxygen, sulfur and nitrogen. The heteroaryl group is preferably 5 to 10 membered (e.g. 5, 6, 7, 8, 9 or 10 membered), more preferably 5 or 6 membered, such as furanyl, thiophenyl, pyridinyl, pyrrolyl, N-alkylpyrrolyl, pyrimidinyl, pyrazinyl, pyridazinyl, imidazolyl, pyrazolyl, triazolyl, tetrazolyl and the like. The heteroaryl ring contains a heteroaryl group as described herein thickened on an aryl, heterocyclic or cycloalkyl ring, wherein the ring attached to the parent structure is a heteroaryl ring. Heteroaryl may be substituted or unsubstituted, and when substituted, the substituent may be attachedat any available position, the substituent preferably being one or more of the following groups independently optionally selected from H atom, halogen, alkyl, alkoxyls, haloalkyl, hydroxyl, hydroxyalkyl, cyano, amino, nitro, cycloalkyls, heterocyclyl, aryl, and heteroalkyl.

The term "haloalkyl" refers to an alkyl group substituted with one or more halogens, wherein the alkyl group is as defined above.

The term "hydroxyalkyl" refers to an alkyl group substituted with one or more hydroxyl groups, wherein alkyl is as defined above.

The term "halogen" refers to fluorine, chlorine, bromine or iodine.

The term "amino" refers to $-NH_2$.

The term "cyano" refers to -CN.

The term "nitro" refers to $-NO_2$.

The term "ubiquitin ligase" refers to a family of proteins that facilitate the transfer of ubiquitin to specific substrate proteins and target the substrate proteins for degradation. For example, cereblon is an E3 ubiquitin ligase protein that, alone or in combination with an E2 ubiquitin-conjugating enzyme, can result in the attachment of ubiquitin to lysines on target proteins, thereby targeting specific protein substrates for degradation via the proteasome. Thus, E3 ubiquitin ligase alone or in complex with an E2 ubiquitin-conjugating enzymes is responsible for the transfer of ubiquitin to the labeled target protein. In general, ubiquitin ligases are involved in polyubiquitination so that the second ubiquitin is attached to the first ubiquitin, the third ubiquitin is attached to the second ubiquitin, and so on. Polyubiquitinated tagged proteins are used for degradation via the proteasome. However, there exist a number of ubiquitination events limited to mono-ubiquitination, wherein only a single ubiquitin is added to the substrate molecule via ubiquitin ligase. Mono-ubiquitinated proteins are not targeted to the proteasome for degradation, but may instead be altered in their cellular location or function, for example, via the binding of other proteins with structural domains capable of binding ubiquitin. To complicate matters further, different lysines on ubiquitin can be targeted by E3 to prepare chains. The most common lysine is Lys48 on the ubiquitin chain, and the lysine used to prepare polyubiquitin, which is recognized by the proteasome.

The term "target protein" refers to proteins and peptides having any biological function or activity, including structural, regulatory, hormonal, enzymatic, genetic, immunological, contractile, storage, transport, and signal transduction. In some embodiments, target proteins include structural proteins, receptors, enzymes, cell surface proteins, and proteins associated with the integrated cellular functions, including proteins involved in each of: catalytic activity, aromatase activity, motility activity, helicase activity, metabolic processes (anabolic and catabolic), antioxidant activity, protein hydrolysis, biosynthesis, proteins with kinase activity, oxidoreductase activity, transferase activity, hydrolase activity, lyase activity, isomerase activity, ligase activity, enzyme regulator activity, signal transducer activity, structural molecule activity, binding activity (proteins, lipids carbohydrates), receptor activity, cellular motility, membrane fusion, cellular communication, regulation of biological processes, development, cellular differentiation, stimulus response, behavior-associated proteins, cell adhesion proteins, proteins involved in cell death, proteins involved in transporter (including protein transporter activity, nuclear transporter, ion transporter activity, channel transporter activity, carrier activity), permease activity, secretory activity, electron transport activity, pathogen, associated protein regulator activity, nucleic acid binding activity, transcriptional regulator activity, extracellular constitutive and biogenesis activity, and translational regulator activity. Said proteins include proteins from eukaryotic and prokaryotic organisms, said eukaryotic and prokaryotic organisms include microorganisms, viruses, fungi, and parasites, and numerous others, including humans, microorganisms, viruses, fungi, and parasites as targets of drug therapy, and other animals including domesticated animals, microorganisms and other anti-microbial agents used to assay the target of an antibiotic, plants, and even viruses, and numerous others.

"Optionally" refers to the event or circumstance described subsequently can, but need not occur, and the description includes occasions when the event or circumstance does or does not occur. For example, "optionally substituted cyclopropyl" refers to cyclopropyl substitution can, but does not have to exist, and the description includes instances in which cyclopropyl is substituted and instances in which cyclopropyl is not substituted.

"Substituted" refers to one or more hydrogen atoms in the group, preferably up to 5, more preferably 1 to 3 hydrogen atoms are independently substituted of each other by a corresponding number of substituents. It is self-evident that the substituents are only at their chemically feasible positions and that a person skilled in the art is able to determine

possible or impossible substitutions without undue effort (by experiment or theory).

"Pharmaceutically acceptable salt" refers to a salt of the compound disclosed herein, which is safe and efficacious when used in mammals and is biologically active as intended.

## Examples

The following examples are directed to the intermediate compounds and final products identified in the specification and synthesis protocols. The preparation of the compounds of the present invention is described in detail using the following embodiments, but the chemical reactions described are disclosed according to their general applicability to the preparation of the compounds of the present invention. At times, the described reactions can not be applicable to every compound within the scope of the present invention as described. Those skilled in the art can readily recognize compounds where this occurs. In these cases, said reaction successfully can be carried out by conventional improvements known to those skilled in the art. In all methods of preparation, all starting materials are known or can be readily prepared using known raw materials.

The starting materials, chemical reagents, and solvents used in the present invention are all commercially available and purchased from companies such as Anergy Chemical, Shanghai Bide Pharma, Beijing Innochem, Jiangsu Aikon Biopharmaceutical, Sinopharm, Beijing J&K Scientific, Yunnan Xinlanjing, and others.

The compounds synthesized in this application were all structurally determined by nuclear magnetic resonance (NMR) and/or mass spectrometry (MS).

The nuclear magnetic resonance (NMR) determination was carried out with a Bruke AVANCE-400/600 NMR instrument, and the deuterated solvents used were deuterated dimethylsulfoxide (DMSO-$d_6$), deuterated chloroform ($CDCl_3$), and deuterated methanol ($CD_3OD$), and the internal standard was tetramethylsilane (TMS).

Mass spectrometry (MS) measurements were performed with a Waters Acquity UPLC® Plus device.

High-performance liquid chromatography preparations were performed using a Waters 2489 device.

The medium pressure rapid preparation chromatograph was a COMBIFLASH NEXTGEN 300+.

Thin layer chromatography silica gel plates used herein were silica gel 60 thin layer chromatography silica gel plates (aluminum plates with fluorescence).

Silica gel (100-200 mesh, 200-300 mesh) used for silica gel thin layer chromatography was purchased from Innochem.

Reaction process detection in the examples was performed by thin layer chromatography (TLC), and the systems for the unfolding agent used to monitor the reaction and the eluent used to purify the compounds by column chromatography included: petroleum ether/ethyl acetate system, dichloromethane/methanol system.

1. Synthesis of E3 ligase inhibitors

1) Synthesis of E3 Ligase Inhibitor 1

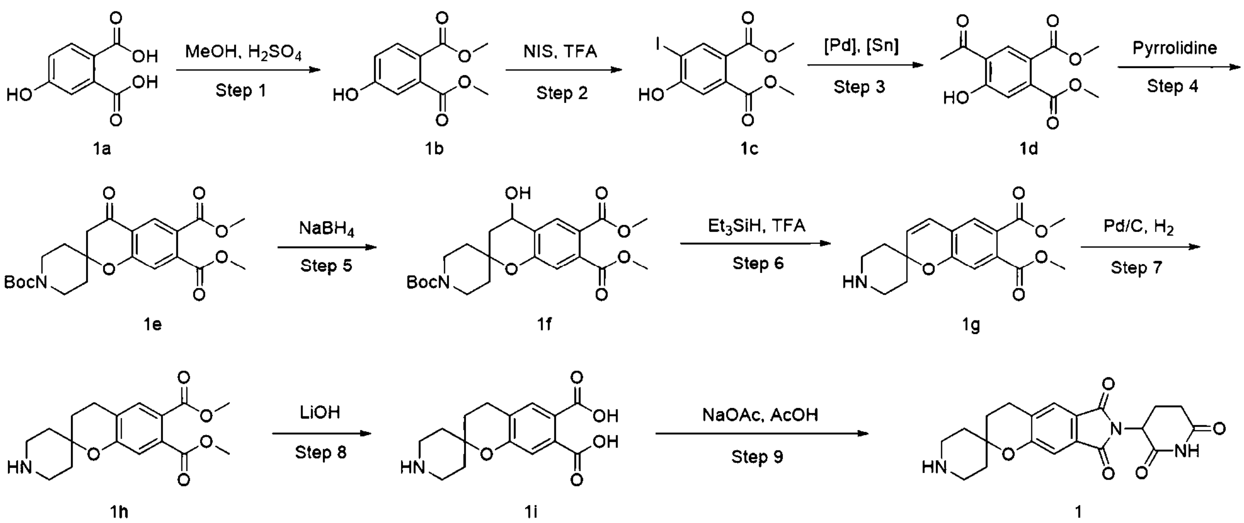

1a 1b 1c 1d 1e 1f 1g 1h 1i 1

Step 1: Dimethyl 4-hydroxyphthalate (Compound 1b)

Concentrated sulfuric acid (45 mL) was slowly added to a solution of compound 1a (30 g, 164.7 mmol) in methanol (300 mL). The mixture was stirred at 65°C for 5 h. The resulting mixture was poured into ice water, filtered, washed with water, and dried under vacuum to afford a white solid compound 1b (30.0 g, 87%).

$^1$H NMR (600 MHz, DMSO-$d_6$) δ 10.66 (s, 1H), 7.70 (d, *J* = 8.5 Hz, 1H), 6.96 (dd, *J* = 8.5, 2.5 Hz, 1H), 6.93 (d, *J*= 2.5 Hz, 1H), 3.79 (s, 3H), 3.76 (s, 3H). LC-MS(ESI): $[M-H]^+$ = 209.22.

Step 2: Dimethyl 4-hydroxy-5-iodophthalate (Compound 1c)

The N-iodosuccinimide (23.6 g, 104.7 mmol) was slowly added to a solution of compound 1b (20.0 g, 95.2 mmol) in trifluoroacetic acid (60 mL). The mixture was stirred at room temperature overnight. The resulting mixture was concentrated under vacuum and purified by C18 reversed-phase column chromatography to afford a white solid compound 1c(16.3 g, 51%).

$^1$H NMR (600 MHz, DMSO-$d_6$) δ 11.59 (s, 1H), 8.11 (s, 1H), 7.02 (s, 1H), 3.79 (s, 3H), 3.77 (s, 3H). LC-MS (ESI): $[M-H]^+$ = 335.06.

Step 3: Dimethyl 4-acetyl-5-hydroxyphthalate (Compound 1d)

Under a nitrogen atmosphere, the compound 1c (15.0 g, 44.6 mmol), tributyl(1-ethoxyvinyl)tin (32.2 g, 89.3 mmol), and bis(triphenylphosphine)palladium(II) dichloride (3.1 g, 4.5 mmol) were dissolved in tetrahydrofuran (75 mL). The mixture was heated to 65°C and stirred for 15 h. After completion, 1M hydrochloric acid solution was added and stirred for 0.5 h, followed by addition of potassium fluoride. The mixture was filtered and the filtrate was concentrated to afford the crude product, which was purified by column chromatography (PE:EA = 0-40%) to afford a yellow oily compound 1d (9.8 g, 87%).

$^1$H NMR (400 MHz, $CDCl_3$) δ 12.65 (s, 1H), 8.37 (s, 1H), 7.12 (s, 1H), 3.96 (s, 3H), 3.92 (s, 3H), 2.73 (s, 3H). LC-MS (ESI): $[M+H]^+$ = 253.15.

Step 4: 1'-(tert-Butyl) 6,7-dimethyl-4-oxaspiro[chroman-2,4'-piperidine]-1',6,7-tricarboxylate (Compound 1e)

Compound 1d (5.0 g, 19.8 mmol), N-(tert-butoxycarbonyl)-4-piperidone (1.4 g, 19.8 mmol), and tetrahydropyrrole (4.0 g, 19.8 mmol) were dissolved in methanol (50 mL). The mixture was stirred at 70°C overnight. The solvent was removed under reduced pressure and the residue was purified by column chromatography (PE:EA = 0-35%) to afford a yellow oily compound 1e (6.9 g, 80%).

$^1$H NMR (600 MHz, DMSO-$d_6$) δ 8.17 (s, 1H), 7.36 (s, 1H), 3.83 (s, 3H), 3.82 (s, 3H), 3.73 (s, 2H), 3.13 (d, *J* = 40.7 Hz, 2H), 2.96 (s, 2H), 1.95 - 1.86 (m, 2H), 1.66 (dq, *J*= 13.2, 5.0 Hz, 2H), 1.40 (s, 9H). LC-MS(ESI): $[M-H]^+$ = 432.22.

Step 5: 1'-(tert-Butyl) 6,7-dimethyl-4-hydroxyspiro[chroman-2,4'-piperidine]-1',6,7-tricarboxylate (Compound 1f)

Under ice bath conditions, sodium borohydride (0.7 g, 17.3 mmol) was added to a solution of compound 1e (5.0 g, 11.5 mmol) in methanol (50 mL). The mixture was stirred at 70°C overnight. The resulting mixture was concentrated under vacuum and purified by column chromatography (PE:EA = 0-50%) to afford a white solid compound 1f (3.3 g, 66%).

$^1$H NMR (600 MHz, DMSO-$d_6$) δ 7.92 (s, 1H), 7.02 (s, 1H), 5.73 (d, *J* = 6.2 Hz, 1H), 4.74 (dt, *J* = 9.3, 6.1 Hz, 1H), 3.79 (s, 6H), 3.74 - 3.64 (m, 2H), 3.06 (s, 2H), 2.18 (dd, *J*= 13.6, 6.1 Hz, 1H), 1.78 (ddt, *J*= 23.0, 13.5, 6.3 Hz, 2H), 1.72 - 1.64 (m, 2H), 1.57 (ddd, *J*= 13.7, 11.3, 4.7 Hz, 1H), 1.40 (s, 9H). LC-MS(ESI): $[M-H]^+$ = 464.39.

Step 6: Dimethyl spiro[chromene-2,4'-piperidine]-6,7-dicarboxylate (Compound 1g)

Compound 1f (3.0 g, 6.9 mmol) and triethylsilane (3.2 g, 27.6 mmol) were dissolved in trifluoroacetic acid (30 mL). The mixture was stirred at 80°C overnight. After concentration under vacuum, the residue was purified by C18 reversed-phase column chromatography to afford a colorless oily compound 1g (1.8 g, 83%).

$^1$H NMR (400 MHz, $CDCl_3$) δ 7.53 (s, 1H), 7.11 (s, 1H), 6.51 (d, *J* = 9.9 Hz, 1H), 5.72 (d, *J*= 9.8 Hz, 1H), 4.66 (s, 2H), 3.93 (s, 3H), 3.89 (s, 3H), 3.42 - 3.31 (m, 4H), 2.23 (d, *J*= 14.6 Hz, 2H), 2.12 - 2.02 (m, 2H). LC-MS(ESI): $[M+H]^+$ = 318.25.

Step 7: Dimethyl Spiro[chroman-2,4'-piperidine]-6,7-dicarboxylate (Compound 1h)

Compound 1g (1.6 g, 5.0 mmol) was dissolved in methanol (20 mL), then palladium on carbon (0.2 g) was added. Under a hydrogen atmosphere, the mixture was stirred at room temperature overnight. After filtration and concentration, a

yellow oily compound 1h was obtained (1.3 g, 81%).

$^1$H NMR (400 MHz, $CDCl_3$) δ 7.61 (s, 1H), 7.14 (s, 1H), 3.93 (s, 3H), 3.89 (s, 3H), 3.35 (d, *J* = 6.2 Hz, 4H), 3.30 (s, 2H), 2.87 (t, *J* = 6.8 Hz, 2H), 2.05 - 2.00 (m, 2H), 1.95 (t, *J*= 6.7 Hz, 2H). LC-MS(ESI): [M+H]$^+$ = 320.28.

Step 8: Spiro[chroman-2,4'-piperidine]-6,7-dicarboxylic Acid (Compound 1i)

Compound 1h (1.5 g, 4.7 mmol) was dissolved in a mixture of methanol and water, then lithium hydroxide (1.7 g, 70.5 mmol) was added. The mixture was stirred at room temperature overnight. After concentration under vacuum, the crude product compound was used directly in the next step without further purification.

LC-MS (ESI): [M+H]$^+$ = 292.21.

Step 9: 7'-(2,6-Dioxopiperidin-3-yl)-3',4'-dihydro-6'H-spiro[piperidine-4,2'-pyrano[2,3-f]isoindole]-6',8'(7'H)-dione (E3 Ligase Inhibitor 1)

Compound 1i (1.3 g, 7.7 mmol) was dissolved in acetic acid (15 mL), and then 3-aminopiperidine-2,6-dione hydrochloride (1.52 g, 9.2 mmol) and sodium acetate (2.1 g, 15.5 mmol) were added. The mixture was stirred at 110°C for 4 h. After purification by reversed-phase column chromatography, a white solid E3 ligase inhibitor 1 was obtained (1.6 g, 81%).

$^1$H NMR (400 MHz, DMSO-*d*$_6$) δ 11.10 (s, 1H), 7.74 (s, 1H), 7.38 (s, 1H), 5.10 (dd, *J*= 12.9, 5.4 Hz, 1H), 3.28 - 3.14 (m, 4H), 2.95 (t, *J* = 6.8 Hz, 2H), 2.91 - 2.83 (m, 1H), 2.65 - 2.53 (m, 2H), 2.07 - 1.99 (m, 1H), 1.93 (q, *J* = 6.2 Hz, 4H), 1.88 - 1.78 (m, 2H). LC-MS(ESI): [M+H]$^+$ = 384.32.

2) Synthesis of E3 Ligase Inhibitor 2

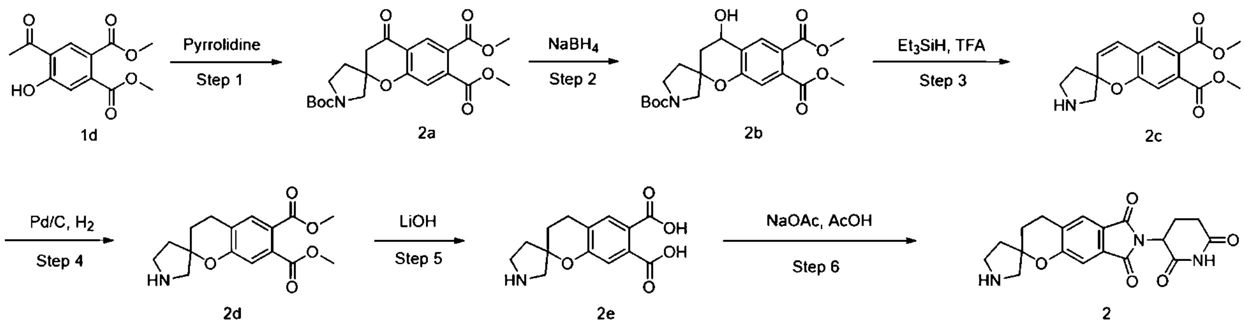


1d 2a 2b 2c 2d 2e 2

Step 1: 1'-(tert-Butyl) 6,7-dimethoxy-4-oxaspiro[chroman-2,3'-pyrrolidine]-1',6,7-tricarboxylate (Compound 2a)

Following the procedure described in Steps 1-3 of example 1 to prepare compound 1d. The ompound 1d (5.0 g, 19.8 mmol), 1-(tert-butoxycarbonyl)-3-pyrrolidinone (1.4 g, 19.8 mmol), and tetrahydropyrrole (4.0 g, 19.8 mmol) were dissolved in methanol (50 mL). The mixture was stirred at 70°C overnight. The solvent was removed under reduced pressure and the residue was purified by column chromatography (PE:EA = 0-35%) to afford a yellow oily compound 2a (5.0 g, 60%).

$^1$H NMR (400 MHz, $CDCl_3$) δ 8.44 (s, 1H), 7.18 (s, 1H), 3.95 (s, 3H), 3.91 (s, 3H), 3.89 - 3.83 (m, 1H), 3.76 - 3.64 (m, 1H), 3.62 - 3.50 (m, 1H), 3.40 (dd, *J*= 17.4, 12.4 Hz, 1H), 3.06 - 2.86 (m, 2H), 2.36 - 2.25 (m, 1H), 1.97 (ddd, *J*= 13.5, 10.4, 9.0 Hz, 1H), 1.47 (d, 9H). LC-MS(ESI): [M-H]$^+$ = 418.40.

Step 2: 1'-(tert-Butyl) 6,7-dimethyl-4-hydroxyspiro[chroman-2,3'-pyrrolidine]-6,7-dicarboxylate (Compound 2b)

Under ice bath conditions, sodium borohydride (0.7 g, 17.9 mmol) was added to a solution of compound 2a (5.0 g, 11.5 mmol) in methanol (50 mL). The mixture was stirred at 70°C overnight. The resulting mixture was concentrated under vacuum and purified by column chromatography (PE:EA = 0-50%) to afford a white solid compound 2b (5.0 g, 99%). The crude product was used directly in the next step without further purification.

LC-MS (ESI): [M+Na]$^+$ = 444.33.

Step 3: Methyl spiro[chromene-2,3'-pyrrolidine]-6,7-dicarboxylate (Compound 2c)

Compound 2b (3.0 g, 7.1 mmol) and triethylsilane (3.3 g, 28.5 mmol) were dissolved in trifluoroacetic acid (30 mL). The mixture was stirred at 80°C overnight. The resulting mixture was concentrated under vacuum and purified by C18 reversed-phase column chromatography to afford a colorless oily compound 2c (1.1 g, 51%).
$^1$H NMR (600 MHz, $CDCl_3$) δ 7.53 (s, 1H), 7.09 (s, 1H), 6.61 (d, *J*= 9.8 Hz, 1H), 5.79 (d, *J* = 9.9 Hz, 1H), 4.53 (br s, 2H), 3.91 (s, 3H), 3.89 (s, 3H), 3.70 - 3.55 (m, 3H), 3.27 (d, *J* = 12.5 Hz, 1H), 2.54 (dd, *J*= 14.2, 6.4 Hz, 1H), 2.14 (ddd, *J* = 13.8, 11.0, 6.5 Hz, 1H). LC-MS(ESI): $[M+H]^+$ = 304.27.

Step 4: Dimethyl spiro[chroman-2,3'-pyrrolidine]-6,7-dicarboxylate (Compound 2d)

A solution of compound 2c (1.0 g, 3.3 mmol) in methanol (20 mL) was treated with palladium on carbon (0.1 g). The mixture was stirred under a hydrogen atmosphere at room temperature overnight. The resulting mixture was filtered and concentrated to afford a yellow oily compound 2d (1.0 g, 99%).
$^1$H NMR (600 MHz, $CDCl_3$) δ 7.61 (s, 1H), 7.09 (s, 1H), 3.90 (s, 3H), 3.88 (s, 3H), 3.52 (dtd, *J* = 17.8, 11.4, 7.8 Hz, 2H), 3.44 (dd, *J*= 12.7, 1.6 Hz, 1H), 3.25 (d, *J* = 12.6 Hz, 1H), 2.90 (dtd, *J* = 17.3, 10.7, 6.9 Hz, 2H), 2.28 - 2.23 (m, 1H), 2.14 - 2.10 (m, 2H), 2.08 - 2.02 (m, 1H). LC-MS(ESI): $[M+H]^+$ = 306.22.

Step 5: Spiro[chroman-2,3'-pyrrolidine]-6,7-dicarboxylic acid (Compound 2e)

A suspension of compound 2d (1.0 g, 3.3 mmol) in methanol and water was treated with lithium hydroxide (1.2 g, 49.1 mmol). The mixture was stirred at room temperature overnight. The resulting mixture was concentrated under vacuum and the crude product was used directly in the next step without further purification.
LC-MS (ESI): $[M+H]^+$ = 278.22.

Step 6: 7-(2,6-Dioxopiperidin-3-yl)-3,4-dihydro-6H-spiro[pyrano[2,3-f]isoindole-2,3'-pyrrolidine]-6,8(7H)-dione (E3 Ligase Inhibitor 2)

A solution of compound 2e (801 mg, 4.9 mmol) in acetic acid (5 mL) was treated with 3-aminopiperidine-2,6-dione hydrochloride (0.97 g, 6.0 mmol) and sodium acetate (1.3 g, 9.7 mmol). The mixture was stirred at 110°C for 4 h. After completion, the resulting mixture was purified by reversed-phase column chromatography to afford a white solid E3 ligase inhibitor 2 (312 mg, 26%).
$^1$H NMR (400 MHz, DMSO-$d_6$) δ 11.11 (s, 1H), 7.78 (s, 1H), 7.18 (s, 1H), 5.10 (dd, *J*= 12.9, 5.4 Hz, 1H), 3.50 - 3.21 (m, 4H), 3.03 - 2.84 (m, 4H), 2.66 - 2.53 (m, 2H), 2.24 - 2.00 (m, 4H). LC-MS(ESI): $[M+H]^+$ = 370.34.

3) Synthesis of E3 Ligase Inhibitor 3

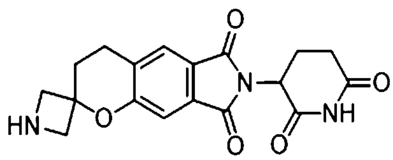

7'-(2,6-Dioxopiperidin-3-yl)-3',4'-dihydro-6'H-spiro[azetidine-3,2'-pyrano[2,3-f]isindole]-6',8'(7'H)-dione

7'-(2,6-Dioxopiperidin-3-yl)-3',4'-dihydro-6'H-spiro[azetidine-3,2'-pyrano[2,3-f]isindole]-6',8'(7'H)-dione was prepared according to Steps 1-6 in example 2.
$^1$H NMR (600 MHz, DMSO-$d_6$) δ 11.12 (s, 1H), 9.12 (d, *J* = 49.0 Hz, 2H, NH), 7.76 (s, 1H), 7.30 (s, 1H), 5.10 (dd, *J*= 12.9, 5.4 Hz, 1H), 4.16 (t, *J* = 8.7 Hz, 4H), 2.97 (t, *J* = 6.5 Hz, 2H), 2.88 (ddd, *J* = 17.0, 13.9, 5.5 Hz, 1H), 2.59 (dt, *J*= 17.1, 3.1 Hz, 1H), 2.54 (dd, *J* = 13.1, 4.5 Hz, 1H), 2.22 (t, *J* = 6.5 Hz, 2H), 2.06 - 2.00 (m, 1H). LC-MS(ESI): $[M+H]^+$ = 356.26.

4) Synthesis of E3 Ligase Inhibitor 4

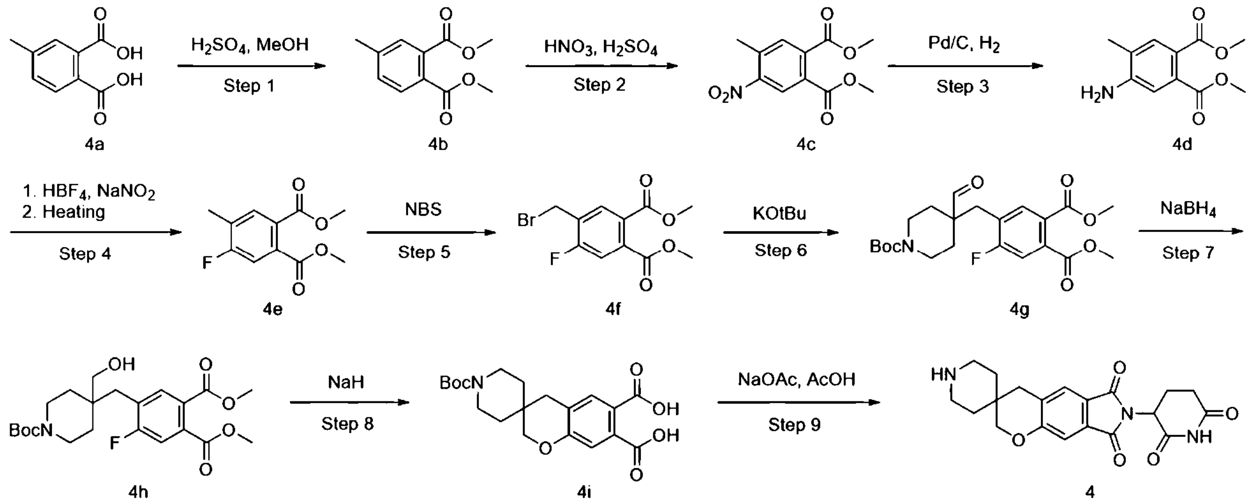


Step 1: Dimethyl 4-methylphthalate (Compound 4b)

Compound 4a (10 g, 55.56 mmol) was dissolved in methanol (100 mL), and concentrated sulfuric acid (15 mL) was added. The mixture was stirred at room temperature overnight. The resulting mixture was poured into ice water, extracted with ethyl acetate, and concentrated to afford a colorless oily compound 4b (10.6 g, 91% yied). The crude product was used directly in the next step.

$^1$H NMR (400 MHz, $CDCl_3$) δ 7.66 (d, *J* = 7.9 Hz, 1H), 7.46 (d, *J* = 1.7 Hz, 1H), 7.32 (dd, *J* = 7.9, 1.8, 1H), 3.89 (s, 3H), 3.88 (s, 3H), 2.40 (s, 3H).

Step 2: Dimethyl 4-methyl-5-nitrophthalate (Compound 4c)

Compound 4b (10 g, 48 mmol) was dissolved in concentrated sulfuric acid (100 mL), and concentrated nitric acid (25 mL, 68%) was slowly added. The mixture was stirred at room temperature overnight. The resulting mixture was poured into ice water, extracted with ethyl acetate, and concentrated. The crude product was purified by column chromatography to afford a white solid compound 4c (5.5 g, 45% yied).

$^1$H NMR (400 MHz, $CDCl_3$) δ 8.43 (s, 1H), 7.64 (s, 1H), 3.97 (s, 3H), 3.96 (s, 3H), 2.69 (s, 3H).

Step 3: Dimethyl 4-amino-5-methylphthalate (Compound 4d)

Compound 4c (5.1 g, 20 mmol) was dissolved in methanol (100 mL), and palladium on carbon (0.51 g) was added. The reaction was purged with nitrogen followed by hydrogen and carried out at room temperature overnight. The mixture was filtered through diatomaceous earth, and the filtrate was concentrated to afford a yellow oily compound 4d (4 g, 91%). The crude product was used directly in the next step.

$^1$H NMR (400 MHz, DMSO-*$d_6$*) δ 7.46 (s, 1H), 6.68 (s, 1H), 5.91 (s, 2H), 3.75 (s, 3H), 3.71 (s, 3H). LC-MS(ESI): $[M+Na]^+$ = 246.18.

Step 4: Dimethyl 4-fluoro-5-methylphthalate (Compound 4e)

Compound 4d (4 g, 17.92 mmol) was suspended in 10% fluoroboric acid (32 mL). After stirring for 0.5 h, the mixture was cooled to 0-5°C, followed by slow addition of an aqueous solution of $NaNO_2$ (1.36 g, 19.71 mmol) for diazotization. The reaction was then stirred in an ice bath for another 0.5 h. The tetrafluoroborate salt was filtered, dried under vacuum, and then stirred in toluene at 110°C. After completion, the resulting mixture was extracted with ethyl acetate, washed with water and saturated brine, dried over anhydrous sodium sulfate, and concentrated. The crude product was purified by column chromatography to afford a pale yellow oily compound 4e(2.31 g, 57%).

$^1$H NMR (400 MHz, $CDCl_3$) δ 7.61 (dd, *J* = 7.2, 0.9 Hz, 1H), 7.38 (d, *J*= 9.4 Hz, 1H), 3.92 (s, 3H), 3.91 (s, 3H), 2.35 (d, *J*= 2.0 Hz, 3H).

Step 5: Dimethyl 4-(bromomethyl)-5-fluorophthalate (Compound 4f)

Compound 4e (2.26 g, 10 mmol) was dissolved in carbon tetrachloride, and N-bromosuccinimide (2.14 g, 12 mmol) and azobisisobutyronitrile (0.16 g, 1 mmol) were added. The mixture was refluxed overnight under heating. After

concentration, the crude product was purified by reversed-phase column chromatography to afford a white solid compound 4f (2.14 g, 70%).

$^1H$ NMR (400 MHz, $CDCl_3$) δ 7.87 (d, *J*= 7.1 Hz, 1H), 7.40 (d, *J*= 9.4 Hz, 1H), 4.51 (d, *J*= 1.0 Hz, 2H), 3.94 (s, 3H), 3.93 (s, 3H).

Step 6: Dimethyl 4-((1-(tert-butoxycarbonyl)-4-formylpiperidin-4-yl)methyl)-5-fluorophthalate (Compound 4g)

Compound 4f (2.14 g, 7 mmol) was dissolved in anhydrous tetrahydrofuran (30 mL), and potassium tert-butoxide (1.18 g, 10.5 mmol) was slowly added at -30°C. After 0.5 h, a solution of 1-(tert-butoxycarbonyl)piperidine-4-carbaldehyde (1.79 g, 8.4 mmol) in tetrahydrofuran (5 mL) was slowly added at the same temperature. The mixture was warmed to room temperature and stirred overnight. The reaction was quenched with saturated ammonium chloride. The mixture was extracted with ethyl acetate, washed with saturated brine, dried over anhydrous sodium sulfate, and concentrated. The crude product was purified by flash silica gel column chromatography to afford a colorless oily compound 4g (1.68 g, 55%).

$^1H$ NMR (400 MHz, $CDCl_3$) δ 9.58 (d, *J*= 2.3 Hz, 1H), 7.50 (d, *J*= 7.1 Hz, 1H), 7.34 (d, *J*= 9.5 Hz, 1H), 3.96 - 3.84 (m, 8H), 2.91 - 2.74 (m, 4H), 1.96 (d, *J* = 13.0 Hz, 2H), 1.59 - 1.46 (m, 2H), 1.44 (s, 9H). LC-MS(ESI): $[M-Boc+H]^+$ = 338.29.

Step 7: Dimethyl 4-((1-(tert-butoxycarbonyl)-4-(hydroxymethyl)piperidin-4-yl)methyl)-5-fluorophthalate (Compound 4h)

Compound 4g (1.66 g, 3.8 mmol) was dissolved in methanol (15 mL), and sodium borohydride (0.215 g, 5.7 mmol) was added under ice bath conditions. The mixture was stirred at room temperature for 3 h. The reaction was quenched with saturated ammonium chloride. The mixture was extracted with ethyl acetate, washed with saturated brine, dried over anhydrous sodium sulfate, and concentrated. The crude product was purified by column chromatography to afford a colorless oily compound 4h (1.47 g, 88%).

$^1H$ NMR (400 MHz, DMSO-$d_6$) δ 7.74 (d, *J* = 7.0 Hz, 1H), 7.54 (d, *J* = 9.7 Hz, 1H), 4.81 (t, *J* = 5.0 Hz, 1H), 3.82 (s, 3H), 3.82 (s, 3H), 3.48 - 3.39 (m, 2H), 3.21 (d, *J*= 5.0 Hz, 4H), 2.74 (s, 2H), 1.42 - 1.32 (m, 11H), 1.27 - 1.14 (m, 2H). LC-MS(ESI): $[M-Boc+H]^+$ = 340.41.

Step 8: 1'-(tert-Butoxycarbonyl)spiro[chroman-3,4'-piperidine]-6,7-dicarboxylic acid (Compound 4i)

Compound 4h (1.45 g, 3.3 mmol) was dissolved in dry N,N-dimethylformamide (6 mL), and sodium hydride (0.4 g, 16.5 mmol) was added. The mixture was stirred at 110°C for 2 h. The reaction was quenched with acetic acid. The mixture was purified by reversed-phase column chromatography to afford a white solid compound 4i (0.65 g, 51%).

$^1H$ NMR (400 MHz, DMSO-$d_6$) δ 12.93 (s, 2H), 7.50 (s, 1H), 6.92 (s, 1H), 3.99 (s, 2H), 3.53-3.42 (m, 2H), 3.33-3.23 (m, 2H), 2.75 (s, 2H), 1.40 (s, 9H), 1.39-1.28 (m, 4H). LC-MS(ESI): $[M-Boc+H]^+$ = 292.23.

Step 9: 7'-(2,6-Dioxopiperidin-3-yl)-2'H-spiro[piperidine-4,3'-pyrano[2,3-f]isoindole]-6',8'(4'H,7'H)-dione (E3 Ligase Inhibitor 4)

Compound 4i (0.63 g, 1.6 mmol) was dissolved in acetic acid (4 mL), and 3-amino-2,6-piperidinedione hydrochloride (0.33 g, 2.0 mmol) and sodium acetate (0.39 g, 4.8 mmol) were added. The mixture was stirred at 110°C overnight. After completion, the resulting mixture was purified by reversed-phase column chromatography to afford a white solid E3 ligase inhibitor 4 (0.5 g, 82%).

$^1H$ NMR (400 MHz, DMSO-$d_6$) δ 11.12 (s, 1H), 8.51 (s, 2H), 7.69 (s, 1H), 7.29 (s, 1H), 5.10 (dd, *J*= 12.7, 5.4 Hz, 1H), 4.16 (s, 2H), 3.25 - 3.05 (m, 4H), 2.92 (s, 2H), 2.90 - 2.82 (m, 1H), 2.64 - 2.52 (m, 2H), 2.05 - 1.99 (m, 1H), 1.67 - 1.51 (m, 4H). LC-MS(ESI): $[M+H]^+$ = 384.36.

5) Synthesis of E3 Ligase Inhibitor 5

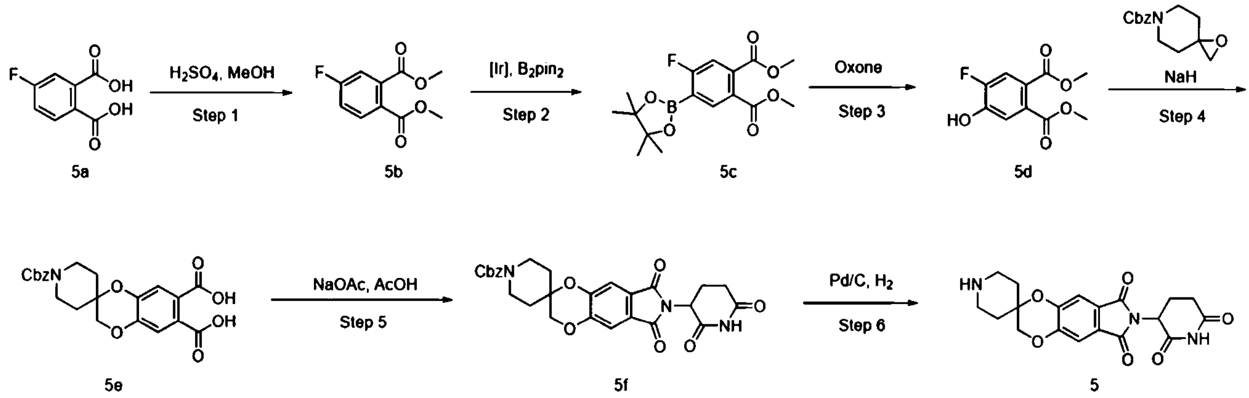

5a 5b 5c 5d
5e 5f 5

Step 1: Dimethyl 4-fluorophthalate (Compound 5b)

Concentrated sulfuric acid (15 mL) was added dropwise to a solution of compound 5a (5.5 g, 30.0 mmol) in methanol (100 mL). The mixture was stirred at room temperature overnight. The resulting mixture was poured into ice water, extracted with ethyl acetate, washed with saturated brine, dried over anhydrous sodium sulfate, and concentrated to afford a colorless oily compound 5b (5.7 g, 90%). The crude product was used directly in the next step without further purification.

$^1$H NMR (400 MHz, $CDCl_3$) δ 7.82 (dd, *J* = 8.6, 5.3 Hz, 1H), 7.38 (dd, *J* = 8.6, 2.6 Hz, 1H), 7.82 (ddd, *J* = 8.6, 5.3, 2.6 Hz, 1H), 3.96 (s, 3H), 3.92 (s, 3H).

Step 2: Dimethyl 4-fluoro-5-(4,4,5,5-tetramethyl-1,3,2-dioxaborolan-2-yl)phthalate (Compound 5c)

Bis(pinacolato)diboron (4.5 g, 17.5 mmol), 4,4'-di-tert-butyl-2,2'-bipyridine (0.13 g, 0.47 mmol), and methoxy(cyclooctadiene)iridium(I) dimer (0.16 g, 0.23 mmol) were dissolved in methyl tert-butyl ether (10 mL). Then a solution of compound 5b (2.5 g, 11.8 mmol) in methyl tert-butyl ether (10 mL) was added. The reaction was purged with nitrogen and the rection mixture was stirred at 100°C for 4 h. The resulting mixture was filtered through diatomaceous earth, and the filtrate was concentrated to afford the crude product (3.2 g), which was used directly in the next step without further purification.

Step 3: Dimethyl 4-fluoro-5-hydroxyphthalate (Compound 5d)

The potassium peroxymonosulfate (6.15 g) was added to a solution of compound 5c (3.38 g, 10 mmol) in acetonitrile (50 mL),. The mixture was stirred at room temperature overnight. The resulting mixture was filtered, and the filtrate was concentrated. The crude product was purified by silica gel column chromatography to afford a white solid compound 5d (1.6 g, 70%).

$^1$H NMR (400 MHz, $CDCl_3$) δ 8.39 (s, 1H), 7.50 (d, *J* = 10.8 Hz, 1H), 7.19 (d, *J* = 8.1 Hz, 1H), 3.88 (s, 3H), 3.86 (s, 3H). LC-MS(ESI): $[M+H]^+$ = 229.18.

Step 4: 1'-((Benzyloxy)carbonyl)-3H-spiro[benzo[b][1,4]dioxin-2,4'-piperidine]-6,7-dicarboxylic acid (Compound 5e)

Compound 5d (23 mg, 0.1 mmol) was dissolved in dry N,N-dimethylformamide (1 mL), and 1-oxa-6-azaspiro[2.5] octane-6-carboxylic acid benzyl ester (25 mg, 0.1 mmol) and sodium hydride (6 mg, 0.25 mmol) were added. The mixture was heated to 110°C for 2 days. The resulting mixture was purified by reversed-phase column chromatography to afford a white solid compound 5e (13 mg, 31%).

$^1$H NMR (600 MHz, $CD_3OD$) δ 7.41-7.35 (m, 4H), 7.33 (dd, *J* = 5.9, 2.9 Hz, 1H), 7.29 (s, 1H), 7.27 (s, 1H), 5.15 (s, 2H), 4.07 (s, 2H), 4.01 (dt, *J* = 13.6, 4.0 Hz, 2H), 3.42-3.32 (m, 2H), 1.82 (d, *J* = 13.9 Hz, 2H), 1.76-1.67 (m, 2H). LC-MS(ESI): $[M+H]^+$ = 428.36.

Step 5: Benzyl 7'-(2,6-dioxopiperidin-3-yl)-6',8'-dioxo-7',8'-dihydro-3'H,6'H-spiro[piperidine-4,2'-[1,4]dioxino[2,3-f]isoindole]-1-carboxylate (Compound 5f)

3-aminopiperidine-2,6-dione hydrochloride (6 mg, 0.036 mmol) and sodium acetate (7 mg, 0.09 mmol) were added to a solution of compound 5e (13 mg, 0.03 mmol) dissolved in acetic acid (1 mL), and the mixture was heated to 110°C and stirred for 4 h. After completion, the resulting mixture was purified by reversed-phase column chromatography

to afford a white solid compound 5f (12 mg, 77%).

$^1$H NMR (600 MHz, $CD_3OD$) δ 7.42 (s, 1H), 7.40-7.31 (m, 6H), 5.15 (s, 2H), 5.08 (dd, *J* = 12.9, 5.5 Hz, 1H), 4.14 (s, 2H), 4.01 (dt, *J*= 13.8, 4.0 Hz, 2H), 3.43-3.32 (m, 2H), 2.91-2.83 (m, 1H), 2.79-2.69 (m, 2H), 2.14-2.09 (m, 1H), 1.83 (d, *J* = 13.9 Hz, 2H), 1.74 (td, *J* = 14.4, 12.9, 4.8 Hz, 2H). LC-MS(ESI): $[M+H]^+$ = 520.29.

Step 6: 7'-(2,6-Dioxopiperidin-3-yl)-7'-hydro-3'H,6'H-spiro[piperidine-4,2'-[1,4]dioxino[2,3-f]isoindole]-6',8'-dione (E3 Ligase Inhibitor 5)

Compound 5f (11 mg, 0.02 mmol) was dissolved in methanol, and palladium on carbon (5 mg) was added. The reaction was purged with nitrogen and the mixture was stirred for 6 h. The resulting mixture was filtered through diatomaceous earth, and the filtrate was concentrated to afford a white solid E3 ligase inhibitor 5 (7 mg, 90%).

$^1$H NMR (400 MHz, DMSO-$d_6$) δ 11.11 (s, 1H), 7.50 (s, 1H), 7.48 (s, 1H), 5.09 (dd, *J*= 12.8, 5.3 Hz, 1H), 4.27 (s, 2H), 3.25 (d, *J* = 12.7 Hz, 2H), 3.18 - 3.11 (m, 2H), 2.89 (ddd, *J* = 16.7, 13.7, 5.3 Hz, 1H), 2.64 - 2.53 (m, 2H), 2.03 (ddd, *J*= 13.3, 5.7, 3.4 Hz, 1H), 1.92 - 1.86 (m, 4H). LC-MS(ESI): $[M+H]^+$ = 386.32.

6) Synthesis of E3 Ligase Inhibitor 6

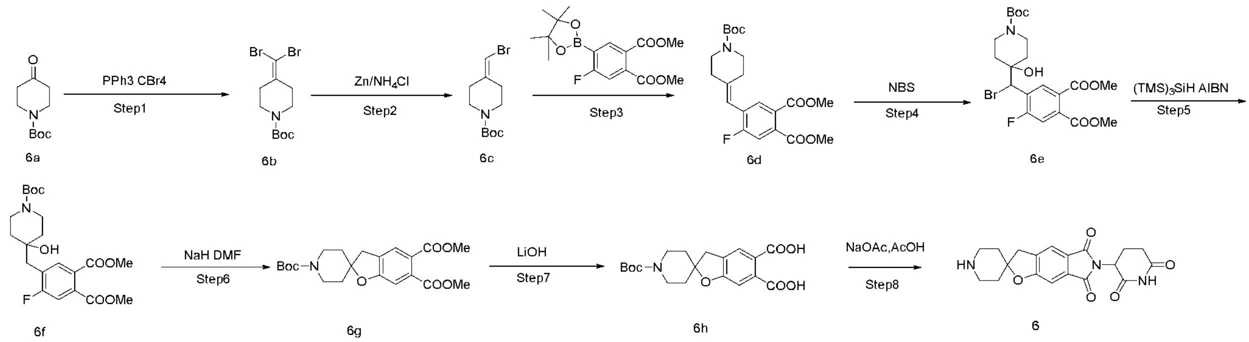


Step 1: tert-Butyl 4-(dibromomethyl)piperidine-1-carboxylate (Compound 6b)

Under nitrogen atmosphere, compound 6a (5 g, 25.1 mmol) and triphenylphosphine (26.3 g, 100.4 mmol) were dissolved in anhydrous acetonitrile (50 mL). Carbon tetrabromide (16.7 g, 50.2 mmol) was added in portions at 0°C. After 30 min, the reaction was warmed to room temperature and carried out overnight. The mixture was filtered, and the filtrate was concentrated under reduced pressure. The crude product was purified by column chromatography to afford a white solid compound 6b (5.5 g, 62%).

$^1$H NMR (600 MHz, DMSO-$d_6$) δ 3.37 (s, 4H), 2.41 (dd, *J*= 7.2, 4.7 Hz, 4H), 1.41 (d, *J* = 0.9 Hz, 9H).

Step 2: tert-butyl 4-(bromomethyl)piperidine-1-carboxylate (Compound 6c)

Under nitrogen atmosphere, compound 6b (5.2 g, 18.9 mmol) was dissolved in tetrahydrofuran (34 mL) and methanol (17 mL). Ammonium chloride (4.04 g, 75.6 mmol) was added at 0°C. After 30 min, zinc powder (4.9 g, 75.6 mmol) was added in portions. The mixture was stirred at room temperature overnight. The mixture was filtered, and the filtrate was concentrated under reduced pressure. The crude product was purified by column chromatography to afford a colorless oily compound 6c (2.6 g, 50%).

$^1$H NMR (600 MHz, DMSO-$d_6$) δ 6.26 (t, *J* = 1.3 Hz, 1H), 3.34 (dt, *J* = 16.4, 6.3 Hz, 4H), 2.32-2.27 (m, 2H), 2.23 (ddd, *J*= 7.2, 4.4, 1.2 Hz, 2H), 1.41 (s, 9H).

Step 3: Dimethyl 4-((1-(tert-butoxycarbonyl)piperidin-4-ylidene)methyl)-5-fluorophthalate (Compound 6d)

Under nitrogen atmosphere, compound 6c (2 g, 7.3 mmol) were prepared according to Steps 1-2 in example 5 to afford compound 5c (2.64 g, 7.8 mmol). The palladium acetate (163 mg, 0.73 mmol), triphenylphosphine (382 mg, 1.46 mmol), and cesium carbonate (7.14 g, 21.9 mmol) were added sequentially to a reaction flask and dissolved in 1,4-dioxane (20 mL) and water (2 mL). The reaction purged with nitrogen and carried out at 110°C for 4 h. The mixture was cooled to room temperature, diluted with water, and extracted with ethyl acetate. The organic phase was washed with saturated brine, dried over anhydrous sodium sulfate, and concentrated. The crude product was purified by column chromatography

to afford a pale yellow oily compound 6d (1.4 g, 47%).

$^1H$ NMR (600 MHz, DMSO-$d_6$) δ 7.65 (d, *J*= 7.0 Hz, 1H), 7.60 (d, *J* = 9.7 Hz, 1H), 6.32 (s, 1H), 3.83 (s, 3H), 3.82 (s, 3H), 3.43 (t, *J* = 5.8 Hz, 2H), 3.35 (s, 1H), 3.33 (s, 1H), 2.37 - 2.32 (m, 2H), 2.25 (t, *J* = 5.9 Hz, 2H), 1.42 (s, 9H). LC-MS(ESI): $[M-Boc+H]^+$ = 308.28.

Step 4: Dimethyl 4-(bromo(1-(tert-butoxycarbonyl)-4-hydroxypiperidin-4-yl)methyl)-5-fluorophthalate (Compound 6e)

Under a nitrogen atmosphere, compound 6d (7 g, 17.2 mmol) was dissolved in tetrahydrofuran (140 mL) and water (140 mL), and N-bromosuccinimide (6.12 g, 34.4 mmol) was added. The mixture was stirred at room temperature overnight. The mixture was concentrated to remove tetrahydrofuran, water was then added, and extracted with ethyl acetate. The organic phase was washed with saturated brine, dried over anhydrous sodium sulfate, and concentrated. The crude product was purified by column chromatography to afford a pale yellow oily compound 6e (4.9 g, 56%).

LC-MS(ESI): $[M-Boc+H]^+$ = 404.30/406.30.

Step 5: Dimethyl 4-((1-(tert-butoxycarbonyl)-4-hydroxypiperidin-4-yl)methyl)-5-fluorophthalate (Compound 6f)

Under a nitrogen atmosphere, compound 6e (10.9 g, 21.61 mmol) was dissolved in toluene (80 mL), and tris(trimethylsilyl)silane (8.06 g, 32.42 mmol) and azobisisobutyronitrile (357 mg, 2.16 mmol) were added. The mixture was stirred at 90°C overnight. The mixture was concentrated to remove toluene, and the crude product was purified by column chromatography to afford a pale yellow oily compound 6f (1.5 g, 16%).

$^1H$ NMR (600 MHz, DMSO-$d_6$) δ 7.77 (d, *J*= 6.9 Hz, 1H), 7.52 (d, *J*= 9.5 Hz, 1H), 4.62 (s, 1H), 3.82 (d, *J*= 2.1 Hz, 6H), 3.66 (s, 2H), 3.34 (s, 4H), 2.79 (s, 2H), 1.38 (s, 9H). LC-MS(ESI): $[M-Boc+H]^+$ = 326.37.

Step 6: 1'-(tert-Butyl) 5,6-dimethyl 3H-spiro[benzofuran-2,4'-piperidine]-1',5,6-tricarboxylate (Compound 6g)

Under a nitrogen atmosphere, compound 6f (1.6 g, 3.76 mmol) was dissolved in N,N-dimethylformamide (2 mL), and sodium hydride (300 mg, 7.52 mmol) was added. The mixture was stirred at 110°C overnight. The mixture was diluted with water and extracted with ethyl acetate. The organic phase was washed with saturated brine, dried over anhydrous sodium sulfate, and concentrated. The crude product was purified by reversed-phase column chromatography to afford a pale yellow solid compound 6g (660 mg, 43%).

$^1H$ NMR (600 MHz, $CDCl_3$) δ 7.58 (s, 1H), 7.15 (s, 1H), 3.93 (s, 3H), 3.89 (s, 3H), 3.56 (t, *J* = 5.7 Hz, 2H), 3.42 (t, *J* = 5.8 Hz, 2H), 2.42 (t, *J* = 5.9 Hz, 2H), 2.24 (t, *J* = 5.9 Hz, 2H), 1.61 (s, 2H), 1.50 (s, 9H). LC-MS(ESI): $[M-Boc+H]^+$ = 306.23

Step 7: 1'-(tert-Butoxycarbonyl)-3H-spiro[benzofuran-2,4'-piperidine]-5,6-dicarboxylic acid (Compound 6h)

Under a nitrogen atmosphere, compound 6g (660 mg, 1.63 mmol) was dissolved in methanol (9 mL) and water (1 mL), and lithium hydroxide (389 mg, 16.28 mmol) was added. The mixture was stirred at room temperature overnight. The mixture was concentrated, and the crude product was purified by reversed-phase column chromatography to afford a pale yellow solid compound 6h (600 mg, 97%).

$^1H$ NMR (400 MHz, DMSO-$d_6$) δ 7.39 (s, 1H), 6.37 (s, 1H), 3.17 (s, 2H), 2.29 (d, *J*= 51.9 Hz, 4H), 1.98 (dd, *J* = 13.7, 6.9 Hz, 2H), 1.64 (s, 2H), 1.41 (s, 9H). LC-MS(ESI): $[M-Boc+H]^+$ = 278.26.

Step 8: 6-(2,6-Dioxopiperidin-3-yl)spiro[furo[2,3-f]isoindole-2,4'-piperidine]-5,7(3H,6H)-dione (E3 Ligase Inhibitor 6)

Compound 6h (400 mg, 1.06 mmol) was dissolved in acetic acid (5 mL), and 3-aminopiperidine-2,6-dione hydrochloride (169 mg, 1.32 mmol) and sodium acetate (260 mg, 3.18 mmol) were added. The mixture was stirred at 110°C for 4 h. After completion, the mixture was purified by reversed-phase column chromatography to afford a white solid E3 ligase inhibitor 6 (170 mg, 43%).

$^1H$ NMR (400 MHz, DMSO-$d_6$) δ 7.46 (s, 1H), 7.11 (s, 1H), 5.04 (dd, *J*= 12.8, 5.4 Hz, 1H), 2.99-2.79 (m, 5H), 2.62-2.54 (m, 1H), 2.53 (s, 1H), 2.38 (q, *J* = 6.3 Hz, 4H), 2.04-1.96 (m, 1H), 1.90 (s, 2H). LC-MS(ESI): $[M+H]^+$ = 370.34.

7) Synthesis of E3 Ligase Inhibitor 7

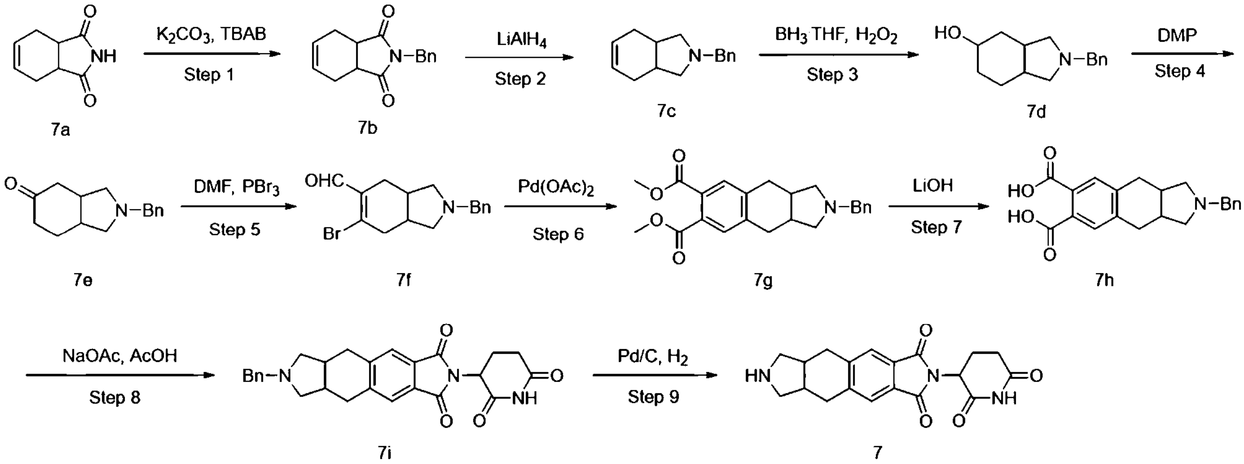


Step 1: 2-Benzyl-3a,4,7,7a-tetrahydro-1H-isoindole-1,3(2H)-dione (Compound 7b)

Compound 7a (5 g, 33 mmol) was dissolved in anhydrous acetonitrile (50 mL). Tetrabutylammonium bromide (1.23 g, 3.3 mmol) and anhydrous potassium carbonate (14 g, 99 mmol) were added, followed by adding the benzyl chloride (5.5 g, 43 mmol). The mixture was stirred at 30°C overnight. After quenching with water, the mixture was extracted with ethyl acetate. The organic phase was washed with saturated brine, dried over anhydrous sodium sulfate, and concentrated to afford a white solid compound 7b (7.5 g, 94%).

$^1H$ NMR (400 MHz, $CDCl_3$) δ 7.37-7.20 (m, 5H), 5.95-5.83 (m, 2H), 4.63 (s, 2H), 3.20-3.01 (m, 2H), 2.61 (m, 2H), 2.31-2.10 (m, 2H). LC-MS(ESI): $[M+H]^+$ = 242.22.

Step 2: 2-Benzyl-2,3,3a,4,7,7a-hexahydro-1H-isoindole (Compound 7c)

Lithium aluminum hydride (3.2 g, 82 mmol) was added to anhydrous tetrahydrofuran (40 mL) at 0°C, followed by adding the compound 7b (5 g, 20.7 mmol). After reacting in an ice bath for 5 min, the mixture was transferred to a 70°C oil bath for 3 h. After completion of the reaction, water was slowly added dropwise to the reaction mixture under ice bath conditions until no further gas evolution was observed. The mixture was filtered, and extracted with ethyl acetate. The organic phase was washed with saturated brine, dried over anhydrous sodium sulfate, and concentrated to afford a yellow oily compound 7c (3.2 g, 73%).

$^1H$ NMR (400 MHz, $CDCl_3$) δ 7.43-7.24 (m, 5H), 5.87 (t, *J*= 2.7 Hz, 2H), 3.67 (s, 2H), 3.05-2.90 (m, 2H), 2.44 (m, 2H), 2.29-2.16 (m, 4H), 1.92 (m, 2H).

Step 3: 2-Benzyloctahydro-1H-isoindol-5-ol (Compound 7d)

Compound 7c (5 g, 23.44 mmol) was dissolved in tetrahydrofuran (20 mL) at 0°C, and 2M boranetetrahydrofuran complex (25 mL) was added under ice bath conditions. After 12 h, methanol (13 mL), 3M sodium hydroxide (10.5 mL), and hydrogen peroxide (10.5 mL) were added. The mixture was heated to 60°C for 6 h. After cooling to room temperature, the resulting mixture was extracted with ethyl acetate. The organic phase was washed with saturated brine, dried over anhydrous sodium sulfate, and concentrated. The crude product was purified by column chromatography to afford the compound 7d (1.2 g, 22.2%).

$^1H$ NMR (400 MHz, $CDCl_3$) δ 7.43-7.18 (m, 5H), 3.84 (m, 1H), 3.78 (s, 2H), 2.94 (dd, *J* = 9.8, 6.7 Hz, 1H), 2.82 (dd, *J*= 9.5, 7.7 Hz, 1H), 2.64 (dd, *J*= 9.5, 8.4 Hz, 1H), 2.60-2.44 (m, 2H), 2.12 (m, 1H), 1.89-1.72 (m, 3H), 1.54 (m, 1H), 1.47-1.35 (m, 1H), 1.35-1.22 (m, 2H). LC-MS(ESI): $[M+H]^+$ = 232.31.

Step 4: 2-Benzyloctahydro-5H-isoindol-5-one (Compound 7e)

Compound 7d (1.2 g, 12.97 mmol) was dissolved in anhydrous dichloromethane, and Dess-Martin periodinane (11 g, 25.95 mmol) was added under ice bath conditions. The reaction was carried out for 12 h. The reaction was quenched with a 1:1 solution of saturated sodium bicarbonate and sodium thiosulfate. The mixture was filtered, and extracted with dichloromethane. The organic phase was washed with saturated brine, dried over anhydrous sodium sulfate, and concentrated to afford a crude product (1 g), which was used directly in the next step.

Step 5: 2-Benzyl-6-bromo-2,3,3a,4,7,7a-hexahydro-1H-isoindole-5-carbaldehyde (Compound 7f)

At 0°C, N,N-dimethylformamide (1.01 mL, 13.08 mmol) was dissolved in dichloromethane (2 mL), and phosphorus tribromide (1.13 mL, 11.77 mmol) was added dropwise and stirred at 0°C for 1 h. A solution of compound 7e (1 g, 2.62 mmol) in dichloromethane was slowly added. The reaction was warmed to room temperature and carried out for 10 h. After completion, the reaction mixture was cooled to 0°C, followed by dropwise addition of saturated sodium bicarbonate solution until gas evolution ceased. The mixture was extracted with dichloromethane. The organic phase was washed with saturated brine, dried over anhydrous sodium sulfate, and concentrated. The crude product was purified by column chromatography to afford compound 7f (270 mg, 20%).

$^1H$ NMR (400 MHz, $CDCl_3$) δ 10.04 (s, 1H), 7.32 (d, *J* = 5.8 Hz, 5H), 3.60 (s, 2H), 3.21-3.10 (m, 2H), 2.85-2.72 (m, 3H), 2.50-2.43 (m, 1H), 2.33 (dd, *J* = 9.2, 5.0 Hz, 1H), 2.24-2.18 (m, 1H), 1.83-1.74 (m, 2H). LC-MS(ESI): $[M+H]^+$ = 320.16.

Step 6: Dimethyl 2-benzyl-2,3,3a,4,9,9a-hexahydro-1H-benzo[f]isoindole-6,7-dicarboxylate (Compound 7g)

Compound 7f (270 mg, 0.84 mmol), dimethyl itaconate (133.4 mg, 0.84 mmol), palladium acetate (9.5 mg, 0.042 mmol), triphenylphosphine (22.11 mg, 0.084 mmol), and sodium acetate (207.5 mg, 2.53 mmol) were sequentially added to a pressure-resistant vessel. The mixture was dissolved in tetrahydrofuran. The reaction was purged with nitrogen and heated to 120°C overnight. The mixture was cooled to room temperature, concentrated, and purified by preparative HPLC to afford compound 7g (71 mg, 22%).

$^1H$ NMR (400 MHz, $CDCl_3$) δ 7.52-7.39 (m, 7H), 4.38-4.14 (m, 3H), 3.91 (d, *J*= 2.8 Hz, 7H), 3.27 (s, 3H), 2.94 (d, *J*= 12.0 Hz, 1H), 2.85-2.72 (m, 3H), 1.55 (d, *J*= 6.6 Hz, 1H). LC-MS(ESI): $[M+H]^+$ = 380.36.

Step 7: 2-Benzyl-2,3,3a,4,9,9a-hexahydro-1H-benzo[f]isoindole-6,7-dicarboxylic acid (Compound 7h)

Compound 7g (71 mg, 0.19 mmol) was dissolved in methanol and water, and lithium hydroxide (90 mg, 3.74 mmol) was added. The mixture was stirred at room temperature for 20 h. The mixture was concentrated to afford the crude product (62 mg, 95%), which was used directly in the next step.

LC-MS(ESI): $[M+H]^+$ = 352.34.

Step 8: 7-Benzyl-2-(2,6-dioxopiperidin-3-yl)-5a,6,7,8,8a,9-hexahydroisoindolo[5,6-f]isoindole-1,3(2H,5H)-dione (Compound 7i)

Compound 7h (62 mg, 0.18 mmol) was dissolved in acetic acid, and sodium acetate (43.2 mg, 0.53 mmol) and 3-amino-2,6-piperidinedione hydrochloride (44 mg, 0.26 mmol) were added. The reaction was heated to 110°C overnight. The mixture was concentrated and purified by preparative HPLC to afford compound 7i (39 mg, 49%).

$^1H$ NMR (400 MHz, DMSO-$d_6$) δ 11.14 (s, 1H), 7.98-7.72 (m, 2H), 7.64-7.41 (m, 5H), 5.14 (dd, *J*= 12.9, 5.4 Hz, 1H), 4.53-4.24 (m, 2H), 4.01 (dd, *J*= 13.9, 7.6 Hz, 1H), 3.76 (s, 3H), 3.12 (d, *J*= 9.7 Hz, 1H), 3.06-2.91 (m, 2H), 2.92-2.76 (m, 3H), 2.67-2.54 (m, 2H), 2.04 (dd, *J* = 12.5, 6.2 Hz, 1H), 1.90-1.59 (m, 2H). LC-MS(ESI): $[M+H]^+$ = 444.41.

Step 9: 2-(2,6-Dioxopiperidin-3-yl)-5a,6,7,8,8a,9-hexahydroisoindolo[5,6-f]isoindole-1,3(2H,5H)-dione (E3 Ligase Inhibitor 7)

Compound 7i (39 mg, 0.088 mmol) was dissolved in methanol (2 mL), and palladium on carbon (4 mg) was added. The reaction was purged with hydrogen and carried out at room temperature overnight. The mixture was filtered, concentrated, and purified by preparative HPLC to afford E3 ligase inhibitor 7 (12 mg, 38%).

$^1H$ NMR (400 MHz, DMSO-$d_6$) δ 11.13 (s, 1H), 9.07-8.95 (m, 1H), 7.95-7.68 (m, 2H), 5.14 (dd, *J* = 13.0, 5.3 Hz, 1H), 3.96-3.87 (m, 2H), 3.51 (m, 2H), 3.12 (m, 1H), 3.04-2.82 (m, 4H), 2.72-2.55 (m, 2H), 2.06 (m, 1H), 1.86 (m, 1H), 1.65-1.46 (m, 1H). LC-MS(ESI): $[M+H]^+$ = 354.41.

8) Synthesis of E3 Ligase Inhibitor 8

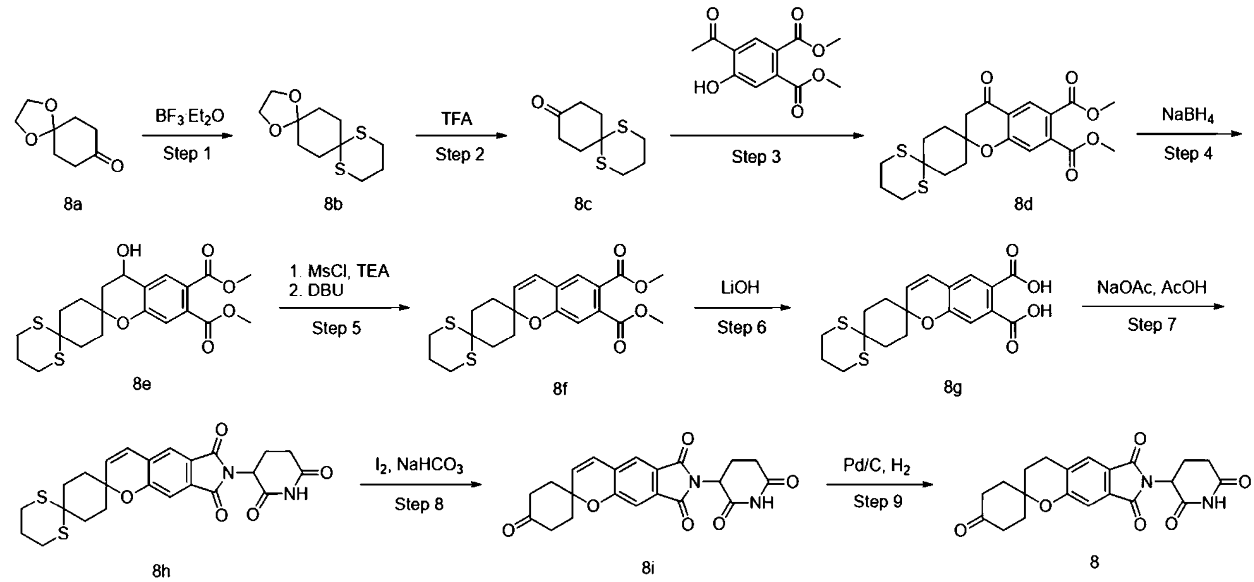


Step 1: 1,4-Dioxa-9,13-dithiaspiro[4.2.5[8].2[5]]pentadecane (Compound 8b)

Compound 8a (10.0 g, 64.0 mmol) was dissolved in dichloromethane (200 mL), and 1,3-propanedithiol (7.0 g, 64.0 mmol) was added. Boron trifluoride etherate (32 mmol) was slowly added dropwise at -18°C. The mixture was stirred at -18°C for 4 h. The solvent was removed under reduced pressure, and the residue was diluted with water (500 mL). The solid was filtered and purified by C18 column chromatography to afford compound 8b (2 g, 12.7%).

$^1$H NMR (400 MHz, DMSO-$d_6$) δ 3.85 (s, 4H), 2.84-2.78 (m, 4H), 2.06-1.98 (m, 4H), 1.93-1.80 (m, 2H), 1.70-1.59 (m, 4H). LC-MS(ESI): [M+H]$^+$ = 247.07.

Step 2: 1,5-Dithiaspiro[5.5]undecan-9-one (Compound 8c)

Compound 8b (2.0 g, 8.0 mmol) was dissolved in dichloromethane (200 mL), and trifluoroacetic acid (100 mL) was added at room temperature. The mixture was stirred for 4 h. The resulting mixture was adjusted with sodium bicarbonate solution to pH 7, extracted, dried, and concentrated. The crude product was purified by column chromatography to afford compound 8c (1.5 g, 91.0%).

$^1$H NMR (400 MHz, DMSO-$d_6$) δ 2.93-2.84 (m, 4H), 2.41-2.33 (m, 4H), 2.31-2.25 (m, 4H), 1.94-1.88 (m, 2H). LC-MS(ESI): [M+H]$^+$ = 203.05.

Step 3: Dimethyl 4-oxadispiro[chroman-2,1'-cyclohexane-4',2"-[1,3]dithiane]-6,7-dicarboxylate (Compound 8d)

Compound 8c (2.0 g, 9.9 mmol) was dissolved in tetrahydrofuran (200 mL), and compound 4 (2.0 g, 9.8 mmol) and pyrrolidine (2.0 g, 28.0 mmol) were added. The mixture was stirred at 70°C for 4 h. The solvent was removed under reduced pressure, and the residue was purified by column chromatography to afford compound 8d (2.0 g, 46.0%).

$^1$H NMR (400 MHz, DMSO-$d_6$) δ 8.18 (s, 1H), 7.35 (s, 1H), 3.83 (s, 3H), 3.81 (s, 3H), 2.92 (s, 2H), 2.90-2.85 (m, 2H), 2.81-2.74 (m, 2H), 2.14-2.10 (m, 2H), 2.02-1.77 (m, 8H). LC-MS(ESI): [M+H]$^+$ = 437.10.

Step 4: Dimethyl 4-hydroxydispiro[chroman-2,1'-cyclohexane-4',2"-[1,3]dithiane]-6,7-dicarboxylate (Compound 8e)

Compound 8d (2.0 g, 4.5 mmol) was dissolved in methanol (50 mL) and tetrahydrofuran (50 mL), and sodium borohydride (350.0 mg, 9.0 mmol) was added at room temperature. The mixture was stirred at 70°C for 4 h. The solvent was removed under reduced pressure, and the residue was purified by column chromatography to afford compound 8e (1.5 g, 75.0%).

$^1$H NMR (600 MHz, DMSO-$d_6$) δ 7.90 (s, 1H), 7.00 (s, 1H), 5.68 (d, *J*= 6.2 Hz, 1H), 4.77-4.71 (m, 1H), 3.79 (s, 6H), 2.96-2.72 (m, 4H), 2.16-2.01 (m, 4H), 1.93-1.79 (m, 5H), 1.77-1.70 (m, 3H). LC-MS(ESI): [M+H]$^+$ = 439.12.

Step 5: Dimethyl dispiro[chromene-2,1'-cyclohexane-4',2"-[1,3]dithiane]-6,7-dicarboxylate (Compound 8f)

Compound 8e (700 mg, 1.5 mmol) was dissolved in dichloromethane (200 mL), and triethylamine (480.0 mg, 4.5

mmol) and 4-dimethylaminopyridine (100.0 mg, 0.8 mmol) were added. Sulfonyl chloride (720.0 mg, 6.0 mmol) was added dropwise at 0°C. The mixture was stirred at 0°C for 4 h, monitored by TLC. The solvent was removed under reduced pressure, and the residue was dissolved in toluene (100 mL). 1,8-Diazabicyclo[5.4.0]undec-7-ene (1.6 g, 10.0 mmol) was added, and the mixture was stirred at 110°C for 16 h. The solvent was removed under reduced pressure, and the residue was purified by column chromatography to afford compound 8f (400 mg, 59.0%).

$^1$H NMR (400 MHz, DMSO-$d_6$) δ 7.58 (s, 1H), 7.05 (s, 1H), 6.59 (d, *J* = 9.9 Hz, 1H), 5.95 (d, *J* = 11.4 Hz, 1H), 3.78 (s, 3H), 3.77 (s, 3H), 2.93 - 2.85 (m, 2H), 2.82 - 2.73 (m, 2H), 2.18-2.11 (m, 2H), 2.09 - 1.96 (m, 2H), 1.92 - 1.77 (m, 6H). LC-MS(ESI): $[M+H]^+$ = 421.11.

Step 6: Dispiro[chromene-2,1'-cyclohexane-4',2"-[1,3]dithiane]-6,7-dicarboxylic acid (Compound 8g)

Compound 8f (400.0 mg, 0.9 mmol) was dissolved in methanol (50 mL), tetrahydrofuran (50 mL), and water (50 mL). Lithium hydroxide (120.0 mg, 4.5 mmol) was added at room temperature. The mixture was stirred for 16 h. The resulting mixture was acidified to pH 6 with 1M dilute hydrochloric acid, extracted, the organic phase was dried, then concentrated. The crude product was purified by column chromatography to afford compound 8g (350 mg, 94.0%).

$^1$H NMR (600 MHz, DMSO-$d_6$) δ 12.90 (s, 2H), 7.52 (s, 1H), 6.99 (s, 1H), 6.57 (d, *J* = 9.9 Hz, 1H), 5.90 (d, *J* = 9.9 Hz, 1H), 2.92 - 2.86 (m, 2H), 2.83 - 2.73 (m, 2H), 2.11 (m, 2H), 2.09 - 1.97 (m, 2H), 1.94 - 1.76 (m, 6H). LC-MS(ESI): $[M+H]^+$ = 393.08.

Step 7: 7-(2,6-Dioxopiperidin-3-yl)-6H-dispiro[pyrano[2,3-f]isoindole-2,1'-cyclohexane-4',2"-[1,3]dithiane]-6,8(7H)-dione (Compound 8h)

Compound 8g (300.0 mg, 0.7 mmol) was dissolved in acetic acid (100 mL), and 3-aminopiperidine-2,6-dione hydrochloride (170.0 mg, 1.3 mmol) and sodium acetate (360.0 mg, 2.1 mmol) were added. The mixture was stirred at 110°C for 4 h. The solvent was removed under reduced pressure, and the residue was purified by column chromatography to afford compound 8h (280 mg, 75.0%).

$^1$H NMR (400 MHz, DMSO-$d_6$) δ 11.12 (s, 1H), 7.72 (s, 1H), 7.30 (s, 1H), 6.70 (d, *J*= 8.0Hz, 1H), 6.03 (d, *J*= 8.8 Hz, 1H), 5.13-5.07 (m, 1H), 2.94 - 2.84 (m, 3H), 2.84 - 2.76 (m, 2H), 2.64-2.55 (m, 1H), 2.18 - 1.98 (m, 6H), 1.95 - 1.78 (m, 6H). LC-MS(ESI): $[M+H]^+$ = 485.11.

Step 8: 7'-(2,6-Dioxopiperidin-3-yl)-6'H-spiro[cyclohexane-1,2'-pyrano[2,3-f]isoindole]-4,6',8'(7'H)-trione (Compound 8i)

Compound 8h (280.0 mg, 0.5 mmol) was dissolved in acetonitrile (50 mL), and sodium bicarbonate solution (50 mL) and iodine (1.5 g, 5.0 mmol) were added. The mixture was stirred at room temperature for 4 h. The solvent was removed under reduced pressure, and the residue was extracted, the organic phase was dried, then concentrated. The crude product was purified by column chromatography to afford compound 8i (100 mg, 44.0%).

$^1$H NMR (600 MHz, DMSO-$d_6$) δ 11.13 (s, 1H), 7.77 (s, 1H), 7.45 (s, 1H), 6.76 (d, *J*= 10.0 Hz, 1H), 6.09 (d, *J* = 9.9 Hz, 1H), 5.13-5.09 (m,1H), 2.92-2.84 (m, 1H), 2.78-2.70 (m, 2H), 2.63-2.57 (m, 1H), 2.26-2.15(m, 4H), 2.12 - 2.00 (m, 4H). LC-MS(ESI): $[M+H]^+$ = 395.12.

Step 9: 7'-(2,6-Dioxopiperidin-3-yl)-3',4'-dihydro-6'H-spiro[cyclohexane-1,2'-pyrano[2,3-f]isoindole]-4,6',8'(7'H)-trione (E3 Ligase Inhibitor 8)

Compound 8i (280.0 mg, 0.7 mmol) was dissolved in tetrahydrofuran (50 mL), and palladium on carbon (150.0 mg) was added. The mixture was stirred under hydrogen at 50°C for 4 h. The resulting mixture was filtered through diatomaceous earth, and the filtrate was concentrated and purified by preparative HPLC to afford E3 ligase inhibitor 8 (195 mg, 70.0%).

$^1$H NMR (600 MHz, DMSO-$d_6$) δ 11.11 (s, 1H), 7.68 (s, 1H), 7.35 (s, 1H), 5.12-5.06 (m, 1H), 2.99-2.93 (m, 2H), 2.92-2.84 (m, 1H), 2.68-2.55 (m, 3H), 2.23-2.15 (m, 2H), 2.13-2.00 (m, 4H), 1.99-1.91 (m, 4H). LC-MS(ESI): $[M+H]^+$ = 397.13

9) Synthesis of E3 Ligase Inhibitor 9

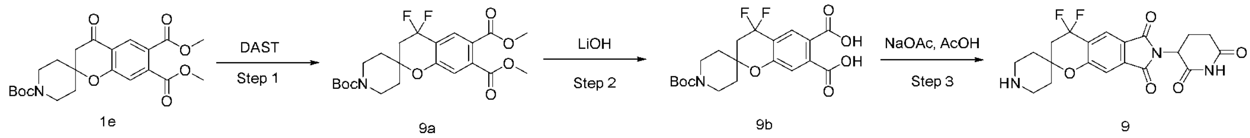


1e 9a 9b 9

Step 1: 1'-(tert-Butyl) 6,7-dimethyl 4,4-difluorospiro[chroman-2,4'-piperidine]-1',6,7-tricarboxylate (Compound 9a)

The diethylaminosulfur trifluoride (4 mL) was added to compound 1e (2 g, 4.6 mmol). The mixture was stirred at 85°C for 2 h. The mixture was poured into ice water, extracted with ethyl acetate three times, and the organic phase was concentrated under vacuum. The crude product was purified by reversed-phase column chromatography to afford a yellow oily compound 9a (0.5 g, 24%).

$^1H$ NMR (600 MHz, DMSO-$d_6$) δ 8.02 (s, 1H), 7.29 (s, 1H), 3.82 (s, 3H), 3.81 (s, 3H), 3.77-3.68 (m, 2H), 3.22-3.02 (m, 2H), 2.72 (t, *J*= 14.7 Hz, 2H), 1.85 (d, *J*= 13.9 Hz, 2H), 1.70 (td, *J*= 14.0, 11.6, 4.7 Hz, 2H), 1.40 (s, 9H). LC-MS(ESI): $[M-tBu+H]^+$ = 400.31.

Step 2: 1'-(tert-Butyl)-4,4-difluorospiro[chroman-2,4'-piperidine]-6,7-dicarboxylic acid (Compound 9b)

The lithium hydroxide (42 mg, 1.8 mmol) was added to a solution of compound 9a (160 mg, 0.4 mmol) in methanol/tetrahydrofuran (2 mL). The mixture was stirred at room temperature overnight. The mixture was extracted with dichloromethane/methanol (10:1), and the organic phase was concentrated to afford a white solid compound 9b (0.1 g, 67%).

$^1H$ NMR (600 MHz, DMSO-$d_6$) δ 13.27 (br s, 2H), 8.12 (s, 1H), 7.36 (s, 1H), 3.21-3.06 (m, 2H), 2.68 (t, *J* = 14.7 Hz, 2H), 2.05-1.93 (m, 2H), 1.84 (d, *J* = 13.8 Hz, 2H), 1.68 (td, *J* = 14.0, 12.9, 4.7 Hz, 2H), 1.40 (s, 9H). LC-MS(ESI): $[M-tBu+H]^+$ = 372.27.

Step 3: 7'-(2,6-Dioxopiperidin-3-yl)-4',4'-difluoro-3',4'-dihydro-6'H-spiro[piperidine-4,2'-pyrano[2,3-f]isoindole]-6',8'(7'H)-dione (E3 Ligase Inhibitor 9)

Compound 9b (100 mg, 0.23 mmol) was dissolved in acetic acid (1 mL), and 3-aminopiperidine-2,6-dione hydrochloride (77 mg, 0.5 mmol) and sodium acetate (96 mg, 1.2 mmol) were added. The mixture was stirred at 110°C for 2 h. After completion, the mixture was purified by reversed-phase column chromatography to afford a brown solid E3 ligase inhibitor 9 (50 mg, 51%).

$^1H$ NMR (600 MHz, DMSO-$d_6$) δ 11.15 (s, 1H), 8.10 (s, 1H), 7.68 (s, 1H), 5.16 (dd, *J*= 13.0, 5.4 Hz, 1H), 3.27-3.15 (m, 4H), 2.92-2.82 (m, 3H), 2.61 (dt, *J* = 17.2, 3.4 Hz, 1H), 2.56-2.51 (m, 1H), 2.11 (d, *J* = 14.4 Hz, 2H), 2.05 (ddt, *J*= 12.9, 5.6, 2.5 Hz, 1H), 1.97-1.90 (m, 2H). LC-MS(ESI): $[M+H]^+$ = 420.36.

10) Synthesis of E3 Ligase Inhibitor 10

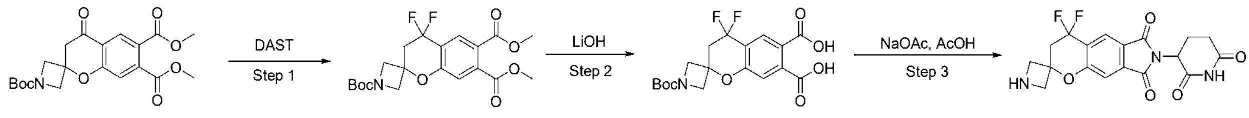


10a 10b 10c 10

Step 1: 1-(tert-Butyl) 6',7'-dimethyl 4',4'-difluorospiro[azetidine-3,2'-chroman]-1,6',7'-tricarboxylate (Compound 10b)

The diethylaminosulfur trifluoride (5 mL) was added to compound 10a (1 g, 2.5 mmol). The mixture was stirred at 50°C overnight. The mixture was poured into ice water, extracted with ethyl acetate, dried over anhydrous sodium sulfate, filtered, and concentrated. The crude product was purified by column chromatography (PE: EA = 0-40%) to afford a pale yellow solid compound 10b (500 mg, 47%).

$^1H$ NMR (400 MHz, DMSO-$d_6$) δ 8.01 (s, 1H), 7.34 (s, 1H), 4.13 (d, *J*= 9.7 Hz, 2H), 3.91 (d, *J*= 9.7 Hz, 2H), 3.83 (s, 3H), 3.82 (s, 3H), 3.04 (t, *J*= 13.4 Hz, 2H), 1.39 (s, 9H). LC-MS(ESI): $[M-Boc+H]^+$ = 328.30.

Step 2: 1-(tert-Butoxycarbonyl)-4',4'-difluorospiro[azetidine-3,2'-chroman]-6',7'-dicarboxylic acid (Compound 10c)

Compound 10b (500 mg, 1.2 mmol) was dissolved in methanol (5 mL), and lithium hydroxide (140.1 mg, 5.9 mmol) in water was slowly added at room temperature. The mixture was stirred for 2 h. The resulting mixture was neutralized with acetic acid to pH=7, concentrated, and extracted with ethyl acetate to afford a white solid crude product, which was used directly in the next step.

LC-MS(ESI): $[M+H]^+$ = 399.28.

Step 3: 7'-(2,6-Dioxopiperidin-3-yl)-4',4'-difluoro-3',4'-dihydro-6'H-spiro[azetidine-3,2'-pyrano[2,3-f]isoindole]-6',8'(7'H)-dione (E3 Ligase Inhibitor 10)

Under nitrogen atmosphere, compound 10c (100 mg, 0.3 mmol) was dissolved in acetic acid (1 mL), and sodium acetate (61.6 mg, 0.8 mmol) and 3-aminopiperidine-2,6-dione hydrochloride (64.5 mg, 0.5 mmol) were added. The mixture was heated to 110°C and stirred for 3 h. After completion, the resulting mixture was purified by C18 reversed-phase column chromatography to afford a white solid E3 ligase inhibitor 10 (3.6 mg, 4%).

$^1$H NMR (600 MHz, DMSO-$d_6$) δ 11.15 (s, 1H), 8.13 (s, 1H), 7.58 (s, 1H), 5.18 (dd, *J*= 13.0, 5.4 Hz, 1H), 4.33 (d, *J* = 11.7 Hz, 2H), 4.22 (d, *J*= 11.6 Hz, 2H), 3.15 (t, *J*= 13.6 Hz, 2H), 2.94 - 2.87 (m, 1H), 2.65 - 2.59 (m, 1H), 2.54 - 2.51 (m, 1H), 2.10 - 2.05 (m, 1H). LC-MS(ESI): $[M+H]^+$ = 392.30.

11) Synthesis of E3 Ligase Inhibitor 11

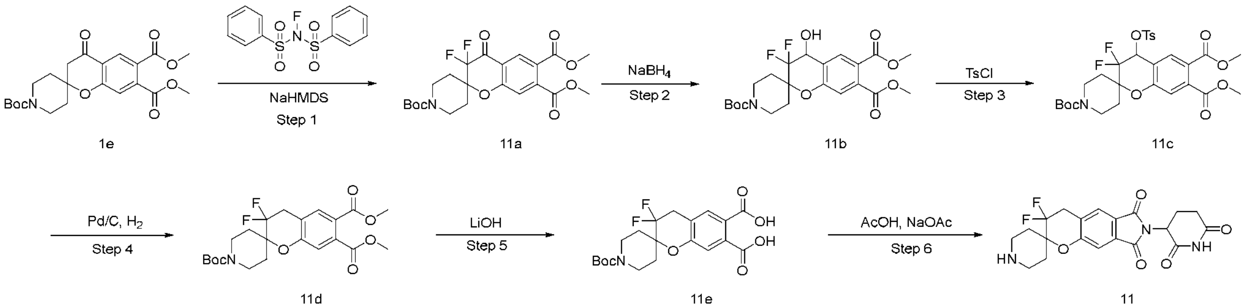


1e 11a 11b 11c

11d 11e 11

Step 1: 1'-(tert-Butyl) 6,7-dimethyl 3,3-difluoro-4-oxospiro[chromane-2,4'-piperidine]-1',6,7-tricarboxylate (Compound 11a)

Compound 1e (5 g, 11.5 mmol) was dissolved in anhydrous tetrahydrofuran (10 mL). The solution was cooled to -78°C, and sodium bis(trimethylsilyl)amide (4.23 g, 23.07 mmol) was slowly added. The mixture was maintained at -78°C for 2 h, followed by dropwise addition of N-fluorobenzenesulfonimide (7.3 g, 23.1 mmol). The mixture was stirred overnight at -78°C. The reaction was quenched by pouring into saturated ammonium chloride solution. The mixture was extracted with ethyl acetate, dried over anhydrous sodium sulfate, filtered, and concentrated. The crude product was purified by column chromatography (PE: EA = 0-40%) to afford a pale yellow solid compound 11a (2.5 g, 46%).

$^1$H NMR (400 MHz, DMSO-$d_6$) δ 8.29 (s, 1H), 7.62 (s, 1H), 3.94 (d, J = 11.6 Hz, 2H), 3.85 (s, 3H), 3.86 (s, 3H), 3.08 (s, 2H), 2.07 (d, J = 13.4 Hz, 2H), 1.74 (td, J = 13.5, 4.7 Hz, 2H), 1.41 (s, 9H). LC-MS(ESI): $[M+H]^+$ = 470.31.

Step 2: 1'-(tert-Butyl) 6,7-dimethyl 3,3-difluoro-4-hydroxyspiro[chromane-2,4'-piperidine]-1',6,7-tricarboxylate (Compound 11b)

Compound 11a (2.5 g, 5.3 mmol) was dissolved in ethanol (5 mL), and sodium borohydride (403 mg, 10.7 mmol) was added slowly at 0°C. The mixture was stirred at room temperature for 1 h. The reaction was quenched with acetone. The mixture was concentrated under vacuum, and purified by column chromatography (PE:EA = 0-40%) to afford a white solid compound 11b (2.4 g, 95%).

$^1$H NMR (400 MHz, DMSO-$d_6$) δ 7.90 (s, 1H), 7.23 (s, 1H), 6.68 (d, *J*= 6.1 Hz, 1H), 5.03 (dt, *J*= 16.1, 7.0 Hz, 1H), 3.97 (d, *J*= 14.1 Hz, 1H), 3.91 - 3.75 (m, 7H), 3.04 (d, *J*= 55.0 Hz, 2H), 1.99 (d, *J*= 13.3 Hz, 1H), 1.84 - 1.74 (m, 2H), 1.70 - 1.56 (m, 1H), 1.41 (s, 9H). LC-MS(ESI): [M-Boc+H]$^+$ = 372.34.

Step 3: 1'-(tert-Butyl) 6,7-dimethyl 3,3-difluoro-4-(tosyloxy)spiro[chromane-2,4'-piperidine]-1',6,7-tricarboxylate (Compound 11c)

Compound 11b (1.5 g, 3.2 mmol) was dissolved in dichloromethane (30 mL), and p-toluenesulfonyl chloride (727 mg, 3.8 mmol) and triethylamine (642 mg, 6.4 mmol) were added under ice bath conditions. The mixture was stirred at room temperature for 2 h. The mixture was concentrated under vacuum, and the crude product was purified by column chromatography (PE: EA = 0-40%) to afford a white solid compound 11c (1.0 g, 50%).

$^1$H NMR (600 MHz, DMSO-$d_6$) δ 7.98 (d, *J*= 8.2 Hz, 2H), 7.56 (d, *J*= 8.1 Hz, 2H), 7.41 (s, 1H), 7.35 (s, 1H), 6.25 (dd, *J*= 12.9, 8.1 Hz, 1H), 3.90 (dd, *J*= 21.4, 8.9 Hz, 2H), 3.81 (s, 3H), 3.79 (s, 3H), 3.06 (s, 2H), 2.48 (s, 3H), 1.99 - 1.91 (m, 2H), 1.67 (dtd, *J* = 39.4, 13.3, 4.7 Hz, 2H), 1.41 (s, 9H). LC-MS(ESI): $[M\text{-}Boc+H]^+$ = 526.34.

Step 4: 1'-(tert-Butyl) 6,7-dimethyl 3,3-difluorospiro[chromane-2,4'-piperidine]-1',6,7-tricarboxylate (Compound 11d)

Compound 11c (1.0 g, 1.6 mmol) was dissolved in methanol (20 mL), and palladium on carbon (100 mg) was added. The mixture was stirred under hydrogen at room temperature for 12 h. The resulting mixture was filtered, washed with methanol, and concentrated under vacuum to afford a white solid compound 11d (700 mg, 96%).

$^1$H NMR (600 MHz, DMSO-$d_6$) δ 7.70 (s, 1H), 7.28 (s, 1H), 3.95 - 3.88 (m, 2H), 3.86 - 3.74 (m, 8H), 3.51 (t, *J*= 15.8 Hz, 2H), 1.81 (d, *J* = 13.2 Hz, 2H), 1.69 (td, *J* = 13.6, 4.9 Hz, 2H), 1.42 (s, 9H). LC-MS(ESI): $[M\text{-}tBu+H]^+$ = 400.44.

Step 5: 1'-(tert-Butoxycarbonyl)-3,3-difluorospiro[chromane-2,4'-piperidine]-6,7-dicarboxylic acid (Compound 11e)

Compound 11d (700 mg, 1.5 mmol) was dissolved in a mixture of methanol and water (10 mL), and lithium hydroxide (184 mg, 7.7 mmol) was added. The mixture was stirred at room temperature for 2 h. The mixture was neutralized with glacial acetic acid to pH 7, concentrated under vacuum, and extracted with ethyl acetate to afford a white solid crude product, which was used directly in the next step without further purification.

LC-MS(ESI): $[M\text{-}Boc+H]^+$ = 328.32.

Step 6: 7'-(2,6-Dioxopiperidin-3-yl)-3',3'-difluoro-3',4'-dihydro-6'H-spiro[piperidine-4,2'-pyrano[2,3-f]isoindole]-6',8'(7'H)-dione (E3 Ligase Inhibitor 11)

Under nitrogen atmosphere, compound 11e (600 mg, 1.4 mmol) was dissolved in acetic acid (10 mL), and sodium acetate (346 mg, 4.2 mmol) and 3-aminopiperidine-2,6-dione hydrochloride (360 mg, 2.8 mmol) were added. The mixture was heated to 110°C and stirred for 3 h. After completion, the resulting mixture was purified by C18 reversed-phase column chromatography to afford a white solid E3 ligase inhibitor 11 (70 mg, 12%).

$^1$H NMR (400 MHz, $CD_3OD$) δ 7.78 (s, 1H), 7.55 (s, 1H), 5.13 (dd, *J*= 12.6, 5.4 Hz, 1H), 3.60 (t, *J* = 15.5 Hz, 2H), 3.50 - 3.34 (m, 4H), 2.93 - 2.82 (m, 1H), 2.80 - 2.66 (m, 2H), 2.24 - 2.09 (m, 5H). LC-MS(ESI): $[M+H]^+$ = 420.41.

12) Synthesis of E3 Ligase Inhibitor 12

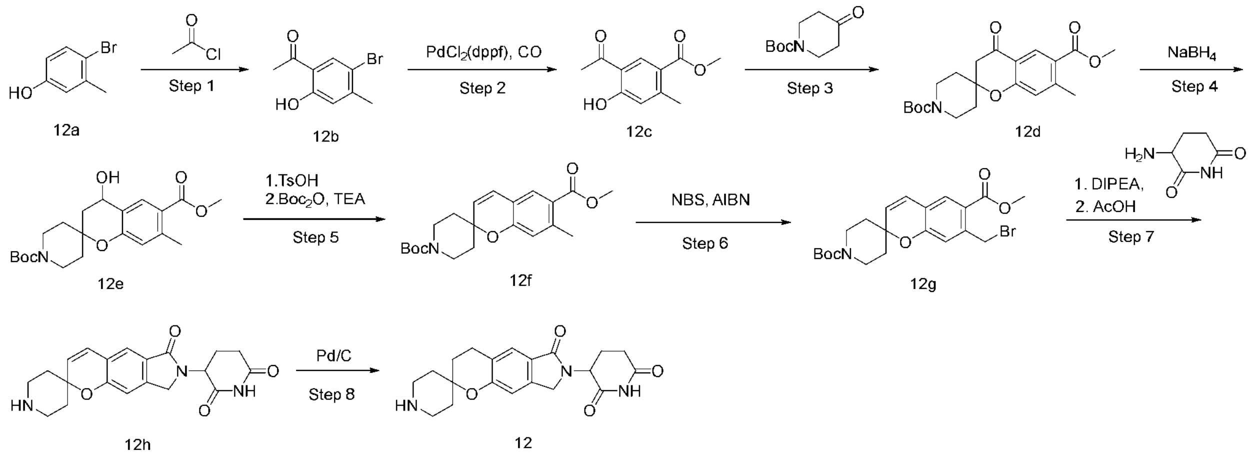

Step 1: 1-(5-Bromo-2-hydroxy-4-methylphenyl)ethan-1-one (Compound 12b)

A mixture of 4-bromo-3-methylphenol 12a (5.0 g, 26.7 mmol) and acetyl chloride (10.5 g, 134 mmol) was stirred at 60°C for 1 h. After cooling to room temperature, aluminum chloride (5.4 g, 40.1 mmol) was added, and the mixture was stirred at 160°C for 2 h. The reaction was quenched by pouring into saturated ammonium chloride solution (50 mL) and the mixture was extracted with ethyl acetate (50 mL × 3). The combined organic layers were dried over anhydrous sodium sulfate, filtered, and concentrated to afford a grayish-white solid crude compound 12b (5.5 g, 90%).
$^1$H NMR (600 MHz, DMSO-$d_6$) δ 11.81 (s, 1H), 8.01 (s, 1H), 7.00 (s, 1H), 2.62 (s, 3H), 2.34 (s, 3H).

Step 2: Methyl 5-acetyl-4-hydroxy-2 methylbenzoate (Compound 12c)

The triethylamine (9.72 g, 96.04 mmol) and Pd (dppf)$Cl_2$ (1.76 g, 2.40 mmol) were added to a solution of compound 12b (5.5 g, 24.01 mmol) in methanol (50 mL). The mixture was purged with CO three times and then stirred at 60°C overnight. The resulting mixture was filtered, and the filtrate was concentrated under reduced pressure. The residue was purified by column chromatography (PE:EA = 6:1) to afford a white solid compound 12c (3.26 g, 65%).
$^1$H NMR (600 MHz, DMSO-$d_6$) δ 12.14 (s, 1H), 8.34 (s, 1H), 6.90 (s, 1H), 3.82 (s, 3H), 2.64 (s, 3H), 2.53 (s, 3H).

Step 3: 1'-(tert-Butyl) 6-methyl 7-methyl-4-oxaspiro[chromane-2,4'-piperidine]-1',6-dicarboxylate (Compound 12d)

N-tert-butoxycarbonyl-3-azetidinone (2.8 g, 16.38 mmol) and pyrrolidine (1.59 g, 22.33 mmol) were added to a solution of 12c (3.1 g, 14.89 mmol) in ethanol (120 mL). The mixture was stirred at 80°C overnight. After completion, the mixture was concentrated under vacuum and purified by column chromatography (PE:EA = 0-20%) to afford a yellow solid compound 12d (1.9 g, 35%).
$^1$H NMR (600 MHz, DMSO-$d_6$) δ 8.25 (s, 1H), 7.08 (s, 1H), 3.82 (s, 3H), 3.72 (s, 2H), 3.14 (s, 2H), 2.89 (s, 2H), 2.55 (s, 3H), 1.91-1.84 (m, 2H), 1.69-1.61 (m, 2H), 1.40 (s, 9H). LC-MS(ESI): $[M-Boc+H]^+$ =290.27.

Step 4: 1'-(tert-Butyl) 6-methyl 4-hydroxy-7-methylspiro[chromane-2,4'-piperidine]-1',6-dicarboxylate (Compound 12e)

The sodium borohydride (504 mg, 13.28 mmol) was added to a solution of 12d (1.6 g, 4.43 mmol) in methanol (40 mL). The mixture was stirred at room temperature for 4 h. After completion, the mixture was concentrated under vacuum and purified by column chromatography (PE: EA = 0-40%) to afford a yellow solid compound 12e (1.5 g, 93.23%).
$^1$H NMR (600 MHz, DMSO-$d_6$) δ 8.01 (s, 1H), 6.73 (s, 1H), 5.52 (d, J = 4.7 Hz, 1H), 4.68 (s, 1H), 3.78 (s, 3H), 3.68 (dd, J = 22.7, 9.0 Hz, 2H), 3.13 (d, J = 77.0 Hz, 2H), 2.46 (s, 3H), 2.13 (dd, J = 13.5, 6.0 Hz, 1H), 1.82-1.62 (m, 4H), 1.59 - 1.50 (m, 1H), 1.41 (s, 9H). LC-MS(ESI): $[M-Boc+H]^+$ =292.30.

Step 5: 1'-(tert-Butyl) 6-methyl 7-methylspiro[chromene-2,4'-piperidine]-1',6-dicarboxylate (Compound 12f)

The p-toluenesulfonic acid (712 mg, 4.13 mmol) was added to a solution of 12e (1.5 g, 4.13 mmol) in toluene (40 mL). The mixture was stirred at 110°C overnight. The mixture was concentrated under vacuum, dissolved in tetrahydrofuran (40 mL), and treated with triethylamine (2.23 g, 22.02 mmol) and di-tert-butyl dicarbonate (2.4 g, 11.01 mmol). The resulting mixture was stirred at room temperature overnight. After concentration under vacuum, the crude product was purified by column chromatography (PE:EA = 0-30%) to afford a yellow solid compound 12f (1.1 g, 77%).
$^1$H NMR (600 MHz, DMSO-$d_6$) δ 7.64 (s, 1H), 6.80 (s, 1H), 6.55 (d, *J*= 9.9 Hz, 1H), 5.80 (d, *J*= 9.8 Hz, 1H), 3.78 (s, 3H), 3.70 (d, *J*= 13.0 Hz, 2H), 3.30 - 3.11 (m, 2H), 2.47 (s, 3H), 1.84-1.78 (m, 2H), 1.68-1.60 (m, 2H), 1.41 (s, 9H). LC-MS(ESI): $[M-Boc+H]^+$ =274.26.

Step 6: 1'-(tert-Butyl) 6-methyl 7-(bromomethyl)spiro[chromene-2,4'-piperidine]-1',6-dicarboxylate (Compound 12g)

N-bromosuccinimide (620 mg, 3.47 mmol) and azobisisobutyronitrile (48 mg, 290 μmol) were added to a solution of compound 12f (1 g, 2.90 mmol) in carbon tetrachloride (10 mL). The reaction was purged with nitrogen and the mixture was stirred under at 80°C overnight. The resulting mixture was concentrated under vacuum, and the crude product was used directly in the next step.

Step 7: 3-(6'-Oxa-6',8'-dihydro-7'H-spiro[piperidine-4,2'-pyrano[2,3-f]isoindol]-7'-yl)piperidine-2,6-dione (Compound 12h)

The crude product from the previous step was dissolved in acetonitrile (40 mL), and diisopropylethylamine (1.12 g, 8.70 mmol) and 3-amino-2,6-piperidinedione (446 mg, 3.48 mmol) were added. The mixture was stirred at 80°C

overnight. After concentration, acetic acid (10 mL) was added, and the reaction was continued for 2 h. The resulting mixture was concentrated and purified by column chromatography (PE:EA = 0-50%) to afford a black solid compound 12g (285 mg, 22%).

$^1$H NMR (600 MHz, $CD_3OD$) δ 7.52 (s, 1H), 7.13 (s, 1H), 6.69 (d, J = 9.9 Hz, 1H), 5.83 (d, J = 9.9 Hz, 1H), 5.13 (dd, J = 13.3, 5.1 Hz, 1H), 4.45 (dd, J = 35.2, 17.3 Hz, 2H), 3.48-3.40 (m, 2H), 3.38-3.33 (m, 2H), 2.91 (ddd, J = 18.2, 11.8, 5.0 Hz, 2H), 2.82-2.77 (m, 1H), 2.49 (ddd, J = 17.9, 12.7, 3.8 Hz, 1H), 2.29-2.22 (m, 2H), 2.17 (tdd, J = 13.4, 6.7, 4.2 Hz, 1H), 2.03 -1.95 (m, 2H). LC-MS(ESI): $[M+H]^+$ =368.38.

Step 8: 3-(6'-Oxa-3',4',6',8'-tetrahydro-7'H-spiro[piperidine-4,2'-pyrano[2,3-f]isoindol]-7'-yl)piperidine-2,6-dione (E3 Ligase Inhibitor 12)

Compound 12g was dissolved in methanol (10 mL), and palladium on carbon (150 mg) was added. The reaction was purged with hydrogen and the mixture was stirred at room temperature for 2 h. After the reaction was completed, the mixture was filtered, and the filtrate was concentrated under vacuum. Purification by preparative HPLC afforded a white solid E3 ligase inhibitor 12 (120 mg, 35% yield over two steps).

$^1$H NMR (600 MHz, DMSO-$d_6$) δ 10.97 (s, 1H), 7.51 (s, 1H), 7.05 (s, 1H), 5.07 (dd, *J*= 13.3, 5.1 Hz, 1H), 4.34 (d, *J*= 16.9 Hz, 1H), 4.22 (d, *J*= 16.9 Hz, 1H), 3.26-3.20 (m, 2H), 3.16-3.08 (m, 2H), 2.94 - 2.86 (m, 3H), 2.63-2.57 (m, 1H), 2.41-2.33 (m, 1H), 2.00 -1.87 (m, 5H), 1.85-1.77 (m, 2H). LC-MS(ESI): $[M+H]^+$ =370.39.

13) Synthesis of compound 13

Synthesis scheme:

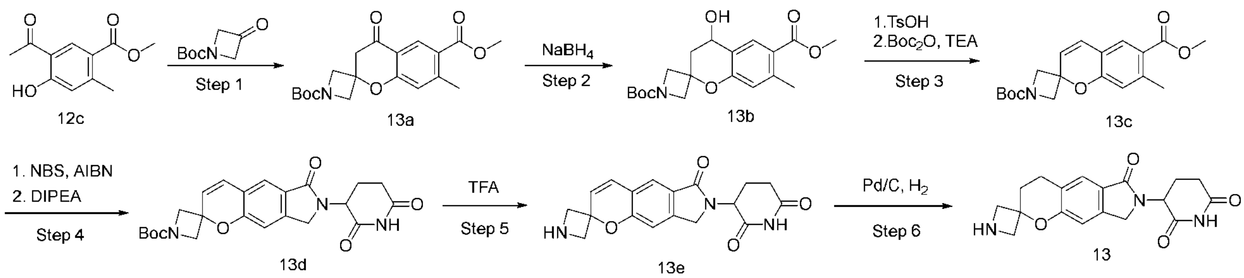


Step 1: 1-(tert-Butyl) 6'-methyl 7'-methyl-4'-oxaspiro[azetidine-3,2'-chromane]-1,6'-dicarboxylate (Compound 13a)

N-tert-butoxycarbonyl-3-azetidinone (2.8 g, 16.38 mmol) and pyrrolidine (1.59 g, 22.33 mmol) were added to a solution of 2-methyl-4-hydroxy-5-acetylbenzoate (3.1 g, 14.89 mmol) in ethanol (120 mL). The mixture was stirred at 80°C overnight. After completion, the mixture was concentrated under vacuum and purified by column chromatography (PE:EA = 0-20%) to afford a yellow solid compound 13a (1.9 g, 35%).

$^1$H NMR (600 MHz, DMSO-$d_6$) δ 8.24 (s, 1H), 7.15 (s, 1H), 4.00 (d, *J*= 8.7 Hz, 2H), 3.89 (d, *J*= 8.7 Hz, 2H), 3.82 (s, 3H), 3.20 (s, 2H), 2.56 (s, 3H), 1.38 (s, 9H). LC-MS(ESI): $[M-Boc+H]^+$ = 262.25.

Step 2: 1-(tert-Butyl) 6'-methyl 4'-hydroxy-7'-methylspiro[azetidine-3,2'-chromane]-1,6'-dicarboxylate (Compound 13b)

The sodium borohydride (504 mg, 13.28 mmol) was added to a solution of 13a (1.6 g, 4.43 mmol) in methanol (40 mL). The mixture was stirred at room temperature for 4 h. After completion, the mixture was concentrated under vacuum and purified by column chromatography (PE:EA = 0-40%) to afford a yellow solid compound 13b (1.5 g, 93.23%).

$^1$H NMR (600 MHz, $CD_3OD$) δ 8.02 (s, 1H), 6.81 (s, 1H), 4.82 (t, *J* = 5.2 Hz, 1H), 4.28 (d, *J* = 9.6 Hz, 1H), 4.04 (dd, *J*= 19.0, 9.1 Hz, 2H), 3.95 (d, *J*= 9.7 Hz, 1H), 3.87-3.84 (m, 3H), 2.54 (s, 3H), 2.32 (d, *J* = 5.2 Hz, 2H), 1.47 (s, 9H). LC-MS(ESI): $[M-Boc+H]^+$ = 264.27.

Step 3: 1-(tert-Butyl) 6'-methyl 7'-methylspiro[azetidine-3,2'-chromene]-1,6'-dicarboxylate (Compound 13c)

The p-toluenesulfonic acid (712 mg, 4.13 mmol) was added to a solution of 13b (1.5 g, 4.13 mmol) in toluene (40 mL). The mixture was stirred at 110°C overnight. The mixture was concentrated under vacuum, dissolved in tetrahydrofuran (40 mL), and treated with triethylamine (2.23 g, 22.02 mmol) and di-tert-butyl dicarbonate (2.4 g, 11.01 mmol).

The resulting mixture was stirred at room temperature overnight. After concentration under vacuum, the residue was purified by column chromatography (PE:EA = 0-30%) to afford a yellow solid compound 13c (1.1 g, 77%).

$^1$H NMR (600 MHz, DMSO-$d_6$) δ 7.67 (s, 1H), 6.84 (s, 1H), 6.65 (d, *J*= 9.9 Hz, 1H), 6.16 (d, *J*= 9.9 Hz, 1H), 4.04 (s, 4H), 3.78 (s, 3H), 2.47 (s, 3H), 1.40 (s, 9H). LC-MS(ESI): [M-Boc+H]$^+$ = 246.23.

Step 4: tert-Butyl 7'-(2,6-dioxopiperidin-3-yl)-6'-oxa-7',8'-dihydro-6'H-spiro[azetidine-3,2'-pyrano[2,3-flisoindole]-1-carboxylate (Compound 13d)

N-bromosuccinimide (620 mg, 3.47 mmol) and azobisisobutyronitrile (48 mg, 290 μmol) were added to a solution of compound 13c (1 g, 2.90 mmol) in carbon tetrachloride (10 mL). The reaction was purged with nitrogen and the mixture was stirred at 80°C overnight. The mixture was concentrated under vacuum, dissolved in acetonitrile (40 mL), and treated with diisopropylethylamine (1.12 g, 8.70 mmol) and 3-amino-2,6-piperidinedione (446 mg, 3.48 mmol). The resulting mixture was stirred at 80°C overnight. After concentration, the residue was purified by column chromatography (PE:EA = 0-50%) to afford a black solid compound 13d (285 mg, 22%).

$^1$H NMR (600 MHz, $CD_3OD$) δ 7.50 (s, 1H), 7.09 (s, 1H), 6.69 (d, *J* = 9.9 Hz, 1H), 6.17 (d, *J* = 9.8 Hz, 1H), 5.12 (dd, *J*= 13.2, 5.2 Hz, 1H), 4.50-4.40 (m, 2H), 4.24- 4.19 (m, 2H), 4.10 (d, *J* = 8.9 Hz, 2H), 2.94-2.86 (m, 1H), 2.82-2.77 (m, 1H), 2.49 (ddd, *J* = 26.5, 13.2, 4.5 Hz, 1H), 2.19-2.15 (m, 1H), 1.48 (s, 9H). LC-MS(ESI): [M+H]$^+$ = 440.38.

Step 5: 3-(6'-Oxa-6',8'-dihydro-7'H-spiro[azetidine-3,2'-pyrano[2,3-f]isoindol]-7'-yl)piperidine-2,6-dione (Compound 13e)

Compound 13d (285 mg, 648.5 μmol) was dissolved in dichloromethane (5 mL), and trifluoroacetic acid (636 mg, 6.49 mmol) was added. The mixture was stirred at room temperature for 2 h. After concentration, the crude product was used directly in the next step.

LC-MS(ESI): [M+H]$^+$ = 340.32.

Step 6: 3-(6'-Oxa-3',4',6',8'-tetrahydro-7'H-spiro[azetidine-3,2'-pyrano[2,3-f]isoindol]-7'-yl)piperidine-2,6-dione (E3 Ligase Inhibitor 13)

The mixture from the previous step was dissolved in methanol (10 mL), and palladium on carbon (10%, 150 mg) was added. The reaction was purged with hydrogen and the mixture was stirred at room temperature for 2 h. The resulting mixture was filtered, and the filtrate was concentrated under vacuum. Purification by preparative HPLC afforded a white solid E3 ligase inhibitor 13 (120 mg, 35% yield over two steps).

$^1$H NMR (400 MHz, $CD_3OD$) δ 7.57 (s, 1H), 7.10 (s, 1H), 5.11 (dd, *J* = 13.3, 5.1 Hz, 1H), 4.50-4.35 (m, 2H), 4.24 (s, 4H), 2.97 (t, *J* = 5.8 Hz, 2H), 2.91- 2.84 (m, 1H), 2.77 (ddd, *J* = 17.6, 4.5, 2.3 Hz, 1H), 2.46 (ddd, *J* = 26.4, 13.2, 4.7 Hz, 1H), 2.28 (t, *J*= 6.1 Hz, 2H), 2.14 (dtd, *J*= 12.7, 5.2, 2.3 Hz, 1H). LC-MS(ESI): [M+H]$^+$ = 342.32.

14) Synthesis of E3 Ligase Inhibitor 14

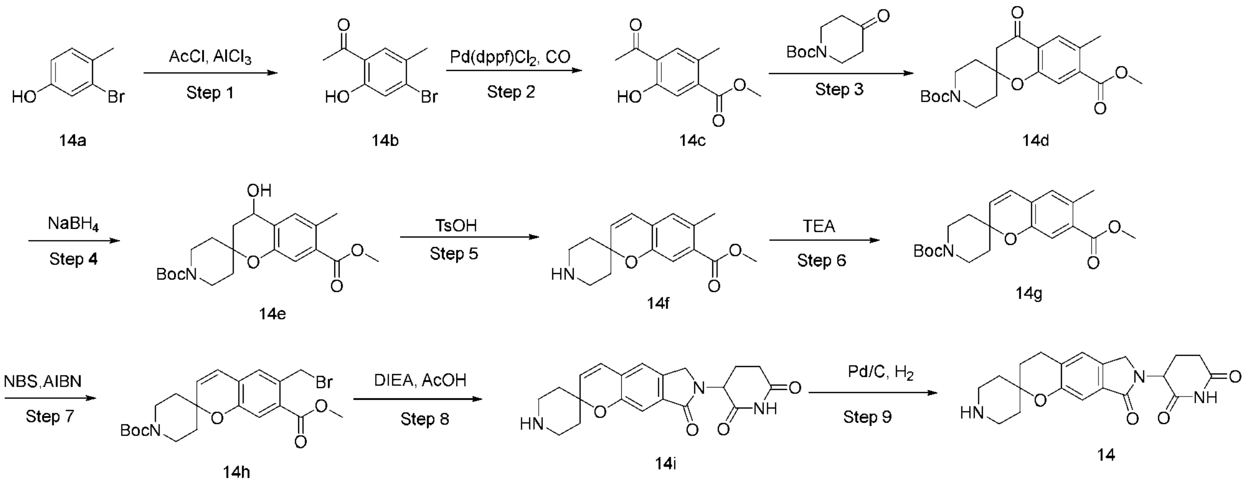


14a 14b 14c 14d 14e 14f 14g 14h 14i 14

Step 1: 1-(4-Bromo-2-hydroxy-5-methylphenyl)ethan-1-one (Compound 14b)

3-Bromo-4-methylphenol 14a (15.2 g, 81.27 mmol) was added to acetyl chloride (30 mL) and stirred at 60°C for 1 h. After cooling to room temperature, aluminum chloride (16.25 g, 121.90 mmol) was added slowly, and the mixture was stirred at 160°C for 3 h. The reaction was quenched by pouring into ice water. The mixture was filtered, and washed with saturated ammonium chloride solution. The crude product was concentrated under vacuum and purified by column chromatography (PE:EA = 0-50%) to afford a brown solid compound 14b (18.0 g, 96%).
$^1$H NMR (600 MHz, $CDCl_3$) δ 12.10 (s, 1H), 7.57 (s, 1H), 7.25 (s, 1H), 2.63 (s, 3H), 2.39 (s, 3H).

Step 2: Methyl 4-acetyl-5-hydroxy-2-methylbenzoate (Compound 14c)

The triethylamine (1.99 g, 19.6 mmol) and Pd(dppf)$Cl_2$ (1.42 g, 1.96 mmol) were added to a solution of compound 14b (3.0 g, 13.1 mmol) in methanol (60 mL). Under a carbon monoxide atmosphere. The mixture was stirred at 60°C overnight. The mixture was filtered, and the filtrate was concentrated under vacuum. The residue was purified by column chromatography (PE: EA = 0-50%) to afford a brown solid compound 14c (16.3 g, 51%).
$^1$H NMR (600 MHz, $CDCl_3$) δ 11.88 (s, 1H), 7.60 (s, 1H), 7.49 (s, 1H), 3.93 (s, 3H), 2.68 (s, 3H), 2.53 (s, 3H).

Step 3: 1'-(tert-Butyl) 7-methyl 6-methyl-4-oxaspiro[chromane-2,4'-piperidine]-1',7-dicarboxylate (Compound 14d)

A mixture of compound 14c (10.11 g, 48.56 mmol), N-tert-butoxycarbonyl-4-piperidone (9.67 g, 48.56 mmol), and pyrrolidine (3.45 g, 48.56 mmol) in methanol (50 mL) was refluxed at 80°C for 6 h. After completion, the mixture was extracted, filtered, and concentrated. The crude product was purified by column chromatography (PE: EA = 0-50%) to afford a pale yellow solid compound 14d (15.53 g, 82%).
$^1$H NMR (600 MHz, DMSO-$d_6$) δ 7.64 (s, 1H), 7.45 (s, 1H), 3.85 (s, 3H), 3.71 (s, 2H), 3.12 (d, *J* = 50.1 Hz, 2H), 2.88 (s, 2H), 2.43 (s, 3H), 1.90-1.84 (m, 2H), 1.62 (td, *J* = 12.7, 4.6 Hz, 2H), 1.40 (s, 9H). LC-MS(ESI): $[M\text{-}Boc+H]^+$ = 290.26.

Step 4: 1'-(tert-Butyl) 7-methyl 4-hydroxy-6-methylspiro[chromane-2,4'-piperidine]-1',7-dicarboxylate (Compound 14e)

The sodium borohydride (6.03 g, 159.51 mmol) was added to a solution of compound 14d (15.53 g, 39.88 mmol) in methanol (70 mL) under the ice bath condition. The mixture was stirred at room temperature for 2 h. After completion, the mixture was concentrated under vacuum and purified by column chromatography (PE: EA = 0-50%) to afford a yellow foam compound 14e (15.39 g, 98%).
$^1$H NMR (600 MHz, $CDCl_3$) δ 7.40 (s, 1H), 7.32 (s, 1H), 4.83 (t, *J* = 7.0 Hz, 1H), 3.90-3.84 (m, 5H), 3.31- 3.05 (m, 2H), 2.93 (s, 1H), 2.49 (s, 3H), 2.11 (dd, *J* = 13.6, 6.1 Hz, 1H), 1.92-1.85 (m, 2H), 1.78-1.72 (m, 1H), 1.63 (td, *J* = 13.3, 4.6 Hz, 1H), 1.52 (ddd, *J* = 24.7, 12.6, 7.9 Hz, 1H), 1.46 (s, 9H). LC-MS(ESI): $[M\text{-}Boc+H]^-$ = 292.27.

Step 5: Methyl 6-methylspiro[chromane-2,4'-piperidine]-7-dicarboxylate (Compound 14f)

Compound 14e (15.23 g, 38.91 mmol) and p-toluenesulfonic acid (6.7 g, 38.91 mmol) were dissolved in toluene and refluxed at 110°C overnight. The mixture was concentrated under vacuum to afford a pale yellow oily compound 14f (8.6 g, 80%). The crude product was used directly in the next step.
LC-MS(ESI): $[M\text{-}Boc+H]^+$ = 274.29.

Step 6: 1'-(tert-Butyl) 7-methyl 6-methylspiro[chromene-2,4'-piperidine]-1',7-dicarboxylate (Compound 14g)

Compound 14f (8.6 g, 31.46 mmol), di-tert-butyl dicarbonate (13.73 g, 62.93 mmol), and triethylamine (9.55 g, 94.39 mmol) were dissolved in dichloromethane (30 mL). The mixture was stirred at room temperature for 4 h. After concentration under vacuum, the crude product was purified by column chromatography (PE:EA = 0-30%) to afford a colorless solid compound 14g (1.8 g, 83%).
$^1$H NMR (600 MHz, $CDCl_3$) δ 7.41 (s, 1H), 6.85 (s, 1H), 6.37 (d, *J* = 9.8 Hz, 1H), 5.65 (d, *J* = 9.6 Hz, 1H), 3.98-3.77 (m, 5H), 3.28 (s, 2H), 2.50 (s, 3H), 1.97 (d, *J* = 13.4 Hz, 2H), 1.59 (td, *J* = 13.4, 4.7 Hz, 2H), 1.47 (s, 9H). LC-MS(ESI): $[M\text{-}Boc+H]^+$ = 274.24.

Step 7: 1'-(tert-Butyl) 7-methyl 6-(bromomethyl)spiro[chromene-2,4'-piperidine]-1',7-dicarboxylate (Compound 14h)

Compound 14g (5.11 g, 13.68 mmol), N-bromosuccinimide (2.73 g, 15.33 mmol), and azobisisobutyronitrile (0.112 g, 6.84 mmol) were dissolved in carbon tetrachloride (30 mL). The reaction was carried out under nitrogen and refluxed at 80°C overnight. The mixture was extracted and concentrated to afford a colorless oily compound 14h (1.32 g,

21%). The crude product was used directly in the next step.

Step 8: 3-(8'-Oxa-6',8'-dihydro-7'H-spiro[piperidine-4,2'-pyrano[2,3-f]isoindol]-7'-yl)piperidine-2,6-dione (Compound 14i)

Compound 14g (1.56 g, 3.45 mmol) was dissolved in acetonitrile (20 mL), and 3-aminopiperidine-2,6-dione hydrochloride (0.53 g, 4.14 mmol) and N,N-diisopropylethylamine (1.33 g, 10.35 mmol) were added. The mixture was stirred at 80°C overnight. The mixture was concentrated, acetic acid (1 mL) was added, and the reaction was refluxed at 110°C for 2 h. After completion, the mixture was purified by reversed-phase column chromatography to afford a brown solid compound 14i (0.96 g, 75%).

$^1$H NMR (600 MHz, DMSO-$d_6$) δ 11.00 (s, 1H), 7.36 (s, 1H), 7.25 (s, 1H), 6.68 (d, *J* = 9.8 Hz, 1H), 5.99 (d, *J* = 9.8 Hz, 1H), 5.08 (dd, *J* = 13.3, 5.2 Hz, 1H), 4.37 (d, *J* = 16.9 Hz, 1H), 4.25 (d, *J* = 16.9 Hz, 1H), 3.26 - 3.19 (m, 4H), 2.90 (ddd, *J* = 17.3, 13.7, 5.5 Hz, 1H), 2.64-2.57 (m, 1H), 2.39 (qd, *J* = 13.2, 4.5 Hz, 1H), 2.10 - 2.03 (m, 2H), 2.00 (dtd, *J* = 12.8, 5.4, 2.4 Hz, 1H), 1.94 - 1.86 (m, 2H). LC-MS(ESI): [M+H]$^+$ = 368.37.

Step 9: 3-(8'-Oxa-3'.4',6',8'-tetrahydro-7'H-spiro[piperidine-4,2'-pyrano[2,3-f]isoindol]-7'-yl)piperidine-2,6-dione (E3 Ligase Inhibitor 14)

Compound 14i (0.668 g, 1.82 mmol) was dissolved in methanol (15 mL), and palladium on carbon (0.2 g) was added. The reaction was stirred under a hydrogen atmosphere at room temperature overnight. The mixture was filtered and concentrated. Purification by reversed-phase column chromatography afforded a white solid E3 ligase inhibitor 14 (0.536 g, 79%).

$^1$H NMR (600 MHz, DMSO-$d_6$) δ 10.99 (s, 1H), 7.36 (s, 1H), 7.18 (s, 1H), 5.07 (dd, *J* = 13.3, 5.1 Hz, 1H), 4.34 (d, *J* = 16.6 Hz, 1H), 4.21 (d, *J* = 16.5 Hz, 1H), 3.25-3.11 (m, 4H), 2.94-2.87 (m, 3H), 2.63- 2.57 (m, 1H), 2.39 (qd, *J* = 13.2, 4.5 Hz, 1H), 2.01-1.95 (m, 1H), 1.93-1.87 (m, 4H), 1.82-1.74 (m, 2H). LC-MS(ESI): [M+H]$^+$ = 370.36.

15) Synthesis of E3 Ligase Inhibitor 15

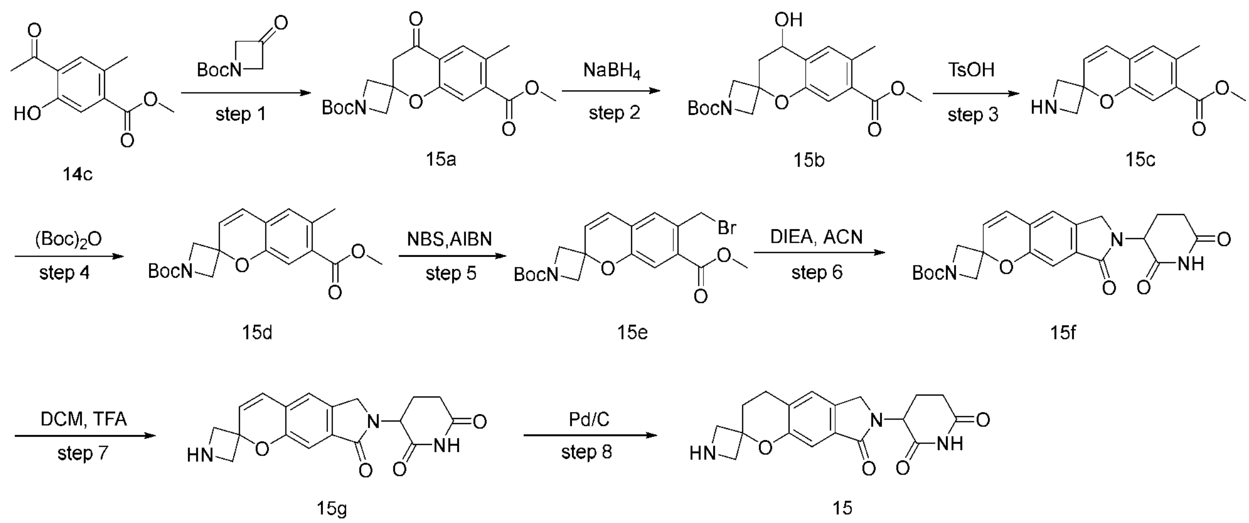


Step 1: 1-(tert-Butyl) 7'-methyl 6'-methyl-4'-oxaspiro[azetidine-3,2'-chromane]-1,7'-dicarboxylate (Compound 15a)

The 1-Boc-3-azetidinone (9.9 g, 57.6 mmol) and pyrrolidine (4.1 g, 57.6 mmol) were added to a solution of compound 14c (12 g, 57.6 mmol) in ethanol (200 mL). The mixture was stirred at 80°C for 4 h. Additional 1-Boc-3-azetidinone (9.9 g, 57.6 mmol) and pyrrolidine (4.1 g, 57.6 mmol) were added, and the mixture was stirred at 80°C for 16 h. The resulting mixture was concentrated under vacuum and purified by column chromatography (PE:EA = 0-40%) to afford a yellow solid compound 15a (8 g, 38%).

$^1$H NMR (600 MHz, $CDCl_3$) δ 7.74 (s, 1H), 7.59 (s, 1H), 4.09 (d, *J* = 9.6 Hz, 2H), 3.97 (d, *J* = 9.5 Hz, 2H), 3.94 (s, 3H), 3.07 (s, 2H), 2.53 (s, 3H), 1.46 (s, 9H). LC-MS(ESI): [M-Boc+H]$^+$ =262.28.

Step 2: 1-(tert-Butyl) 7'-methyl 4'-hydroxy-6'-methylspiro[azetidine-3,2'-chromane]-1,7'-dicarboxylate (Compound 15b)

The sodium borohydride (786 mg, 20.8 mmol) was slowly added to a solution of compound 15a (5 g, 13.8 mmol) in methanol (100 mL). The mixture was stirred at room temperature for 4 h. The resulting mixture was extracted with ethyl acetate three times, and the organic phase was dried over anhydrous sodium sulfate and concentrated to afford a yellow oily compound 15b (4.2 g, 83%).

$^1$H NMR (600 MHz, $CDCl_3$) δ 7.50 (s, 1H), 7.24 (s, 1H), 4.89 (q, *J* = 5.2 Hz, 1H), 4.36 (q, *J* = 7.2 Hz, 1H), 4.27 - 4.23 (m, 1H), 4.10 (d, *J* = 9.3 Hz, 1H), 4.00 (dd, *J* = 17.3, 9.6 Hz, 2H), 3.90 (s, 3H), 2.54 (s, 3H), 2.35 (d, *J* = 5.3 Hz, 2H), 1.47 (s, 9H). LC-MS(ESI): $[M-Boc+H]^+$ =264.27.

Step 3: Methyl 6'-methylspiro[azetidine-3,2'-chromene]-7'-carboxylate (Compound 15c)

The p-toluenesulfonic acid monohydrate (2.3 g, 12.1 mmol) was added to a solution of compound 15b (4 g, 13.8 mmol) in toluene (100 mL). The mixture was stirred at 110°C for 18 h. The resulting mixture was concentrated under vacuum to afford a yellow solid crude product, which was used directly in the next step without purification.

LC-MS(ESI): $[M+H]^+$ = 246.22.

Step 4: 1-(tert-Butyl) 7'-methyl 6'-methylspiro[azetidine-3,2'-chromene]-1,7'-dicarboxylate (Compound 15d)

The di-tert-butyl dicarbonate (2.1 g, 9.8 mmol) and triethylamine (3.2 mL) were added to a solution of compound 15c (2 g, 8.2 mmol) in dichloromethane (30 mL). The mixture was stirred at room temperature for 4 h. The resulting mixture was concentrated under vacuum and purified by column chromatography (PE: EA = 0-40%) to afford a colorless liquid compound 15d (2.5 g, 88%).

$^1$H NMR (600 MHz, $CDCl_3$) δ 7.45 (s, 1H), 6.88 (s, 1H), 6.47 (d, *J* = 9.8 Hz, 1H), 6.06 (d, *J* = 9.7 Hz, 1H), 4.24 (d, *J* = 9.5, 2H), 4.01 (d, *J* = 9.5, 2H), 3.89 (s, 3H), 2.51 (s, 3H), 1.48 (s, 9H). LC-MS(ESI): $[M-tBu+H]^+$ =290.20.

Step 5: 1-(tert-butyl) 7'-methyl 6'-(bromomethyl)spiro[azetidine-3,2'-chromene]-1,7'-dicarboxylate (Compound 15e)

N-bromosuccinimide (1.2 g, 6.4 mmol) and azobisisobutyronitrile (99 mg, 0.6 mmol) were added to a solution of compound 15d (2 g, 5.8 mmol) in carbon tetrachloride (30 mL). The mixture was stirred at 80°C for 12 h. The resulting mixture was concentrated under vacuum, and the crude product was used directly in the next step without purification.

Step 6: tert-Butyl 7'-(2,6-dioxopiperidin-3-yl)-8'-oxa-7',8'-dihydro-6'H-spiro[azetidine-3,2'-pyrano[2,3-flisoindole]-1-carboxylate (Compound 15f)

The 3-amino-2,6-piperidinedione hydrochloride (173 mg, 1.05 mmol) and N,N-diisopropylethylamine (0.4 mL) were added to a solution of compound 15e (2 g, 0.7 mmol) in acetonitrile (15 mL). The mixture was stirred at 80°C for 12 h. The resulting mixture was concentrated under vacuum and purified by C18 reversed-phase column chromatography to afford a gray solid compound 15f (120 mg, 38%).

$^1$H NMR (400 MHz, DMSO-$d_6$) δ 10.97 (s, 1H), 7.35 (s, 1H), 7.13 (s, 1H), 6.71 (d, *J* = 9.9 Hz, 1H), 6.33 (d, *J* = 9.8 Hz, 1H), 5.08 (dd, *J* = 13.3, 5.1 Hz, 1H), 4.41 - 4.19 (m, 2H), 4.15 - 3.96 (m, 4H), 2.97 - 2.85 (m, 1H), 2.66 - 2.55 (m, 1H), 2.41 - 2.30 (m, 1H), 2.03 - 1.95 (m, 1H), 1.40 (s, 9H). LC-MS(ESI): $[M-tBu+H]^+$ =384.35.

Step 7: 3-(8'-Oxa-6',8'-dihydro-7'H-spiro[azetidine-3,2'-pyrano[2,3-f]isoindol]-7'-yl)piperidine-2,6-dione (Compound 15g)

Compound 15f (120 mg, 0.7 mmol) was dissolved in dichloromethane (3 mL), and trifluoroacetic acid (1 mL) was added. The mixture was stirred at room temperature for 2 h. The resulting mixture was concentrated, and the crude product was used directly in the next step.

LC-MS(ESI): $[M+H]^+$ =340.34.

Step 8: 3-(8'-Oxa-3',4',6',8'-tetrahydro-7'H-spiro[azetidine-3,2'-pyrano[2,3-f]isoindol]-7'-yl)piperidine-2,6-dione (E3 Ligase Inhibitor 15)

The compound 15g(50mg, mmol) was dissolved in methanol (3 mL), and palladium on carbon (5 mg) was added. The mixture was stirred at room temperature for 12 h. The resulting mixture was filtered through diatomaceous earth, and the filtrate was concentrated. Purification by preparative HPLC afforded a white solid E3 ligase inhibitor 15 (30 mg, 59%).

$^1$H NMR (400 MHz, DMSO-$d_6$) δ 10.97 (s, 1H), 7.36 (s, 1H), 7.11 (s, 1H), 5.07 (dd, *J* = 13.3, 5.1 Hz, 1H), 4.34 (d, *J* =

16.8 Hz, 1H), 4.21 (d, $J$ = 16.8 Hz, 1H), 4.15 - 4.04 (m, 4H), 2.91 (q, $J$ = 5.6, 4.4 Hz, 2H), 2.67 - 2.56 (m, 2H), 2.40 - 2.31 (m, 1H), 2.20 (t, $J$ = 6.9 Hz, 2H), 1.98 (m, 1H). LC-MS(ESI): $[M+H]^+$ =342.43.

16) Synthesis of E3 Ligase Inhibitor 16

Synthesis scheme:

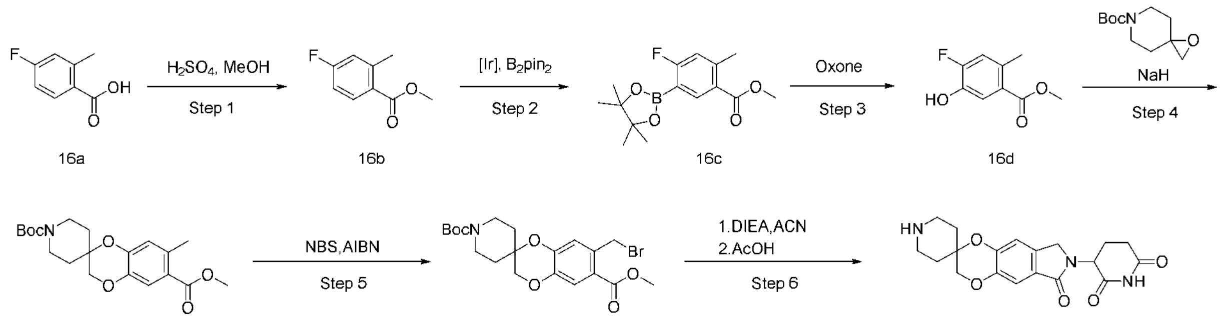


16a 16b 16c 16d

16e 16f 16

Step 1: 4-Fluoro-2-methylbenzoic acid methyl ester (Compound 16b)

Concentrated sulfuric acid (15 mL) was added dropwise to a solution of compound 16a (5.5 g, 35.7 mmol) in methanol (100 mL). The mixture was stirred at room temperature overnight. The resulting mixture was poured into ice water and extracted with ethyl acetate. The organic phase was dried over anhydrous sodium sulfate, filtered, and concentrated to afford compound 16b as a colorless oil (5.0 g, 83%). The crude product was used directly in the next step without purification.

$^1H$ NMR (600 MHz, DMSO-$d_6$) δ 7.90 (dd, $J$ = 8.7, 6.2 Hz, 1H), 7.22 (dd, $J$ = 10.1, 2.8 Hz, 1H), 7.15 (td, $J$ = 8.5, 2.7 Hz, 1H), 3.82 (s, 3H), 2.53 (s, 3H). LC-MS(ESI): $[M+H]^+$ = 169.19.

Step 2: Methyl 4-fluoro-2-methyl-5-(4,4,5,5-tetramethyl-1,3,2-dioxaborolan-2-yl)benzoate (Compound 16c)

Pinacol boronate (5.7 g, 22.3 mmol), 4,4'-di-tert-butyl-2,2'-dipyridyl (0.16 g, 0.59 mmol), and methoxy(cyclooctadiene)iridium dimer (0.20 g, 0.30 mmol) were added to a solution of methyl tert-Butyl ether (10 mL). Compound 16b (2.5 g, 14.9 mmol) in methyl tert-butyl ether (10 mL) was added, and the system was purged with nitrogen three times. The mixture was stirred at 85°C for 4 h. After completion, the resulting mixture was filtered through diatomaceous earth, and the filtrate was concentrated to afford the crude product (4.0 g), which was used directly in the next step without purification.

$^1H$ NMR (600 MHz, DMSO-$d_6$) δ 8.16 (d, $J$ = 6.3 Hz, 1H), 7.19 (dd, $J$ = 10.4, 3.2 Hz, 1H), 3.83 (s, 3H), 2.55 (s, 3H), 1.30 (s, 12H).

Step 3: 4-Fluoro-5-hydroxy-2-methylbenzoic acid methyl ester (Compound 16d)

The potassium peroxymonosulfate (10.9 g) was added to a solution of compound 16c (4.0 g, 13.6 mmol) in acetonitrile (50 mL). The reaction was stirred at room temperature overnight. The resulting mixture was filtered, and the filtrate was concentrated. The crude product was purified by silica gel column chromatography to afford a white solid compound 16d (2.0 g, 80%).

$^1H$ NMR (600 MHz, DMSO-$d_6$) δ 10.04 (s, 1H), 7.47 (d, $J$ = 9.3 Hz, 1H), 7.13 (d, $J$ = 12.2 Hz, 1H), 3.80 (s, 3H), 2.41 (s, 3H). LC-MS(ESI): $[M+H]^-$ = 183.21.

Step 4: 1'-(tert-Butyl) 6-methyl 7-methyl-3H-spiro[benzo[b][1,4]dioxine-2,4'-piperidine]-1',6-dicarboxylate (Compound 16e)

Compound 16d (1.0 g, 5.4 mmol) was dissolved in anhydrous N,N-dimethylformamide (10 mL), and 1-oxa-6-azaspiro[2.5]octane-6-carboxylic acid tert-butyl ester (1.2 g, 5.3 mmol) and sodium hydride (196 mg, 4.9 mmol) were added. The mixture was heated to 110°C and stirred overnight. The resulting mixture was purified by silica gel column chromatography to afford a white solid compound 16e (1.4 g, 68%).

$^1H$ NMR (600 MHz, DMSO-$d_6$) δ 7.37 (s, 1H), 6.85 (s, 1H), 4.04 (s, 2H), 3.77 (s, 3H), 3.73 (d, $J$ = 13.4 Hz, 2H), 3.24

- 3.04 (m, 2H), 2.42 (s, 3H), 1.66 - 1.61 (m, 4H), 1.41 (s, 9H). LC-MS(ESI): $[M+H]^+$ = 278.29.

Step 5: 1'-(tert-Butyl) 6-methyl 7-(bromomethyl)-3H-spiro[benzo[b][1,4]dioxine-2,4'-piperidine]-1',6-dicarboxylate (Compound 16f)

To a solution of compound 16e (1.0 g, 2.7 mmol) in carbon tetrachloride (10 mL), N-bromosuccinimide (613 mg, 3.4 mmol) and azobisisobutyronitrile (44 mg, 0.3 mmol) were added. The system was purged with nitrogen three times. The mixture was stirred under nitrogen at 85°C overnight. The resulting mixture was filtered, and the filtrate was concentrated to afford the crude product (1.2 g), which was used directly in the next step.

Step 6: 3-(6'-Oxa-6',8'-dihydro-3'H,7'H-spiro[piperidine-4,2'-[1,4]dioxino[2,3-f]isoindol]-7'-yl)piperidine-2,6-dione (E3 Ligase Inhibitor 16)

The 3-aminopiperidine-2,6-dione hydrochloride (655 mg, 4.0 mmol) and N,N-diisopropylethylamine (1.0 g, 8.0 mmol) were added to a solution of compound 16f (1.2 g, 2.7 mmol) in acetonitrile (10 mL). The reaction was stirred at 80°C overnight. The resulting mixture was concentrated, dissolved in acetic acid (10 mL), and stirred at 110°C for 2 h. After completion, the mixture was purified by reversed-phase column chromatography to afford a white solid E3 ligase inhibitor 16 (235 mg, 24%).

$^1H$ NMR (400 MHz, DMSO-$d_6$) δ 10.96 (s, 1H), 7.22 (s, 1H), 7.17 (s, 1H), 5.06 (dd, $J$ = 13.3, 5.1 Hz, 1H), 4.32 (d, $J$ = 16.8 Hz, 1H), 4.20 (d, $J$ = 16.8 Hz, 1H), 4.19 - 4.11 (m, 2H), 3.30 - 3.25 (m, 2H), 3.18 - 3.08 (m, 2H), 2.90 (ddd, $J$ = 17.3, 13.6, 5.4 Hz, 1H), 2.59 (dt, $J$ = 16.6, 3.4 Hz, 1H), 2.42 - 2.30 (m, 1H), 2.00 - 1.93 (m, 1H), 1.92 - 1.85 (m, 4H). LC-MS(ESI): $[M+H]^+$ = 373.35.

17) Synthesis of E3 Ligase Inhibitor 17

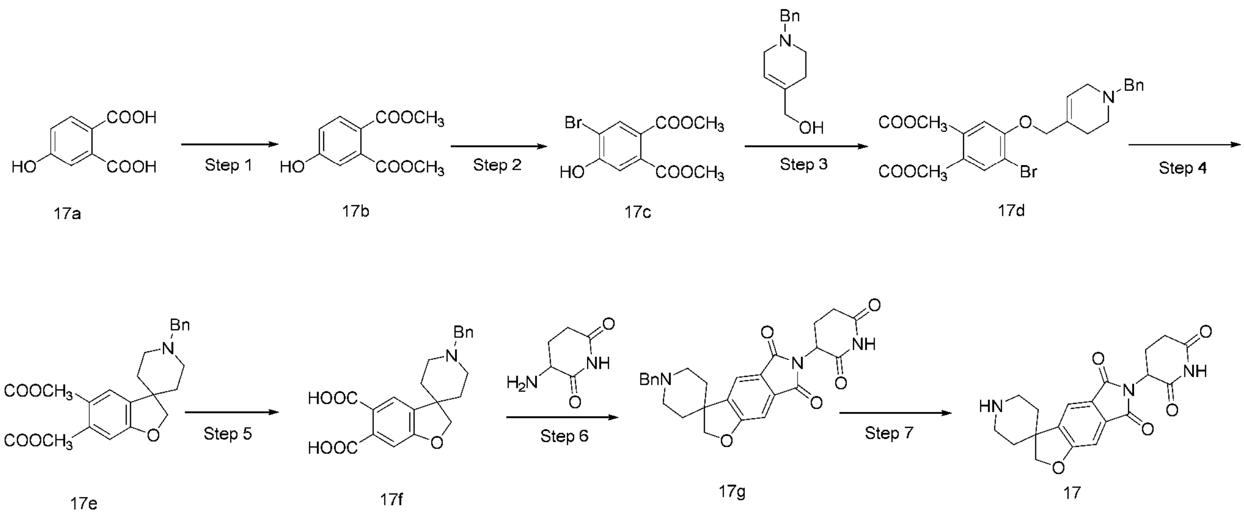


Step 1: Dimethyl 4-hydroxyphthalate (Compound 17b)

Under a nitrogen atmosphere, compound 17a (30 g, 165 mmol) was dissolved in methanol (300 mL), and concentrated sulfuric acid (36 mL) was added slowly. The mixture was heated to 66°C and stirred for 8 h. After cooling to room temperature, the resulting mixture was concentrated under reduced pressure, the water was added (200 mL), and the mixture was extracted with ethyl acetate (300 mL×3 times). The organic phase was washed with saturated brine (300 mL), dried over anhydrous sodium sulfate, and concentrated to afford a pale yellow solid compound 17b (31 g, 89.5%).

LC-MS(ESI): $[M-OMe+H]^+$ = 179.12.

Step 2: Dimethyl 4-bromo-5-hydroxyphthalate (Compound 17c)

Under a nitrogen atmosphere, compound 17b (10 g, 47 mmol) was dissolved in trifluoroacetic acid (100 mL), and N-bromosuccinimide (4.24 g, 47 mmol) was added. The mixture was stirred at room temperature overnight. The mixture

was concentrated under reduced pressure, and then the water was added (100 mL). The solution was extracted with ethyl acetate (100 mL×3 times), washed with saturated brine (100 mL), dried over anhydrous sodium sulfate, and concentrated. The crude product was purified by reversed-phase column chromatography to afford a pale yellow solid compound 17c (4.6 g, 33%).

$^1$H NMR (600 MHz, $CD_3OD$) δ 7.98 (s, 1H), 7.07 (s, 1H), 3.88 (s, 3H), 3.86 (s, 3H). LC-MS(ESI): $[M+H]^+$ = 289.04.

Step 3: Dimethyl 4-((1-benzyl-1,2,3,6-tetrahydropyridin-4-yl)methoxy)-5-bromophthalate (Compound 17d)

Under a nitrogen atmosphere, compound 17c (8 g, 27 mmol, 1.0 eq), (1-benzyl-1,2,3,6-tetrahydropyridin-4-yl) methanol (5.64 g, 27 mmol), and triphenylphosphine (7.2 g, 27 mmol) were dissolved in tetrahydrofuran (80 mL). Diethyl azodicarboxylate (4.08 mL, 27 mmol, 1.0 eq) was added, and the mixture was stirred at room temperature for 4 h. The mixture was concentrated under reduced pressure, and water (50 mL) was added. The solution was extracted with ethyl acetate (100 mL × 3 times), washed with saturated brine (100 mL), and dried over anhydrous sodium sulfate. After purification by column chromatography to afford a reddish-brown solid, compound 17d (6 g, 46%).

$^1$H NMR (600 MHz, $CDCl_3$) δ 8.04 (s, 1H), 7.47 (hd, *J* = 5.4, 1.9 Hz, 5H), 7.09 (s, 1H), 5.90 (tt, *J* = 3.2, 1.5 Hz, 1H), 4.67 - 4.55 (m, 2H), 4.38 (d, *J* = 12.9 Hz, 1H), 4.28 (d, *J* = 12.8 Hz, 1H), 3.98 (d, *J* = 16.7 Hz, 1H), 3.93 (s, 3H), 3.91 (s, 3H), 3.78 (dd, *J* = 19.0, 12.3 Hz, 1H), 3.48 (d, *J* = 16.5 Hz, 1H), 3.06 (s, 1H), 2.75 (s, 1H), 2.54 (d, *J* = 18.6 Hz, 1H). LC-MS(ESI): $[M+H]^+$ = 474.26/476.27.

Step 4: 1'-Benzyl-2H-spiro[benzofuran-3,4'-piperidine]-5,6-dicarboxylate (Compound 17e)

Under nitrogen atmosphere, compound 17d (8.4 g, 17 mmol), tri-n-butyltin hydride (10.2 g, 34 mmol), and azobisisobutyronitrile (574 mg, 3.4 mmol) were dissolved in toluene (84 mL). The mixture was heated to 110°C for 4 h. After cooling to room temperature, the resulting mixture was concentrated under reduced pressure. Water (100 mL) was added, and the solution was extracted with ethyl acetate (100 mL × 3), washed with saturated brine (100 mL), and dried over anhydrous sodium sulfate. After purification by column chromatography to afford a reddish-brown oily compound 17e (5 g,71%).

$^1$H NMR (400 MHz, $CDCl_3$) δ 7.67 (s, 1H), 7.55 - 7.41 (m, 5H), 6.97 (s, 1H), 4.47 (s, 2H), 4.27 (s, 2H), 3.91 (s, 3H), 3.89 (s, 3H), 3.70 (d, *J* = 12.3 Hz, 2H), 2.78 - 2.63 (m, 2H), 2.52 (td, *J* = 14.3, 4.0 Hz, 2H), 1.94 (d, *J* = 14.5 Hz, 2H). LC-MS(ESI): $[M+H]^+$ = 396.40.

Step 5: 1'-Benzyl-2H-spiro[benzofuran-3,4'-piperidine]-5,6-dicarboxylic acid (Compound 17f)

Compound 17e (2.3 g, 5.8 mmol) was dissolved in methanol (20 mL) and water (2 mL), and lithium hydroxide (1.39 g, 58 mmol) was added. The mixture was stirred at room temperature for 4 h. The mixture was concentrated under reduced pressure and purified by reversed-phase chromatography to afford a pale reddish-brown solid, compound 17f (1.28 g, 60%).

$^1$H NMR (600 MHz, DMSO-$d_6$) δ 7.51 (ddd, *J* = 12.6, 6.6, 3.4 Hz, 6H), 6.98 (s, 1H), 4.64 (s, 2H), 4.36 (s, 2H), 3.39 (s, 2H), 3.13 (t, *J* = 13.4 Hz, 2H), 2.15 (dd, *J* = 20.6, 8.4 Hz, 2H), 1.96 (d, *J* = 14.1 Hz, 2H). LC-MS(ESI): $[M+H]^+$ = 368.34.

Step 6: 1'-Benzyl-6-(2,6-dioxopiperidin-3-yl)-6H-2H,5H-spiro[[2,3-f]isoindole-3,4'-piperidine]-5,7-dione (Compound 17g)

Under nitrogen atmosphere, compound 17f (3.0 g, 8 mmol), sodium acetate (3.3 g, 24 mmol), and 3-amino-2,6-piperidinedione hydrochloride (1.3 g, 10 mmol, 1.25 eq) were dissolved in acetic acid (30 mL). The mixture was heated to 110°C for 4 h. After cooling to room temperature, the resulting mixture was concentrated under reduced pressure. Water (50 mL) was added, and the solution was extracted with ethyl acetate (100 mL × 3 times), washed with saturated brine (80 mL), and dried over anhydrous sodium sulfate. Purification by column chromatography afforded a pale yellow solid, compound 17g (1.8 g, 48%).

$^1$H NMR (600 MHz, $CDCl_3$) δ 7.69 (s, 1H), 7.54 - 7.43 (m, 5H), 7.25 (s, 1H), 4.96 (dd, *J* = 12.7, 5.4 Hz, 1H), 4.56 (s, 2H), 4.28 (s, 2H), 2.97 - 2.75 (m, 4H), 2.70 (t, *J* = 13.6 Hz, 2H), 2.63 - 2.53 (m, 2H), 1.99 (d, *J* = 14.6 Hz, 2H), 1.63 - 1.57 (m, 2H). LC-MS(ESI): $[M+H]^+$ = 460.34.

Step 7: 6-(2,6-Dioxopiperidin-3-yl)-6H-2H,5H-spiro[[2,3-f]isoindole-3,4'-piperidine]-5,7-dione (E3 Ligase Inhibitor 17)

Under nitrogen atmosphere, compound 17g (3.0 g, 6.5 mmol, 1.0 eq) was dissolved in methanol (30 mL), and 10% wet palladium on carbon (600 mg) was added. The system was purged with hydrogen three times, and the mixture was stirred at room temperature overnight. The mixture was filtered, and the filter cake was washed with methanol (15 mL

× 3 times). The filtrate was concentrated under reduced pressure, and the crude product was purified by reversed-phase column chromatography to afford a white solid, E3 ligase inhibitor 17 (630 mg, 26%).

$^1$H NMR (600 MHz, $CD_3OD$) δ 7.78 (s, 1H), 7.26 (d, *J* = 0.6 Hz, 1H), 5.14 - 5.10 (m, 1H), 4.74 (s, 2H), 3.38 (dt, *J* = 12.7, 3.2 Hz, 2H), 3.05 (td, *J* = 13.1, 3.0 Hz, 2H), 2.90 - 2.85 (m, 1H), 2.78 (dd, *J* = 4.4, 2.6 Hz, 1H), 2.77 - 2.69 (m, 2H), 2.14 (ddt, *J* = 13.2, 11.0, 4.1 Hz, 4H). LC-MS(ESI): $[M+H]^+$ = 370.33.

18) Synthesis of E3 Ligase Inhibitor 18

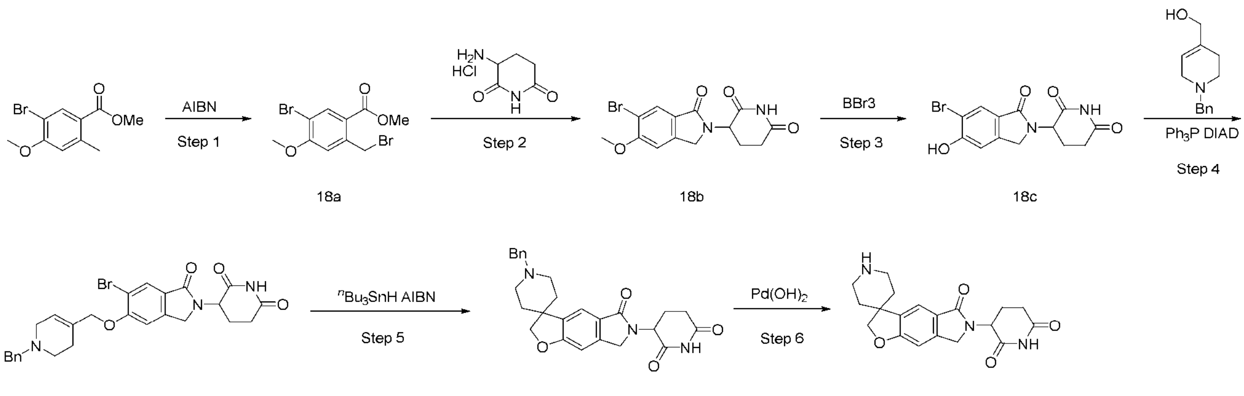


18a 18b 18c

18d 18e 18

Step 1: Methyl 5-bromo-2-(bromomethyl)-4-methoxybenzoate (Compound 18a)

Under nitrogen atmosphere, methyl 5-bromo-4-methoxy-2-methylbenzoate (5 g, 19.30 mmol), NBS (3.61 g, 20.30 mmol), and AIBN (316.9 mg, 1.90 mmol) were dissolved in carbon tetrachloride (50 mL). The mixture was heated to 75°C overnight. After completion (monitored by TLC), the resulting mixture was cooled to room temperature, and saturated sodium thiosulfate solution was added. After stirring for 30 min, the solution was extracted with dichloromethane, washed with saturated brine, and dried over anhydrous sodium sulfate. The crude product was purified by column chromatography to afford a white solid, compound 18a (6.9 g, 96.7%).

LC-MS (ESI): $[M+H]^+$ = 336.90.

Step 2: 3-(6-Bromo-5-methoxy-1-oxoisoindolin-2-yl)piperidine-2,6-dione (Compound 18b)

Under nitrogen atmosphere, compound 18a (7 g, 20.70 mmol) and 3-amino-2,6-piperidinedione hydrochloride (5.1 g, 31.10 mmol) were dissolved in acetonitrile (100 mL). N,N-Diisopropylethylamine (13.4 g, 103.60 mmol) was added, and the reaction was heated to 80°C overnight. The mixture was concentrated under reduced pressure, and acetic acid (50 mL) was added. The reaction was heated to 120°C for 2 h. After completion (monitored by TLC), the mixture was concentrated and purified by reversed-phase column chromatography to afford a solid, compound 18b (5.3 g, 72.5%).

LC-MS (ESI): $[M+H]^+$ = 353.15.

Step 3: 3-(6-Bromo-5-hydroxy-1-oxoisoindolin-2-yl)piperidine-2,6-dione (Compound 18c)

Under nitrogen atmosphere, compound 18b (5 g, 14.20 mmol) was dissolved in dichloromethane (10 mL). A solution of boron tribromide in dichloromethane (1 M, 140 mL) was slowly added at 0°C. After addition, the mixture was warmed to room temperature and stirred overnight. After completion (monitored by TLC), the resulting mixture was concentrated under reduced pressure to remove most of the boron tribromide. Dichloromethane (50 mL) was added, and the reaction was quenched with a small amount of methanol. The mixture was concentrated and purified by reversed-phase column chromatography to afford a grayish-white solid, compound 18c (3.6 g, 75.0%).

LC-MS (ESI): $[M+H]^+$ = 338.99.

Step 4: 3-(5-((1-Benzyl-1,2,3,6-tetrahydropyridin-4-yl)methoxy)-6-bromo-1-oxoisoindolin-2-yl)piperidine-2,6-dione (Compound 18d)

Under nitrogen atmosphere, compound 18c (180 mg, 0.53 mmol), (1-benzyl-1,2,3,6-tetrahydropyridin-4-yl)methanol (162 mg, 0.80 mmol), and triphenylphosphine (320 mg, 1.22 mmol) were dissolved in tetrahydrofuran (10 mL). Diethyl azodicarboxylate (0.2 mL, 1.2 mmol) was added, and the mixture was stirred at room temperature for 4 h. After completion (monitored by TLC), the mixture was concentrated under reduced pressure and purified by column chromatography to afford a pale yellow solid, compound 18d (147 mg, 53%).

$^1H$ NMR (600 MHz, DMSO-$d_6$) δ 11.00 (s, 1H), 7.90 (s, 1H), 7.55-7.46 (m, 5H), 7.42 (s, 1H), 5.91 (s, 1H), 5.08 (dd, *J* = 13.3, 5.1 Hz, 1H), 4.75 (s, 2H), 4.45-4.34 (m, 3H), 4.27 (d, *J* = 17.5 Hz, 1H), 3.71 (s, 2H), 3.56 (s, 1H), 3.17 (s, 1H), 2.91 (m, 1H), 2.63-2.57 (m, 1H), 2.41 (m, 3H), 2.00 (m, 1H). LC-MS (ESI): $[M+H]^+$ = 524.09.

Step 5: 3-(1'-Benzyl-5-oxo-5,7-dihydro-2H,6H-spiro[furo[2,3-f]isoindole-3,4'-piperidin]-6-yl)piperidine-2,6-dione (Compound 18e)

Under nitrogen atmosphere, compound 18d (120 mg, 0.23 mmol), tri-n-butyltin hydride (80 mg, 0.27 mmol), and azobisisobutyronitrile (19 mg, 0.12 mmol) were dissolved in toluene (10 mL). The reaction was heated to 110°C for 2 h. After completion (monitored by TLC), the mixture was cooled to room temperature and concentrated under reduced pressure. Purification by reversed-phase column chromatography afforded a white solid, compound 18e (51 mg, 50%).

$^1H$ NMR (600 MHz, DMSO-$d_6$) δ 10.97 (s, 1H), 7.56-7.47 (m, 5H), 7.42 (s, 1H), 7.04 (s, 1H), 5.03 (dd, *J* = 13.2, 4.9 Hz, 1H), 4.64 (q, *J* = 9.3 Hz, 2H), 4.39-4.33 (m, 3H), 4.23 (d, *J* = 17.3 Hz, 1H), 3.33-3.24 (m, 2H), 3.16 (d, *J* = 16.2 Hz, 2H), 2.94-2.84 (m, 1H), 2.59 (d, J = 16.7 Hz, 1H), 2.41-2.32 (m, 1H), 2.16 (t, *J* = 12.4 Hz, 2H), 2.00-1.92 (m, 3H). LC-MS (ESI): $[M+H]^+$ = 446.35.

Step 6: 3-(5-Oxo-5,7-dihydro-2H,6H-spiro[furo[2,3-f]isoindole-3,4'-piperidin]-6-yl)piperidine-2,6-dione (E3 Ligase Inhibitor 18)

Under nitrogen atmosphere, compound 18e (25 mg, 0.06 mmol) was dissolved in methanol (2 mL), and 10% wet palladium on carbon (24 mg) was added. The system was purged with hydrogen three times, and the mixture was stirred at room temperature overnight. The mixture was filtered, and the filter cake was washed with methanol (5 mL × 3 times). The filtrate was concentrated under reduced pressure, and the crude product was purified by reversed-phase column chromatography to afford a white solid, E3 ligase inhibitor 18 (8.7 mg, 43%).

$^1H$ NMR (600 MHz, DMSO-$d_6$) δ 10.97 (s, 1H), 7.47 (s, 1H), 7.04 (s, 1H), 5.06 (dd, *J* = 13.3, 5.1 Hz, 1H), 4.65-4.60 (m, 2H), 4.36 (d, *J* = 17.2 Hz, 1H), 4.24 (d, *J* = 17.2 Hz, 1H), 3.36-3.34 (m, 2H), 3.04 (d, *J* = 8.5 Hz, 2H), 2.95-2.86 (m, 1H), 2.60 (d, *J* = 17.1 Hz, 1H), 2.38 (m, 1H), 2.10-2.03 (m, 2H), 2.00-1.95 (m, 1H), 1.91-1.85 (m, 2H). LC-MS (ESI): $[M+H]^+$ = 356.35.

19) Synthesis of E3 Ligase Inhibitor 19

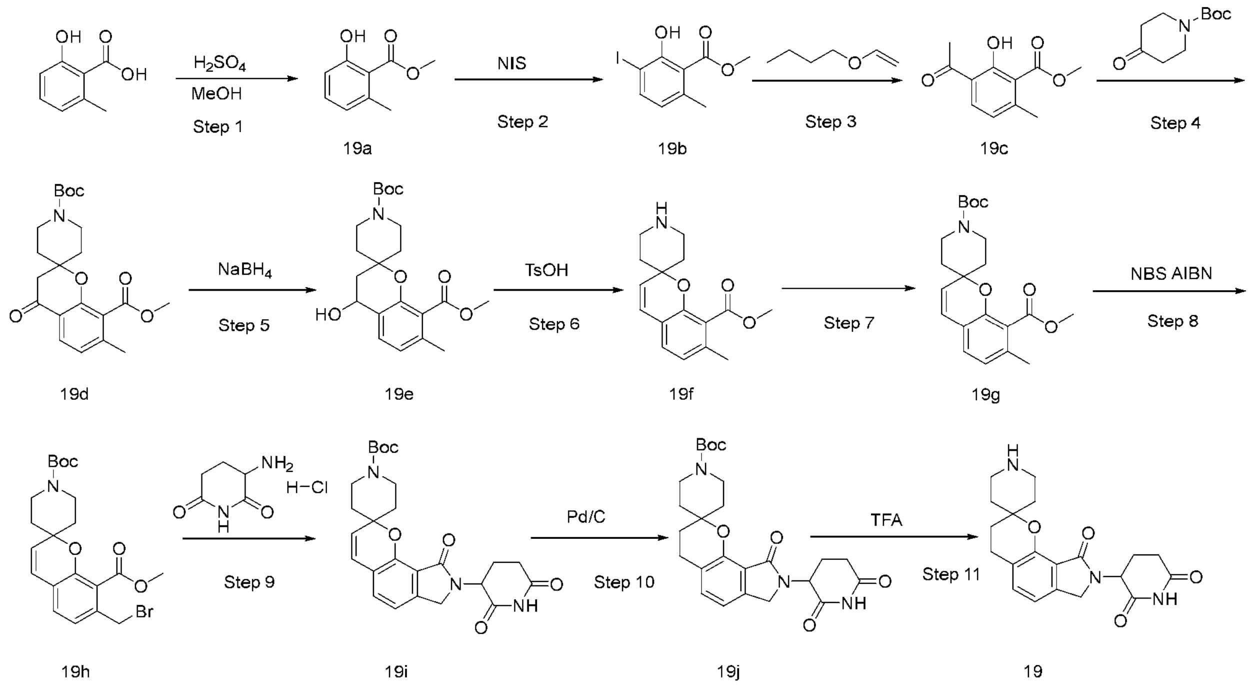


19a 19b 19c 19d 19e 19f 19g 19h 19i 19j 19

Step 1: Methyl 2-hydroxy-6-methylbenzoate (Compound 19a)

Under nitrogen atmosphere, 2-hydroxy-6-methylbenzoic acid (10 g, 65.79 mmol) was dissolved in methanol (30 mL), and concentrated sulfuric acid (10 mL) was added. The reaction was heated to 80°C for 16 h. After completion (monitored by TLC), the mixture was neutralized with 1 M NaOH, washed with water, and extracted with ethyl acetate. The organic phases were combined and concentrated to afford compound 19a (10 g, 91.6%), which was used directly in the next step without purification.

$^1$H NMR (600 MHz, $CDCl_3$) δ 11.31 (s, 1H), 7.31-7.28 (m, 1H), 6.87-6.86 (m, 1H), 6.75-6.74 (m, 1H), 3.99 (s, 3H), 2.56 (s, 3H). LC-MS (ESI): $[M+H]^+$ = 167.13.

Step 2: Methyl 2-hydroxy-3-iodo-6-methylbenzoate (Compound 19b)

Under nitrogen atmosphere, compound 19a (10 g, 60.24 mmol) was dissolved in trifluoroacetic acid (20 mL), and N-iodosuccinimide (16.2 g, 72.00 mmol) was added. The mixture was stirred at room temperature for 16 h. After completion (monitored by TLC), the reaction was quenched with saturated $Na_2S_2O_3$ solution. The mixture was washed with water, and extracted with ethyl acetate. The organic phases were dried over anhydrous sodium sulfate and concentrated to obtain a crude product. The crude product was purified by reversed-phase chromatography to afford compound 19b (3.8 g, 21.6%).

$^1$H NMR (600 MHz, $CDCl_3$) δ 12.19 (s, 1H), 7.77 (d, *J* = 8.0 Hz, 1H), 6.56 (dd, *J* = 8.0, 0.8 Hz, 1H), 4.01 (s, 3H), 2.54 (s, 3H).

Step 3: Methyl 3-acetyl-2-hydroxy-6-methylbenzoate (Compound 19c)

Under nitrogen atmosphere, compound 19b (3.8 g, 13.02 mmol) and butyl vinyl ether (3.9 g, 39.00 mmol) were dissolved in methanol (20 mL). Pd(dppf)$Cl_2$ (951 mg, 1.30 mmol) and TEA (3.95 g, 39.00 mmol) were added. The system was purged with nitrogen three times, and the mixture reacted at 60°C for 16 h. After completion (monitored by TLC), the resulting mixture was concentrated, dissolved in dichloromethane, washed with 5 M/L HCl and saturated brine, dried over anhydrous sodium sulfate, and concentrated. Purification by column chromatography afforded a compound 19c (2.0 g, 73.8%).

$^1$H NMR (600 MHz, $CDCl_3$) δ 12.74 (s, 1H), 7.70 (d, *J* = 8.2 Hz, 1H), 6.79 (d, *J* = 8.2 Hz, 1H), 3.97 (s, 3H), 2.64 (s, 3H), 2.38 (s, 3H). LC-MS (ESI): $[M-OCH_3+H]^+$ = 177.14.

Step 4: 1'-tert-Butyl 8-methyl 7-methyl-4-oxospiro[chroman-2,4'-piperidine]-1,8-dicarboxylate (Compound 19d)

Under nitrogen atmosphere, compound 19c (1 g, 4.80 mmol) was dissolved in methanol (30 mL), and pyrrolidine

(340 mg, 4.80 mmol) and N-tert-butoxycarbonyl-4-piperidone (960 mg, 4.80 mmol) were added slowly. The mixture was heated to 70°C for 12 h. After completion (monitored by TLC), the resulting mixture was cooled to room temperature and concentrated under reduced pressure. Water was added, and the solution was extracted with ethyl acetate, washed with saturated brine, and dried over anhydrous sodium sulfate. Purification by column chromatography to afforded a yellow solid, compound 19d (1.5 g, 80%).

$^1$H NMR (600 MHz, DMSO-$d_6$) δ 7.72 (d, *J* = 8.0 Hz, 1H), 6.99 (d, *J* = 8.0 Hz, 1H), 3.87 (s, 3H), 3.78 (br, 2H), 2.99 (br, 2H), 2.84 (s, 2H), 2.28 (s, 3H), 1.96-1.78 (m, 2H), 1.59 (m, 2H), 1.40 (s, 9H). LC-MS (ESI): $[M+H]^+$ = 390.35.

Step 5: 1'-tert-Butyl 8-methyl 4-hydroxy-7-oxospiro[chroman-2,4'-piperidine]-1,8-dicarboxylate (Compound 19e)

Under a nitrogen atmosphere, compound 19d (1.5 g, 3.85 mmol) was dissolved in methanol (25 mL), and sodium borohydride (340 mg, 7.7 mmol) was added. The mixture was stirred at room temperature for 1 h. After completion (monitored by TLC), the mixture was concentrated under reduced pressure. Water was added, and the solution was extracted with ethyl acetate, washed with saturated brine, dried over anhydrous sodium sulfate and was then concentrated. The crude product, compound 19e (1.5 g, 99%), was affordded.

$^1$H NMR (600 MHz, DMSO-$d_6$) δ 7.37 (d, *J* = 7.8 Hz, 1H), 6.80 (d, *J* = 7.9 Hz, 1H), 5.44 (d, *J* = 5.5 Hz, 1H), 4.67 (q, *J* = 6.7 Hz, 1H), 3.80 (s, 3H), 3.76 (br, 2H), 2.97 (br, 2H), 2.17 (s, 3H), 2.06 (m, 1H), 1.85-1.66 (m, 3H), 1.60-1.47 (m, 2H), 1.41 (s, 9H). LC-MS (ESI): $[M+H]^+$ = 392.40.

Step 6: 7-Methylspiro[chromene-2,4'-piperidine]-8-carboxylate (Compound 19f)

Under nitrogen atmosphere, compound 19e (1.5 g, 3.83 mmol) was dissolved in toluene (25 mL), and p-toluenesulfonic acid (659.8 mg, 3.83 mmol) was added. The mixture was heated to 110°C for 12 h. After completion (monitored by TLC), the resulting mixture was concentrated under reduced pressure to afford the crude product, compound 19f (850 mg, 81%), which was used directly in the next step.

LC-MS (ESI): $[M+H]^+$ = 274.25.

Step 7: 1'-tert-Butyl 8-methyl 7-methylspiro[chromene-2,4'-piperidine]-1',8-dicarboxylate (Compound 19g)

Under nitrogen atmosphere, compound 19f (850 mg, 3.11 mmol) was dissolved in dichloromethane (20 mL), and $Boc_2O$(1.36 g, 6.22 mmol) and TEA (600 mg, 6.22 mmol) were added. The mixture was stirred at 25°C for 1 h. After completion (monitored by TLC), the mixture was cooled to room temperature and concentrated under reduced pressure. Water was added, and the solution was extracted with ethyl acetate, washed with saturated brine, and dried over anhydrous sodium sulfate. Purification by column chromatography afforded a white solid, compound 19g (1 g, 86%).

$^1$H NMR (600 MHz, DMSO-$d_6$) δ 7.07 (d, *J* = 7.6 Hz, 1H), 6.78 (d, *J* = 7.6 Hz, 1H), 6.48 (d, *J* = 9.8 Hz, 1H), 5.72 (d, *J* = 9.8 Hz, 1H), 3.83 (s, 5H), 3.07 (s, 2H), 2.18 (s, 3H), 1.81 (m, 2H), 1.57 (m, 2H), 1.41 (s, 9H). LC-MS (ESI): $[M+H]^+$ = 374.40.

Step 8: 1'-tert-Butyl 8-methyl 7-(bromomethyl)spiro[chromene-2,4'-piperidine]-1',8-dicarboxylate (Compound 19h)

Under nitrogen atmosphere, compound 19g (1 g, 2.68 mmol) was dissolved in carbon tetrachloride (20 mL), and N-bromosuccinimide (620 mg, 3.50 mmol) and AIBN (43 mg, 0.27 mmol) were added. The mixture was heated to 85°C for 12 h. After completion (monitored by TLC), the resulting mixture was concentrated under reduced pressure to afford the crude product, compound 19h (1.2 g, 99%), which was used directly in the next step.

LC-MS (ESI): $[M+H]^+$ = 452.25.

Step 9: 8'-(2,6-Dioxopiperidin-3-yl)-9'-oxo-8',9'-dihydro-7'H-spiro[piperidine-4,2'-pyrano[2,3-e]isoindole]-1-carboxylate (Compound 19i)

Under nitrogen atmosphere, compound 19h (1.2 g, 2.65 mmol), DIPEA (1 g, 7.95 mmol), and 3-amino-2,6-piperidinedione hydrochloride (654.9 mg, 3.98 mmol) were dissolved in acetonitrile (30 mL). The mixture was heated to 85°C for 16 h. After completion (monitored by TLC), the resulting mixture was cooled to room temperature and concentrated under reduced pressure. Water was added, and the solution was extracted with ethyl acetate, washed with saturated brine, and dried over anhydrous sodium sulfate. Purification by column chromatography afforded a gray solid, compound 19i (400 mg, 32%).

$^1$H NMR (600 MHz, DMSO-$d_6$) δ 11.00 (s, 1H), 7.31 (d, J = 7.5 Hz, 1H), 7.04 (d, J = 7.5 Hz, 1H), 6.57 (d, J = 9.9 Hz, 1H), 5.79 (d, J = 9.9 Hz, 1H), 4.99 (d, J = 5.1 Hz, 1H), 4.44-4.16 (m, 2H), 3.79 (s, 2H), 3.19 (d, J = 22.8 Hz, 4H), 2.87 (ddd, J = 18.2, 13.6, 5.4 Hz, 1H), 2.73-2.56 (m, 1H), 2.51 (p, J = 1.9 Hz, 1H), 2.06-1.56 (m, 3H), 1.41 (s, 9H). LC-MS(ESI): [M-Boc+H]$^+$ = 368.19.

Step 10: 8'-(2,6-Dioxopiperidin-3-yl)-9'-oxo-4',7',8',9'-tetrahydro-3'H-spiro[piperidine-4,2'-pyrano[2,3-e]isoindole]-1-carboxylate (Compound 19j)

Under a nitrogen atmosphere, compound 19i (400 mg, 0.85 mmol) was dissolved in THF (10 mL), and 10% wet palladium on carbon (250 mg) was added. The system was purged with hydrogen three times, and the mixture was heated to 50°C overnight. After completion (monitored by TLC), the resulting mixture was filtered, and the filter cake was washed with methanol (15 mL $\times$ 3). The filtrate was concentrated under reduced pressure to afford the crude product, compound 19j (380 mg, 94%), which was used directly in the next step.
LC-MS (ESI): [M-Boc+H]$^+$ = 370.29.

Step 11: 3-(9'-Oxo-3',4',7',9'-tetrahydro-8'H-spiro[piperidine-4,2'-pyrano[2,3-e]isoindol]-8'-yl)piperidine-2,6-dione (E3 Ligase Inhibitor 19)

Under nitrogen atmosphere, compound 19j (380 mg, 0.80 mmol) was dissolved in dichloromethane (5 mL), and trifluoroacetic acid (2 mL) was added. The mixture was stirred at 25°C for 1 h. After completion (monitored by TLC), the mixture was concentrated under reduced pressure. Purification by preparative HPLC afforded a yellow solid, E3 ligase inhibitor 19 (290 mg, 97%).
$^1$H NMR (600 MHz, DMSO-$d_6$) δ 10.98 (s, 1H), 7.34 (d, J = 7.7 Hz, 1H), 7.04 (d, J = 7.6 Hz, 1H), 4.97 (dd, J = 13.3, 5.2, 1H), 4.43-4.20 (m, 2H), 3.30-3.08 (m, 4H), 2.97-2.78 (m, 3H), 2.61 (dt, J = 17.1, 3.7 Hz, 1H), 2.47-2.31 (m, 1H), 2.04-1.88 (m, 5H), 1.80 (tt, J = 12.6, 5.0 Hz, 2H). LC-MS(ESI): [M+H]$^+$ = 370.39.

20) Synthesis of E3 Ligase Inhibitor 20

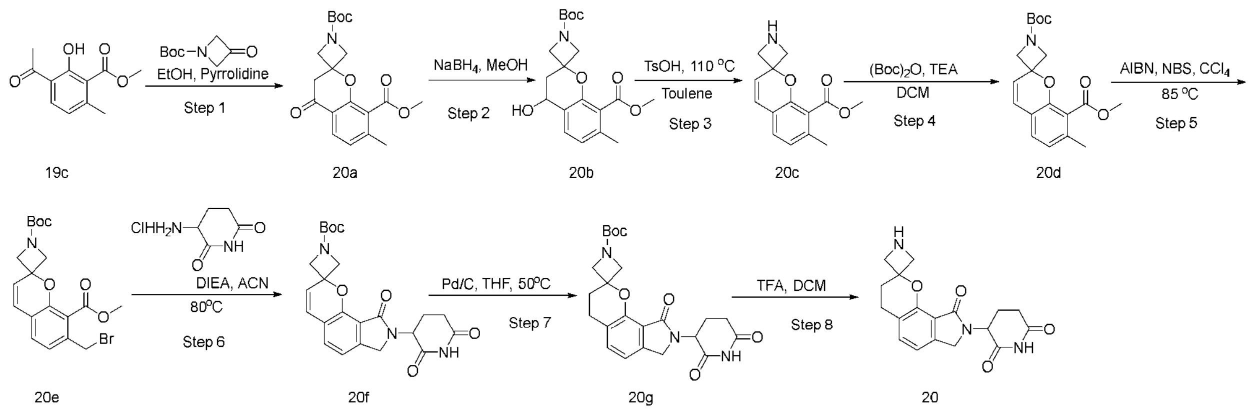


Step 1: 1-tert-Butyl 8'-methyl 7'-methyl-4'-oxospiro[azetidine-3,2'-chroman]-1,8'-dicarboxylate (Compound 20a)

Under nitrogen atmosphere, compound 19c (2.0 g, 9.61 mmol) was dissolved in ethanol (15 mL), followed by the addition of 1-Boc-3-azetidinone (1.63 g, 9.61 mmol) and pyrrolidine (682 mg, 9.61 mmol). The mixture was stirred at 70°C for 4 h. TLC monitoring indicated incomplete conversion, prompting the addition of 1-Boc-3-azetidinone (0.82 g, 4.81 mmol) and pyrrolidine (340 mg, 4.81 mmol). The mixture was stirred at 70°C for 16 h. After completion, the solvent was evaporated under reduced pressure to afford the crude product, which was purified by column chromatography to yield compound 20a (1.0 g, 28.8%).
$^1$H NMR (600 MHz, $CDCl_3$) δ 7.84 (d, *J* = 8.0 Hz, 1H), 6.96 (d, *J* = 8.0 Hz, 1H), 4.09 (d, *J* = 9.5 Hz, 2H), 3.99 (s, 3H), 3.96 (d, *J* = 9.5 Hz, 2H), 3.06 (s, 2H), 2.39 (s, 3H), 1.46 (s, 9H). LC-MS (ESI): [M-Boc+H]$^+$ = 262.17.

Step 2: 1-tert-Butyl 8'-methyl 4'-hydroxy-7'-methylspiro[azetidine-3,2'-chroman]-1,8'-dicarboxylate (Compound 20b)

Under nitrogen atmosphere, compound 20a (1.0 g, 2.77 mmol) was dissolved in methanol (15 mL), and $NaBH_4$ (157 mg, 4.16 mmol) was added at room temperature. The mixture was stirred at room temperature for 2 h. After completion of the reaction as monitored by TLC, the reaction was quenched with saturated $NH_4Cl$. The mixture was extracted with ethyl acetate, washed with saturated brine, and concentrated to afford 20b (900.0 mg, 89.5%), which was

used directly in the next step.

LC-MS (ESI): $[M-Boc+H]^+$ = 264.17.

Step 3: 7'-methylspiro[azetidine-3,2'-chromene]-8'-carboxylate (Compound 20c)

Under nitrogen atmosphere, compound 20b (900.0 mg, 2.48 mmol) was dissolved in toluene (10 mL), and TsOH (471 mg, 2.48 mmol) was added. The mixture was stirred at 110°C for 2 h. After completion of the reaction as monitored by TLC, the solvent was evaporated to afford a crude compound 20c (1.1 g, 181.1%), which was used directly in the next step.

LC-MS (ESI): $[M+H]^+$ = 246.11.

Step 4: 1-tert-Butyl 8'-methyl 7'-methylspiro[azetidine-3,2'-chromene]-1,8'-dicarboxylate (Compound 20d)

Undernitrogen atmosphere, compound 20c (1.1 g, 1 eq) was dissolved in dichloromethane (15 mL), followed by adding the $(Boc)_2O$ (1.95 g, 2.0 eq) and TEA (1.36 g, 3.0 eq). The mixture was stirred at room temperature for 2 h. After completion of the reaction as monitored by TLC, the solvent was evaporated. The mixture was purified by column chromatography to afford a compound 20d (300.0 mg, 19.4%).

$^1H$ NMR (600 MHz, CDCl3) δ 6.95 (d, *J* = 7.6 Hz, 1H), 6.76 (d, *J* = 7.6 Hz, 1H), 6.45 (d, *J* = 9.8 Hz, 1H), 5.93 (d, *J* = 9.8 Hz, 1H), 4.24 (d, *J* = 9.5 Hz, 2H), 3.99 (d, *J* = 9.5 Hz, 2H), 3.96 (s, 3H), 2.30 (s, 3H), 1.48 (s, 9H). LC-MS (ESI): $[M-Boc+H]^+$ = 256.17.

Step 5: 1-tert-Butyl 8'-methyl 7'-(bromomethyl)spiro[azetidine-3,2'-chromene]-1,8'-dicarboxylate (Compound 20e)

Under nitrogen atmosphere, compound 20d (400.0 mg, 1 eq) was dissolved in $CCl_4$ (10 mL), followed by adding the AIBN (19 mg, 0.1 eq) and NBS (268 mg, 1.3 eq). The mixture was stirred at 85°C for 16 h. Upon completion of the reaction as monitored by TLC, the solvent was evaporated to afford crude compound 20e (600 mg), which was used directly in the next step without purification.

LC-MS (ESI): $[M-Boc+H]^+$ = 324.07.

Step 6: 8'-(2,6-dioxopiperidin-3-yl)-9'-oxo-8',9'-dihydro-7'H-spiro[azetidine-3,2'-pyrano[2,3-e]isoindole]-1-carboxylate (Compound 20f)

Under nitrogen atmosphere, compound 20e (600 mg, 1 eq) was dissolved in CAN (10 mL), followed by adding the 3-aminopiperidine-2,6-dione hydrochloride (287 mg, 1.5 eq) and DIEA (448 mg, 3.0 eq). The mixture was stirred at 80°C for 16 h. Upon completion of the reaction as monitored by TLC, the solvent was evaporated. The mixture was purified by column chromatography to afford a compound 20f (60 mg, 21.4% yield over two steps).

$^1H$ NMR (600 MHz, DMSO-$d_6$) δ 11.00 (s, 1H), 7.34 (d, *J* = 7.6 Hz, 1H), 7.08 (d, *J* = 7.6 Hz, 1H), 6.67 (d, *J* = 9.9 Hz, 1H), 6.20 (d, *J* = 9.9 Hz, 1H), 4.99 (dd, *J* = 13.2, 5.2 Hz, 1H), 4.37 (d, *J* = 17.6 Hz, 1H), 4.25 (d, *J* = 17.6 Hz, 1H), 4.04-4.01 (m, 4H), 2.93-2.87 (m, 1H), 2.62-2.57 (m, 1H), 2.41-2.33 (m, 1H), 2.00-1.96 (m, 1H), 1.41 (s, 9H). LC-MS (ESI): $[M-Boc+H]^+$ = 340.19.

Step 7: 8'-(2,6-dioxopiperidin-3-yl)-9'-oxo-4',7',8',9'-tetrahydro-3'H-spiro[azetidine-3,2'-pyrano[2,3-e]isoindole]-1-carboxylate (Compound 20g)

Under nitrogen atmosphere, compound 20f (60.0 mg, 1 eq) was dissolved in THF (10 mL), and Pd/C (60 mg, 1 eq) was added. The system was purged with hydrogen three times, and the mixture was stirred at 50°C for 16 h. Upon completion of the reaction as monitored by TLC, the mixture was concentrated under reduced pressure to afford the crude compound 20g (80 mg), which was used directly in the next step without further purification.

LC-MS (ESI): $[M-Boc+H]^+$ = 342.17.

Step 8: 3-(9'-oxo-3',4',7',9'-tetrahydro-8'H-spiro[azetidine-3,2'-pyrano[2,3-e]isoindol]-8'-yl)piperidine-2,6-dione (E3 Ligase Inhibitor 20)

Under nitrogen atmosphere, compound 20g (80 mg, 1 eq) was dissolved in dichloromethane (5 mL), and TFA (1 mL) was added. The mixture was stirred at room temperature for 2 h. Upon completion of the reaction as monitored by TLC, the mixture was concentrated under reduced pressure and purified by preparative HPLC, followed by lyophilization to afford E3 ligase inhibitor 20 (15 mg, 24.3% yield over two steps).

$^1H$ NMR (600 MHz, DMSO-$d_6$) δ 10.98 (s, 1H), 7.35 (d, J = 7.6 Hz, 1H), 7.08 (d, J = 7.6 Hz, 1H), 4.97 (dd, J = 13.2, 5.2 Hz, 1H), 4.35 (d, J = 17.3 Hz, 1H), 4.23 (d, J = 17.3 Hz, 1H), 4.14-4.10 (m, 4H), 2.93 - 2.85 (m, 3H), 2.62 - 2.58 (m, 1H),

2.42-2.35 (m, 1H), 2.22 - 2.19 (m, 2H), 1.99-1.95 (m, 1H).

LC-MS (ESI): $[M+H]^+$ = 342.29.

21) Synthesis of E3 Ligase Inhibitor 21

Synthesis scheme:

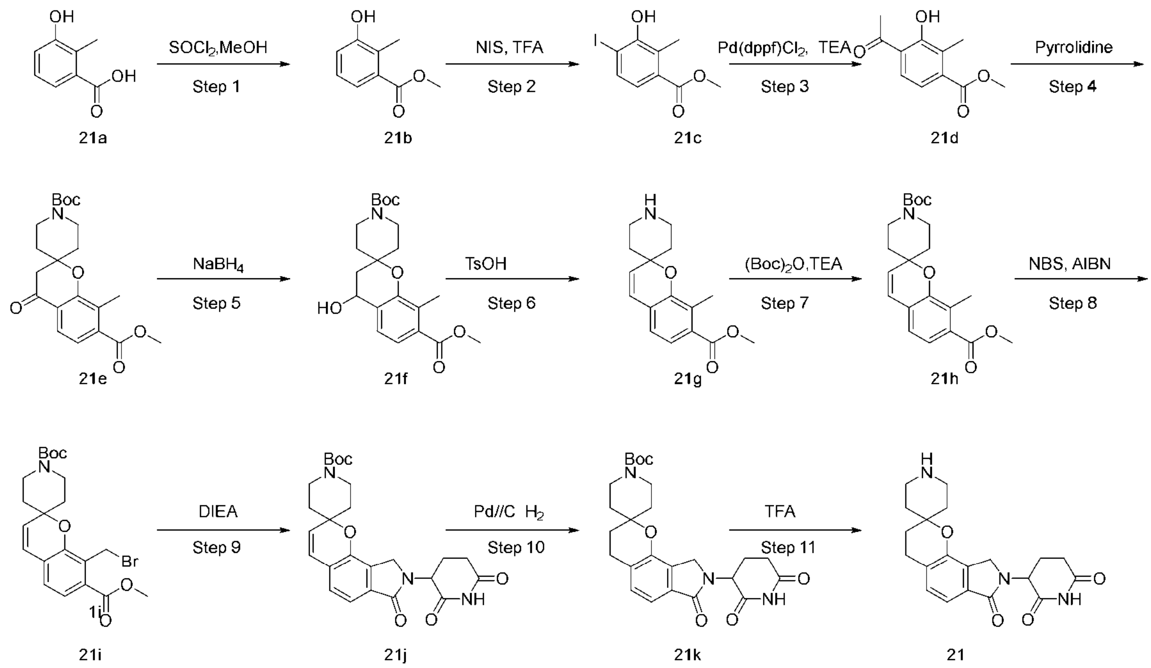


Step 1: Methyl 3-hydroxy-2-methylbenzoate (Compound 21b)

The thionyl chloride was slowly added to a solution of 3-hydroxy-2-methylbenzoic acid 21a (10 g, 65.73 mmol) in methanol (100 mL) (15.64 g, 131.45 mmol). The mixture was stirred at 80°C for 2 h. The solvent was removed under reduced pressure to afford a gray solid compound 21b (10.0 g, 91.56%).

$^1$H NMR (600 MHz, DMSO-$d_6$) δ 9.71 (s, 1H), 7.19 (dd, *J*=7.7, 1.3 Hz, 1H), 7.09 (t, J=7.9 Hz, 1H), 7.02 (dd, *J*=8.0, 1.4 Hz, 1H), 3.80 (s, 3H), 2.29 (s, 3H). LC-MS (ESI): $[M+H]^+$ = 167.23.

Step 2: Methyl 3-hydroxy-4-iodo-2-methylbenzoate (Compound 21c)

N-iodosuccinimide was slowly added to a solution of compound 21b (10.0 g, 60.18 mmol) in trifluoroacetic acid (80 mL) and methanol (40 mL) (17.6 g, 78.23 mmol). The mixture was stirred at room temperature for 2 h. The resulting mixture was concentrated under vacuum and purified by C18 reversed-phase column chromatography to afford a white solid compound 21c (4 g, 22.76%).

$^1$H NMR (600 MHz, DMSO-$d_6$) δ 9.36 (s, 1H), 7.67 (d, *J* = 8.2 Hz, 1H), 7.03 (d, *J* = 8.2 Hz, 1H), 3.80 (s, 3H), 2.38 (s, 3H). LC-MS(ESI): $[M+H]^+$ = 364.22.

Step 3: Methyl 4-acetyl-3-hydroxy-2-methylbenzoate (Compound 21d)

Under a nitrogen atmosphere, a mixture of compound 21c (5.4 g, 18.49 mmol), butyl vinyl ether (5.56 g, 55.47 mmol), Pd(dppf)$Cl_2$ (1.35 g, 1.85 mmol), and triethylamine (5.61 g, 55.47 mmol) in methanol (80 mL) was heated to 60°C and stirred for 4 h. The mixture was filtered and concentrated. The crude product was purified by column chromatography (PE: EA = 20%) to afford a yellow oily compound 21d (3 g, 77.93%).

$^1$H NMR (600 MHz, $CD_3OD$) δ 7.28 (d, *J* = 8.2 Hz, 1H), 7.18 (d, *J* = 8.3 Hz, 1H), 3.87 (s, 3H), 2.38 (s, 3H), 1.59 (s, 3H). LC-MS(ESI): $[M+H]^+$ = 209.15.

Step 4: 1'-(tert-Butyl) 7-methyl 8-methyl-4-oxospiro[chroman-2,4'-piperidine]-1',7-dicarboxylate (Compound 21e)

A mixture of compound 21d (3.0 g, 14.41 mmol), N-Boc-4-piperidone (3.16 g, 15.85 mmol), and pyrrolidine (1.13 g, 15.85 mmol) in methanol (60 mL) was stirred at 70°C overnight. The solvent was removed under reduced pressure, and the residue was purified by column chromatography (PE:EA = 30%) to afford a yellow solid compound 21e (5 g, 89.11%).

$^1$H NMR (600 MHz, DMSO-$d_6$) δ 7.65 (d, *J* = 8.2 Hz, 1H), 7.33 (d, *J* = 8.2 Hz, 1H), 3.86 (s, 3H), 2.89 (s, 2H), 2.38 (s, 3H), 1.92 (d, *J* = 2.5 Hz, 1H), 1.89 (q, *J* = 2.8 Hz, 1H), 1.64 (td, *J* = 13.1, 4.8 Hz, 2H), 1.43 (s, 4H), 1.40 (s, 9H). LC-MS(ESI): $[M-Boc+H]^+$ = 290.18.

Step 5: 1'-(tert-Butyl) 7-methyl 4-hydroxy-8-methylspiro[chroman-2,4'-piperidine]-1',7-dicarboxylate (Compound 21f)

Under ice-bath conditions, sodium borohydride (0.97 g, 25.68 mmol) was added to a solution of compound 21e (5.0 g, 12.84 mmol) in methanol (80 mL). The mixture was stirred at 25°C for 1 h. The reaction was quenched with ammonium chloride solution. The mixture was extracted with ethyl acetate, and concentrated to afford a yellow oily compound 21f (5.5 g), which was used directly in the next step.

LC-MS(ESI): $[M-Boc+H]^+$ = 292.28.

Step 6: Methyl 8-methylspiro[chromene-2,4'-piperidine]-7-carboxylate (Compound 21g)

A mixture of compound 21f (5.5 g, 14.05 mmol) and p-toluenesulfonic acid (2.66 g, 15.45 mmol) in toluene (80 mL) was stirred at 110°C for 5 h. The solvent was removed under vacuum to afford a brown oily compound 21g (3.84 g), which was used directly in the next step.

LC-MS(ESI): $[M+H]^+$ = 274.47.

Step 7: 1'-(tert-Butyl) 7-methyl 8-methylspiro[chromene-2,4'-piperidine]-1',7-dicarboxylate (Compound 21h)

A mixture of compound 21g (3.84 g, 14.05 mmol), Boc anhydride (6.13 g, 28.10 mmol), and triethylamine (4.26 g, 442.15 mmol) in dichloromethane (100 mL) was stirred at room temperature for 2 h. The solvent was removed, and the residue was purified by column chromatography (PE:EA = 25%) to afford a yellow oily compound 21h (5 g, 95.30%).

$^1$H NMR (600 MHz, $CD_3OD$) δ 7.37 (d, *J* = 7.9 Hz, 1H), 6.94 (d, *J* = 7.9 Hz, 1H), 6.48 (d, *J* = 9.8 Hz, 1H), 5.77 (d, *J* = 9.7 Hz, 1H), 3.87 (s, 3H), 2.45 (s, 3H), 1.97 - 1.93 (m, 2H), 1.66 (td, *J* = 13.9, 12.2, 4.8 Hz, 2H), 1.58 (s, 2H), 1.53 (s, 2H), 1.49 (s, 9H). LC-MS(ESI): $[M-Boc+H]^+$ = 274.17.

Step 8: 1'-(tert-Butyl) 7-methyl 8-(bromomethyl)spiro[chromene-2,4'-piperidine]-1',7-dicarboxylate (Compound 21i)

A mixture of compound 21h (3.3 g, 8.84 mmol), N-bromosuccinimide (1.89 g, 10.60 mmol), and azobisisobutyronitrile (145.11 mg, 883.65 μmol) in carbon tetrachloride (50 mL) was stirred at 80°C overnight. The solvent was removed under vacuum to afford a brown solid compound 21i (4 g), which was used directly in the next step.

LC-MS(ESI): $[M+H]^+$ = 316.08.

Step 9: 8'-(2,6-dioxopiperidin-3-yl)-7'-oxo-8',9'-dihydro-7'H-spiro[piperidine-4,2'-pyrano[2,3-e]isoindole]-1-carboxylate (Compound 21j)

A mixture of compound 21i (4 g, 8.84 mmol), 3-aminopiperidine-2,6-dione hydrochloride (2.18 g, 13.26 mmol), and N,N-diisopropylethylamine (3.43 g, 26.53 mmol) in acetonitrile (100 mL) was stirred at 80°C overnight. The mixture was purified by normal-phase column chromatography (100% EA) to afford a blue solid compound 21j (1 g, 24.19%).

LC-MS(ESI): $[M-tBu+H]^+$ = 412.29.

Step 10: 8'-(2,6-dioxopiperidin-3-yl)-7'-oxo-4',7',8',9'-tetrahydro-3'H-spiro[piperidine-4,2'-pyrano[2,3-e]isoindole]-1-carboxylate (Compound 21k)

A mixture of compound 21j (90 mg, 192.50 μmol) and palladium on carbon (20 mg) in tetrahydrofuran (5 mL) was stirred under a hydrogen atmosphere at 50°C overnight. After the reation was completed, the mixture was filtered to afford a white solid compound 21k (90 mg, 99.57%).

LC-MS(ESI): $[M-tBu+H]^+$ = 414.39.

Step 11: 3-(7'-Oxo-3',4',7',9'-tetrahydro-8'H-spiro[piperidine-4,2'-pyrano[2,3-e]isoindole]-8'-yl)piperidine-2,6-dione (E3 Ligase Inhibitor 21)

A mixture of compound 21k (90 mg, 191.68 μmol) and trifluoroacetic acid (1 mL) in dichloromethane (2 mL) was stirred at room temperature for 2 h. The solvent was removed, and the residue was purified by preparative HPLC to afford a white solid E3 Ligase Inhibitor 21 (50 mg, 70.61%).

$^1$H NMR (400 MHz, $CD_3OD$) δ 7.36 (d, *J* = 7.8 Hz, 1H), 7.32 (d, *J* = 7.8 Hz, 1H), 5.18 (dd, *J* = 13.3, 5.2 Hz, 1H), 4.55 - 4.40 (m, 2H), 3.38 (dd, *J* = 12.5, 3.3 Hz, 4H), 3.03 - 2.89 (m, 3H), 2.81 (m, *J* = 17.6, 4.6, 2.4 Hz, 1H), 2.51 (m, *J* = 13.3, 4.7 Hz, 1H), 2.24 - 2.09 (m, 3H), 2.02 (t, *J* = 6.8 Hz, 2H), 1.98 - 1.87 (m, 2H). LC-MS(ESI): $[M+H]^+$ = 370.39.

22) Synthesis of E3 Ligase Inhibitor 22

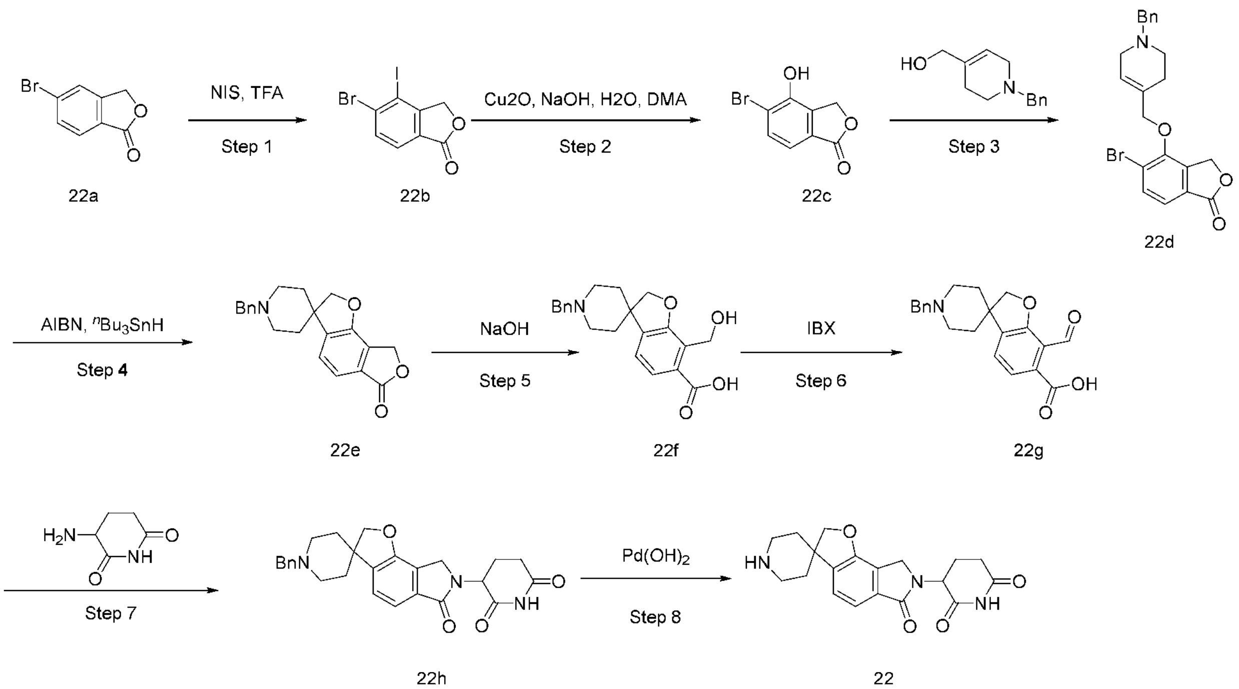

22a 22b 22c 22d 22e 22f 22g 22h 22

Step 1: 5-Bromo-4-iodoisobenzofuran-1(3H)-one (Compound 22b)

The compound 22a (1 g, 4.69 mmol) was dissolved in trifluoromethanesulfonic acid (10 mL) under nitrogen and cooled to 0°C. N-iodosuccinimide (1.16 g, 5.16 mmol) was added to the solution 0°C. The mixture was warmed to room temperature and stirred overnight. The resulting mixture was poured into ice water, filtered, and the filter cake was washed with water and purified by column chromatography (EA:PE = 0-15%) to afford a white solid compound 22b (800 mg, 50.28%).

$^1$H NMR (600 MHz, DMSO-*d*$_6$) δ 7.94 - 7.91 (m, 1H), 7.78 (dd, *J* = 8.1, 1.1 Hz, 1H), 5.21 (s, 2H). LC-MS(ESI): $[M-H]^-$ = 230.86.

Step 2: 5-Bromo-4-hydroxyisobenzofuran-1(3H)-one (Compound 22c)

A solution of sodium hydroxide (413.04 mg, 10.33 mmol) in water (7 mL) was added to a solution of compound 22b in N,N-dimethylaniline (3.5 mL), followed by adding the cuprous oxide (59.11 mg, 413.07 μmol). The mixture was heated to 80°C and stirred overnight. The resulting mixture was neutralized with 1N HCl, extracted with ethyl acetate, and washed with saturated brine. The organic phase was concentrated and purified by column chromatography (EA:PE = 0-35%) to afford a white solid, compound 22c (249 mg, 52.64%).

$^1$H NMR (600 MHz, DMSO-*d*$_6$) δ 10.91 (s, 1H), 7.73 (d, *J* = 8.0 Hz, 1H), 7.24 (d, *J* = 8.0 Hz, 1H), 5.35 (s, 2H). LC-MS(ESI): $[M-H]^-$ = 230.86.

Step 3: 4-((1-Benzyl-1,2,3,6-tetrahydropyridin-4-yl)methoxy)-5-bromoisobenzofuran-1(3H)-one (Compound 22d)

Under nitrogen atmosphere, a mixture of compound 22c (100 mg, 436.63 μmol), (1-benzyl-1,2,3,6-tetrahydropyridin-4-yl)methanol (106.51 mg, 523.95 μmol), triphenylphosphine (229.05 mg, 873.25 μmol), and diethyl azodicarboxylate (152.08 mg, 873.25 μmol) in tetrahydrofuran (1.0 mL) was stirred at 0°C and then warmed to room temperature overnight. The mixture was purified by preparative column chromatography to afford a yellow oil, compound 22d (105 mg, 58.05%).

$^1$H NMR (600 MHz, DMSO-*d*$_6$) δ 7.83 (d, *J* = 8.0 Hz, 1H), 7.45 (d, *J* = 8.0 Hz, 1H), 7.37 - 7.30 (m, 4H), 7.29 - 7.19 (m, 1H), 5.84 (s, 1H), 5.68 (s, 2H), 4.64 (s, 2H), 3.56 (s, 2H), 2.93 (s, 2H), 2.57 (t, *J* = 5.7 Hz, 2H), 2.25 (s, 2H). LC-MS(ESI): $[M+H]^+$ = 414.20.

Step 4: 1'-Benzyl-2H-spiro[benzo[2,1-b:3,4-c']difuran-3,4'-piperidine]-6(8H)-one (Compound 22e)

A mixture of compound 22d (100 mg, 241.37 μmol), azobisisobutyronitrile (79.27 mg, 482.74 μmol), and tributyltin hydride (210.77 mg, 724.11 μmol) in toluene (1.0 mL) was stirred at 110°C overnight. The solvent was removed under vacuum, and the residue was purified by C18 reversed-phase column chromatography to afford a yellow oil, compound 22e (27 mg, 33.35%).

$^1$H NMR (600 MHz, $CDCl_3$) δ 7.56 (d, *J* = 7.7 Hz, 1H), 7.52 - 7.46 (m, 6H), 5.26 (s, 2H), 4.54 (s, 2H), 4.29 (s, 2H), 2.73 (t, *J* = 13.1 Hz, 2H), 2.60 (td, *J* = 14.6, 4.0 Hz, 2H), 2.12 - 1.91 (m, 4H). LC-MS(ESI): $[M-H]^-$ = 336.30.

Step 5: 1'-Benzyl-7-(hydroxymethyl)-2H-spiro[benzofuran-3,4'-piperidine]-6-carboxylic acid (Compound 22f)

A mixture of compound 22e (202 mg, 602.26 μmol) and sodium hydroxide (120.44 mg, 3.01 mmol) in tetrahydrofuran (2.0 mL) and water (2.0 mL) was stirred at room temperature overnight. The solvent was removed under vacuum, and the residue was purified by reversed-phase column chromatography to afford a white solid, compound 22f (98 mg, 46.04%).

LC-MS(ESI): $[M-H]^-$ = 354.59.

Step 6: 1'-Benzyl-7-formyl-2H-spiro[benzofuran-3,4'-piperidine]-6-carboxylic acid (Compound 22g)

A mixture of compound 1f (20 mg, 56.59 μmol) and IBX (31.69 mg, 113.18 μmol) in tetrahydrofuran and dimethyl sulfoxide (20:1, 0.5 mL) was stirred at 80°C for 2 h. The mixture was filtered and concentrated under vacuum. The crude product was used directly in the next step without purification.

LC-MS(ESI): $[M+H]^+$ = 352.30.

Step 7: 3-(1'-Benzyl-6-oxo-6,8-dihydro-2H,7H-spiro[furo[2,3-e]isoindole-3,4'-piperidine]-7-yl)piperidine-2,6-dione (Compound 22h)

A mixture of compound 22g (48 mg, 136.60 μmol), 3-aminopiperidine-2,6-dione hydrochloride (44.96 mg, 273.19 μmol), and N,N-diisopropylethylamine (1 drop) in tetrahydrofuran and dimethyl sulfoxide (20:1, 1.0 mL) was stirred at 50°C for 1 h. Sodium triacetoxyborohydride (86.85 mg, 409.79 μmol) and acetic acid (1 drop) were added, and the mixture was stirred at 50°C for 1 h. The solvent was removed under vacuum, and the residue was purified by reversed-phase HPLC to afford a white solid, compound 22h (16 mg, 26.29%).

$^1$H NMR (600 MHz, DMSO-*d*$_6$) δ 11.00 (s, 1H), 7.46 - 7.56 (m, 5H), 7.34 (d, *J* = 7.6 Hz, 1H), 7.26 (d, *J* = 7.7 Hz, 1H), 5.09 (dd, *J* = 13.3, 5.1 Hz, 1H), 4.67 (t, *J* = 6.3 Hz, 2H), 4.43 - 4.34 (m, 3H), 4.24 (d, *J* = 17.1 Hz, 1H), 3.16 (d, *J* = 14.4 Hz, 2H), 2.91 (ddd, *J* = 18.0, 13.7, 5.4 Hz, 1H), 2.64 - 2.56 (m, 1H), 2.44 (td, *J* = 13.2, 4.6 Hz, 2H), 2.12 (d, *J* = 15.4 Hz, 2H), 2.03 - 1.89 (m, 4H). LC-MS(ESI): $[M+H]^+$ = 446.35.

Step 8: 3-(6-Oxo-6,8-dihydro-2H,7H-spiro[furo[2,3-e]isoindole-3,4'-piperidine]-7-yl)piperidine-2,6-dione (E3 Ligase Inhibitor 22)

The compound 22h (150 mg, 336.69 μmol) was dissolved in hexafluoroisopropanol (4.5 mL), then 20% palladium hydroxide on carbon (75 mg) was added. After purging with hydrogen three times, the mixture was stirred under a hydrogen atmosphere at room temperature for 5 h. The mixture was filtered, concentrated, and purified by reversed-phase HPLC to afford a white solid, E3 Ligase Inhibitor 22 (35 mg, 29.25%).

$^1$H NMR (600 MHz, $CD_3OD$) δ 7.46 - 7.40 (m, 2H), 5.16 (dd, J = 13.4, 5.2 Hz, 1H), 4.70 (s, 2H), 4.52 - 4.40 (m, 2H), 3.54 - 3.47 (m, 2H), 3.24 - 3.14 (m, 2H), 2.96 - 2.87 (m, 2H), 2.80 (m, J = 17.7, 4.6, 2.2 Hz, 1H), 2.52 (m, J = 13.3, 4.6 Hz, 1H), 2.25 - 2.16 (m, 3H), 2.05 - 2.01 (m, 1H). LC-MS(ESI): $[M+H]^+$ = 356.29.

23) Synthesis of E3 Ligase Inhibitor 23

Synthesis scheme:

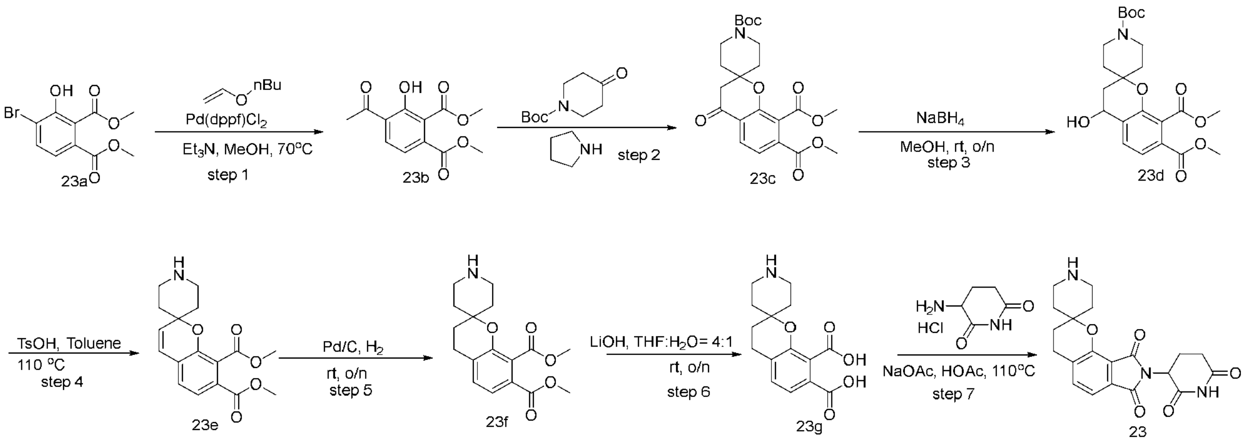


Step 1: Dimethyl 4-acetyl-3-hydroxyphthalate (Compound 23b)

Under a nitrogen atmosphere, a mixture of dimethyl 4-bromo-3-hydroxyphthalate (10 g, 34.59 mmol), **1-**(vinyloxy)butane (13.86 g, 138.87 mmol), Pd(dppf)$Cl_2$ (2.53 g, 3.46 mmol), and triethylamine (10.50 g, 103.78 mmol) in methanol (100 mL) was stirred at 70°C for 12 h. The reaction was quenched with HCl in 1,4-dioxane (100 mL), stirred for 30 min. The mixture was extracted with ethyl acetate, washed with saturated brine, dried over sodium sulfate, and concentrated to afford compound 23b as a yellow oil (8 g, 91.6%), which was used directly in the next step.

LC-MS(ESI):$[M-32+H]^+$= 211.06.

Step 2: 1'-(tert-Butyl) 7,8-dimethyl 4-oxospiro[chroman-2,4'-piperidine]-1',7,8-tricarboxylate (Compound 23c)

A mixture of compound 23b (8.0 g, 31.72 mmol), N-Boc-4-piperidone (7.72 g, 31.72 mmol), and pyrrolidine (2.26 g, 31.72 mmol) in ethanol (80 mL) was reacted at 85°C for 12 h. The solvent was removed, and the residue was purified by column chromatography (EA:PE = 29%) to afford a yellow oil, compound 23c (4.3 g, 31.28%).

$^1$H NMR (600 MHz, $CDCl_3$) δ 7.93 (d, $J$ = 8.2 Hz, 1H), 7.68 (d, $J$ = 8.2 Hz, 1H), 4.00 (s, 3H), 3.95 (s, 3H), 3.30 (t, $J$ = 9.7 Hz, 2H), 3.17 (t, $J$= 9.7 Hz, 2H), 3.14 (s, 2H), 2.47 (t, $J$ = 9.7 Hz, 2H), 2.06 (t, $J$ = 9.7 Hz, 2H). LC-MS(ESI):$[M+H]^+$= 334.19.

Step 3: 1'-(tert-Butyl) 7,8-dimethyl 4-hydroxyspiro[chroman-2,4'-piperidine]-1',7,8-tricarboxylate (Compound 23d)

The sodium borohydride (1.13 g, 29.76 mmol) was added to a solution of compound 23c (4.30 g, 9.92 mmol) in methanol (40 mL) under ice bath. The mixture was stirred overnight. The solvent was removed, and the residue was extracted with ethyl acetate to afford a yellow oily compound 23d (2.8 g, 64.81%), which was used directly in the next step.

LC-MS(ESI): $[M+H]^+$ = 336.29.

Step 4: Dimethyl spiro[chromene-2,4'-piperidine]-7,8-dicarboxylate (Compound 23e)

A mixture of compound 23d (2.80 g, 6.43 mmol) and p-toluenesulfonic acid (2.21 g, 12.86 mmol) in toluene (3 mL) was refluxed at 110°C overnight. The solvent was removed, and the residue was purified by column chromatography to afford a yellow oil, compound 23e (1.4 g, 68.6%).

LC-MS (ESI): $[M+H]^+$ = 318.38.

Step 5: Dimethyl spiro[chroman-2,4'-piperidine]-7,8-dicarboxylate (Compound 23f)

The compound 23e (1.40 g, 4.41 mmol) was dissolved in MeOH, and Pd/C (700 mg) was added. After purging with hydrogen, the mixture was stirred under a hydrogen atmosphere for 4 h. After the reaction was completed, the mixture was filtered and concentrated to afford a yellow oily compound 23f (1.3 g, 93.8%), which was used directly in the next step.

LC-MS (ESI): [M+H] $^+$ = 320.58.

Step 6: Spiro[chroman-2,4'-piperidine]-7,8-dicarboxylic acid (Compound 23g)

A mixture of compound 23f (1.4 g, 4.38 mmol) and lithium hydroxide (629.87 mg, 26.30 mmol) in THF:$H_2O$ (4:1) was stirred at room temperature overnight. The solvent was removed under reduced pressure to afford a yellow oil, compound 23g (1.35 g, 96.84%).

LC-MS (ESI): [M+H] $^+$ = 306.58.

Step 7: 8'-(2,6-Dioxopiperidin-3-yl)-3',4'-dihydro-7'H-spiro[piperidine-4,2'-pyrano[2,3-e]isoindole]-7',9'(8'H)-dione (E3 Ligase Inhibitor 23)

A mixture of compound 23g (1.50 g, 4.91 mmol), 3-aminopiperidine-2,6-dione hydrochloride (970.30 mg, 5.90 mmol), and sodium acetate (806.02 mg, 9.83 mmol) in acetic acid (5 mL) was stirred at 110°C for 5 h. The mixture was filtered, concentrated, and purified by preparative HPLC (Phase A = water, Phase B = ACN, 0.1% TFA, B% = 0-50% in 30 min) to afford a white solid, E3 Ligase Inhibitor 23 (0.55 g, 31.3%).

$^1$H NMR (600 MHz, DMSO-$d_6$) δ 11.11 (s, 1H), 7.62 (d, *J* = 7.5 Hz, 1H), 7.39 (d, *J* = 7.4 Hz, 1H), 5.08 (dd, *J* = 12.9, 5.5 Hz, 1H), 3.28 (t, *J* = 11.8 Hz, 2H), 3.09 (t, *J* = 11.8 Hz, 2H), 2.96 (t, *J* = 12,4 Hz, 2H), 2.91-2.78 (m, 3H), 2.63 - 2.52 (m, 2H), 2.05-1.94 (m, 3H), 1.91-1.81 (m, 2H). LC-MS(ESI): [M+H]$^+$ = 384.19.

24) Synthesis of E3 Ligase Inhibitor 24

Synthesis scheme:

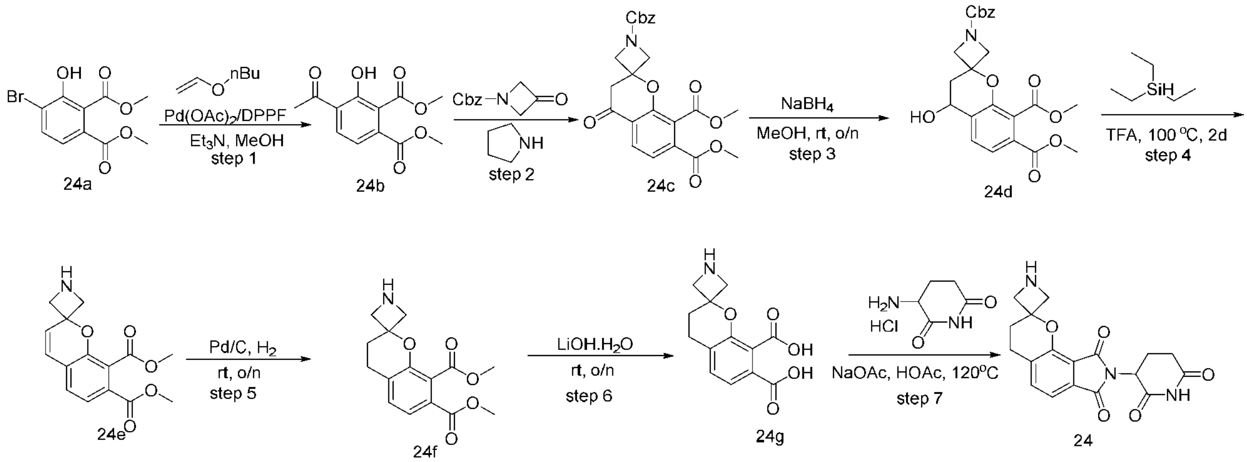


Step 1: Dimethyl 4-acetyl-3-hydroxyphthalate (Compound 24b)

Under a nitrogen atmosphere, dimethyl 4-bromo-3-hydroxyphthalate (2.40 g, 8.30 mmol), butyl vinyl ether (4.15 g, 41.5 mmol), [1,1'-bis(diphenylphosphino)ferrocene]dichloropalladium(II) (610 mg, 834.47 μmol), and triethylamine (2.53 g, 25.04 mmol) were dissolved in methanol. The mixture was stirred in an oil bath at 70 °C overnight. After completion, the resulting mixture was concentrated under reduced pressure. The crude product was purified by silica gel column chromatography (EA/PE = 28%) to afford a yellow oily compound 24b (418 mg, 20.3%).

$^1$H NMR (600 MHz, DMSO-$d_6$) δ 10.70 (s, 1H), 7.48 (d, *J* = 7.5 Hz, 1H), 7.12 (d, *J* = 7.4 Hz, 1H), 3.88 (s, 3H), 3.87 (s, 3H), 2.42 (s, 3H). LC-MS(ESI): [M-32+H]$^+$ = 221.16.

Step 2: 1-Benzyl 7',8'-dimethyl 4'-oxospiro[azetidine-3,2'-chroman]-1,7',8'-tricarboxylate (Compound 24c)

Compound 24b (325 mg, 1.58 mmol) was dissolved in methanol, followed by the addition of pyrrolidine (125 mg, 1.76 mmol). The mixture was stirred in an oil bath at 80 °C overnight. After completion, the resulting mixture was concentrated, and the crude product was purified by silica gel column chromatography (EA/PE = 30%) to afford compound 24c as a yellow oil (310 mg, 44.5%).

$^1H$ NMR (600 MHz, $CDCl_3$) δ 7.99 (dd, *J* = 8.2, 0.9 Hz, 1H), 7.71 (dd, *J* = 8.2, 1.0 Hz, 1H), 7.42 - 7.32 (m, 5H), 5.13 (s, 2H), 4.20 (d, *J* = 9.7 Hz, 2H), 4.06 (d, *J* = 9.7 Hz, 2H), 4.00 (s, 3H), 3.95 (s, 3H), 3.14 (s, 2H). LC-MS(ESI): $[M+H]^+$ = 438.20.

Step 3: 1-Benzyl 7',8'-dimethyl4'-hydroxyspiro[azetidine-3,2'-chroman]-1,7',8'-tricarboxylate (Compound 24d)

Compound 24c (310 mg, 705.48 μmol) was dissolved in methanol, and sodium borohydride (80 mg, 2.11 mmol) was added portionwise under ice-water bath conditions. The mixture was stirred at room temperature for 3 h. After completion, the resulting mixture was concentrated, and the crude product was purified by silica gel column chromatography (DCM/MeOH = 5%) to afford a yellow oily compound 24d (180 mg, 57%).

$^1H$ NMR (600 MHz, DMSO-$d_6$) δ 7.63 (d, *J* = 7.5 Hz, 1H), 7.44 (d, *J* = 7.0 Hz, 1H), 7.42 - 7.30 (m, 5H), 5.11 (s, 2H), 4.50 (s, 1H), 4.25 -4.15 (m, 1H), 4.10 (d, *J* = 9.7 Hz, 4H), 3.88 (s, 3H), 3.86 (s, 3H), 2.60 (d, *J* = 17.6, 2H). LC-MS(ESI):$[M+H]^+$ = 442.29.

Step 4: Dimethyl Spiro[azetidine-3,2'-chromene]-7',8'-dicarboxylate (Compound 24e)

Compound 24d (180 mg, 407.76 μmol) was dissolved in trifluoroacetic acid, followed by the addition of triethylsilane (330 mg, 2.84 mmol). The mixture was stirred at 100 °C for 6 h. After cooling to room temperature, another batch of triethylsilane (330 mg, 2.84 mmol) was added. The resulting mixture was concentrated, dissolved in methanol, stirred for 1 h, and concentrated again. The lower layer was collected as the product without further purification to afford a yellow oily compound 24e (160 mg, 96.6%), which was used directly in the next step.

LC-MS (ESI): $[M+H]^+$ = 289.98.

Step 5: Dimethyl Spiro[azetidine-3,2'-chroman]-7',8'-dicarboxylate (Compound 24f)

Under a nitrogen atmosphere, compound 24e (160 mg, 409.28 μmol) was dissolved in methanol, and Pd/C (50 mg, 10%) was added. The system was purged with hydrogen, and the mixture was stirred under a hydrogen atmosphere at room temperature overnight. After filtration, the filtrate was concentrated to afford a yellow oily compound 24f (130 mg, 81.3%), which was used directly in the next step without purification.

LC-MS (ESI): $[M+H]^+$ = 292.18.

Step 6: Spiro[azetidine-3,2'-chroman]-7',8'-dicarboxylic Acid (Compound 24g)

Compound 24f (130 mg, 410.57 μmol) was dissolved in a mixed solvent of THF/MeOH/$H_2O$ (1:1:5). Lithium hydroxide (150 mg, 6.26 mmol) was added under ice-water bath condition, and the mixture was stirred at room temperature overnight. The pH was adjusted to 7 with hydrochloric acid, and the filtrate was concentrated to afford a yellow oily compound 24g (140 mg, 105%), which was used directly in the next step without purification.

LC-MS (ESI): $[M+H]^+$ = 264.17.

Step 7: 8'-(2,6-Dioxopiperidin-3-yl)-3',4'-dihydro-7'H-spiro[azetidine-3,2'-pyrano[2,3-e]isoindole]-7',9'(8'H)-dione (E3 Ligase Inhibitor 24)

Compound 24g (200 mg, 1.22 mmol) and sodium acetate (200 mg, 2.44 mmol) were dissolved in acetic acid and stirred at 120 °C overnight. After concentration, the residue was dissolved in methanol and purified by reversed-phase chromatography (ACN/$H_2O$, 0-20%, 40 min) to afford a white solid E3 ligase inhibitor 24 (80 mg, 18.4%).

$^1H$ NMR (600 MHz, DMSO-$d_6$) δ 11.13 (s, 1H), 7.63 (d, *J* = 7.5 Hz, 1H), 7.44 (d, *J* = 7.0 Hz, 1H), 5.09 (dd, *J* = 12.9, 5.5 Hz, 1H), 4.15 (s, 4H), 2.96 (t, *J* = 6.5 Hz, 2H), 2.94-2.87(m, 1H), 2.60-2.54 (m, 2H), 2.31 - 2.20 (m, 2H), 2.03-1.94 (m, 1H). LC-MS(ESI): $[M+H]^+$ = 356.30.

25) Synthesis of E3 Ligase Inhibitor 25

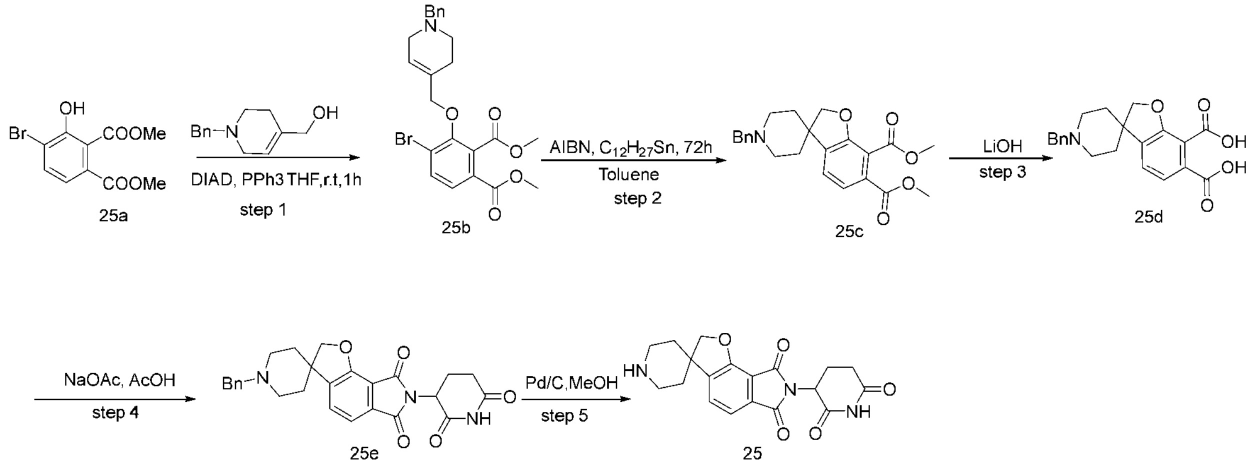


25a 25b 25c 25d 25e 25

Step 1: Dimethyl 3-((1-benzyl-1,2,3,6-tetrahydropyridin-4-yl)methoxy)-4-bromophthalate (Compound 25b)

Under a nitrogen atmosphere, 4-bromo-3-hydroxyphthalic acid dimethyl ester (1.80 g, 6.23 mmol) was dissolved in tetrahydrofuran (10 mL). (1-Benzyl-1,2,3,6-tetrahydropyridin-4-yl)methanol (1.90 g, 9.34 mmol) and triphenylphosphine (3.27 g, 12.45 mmol) were added, followed by slow dropwise addition of diisopropyl azodicarboxylate (2.52 g, 12.45 mmol). After the addition is completed, the mixture was stirred at room temperature for 2 h. After completion, the solvent was removed under vacuum, and the crude product was purified by column chromatography ($H_2O$:ACN = 0-30%) to afford a yellow oily target compound (2.10 g, 71.10%).

$^1H$ NMR (600 MHz, DMSO-$d_6$) δ 7.95 (d, *J* = 8.4 Hz, 1H), 7.70 (d, *J* = 8.4 Hz, 1H), 7.58 - 7.45 (m, 5H), 5.87 - 5.83 (m, 1H), 4.45 (s, 2H), 3.84 (s, 3H), 3.83 (s, 3H), 3.81 (s, 2H), 3.47 - 3.30 (m, 2H), 3.25 - 3.10 (m, 2H), 2.47 - 2.34 (m, 2H). LC-MS(ESI): $[M+H]^+$ = 474.09.

Step 2: Dimethyl 1'-benzyl-2H-spiro[benzofuran-3,4'-piperidine]-6,7-dicarboxylate (Compound 25c)

Under a nitrogen atmosphere, compound 25b (2.10 g, 4.43 mmol) was dissolved in toluene (25 mL), and azobisisobutyronitrile (2.18 g, 13.28 mmol) and tri-n-butyltin hydride (2.58 g, 8.85 mmol) were added. The mixture was stirred at 110°C for 4 h. After completion, the solvent was removed under vacuum, and the crude product was purified by column chromatography ($H_2O$:ACN = 0-30%) to afford the target compound as a yellow oil (1.50 g, 85.68%).

$^1H$ NMR (600 MHz, DMSO-$d_6$) δ 7.55-7.47 (m, 5H), 7.46 (d, *J* = 8.4 Hz, 1H), 7.31 (d, *J* = 8.4 Hz, 1H), 4.65 (s, 2H), 4.35 (s, 2H), 3.80 (s, 3H), 3.78 (s, 3H), 3.43 - 3.30 (m, 2H), 3.15 - 3.06 (m, 2H), 2.17 - 2.07 (m, 2H), 2.01 - 1.94 (m, 2H). LC-MS(ESI): $[M+H]^+$=360.29.

Step 3: 1'-Benzyl-2H-spiro[benzofuran-3,4'-piperidine]-6,7-dicarboxylic acid (Compound 25d)

Compound 25c (1.50 g, 3.79 mmol) was dissolved in a mixed solution of methanol and water (3:1, 15 mL), and lithium hydroxide (454.17 mg, 18.97 mmol) was added. The mixture was stirred at 50°C for 2 h. After completion, the filtrate was concentrated to afford a yellow solid crude product (1.35 g), which was used directly in the next step without further purification.

LC-MS (ESI): $[M+H]^+$ = 367.99.

Step 4: 1'-Benzyl-7-(2,6-dioxopiperidin-3-yl)-7H-2H,6H-spiro[furo[2,3-e]isoindole-3,4'-piperidine]-6,8-dione (Compound 25e)

Compound 25d (1.35 g, 3.67 mmol) was dissolved in acetic acid (12 mL), and 3-aminopiperidine-2,6-dione hydrochloride (1.21 g, 7.35 mmol) and sodium acetate (1.21 g, 14.70 mmol) were added. The mixture was stirred at 115°C overnight. After completion, the mixture was concentrated under vacuum, and the crude product was purified by column chromatography ($H_2O$:ACN = 0-30%) to afford the target compound as a purple oil (1.52 g, 90.02%).

$^1H$ NMR (600 MHz, DMSO-$d_6$) δ 11.10 (s, 1H), 7.62 (d, *J* = 7.4 Hz, 1H), 7.55-7.47 (m, 5H), 7.43 (d, *J* = 7.3 Hz, 1H), 5.11 (dd, *J* = 13.0, 5.4 Hz, 1H), 4.81 (s, 2H), 3.96 (s, 2H), 3.38 (d, *J* = 13.0 Hz, 2H), 3.09 - 3.01 (m, 2H), 2.91 - 2.84 (m, 1H), 2.63-2.58 (m, 2H), 2.08 - 2.00 (m, 3H), 1.98 - 1.90 (m, 2H). LC-MS(ESI): $[M+H]^+$=460.49.

Step 5: 7-(2,6-Dioxopiperidin-3-yl)-7H-2H,6H-spiro[furo[2,3-e]isoindole-3,4'-piperidine]-6,8-dione (E3 Ligase Inhibitor 25)

Compound 25e (489 mg, 1.06 mmol) was dissolved in methanol, and palladium on carbon (180 mg) was added. The system was purged with hydrogen two to three times. The mixture was stirred at room temperature for 2 h. After completion, the mixture was filtered through diatomaceous earth, and the organic phase was dried and concentrated. The crude product was purified by column chromatography ($H_2O$:ACN = 15-55%) to afford E3 ligase inhibitor 25 (170 mg, 43.25%).

$^1$H NMR (600 MHz, DMSO-$d_6$) δ 11.12 (s, 1H), 7.61 (d, *J* = 7.4 Hz, 1H), 7.49 (d, *J* = 7.3 Hz, 1H), 5.11 (dd, *J* = 13.0, 5.4 Hz, 1H), 4.81 (s, 2H), 3.38 (d, *J*= 13.0 Hz, 2H), 3.09 - 3.00 (m, 2H), 2.92 - 2.85 (m, 1H), 2.62-2.59 (m, 2H), 2.09 - 2.01 (m, 3H), 1.98 - 1.91 (m, 2H). LC-MS(ESI): $[M+H]^+$=370.09.

26) Synthesis of E3 Ligase Inhibitor 26

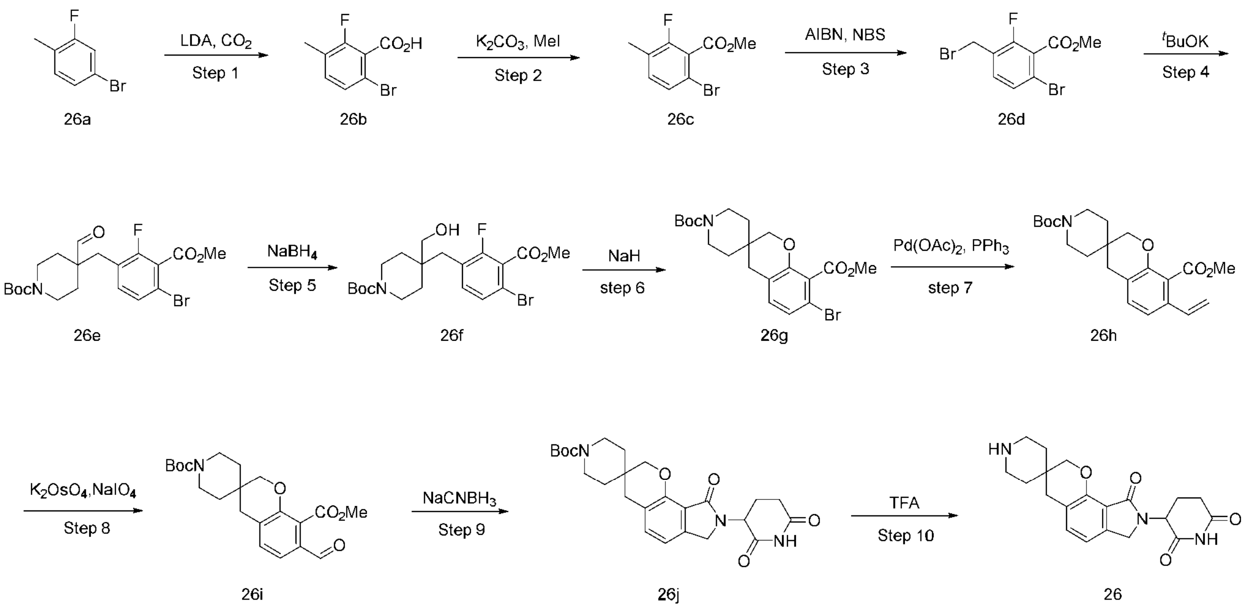


Step 1: 6-Bromo-2-fluoro-3-methylbenzoic acid (Compound 26b)

At -78°C, compound 26a (2.0 g, 10.6 mmol) was added to a solution of lithium diisopropylamide (1.3 M in THF/hexane, 6.9 mL, 13 mmol) in tetrahydrofuran (10.6 mL). The reaction was maintained at this temperature for 2 h and then quickly poured into dry ice. After warming to room temperature, the solvent was evaporated to afford compound 26b as a white solid (2.84 g, 115%), which was used directly in the next step.

LC-MS (ESI): $[M-H]^-$ = 230.95.

Step 2: Methyl 6-bromo-2-fluoro-3-methylbenzoate (Compound 26c)

The iodomethane (2.5 g, 17.8 mmol) and potassium carbonate (3.7 g, 26.7 mmol) were added to a solution of compound 26b (2.1 g, 8.9 mmol) in N,N-dimethylformamide (12 mL). The mixture was stirred at 80°C for 1 h. After completion, water (50 mL) was added, and the mixture was extracted with ethyl acetate (100 mL × 3). The combined organic phases were washed with saturated brine (100 mL), dried over anhydrous sodium sulfate, and concentrated. The crude product was purified by column chromatography (PE:EA = 0-20%) to afford a yellow oily compound 26c (1.9 g, 86%).

$^1$H NMR (600 MHz, $CDCl_3$) δ 7.27 (d, *J* = 9.2 Hz, 1H), 7.14 - 7.09 (m, 1H), 3.97 (s, 3H), 2.25 (d, *J* = 1.9 Hz, 3H). LC-MS(ESI): $[M+H]^+$ = 247.66.

Step 3: Methyl 6-bromo-3-(bromomethyl)-2-fluorobenzoate (Compound 26d)

Under a nitrogen atmosphere, N-bromosuccinimide (1.6 g, 9.2 mmol) and azobisisobutyronitrile (62.8 mg, 0.38

mmol) were added to a solution of compound 26c (1.9 g, 7.6 mmol) in carbon tetrachloride (98 mL). The mixture was stirred at 85°C overnight. The solvent was evaporated, and the crude product was purified by column chromatography (PE:EA = 0-30%) to afford a yellow oily compound 26d (219.9 mg, 8%).

$^1$H NMR (600 MHz, $CDCl_3$) δ 7.39 (d, *J* = 8.4 Hz, 1H), 7.35 - 7.31 (m, 1H), 4.45 (s, 2H), 3.98 (s, 3H).

Step 4: tert-Butyl 4-(4-bromo-2-fluoro-3-(methoxycarbonyl)benzyl)-4-formylpiperidine-1-carboxylate (Compound 26e)

Under a nitrogen atmosphere, at -30°C, potassium tert-butoxide (75.7 mg, 0.67 mmol) was added to a solution of 1-(tert-butoxycarbonyl)piperidine-4-carbaldehyde (158.4 mg, 0.74 mmol) in tetrahydrofuran (4 mL). The mixture was stirred at -30°C for 1 h, and then a solution of compound 26d (219.9 mg, 0.67 mmol) in tetrahydrofuran (3 mL) was slowly added. The reaction was placed at -30°C for 2 h and then allowed to warm to room temperature overnight. The solvent was evaporated, and the crude product was purified by column chromatography (PE:EA = 0-40%) to afford a colorless oily compound 26e (147.6 mg, 48%).

$^1$H NMR (600 MHz, $CDCl_3$) δ 9.55 (s, 1H), 7.30 (d, *J* = 8.3 Hz, 1H), 7.00 - 6.95 (m, 1H), 3.96 (s, 3H), 4.00 - 3.75 (m, 2H), 2.95 - 2.70 (m, 2H), 2.77 (s, 2H), 1.94 (d, *J* = 13.0 Hz, 2H), 1.58 - 1.46 (m, 2H), 1.43 (s, 9H). LC-MS(ESI): $[M\text{-}Boc+H]^+$ = 358.19.

Step 5: tert-Butyl 4-(4-bromo-2-fluoro-3-(methoxycarbonyl)benzyl)-4-(hydroxymethyl)piperidine-1-carboxylate (Compound 26f)

At 0°C, sodium borohydride (1.9 g, 51.4 mmol) was added in portions to a solution of compound 26e (4.7 g, 10.3 mmol) in methanol (50 mL). The mixture was stirred at room temperature for 2 h. Water was added slowly to quench the reaction, and the solvent was evaporated. The crude product was purified by column chromatography (PE:EA = 0-50%) to afford compound 26f as a colorless oil (4.14 g, 88% yield).

$^1$H NMR (600 MHz, DMSO-$d_6$) δ 7.51 (d, *J* = 8.3 Hz, 1H), 7.41 - 7.36 (m, 1H), 4.78 (t, *J* = 5.0 Hz, 1H), 3.90 (s, 3H), 3.46 - 3.39 (m, 2H), 3.20 (d, *J* = 4.5 Hz, 4H), 2.66 (s, 2H), 1.99 (s, 2H), 1.42 - 1.32 (m, 11H). LC-MS(ESI): $[M\text{-}Boc+H]^+$ = 360.29.

Step 6: 1'-(tert-Butyl) 8-methyl 7-bromospiro[chroman-3,4'-piperidine]-1',8-dicarboxylate (Compound 26g)

Under nitrogen atmosphere, compound 26f (125 mg, 0.27 mmol) was added slowly to a solution of sodium hydride (11.9 mg, 0.27 mmol, 60% dispersion in mineral oil) in N,N-dimethylformamide (1 mL). The mixture was heated to 110°C for 1 h. After cooling to room temperature, water (2 mL) was added to quench the reaction. Water (10 mL) was added, and the resulting mixture was extracted with ethyl acetate (20 mL × 3), washed with saturated brine (40 mL), and the combined organic phases were purified by column chromatography (PE:EA = 0-40%) to afford a white solid compound 26g (73.0 mg, 61%).

$^1$H NMR (600 MHz, $CDCl_3$) δ 7.03 (d, *J* = 8.2 Hz, 1H), 6.92 (d, *J* = 8.2 Hz, 1H), 3.92 (s, 3H), 3.90 (s, 2H), 3.59-3.47 (m, 2H), 3.37 - 3.28 (m, 2H), 2.61 (s, 2H), 1.50 - 1.40 (m, 13H). LC-MS(ESI): $[M\text{-}Boc+H]^+$ = 339.99.

Step 7: 1'-(tert-Butyl) 8-methyl 7-vinylspiro[chroman-3,4'-piperidine]-1',8-dicarboxylate (Compound 26h)

Under nitrogen atmosphere, compound 26g (73.0 mg, 0.17 mmol), potassium vinyltrifluoroborate (33.5 mg, 0.25 mmol), palladium acetate (7.5 mg, 0.033 mmol), triphenylphosphine (6.1 mmol, 0.023 mmol), and cesium carbonate (108.2 mg, 0.33 mmol) were added to a mixed solvent of tetrahydrofuran and water (10:1, 1.1 mL). The reaction was carried out in oil bath at 85°C overnight. The solvent was evaporated, and the crude product was purified by column chromatography (PE:EA = 0-40%) to afford a white solid compound 26h (38.9 mg, 45%).

$^1$H NMR (600 MHz, $CDCl_3$) δ 7.08 (d, *J* = 7.9 Hz, 1H), 7.04 (d, *J* = 7.8 Hz, 1H), 6.63 (dd, *J* = 17.4, 11.0 Hz, 1H), 5.69 (d, *J* = 17.4 Hz, 1H), 5.28 (d, *J* = 11.0 Hz, 1H), 3.96 - 3.88 (m, 5H), 3.60 - 3.50 (m, 2H), 3.38 - 3.29 (m, 2H), 2.67 (s, 2H), 1.52 - 1.42 (m, 13H). LC-MS(ESI): $[M\text{-}Boc+H]^+$ = 288.18.

Step 8: 1'-(tert-Butyl) 8-methyl 7-formylspiro[chroman-3,4'-piperidine]-1',8-dicarboxylate (Compound 26i)

The sodium periodate (64.9 mg, 0.3 mmol) and osmium tetroxide dihydrate (1.4 mg, 0.003 mmol) were added to a mixed solution of compound 26h (29.4 mg, 0.076 mmol) in acetone and water (5:1, 2.7 mL). The mixture was stirred at room temperature overnight. The solvent was evaporated, and the crude product was purified by column chromatography (PE:EA = 0-40%) to afford a white solid compound 26i (8.9 mg, 30%).

$^1$H NMR (600 MHz, $CDCl_3$) δ 9.89 (s, 1H), 7.37 (d, *J* = 7.7 Hz, 1H), 7.25 (d, *J* = 6.4 Hz, 1H), 4.00 - 3.94 (m, 5H), 3.59 - 3.48 (m, 2H), 3.42 - 3.33 (m, 2H), 2.75 (s, 2H), 1.54 - 1.48 (m, 2H), 1.48 - 1.41 (m, 11H). LC-MS(ESI): $[M\text{-}Boc+H]^+$ =

290.18.

Step 9: tert-butyl 8'-(2,6-dioxopiperidin-3-yl)-9'-oxo-4',7',8',9'-tetrahydro-2'H-spiro[piperidine-4,3'-pyrano[2,3-e]isoindole]-1-carboxylate (Compound 26j)

Compound 26i (500 mg, 1.3 mmol) and 3-amino-2,6-piperidinedione hydrochloride (316 mg, 1.9 mmol) were mixed in a solvent of dichloromethane and methanol (1:2, 39 mL). After stirring at room temperature for 1 h, sodium cyanoborohydride (241 mg, 3.8 mmol) was added, and the mixture was stirred at room temperature overnight. The solvent was evaporated, and the mixture was purified by reversed-phase column chromatography to afford a white solid compound 26j (233 mg, 39%).

$^1$H NMR (600 MHz, DMSO-$d_6$) δ 10.95 (s, 1H), 7.28 (d, *J* = 7.6 Hz, 1H), 7.00 (d, *J* = 7.6 Hz, 1H), 5.02 (dd, *J* = 13.3, 5.1 Hz, 1H), 4.30 (d, *J* = 17.2 Hz, 1H), 4.17 (d, *J* = 17.1 Hz, 1H), 4.01 (s, 2H), 3.50 - 3.43 (m, 2H), 3.30 - 3.25 (m, 2H), 2.94 - 2.87 (m, 1H), 2.73 (s, 2H), 2.60 - 2.55 (m, 1H), 2.38 - 2.30 (m, 1H), 1.99 - 1.91 (m, 1H), 1.45 - 1.35 (m, 11H), 1.35 - 1.29 (m, 2H). LC-MS(ESI): $[M+H]^+$ = 470.40.

Step 10: 3-(9'-Oxo-7',9'-dihydro-2'H-spiro[piperidine-4,3'-pyrano[2,3-e]isoindol]-8'(4'H)-yl)piperidine-2,6-dione (E3 Ligase Inhibitor 26)

Trifluoroacetic acid (2 mL) was added to a solution of compound 26j (230 mg, 0.49 mmol) in dichloromethane (5 mL). The mixture was stirred at room temperature for 1.5h. The solvent was evaporated, and the mixture was purified by preparative chromatography to afford a white solid E3 ligase inhibitor 26 (151.2 mg, 84%).

$^1$H NMR (600 MHz, DMSO-$d_6$) δ 10.96 (s, 1H), 7.30 (d, *J* = 7.6 Hz, 1H), 7.04 (d, *J* = 7.4 Hz, 1H), 5.01 (dd, *J* = 13.2, 4.9 Hz, 1H), 4.31 *(d, J* = 17.1 Hz, 1H), 4.18 (d, *J* = 17.2 Hz, 1H), 4.11 - 4.04 (m, 2H), 3.20 - 3.06 (m, 4H), 2.94 - 2.86 (m, 1H), 2.77 (s, 2H), 2.60 - 2.53 m, 1H), 2.39 - 2.30 (m, 1H), 1.98 - 1.91 (m, 1H), 1.69 - 1.53 (m, 4H). LC-MS(ESI): $[M+H]^+$ = 370.29.

27) Synthesis of E3 Ligase Inhibitor 27

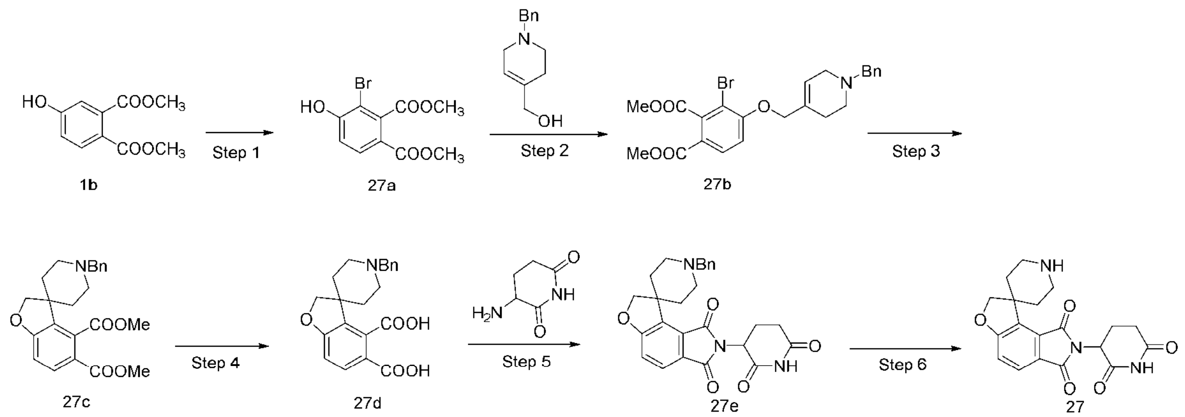


Step 1: Dimethyl 3-bromo-4-hydroxyphthalate (Compound 27a)

Compound 1b (33.6 g, 160 mmol, 1.0 eq) was dissolved in trifluoroacetic acid (100 mL), and N-bromosuccinimide (34.2 g, 192 mmol, 1.2 eq) was added. The mixture was stirred at room temperature overnight. The mixture was concentrated under reduced pressure, and water (100 mL) was added, followed by extraction with ethyl acetate (100 mL × 3). The organic phase was washed with saturated brine (100 mL), dried over anhydrous sodium sulfate, and concentrated. The crude product was purified by reversed-phase column chromatography to afford compound 27a as a pale yellow solid (9.8 g, 21.3%).

$^1$H NMR (600 MHz, DMSO-$d_6$) δ 11.68 (s, 1H), 7.87 (d, *J* = 12.0 Hz, 1H), 7.10 (d, *J* = 12.0 Hz, 1H), 3.84 (s, 3H), 3.79 (s, 3H). LC-MS (ESI): $[M-H]^-$ = 287.09.

Step 2: Dimethyl 4-((1-benzyl-1,2,3,6-tetrahydropyridin-4-yl)methoxy)-3-bromophthalate (Compound 27b)

Compound 27a (9.8 g, 33.9 mmol, 1.0 eq), (1-benzyl-1,2,3,6-tetrahydropyridin-4-yl)methanol (10.3 g, 50.8

mmol, 1.5 eq), and triphenylphosphine (33.3 g, 127 mmol, 2.5 eq) were dissolved in tetrahydrofuran (80 mL). Diethyl azodicarboxylate (22.1 g, 127 mmol, 2.5 eq) was added, and the mixture was stirred at room temperature for 4 h. The mixture was concentrated under reduced pressure, and water (50 mL) was added, followed by extraction with ethyl acetate (100 mL × 3). The organic phase was washed with saturated brine (100 mL), dried over anhydrous sodium sulfate, and concentrated. The crude product was purified by column chromatography to afford a reddish-brown solid compound 27b (8.7 g, 51.3%).

$^1H$ NMR (600 MHz, DMSO-$d_6$) δ 8.00 (d, *J* = 8.8 Hz, 1H), 7.40 - 7.29 (m, 5H), 7.27 - 7.24(m, 1H), 5.84 (tt, *J* = 3.4, 1.6 Hz, 1H), 4.68 (s, 2H), 3.86 (s, 3H), 3.81 (s, 3H), 3.54 (s, 2H), 2.93 - 2.92 (m, 2H), 2.54 (t, *J* = 5.7 Hz, 2H), 2.16 (s, 2H). LC-MS (ESI): $[M+H]^+$ = 474.09.

Step 3: Dimethyl 1'-benzyl-2H-spiro[benzofuran-3,4'-piperidine]-4,5-dicarboxylate (Compound 27c)

Compound 27b (8.7 g, 18.3 mmol, 1.0 eq), tri-n-butyltin hydride (10.6 g, 36.6 mmol, 2.0 eq), and azobisisobutyronitrile (0.6 g, 3.7 mmol, 0.2 eq) were dissolved in toluene (84 mL). Under nitrogen, the mixture was stirred at 110°C for 4 h. After cooling to room temperature, the mixture was concentrated under reduced pressure, and water (100 mL) was added, followed by extraction with ethyl acetate (50 mL × 3). The organic phase was washed with saturated brine (100 mL), dried over anhydrous sodium sulfate, and concentrated. The crude product was purified by column chromatography to afford a reddish-brown oily compound 27c (4.7 g, 64.8%).

$^1H$ NMR (600 MHz, DMSO-$d_6$) δ 7.84 (d, *J* = 8.5 Hz, 1H), 7.38 -7.29 (m, 4H), 7.29 - 7.23 (m, 1H), 7.00 (d, *J* = 8.5 Hz, 1H), 4.52 (s, 2H), 3.86 (s, 3H), 3.78 (s, 3H), 3.48 (s, 2H), 2.75 (dd, *J* = 11.6, 3.7 Hz, 2H), 2.08 ~ 1.93 (m, 4H), 1.58 (d, *J* = 12.2 Hz, 2H). LC-MS (ESI): $[M+H]^+$ = 395.71

Step 4: 1'-Benzyl-2H-spiro[benzofuran-3,4'-piperidine]-4,5-dicarboxylic acid (Compound 27d)

Compound 27c (4.7 g, 11.9 mmol, 1.0 eq) was dissolved in a mixture of methanol (10 mL) and water (10 mL), and potassium hydroxide (33.3 g, 594 mmol, 50 eq) was added. The mixture was stirred at 90°C for 12 h. The mixture was concentrated under reduced pressure, and the crude product was purified by column chromatography to afford a white solid compound 27d (4.3 g, 92.1%).

$^1H$ NMR (400 MHz, DMSO-$d_6$) δ 7.81 (d, *J* = 8.5 Hz, 1H), 7.60 -7.65 (m, 2H), 7.44 - 7.39 (m, 3H), 6.94 (d, *J* = 8.5 Hz, 1H), 4.61 (s, 2H), 4.25 (s, 2H), 3.16 (s, 4H), 2.39 (s, 2H), 1.78 (d, *J* = 12.2 Hz, 2H). LC-MS (ESI) $[M+H]^+$ = 368.30.

Step 5: 1'-Benzyl-2-(2,6-dioxopiperidin-3-yl)-2-hydro-1H,7H-spiro[furo[3,2-e]isoindole-8,4'-piperidine]-1,3-dione (Compound 27e)

Compound 27d (4.3 g, 11.7 mmol, 1.0 eq), sodium acetate (3.8 g, 46.8 mmol, 4.0 eq), and 3-amino-2,6-piperidinedione hydrochloride (2.99 g, 23.4 mmol, 2.0 eq) were dissolved in acetic acid (30 mL). The mixture was stirred at 110°C for 12 h. After cooling to room temperature, the mixture was concentrated under reduced pressure, and water (50 mL) was added, followed by extraction with ethyl acetate (50 mL × 3). The organic phase was washed with saturated brine (80 mL), dried over anhydrous sodium sulfate, and concentrated. The crude product was purified by column chromatography to afford a white solid compound 27e (2.5 g, 47.2%).

$^1H$ NMR (400 MHz, $CD_3OD$) δ 7.79 (d, *J* = 8.2 Hz, 1H), 7.69 - 7.44 (m, 5H), 7.19 (d, *J* = 8.1 Hz, 1H), 5.13 (dd, *J* = 12.6, 5.4 Hz, 1H), 4.80 (s, 2H), 4.38 (s, 2H), 3.55 (d, *J* = 13.0 Hz, 2H), 3.35 - 3.36 (m, 1H), 3.17 (t, *J* = 13.4 Hz, 2H), 2.97 - 2.85 (m, 2H), 2.81 - 2.65 (m, 2H), 2.17 - 1.98 (m, 3H). LC-MS (ESI) $[M+H]^+$ = 460.29.

Step 6: 2-(2,6-Dioxopiperidin-3-yl)-2-hydro-1H,7H-spiro[furo[3,2-e]isoindole-8,4'-piperidine]-1,3-dione (E3 Ligase Inhibitor 27)

Compound 27e (50 mg, 1.1 mmol, 1.0 eq) was dissolved in methanol (50 mL), and palladium hydroxide on carbon (14 mg, 0.1 mmol, 0.1 eq) was added. The system was purged with hydrogen three times, and the mixture was stirred at room temperature for 2 h. The mixture was filtered, and the filter cake was washed with methanol (10 mL×3). The filtrate was concentrated under reduced pressure and the crude product was purified by reversed-phase column chromatography to afford a white solid E3 ligase inhibitor 27 (16 mg, 26.2%).

$^1H$ NMR (600 MHz, $CD_3OD$) δ 7.79 (dd, *J* = 8.2, 1.2 Hz, 1H), 7.19 (dd, *J* = 8.1, 1.3 Hz, 1H), 5.15 (dd, *J* = 12.9, 5.4 Hz, 1H), 4.78 (s, 2H), 3.49 (dt, *J* = 13.3, 2.7 Hz, 2H), 3.14 (td, *J* = 13.7, 2.8 Hz, 2H), 2.94 - 2.87 (m, 3H), 2.80 - 2.70 (m, 2H), 2.18 - 2.14 (m, 1H), 2.04 (d, *J* = 14.3 Hz, 2H). LC-MS (ESI) $[M+H]^+$ = 370.39.

28) Synthesis of E3 Ligase Inhibitor 28

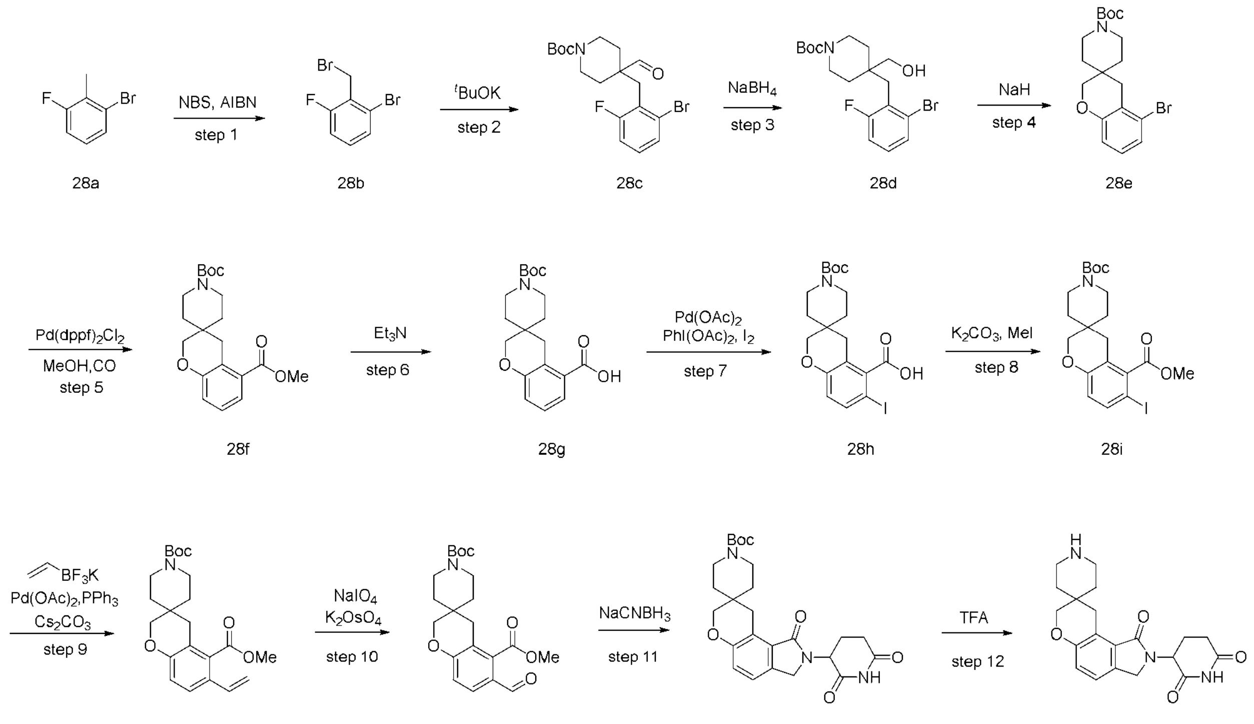


28a, 28b, 28c, 28d, 28e, 28f, 28g, 28h, 28i, 28j, 28k, 28l, 28

Step 1: 1-Bromo-2-(bromomethyl)-3-fluorobenzene (Compound 28b)

Under nitrogen, N-bromosuccinimide (1.2 g, 6.9 mmol) and azobisisobutyronitrile (173.7 mg, 1.1 mmol) were added to a solution of compound 28a (1 g, 5.3 mmol) in carbon tetrachloride (20 mL). The mixture was stirred at 80°C overnight. The solvent was evaporated, and the residue was purified by column chromatography (PE:EA = 0-30%) to afford a colorless oily compound 28b (1.3 g, 90%).

$^1H$ NMR (600 MHz, $CDCl_3$) δ 7.39 (d, *J* = 8.1 Hz, 1H), 7.21 - 7.15 (m, 1H), 7.08 - 7.03 (m, 1H), 4.65 (d, *J* = 1.7 Hz, 2H).

Step 2: tert-Butyl 4-(2-bromo-6-fluorobenzyl)-4-formylpiperidine-1-carboxylate (Compound 28c)

Under nitrogen, at -30°C, potassium tert-butoxide (592.5 mg, 5.3 mmol) was added to a solution of tert-butyl 4-formylpiperidine-1-carboxylate (1.3 g, 4.8 mmol) in tetrahydrofuran (10 mL). After stirring at -30°C for 1 h, a solution of compound 28b (1.3 g, 4.8 mmol) in tetrahydrofuran (10 mL) was slowly added. The mixture was stirred at -30°C for 2 h and then at room temperature overnight. The solvent was evaporated, and the residue was purified by column chromatography (PE:EA = 0-20%) to afford a colorless oily compound 28c (710.7 mg, 37%).

$^1H$ NMR (600 MHz, $CDCl_3$) δ 9.65 (d, *J* = 2.9 Hz, 1H), 7.37 (d, *J* = 8.0 Hz, 1H), 7.14 - 7.08 (m, 1H), 7.04 - 6.98 (m, 1H), 4.15 - 3.70 (m, 2H), 3.02 (d, *J* = 2.4 Hz, 2H), 2.90 - 2.60 (m, 2H), 2.18 - 1.94 (m, 2H), 1.76 - 1.62 (m, 2H), 1.44 (s, 9H). LC-MS(ESI): $[M-Boc+H]^+$ = 300.06.

Step 3: tert-Butyl 4-(2-bromo-6-fluorobenzyl)-4-(hydroxymethyl)piperidine-1-carboxylate (Compound 28d)

At 0°C, sodium borohydride (1.9 g, 51.4 mmol) was added portionwise to a solution of compound 28c (4.7 g, 10.3 mmol) in methanol (50 mL). The mixture was stirred at room temperature for 2 h. Water was added to quench the reaction, and the mixture was concentrated. The residue was purified by column chromatography (PE:EA = 0-50%) to afford a colorless oily compound 28d (4.14 g, 88% yield).

$^1H$ NMR (600 MHz, $CDCl_3$) δ 7.39 (d, *J* = 8.0 Hz, 1H), 7.12 - 7.07 (m, 1H), 7.05 - 6.99 (m, 1H), 3.80 - 3.65 (m, 2H), 3.62 (d, *J* = 6.2 Hz, 2H), 3.11 (t, *J* = 11.0 Hz, 2H), 2.94 (d, *J* = 2.6 Hz, 2H), 1.67 - 1.62 (m, 2H), 1.62 - 1.59 (m, 1H), 1.55 - 1.47 (m, 2H), 1.44 (s, 9H). LC-MS(ESI): $[M-Boc+H]^+$ = 301.98.

Step 4: tert-Butyl 5-bromospiro[chroman-3,4'-piperidine]-1'-carboxylate (Compound 28e)

Under nitrogen, compound 28d (659.4 mg, 1.64 mmol) was slowly added to a suspension of sodium hydride (72.1 mg, 1.8 mmol, 60% dispersion in mineral oil) in N,N-dimethylformamide (7 mL). The mixture was stirred at 110°C for 1 h. After cooling to room temperature, water (10 mL) was added to quench the reaction. The mixture was extracted with ethyl acetate (40 mL × 3), washed with saturated brine (80 mL), and dried over anhydrous sodium sulfate. The solvent was evaporated, and the residue was purified by column chromatography (PE:EA = 0-10%) to afford compound 28e as a white solid (583.1 mg, 93%).
$^1$H NMR (400 MHz, $CDCl_3$) δ 7.15 (dd, *J* = 7.9, 1.1 Hz, 1H), 7.02 - 6.95 (m, 1H), 6.78 (dd, *J* = 8.2, 1.1 Hz, 1H), 3.85 (s, 2H), 3.64 - 3.51 (m, 2H), 3.45 - 3.32 (m, 2H), 2.64 (s, 2H), 1.52 - 1.44 (m, 13H). LC-MS(ESI): $[M-tBu+H]^+$ = 325.98.

Step 5: 1'-tert-Butyl 5-methylspiro[chroman-3,4'-piperidine]-1',5-dicarboxylate (Compound 28f)

Under carbon monoxide atmosphere, triethylamine (2.5 g, 24.6 mmol) and $Pd(dppf)_2Cl_2{\cdot}CH_2Cl_2$ (670.7 mg, 0.8 mmol) were added to a solution of compound 28e (3.1 g, 8.2 mmol) in methanol (60 mL). The mixture was stirred at 65°C overnight. The solvent was evaporated, and the residue was purified by column chromatography (PE: EA = 0-20%) to afford a colorless oily compound 28f (2.7 g, 90%).
$^1$H NMR (400 MHz, $CDCl_3$) δ 7.52 (d, *J* = 7.5 Hz, 1H), 7.20 - 7.11 (m, 1H), 6.99 (d, *J* = 8.0 Hz, 1H), 3.90 (s, 2H), 3.88 (s, 3H), 3.60-3.46 (m, 2H), 3.46 - 3.34 (m, 2H), 3.00 (s, 2H), 1.55-1.40 (m, 13H). LC-MS(ESI): $[M-Boc+H]^+$ = 262.17.

Step 6: 1'-tert-Butyl 5-carboxyspiro[chroman-3,4'-piperidine]-1'-carboxylate (Compound 28g)

At 0°C, a solution of potassium hydroxide (1.5 g, 37 mmol) in water (20 mL) was added to a solution of compound 28f in a mixture of methanol and tetrahydrofuran (1:1, 40 mL). The mixture was stirred at 60°C overnight. The solvent was evaporated, and 1 M hydrochloric acid (50 mL) was added. The mixture was extracted with ethyl acetate (40 mL × 3), washed with saturated brine (80 mL), and dried over anhydrous sodium sulfate. The solvent was evaporated to afford a white solid compound 28g (2.5 g, 97%), which was used directly in the next step.
LC-MS(ESI): $[M- H]^-$ = 346.20.

Step 7: tert-Butyl 6-iodo-5-carboxyspiro[chroman-3,4'-piperidine]-1'-carboxylate (Compound 28h)

Under air atmosphere, iodine (62.0 mg, 0.244 mmol), diethyl iodate (78.7 mg, 0.244 mmol), and palladium acetate (2.7 mg, 0.012 mmol) were added to a solution of compound 28g (84.9 mg, 0.244 mmol) in N,N-dimethylformamide (1 mL). The mixture was stirred at 80°C for 30 min. The mixture was extracted with ethyl acetate (10 mL × 3), washed with saturated brine (20 mL), and dried over anhydrous sodium sulfate. The solvent was evaporated, and the residue was purified by preparative HPLC and lyophilized to afford a white solid compound 28h (72.3 mg, 65%).
$^1$H NMR (400 MHz, $CDCl_3$) δ 7.54 (d, *J* = 8.6 Hz, 1H), 6.63 (d, *J* = 8.5 Hz, 1H), 3.89 (s, 2H), 3.64 - 3.45 (m, 2H), 3.45 - 3.26 (m, 2H), 2.71 (s, 2H), 1.52 - 1.36 (m, 13H). LC-MS(ESI): $[M- H]^-$ = 472.10.

Step 8: 1'-tert-Butyl 6-iodo-5-methylspiro[chroman-3,4'-piperidine]-1',5-dicarboxylate (Compound 28i)

Iodomethane (120 mg, 0.85 mmol) and potassium carbonate (175.2 mg, 1.27 mmol) were added to a solution of compound 28h (200.0 mg, 0.42 mmol) in N,N-dimethylformamide (5 mL). The mixture was stirred at 80°C for 1 h. Water (20 mL) was added, and the mixture was extracted with ethyl acetate (40 mL × 3), washed with saturated brine (40 mL), and dried over anhydrous sodium sulfate. The solvent was evaporated, and the residue was purified by column chromatography (PE:EA = 0-20%) to afford compound 28i as a yellow oil (203.9 mg, 99%).
$^1$H NMR (600 MHz, $CDCl_3$) δ 7.51 (d, *J* = 8.7 Hz, 1H), 6.62 (d, *J* = 8.7 Hz, 1H), 3.95 (s, 3H), 3.88 (s, 2H), 3.57 - 3.47 (m, 2H), 3.40 - 3.27 (m, 2H), 2.59 (s, 2H), 1.49 - 1.41 (m, 13H). LC-MS(ESI): $[M-Boc+H]^+$ = 388.19.

Step 9: 1'-tert-Butyl 5-methyl-6-vinylspiro[chroman-3,4'-piperidine]-1',5-dicarboxylate (Compound 28j)

Under nitrogen atmosphere, potassium vinyltrifluoroborate (560.7 mg, 4.2 mmol), palladium acetate (125.3 mg, 0.558 mmol), triphenylphosphine (102.5 mg, 0.391 mmol), and cesium carbonate (1.82 g, 5.58 mmol) were added to a solution of compound 28i (1.36 g, 2.8 mmol) in a mixture of tetrahydrofuran and water (10:1, 33 mL). The mixture was stirred at 85°C overnight. The solvent was evaporated, and the residue was purified by column chromatography (PE:EA = 0-40%) to afford a colorless oily compound 28j (790 mg, 73%), which was used directly in the next step.
LC-MS(ESI): $[M+H]^+$ = 388.09.

Step 10: 1'-tert-Butyl 6-formyl-5-methylspiro[chroman-3,4'-piperidine]-1',5-dicarboxylate (Compound 28k)

Sodium periodate (6.07 g, 28.4 mmol) and osmium tetroxide dihydrate (126.8 mg, 0.284 mmol) were added to a solution of compound 28j (2.75 g, 7.1 mmol) in a mixture of acetone and water (5:1, 210 mL). The mixture was stirred at room temperature overnight. The solvent was evaporated, and the residue was purified by column chromatography (PE:EA = 0-40%) to afford a white solid compound 28k (930 mg, 33%).

$^1$H NMR (600 MHz, $CDCl_3$) δ 9.83 (s, 1H), 7.65 (d, $J$ = 8.5 Hz, 1H), 6.99 (d, $J$ = 8.5 Hz, 1H), 4.05 - 3.95 (m, 5H), 3.58 - 3.47 (m, 2H), 3.40 - 3.31 (m, 2H), 2.64 (s, 2H), 1.55 - 1.29 (m, 13H). LC-MS(ESI): $[M\text{-}tBu+H]^+$ = 334.19.

Step 11: tert-Butyl 2'-(2,6-dioxopiperidin-3-yl)-1'-oxo-1',2',3',9'-tetrahydro-7'H-spiro[piperidine-4,8'-pyrano[3,2-e]isoindole]-1-carboxylate (Compound 28l)

Compound 28k (451 mg, 1.16 mmol) and 3-amino-2,6-piperidinedione hydrochloride (286.2 mg, 1.74 mmol) were mixed in a solution of dichloromethane and methanol (1:2, 21 mL). After stirring at room temperature for 1 h, sodium cyanoborohydride (218.5 mg, 3.5 mmol) was added, and the mixture was stirred at room temperature overnight. The solvent was evaporated, and the residue was purified by reversed-phase column chromatography to afford a white solid compound 28l (30.2 mg, 5%).

$^1$H NMR (600 MHz, DMSO-$d_6$) δ 10.99 (s, 1H), 7.29 (d, $J$ = 8.2 Hz, 1H), 7.02 (d, $J$ = 8.2 Hz, 1H), 5.01 (dd, $J$ = 13.2, 5.1 Hz, 1H), 4.30 (d, $J$ = 16.9 Hz, 1H), 4.19 (d, $J$ = 16.9 Hz, 1H), 3.98 (s, 2H), 3.47 - 3.38 (m, 2H), 3.31 - 3.24 (m, 2H), 3.12 - 3.02 (m, 2H), 2.92 - 2.85 (m, 1H), 2.63 - 2.56 (m, 1H), 2.42 - 2.32 (m, 1H), 2.00 - 1.94 (m, 1H), 1.51 - 1.30 (m, 11H), 1.38 - 1.30 (m, 2H). LC-MS(ESI): $[M\text{-}Boc+H]^+$ = 370.29.

Step 12: 3-(1'-Oxo-1',9'-dihydro-7'H-spiro[piperidine-4,8'-pyrano[3,2-e]isoindol]-2'(3'H)-yl)piperidine-2,6-dione (E3 Ligase Inhibitor 28)

Trifluoroacetic acid (1 mL) was added to compound 28l (30.2 mg, 0.064 mmol), and the mixture was stirred at room temperature overnight. The mixture was purified by preparative HPLC to afford a white solid E3 ligase inhibitor 28 (21.7 mg, 91%).

$^1$H NMR (600 MHz, DMSO-$d_6$) δ 11.00 (s, 1H), 7.31 (d, $J$ = 8.2 Hz, 1H), 7.04 (d, $J$ = 8.2 Hz, 1H), 5.01 (dd, $J$ = 13.3, 5.1 Hz, 1H), 4.31 (d, $J$ = 17.0 Hz, 1H), 4.20 (d, $J$ = 17.0 Hz, 1H), 4.05 (s, 2H), 3.21 - 3.03 (m, 6H), 2.94 - 2.84 (m, 1H), 2.64 - 2.56 (m, 1H), 2.38 (qd, $J$ = 13.0, 4.2 Hz, 1H), 2.02 - 1.91 (m, 1H), 1.70 - 1.52 (m, 4H). LC-MS(ESI): $[M+H]^+$ = 370.29.

29)

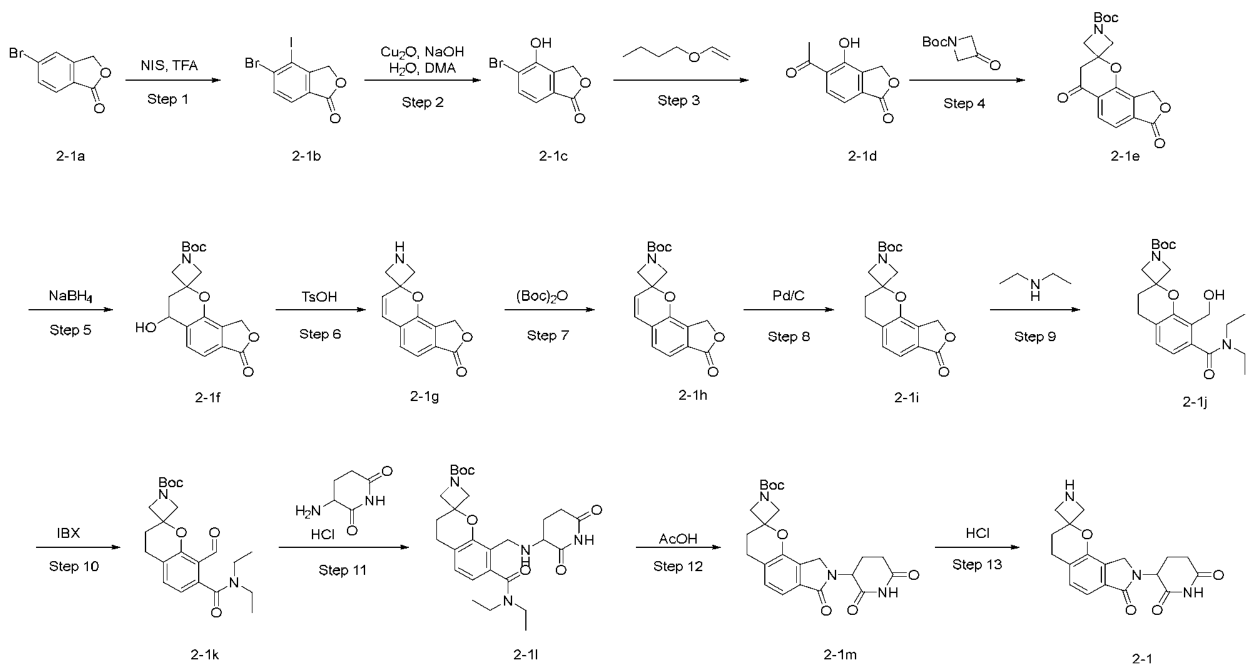

Step 1: Compound 2-1a (1 g, 4.69 mmol) was dissolved in trifluoromethanesulfonic acid (10 mL) under nitrogen atmosphere and cooled to 0°C. N-Iodosuccinimide (1.16 g, 5.16 mmol) was added at 0°C, and the mixture was warmed to room temperature and stirred overnight. The resulting mixture was poured into ice water, filtered, and the filter cake was washed with water. The crude product was purified by column chromatography to afford a white solid compound 2-1b (800 mg, 50.28%).

$^1$H NMR (600 MHz, DMSO-$d_6$) δ 7.94 - 7.91 (m, 1H), 7.78 (dd, *J* = 8.1, 1.1 Hz, 1H), 5.21 (s, 2H).

Step 2: Aqueous sodium hydroxide (413 mg, 10.33 mmol in 7 mL water) was added to a solution of compound 2-1b in N,N-dimethylethylacetamide (3.5 mL). Cuprous oxide (59 mg, 413.07 μmol) was added, and the mixture was heated to 80°C and stirred overnight. The resulting mixture was neutralized with 1 N hydrochloric acid, extracted with ethyl acetate, and washed with saturated brine. The organic phase was concentrated under vacuum, and the residue was purified by column chromatography to afford a white solid compound 2-1c (249 mg, 52.64%).

$^1$H NMR (600 MHz, DMSO-$d_6$) δ 10.91 (s, 1H), 7.73 (d, *J* = 8.0 Hz, 1H), 7.24 (d, *J* = 8.0 Hz, 1H), 5.35 (s, 2H). LC-MS(ESI): $[M+H]^+$ = 230.86.

Step 3: Under nitrogen atmosphere, compound 2-1c (11.85 g, 51.74 mmol), butyl vinyl ether (6.74 g, 67.26 mmol), triethylamine (10.47 g, 103.48 mmol), palladium acetate (1.16 g, 5.17 mmol), and 1,1'-bis(diphenylphosphino) ferrocene (4.30 g, 7.7 mmol) were dissolved in methanol (200 mL). The mixture was heated to 70°C and stirred overnight. The resulting mixture was purified by preparative column chromatography to afford a yellow oily compound 2-1d (6.42 g, 45.20%).

$^1$H NMR (400 MHz, DMSO-$d_6$) δ 12.15 (s, 1H), 8.07 (d, *J* = 8.0 Hz, 1H), 7.41 (d, *J* = 8.0 Hz, 1H), 5.42 (s, 2H), 2.73 (s, 3H). LC-MS(ESI): $[M+H]^+$ = 193.24.

Step 4: Compound 2-1d (6.28 g, 32.68 mmol), tert-butyl 3-oxoazetidine-1-carboxylate (6.15 g, 35.96 mmol), and pyrrolidine (2.58 g, 35.96 mmol) were dissolved in methanol (100 mL). The mixture was stirred at 70°C overnight. The solvent was evaporated under vacuum, and the residue was purified by normal-phase column chromatography to afford a yellow solid compound 2-1e (2.52 g, 22.33%).

$^1$H NMR (600 MHz, DMSO-$d_6$) δ 7.93 (d, *J* = 7.9 Hz, 1H), 7.54 (d, *J* = 7.9 Hz, 1H), 5.51 (s, 2H), 4.08 - 3.94 (m, 4H), 3.32 (s, 2H), 1.39 (s, 9H). LC-MS(ESI): $[M-tBu+H]^+$ = 290.16.

Step 5: Sodium borohydride (552 mg, 14.59 mmol) was added to a solution of compound 2-1e (2.52 g, 7.30 mmol) in methanol (30 mL) at room temperature. The mixture was stirred for 1 h. Saturated ammonium chloride was added to quench the reaction, and the resulting mixture was extracted with ethyl acetate, dried over anhydrous sodium sulfate, filtered, and concentrated to afford a yellow solid compound 2-1f(2.5 g, 98.63%).

LC-MS(ESI): $[M-tBu+H]^+$ = 292.08.

Step 6: Compound 2-1f (2.57 g, 7.39 mmol) and p-toluenesulfonic acid (2.54 g, 14.77 mmol) were dissolved in toluene (60 mL). The mixture was stirred at 110°C for 12 h. The solvent was evaporated under vacuum, and the crude product was used directly in the next step without purification.

LC-MS(ESI): $[M+H]^+$ = 230.46.

Step 7: Triethylamine (5.51 g, 54.55 mmol) was added to a solution of compound 2-1g (2.5 g, 10.91 mmol) in dichloromethane (30 mL) at 0°C. Di-tert-butyl dicarbonate (7.14 g, 32.72 mmol) was added, and the mixture was stirred at room temperature for 1 h. The solvent was evaporated under vacuum, and the residue was purified by normal-phase column chromatography to afford a white solid compound 2-1h (800 mg, 22.27%).

LC-MS(ESI): $[M-tBu+H]^+$ = 274.17.

Step 8: Palladium hydroxide on carbon (1 g) was added to a solution of compound 2-1h (800 mg, 2.43 mmol) in hexafluoroisopropanol (20 mL). The system was purged with hydrogen three times and the reaction mixture was stirred under a hydrogen atmosphere at room temperature for 5 h. The resulting mixture was filtered, and the filtrate was concentrated under reduced pressure to afford compound 2-1i as a white solid (700 mg, 86.97%).

LC-MS(ESI): $[M+H]^+$ = 276.27.

Step 9: Aluminum trichloride (322 mg, 2.41 mmol) was added to a solution of diethylamine (353 mg, 4.83 mmol) in dry dichloromethane (5 mL) at 0°C. The mixture was stirred at 0°C for 1 h, and compound 2-1i (200 mg, 603.56 μmol) was added. The mixture was stirred at room temperature overnight. Saturated ammonium chloride was added to quench the reaction, and the resulting mixture was extracted with ethyl acetate, dried over anhydrous sodium sulfate, and concentrated. The residue was purified by normal-phase column chromatography to afford a white solid compound 2-1j (100 mg, 40.96%).

$^1$H NMR (600 MHz, DMSO-$d_6$) δ 7.04 (d, *J* = 7.7 Hz, 1H), 6.65 (d, *J* = 7.7 Hz, 1H), 4.56 (t, *J* = 5.6 Hz, 1H), 4.38 (d, *J* = 86.7 Hz, 2H), 3.91 (s, 4H), 3.52 (s, 1H), 3.22 - 3.32 (m, 1H),3.08 (s, 2H), 2.80 (t, *J* = 6.5 Hz, 2H), 2.08 (t, *J* = 6.6 Hz, 2H), 1.40 (s, 9H), 1.14 (t, *J* = 7.0 Hz, 3H), 0.97 (t, *J* = 7.1 Hz, 3H). LC-MS(ESI): $[M+H]^+$ = 405.40.

Step 10: 2-Iodoxybenzoic acid (83 mg, 198.66 μmol) was added to a solution of compound 2-1j (80 mg, 197.77 μmol) in tetrahydrofuran (3 mL). The mixture was stirred at 70°C for 3 h. After cooling, the resulting mixture was filtered, and the filtrate was concentrated under vacuum to afford a white solid compound 2-1k (70 mg, 87.94%).

LC-MS(ESI): $[M+H]^+$ = 403.35.

Step 11: Compound 2-1k (300 mg, 745.36 μmol), 3-aminopiperidine-2,6-dione hydrochloride (184 mg, 1.12 mmol), and sodium acetate (92 mg, 1.12 mmol) were dissolved in methanol (10 mL). After stirring at room temperature for 30 min, and sodium cyanoborohydride (94 mg, 1.49 mmol) was added. The mixture was stirred at room temperature overnight. The solvent was evaporated under vacuum to afford a blue solid compound 2-1l (350 mg, 91.26%).

LC-MS(ESI): $[M+H]^+$ = 515.40.

Step 12: Compound 2-1l (300 mg, 582.95 μmol) and glacial acetic acid (1.23 g, 20.40 mmol) were dissolved in toluene (30 mL). The mixture was stirred at 110°C overnight. The solvent was evaporated under vacuum, and the residue was purified by normal-phase column chromatography to afford a white solid compound 2-1m (237 mg, 92.49%).

$^1$H NMR (600 MHz, DMSO-$d_6$) δ 10.99 (s, 1H), 7.25 (d, *J* = 9.0 Hz, 2H), 5.10 (dd, *J* = 13.4, 5.1 Hz, 1H), 4.39 (d, *J* = 17.3 Hz, 1H), 4.23 (d, *J* = 17.3 Hz, 1H), 3.98 (s, 2H), 3.88 (s, 2H), 2.96 - 2.87 (m, 3H), 2.57 - 2.61 (m, 1H), 2.44 (dd, *J* = 13.2, 4.5 Hz, 1H), 2.15 (s, 2H), 2.02 - 1.94 (m, 1H), 1.40 (s, 9H). LC-MS(ESI): $[M\text{-}tBu+H]^+$ = 386.30.

Step 13: Compound 2-1m (53 mg, 32.68 μmol) was dissolved in 1,4-dioxane (1 mL), followed by addition of 4M hydrochloric acid in 1,4-dioxane solution (1 mL). The mixture was stirred at room temperature for 1 h. The solvent was evaporated under vacuum, and the residue was purified by reversed-phase preparative HPLC to afford a white solid compound 2-1 (20 mg, 48.80%).

$^1$H NMR (400 MHz, Methanol-$d_4$) δ 7.39 (d, *J* = 7.7 Hz, 1H), 7.32 (d, *J* = 7.8 Hz, 1H), 5.18 (dd, *J* = 13.4, 5.2 Hz, 1H), 4.55 - 4.41 (m, 2H), 4.32 - 4.22 (m, 4H), 3.05 - 2.88 (m, 3H), 2.81 (ddd, *J* = 17.6, 4.7, 2.4 Hz, 1H), 2.50 (qd, *J* = 13.2, 4.7 Hz, 1H), 2.33 (dq, *J* = 10.2, 7.2 Hz, 2H), 2.20 (dtd, *J* = 12.9, 5.3, 2.4 Hz, 1H). LC-MS(ESI): $[M+H]^+$ = 342.29.

30)

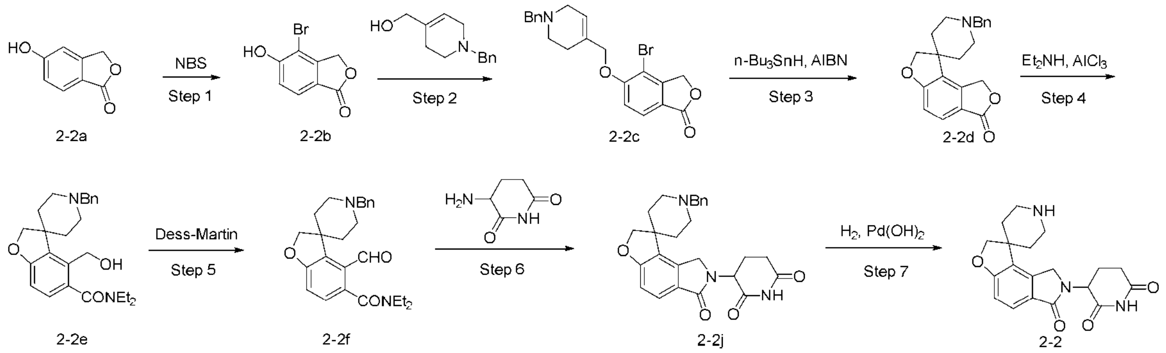


Step 1: Compound 2-2a (5.0 g, 33.30 mmol) was dissolved in acetonitrile (50 mL), followed by the addition of N-bromosuccinimide (8.89 g, 49.95 mmol) and trifluoroacetic acid (11.39 g, 99.89 mmol). The mixture was heated to 80°C and stirred for 12 h. The resulting mixture was concentrated under the reduced pressure condition, and the crude product was purified by reversed-phase column chromatography to afford a pale yellow solid compound 2-2b (1.41 g, 18.5%).

$^1$H NMR (600 MHz, DMSO-$d_6$) δ 11.63 (s, 1H), 7.70 (d, *J* = 8.3 Hz, 1H), 7.13 (d, *J* = 8.3 Hz, 1H), 5.25 (s, 2H). LC-MS (ESI): $[M+H]^+$ = 229.06.

Step 2: Compound 2-2b (2.6 g, 11.35 mmol), (1-benzyl-1,2,3,6-tetrahydropyridin-4-yl)methanol (3.46 g, 17.03 mmol), and triphenylphosphine (7.44 g, 28.38 mmol) were dissolved in tetrahydrofuran (60 mL). The mixture was cooled to 0°C, and diethyl azodicarboxylate (4.44 g, 28.38 mmol) was slowly added. After the addition was completed, the mixture was warmed to room temperature and stirred for 12 h. The resulting mixture was concentrated under reduced pressure, and water (50 mL) was added. The aqueous layer was extracted with ethyl acetate (100 mL × 3), washed with saturated brine (100 mL), and the combined organic phases were dried over anhydrous sodium sulfate. The crude product obtained from concentration was purified by reversed-phase column chromatography to afford a white solid compound 2-2c (4.27 g, 90.79%).

$^1$H NMR (400 MHz, DMSO-$d_6$) δ 7.85 (d, *J* = 8.4 Hz, 1H), 7.56 - 7.38 (m, 5H), 7.35 (d, *J* = 8.5 Hz, 1H), 5.87 (d, *J* = 3.8 Hz, 1H), 5.29 (s, 2H), 4.79 (s, 2H), 4.31 - 4.04 (m, 2H), 3.52 (s, 2H), 3.10 (q, *J* = 7.3 Hz, 2H), 2.41 (s, 2H). LC-MS(ESI): $[M+H]^+$ = 414.19.

Step 3: Compound 2-2c (6.27 g, 15.13 mmol), tri-n-butyltin hydride (8.81 g, 30.26 mmol), and azobisisobutyronitrile (497 mg, 3.03 mmol) were dissolved in toluene (200 mL). Under nitrogen atmosphere, the mixture was stirred at 110°C for 12 h. After cooling to room temperature, the mixture was concentrated under reduced pressure, and water (100 mL) was added. The aqueous layer was extracted with ethyl acetate (100 mL × 3), washed with saturated brine (100 mL), and the combined organic phases were dried over anhydrous sodium sulfate. The crude product obtained after concentration was purified by column chromatography to afford a yellow solid compound 2-2d (3.0 g, 59.1%).

$^1$H NMR (600 MHz, DMSO-$d_6$) δ 7.65 (d, *J* = 8.2 Hz, 1H), 7.34 (d, *J* = 5.5 Hz, 4H), 7.28 - 7.25 (m, 1H), 7.00 (d, *J* =

8.3 Hz, 1H), 5.54 (s, 2H), 4.58 (s, 2H), 3.49 (s, 2H), 2.02 - 1.95 (m, 4H), 1.68 (d, $J$ = 10.6 Hz, 2H), 1.61 (t, $J$ = 8.1 Hz, 2H). LC-MS(ESI): $[M+H]^+$ = 336.30.

Step 4: Aluminum trichloride (3.18 g, 23.85 mmol) was dissolved in dichloromethane (15 mL), and the mixture was cooled to 0°C. Diethylamine (3.49 g, 47.7 mmol) was slowly added, and the mixture was stirred at 0°C for 30 min. Compound 2-2d (2.0 g, 5.96 mmol) was then slowly added. After the addition, the mixture was warmed to room temperature and stirred for 3 h. Saturated ammonium chloride solution (50 mL) was added, and the resulting mixture was extracted with dichloromethane (50 mL × 3). The combined organic phases were washed with saturated brine (100 mL) and dried over anhydrous sodium sulfate. The crude product obtained from concentration was purified by column chromatography to afford a reddish-brown oily compound 2-2e (1.85 g, 75.9%).

$^1$H NMR (400 MHz, DMSO-$d_6$) δ 7.57 - 7.25 (m, 5H), 6.94 (d, $J$ = 7.5 Hz, 1H), 6.73 (d, $J$ = 7.5 Hz, 1H), 4.81 (t, $J$ = 4.6 Hz, 1H), 4.51 (d, $J$ = 4.6 Hz, 2H), 4.46 - 4.26 (m, 2H), 3.63 - 3.43 (m, 3H), 3.17 (d, $J$=5.2 Hz, 1H), 3.09 (s, 3H), 2.80 (s, 1H), 2.36 (s, 2H), 1.98 (s, 2H), 1.64 - 1.60 (m, 2H), 1.13 (t, $J$ = 7.5 Hz, 3H), 0.98 (t, $J$ = 7.1 Hz, 3H). LC-MS(ESI): $[M+H]^+$ = 409.49.

Step 5: Compound 2-2e (1.85 g, 4.53 mmol) was dissolved in dichloromethane (20 mL), and [bis(acetoxy)iodo]benzene (2.88 g, 6.79 mmol) was added. The mixture was stirred at room temperature for 2 h. The mixture was filtered, and the filtrate was concentrated under the reduced pressure condition to afford the crude product 2-2f (1.57 g, 85.3%), which was used directly in the next step without further purification. LC-MS (ESI): $[M+H]^+$ = 407.69.

Step 6: Compound 2-2f (1.4 g, 3.44 mmol), 3-amino-2,6-piperidinedione hydrochloride (850 mg, 5.16 mmol), and sodium acetate (424 mg, 5.16 mmol) were dissolved in methanol (30 mL). The mixture was stirred at room temperature for 30 min, followed by addition of sodium cyanoborohydride (433 mg, 6.88 mmol). The mixture was stirred at room temperature for 12 h. The resulting mixture was concentrated, and acetic acid (7.0 mL) and toluene (40 mL) were added. The resulting mixture was heated at 110°C for 12 h. After concentration under the reduced pressure condition, the crude product was purified by column chromatography to afford a pale blue solid compound 2-2g (1.32 g, 86.1%).

$^1$H NMR (600 MHz, Methanol-$d_4$) δ 7.64 (d, $J$ = 8.2 Hz, 1H), 7.49 - 7.34 (m, 5H), 6.93 (d, $J$ = 8.2 Hz, 1H), 5.15 (dd, $J$ = 13.4, 5.2 Hz, 1H), 4.65 (dd, $J$ = 12.8, 5.2 Hz, 2H), 4.60 (d, $J$ = 5.0 Hz, 2H), 3.86 - 3.78 (m, 2H), 3.18 - 3.13 (m, 2H), 2.82 - 2.75 (m, 2H), 2.70 - 2.65 (m, 2H), 2.47 - 2.40 (m, 2H), 2.31 - 2.23 (m, 2H), 2.18 - 2.12 (m, 2H). LC-MS (ESI): $[M+H]^+$ = 446.29.

Step 7: Compound 2-2g (500 mg, 1.12 mmol) was dissolved in hexafluoroisopropanol (15 mL), and palladium hydroxide on carbon (394 mg) was added. The system was purged with hydrogen three times, and the mixture was stirred at room temperature for 12 h. The resulting mixture was filtered, and the filter cake was washed with hexafluoroisopropanol (15 mL × 3). The filtrate was concentrated under reduced pressure, and the crude product was purified by reversed-phase column chromatography to afford a white solid compound 2-2 (277 mg, 69.4%).

$^1$H NMR (600 MHz, Methanol-$d4$) δ 7.69 (d, $J$ = 8.2 Hz, 1H), 6.99 (d, $J$ = 8.2 Hz, 1H), 5.18 (dd, $J$ = 13.4, 5.1 Hz, 1H), 4.69 (s, 2H), 4.66 - 4.53 (m, 2H), 3.50 (d, $J$ = 13.1 Hz, 2H), 3.20 - 3.12 (m, 2H), 2.99 - 2.91 (m, 1H), 2.85 - 2.78 (m, 1H), 2.53 - 2.42 (m, 1H), 2.41 - 2.29 (m, 2H), 2.25 - 2.19 (m, 1H), 2.07 (d, $J$ = 14.5 Hz, 2H). LC-MS(ESI): $[M+H]^+$ = 356.39

31)

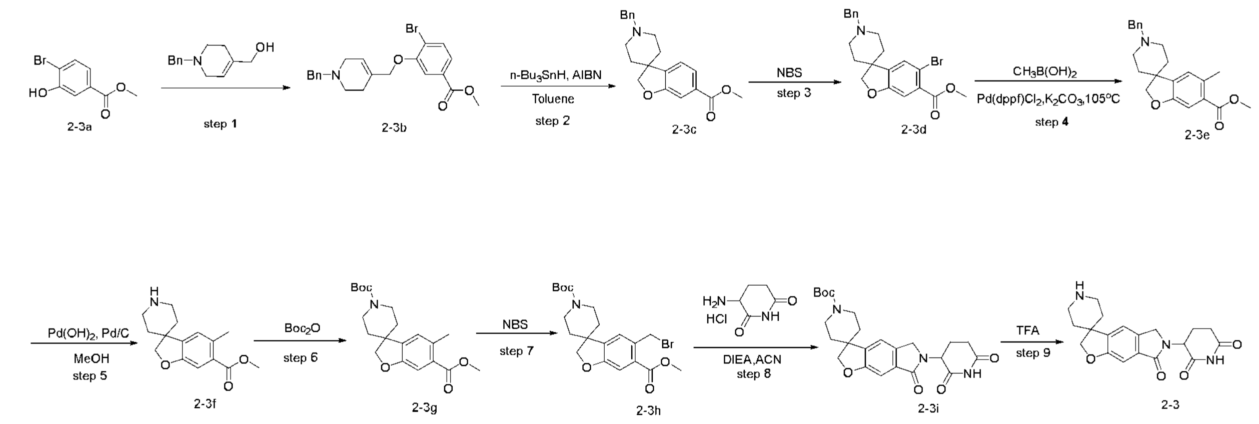


Step 1: Compound 2-3a (5.00 g, 21.64 mmol), (1-benzyl-1,2,3,6-tetrahydropyridin-4-yl)methanol (6.60 g, 32.46 mmol), and diphenyl-2-pyridylphosphine (5.71 g, 21.62 mmol) were dissolved in anhydrous tetrahydrofuran (50 mL). Under a nitrogen atmosphere and ice bath cooling, diisopropyl azodicarboxylate (4.40 g, 21.67 mmol) was slowly added. After completion, the mixture was warmed to room temperature and stirred overnight. The resulting mixture was concentrated under the reduced pressure condition, and the crude product was purified by normal-phase column chromatography to afford a white solid compound 2-3b (8.61 g, 95.5%).

LC-MS (ESI): $[M+H]^+$ = 416.20.

Step 2: Compound 2-3b (7.61 g, 18.28 mmol) was dissolved in toluene (100 mL), followed by the addition of azobisisobutyronitrile (6.00 g, 36.58 mmol) and tri-n-butyltin hydride (10.64 g, 36.56 mmol). The mixture was heated to 110°C and stirred overnight. After concentration under the reduced pressure condition, the crude product was purified by normal-phase column chromatography to afford a yellow oily compound 2-3c (5.15 g, 83.5%).

$^1$H NMR (600 MHz, DMSO-$d_6$) δ 7.57 (dd, *J* = 7.8, 1.3 Hz, 1H), 7.54 - 7.48 (m, 5H), 7.31 (d, *J* = 1.1 Hz, 1H), 7.25 (t, *J* = 6.5 Hz, 1H), 4.59 (d, *J* = 7.5 Hz, 2H), 4.36 (d, *J* = 4.0 Hz, 2H), 3.83 (s, 3H), 3.38 (d, *J* = 12.5Hz, 2H), 3.14 (dd, *J* = 22.5, 10.7 Hz, 2H), 2.11 (td, *J* = 14.2, 3.9 Hz, 2H), 1.98 - 1.91 (m, 2H). LC-MS(ESI): $[M+H]^+$ = 338.35.

Step 3: Compound 2-3c (5.15 g, 15.26 mmol) and p-toluenesulfonic acid (5.26 g, 30.53 mmol) were dissolved in toluene (200 mL), followed by the addition of N-bromosuccinimide (3.80 g, 21.37 mmol). The mixture was stirred at 60°C overnight. After cooling to room temperature, the mixture was concentrated under reduced pressure, and the crude product was purified by column chromatography to afford a yellow solid compound 2-3d (3.93 g, 61.5%).

$^1$H NMR (600 MHz, DMSO-$d_6$) δ 7.57 (s, 1H), 7.54 - 7.48 (m, 5H), 7.17 (s, 1H), 4.58 (s, 2H), 4.34 (s, 2H), 3.82 (s, 3H), 3.16 - 3.06 (m, 2H), 2.33 - 2.24 (m, 1H), 2.09 (dd, *J* = 17.0, 7.4 Hz, 2H), 1.98 - 1.92 (m, 2H), 1.90 - 1.83 (m, 1H). LC-MS(ESI): $[M+H]^+$ = 416.25.

Step 4: Compound 2-3d (3.93 g, 9.44 mmol), methylboronic acid (1.70 g, 28.32 mmol), potassium carbonate (2.61 g, 18.88 mmol), and [1,1'-bis(diphenylphosphino)ferrocene]palladium(II) dichloride (890 mg, 1.21 mmol) were dissolved in a mixture of 1,4-dioxane and water (100/15 mL). The mixture was heated to 105°C and stirred overnight. The resulting mixture was concentrated, and the crude product was purified by reversed-phase column chromatography to afford a white solid compound 2-3e (1.53 g, 46.1%).

$^1$H NMR (600 MHz, DMSO-$d_6$) δ 7.51 (t, *J* = 6.3 Hz, 5H), 7.19 (s, 1H), 7.00 (s, 1H), 4.54 (s, 2H), 4.37 - 4.34 (m, 2H), 3.80 (d, *J* = 3.4 Hz, 3H), 3.37 (d, *J* = 12.8 Hz, 2H), 3.16 - 3.08 (m, 2H), 2.44 (d, *J* = 4.0 Hz, 3H), 2.13 - 2.05 (m, 2H), 1.92 (d, *J* = 14.0 Hz, 2H). LC-MS(ESI): $[M+H]^+$ = 351.89.

Step 5: Compound 2-3e (1.83 g, 5.21 mmol) and ammonia solution (0.5 mL) were dissolved in anhydrous methanol (50 mL), followed by the addition of palladium hydroxide on carbon (900 mg) and palladium on carbon (900 mg). The system was purged with hydrogen, and the mixture was heated to 40°C and stirred overnight. After filtration, the filtrate was concentrated to afford the crude product 2-3f (1.23 g, 90.4%).
LC-MS (ESI): $[M+H]^+$ = 261.87.

Step 6: Compound 2-3f (1.23 g, 4.71 mmol) was dissolved in dichloromethane (50 mL), followed by the addition of di-tert-butyl dicarbonate (2.05 g, 9.41 mmol) and triethylamine (1.43 g, 14.12 mmol). The reaction mixture was stirred at room temperature for 1 h. The resulting mixture was concentrated, and the crude product was purified by normal-phase column chromatography to afford a white solid compound 2-3g (940 mg, 55.2%).

LC-MS (ESI): $[M\text{-t-Bu}+H]^+$ = 306.25.

Step 7: Compound 2-3g (940 mg, 2.60 mmol), N-bromosuccinimide (555 mg, 3.12 mmol), and azobisisobutyronitrile (130 mg, 792.68 μmol) were dissolved in carbon tetrachloride (30 mL). Under nitrogen atmosphere, the mixture was heated to 80°C and stirred overnight. The resulting mixture was extracted with dichloromethane (100 mL × 2), and the combined organic phases were dried over anhydrous sodium sulfate. The crude product 2-3h (1.20 g) was afforded after concentration.

Step 8: Compound 2-3h (1.20 g, 2.59 mmol), 3-amino-2,6-piperidinedione hydrochloride (1.28 g, 7.77 mmol), and N,N-diisopropylethylamine (1.67 g, 12.94 mmol) were dissolved in acetonitrile (50 mL). The reaction mixture was heated to 80°C and stirred overnight. The resulting mixture was concentrated, and the crude product was purified by reversed-phase column chromatography to afford compound 2-3i as a blue solid (290 mg, 24.5%).

$^1$H NMR (600 MHz, DMSO-$d_6$) δ 10.98 (s, 1H), 7.50 (s, 1H), 7.03 (s, 1H), 5.08 (dd, *J* = 13.3, 5.1 Hz, 1H), 4.56 - 4.51 (m, 2H), 4.34 (d, *J* = 16.9 Hz, 1H), 4.21 (d, *J* = 16.9 Hz, 1H), 3.94 (s, 2H), 2.95 - 2.87 (m, 2H), 2.63 - 2.56 (m, 1H), 2.38 (dd, *J* = 13.1, 4.4 Hz, 1H), 2.03 - 1.94 (m, 2H), 1.82 - 1.73 (m, 2H), 1.72 - 1.65 (m, 2H), 1.43 (s, 9H). LC-MS(ESI): $[M+H]^+$ = 456.29.

Step 9: Compound 2-3i (25 mg, 54.88 μmol) was dissolved in dichloromethane (4 mL), followed by the addition of trifluoroacetic acid (4 mL). The mixture was stirred at room temperature for 1 h. The resulting mixture was concentrated, and the crude product was purified by reversed-phase column chromatography to afford a white solid compound 2-3 (10 mg, 51.2%).

$^1$H NMR (600 MHz, DMSO-$d_6$) δ 10.99 (s, 1H), 7.38 (s, 1H), 7.07 (s, 1H), 5.09 (dd, *J* = 13.3, 5.1 Hz, 1H), 4.60 (s, 2H), 4.38 (d, *J* = 16.8 Hz, 1H), 4.26 (d, *J* = 16.8 Hz, 1H), 3.36 - 3.32 (m, 2H), 3.04 (q, *J* = 11.7 Hz, 2H), 2.91 (ddd, *J* = 17.3, 13.6, 5.4 Hz, 1H), 2.62 (dd, *J* = 4.4, 2.4 Hz, 1H), 2.38 (qd, *J* = 13.2, 4.5 Hz, 1H), 2.10 - 1.97 (m, 3H), 1.89 (ddt, *J* = 11.9, 9.4, 2.6 Hz, 2H). LC-MS(ESI): $[M+H]^+$ = 356.30.

32)

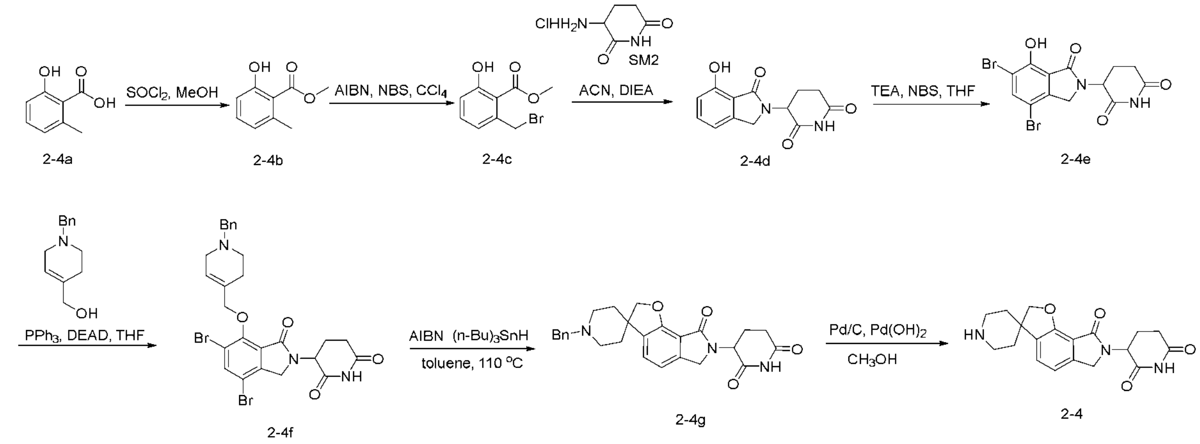


Step 1: 2-Hydroxy-6-methylbenzoic acid (10 g, 65.73 mmol) was dissolved in methanol (30 mL), and thionyl chloride (23.6 mL, 328.65 mmol) was added at 0°C. The mixture was stirred at 60°C overnight. The mixture was concentrated under the reduced pressure condition, and the crude product was purified by column chromatography to afford a colorless liquid compound 2-4b (10.5 g, 96.1%).

LC-MS (ESI): $[M+H]^+$ = 167.13.

Step 2: Compound 2-4b (6.6 g, 39.72 mmol) and N-bromosuccinimide (8.5 g, 47.66 mmol) were dissolved in carbon tetrachloride (30 mL), followed by the addition of azobisisobutyronitrile (1.4 g, 7.94 mmol). Under a nitrogen atmosphere, the mixture was stirred at 85°C overnight. The mixture was concentrated under reduced pressure to afford the crude product 2-4c (11.0 g), which was used directly in the next step without purification.

LC-MS (ESI): $[M+H]^+$ = 247.07.

Step 3: The crude compound 2-4c (11.0 g, 44.88 mmol) was dissolved in acetonitrile (50 mL), followed by the addition of 3-aminopiperidine-2,6-dione hydrochloride (9.7 g, 67.33 mmol) and N,N-diisopropylethylamine (22 mL, 134.64 mmol). The mixture was stirred at 80°C overnight. The mixture was concentrated under the reduced pressure condition, and the crude product was purified by column chromatography to afford a blue solid compound 2-4d (1.5 g, 12.8%).

LC-MS (ESI): $[M+H]^+$ = 261.17.

Step 4: Compound 2-4d (1.0 g, 3.84 mmol) was dissolved in tetrahydrofuran (15 mL), followed by the addition of triethylamine (1.65 mL, 11.52 mmol) and N-bromosuccinimide (820 mg, 7.68 mmol). The mixture was stirred at room temperature for 1 h. After completion as monitored by TLC, the mixture was used directly in the next step.

LC-MS (ESI): $[M+H]^+$ = 419.00.

Step 5: To the above mixture were added (1-benzyl-1,2,3,6-tetrahydropyridin-4-yl)methanol (760 mg, 3.73 mmol), triphenylphosphine (980 mg, 3.73 mmol), and diethyl azodicarboxylate (775 mg, 3.73 mmol). Under nitrogen atmosphere, the mixture was stirred at room temperature overnight. The mixture was concentrated under the reduced pressure condition, and the crude product was purified by column chromatography to afford compound 2-4f as a yellow oil (820 mg, 43.7%).

$^1$H NMR (600 MHz, DMSO-$d_6$) δ 11.05 (s, 1H), 8.20 (s, 1H), 7.54 - 7.48 (m, 5H), 5.87 - 5.86 (m, 1H), 5.10 - 5.06 (m, 1H), 4.79 - 4.71 (m, 2H), 4.43 - 4.34 (m, 3H), 4.23 (dd, *J* = 18.1, 6.0 Hz, 1H), 3.70 - 3.64 (m, 2H), 3.58 - 3.52 (m, 2H), 3.21 - 3.15 (m, 1H), 2.91 - 2.85 (m, 1H), 2.62 - 2.60 (m, 3H), 2.02 - 1.99 (m, 1H). LC-MS(ESI): $[M+H]^+$ = 604.08.

Step 6: Compound 2-4f (820 mg, 1.36 mmol) was dissolved in toluene (25 mL), followed by the addition of azobisisobutyronitrile (2.3 g, 13.6 mmol) and tri-n-butyltin hydride (7.9 g, 27.2 mmol). Under nitrogen atmosphere, the mixture was stirred at 110°C overnight. The mixture was concentrated under the reduced pressure condition. The crude product was purified by column chromatography and the obtained compound was further purified by trituration with methanol to afford a white solid compound 2-4g (430 mg, 71.0%).

$^1$H NMR (600 MHz, DMSO-$d_6$) δ 10.97 (s, 1H), 7.46 (d, *J* = 7.6 Hz, 1H), 7.34 (d, *J* = 4.4 Hz, 4H), 7.28 - 7.24 (m, 1H), 7.02 (d, *J* = 7.6 Hz, 1H), 5.05 (dd, *J* = 13.3, 5.1 Hz, 1H), 4.53 (s, 2H), 4.37 *(d, J* = 17.1 Hz, 1H), 4.24 (d, *J* = 17.1 Hz, 1H), 3.50 (s, 2H), 2.90 (ddd, *J* = 17.2, 13.7, 5.5 Hz, 1H), 2.81 (dt, *J* = 11.8, 4.0 Hz, 2H), 2.63 - 2.55 (m, 1H), 2.35 (dd, *J* = 13.2, 4.6 Hz, 1H), 2.04 (t, *J* = 12.1 Hz, 2H), 1.99 - 1.84 (m, 3H), 1.65 (tt, *J* = 10.9, 2.6 Hz, 2H). LC-MS(ESI): $[M+H]^+$ = 446.29.

Step 7: Compound 2-4g (430 mg, 0.96 mmol) was dissolved in methanol (25 mL), followed by the addition of catalytic hydrochloric acid (5 drops), palladium on carbon (215 mg), and palladium hydroxide (215 mg). The system was purged with hydrogen three times, and the mixture was heated to 40°C and stirred overnight. After filtration through diatomaceous earth, the filtrate was concentrated under reduced pressure. The crude product was purified by trituration with methanol to afford a white solid compound 2-4 (260 mg, 75.8%).

$^1$H NMR (600 MHz, DMSO-*d6*) δ 10.97 (s, 1H), 7.36 (d, *J* = 7.6 Hz, 1H), 7.10 (d, *J* = 7.6 Hz, 1H), 5.05 (dd, *J* = 13.3, 5.1 Hz, 1H), 4.67 (s, 2H), 4.41 (d, *J* = 17.3 Hz, 1H), 4.27 (d, *J* = 17.3 Hz, 1H), 3.36 - 3.34 (m, 2H), 3.07 - 3.01 (m, 2H), 2.94 - 2.88 (m, 1H), 2.62 - 2.58 (m, 1H), 2.40 - 2.33 (m, 1H), 2.03 - 1.95 (m, 3H), 1.89 - 1.85 (m, 2H). LC-MS(ESI): $[M+H]^+$ = 356.30.

33)

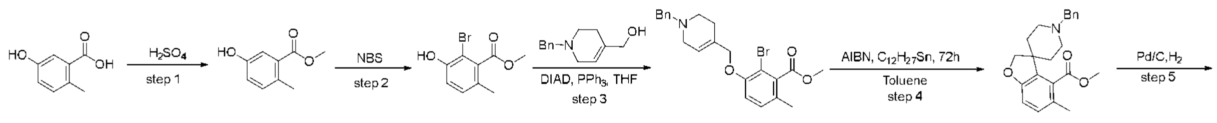


2-5a 2-5b 2-5c 2-5d 2-5e

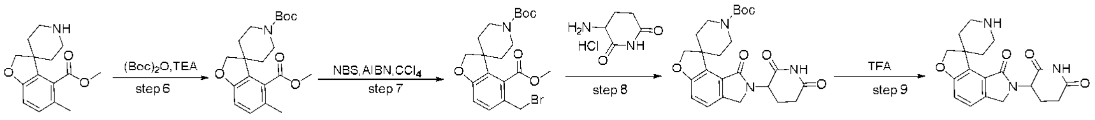


2-5f 2-5g 2-5h 2-5i 2-5

Step 1: Compound 2-5a (23.0 g, 151.2 mmol) was dissolved in anhydrous methanol (120 mL), followed by the slow addition of concentrated sulfuric acid (12 mL). The mixture was stirred at 60°C for 12 h. After cooling to room temperature, the mixture was poured into ice water and extracted with ethyl acetate and water. The organic phase was dried over anhydrous sodium sulfate and concentrated under the reduced pressure condition to afford compound 2-5b as a colorless oil (21.45 g, 85.4%), which was used directly in the next step without further purification.

LC-MS (ESI): $[M+H]^+$ = 167.30.

Step 2: Compound 2-5b (5.0 g, 30.11 mmol) was dissolved in acetonitrile (50 mL), followed by the addition of N-bromosuccinimide (8.04 g, 45.16 mmol). The mixture was stirred at 80°C for 12 h. The mixture was concentrated under the reduced pressure condition, and the crude product was purified by reversed-phase column chromatography to afford compound 2-5c as a pale yellow solid (4.27 g, 58.13%).

Step 3: Compound 2-5c (1.00 g, 4.08 mmol) was dissolved in anhydrous tetrahydrofuran (10 mL), followed by the addition of (1-benzyl-1,2,3,6-tetrahydropyridin-4-yl)methanol (1.24 g, 6.12 mmol), triphenylphosphine (2.14 g, 8.16 mmol), and diisopropyl azodicarboxylate (1.65 g, 8.16 mmol). Under nitrogen atmosphere, the mixture was stirred at room temperature for 12 h. After filtration, the mixture was extracted with ethyl acetate and water. The organic phase was dried over anhydrous sodium sulfate and concentrated under reduced pressure. The crude product was purified by reversed-phase column chromatography to afford a yellow oily compound 2-5d (1.72 g, 97.9%).

$^1$H NMR (600 MHz, DMSO-$d_6$) δ 7.54 - 7.47 (m, 5H), 7.26 (d, *J* = 8.5 Hz, 1H), 7.14 (d, *J* = 8.5 Hz, 1H), 5.85 (s, 1H), 4.65 (s, 2H), 4.37 (s, 2H), 3.87 (s, 3H), 3.70 (s, 2H), 3.58 - 3.52 (m, 1H), 3.19 - 3.12 (m, 1H), 2.45 (m, 2H), 2.18 (s, 3H). LC-MS(ESI): $[M+H]^+$ = 430.25.

Step 4: Compound 2-5d (1.72 g, 4.00 mmol), tri-n-butyltin hydride (2.3 g, 7.99 mmol), and azobisisobutyronitrile (1.97 g, 11.99 mmol) were dissolved in toluene (15 mL). Under nitrogen atmosphere, the mixture was stirred at 110°C for 12 h. After cooling to room temperature, water was added, and the mixture was extracted with ethyl acetate. The organic phase was dried over anhydrous sodium sulfate and concentrated under the reduced pressure condition. The crude product was purified by reversed-phase column chromatography to afford a yellow oily compound 2-5e (1.11 g, 79.0%).

LC-MS (ESI): $[M+H]^+$ = 352.49.

Step 5: Compound 2-5e (1.05 g, 2.99 mmol) was dissolved in anhydrous methanol (15 mL), followed by the addition of palladium on carbon (500 mg). The system was purged with hydrogen three times, and the mixture was stirred at room temperature for 4 h. After filtration, the filtrate was concentrated under the reduced pressure condition to afford the crude product 2-5f (570 mg, 73.1%), which was used directly in the next step without further purification.

LC-MS (ESI): $[M+H]^+$ = 262.57.

Step 6: Compound 2-5f (570 mg, 2.19 mmol) was dissolved in dichloromethane (16 mL), followed by the addition of di-tert-butyl dicarbonate (718 mg, 3.29 mmol) and triethylamine (444 mg, 4.39 mmol). The mixture was stirred at room temperature for 1 h. The mixture was concentrated under the reduced pressure condition, and the crude product was purified by column chromatography to afford a yellow oily compound 2-5g (570 mg, 72.1%).

LC-MS (ESI): $[M-Boc+H]^+$ = 262.27.

Step 7: Compound 2-5g (521 mg, 1.44 mmol) was dissolved in carbon tetrachloride (5 mL), followed by the addition of N-bromosuccinimide (257 mg, 1.44 mmol) and azobisisobutyronitrile (24 mg, 0.14 mmol). Under nitrogen atmosphere, the mixture was stirred at 80°C for 2 h. The mixture was concentrated under the reduced pressure condition to afford a yellow oily crude product 2-5h (521 mg, 82.1%).

LC-MS (ESI): [M-tBu+H] + = 384.27.

Step 8: Compound 2-5h (520 mg, 1.18 mmol) was dissolved in acetonitrile (12 mL), followed by the addition of 3-amino-2,6-piperidinedione hydrochloride (292 mg, 1.77 mmol) and N,N-diisopropylethylamine (459 mg, 3.55 mmol). The mixture was stirred at 80°C for 4 h. The mixture was concentrated under the reduced pressure condition, and the crude product was purified by reversed-phase column chromatography to afford a yellow oily compound 2-5i (181 mg, 33.6%).

LC-MS (ESI): [M-tBu+H] + = 400.35.

Step 9: Compound 2-5i (181 mg, 397.4 μmol) was dissolved in dichloromethane (8 mL), followed by the addition of trifluoroacetic acid (1 mL). The mixture was stirred at room temperature for 2 h. The mixture was concentrated under the reduced pressure condition and purified by preparative HPLC to afford a white solid compound 2-5 (81 mg, 57.4%).

$^1H$ NMR (600 MHz, DMSO-*d*6) δ 11.02 (s, 1H), 7.40 (d, *J* = 8.2 Hz, 1H), 7.09 (d, *J* = 8.2 Hz, 1H), 4.95 (dd, *J* = 13.2, 5.2 Hz, 1H), 4.62 (s, 2H), 4.46 - 4.33 (m, 2H), 3.36 - 3.28 (m, 2H), 3.02 - 2.92 (m, 2H), 2.92 - 2.80 (m, 3H), 2.66 - 2.59 (m, 1H), 2.43 (qd, *J* = 13.3, 4.5 Hz, 1H), 2.07 - 1.95 (m, 1H), 1.84 - 1.77 (m, 2H). LC-MS(ESI): [M+H]+ = 356.35.

34)

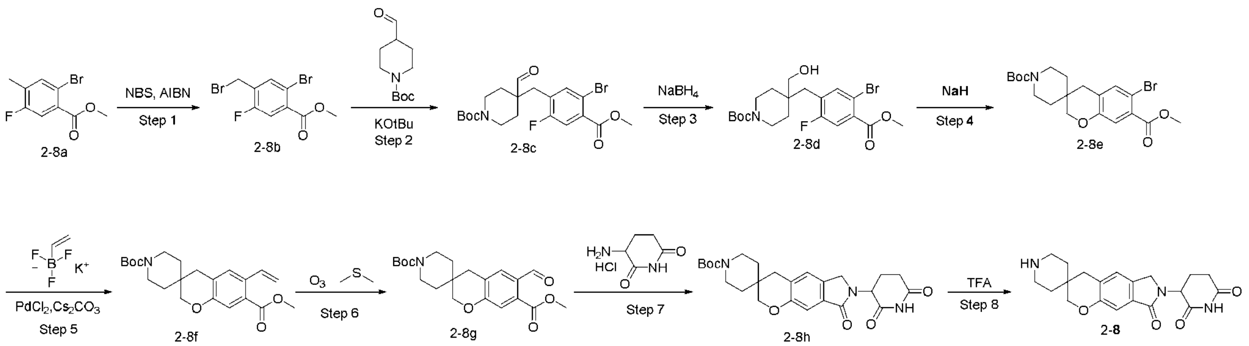


Step 1: Compound 2-8a (3.0 g, 21.14 mmol), N-bromosuccinimide (2.59 g, 14.57 mmol), and azobisisobutyronitrile (398 mg, 2.43 mmol) were dissolved in carbon tetrachloride (30 mL). The system was purged with nitrogen three times, and the mixture was heated to 80°C and stirred overnight. After cooling to room temperature, the mixture was concentrated under reduced pressure, and the crude product was purified by column chromatography to afford a yellow oily compound 2-8b (2.5 g, 63.18%).

Step 2: Compound 2-8b (2.36 g, 11.04 mmol) and potassium tert-butoxide (1.34 g, 11.96 mmol) were dissolved in tetrahydrofuran (20 mL). The system was purged with nitrogen three times, and the mixture was cooled to -30°C and stirred for 30 min. A solution of 4-formylpiperidine-1-carboxylic acid tert-butyl ester (3.0 g, 13.2 mmol) in tetrahydrofuran was slowly added. The mixture was stirred at -30°C for 2 h. The mixture was concentrated under reduced pressure, and the crude product was purified by column chromatography to afford a yellow solid compound 2-8c (1.75 g, 41.49%).

LC-MS (ESI): [M-Boc+H]+ = 358.18.

Step 3: Compound 2-8c (1.7 g, 3.71 mmol) was dissolved in methanol (20 mL), and the mixture was cooled to 0°C. Sodium borohydride (210 mg, 5.56 mmol) was added, and the mixture was stirred at room temperature overnight. The mixture was concentrated under reduced pressure, and water (10 mL) was added. The aqueous layer was extracted with ethyl acetate (20 mL × 3), washed with saturated brine (20 mL), and the combined organic phases were dried over anhydrous sodium sulfate. The crude product was purified by column chromatography to afford a yellow oily compound 2-8d (1.0 g, 57%).

LC-MS (ESI): [M-Boc+H] + = 360.21.

Step 4: Compound 2-8d (750 mg, 1.63 mmol) and sodium hydride (120 mg, 1.96 mmol) were dissolved in N,N-dimethylformamide (10 mL). The reaction was heated to 110°C and stirred for 5 h. After cooling to room temperature, the mixture was concentrated under reduced pressure, and water (10 mL) was added. The aqueous layer was extracted with ethyl acetate (20 mL × 3), washed with saturated brine (20 mL), and the combined organic phases were dried over anhydrous sodium sulfate. The crude product was purified by column chromatography to afford compound 2-8e as a yellow oil (300 mg, 41.82%).

Step 5: Compound 2-8e (550 mg, 1.25 mmol), [1,1'-bis(diphenylphosphino)ferrocene]palladium(II) dichloride (493 mg, 0.674 mmol), and triethylamine (1.37 g, 13.49 mmol) were dissolved in methanol (30 mL). The system was purged with carbon monoxide three times, and the mixture was heated to 70°C and stirred overnight. After cooling to room temperature, the mixture was concentrated under reduced pressure, and the crude product was purified by column chromatography to afford a yellow oily compound 2-8f (200 mg, 41.32%).

$^1H$ NMR (600 MHz, DMSO-d6) δ 7.42 (s, 1H), 7.24 (dd, *J* = 17.5, 11.0 Hz, 1H), 7.15 (s, 1H), 5.61 (dd, *J* = 17.5, 1.4 Hz, 1H), 5.21 (dd, *J* = 11.0, 1.3 Hz, 1H), 3.95 (s, 2H), 3.80 (s, 3H), 3.60 - 3.43 (m, 2H), 3.28 (s, 2H), 2.75 (s, 2H), 1.42 - 1.32

(m, 13H). LC-MS(ESI): [M-tBu+H]$^+$ = 332.40.

Step 6: Compound 2-8f (200 mg, 0.52 mmol) was dissolved in dichloromethane (10 mL), and ozone was bubbled through the solution. The mixture was cooled to -30°C and stirred for 30 min. Nitrogen was then bubbled through the solution for 30 min, followed by the addition of dimethyl sulfide (15 mL). The mixture was stirred at room temperature for 2 h. The mixture was concentrated under reduced pressure, and the crude product was purified by column chromatography to afford a yellow solid compound 2-8g (200 mg, 41.32%).

LC-MS (ESI): [M-tBu+H] $^+$ = 334.36.

Step 7: Compound 2-8g (20 mg, 0.05 mmol), 3-aminopiperidine-2,6-dione hydrochloride (11 mg, 0.068 mmol), and triethylamine (0.2 mL) were dissolved in dichloromethane (2 mL). The mixture was stirred at room temperature for 30 min, followed by the addition of sodium triacetoxyborohydride (9 mg, 0.136 mmol) and acetic acid (0.2 mL). The mixture was stirred at room temperature for 3 h. The mixture was concentrated under reduced pressure, and water (10 mL) was added. The aqueous layer was extracted with dichloromethane (20 mL × 3), washed with saturated brine (20 mL), and the combined organic phases were dried over anhydrous sodium sulfate. The crude product was purified by column chromatography to afford a yellow oily compound 2-8h (5.0 mg, 21%).

$^1$H NMR (600 MHz, DMSO-$d_6$) δ 10.97 (s, 1H), 7.29 (s, 1H), 7.04 (s, 1H), 5.08 (dd, *J* = 13.3, 5.1 Hz, 1H), 4.34 (d, *J* = 16.6 Hz, 1H), 4.20 (d, *J* = 16.6 Hz, 1H), 3.97 (s, 2H), 3.40 - 3.54 (m, 2H), 3.26 - 3.29 (m, 1H), 2.85 - 2.95 (m, 1H), 2.81 (s, 2H), 2.60 (d, *J* = 17.7 Hz, 1H), 2.31 - 2.41 (m, 1H), 1.95 - 2.03 (m, 2H), 1.29 - 1.42 (m, 13H). LC-MS(ESI): [M+H]$^+$ = 470.43.

Step 8: Compound 2-8h (5 mg, 0.01 mmol) and trifluoroacetic acid (0.5 mL) were dissolved in dichloromethane (3 mL). The mixture was stirred at room temperature for 1 h. The mixture was concentrated, and the crude product was purified by preparative HPLC to afford a white solid compound 2-8 (3 mg, 76.26%).

$^1$H NMR (600 MHz, DMSO-$d_6$) δ 7.32 (s, 1H), 7.08 (s, 1H), 5.07 - 5.00 (m, 1H), 4.34 (d, *J* = 16.8 Hz, 1H), 4.21 (d, *J* = 16.7 Hz, 1H), 4.04 - 3.96 (m, 2H), 3.18 - 3.06 (m, 4H), 2.92 - 2.84 (m, 1H), 2.85 (s, 2H), 2.64 - 2.53 (m, 1H), 2.42 - 2.31 (m, 1H), 2.02 - 1.95 (m, 1H), 1.66 - 1.52 (m, 4H). LC-MS(ESI): [M+H]$^+$ = 370.42.

35)

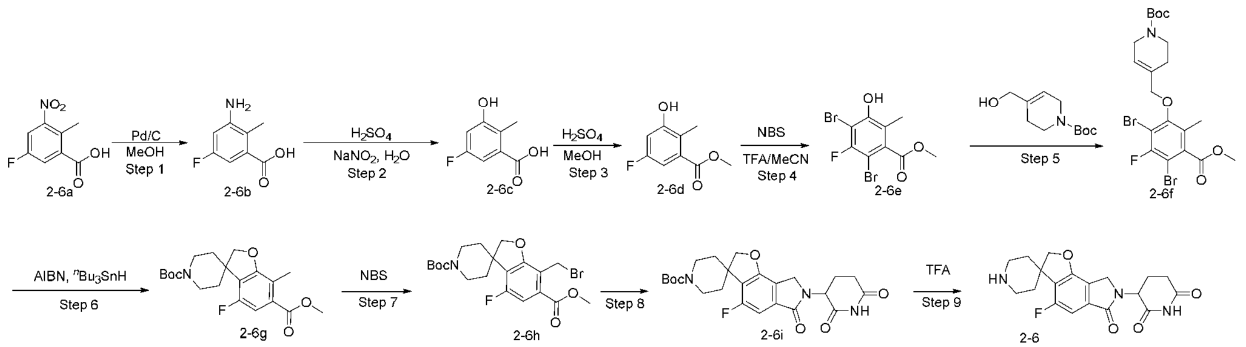


Step 1: Compound 2-6a (5.0 g, 25.11 mmol) was dissolved in methanol (50 mL), followed by the addition of palladium on carbon (4.0 g). The system was purged with hydrogen three times, and the mixture was stirred at room temperature for 12 h. After filtration, the filter cake was washed with methanol (15 mL × 3), and the filtrate was concentrated under reduced pressure to afford the crude product 2-6b (4.23 g, 99.59%), which was used directly in the next step without further purification.

$^1$H NMR (600 MHz, DMSO-$d_6$) δ 6.58 (dd, *J* = 9.4, 2.7 Hz, 1H), 6.54 (dd, *J* = 11.1, 2.7 Hz, 1H), 5.37 (s, 2H), 2.14 (s, 3H). LC-MS(ESI): [M+H]$^+$ = 170.25.

Step 2: Compound 2-6b (4.23 g, 25.01 mmol) was dissolved in a mixture of sulfuric acid and water (35 mL). The mixture was quickly heated to 90°C, stirred for 10 min, and then cooled to below 5°C. A solution of sodium nitrite (1.90 g, 27.51 mmol) in a small amount of water (3 mL) was added, and the mixture was stirred for 30 min. Urea (750.90 mg, 12.50 mmol) was then added, followed by the addition of an ice-cold mixture of sulfuric acid and water (5 mL). The reaction was heated to 90°C and stirred for 3 h. After cooling to room temperature, the mixture was concentrated under reduced pressure, and the pH was adjusted to 4-5 with sodium hydroxide solution. The aqueous layer was extracted with ethyl acetate (50 mL × 3), washed with saturated brine (50 mL), and the combined organic phases were dried over anhydrous sodium sulfate. The filtrate was concentrated under reduced pressure to afford the crude product 2-6c (3.72 g, 87.43%), which was used directly in the next step without further purification.

$^1$H NMR (600 MHz, DMSO-$d_6$) δ 6.91 (dd, *J* = 9.3, 2.6 Hz, 1H), 6.83 (dd, *J* = 10.3, 2.6 Hz, 1H), 2.24 (s, 3H). LC-MS(ESI): [M-H]$^-$ = 169.05.

Step 3: Methyl 5-fluoro-3-hydroxy-2-methylbenzoate (compound 2-6d)

Compound 2-6c (3.72 g, 21.86 mmol) was dissolved in methanol (16 mL), followed by the addition of sulfuric acid (1.6 mL). The reaction was heated to 60°C and stirred for 2 h. After cooling to room temperature, the mixture was concentrated, and water was added with rapid stirring. The filter cake was washed with water (50 mL), and the filtrate was concentrated at pH=7 to afford the crude product 2-6d (3.60 g, 89.40%), which was used directly in the next step without further purification.

$^1$H NMR (600 MHz, Chloroform-*d*) δ 7.75 (dd, *J* = 8.3, 2.8 Hz, 1H), 7.61 (dd, *J* = 7.3, 2.8 Hz, 1H), 3.95 (s, 3H), 2.59 (s, 3H). LC-MS(ESI): $[M-H]^-$ = 183.24.

Step 4: Methyl 2,4-dibromo-3-fluoro-5-hydroxy-6-methylbenzoate (Compound 2-6e)

Compound 2-6d (1.13 g, 6.14 mmol) was dissolved in acetonitrile (15 mL), followed by the addition of N-bromosuccinimide (2.40 g, 13.50 mmol) and dropwise addition of trifluoroacetic acid (1.5 mL). The reaction was heated to 50°C and stirred for 2 h, then further heated to 50°C and stirred for 12 h. The mixture was concentrated under reduced pressure, and the crude product was purified by reversed-phase column chromatography to afford compound 2-6e as a pale yellow solid (1.20 g, 57.19%).

$^1$H NMR (600 MHz, Chloroform-*d*) δ 5.83 (s, 1H), 3.96 (s, 3H), 2.23 (s, 3H). LC-MS(ESI): $[M+H]^+$ = 340.88.

Step 5: tert-butyl 4-((2,4-dibromo-3-fluoro-5-(methoxycarbonyl)-6-methylphenoxy)methyl)-3,6-dihydropyridine-1(2H)-carboxylate (Compound 2-6f)

Triphenylphosphine (1.86 g, 7.08 mmol) was dissolved in tetrahydrofuran (15 mL) and cooled to 0°C. Diisopropyl azodicarboxylate (1.43 g, 7.08 mmol) was added, and the mixture was stirred for 20 min. 4-(Hydroxymethyl)-3,6-dihydropyridine-1(2H)-carboxylic acid tert-butyl ester (1.13 g, 5.31 mmol) was added, and the mixture was stirred for 30 min. Compound 2-6e (1.21 g, 3.54 mmol) was then slowly added. After completion, the reaction was warmed to room temperature and stirred for 12 h. The mixture was concentrated under reduced pressure, and water (25 mL) was added. The aqueous layer was extracted with ethyl acetate (50 mL × 3), washed with saturated brine (50 mL), and the combined organic phases were dried over anhydrous sodium sulfate. The crude product was purified by reversed-phase column chromatography to afford a white solid compound 2-6f (952 mg, 50.08%).

$^1$H NMR (600 MHz, DMSO-$d_6$) δ 5.88 (s, 1H), 4.35 (s, 2H), 3.92 (s, 3H), 3.88 (s, 2H), 3.48 (t, *J* = 5.8 Hz, 2H), 2.27 - 2.24 (m, 2H), 2.20 (d, *J* = 1.2 Hz, 3H), 1.41 (s, 9H). LC-MS(ESI): $[M-tBu+H]^+$ = 482.00.

Step 6: Compound 2-6f(952 mg, 1.77 mmol), tri-n-butyltin hydride (1.03 g, 3.54 mmol), and azobisisobutyronitrile (873 mg, 5.32 mmol) were dissolved in toluene (10 mL). Under a nitrogen atmosphere, the reaction was heated to 110°C and stirred for 12 h. After cooling to room temperature, the mixture was concentrated under reduced pressure, and water (10 mL) was added. The aqueous layer was extracted with ethyl acetate (10 mL × 3), washed with saturated brine (10 mL), and the combined organic phases were dried over anhydrous sodium sulfate. The crude product was purified by column chromatography to afford a yellow solid compound 2-6g (416 mg, 61.87%).

LC-MS (ESI): $[M-Boc+H]^+$ = 280.28.

Step 7: Compound 2-6g (416 mg, 1.10 mmol) was dissolved in carbon tetrachloride (8 mL), followed by the addition of N-bromosuccinimide (195 mg, 1.10 mmol) and azobisisobutyronitrile (18 mg, 0.11 mmol). The reaction was heated to 85°C and stirred for 12 h. The mixture was concentrated under reduced pressure to afford the crude product 2-6h (416 mg, 82.79%), which was used directly in the next step without further purification.

Step 8: Compound 2-6h (416 mg, 0.91 mmol), 3-amino-2,6-piperidinedione hydrochloride (224 mg, 1.36 mmol), and N,N-diisopropylethylamine (352 mg, 2.72 mmol) were dissolved in acetonitrile (8 mL). The reaction was heated to 85°C and stirred for 12 h. The mixture was concentrated under reduced pressure, and the crude product was purified by column chromatography to afford compound 2-6i as a pale blue solid (55 mg, 12.80%).

LC-MS (ESI): $[M+H]^+$ = 474.39.

Step 9: 3-(4-Fluoro-6-oxo-6,8-dihydro-2H,7H-spiro[furano[2,3-e]isoindole-3,4'-piperidine]-7-yl)piperidine-2,6-dione (compound 2-6)

Compound 2-6i (55 mg, 0.12 mmol) was dissolved in dichloromethane (5 mL), followed by the dropwise addition of trifluoroacetic acid (0.5 mL). The mixture was stirred at room temperature for 2 h. The filtrate was concentrated under reduced pressure, and the crude product was purified by reversed-phase column chromatography to afford a white solid compound 2-6 (10 mg, 23.06%).

$^1$H NMR (600 MHz, DMSO-$d_6$) δ 11.00 (s, 1H), 7.11 (d, *J* = 8.5 Hz, 1H), 5.10 (dd, *J* = 13.3, 5.1 Hz, 1H), 4.77 - 4.72 (m, 2H), 4.38 (d, *J* = 17.1 Hz, 1H), 4.22 (d, *J* = 17.0 Hz, 1H), 3.06 - 2.97 (m, 2H), 2.95 - 2.87 (m, 1H), 2.63 - 2.57 (m, 1H), 2.46 -

2.38 (m, 1H), 2.35 -2.28 (m, 2H), 2.03 - 1.95 (m, 4H). LC-MS(ESI): $[M+H]^+$ = 374.29.

36)

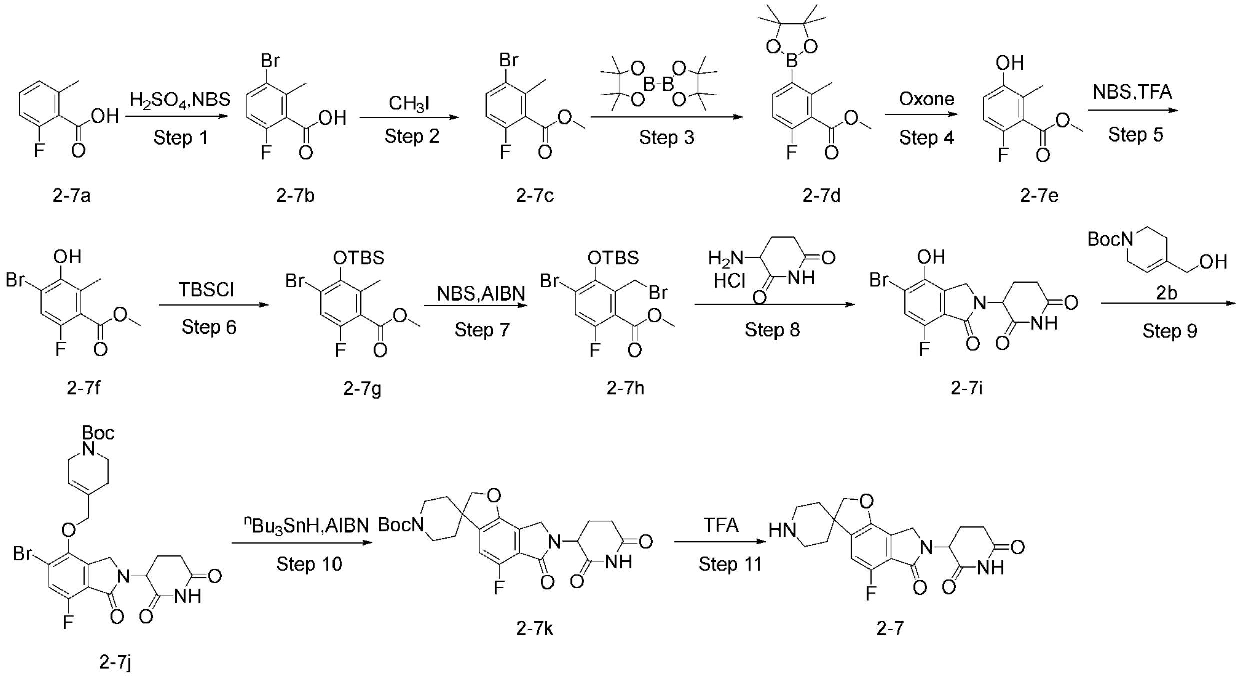


2-7a 2-7b 2-7c 2-7d 2-7e 2-7f 2-7g 2-7h 2-7i 2-7j 2-7k 2-7

Step 1: Compound 2-7a (5.0 g, 32.4 mmol) was dissolved in sulfuric acid (50 mL), and N-bromosuccinimide (6.1 g, 34.1 mmol) was added at room temperature. The mixture was stirred at room temperature for 12 h. The reaction was quenched with ice water. The mixture was extracted with ethyl acetate three times. The organic phase was concentrated to afford a white solid crude product 2-7b (5.0 g).

LC-MS (ESI): $[M-H]^-$ = 231.16.

Step 2: Methyl 3-bromo-6-fluoro-2-methylbenzoate(compound 2-7c)

Compound 2-7b (5.0 g, 21.5 mmol) was dissolved in acetonitrile (50 mL), followed by the addition of cesium carbonate (10.5 g, 32.2 mmol) and iodomethane (6.1 g, 42.9 mmol). The mixture was stirred at room temperature for 12 h. The mixture was concentrated, and the crude product was purified by column chromatography to afford a white solid compound 2-7c (4.5 g, 85%).

$^1$H NMR (600 MHz, DMSO-$d_6$) δ 7.80 (dd, *J* = 8.9, 5.4 Hz, 1H), 7.21 (t, *J* = 9.0 Hz, 1H), 3.91 (s, 3H), 2.33 (s, 3H).

Step 3: Compound 2-7c (2.0 g, 8.1 mmol), bis(pinacolato)diboron (3.1 g, 12.1 mmol), and potassium acetate (2.4 g, 24.3 mmol) were dissolved in anhydrous 1,4-dioxane (20 mL). [1,1'-Bis(diphenylphosphino)ferrocene]palladium(II) dichloride (118 mg, 0.2 mmol) was added. Under nitrogen atmosphere, the reaction was heated to 90°C and stirred for 12 h. The mixture was concentrated, and the crude product was purified by column chromatography to afford a white solid compound 2-7d (1.8 g, 76%).

$^1$H NMR (600 MHz, DMSO-$d_6$) δ 7.77 (dd, *J* = 8.4, 6.7 Hz, 1H), 7.16 (t, *J* = 9.0 Hz, 1H), 3.88 (s, 3H), 2.45 (s, 3H), 1.30 (s, 12H). LC-MS(ESI): $[M+H]^+$ = 295.28.

Step 4: Methyl 6-fluoro-3-hydroxy-2-methylbenzoate (compound 2-7e)

Compound 2-7d (1.8 g, 6.1 mmol) was dissolved in a mixture of acetonitrile and water (20:1, 20 mL), and potassium peroxymonosulfate (3.8 g, 6.1 mmol) was added under ice cooling. The mixture was stirred at room temperature for 2 h. The reaction was quenched with ice water. The mixture was extracted with ethyl acetate three times. The organic phase was concentrated, and the crude product was purified by column chromatography to afford compound 2-7e as a white solid (850 mg, 75%).

$^1$H NMR (600 MHz, DMSO-$d_6$) δ 9.69 (s, 1H), 6.95 (t, *J* = 9.1 Hz, 1H), 6.89 (dd, *J* = 8.9, 4.9 Hz, 1H), 3.86 (s, 3H), 2.08 (s, 3H). LC-MS(ESI): $[M-H]^-$= 183.03.

Step 5: Methyl 4-bromo-6-fluoro-3-hydroxy-2-methylbenzoate (compound 2-7f)

Compound 2-7e (500 mg, 2.7 mmol) was dissolved in acetonitrile (5 mL), followed by the addition of trifluoroacetic acid (0.5 mL) and N-bromosuccinimide (966 mg, 5.4 mmol). The mixture was stirred at room temperature for 2 h. The mixture was concentrated, and the crude product was purified by column chromatography to afford compound 2-7f as a colorless oil (500 mg, 70%).

$^1$H NMR (600 MHz, DMSO-$d_6$) δ 9.40 (s, 1H), 7.47 (d, *J*= 8.9 Hz, 1H), 3.87 (s, 3H), 2.19 (s, 3H). LC-MS(ESI): $[M+H]^-$ = 263.17.

Step 6: Compound 2-7f (300 mg, 1.1 mmol) was dissolved in dichloromethane (5 mL), followed by the addition of imidazole (155 mg, 2.28 mmol) and tert-butyldimethylsilyl chloride (258 mg, 1.7 mmol) under ice bath conditions. The mixture was stirred at room temperature for 1 h. The mixture was concentrated, and the crude product was purified by column chromatography to afford compound 2-7g as a white solid (350 mg, 81%).

$^1$H NMR (600 MHz, DMSO-$d_6$) δ 7.57 (d, *J* = 8.8 Hz, 1H), 3.88 (s, 3H), 2.20 (s, 3H), 1.01 (s, 9H), 0.26 (s, 6H).

Step 7: Compound 2-7g (300 mg, 0.8 mmol) was dissolved in carbon tetrachloride (5 mL), followed by the addition of N-bromosuccinimide (170 mg, 1.0 mmol) and azobisisobutyronitrile (13 mg, 0.08 mmol). Under nitrogen atmosphere, the reaction was heated to 85°C and stirred for 12 h. The mixture was concentrated to afford a yellow oily crude product 2-7h.

Step 8: Compound 2-7h (300 mg, 0.7 mmol) was dissolved in acetonitrile (5 mL), followed by the addition of 3-amino-2,6-piperidinedione hydrochloride (162 mg, 1.0 mmol) and N,N-diisopropylethylamine (255 mg, 2.0 mmol). The reaction was heated to 80°C and stirred for 12 h. The mixture was concentrated, and the crude product was purified by column chromatography to afford a pale blue solid compound 2-7i (130 mg, 55%).

$^1$H NMR (600 MHz, DMSO-$d_6$) δ 11.02 (s, 1H), 10.36 (s, 1H), 7.59 (d, *J* = 8.4 Hz, 1H), 5.07 (dd, *J*= 13.3, 5.1 Hz, 1H), 4.41 (d, *J* = 17.8 Hz, 1H), 4.28 (d, *J* = 17.8 Hz, 1H), 2.90 (ddd, *J* = 17.4, 13.6, 5.4 Hz, 1H), 2.64 - 2.57 (m, 1H), 2.35 (qd, *J* = 13.3, 4.6 Hz, 1H), 2.06 - 2.00 (m, 1H). LC-MS(ESI): $[M+H]^+$ = 359.09.

Step 9: Compound 2-7i (100 mg, 0.3 mmol), tert-butyl 4-(hydroxymethyl)-3,6-dihydropyridine-1(2H)-carboxylate (90 mg, 0.4 mmol), and triphenylphosphine (147 mg, 0.6 mmol) were dissolved in tetrahydrofuran (1 mL). Under nitrogen atmosphere, the mixture was stirred at 0°C for 15 min, followed by the dropwise addition of diethyl azodicarboxylate (98 mg, 0.6 mmol). The mixture was stirred at room temperature for 15 h. The mixture was concentrated, and the crude product was purified by column chromatography to afford a white solid compound 2-7j (50 mg, 32%).

LC-MS (ESI): $[M-Boc+H]^+$ = 454.19.

Step 10: Compound 2-7j (50 mg, 0.09 mmol) was dissolved in toluene (1 mL), followed by the addition of azobisisobutyronitrile (22 mg, 0.1 mmol) and tri-n-butyltin hydride (79 mg, 0.3 mmol). The reaction was heated to 110°C and stirred for 15 h. The mixture was concentrated, and the crude product was purified by column chromatography to afford a white solid compound 2-7k (25 mg, 58%).

LC-MS (ESI): $[M+H]^+$ = 474.39.

Step 11: Compound 2-7k (20 mg, 0.04 mmol) was dissolved in a mixture of dichloromethane and trifluoroacetic acid (10:1, 1 mL). The mixture was stirred at room temperature for 2 h. The mixture was concentrated, and the crude product was purified by reversed-phase column chromatography to afford a white solid compound 2-7 (10 mg, 63%).

$^1$H NMR (400 MHz, DMSO-$d_6$) δ 11.00 (s, 1H), 8.71 (s, 1H), 7.18 (d, *J* = 8.8 Hz, 1H), 5.05 (dd, *J* = 13.3, 5.1 Hz, 1H), 4.70 - 4.62 (m, 2H), 4.40 (d, *J* = 17.7 Hz, 1H), 4.23 (d, *J* = 17.6 Hz, 1H), 3.11 - 2.84 (m, 4H), 2.66 - 2.55 (m, 1H), 2.49 - 2.34 (m, 2H), 2.05 - 1.86 (m, 5H). LC-MS(ESI): $[M+H]^+$ = 374.35.

The PROTAC molecules are synthesized according to the following General Synthesis Formula, wherein: each occurrence of n1, n2, n3, and n4, is independently selected from an integer, preferably selected from 1, 2, 3, 4, 5, and 6; In the following PROTAC General Synthesis Formulas 1-82, R and $R_1$ are commonly used substituents, each occurrence independently selected from H, halogen, $C_{1-8}$ alkyl, O-$C_{1-8}$ alkyl, S-$C_{1-8}$ alkyl, NH-$C_{1-8}$ alkyl, N($C_{1-8}$ alkyl)$_2$, $C_{3-11}$ cycloalkyl, aryl, heteroaryl, $C_{3-11}$ heterocyclyl, O-$C_{3-8}$ cycloalkyl, O-$C_{3-11}$ heterocyclyl, C(O)-$C_{3-8}$ cycloalkyl, C(O)-$C_{3-11}$ heterocyclyl, O-aryl, O-heteroaryl, S-$C_{3-8}$ cycloalkyl, NH-$C_{3-8}$ cycloalkyl, N($C_{3-8}$ cycloalkyl)$_2$, N($C_{3-8}$ cycloalkyl)($C_{1-8}$ alkyl), N($C_{1-8}$ alkylene)($C_{3-8}$ cycloalkyl), NH-$C_{3-8}$ heterocyclyl, N($C_{3-8}$ heterocyclyl)$_2$, N($C_{3-8}$ heterocyclyl)($C_{1-8}$ alkyl), NH-aryl, N(aryl)($C_{1-8}$ alkyl), NH-heteroaryl, N(heteroaryl)($C_{1-8}$ alkyl), OH, $NH_2$, SH, $S(O)_2$, P(O)(O-$C_{1-8}$ alkyl)($C_{1-8}$ alkyl), P(O)(O-$C_{1-8}$ alkyl)$_2$, C≡C-$C_{1-8}$ alkyl, C≡CH, CH=CH-($C_{1-8}$ alkyl), C($C_{1-8}$ alkyl)=CH-($C_{1-8}$ alkyl), C($C_{1-8}$ alkyl)=C($C_{1-8}$ alkyl)$_2$, $Si(OH)_3$, Si($C_{1-8}$ alkyl)$_3$, Si(OH)($C_{1-8}$ alkyl)$_2$, C(O)-$C_{1-8}$ alkyl, C(0)O-$C_{1-8}$ alkyl, $CO_2H$, CN, $CF_3$, $CHF_2$, $CH_2F$, $NO_2$, $SF_5$, $S(O)_2$NH-$C_{1-8}$ alkyl, $S(O)_2$N($C_{1-8}$ alkyl)$_2$, S(O)NH-$C_{1-8}$ alkyl, S(O)N($C_{1-8}$ alkyl)$_2$, C(O)NH-$C_{1-8}$ alkyl, C(O)NH-$C_{3-8}$ cycloalkyl, C(O)NH-$C_{3-11}$ heterocyclyl, C(O)N($C_{1-8}$ alkyl)$_2$, N($C_{1-8}$ alkyl)C(O)NH($C_{1-8}$ alkyl), N($C_{1-8}$ alkyl)C(O)N($C_{1-8}$ alkyl)$_2$, NHC(O)NH($C_{1-8}$ alkyl), NHC(O)N($C_{1-8}$ alkyl)$_2$, NHC(O)$NH_2$, N($C_{1-8}$ alkyl)$S(O)_2$NH($C_{1-8}$ alkyl), N($C_{1-8}$ alkyl) $S(O)_2$N($C_{1-8}$ alkyl)$_2$, NH$S(O)_2$NH($C_{1-8}$ alkyl), NH$S(O)_2$N($C_{1-8}$ alkyl)$_2$, and NH$S(O)_2NH_2$; optionally, the $C_{1-8}$ alkyl, $C_{3-11}$ cycloalkyl, $C_{3-8}$ cycloalkyl, $C_{3-11}$ heterocyclyl, $C_{6-10}$ aryl, and 5-10 membered heteroaryl may each be independently substituted with one or more substituents selected from halogen, alkyl, heteroalkyl, alkenyl, alkynyl, alkoxy, hydroxyl, haloalkyl, hydroxyalkyl, cyano, amino, nitro, cycloalkyl, heterocyclyl, halocycloalkyl, haloheteroalkyl, alkylamino, aryl,

heteroaryl, haloaryl, and haloheteroaryl; preferably, the $C_{1-8}$ alkyl, $C_{3-11}$ cycloalkyl, $C_{3-8}$ cycloalkyl, $C_{3-11}$ heterocyclyl, $C_{6-10}$ aryl, and 5-10 membered heteroaryl may each be independently substituted with one or more substituents selected from F, Cl, Br, I, $C_{1-6}$ alkyl, methoxy, and ethoxy.

Additionally, for the purpose of illustrative description in this invention, the PROTAC General Synthesis Formulas 1-82 described herein are using E3 ligase inhibitors as examples

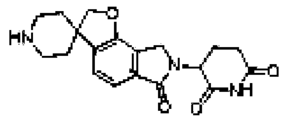

.

However, these E3 ligase inhibitors may be replaced with any E3 ligase inhibitor provided in the present invention, such as

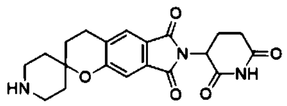

and

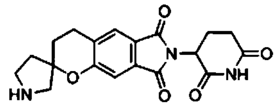

,

etc.

General Synthesis Formula 1 for Intermediates

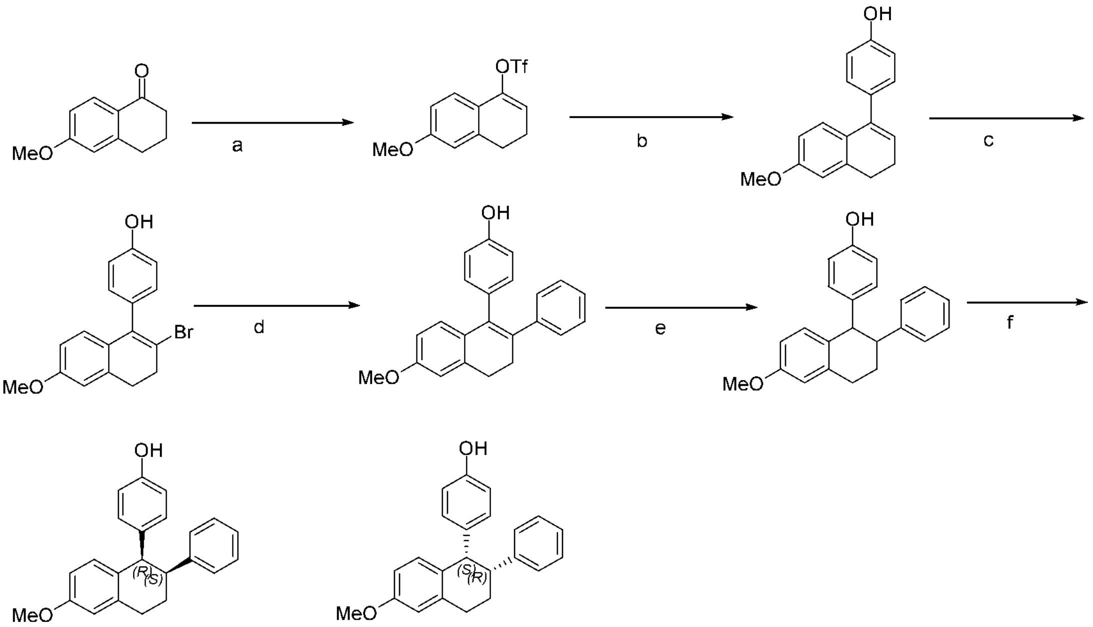


a:LDA(2.0eq) 2-MeTHF(10V) -20°C-25°C 2h

b:4-Hydroxyphenylboronic acid(1.0eq) Pd(dppf)$Cl_2$(0.05eq) $K_2CO_3$(2.0eq) 1,4-dioxane/water=10/4(14V) 25°C 2h

c:NBS(1.0eq) ACN(20V) 25°C 2h

d:phenylboronic acid(1.0eq) Pd(dppf)$Cl_2$(0.05eq) $Cs_2CO_3$(1.85eq) 1,4-dioxane/water=2/1(10V) 25°C-40°C 2h

e: Pd/C(20%) $H_2$ MeOH(10V) 40°C 4h

f: Chiral-IC, Column size : 0.46 cm I.D. ¡Á 15 cm L Mobile phase : $CO_2$/(MeOH70/DCM30)=80/20(V/V). Flow rate : 2.0 ml/min

General Synthesis Formula 1 for PROTACs

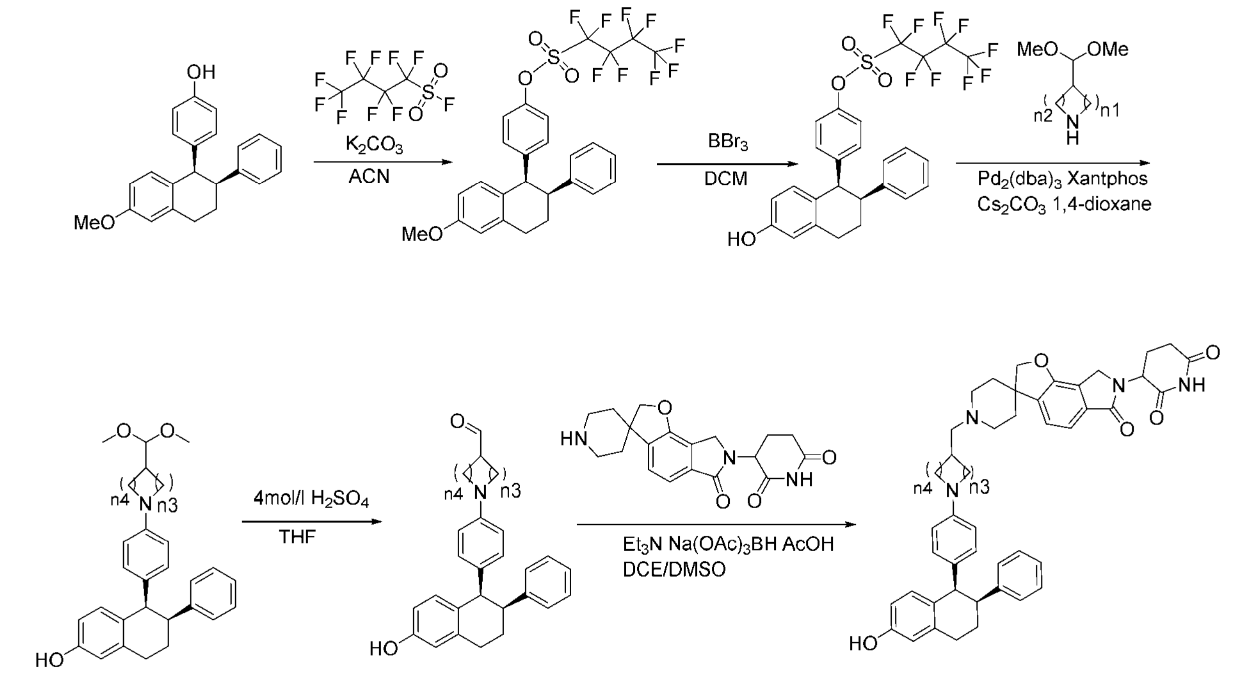


General Synthesis Formula 2 for PROTACs

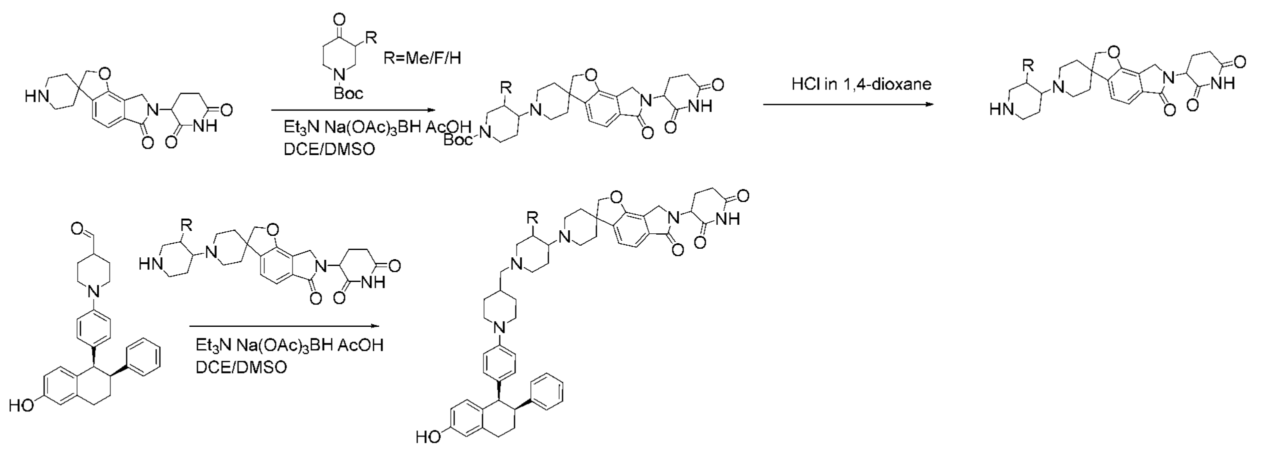


General Synthesis Formula 3 for PROTACs

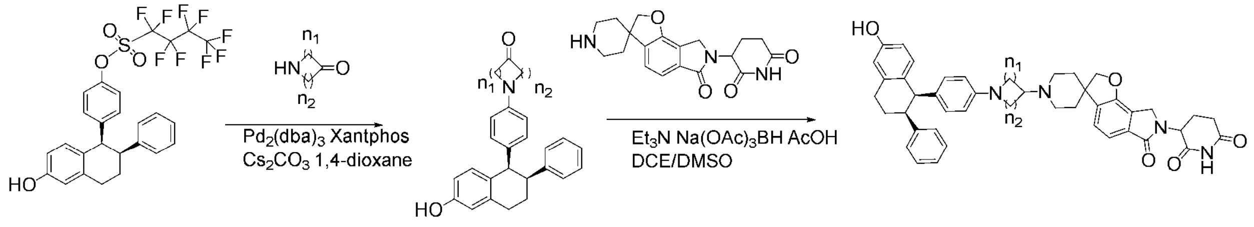


General Synthesis Formula 4 for PROTACs

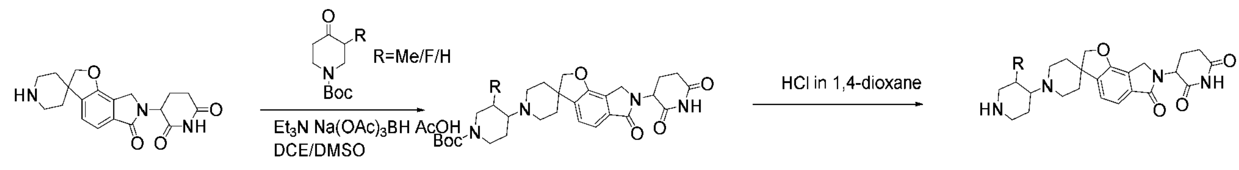


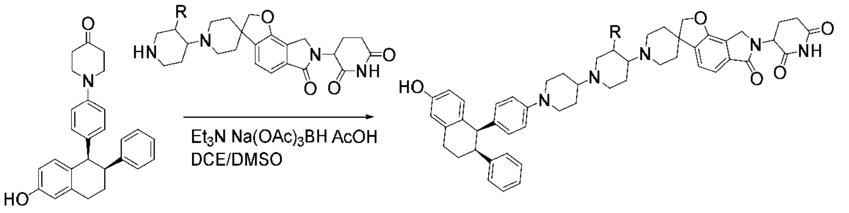


General Synthesis Formula 5 for PROTACs

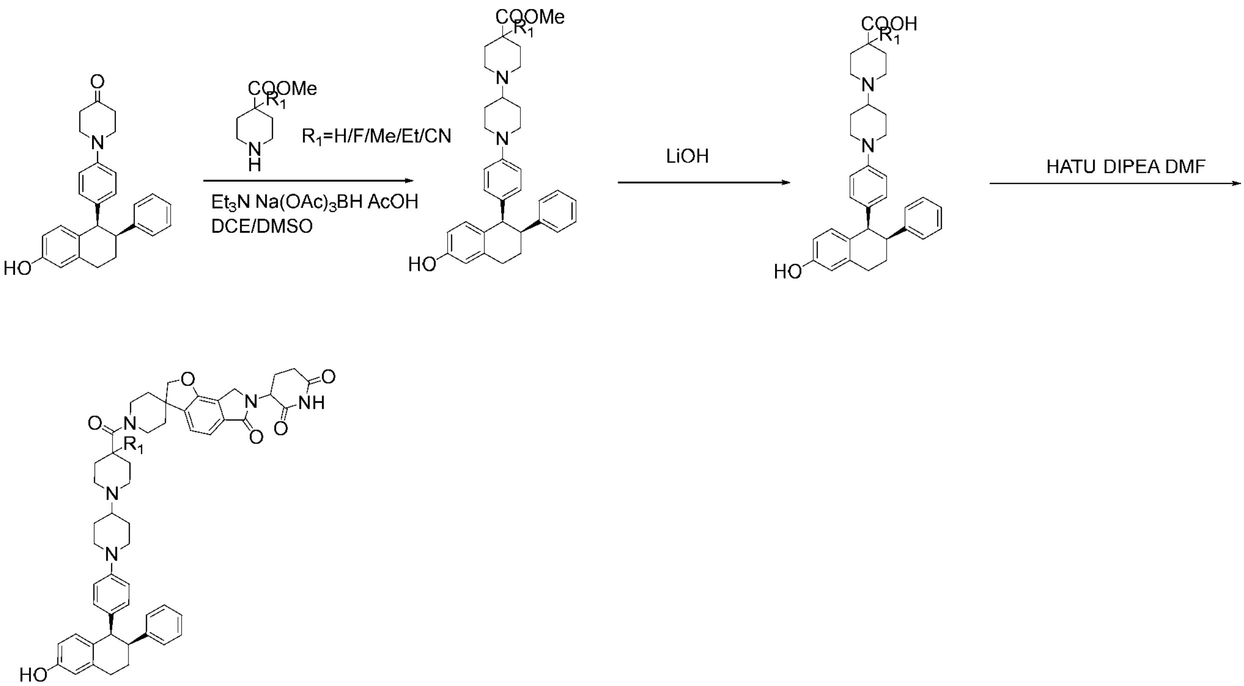


General Synthesis Formula 6 for PROTACs

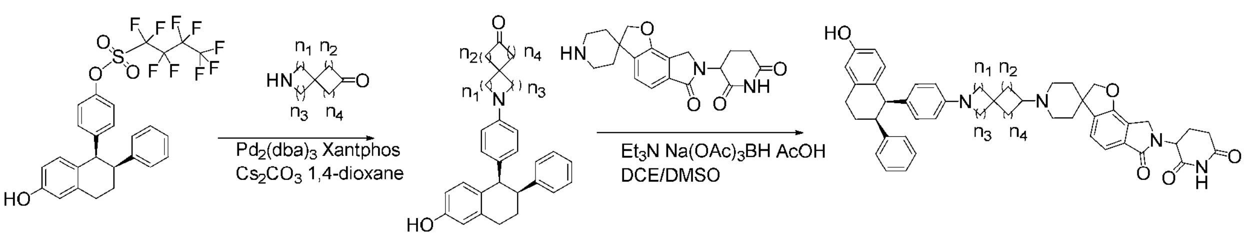


General Synthesis Formula 7 for PROTACs

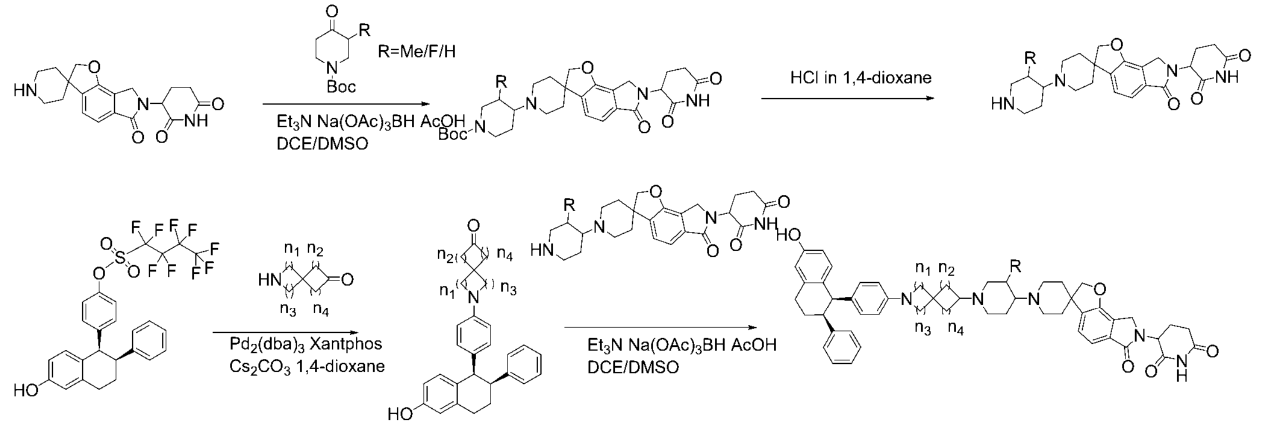


General Synthesis Formula 8 for PROTACs

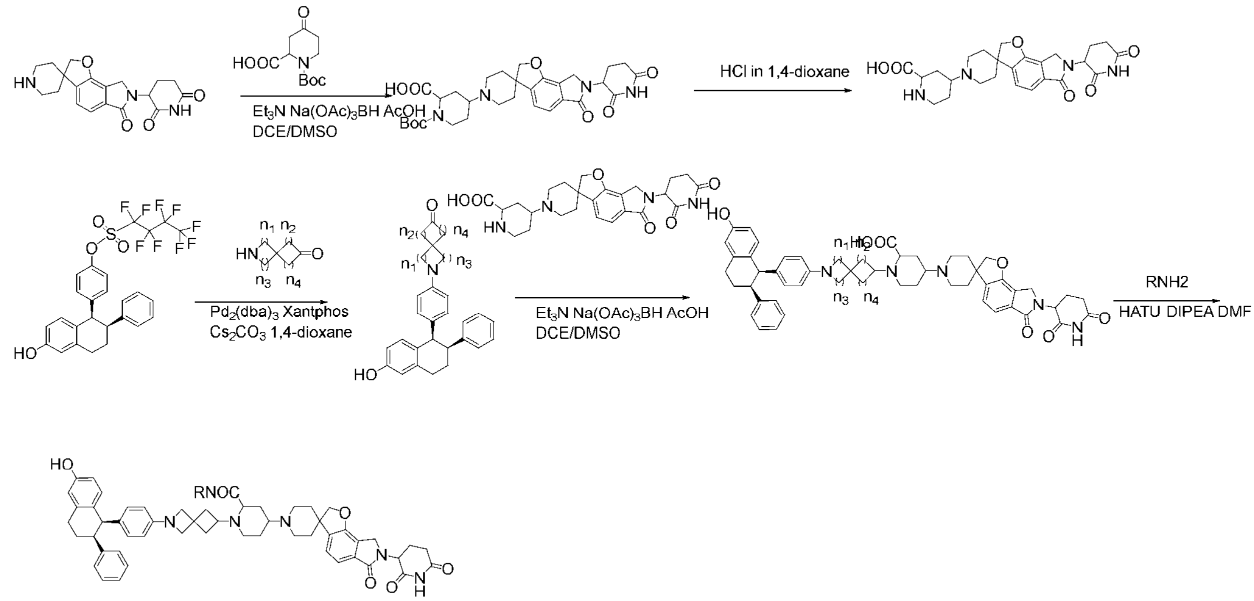


General Synthesis Formula 9 for PROTACs

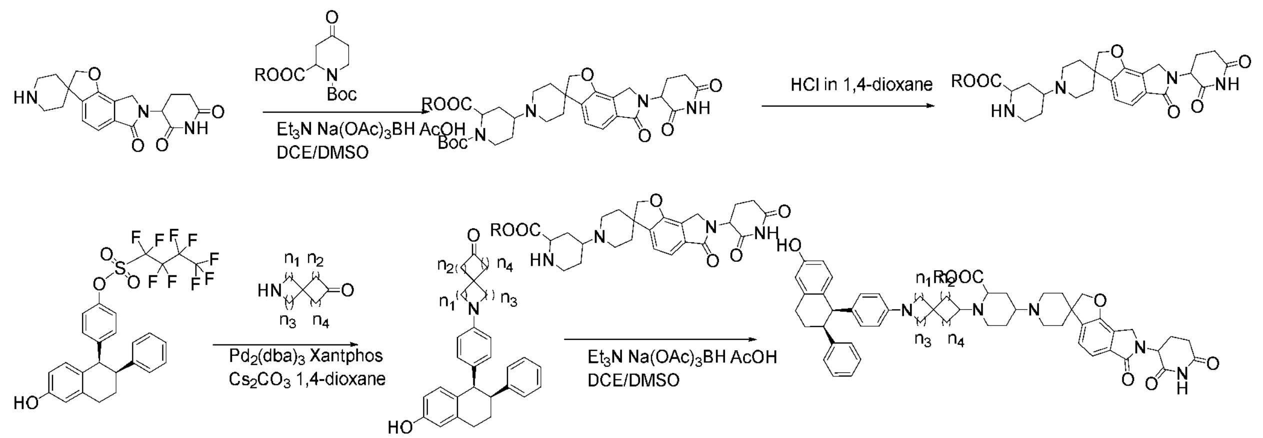

General Synthesis Formula 10 for PROTACs

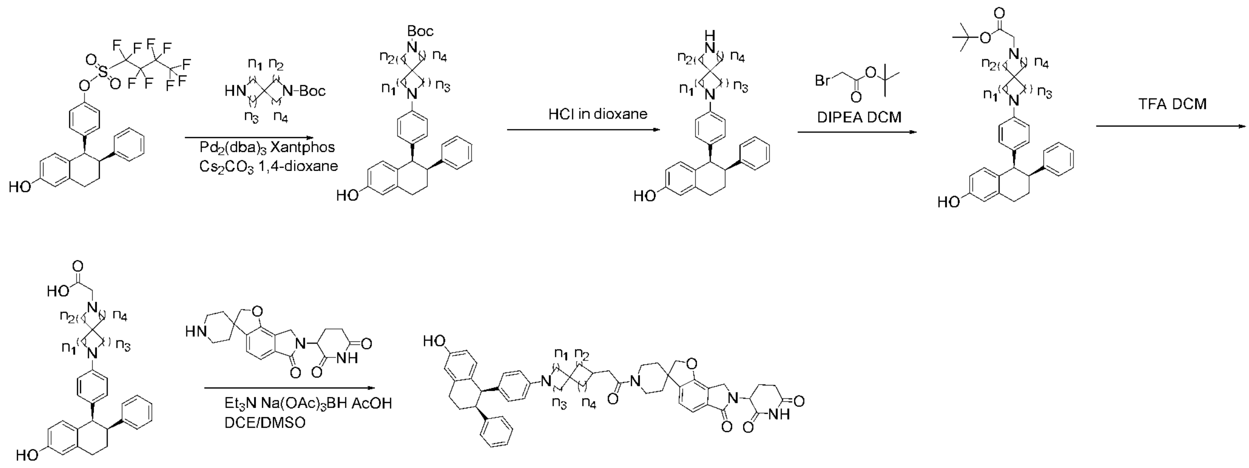


General Synthesis Formula 11 for PROTACs

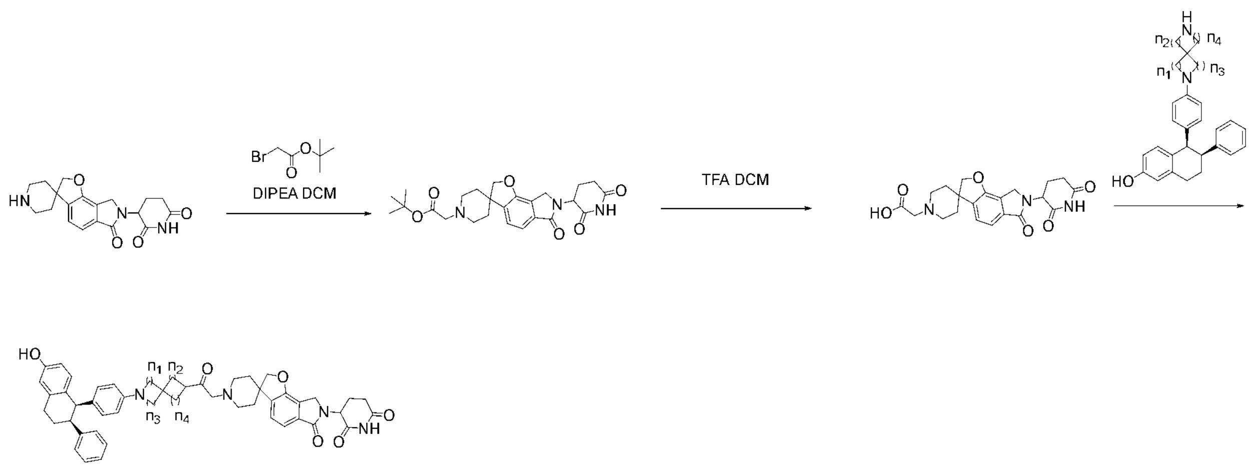


General Synthesis Formula 12 for PROTACs

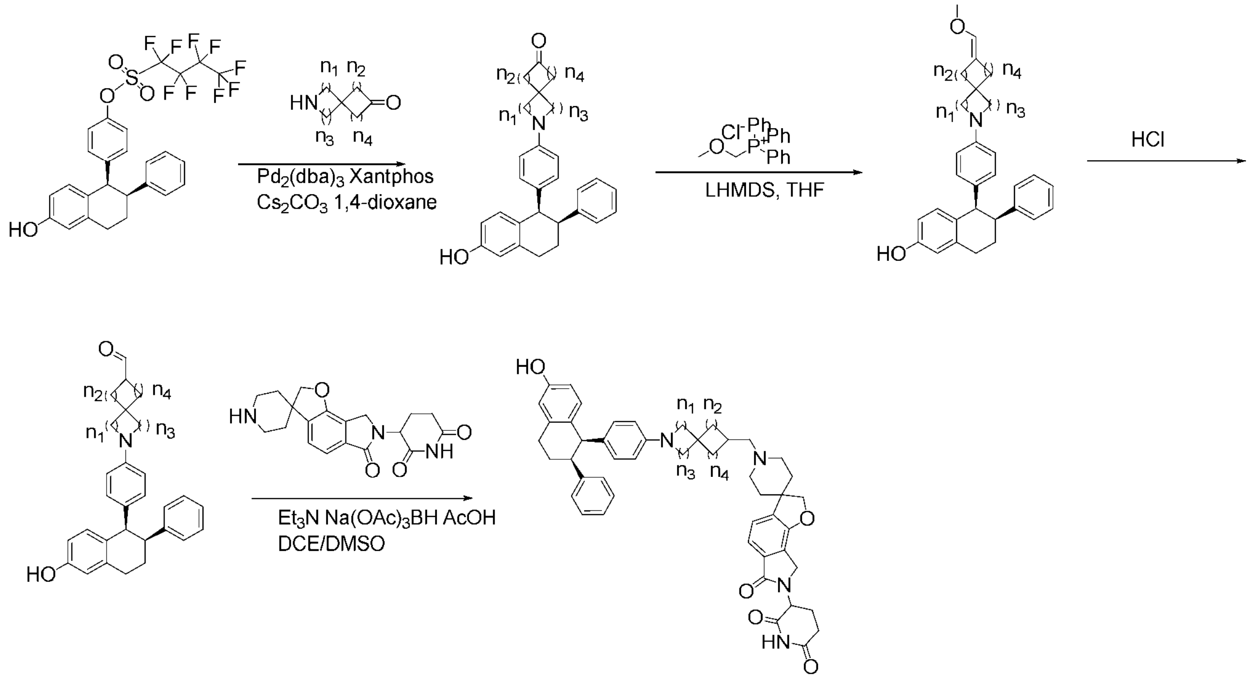


General Synthesis Formula 13 for PROTACs

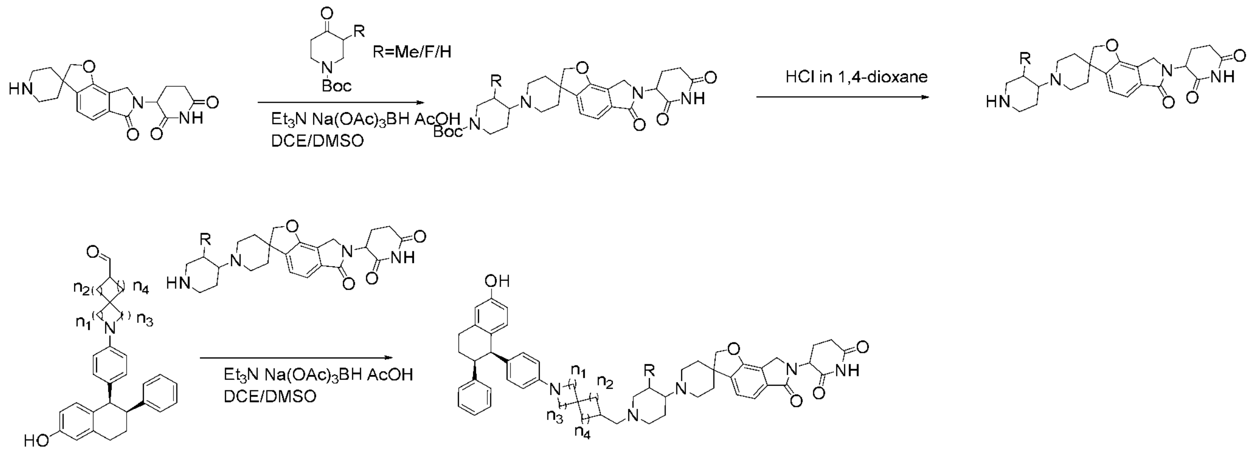


General Synthesis Formula 14 for PROTACs

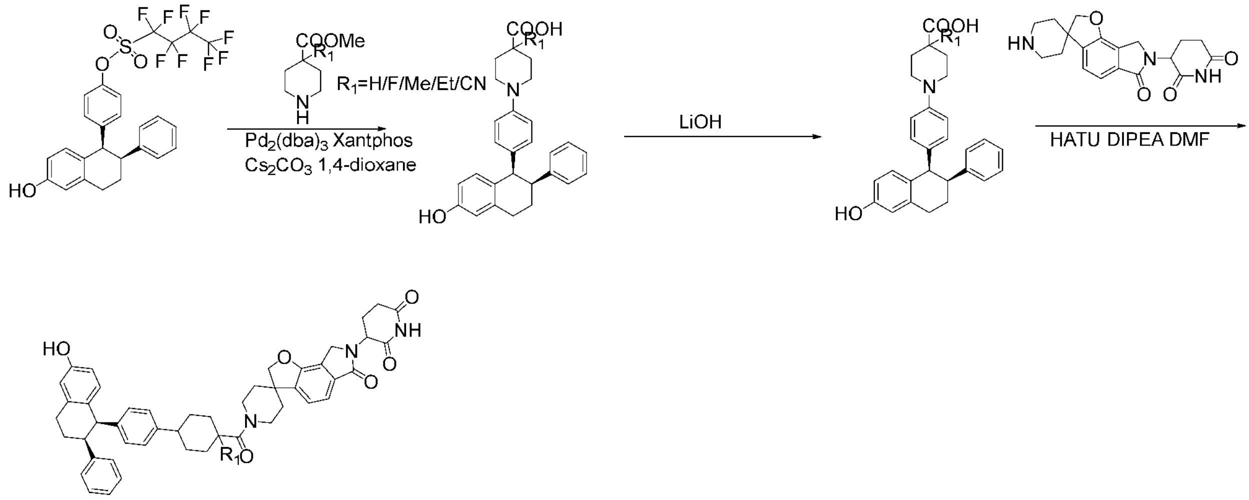


General Synthesis Formula 15 for PROTACs

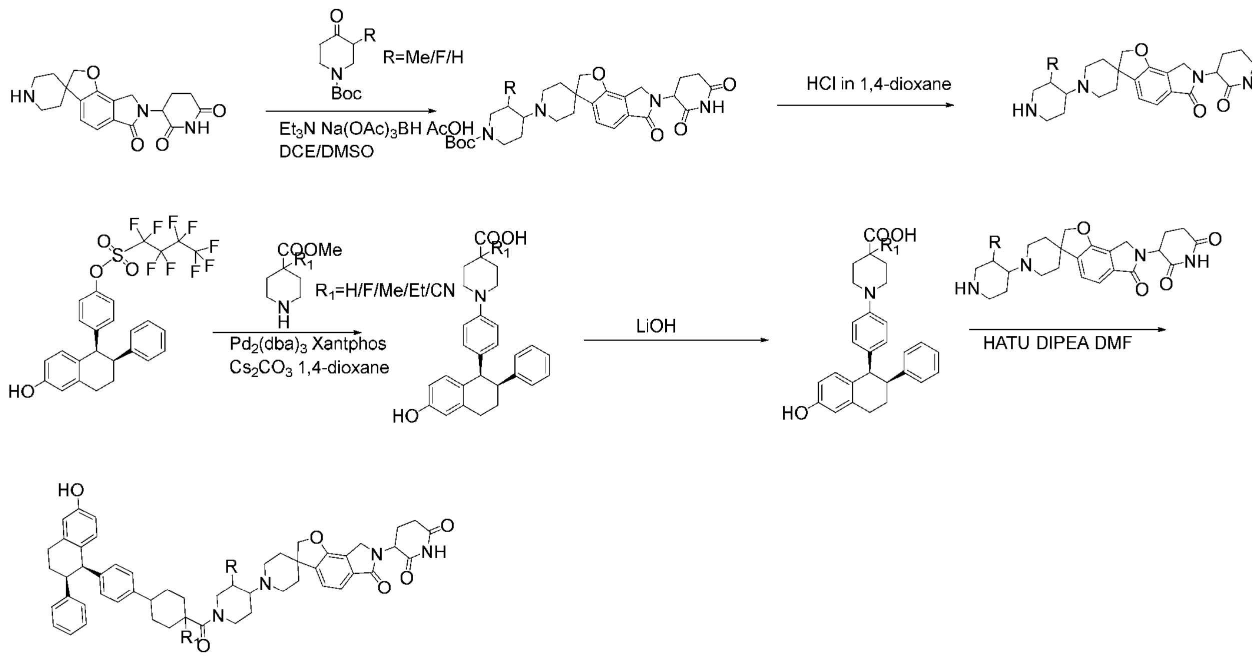


General Synthesis Formula 16 for PROTACs

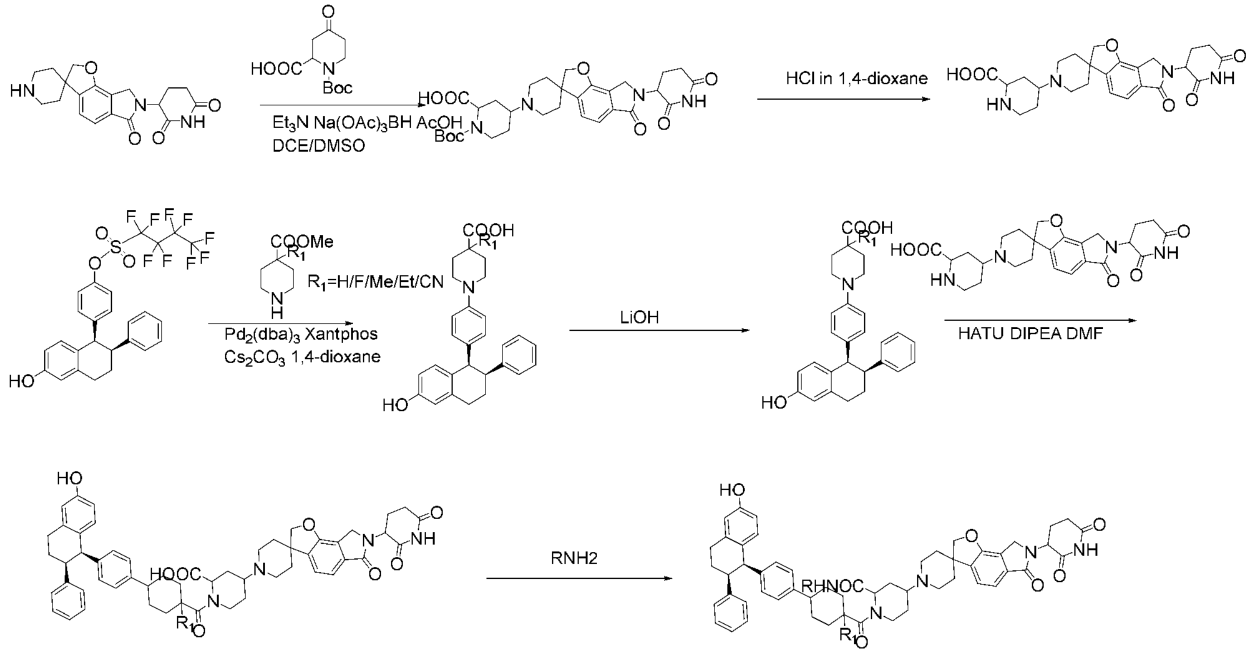


General Synthesis Formula 17 for PROTACs

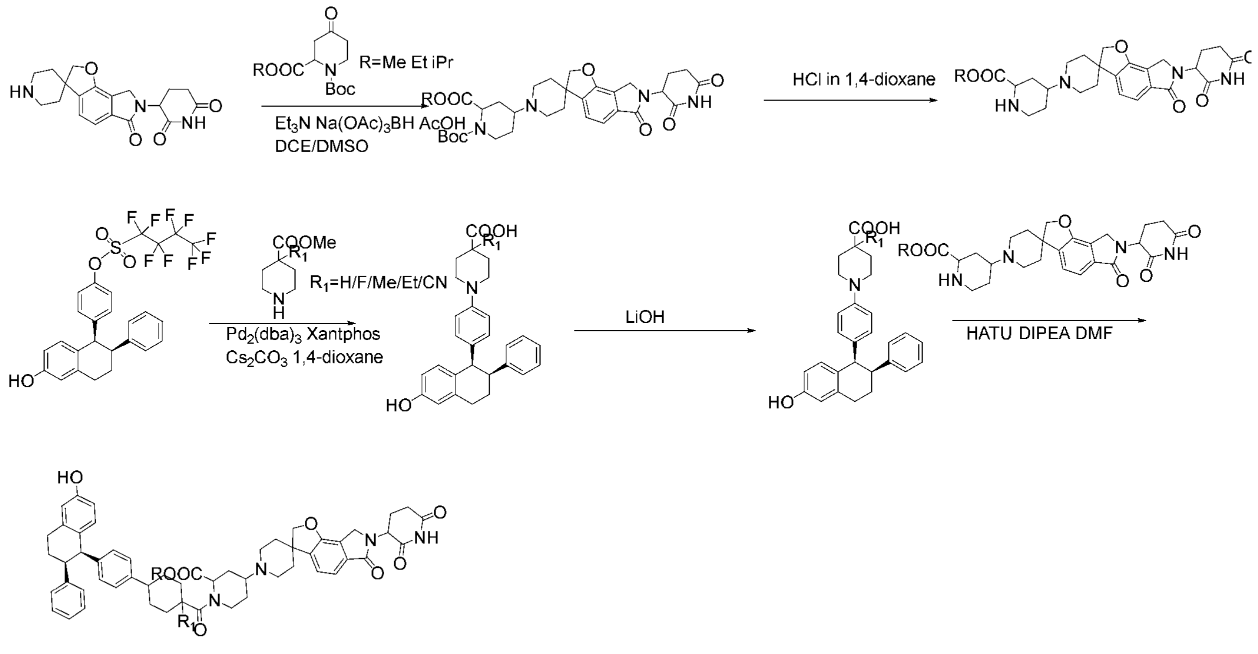


General Synthesis Formula 18 for PROTACs

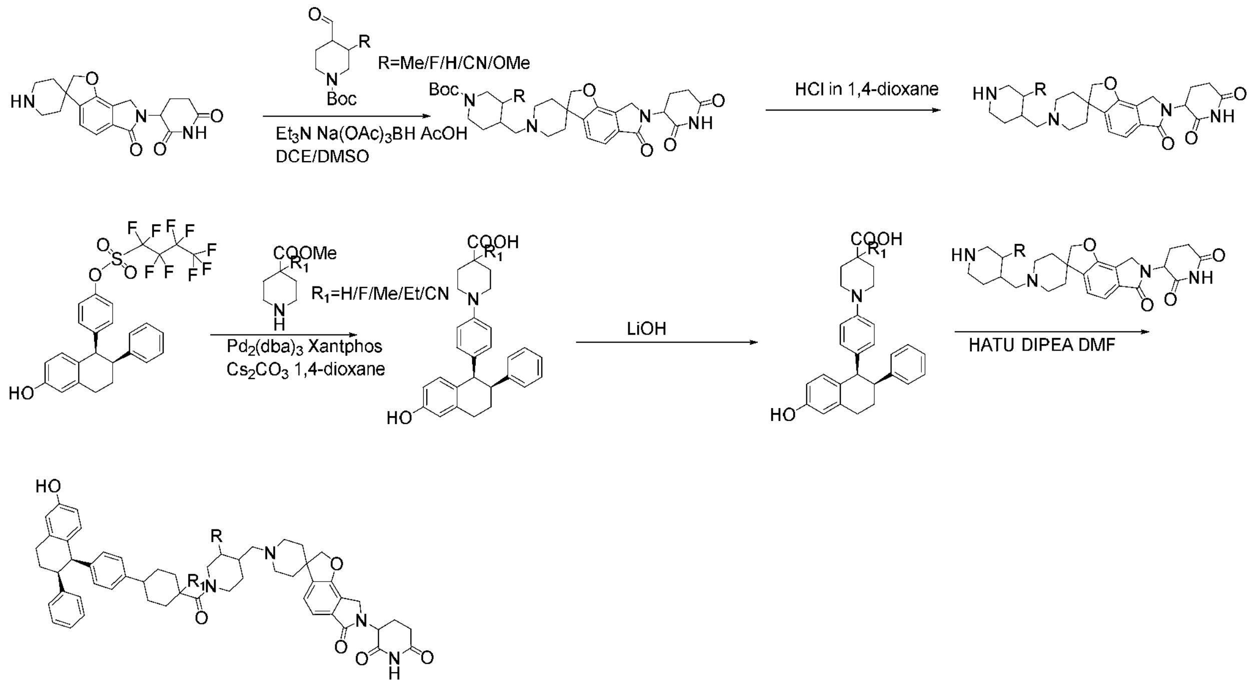


General Synthesis Formula 19 for PROTACs

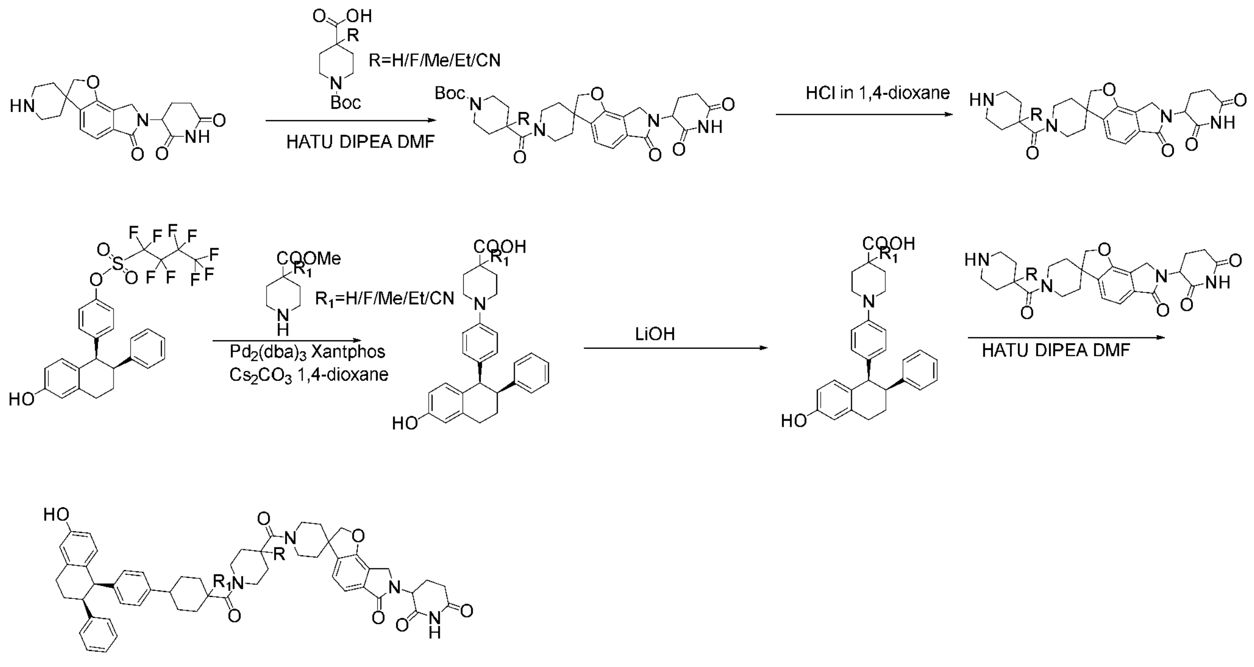


General Synthesis Formula 20 for PROTACs

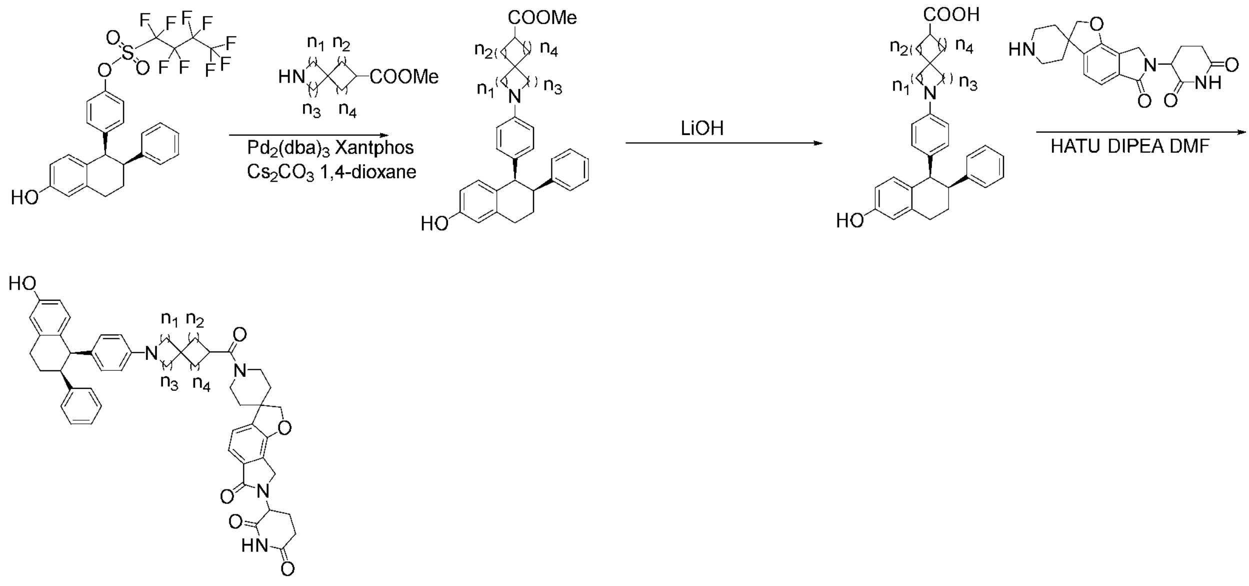


General Synthesis Formula 21 for PROTACs

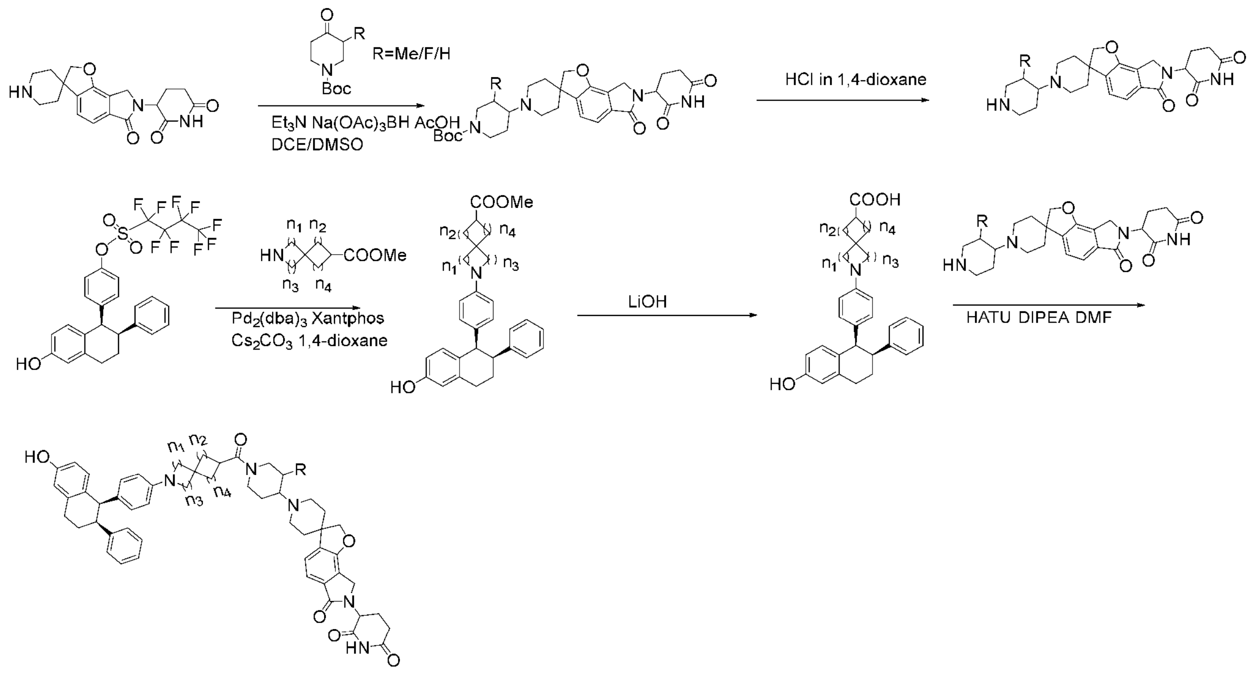


General Synthesis Formula 22 for PROTACs

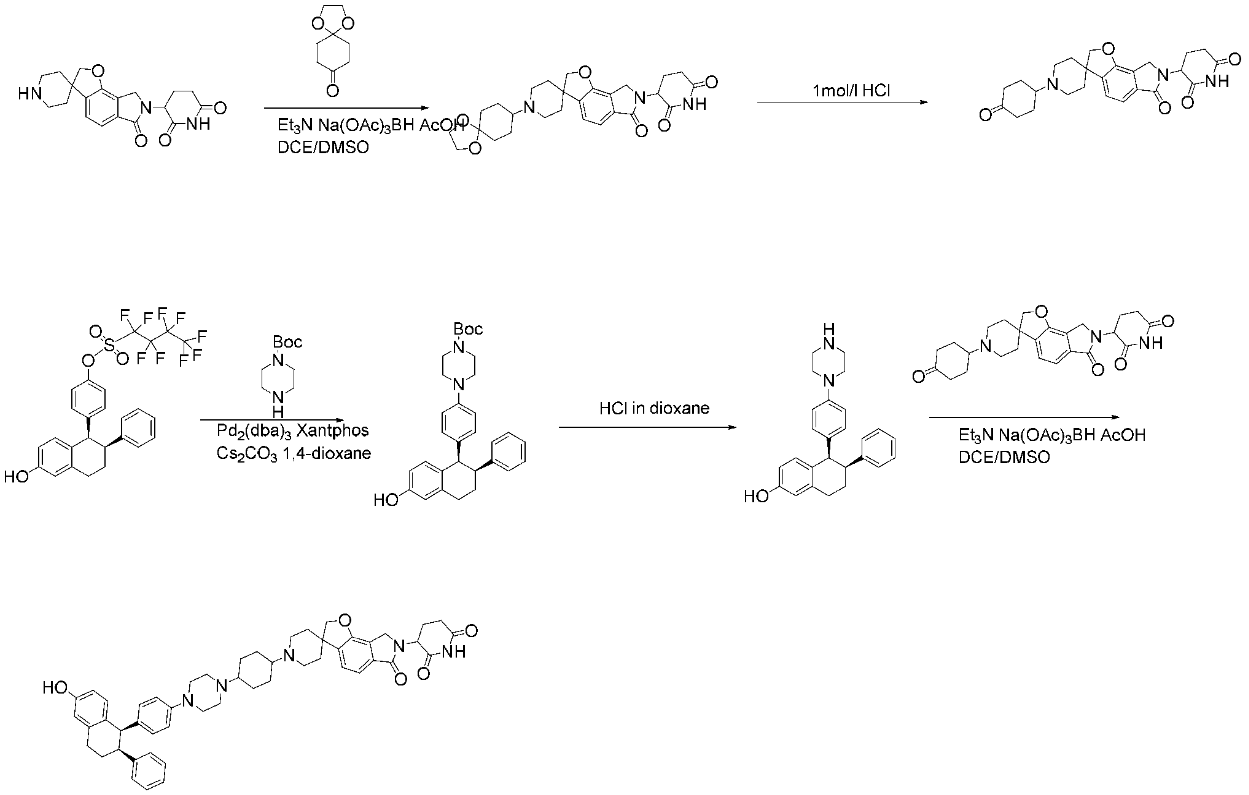


General Synthesis Formula 23 for PROTACs

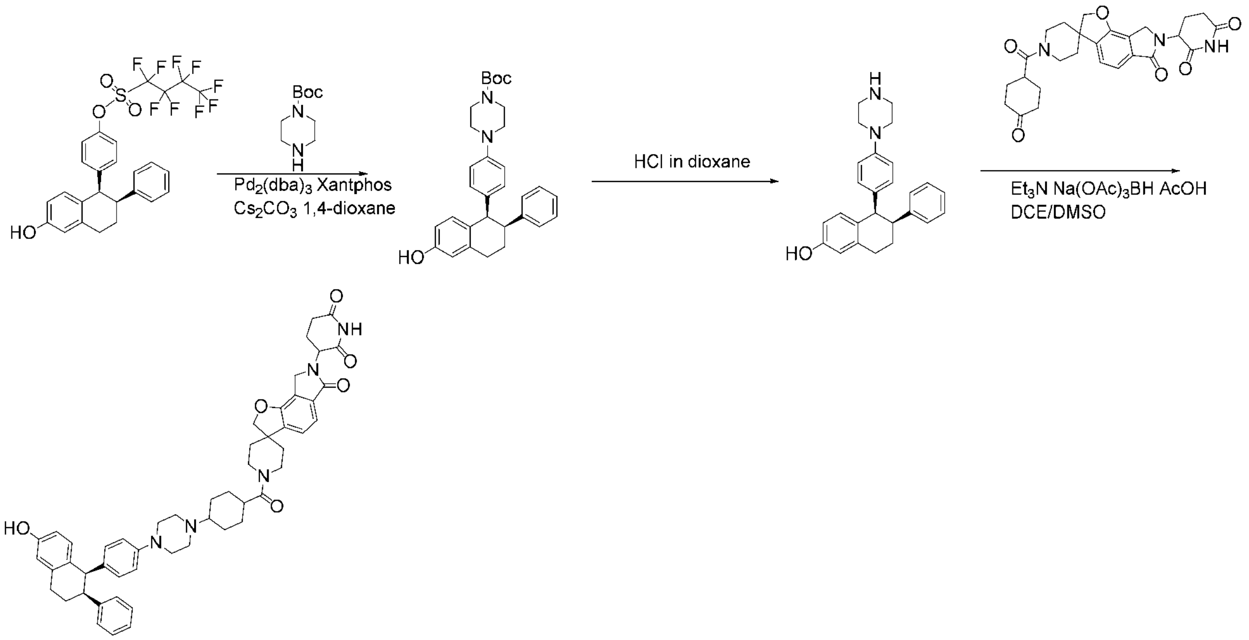


General Synthesis Formula 24 for PROTACs

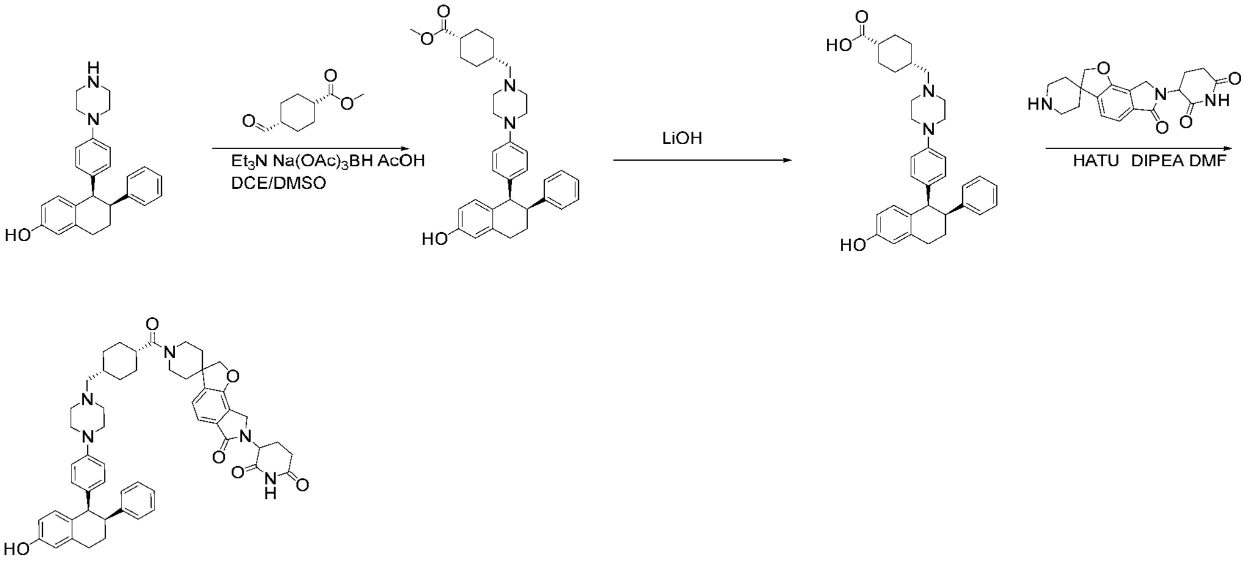


General Synthesis Formula 25 for PROTACs

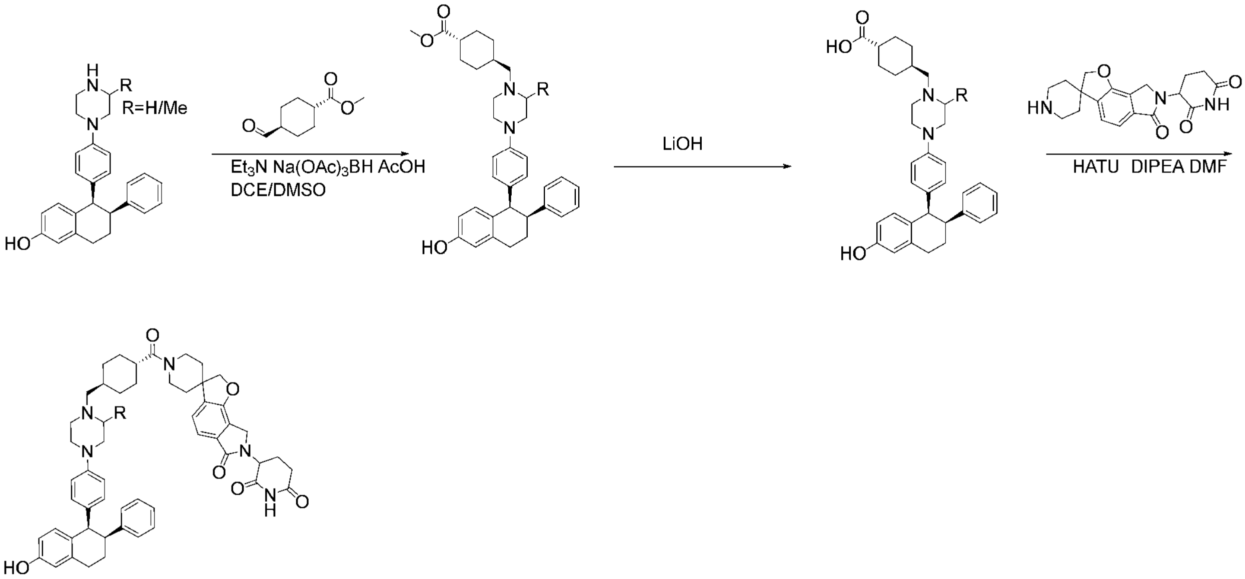


General Synthesis Formula 26 for PROTACs

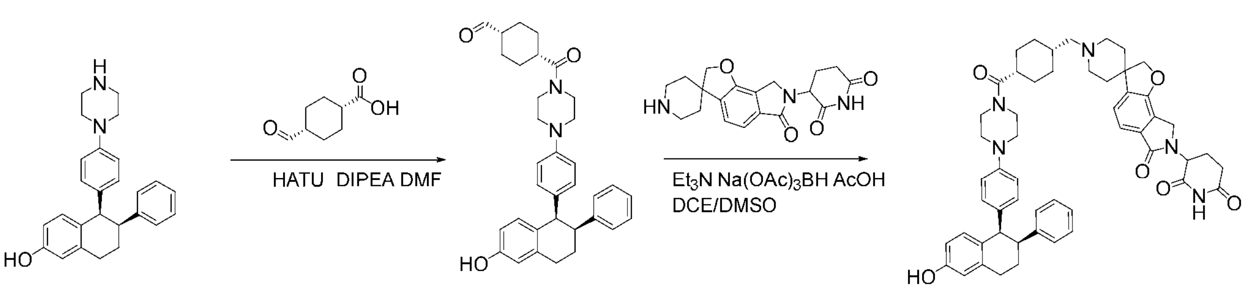

General Synthesis Formula 27 for PROTACs

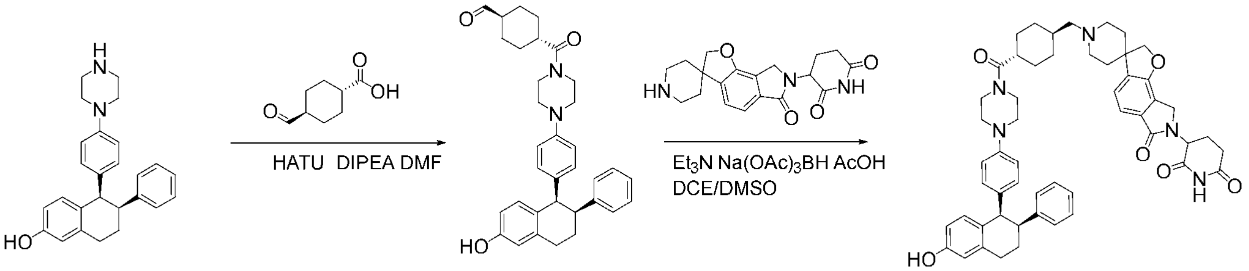


General Synthesis Formula 28 for PROTACs

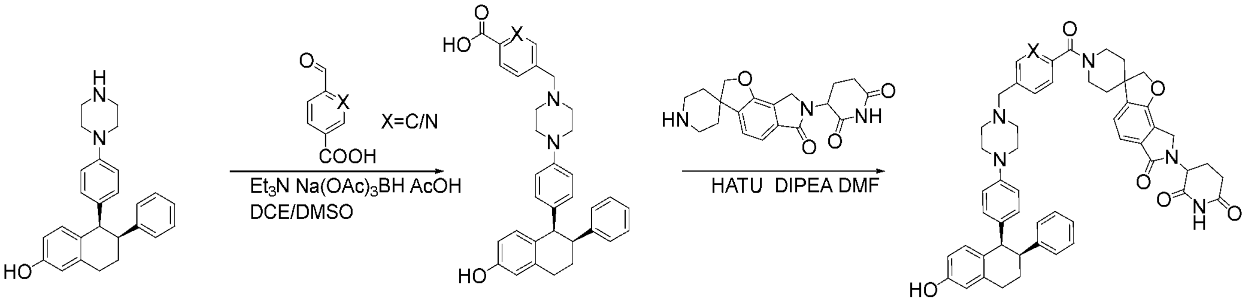


General Synthesis Formula 29 for PROTACs

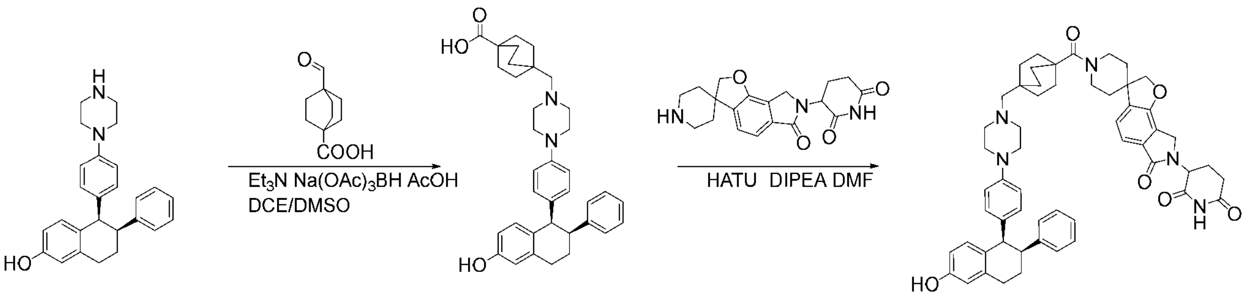


General Synthesis Formula 30 for PROTACs

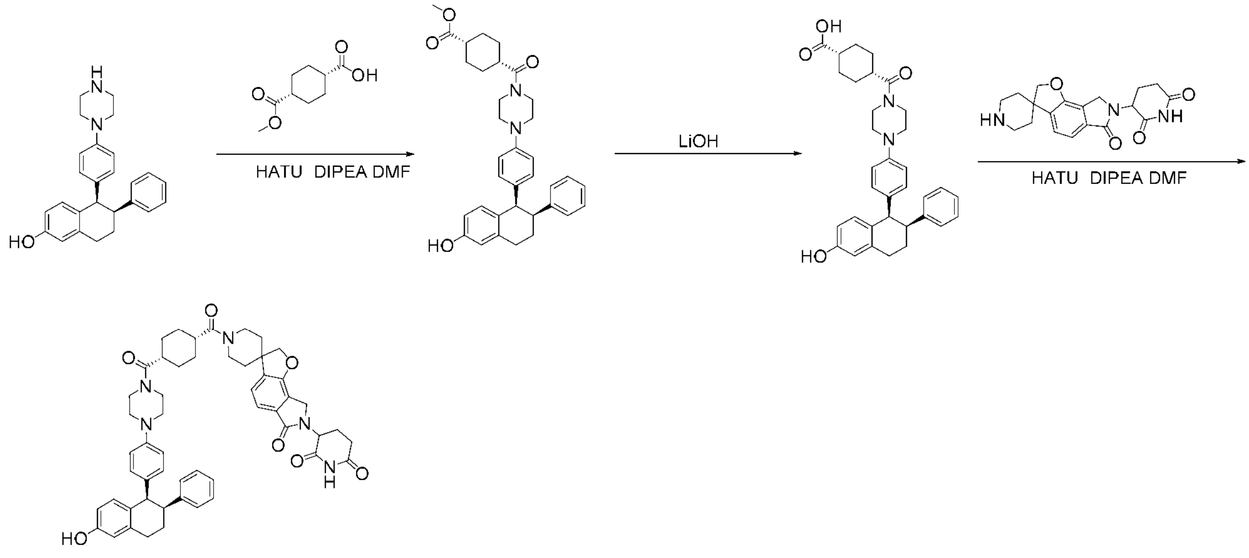


General Synthesis Formula 31 for PROTACs

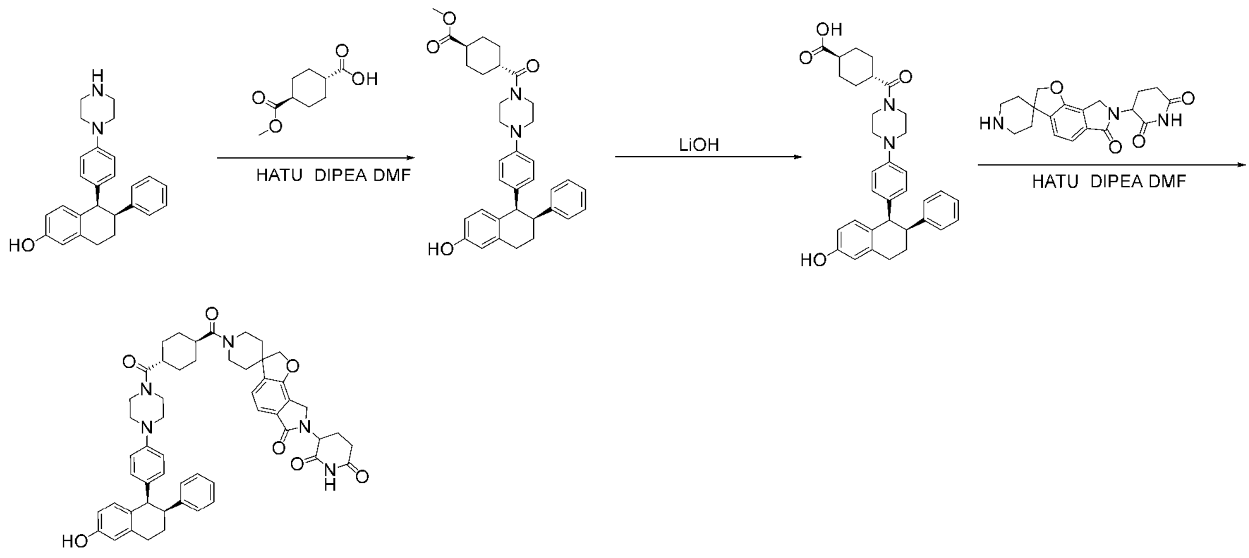


General Synthesis Formula 32 for PROTACs

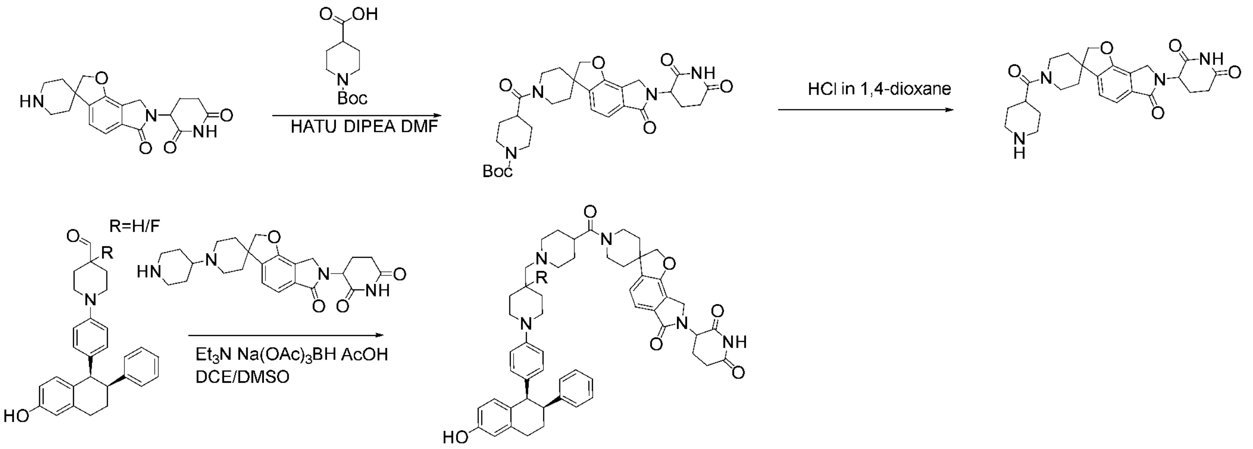


General Synthesis Formula 33 for PROTACs

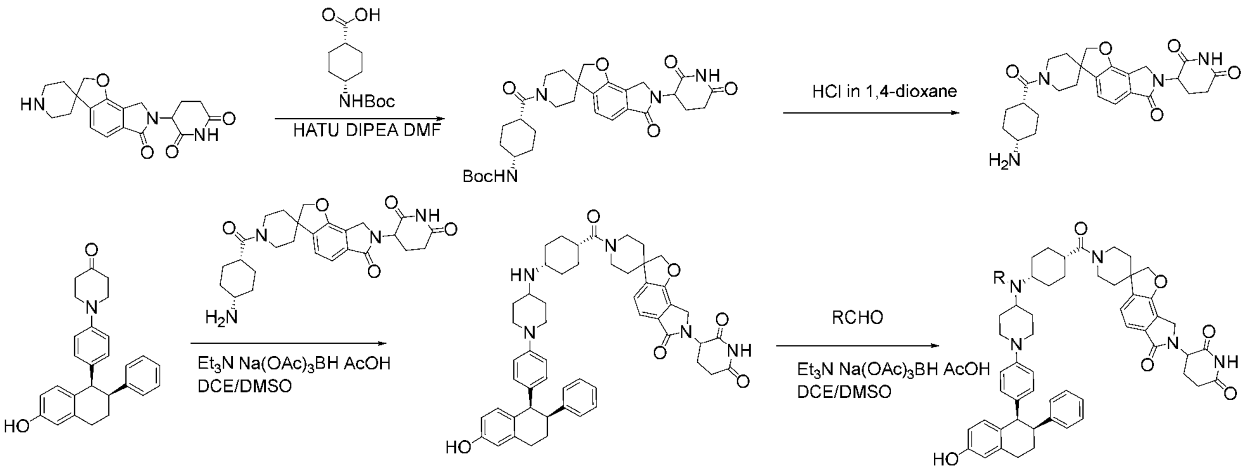


General Synthesis Formula 34 for PROTACs

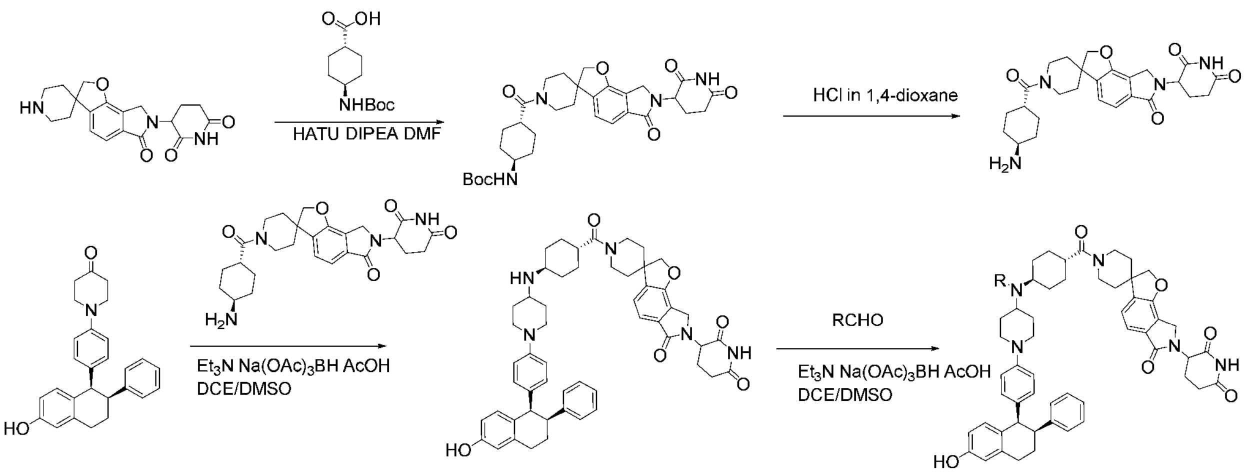

General Synthesis Formula 35 for PROTACs

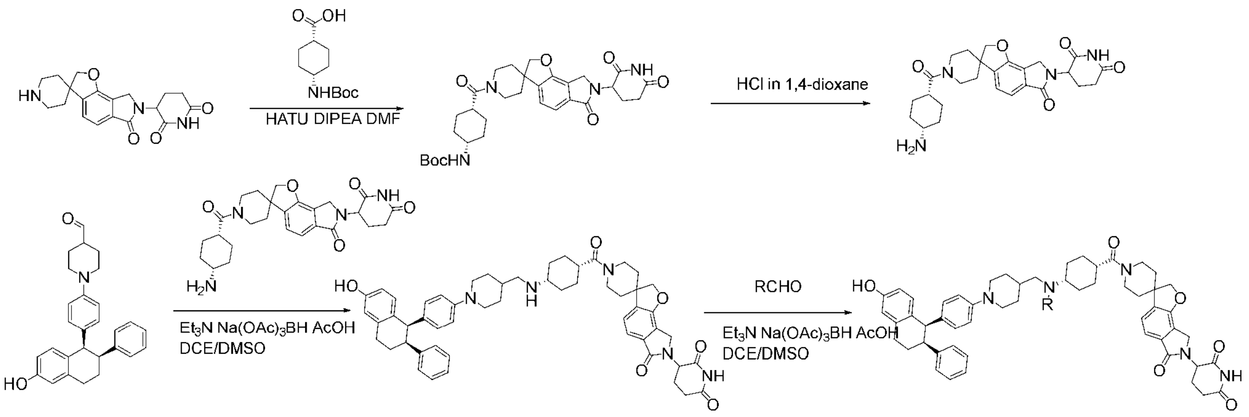


General Synthesis Formula 36 for PROTACs

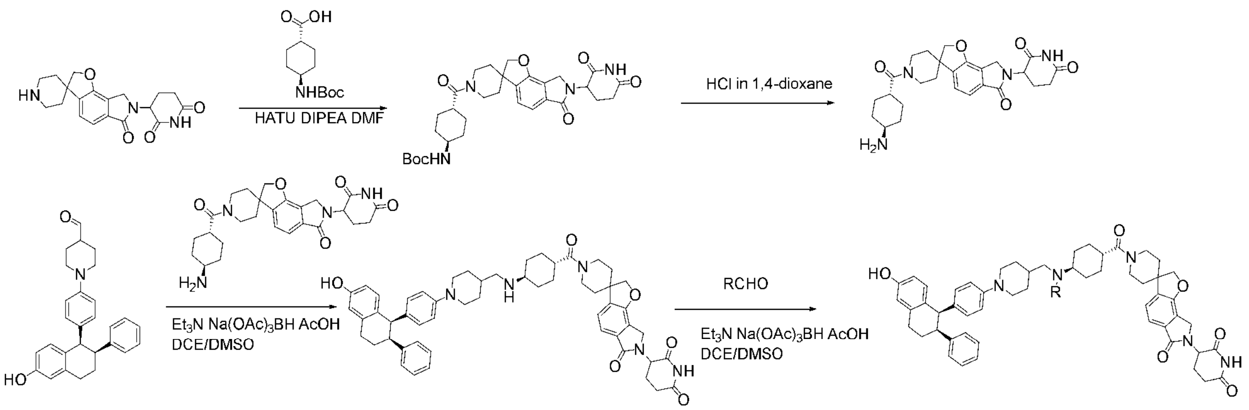


General Synthesis Formula 37 for PROTACs

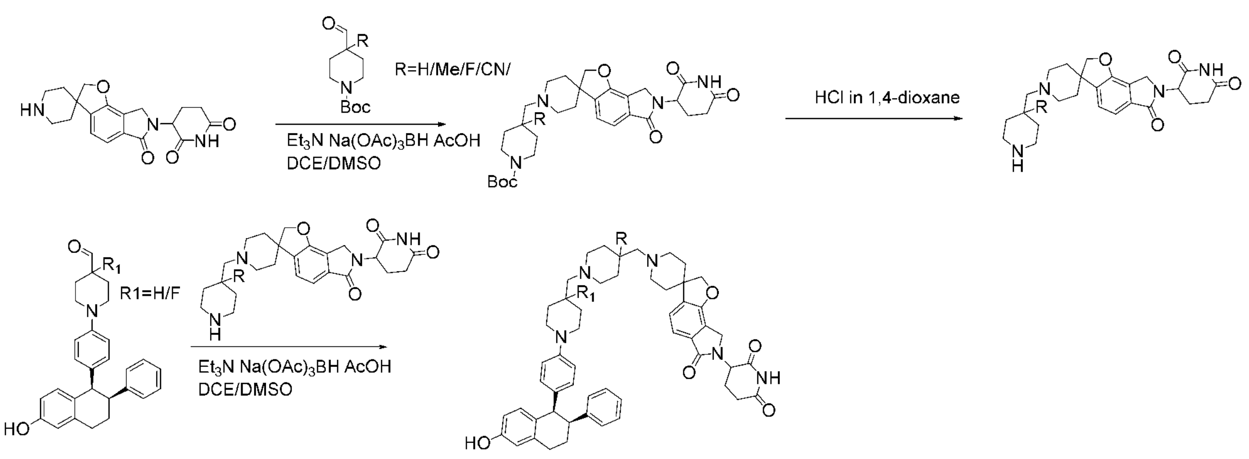

General Synthesis Formula 38 for PROTACs

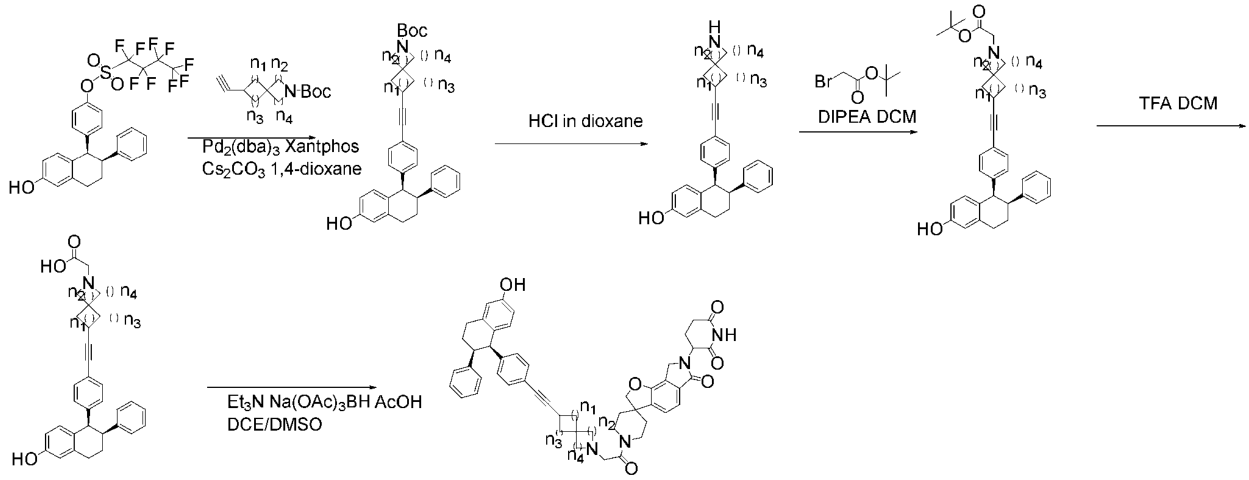


General Synthesis Formula 39 for PROTACs

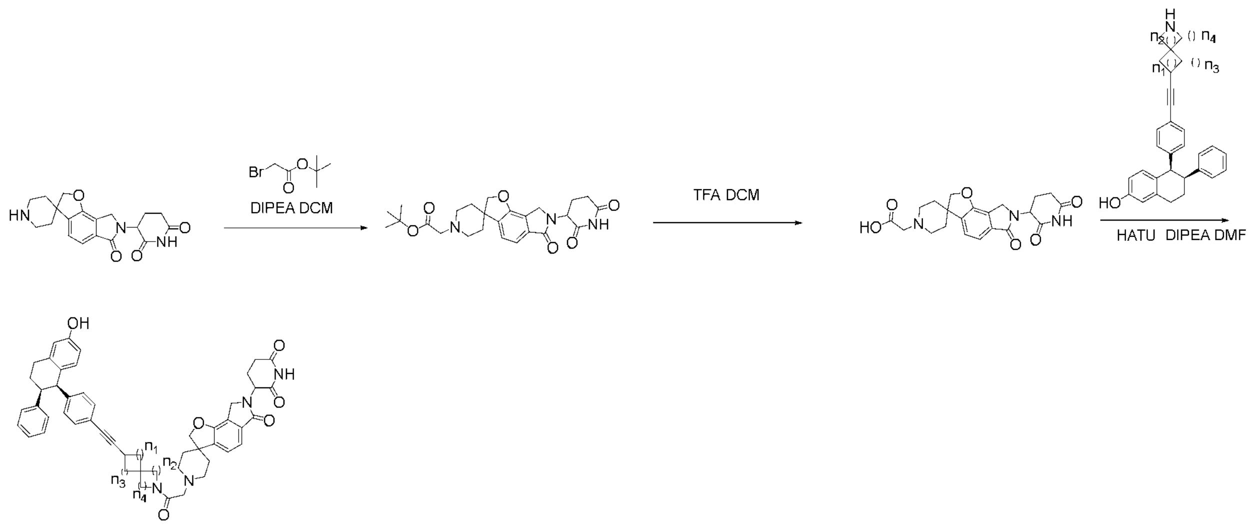


General Synthesis Formula 40 for PROTACs

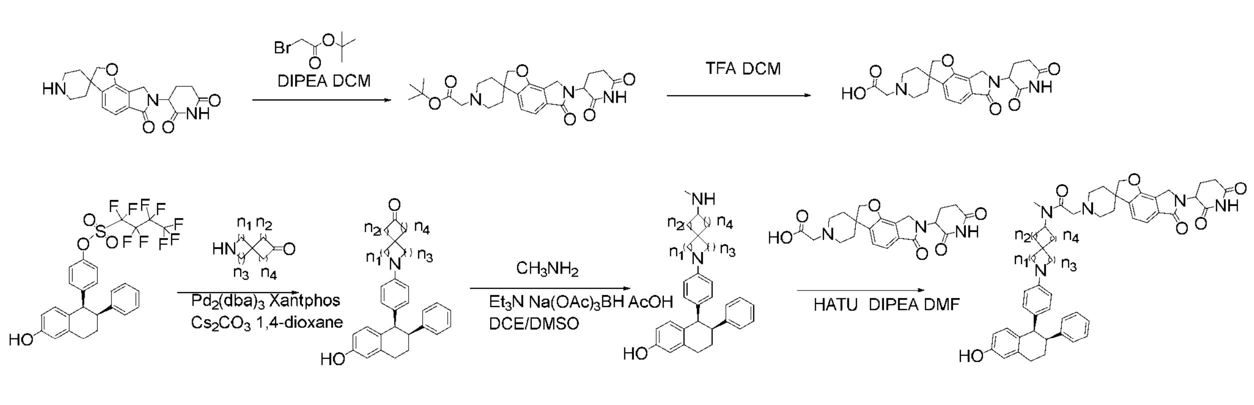

General Synthesis Formula 41 for PROTACs

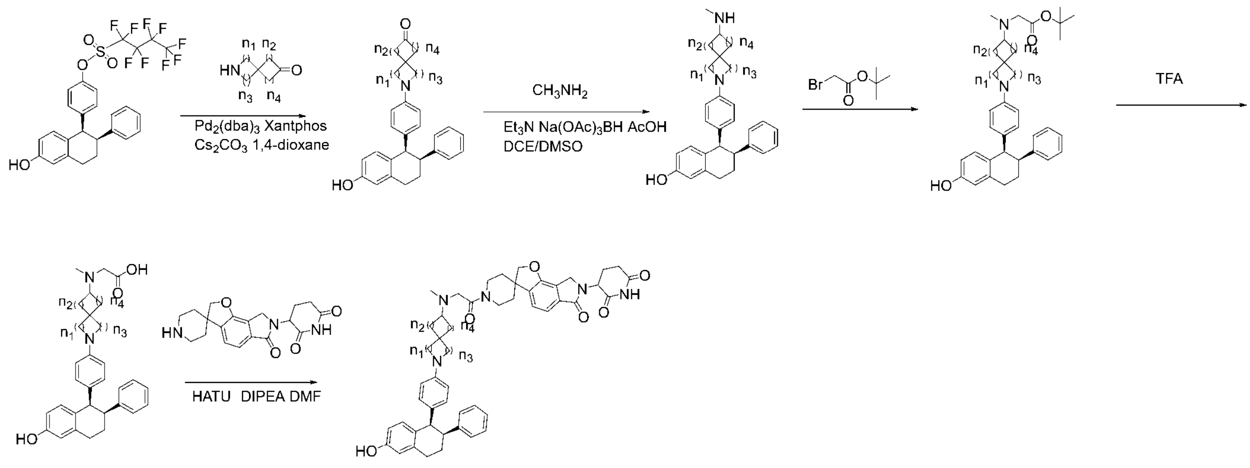


General Synthesis Formula 42 for PROTACs

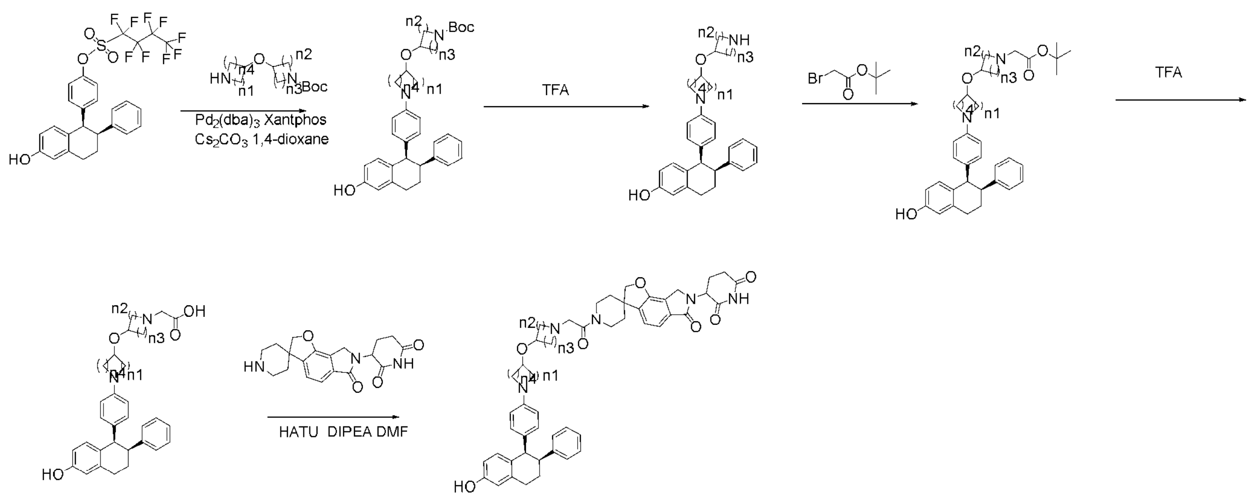

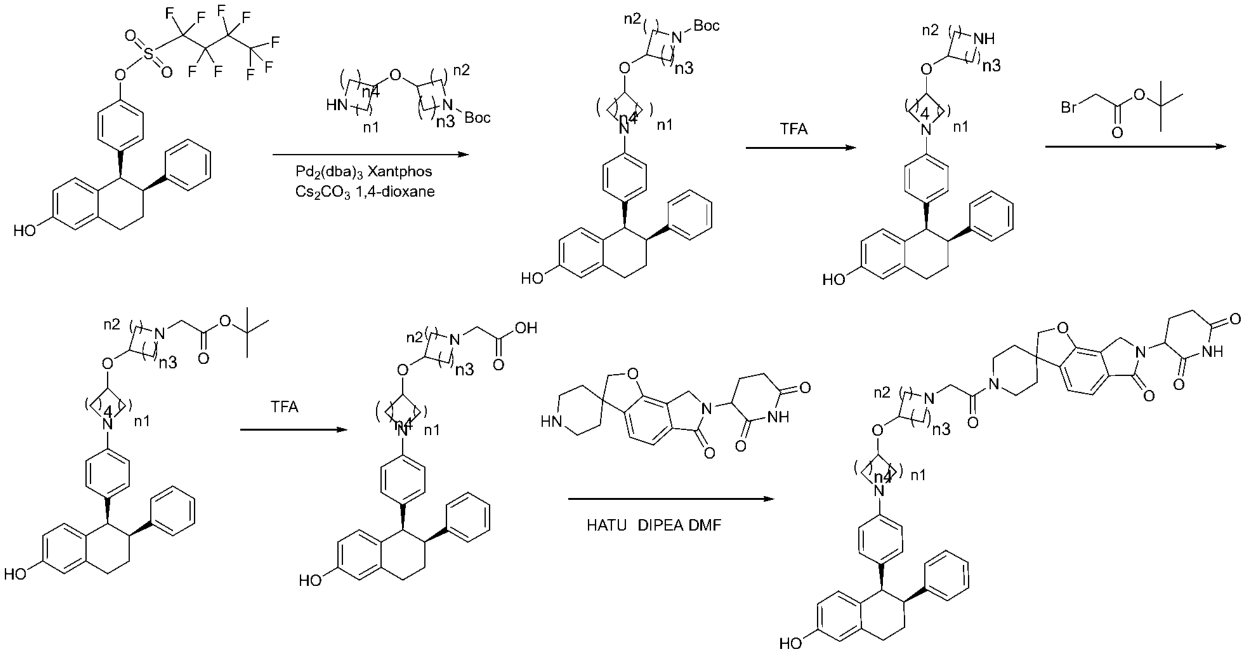


General Synthesis Formula 43 for PROTACs

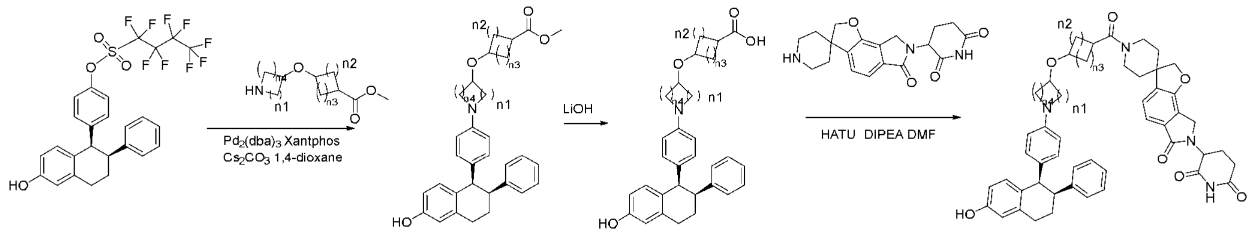


General Synthesis Formula 44 for PROTACs

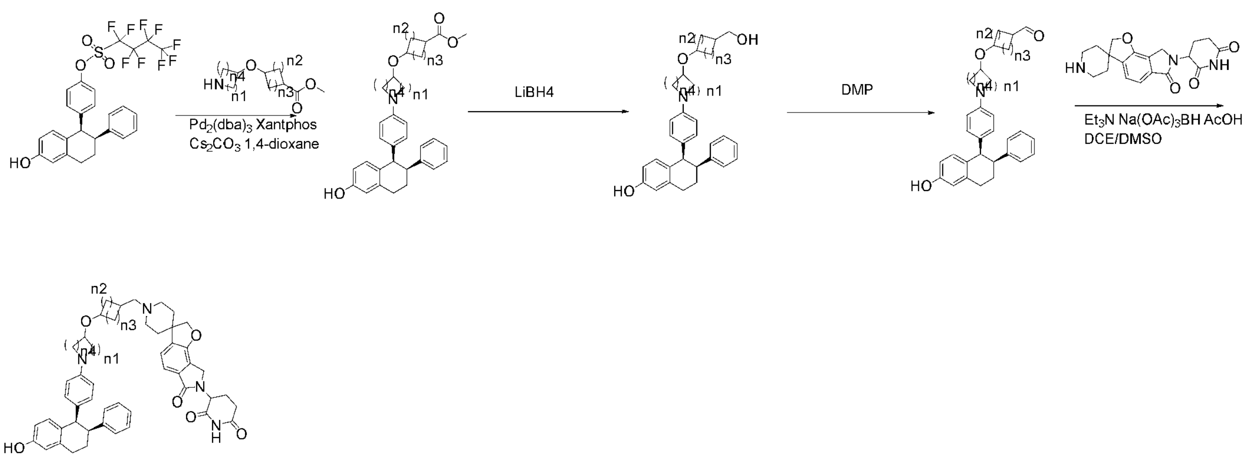


General Synthesis Formula 2 for intermediates

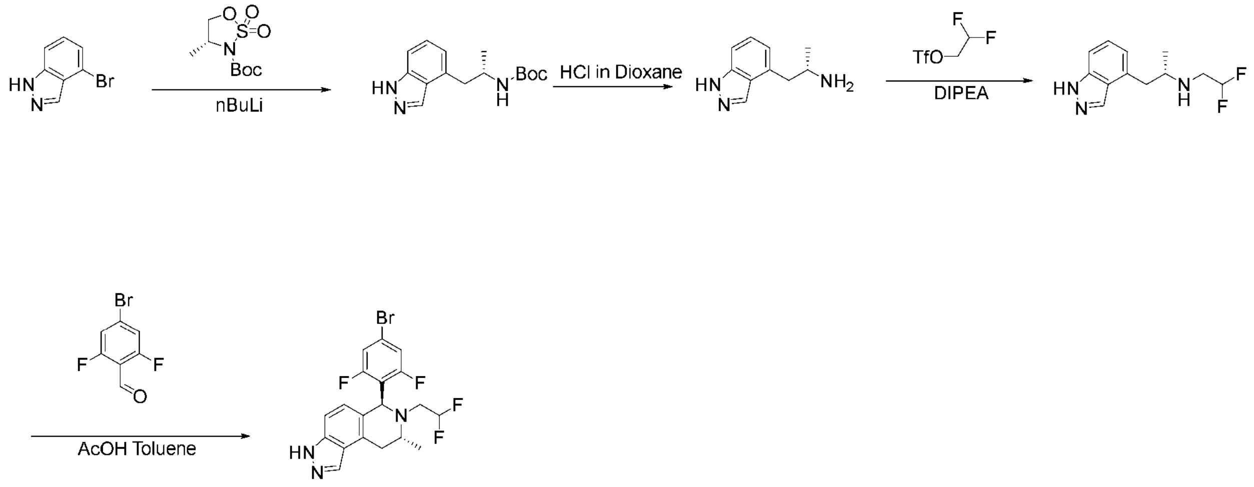


General Synthesis Formula 45 for PROTACs

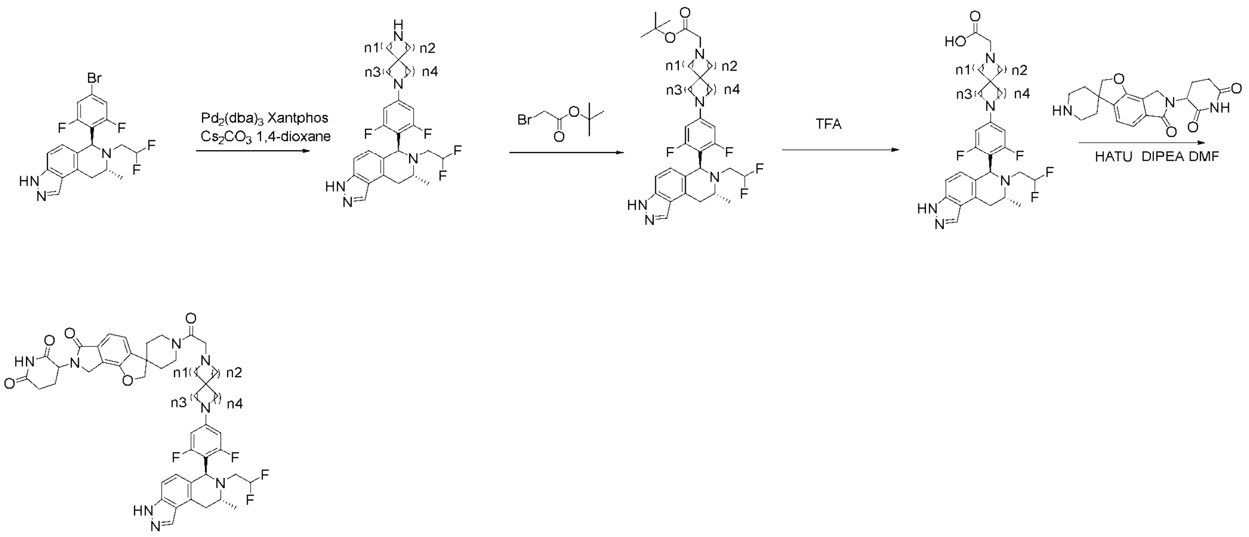


General Synthesis Formula 46 for PROTACs

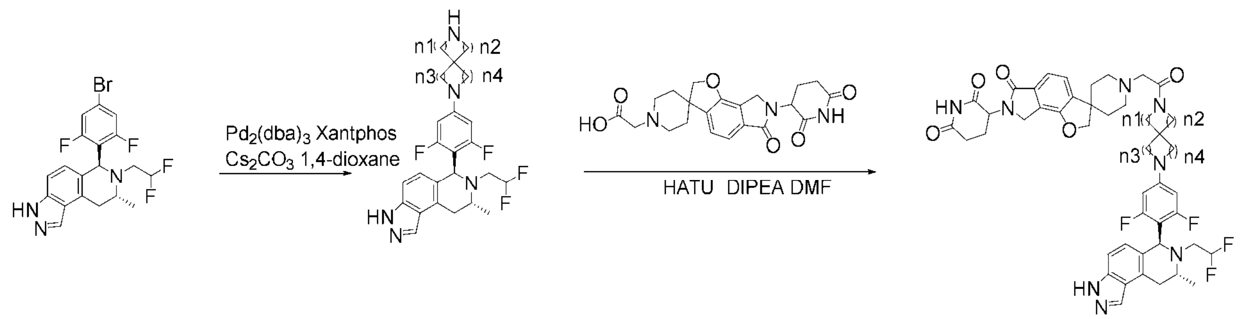


General Synthesis Formula 47 for PROTACs

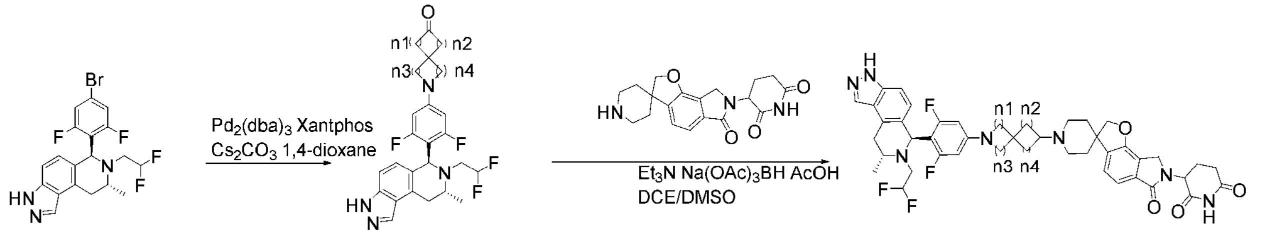


General Synthesis Formula 48 for PROTACs

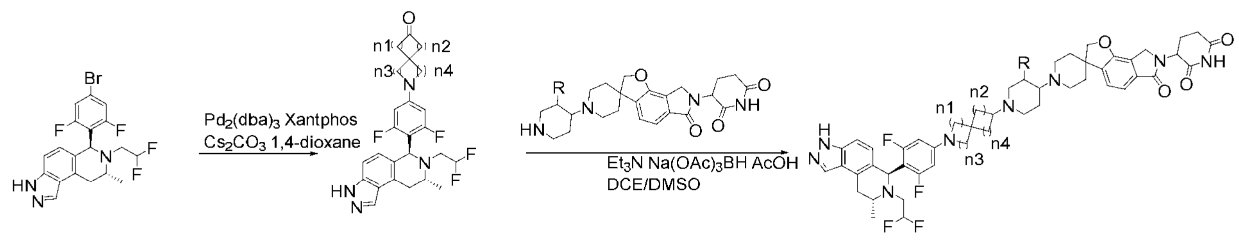


General Synthesis Formula 49 for PROTACs

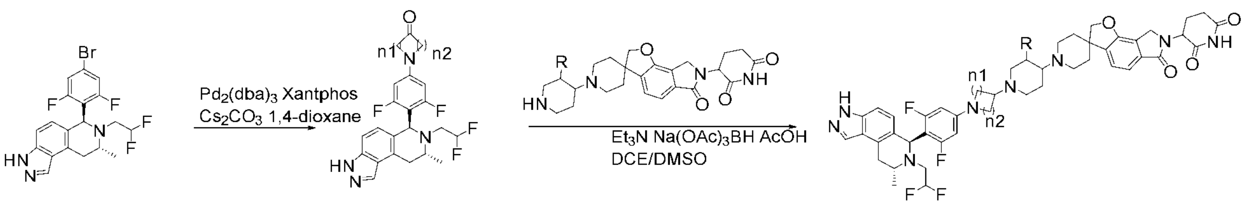


General Synthesis Formula 50 for PROTACs

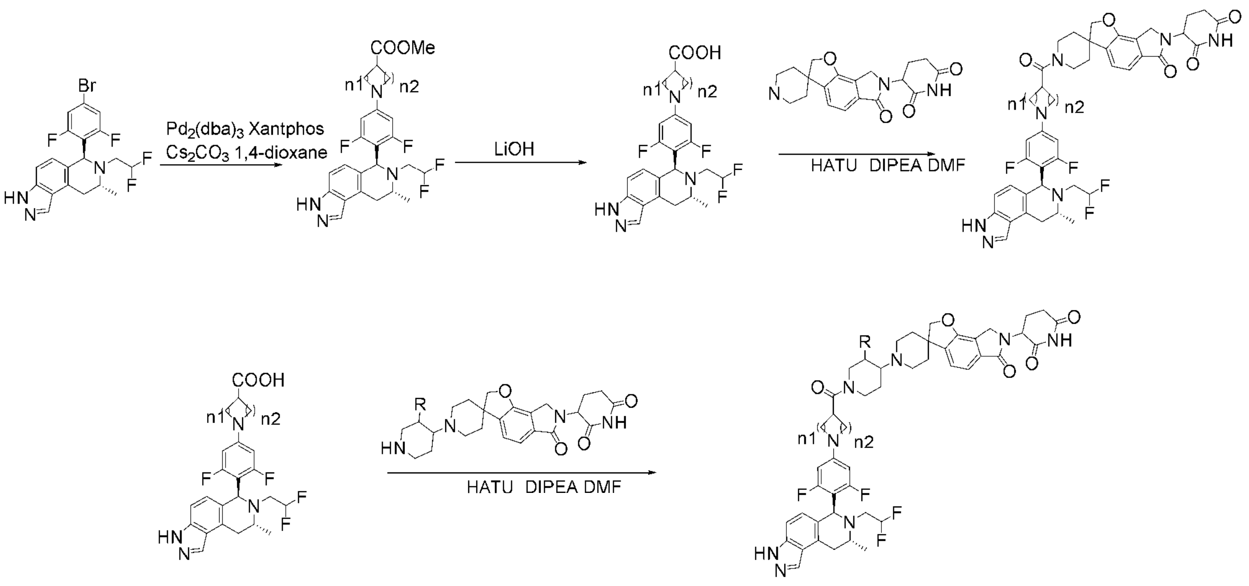

General Synthesis Formula 51 for PROTACs

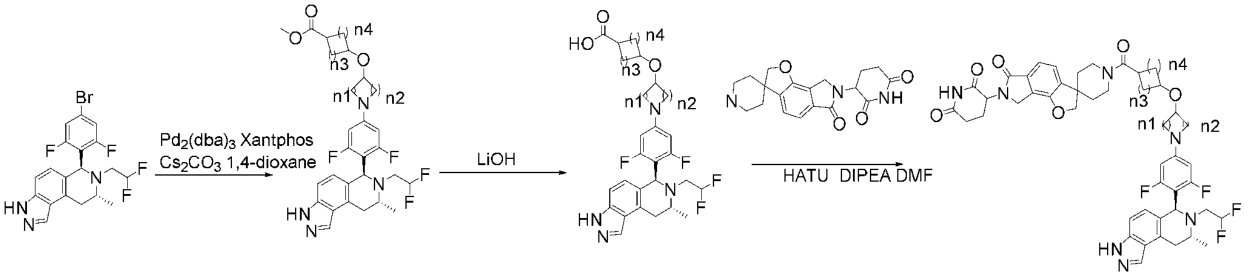


General Synthesis Formula 52 for PROTACs

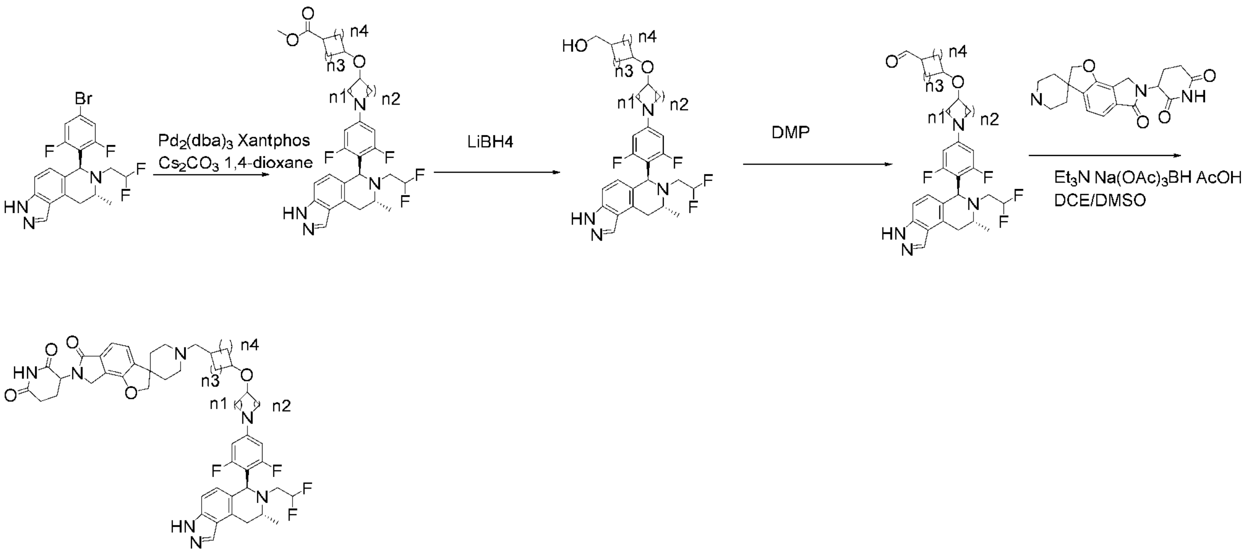


General Synthesis Formula 3 for intermediates

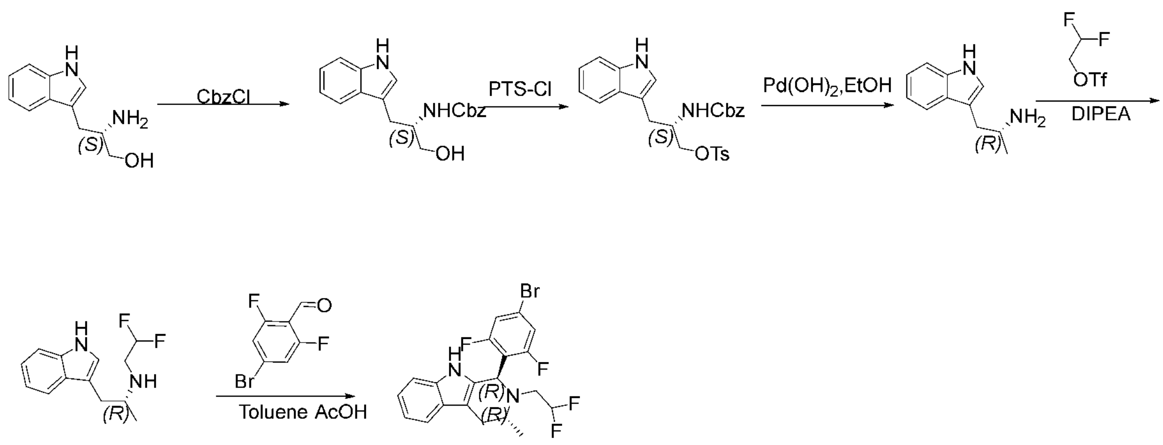

General Synthesis Formula 53 for PROTACs

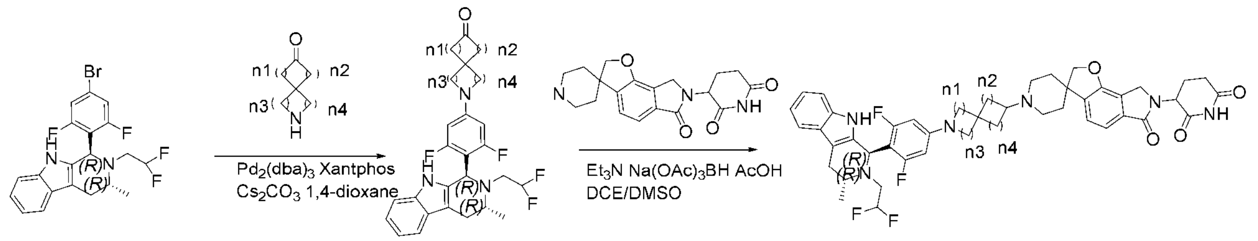


General Synthesis Formula 54 for PROTACs

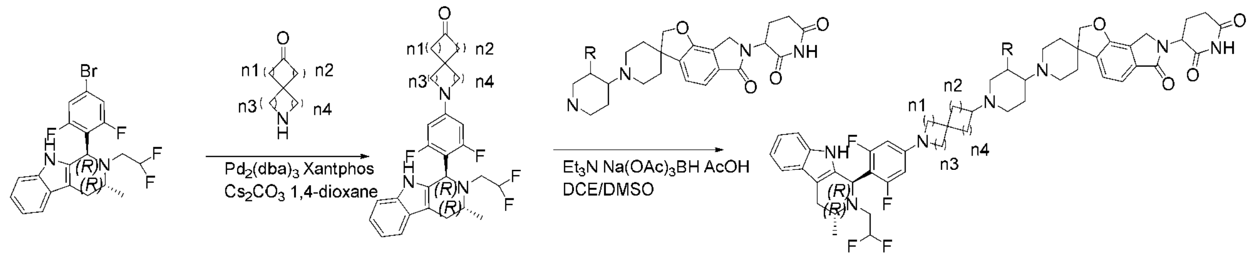


General Synthesis Formula 55 for PROTACs

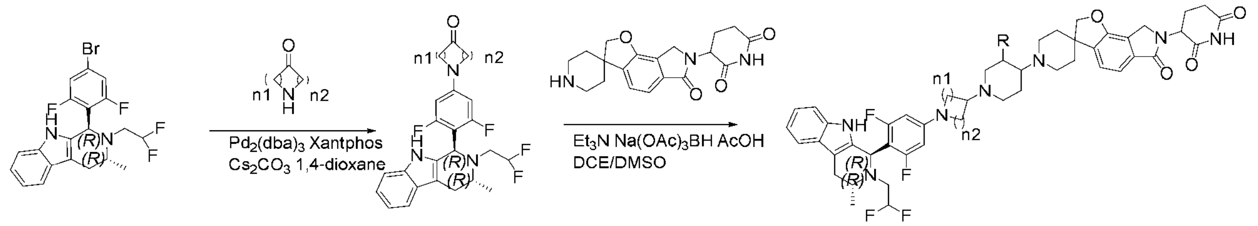


General Synthesis Formula 56 for PROTACs

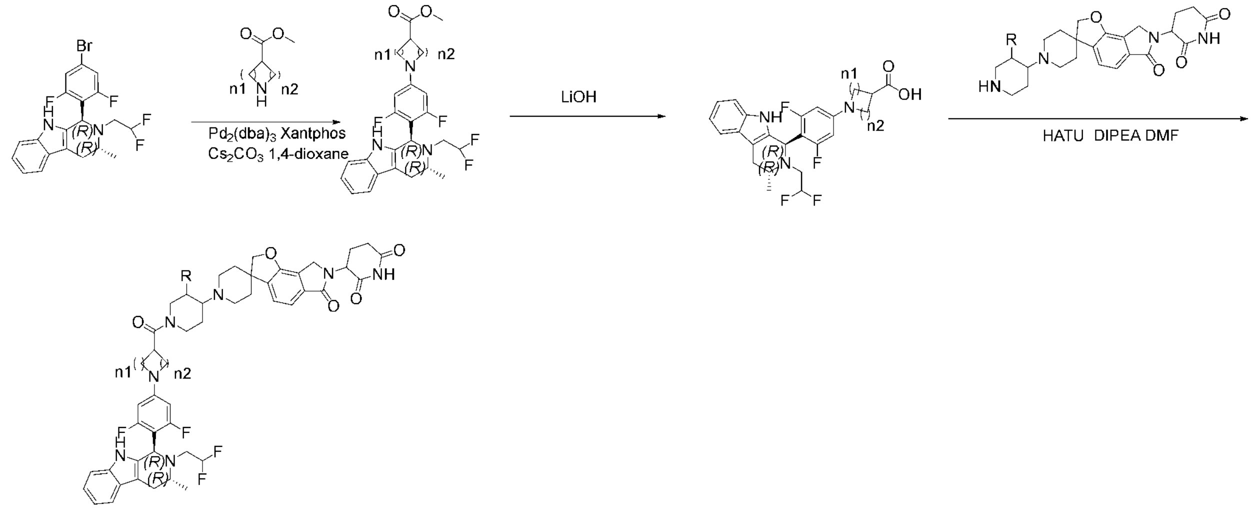


General Synthesis Formula 57 for PROTACs

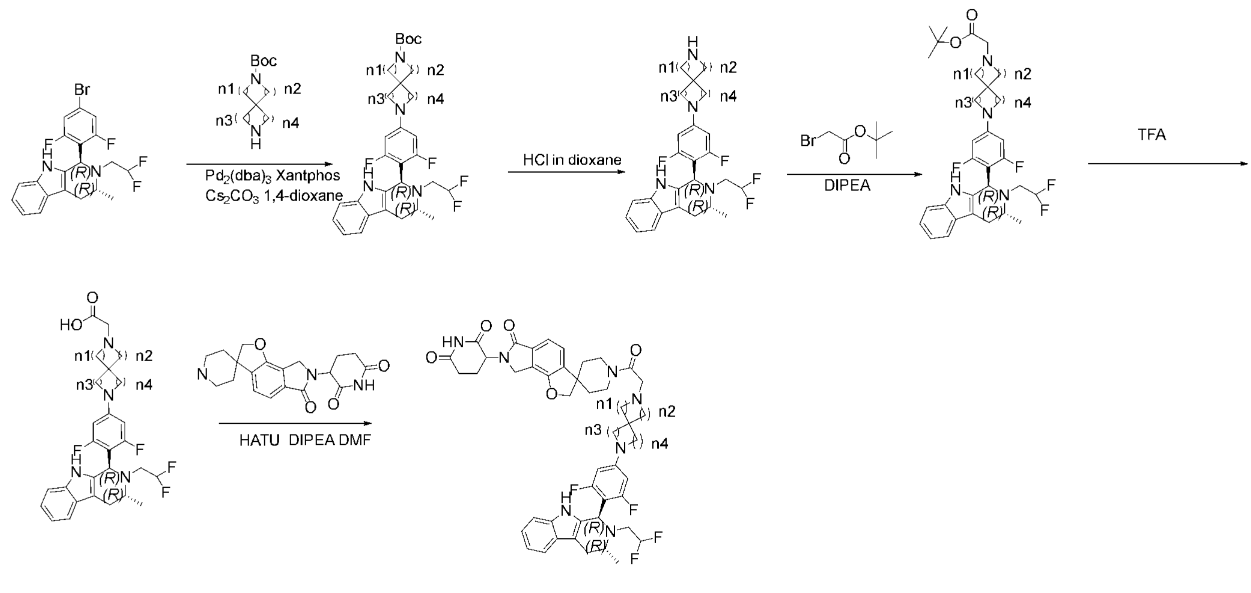


General Synthesis Formula 58 for PROTACs

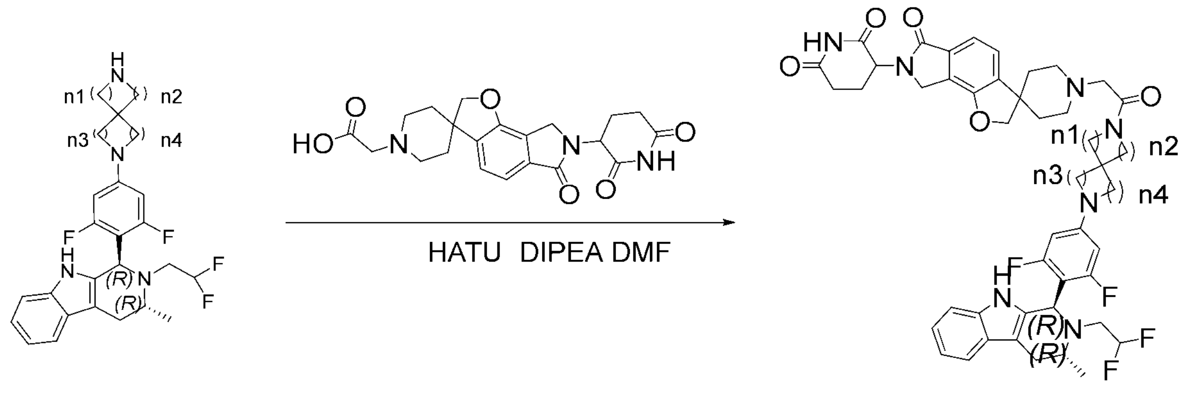

General Synthesis Formula 4 for Intermediates

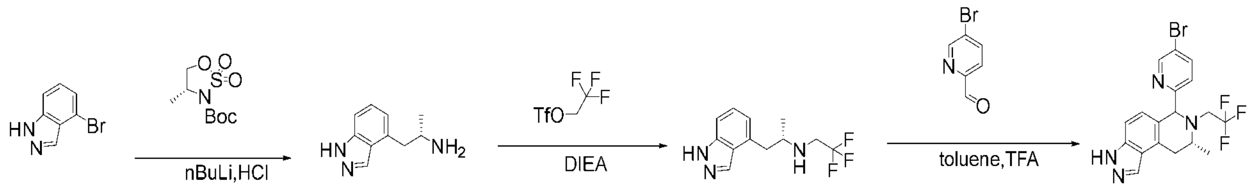


General Synthesis Formula 59 for PROTACs

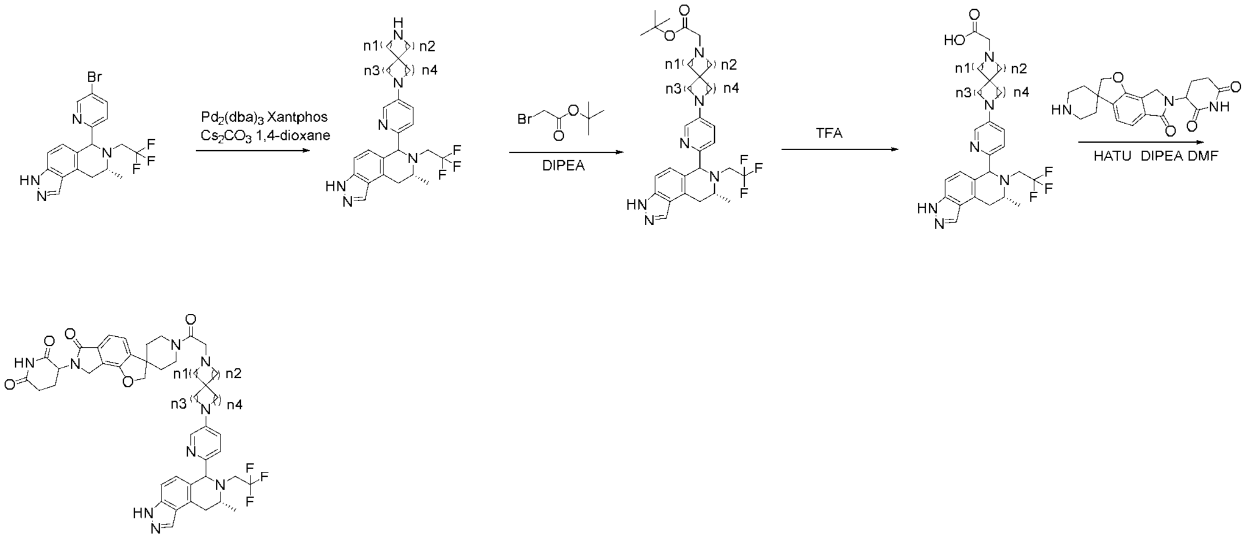


General Synthesis Formula 60 for PROTACs

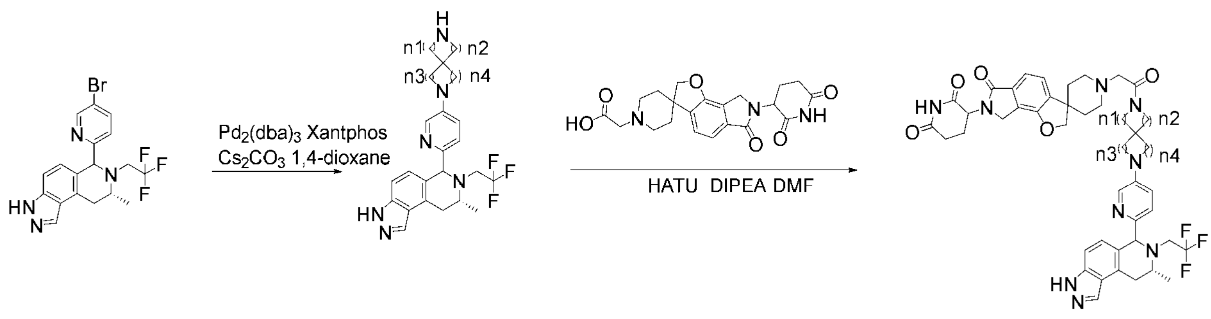


General Synthesis Formula 61 for PROTACs

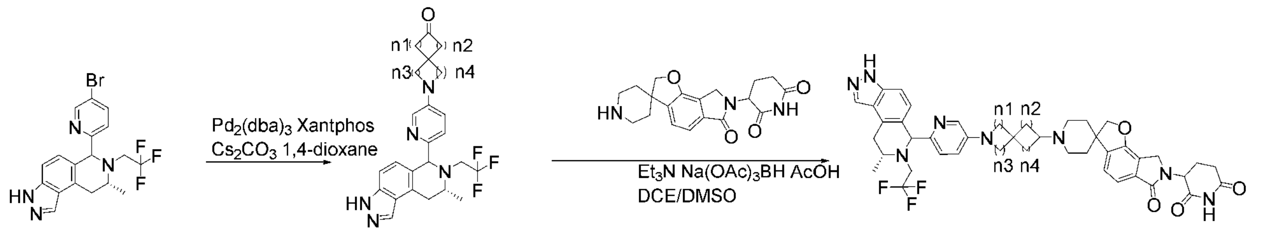


General Synthesis Formula 62 for PROTACs

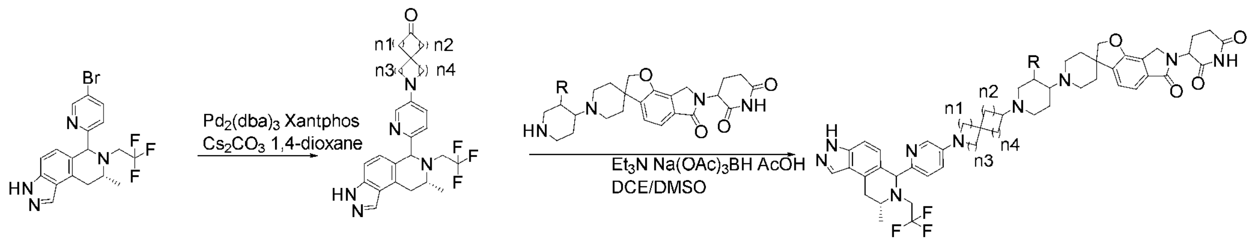


General Synthesis Formula 63 for PROTACs

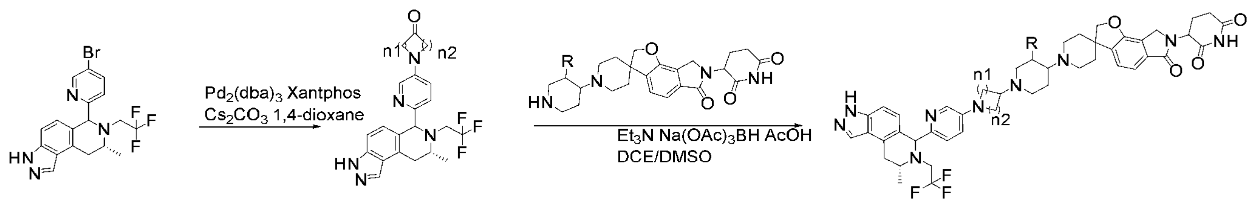


General Synthesis Formula 64 for PROTACs

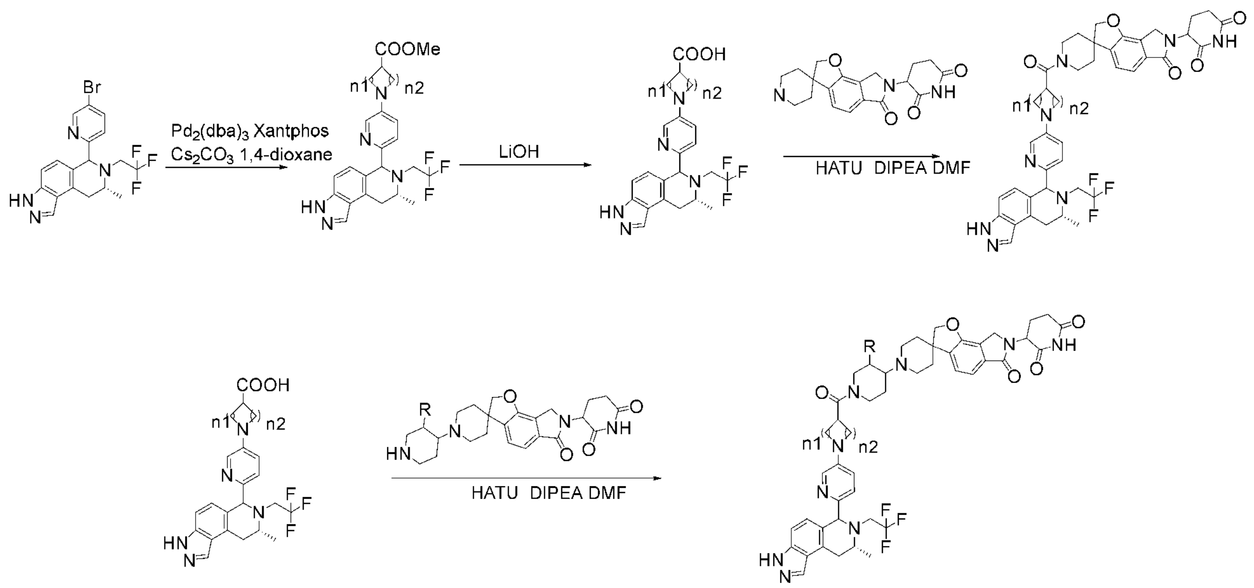

General Synthesis Formula 5 for intermediates

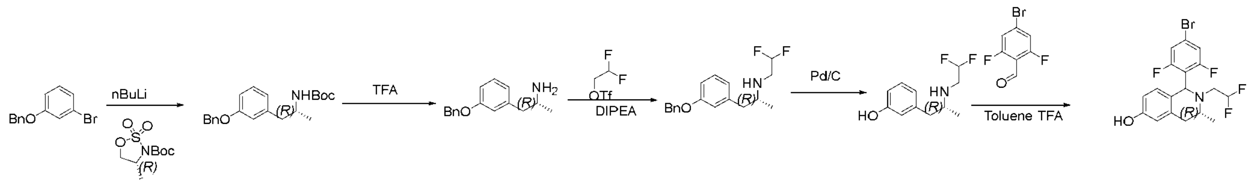


General Synthesis Formula 65 for PROTACs

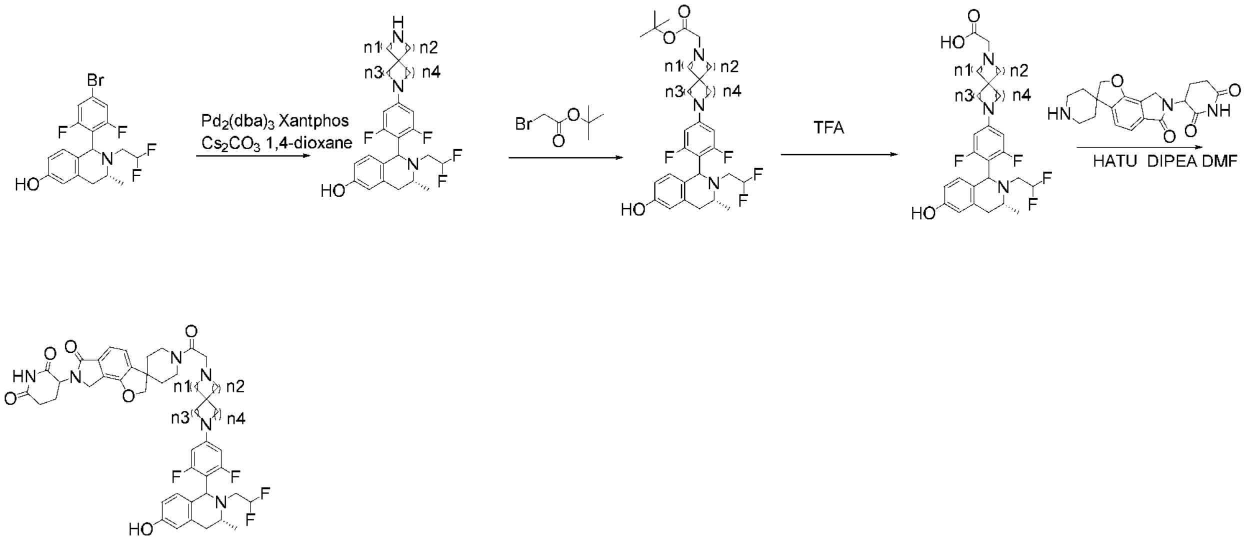


General Synthesis Formula 66 for PROTACs

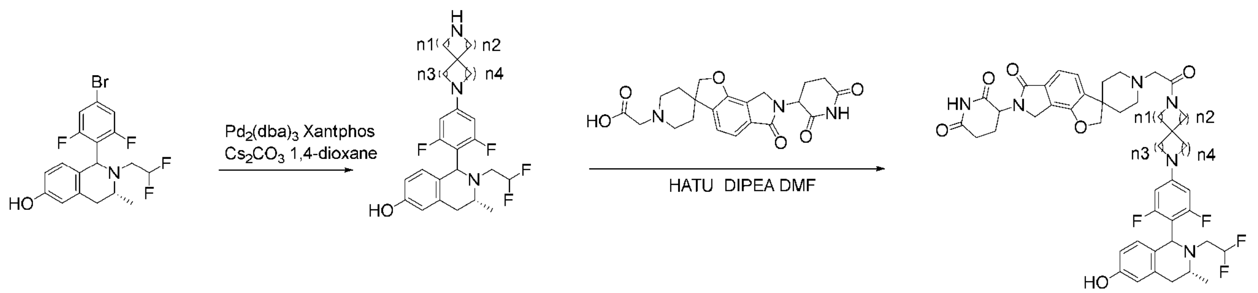


General Synthesis Formula 67 for PROTACs

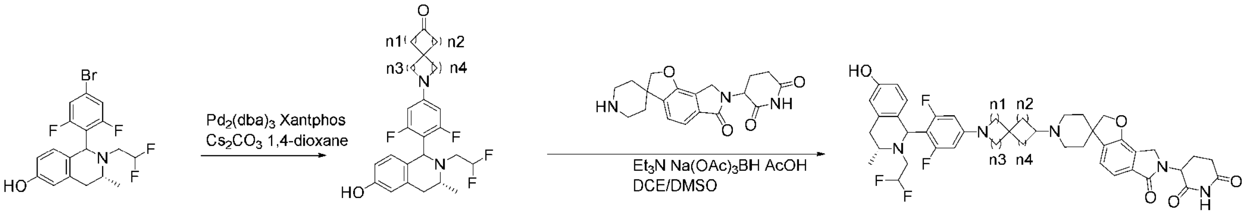


General Synthesis Formula 68 for PROTACs

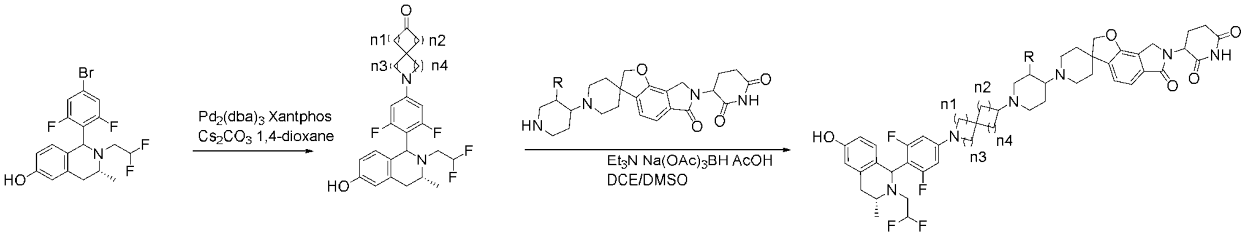


General Synthesis Formula 69 for PROTACs

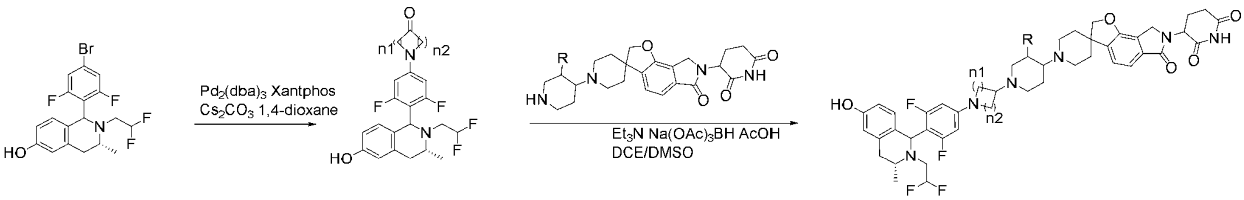


General Synthesis Formula 70 for PROTACs

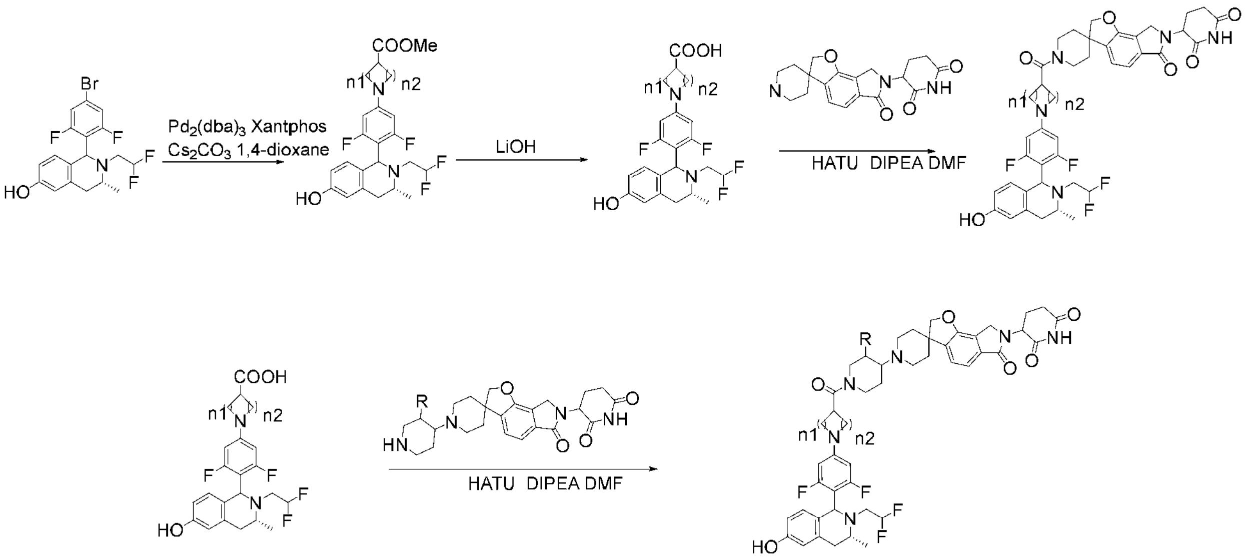

General Synthesis Formula 71 for PROTACs

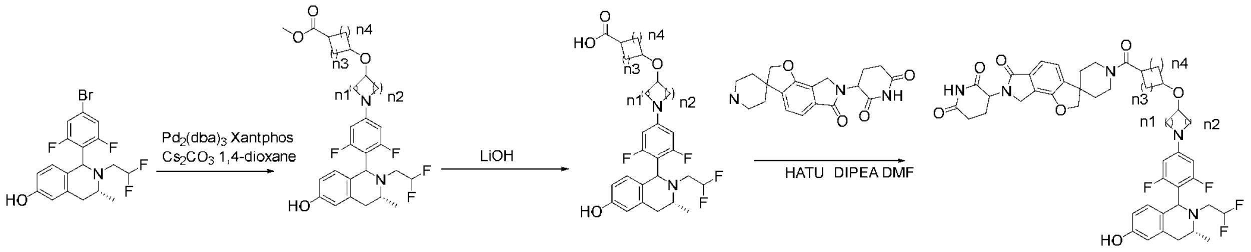


General Synthesis Formula 72 for PROTACs

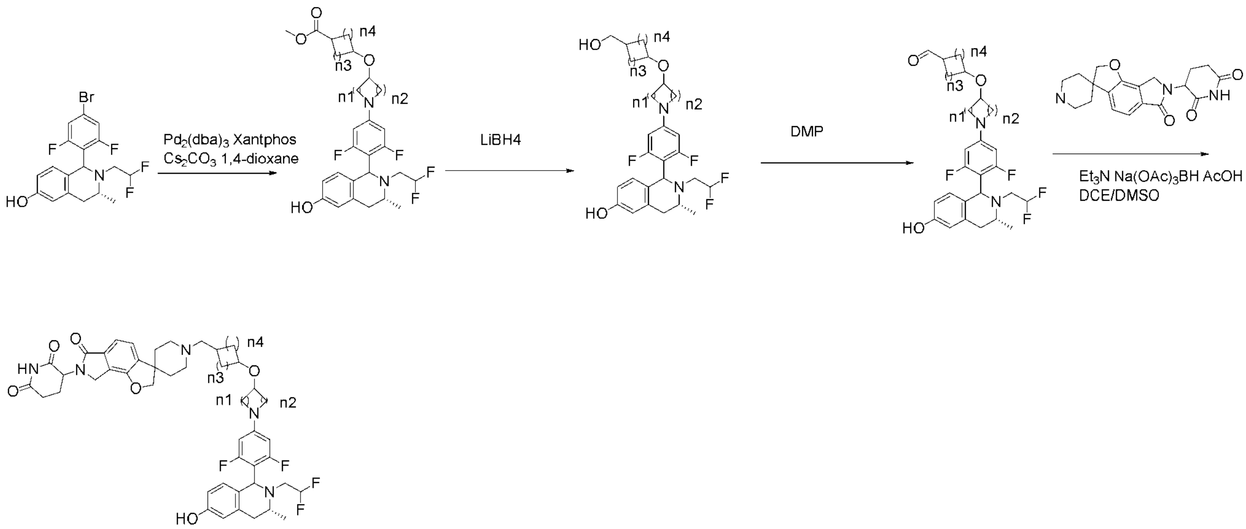


General Synthesis Formula 73 for PROTACs

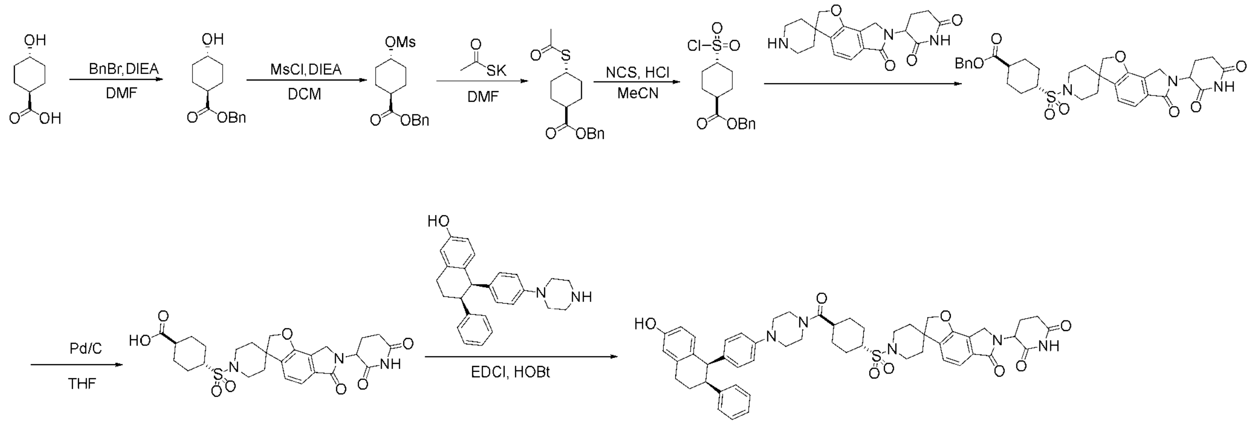


General Synthesis Formula 74 for PROTACs

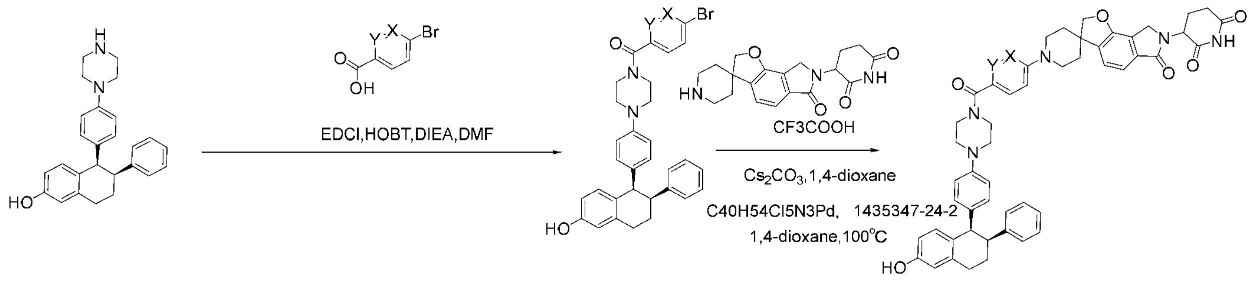


General Synthesis Formula 75 for PROTACs

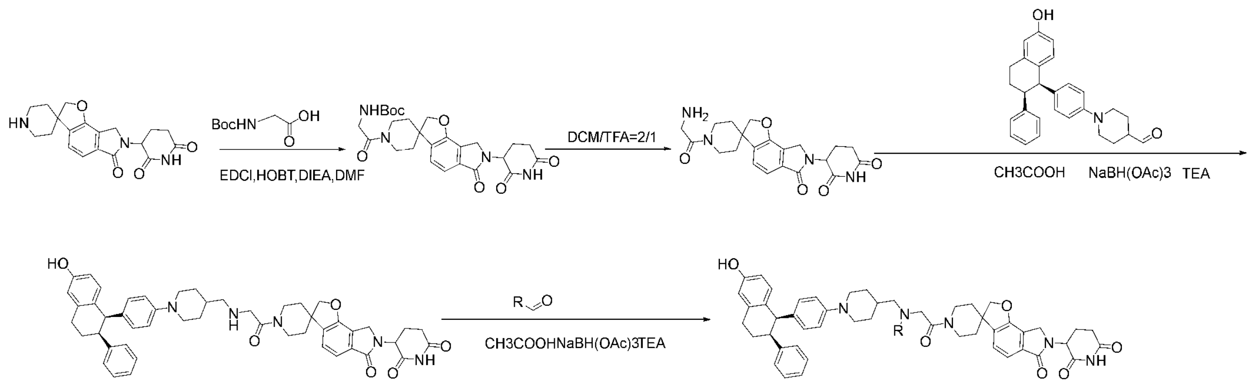


General Synthesis Formula 76 for PROTACs

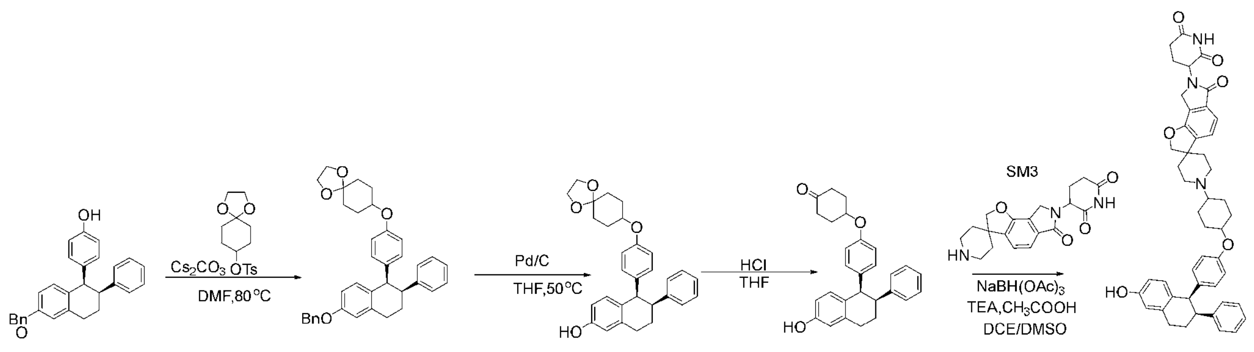


General Synthesis Formula 77 for PROTACs

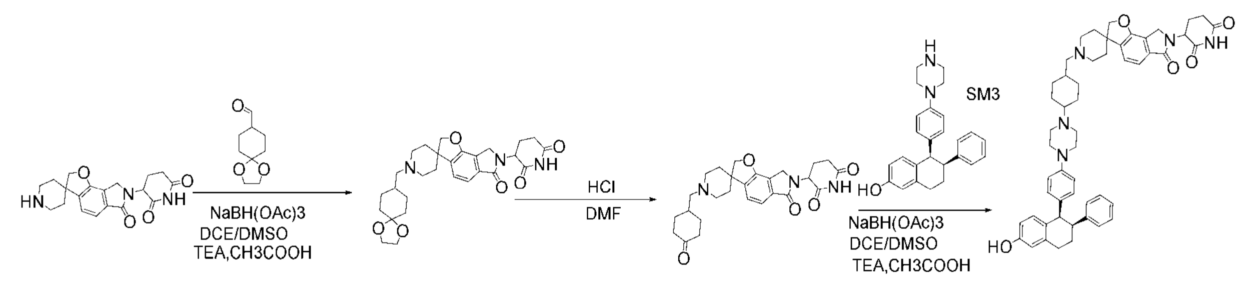

General Synthesis Formula 78 for PROTACs

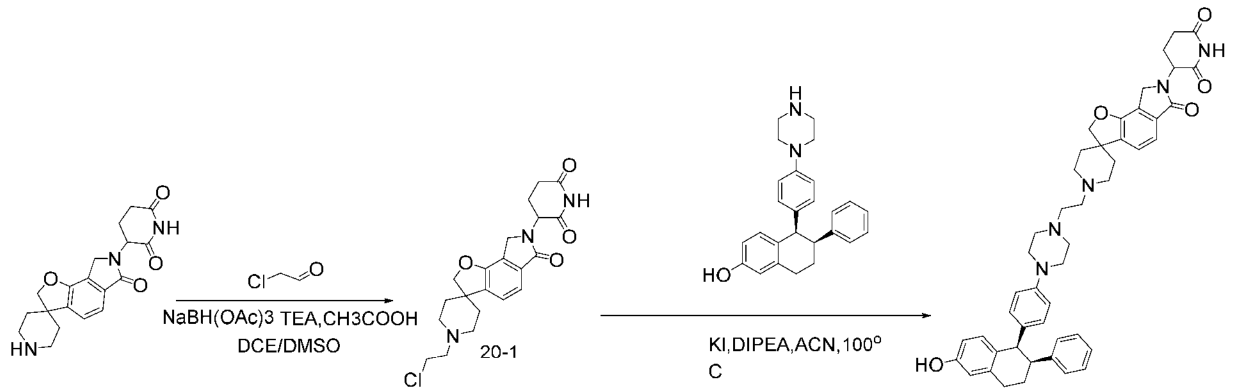


General Synthesis Formula 79 for PROTACs

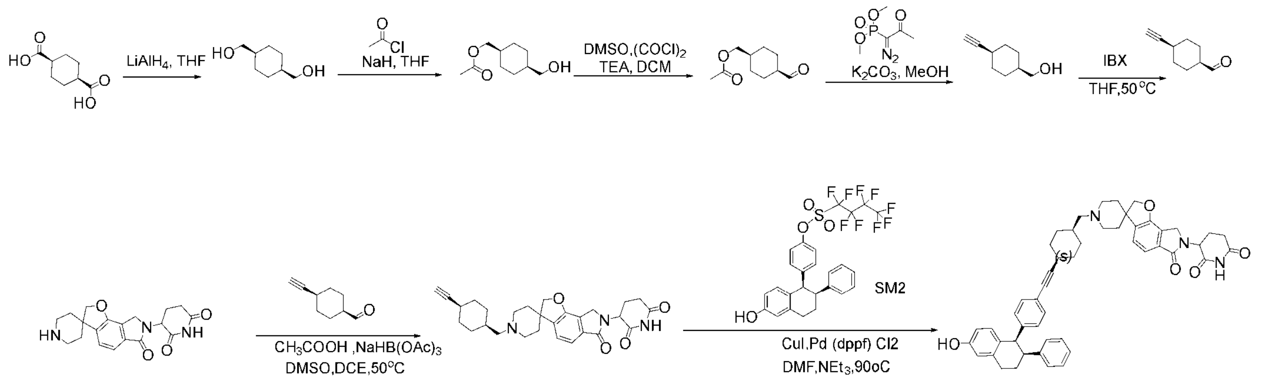


General Synthesis Formula 80 for PROTACs

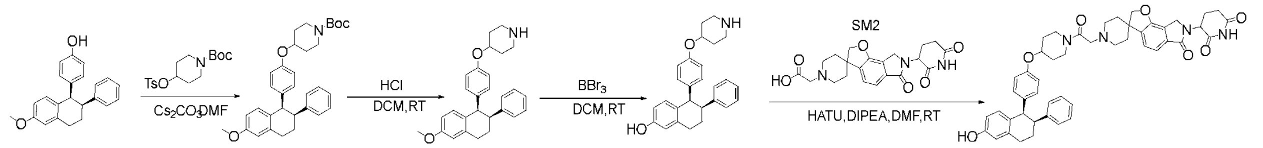


General Synthesis Formula 81 for PROTACs

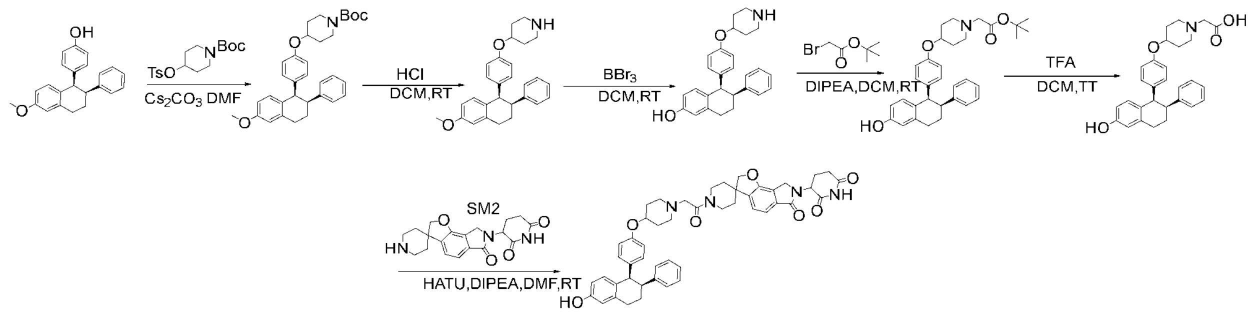


General Synthesis Formula 6 for Intermediates

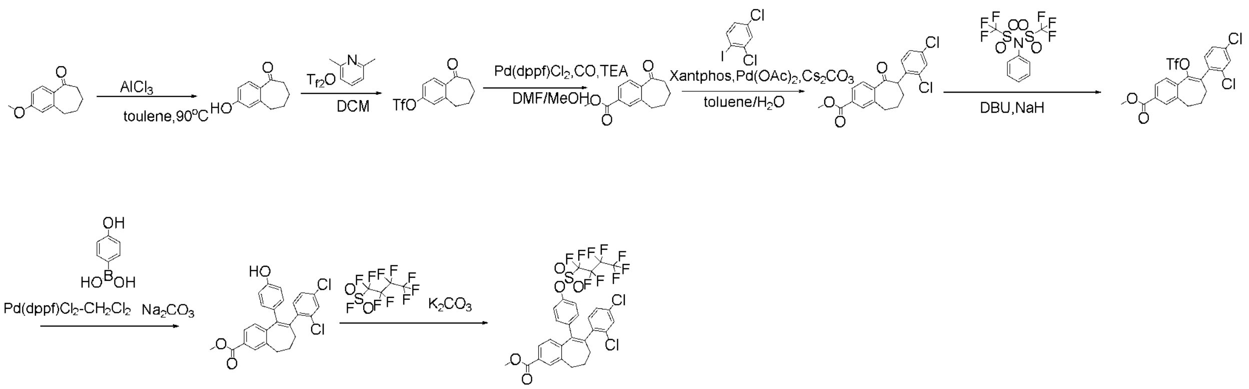


General Synthesis Formula 82 for PROTACs

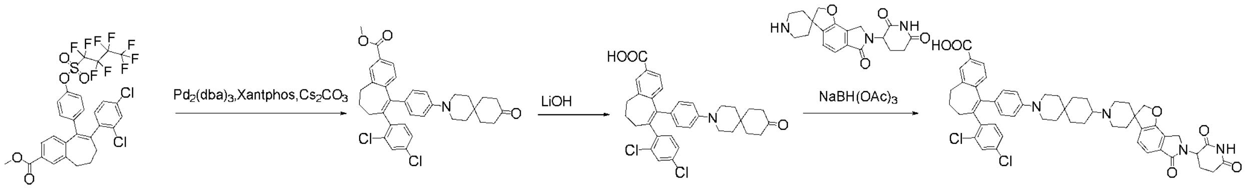


Characterization of PROTAC molecules

| Example | Compound | Structure and name | $^1$H-NMR | LC-MS | Synthesis Formula for PROTACs |
|---|---|---|---|---|---|
| **1** | A1 | 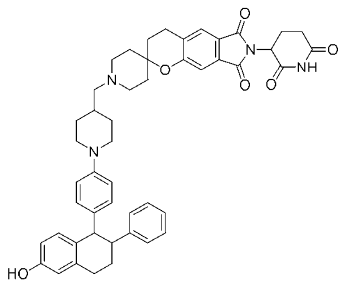 7'-(2,6-Dioxopiperidin-3-yl)-1-((1-(4-(6-hydroxy-2-phenyl-1,2,3,4-tetrahydronaphthalen-1-yl)phenyl)piperidin-4-yl)methyl)-3',4'-dihydro-6'H-spiro[piperidine-4,2'-pyrano[2,3-f]isoindole]-6',8'(7'H)-dione | $^1$H NMR (600 MHz, DMSO-$d_6$) δ 11.12 (s, 1H), 9.18 (s, 1H), 7.75 (d, $J$ = 3.8 Hz, 1H), 7.38 (s, 1H), 7.18 – 7.09 (m, 3H), 6.84 (dd, $J$ = 6.9, 1.9 Hz, 2H), 6.73 – 6.59 (m, 4H), 6.49 (dd, $J$ = 8.3, 2.6 Hz, 1H), 6.28 (d, $J$ = 8.2 Hz, 2H), 5.10 (dd, $J$ = 12.9, 5.4 Hz, 1H), 4.18 (d, $J$ = 5.0 Hz, 1H), 3.56 (t, $J$ = 9.7 Hz, 2H), 3.50 – 3.43 (m, 2H), 3.33 – 3.28 (m, 1H), 3.23 (d, $J$ = 11.5 Hz, 2H), 3.10 (d, $J$ = 6.2 Hz, 2H), 3.04 – 2.87 (m, 5H), 2.72 (d, $J$ = 17.5 Hz, 2H), 2.60 (dt, $J$ = 17.3, 3.0 Hz, 2H), 2.23 – 1.88 (m, 6H), 1.83 (d, $J$ = 12.7 Hz, 2H), 1.72 (dd, $J$ = 12.6, 6.4 Hz, 1H), 1.34 (d, $J$ = 12.2 Hz, 2H). | 779.61 | General Synthesis Formula 6 |

| | | | | | |
|---|---|---|---|---|---|
| **2** | A2 | 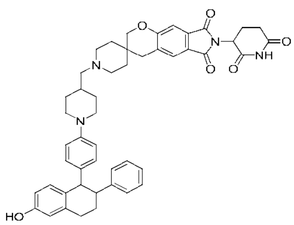 7'-(2,6-Dioxopiperidin-3-yl)-1-((1-(4-(6-hydroxy-2-phenyl-1,2,3,4-tetrahydronaphthalen-1-yl)phenyl)piperidin-4-yl)methyl)-2'H-spiro[piperidine-4,3'-pyrano[2,3-f]isoindole]-6',8'(4'H,7'H)-dione | $^1$H NMR (600 MHz, DMSO-$d_6$) δ 11.12 (s, 1H), 9.06 (s, 1H), 7.73 (s, 1H), 7.61 (s, 1H), 7.30 (d, *J* = 4.9 Hz, 1H), 7.20 – 7.08 (m, 3H), 6.87 – 6.80 (m, 2H), 6.69 – 6.62 (m, 3H), 6.49 (dd, *J* = 8.3, 2.5 Hz, 1H), 6.28 (d, *J* = 8.1 Hz, 2H), 5.10 (dd, *J* = 12.9, 5.4 Hz, 1H), 4.32 (s, 1H), 4.17 (d, *J* = 5.0 Hz, 1H), 3.99 (d, *J* = 45.5 Hz, 1H), 3.44 (dt, *J* = 18.1, 8.9 Hz, 3H), 3.34 – 3.26 (m, 1H), 3.13 (dt, *J* = 21.8, 10.5 Hz, 2H), 3.06 (d, *J* = 8.1 Hz, 3H), 3.02 – 2.83 (m, 2H), 2.79 (s, 1H), 2.69 (s, 2H), 2.63 – 2.57 (m, 1H), 2.13 – 2.00 (m, 2H), 1.94 (dd, *J* = 16.3, 9.3 Hz, 2H), 1.81 (d, *J* = 12.9 Hz, 3H), 1.73 (q, *J* = 8.7, 5.3 Hz, 4H), 1.60 (d, *J* = 14.0 Hz, 1H), 1.44 – 1.29 (m, 2H). | 777.60 | General Synthesis Formula 6 |
| **3** | A4 | 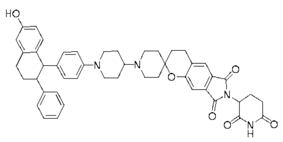 7'-(2,6-Dioxopiperidin-3-yl)-1-((1-(4-(6-hydroxy-2-phenyl-1,2,3,4-tetrahydronaphthalen-1-yl)phenyl)piperidin-4-yl)methyl)-2'H-spiro[piperidine-4,3'-pyrano[2,3-f]isoindole]-6',8'(4'H,7'H)-dione | $^1$H NMR (600 MHz, DMSO-$d_6$) δ 11.11 (s, 1H), 9.22 (s, 1H), 7.75 (s, 1H), 7.41 (s, 1H), 7.14 (ddd, *J* = 14.6, 6.2, 3.2 Hz, 3H), 6.85 (d, *J* = 7.3 Hz, 2H), 6.67 – 6.58 (m, 4H), 6.49 (dd, *J* = 8.3, 2.6 Hz, 1H), 6.24 (d, *J* = 8.5 Hz, 2H), 5.10 (dd, *J* = 12.9, 5.5 Hz, 1H), 4.16 (d, *J* = 5.0 Hz, 1H), 3.50 – 3.42 (m, 3H), 3.39 – 3.20 (m, 4H), 3.03 – 2.86 (m, 5H), 2.70 – 2.54 (m, 4H), 2.21 – 1.98 (m, 6H), 1.96 – 1.84 (m, 5H), 1.71 (td, *J* = 12.3, 4.4 Hz, 2H). | 765.64 | General Synthesis Formula 6 |
| **4** | A6 | 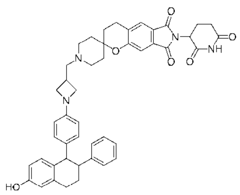 7'-(2,6-Dioxopiperidin-3-yl)-1-(1-(4-(6-hydroxy-2-phenyl-1,2,3,4-tetrahydronaphthalen-1-yl)phenyl)piperidin-3-yl)methyl)-3',4'-dihydro-6'H-spiro[piperidine-4,2'-pyrano[2,3-f]isoindole]-6',8'(7'H)-dione | $^1$H NMR (600 MHz, DMSO-$d_6$) δ 11.12 (s, 1H), 9.11 (d, *J* = 10.0 Hz, 1H), 7.88 – 7.81 (m, 1H), 7.75 (s, 1H), 7.45 – 7.37 (m, 1H), 7.34–7.27 (m, 1H), 7.23 – 7.09 (m, 3H), 6.87 – 6.81 (m, 2H), 6.67 – 6.57 (m, 2H), 6.48 (td, *J* = 9.0, 8.2, 3.6 Hz, 1H), 6.27 – 6.18 (m, 1H), 6.15 – 6.06 (m, 1H), 5.10 (dd, *J* = 13.0, 5.3 Hz, 1H), 4.29 (s, 1H), 4.11 (dd, *J* = 27.0, 5.1 Hz, 1H), 3.89 (q, *J* = 7.7 Hz, 4H), 3.05 – 2.73 (m, 7H), 2.64 – 2.57 (m, 4H), 2.24 –1.79 (m, 9H), 1.73 (d, *J* = 31.9 Hz, 1H), 1.24 (s, 1H), 1.07 – 0.69 (m, 1H). | 751.61 | General Synthesis Formula 6 |

| | | | | | |
|---|---|---|---|---|---|
| **5** | A7 | 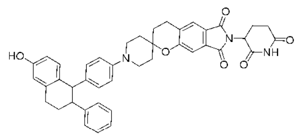 7'-(2,6-Dioxopiperidin-3-yl)-1-(4-(6-hydroxy-2-phenyl-1,2,3,4-tetrahydronaphthalen-1-yl)phenyl)-3',4'-dihydro-6'H-spiro[piperidine-4,2'-pyrano[2,3-f]isoindole]-6',8'(7'H)-dione | $^1$H NMR (600 MHz, DMSO-$d_6$) δ 11.11 (s, 1H), 9.15 (s, 1H), 7.71 (s, 1H), 7.30 (s, 1H), 7.15 (dt, $J$ = 15.1, 7.1 Hz, 3H), 7.00 (s, 1H), 6.89 – 6.81 (m, 3H), 6.74 – 6.58 (m, 2H), 6.49 (dd, $J$ = 8.3, 2.5 Hz, 1H), 6.29 (s, 2H), 5.09 (dd, $J$ = 12.9, 5.4 Hz, 1H), 3.34 (d, $J$ = 13.0 Hz, 3H), 3.07 – 2.82 (m, 5H), 2.66 – 2.52 (m, 2H), 2.44 – 2.32 (m, 1H), 2.18 – 1.97 (m, 2H), 1.94 – 1.65 (m, 7H), 1.32 – 1.18 (m, 2H). | 682.53 | General Synthesis Formula 6 |
| **6** | A8 | 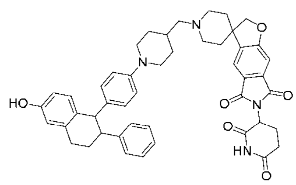 7'-(2,6-Dioxopiperidin-3-yl)-1-(1-(4-(6-hydroxy-2-phenyl-1,2,3,4-tetrahydronaphthalen-1-yl)phenyl)piperidin-4-yl)methyl)-2'H-spiro[piperidine-4,3'-pyrano[2,3-f]isoindole]-6',8'(4'H,7'H)-dione | $^1$H NMR (600 MHz, DMSO-$d_6$) δ 11.13 (d, $J$ = 2.7 Hz, 1H), 9.19 (s, 1H), 7.60 (s, 1H), 7.37 (d, $J$ = 14.3 Hz, 1H), 7.22 – 7.08 (m, 3H), 6.90 – 6.82 (m, 2H), 6.79 – 6.57 (m, 4H), 6.49 (dd, $J$ = 8.3, 2.5 Hz, 1H), 6.28 (d, $J$ = 8.1 Hz, 2H), 5.11 (dd, $J$ = 13.0, 5.3 Hz, 1H), 4.73 (d, $J$ = 18.2 Hz, 2H), 4.18 (d, $J$ = 5.0 Hz, 1H), 3.62 – 3.48 (m, 7H), 3.43 – 3.27 (m, 5H), 3.13 – 2.84 (m, 7H), 2.26 (s, 1H), 2.00 (d, $J$ = 13.9 Hz, 2H), 1.85 (dd, $J$ = 30.2, 12.9 Hz, 2H), 1.72 (dd, $J$ = 12.5, 6.1 Hz, 1H), 1.34 (s, 2H). | 765.68 | General Synthesis Formula 6 |
| **7** | A9 | 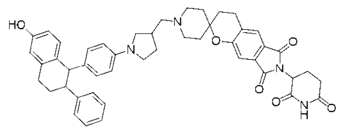 7'-(2,6-Dioxopiperidin-3-yl)-1-(1-(4-(6-hydroxy-2-phenyl-1,2,3,4-tetrahydronaphthalen-1-yl)phenyl)pyrrolidin-3-yl)methyl)-3',4'-dihydro-6'H-spiro[piperidine-4,2'-pyrano[2,3-f]isoindole]-6',8'(7'H)-dione | $^1$H NMR (600 MHz, DMSO-$d_6$) δ 11.13 (s, 1H), 9.18 (s, 1H), 7.76 (s, 1H), 7.40 (d, $J$ = 1.7 Hz, 1H), 7.27 – 7.07 (m, 3H), 6.93 – 6.82 (m, 2H), 6.75 – 6.57 (m, 2H), 6.59 – 6.47 (m, 1H), 6.28 – 6.09 (m, 4H), 5.10 (dd, $J$ = 12.9, 5.5 Hz, 1H), 4.12 (d, $J$ = 4.9 Hz, 1H), 3.30 – 3.04 (m, 10H), 3.02 – 2.80 (m, 6H), 2.78 – 2.64 (m, 1H), 2.24 – 1.86 (m, 8H), 1.80 – 1.61 (m, 2H), 1.34 – 1.12 (m, 2H). | 765.63 | General Synthesis Formula 6 |
| **8** | A10 | 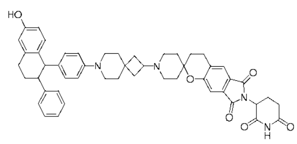 7'-(2,6-Dioxopiperidin-3-yl)-1-(7-(4-(6-hydroxy-2-phenyl-1,2,3,4-tetrahydronaphthalen-1-yl)phenyl)-7-azaspiro[3.5]nonan-2-yl)-3',4'-dihydro- | $^1$H NMR (600 MHz, DMSO-$d_6$) δ 11.10 (s, 1H), 9.52 (s, 1H), 7.75 (d, $J$ = 5.7 Hz, 1H), 7.35 (d, $J$ = 9.5 Hz, 1H), 7.14 (dt, $J$ = 12.4, 6.7 Hz, 3H), 6.84 (d, $J$ = 7.3 Hz, 2H), 6.72 – 6.57 (m, 4H), 6.49 (dd, $J$ = 8.3, 2.5 Hz, 1H), 6.25 (d, $J$ = 7.8 Hz, 2H), 5.10 (dd, $J$ = 12.9, 5.5 Hz, 1H), 4.16 (d, $J$ = 5.0 Hz, 1H), 3.82 (d, $J$ = 8.5 Hz, 1H), | 805.73 | General Synthesis Formula 6 |

| | | | | | |
|---|---|---|---|---|---|
| | | 6'H-spiro[piperidine-4,2'-pyrano[2,3-f]isoindole]-6',8'(7'H)-dione | 3.31 (d, $J$ = 11.7 Hz, 2H), 3.12 – 2.86 (m, 12H), 2.67 – 2.55 (m, 2H), 2.20 (q, $J$ = 9.6, 8.6 Hz, 2H), 2.01 (dddd, $J$ = 61.3, 31.4, 14.3, 7.8 Hz, 7H), 1.88 – 1.55 (m, 7H). | | |
| **9** | A11 | 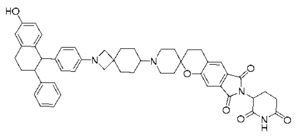 7'-(2,6-Dioxopiperidin-3-yl)-1-(2-(4-(6-hydroxy-2-phenyl-1,2,3,4-tetrahydronaphthalen-1-yl)phenyl)-2-azaspiro[3.5]nonan-7-yl)-3',4'-dihydro-6'H-spiro[piperidine-4,2'-pyrano[2,3-f]isoindole]-6',8'(7'H)-dione | $^1$H NMR (400 MHz, DMSO-$d_6$) δ 11.10 (s, 1H), 9.21 (s, 1H), 7.74 (s, 1H), 7.43 (s, 1H), 7.20 – 7.06 (m, 3H), 6.89 – 6.78 (m, 2H), 6.67 – 6.54 (m, 2H), 6.47 (dd, $J$ = 8.3, 2.5 Hz, 1H), 6.18 (d, $J$ = 8.2 Hz, 2H), 6.03 (d, $J$ = 8.2 Hz, 2H), 5.09 (dd, $J$ = 12.9, 5.4 Hz, 1H), 4.11 (d, $J$ = 4.9 Hz, 1H), 3.46 – 3.34 (m, 5H), 3.31 – 3.17 (m, 4H), 2.91 (dt, $J$ = 31.6, 10.7 Hz, 5H), 2.58 (td, $J$ = 14.2, 4.1 Hz, 1H), 2.20 – 1.83 (m, 11H), 1.74 – 1.65 (m, 3H), 1.57 – 1.39 (m, 4H). | 805.78 | General Synthesis Formula 6 |
| **10** | A12 | 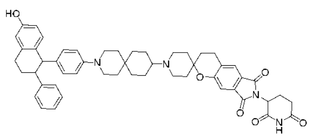 7'-(2,6-Dioxopiperidin-3-yl)-1-(9-(4-(6-hydroxy-2-phenyl-1,2,3,4-tetrahydronaphthalen-1-yl)phenyl)spiro[5.5]undecan-3-yl]-3',4'-dihydro-6'H-spiro[piperidine-4,2'-pyrano[2,3-f]isoindole]-6',8'(7'H)-dione | $^1$H NMR (400 MHz, DMSO-$d_6$) δ 11.10 (s, 1H), 9.19 (s, 1H), 7.74 (s, 1H), 7.42 (s, 1H), 7.22 – 7.08 (m, 4H), 7.04 – 6.93 (m, 1H), 6.88 – 6.77 (m, 2H), 6.77 – 6.56 (m, 2H), 6.48 (dd, $J$ = 8.3, 2.5 Hz, 1H), 6.28 (s, 2H), 5.12 (s, 2H) , 5.10 (dd, $J$ = 13.0, 5.3 Hz, 1H), 4.17 (d, $J$ = 4.9 Hz, 1H), 3.42 (d, $J$ = 11.8 Hz, 2H), 3.28 (dd, $J$ = 23.2, 11.5 Hz, 2H), 3.07 (s, 3H), 2.98 – 2.83 (m, 9H), 2.68 – 2.55 (m, 3H), 2.11 – 1.96 (m, 6H), 1.97 – 1.84 (m, 5H), 1.76 – 1.57 (m, 3H), 1.44 (s, 2H), 1.18 (d, $J$ = 14.0 Hz, 2H). | 833.87 | General Synthesis Formula 6 |
| **11** | A13 | 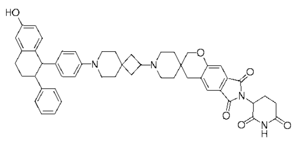 7'-(2,6-Dioxopiperidin-3-yl)-1-(7-(4-(6-hydroxy-2-phenyl-1,2,3,4-tetrahydronaphthalen-1-yl)phenyl)-7-azaspiro[3.5]nonan-2-yl)-2'H-spiro[piperidine-4,3'-pyrano[2,3-f]isoindole]-6',8'(4'H,7'H)-dione | $^1$H NMR (600 MHz, DMSO-$d_6$) δ 11.12 (s, 1H), 9.37 (s, 1H), 7.80 – 7.57 (m, 2H), 7.30 (d, $J$ = 5.3 Hz, 1H), 7.23 – 7.09 (m, 3H), 7.05 – 6.79 (m, 3H), 6.75 – 6.59 (m, 3H), 6.48 (dd, $J$ = 8.2, 2.6 Hz, 1H), 6.32 – 6.19 (m, 1H), 5.10 (dd, $J$ = 12.9, 5.4 Hz, 1H), 4.38 – 4.05 (m, 3H), 3.31 (d, $J$ = 13.0 Hz, 3H), 3.11 – 2.78 (m, 10H), 2.68 – 2.56 (m, 1H), 2.29 – 1.89 (m, 7H), 1.81 – 1.45 (m, 10H). | 805.67 | General Synthesis Formula 6 |

| | | | | | |
|---|---|---|---|---|---|
| **12** | A14 | 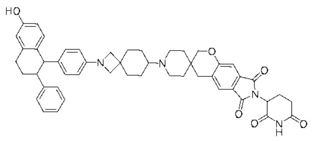 7'-(2,6-Dioxopiperidin-3-yl)-1-(2-(4-(6-hydroxy-2-phenyl-1,2,3,4-tetrahydronaphthalen-1-yl)phenyl)-2-azaspiro[3.5]nonan-7-yl)-2'H-spiro[piperidine-4,3'-pyrano[2,3-f]isoindole]-6',8'(4'H,7'H)-dione | $^1$H NMR (600 MHz, DMSO-$d_6$) δ 11.12 (s, 1H), 9.11 (s, 1H), 7.68 (d, $J$ = 53.4 Hz, 1H), 7.30 (d, $J$ = 2.4 Hz, 1H), 7.21 – 7.09 (m, 3H), 6.90 – 6.79 (m, 2H), 6.66 – 6.57 (m, 2H), 6.48 (dd, $J$ = 8.2, 2.5 Hz, 1H), 6.18 (d, $J$ = 8.2 Hz, 2H), 6.10 – 6.01 (m, 2H), 5.10 (dd, $J$ = 12.9, 5.5 Hz, 1H), 4.31 (s, 1H), 4.12 (d, $J$ = 5.0 Hz, 1H), 4.03 (s, 1H), 3.47 – 3.33 (m, 6H), 3.30 – 3.04 (m, 7H), 3.01 – 2.85 (m, 3H), 2.78 (s, 1H), 2.65 – 2.57 (m, 1H), 2.15 – 1.96 (m, 5H), 1.80 – 1.60 (m, 4H), 1.56 – 1.43 (m, 4H). | 805.67 | General Synthesis Formula 6 |
| **13** | A15 | 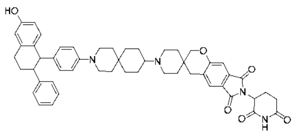 7'-(2,6-Dioxopiperidin-3-yl)-1-(2-(4-(6-hydroxy-2-phenyl-1,2,3,4-tetrahydronaphthalen-1-yl)phenyl)-2-azaspiro[3.5]nonan-7-yl)-3',4'-dihydro-6'H-spiro[piperidine-4,2'-pyrano[2,3-f]isoindole]-6',8'(7'H)-dione | $^1$H NMR (600 MHz, DMSO-$d_6$) δ 11.12 (s, 1H), 9.14 (s, 1H), 7.72 (s, 1H), 7.64 (s, 1H), 7.30 (d, $J$ = 3.0 Hz, 1H), 7.23 – 7.08 (m, 3H), 6.89 – 6.79 (m, 2H), 6.67 – 6.59 (m, 2H), 6.49 (dd, $J$ = 8.3, 2.6 Hz, 1H), 6.27 (s, 3H), 5.10 (dd, $J$ = 12.9, 5.5 Hz, 1H), 4.31 (s, 1H), 4.17 (s, 1H), 4.03 (s, 1H), 3.40 (d, $J$ = 12.0 Hz, 2H), 3.31 (d, $J$ = 13.5 Hz, 2H), 3.18 (q, $J$ = 12.0, 11.6 Hz, 4H), 3.10 – 2.85 (m, 4H), 2.79 (s, 1H), 2.62 (dt, $J$ = 4.1, 2.0 Hz, 2H), 2.16 – 2.00 (m, 3H), 1.90 (d, $J$ = 19.6 Hz, 3H), 1.79 – 1.54 (m, 10H), 1.43 (s, 2H), 1.16 (s, 2H). | 833.70 | General Synthesis Formula 6 |
| **14** | A16 | 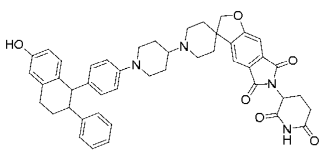 6-(2,6-Dioxopiperidin-3-yl)-1'-(4-(6-hydroxy-2-phenyl-1,2,3,4-tetrahydronaphthalen-1-yl)phenyl)piperidin-4-yl)-6-hydro-2H,5H-spiro[furo[2,3-f]isoindole-3,4'-piperidine]-5,7-dione | $^1$H NMR (600 MHz, DMSO-$d_6$) δ 11.12 (d, $J$ = 3.8 Hz, 1H), 9.37 (s, 1H), 7.62 (s, 1H), 7.36 (d, $J$= 12.9 Hz, 1H), 7.20 – 7.10 (m, 3H), 6.89 – 6.81 (m, 2H), 6.68 – 6.59 (m, 4H), 6.49 (dt, $J$ = 8.3, 2.9 Hz, 1H), 6.25 (dd, $J$ = 8.4, 6.0 Hz, 2H), 5.12 (ddd, $J$ = 13.1, 8.6, 5.4 Hz, 1H), 4.74 (d, $J$ = 18.7 Hz, 2H), 4.16 (d, $J$ = 5.0 Hz, 1H), 3.39 – 3.28 (m, 2H), 3.16 – 3.04 (m, 2H), 3.02 – 2.84 (m, 3H), 2.81 – 2.55 (m, 4H), 2.27 – 2.15 (m, 2H), 2.14 – 1.97 (m, 6H), 1.92 – 1.82 (m, 1H), 1.71 (tt, $J$ = 13.9, 7.6 Hz, 3H), 1.63 – 1.44 (m, 1H), 1.24 (s, 1H). | 751.57 | General Synthesis Formula 6 |

| | | | | | |
|---|---|---|---|---|---|
| **15** | A17 | 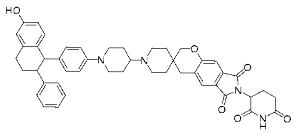 6-(2,6-Dioxopiperidin-3-yl)-1'-(4-(6-hydroxy-2-phenyl-1,2,3,4-tetrahydronaphthalen-1-yl)phenyl)piperidin-4-yl)-6-hydro-2H,5H-spiro[furo[2,3-f]isoindole-3,4'-piperidine]-5,7-dione | $^1$H NMR (400 MHz, DMSO-$d_6$) δ 11.11 (s, 1H), 9.82 (d, $J$ = 36.2 Hz, 1H), 7.66 (s, 1H), 7.36 (d, $J$ = 8.9 Hz, 1H), 7.19 – 6.97 (m, 5H), 6.84 (s, 4H), 6.55 (d, $J$ = 74.8 Hz, 3H), 5.11 (dd, $J$ = 12.6, 5.6 Hz, 1H), 4.73 (d, $J$ = 9.5 Hz, 2H), 3.42 – 2.99 (m, 5H), 2.99 – 2.77 (m, 4H), 2.67 – 2.54 (m, 4H), 2.37 – 1.87 (m, 9H), 1.85 – 1.55 (m, 4H). | 765.70 | General Synthesis Formula 6 |
| **16** | A18 | 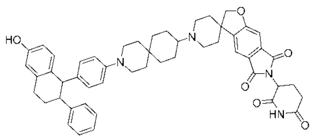 6-(2,6-Dioxopiperidin-3-yl)-1'-(9-(4-(6-hydroxy-2-phenyl-1,2,3,4-tetrahydronaphthalen-1-yl)phenyl)-3-azaspiro[5.5]undecan-3-yl)-6-hydro-2H,5H-spiro[furo[2,3-f]isoindole-3,4'-piperidine]-5,7-dione | $^1$H NMR (400 MHz, DMSO-$d_6$) δ 11.11 (s, 1H), 9.30 (s, 1H), 7.61 (s, 1H), 7.35 (d, $J$ = 8.1 Hz, 1H), 7.26 – 7.06 (m, 4H), 6.88 – 6.81 (m, 2H), 6.76 – 6.59 (m, 4H), 6.48 (dd, $J$ = 8.3, 2.5 Hz, 1H), 6.27 (d, $J$ = 8.1 Hz, 2H), 5.11 (dt, $J$ = 12.5, 5.8 Hz, 1H), 4.73 (d, $J$ = 14.6 Hz, 2H), 4.17 (d, $J$ = 5.0 Hz, 1H), 3.62 – 3.52 (m, 5H), 3.20 – 2.82 (m, 8H), 2.74 – 2.57 (m, 2H), 2.37 – 1.98 (m, 6H), 1.93 – 1.38 (m, 8H), 1.30 – 0.83 (m, 4H). | 819.76 | General Synthesis Formula 6 |
| **17** | A19 | 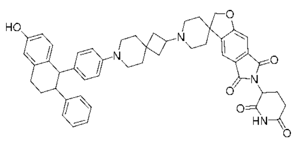 6-(2,6-Dioxopiperidin-3-yl)-1'-(7-(4-(6-hydroxy-2-phenyl-1,2,3,4-tetrahydronaphthalen-1-yl)phenyl)-7-azaspiro[3.5]nonan-2-yl)-6-hydro-2H,5H-spiro[furo[2,3-f]isoindole-3,4'-piperidine]-5,7-dione | $^1$H NMR (600 MHz, DMSO-$d_6$) δ 11.12 (s, 1H), 9.30 (s, 1H), 8.24 (s, 1H), 7.65 (s, 1H), 7.36 (d, $J$ = 13.7 Hz, 1H), 7.16 (ddt, $J$ = 19.8, 12.5, 6.7 Hz, 3H), 6.84 (d, $J$ = 7.2 Hz, 2H), 6.73 – 6.56 (m, 6H), 6.49 (dd, $J$ = 8.3, 2.5 Hz, 2H), 6.26 (d, $J$ = 8.1 Hz, 3H), 5.10 (dd, $J$ = 12.9, 5.5 Hz, 1H), 4.72 (d, $J$ = 13.8 Hz, 2H), 4.17 (d, $J$ = 5.0 Hz, 1H), 3.42 – 3.13 (m, 7H), 3.10 – 2.74 (m, 4H), 2.60 (dt, $J$ = 17.0, 3.4 Hz, 1H), 2.28 – 1.90 (m, 7H), 1.85 – 1.57 (m, 7H), 1.37 – 1.03 (m, 1H). | 791.70 | General Synthesis Formula 6 |
| **18** | A20 | 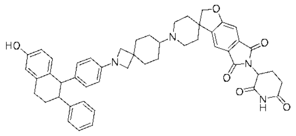 6-(2,6-Dioxopiperidin-3-yl)-1'-(2-(4-(6-hydroxy-2-phenyl-1,2,3,4-tetrahydronaphthalen-1-yl)phenyl)-2-azaspiro[3.5]nonan-7-yl)-6-hydro-2H,5H-spiro[furo[2,3-f]isoindole-3,4'- | $^1$H NMR (600 MHz, DMSO-$d_6$) δ 11.12 (s, 1H), 9.22 (s, 1H), 7.61 (s, 1H), 7.36 (d, $J$ = 11.4 Hz, 1H), 7.20 – 7.08 (m, 3H), 6.84 (d, $J$ = 7.4 Hz, 2H), 6.66 – 6.59 (m, 2H), 6.48 (dd, $J$ = 8.3, 2.5 Hz, 1H), 6.19 (d, $J$ = 8.1 Hz, 2H), 6.05 (t, $J$ = 10.2 Hz, 2H), 5.10 (dd, $J$ = 12.9, 5.5 Hz, 1H), 4.75 (s, 2H), 4.12 (d, $J$ = 4.9 Hz, 1H), 3.39 – 3.20 (m, 4H), 3.18 – 3.05 (m, 2H), 3.01 – | 791.69 | General Synthesis Formula 6 |

| | | | | | |
|---|---|---|---|---|---|
| | | piperidine]-5,7-dione | 2.85 (m, 4H), 2.69 – 2.57 (m, 2H), 2.27 – 1.95 (m, 9H), 1.78 – 1.65 (m, 2H), 1.60 – 1.36 (m, 5H), 1.25 (d, *J* = 5.4 Hz, 1H). | | |
| **19** | A21 | 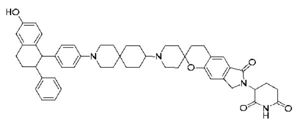 3-(1-(3-(4-(6-Hydroxy-2-phenyl-1,2,3,4-tetrahydronaphthalen-1-yl)phenyl)-3-azaspiro[5.5]undecan-9-yl)-6'-oxo-3',4',6',8'-tetrahydro-7'H-spiro[piperidine-4,2'-pyrano[2,3-f]isoindol]-7'-yl)piperidine-2,6-dione | $^1$H NMR (600 MHz, DMSO-$d_6$) δ 10.96 (s, 1H), 9.12 (s, 1H), 7.51 (s, 1H), 7.23 – 7.10 (m, 3H), 7.03 (d, *J* = 33.0 Hz, 1H), 6.84 (d, *J* = 7.3 Hz, 3H), 6.66 – 6.58 (m, 3H), 6.49 (dd, *J* = 8.3, 2.5 Hz, 1H), 6.24 (d, *J* = 7.6 Hz, 2H), 5.07 (dd, *J* = 13.3, 5.3 Hz, 1H), 4.37 – 4.30 (m, 1H), 4.25 – 4.13 (m, 2H), 3.32 – 3.14 (m, 1H), 3.08 – 2.86 (m, 9H), 2.74 (d, *J* = 5.0 Hz, 1H), 2.64 – 2.56 (m, 1H), 2.41 – 2.30 (m, 1H), 2.14 – 1.69 (m, 11H), 1.68 – 1.33 (m, 7H), 1.31 – 1.06 (m, 4H). | 819.77 | General Synthesis Formula 6 |
| **20** | A22 | 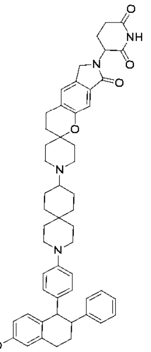 3-(1-(3-(4-(6-Hydroxy-2-phenyl-1,2,3,4-tetrahydronaphthalen-1-yl)phenyl)-3-azaspiro[5.5]undecan-9-yl)-8'-oxo-3',4',6',8'-tetrahydro-7'H-spiro[piperidine-4,2'-pyrano[2,3-f]isoindol]-7'-yl)piperidine-2,6-dione | $^1$H NMR (600 MHz, DMSO-$d_6$) δ 10.97 (s, 1H), 9.15 (s, 2H), 7.36 (s, 1H), 7.23 (s, 1H), 7.21 – 7.09 (m, 4H), 6.86 – 6.78 (m, 2H), 6.73 – 6.55 (m, 4H), 6.49 (dd, *J* = 8.3, 2.5 Hz, 1H), 6.27 (s, 2H), 5.07 (dd, *J* = 13.3, 5.2 Hz, 1H), 4.34 (d, *J* = 16.6 Hz, 1H), 4.25 – 4.13 (m, 2H), 3.34 – 3.18 (m, 3H), 3.13 – 2.80 (m, 9H), 2.63 – 2.57 (m, 1H), 2.44 – 2.35 (m, 1H), 2.14 – 1.77 (m, 10H), 1.77 – 1.54 (m, 5H), 1.43 (s, 2H), 1.20 (d, *J* = 46.5 Hz, 3H). | 819.74 | General Synthesis Formula 6 |
| **21** | A23 | 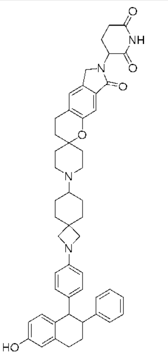 3-(1-(2-(4-(6-hydroxy-2-phenyl-1,2,3,4-tetrahydronaphthalen-1-yl)phenyl)-2-azaspiro[3.5]nonane-7- | $^1$H NMR (600 MHz, DMSO-$d_6$) δ 10.98 (s, 1H), 9.15 (s, 1H), 7.35 (s, 1H), 7.24 (s, 1H), 7.19 – 7.08 (m, 3H), 6.83 (d, *J* = 7.4 Hz, 2H), 6.66 – 6.56 (m, 2H), 6.48 (dd, *J* = 8.3, 2.6 Hz, 1H), 6.18 (d, *J* = 8.1 Hz, 2H), 6.04 (d, *J* = 8.0 Hz, 2H), 5.07 (dd, *J* = 13.3, 5.2 Hz, 1H), 4.34 (d, *J* = 16.6 Hz, 1H), 4.23 (s, 1H), 4.12 (d, *J* = 5.0 Hz, 1H), 3.46 – 3.32 (m, 7H), 3.26 (dt, *J* = 24.3, 7.5 Hz, 5H), 2.91 (ddq, *J* = 18.8, 12.6, 5.7 Hz, 5H), 2.65 – 2.51 (m, 1H), 2.39 (s, 0H), 2.11 – 1.95 (m, 8H), 1.87 (q, *J* = 6.6 Hz, | 791.75 | General Synthesis Formula 6 |

| | | | | | |
|---|---|---|---|---|---|
| | | yl)-8'-oxo-3',4',6',8'-tetrahydro-7'H-spiro[piperidine-4,2'-pyrrolo[2,3-f]isoindole]-7'-yl)piperidine-2,6-dione | 4H), 1.70 (d, *J* = 6.3 Hz, 1H), 1.52 (d, *J* = 10.1 Hz, 4H), 1.24 (s, 1H). | | |
| **22** | A24 | 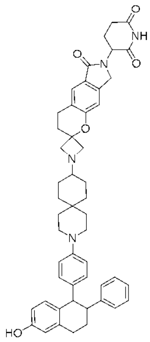 3-(1-(3-(4-(6-hydroxy-2-phenyl-1,2,3,4-tetrahydronaphthalen-1-yl)phenyl)-3-azaspiro[5.5]undecane-9-yl)-6'-oxo-3',4',6',8'-tetrahydro-7'H-spiro[azetidine-3,2'-pyrrolo[2,3-f]isoindole]-7'-yl)piperidine-2,6-dione | $^1$H NMR (600 MHz, DMSO-$d_6$) δ 10.97 (s, 1H), 10.15 (s, 0H), 7.54 (s, 1H), 7.22 – 7.00 (m, 4H), 6.84 (d, *J* = 7.3 Hz, 2H), 6.74 – 6.58 (m, 3H), 6.49 (dd, *J* = 8.3, 2.5 Hz, 1H), 6.41 – 6.22 (m, 2H), 5.07 (ddd, *J* = 13.4, 5.2, 2.2 Hz, 1H), 4.47 – 4.30 (m, 4H), 4.29 – 4.13 (m, 3H), 3.36 – 3.28 (m, 1H), 3.22 (s, 1H), 3.08 (s, 3H), 2.93 (dddt, *J* = 34.2, 28.9, 13.6, 6.3 Hz, 5H), 2.71 – 2.56 (m, 1H), 2.36 (qd, *J* = 13.3, 12.7, 4.6 Hz, 1H), 2.24 – 1.94 (m, 4H), 1.87 – 1.68 (m, 5H), 1.52 (d, *J* = 66.8 Hz, 5H), 1.40 – 1.22 (m, 3H), 1.10 (q, *J* = 17.7, 13.8 Hz, 2H). | 791.77 | General Synthesis Formula 6 |
| **23** | A25 | 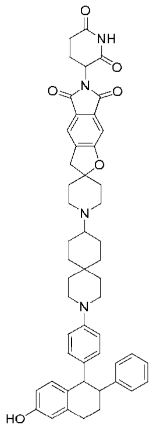 6-(2,6-dioxopiperidin-3-yl)-1'-(3-(4-(6-hydroxy-2-phenyl-1,2,3,4-tetrahydronaphthalen-1-yl)phenyl)-3-azaspiro[5.5]undecane-9-yl)spiro[furo[2,3-f]isoindole-2,4'-piperidine]-5,7(3H,6H)-dione | $^1$H NMR (600 MHz, DMSO-$d_6$) δ 11.11 (s, 1H), 9.37 (s, 1H), 7.63 (s, 1H), 7.28 (s, 1H), 7.18 – 7.08 (m, 3H), 6.84 (d, *J* = 7.3 Hz, 2H), 6.72 (s, 2H), 6.67 – 6.58 (m, 2H), 6.53 – 6.42 (m, 2H), 6.36 – 6.19 (m, 2H), 5.09 (dd, *J* = 12.9, 5.5 Hz, 1H), 4.18 (d, *J* = 5.1 Hz, 1H), 3.60 (d, *J* = 11.4 Hz, 1H), 3.07 (d, *J* = 10.8 Hz, 5H), 2.93 – 2.79 (m, 6H), 2.81 – 2.59 (m, 4H), 2.18 – 1.97 (m, 2H), 1.89 (d, *J* = 28.7 Hz, 3H), 1.72 (dd, *J* = 12.5, 6.1 Hz, 1H), 1.66 – 1.54 (m, 4H), 1.44 (s, 2H), 1.31 – 1.07 (m, 11H). | 819.80 | General Synthesis Formula 6 |

| | | | | | |
|---|---|---|---|---|---|
| **24** | A26 | 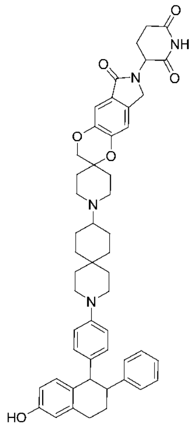 3-(1-(3-(4-(6-hydroxy-2-phenyl-1,2,3,4-tetrahydronaphthalen-1-yl)phenyl)-3-azaspiro[5.5]undecane-9-yl)-6'-oxo-6',8'-dihydro-3'H,7'H-spiro[1,4]dioxacyclohexa[2,3-f]isoindole]-7'-yl)piperidine-2,6-dione | $^1$H NMR (600 MHz, DMSO-$d_6$) δ 10.97 (s, 1H), 9.55 (d, $J$ = 10.1 Hz, 1H), 7.23 (s, 1H), 7.20 – 7.13 (m, 4H), 6.84 (d, $J$ = 7.3 Hz, 2H), 6.73 (s, 2H), 6.67 – 6.59 (m, 2H), 6.49 (dd, $J$ = 8.3, 2.5 Hz, 1H), 6.29 (d, $J$ = 8.1 Hz, 2H), 5.07 (dd, $J$ = 13.2, 5.4 Hz, 1H), 4.37 – 4.27 (m, 2H), 4.26 – 4.11 (m, 4H), 3.34 – 3.15 (m, 3H), 3.12 – 2.80 (m, 9H), 2.65 – 2.55 (m, 1H), 2.37 (qd, $J$ = 13.2, 5.0 Hz, 1H), 2.10 (ddt, $J$ = 19.1, 12.8, 5.8 Hz, 2H), 2.01 – 1.76 (m, 10H), 1.65 (tdd, $J$ = 38.8, 35.1, 18.9, 9.3 Hz, 6H), 1.45 (s, 2H), 1.19 – 1.10 (m, 3H). | 821. 84 | General Synthesis Formula 6 |
| **25** | A27 | 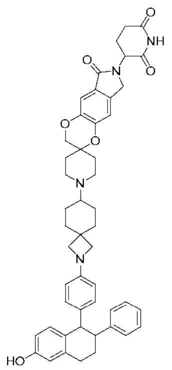 3-(1-(2-(4-(6-hydroxy-2-phenyl-1,2,3,4-tetrahydronaphthalen-1-yl)phenyl)-2-azaspiro[3.5]nonane-7-yl)-6'-oxo-6',8'-dihydro-3'H,7'H-spiro[piperidine-4,2'-[1,4]dioxacyclohexa[2,3-f]isoindole]-7'-yl)piperidine-2,6-dione | $^1$H NMR (600 MHz, DMSO-$d_6$) δ 10.97 (s, 1H), 9.25 (s, 1H), 7.23 (s, 1H), 7.19 – 7.08 (m, 4H), 6.83 (d, $J$ = 7.4 Hz, 2H), 6.64 – 6.54 (m, 2H), 6.48 (dd, $J$ = 8.2, 2.5 Hz, 1H), 6.18 (d, $J$ = 8.1 Hz, 2H), 6.03 (d, $J$ = 8.0 Hz, 2H), 5.07 (dd, $J$ = 13.2, 5.3 Hz, 1H), 4.37 – 4.24 (m, 1H), 4.24 – 4.08 (m, 3H), 3.30 – 3.25 (m, 2H), 3.22 – 3.12 (m, 1H), 2.91 (s, 1H), 2.71 – 2.54 (m, 1H), 2.46 – 2.30 (m, 2H), 2.19 – 1.89 (m, 10H), 1.79 – 1.66 (m, 1H), 1.58 – 1.33 (m, 4H), 1.24 (s, 1H). | 793. 80 | General Synthesis Formula 6 |

| | | | | | |
|---|---|---|---|---|---|
| **26** | A28 | 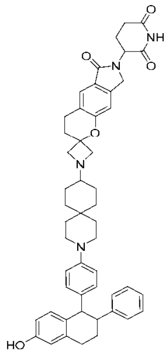 3-(1-(2-(4-(6-hydroxy-2-phenyl-1,2,3,4-tetrahydronaphthalen-1-yl)phenyl)-2-azaspiro[3.5]nonane-7-yl)-6'-oxo-3',4',6',8'-tetrahydro-7'H-spiro[piperidine-4,2'-pyrrolo[2,3-f]isoindole]-7'-yl)piperidine-2,6-dione | $^1$H NMR (600 MHz, DMSO-$d_6$) δ 10.97 (s, 1H), 9.11 (s, 2H), 7.51 (d, *J* = 4.1 Hz, 1H), 7.20 – 7.07 (m, 5H), 6.88 – 6.80 (m, 2H), 6.66 – 6.58 (m, 2H), 6.48 (dd, *J* = 8.2, 2.5 Hz, 1H), 6.19 (s, 1H), 6.04 (d, *J* = 8.1 Hz, 2H), 5.07 (dd, *J* = 13.3, 5.3 Hz, 1H), 4.34 (dd, *J* = 17.0, 4.9 Hz, 1H), 4.21 (dd, *J* = 16.9, 5.9 Hz, 1H), 4.12 (d, *J* = 5.0 Hz, 1H), 3.52 – 3.35 (m, 5H), 3.30 – 3.10 (m, 4H), 2.99 – 2.81 (m, 5H), 2.77 – 2.56 (m, 1H), 2.43 – 2.33 (m, 1H), 1.94 (dt, *J* = 80.0, 6.8 Hz, 9H), 1.77 – 1.63 (m, 1H), 1.58 – 1.32 (m, 4H). | 791.84 | General Synthesis Formula 6 |
| **27** | A29 | 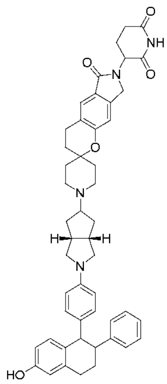 3-(1-(3aR,6aS)-2-(4-(6-hydroxy-2-phenyl-1,2,3,4-tetrahydronaphthalen-1-yl)phenyl)octahydrocyclopenta[6'-oxo-3',4',6',8'-tetrahydro-7'H-spiro[piperidine-4,2'-pyrrolo[2,3-f]isoindole]-7'-yl)piperidine-2,6-dione | $^1$H NMR (600 MHz, DMSO-$d_6$) δ 10.98 (s, 1H), 9.15 (s, 1H), 7.35 (s, 1H), 7.24 (s, 1H), 7.19 – 7.08 (m, 3H), 6.83 (d, *J* = 7.4 Hz, 2H), 6.66 – 6.56 (m, 2H), 6.48 (dd, *J* = 8.3, 2.6 Hz, 1H), 6.18 (d, *J* = 8.1 Hz, 2H), 6.04 (d, *J* = 8.0 Hz, 2H), 5.07 (dd, *J* = 13.3, 5.2 Hz, 1H), 4.34 (d, *J* = 16.6 Hz, 1H), 4.23 (s, 1H), 4.12 (d, *J* = 5.0 Hz, 1H), 3.46 – 3.32 (m, 7H), 3.26 (dt, *J* = 24.3, 7.5 Hz, 5H), 2.91 (ddq, *J* = 18.8, 12.6, 5.7 Hz, 5H), 2.65 – 2.51 (m, 1H), 2.39 (s, 0H), 2.11 – 1.95 (m, 8H), 1.87 (q, *J* = 6.6 Hz, 4H), 1.70 (d, *J* = 6.3 Hz, 1H), 1.52 (d, *J* = 10.1 Hz, 4H), 1.24 (s, 1H). | 777.81 | General Synthesis Formula 6 |
| **28** | A30 | 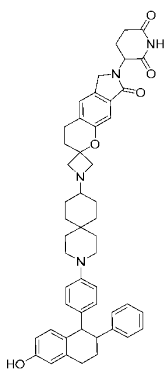 3-(1-(3-(4-(6-hydroxy-2-phenyl- | $^1$H NMR (600 MHz, DMSO-$d_6$) δ 10.98 (s, 1H), 9.13 (s, 1H), 7.39 (d, *J* = 3.4 Hz, 1H), 7.21 – 7.08 (m, 5H), 6.84 (d, *J* = 7.3 Hz, 2H), 6.71 – 6.57 (m, 2H), 6.49 (dd, *J* = 8.3, 2.5 Hz, 1H), 6.26 (s, 2H), 5.08 (dd, *J* = 13.3, 5.2 Hz, 1H), 4.51 – 4.32 (m, 4H), 4.27 – 4.13 (m, 3H), 3.12 – 2.83 (m, 3H), 2.67 – 2.54 (m, 2H), 2.44 – 2.32 (m, 1H), 2.23 – 2.04 (m, 3H), 1.99 (ddd, *J* = 10.8, 5.3, 3.0 Hz, 1H), 1.77 (d, *J* = 50.8 Hz, 4H), 1.49 (d, *J* = 65.7 Hz, 3H), 1.37 – 1.23 | 791.75 | General Synthesis Formula 6 |

| | | | | | |
|---|---|---|---|---|---|
| | | 1,2,3,4-tetrahydronaphthalen-1-yl)phenyl)-3-azaspiro[5.5]undecane-9-yl)-8'-oxo-3',4',6',8'-tetrahydro-7'H-spiro[azetidine-3,2'-pyrrolo[2,3-f]isoindole]-7'-yl)piperidine-2,6-dione | (m, 3H), 1.16 – 0.99 (m, 1H). | | |
| **29** | A32 | 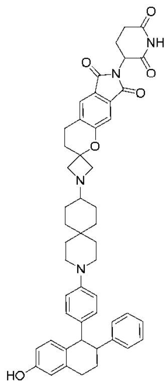 7'-(2,6-dioxopiperidin-3-yl)-1-(3-(4-(6-hydroxy-2-phenyl-1,2,3,4-tetrahydronaphthalen-1-yl)phenyl)-3-azaspiro[5.5]undecane-9-yl)-3',4'-dihydro-6'H-spiro[azetidine-3,2'-pyrrolo[2,3-f]isoindole]-6',8'(7'H)-dione | $^1$H NMR (600 MHz, DMSO-$d_6$) δ 11.12 (s, 1H), 9.15 (s, 1H), 7.79 (s, 1H), 7.29 (d, $J$ = 16.9 Hz, 1H), 7.19 – 7.10 (m, 3H), 6.90 – 6.71 (m, 2H), 6.66 – 6.58 (m, 2H), 6.49 (dd, $J$ = 8.3, 2.5 Hz, 1H), 6.30 (s, 2H), 5.11 (dd, $J$ = 12.9, 5.5 Hz, 1H), 4.53 – 4.35 (m, 3H), 4.30 (dd, $J$ = 12.2, 6.6 Hz, 1H), 4.19 (s, 1H), 3.36 – 3.26 (m, 1H), 3.15 – 2.84 (m, 9H), 2.68 – 2.53 (m, 1H), 2.43 – 2.18 (m, 2H), 2.14 – 1.99 (m, 2H), 1.86 – 1.72 (m, 4H), 1.52 (d, $J$ = 65.9 Hz, 4H), 1.37 – 1.22 (m, 3H), 1.12 (q, $J$ = 8.8, 8.1 Hz, 2H). | 805.81 | General Synthesis Formula 6 |
| **30** | A33 | 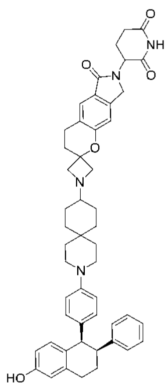 3-(1-(3-(4-(1R,2S)-6-hydroxy-2-phenyl-1,2,3,4-tetrahydronaphthalen-1-ylphenyl)-3-azaspiro[5.5]undecane-9-yl)-6'-oxo-3',4',6',8'-tetrahydro-7'H-spiro[azetidine-3,2'-pyrrolo[2,3-f]isoindole]-7'-yl)piperidine-2,6-dione | $^1$H NMR (400 MHz, DMSO-$d_6$) δ 10.96 (s, 1H), 9.15 (s, 1H), 7.53 (s, 1H), 7.17 – 7.03 (m, 4H), 6.95 – 6.70 (m, 3H), 6.67 – 6.59 (m, 2H), 6.48 (dd, $J$ = 8.3, 2.6 Hz, 1H), 6.31 (d, $J$ = 8.1 Hz, 2H), 5.06 (dd, $J$ = 13.2, 5.1 Hz, 1H), 4.48 – 4.13 (m, 7H), 3.37 – 3.05 (m, 7H), 3.05 – 2.78 (m, 7H), 2.66 – 2.56 (m, 1H), 2.41 – 2.28 (m, 1H), 2.24 – 1.92 (m, 6H), 1.88 – 1.67 (m, 6H), 1.54 (d, $J$ = 45.2 Hz, 5H), 1.42 – 1.21 (m, 3H), 1.11 (h, $J$ = 11.3, 9.6 Hz, 3H). | 791.74 | General Synthesis Formula 6 |

| | | | | | |
|---|---|---|---|---|---|
| **31** | A34 | 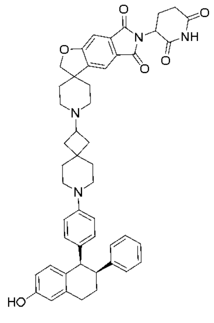<br>6-(2,6-Dioxopiperidin-3-yl)-1'-(7-(4-((1R,2S)-6-hydroxy-2-phenyl-1,2,3,4-tetrahydronaphthalen-1-yl)phenyl)-7-azaspiro[3.5]nonane-2-yl)-6-hydro-2H,5H-spiro[furo[2,3-f]isoindole-3,4'-piperidine]-5,7-dione | $^1$H NMR (600 MHz, DMSO-$d_6$) δ 11.12 (s, 1H), 8.24 (s, 1H), 7.65 (s, 1H), 7.36 (d, $J$ = 13.7 Hz, 1H), 7.16 (ddt, $J$ = 19.8, 12.5, 6.7 Hz, 3H), 6.84 (d, $J$ = 7.2 Hz, 2H), 6.73 – 6.56 (m, 3H), 6.49 (dd, $J$ = 8.3, 2.5 Hz, 1H), 6.26 (d, $J$ = 8.1 Hz, 2H), 5.12 (ddd, $J$ = 12.9, 7.7, 5.3 Hz, 1H), 4.72 (d, $J$ = 13.8 Hz, 2H), 4.17 (d, $J$ = 5.0 Hz, 1H), 3.42 – 3.13 (m, 7H), 3.10 – 2.74 (m, 8H), 2.60 (dt, $J$ = 17.0, 3.4 Hz, 1H), 2.28 – 1.90 (m, 9H), 1.85 – 1.57 (m, 5H), 1.37 – 1.03 (m, 1H). | 791.74 | General Synthesis Formula 6 |
| **32** | A36 | 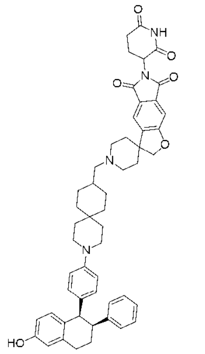<br>6-(2,6-Dioxopiperidin-3-yl)-1'-((3-(4-((1R,2S)-6-hydroxy-2-phenyl-1,2,3,4-tetrahydronaphthalen-1-yl)phenyl)-3-azaspiro[5.5]undecane-9-yl)methyl)-6-hydro-2H,5H-spiro[furo[2,3-f]isoindole-3,4'-piperidine]-5,7-dione | $^1$H NMR (600 MHz, DMSO-$d_6$) δ 11.12 (d, $J$ = 2.3 Hz, 1H), 9.22 (d, $J$ = 74.3 Hz, 1H), 7.59 (s, 2H), 7.36 (d, $J$ = 14.5 Hz, 1H), 7.22 – 7.08 (m, 3H), 6.84 (dd, $J$ = 6.8, 1.8 Hz, 3H), 6.72 – 6.58 (m, 2H), 6.49 (dd, $J$ = 8.3, 2.6 Hz, 1H), 6.32 (s, 2H), 5.16 – 5.06 (m, 2H), 4.73 (d, $J$ = 17.0 Hz, 2H), 4.21 (s, 2H), 3.56 (d, $J$ = 12.1 Hz, 2H), 3.33 (d, $J$ = 14.1 Hz, 3H), 3.23 – 2.81 (m, 10H), 2.69 – 2.56 (m, 2H), 2.26 (t, $J$ = 13.2 Hz, 2H), 2.15 – 1.96 (m, 3H), 1.91 – 1.40 (m, 8H), 1.18 (s, 4H). | 833.16 | General Synthesis Formula 12 |
| **33** | A37 | 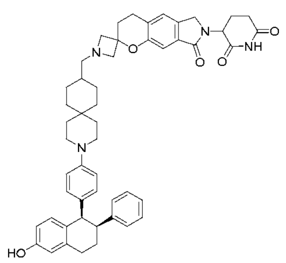<br>3-(1-((3-(4-((1R,2S)-6-hydroxy-2-phenyl-1,2,3,4-tetrahydronaphthalen-1-yl)phenyl)-3-azaspiro[5.5]undecane-9-yl)methyl)-8'-oxo-3',4',6',8'-tetrahydro-7'H-spiro[azetidine-3,2'-pyrano[2,3-f]isoindole]-7'-yl)piperidine-2,6-dione | $^1$H NMR (600 MHz, DMSO-$d_6$) δ 10.99 (s, 1H), 9.15 (s, 1H), 7.38 (d, $J$ = 1.8 Hz, 1H), 7.21 – 7.07 (m, 4H), 6.89 – 6.80 (m, 3H), 6.69 – 6.58 (m, 3H), 6.49 (dd, $J$ = 8.3, 2.6 Hz, 1H), 6.30 (s, 2H), 5.08 (dd, $J$ = 13.3, 5.2 Hz, 1H), 4.50 – 4.13 (m, 8H), 3.35 – 2.83 (m, 11H), 2.67 – 2.57 (m, 1H), 2.43 – 2.33 (m, 1H), 2.28 – 1.93 (m, 5H), 1.80 – 1.37 (m, 9H), 1.14 (td, $J$ = 30.2, 27.1, 19.9 Hz, 4H). | 805.26 | General Synthesis Formula 12 |

| | | | | | |
|---|---|---|---|---|---|
| **34** | A38 | 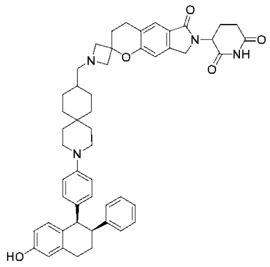 3-(1-((3-(4-((1R,2S)-6-hydroxy-2-phenyl-1,2,3,4-tetrahydronaphthalen-1-yl)phenyl)-3-azaspiro[5.5]undecane-9-yl)methyl)-6'-oxo-3',4',6',8'-tetrahydro-7'H-spiro[azetidine-3,2'-pyrano[2,3-f]isoindole]-7'-yl)piperidine-2,6-dione | $^1$H NMR (600 MHz, DMSO-$d_6$) δ 10.98 (s, 1H), 9.14 (s, 1H), 7.53 (s, 2H), 7.21 – 7.01 (m, 4H), 6.84 (d, $J$ = 7.3 Hz, 2H), 6.74 – 6.45 (m, 4H), 6.26 (s, 2H), 5.07 (ddd, $J$ = 13.2, 5.1, 2.3 Hz, 1H), 4.58 – 4.06 (m, 7H), 3.46 – 2.77 (m, 12H), 2.67 – 2.56 (m, 1H), 2.42 – 1.94 (m, 6H), 1.80 – 1.32 (m, 8H), 1.23 – 0.85 (m, 5H). | 805. 18 | General Synthesis Formula 12 |
| **35** | A39 | 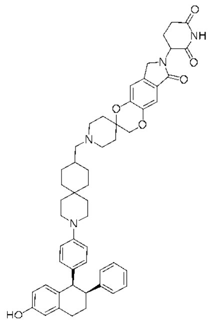 3-(1-((3-(4-((1R,2S)-6-hydroxy-2-phenyl-1,2,3,4-tetrahydronaphthalen-1-yl)phenyl)-3-azaspiro[5.5]undecane-9-yl)methyl)-6'-oxo-6',8'-dihydro-3'H,7'H-spiro[piperidine-4,2'-[1,4]dioxacyclohexa[2,3-f]isoindole]-7'-yl)piperidine-2,6-dione | $^1$H NMR (600 MHz, DMSO-$d_6$) δ 10.98 (d, $J$ = 2.7 Hz, 1H), 9.14 (s, 1H), 7.28 – 7.10 (m, 6H), 6.89 – 6.80 (m, 2H), 6.70 – 6.60 (m, 2H), 6.49 (dd, $J$ = 8.3, 2.5 Hz, 1H), 6.28 (s, 3H), 5.07 (dt, $J$ = 13.3, 5.2 Hz, 2H), 4.37 – 4.09 (m, 6H), 3.51 (d, $J$ = 19.4 Hz, 2H), 3.23 – 2.86 (m, 9H), 2.64 – 2.56 (m, 1H), 2.41 – 2.31 (m, 3H), 2.15 – 1.90 (m, 5H), 1.81 – 1.37 (m, 9H), 1.20 (d, $J$ = 54.9 Hz, 5H). | 835. 17 | General Synthesis Formula 12 |
| **36** | A40 | 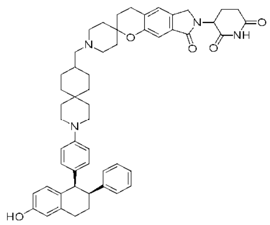 7'-(2,6-Dioxopiperidin-3-yl)-1-((3-(4-((1R,2S)-6-hydroxy-2-phenyl-1,2,3,4-tetrahydronaphthalen-1-yl)phenyl)-3-azaspiro[5.5]undecane-9-yl)methyl)-3',4'-dihydro-6'H-spiro[azetidine-3,2'-pyrano[2,3-f]isoindole]-6',8'(7'H)-dione | $^1$H NMR (600 MHz, DMSO-$d_6$) δ 10.98 (s, 1H), 8.91 (s, 1H), 7.36 (s, 1H), 7.28 – 7.07 (m, 4H), 6.91 – 6.80 (m, 2H), 6.71 – 6.60 (m, 3H), 6.49 (dd, $J$ = 8.3, 2.6 Hz, 1H), 6.31 (s, 3H), 5.07 (dd, $J$ = 13.3, 5.1 Hz, 1H), 4.34 (d, $J$ = 16.8 Hz, 2H), 4.25 – 4.15 (m, 2H), 3.70 – 3.29 (m, 7H), 3.24 – 2.86 (m, 10H), 2.65 – 2.57 (m, 1H), 2.43 – 2.34 (m, 1H), 2.29 – 1.84 (m, 8H), 1.82 – 1.36 (m, 8H), 1.15 (s, 4H). | 833. 17 | General Synthesis Formula 12 |

| | | | | | |
|---|---|---|---|---|---|
| **37** | A41 | 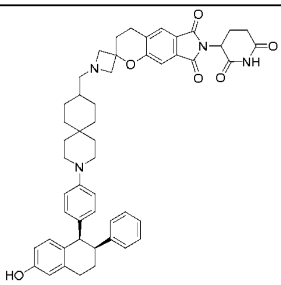 7'-(2,6-Dioxopiperidin-3-yl)-1-((3-(4-((1R,2S)-6-hydroxy-2-phenyl-1,2,3,4-tetrahydronaphthalen-1-yl)phenyl)-3-azaspiro[5.5]undecane-9-yl)methyl)-3',4'-dihydro-6'H-spiro[azetidine-3,2'-pyrano[2,3-f]isoindole]-6',8'(7'H)-dione | $^1$H NMR (600 MHz, DMSO-$d_6$) δ 11.11 (d, $J$ = 4.0 Hz, 1H), 9.14 (s, 1H), 7.78 (s, 1H), 7.34 – 7.09 (m, 4H), 6.92 – 6.81 (m, 2H), 6.71 – 6.59 (m, 3H), 6.49 (dd, $J$ = 8.3, 2.5 Hz, 1H), 6.27 (s, 3H), 5.11 (dd, $J$ = 12.9, 5.6 Hz, 1H), 4.56 – 4.12 (m, 6H), 3.35 – 2.84 (m, 11H), 2.65 – 2.55 (m, 1H), 2.24 (d, $J$ = 6.4 Hz, 2H), 2.14 – 1.91 (m, 4H), 1.77 – 1.34 (m, 9H), 1.13 (dd, $J$ = 35.0, 23.2 Hz, 4H). | 819.14 | General Synthesis Formula 12 |
| **38** | A42 | 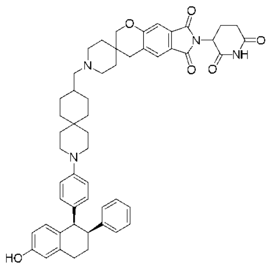 7'-(2,6-Dioxopiperidin-3-yl)-1-((3-(4-((1R,2S)-6-hydroxy-2-phenyl-1,2,3,4-tetrahydronaphthalen-1-yl)phenyl)-3-azaspiro[5.5]undecane-9-yl)methyl)-2'H-spiro[piperidine-4,3'-pyrano[2,3-f]isoindole]-6',8'(4'H,7'H)-dione | $^1$H NMR (600 MHz, DMSO-$d_6$) δ 11.11 (s, 1H), 9.15 (s, 1H), 7.73 (s, 1H), 7.60 (s, 1H), 7.30 (d, $J$ = 4.6 Hz, 1H), 7.19 – 7.07 (m, 3H), 6.88 – 6.81 (m, 2H), 6.67 – 6.60 (m, 2H), 6.49 (dd, $J$ = 8.3, 2.6 Hz, 1H), 6.29 (s, 3H), 5.10 (dd, $J$ = 12.9, 5.5 Hz, 1H), 4.31 (s, 1H), 4.19 (s, 1H), 4.03 (s, 1H), 3.46 – 3.25 (m, 5H), 3.19 – 2.85 (m, 12H), 2.79 (s, 1H), 2.65 – 2.58 (m, 1H), 2.14 – 1.99 (m, 2H), 1.79 – 1.43 (m, 12H), 1.15 (s, 5H). | 847.07 | General Synthesis Formula 12 |
| **39** | A43 | 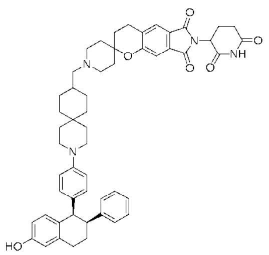 7'-(2,6-Dioxopiperidin-3-yl)-1-((3-(4-((1R,2S)-6-hydroxy-2-phenyl-1,2,3,4-tetrahydronaphthalen-1-yl)phenyl)-3-azaspiro[5.5]undecane-9-yl)methyl)-3',4'-dihydro-6'H-spiro[piperidine-4,2'-pyrano[2,3-f]isoindole]-6',8'(7'H)-dione | $^1$H NMR (600 MHz, DMSO-$d_6$) δ 11.11 (s, 1H), 9.16 (s, 1H), 7.75 (d, $J$ = 3.4 Hz, 1H), 7.39 (s, 2H), 7.22 – 7.09 (m, 3H), 6.88 – 6.80 (m, 2H), 6.66 – 6.58 (m, 2H), 6.49 (dd, $J$ = 8.3, 2.5 Hz, 1H), 6.31 (s, 3H), 5.10 (dd, $J$ = 12.9, 5.4 Hz, 1H), 4.20 (s, 2H), 3.48 – 3.30 (m, 3H), 3.27 – 2.83 (m, 12H), 2.67 – 2.56 (m, 1H), 2.16 – 1.86 (m, 8H), 1.82 – 1.35 (m, 10H), 1.17 (d, $J$ = 14.1 Hz, 5H). | 847.10 | General Synthesis Formula 12 |

| | | | | | |
|---|---|---|---|---|---|
| **40** | A45 | 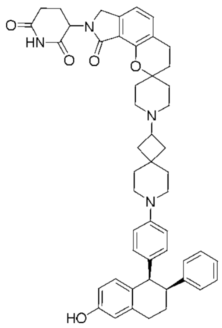 3-(1-(7-(4-((1R,2S)-6-hydroxy-2-phenyl-1,2,3,4-tetrahydronaphthalen-1-yl)phenyl)-7-azaspiro[3.5]nonane-2-yl)-9'-oxo-3',4',7',9'-tetrahydro-8'H-spiro[piperidine-4,2'-pyrano[2,3-e]isoindole]-8'-yl)piperidine-2,6-dione | $^1$H NMR (600 MHz, DMSO-$d_6$) δ 10.99 (s, 1H), 9.13 (s, 1H), 7.35 (d, *J* = 7.6 Hz, 1H), 7.14 (dt, *J* = 13.2, 7.1 Hz, 3H), 7.04 (d, *J* = 7.5 Hz, 2H), 6.83 (d, *J* = 7.3 Hz, 2H), 6.70 – 6.43 (m, 3H), 6.22 (d, *J* = 8.0 Hz, 2H), 4.97 (ddd, *J* = 35.2, 13.2, 5.2 Hz, 1H), 4.40 – 4.06 (m, 3H), 3.56 (q, *J* = 8.3 Hz, 1H), 3.07 – 2.72 (m, 11H), 2.66 – 2.57 (m, 3H), 2.42 – 2.15 (m, 4H), 2.12 – 1.76 (m, 10H), 1.68 (dd, *J* = 42.3, 19.7 Hz, 6H). | 791. 46 | General Synthesis Formula 6 |
| **41** | A46 | 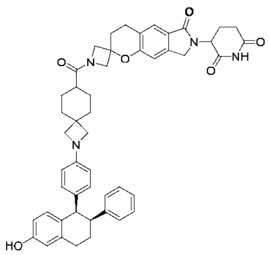 3-(1-(2-(4-((1R,2S)-6-hydroxy-2-phenyl-1,2,3,4-tetrahydronaphthalen-1-yl)phenyl)-2-azaspiro[3.5]nonane-7-carbonyl)-6'-oxo-3',4',6',8'-tetrahydro-7'H-spiro[azetidine-3,2'-pyrano[2,3-f]isoindole]-7'-yl)piperidine-2,6-dione | $^1$H NMR (600 MHz, DMSO-$d_6$) δ 10.98 (s, 1H), 9.05 (s, 1H), 7.50 (s, 1H), 7.19 – 7.09 (m, 3H), 7.05 (d, *J* = 1.5 Hz, 1H), 6.87 – 6.80 (m, 2H), 6.65 – 6.58 (m, 2H), 6.48 (dd, *J* = 8.3, 2.5 Hz, 1H), 6.18 (d, *J* = 8.1 Hz, 2H), 6.10 (d, *J* = 8.1 Hz, 2H), 5.06 (dt, *J* = 13.4, 4.5 Hz, 1H), 4.37 – 4.27 (m, 2H), 4.24 – 4.08 (m, 4H), 3.45 (s, 2H), 3.37 (s, 2H), 3.28 (ddd, *J* = 13.0, 5.1, 2.3 Hz, 1H), 3.05 – 2.85 (m, 5H), 2.63 – 2.56 (m, 2H), 2.42 – 2.31 (m, 1H), 2.25 – 2.06 (m, 4H), 1.97 (dtt, *J* = 7.8, 5.5, 2.3 Hz, 1H), 1.90 – 1.82 (m, 2H), 1.74 – 1.57 (m, 3H), 1.45 (dd, *J* = 14.7, 11.2 Hz, 2H), 1.33 (d, *J* = 14.9 Hz, 2H) | 791. 65 | General Synthesis Formula 20 |
| **42** | A47 | 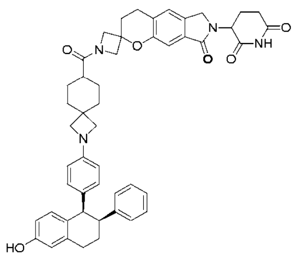 3-(1-(2-(4-((1R,2S)-6-hydroxy-2-phenyl-1,2,3,4-tetrahydronaphthalen-1-yl)phenyl)-2-azaspiro[3.5]nonane-7-carbonyl)-8'-oxo-3',4',6',8'-tetrahydro-7'H-spiro[azetidine-3,2'-pyrano[2,3- | $^1$H NMR (600 MHz, DMSO-$d_6$) δ 10.99 (s, 1H), 9.13 (s, 1H), 7.35 (s, 1H), 7.21 – 7.05 (m, 4H), 6.83 (dd, *J* = 6.9, 1.8 Hz, 2H), 6.66 – 6.55 (m, 2H), 6.48 (dd, *J* = 8.3, 2.5 Hz, 1H), 6.31 – 5.99 (m, 4H), 5.08 (dt, *J* = 13.3, 4.7 Hz, 1H), 4.36 – 4.06 (m, 5H), 3.97 – 3.77 (m, 2H), 3.45 (d, *J* = 55.1 Hz, 5H), 3.28 (ddd, *J* = 12.9, 5.1, 2.3 Hz, 1H), 3.03 – 2.83 (m, 5H), 2.64 – 2.56 (m, 1H), 2.43 – 2.31 (m, 1H), 2.25 – 2.06 (m, 3H), 1.98 (tt, *J* = 7.5, 4.3 Hz, 1H), 1.88 (d, *J* = 12.5 Hz, 2H), 1.70 (dd, *J* = | 791. 65 | General Synthesis Formula 20 |

| | | | | | |
|---|---|---|---|---|---|
| | | f]isoindole]-7'-yl)piperidine-2,6-dione | 11.9, 6.1 Hz, 1H), 1.62 (t, $J$ = 9.2 Hz, 2H), 1.49 – 1.42 (m, 2H), 1.37 – 1.30 (m, 2H) | | |
| **43** | A48 | 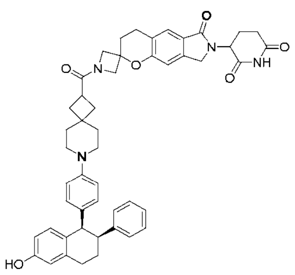 3-(1-(7-(4-((1R,2S)-6-hydroxy-2-phenyl-1,2,3,4-tetrahydronaphthalen-1-yl)phenyl)-7-azaspiro[3.5]nonane-2-carbonyl)-6'-oxo-3',4',6',8'-tetrahydro-7'H-spiro[azetidine-3,2'-pyrano[2,3-f]isoindole]-7'-yl)piperidine-2,6-dione | $^1$H NMR (600 MHz, DMSO-$d_6$) δ 10.98 (s, 1H), 9.19 (s, 1H), 7.50 (s, 1H), 7.19 – 7.10 (m, 3H), 7.05 (d, $J$ = 1.9 Hz, 3H), 6.87 – 6.80 (m, 2H), 6.68 – 6.61 (m, 2H), 6.50 (dd, $J$ = 8.3, 2.5 Hz, 1H), 6.40 (s, 2H), 5.06 (dt, $J$ = 13.3, 5.1 Hz, 1H), 4.34 (dd, $J$ = 17.1, 3.8 Hz, 1H), 4.22 (dt, $J$ = 17.0, 8.9 Hz, 2H), 4.15 (d, $J$ = 9.3 Hz, 1H), 4.06 (d, $J$ = 9.3 Hz, 1H), 3.99 (d, $J$ = 10.3 Hz, 1H), 3.85 (d, $J$ = 10.3 Hz, 1H), 3.35 (d, $J$ = 12.9 Hz, 1H), 3.23 – 3.08 (m, 5H), 3.02 – 2.86 (m, 5H), 2.64 – 2.56 (m, 1H), 2.36 (qt, $J$ = 13.1, 3.4 Hz, 1H), 2.16 – 1.92 (m, 7H), 1.87 – 1.63 (m, 6H). | 791.60 | General Synthesis Formula 20 |
| **44** | A49 | 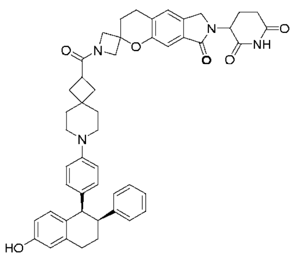 3-(1-(7-(4-((1R,2S)-6-hydroxy-2-phenyl-1,2,3,4-tetrahydronaphthalen-1-yl)phenyl)-7-azaspiro[3.5]nonane-2-carbonyl)-8'-oxo-3',4',6',8'-tetrahydro-7'H-spiro[azetidine-3,2'-pyrano[2,3-f]isoindole]-7'-yl)piperidine-2,6-dione | $^1$H NMR (600 MHz, DMSO-$d_6$) δ 10.99 (s, 1H), 9.18 (s, 1H), 7.35 (s, 1H), 7.24 – 6.94 (m, 4H), 6.87 – 6.76 (m, 3H), 6.72 – 6.59 (m, 3H), 6.57 – 6.26 (m, 3H), 5.08 (dt, $J$ = 13.3, 5.3 Hz, 1H), 4.34 (dd, $J$ = 16.9, 2.2 Hz, 1H), 4.28 – 4.18 (m, 2H), 4.14 (d, $J$ = 9.3 Hz, 1H), 4.06 (d, $J$ = 9.3 Hz, 1H), 3.97 (d, $J$ = 10.3 Hz, 1H), 3.85 (d, $J$ = 10.3 Hz, 1H), 3.35 (d, $J$ = 12.6 Hz, 1H), 3.17 (s, 5H), 3.11 (p, $J$ = 8.6 Hz, 1H), 3.03 – 2.84 (m, 5H), 2.63 – 2.56 (m, 1H), 2.53 – 2.52 (m, 1H), 2.43 – 2.32 (m, 1H), 2.18 – 1.91 (m, 6H), 1.86 – 1.59 (m, 5H). | 791.60 | General Synthesis Formula 20 |
| **45** | A50 | 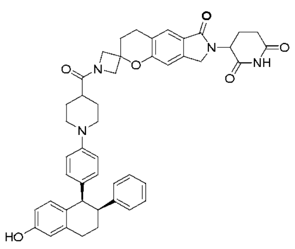 3-(1-(1-(4-((1R,2S)-6-hydroxy-2-phenyl-1,2,3,4-tetrahydronaphthalen-1-yl)phenyl)piperidin-4-carbonyl)-6'-oxo-3',4',6',8'-tetrahydro-7'H-spiro[azetidine-3,2'-pyrano[2,3-f]isoindole]-7'-yl)piperidine-2,6-dione | $^1$H NMR (600 MHz, DMSO-$d_6$) δ 10.98 (s, 1H), 9.18 (s, 1H), 7.51 (s, 1H), 7.33 (d, $J$ = 56.2 Hz, 1H), 7.19 – 7.10 (m, 3H), 7.05 (d, $J$ = 2.0 Hz, 1H), 6.95 (d, $J$ = 44.1 Hz, 1H), 6.82 (dd, $J$ = 6.8, 1.9 Hz, 2H), 6.69 – 6.60 (m, 2H), 6.50 (dt, $J$ = 8.4, 2.0 Hz, 1H), 6.38 (s, 2H), 5.06 (dt, $J$ = 13.3, 5.2 Hz, 1H), 4.38 – 4.30 (m, 2H), 4.28 – 4.17 (m, 3H), 4.00 (d, $J$ = 10.5 Hz, 1H), 3.87 (d, $J$ = 10.4 Hz, 1H), 3.53 (d, $J$ = 10.6 Hz, 2H), 3.35 (d, $J$ = 12.5 Hz, 1H), 3.02 – 2.85 (m, 6H), 2.64 – 2.56 (m, 3H), 2.37 (qq, $J$ = 13.1, 4.5 Hz, 1H), 2.11 (dtt, $J$ = | 751.55 | General Synthesis Formula 14 |

| | | | | | |
|---|---|---|---|---|---|
| | | | 44.4, 12.7, 6.6 Hz, 3H), 2.00 – 1.70 (m, 6H). | | |
| **46** | A51 | 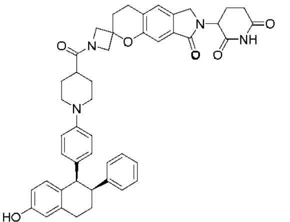 3-(1-(7-(4-((1R,2S)-6-hydroxy-2-phenyl-1,2,3,4-tetrahydronaphthalen-1-yl)phenyl)-7-azaspiro[3.5]nonane-2-carbonyl)-8'-oxo-3',4',6',8'-tetrahydro-7'H-spiro[azetidine-3,2'-pyrano[2,3-f]isoindole]-7'-yl)piperidine-2,6-dione | $^1$H NMR (600 MHz, DMSO-$d_6$) δ 10.99 (s, 1H), 9.18 (s, 1H), 7.36 (s, 1H), 7.20 – 7.08 (m, 4H), 7.04 – 6.79 (m, 4H), 6.71 – 6.61 (m, 2H), 6.54 – 6.30 (m, 3H), 5.08 (dt, *J* = 13.2, 5.4 Hz, 1H), 4.38 – 4.16 (m, 6H), 3.99 (d, *J* = 10.4 Hz, 1H), 3.86 (d, *J* = 10.4 Hz, 1H), 3.60 – 3.48 (m, 2H), 3.42 – 3.28 (m, 1H), 3.09 – 2.85 (m, 6H), 2.59 (dt, *J* = 17.4, 3.7 Hz, 1H), 2.38 (qt, *J* = 13.0, 4.6 Hz, 1H), 2.21 – 2.04 (m, 3H), 2.01 – 1.69 (m, 7H). | 751. 55 | General Synthesis Formula 14 |
| **47** | A52 | 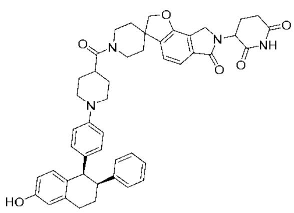 3-(1-(1-(4-((1R,2S)-6-hydroxy-2-phenyl-1,2,3,4-tetrahydronaphthalen-1-yl)phenyl)piperidin-4-carbonyl)-6'-oxo-3',4',6',8'-tetrahydro-7'H-spiro[azetidine-3,2'-pyrano[2,3-f]isoindole]-7'-yl)piperidine-2,6-dione | $^1$H NMR (600 MHz, DMSO-$d_6$) δ 10.99 (s, 1H), 9.19 (s, 1H), 7.43 (d, *J* = 7.5 Hz, 1H), 7.28 (d, *J* = 7.5 Hz, 1H), 7.20 – 7.11 (m, 3H), 6.99 (d, *J* = 30.2 Hz, 1H), 6.90 – 6.80 (m, 4H), 6.69 – 6.62 (m, 2H), 6.55 – 6.36 (m, 3H), 5.10 (dt, *J* = 12.2, 5.6 Hz, 1H), 4.71 – 4.58 (m, 2H), 4.40 (dd, *J* = 17.1, 6.5 Hz, 2H), 4.30 – 4.20 (m, 2H), 4.00 (d, *J* = 13.5 Hz, 1H), 3.56 (d, *J* = 11.0 Hz, 2H), 3.36 (d, *J* = 13.1 Hz, 1H), 3.23 (t, *J* = 12.7 Hz, 1H), 2.95 (dddd, *J* = 41.1, 17.8, 11.5, 5.2 Hz, 3H), 2.79 – 2.66 (m, 2H), 2.65 – 2.57 (m, 1H), 2.47 – 2.36 (m, 2H), 2.16 – 1.60 (m, 11H). | 765. 65 | General Synthesis Formula 14 |
| **48** | A53 | 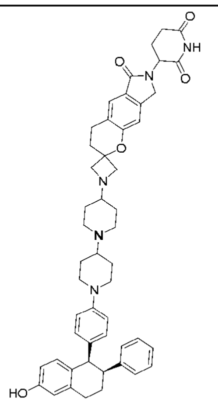 3-(1-(1'-(4-((1R,2S)-6-hydroxy-2-phenyl-1,2,3,4-tetrahydronaphthalen-1-yl)phenyl)-[1,4'-bipiperidin]-4-yl)-6'-oxo-3',4',6',8'-tetrahydro-7'H- | $^1$H NMR (600 MHz, DMSO-$d_6$) δ 10.98 (s, 1H), 9.13 (s, 1H), 7.55 (s, 1H), 7.19 – 7.05 (m, 4H), 6.89 – 6.79 (m, 2H), 6.69 – 6.57 (m, 4H), 6.49 (dd, *J* = 8.3, 2.6 Hz, 1H), 6.23 (d, *J* = 8.4 Hz, 2H), 5.07 (dd, *J* = 13.3, 5.1 Hz, 1H), 4.51 – 4.32 (m, 5H), 4.24 (d, *J* = 17.1 Hz, 1H), 4.15 (d, *J* = 5.0 Hz, 1H), 3.70 (s, 6H), 3.35 – 3.18 (m, 3H), 3.05 – 2.79 (m, 2H), 2.66 – 2.52 (m, 5H), 2.44 – 2.29 (m, 3H), 2.23 – 1.92 (m, 4H), 1.65 (d, *J* = 13.4 Hz, 4H), 1.35 – 1.12 (m, 5H). | 806. 65 | General Synthesis Formula 4 |

| | | | | | |
|---|---|---|---|---|---|
| | | spiro[azetidine-3,2'-pyrano[2,3-f]isoindole]-7'-yl)piperidine-2,6-dione | | | |
| **49** | A54 | 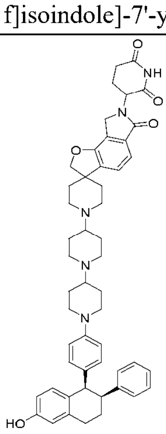 3-(1'-(1'-(4-((1R,2S)-6-hydroxy-2-phenyl-1,2,3,4-tetrahydronaphthalen-1-yl)phenyl)-[1,4'-bipiperidin]-4-yl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 11.00 (s, 1H), 9.49 (s, 1H), 7.36 (d, $J$ = 7.6 Hz, 1H), 7.29 (d, $J$ = 7.6 Hz, 1H), 7.20 – 7.09 (m, 3H), 6.89 – 6.81 (m, 2H), 6.68 – 6.57 (m, 4H), 6.49 (dd, $J$ = 8.2, 2.6 Hz, 1H), 6.24 (d, $J$ = 8.3 Hz, 2H), 5.10 (dd, $J$ = 13.3, 5.1 Hz, 1H), 4.69 (q, $J$ = 9.4 Hz, 2H), 4.40 (d, $J$ = 17.2 Hz, 1H), 4.24 (d, $J$ = 17.2 Hz, 1H), 4.16 (d, $J$ = 5.0 Hz, 1H), 3.69 (d, $J$ = 24.4 Hz, 6H), 3.39 – 3.22 (m, 3H), 3.18 – 2.85 (m, 7H), 2.67 – 2.55 (m, 3H), 2.47 – 2.27 (m, 4H), 2.24 – 1.86 (m, 9H), 1.69 (dd, $J$ = 29.8, 10.6 Hz, 3H). | 820.65 | General Synthesis Formula 4 |
| **50** | A55 | 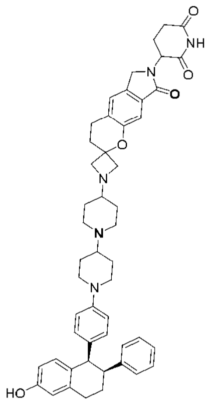 3-(1-(1'-(4-((1R,2S)-6-hydroxy-2-phenyl-1,2,3,4-tetrahydronaphthalen-1-yl)phenyl)-[1,4'-bipiperidin]-4-yl)-8'-oxo-3',4',6',8'-tetrahydro-7'H-spiro[azetidine-3,2'-pyrano[2,3-f]isoindole]-7'-yl)piperidine-2,6-dione | $^1$H NMR (600 MHz, DMSO-$d_6$) δ 10.99 (s, 1H), 7.39 (s, 1H), 7.18 – 7.06 (m, 4H), 6.89 – 6.81 (m, 2H), 6.66 – 6.56 (m, 4H), 6.49 (dd, $J$ = 8.3, 2.5 Hz, 1H), 6.23 (d, $J$ = 8.4 Hz, 2H), 5.08 (dd, $J$ = 13.3, 5.1 Hz, 1H), 4.51 – 4.31 (m, 5H), 4.26 – 4.09 (m, 2H), 3.71 (d, $J$ = 13.0 Hz, 3H), 3.38 – 3.24 (m, 2H), 3.12 – 2.79 (m, 8H), 2.64 – 2.54 (m, 3H), 2.39 (tt, $J$ = 13.3, 6.5 Hz, 1H), 2.28 – 1.94 (m, 8H), 1.76 – 1.57 (m, 5H). | 806.65 | General Synthesis Formula 4 |

| | | | | | |
|---|---|---|---|---|---|
| **51** | A56 | 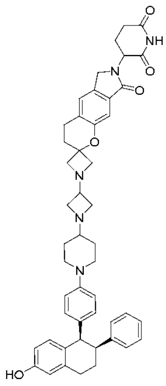<br>3-(1-(1-(1-(4-((1R,2S)-6-hydroxy-2-phenyl-1,2,3,4-tetrahydronaphthalen-1-yl)phenyl)piperidin-4-yl)azetidin-3-yl)-6'-oxo-3',4',6',8'-tetrahydro-7'H-spiro[azetidine-3,2'-pyrano[2,3-f]isoindole]-7'-yl)piperidine-2,6-dione | $^1$H NMR (600 MHz, DMSO-$d_6$) δ 10.98 (s, 1H), 9.12 (s, 1H), 7.35 (s, 1H), 7.24 – 7.04 (m, 4H), 6.93 – 6.77 (m, 2H), 6.72 – 6.56 (m, 4H), 6.49 (dd, $J$ = 8.3, 2.5 Hz, 1H), 6.23 (d, $J$ = 8.5 Hz, 2H), 5.07 (dd, $J$ = 13.3, 5.2 Hz, 1H), 4.43 – 3.97 (m, 5H), 3.93 – 3.37 (m, 7H), 3.34 – 3.20 (m, 2H), 3.05 – 2.76 (m, 5H), 2.66 – 2.51 (m, 6H), 2.38 (qd, $J$ = 13.2, 4.6 Hz, 1H), 2.21 – 1.58 (m, 8H), 1.36 (s, 2H). | 778.60 | General Synthesis Formula 4 |
| **52** | A57 | 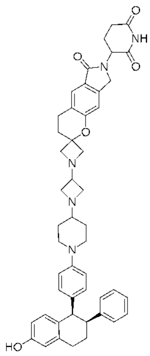<br>3-(1-(1-(1-(4-((1R,2S)-6-hydroxy-2-phenyl-1,2,3,4-tetrahydronaphthalen-1-yl)phenyl)piperidin-4-yl)azetidin-3-yl)-6'-oxo-3',4',6',8'-tetrahydro-7'H-spiro[azetidine-3,2'-pyrano[2,3-f]isoindole]-7'-yl)piperidine-2,6-dione | $^1$H NMR (600 MHz, DMSO-$d_6$) δ 10.96 (s, 1H), 9.13 (s, 1H), 7.50 (s, 1H), 7.26 – 6.95 (m, 4H), 6.91 – 6.76 (m, 2H), 6.70 – 6.41 (m, 5H), 6.23 (d, $J$ = 8.2 Hz, 2H), 5.06 (dd, $J$ = 13.3, 5.1 Hz, 1H), 4.44 – 3.40 (m, 7H), 3.30 (ddd, $J$ = 13.1, 5.1, 2.3 Hz, 1H), 3.04 – 2.77 (m, 5H), 2.66 – 2.46 (m, 11H), 2.36 (qd, $J$ = 13.1, 4.4 Hz, 1H), 2.24 – 1.61 (m, 8H), 1.30 (d, $J$ = 74.8 Hz, 2H). | 778.60 | General Synthesis Formula 4 |
| **53** | A58 | 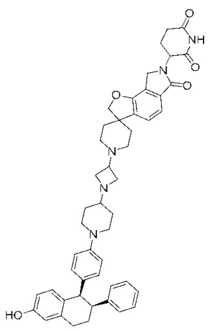<br>3-(1-(2-(4-((1R,2S)-6-hydroxy-2-phenyl-1,2,3,4-tetrahydronaphthalen-1- | $^1$H NMR (600 MHz, DMSO-$d_6$) δ 10.99 (s, 1H), 9.13 (s, 1H), 7.54 – 7.26 (m, 2H), 7.21 – 7.08 (m, 3H), 6.96 – 6.80 (m, 2H), 6.72 – 6.56 (m, 4H), 6.49 (dd, $J$ = 8.3, 2.5 Hz, 1H), 6.24 (d, $J$ = 8.4 Hz, 2H), 5.10 (dd, $J$ = 13.3, 5.1 Hz, 1H), 4.60 (q, $J$ = 9.3 Hz, 2H), 4.47 – 4.08 (m, 7H), 4.02 – 3.54 (m, 3H), 3.45 – 3.06 (m, 4H), 3.04 – 2.80 (m, 3H), 2.70 – 2.26 (m, 11H), 2.15 – 1.58 (m, 6H), 1.40 (d, $J$ = 13.1 Hz, 2H). | 792.65 | General Synthesis Formula 4 |

| | | | | | |
|---|---|---|---|---|---|
| | | yl)phenyl)-2-azaspiro[3.5]nonane-7-carbonyl)-8'-oxo-3',4',6',8'-tetrahydro-7'H-spiro[azetidine-3,2'-pyrano[2,3-f]isoindole]-7'-yl)piperidine-2,6-dione | | | |
| **54** | A59 | 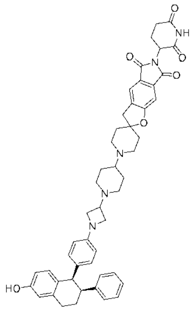 6-(2,6-Dioxopiperidin-3-yl)-1'-(1-(1-(4-((1R,2S)-6-hydroxy-2-phenyl-1,2,3,4-tetrahydronaphthalen-1-yl)phenyl)azetidin-3-yl)piperidin-4-yl)spiro[furo[2,3-f]isoindole-2,4'-piperidine]-5,7(3H,6H)-dione | $^1$H NMR (600 MHz, DMSO-$d_6$) δ 11.12 (s, 1H), 9.15 (s, 1H), 7.82 (s, 1H), 7.38 – 7.05 (m, 4H), 6.95 – 6.76 (m, 2H), 6.62 (d, $J$ = 8.5 Hz, 2H), 6.48 (dd, $J$ = 8.2, 2.6 Hz, 1H), 6.18 (dd, $J$ = 62.7, 8.1 Hz, 4H), 5.10 (dd, $J$ = 12.9, 5.4 Hz, 1H), 4.26 – 3.88 (m, 3H), 3.80 (s, 15H), 3.39 – 3.13 (m, 5H), 3.04 – 2.80 (m, 3H), 2.73 – 2.54 (m, 1H), 2.43 – 1.99 (m, 4H), 1.93 – 1.47 (m, 3H). | 806.60 | General Synthesis Formula 3 |
| **55** | A60 | 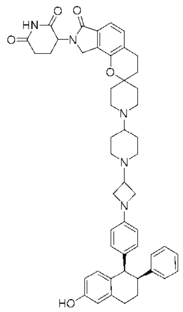 3-(1'-(1-(1-(4-((1R,2S)-6-hydroxy-2-phenyl-1,2,3,4-tetrahydronaphthalen-1-yl)phenyl)azetidin-3-yl)piperidin-4-yl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 11.04 (s, 1H), 9.14 (s, 1H), 7.44 – 7.06 (m, 5H), 6.91 – 6.81 (m, 2H), 6.69 – 6.45 (m, 3H), 6.18 (dd, $J$ = 64.4, 8.0 Hz, 4H), 5.14 (dd, $J$ = 13.4, 5.2 Hz, 1H), 4.48 – 3.91 (m, 5H), 3.81 (s, 12H), 3.32 – 2.57 (m, 9H), 2.45 – 2.22 (m, 2H), 2.14 – 1.57 (m, 8H), 1.48 – 1.16 (m, 1H). | 806.65 | General Synthesis Formula 3 |

| | | | | | |
|---|---|---|---|---|---|
| **56** | A61 | 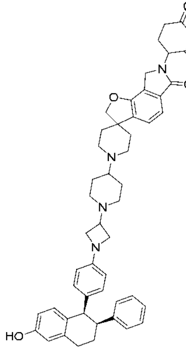<br>3-(1'-(1-(1-(4-((1R,2S)-6-hydroxy-2-phenyl-1,2,3,4-tetrahydronaphthalen-1-yl)phenyl)azetidin-3-yl)piperidin-4-yl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 11.00 (d, $J$ = 4.9 Hz, 1H), 9.14 (s, 1H), 7.33 (dd, $J$ = 39.0, 7.3 Hz, 2H), 7.14 (dt, $J$ = 12.2, 7.2 Hz, 3H), 6.84 (d, $J$ = 7.3 Hz, 2H), 6.68 – 6.58 (m, 2H), 6.48 (dd, $J$ = 8.2, 2.6 Hz, 1H), 6.30 – 6.07 (m, 4H), 5.10 (dd, $J$ = 13.3, 5.3 Hz, 1H), 4.74 – 4.61 (m, 1H), 4.46 – 4.09 (m, 3H), 4.05 – 3.80 (m, 3H), 3.47 (s, 17H), 3.21 – 2.70 (m, 4H), 2.47 – 2.29 (m, 1H), 2.23 – 1.63 (m, 6H), 1.58 – 1.07 (m, 1H). | 792.60 | General Synthesis Formula 3 |
| **57** | A62 | 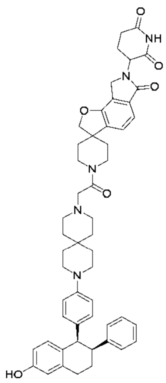<br>3-(1'-(2-(9-(4-((1R,2S)-6-hydroxy-2-phenyl-1,2,3,4-tetrahydronaphthalen-1-yl)phenyl)-3,9-diazaspiro[5.5]undecane-3-yl)acetyl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.99 (d, $J$ = 1.9 Hz, 1H), 9.42 (s, 1H), 7.43 – 7.30 (m, 2H), 7.20 – 7.09 (m, 3H), 6.90 – 6.59 (m, 6H), 6.49 (dd, $J$ = 8.3, 2.6 Hz, 1H), 6.30 (s, 2H), 5.10 (dt, $J$ = 13.3, 5.6 Hz, 1H), 4.74 – 4.62 (m, 2H), 4.49 – 3.98 (m, 6H), 3.77 – 3.56 (m, 1H), 3.42 – 2.77 (m, 13H), 2.47 – 2.33 (m, 1H), 2.17 – 1.36 (m, 15H). | 848.60 | General Synthesis Formula 10 |
| **58** | A63 | 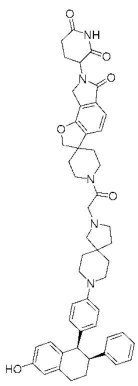 | $^1$H NMR (600 MHz, DMSO-$d_6$) δ 10.99 (d, $J$ = 5.1 Hz, 1H), 10.11 (s, 1H), 7.38 – 7.28 (m, 2H), 7.18 – 7.10 (m, 3H), 6.89 – 6.81 (m, 2H), 6.75 – 6.57 (m, 4H), 6.49 (dd, $J$ = 8.2, 2.6 Hz, 1H), 6.27 (d, $J$ = 8.1 Hz, 2H), 5.09 (dd, $J$ = 13.3, 5.4 Hz, 1H), 4.76 – 4.63 (m, 2H), 4.56 – 4.14 (m, 5H), 3.73 – 3.52 (m, 3H), 3.36 – 3.19 (m, 3H), 3.11 – 2.84 (m, 11H), 2.69 – 2.57 (m, 1H), 2.48 – 2.33 (m, 1H), 2.17 – 1.87 (m, 4H), | 834.60 | General Synthesis Formula 10 |

| | | | | | |
|---|---|---|---|---|---|
| | | 3-(1'-(2-(8-(4-((1R,2S)-6-hydroxy-2-phenyl-1,2,3,4-tetrahydronaphthalen-1-yl)phenyl)-2,8-diazaspiro[4.5]decane-2-yl)acetyl)-6-oxo-6,8-dihydro-2H,7H-spiro[furo[2,3-e]isoindole-3,4'-piperidine]-7-yl)piperidine-2,6-dione | 1.83 – 1.57 (m, 8H). | | |
| **59** | A64 | 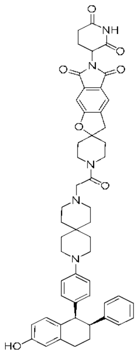 6-(2,6-Dioxopiperidin-3-yl)-1'-(2-(9-(4-((1R,2S)-6-hydroxy-2-phenyl-1,2,3,4-tetrahydronaphthalen-1-yl)phenyl)-3,9-diazaspiro[5.5]undecane-3-yl)acetyl)spiro[furo[2,3-f]isoindole-2,4'-piperidine]-5,7(3H,6H)-dione | $^1$H NMR (600 MHz, DMSO-$d_6$) δ 11.11 (s, 1H), 9.40 (s, 1H), 7.80 (s, 1H), 7.26 (s, 1H), 7.20 – 7.08 (m, 3H), 6.89 – 6.58 (m, 6H), 6.49 (dd, $J$ = 8.3, 2.5 Hz, 1H), 6.29 (s, 2H), 5.10 (dd, $J$ = 12.9, 5.4 Hz, 1H), 4.34 (t, $J$ = 4.3 Hz, 2H), 4.18 (d, $J$ = 4.9 Hz, 1H), 3.84 (dt, $J$ = 13.3, 5.0 Hz, 1H), 3.57 – 3.43 (m, 3H), 3.25 (s, 2H), 3.17 – 2.83 (m, 8H), 2.64 – 2.55 (m, 1H), 2.15 – 1.63 (m, 16H), 1.52 (s, 2H), 1.30 – 1.14 (m, 1H). | 862.60 | General Synthesis Formula 10 |
| **60** | A65 | 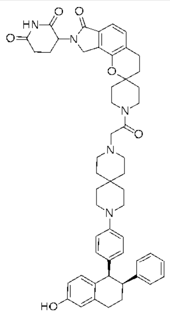 3-(1-(2-(9-(4-((1R,2S)-6-hydroxy-2-phenyl-1,2,3,4-tetrahydronaphthalen-1-yl)phenyl)-3,9-diazaspiro[5.5]undecane-3-yl)acetyl)-7'-oxo-3',4',7',9'-tetrahydro-8'H-spiro[piperidine-4,2'-pyrano[2,3-e]isoindole]-8'-yl)piperidine-2,6-dione | $^1$H NMR (600 MHz, DMSO-$d_6$) δ 11.00 (d, $J$ = 4.5 Hz, 1H), 9.39 (s, 1H), 7.32 – 7.12 (m, 5H), 6.84 (t, $J$ = 3.5 Hz, 2H), 6.75 – 6.62 (m, 4H), 6.49 (dd, $J$ = 8.3, 2.5 Hz, 1H), 6.29 (s, 2H), 5.12 (ddd, $J$ = 13.3, 8.2, 5.1 Hz, 1H), 4.43 – 4.15 (m, 6H), 3.56 – 3.27 (m, 6H), 3.21 – 2.85 (m, 13H), 2.66 – 2.57 (m, 1H), 2.44 – 2.32 (m, 1H), 2.15 – 1.96 (m, 2H), 1.92 – 1.35 (m, 11H), 1.23 (d, $J$ = 6.5 Hz, 1H). | 862.65 | General Synthesis Formula 10 |

| | | | | | |
|---|---|---|---|---|---|
| **61** | A66 | 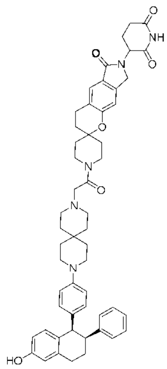 3-(1-(2-(9-(4-((1R,2S)-6-hydroxy-2-phenyl-1,2,3,4-tetrahydronaphthalen-1-yl)phenyl)-3,9-diazaspiro[5.5]undecane-3-yl)acetyl)-6'-oxo-3',4',6',8'-tetrahydro-7'H-spiro[piperidine-4,2'-pyrano[2,3-f]isoindole]-7'-yl)piperidine-2,6-dione | $^1$H NMR (400 MHz, DMSO-$d_6$) δ 10.97 (s, 1H), 9.37 (s, 1H), 7.51 (s, 1H), 7.15 (d, *J* = 7.3 Hz, 3H), 7.01 (s, 1H), 6.88 – 6.79 (m, 2H), 6.75 – 6.59 (m, 4H), 6.52 – 6.47 (m, 1H), 6.29 (s, 2H), 5.06 (ddd, *J* = 13.4, 5.1, 2.6 Hz, 1H), 4.41 – 4.06 (m, 10H), 3.46 (dd, *J* = 33.8, 12.4 Hz, 2H), 3.18 – 2.85 (m, 11H), 2.69 – 2.56 (m, 1H), 2.43 – 2.32 (m, 1H), 2.09 (dd, *J* = 12.3, 6.5 Hz, 2H), 2.02 – 1.94 (m, 1H), 1.90 – 1.64 (m, 10H), 1.54 (d, *J* = 26.7 Hz, 2H), 1.31 – 1.07 (m, 2H). | 862.53 | General Synthesis Formula 10 |
| **62** | A67 | 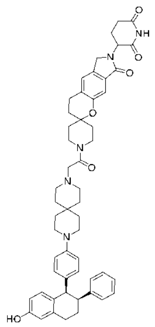 3-(1-(2-(9-(4-((1R,2S)-6-hydroxy-2-phenyl-1,2,3,4-tetrahydronaphthalen-1-yl)phenyl)-3,9-diazaspiro[5.5]undecane-3-yl)acetyl)-8'-oxo-3',4',6',8'-tetrahydro-7'H-spiro[piperidine-4,2'-pyrano[2,3-f]isoindole]-7'-yl)piperidine-2,6-dione | $^1$H NMR (400 MHz, DMSO-$d_6$) δ 10.98 (s, 1H), 9.37 (s, 1H), 7.35 (s, 1H), 7.16 – 7.11 (m, 4H), 6.89 – 6.61 (m, 6H), 6.49 (dd, *J* = 8.2, 2.5 Hz, 1H), 6.30 (s, 2H), 5.07 (ddd, *J* = 13.3, 5.2, 2.6 Hz, 1H), 4.48 – 3.99 (m, 11H), 3.58 – 3.28 (m, 5H), 3.02 – 2.84 (m, 5H), 2.74 – 2.58 (m, 1H), 2.43 – 2.34 (m, 1H), 2.04 (ddd, *J* = 38.6, 11.5, 6.1 Hz, 2H), 1.90 – 1.40 (m, 17H), 1.29 – 1.10 (m, 1H). | 862.65 | General Synthesis Formula 10 |

| | | | | | |
|---|---|---|---|---|---|
| **63** | A68 | 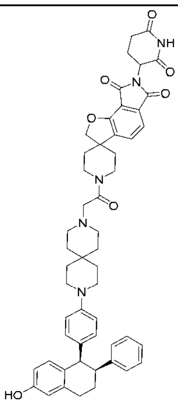 7-(2,6-Dioxopiperidin-3-yl)-1'-(2-(9-(4-((1R,2S)-6-hydroxy-2-phenyl-1,2,3,4-tetrahydronaphthalen-1-yl)phenyl)-3,9-diazaspiro[5.5]undecane-3-yl)acetyl)-7-hydro-2H,6H-spiro[furo[2,3-e]isoindole-3,4'-piperidine]-6,8-dione | $^1$H NMR (400 MHz, DMSO-$d_6$) δ 11.11 (s, 1H), 9.42 (s, 1H), 7.69 (d, $J$ = 7.4 Hz, 1H), 7.46 (d, $J$ = 7.3 Hz, 1H), 7.15 (q, $J$ = 7.6, 6.9 Hz, 4H), 6.91 – 6.78 (m, 3H), 6.74 – 6.57 (m, 3H), 6.49 (dd, $J$ = 8.3, 2.5 Hz, 1H), 6.27 (d, $J$ = 8.0 Hz, 2H), 5.10 (dd, $J$ = 12.7, 5.4 Hz, 1H), 4.91 – 4.76 (m, 2H), 4.35 (td, $J$ = 22.2, 21.7, 13.3 Hz, 4H), 4.17 (d, $J$ = 4.9 Hz, 1H), 3.29 (d, $J$ = 36.7 Hz, 4H), 3.15 – 2.81 (m, 10H), 2.70 – 2.55 (m, 1H), 2.20 – 1.99 (m, 1H), 1.93 – 1.63 (m, 12H), 1.51 (dd, $J$ = 8.8, 4.8 Hz, 2H), 1.28 – 1.04 (m, 1H). | 862.56 | General Synthesis Formula 10 |
| **64** | A69 | 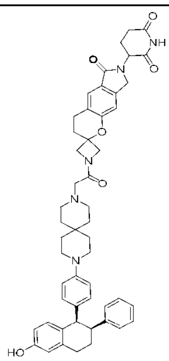 3-(1-(2-(9-(4-((1R,2S)-6-hydroxy-2-phenyl-1,2,3,4-tetrahydronaphthalen-1-yl)phenyl)-3,9-diazaspiro[5.5]undecane-3-yl)acetyl)-6'-oxo-3',4',6',8'-tetrahydro-7'H-spiro[azetidine-3,2'-pyrano[2,3-f]isoindole]-7'-yl)piperidine-2,6-dione | $^1$H NMR (600 MHz, DMSO-$d_6$) δ 10.97 (d, $J$ = 1.9 Hz, 1H), 9.62 (s, 1H), 7.53 (s, 1H), 7.18 – 7.10 (m, 3H), 7.06 (d, $J$ = 1.6 Hz, 1H), 6.88 – 6.81 (m, 2H), 6.73 – 6.60 (m, 4H), 6.49 (dd, $J$ = 8.3, 2.5 Hz, 1H), 6.27 (s, 2H), 5.07 (dt, $J$ = 13.3, 4.4 Hz, 1H), 4.41 – 3.97 (m, 10H), 3.31 (d, $J$ = 11.0 Hz, 3H), 3.19 – 2.83 (m, 10H), 2.66 – 2.56 (m, 1H), 2.37 (dtt, $J$ = 17.6, 13.2, 3.9 Hz, 1H), 2.24 – 2.04 (m, 3H), 1.98 (dtt, $J$ = 7.7, 5.4, 2.4 Hz, 1H), 1.87 – 1.61 (m, 5H), 1.54 – 1.43 (m, 2H), 1.31 – 1.22 (m, 1H). | 834.60 | General Synthesis Formula 10 |

| | | | | | |
|---|---|---|---|---|---|
| **65** | A70 | 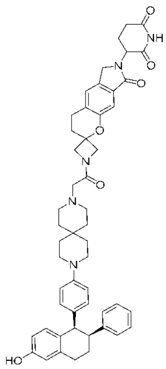 3-(1-(2-(9-(4-((1R,2S)-6-hydroxy-2-phenyl-1,2,3,4-tetrahydronaphthalen-1-yl)phenyl)-3,9-diazaspiro[5.5]undecane-3-yl)acetyl)-8'-oxo-3',4',6',8'-tetrahydro-7'H-spiro[azetidine-3,2'-pyrano[2,3-f]isoindole]-7'-yl)piperidine-2,6-dione | $^1$H NMR (600 MHz, DMSO-$d_6$) δ 10.98 (s, 1H), 9.62 (s, 1H), 7.38 (s, 1H), 7.19 – 7.10 (m, 4H), 6.90 – 6.82 (m, 2H), 6.73 – 6.61 (m, 4H), 6.49 (dd, $J$ = 8.3, 2.5 Hz, 1H), 6.33 – 6.23 (m, 2H), 5.08 (dt, $J$ = 13.3, 4.4 Hz, 1H), 4.40 – 3.94 (m, 10H), 3.32 (d, $J$ = 12.8 Hz, 4H), 3.18 – 2.87 (m, 6H), 2.66 – 2.57 (m, 1H), 2.43 – 2.31 (m, 1H), 2.21 – 2.06 (m, 3H), 2.02 – 1.94 (m, 1H), 1.81 (s, 2H), 1.75 – 1.68 (m, 4H), 1.64 (d, $J$ = 13.7 Hz, 3H), 1.55 – 1.42 (m, 2H). | 834.60 | General Synthesis Formula 10 |
| **66** | A71 | 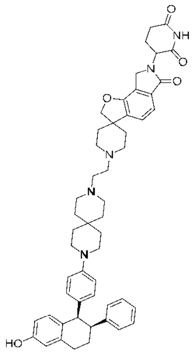 3-(1'-(2-(9-(4-((1R,2S)-6-hydroxy-2-phenyl-1,2,3,4-tetrahydronaphthalen-1-yl)phenyl)-3,9-diazaspiro[5.5]undecane-3-yl)ethyl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.99 (s, 1H), 9.13 (s, 1H), 7.41 – 7.30 (m, 2H), 7.16 – 7.14 (m, 2H), 6.87 – 6.82 (m, 3H), 6.66 – 6.61 (m, 3H), 6.49 (dt, $J$ = 8.4, 2.2 Hz, 2H), 6.27 (s, 2H), 5.10 (dd, $J$ = 13.3, 5.2 Hz, 1H), 4.66 (s, 2H), 4.40 (d, $J$ = 17.1 Hz, 1H), 4.28 – 4.14 (m, 2H), 3.70 – 3.45 (m, 2H), 3.35 – 3.26 (m, 2H), 3.19 – 2.86 (m, 15H), 2.68 – 2.57 (m, 1H), 2.48 – 2.39 (m, 1H), 2.28 – 1.93 (m, 5H), 1.85 – 1.43 (m, 14H), 1.28 – 1.14 (m, 1H). | 834.70 | General Synthesis Formula 10 |
| **67** | A72 | 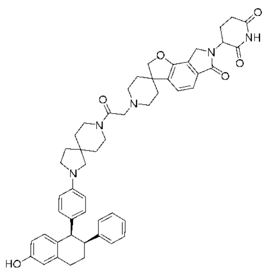 3-(1'-(2-(2-(4-((1R,2S)-6-hydroxy-2-phenyl-1,2,3,4-tetrahydronaphthalen-1- | $^1$H NMR (600 MHz, DMSO-$d_6$) δ 10.99 (s, 1H), 9.66 (s, 1H), 7.40 – 7.29 (m, 2H), 7.20 – 7.10 (m, 3H), 6.92 – 6.82 (m, 2H), 6.67 – 6.57 (m, 2H), 6.48 (dd, $J$ = 8.3, 2.5 Hz, 1H), 6.24 – 6.12 (m, 4H), 5.10 (dd, $J$ = 13.3, 5.1 Hz, 1H), 4.70 – 4.50 (m, 2H), 4.46 – 4.31 (m, 2H), 4.29 – 4.10 (m, 2H), 3.67 – 3.48 (m, 4H), 3.44 – 3.26 (m, 3H), 3.18 (dq, $J$ = 21.1, 11.1, 9.4 Hz, 2H), 3.07 | 834.65 | General Synthesis Formula 11 |

| | | | | | |
|---|---|---|---|---|---|
| | | yl)phenyl)-2,8-diazaspiro[4.5]decane-8-yl)-2-oxoethyl)-6-oxo-6,8-dihydro-2H,7H-spiro[furo[2,3-e]isoindole-3,4'-piperidine]-7-yl)piperidine-2,6-dione | – 2.86 (m, 4H), 2.64 – 2.56 (m, 1H), 2.46 – 2.26 (m, 2H), 2.23 – 2.06 (m, 1H), 2.03 – 1.78 (m, 6H), 1.56 (d, *J* = 36.3 Hz, 3H). | | |
| **68** | A73 | 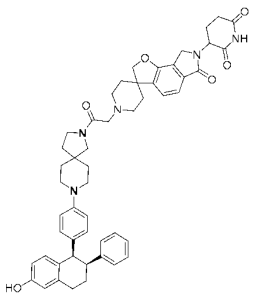 3-(1'-(2-(8-(4-((1R,2S)-6-hydroxy-2-phenyl-1,2,3,4-tetrahydronaphthalen-1-yl)phenyl)-2,8-diazaspiro[4.5]decane-2-yl)-2-oxoethyl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 11.02 – 10.95 (m, 1H), 9.14 – 9.07 (m, 1H), 7.36 – 7.20 (m, 2H), 7.13 (dt, *J* = 16.3, 8.2 Hz, 3H), 6.83 (t, *J* = 6.7 Hz, 2H), 6.70 – 6.44 (m, 5H), 6.22 (dd, *J* = 12.7, 8.5 Hz, 2H), 5.09 (dq, *J* = 12.8, 5.9 Hz, 1H), 4.51 (d, *J* = 11.9 Hz, 1H), 4.40 – 4.29 (m, 1H), 4.26 – 4.09 (m, 2H), 3.57 (dt, *J* = 36.7, 8.2 Hz, 1H), 3.29 (d, *J* = 15.4 Hz, 1H), 3.23 – 2.81 (m, 14H), 2.66 – 2.57 (m, 1H), 2.45 – 2.34 (m, 1H), 2.27 – 2.06 (m, 2H), 2.02 – 1.84 (m, 1H), 1.81 – 1.45 (m, 11H), 1.24 (s, 2H). | 834. 65 | General Synthesis Formula 11 |
| **69** | A74 | 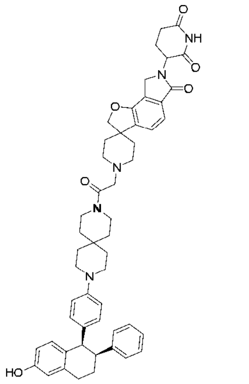 3-(1'-(2-(9-(4-((1R,2S)-6-hydroxy-2-phenyl-1,2,3,4-tetrahydronaphthalen-1-yl)phenyl)-3,9-diazaspiro[5.5]undecane-3-yl)-2-oxoethyl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.98 (s, 1H), 9.10 (d, *J* = 2.8 Hz, 1H), 7.38 (d, *J* = 7.6 Hz, 1H), 7.31 – 7.22 (m, 1H), 7.18 – 7.08 (m, 3H), 6.84 (dd, *J* = 7.0, 1.7 Hz, 2H), 6.68 – 6.48 (m, 5H), 6.21 (dd, *J* = 8.6, 6.7 Hz, 2H), 5.09 (dd, *J* = 13.2, 5.3 Hz, 1H), 4.57 – 4.48 (m, 2H), 4.41 – 4.28 (m, 1H), 4.26 – 4.07 (m, 2H), 3.48 (d, *J* = 47.8 Hz, 4H), 3.31 – 3.26 (m, 2H), 3.18 (s, 2H), 3.04 – 2.96 (m, 5H), 2.95 – 2.81 (m, 5H), 2.64 – 2.56 (m, 1H), 2.45 – 2.36 (m, 1H), 2.20 – 2.06 (m, 3H), 2.02 – 1.85 (m, 2H), 1.75 – 1.64 (m, 4H), 1.51 (d, *J* = 35.4 Hz, 3H), 1.36 (s, 2H), 1.24 (s, 2H). | 848. 65 | General Synthesis Formula 11 |

| | | | | | |
|---|---|---|---|---|---|
| **70** | A75 | 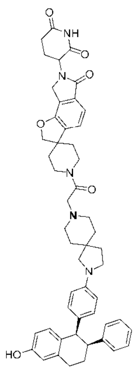 3-(1'-(2-(2-(4-((1R,2S)-6-hydroxy-2-phenyl-1,2,3,4-tetrahydronaphthalen-1-yl)phenyl)-2,8-diazaspiro[4.5]decane-8-yl)acetyl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.98 (s, 1H), 9.08 (s, 1H), 7.37 – 7.28 (m, 2H), 7.20 – 7.12 (m, 3H), 6.89 – 6.84 (m, 2H), 6.65 – 6.58 (m, 2H), 6.47 (dd, *J* = 8.2, 2.6 Hz, 1H), 6.19 – 6.11 (m, 4H), 5.33 (t, *J* = 5.0 Hz, 1H), 5.10 (dd, *J* = 11.0, 6.8 Hz, 1H), 4.73 – 4.59 (m, 2H), 4.37 (dd, *J* = 30.0, 15.2 Hz, 2H), 4.23 (dd, *J* = 17.0, 5.4 Hz, 1H), 4.11 (d, *J* = 5.1 Hz, 2H), 3.29 – 3.22 (m, 2H), 3.19 – 3.08 (m, 1H), 3.04 – 2.87 (m, 3H), 2.75 (d, *J* = 12.9 Hz, 1H), 2.64 – 2.56 (m, 1H), 2.47 – 2.33 (m, 6H), 2.19 – 2.10 (m, 1H), 2.04 – 1.96 (m, 2H), 1.87 (d, *J* = 11.0 Hz, 1H), 1.81 – 1.69 (m, 2H), 1.49 (d, *J* = 40.2 Hz, 5H), 1.24 (s, 5H). | 834.65 | General Synthesis Formula 11 |
| **71** | A76 | 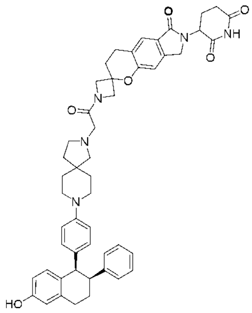 3-(1-(2-(8-(4-((1R,2S)-6-hydroxy-2-phenyl-1,2,3,4-tetrahydronaphthalen-1-yl)phenyl)-2,8-diazaspiro[4.5]decane-2-yl)acetyl)-6'-oxo-3',4',6',8'-tetrahydro-7'H-spiro[azetidine-3,2'-pyrano[2,3-f]isoindole]-7'-yl)piperidine-2,6-dione | $^1$H NMR (600 MHz, DMSO-$d_6$) δ 10.97 (s, 1H), 10.25 (s, 1H), 7.53 (s, 1H), 7.21 – 7.02 (m, 4H), 6.91 – 6.80 (m, 2H), 6.73 – 6.58 (m, 4H), 6.49 (dd, *J* = 8.3, 2.5 Hz, 1H), 6.25 (d, *J* = 8.1 Hz, 2H), 5.07 (ddd, *J* = 13.3, 5.2, 3.4 Hz, 1H), 4.39 – 3.94 (m, 11H), 3.69 – 3.48 (m, 2H), 3.34 – 3.20 (m, 2H), 3.11 – 2.84 (m, 9H), 2.62 – 2.56 (m, 1H), 2.40 – 2.32 (m, 1H), 2.20 – 2.06 (m, 3H), 1.98 (ddd, *J* = 15.2, 7.6, 3.6 Hz, 2H), 1.84 (d, *J* = 10.5 Hz, 1H), 1.71 (d, *J* = 23.4 Hz, 4H). | 820.60 | General Synthesis Formula 10 |
| **72** | A77 | 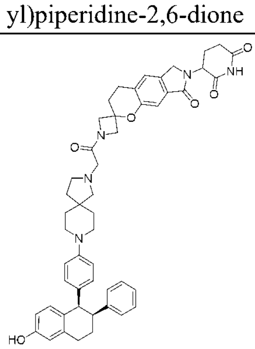 3-(1-(2-(8-(4-((1R,2S)-6-hydroxy-2-phenyl-1,2,3,4-tetrahydronaphthalen-1-yl)phenyl)-2,8-diazaspiro[4.5]decane- | $^1$H NMR (600 MHz, DMSO-$d_6$) δ 10.98 (s, 1H), 10.25 (s, 1H), 7.38 (s, 1H), 7.22 – 7.04 (m, 4H), 6.88 – 6.80 (m, 2H), 6.71 – 6.56 (m, 4H), 6.49 (dd, *J* = 8.3, 2.5 Hz, 1H), 6.25 (d, *J* = 8.0 Hz, 2H), 5.08 (ddd, *J* = 13.4, 5.2, 3.4 Hz, 1H), 4.40 – 4.07 (m, 9H), 3.97 (dd, *J* = 10.6, 6.4 Hz, 1H), 3.67 – 3.48 (m, 3H), 3.34 – 3.20 (m, 3H), 3.09 – 2.86 (m, 10H), 2.63 – 2.56 (m, 1H), 2.44 – 2.33 (m, 1H), 2.20 – 2.11 (m, 2H), 2.03 – 1.94 (m, 2H), 1.85 (t, *J* = 8.9 Hz, 1H), | 820.65 | General Synthesis Formula 10 |

| | | | | | |
|---|---|---|---|---|---|
| | | 2-yl)acetyl)-8'-oxo-3',4',6',8'-tetrahydro-7'H-spiro[azetidine-3,2'-pyrano[2,3-f]isoindole]-7'-yl)piperidine-2,6-dione | 1.71 (d, *J* = 26.2 Hz, 3H). | | |
| **73** | A78 | 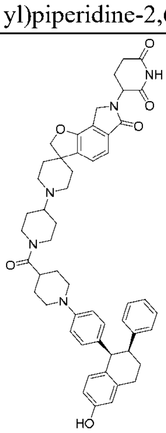 3-(1'-(1-(1-(4-((1R,2S)-6-hydroxy-2-phenyl-1,2,3,4-tetrahydronaphthalen-1-yl)phenyl)piperidin-4-carbonyl)piperidin-4-yl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.99 (s, 1H), 9.61 (s, 1H), 7.32 (dd, *J* = 42.1, 7.6 Hz, 2H), 7.19 – 7.11 (m, 3H), 6.89 – 6.60 (m, 6H), 6.50 (dd, *J* = 8.3, 2.5 Hz, 1H), 6.32 (s, 2H), 5.10 (dd, *J* = 13.3, 5.1 Hz, 1H), 4.75 – 4.54 (m, 4H), 4.40 (d, *J* = 17.2 Hz, 2H), 4.21 (dd, *J* = 39.9, 23.2 Hz, 4H), 3.64 – 3.47 (m, 4H), 3.44 – 3.25 (m, 1H), 3.09 (d, *J* = 16.6 Hz, 3H), 3.02 – 2.79 (m, 5H), 2.66 – 2.53 (m, 1H), 2.47 – 2.36 (m, 1H), 2.28 – 1.91 (m, 7H), 1.85 – 1.37 (m, 8H). | 848.65 | General Synthesis Formula 14 |
| **74** | A79 | 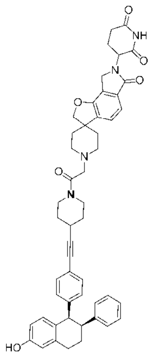 3-(1'-(2-(4-((4-((1R,2S)-6-hydroxy-2-phenyl-1,2,3,4-tetrahydronaphthalen-1-yl)phenyl)ethynyl)piperidin-1-yl)-2-oxoethyl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.99 (s, 1H), 9.66 (s, 1H), 7.41 – 7.35 (m, 1H), 7.31 (d, *J* = 7.6 Hz, 1H), 7.20 – 7.09 (m, 3H), 7.00 (d, *J* = 8.1 Hz, 2H), 6.90 – 6.81 (m, 2H), 6.68 – 6.60 (m, 2H), 6.50 (dd, *J* = 8.3, 2.6 Hz, 1H), 6.37 (d, *J* = 8.1 Hz, 2H), 5.10 (dd, *J* = 13.3, 5.1 Hz, 1H), 4.69 – 4.58 (m, 2H), 4.51 (s, 0H), 4.43 – 4.20 (m, 5H), 3.97 – 3.88 (m, 1H), 3.52 (d, *J* = 12.5 Hz, 3H), 3.38 (ddd, *J* = 13.1, 5.5, 2.4 Hz, 2H), 3.26 (td, *J* = 9.3, 4.3 Hz, 2H), 3.15 (d, *J* = 11.4 Hz, 1H), 3.09 – 2.87 (m, 4H), 2.63 – 2.57 (m, 1H), 2.45 – 2.39 (m, 1H), 2.31 (td, *J* = 14.2, 4.2 Hz, 2H), 2.08 (qd, *J* = 12.6, 6.0 Hz, 1H), 2.01 – 1.84 (m, 4H), 1.79 – 1.60 (m, 2H), 1.57 – 1.46 (m, 1H). | 803.56 | GENERAL SYNTHESIS FORMULA 39 |

| | | | | | |
|---|---|---|---|---|---|
| **75** | A81 | 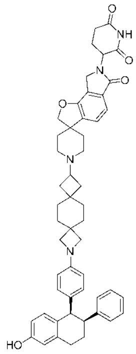 3-(1'-(2-(4-((1R,2S)-6-hydroxy-2-phenyl-1,2,3,4-tetrahydronaphthalen-1-yl)phenyl)-2-azadispiro[3.2.3$^{7}$.2$^{4}$]dodecane-9-yl)-6-oxo-6,8-dihydro-2H,7H-spiro[furo[2,3-e]isoindole-3,4'-piperidine]-7-yl)piperidine-2,6-dione | $^{1}$H NMR (600 MHz, DMSO-$d_6$) δ 10.99 (s, 1H), 9.11 (s, 1H), 7.40 – 7.27 (m, 2H), 7.20 – 7.06 (m, 3H), 6.88 – 6.79 (m, 2H), 6.68 – 6.58 (m, 2H), 6.48 (dd, *J* = 8.3, 2.5 Hz, 1H), 6.17 (d, *J* = 8.2 Hz, 2H), 6.03 (dd, *J* = 8.7, 2.5 Hz, 2H), 5.10 (dd, *J* = 13.3, 5.0 Hz, 1H), 4.71 – 4.57 (m, 2H), 4.40 (d, *J* = 17.3 Hz, 1H), 4.24 (d, *J* = 17.2 Hz, 1H), 4.11 (d, *J* = 4.9 Hz, 2H), 3.53 – 3.24 (m, 6H), 3.17 (s, 1H), 3.03 – 2.82 (m, 5H), 2.68 – 2.55 (m, 1H), 2.46 – 2.37 (m, 1H), 2.21 – 1.83 (m, 10H), 1.75 – 1.39 (m, 9H). | 817. 65 | General Synthesis Formula 6 |
| **76** | A82 | 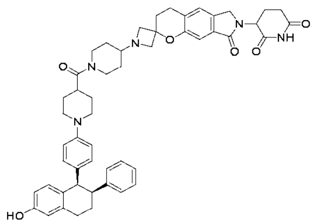 3-(1-(1-(1-(4-((1R,2S)-6-hydroxy-2-phenyl-1,2,3,4-tetrahydronaphthalen-1-yl)phenyl)piperidin-4-carbonyl)piperidin-4-yl)-8'-oxo-3',4',6',8'-tetrahydro-7'H-spiro[azetidine-3,2'-pyrano[2,3-f]isoindole]-7'-yl)piperidine-2,6-dione | $^{1}$H NMR (600 MHz, DMSO-$d_6$) δ 10.98 (s, 1H), 9.14 (s, 1H), 7.39 (d, *J* = 3.4 Hz, 1H), 7.20 – 7.07 (m, 4H), 6.89 – 6.80 (m, 2H), 6.75 – 6.60 (m, 4H), 6.49 (dd, *J* = 8.3, 2.5 Hz, 1H), 6.30 (s, 2H), 5.08 (dd, *J* = 13.3, 5.1 Hz, 1H), 4.58 – 4.08 (m, 10H), 3.43 (dt, *J* = 143.9, 8.1 Hz, 5H), 3.10 – 2.78 (m, 7H), 2.64 – 2.56 (m, 1H), 2.45 – 2.34 (m, 1H), 2.20 (dt, *J* = 14.4, 7.1 Hz, 2H), 2.14 – 1.89 (m, 4H), 1.76 – 1.62 (m, 5H), 1.22 (ddd, *J* = 53.2, 16.6, 10.3 Hz, 3H). | 834. 60 | General Synthesis Formula 14 |
| **77** | A83 | 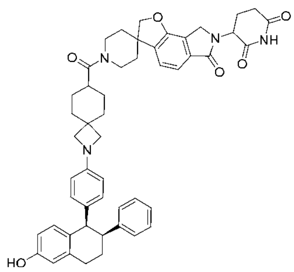 3-(1'-(2-(4-((1R,2S)-6-hydroxy-2-phenyl-1,2,3,4-tetrahydronaphthalen-1-yl)phenyl)-2-azaspiro[3.5]nonane-7-carbonyl)-6-oxo-6,8-dihydro-2H,7H-spiro[furo[2,3-e]isoindole-3,4'-piperidine]-7-yl)piperidine-2,6-dione | $^{1}$H NMR (600 MHz, DMSO-$d_6$) δ 10.98 (s, 1H), 9.10 (s, 1H), 7.42 (d, *J* = 7.6 Hz, 1H), 7.27 (d, *J* = 7.6 Hz, 1H), 7.20 – 7.07 (m, 3H), 6.89 – 6.80 (m, 2H), 6.67 – 6.57 (m, 2H), 6.48 (dd, *J* = 8.3, 2.5 Hz, 1H), 6.19 (d, *J* = 8.2 Hz, 2H), 6.08 (d, *J* = 8.0 Hz, 2H), 5.14 – 5.01 (m, 1H), 4.64 (dq, *J* = 17.4, 9.5 Hz, 2H), 4.39 (d, *J* = 17.2 Hz, 2H), 4.29 – 4.10 (m, 2H), 3.45 (s, 2H), 3.36 (t, *J* = 5.2 Hz, 2H), 3.28 (ddd, *J* = 12.9, 5.2, 2.3 Hz, 1H), 3.19 (t, *J* = 12.5 Hz, 1H), 3.01 – 2.86 (m, 3H), 2.75 – 2.56 (m, 2H), 2.47 – 2.36 (m, 1H), 2.10 (qd, *J* | 805. 65 | General Synthesis Formula 14 |

| | | | | | |
|---|---|---|---|---|---|
| | | | = 12.6, 6.3 Hz, 1H), 2.01 – 1.94 (m, 1H), 1.92 – 1.21 (m, 15H). | | |
| **78** | A84 | 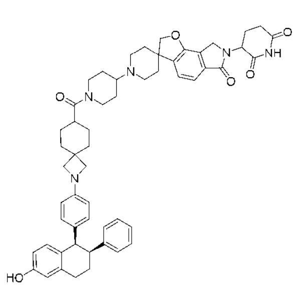 3-(1'-(1-(2-(4-((1R,2S)-6-hydroxy-2-phenyl-1,2,3,4-tetrahydronaphthalen-1-yl)phenyl)-2-azaspiro[3.5]nonane-7-carbonyl)piperidin-4-yl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.99 (s, 1H), 9.09 (s, 1H), 7.36 (d, *J* = 7.6 Hz, 1H), 7.28 (d, *J* = 7.6 Hz, 1H), 7.20 – 7.05 (m, 3H), 6.87 – 6.77 (m, 2H), 6.65 – 6.57 (m, 2H), 6.48 (dd, *J* = 8.3, 2.5 Hz, 1H), 6.18 (d, *J* = 8.1 Hz, 2H), 6.05 (d, *J* = 8.0 Hz, 2H), 5.10 (dd, *J* = 13.3, 5.1 Hz, 1H), 4.77 – 4.48 (m, 3H), 4.40 (d, *J* = 17.2 Hz, 1H), 4.24 (d, *J* = 17.2 Hz, 1H), 4.17 – 4.05 (m, 2H), 3.52 (d, *J* = 19.9 Hz, 4H), 3.45 – 3.25 (m, 5H), 3.18 – 2.86 (m, 7H), 2.65 – 2.56 (m, 1H), 2.47 – 2.36 (m, 1H), 2.22 – 1.95 (m, 5H), 1.88 (d, *J* = 12.5 Hz, 2H), 1.70 (d, *J* = 5.8 Hz, 3H), 1.64 – 1.32 (m, 9H). | 888.65 | General Synthesis Formula 14 |
| **79** | A85 | 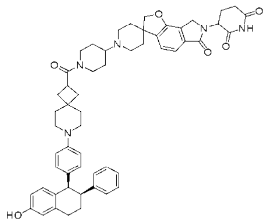 3-(1'-(1-(7-(4-((1R,2S)-6-hydroxy-2-phenyl-1,2,3,4-tetrahydronaphthalen-1-yl)phenyl)-7-azaspiro[3.5]nonane-2-carbonyl)piperidin-4-yl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.99 (s, 1H), 9.40 (s, 1H), 7.36 (d, *J* = 7.6 Hz, 1H), 7.28 (d, *J* = 7.6 Hz, 1H), 7.14 (dt, *J* = 11.8, 6.6 Hz, 3H), 6.94 – 6.78 (m, 2H), 6.72 – 6.58 (m, 4H), 6.49 (dd, *J* = 8.3, 2.5 Hz, 1H), 6.36 – 6.17 (m, 2H), 5.10 (dd, *J* = 13.3, 5.1 Hz, 1H), 4.72 – 4.50 (m, 3H), 4.40 (d, *J* = 17.2 Hz, 1H), 4.28 – 4.08 (m, 2H), 3.87 (d, *J* = 12.8 Hz, 1H), 3.33 (p, *J* = 7.9, 7.2 Hz, 2H), 3.13 – 2.83 (m, 9H), 2.67 – 2.55 (m, 1H), 2.46 – 2.35 (m, 1H), 2.23 – 1.91 (m, 17H), 1.79 – 1.41 (m, 7H). | 888.70 | General Synthesis Formula 14 |
| **80** | A86 | 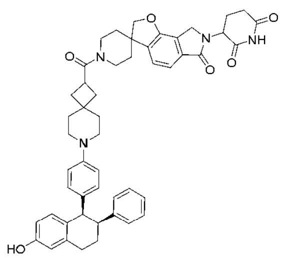 3-(1'-(7-(4-((1R,2S)-6-hydroxy-2-phenyl-1,2,3,4-tetrahydronaphthalen-1-yl)phenyl)-7-azaspiro[3.5]nonane-2-carbonyl)-6-oxo-6,8-dihydro-2H,7H-spiro[furo[2,3-e]isoindole-3,4'- | $^1$H NMR (600 MHz, DMSO-$d_6$) δ 10.97 (s, 1H), 9.10 (s, 1H), 7.41 (d, *J* = 7.6 Hz, 1H), 7.27 (d, *J* = 7.6 Hz, 1H), 7.14 (dt, *J* = 13.9, 6.7 Hz, 3H), 6.89 – 6.77 (m, 2H), 6.69 – 6.43 (m, 5H), 6.20 (d, *J* = 8.3 Hz, 2H), 5.09 (dt, *J* = 13.5, 4.2 Hz, 1H), 4.70 – 4.56 (m, 2H), 4.44 – 4.33 (m, 2H), 4.23 (dd, *J* = 17.1, 6.4 Hz, 1H), 4.13 (d, *J* = 5.0 Hz, 1H), 3.73 (d, *J* = 13.5 Hz, 1H), 3.32 – 3.24 (m, 1H), 3.09 (t, *J* = 12.9 Hz, 1H), 3.02 – 2.84 (m, 7H), 2.73 (t, *J* = 12.7 Hz, 1H), 2.62 – 2.56 (m, 1H), 2.46 – 2.38 (m, | 805.60 | General Synthesis Formula 14 |

| | | | | | |
|---|---|---|---|---|---|
| | | piperidine]-7-yl)piperidine-2,6-dione | 1H), 2.14 – 1.88 (m, 6H), 1.82 – 1.64 (m, 7H), 1.50 (t, $J$ = 5.9 Hz, 2H), 1.24 (s, 1H). | | |
| **81** | A88 | 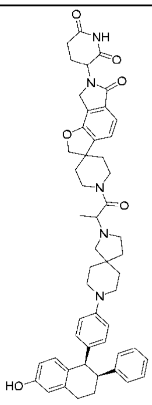 3-(1'-(2-(8-(4-((1R,2S)-6-hydroxy-2-phenyl-1,2,3,4-tetrahydronaphthalen-1-yl)phenyl)-2,8-diazaspiro[4.5]decan-2-yl)propanoyl)-6-oxo-6,8-dihydro-2H,7H-spiro[furo[2,3-e]isoindole-3,4'-piperidin]-7-yl)piperidine-2,6-dione | $^1$H NMR (400 MHz, DMSO-$d_6$) δ 10.98 (s, 1H), 10.11 (s, 1H), 7.51 (dd, $J$ = 7.6, 2.7 Hz, 1H), 7.46 – 7.38 (m, 1H), 7.35 – 7.27 (m, 1H), 7.20 – 7.09 (m, 3H), 6.91 – 6.82 (m, 2H), 6.64 (dq, $J$ = 13.2, 3.1, 2.5 Hz, 3H), 6.49 (dd, $J$ = 8.3, 2.6 Hz, 1H), 6.26 (d, $J$ = 7.9 Hz, 2H), 5.10 (dt, $J$ = 13.3, 4.7 Hz, 1H), 4.68 (tt, $J$ = 18.1, 8.4 Hz, 3H), 4.40 (dd, $J$ = 17.2, 5.0 Hz, 2H), 4.28 – 4.09 (m, 2H), 3.31 (d, $J$ = 12.6 Hz, 4H), 3.19 – 2.79 (m, 10H), 2.71 – 2.57 (m, 1H), 2.47 – 2.33 (m, 2H), 2.18 – 1.58 (m, 12H), 1.45 (dt, $J$ = 34.8, 6.2 Hz, 3H), 1.24 (s, 1H). | 848.60 | General Synthesis Formula 10 |
| **82** | A89 | 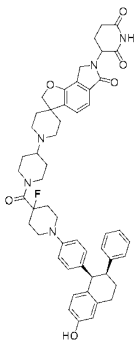 3-(1'-(1-(4-fluoro-1-(4-((1R,2S)-6-hydroxy-2-phenyl-1,2,3,4-tetrahydronaphthalen-1-yl)phenyl)piperidine-4-carbonyl)piperidin-4-yl)-6-oxo-6,8-dihydro-2H,7H-spiro[furo[2,3-e]isoindole-3,4'-piperidin]-7-yl)piperidine-2,6-dione | $^1$H NMR (400 MHz, DMSO-$d_6$) δ 10.99 (s, 1H), 9.43 (s, 1H), 7.40 – 7.26 (m, 2H), 7.22 – 7.05 (m, 3H), 6.90 – 6.78 (m, 2H), 6.70 – 6.57 (m, 4H), 6.49 (dd, $J$ = 8.2, 2.6 Hz, 1H), 6.25 (d, $J$ = 8.4 Hz, 2H), 5.10 (dd, $J$ = 13.3, 5.1 Hz, 1H), 4.74 – 4.63 (m, 2H), 4.57 – 4.35 (m, 3H), 4.29 – 4.08 (m, 2H), 3.46 (d, $J$ = 12.1 Hz, 3H), 3.30 (ddd, $J$ = 13.0, 5.3, 2.2 Hz, 1H), 3.19 – 3.06 (m, 4H), 3.01 – 2.85 (m, 5H), 2.72 – 2.56 (m, 1H), 2.45 – 2.31 (m, 1H), 2.24 – 1.88 (m, 15H), 1.78 – 1.42 (m, 3H). | 866.60 | General Synthesis Formula 14 |

| | | | | | |
|---|---|---|---|---|---|
| **83** | A90 | 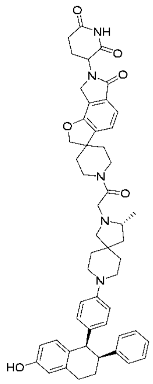 3-(1'-(2-((R)-8-(4-((1R,2S)-6-hydroxy-2-phenyl-1,2,3,4-tetrahydronaphthalen-1-yl)phenyl)-3-methyl-2,8-diazaspiro[4.5]decan-2-yl)acetyl)-6-oxo-6,8-dihydro-2H,7H-spiro[furo[2,3-e]isoindole-3,4'-piperidin]-7-yl)piperidine-2,6-dione | $^1$H NMR (400 MHz, DMSO-$d_6$) δ 10.98 (s, 1H), 9.72 (s, 1H), 7.35 (dd, $J$ = 24.2, 7.2 Hz, 2H), 7.14 (tt, $J$ = 7.5, 4.4 Hz, 3H), 6.84 (ddt, $J$ = 5.7, 3.9, 1.7 Hz, 2H), 6.70 – 6.57 (m, 4H), 6.49 (dt, $J$ = 8.3, 2.7 Hz, 1H), 6.34 – 6.14 (m, 2H), 5.10 (dt, $J$ = 13.2, 4.5 Hz, 1H), 4.76 – 4.60 (m, 2H), 4.56 – 4.09 (m, 7H), 3.36 – 3.17 (m, 3H), 3.14 – 2.78 (m, 8H), 2.71 – 2.55 (m, 2H), 2.46 – 2.30 (m, 5H), 2.24 – 1.89 (m, 1H), 1.86 – 1.52 (m, 8H), 1.48 – 1.20 (m, 4H). | 848.60 | General Synthesis Formula 10 |
| **84** | A91 | 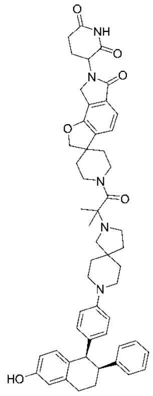 3-(1'-(2-(8-(4-((1R,2S)-6-hydroxy-2-phenyl-1,2,3,4-tetrahydronaphthalen-1-yl)phenyl)-2,8-diazaspiro[4.5]decan-2-yl)-2-methylpropanoyl)-6-oxo-6,8-dihydro-2H,7H-spiro[furo[2,3-e]isoindole-3,4'-piperidin]-7-yl)piperidine-2,6-dione | $^1$H NMR (400 MHz, DMSO-$d_6$) δ 10.98 (s, 1H), 9.98 (s, 1H), 7.46 (d, $J$ = 7.6 Hz, 1H), 7.32 (d, $J$ = 7.6 Hz, 1H), 7.15 (qd, $J$ = 7.4, 6.2, 3.7 Hz, 3H), 6.90 – 6.80 (m, 2H), 6.73 – 6.57 (m, 4H), 6.49 (dd, $J$ = 8.3, 2.5 Hz, 1H), 6.26 (d, $J$ = 8.1 Hz, 2H), 5.10 (dd, $J$ = 13.2, 5.1 Hz, 1H), 4.73 – 4.57 (m, 2H), 4.40 (d, $J$ = 17.2 Hz, 1H), 4.31 – 4.11 (m, 3H), 3.43 – 3.26 (m, 4H), 3.20 – 2.85 (m, 10H), 2.74 – 2.56 (m, 1H), 2.46 – 2.32 (m, 1H), 2.04 (ddd, $J$ = 45.1, 9.6, 4.4 Hz, 1H), 1.93 – 1.52 (m, 17H), 1.24 (s, 1H). | 862.60 | General Synthesis Formula 10 |

| | | | | | |
|---|---|---|---|---|---|
| **85** | A92 | 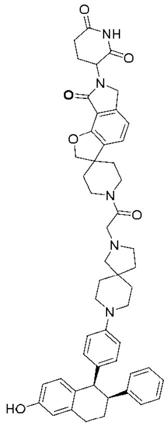 3-(1'-(2-(8-(4-((1R,2S)-6-hydroxy-2-phenyl-1,2,3,4-tetrahydronaphthalen-1-yl)phenyl)-2,8-diazaspiro[4.5]decan-2-yl)acetyl)-8-oxo-6,8-dihydro-2H,7H-spiro[furo[2,3-e]isoindole-3,4'-piperidin]-7-yl)piperidine-2,6-dione | $^1$H NMR (400 MHz, DMSO-$d_6$) δ 10.97 (s, 1H), 10.08 (s, 1H), 7.41 (d, *J* = 7.5 Hz, 1H), 7.22 – 7.03 (m, 4H), 6.84 (d, *J* = 7.1 Hz, 2H), 6.72 – 6.57 (m, 4H), 6.49 (dd, *J* = 8.3, 2.5 Hz, 1H), 6.26 (d, *J* = 8.2 Hz, 2H), 5.05 (ddd, *J* = 13.3, 5.1, 3.3 Hz, 1H), 4.68 (q, *J* = 9.4 Hz, 2H), 4.52 – 4.12 (m, 7H), 3.33 – 3.17 (m, 4H), 3.10 – 2.81 (m, 9H), 2.68 – 2.55 (m, 1H), 2.42 – 2.27 (m, 1H), 2.20 – 1.85 (m, 6H), 1.83 – 1.60 (m, 7H), 1.24 (s, 1H). | 834.55 | General Synthesis Formula 10 |
| **86** | A93 | 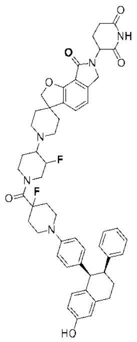 3-(1'-(3-fluoro-1-(4-fluoro-1-(4-((1R,2S)-6-hydroxy-2-phenyl-1,2,3,4-tetrahydronaphthalen-1-yl)phenyl)piperidine-4-carbonyl)piperidin-4-yl)-8-oxo-6,8-dihydro-2H,7H-spiro[furo[2,3-e]isoindole-3,4'-piperidin]-7-yl)piperidine-2,6-dione | $^1$H NMR (400 MHz, DMSO-$d_6$) δ 10.99 (s, 1H), 9.79 (s, 1H), 7.44 – 7.25 (m, 2H), 7.22 – 7.08 (m, 3H), 6.91 – 6.77 (m, 2H), 6.69 – 6.58 (m, 4H), 6.49 (dd, *J* = 8.3, 2.5 Hz, 1H), 6.36 – 6.18 (m, 2H), 5.56 – 5.32 (m, 2H), 5.10 (dd, *J* = 13.3, 5.1 Hz, 1H), 4.93 – 4.60 (m, 4H), 4.58 – 4.09 (m, 6H), 3.34 – 3.12 (m, 3H), 3.08 – 2.76 (m, 7H), 2.71 – 2.58 (m, 1H), 2.48 – 2.35 (m, 1H), 2.29 – 1.84 (m, 11H), 1.71 (d, *J* = 12.0 Hz, 1H), 1.24 (s, 1H). | 884.55 | General Synthesis Formula 14 |

| | | | | | |
|---|---|---|---|---|---|
| **87** | A94 | 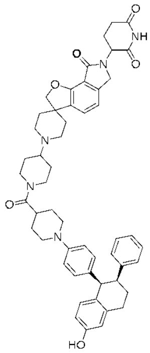 3-(1'-(1-(1-(4-((1R,2S)-6-hydroxy-2-phenyl-1,2,3,4-tetrahydronaphthalen-1-yl)phenyl)piperidine-4-carbonyl)piperidin-4-yl)-8-oxo-6,8-dihydro-2H,7H-spiro[furo[2,3-e]isoindole-3,4'-piperidin]-7-yl)piperidine-2,6-dione | $^1$H NMR (400 MHz, DMSO-$d_6$) δ 10.97 (s, 1H), 10.08 (s, 1H), 7.41 (d, *J* = 7.5 Hz, 1H), 7.23 – 7.10 (m, 3H), 7.06 (dd, *J* = 7.6, 1.9 Hz, 1H), 6.84 (d, *J* = 7.1 Hz, 2H), 6.70 – 6.57 (m, 4H), 6.49 (dd, *J* = 8.3, 2.5 Hz, 1H), 6.26 (d, *J* = 8.2 Hz, 2H), 5.05 (ddd, *J* = 13.3, 5.1, 3.3 Hz, 1H), 4.68 (q, *J* = 9.4 Hz, 2H), 4.55 – 4.09 (m, 7H), 3.36 – 3.17 (m, 4H), 3.10 – 2.83 (m, 9H), 2.70 – 2.54 (m, 1H), 2.43 – 2.29 (m, 1H), 2.16 – 1.53 (m, 13H), 1.24 (s, 1H). | 848.55 | General Synthesis Formula 14 |
| **88** | A95 | 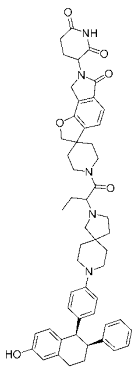 3-(1'-(2-(8-(4-((1R,2S)-6-hydroxy-2-phenyl-1,2,3,4-tetrahydronaphthalen-1-yl)phenyl)-2,8-diazaspiro[4.5]decan-2-yl)butanoyl)-6-oxo-6,8-dihydro-2H,7H-spiro[furo[2,3-e]isoindole-3,4'-piperidin]-7-yl)piperidine-2,6-dione | $^1$H NMR (400 MHz, DMSO-$d_6$) δ 10.99 (s, 1H), 10.06 (d, *J* = 26.1 Hz, 1H), 7.49 – 7.39 (m, 1H), 7.34 – 7.24 (m, 1H), 7.15 (q, *J* = 7.9 Hz, 3H), 6.85 (ddd, *J* = 7.7, 4.8, 1.7 Hz, 2H), 6.64 (dq, *J* = 12.8, 3.1, 2.6 Hz, 4H), 6.49 (dd, *J* = 8.3, 2.6 Hz, 1H), 6.27 (d, *J* = 7.7 Hz, 2H), 5.16 – 5.02 (m, 1H), 4.76 – 4.65 (m, 3H), 4.53 – 4.35 (m, 2H), 4.27 – 4.12 (m, 2H), 3.31 (dd, *J* = 12.7, 5.4 Hz, 2H), 3.20 – 2.86 (m, 11H), 2.70 – 2.55 (m, 1H), 2.42 (dd, *J* = 18.0, 9.1 Hz, 1H), 2.08 – 1.68 (m, 14H), 1.25 (td, *J* = 7.0, 4.9 Hz, 2H), 0.93 (dtd, *J* = 40.2, 7.4, 4.5 Hz, 4H). | 862.65 | General Synthesis Formula 10 |
| **89** | A96 | 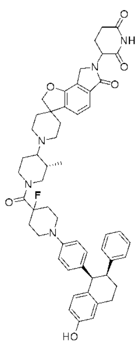 3-(1'-((3R)-1-(4-fluoro-1-(4-((1R,2S)- | $^1$H NMR (600 MHz, DMSO-$d_6$) δ 10.99 (s, 1H), 9.06 (s, 1H), 7.36 (d, *J* = 7.5 Hz, 1H), 7.28 (d, *J* = 7.6 Hz, 1H), 7.19 – 7.07 (m, 3H), 6.84 (d, *J* = 7.4 Hz, 2H), 6.71 – 6.59 (m, 4H), 6.49 (dd, *J* = 8.3, 2.5 Hz, 1H), 6.25 (d, *J* = 8.3 Hz, 2H), 5.10 (dd, *J* = 13.2, 5.2 Hz, 1H), 4.73 – 4.62 (m, 2H), 4.55 – 4.37 (m, 3H), 4.28 – 4.13 (m, 2H), 3.75 (s, 1H), 3.59 (s, 1H), 3.34 (s, 0H), 3.33 – 3.13 (m, 3H), 3.05 – 2.85 (m, 11H), | 880.50 | General Synthesis Formula 14 |

| | | | | | |
|---|---|---|---|---|---|
| | | 6-hydroxy-2-phenyl-1,2,3,4-tetrahydronaphthalen-1-yl)phenyl)piperidine-4-carbonyl)-3-methylpiperidin-4-yl)-6-oxo-6,8-dihydro-2H,7H-spiro[furo[2,3-e]isoindole-3,4'-piperidin]-7-yl)piperidine-2,6-dione | 2.75 – 2.58 (m, 3H), 2.47 – 2.37 (m, 2H), 2.27 – 1.89 (m, 9H), 1.71 (dd, *J* = 12.9, 6.3 Hz, 3H), 1.24 (s, 1H), 1.06 – 0.79 (m, 4H). | | |
| **90** | A97 | 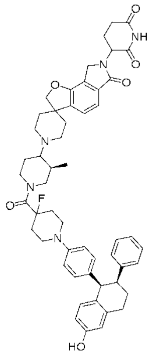 3-(1'-((3S)-1-(4-fluoro-1-(4-((1R,2S)-6-hydroxy-2-phenyl-1,2,3,4-tetrahydronaphthalen-1-yl)phenyl)piperidine-4-carbonyl)-3-methylpiperidin-4-yl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 10.99 (s, 1H), 9.06 – 8.93 (m, 1H), 7.36 (d, *J* = 7.4 Hz, 1H), 7.28 (d, *J* = 7.6 Hz, 1H), 7.19 – 7.08 (m, 3H), 6.84 (d, *J* = 7.4 Hz, 2H), 6.70 – 6.58 (m, 4H), 6.49 (dd, *J* = 8.3, 2.5 Hz, 1H), 6.25 (d, *J* = 8.3 Hz, 2H), 5.10 (dd, *J* = 13.2, 5.2 Hz, 1H), 4.69 – 4.60 (m, 2H), 4.49 (s, 2H), 4.41 (dd, *J* = 17.2, 6.4 Hz, 1H), 4.33 – 4.22 (m, 2H), 4.16 (d, *J* = 5.0 Hz, 1H), 4.07 – 3.93 (m, 1H), 3.81 – 3.69 (m, 1H), 3.69 (s, 1H), 3.60 (d, *J* = 12.5 Hz, 1H), 3.30 (ddd, *J* = 13.2, 5.1, 2.3 Hz, 3H), 3.15 – 3.06 (m, 1H), 3.04 – 2.84 (m, 6H), 2.79 – 2.57 (m, 1H), 2.48 – 2.36 (m, 1H), 2.29 – 2.08 (m, 6H), 2.06 – 1.93 (m, 5H), 1.71 (dd, *J* = 12.4, 6.2 Hz, 1H), 1.24 (s, 1H), 1.01 – 0.89 (m, 3H). | 880.55 | General Synthesis Formula 14 |
| **91** | A98 | 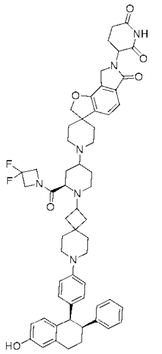 3-(1-(1'-((2R)-2-(3,3-difluoroazetidin-1-ylcarbonyl)-1-(7-(4-((1R,2S)-6-hydroxy-2-phenyl-1,2,3,4-tetrahydronaphthalen-1-yl)phenyl)-7-azaspiro[3.5]nonan-2-yl)dihydronaphthalenebis-piperidine-2,6-dione | $^1$H NMR (600 MHz, DMSO-$d_6$) δ 10.99 (s, 1H), 9.14 (s, 1H), 7.33 (dd, *J* = 42.7, 7.6 Hz, 2H), 7.19 – 7.06 (m, 3H), 6.91 – 6.79 (m, 2H), 6.72 – 6.57 (m, 4H), 6.48 (dd, *J* = 8.2, 2.5 Hz, 1H), 6.25 (d, *J* = 8.1 Hz, 2H), 5.10 (dd, *J* = 13.3, 5.1 Hz, 1H), 4.88 (dd, *J* = 55.9, 12.1 Hz, 3H), 4.67 (q, *J* = 10.4, 9.5 Hz, 2H), 4.56 – 4.34 (m, 4H), 4.29 – 4.12 (m, 3H), 3.76 (s, 5H), 3.10 – 2.87 (m, 8H), 2.68 – 2.57 (m, 2H), 2.47 – 2.38 (m, 2H), 2.25 – 1.90 (m, 11H), 1.76 – 1.50 (m, 6H), 1.24 (s, 1H). | 979.65 | General Synthesis Formula 6 |

| | | | | | |
|---|---|---|---|---|---|
| **92** | A99 | 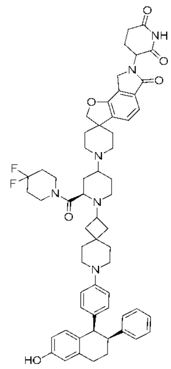 3-(1-(1'-((2R)-2-(4,4-difluoropropylpiperidine-1-carbonyl)-1-(7-(4-((1R,2S)-6-hydroxy-2-phenyl-1,2,3,3,4-tetrahydronaphthalen-1-yl)phenyl)-7-azaspiro[3.5]nonan-2-yl)difluoropiperidinebis-piperidine-2,6-dione | $^1$H NMR (600 MHz, DMSO-$d_6$) δ 10.99 (s, 1H), 9.14 (s, 1H), 7.39 – 7.26 (m, 2H), 7.14 (dt, $J$ = 11.9, 6.6 Hz, 3H), 6.91 – 6.76 (m, 2H), 6.72 – 6.57 (m, 4H), 6.48 (dd, $J$ = 8.3, 2.5 Hz, 1H), 6.24 (d, $J$ = 8.1 Hz, 2H), 5.10 (dd, $J$ = 13.3, 5.1 Hz, 1H), 4.73 – 4.51 (m, 4H), 4.40 (d, $J$ = 17.2 Hz, 1H), 4.31 – 4.12 (m, 3H), 3.86 – 3.64 (m, 5H), 3.15 – 2.81 (m, 9H), 2.65 – 2.57 (m, 2H), 2.41 (ddd, $J$ = 22.9, 17.1, 12.0 Hz, 3H), 2.02 (tq, $J$ = 67.5, 27.5, 22.8 Hz, 15H), 1.79 – 1.48 (m, 7H), 1.24 (s, 2H). | 100 7.75 | General Synthesis Formula 6 |
| **93** | A100 | 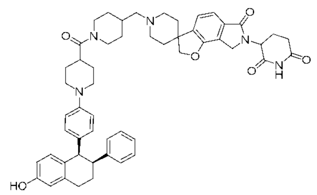 3-(1'-((1-(4-((1R,2S)-6-hydroxy-2-phenyl-1,2,3,4-tetrahydronaphthalen-1-yl)phenyl)piperidine-4-carbonyl)piperidin-4-yl)methyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 10.99 (s, 1H), 9.11 (s, 1H), 7.42 – 7.34 (m, 1H), 7.29 (d, $J$ = 7.6 Hz, 1H), 7.14 (dq, $J$ = 14.1, 7.0 Hz, 3H), 6.83 (d, $J$ = 7.3 Hz, 4H), 6.67 – 6.61 (m, 2H), 6.49 (dd, $J$ = 8.3, 2.5 Hz, 1H), 6.32 (s, 2H), 5.10 (dd, $J$ = 13.3, 5.1 Hz, 1H), 4.76 – 4.61 (m, 2H), 4.41 (dd, $J$ = 15.9, 8.9 Hz, 2H), 4.26 – 4.16 (m, 2H), 4.01 (dd, $J$ = 14.3, 8.5 Hz, 1H), 3.66 – 3.52 (m, 1H), 3.15 – 2.92 (m, 8H), 2.78 (d, $J$ = 50.8 Hz, 3H), 2.64 – 2.56 (m, 3H), 2.48 – 2.37 (m, 2H), 2.30 – 2.09 (m, 5H), 2.03 – 1.93 (m, 3H), 1.75 (d, $J$ = 55.6 Hz, 6H), 1.25 (d, $J$ = 5.7 Hz, 1H), 1.18 – 0.99 (m, 3H). | 862. 65 | General Synthesis Formula 14 |
| **94** | A101 | 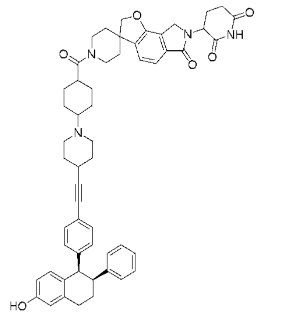 3-(1'-(4-(4-((1R,2S)-6-hydroxy-2-phenyl-1,2,3,4-tetrahydronaphthalen-1- | $^1$H NMR (600 MHz, DMSO-$d_6$) δ 10.98 (s, 1H), 9.18 (d, $J$ = 6.7 Hz, 1H), 7.42 (dd, $J$ = 7.7, 5.3 Hz, 1H), 7.28 (dd, $J$ = 7.6, 1.8 Hz, 1H), 7.20 – 7.10 (m, 3H), 6.99 (s, 2H), 6.85 (d, $J$ = 7.3 Hz, 2H), 6.69 – 6.61 (m, 2H), 6.50 (dd, $J$ = 8.3, 2.5 Hz, 1H), 6.37 (t, $J$ = 9.2 Hz, 2H), 5.33 (t, $J$ = 5.1 Hz, 1H), 5.09 (dd, $J$ = 13.3, 5.0 Hz, 1H), 4.64 (q, $J$ = 14.2, 9.5 Hz, 2H), 4.40 (d, $J$ = 16.7 Hz, 2H), 4.30 – 4.18 (m, 2H), 3.93 | 871. 60 | General Synthesis Formula 38 |

| | | | | | |
|---|---|---|---|---|---|
| | | yl)phenylethynyl)piperidin-1-yl)cyclohexane-1-carbonyl)-6-oxo-6,8-dihydro-2H,7H-spiro[furo[2,3-e]isoindole-3,4'-piperidin]-7-yl)piperidine-2,6-dione | (d, *J* = 33.6 Hz, 2H), 3.18 (d, *J* = 11.6 Hz, 2H), 3.05 – 2.89 (m, 5H), 2.71 (s, 1H), 2.64 – 2.57 (m, 1H), 2.45 – 2.39 (m, 1H), 2.16 – 1.68 (m, 14H), 1.57 – 1.36 (m, 3H), 1.24 (s, 4H), 0.88 (h, *J* = 9.3, 8.4 Hz, 1H). | | |
| **95** | A102 | 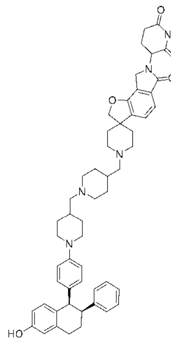 3-(1'-((1-(4-((1R,2S)-6-hydroxy-2-phenyl-1,2,3,4-tetrahydronaphthalen-1-yl)phenyl)piperidin-4-yl)methyl)piperidin-4-yl)methyl-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 10.99 (s, 1H), 9.51 (s, 1H), 7.40 – 7.27 (m, 2H), 7.18 – 7.06 (m, 3H), 6.93 – 6.78 (m, 2H), 6.68 – 6.56 (m, 4H), 6.49 (dd, *J* = 8.3, 2.5 Hz, 1H), 6.25 (d, *J* = 8.3 Hz, 2H), 5.10 (dd, *J* = 13.3, 5.1 Hz, 1H), 4.67 (q, *J* = 9.4 Hz, 2H), 4.40 (d, *J* = 17.2 Hz, 1H), 4.30 – 4.10 (m, 2H), 3.58 (d, *J* = 23.4 Hz, 5H), 3.30 (ddd, *J* = 13.0, 5.1, 2.3 Hz, 2H), 3.13 – 2.86 (m, 10H), 2.67 – 2.57 (m, 3H), 2.47 – 2.37 (m, 1H), 2.27 – 2.20 (m, 2H), 2.11 (td, *J* = 12.6, 6.2 Hz, 2H), 2.01 – 1.91 (m, 6H), 1.86 – 1.69 (m, 3H), 1.51 (d, *J* = 12.5 Hz, 2H), 1.38 – 1.21 (m, 3H). | 848.60 | General Synthesis Formula 37 |
| **96** | A103 | 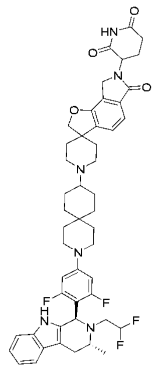 3-(1'-(3-(4-((1R,3R)-2-(2,2-difluoroethyl)-3-methyl-2,3,4,9-tetrahydro-1H-pyrido[3,4-b]indol-1-yl)-3,5-difluorophenyl)-3-azaspiro[5.5]undecan-9-yl)-6-oxo-6,8-dihydro-2H,7H-spiro[furo[2,3-e]isoindole-3,4'-piperidin]-7-yl | $^1$H NMR (600 MHz, DMSO-$d_6$) δ 10.98 (s, 1H), 10.58 (s, 1H), 7.38 (dd, *J* = 13.1, 7.2 Hz, 2H), 7.29 (s, 1H), 7.20 (t, *J* = 9.2 Hz, 1H), 6.97 (dt, *J* = 33.7, 7.3 Hz, 2H), 6.54 (d, *J* = 13.6 Hz, 2H), 6.01 – 5.64 (m, 1H), 5.14 – 5.04 (m, 2H), 4.54 (s, 2H), 4.38 (d, *J* = 17.1 Hz, 1H), 4.22 (d, *J* = 17.0 Hz, 2H), 3.20 (s, 4H), 3.06 (dt, *J* = 15.0, 7.8 Hz, 2H), 2.95 – 2.78 (m, 3H), 2.71 – 2.54 (m, 3H), 2.47 – 2.38 (m, 2H), 2.35 – 2.20 (m, 2H), 2.06 – 1.84 (m, 3H), 1.81 – 1.63 (m, 5H), 1.47 (d, *J* = 102.7 Hz, 5H), 1.31 – 1.05 (m, 7H). | 867.56 | General Synthesis Formula 53 |

| | | | | | |
|---|---|---|---|---|---|
| **97** | A104 | 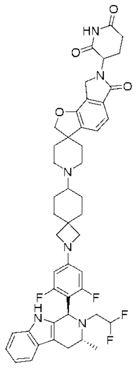 3-(1'-(2-(4-((1R,3R)-2-(2,2-difluoroethyl)-3-methyl-2,3,4,9-tetrahydro-1H-pyrido[3,4-b]indol-1-yl)-3,5-difluorophenyl)-2-azaspiro[3.5]nonan-7-yl)-6-oxo-6,8-dihydro-2H,7H-spiro[furo[2,3-e]isoindole-3,4'-piperidin]-7-yl | $^1$H NMR (600 MHz, DMSO-$d_6$) δ 10.98 (s, 1H), 10.55 (s, 1H), 7.53 – 7.13 (m, 4H), 6.97 (dddd, *J* = 34.0, 8.0, 7.1, 1.2 Hz, 2H), 6.03 (d, *J* = 11.7 Hz, 2H), 5.80 (dt, *J* = 56.4, 4.4 Hz, 2H), 5.16 – 5.04 (m, 2H), 4.54 (s, 2H), 4.38 (d, *J* = 17.1 Hz, 1H), 4.23 (s, 1H), 3.55 (d, *J* = 39.0 Hz, 3H), 3.10 – 3.01 (m, 2H), 2.93 – 2.78 (m, 4H), 2.67 – 2.55 (m, 4H), 2.44 – 2.37 (m, 2H), 2.01 – 1.88 (m, 4H), 1.74 (d, *J* = 23.7 Hz, 4H), 1.52 (s, 3H), 1.39 – 1.22 (m, 3H), 1.09 (d, *J* = 6.5 Hz, 3H). | 839.66 | General Synthesis Formula 53 |
| **98** | A105 | 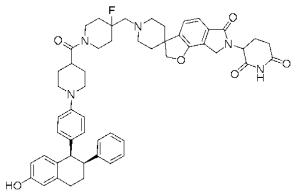 3-(1'-((4-fluoro-1-(1-(4-((1R,2S)-6-hydroxy-2-phenyl-1,2,3,4-tetrahydronaphthalen-1-yl)phenyl)piperidine-4-carbonyl)piperidin-4-yl)methyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 10.99 (s, 1H), 9.44 (s, 1H), 7.32 (dd, *J* = 50.7, 7.6 Hz, 2H), 7.19 – 7.04 (m, 3H), 6.97 – 6.77 (m, 2H), 6.71 – 6.58 (m, 3H), 6.49 (dd, *J* = 8.2, 2.5 Hz, 1H), 6.29 (s, 3H), 5.10 (dd, *J* = 13.3, 5.1 Hz, 1H), 4.78 – 4.57 (m, 3H), 4.40 (d, *J* = 17.2 Hz, 1H), 4.24 (q, *J* = 25.8, 21.8 Hz, 3H), 3.93 (d, *J* = 11.8 Hz, 3H), 3.42 – 3.15 (m, 6H), 3.05 – 2.76 (m, 6H), 2.67 – 2.57 (m, 2H), 2.45 – 2.24 (m, 4H), 2.12 – 1.92 (m, 6H), 1.85 – 1.54 (m, 8H). | 880.60 | General Synthesis Formula 14 |
| **99** | A106 | 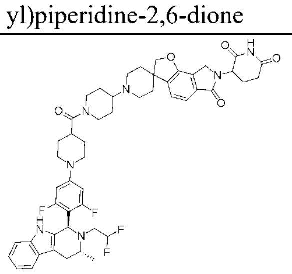 3-(1'-(1-(4-((1R,3R)-2-(2,2-difluoroethyl)-3-methyl-2,3,4,9-tetrahydro-1H-pyrido[3,4-b]indol-1-yl)-3,5-difluorophenyl)piperidine-4-carbonyl)piperidin-4-yl)-6-oxo-6,8-dihydro-2H,7H-spiro[furo[2,3- | $^1$H NMR (600 MHz, DMSO-$d_6$) δ 10.99 (s, 1H), 10.61 (s, 1H), 7.38 (dd, *J* = 25.3, 7.7 Hz, 2H), 7.29 (d, *J* = 7.6 Hz, 1H), 7.21 (d, *J* = 8.1 Hz, 1H), 7.03 – 6.90 (m, 2H), 6.58 (d, *J* = 13.4 Hz, 2H), 5.17 – 5.07 (m, 2H), 4.68 (d, *J* = 6.8 Hz, 2H), 4.58 (t, *J* = 12.5 Hz, 1H), 4.40 (d, *J* = 17.2 Hz, 1H), 4.31 – 4.20 (m, 2H), 3.55 (s, 3H), 3.41 (s, 1H), 3.10 (d, *J* = 13.0 Hz, 5H), 2.96 – 2.81 (m, 5H), 2.68 – 2.55 (m, 3H), 2.46 – 2.38 (m, 2H), 2.23 – 1.89 (m, 8H), 1.72 – 1.42 (m, 7H), 1.11 (d, *J* = 6.5 Hz, 3H). | 910.60 | General Synthesis Formula 56 |

| | | | | | |
|---|---|---|---|---|---|
| | | e]isoindole-3,4'-piperidin]-7-yl)piperidine-2,6-dione | | | |
| **100** | A107 | 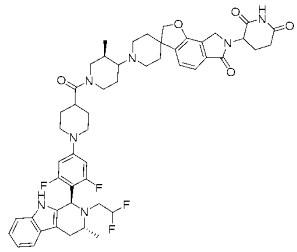 3-(1'-((3R)-1-(1-(4-((1R,3R)-2-(2,2-difluoroethyl)-3-methyl-2,3,4,9-tetrahydro-1H-pyrido[3,4-b]indol-1-yl)-3,5-difluorophenyl)piperidine-4-carbonyl)-3-methylpiperidin-4-yl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 11.00 (s, 1H), 10.62 (s, 1H), 7.43 – 7.34 (m, 2H), 7.28 (d, *J* = 7.7 Hz, 1H), 7.21 (d, *J* = 8.1 Hz, 1H), 7.07 – 6.89 (m, 2H), 6.66 – 6.51 (m, 2H), 5.11 (td, *J* = 13.2, 6.4 Hz, 2H), 4.72 – 4.57 (m, 3H), 4.48 – 4.35 (m, 2H), 4.25 (d, *J* = 17.2 Hz, 2H), 3.83 (s, 3H), 3.43 (s, 2H), 3.23 – 2.98 (m, 5H), 2.95 – 2.78 (m, 3H), 2.69 – 2.57 (m, 2H), 2.46 – 2.37 (m, 2H), 2.29 – 1.86 (m, 8H), 1.56 (d, *J* = 96.1 Hz, 5H), 1.36 – 1.23 (m, 2H), 1.11 (d, *J* = 6.1 Hz, 2H), 1.05 – 0.95 (m, 2H), 0.92 – 0.77 (m, 2H). | 924.60 | General Synthesis Formula 56 |
| **101** | A108 | 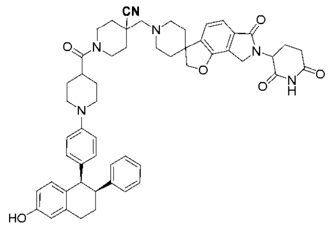 4-((7-(2,6-Dioxopiperidin-3-yl)-6-oxo-7,8-dihydro-2H,6H-spiro[furo[2,3-e]isoindole-3,4'-piperidin]-1'-yl)methyl)-1-(1-(4-((1R,2S)-6-hydroxy-2-phenyl-1,2,3,4-tetrahydronaphthalen-1-yl)phenyl)piperidine-4-carbonyl)piperidine-4-carbonitrile | $^1$H NMR (600 MHz, DMSO-$d_6$) δ 10.99 (s, 1H), 9.16 (s, 1H), 7.35 (s, 2H), 7.19 – 7.07 (m, 3H), 6.89 – 6.78 (m, 3H), 6.73 – 6.58 (m, 3H), 6.50 (dd, *J* = 8.3, 2.5 Hz, 1H), 6.36 (s, 2H), 5.10 (dd, *J* = 13.4, 5.1 Hz, 1H), 4.59 (d, *J* = 99.7 Hz, 4H), 4.40 (d, *J* = 17.1 Hz, 1H), 4.24 (d, *J* = 17.0 Hz, 2H), 4.12 (s, 1H), 3.29 (d, *J* = 59.7 Hz, 5H), 3.04 – 2.87 (m, 5H), 2.78 – 2.58 (m, 4H), 2.46 – 2.35 (m, 2H), 2.28 – 1.93 (m, 7H), 1.87 – 1.40 (m, 4H), 1.42 (s, 4H), 1.25 (d, *J* = 11.6 Hz, 2H). | 887.65 | General Synthesis Formula 14 |
| **102** | A109 | 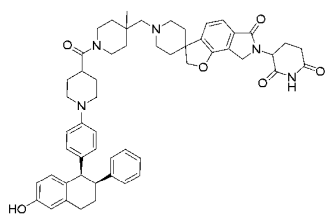 3-(1'-((1-(4-((1R,2S)-6-hydroxy-2-phenyl-1,2,3,4-tetrahydronaphthalen-1-yl)phenyl)piperidine-4-carbonyl)-4-methylpiperidin-4-yl)methyl)-6-oxo-6,8-dihydro-2H,7H-spiro[furo[2,3- | $^1$H NMR (600 MHz, DMSO-$d_6$) δ 10.99 (s, 1H), 9.15 (s, 1H), 7.37 (d, *J* = 7.6 Hz, 1H), 7.28 (d, *J* = 7.6 Hz, 1H), 7.20 – 7.09 (m, 3H), 6.83 (d, *J* = 7.4 Hz, 2H), 6.69 – 6.59 (m, 3H), 6.49 (dd, *J* = 8.3, 2.6 Hz, 1H), 6.30 (s, 3H), 5.10 (dd, *J* = 13.2, 5.5 Hz, 1H), 4.72 – 4.62 (m, 2H), 4.40 (dd, *J* = 17.1, 8.0 Hz, 1H), 4.31 – 4.14 (m, 2H), 3.98 (s, 1H), 3.73 (d, *J* = 14.4 Hz, 2H), 3.55 (s, 4H), 3.36 (d, *J* = 16.2 Hz, 4H), 3.25 (d, *J* = 11.3 Hz, 1H), 3.17 – 2.89 (m, | 876.66 | General Synthesis Formula 14 |

| | | | | | |
|---|---|---|---|---|---|
| | | e]isoindole-3,4'-piperidin]-7-yl)piperidine-2,6-dione | 7H), 2.72 – 2.58 (m, 1H), 2.45 – 2.24 (m, 4H), 2.16 – 1.86 (m, 5H), 1.74 (d, *J* = 23.6 Hz, 3H), 1.50 (d, *J* = 41.3 Hz, 4H), 1.35 – 1.15 (m, 4H). | | |
| **103** | A111 | 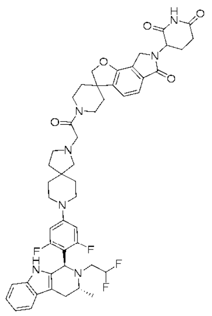 3-(1'-(2-(8-(4-((1R,3R)-2-(2,2-difluoroethyl)-3-methyl-2,3,4,9-tetrahydro-1H-pyrido[3,4-b]indol-1-yl)-3,5-difluorophenyl)-2,8-diazaspiro[4.5]decan-2-yl)acetyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 10.98 (s, 1H), 10.58 (s, 1H), 7.39 (d, *J* = 7.7 Hz, 1H), 7.36 (d, *J* = 7.8 Hz, 1H), 7.30 (d, *J* = 7.6 Hz, 1H), 7.20 (d, *J* = 8.0 Hz, 1H), 7.03 – 6.97 (m, 1H), 6.94 (t, *J* = 7.3 Hz, 1H), 6.57 (d, *J* = 13.4 Hz, 2H), 5.86 (t, *J* = 4.3 Hz, 1H), 5.10 (d, *J* = 23.9 Hz, 2H), 4.70 – 4.59 (m, 2H), 4.43 – 4.33 (m, 3H), 4.23 (dd, *J* = 17.1, 9.4 Hz, 2H), 4.10 (s, 1H), 3.38 (dt, *J* = 12.1, 6.8 Hz, 1H), 3.22 (d, *J* = 39.2 Hz, 5H), 3.10 – 3.00 (m, 2H), 2.96 – 2.87 (m, 1H), 2.82 (dd, *J* = 15.1, 4.6 Hz, 2H), 2.73 (d, *J* = 29.3 Hz, 2H), 2.64 – 2.54 (m, 3H), 2.45 – 2.37 (m, 2H), 2.04 – 1.96 (m, 2H), 1.86 (s, 1H), 1.76 (s, 2H), 1.62 (s, 4H), 1.24 (s, 3H), 1.10 (d, *J* = 6.6 Hz, 2H), 0.85 (q, *J* = 8.0, 7.4 Hz, 1H). | 896.60 | General Synthesis Formula 57 |
| **104** | A112 | 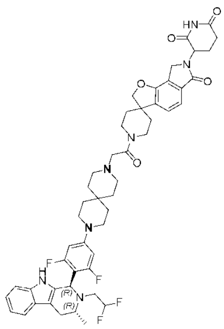 3-(1'-(2-(9-(4-((1R,3R)-2-(2,2-difluoroethyl)-3-methyl-2,3,4,9-tetrahydro-1H-pyrido[3,4-b]indol-1-yl)-3,5-difluorophenyl)-3,9-diazaspiro[5.5]undecan-3-yl)acetyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 10.98 (s, 1H), 10.58 (s, 1H), 7.42 – 7.34 (m, 2H), 7.29 (d, *J* = 7.4 Hz, 1H), 7.20 (d, *J* = 8.1 Hz, 1H), 6.97 (dt, *J* = 33.8, 7.5 Hz, 2H), 6.57 (dd, *J* = 25.8, 13.4 Hz, 2H), 5.92 – 5.80 (m, 1H), 5.10 (d, *J* = 22.6 Hz, 2H), 4.64 (q, *J* = 9.6, 9.0 Hz, 2H), 4.38 (dd, *J* = 28.0, 15.6 Hz, 3H), 4.23 (dd, *J* = 17.0, 6.9 Hz, 1H), 4.09 (s, 1H), 3.18 (d, *J* = 31.3 Hz, 5H), 3.10 – 2.98 (m, 2H), 2.96 – 2.80 (m, 2H), 2.71 (d, *J* = 36.8 Hz, 2H), 2.64 – 2.54 (m, 3H), 2.47 – 2.36 (m, 4H), 2.26 (t, *J* = 6.7 Hz, 1H), 2.00 (td, *J* = 13.5, 11.9, 6.8 Hz, 2H), 1.90 (d, *J* = 17.5 Hz, 2H), 1.80 – 1.69 (m, 2H), 1.49 (s, 4H), 1.25 (d, *J* = 8.3 Hz, 3H), 1.10 (d, *J* = 6.6 Hz, 2H), 0.91 – 0.76 (m, 2H). | 910.65 | General Synthesis Formula 57 |

| | | | | | |
|---|---|---|---|---|---|
| **105** | A113 | 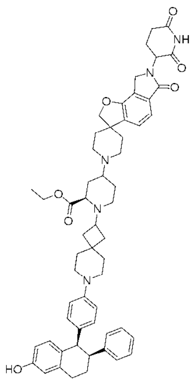<br>(2R)-4-(7-(2,6-Dioxopiperidin-3-yl)-6-oxo-7,8-dihydro-2H,6H-spiro[furo[2,3-e]isoindole-3,4'-piperidin]-1'-yl)-1-(7-(4-((1R,2S)-6-hydroxy-2-phenyl-1,2,3,4-tetrahydronaphthalen-1-yl)phenyl)-7-azaspiro[3.5]nonan-2-yl)piperidine-2-carboxylic acid ethyl ester | $^{1}$H NMR (600 MHz, DMSO-$d_6$) δ 10.99 (s, 1H), 9.13 (s, 1H), 7.42 – 7.26 (m, 2H), 7.21 – 7.09 (m, 3H), 6.88 – 6.81 (m, 2H), 6.75 – 6.57 (m, 3H), 6.49 (dd, *J* = 8.3, 2.5 Hz, 2H), 6.30 (d, *J* = 63.1 Hz, 2H), 5.10 (dd, *J* = 13.3, 5.1 Hz, 1H), 4.66 (q, *J* = 7.7, 6.6 Hz, 2H), 4.40 (d, *J* = 17.2 Hz, 1H), 4.33 – 4.06 (m, 4H), 3.86 (d, *J* = 28.9 Hz, 1H), 3.70 (s, 1H), 3.34 – 2.87 (m, 12H), 2.85 – 2.55 (m, 5H), 2.47 – 2.29 (m, 3H), 2.23 – 1.96 (m, 10H), 1.95 – 1.42 (m, 7H), 1.25 (d, *J* = 6.4 Hz, 3H). | 932.45 | General Synthesis Formula 6 |
| **106** | A114 | 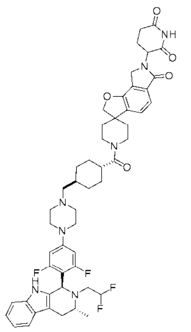<br>3-(1'-((1R,4R)-4-((4-((1R,3R)-2-(2,2-difluoroethyl)-3-methyl-2,3,4,9-tetrahydro-1H-pyrido[3,4-b]indol-1-yl)-3,5-difluorophenyl)piperazin-1-yl)methyl)cyclohexane-1-carbonyl)-6-oxo-6,8-dihydro-2H,7H-spiro[furo[2,3-e]isoindole-3,4'-piperidin]-7-yl)piperidine-2,6-dione | $^{1}$H NMR (600 MHz, DMSO-$d_6$) δ 10.98 (s, 1H), 10.58 (s, 1H), 7.41 (dd, *J* = 15.8, 7.7 Hz, 2H), 7.28 (d, *J* = 7.6 Hz, 1H), 7.22 – 7.17 (m, 1H), 7.03 – 6.91 (m, 2H), 6.70 – 6.48 (m, 2H), 5.86 (t, *J* = 56.4 Hz, 1H), 5.19 – 5.03 (m, 2H), 4.64 (dt, *J* = 23.3, 9.8 Hz, 2H), 4.40 (d, *J* = 16.2 Hz, 2H), 4.23 (dd, *J* = 16.9, 5.5 Hz, 1H), 3.96 (d, *J* = 11.1 Hz, 1H), 3.19 (s, 5H), 3.10 – 2.81 (m, 2H), 2.74 – 2.54 (m, 4H), 2.41 (d, *J* = 20.7 Hz, 4H), 2.13 (d, *J* = 7.0 Hz, 2H), 2.04 – 1.67 (m, 10H), 1.55 – 1.43 (m, 2H), 1.39 – 1.22 (m, 5H), 1.10 (d, *J* = 6.6 Hz, 2H), 1.03 – 0.80 (m, 2H). | 924.70 | General Synthesis Formula 57 |
| **107** | A115 | 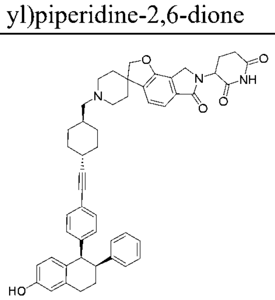 | $^{1}$H NMR (600 MHz, DMSO-$d_6$) δ 10.99 (s, 1H), 9.18 (s, 1H), 7.35 (dd, *J* = 13.6, 7.7 Hz, 1H), 7.27 (d, *J* = 7.6 Hz, 1H), 7.20 – 7.10 (m, 3H), 7.00 – 6.95 (m, 2H), 6.89 – 6.80 (m, 2H), 6.70 – 6.61 (m, 2H), 6.50 (dd, *J* = 8.3, 2.6 Hz, 1H), 6.35 (d, *J* = 8.1 Hz, 2H), 5.10 (dd, *J* = 13.2, 5.1 Hz, 1H), | 774.60 | General Synthesis Formula 38 |

| | | | | | |
|---|---|---|---|---|---|
| | | 3-(1'-(((1R,4r)-4-((4-((1R,2S)-6-hydroxy-2-phenyl-1,2,3,4-tetrahydronaphthalen-1-yl)phenyl)ethynyl)cyclohexyl)methyl)-6-oxo-6,8-dihydro-2H,7H-spiro[furo[2,3-e]isoindole-3,4'-piperidin]-7-yl)piperidine-2,6-dione | 4.65 (d, $J$ = 5.4 Hz, 1H), 4.40 (d, $J$ = 17.1 Hz, 1H), 4.26 (s, 1H), 3.55 (s, 1H), 3.23 (s, 2H), 3.09 – 2.86 (m, 6H), 2.64 – 2.57 (m, 1H), 2.46 – 2.33 (m, 2H), 2.20 (t, $J$ = 13.8 Hz, 2H), 2.11 – 1.90 (m, 6H), 1.78 (dd, $J$ = 29.3, 10.6 Hz, 4H), 1.41 (q, $J$ = 12.3 Hz, 2H), 1.24 (s, 2H), 1.06 (d, $J$ = 12.7 Hz, 2H), 0.86 (t, $J$ = 6.9 Hz, 1H). | | |
| **108** | A116 | 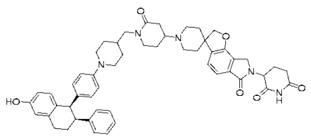 3-(1'-(1-(1-(1-(4-((1R,2S)-6-hydroxy-2-phenyl-1,2,3,4-tetrahydronaphthalen-1-yl)phenyl)piperidin-4-yl)methyl)-2-oxopiperidin-4-yl)-6-oxo-6,8-dihydro-2H,7H-spiro[furo[2,3-e]isoindole-3,4'-piperidin]-7-yl)piperidine-2,6-dione | $^{1}$H NMR (600 MHz, DMSO-$d_6$) δ 11.00 (s, 1H), 9.14 (s, 1H), 7.96 – 7.71 (m, 1H), 7.38 – 7.21 (m, 2H), 7.18 – 7.08 (m, 3H), 6.83 (t, $J$ = 6.1 Hz, 2H), 6.69 – 6.53 (m, 3H), 6.49 (d, $J$ = 8.3 Hz, 1H), 6.25 (s, 2H), 5.10 (dd, $J$ = 13.3, 5.4 Hz, 1H), 4.64 (tt, $J$ = 21.1, 11.9 Hz, 2H), 4.44 – 4.35 (m, 1H), 4.33 – 4.12 (m, 2H), 3.47 (d, $J$ = 58.6 Hz, 18H), 3.04 – 2.58 (m, 5H), 2.47 – 1.86 (m, 6H), 1.85 – 1.12 (m, 6H). | 848.60 | General Synthesis Formula 32 |
| **109** | A117 | 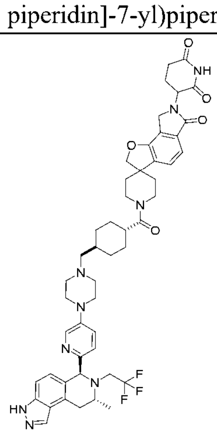 3-(1'-((1S,4R)-4-((4-(6-((6S,8R)-8-methyl-7-(2,2,2-trifluoroethyl)-6,7,8,9-tetrahydro-3H-pyrazolo[4,3-f]isoquinolin-6-yl)pyridin-3-yl)piperazin-1-yl)methyl)cyclohexane-1-carbonyl)-6-oxo-6,8-dihydro-2H,7H-spiro[furo[2,3-e]isoindole-3,4'-piperidin]-7-yl)piperidine-2,6-dione | $^{1}$H NMR (600 MHz, DMSO-$d_6$) δ 13.02 (s, 1H), 9.25 (s, 1H), 8.22 (d, $J$ = 2.9 Hz, 1H), 8.09 (s, 1H), 7.51 – 7.38 (m, 2H), 7.32 – 7.16 (m, 3H), 6.85 (d, $J$ = 8.6 Hz, 1H), 5.16 – 5.03 (m, 2H), 4.65 (dt, $J$ = 21.3, 9.7 Hz, 2H), 4.40 (d, $J$ = 14.8 Hz, 2H), 4.23 (dd, $J$ = 17.0, 11.8 Hz, 1H), 3.92 (dd, $J$ = 73.0, 12.5 Hz, 3H), 3.59 (d, $J$ = 20.8 Hz, 1H), 3.39 (s, 1H), 3.22 – 2.85 (m, 9H), 2.78 – 2.57 (m, 3H), 2.47 – 2.31 (m, 1H), 2.01 – 1.61 (m, 12H), 1.55 – 1.38 (m, 2H), 1.35 – 1.18 (m, 1H), 1.11 (d, $J$ = 6.6 Hz, 5H). | 908.65 | General Synthesis Formula 59 |

| | | | | | |
|---|---|---|---|---|---|
| **110** | A118 | 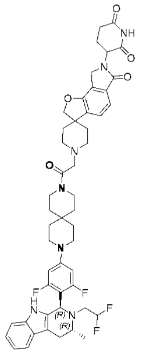<br>3-(1'-(2-(9-(4-((1R,3R)-2-(2,2-difluoroethyl)-3-methyl-2,3,4,9-tetrahydro-1H-pyrido[3,4-b]indol-1-yl)-3,5-difluorophenyl)-3,9-diazaspiro[5.5]undecan-3-yl)-2-oxoethyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 10.99 (s, 1H), 10.59 (s, 1H), 7.39 (dd, $J$ = 12.0, 7.7 Hz, 2H), 7.29 (d, $J$ = 7.4 Hz, 1H), 7.21 (d, $J$ = 8.1 Hz, 1H), 7.03 – 6.91 (m, 2H), 6.66 – 6.53 (m, 2H), 6.00 – 5.74 (m, 1H), 5.17 – 5.05 (m, 2H), 4.54 (s, 2H), 4.38 (d, $J$ = 17.0 Hz, 1H), 4.22 (d, $J$ = 17.0 Hz, 1H), 3.47 (t, $J$ = 47.0 Hz, 4H), 3.22 (d, $J$ = 28.9 Hz, 10H), 3.11 – 3.01 (m, 1H), 2.96 – 2.78 (m, 3H), 2.75 – 2.55 (m, 2H), 2.46 – 2.37 (m, 1H), 2.15 (s, 2H), 2.04 – 1.82 (m, 3H), 1.73 (d, $J$ = 37.8 Hz, 2H), 1.54 (d, $J$ = 22.5 Hz, 5H), 1.40 (s, 2H), 1.33 – 1.20 (m, 2H), 1.10 (d, $J$ = 6.6 Hz, 2H). | 910.65 | General Synthesis Formula 58 |
| **111** | A119 | 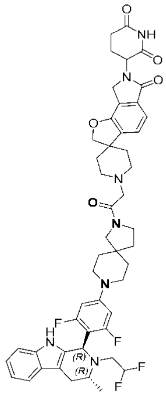<br>3-(1'-(2-(8-(4-((1R,3R)-2-(2,2-difluoroethyl)-3-methyl-2,3,4,9-tetrahydro-1H-pyrido[3,4-b]indol-1-yl)-3,5-difluorophenyl)-2,8-diazaspiro[4.5]decan-2-yl)-2-oxoethyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 10.98 (s, 1H), 10.60 (s, 1H), 7.39 (td, $J$ = 19.4, 16.8, 7.2 Hz, 2H), 7.28 (t, $J$ = 7.6 Hz, 1H), 7.21 (d, $J$ = 8.0 Hz, 1H), 6.97 (dt, $J$ = 33.6, 7.3 Hz, 2H), 6.60 (dd, $J$ = 21.7, 13.3 Hz, 2H), 5.99 – 5.76 (m, 1H), 5.17 – 5.06 (m, 2H), 4.53 (s, 2H), 4.38 (d, $J$ = 17.0 Hz, 1H), 4.22 (d, $J$ = 17.0 Hz, 1H), 3.66 – 3.41 (m, 2H), 3.34 (s, 12H), 3.27 – 2.98 (m, 2H), 2.96 – 2.80 (m, 1H), 2.78 – 2.52 (m, 2H), 2.46 – 2.35 (m, 1H), 2.30 – 2.13 (m, 2H), 2.07 – 1.80 (m, 3H), 1.78 – 1.50 (m, 6H), 1.50 – 1.14 (m, 3H), 1.09 (dd, $J$ = 9.4, 6.6 Hz, 2H). | 896.60 | General Synthesis Formula 58 |
| **112** | A120 | 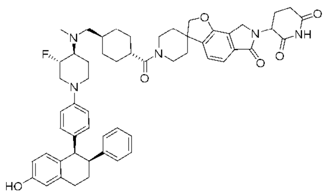<br>3-(1'-((1S,4R)-4-((((3S,4S)-3-fluoro-1-(4-((1R,2S)-6-hydroxy-2-phenyl- | $^1$H NMR (400 MHz, DMSO-$d_6$) δ 11.00 (s, 1H), 9.11 (s, 1H), 7.42 (d, $J$ = 7.6 Hz, 1H), 7.28 (d, $J$ = 7.6 Hz, 1H), 7.19 – 7.08 (m, 3H), 6.89 – 6.79 (m, 2H), 6.63 (dq, $J$ = 9.0, 3.2, 2.7 Hz, 4H), 6.48 (dd, $J$ = 8.3, 2.6 Hz, 1H), 6.30 – 6.19 (m, 2H), 5.18 – 4.93 (m, 2H), 4.63 (q, $J$ = 9.5, 8.1 Hz, | 908.70 | General Synthesis Formula 34 |

| | | | | | |
|---|---|---|---|---|---|
| | | 1,2,3,4-tetrahydronaphthalen-1-yl)phenyl)piperidin-4-yl)(methyl)amino)methyl)cyclohexane-1-carbonyl)-6-oxo-6,8-dihydro-2H,7H-spiro[furo[2,3-e]isoindole-3,4'-piperidin]-7-yl)piperidine-2,6-dione | 2H), 4.47 – 4.32 (m, 2H), 4.28 – 4.12 (m, 2H), 3.96 (s, 5H), 3.37 – 3.09 (m, 3H), 3.03 – 2.55 (m, 10H), 2.47 – 2.27 (m, 1H), 2.19 – 1.91 (m, 3H), 1.73 (s, 12H), 1.60 – 0.94 (m, 4H). | | |
| **113** | A121 | 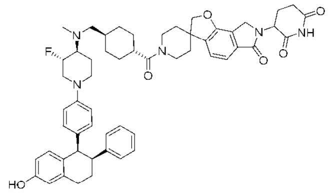 3-(1'-((1S,4R)-4-((((3S,4S)-3-fluoro-1-(4-((1R,2S)-6-hydroxy-2-phenyl-1,2,3,4-tetrahydronaphthalen-1-yl)phenyl)piperidin-4-yl)(methyl)amino)methyl)cyclohexane-1-carbonyl)-6-oxo-6,8-dihydro-2H,7H-spiro[furo[2,3-e]isoindole-3,4'-piperidin]-7-yl)piperidine-2,6-dione | $^1$H NMR (400 MHz, DMSO-$d_6$) δ 11.00 (s, 1H), 9.12 (s, 1H), 7.42 (d, *J* = 7.6 Hz, 1H), 7.28 (d, *J* = 7.6 Hz, 1H), 7.19 – 7.06 (m, 3H), 6.87 – 6.79 (m, 2H), 6.63 (dq, *J* = 9.7, 3.0 Hz, 4H), 6.48 (dd, *J* = 8.3, 2.5 Hz, 1H), 6.24 (d, *J* = 8.2 Hz, 2H), 5.20 – 4.96 (m, 2H), 4.63 (q, *J* = 9.4, 8.1 Hz, 2H), 4.39 (dd, *J* = 16.7, 3.4 Hz, 2H), 4.29 – 4.12 (m, 2H), 3.97 (s, 7H), 3.38 – 3.09 (m, 3H), 3.06 – 2.55 (m, 9H), 2.46 – 2.25 (m, 1H), 2.21 – 1.90 (m, 3H), 1.73 (s, 11H), 1.59 – 0.89 (m, 4H). | 908.70 | General Synthesis Formula 34 |
| **114** | A122 | 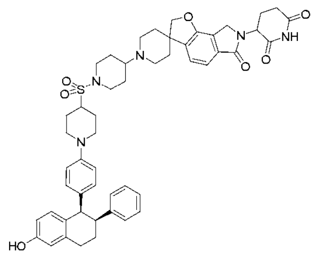 3-(1'-(1-((1-(4-((1R,2S)-6-hydroxy-2-phenyl-1,2,3,4-tetrahydronaphthalen-1-yl)phenyl)piperidin-4-yl)sulfonyl)piperidin-4-yl)-6-oxo-6,8-dihydro-2H,7H-spiro[furo[2,3-e]isoindole-3,4'-piperidin]-7-yl)piperidine-2,6-dione | $^1$H NMR (400 MHz, DMSO-$d_6$) δ 11.00 (s, 1H), 9.45 (s, 1H), 7.37 – 7.26 (m, 2H), 7.14 (d, *J* = 7.3 Hz, 2H), 6.87 – 6.79 (m, 2H), 6.68 – 6.54 (m, 5H), 6.48 (dd, *J* = 8.3, 2.5 Hz, 1H), 6.23 (d, *J* = 8.2 Hz, 2H), 5.10 (dd, *J* = 13.3, 5.1 Hz, 1H), 4.67 (d, *J* = 2.6 Hz, 1H), 4.40 (d, *J* = 17.3 Hz, 1H), 4.24 (d, *J* = 17.2 Hz, 1H), 4.15 (d, *J* = 5.0 Hz, 1H), 3.84 – 3.77 (m, 3H), 3.70 – 3.62 (m, 4H), 3.31 (ddd, *J* = 16.5, 12.1, 4.1 Hz, 1H), 3.16 – 2.87 (m, 7H), 2.69 – 2.56 (m, 3H), 2.46 – 2.30 (m, 1H), 2.21 – 1.92 (m, 11H), 1.76 – 1.56 (m, 5H), 1.24 (s, 1H). | 884.70 | General Synthesis Formula 21 |

| | | | | | |
|---|---|---|---|---|---|
| **115** | A123 | 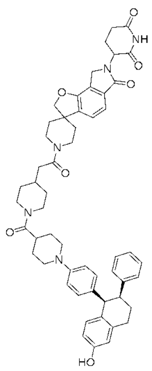 3-(1'-(2-(1-(1-(4-((1R,2S)-6-hydroxy-2-phenyl-1,2,3,4-tetrahydronaphthalen-1-yl)phenyl)piperidine-4-carbonyl)piperidin-4-yl)acetyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 10.99 (s, 1H), 9.18 (s, 1H), 7.40 (t, $J$ = 6.9 Hz, 1H), 7.28 (d, $J$ = 7.6 Hz, 1H), 7.22 – 7.09 (m, 3H), 6.98 (d, $J$ = 39.7 Hz, 1H), 6.87 – 6.75 (m, 2H), 6.74 – 6.59 (m, 2H), 6.57 – 6.28 (m,4H), 5.09 (dt, $J$ = 13.4, 4.8 Hz, 1H), 4.63 (q, $J$ = 9.1 Hz, 2H), 4.40 (dt, $J$ = 17.1, 4.6 Hz, 3H), 4.23 (dd, $J$ = 17.1, 7.8 Hz, 2H), 3.93 (t, $J$ = 14.6 Hz, 2H), 3.52 (d, $J$ = 9.1 Hz, 3H), 3.44 – 3.33 (m, 1H), 3.23 – 2.84 (m, 6H), 2.77 – 2.54 (m, 3H), 2.46 – 2.27 (m, 3H), 2.15 – 1.65 (m, 15H), 1.32 – 0.83 (m, 2H). | 890.70 | General Synthesis Formula 14 |
| **116** | A124 | 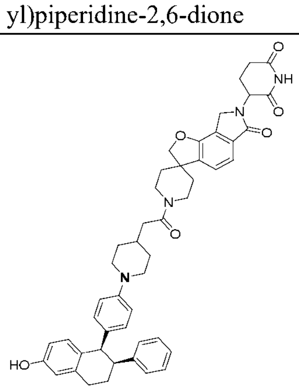 3-(1'-(2-(1-(4-((1R,2S)-6-hydroxy-2-phenyl-1,2,3,4-tetrahydronaphthalen-1-yl)phenyl)piperidin-4-yl)acetyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 11.00 (s, 1H), 9.13 (s, 1H), 7.41 (d, $J$ = 7.6 Hz, 1H), 7.27 (d, $J$ = 7.6 Hz, 1H), 7.19 – 7.08 (m, 3H), 6.84 (dd, $J$ = 7.1, 1.7 Hz, 2H), 6.71 – 6.44 (m, 5H), 6.20 (d, $J$ = 8.5 Hz, 2H), 5.10 (dt, $J$ = 13.5, 4.3 Hz, 1H), 4.63 (p, $J$ = 9.2 Hz, 2H), 4.43 – 4.34 (m, 2H), 4.28 – 4.08 (m, 2H), 3.93 (d, $J$ = 12.9 Hz, 1H), 3.51 (s, 2H), 3.30 – 3.26 (m, 1H), 3.16 (t, $J$ = 13.0 Hz, 1H), 3.03 – 2.84 (m, 3H), 2.72 (t, $J$ = 11.9 Hz, 1H), 2.66 – 2.56 (m, 1H), 2.47 – 2.24 (m, 3H), 2.20 – 2.05 (m, 1H), 2.03 – 1.62 (m, 10H), 1.24 (t, $J$ = 13.0 Hz, 3H). | 779.60 | General Synthesis Formula 10 |
| **117** | B1 | 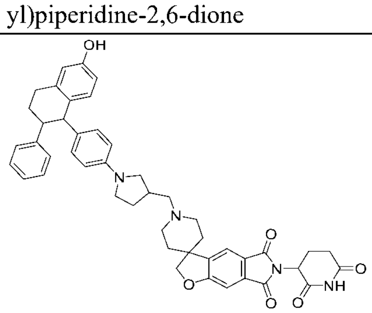 6-(2,6-Dioxopiperidin-3-yl)-1'-((1-(4-(-6-hydroxy-2-phenyl-1,2,3,4-tetrahydronaphthalen-1- | $^1$H NMR (600 MHz, DMSO-$d_6$) δ 11.12 (s, 1H), 9.24 (s, 1H), 9.11 (s, 1H), 7.63 (s, 1H), 7.38 – 7.35 (m, 1H), 7.17 – 7.11 (m, 3H), 6.86 (d, $J$ = 7.3 Hz, 2H), 6.64 – 6.60 (m, 2H), 6.48 (dd, $J$ = 8.2, 2.5 Hz, 1H), 6.22 – 6.18 (m, 4H), 5.13 – 5.10 (m, 1H), 4.75 – 4.72 (m, 2H), 4.13 (d, $J$ = 4.9 Hz, 1H), 3.65 – 3.61 (m, 2H), 3.38 – 3.35 (s, 2H), 3.30 – 3.19 (m, 4H), 3.16 – 3.08 (m, 2H), 3.01 – 2.86 (m, 3H), 2.77 – 2.70 (m, | 751.63 | General Synthesis Formula 1 |

| | | | | | |
|---|---|---|---|---|---|
| | | yl)phenyl)pyrrolidin-3-yl)methyl)-6-hydro-2H,5H-spiro[furo[2,3-f]isoindole-3,4'-piperidine]-5,7-dione | 1H), 2.62 – 2.53 (m, 2H), 2.29 – 2.14 (m, 3H), 2.13 – 2.08 (m, 1H), 2.06 – 1.98 (m, 2H), 1.90 – 1.88 (m, 1H), 1.78 – 1.71 (m, 2H). | | |
| **118** | B2 | 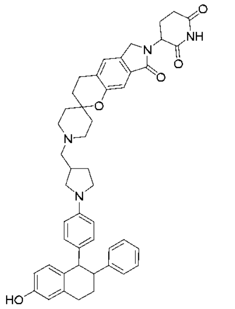 3-(1-((1-(4-(-6-hydroxy-2-phenyl-1,2,3,4-tetrahydronaphthalen-1-yl)phenyl)pyrrolidin-3-yl)methyl)-8'-oxo-3',4',6',8'-tetrahydro-7'-spiro[piperidine-4,2'-pyrano[2,3-f]isoindole]-7'-yl)piperidine-2,6-dione | $^{1}$H NMR (400 MHz, DMSO-$d_6$) δ 10.96 (s, 1H), 9.21 (s, 1H), 9.10 (s, 1H), 7.35 (s, 1H), 7.24 (s, 1H), 7.20 – 7.06 (m, 3H), 6.86 – 6.4 (m, 2H), 6.63 – 6.59 (m, 2H), 6.47 (dd, $J$ = 8.2, 2.5 Hz, 1H), 6.21 – 6.15 (m, 4H), 5.06 (dd, $J$ = 13.3, 5.1 Hz, 1H), 4.34 (d, $J$ = 16.7 Hz, 1H), 4.22 (d, $J$ = 16.6 Hz, 1H), 4.12 (d, $J$ = 4.9 Hz, 1H), 3.54 – 3.48 (m, 2H), 3.29 – 3.17 (m, 7H), 3.13 – 3.07 (m, 1H), 2.99 – 2.84 (m, 6H), 2.75 – 2.69 (m, 1H), 2.63 – 2.55 (m, 1H), 2.44 – 2.32 (m, 2H), 2.16 – 2.05 (m, 2H), 2.00 – 1.86 (m, 5H), 1.73 – 1.68 (m, 2H). | 751.69 | General Synthesis Formula 1 |
| **119** | B3 | 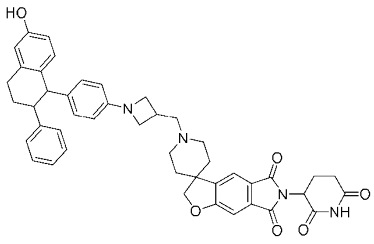 6-(2,6-Dioxopiperidin-3-yl)-1'-((1-(4-((1R,2S)-6-hydroxy-2-phenyl-1,2,3,4-tetrahydronaphthalen-1-yl)phenyl)azetidin-3-yl)methyl)-6-hydro-2H,5H-spiro[2,3-f]isoindole-3,4'-piperidine]-5,7-dione | $^{1}$H NMR (400 MHz, DMSO-$d_6$) δ 11.10 (s, 1H), 9.35 (s, 1H), 9.11 (s, 1H), 7.64 (s, 1H), 7.36-7.33 (m, 1H), 7.21 – 7.09 (m, 3H), 6.84 – 6.82 (m, 2H), 6.63 – 6.60 (m, 2H), 6.48 (dd, $J$ = 8.3, 2.5 Hz, 1H), 6.21 (d, $J$ = 8.1 Hz, 2H), 6.08 – 6.06 (m, 2H), 5.10 (dd, $J$ = 12.8, 5.4 Hz, 1H), 4.73 – 4.68 (m, 2H), 4.13 (d, $J$ = 5.0 Hz, 1H), 3.93 – 3.88 (m, 2H), 3.74 (s, 1H), 3.50 – 3.43 (m, 2H), 3.17 – 3..95 (m, 5H), 2.99 – 2.84 (m, 3H), 2.67 – 2.57 (m, 3H), 2.33 – 2.32 (m, 1H), 2.18 – 1.99 (m, 4H), 1.91 – 1.85 (m, 1H), 1.72 – 1.69 (m, 1H). | 737.30 | General Synthesis Formula 1 |
| **120** | B4 | 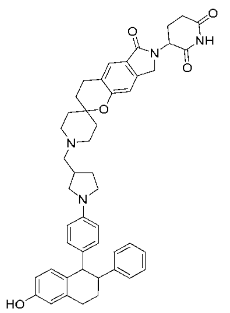 3-(1-((1-(4-(6-hydroxy-2-phenyl-1,2,3,4-tetrahydronaphthalen-1-yl)phenyl)pyrrolidin-3-yl)methyl)-6'- | $^{1}$H NMR (600 MHz, DMSO-$d_6$) δ 10.97 (s, 1H), 9.23 (s, 1H), 9.11 (s, 1H), 7.52 – 7.51 (m, 1H), 7.21 – 7.09 (m, 3H), 7.05 – 7.03 (m, 1H), 6.85 (d, $J$ = 7.4 Hz, 2H), 6.65 – 6.60 (m, 2H), 6.48 (dd, $J$ = 8.3, 2.5 Hz, 1H), 6.21 – 6.17 (m, 4H), 5.07 (dd, $J$ = 13.3, 5.1 Hz, 1H), 4.37 – 4.32 (m, 1H), 4.24 – 4.20 (m, 1H), 4.13 (d, $J$ = 4.9 Hz, 1H), 3.54 – 3.48 (m, 2H), 3.40 – 3.27 (m, 5H), 3.25 – 3.14 (m, 3H), 3.13 – 3.09 (m, 1H), 3.01 – 2.87 (m, 6H), 2.76 – 2.69 (m, | 751.63 | General Synthesis Formula 1 |

| | | | | | |
|---|---|---|---|---|---|
| | | oxo-3',4',6',8'-tetrahydro-7'-spiro[piperidine-4,2'-pyrano[2,3-f]isoindole]-7'-yl)piperidine-2,6-dione | 1H), 2.62 – 2.58 (m, 1H), 2.40 – 2.33 (m, 1H), 2.19 – 2.04 (m, 3H), 2.02 – 1.94 (m, 3H), 1.90 – 1.87 (m, 3H), 1.73 – 1.70 (m, 2H) | | |
| **121** | B5 | 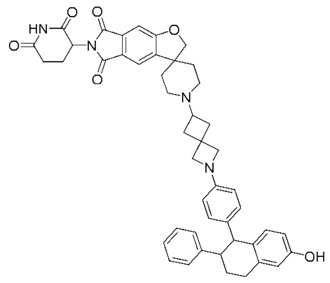 6-(2,6-Dioxopiperidin-3-yl)-1'-(2-(4-(6-hydroxy-2-phenyl-1,2,3,4-tetrahydronaphthalen-1-yl)phenyl)-2-azaspiro[3.3]heptan-6-yl)-6-hydro-2H,5H-spiro[furo[2,3-f]isoindole-3,4'-piperidine]-5,7-dione | $^1$H NMR (600 MHz, DMSO-$d_6$) δ 11.12 (s, 1H), 9.68 – 9.66 (m, 1H), 9.12 (s, 1H), 7.66 (s, 1H), 7.37 – 7.35 (m, 1H), 7.16 – 7.10 (m, 3H), 6.83 (d, $J$ = 7.3 Hz, 2H), 6.63 – 6.60 (m, 2H), 6.48 (dd, $J$ = 8.3, 2.5 Hz, 1H), 6.20 – 6.18 (m, 2H), 6.08 – 6.06 (m, 2H), 5.13 – 5.10 (m, 1H), 4.73 – 4.70 (m, 2H), 4.12 (d, $J$ = 4.9 Hz, 1H), 3.79 – 3.72 (m, 2H), 3.68 – 3.64 (m, 2H), 3.30 – 3.26 (m, 2H), 3.21 – 3.17 (m, 1H), 2.99 – 2.86 (m, 5H), 2.63 – 2.57 (m, 1H), 2.55 – 2.53 (m, 2H), 2.46 – 2.43 (m, 2H), 2.39 – 2.35 (m, 1H), 2.26 – 2.22 (m, 1H), 2.12 – 2.02 (m, 4H), 1.83 (d, $J$ = 14.4 Hz, 1H), 1.72 – 1.69 (m, 1H). | 763.68 | General Synthesis Formula 6 |
| **122** | B7 | 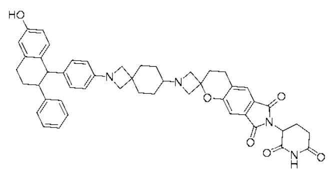 7'-(2,6-Dioxopiperidin-3-yl)-1-(2-(4-(6-hydroxy-2-phenyl-1,2,3,4-tetrahydronaphthalen-1-yl)phenyl)-2-azaspiro[3.5]nonan-7-yl)-3',4'-dihydro-6'H-spiro[azetidine-3,2'-pyrano[2,3-f]isoindole]-6',8'-7'H-dione | $^1$H NMR (600 MHz, DMSO-$d_6$) δ 11.13 (d, $J$ = 5.8 Hz, 1H), 9.09 (s, 1H), 7.79 (s, 1H), 7.34 (d, $J$ = 1.8 Hz, 1H), 7.16 – 7.09 (m, 3H), 6.86 – 6.85 (m, 2H), 6.64 (dd, $J$ = 8.4, 3.4 Hz, 1H), 6.59 (d, $J$ = 2.5 Hz, 1H), 6.47 – 6.46 (m, 1H), 6.25 (d, $J$ = 8.2 Hz, 2H), 6.14 (d, $J$ = 8.2 Hz, 2H), 5.16 – 5.12 (m, 1H), 4.86 (d, $J$ = 12.2 Hz, 2H), 4.50 – 4.46 (m, 2H), 4.14 (d, $J$ = 4.9 Hz, 1H), 3.91 – 3.81 (m, 2H), 3.71 (t, $J$ = 2.9 Hz, 1H), 3.28 – 3.24 (m, 1H), 2.99 – 2.87 (m, 5H), 2.78 (s, 2H), 2.64 – 2.55 (m, 2H), 2.20 – 2.18 (m, 2H), 2.12 – 2.05 (m, 2H), 1.95 – 1.90 (m, 2H), 1.78 – 1.69 (m, 3H), 1.52 – 1.40 (m, 4H). | 777.69 | General Synthesis Formula 6 |

| | | | | | |
|---|---|---|---|---|---|
| **123** | B8 | 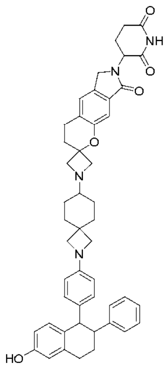 3-(1-(2-(4-(6-Hydroxy-2-phenyl-1,2,3,4-tetrahydronaphthalen-1-yl)phenyl)-2-azaspiro[3.5]nonan-7-yl)-8'-oxo-3',4',6',8'-tetrahydro-7'H-spiro[azetidine-3,2'-pyrano[2,3-f]isoindol]-7'-yl)piperidine-2,6-dione | $^1$H NMR (600 MHz, DMSO-$d_6$) δ 10.98 (s, 1H), 9.11 (s, 1H), 7.38 (d, $J$ = 3.1 Hz, 1H), 7.16 – 7.09 (m, 4H), 6.83 (d, $J$ = 7.4 Hz, 2H), 6.63 – 6.60 (m, 2H), 6.48 (dd, $J$ = 8.4, 2.5 Hz, 1H), 6.17 (d, $J$ = 8.1 Hz, 2H), 6.05 (d, $J$ = 8.1 Hz, 2H), 5.08 (dd, $J$ = 13.2, 5.2 Hz, 1H), 4.42 – 4.33 (m, 4H), 4.24 – 4.21 (m, 2H), 4.12 (d, $J$ = 5.1 Hz, 1H), 3.38 – 3.35 (m, 2H), 3.29 – 3.26 (m, 2H), 3.22 – 3.18 (m, 1H), 2.97 – 2.87 (m, 5H), 2.62 – 2.59 (m, 1H), 2.40 – 2.37 (m, 1H), 2.21 – 2.16 (m, 2H), 2.11 – 2.08 (m, 1H), 2.01 – 2.91 (m, 4H), 1.85 – 1.83 (m, 1H), 1.72 – 1.69 (m, 1H), 1.50 – 1.42 (m, 3H), 1.20 – 1.16 (m, 2H). | 763.70 | General Synthesis Formula 6 |
| **124** | B9 | 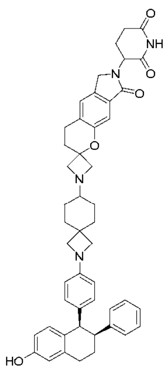 3-(1-(2-(4-((1R,2S)-6-Hydroxy-2-phenyl-1,2,3,4-tetrahydronaphthalen-1-yl)phenyl)-2-azaspiro[3.5]nonan-7-yl)-8'-oxo-3',4',6',8'-tetrahydro-7'H-spiro[azetidine-3,2'-pyrano[2,3-f]isoindol]-7'-yl)piperidine-2,6-dione | $^1$H NMR (400 MHz, DMSO-$d_6$) δ 10.97 (s, 1H), 9.10 (s, 1H), 7.54 (s, 1H), 7.19 – 7.02 (m, 4H), 6.86 – 6.80 (m, 2H), 6.64 – 6.58 (m, 2H), 6.48 (dd, $J$ = 8.3, 2.5 Hz, 1H), 6.18 (d, $J$ = 8.2 Hz, 2H), 6.05 (d, $J$ = 8.3 Hz, 2H), 5.10 – 5.03 (m, 1H), 4.51 – 4.30 (m, 4H), 4.27 – 4.18 (m, 2H), 4.12 (d, $J$ = 4.9 Hz, 1H), 3.43 – 3.36 (m, 3H), 3.30 – 3.26 (m, 2H), 2.98 – 2.82 (m, 5H), 2.69 – 2.56 (m, 2H), 2.42 – 2.30 (m, 2H), 2.20 – 2.16 (m, 2H), 2.12 – 2.07 (m,1H), 1.98 – 1.91 (m, 3H), 1.85 (d, $J$ = 11.4 Hz, 1H), 1.70 (d, $J$ = 11.9 Hz, 1H), 1.52 – 1.38 (m, 2H), 1.17 (d, $J$ = 12.2 Hz, 2H). | 763.70 | General Synthesis Formula 6 |
| **125** | B10 | 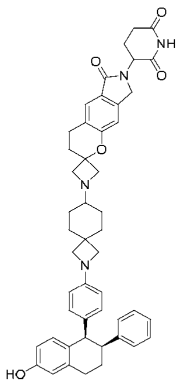 | $^1$H NMR (600 MHz, DMSO-$d_6$) δ 10.98 (d, $J$ = 2.1 Hz, 1H), 9.11 (s, 1H), 7.38 (d, $J$ = 3.1 Hz, 1H), 7.20 – 7.04 (m, 4H), 6.86 – 6.79 (m, 2H), 6.65 – 6.58 (m, 2H), 6.48 (dd, $J$ = 8.3, 2.5 Hz, 1H), 6.18 (d, $J$ = 8.2 Hz, 2H), 6.05 (d, $J$ = 8.1 Hz, 2H), 5.08 (dd, $J$ = 13.3, 5.2 Hz, 1H), 4.45 – 4.29 (m, 4H), 4.26 – 4.17 (m, 2H), 4.12 (d, $J$ = 5.0 Hz, 1H), 3.45 – 3.33 (m, 4H), 3.31 – 3.18 (m, 2H), 3.04 – 2.80 (m, 5H), 2.64 – 2.55 | 763.70 | General Synthesis Formula 6 |

| | | | | | |
|---|---|---|---|---|---|
| | | 3-(1-(2-(4-((1R,2S)-6-Hydroxy-2-phenyl-1,2,3,4-tetrahydronaphthalen-1-yl]phenyl)-2-azaspiro[3.5]nonan-7-yl)-6'-oxo-3',4',6',8'-tetrahydro-7'H-spiro[azetidine-3,2'-pyrano[2,3-f]isoindol]-7'-yl)piperidine-2,6-dione | (m, 1H), 2.40 – 2.35 (m, 1H), 2.22 – 2.16 (m, 2H), 2.12 – 2.05 (m, 1H), 2.03 – 1.89 (m, 4H), 1.84 (d, $J$ = 11.7 Hz, 1H), 1.72 – 1.69 (m, 1H), 1.50 – 1.42 (m, 2H), 1.21 – 1.14 (m, 2H). | | |
| **126** | B11 | 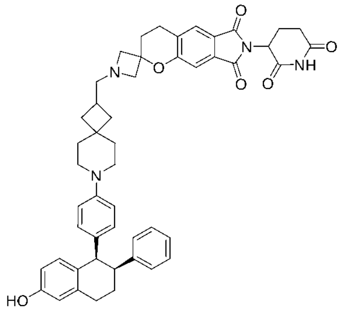 7'-(2,6-Dioxopiperidin-3-yl)-1-((7-(4-(((1R,2S)-6-hydroxy-2-phenyl-1,2,3,4-tetrahydronaphthalen-1-yl)phenyl)-7-azaspiro[3.5]nonan-2-yl)methyl)-3'-dihydro-6'- azetidine-3,2'-pyrano[2,3-f]isoindole]-6',8'-dione | $^1$H NMR (600 MHz, DMSO-$d_6$) δ 11.12 (s, 1H), 7.78 (s, 1H), 7.29 – 7.28 (m, 1H), 7.18 – 7.08 (m, 3H), 6.87 – 6.80 (m, 2H), 6.74 – 6.54 (m, 4H), 6.49 – 6.48 (m, 1H), 6.28 – 6.25 (m, 2H), 5.11 (dd, $J$ = 12.9, 5.4 Hz, 1H), 4.38 (s, 1H), 4.30 (d, $J$ = 6.4 Hz, 2H), 4.25 (d, $J$ = 10.1 Hz, 1H), 4.17 (d, $J$ = 11.7 Hz, 1H), 3.41 – 3.40 (m, 1H), 3.34 – 3.32 (m, 2H), 3.11 – 2.83 (m, 9H), 2.65 – 2.57 (m, 2H), 2.47 – 2.38 (m, 1H), 2.26 – 2.19 (m, 2H), 2.10 – 2.03 (m, 3H), 1.93 – 1.92 (m, 2H), 1.70 – 1.59 (m, 7H). | 791.09 | General Synthesis Formula 12 |
| **127** | B12 | 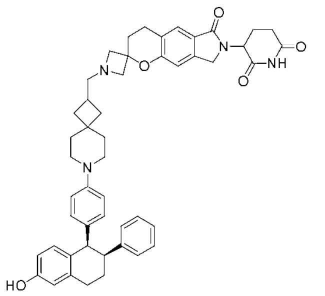 3-(1-((7-(4-((1R,2S)-6-Hydroxy-2-phenyl-1,2,3,4-tetrahydronaphthalen-1-yl)phenyl)-7-azaspiro[3.5]nonan-2-yl)methyl)-6'-oxo-3',4',6',8'-tetrahydro-7'H-spiro[azetidine-3,2'-pyrano[2,3-f]isoindol]-7'-yl)piperidine-2,6-dione | $^1$H NMR (600 MHz, DMSO-$d_6$) δ 10.98 (s, 1H), 9.15 (s, 1H), 7.52 (s, 1H), 7.20 – 7.09 (m, 3H), 7.07 (d, $J$ = 15.7 Hz, 1H), 6.88 – 6.81 (m, 2H), 6.66 – 6.54 (m, 4H), 6.48 (dd, $J$ = 8.3, 2.6 Hz, 1H), 6.23 (d, $J$ = 8.1 Hz, 2H), 5.06 (m, 1H), 4.36 (m, 2H), 4.31 – 4.18 (m, 4H), 4.15 (d, $J$ = 5.0 Hz, 1H), 3.47 – 3.43 (m, 2H), 3.32 – 3.28 (m, 2H), 2.99 – 2.84 (m, 8H), 2.63 – 2.57 (m, 1H), 2.45 – 2.42 (m, 1H), 2.41 – 2.31 (m, 2H), 2.19 (m, 2H), 2.13 – 2.04 (m, 2H), 2.01 – 1.88 (m, 3H), 1.74 – 1.52 (m, 5H). | 777.06 | General Synthesis Formula 12 |

| | | | | | |
|---|---|---|---|---|---|
| **128** | B13 | 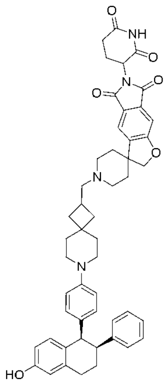 6-(2,6-Dioxopiperidin-3-yl)-1'-((7-(4-(((1R,2S)-6-hydroxy-2-phenyl-1,2,3,4-tetrahydronaphthalen-1-yl)phenyl)-7-azaspiro[3.5]nonan-2-yl)methyl)-6-hydro-2H,5H-spiro[furo[2,3-f]isoindole-3,4'-piperidine]-5,7-dione | $^{1}$H NMR (600 MHz, DMSO-$d_6$) δ 11.12 (s, 1H), 7.63 (s, 1H), 7.36 (d, $J$ = 14.1 Hz, 1H), 7.18 – 7.09 (m, 3H), 6.84 – 6.83 (m, 2H), 6.75 – 6.55 (m, 4H), 6.48 (dd, $J$ = 8.3, 2.5 Hz, 1H), 6.25 (s, 2H), 5.15 – 5.07 (m, 1H), 4.74 – 4.69 (m, 2H), 4.16 (s, 1H), 3.54 – 3.53 (m, 1H), 3.46 – 3.45 (s, 2H), 3.33 – 3.27 (m, 2H), 3.22 (t, $J$ = 6.0 Hz, 1H), 3.11 – 2.83 (m, 9H), 2.71 – 2.65 (s, 1H), 2.62 – 2.61 (m, 1H), 2.24 (s, 1H), 2.20 – 2.10 (m, 2H), 2.09 – 1.96 (m, 4H), 1.88 – 1.86 (d, $J$ = 14.4 Hz, 1H), 1.76 – 1.52 (m, 7H). | 805.26 | General Synthesis Formula 12 |
| **129** | B14 | 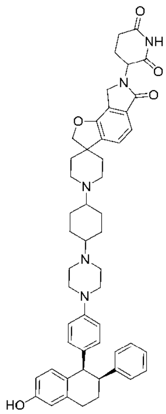 3-(1'-(4-(4-(((1R,2S)-6-Hydroxy-2-phenyl-1,2,3,4-tetrahydronaphthalen-1-yl)phenyl)piperazin-1-yl)cyclohexyl)-6-oxo-6,8-dihydro-2H,7H-spiro[furo[2,3-e]isoindole-3,4'-piperidin]-7-yl)piperidine-2,6-dione | $^{1}$H NMR (600 MHz, DMSO-$d_6$) δ 11.00 (s, 1H), 9.15 (s, 1H), 7.36 (d, $J$ = 7.6 Hz, 1H), 7.29 (d, $J$ = 7.6 Hz, 1H), 7.17 – 7.12 (m, 3H), 6.87 – 6.81 (m, 2H), 6.69 – 6.59 (m, 4H), 6.49 (dd, $J$ = 8.3, 2.5 Hz, 1H), 6.28 (d, $J$ = 8.2 Hz, 2H), 5.10 (dd, $J$ = 13.3, 5.1 Hz, 1H), 4.72 – 4.63 (m, 2H), 4.40 (d, $J$ = 17.2 Hz, 1H), 4.24 (d, $J$ = 17.2 Hz, 1H), 4.18 (d, $J$ = 5.1 Hz, 1H), 3.80 (d, $J$ = 18.4 Hz, 1H), 3.75 – 3.72 (m, 2H), 3.54 – 3.52 (m, 3H), 3.27 (s, 2H), 3.19 – 3.05 (m, 4H), 2.99 – 2.91 (m, 4H), 2.84 (t, $J$ = 12.2 Hz, 2H), 2.65 – 2.55 (m, 1H), 2.45 – 2.36 (m, 2H), 2.25 – 2.17 (m, 6H), 2.11 – 2.07 (s, 1H), 2.05 – 1.94 (m, 3H), 1.74 – 1.71 (m, 1H), 1.58 – 1.52 (m, 3H). | 820.70 | General Synthesis Formula 22 |

| | | | | | |
|---|---|---|---|---|---|
| **130** | B15 | 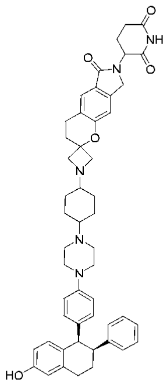 3-(1-(4-(4-(((1R,2S)-6-hydroxy-2-phenyl-1,2,3,4-tetrahydronaphthalen-1-yl)phenyl)piperazin-1-yl)cyclohexyl)-6'-oxo-3',4',6'-tetrahydro-7'H-pyridinazetidine-3,2'-pyrano[2,3-f]isoindole]-7'-yl)piperidine-2,6-dione | $^1$H NMR (600 MHz, DMSO-$d_6$) δ 10.97 (s, 1H), 9.15 (s, 1H), 7.55 (s, 1H), 7.19 – 7.01 (m, 4H), 6.87 – 6.81 (m, 2H), 6.67 – 6.59 (m, 4H), 6.49 (dd, *J* = 8.2, 2.6 Hz, 1H), 6.28 (d, *J* = 8.2 Hz, 2H), 5.07 (dd, *J* = 13.6, 5.0 Hz, 1H), 4.55 – 4.30 (m, 5H), 4.24 (d, *J* = 16.8 Hz, 1H), 4.18 (d, *J* = 5.0 Hz, 1H), 3.80 – 3.67 (m, 2H), 3.61 (s, 1H), 3.32 – 3.29 (m, 3H), 3.18 (s, 2H), 3.02 – 2.81 (m, 7H), 2.64 – 2.57 (m, 1H), 2.42 – 2.31 (m, 1H), 2.26 – 2.21 (m, 2H), 2.13 – 2.04 (m, 2H), 1.98 (s, 3H), 1.83 – 1.69 (m, 7H). | 806. 56 | General Synthesis Formula 22 |
| **131** | B16 | 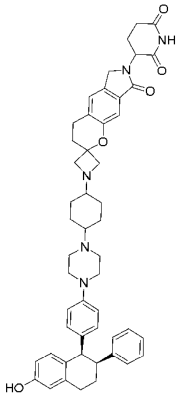 3-(1-(4-(4-((1R,2S)-6-Hydroxy-2-phenyl-1,2,3,4-tetrahydronaphthalen-1-yl)phenyl)piperazin-1-yl)cyclohexyl)-8'-oxo-3',4',6',8'-tetrahydro-7'H-pyridinazetidine-3,2'-pyrano[2,3-f]isoindol]-7'-yl)piperidine-2,6-dione | $^1$H NMR (600 MHz, DMSO-$d_6$) δ 10.98 (s, 1H), 7.39 (d, *J* = 2.6 Hz, 1H), 7.19 – 7.07 (m, 4H), 6.88 – 6.78 (m, 2H), 6.66 – 6.57 (m, 4H), 6.49 (dd, *J* = 8.3, 2.6 Hz, 1H), 6.28 (d, *J* = 8.3 Hz, 2H), 5.08 (dd, *J* = 13.3, 5.1 Hz, 1H), 4.49 – 4.32 (m, 4H), 4.30 – 4.15 (m, 3H), 3.73 – 3.69 (m, 2H), 3.56 (s, 2H), 3.33 – 3.30 (m, 1H), 3.26 (s, 2H), 3.18 – 3.05 (m, 2H), 2.99 – 2.86 (m, 4H), 2.83 (t, *J* = 12.3 Hz, 2H), 2.64 – 2.55 (m, 1H), 2.43 – 2.34 (m, 1H), 2.25 – 2.13 (m, 6H), 2.12 – 2.03 (m, 2H), 2.01 – 1.95 (m, 1H), 1.74 – 171 (m, 1H), 1.48 – 1.41 (m, 2H), 1.30 – 1.19 (m, 2H). | 806. 46 | General Synthesis Formula 22 |
| **132** | B17 | 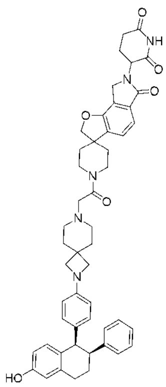 | $^1$H NMR (600 MHz, DMSO-$d_6$) δ 10.99 (s, 1H), 9.53 (s, 1H), 7.37 (d, *J* = 7.6 Hz, 1H), 7.31 (d, *J* = 7.6 Hz, 1H), 7.17 – 7.10 (m, 3H), 6.87 – 6.78 (m, 2H), 6.66 – 6.56 (m, 2H), 6.48 (dd, *J* = 8.2, 2.5 Hz, 1H), 6.20 (d, *J* = 8.2 Hz, 2H), 6.07 (d, *J* = 8.2 Hz, 2H), 5.12 – 5.08 (m, 1H), 4.72 – 4.58 (m, 2H), 4.42 – 4.34 (m, 3H), 4.30 – 4.22 (m, 2H), 4.13 (d, *J* = 4.9 Hz, 1H), 3.55 – 3.51 (m, 3H), 3.47 – 3.45 (m, 2H), 3.41 | 820. 60 | General Synthesis Formula 10 |

| | | | | | |
|---|---|---|---|---|---|
| | | 3-(1'-(2-(4-((1R,2S)-6-Hydroxy-2-phenyl-1,2,3,4-tetrahydronaphthalen-1-yl)phenyl)-2,7-diazaspiro[3.5]nonan-7-yl)acetyl)-6-oxo-6,8-dihydro-2H,7H-spiro[furo[2,3-e]isoindole-3,4'-piperidin]-7-yl)piperidine-2,6-dione | (s, 2H), 3.32 – 3.18 (m, 2H), 3.10 – 2.97 (m, 3H), 2.95 – 2.84 (m, 3H), 2.64 – 2.56 (m, 1H), 2.48 – 2.35 (m, 1H), 2.11 – 2.03 (m, 3H), 2.01 – 2.96 (m, 3H), 1.93 – 1.87 (t, *J* = 12.8 Hz, 1H), 1.83 – 1.76 (m, 3H), 1.72 – 1.68 (m, 1H). | | |
| **133** | B18 | 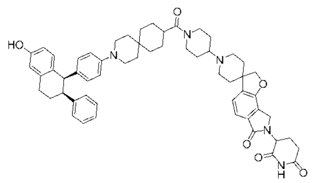 3-(1'-(1-(3-(4-((1R,2S)-6-Hydroxy-2-phenyl-1,2,3,4-tetrahydronaphthalen-1-yl)phenyl)-3-azaspiro[5.5]undecane-9-carbonyl)piperidin-4-yl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 11.00 (s, 1H), 9.47 (s, 1H), 7.36 (d, *J* = 7.6 Hz, 1H), 7.28 (d, *J* = 7.6 Hz, 1H), 7.17 – 7.11 (m, 3H), 6.88 – 6.79 (m, 2H), 6.69 – 6.58 (m, 3H), 6.49 (dd, *J* = 8.3, 2.5 Hz, 1H), 6.32 (s, 3H), 5.10 (dd, *J* = 13.2, 5.1 Hz, 1H), 4.70 – 4.65 (m, 2H), 4.59 – 4.53 (m, 1H), 4.41 – 4.38 (m, 1H), 4.29 – 4.16 (m, 2H), 4.13 (d, *J* = 13.9 Hz, 1H), 3.34 – 3.31 (s, 4H), 3.15 – 3.01 (m, 6H), 3.00 – 2.86 (m, 4H), 2.68 – 2.56 (m, 2H), 2.47 – 2.35 (m, 2H), 2.18 – 2.15 (m, 2H), 2.13 – 2.04 (m, 3H), 2.03 – 1.95 (m, 3H), 1.73 (d, *J* = 6.3 Hz, 2H), 1.63 – 1.57 (m, 3H), 1.55 – 1.43 (m, 7H), 1.24 – 1.18 (m, 3H). | 916.65 | General Synthesis Formula 21 |
| **134** | B19 | 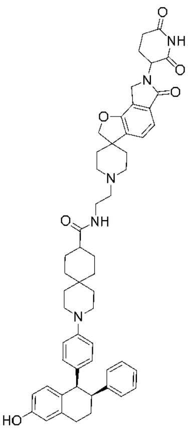 N-(2-(7-(2,6-Dioxopiperidin-3-yl)-6-oxo-7,8-dihydro-2H,6H-spiro[furo[2,3-e]isoindole-3,4'-piperidin]-1'-yl)ethyl)-3-(4-((1R,2S)-6-hydroxy-2-phenyl-1,2,3,4-tetrahydronaphthalen-1-yl)phenyl)-3-azaspiro[5.5]undecane-9-carboxamide | $^1$H NMR (600 MHz, DMSO-$d_6$) δ 11.00 (s, 1H), 9.15 (s, 1H), 8.10 – 8.07 (m, 1H), 7.84 (s, 2H), 7.35 (d, *J* = 7.6 Hz, 1H), 7.29 (d, *J* = 7.6 Hz, 1H), 7.19 – 7.11 (m, 3H), 6.83 (d, *J* = 7.3 Hz, 2H), 6.66 – 6.59 (m, 2H), 6.49 (dd, *J* = 8.3, 2.6 Hz, 1H), 6.28 (s, 2H), 5.10 (dd, *J* = 13.2, 5.1 Hz, 1H), 4.67 – 4.63 (m, 1H), 4.39 (d, *J* = 17.2 Hz, 1H), 4.24 (d, *J* = 17.2 Hz, 1H), 4.17 (t, *J* = 5.3 Hz, 2H), 3.59 (d, *J* = 12.4 Hz, 1H), 3.31 – 3.24 (m, 3H), 3.19 – 3.15 (m, 2H), 3.13 – 3.06 (m, 4H), 3.04 – 3.00 (m, 1H), 2.98 – 3.90 (m, 4H), 2.64 – 2.56 (m, 1H), 2.46 – 2.39 (m, 2H), 2.34 – 2.30 (m, 1H), 2.14 – 2.06 (m, 3H), 2.03 – 1.94 (m, 3H), 1.74 – 1.70 (m, 2H), 1.67 – 1.65 (m, 1H), 1.61 – 1.50 (m, 5H), 1.27 – 1.22 (m, 2H), 1.15 – 1.09 (m, 2H). | 876.56 | General Synthesis Formula 11 |

| | | | | | |
|---|---|---|---|---|---|
| **135** | B20 | 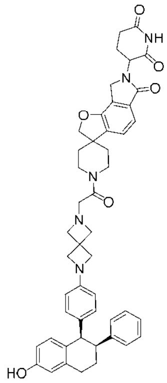<br>3-(1'-(2-(6-(4-(((1R,2S)-6-Hydroxy-2-phenyl-1,2,3,4-tetrahydronaphthalen-1-yl)phenyl)-2,6-diazaspiro[3.3]heptan-2-yl)acetyl)-6-oxo-6,8-dihydro-2H,7H-spiro[furo[2,3-e]isoindole-3,4'-piperidin]-7-yl)piperidine-2,6-dione | $^{1}$H NMR (600 MHz, DMSO-$d_6$) δ 10.99 (s, 1H), 9.12 (s, 1H), 7.36 (d, $J$ = 7.6 Hz, 1H), 7.31 (d, $J$ = 7.5 Hz, 1H), 7.18 – 7.08 (m, 3H), 6.87 – 6.78 (m, 2H), 6.64 – 6.56 (m, 2H), 6.48 (dd, $J$ = 8.2, 2.6 Hz, 1H), 6.20 (d, $J$ = 8.2 Hz, 2H), 6.09 (d, $J$ = 8.2 Hz, 2H), 6.11 – 5.07 (m, 1H), 4.72 – 4.60 (m, 2H), 4.45 – 4.34 (m, 4H), 4.31 (d, $J$ = 13.6 Hz, 1H), 4.26 – 4.20 (m, 3H), 4.13 (d, $J$ = 5.0 Hz, 1H), 3.95 – 3.88 (m, 2H), 3.85 – 3.76 (m, 2H), 3.57 (d, $J$ = 13.9 Hz, 1H), 3.31 – 3.25 (m, 2H), 3.23 – 3.15 (m, 1H), 3.02 – 2.87 (m, 3H), 2.85 (s, 1H), 2.64 – 2.57 (m, 1H), 2.45 – 2.36 (m, 1H), 2.13 – 2.04 (m, 1H), 2.01 – 1.95 (m, 1H), 1.90 – 1.88 (m, 1H), 1.81 – 1.76 (m, 3H), 1.72 – 1.69 (m, 1H). | 792.46 | General Synthesis Formula 10 |
| **136** | B21 | 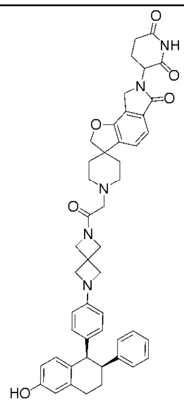<br>3-(1'-(2-(6-(4-(((1R,2S)-6-Hydroxy-2-phenyl-1,2,3,4-tetrahydronaphthalen-1-yl]phenyl)-2,6-diazaspiro[3.3]heptan-2-yl)-2-oxoethyl)-6-oxo-6,8-dihydro-2H,7H-spiro[furo[2,3-e]isoindole-3,4'-piperidin]-7-yl)piperidine-2,6-dione | $^{1}$H NMR (600 MHz, DMSO-$d_6$) δ 10.99 (s, 1H), 9.87 (s, 1H), 7.36 (d, $J$ = 7.6 Hz, 1H), 7.29 (d, $J$ = 7.4 Hz, 1H), 7.17 – 7.08 (m, 3H), 6.86 – 6.77 (m, 2H), 6.66 – 6.56 (m, 2H), 6.48 (dd, $J$ = 8.3, 2.6 Hz, 1H), 6.19 (d, $J$ = 8.0 Hz, 2H), 6.09 – 6.07 (m, 2H), 5.10 (dd, $J$ = 13.3, 5.2 Hz, 1H), 4.68 – 4.59 (m, 1H), 4.45 – 4.34 (m, 1H), 4.33 – 4.29 (m, 2H), 4.24 (d, $J$ = 17.2 Hz, 1H), 4.18 – 4.09 (m, 3H), 3.98 (s, 2H), 3.87 – 3.82 (m, 5H), 3.30 – 3.27 (m, 1H), 3.16 – 3.11 (m, 2H), 3.02 – 2.87 (m, 4H), 2.66 – 2.56 (m, 1H), 2.47 – 2.37 (m, 1H), 2.32 – 2.23 (m, 2H), 2.15 – 2.06 (m, 2H), 2.02 – 1.89 (m, 3H), 1.74 – 1.66 (m, 1H). | 792.46 | General Synthesis Formula 11 |

| | | | | | |
|---|---|---|---|---|---|
| **137** | B22 | 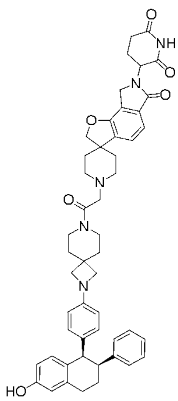 3-(1'-(2-(4-((1R,2S)-6-Hydroxy-2-phenyl-1,2,3,4-tetrahydronaphthalen-1-yl)phenyl)-2,7-diazaspiro[3.5]nonan-7-yl)-2-oxoethyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 10.99 (s, 1H), 9.66 (s, 1H), 9.12 (s, 1H), 7.37 (d, $J$ = 7.6 Hz, 1H), 7.31 (d, $J$ = 7.6 Hz, 1H), 7.18 – 7.07 (m, 3H), 6.87 – 6.80 (m, 2H), 6.66 – 6.57 (m, 2H), 6.48 (dd, $J$ = 8.3, 2.5 Hz, 1H), 6.20 – 6.18 (m, 2H), 6.10 – 5.99 (m, 2H), 5.10 (dd, $J$ = 13.3, 5.2 Hz, 1H), 4.69 – 4.61 (m, 1H), 4.59 – 4.51 (m, 1H), 4.40 – 4.37 (m, 1H), 4.33 – 4.30 (m, 1H), 4.25 – 4.22 (m, 1H), 4.12 (d, $J$ = 4.9 Hz, 1H), 3.46 – 3.43 (m, 5H), 3.42 – 3.35 (m, 3H), 3.34 – 3.24 (m, 3H), 3.18 – 3.11 (m, 2H), 2.99 – 2.88 (m, 3H), 2.66 – 2.56 (m, 1H), 2.47 – 2.37 (m, 1H), 2.33 – 2.28 (m, 1H), 2.23 – 2.19 (s, 1H), 2.16 – 2.05 (m, 1H), 2.02 – 1.90 (m, 3H), 1.87 – 1.75 (m, 2H), 1.73 – 1.65 (m, 3H). | 820.60 | General Synthesis Formula 11 |
| **138** | B23 | 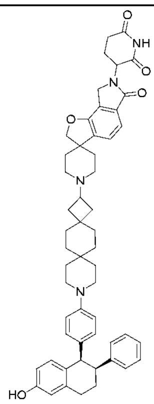 3-(1'-(10-(4-((1R,2S)-6-Hydroxy-2-phenyl-1,2,3,4-tetrahydronaphthalen-1-yl]phenyl)-10-azadispiro[3.2.7.2$^4$]tetradecan-2-yl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 10.99 (s, 1H), 7.37 – 7.33 (m, 1H), 7.29 (d, $J$ = 7.6 Hz, 1H), 7.19 – 7.10 (m, 3H), 7.06 – 6.77 (m, 4H), 6.67 – 6.61 (m, 2H), 6.50 (dd, $J$ = 8.4, 2.5 Hz, 1H), 6.37 (s, 2H), 5.10 (dd, $J$ = 13.3, 5.1 Hz, 1H), 4.67 – 4.63 (m, 2H), 4.40 (d, $J$ = 17.1 Hz, 1H), 4.24 (d, $J$ = 17.1 Hz, 1H), 3.43 (d, $J$ = 12.1 Hz, 2H), 3.35 (d, $J$ = 13.7 Hz, 1H), 3.17 (s, 3H), 3.03 – 2.87 (m, 5H), 2.64 – 2.57 (m, 1H), 2.55 (s, 3H), 2.47 – 2.39 (m, 2H), 2.17 – 2.05 (m, 4H), 2.04 – 1.89 (m, 5H), 1.74 (d, $J$ = 6.7 Hz, 1H), 1.58 (s, 3H), 1.49 – 1.147 (m, 4H), 1.40 – 1.27 (m, 4H). | 845.56 | General Synthesis Formula 6 |

| | | | | | |
|---|---|---|---|---|---|
| **139** | B29 | 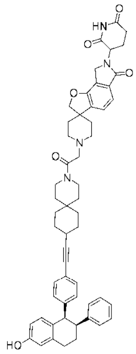<br>3-(1'-(2-(9-(4-(((1R,2S)-6-Hydroxy-2-phenyl-1,2,3,4-tetrahydronaphthalen-1-yl]phenyl)ethynyl)-3-azaspiro[5.5]undecan-3-yl)-2-oxoethyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 10.99 (s, 1H), 9.18 (s, 1H), 7.38 – 7.36 (m, 1H), 7.32 – 7.29 (m, 1H), 7.19 – 7.12 (m, 3H), 6.99 – 6.96 (m, 2H), 6.85 (d, $J$ = 7.4 Hz, 2H), 6.66 – 6.62 (m, 2H), 6.53 – 6.47 (m, 1H), 6.38 – 6.33 (m, 2H), 5.10 (dd, $J$ = 13.2, 5.2 Hz, 1H), 4.67 – 4.62 (m, 2H), 4.60 – 4.56 (m, 1H), 4.51 – 4.48 (m, 1H), 4.40 (d, $J$ = 17.1 Hz, 1H), 4.33 – 4.20 (m, 3H), 3.51 (s, 3H), 3.16 (s, 2H), 3.05 – 2.87 (m, 4H), 2.65 – 2.56 (m, 2H), 2.46 – 2.41 (m, 2H), 2.30 (t, $J$ = 13.3 Hz, 2H), 2.21 – 2.18 (m, 1H), 2.14 – 2.04 (m, 1H), 2.01 – 1.91 (m, 3H), 1.74 (s, 2H), 1.65 (d, $J$ = 11.8 Hz, 2H), 1.54 – 1.51 (m, 3H), 1.42 (d, $J$ = 20.1 Hz, 2H), 1.27 – 1.23 (m, 4H). | 871.66 | General Synthesis Formula 39 |
| **140** | B30 | 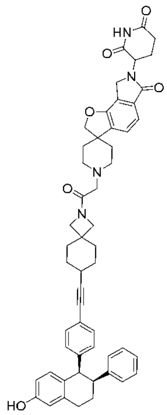<br>3-(1'-(2-(7-((4-((1R,2S)-6-Hydroxy-2-phenyl-1,2,3,4-tetrahydronaphthalen-1-yl]phenyl)ethynyl)-2-azaspiro[3.5]nonan-2-yl)-2-oxoethyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 10.99 (s, 1H), 9.19 (s, 1H), 7.36 (d, $J$ = 7.6 Hz, 1H), 7.29 (d, $J$ = 7.6 Hz, 1H), 7.18 – 7.10 (m, 3H), 6.98 – 6.96 (m, 2H), 6.86 – 6.84 (m, 2H), 6.66 – 6.61 (m, 2H), 6.50 (dd, $J$ = 8.4, 2.6 Hz, 1H), 6.36 – 6.33 (m, 2H), 5.10 (dd, $J$ = 13.3, 5.1 Hz, 1H), 4.66 – 4.62 (m, 2H), 4.40 (d, $J$ = 17.1 Hz, 1H), 4.29 – 4.20 (m, 2H), 4.00 (s, 1H), 3.84 (d, $J$ = 23.1 Hz, 2H), 3.66 (d, $J$ = 22.9 Hz, 2H), 3.49 (s, 2H), 3.18 – 3.12 (m, 2H), 3.04 – 2.87 (m, 4H), 2.65 – 2.56 (m, 2H), 2.46 – 2.36 (m, 2H), 2.27 (t, $J$ = 12.9 Hz, 2H), 2.11 – 2.06 (m, 1H), 2.01 – 1.90 (m, 3H), 1.87 – 1.82 (m, 2H), 1.79 – 1.71 (m, 2H), 1.58 (s, 2H), 1.51 – 1.39 (m, 2H). | 843.66 | General Synthesis Formula 39 |

| | | | | | |
|---|---|---|---|---|---|
| **141** | B31 | 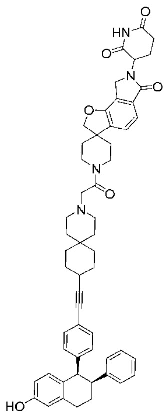 3-(1'-(2-(9-(4-(((1R,2S)-6-Hydroxy-2-phenyl-1,2,3,4-tetrahydronaphthalen-1-yl)phenyl)ethynyl)-3-azaspiro[5.5]undecan-3-yl)acetyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 10.99 (s, 1H), 9.18 (s, 1H), 7.37 (d, $J$ = 7.6 Hz, 1H), 7.32 (d, $J$ = 7.6 Hz, 1H), 7.21 – 7.10 (m, 3H), 6.97 (d, $J$ = 7.8 Hz, 2H), 6.85 (d, $J$ = 7.4 Hz, 2H), 6.67 – 6.60 (m, 2H), 6.50 (dd, $J$ = 8.3, 2.5 Hz, 1H), 6.35 (dd, $J$ = 8.4, 2.0 Hz, 2H), 5.12 – 5.06 (m, 1H), 4.74 – 4.60 (m, 2H), 4.43 – 4.35 (m, 3H), 4.33 – 4.20 (m, 3H), 3.67 – 3.65 (m, 1H), 3.33 – 3.32 (s, 2H), 3.24 (t, $J$ = 13.0 Hz, 1H), 3.13 – 3.05 (m, 2H), 3.00 – 2.86 (m, 5H), 2.64 – 2.55 (m, 2H), 2.46 – 2.37 (m, 2H), 2.12 – 2.03 (m, 1H), 2.01 – 1.95 (m, 1H), 1.94 – 1.91 (m, 2H), 1.84 – 1.79 (m, 3H), 1.76 – 1.72 (m, 3H), 1.67 – 1.62 (m, 2H), 1.57 (d, $J$ = 9.7 Hz, 1H), 1.53 – 1.49 (m, 2H), 1.47 – 1.42 (m, 1H), 1.28 – 1.23 (m, 2H). | 871.66 | General Synthesis Formula 38 |
| **142** | B32 | 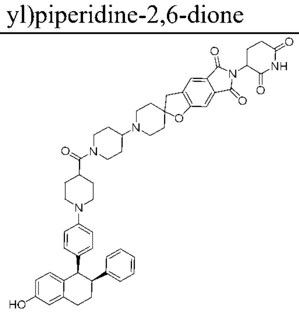 6-(2,6-Dioxopiperidin-3-yl)-1'-(1-(4-(((1R,2S)-6-hydroxy-2-phenyl-1,2,3,4-tetrahydronaphthalen-1-yl)phenyl)piperidine-4-carbonyl)piperidin-4-yl)spiro[furo[2,3-f]isoindole-2,4'-piperidine]-5,7(3H,6H)-dione | $^1$H NMR (600 MHz, DMSO-$d_6$) δ 11.11 (s, 1H), 7.82 (s, 1H), 7.29 – 7.22 (m, 1H), 7.17 – 7.12 (m, 3H), 6.83 (d, $J$ = 7.3 Hz, 2H), 6.76 (s, 2H), 6.67 – 6.60 (m, 2H), 6.49 (dd, $J$ = 8.3, 2.6 Hz, 1H), 6.32 (s, 2H), 5.10 (dd, $J$ = 12.9, 5.5 Hz, 1H), 4.56 (d, $J$ = 12.9 Hz, 1H), 4.19 – 4.14 (m, 2H), 3.59 (s, 4H), 3.38 – 3.30 (m, 3H), 3.28 – 3.17 (m, 4H), 3.09 – 3.06 (m, 2H), 3.02 – 2.80 (m, 6H), 2.64 – 2.53 (m, 2H), 2.22 (t, $J$ = 15.4 Hz, 2H), 2.16 – 2.00 (m, 6H), 1.72 – 1.69 (m, 4H), 1.62 – 1.58 (m, 1H), 1.51 – 1.44 (m, 1H). | 862.66 | General Synthesis Formula 15 |
| **143** | B33 | 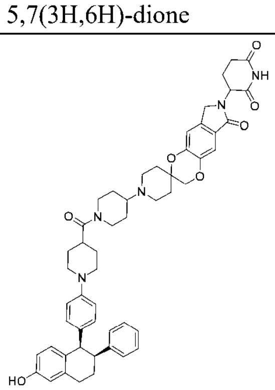 6-(2,6-Dioxopiperidin-3-yl)-1'-(1-(4- | $^1$H NMR (600 MHz, DMSO-$d_6$) δ 10.98 (s, 1H), 7.24 (s, 1H), 7.18 – 7.08 (m, 4H), 6.86 – 6.80 (m, 2H), 6.71 – 6.62 (m, 4H), 6.49 (dd, $J$ = 8.3, 2.5 Hz, 1H), 6.29 (s, 2H), 5.08 (dd, $J$ = 13.3, 5.3 Hz, 1H), 4.56 (d, $J$ = 13.0 Hz, 1H), 4.37 – 4.28 (m, 2H), 4.21 – 4.12 (m, 4H), 3.60 – 3.53 (m, 4H), 3.32 (dd, $J$ = 14.1, 5.0 Hz, 3H), 3.23 – 3.14 (m, 2H), 3.06 (t, $J$ = 12.9 Hz, 1H), 3.03 – 2.87 (m, 4H), 2.82 (s, 2H), 2.65 – | 864.76 | General Synthesis Formula 15 |

| | | | | | |
|---|---|---|---|---|---|
| | | (((1R,2S)-6-hydroxy-2-phenyl-1,2,3,4-tetrahydronaphthalen-1-yl]phenyl)piperidine-4-carbonyl)piperidin-4-yl)spiro[furo[2,3-f]isoindole-2,4'-piperidine]-5,7(3H,6H)-dione | 2.53 (m, 2H), 2.42 – 2.31 (m, 2H), 2.18 – 2.06 (m, 3H), 2.04 – 1.91 (m, 4H), 1.73 – 1.67 (m, 4H), 1.62 – 1.59 (d, $J$ = 11.0 Hz, 1H), 1.53 – 1.44 (m, 1H). | | |
| **144** | B34 | 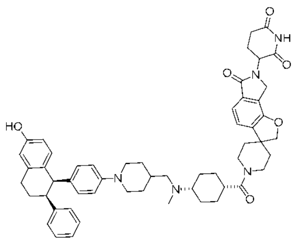 3-(1'-((1S,4s)-4-((1-(4-(((1R,2S)-6-Hydroxy-2-phenyl-1,2,3,4-tetrahydronaphthalen-1-yl)phenyl)piperidin-4-yl)(methyl)amino)cyclohexane-1-carbonyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 10.98 (s, 1H), 8.73 (s, 1H), 7.44 – 7.38 (m, 1H), 7.29 (d, $J$ = 7.6 Hz, 1H), 7.17 – 7.10 (m, 3H), 6.84 (d, $J$ = 7.2 Hz, 2H), 6.69 – 6.60 (m, 4H), 6.49 (dd, $J$ = 8.3, 2.5 Hz, 1H), 6.27 (d, $J$ = 8.1 Hz, 2H), 5.09 (dd, $J$ = 13.2, 5.1 Hz, 1H), 4.67 – 4.61 (m, 2H), 4.40 (d, $J$ = 17.0 Hz, 1H), 4.23 (d, $J$ = 17.0 Hz, 1H), 4.17 (d, $J$ = 5.1 Hz, 1H), 3.94 – 3.83 (m, 1H), 3.59 – 3.52 (m, 2H), 3.34 – 3.28 (m, 2H), 3.24 – 3.18 (m, 3H), 3.08 – 3.03 (m, 1H), 3.03 – 2.96 (m, 2H), 2.94 – 2.88 (m, 3H), 2.73 (d, $J$ = 4.8 Hz, 3H), 2.69 – 2.63 (m, 2H), 2.63 – 2.60 (m, 1H), 2.45 – 2.37 (m, 1H), 2.11 – 2.07 (m, 1H), 2.03 – 1.94 (m, 3H), 1.91 – 1.81 (m, 8H), 1.79 – 1.69 (m, 5H), 1.63 – 1.58 (m, 2H), 1.40 – 1.21 (m, 2H). | 890.86 | General Synthesis Formula 36 |
| **145** | B35 | 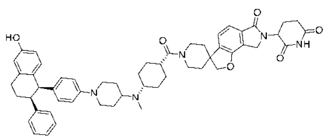 3-(1'-((1S,4s)-4-((1-(4-(((1R,2S)-6-Hydroxy-2-phenyl-1,2,3,4-tetrahydronaphthalen-1-yl)phenyl)piperidin-4-yl)(methyl)amino)cyclohexane-1-carbonyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 10.98 (s, 1H), 8.90 (s, 1H), 7.41 (d, $J$ = 7.6 Hz, 1H), 7.29 (d, $J$ = 7.6 Hz, 1H), 7.17 – 7.10 (m, 3H), 6.89 – 6.77 (m, 2H), 6.70 – 6.52 (m, 4H), 6.49 (dd, $J$ = 8.2, 2.6 Hz, 1H), 6.24 (d, $J$ = 8.4 Hz, 2H), 5.09 (dd, $J$ = 13.3, 5.1 Hz, 1H), 4.68 – 4.60 (m, 2H), 4.46 – 4.34 (m, 2H), 4.23 (d, $J$ = 17.1 Hz, 1H), 4.15 (d, $J$ = 5.0 Hz, 1H), 3.90 (d, $J$ = 13.5 Hz, 1H), 3.71 – 3.64 (m, 2H), 3.33 – 3.25 (m, 2H), 3.20 (t, $J$ = 13.1 Hz, 1H), 3.01 – 2.96 (m, 2H), 2.94 – 2.88 (m, 2H), 2.75 – 2.70 (m, 2H), 2.67 – 2.57 (m, 6H), 2.47 – 2.37 (m, 2H), 2.15 – 2.06 (m, 1H), 2.05 – 1.91 (m, 5H), 1.89 – 1.82 (m, 4H), 1.75 – 1.72 (m, 5H), 1.66 – 1.62 (m, 3H). | 876.66 | General Synthesis Formula 36 |

| | | | | | |
|---|---|---|---|---|---|
| **146** | B36 | 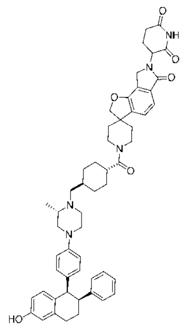 3-(1'-((1S,4r)-4-((S)-4-(4-(((1R,2S)-6-Hydroxy-2-phenyl-1,2,3,4-tetrahydronaphthalen-1-yl)phenyl)-2-methylpiperazin-1-yl)methyl)cyclohexane-1-carbonyl)-6-oxo-6,8-dihydro-2H,7H-spiro[furo[2,3-e]isoindole-3,4'-piperidin]-7-yl)piperidine-2,6-dione | $^1$H NMR (400 MHz, DMSO-$d_6$) δ 10.99 (s, 1H), 7.41 (d, $J$ = 7.6 Hz, 1H), 7.28 (d, $J$ = 7.6 Hz, 1H), 7.20 – 7.08 (m, 3H), 6.90 – 6.82 (m, 2H), 6.69 – 6.58 (m, 4H), 6.52 – 6.47 (m, 1H), 6.27 (d, $J$ = 8.3 Hz, 2H), 5.09 (d, $J$ = 13.0 Hz, 1H), 4.66 – 4.63 (m, 2H), 4.44 – 4.34 (m, 2H), 4.26 – 4.16 (m, 2H), 3.96 – 3.94 (m, 1H), 3.75 – 3.57 (m, 2H), 3.35 – 3.28 (m, 3H), 3.22 – 3.13 (m, 3H), 3.10 – 3.00 (m, 1H), 3.00 – 2.83 (m, 5H), 2.78 – 2.68 (m, 2H), 2.65 – 2.55 (m, 2H), 2.46 – 2.31 (m, 1H), 2.13 – 2.04 (m, 1H), 2.03 – 1.90 (m, 2H), 1.88 – 1.67 (m, 8H), 1.54 – 1.37 (m, 2H), 1.31 (d, $J$ = 6.2 Hz, 3H), 1.19 – 1.02 (m, 2H). | 876.66 | General Synthesis Formula 25 |
| **147** | B37 | 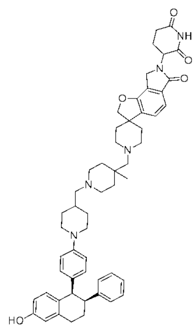 3-(1'-((1-(4-((1R,2S)-6-Hydroxy-2-phenyl-1,2,3,4-tetrahydronaphthalen-1-yl)phenyl)piperidin-4-yl)methyl)-4-methylpiperidin-4-ylmethyl)-6-oxo-6,8-dihydro-2H,7H-spiro[furo[2,3-e]isoindole-3,4'-piperidin]-7-yl)piperidine-2,6-dione | $^1$H NMR (400 MHz, DMSO-$d_6$) δ 11.00 (s, 1H), 7.39 – 7.25 (m, 2H), 7.20 – 7.08 (m, 3H), 6.87 – 6.80 (m, 2H), 6.67 – 6.57 (m, 4H), 6.48 (dd, $J$ = 8.3, 2.6 Hz, 1H), 6.24 (d, $J$ = 8.3 Hz, 2H), 5.10 (dd, $J$ = 13.3, 5.1 Hz, 1H), 4.67 (s, 2H), 4.40 (d, $J$ = 17.2 Hz, 1H), 4.24 (d, $J$ = 17.2 Hz, 1H), 4.15 (d, $J$ = 5.0 Hz, 1H), 3.55 (s, 3H), 3.35 – 3.25 (m, 5H), 3.15 – 3.01 (m, 6H), 2.99 – 2.85 (m, 3H), 2.61 (d, $J$ = 15.1 Hz, 3H), 2.46 – 2.28 (m, 3H), 2.12 – 2.05 (m, 1H), 2.02 – 1.87 (m, 5H), 1.82 – 1.69 (m, 6H), 1.32 – 1.23 (m, 6H). | 862.76 | General Synthesis Formula 37 |
| **148** | B38 | 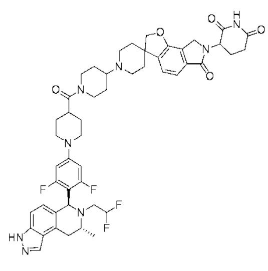 3-(1'-(1-(1-(4-((6S,8R)-7-(2,2-Difluoroethyl)-8-methyl-6,7,8,9- | $^1$H NMR (600 MHz, DMSO-$d_6$) δ 10.99 (s, 1H), 9.49 (s, 1H), 8.07 (s, 1H), 7.36 (d, $J$ = 7.6 Hz, 1H), 7.29 (d, $J$ = 7.6 Hz, 1H), 7.24 (d, $J$ = 8.6 Hz, 1H), 6.74 (d, $J$ = 8.6 Hz, 1H), 6.55 (d, $J$ = 13.8 Hz, 2H), 5.83 (t, $J$ = 56.3 Hz, 1H), 5.19 (s, 1H), 5.10 (dd, $J$ = 13.3, 5.1 Hz, 1H), 4.70 – 4.63 (m, 2H), 4.59 – 4.56 (m, 1H), 4.40 (d, $J$ = 17.2 Hz, 1H), 4.24 (d, $J$ = 17.2 Hz, 1H), 4.20 | 911.65 | General Synthesis Formula 49 |

| | | | | | |
|---|---|---|---|---|---|
| | | tetrahydro-3H-pyrazolo[4,3-f]isoquinolin-6-yl)-3,5-difluorophenyl)piperidine-4-carbonyl)piperidin-4-yl)-6-oxo-6,8-dihydro-2H,7H-spiro[furo[2,3-e]isoindole-3,4'-piperidin]-7-yl)piperidine-2,6-dione | (d, $J$ = 13.2 Hz, 1H), 3.80 – 3.6 (m, 2H), 3.56 – 3.52 (m, 2H), 3.22 – 3.18 (m, 1H), 3.13 – 3.05 (m, 5H), 2.95 – 2.79 (m, 6H), 2.71 – 2.66 (m, 1H), 2.63 – 2.57 (m, 2H), 2.46 – 2.38 (m, 1H), 2.23 – 2.07 (m, 4H), 2.04 – 1.96 (m, 3H), 1.69 – 1.65 (m, 2H), 1.60 (s, 3H), 1.53 – 1.44 (m, 1H), 1.07 (d, $J$ = 6.5 Hz, 3H) | | |
| **149** | B39 | 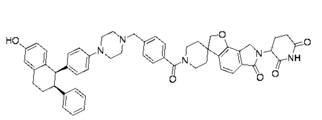 3-(1'-((4-(4-((1R,2S)-6-Hydroxy-2-phenyl-1,2,3,4-tetrahydronaphthalen-1-yl]phenyl)piperazin-1-yl)methyl)benzoyl)-6-oxo-6,8-dihydro-2H,7H-spiro[furo[2,3-e]isoindole-3,4'-piperidin]-7-yl)piperidine-2,6-dione | $^1$H NMR (400 MHz, DMSO-$d_6$) δ 10.98 (s, 1H), 9.15 (s, 1H), 7.64 – 7.54 (m, 4H), 7.51 (d, $J$ = 7.6 Hz, 1H), 7.31 (d, $J$ = 7.6 Hz, 1H), 7.18 – 7.07 (m, 3H), 6.88 – 6.79 (m, 2H), 6.66 – 6.54 (m, 4H), 6.48 (dd, $J$ = 8.3, 2.6 Hz, 1H), 6.26 (d, $J$ = 8.4 Hz, 2H), 5.09 (dd, $J$ = 13.3, 5.1 Hz, 1H), 4.66 (s, 2H), 4.40 (d, $J$ = 18.7 Hz, 4H), 4.29 – 4.12 (m, 2H), 3.67 (s, 2H), 3.54 – 3.52 (m, 1H), 3.30 – 3.28 (m, 2H), 3.16 – 3.10 (m, 3H), 3.03 – 2.78 (m, 7H), 2.65 – 2.55 (m, 1H), 2.42 (dd, $J$ = 13.2, 4.7 Hz, 1H), 2.10 – 2.06 (m, 1H), 2.01 – 1.90 (m, 3H), 1.84 (s, 1H), 1.74 – 1.66 (m, 2H). | 856.56 | General Synthesis Formula 28 |
| **150** | B40 | 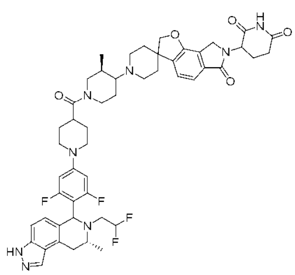 3-(1'-((3R)-1-(1-(4-((8R)-7-(2,2-Difluoroethyl)-8-methyl-6,7,8,9-tetrahydro-3H-pyrazolo[4,3-f]isoquinolin-6-yl)-3,5-difluorophenyl)piperidine-4-carbonyl)-3-methylpiperidin-4-yl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 12.99 (s, 1H), 10.99 (s, 1H), 9.12 (s, 1H), 8.07 (s, 1H), 7.36 (d, $J$ = 7.6 Hz, 1H), 7.29 (d, $J$ = 7.6 Hz, 1H), 7.24 (d, $J$ = 8.6 Hz, 1H), 6.75 (d, $J$ = 8.6 Hz, 1H), 6.54 (d, $J$ = 13.9 Hz, 2H), 5.82 (t, $J$ = 54 Hz, 1H), 5.17 (s, 1H), 5.10 (dd, $J$ = 13.2, 5.1 Hz, 1H), 4.70 – 4.64 (m, 2H), 4.62 – 4.56 (m, 1H), 4.51 – 4.38 (m, 1H), 4.25 (d, $J$ = 17.3 Hz, 2H), 4.03 (d, $J$ = 13.8 Hz, 1H), 3.78 (s, 3H), 3.62 – 3.58 (m, 1H), 3.21 – 3.17 (m, 2H), 3.10 – 3.00 (m, 3H), 2.93 – 2.81 (m, 4H), 2.71 – 2.57 (m, 3H), 2.46 – 2.40 (m, 2H), 2.26 – 2.13 (m, 4H), 2.03 – 1.98 (m, 3H), 1.71 – 1.60 (m, 5H), 1.51 – 1.46 (m, 1H), 1.06 (d, $J$ = 6.5 Hz, 3H), 1.02 – 1.01 (m, 1H), 0.90 – 0.88 (m, 1H). | 925.55 | General Synthesis Formula 50 |
| **151** | B41 | 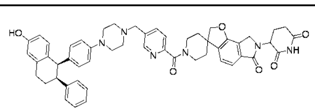 | $^1$H NMR (600 MHz, DMSO-$d_6$) δ 10.98 (d, $J$ = 6.6 Hz, 1H), 8.77 – 8.66 (m, 1H), 8.07 (d, $J$ = 8.0 Hz, 1H), 7.74 (d, $J$ = 8.0 | 857.66 | General Synthesis Formula |

| | | | | | |
|---|---|---|---|---|---|
| | | 3-(1'-(5-((4-((1R,2S)-6-Hydroxy-2-phenyl-1,2,3,4-tetrahydronaphthalen-1-yl)phenyl)piperazin-1-yl)methyl)pyridin-2-yl)-6-oxo-6,8-dihydro-2H,7H-spiro[furo[2,3-e]isoindole-3,4'-piperidin]-7-yl)piperidine-2,6-dione | Hz, 1H), 7.47 (d, *J* = 7.6 Hz, 1H), 7.30 (dd, *J* = 7.6, 1.4 Hz, 1H), 7.17 – 7.06 (m, 3H), 6.84 (dd, *J* = 7.2, 1.8 Hz, 2H), 6.66 – 6.54 (m, 4H), 6.48 (dd, *J* = 8.3, 2.6 Hz, 1H), 6.26 (d, *J* = 8.4 Hz, 2H), 5.11 – 5.07 (m, 1H), 4.74 – 4.63 (m, 2H), 4.56 – 4.34 (m, 4H), 4.28 – 4.12 (m, 2H), 3.70 – 3.65 (m, 1H), 3.28 – 3.21 (m, 4H), 3.17 (s, 2H), 3.06 – 2.87 (m, 5H), 2.83 (s, 2H), 2.65 – 2.55 (m, 1H), 2.46 – 2.36 (m, 1H), 2.11 – 2.08 (m, 1H), 1.98 – 1.94 (m, 2H), 1.93 – 1.80 (m, 2H), 1.72 – 1.66 (m, 2H). | | 28 |
| **152** | B42 | 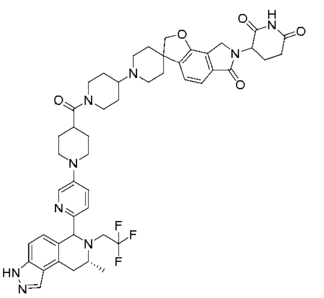 3-(1'-(1-(1-(6-((8R)-8-Methyl-7-(2,2,2-trifluoroethyl)-6,7,8,9-tetrahydro-3H-pyrazolo[4,3-f]isoquinolin-6-yl)pyridin-3-yl)piperidine-4-carbonyl)piperidin-4-yl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 10.99 (s, 1H), 8.15 (d, *J* = 3.0 Hz, 1H), 7.54 (d, *J* = 8.7 Hz, 1H), 7.41 – 7.33 (m, 2H), 7.29 (d, *J* = 8.9 Hz, 2H), 7.19 – 7.16 (m, 1H), 7.13 (d, *J* = 8.4 Hz, 1H), 6.87 – 6.84 (m, 1H), 5.51 (s, 1H), 5.09 (dd, *J* = 13.3, 5.1 Hz, 1H), 4.67 (s, 2H), 4.59 – 4.49 (m, 2H), 4.40 (d, *J* = 17.1 Hz, 1H), 4.32 – 4.29 (m, 1H), 4.24 (d, *J* = 17.2 Hz, 1H), 4.20 (d, *J* = 13.3 Hz, 1H), 3.83 – 3.76 (m, 3H), 3.71 – 3.64 (m, 1H), 3.58 – 3.50 (m, 3H), 3.23 – 3.15 (m, 1H), 3.11 – 3.07 (m, 3H), 2.96 – 2.76 (m, 4H), 2.64 – 2.56 (m, 2H), 2.46 – 2.37 (m, 1H), 2.26 – 2.19 (m, 2H), 2.17 – 2.07 (m, 2H), 2.05 – 1.94 (m, 3H), 1.73 – 1.58 (m, 5H), 1.49 (s, 1H), 1.17 (d, *J* = 6.7 Hz, 3H). | 894.76 | General Synthesis Formula 50 |
| **153** | B43 | 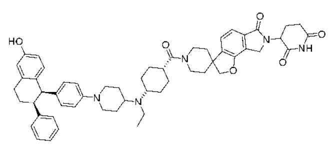 3-(1'-((1S,4S)-4-(Ethyl(1-(4-((1R,2S)-6-hydroxy-2-phenyl-1,2,3,4-tetrahydronaphthalen-1-yl)phenyl)piperidin-4-yl)amino)cyclohexane-1-carbonyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 10.98 (s, 1H), 8.51 (s, 1H), 7.41 (d, *J* = 7.6 Hz, 1H), 7.29 (d, *J* = 7.6 Hz, 1H), 7.19 – 7.10 (m, 3H), 6.87 – 6.80 (m, 2H), 6.68 – 6.56 (m, 4H), 6.49 (dd, *J* = 8.3, 2.5 Hz, 1H), 6.24 (d, *J* = 8.3 Hz, 2H), 5.09 (dd, *J* = 13.4, 5.1 Hz, 1H), 4.67 – 4.61 (m, 2H), 4.42 – 4.38 (m, 2H), 4.23 (d, *J* = 17.1 Hz, 1H), 4.15 (d, *J* = 5.0 Hz, 1H), 3.89 (d, *J* = 13.6 Hz, 1H), 3.69 – 3.66 (m, 2H), 3.32 – 3.29 (m, 3H), 3.25 – 3.14 (m, 3H), 3.03 – 2.87 (m, 5H), 2.72 (s, 1H), 2.66 – 2.61 (m, 3H), 2.45 – 2.38 (m, 2H), 2.13 – 2.05 (m, | 890.76 | General Synthesis Formula 33 |

| | | | | | |
|---|---|---|---|---|---|
| | | | 2H), 2.04 – 1.91 (m, 4H), 1.88 – 1.82 (m, 4H), 1.77 – 1.71 (m, 5H), 1.67 – 1.63 (m, 2H), 1.25 (t, *J* = 7.3 Hz, 3H). | | |
| **154** | B44 | 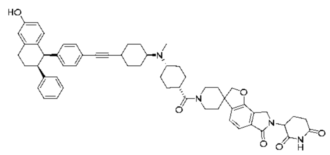 3-(1'-((1S,4S)-4-((4-((4-((1R,2S)-6-Hydroxy-2-phenyl-1,2,3,4-tetrahydronaphthalen-1-yl)phenyl)ethynyl)cyclohexyl)(methyl)amino)cyclohexane-1-carbonyl)-6-oxo-6,8-dihydro-2H,7H-spiro[furo[2,3-e]isoindole-3,4'-piperidin]-7-yl)piperidine-2,6-dione | $^1$H NMR (400 MHz, DMSO-$d_6$) δ 10.99 (s, 1H), 9.25 (s, 2H), 7.41 (d, *J* = 7.6 Hz, 1H), 7.29 (d, *J* = 7.6 Hz, 1H), 7.17 – 7.10 (m, 3H), 6.99 – 6.93 (m, 2H), 6.86 – 6.80 (m, 2H), 6.66 – 6.62 (m, 2H), 6.50 (dd, *J* = 8.4, 2.5 Hz, 1H), 6.35 (d, *J* = 8.2 Hz, 2H), 5.09 (dd, *J* = 13.3, 5.1 Hz, 1H), 4.64 (s, 2H), 4.42 – 4.38 (m, 2H), 4.28 – 4.17 (m, 2H), 3.91 – 3.87 (m, 1H), 3.23 – 3.17 m, 1H), 3.02 – 2.90 (m, 3H), 2.75 – 2.71 (m, 1H), 2.60 (d, *J* = 5.1 Hz, 4H), 2.46 – 2.38 (m, 1H), 2.10 – 1.92 (m, 8H), 1.88 – 1.82 (m, 4H), 1.75 – 1.73 (m, 4H), 1.61 – 1.47 (m, 7H), 1.24 (s, 5H). | 899. 75 | General Synthesis Formula 38 |
| **155** | B45 | 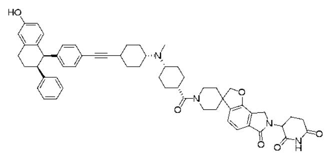 3-(1'-(((1S,4S)-4-((4-((4-((1R,2S)-6-Hydroxy-2-phenyl-1,2,3,4-tetrahydronaphthalen-1-yl)phenyl)ethynyl)cyclohexyl)(methyl)amino)cyclohexane-1-carbonyl)-6-oxo-6,8-dihydro-2H,7H-spiro[furo[2,3-e]isoindole-3,4'-piperidin]-7-yl)piperidine-2,6-dione | $^1$H NMR (400 MHz, DMSO-$d_6$) δ 10.99 (s, 1H), 9.21 (s, 1H), 8.81 (s, 1H), 7.41 (d, *J* = 7.6 Hz, 1H), 7.29 (d, *J* = 7.6 Hz, 1H), 7.21 – 7.09 (m, 3H), 7.04 (dd, *J* = 8.5, 2.4 Hz, 2H), 6.90 – 6.82 (m, 2H), 6.63 (dd, *J* = 5.5, 2.9 Hz, 2H), 6.50 (dd, *J* = 8.3, 2.5 Hz, 1H), 6.37 (dd, *J* = 8.4, 1.9 Hz, 2H), 5.09 (dd, *J* = 13.3, 5.1 Hz, 1H), 4.68 – 4.62 (m, 2H), 4.47 – 4.34 (m, 2H), 4.32 – 4.19 (m, 2H), 3.89 (d, *J* = 13.5 Hz, 1H), 3.20 (t, *J* = 11.5 Hz, 1H), 2.99 (d, *J* = 5.0 Hz, 3H), 2.75 – 2.72 (m, 1H), 2.64 – 2.62 (m, 4H), 2.43 (dd, *J* = 13.1, 4.6 Hz, 1H), 2.12 – 2.05 (m, 1H), 2.02 – 1.91 (m, 5H), 1.85 (s, 6H), 1.78 – 1.72 (m, 5H), 1.68 – 1.58 (m, 5H), 1.49 – 1.45 (m, 1H), 1.24 (s, 5H). | 899. 75 | General Synthesis Formula 38 |
| **156** | B46 | 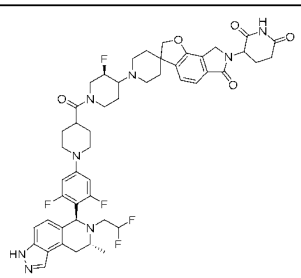 3-(1'-((3R)-1-(1-(4-((6S,8R)-7-(2,2- | $^1$H NMR (400 MHz, DMSO-$d_6$) δ 13.00 (s, 1H), 11.00 (s, 1H), 9.83 (s, 1H), 8.07 (s, 1H), 7.40 – 7.27 (m, 2H), 7.26 – 7.20 (m, 1H), 6.74 (d, *J* = 8.6 Hz, 1H), 6.54 (d, *J* = 13.6 Hz, 2H), 6.00 – 5.65 (m, 1H), 5.17 (s, 1H), 5.10 (dd, *J* = 13.2, 5.1 Hz, 1H), 4.66 (s, 2H), 4.40 (d, *J* = 17.3 Hz, 1H), 4.24 (d, *J* = 17.2 Hz, 1H), 3.80 – 3.76 | 929. 35 | General Synthesis Formula 50 |

| | | | | | |
|---|---|---|---|---|---|
| | | Difluoroethyl)-8-methyl-6,7,8,9-tetrahydro-3H-pyrazolo[4,3-f]isoquinolin-6-yl)-3,5-difluorophenyl)piperidine-4-carbonyl)-3-fluoropiperidin-4-yl)-6-oxo-6,8-dihydro-2H,7H-spiro[furo[2,3-e]isoindole-3,4'-piperidin]-7-yl)piperidine-2,6-dione | (s, 2H), 3.63 (s, 2H), 3.51 – 3.44 (m, 2H), 3.27 – 3.25 (m, 2H), 3.23 – 3.16 (m, 2H), 3.11 – 3.00 (m, 2H), 2.99 – 2.76 (m, 6H), 2.69 – 2.55 (m, 3H), 2.43 (dd, $J$ = 13.1, 4.6 Hz, 1H), 2.25 – 2.14 (m, 4H), 2.01 – 1.95 (m, 3H), 1.74 – 1.52 (m, 5H), 1.06 (d, $J$ = 6.4 Hz, 3H). | | |
| **157** | B47 | 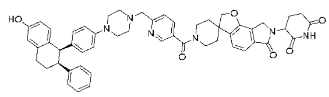 3-(1'-(6-((4-((1R,2S)-6-Hydroxy-2-phenyl-1,2,3,4-tetrahydronaphthalen-1-yl)phenyl)piperazin-1-yl)methyl)nicotinoyl)-6-oxo-6,8-dihydro-2H,7H-spiro[furo[2,3-e]isoindole-3,4'-piperidin]-7-yl)piperidine-2,6-dione | $^1$H NMR (400 MHz, DMSO-$d_6$) δ 11.00 (s, 1H), 8.78 (d, $J$ = 2.1 Hz, 1H), 8.06 (dd, $J$ = 7.9, 2.2 Hz, 1H), 7.63 (d, $J$ = 8.0 Hz, 1H), 7.51 (d, $J$ = 7.6 Hz, 1H), 7.32 (d, $J$ = 7.6 Hz, 1H), 7.17 – 7.09 (m, 3H), 6.88 – 6.78 (m, 2H), 6.66 – 6.56 (m, 3H), 6.48 (dd, $J$ = 8.3, 2.6 Hz, 1H), 6.26 (d, $J$ = 8.5 Hz, 2H), 5.10 (dd, $J$ = 13.2, 4.9 Hz, 1H), 4.67 (s, 2H), 4.60 (s, 2H), 4.51 (d, $J$ = 12.6 Hz, 1H), 4.39 (dd, $J$ = 17.3, 6.3 Hz, 1H), 4.24 (d, $J$ = 12.3 Hz, 1H), 4.17 (d, $J$ = 5.1 Hz, 1H), 3.54 – 3.52 (m, 3H), 3.40 (d, $J$ = 17.3 Hz, 2H), 3.35 – 3.26 (m, 4H), 3.06 – 2.85 (m, 5H), 2.61 – 2.57 (m, 1H), 2.42 (dd, $J$ = 13.1, 4.6 Hz, 2H), 2.15 – 2.04 (m, 1H), 2.03 – 1.93 (m, 3H), 1.86 – 1.81 (m, 1H), 1.73 – 1.66 (m, 2H). | 857.55 | General Synthesis Formula 28 |
| **158** | B48 | 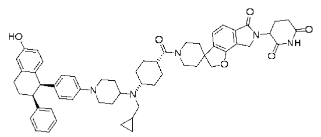 3-(1'-((1S,4S)-4-((Cyclopropylmethyl)(1-(4-((1R,2S)-6-hydroxy-2-phenyl-1,2,3,4-tetrahydronaphthalen-1-yl)phenyl)piperidin-4-yl)amino)cyclohexane-1-carbonyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 11.00 (s, 1H), 8.69 (s, 1H), 7.41 (d, $J$ = 7.6 Hz, 1H), 7.29 (d, $J$ = 7.6 Hz, 1H), 7.18 – 7.10 (m, 3H), 6.87 – 6.80 (m, 2H), 6.67 – 6.55 (m, 4H), 6.49 (dd, $J$ = 8.3, 2.5 Hz, 1H), 6.24 (d, $J$ = 8.3 Hz, 2H), 5.10 (dd, $J$ = 13.3, 5.1 Hz, 1H), 4.70 – 4.59 (m, 2H), 4.42 – 4.38 (m, 2H), 4.27 – 4.19 (m, 1H), 4.15 (d, $J$ = 5.0 Hz, 1H), 3.89 – 3.87 (m, 1H), 3.69 – 3.67 (m, 3H), 3.50 – 3.42 (m, 3H), 3.32 – 3.28 (m, 1H), 3.20 – 3.17 (m, 1H), 3.04 – 3.01 (m, 3H), 2.98 – 3.89 (m, 3H), 2.71 (s, 1H), 2.67 – 2.56 (m, 3H), 2.47 – 2.39 (m, 1H), 2.13 – 2.07 (m, 2H), 2.04 – 1.94 (m, 4H), 1.91 – 1.84 (m, 4H), 1.79 – 1.72 (m, 5H), 1.67 – 1.64 (m, 2H), 1.05 (s, 1H), 0.66 (d, $J$ = 7.8 Hz, 2H), 0.39 | 916.75 | General Synthesis Formula 33 |

| | | | | | |
|---|---|---|---|---|---|
| | | | (d, $J$ = 4.8 Hz, 2H). | | |
| **159** | B49 | 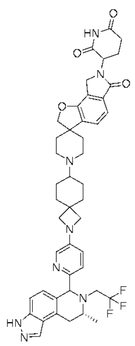 3-(1'-(2-(6-((8R)-8-Methyl-7-(2,2,2-trifluoroethyl)-6,7,8,9-tetrahydro-3H-pyrazolo[4,3-f]isoquinolin-6-yl)pyridin-3-yl)-2-azaspiro[3.5]nonan-7-yl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 13.05 (s, 1H), 11.00 (s, 1H), 9.30 (s, 1H), 8.10 (s, 1H), 7.67 (d, $J$ = 2.8 Hz, 1H), 7.36 (d, $J$ = 7.6 Hz, 1H), 7.28 (dd, $J$ = 11.6, 8.1 Hz, 2H), 7.01 – 6.98 (m, 1H), 6.80 (d, $J$ = 8.7 Hz, 1H), 5.10 (dd, $J$ = 13.3, 4.9 Hz, 2H), 4.72 – 4.62 (m, 2H), 4.40 (d, $J$ = 17.2 Hz, 1H), 4.24 (d, $J$ = 17.2 Hz, 1H), 3.67 (s, 3H), 3.63 – 3.61 (m, 3H), 3.47 – 3.45 (m, 2H), 3.28 – 3.25 (m, 1H), 3.17 – 3.08 (m, 3H), 3.04 – 2.99 (m, 1H), 2.97 – 2.86 (m, 2H), 2.66 – 2.56 (m, 1H), 2.46 – 2.36 (m, 2H), 2.20 – 2.17 (m, 2H), 2.09 (d, $J$ = 11.8 Hz, 2H), 2.06 – 1.94 (m, 4H), 1.60 – 1.50 (m, 4H), 1.09 (d, $J$ = 6.5 Hz, 3H). | 823.76 | General Synthesis Formula 61 |
| **160** | B50 | 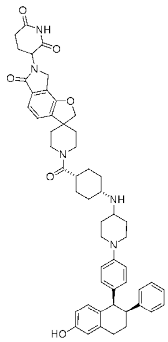 3-(1'-((1S,4S)-4-((1-(4-((1R,2S)-6-Hydroxy-2-phenyl-1,2,3,4-tetrahydronaphthalen-1-yl)phenyl)piperidin-4-yl)amino)cyclohexane-1-carbonyl)-6-oxo-6,8-dihydro-2H,7H-spiro[furo[2,3-e]isoindole-3,4'-piperidin]-7-yl)piperidine-2,6-dione | $^1$H NMR (400 MHz, DMSO-$d_6$) δ 11.00 (s, 1H), 9.14 (s, 1H), 8.26 (s, 2H), 7.41 (d, $J$ = 7.6 Hz, 1H), 7.29 (d, $J$ = 7.6 Hz, 1H), 7.17 – 7.11 (m, 3H), 6.89 – 6.79 (m, 2H), 6.70 – 6.54 (m, 4H), 6.48 (dd, $J$ = 8.3, 2.6 Hz, 1H), 6.23 (d, $J$ = 8.4 Hz, 2H), 5.09 (dd, $J$ = 13.2, 5.1 Hz, 1H), 4.64 (s, 1H), 4.41 – 4.37 (m, 2H), 4.22 (d, $J$ = 17.1 Hz, 2H), 4.15 (d, $J$ = 5.0 Hz, 2H), 3.91 (d, $J$ = 13.8 Hz, 1H), 3.69 – 3.59 (m, 2H), 3.35 – 3.13 (m, 4H), 3.06 – 2.83 (m, 4H), 2.73 – 2.71 (m, 1H), 2.68 – 2.57 (m, 3H), 2.44 – 2.38 (m, 1H), 2.11 – 2.05 (m, 1H), 2.01 – 1.96 (m, 3H), 1.91 – 1.72 (m, 10H), 1.58 – 1.51 (m, 4H). | 862.66 | General Synthesis Formula 33 |
| **161** | C1 | 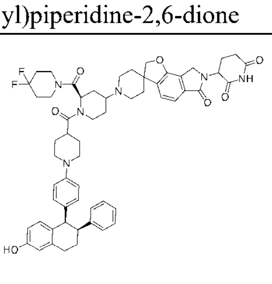 3-(1-(1'-((2R)-2-(4,4-Difluoropiperidin-1-yl)carbonyl)-1-(1- | $^1$H NMR (600 MHz, DMSO-$d_6$) δ 10.99 (s, 1H), 8.73 (s, 1H), 7.36 (d, $J$ = 7.5 Hz, 1H), 7.29 (d, $J$ = 7.6 Hz, 1H), 7.25 – 7.06 (m, 3H), 6.83 (d, $J$ = 7.3 Hz, 1H), 6.69 – 6.53 (m, 3H), 6.49 (dd, $J$ = 8.3, 2.5 Hz, 1H), 6.24 (s, 1H), 5.33 (t, $J$ = 4.9 Hz, 1H), 5.10 (dd, $J$ = 13.2, 5.2 Hz, 1H), 4.68 (q, $J$ = 9.9 Hz, 2H), 4.57 (q, $J$ = 6.9 Hz, 1H), | 995.60 | General Synthesis Formula 16 |

| | | | | | |
|---|---|---|---|---|---|
| | | (4-((1R,2S)-6-hydroxy-2-phenyl-1,2,3,3,4-tetrahydronaphthalen-1-yl)phenyl)piperidine-4-carbonyl)-4-dihydrodiphenyl-2,6-dione | 4.40 (d, *J* = 17.2 Hz, 1H), 4.24 (d, *J* = 17.2 Hz, 1H), 4.15 (d, *J* = 4.9 Hz, 1H), 3.83 – 3.48 (m, 9H), 3.14 – 2.88 (m, 5H), 2.82 (d, *J* = 21.2 Hz, 1H), 2.32 – 1.79 (m, 12H), 1.69 (dd, *J* = 27.0, 16.9 Hz, 3H), 1.52 (d, *J* = 7.0 Hz, 3H), 1.33 – 1.15 (m, 6H), 0.86 (t, *J* = 6.9 Hz, 1H). | | |
| **162** | C2 | 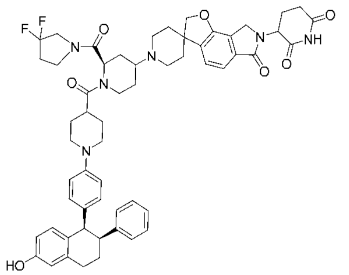 3-(1'-((2R)-2-(3,3-Difluoropyrrolidin-1-carbonyl)-1-(1-(4-(((1R,2S)-6-hydroxy-2-phenyl-1,2,3,4-tetrahydronaphthalen-1-yl)phenyl)piperidine-4-carbonyl)piperidin-4-yl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 10.99 (s, 1H), 8.82 (s, 1H), 7.36 (d, J = 7.5 Hz, 1H), 7.29 (d, J = 7.6 Hz, 1H), 7.15 (t, J = 7.4 Hz, 2H), 7.12 (t, J = 7.1 Hz, 1H), 6.84 (d, J = 7.5 Hz, 2H), 6.65 (d, J = 8.3 Hz, 1H), 6.61 (d, J = 2.5 Hz, 1H), 6.58 (d, J = 8.2 Hz, 2H), 6.49 (dd, J = 8.3, 2.5 Hz, 1H), 6.23 (d, J = 8.2 Hz, 2H), 5.33 (t, J = 5.0 Hz, 1H), 5.10 (dd, J = 13.3, 5.1 Hz, 1H), 4.68 (d, J = 4.5 Hz, 1H), 4.56 (q, J = 7.0 Hz, 1H), 4.40 (d, J = 17.2 Hz, 1H), 4.24 (d, J = 17.1 Hz, 1H), 4.14 (d, J = 5.0 Hz, 1H), 4.09 – 3.93 (m, 2H), 3.81 (t, J = 14.0 Hz, 2H), 3.73 – 3.66 (m, 2H), 3.02 – 2.87 (m, 5H), 2.70 – 2.54 (m, 4H), 2.34 – 2.22 (m, 3H), 2.04 – 1.89 (m, 6H), 1.76 – 1.49 (m, 8H), 1.33 – 1.18 (m, 6H), 0.86 (t, J = 6.9 Hz, 1H). | 981.60 | General Synthesis Formula 16 |
| **163** | C3 | 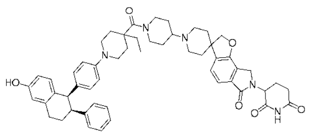 3-(1-(4-Ethyl-1-(4-(1R,2S)-6-hydroxy-2-phenyl-1,2,3,4-tetrahydronaphthalen-1-yl)phenyl)piperidine-4-carbonyl)piperidin-4-yl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 10.99 (s, 1H), 9.13 (s, 1H), 7.33 (d, *J* = 12.1 Hz, 2H), 7.15 (dd, *J* = 8.0, 6.2 Hz, 2H), 7.13 – 7.10 (m, 1H), 6.85 – 6.82 (m, 2H), 6.63 (d, *J* = 8.4 Hz, 1H), 6.60 (d, *J* = 2.5 Hz, 1H), 6.51 – 6.46 (m, 3H), 6.20 (d, *J* = 8.5 Hz, 2H), 5.35 – 5.32 (m, 1H), 5.11 – 5.08 (m, 1H), 4.46 (s, 2H), 4.39 (d, *J* = 17.1 Hz, 2H), 4.24 (s, 1H), 4.12 (d, *J* = 5.0 Hz, 1H), 3.00 – 2.88 (m, 5H), 2.74 (t, *J* = 11.3 Hz, 3H), 2.62 (dq, *J* = 5.1, 3.2, 2.5 Hz, 2H), 2.17 (d, *J* = 13.0 Hz, 3H), 2.00 (dt, *J* = 18.9, 7.1 Hz, 4H), 1.70 (d, *J* = 10.9 Hz, 2H), 1.65 (d, *J* = 7.6 Hz, 2H), 1.47 (dd, *J* = 17.6, 10.4 Hz, 4H), 1.29 (d, *J* = 7.0 Hz, 1H), 1.26 – 1.22 (m, 6H), 0.86 (t, *J* = 6.9 Hz, 2H), 0.78 (t, *J* = 7.3 Hz, 3H). | 976.60 | General Synthesis Formula 15 |

| | | | | | |
|---|---|---|---|---|---|
| **164** | C4 | 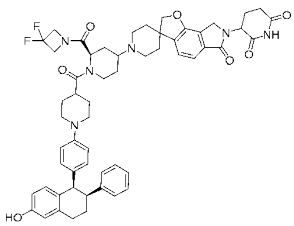 3-(1'-((2R)-2-(3,3-Difluoroazetidin-1-carbonyl)-1-(1-(4-((1R,2S)-6-hydroxy-2-phenyl-1,2,3,4-tetrahydronaphthalen-1-yl)phenyl)piperidine-4-carbonyl)piperidin-4-yl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 10.99 (s, 1H), 9.15 (s, 1H), 7.36 (d, $J$ = 7.5 Hz, 1H), 7.29 (d, $J$ = 7.6 Hz, 1H), 7.15 (dt, $J$ = 13.8, 6.8 Hz, 3H), 6.85 – 6.83 (m, 2H), 6.72 (s, 1H), 6.69 – 6.60 (m, 3H), 6.49 (dd, $J$ = 8.3, 2.5 Hz, 1H), 6.31 (s, 2H), 5.10 (dd, $J$ = 13.3, 5.1 Hz, 1H), 4.67 (h, $J$ = 17.7, 13.5 Hz, 5H), 4.40 (d, $J$ = 17.2 Hz, 3H), 4.28 – 4.18 (m, 4H), 3.35 – 3.30 (m, 2H), 3.20 (d, $J$ = 10.8 Hz, 1H), 3.08 – 2.83 (m, 8H), 2.68 – 2.54 (m, 1H), 2.45 – 2.33 (m, 3H), 2.22 – 2.14 (m, 3H), 2.09 (dd, $J$ = 12.5, 6.1 Hz, 1H), 2.06 – 1.90 (m, 6H), 1.74 – 1.63 (m, 5H), 1.24 (s, 1H). | 967. 60 | General Synthesis Formula 16 |
| **165** | C5 | 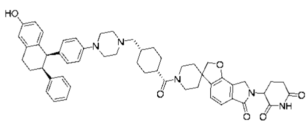 3-(4-(4-(1R,2S)-6-Hydroxy-2-phenyl-1,2,3,4-tetrahydronaphthalen-1-yl)phenyl)methyl)cyclohexane-1-carbonyl)-6-oxo-6,8-dihydro-2H,7H-spiro[isoindole-3,4'-piperidin]-7-yl)piperidine-2,6-dione | $^1$H NMR (600 MHz, DMSO-$d_6$) δ 10.98 (s, 1H), 9.14 (s, 1H), 7.41 (d, $J$ = 7.6 Hz, 1H), 7.28 (d, $J$ = 7.6 Hz, 1H), 7.21 (s, 1H), 7.18 – 7.11 (m, 3H), 6.85 (d, $J$ = 6.8 Hz, 2H), 6.69 – 6.60 (m, 4H), 6.49 (dd, $J$ = 8.3, 2.7 Hz, 1H), 6.27 (d, $J$ = 8.4 Hz, 1H), 5.33 (t, $J$ = 4.9 Hz, 1H), 5.11 – 5.07 (m, 1H), 4.68 – 4.61 (m, 2H), 4.39 (d, $J$ = 13.7 Hz, 2H), 4.24 (t, $J$ = 8.7 Hz, 1H), 4.18 (d, $J$ = 5.1 Hz, 1H), 3.97 (d, $J$ = 13.8 Hz, 1H), 3.64 (s, 2H), 3.55 (s, 2H), 3.21 – 3.16 (m, 2H), 3.07 (d, $J$ = 11.1 Hz, 2H), 3.02 – 2.98 (m, 2H), 2.92 (d, $J$ = 12.5 Hz, 2H), 2.61 (s, 1H), 2.00 (dt, $J$ = 12.4, 6.8 Hz, 3H), 1.84 – 1.80 (m, 2H), 1.73 (s, 2H), 1.47 (q, $J$ = 7.5, 7.1 Hz, 2H), 1.32 – 1.27 (m, 2H), 1.26 – 1.22 (m, 7H), 1.08 (d, $J$ = 12.9 Hz, 1H), 0.86 (t, $J$ = 6.9 Hz, 2H). | 862. .65 | General Synthesis Formula 26 |
| **166** | C6 | 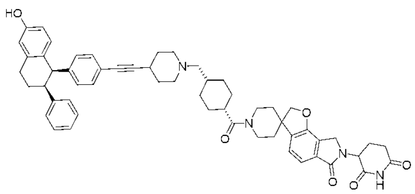 3-(4-(4-(1R,2S)-6-Hydroxy-2-phenyl-1,2,3,4-tetrahydronaphthalen-1-yl)phenyl)ethynyl)piperidin-1-yl)methyl)cyclohexane-1-carbonyl)-6-oxo-6,8-dihydro-2H,7H-spiro[2,3- | $^1$H NMR (600 MHz, DMSO-$d_6$) δ 10.99 (s, 1H), 8.79 (s, 1H), 7.42 (d, $J$ = 7.6 Hz, 1H), 7.28 (d, $J$ = 7.6 Hz, 1H), 7.21 (s, 1H), 7.17 – 7.14 (m, 2H), 7.09 – 7.08 (m, 1H), 7.01 – 6.99 (m, 1H), 6.87 – 6.84 (m, 2H), 6.67 (s, 1H), 6.63 (s, 1H), 6.50 (dd, $J$ = 8.1, 2.5 Hz, 1H), 6.38 (dd, $J$ = 8.1, 5.7 Hz, 2H), 5.11 – 5.07 (m, 2H), 4.66 (d, $J$ = 11.6 Hz, 2H), 4.40 (d, $J$ = 13.4 Hz, 2H), 4.28 (dd, $J$ = 10.3, 5.2 Hz, 2H), 3.97 (d, $J$ = | 885. 65 | General Synthesis Formula 39 |

| | | | | | |
|---|---|---|---|---|---|
| | | e]isoindole-3,4'-piperidin]-7-yl)piperidine-2,6-dione | 13.3 Hz, 3H), 3.51 (d, *J* = 11.4 Hz, 2H), 3.00 (s, 2H), 2.91 (s, 2H), 2.78 (s, 1H), 2.71 (s, 1H), 2.63 – 2.61 (m, 2H) , 2.11 (s, 1H), 2.09 (s, 1H), 2.01 (t, *J* = 7.4 Hz, 3H), 1.99 (d, *J* = 6.9 Hz, 2H), 1.80 (s, 4H), 1.74 (d, *J* = 12.8 Hz, 5H), 1.48 – 1.44 (m, 3H), 1.06 (s, 1H), 0.86 (t, *J* = 6.9 Hz, 3H). | | |
| **167** | C7 | 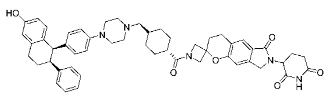 3-(4-(1R,2S)-6-Hydroxy-2-phenyl-1,2,3,4-tetrahydronaphthalen-1-yl)phenyl)piperazin-1-yl)methyl)cyclohexane-1-carbonyl)-6-oxo-3,4,6,8-tetrahydro-7spiro[pyrano[2,3-f]isoindole-3,2'-pyran]-7-yl)piperidine-2,6-dione | $^1$H NMR (600 MHz, DMSO-$d_6$) δ 10.97 (d, *J* = 3.6 Hz, 1H), 9.16 (s, 1H), 7.51 (s, 1H), 7.16 (dd, *J* = 7.9, 6.1 Hz, 2H), 7.14 – 7.11 (m, 1H), 7.05 (d, *J* = 2.0 Hz, 1H), 6.86 – 6.83 (m, 2H), 6.65 – 6.60 (m, 4H), 6.49 (dd, *J* = 8.3, 2.6 Hz, 1H), 6.27 (d, *J* = 8.3 Hz, 2H), 5.06 (ddd, *J* = 12.6, 7.0, 5.0 Hz, 1H), 4.35 (dd, *J* = 17.1, 5.2 Hz, 1H), 4.29 (d, *J* = 9.3 Hz, 1H), 4.20 (dt, *J* = 24.8, 7.4 Hz, 3H), 3.97 (d, *J* = 10.4 Hz, 1H), 3.84 (d, *J* = 10.4 Hz, 1H), 3.55 (s, 3H), 3.31 (dt, *J* = 10.7, 2.8 Hz, 1H), 3.06 (d, *J* = 11.2 Hz, 2H), 2.99 (p, *J* = 4.4, 3.4 Hz, 3H), 2.91 (qd, *J* = 13.4, 12.5, 6.7 Hz, 6H), 2.62 – 2.57 (m, 1H), 2.36 (td, *J* = 8.0, 4.0 Hz, 1H), 2.26 – 2.02 (m, 5H), 1.98 (d, *J* = 9.4 Hz, 1H), 1.82 – 1.70 (m, 6H), 1.38 (q, *J* = 12.5 Hz, 2H), 1.00 (q, *J* = 12.3 Hz, 2H). | 848. 60 | General Synthesis Formula 26 |
| **168** | C8 | 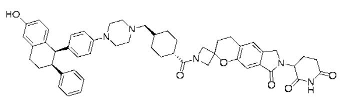 3-(4-(1R,2S)-6-Hydroxy-2-phenyl-1,2,3,4-tetrahydronaphthalen-1-yl)phenyl)piperazin-1-yl)methyl)cyclohexane-1-carbonyl)-8-oxo-3,4,6,8-tetrahydro-7-spiro[azetidine-3,2-pyrano[2,3-f]isoindol]-7-yl)piperidine-2,6-dione | $^1$H NMR (600 MHz, DMSO-$d_6$) δ 10.98 (d, *J* = 3.5 Hz, 1H), 9.16 (s, 1H), 7.36 (s, 1H), 7.17 – 7.09 (m, 4H), 6.85 (dd, *J* = 6.9, 1.6 Hz, 2H), 6.65 – 6.60 (m, 4H), 6.49 (dd, *J* = 8.3, 2.6 Hz, 1H), 6.27 (d, *J* = 8.5 Hz, 2H), 5.10 – 5.06 (m, 1H), 4.34 (dd, *J* = 16.9, 2.6 Hz, 1H), 4.27 (d, *J* = 9.2 Hz, 1H), 4.24 – 4.20 (m, 1H), 4.18 (dd, *J* = 11.7, 4.7 Hz, 2H), 3.95 (d, *J* = 10.4 Hz, 1H), 3.83 (d, *J* = 10.3 Hz, 2H), 3.53 (d, *J* = 10.9 Hz, 3H), 3.33 – 3.30 (m, 1H), 3.06 (d, *J* = 10.9 Hz, 2H), 2.99 (td, *J* = 7.3, 6.5, 3.1 Hz, 3H), 2.91 (dt, *J* = 23.3, 10.9 Hz, 6H), 2.62 – 2.58 (m, 1H), 2.41 – 2.35 (m, 1H), 2.21 (td, *J* = 10.2, 8.7, 5.5 Hz, 1H), 2.14 (p, *J* = 5.8 Hz, 2H), 2.11 – 2.06 (m, | 848. 60 | General Synthesis Formula 26 |

| | | | | | |
|---|---|---|---|---|---|
| | | | 1H), 2.00 – 1.96 (m, 1H), 1.84 – 1.71 (m, 6H), 1.38 (q, *J* = 12.5 Hz, 2H), 0.99 (q, *J* = 12.4 Hz, 2H). | | |
| **169** | C9 | 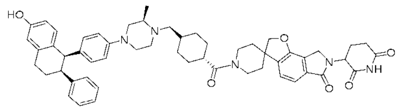 3-(1S,4S)-4-(4-(1R,2S)-6-Hydroxy-2-phenyl-1,2,3,4-tetrahydronaphthalen-1-yl)phenyl)-2-methylpiperazin-1-yl)methyl)cyclohexane-1-carbonyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 10.99 (s, 1H), 8.90 (s, 1H), 7.42 (d, *J* = 7.6 Hz, 1H), 7.28 (d, *J* = 7.6 Hz, 1H), 7.17 – 7.12 (m, 3H), 6.86 (dd, *J* = 9.5, 7.7 Hz, 2H), 6.65 – 6.61 (m, 4H), 6.49 (dd, *J* = 8.3, 2.4 Hz, 1H), 6.27 (dd, *J* = 8.7, 4.1 Hz, 2H), 5.09 (dd, *J* = 12.5, 6.1 Hz, 1H), 4.65 (t, *J* = 11.1 Hz, 2H), 4.40 (dd, *J* = 17.0, 5.4 Hz, 2H), 4.26 – 4.22 (m, 1H), 4.18 (t, *J* = 4.4 Hz, 1H), 3.96 (s, 1H), 3.33 – 3.30 (m, 2H), 3.18 (d, *J* = 12.3 Hz, 2H), 3.12 – 3.07 (m, 1H), 3.00 (d, *J* = 10.5 Hz, 1H), 2.98 – 2.85 (m, 5H), 2.74 (dd, *J* = 13.5, 10.6 Hz, 2H), 2.63 – 2.57 (m, 2H), 2.43 (d, *J* = 13.7 Hz, 1H), 2.11 – 2.06 (m, 1H), 2.00 – 1.96 (m, 1H), 1.95 – 1.90 (m, 1H), 1.87 – 1.70 (m, 10H), 1.50 (d, *J* = 12.9 Hz, 1H), 1.41 (d, *J* = 12.8 Hz, 1H), 1.31 (dd, *J* = 10.5, 6.4 Hz, 3H), 1.26 – 1.23 (m, 1H), 1.19 – 1.12 (m, 1H), 1.08 (d, *J* = 12.3 Hz, 1H). | 876.65 | General Synthesis Formula 26 |
| **170** | C10 | 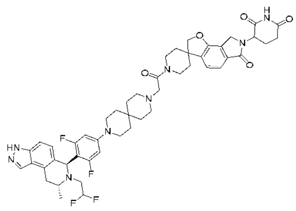 3-(1'-(2-(9-(4-((6S,8R)-7-(2,2-Difluoroethyl)-8-methyl-6,7,8,9-tetrahydro-3H-pyrazolo[4,3-f]isoquinolin-6-yl)-3,5-difluorophenyl)-3,9-diazaspiro[5.5]undecan-3-yl)acetyl)-6-oxo-6,8-dihydro-2H,7H-spiro[furo[2,3-e]isoindole-3,4'-piperidin]-7-yl)piperidine-2,6-dione | $^1$H NMR (400 MHz, DMSO-$d_6$) δ 12.99 (s, 1H), 10.99 (s, 1H), 8.07 (s, 1H), 7.38 (d, *J* = 7.6 Hz, 1H), 7.32 (d, *J* = 7.6 Hz, 1H), 7.23 (d, *J* = 8.7 Hz, 1H), 6.72 (d, *J* = 8.6 Hz, 1H), 6.56 (d, *J* = 13.6 Hz, 2H), 5.17 (s, 1H), 5.12 – 5.08 (m, 1H), 4.72 – 4.65 (m, 2H), 4.38 (d, *J* = 5.5 Hz, 2H), 4.26 (d, *J* = 7.3 Hz, 1H), 3.09 – 2.87 (m, 8H), 2.68 (p, *J* = 1.9 Hz, 2H), 2.33 (p, *J* = 1.9 Hz, 1H), 2.03 – 1.99 (m, 2H), 1.90 (d, *J* = 11.9 Hz, 6H), 1.68 (s, 4H), 1.47 (d, *J* = 7.0 Hz, 4H), 1.24 (s, 8H), 0.86 (t, *J* = 6.7 Hz, 3H). | 911.65 | General Synthesis Formula 45 |
| **171** | C11 | 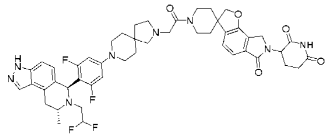 | $^1$H NMR (600 MHz, DMSO-$d_6$) δ 13.01 (s, 1H), 10.99 (s, 1H), 8.08 (s, 1H), 7.37 (d, *J* = 7.6 Hz, 1H), 7.32 (d, *J* = 7.5 Hz, 1H), 7.24 (d, *J* = 8.6 Hz, 1H), 6.73 (d, *J* = | 897.60 | General Synthesis Formula 45 |

| | | | | | |
|---|---|---|---|---|---|
| | | 3-(1'-(2-(8-(4-((6S,8R)-7-(2,2-Difluoroethyl)-8-methyl-6,7,8,9-tetrahydro-3H-pyrazolo[4,3-f]isoquinolin-6-yl)-3,5-difluorophenyl)-2,8-diazaspiro[4.5]decan-2-yl)acetyl)-6-oxo-6,8-dihydro-2H,7H-spiro[furo[2,3-e]isoindole-3,4'-piperidin]-7-yl)piperidine-2,6-dione | 8.6 Hz, 1H), 6.58 (d, $J$ = 13.2 Hz, 2H), 5.83 (s, 1H), 5.19 (s, 1H), 5.12 – 5.08 (m, 1H), 4.74 – 4.63 (m, 3H), 4.53 – 4.47 (m, 2H), 4.40 (qd, $J$ = 16.1, 15.0, 8.5 Hz, 4H), 4.24 (dd, $J$ = 17.1, 11.0 Hz, 2H), 3.72 – 3.56 (m, 4H), 3.50 – 3.46 (m, 1H), 3.29 – 3.18 (m, 6H), 2.96 – 2.87 (m, 3H), 2.03 – 1.97 (m, 2H), 1.90 (dt, $J$ = 14.3, 9.1 Hz, 2H), 1.79 (d, $J$ = 10.9 Hz, 3H), 1.75 – 1.62 (m, 4H), 1.24 (s, 1H), 1.07 (d, $J$ = 6.5 Hz, 3H) | | |
| **172** | C12 | 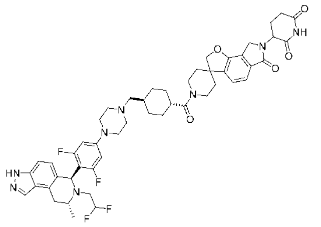 3-(1'-((1S,4R)-4-((4-(4-((6S,8R)-7-(2,2-difluoroethyl)-8-methyl-6,7,8,9-tetrahydro-3H-pyrazolo[4,3-f]isoquinolin-6-yl]-3,5-difluorophenyl)piperazin-1-yl)methyl)cyclohexane-1-carbonyl)-6-oxo-6,8-dihydro-2H,7H-spiro[furo[2,3-e]isoindole-3,4'-piperidin]-7-yl)piperidine-2,6-dione | $^1$H NMR (400 MHz, DMSO-$d_6$) δ 13.01 (s, 1H), 10.99 (s, 1H), 8.09 (d, $J$ = 9.7 Hz, 1H), 7.41 (dd, $J$ = 8.4, 6.0 Hz, 2H), 7.30 – 7.18 (m, 4H), 6.75 – 6.63 (m, 4H), 5.32 (t, $J$ = 4.9 Hz, 2H), 5.20 (s, 1H), 5.09 (dd, $J$ = 13.1, 5.0 Hz, 2H), 4.64 (d, $J$ = 7.4 Hz, 3H), 4.40 (d, $J$ = 16.3 Hz, 3H), 4.25 (s, 2H), 2.00 (q, $J$ = 7.1, 6.7 Hz, 5H), 1.34 (d, $J$ = 6.0 Hz, 3H), 1.24 (d, $J$ = 6.0 Hz, 19H), 0.85 (t, $J$ = 6.7 Hz, 3H). | 925.65 | General Synthesis Formula 52 |
| **173** | C13 | 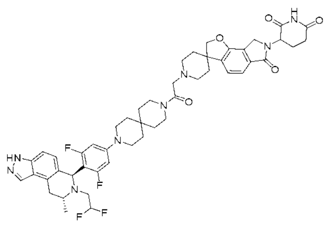 3-(1'-(2-(9-(4-((6S,8R)-7-(2,2-Difluoroethyl)-8-methyl-6,7,8,9-tetrahydro-3H-pyrazolo[4,3-f]isoquinolin-6-yl)-3,5-difluorophenyl)-3,9-diazaspiro[5.5]undecan-3-yl)-2-oxoethyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 13.00 (s, 1H), 11.00 (s, 1H), 8.08 (s, 1H), 7.39 – 7.35 (m, 1H), 7.31 (d, $J$ = 7.7 Hz, 1H), 7.24 (d, $J$ = 8.6 Hz, 1H), 6.73 (d, $J$ = 8.7 Hz, 1H), 6.55 (d, $J$ = 13.8 Hz, 2H), 5.83 (s, 1H), 5.19 (s, 1H), 5.10 (dd, $J$ = 13.3, 5.1 Hz, 1H), 4.69 – 4.62 (m, 2H), 4.51 (d, $J$ = 4.8 Hz, 1H), 4.40 (dd, $J$ = 17.2, 5.3 Hz, 2H), 3.59 – 3.47 (m, 5H), 3.43 – 3.32 (m, 3H), 3.22 (d, $J$ = 11.6 Hz, 5H), 2.92 (ddd, $J$ = 18.1, 13.4, 5.2 Hz, 2H), 2.43 (dt, $J$ = 13.2, 6.6 Hz, 2H), 2.36 – 2.25 (m, 2H), 2.04 – 1.90 (m, 4H), 1.59 – 1.52 (m, 5H), 1.47 (p, $J$ = 6.9 Hz, 3H), 1.24 (d, $J$ = 5.4 Hz, 3H), 1.07 (d, $J$ = 6.4 Hz, 3H). | 911.80 | General Synthesis Formula 46 |

| | | | | | |
|---|---|---|---|---|---|
| **174** | C14 | 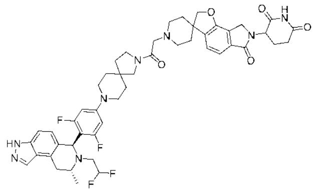 3-(1'-(2-(8-(4-((6S,8R)-7-(2,2-Difluoroethyl)-8-methyl-6,7,8,9-tetrahydro-3H-pyrazolo[4,3-f]isoquinolin-6-yl)-3,5-difluorophenyl)-2,8-diazaspiro[4.5]decan-2-yl)-2-oxoethyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 13.01 (s, 1H), 11.00 (s, 1H), 8.08 (t, $J$ = 1.3 Hz, 1H), 7.37 (d, $J$ = 7.6 Hz, 1H), 7.31 (d, $J$ = 7.6 Hz, 1H), 7.24 (d, $J$ = 8.5 Hz, 1H), 6.73 (dd, $J$ = 8.7, 3.9 Hz, 1H), 6.62 – 6.54 (m, 2H), 5.83 (s, 1H), 5.21 – 5.16 (m, 1H), 5.10 (dd, $J$ = 13.2, 5.2 Hz, 1H), 4.70 – 4.52 (m, 3H), 4.40 (dd, $J$ = 17.2, 5.6 Hz, 2H), 4.27 – 4.17 (m, 4H), 3.56 – 3.45 (m, 5H), 3.35 (s, 4H), 3.25 – 3.11 (m, 5H), 2.95 – 2.89 (m, 2H), 1.98 (td, $J$ = 13.5, 11.5, 6.2 Hz, 3H), 1.89 (t, $J$ = 6.9 Hz, 1H), 1.80 (t, $J$ = 7.3 Hz, 1H), 1.60 (s, 4H), 1.29 – 1.21 (m, 3H), 1.06 (d, $J$ = 6.5 Hz, 3H). | 897.80 | General Synthesis Formula 46 |
| **175** | C15 | 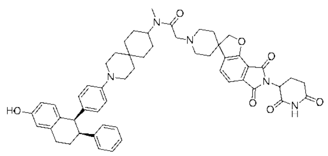 2-(7-(2,6-Dioxopiperidin-3-yl)-6,8-dioxo-7,8-dihydro-2H,6H-spiro[furo[2,3-e]isoindole-3,4'-piperidin]-1'-yl)-N-(3-(4-((1R,2S)-6-hydroxy-2-phenyl-1,2,3,4-tetrahydronaphthalen-1-yl)phenyl)-3-azaspiro[5.5]undecan-9-yl)-N-methylacetamide | $^1$H NMR (400 MHz, DMSO-$d_6$) δ 11.13 (s, 1H), 9.18 (s, 1H), 7.60 (d, $J$ = 7.4 Hz, 1H), 7.51 (dd, $J$ = 7.4, 1.6 Hz, 1H), 7.15 (q, $J$ = 6.9, 6.4 Hz, 3H), 6.86 – 6.81 (m, 2H), 6.66 – 6.60 (m, 2H), 6.49 (dd, $J$ = 8.3, 2.5 Hz, 1H), 6.35 (s, 2H), 5.13 – 5.09 (m, 1H), 4.80 (s, 2H), 4.36 (s, 1H), 4.26 (s, 3H), 3.52 (d, $J$ = 11.7 Hz, 2H), 3.34 (d, $J$ = 15.2 Hz, 2H), 3.00 – 2.82 (m, 6H), 2.62 – 2.56 (m, 1H), 2.27 (d, $J$ = 14.0 Hz, 3H), 2.00 (d, $J$ = 14.0 Hz, 6H), 1.72 (q, $J$ = 12.4, 11.1 Hz, 6H), 1.50 (d, $J$ = 11.8 Hz, 4H), 1.36 (d, $J$ = 11.1 Hz, 2H), 1.24 (s, 4H). | 890.65 | General Synthesis Formula 41 |
| **176** | C17 | 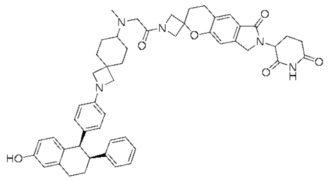 3-(1-(N-(2-(4-((1R,2S)-6-Hydroxy-2-phenyl-1,2,3,4-tetrahydronaphthalen-1-yl)phenyl)-2-azaspiro[3.5]nonan-7-yl)-N-methylglycyl)-6'-oxo-3',4',6',8'-tetrahydro-7'H-spiro[azetidine-3,2'-pyrano[2,3-f]isoindol]-7'-yl)piperidine-2,6-dione | $^1$H NMR (600 MHz, DMSO-$d_6$) δ 10.98 (s, 1H), 9.11 (s, 1H), 7.53 (s, 1H), 7.14 (td, $J$ = 16.4, 15.7, 8.4 Hz, 3H), 7.06 (d, $J$ = 2.2 Hz, 1H), 6.83 (d, $J$ = 7.5 Hz, 2H), 6.64 – 6.59 (m, 2H), 6.48 (dd, $J$ = 8.3, 2.5 Hz, 1H), 6.18 (dd, $J$ = 8.5, 2.6 Hz, 2H), 6.04 (dt, $J$ = 8.7, 4.9 Hz, 2H), 5.09 – 5.05 (m, 1H), 4.37 – 4.21 (m, 5H), 4.17 – 4.09 (m, 4H), 3.45 (d, $J$ = 7.5 Hz, 1H), 3.41 (d, $J$ = 6.9 Hz, 1H), 3.35 (d, $J$ = 2.6 Hz, 2H), 3.28 (d, $J$ = 10.4 Hz, 1H), 3.20 – 3.15 (m, 1H), 2.99 – 2.86 (m, 5H), 2.74 (d, $J$ = 4.8 Hz, 2H), 2.62 – 2.58 (m, 1H), 2.37 (dd, $J$ = 12.5, 3.8 Hz, 1H), 2.17 (t, $J$ = 6.5 Hz, | 834.65 | General Synthesis Formula 41 |

| | | | | | |
|---|---|---|---|---|---|
| | | | 2H), 1.98 (dt, *J* = 11.8, 5.5 Hz, 4H), 1.70 (d, *J* = 6.2 Hz, 1H), 1.48 (d, *J* = 12.1 Hz, 3H), 1.29 – 1.21 (m, 4H). | | |
| **177** | C18 | 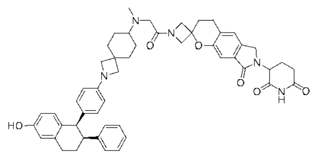 3-(1-(N-(2-(4-((1R,2S)-6-Hydroxy-2-phenyl-1,2,3,4-tetrahydronaphthalen-1-yl)phenyl)-2-azaspiro[3.5]nonan-7-yl)-N-methylglycyl)-8'-oxo-3',4',6',8'-tetrahydro-7'H-spiro[azetidine-3,2'-pyrano[2,3-f]isoindol]-7'-yl)piperidine-2,6-dione | $^1$H NMR (400 MHz, DMSO-$d_6$) δ 10.99 (s, 1H), 9.11 (s, 1H), 7.38 (s, 1H), 7.16 – 7.10 (m, 4H), 6.84 – 6.81 (m, 2H), 6.62 (d, *J* = 8.4 Hz, 1H), 6.59 (d, *J* = 2.5 Hz, 1H), 6.47 (d, *J* = 5.7 Hz, 1H), 6.17 (d, *J* = 8.0 Hz, 2H), 6.04 – 6.00 (m, 2H), 5.08 (dd, *J* = 13.4, 5.1 Hz, 3H), 4.35 (d, *J* = 16.9 Hz, 3H), 4.29 (s, 3H), 4.15 – 4.10 (m, 3H), 3.99 (d, *J* = 10.7 Hz, 2H), 2.93 (dd, *J* = 16.9, 5.8 Hz, 7H), 2.74 (d, *J* = 4.2 Hz, 2H), 2.26 (s, 1H), 2.18 (s, 2H), 1.98 (s, 4H), 1.70 (s, 3H), 1.48 (d, *J* = 11.1 Hz, 3H), 1.24 (s, 3H). | 834.60 | General Synthesis Formula 41 |
| **178** | C19 | 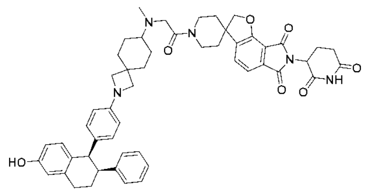 7-(2,6-Dioxopiperidin-3-yl)-1'-(N-(2-(4-((1R,2S)-6-hydroxy-2-phenyl-1,2,3,4-tetrahydronaphthalen-1-yl)phenyl)-2-azaspiro[3.5]nonan-7-yl)-N-methylglycyl)-7-hydro-2H,6H-spiro[furo[2,3-e]isoindole-3,4'-piperidine]-6,8-dione | $^1$H NMR (400 MHz, DMSO-$d_6$) δ 11.12 (s, 1H), 9.11 (s, 1H), 7.69 (dd, *J* = 7.3, 4.1 Hz, 1H), 7.46 (dd, *J* = 7.3, 2.1 Hz, 1H), 7.18 – 7.08 (m, 3H), 6.86 – 6.79 (m, 2H), 6.64 – 6.57 (m, 2H), 6.47 (dd, *J* = 8.2, 2.5 Hz, 1H), 6.18 (d, *J* = 8.2 Hz, 1H), 6.02 (d, *J* = 8.1 Hz, 1H), 5.10 (dd, *J* = 12.7, 5.5 Hz, 2H), 4.88 – 4.77 (m, 3H), 4.34 (d, *J* = 18.2 Hz, 3H), 4.21 (s, 2H), 4.11 (d, *J* = 5.0 Hz, 1H), 3.77 (d, *J* = 13.4 Hz, 2H), 2.90 (t, *J* = 18.4 Hz, 4H), 2.76 (dd, *J* = 11.9, 4.7 Hz, 2H), 2.62 (s, 1H), 2.01 (s, 8H), 1.84 (s, 3H), 1.70 (s, 2H), 1.50 (s, 5H), 1.24 (s, 3H). | 862.65 | General Synthesis Formula 41 |
| **179** | C22 | 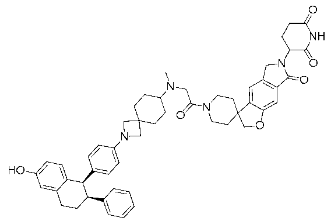 3-(1’-(N-(2-(4-((1R,2S)-6-Hydroxy-2-phenyl-1,2,3,4-tetrahydronaphthalen-1-yl)phenyl)-2-azaspiro[3.5]nonan-7-yl)-N-methylglycyl)-7-oxo-5,7-dihydro-2H,6H-spiro[furo[2,3-f]isoindole-3,4'-piperidin]-6-yl)piperidine-2,6-dione | $^1$H NMR (600 MHz, DMSO-$d_6$) δ 10.99 (s, 1H), 9.12 (s, 1H), 7.44 (d, *J* = 4.5 Hz, 1H), 7.16 – 7.10 (m, 3H), 7.07 (s, 1H), 6.86 – 6.81 (m, 2H), 6.63 – 6.59 (m, 2H), 6.48 (dd, *J* = 8.3, 2.5 Hz, 1H), 6.18 (d, *J* = 8.2 Hz, 2H), 6.03 (d, *J* = 8.2 Hz, 2H), 5.10 (dd, *J* = 9.1, 4.3 Hz, 1H), 4.65 – 4.60 (m, 2H), 4.35 (dd, *J* = 16.7, 5.2 Hz, 3H), 4.23 (dd, *J* = 16.7, 6.2 Hz, 2H), 4.11 (d, *J* = 4.9 Hz, 1H), 3.18 (d, *J* = 8.1 Hz, 3H), 3.01 – 2.87 (m, 5H), 2.79 – 2.72 (m, 3H), 2.61 (s, 1H), 2.38 (s, 1H), 2.09 (dd, *J* = | 848.65 | General Synthesis Formula 41 |

| | | | | | |
|---|---|---|---|---|---|
| | | | 12.2, 6.1 Hz, 2H), 2.03 – 1.90 (m, 6H), 1.79 (s, 3H), 1.70 (d, *J* = 6.2 Hz, 1H), 1.50 (d, *J* = 4.2 Hz, 4H), 1.29 – 1.20 (m, 3H). | | |
| **180** | C23 | 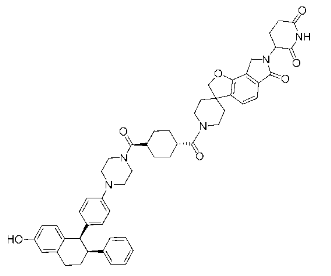 3-(1'-((1S,4S)-4-(4-(4-((1R,2S)-6-Hydroxy-2-phenyl-1,2,3,4-tetrahydronaphthalen-1-yl)phenyl)piperazine-1-carbonyl)cyclohexane-1-carbonyl)-6-oxo-6,8-dihydro-2H,7H-spiro[furo[2,3-e]isoindole-3,4'-piperidin]-7-yl)piperidine-2,6-dione | $^1$H NMR (400 MHz, DMSO-$d_6$) δ 11.00 (s, 1H), 9.14 (s, 1H), 7.42 (d, *J* = 7.6 Hz, 1H), 7.27 (d, *J* = 7.6 Hz, 1H), 7.13 (p, *J* = 7.4, 6.9 Hz, 4H), 6.85 – 6.82 (m, 2H), 6.64 – 6.58 (m, 4H), 6.50 – 6.47 (m, 1H), 6.24 (d, *J* = 8.1 Hz, 2H), 5.09 (dd, *J* = 13.4, 5.1 Hz, 1H), 4.63 (q, *J* = 9.9, 7.9 Hz, 2H), 4.39 (d, *J* = 17.2 Hz, 2H), 4.24 (s, 1H), 4.16 (d, *J* = 5.0 Hz, 1H), 3.64 – 3.48 (m, 7H), 3.00 (d, *J* = 6.9 Hz, 2H), 2.96 (s, 4H), 2.63 (dd, *J* = 17.4, 9.6 Hz, 3H), 2.09 (dd, *J* = 9.6, 4.5 Hz, 1H), 2.00 (d, *J* = 7.2 Hz, 1H), 1.69 (d, *J* = 16.8 Hz, 7H), 1.47 (q, *J* = 24.0, 17.7 Hz, 6H), 1.27 – 1.22 (m, 2H). | 776.70 | General Synthesis Formula 31 |
| **181** | C24 | 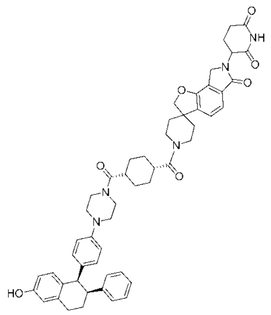 3-(1'-((1S,4S)-4-(4-(4-((1R,2S)-6-Hydroxy-2-phenyl-1,2,3,4-tetrahydronaphthalen-1-yl)phenyl)piperazine-1-carbonyl)cyclohexane-1-carbonyl)-6-oxo-6,8-dihydro-2H,7H-spiro[furo[2,3-e]isoindole-3,4'-piperidin]-7-yl)piperidine-2,6-dione | $^1$H NMR (400 MHz, DMSO-$d_6$) δ 11.00 (s, 1H), 9.13 (s, 1H), 7.42 (d, *J* = 7.6 Hz, 1H), 7.26 (d, *J* = 7.5 Hz, 1H), 7.18 – 7.11 (m, 3H), 6.86 – 6.82 (m, 2H), 6.66 – 6.59 (m, 4H), 6.48 (dd, *J* = 8.3, 2.5 Hz, 1H), 6.25 (d, *J* = 8.3 Hz, 2H), 5.09 (dd, *J* = 13.2, 5.1 Hz, 1H), 4.63 (t, *J* = 5.3 Hz, 2H), 4.39 (d, *J* = 16.9 Hz, 3H), 3.96 – 3.88 (m, 3H), 3.55 (s, 4H), 3.30 (d, *J* = 10.4 Hz, 1H), 3.04 – 2.89 (m, 7H), 2.77 (d, *J* = 7.7 Hz, 2H), 2.13 – 1.94 (m, 3H), 1.75 (q, *J* = 18.4, 9.6 Hz, 9H), 1.52 (s, 4H), 1.25 (d, *J* = 8.9 Hz, 2H). | 776.70 | General Synthesis Formula 30 |
| **182** | D1 | 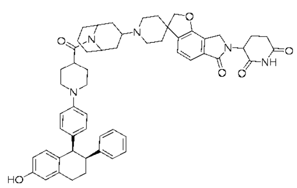 3-(1'-(9-(1-(4-(((1R,2S)-6-Hydroxy-2-phenyl-1,2,3,4-tetrahydronaphthalen-1-yl)phenyl)piperidine-4-carbonyl)-9- | $^1$H NMR (600 MHz, DMSO-$d_6$) δ 10.98 (s, 1H), 7.37 (d, *J* = 7.5 Hz, 1H), 7.30 (d, *J* = 7.6 Hz, 1H), 7.14 (dt, *J* = 13.7, 7.0 Hz, 3H), 6.83 (d, *J* = 7.3 Hz, 2H), 6.69 (dd, *J* = 43.1, 9.4 Hz, 3H), 6.61 (d, *J* = 2.6 Hz, 1H), 6.49 (dd, *J* = 8.3, 2.5 Hz, 1H), 6.30 (d, *J* = 8.2 Hz, 2H), 5.09 (dd, *J* = 13.2, 5.1 Hz, 1H), 4.71 – 4.56 (m, 3H), 4.40 (d, *J* = | 888.76 | General Synthesis Formula 15 |

| | | | | | |
|---|---|---|---|---|---|
| | | azabicyclo[3.3.1]nonan-3-yl)-6-oxo-6,8-dihydro-2H,7H-spiro[furo[2,3-e]isoindole-3,4'-piperidin]-7-yl)piperidine-2,6-dione | 17.2 Hz, 1H), 4.28 – 4.15 (m, 3H), 3.78 (s, 2H), 3.07 – 2.79 (m, 10H), 2.61 (d, *J* = 3.5 Hz, 1H), 2.54 (s, 1H), 2.40 (dt, *J* = 25.3, 14.8 Hz, 5H), 2.23 – 1.93 (m, 6H), 1.72 (td, *J* = 77.5, 68.2, 24.0 Hz, 11H), 1.46 (d, *J* = 11.4 Hz, 1H). | | |
| **183** | D2 | 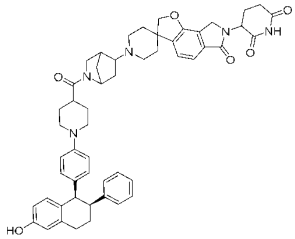 3-(1'-(2-(1-(4-(((1R,2S)-6-Hydroxy-2-phenyl-1,2,3,4-tetrahydronaphthalen-1-yl)phenyl)piperidine-4-carbonyl)-2-azabicyclo[2.2.1]heptan-5-yl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 10.98 (s, 1H), 9.26 (s, 1H), 7.35 (d, *J* = 7.6 Hz, 1H), 7.25 (d, *J* = 7.6 Hz, 1H), 7.14 (dt, *J* = 13.1, 7.1 Hz, 3H), 6.83 (d, *J* = 7.3 Hz, 2H), 6.75 (s, 2H), 6.65 (dd, *J* = 8.4, 2.5 Hz, 1H), 6.62 (d, *J* = 2.5 Hz, 1H), 6.49 (dd, *J* = 8.3, 2.5 Hz, 1H), 6.31 (d, *J* = 8.1 Hz, 2H), 5.08 (dd, *J* = 13.2, 5.2 Hz, 1H), 4.66 (t, *J* = 4.1 Hz, 2H), 4.49 (s, 1H), 4.39 (d, *J* = 17.1 Hz, 1H), 4.30 – 4.16 (m, 3H), 3.31 (td, *J* = 13.1, 12.0, 3.9 Hz, 4H), 3.17 (s, 1H), 3.06 (s, 1H), 3.03 – 2.96 (m, 2H), 2.92 (dtd, *J* = 18.0, 12.9, 12.0, 6.0 Hz, 3H), 2.84 (s, 1H), 2.66 (d, *J* = 7.9 Hz, 1H), 2.64 – 2.57 (m, 1H), 2.54 (s, 1H), 2.47 – 2.38 (m, 1H), 2.35 – 2.26 (m, 1H), 2.20 (t, *J* = 14.0 Hz, 2H), 2.08 (dd, *J* = 12.9, 6.3 Hz, 1H), 2.02 – 1.89 (m, 4H), 1.81 – 1.66 (m, 6H), 1.61 (d, *J* = 13.9 Hz, 1H). | 860.76 | General Synthesis Formula 15 |
| **184** | D3 | 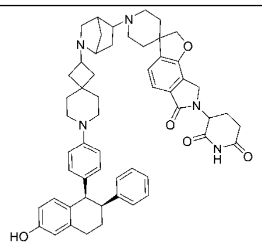 3-(1'-(2-(4-(((1R,2S)-6-Hydroxy-2-phenyl-1,2,3,4-tetrahydronaphthalen-1-yl)phenyl)-7-azaspiro[3.5]nonan-2-yl)-2-azabicyclo[2.2.1]heptan-5-yl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 10.99 (s, 1H), 9.16 (s, 1H), 7.35 (d, *J* = 7.3 Hz, 1H), 7.23 (d, *J* = 37.1 Hz, 1H), 7.14 (dd, *J* = 12.5, 7.0 Hz, 3H), 6.84 (d, *J* = 7.2 Hz, 2H), 6.72 – 6.58 (m, 4H), 6.49 (dd, *J* = 8.3, 2.5 Hz, 1H), 6.27 (d, *J* = 8.1 Hz, 2H), 5.09 (dd, *J* = 13.4, 5.1 Hz, 1H), 4.65 (s, 2H), 4.40 (d, *J* = 17.1 Hz, 1H), 4.31 – 4.09 (m, 3H), 3.88 (s, 1H), 3.14 – 2.84 (m, 12H), 2.67 – 2.54 (m, 2H), 2.47 – 2.39 (m, 2H), 2.28 – 2.04 (m, 7H), 2.04 – 1.80 (m, 8H), 1.69 (d, *J* = 39.8 Hz, 6H). | 872.76 | General Synthesis Formula 9 |

| | | | | | |
|---|---|---|---|---|---|
| **185** | D4 | 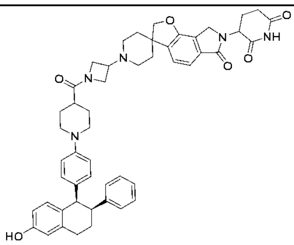 3-(1'-(1-(4-(((1R,2S)-6-Hydroxy-2-phenyl-1,2,3,4-tetrahydronaphthalen-1-yl)phenyl)piperidine-4-carbonyl)azetidin-3-yl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 10.49 (s, 1H), 9.13 (s, 1H), 7.39 – 7.24 (m, 2H), 7.18 – 7.07 (m, 3H), 6.86 – 6.78 (m, 2H), 6.77 – 6.57 (m, 4H), 6.48 (dd, $J$ = 8.2, 2.6 Hz, 1H), 6.27 (s, 2H), 5.10 (dd, $J$ = 13.3, 5.1 Hz, 1H), 4.65 (s, 2H), 4.47 (d, $J$ = 10.2 Hz, 2H), 4.39 (d, $J$ = 17.2 Hz, 1H), 4.23 (d, $J$ = 17.1 Hz, 1H), 4.17 (d, $J$ = 4.9 Hz, 1H), 4.11 (d, $J$ = 6.5 Hz, 2H), 3.56 (s, 4H), 3.48 (s, 1H), 3.33 – 3.28 (m, 1H), 3.06 – 2.87 (m, 5H), 2.79 – 2.55 (m, 3H), 2.45 – 2.35 (m, 2H), 2.15 – 1.95 (m, 6H), 1.77 – 1.58 (m, 5H). | 820.00 | General Synthesis Formula 15 |
| **186** | D5 | 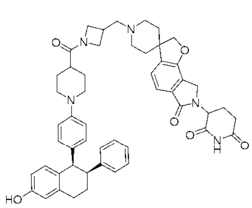 3-(1'-((1-(4-((1R,2S)-6-Hydroxy-2-phenyl-1,2,3,4-tetrahydronaphthalen-1-yl)phenyl)piperidine-4-carbonyl)azetidin-3-yl)methyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 9.57 (s, 1H), 9.12 (s, 1H), 7.40 – 7.23 (m, 2H), 7.19 – 7.08 (m, 3H), 6.88 – 6.80 (m, 2H), 6.76 – 6.54 (m, 4H), 6.48 (dd, $J$ = 8.2, 2.5 Hz, 1H), 6.25 (s, 2H), 5.09 (dd, $J$ = 13.3, 5.1 Hz, 1H), 4.70 – 4.54 (m, 2H), 4.43 – 4.32 (m, 2H), 4.23 (d, $J$ = 17.2 Hz, 1H), 4.15 (d, $J$ = 5.0 Hz, 1H), 4.00 (dt, $J$ = 12.8, 7.4 Hz, 2H), 3.69 (dd, $J$ = 9.9, 5.5 Hz, 3H), 3.30 (dd, $J$ = 14.1, 4.7 Hz, 3H), 3.08 (d, $J$ = 9.8 Hz, 3H), 3.03 – 2.87 (m, 4H), 2.63 – 2.56 (m, 2H), 2.45 – 2.38 (m, 2H), 2.31 (s, 1H), 2.19 – 1.93 (m, 7H), 1.73 – 1.64 (m, 3H), 1.59 (s, 2H). | 834.60 | General Synthesis Formula 15 |
| **187** | D6 | 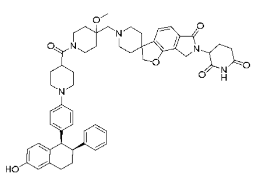 3-(1'-((1-(4-((1R,2S)-6-Hydroxy-2-phenyl-1,2,3,4-tetrahydronaphthalen-1-yl)phenyl)piperidine-4-carbonyl)-4-methoxypiperidin-4-yl)methyl)-6-oxo-6,8-dihydro-2H,7H-spiro[furo[2,3-e]isoindole-3,4'-piperidin]-7-yl)piperidine-2,6-dione | $^1$H NMR (400 MHz, DMSO-$d_6$) δ 10.99 (s, 1H), 9.11 (s, 1H), 7.36 (d, $J$ = 7.6 Hz, 1H), 7.25 (d, $J$ = 7.7 Hz, 1H), 7.21 – 7.10 (m, 5H), 6.83 (d, $J$ = 7.1 Hz, 2H), 6.64 (d, $J$ = 8.3 Hz, 1H), 6.61 – 6.54 (m, 3H), 6.48 (dd, $J$ = 8.3, 2.6 Hz, 1H), 6.22 (d, $J$ = 8.1 Hz, 2H), 5.09 (dd, $J$ = 13.2, 5.0 Hz, -1H), 4.65 (d, $J$ = 11.1 Hz, 2H), 4.28 – 4.11 (m, 3H), 3.80 (s, -1H), 3.57 (s, 5H), 2.99 – 2.83 (m, 7H), 2.62 (d, $J$ = 6.4 Hz, 3H), 2.39 (d, $J$ = 17.9 Hz, 3H), 2.17 – 2.03 (m, 1H), 1.95 (d, $J$ = 32.1 Hz, 8H), 1.63 (s, 9H). | 892.60 | General Synthesis Formula 18 |
| **188** | D7 | | $^1$H NMR (600 MHz, DMSO-$d_6$) δ 10.97 | 863. | General |

| | | | | | |
|---|---|---|---|---|---|
| | | 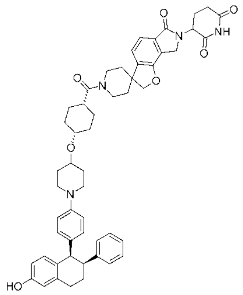 3-(1'-((1S,4s)-4-((1-(4-(((1R,2S)-6-Hydroxy-2-phenyl-1,2,3,4-tetrahydronaphthalen-1-yl)phenyl)piperidin-4-yl)oxy)cyclohexane-1-carbonyl)-6-oxo-6,8-dihydro-2H,7H-spiro[furo[2,3-e]isoindole-3,4'-piperidin]-7-yl)piperidine-2,6-dione | (s, 1H), 9.14 (s, 1H), 7.40 (d, *J* = 7.6 Hz, 1H), 7.27 (d, *J* = 7.5 Hz, 1H), 7.22 – 6.97 (m, 4H), 6.92 – 6.70 (m, 3H), 6.66 – 6.61 (m, 2H), 6.49 (dd, *J* = 8.2, 2.6 Hz, 1H), 6.33 (s, 2H), 5.13 – 5.04 (m, 1H), 4.62 (dt, *J* = 17.8, 9.2 Hz, 2H), 4.39 (d, *J* = 17.6 Hz, 2H), 4.27 – 4.16 (m, 2H), 3.96 (d, *J* = 13.6 Hz, 1H), 3.68 (q, *J* = 3.3 Hz, 1H), 3.53 (s, 2H), 3.03 – 2.87 (m, 5H), 2.71 – 2.57 (m, 4H), 2.46 – 2.37 (m, 2H), 2.12 – 2.06 (m, 1H), 1.97 (ddd, *J* = 10.6, 6.2, 3.8 Hz, 1H), 1.94 – 1.86 (m, 2H), 1.84 – 1.67 (m, 9H), 1.59 (s, 2H), 1.44 (dt, *J* = 43.8, 11.1 Hz, 5H). | 65 | Synthesis Formula 43 |
| **189** | D8 | 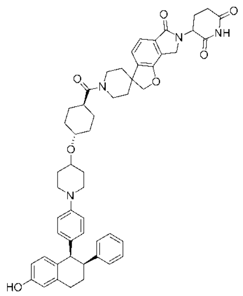 3-(1'-((1R,4r)-4-((1-(4-((1R,2S)-6-Hydroxy-2-phenyl-1,2,3,4-tetrahydronaphthalen-1-yl)phenyl)piperidin-4-yl)oxy)cyclohexane-1-carbonyl)-6-oxo-6,8-dihydro-2H,7H-spiro[furo[2,3-e]isoindole-3,4'-piperidin]-7-yl)piperidine-2,6-dione | $^1$H NMR (400 MHz, DMSO-$d_6$) δ 10.98 (s, 1H), 9.13 (s, 1H), 7.42 (d, *J* = 7.6 Hz, 1H), 7.27 (d, *J* = 7.6 Hz, 1H), 7.18 – 7.08 (m, 3H), 6.86 – 6.81 (m, 2H), 6.80 – 6.51 (m, 4H), 6.48 (dd, *J* = 8.3, 2.5 Hz, 1H), 6.25 (s, 2H), 5.09 (dd, *J* = 13.4, 5.1 Hz, 1H), 4.63 (d, *J* = 6.7 Hz, 2H), 4.39 (d, *J* = 17.3 Hz, 2H), 4.27 – 4.13 (m, 2H), 4.07 – 3.86 (m, 2H), 3.19 (dd, *J* = 9.0, 5.0 Hz, 2H), 2.93 (dt, *J* = 17.1, 9.9 Hz, 3H), 2.76 (s, 2H), 2.65 – 2.56 (m, 2H), 2.42 (dd, *J* = 13.2, 4.4 Hz, 1H), 2.35 – 2.16 (m, 1H), 2.12 (s, 1H), 2.01 – 1.91 (m, 4H), 1.78 (d, *J* = 46.5 Hz, 9H), 1.53 – 1.34 (m, 8H). | 863.65 | General Synthesis Formula 43 |
| **190** | D9 | 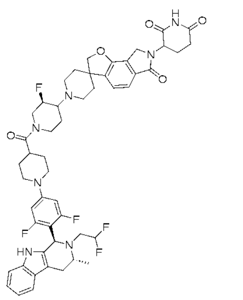 3-(1'-((3R)-1-(1-(4-(((1R,3R)-2-(2,2-Difluoroethyl)-3-methyl-2,3,4,9- | $^1$H NMR (400 MHz, DMSO-$d_6$) δ 10.99 (s, 1H), 10.60 (s, 1H), 7.41 – 7.35 (m, 1H), 7.28 (d, *J* = 7.8 Hz, 1H), 7.20 (d, *J* = 6.6 Hz, 2H), 7.02 – 6.94 (m, 1H), 6.66 (s, 2H), 6.57 (d, *J* = 13.7 Hz, 1H), 5.32 (dd, *J* = 5.4, 4.0 Hz, 3H), 5.12 (s, 1H), 4.67 (s, 1H), 4.40 (d, *J* = 17.3 Hz, 1H), 4.23 (d, *J* = 17.2 Hz, 1H), 3.81 (s, 2H), 3.65 (s, 3H), 3.17 (s, 4H), 3.00 – 2.78 (m, 2H), 2.59 (d, *J* = 18.8 Hz, -1H), 2.20 (s, 2H), 2.00 (h, *J* | 928.65 | General Synthesis Formula 56 |

| | | | | | |
|---|---|---|---|---|---|
| | | tetrahydro-1H-pyrido[3,4-b]indol-1-yl)-3,5-difluorophenyl)piperidine-4-carbonyl)-3-fluoropiperidin-4-yl)-6-oxo-6,8-dihydro-2H,7H-spiro[furo[2,3-e]isoindole-3,4'-piperidin]-7-yl)piperidine-2,6-dione | = 6.5 Hz, 12H), 1.64 (s, 2H), 1.45 (t, *J* = 7.2 Hz, 4H), 1.09 (d, *J* = 6.5 Hz, 3H). | | |
| **191** | D10 | 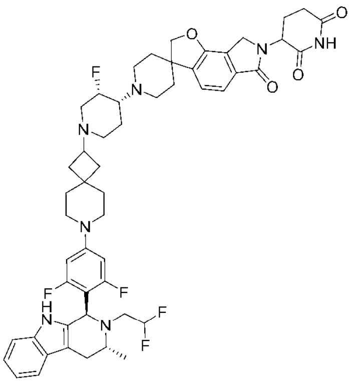 3-(1'-((3S,4R)-1-(7-(4-(((1R,3R)-2-(2,2-Difluoroethyl)-3-methyl-2,3,4,9-tetrahydro-1H-pyrido[3,4-b]indol-1-yl)-3,5-difluorophenyl)-7-azaspiro[3.5]nonan-2-yl)-3-fluoropiperidin-4-yl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 10.99 (s, 1H), 10.57 (s, 1H), 7.39 (d, *J* = 7.8 Hz, 1H), 7.35 (d, *J* = 7.1 Hz, 2H), 7.20 (d, *J* = 8.1 Hz, 1H), 7.00 (t, *J* = 7.5 Hz, 1H), 6.94 (t, *J* = 7.3 Hz, 1H), 6.57 (d, *J* = 13.4 Hz, 2H), 5.87 (dd, *J* = 58.9, 53.9 Hz, 1H), 5.60 (s, 1H), 5.32 (t, *J* = 4.8 Hz, 1H), 5.17 – 5.01 (m, 2H), 4.65 (s, 3H), 4.39 (d, *J* = 17.2 Hz, 2H), 4.23 (d, *J* = 17.1 Hz, 2H), 3.27 – 3.13 (m, 7H), 3.05 (d, *J* = 14.9 Hz, 2H), 2.97 – 2.86 (m, 3H), 2.86 – 2.76 (m, 2H), 2.61 (p, *J* = 1.9 Hz, 2H), 2.55 (d, *J* = 7.3 Hz, 1H), 2.43 (dd, *J* = 13.2, 4.8 Hz, 2H), 2.17 (d, *J* = 7.5 Hz, 6H), 1.99 (dd, *J* = 13.2, 6.6 Hz, 4H), 1.60 (d, *J* = 20.4 Hz, 4H), 1.51 (d, *J* = 7.0 Hz, 1H), 1.09 (d, *J* = 6.6 Hz, 2H). | 940.70 | General Synthesis Formula 54 |
| **192** | D11 | 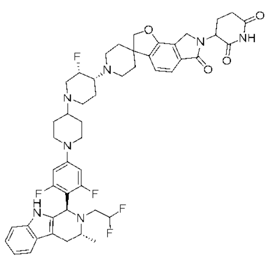 3-(1'-((3S,4R)-1'-(4-((1R,3R)-2-(2,2-Difluoroethyl)-3-methyl-2,3,4,9-tetrahydro-1H-pyrido[3,4-b]indol-1-yl)-3,5-difluorophenyl)-3-fluoro-[1,4'-bipiperidin]-4-yl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 10.99 (s, 1H), 10.58 (s, 1H), 7.40 (d, *J* = 7.7 Hz, 1H), 7.39 – 7.26 (m, 2H), 7.20 (d, *J* = 7.8 Hz, 1H), 7.03 – 6.93 (m, 2H), 6.63 (d, *J* = 14.7 Hz, 2H), 5.88 (t, *J* = 56.5 Hz, 1H), 5.32 (s, 1H), 5.15 – 5.07 (m, 2H), 4.71 – 4.54 (m, 2H), 4.39 (d, *J* = 17.1 Hz, 1H), 4.23 (d, *J* = 17.1 Hz, 1H), 3.96 (d, *J* = 18.4 Hz, 3H), 3.05 (s, 4H), 2.95 – 2.87 (m, 2H), 2.85 – 2.71 (m, 3H), 2.72 – 2.64 (m, 1H), 2.56 (d, *J* = 25.5 Hz, 2H), 2.46 – 2.40 (m, 2H), 2.30 – 2.04 (m, 5H), 1.99 (dq, *J* = 11.9, 6.8, 6.1 Hz, 4H), 1.74 – 1.55 (m, 2H), 1.51 (d, *J* = 7.0 Hz, 2H), 1.49 – 1.40 (m, 1H), 1.32 – 1.27 (m, 2H), 1.10 (d, *J* = 6.6 Hz, 3H). | 900.65 | General Synthesis Formula 55 |

| | | | | | |
|---|---|---|---|---|---|
| **193** | D12 | 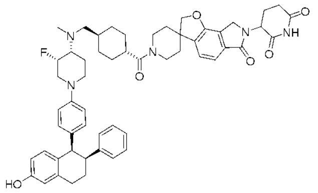 3-(1'-((1R,4r)-4-((((3S,4R)-3-Fluoro-1-(4-((1R,2S)-6-hydroxy-2-phenyl-1,2,3,4-tetrahydronaphthalen-1-yl)phenyl)piperidin-4-yl)(methyl)amino)methyl)cyclohexane-1-carbonyl)-6-oxo-6,8-dihydro-2H,7H-spiro[furo[2,3-e]isoindole-3,4'-piperidin]-7-yl)piperidine-2,6-dione | $^1$H NMR (400 MHz, DMSO-$d_6$) δ 10.99 (s, 1H), 9.14 (s, 1H), 7.41 (t, $J$ = 7.7 Hz, 1H), 7.28 (d, $J$ = 7.3 Hz, 1H), 7.23 – 7.08 (m, 4H), 6.85 (d, $J$ = 7.2 Hz, 2H), 6.60 (tt, $J$ = 9.9, 5.2 Hz, 5H), 6.48 (dd, $J$ = 8.1, 2.5 Hz, 1H), 6.23 (d, $J$ = 8.2 Hz, 2H), 5.37 (dd, $J$ = 49.2, 13.7 Hz, 1H), 5.09 (d, $J$ = 13.2 Hz, 1H), 4.64 (s, 2H), 4.39 (d, $J$ = 16.5 Hz, 2H), 4.27 – 4.12 (m, 2H), 3.94 (s, 2H), 3.75 (s, 1H), 3.17 (s, 3H), 2.94 (d, $J$ = 18.7 Hz, 4H), 2.89 – 2.80 (m, 4H), 2.67 (p, $J$ = 1.9 Hz, 3H), 2.33 (t, $J$ = 1.9 Hz, 1H), 2.09 – 1.95 (m, 6H), 1.75 – 1.44 (m, 12H). | 908. 70 | General Synthesis Formula 24 |
| **194** | D13 | 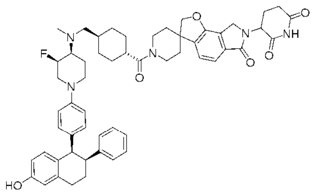 3-(1'-((1S,4r)-4-((((3R,4S)-3-Fluoro-1-(4-((1R,2S)-6-hydroxy-2-phenyl-1,2,3,4-tetrahydronaphthalen-1-yl)phenyl)piperidin-4-yl)(methyl)amino)methyl)cyclohexane-1-carbonyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 10.99 (s, 1H), 9.13 (s, 1H), 7.41 (d, $J$ = 7.6 Hz, 1H), 7.28 (d, $J$ = 7.7 Hz, 1H), 7.22 – 7.08 (m, 4H), 6.95 – 6.78 (m, 2H), 6.77 – 6.54 (m, 5H), 6.48 (dd, $J$ = 8.2, 2.6 Hz, 1H), 6.32 – 6.16 (m, 1H), 5.42 – 5.26 (m, 3H), 5.15 – 5.02 (m, 1H), 4.64 (p, $J$ = 9.4 Hz, 2H), 4.39 (dd, $J$ = 17.0, 5.5 Hz, 2H), 4.28 – 4.12 (m, 2H), 3.94 (s, 2H), 3.73 (s, 1H), 3.16 (s, 3H), 2.99 – 2.80 (m, 6H), 2.77 – 2.63 (m, 2H), 2.42 (d, $J$ = 13.6 Hz, 1H), 1.99 (dt, $J$ = 18.7, 7.0 Hz, 10H), 1.74 (d, $J$ = 25.8 Hz, 7H), 1.47 – 1.42 (m, 3H), 1.28 (d, $J$ = 6.8 Hz, 3H). | 908. 70 | General Synthesis Formula 24 |
| **195** | E1 | 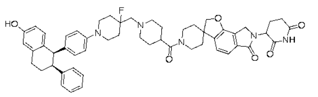 3-(1'-((1-((4-Fluoro-1-(4-((1R,2S)-6-hydroxy-2-phenyl-1,2,3,4-tetrahydronaphthalen-1-yl)phenyl)piperidin-4-yl)methyl)piperidine-4-carbonyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 10.99 (s, 1H), 9.26 (s, 1H), 7.41 (d, $J$ = 7.6 Hz, 1H), 7.29 (d, $J$ = 7.5 Hz, 1H), 7.15 (q, $J$ = 7.6, 7.1 Hz, 3H), 6.86 – 6.84 (m, 2H), 6.65 – 6.61 (m, 4H), 6.49 (dd, $J$ = 8.3, 2.5 Hz, 1H), 6.25 (d, $J$ = 8.2 Hz, 2H), 5.12 – 5.08 (m, 1H), 4.70 – 4.62 (m, 3H), 4.40 (dd, $J$ = 17.0, 7.8 Hz, 3H), 4.27 – 4.21 (m, 2H), 4.16 (d, $J$ = 4.9 Hz, 1H), 4.01 (s, 1H), 3.33 – 3.28 (m, 2H), 3.23 (d, $J$ = 12.8 Hz, 1H), 3.11 (d, $J$ = 11.9 Hz, 2H), 3.01 – 2.97 (m, 2H), 2.96 – 2.86 (m, 5H), 2.75 (s, 1H), 2.14 – 2.03 (m, 3H), 2.02 – 1.95 (m, 5H), 1.91 – 1.83 (m, 4H), 1.75 (dd, $J$ = 20.2, | 880. 65 | General Synthesis Formula 33 |

| | | | | | |
|---|---|---|---|---|---|
| | | | 12.6 Hz, 6H). | | |
| **196** | E2 | 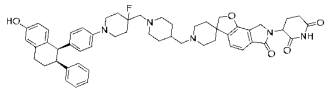 3-(1'-((4-Fluoro-1-(4-((1R,2S)-6-hydroxy-2-phenyl-1,2,3,4-tetrahydronaphthalen-1-yl)phenyl)piperidin-4-yl)methyl)piperidin-4-yl)methyl)-6-oxo-6,8-dihydro-2H,7H-spiro[furo[2,3-e]isoindole-3,4'-piperidin]-7-yl)piperidine-2,6-dione | $^{1}$H NMR (400 MHz, DMSO-$d_6$) δ 11.00 (s, 1H), 9.41 (s, 1H), 7.36 (d, *J* = 7.6 Hz, 1H), 7.29 (d, *J* = 7.6 Hz, 1H), 7.18 – 7.11 (m, 3H), 6.87 – 6.83 (m, 2H), 6.65 – 6.60 (m, 4H), 6.49 (dd, *J* = 8.3, 2.6 Hz, 1H), 6.24 (d, *J* = 8.4 Hz, 2H), 5.10 (dd, *J* = 13.2, 5.1 Hz, 2H), 4.67 (d, *J* = 3.5 Hz, 2H), 4.40 (d, *J* = 17.2 Hz, 2H), 4.24 (d, *J* = 17.3 Hz, 2H), 4.16 (d, *J* = 4.9 Hz, 2H), 3.60 (s, 1H), 3.29 (s, 1H), 3.05 (s, 3H), 2.99 – 2.82 (m, 7H), 2.59 (d, *J* = 32.1 Hz, 2H), 2.21 (t, *J* = 13.7 Hz, 4H), 2.10 (dd, *J* = 11.6, 6.3 Hz, 3H), 2.04 – 1.92 (m, 8H), 1.74 (d, *J* = 9.4 Hz, 3H), 1.58 (d, *J* = 13.2 Hz, 2H). | 866.70 | General Synthesis Formula 12 |
| **197** | F1 | 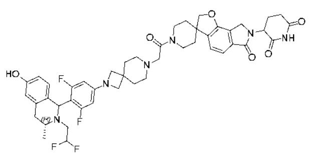 3-(1'-(2-(2-(4-((3R)-2-(2,2-Difluoroethyl)-6-hydroxy-3-methyl-1,2,3,4-tetrahydroisoquinolin-1-yl)-3,5-difluorophenyl)-2,7-diazaspiro[3.5]nonan-7-yl)acetyl)-6-oxo-6,8-dihydro-2H,7H-spiro[furo[2,3-e]isoindole-3,4'-piperidin]-7-yl)piperidine-2,6-dione | $^{1}$H NMR (600 MHz, DMSO- $d_6$) δ 10.99 (s, 1H), 9.16 (s, 1H), 7.38 (d, J = 7.6 Hz, 1H), 7.32 (d, J = 7.6 Hz, 1H), 6.52 – 6.44 (m, 1H), 6.04 (d, J = 12.1 Hz, 2H), 5.72 (t, J = 55.5 Hz, 1H), 5.10 (dt, J = 13.3, 5.4 Hz, 1H), 4.96 (s, 1H), 4.74 – 4.63 (m, 2H), 4.40 (ddd, J = 14.7, 10.1, 4.8 Hz, 4H), 4.32 (dd, J = 16.5, 4.6 Hz, 1H), 4.24 (dd, J = 17.1, 10.1 Hz, 1H), 3.70 (d, J = 6.4 Hz, 2H), 3.64 (d, J = 6.0 Hz, 2H), 3.43 (s, 3H), 3.35 (d, J = 5.8 Hz, 1H), 3.24 (t, J = 13.1 Hz, 1H), 3.03 (dt, J = 15.5, 8.4 Hz, 3H), 2.92 (td, J = 16.2, 13.7, 9.7 Hz, 2H), 2.64 – 2.57 (m, 2H), 2.49 – 2.38 (m, 1H), 2.11 (d, J = 12.6 Hz, 2H), 2.08 – 1.96 (m, 3H), 1.92 (t, J = 12.8 Hz, 1H), 1.86 – 1.77 (m, 3H), 0.97 (d, J = 6.4 Hz, 3H). | 859.56 | General Synthesis Formula 65 |
| **198** | F2 | 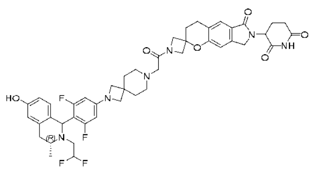 3-(1-(2-(2-(4-((3R)-2-(2,2-Difluoroethyl)-6-hydroxy-3-methyl-1,2,3,4-tetrahydroisoquinolin-1-yl)-3,5-difluorophenyl)-2,7- | $^{1}$H NMR (600 MHz, DMSO-$d_6$) δ 10.97 (s, 1H), 9.16 (s, 1H), 7.53 (s, 1H), 7.06 (s, 1H), 6.51 – 6.47 (m, 2H), 6.47 – 6.44 (m, 1H), 6.02 (d, J = 12.0 Hz, 2H), 5.71 (t, J = 55.6 Hz, 1H), 5.09 – 5.04 (m, 1H), 4.96 (s, 1H), 4.35 (d, J = 16.9 Hz, 1H), 4.30 (d, J = 9.5 Hz, 1H), 4.28 – 4.20 (m, 2H), 4.15 (d, J = 10.6 Hz, 1H), 4.09-3.98 (m, 3H), 3.70 – 3.66 (m, 2H), 3.63-3.58 (m, 2H), | 845.56 | General Synthesis Formula 65 |

| | | | | | |
|---|---|---|---|---|---|
| | | diazaspiro[3.5]nonan-7-yl]acetyl)-6'-oxo-3',4',6',8'-tetrahydro-7'H-spiro[azetidine-3,2'-pyrano[2,3-f]isoindol]-7'-yl)piperidine-2,6-dione | 3.45 – 3.31 (m, 3H), 3.09 – 2.86 (m, 7H), 2.63 – 2.56 (m, 2H), 2.42 – 2.32 (m, 2H), 2.21 – 2.15 (m, 2H), 2.12 – 2.05 (m, 2H), 2.04 – 1.95 (m, 3H), 0.97 (d, J = 6.5 Hz, 3H). | | |
| **199** | F3 | 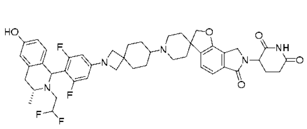 3-(1'-(2-(4-((3R)-2-(2,2-Difluoroethyl)-6-hydroxy-3-methyl-1,2,3,4-tetrahydroisoquinolin-1-yl)-3,5-difluorophenyl)-2-azaspiro[3.5]nonan-7-yl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ 10.99 (s, 1H), 9.15 (s, 1H), 7.36 (d, J = 7.5 Hz, 1H), 7.29 (d, J = 7.6 Hz, 1H),6.50 (d, J = 8.3 Hz, 1H), 6.49 (d, J = 2.4 Hz, 1H), 6.46 (dd, J = 8.3, 2.5 Hz, 1H), 6.01 (d, J = 11.9 Hz, 2H), 5.72 (t, J = 55.6 Hz, 1H), 5.10 (dd, J = 13.3, 5.1 Hz, 1H), 4.96 (s, 1H), 4.71 – 4.65 (m, 2H), 4.40 (d, J = 17.2 Hz, 1H), 4.24 (d, J = 17.1 Hz, 1H), 3.63 – 3.58 (m, 2H), 3.56 – 3.49 (m, 4H), 3.38 – 3.32 (m, 1H), 3.30 – 3.22 (m, 1H), 3.12 (q, J = 11.9 Hz, 2H), 3.02 (dd, J = 15.9, 4.8 Hz, 1H), 2.97-2.87 (m, 2H), 2.64 – 2.57 (m, 1H), 2.49 – 2.38 (m, 2H), 2.23 – 2.13 (m, 2H), 2.11 – 1.95 (m, 7H), 1.62 – 1.55 (m, 2H), 1.55-1.46 (m, 2H), 0.97 (d, J = 6.4 Hz, 3H). | 816. 56 | General Synthesis Formula 67 |
| **200** | F4 | 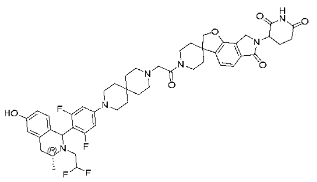 3-(1'-(2-(9-(4-((3R)-2-(2,2-Difluoroethyl)-6-hydroxy-3-methyl-1,2,3,4-tetrahydroisoquinolin-1-yl)-3,5-difluorophenyl)-3,9-diazaspiro[5.5]undecan-3-yl)acetyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$)δ 10.98 (s, 1H), 9.15 (s, 1H), 7.38 (d, J = 7.7 Hz, 1H), 7.32 (d, J = 7.6 Hz, 1H), 6.56 (d, J = 13.6 Hz, 2H), 6.51 (d, J = 8.4 Hz, 1H), 6.49 (d, J = 2.4 Hz, 1H), 6.46 (dd, J = 8.4, 2.7 Hz, 1H), 5.83 – 5.61 (m, 1H), 5.14-5.06 (m, 1H), 4.97 (s, 1H), 4.73 – 4.62 (m, 3H), 4.44-4.36 (m, J 3H), 4.34 (m, 1H), 4.24 (dd, J = 17.1, 10.7 Hz, 1H), 3.67 (d, J = 13.8 Hz, 1H), 3.38 – 3.32 (m, 3H), 3.30 – 3.18 (m,5H), 3.18 – 3.09 (m, 2H), 3.05 – 2.98 (m, 1H), 2.97 – 2.87 (m, 2H), 2.64 – 2.56 (m, 2H), 2.49 – 2.38 (m, 1H), 2.02 – 1.96 (m, 1H), 1.96 – 1.78 (m, 6H), 1.72-1.62 (m, 4H), 1.52 – 1.42 (m, 2H), 0.98 (d, J = 6.5 Hz, 3H). | 887. 56 | General Synthesis Formula 65 |

| | | | | | |
|---|---|---|---|---|---|
| **201** | F5 | 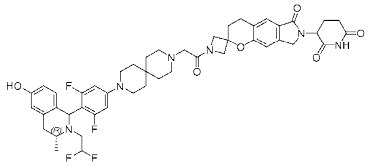 3-(1-(2-(9-(4-((3R)-2-(2,2-Difluoroethyl)-6-hydroxy-3-methyl-1,2,3,4-tetrahydroisoquinolin-1-yl)-3,5-difluorophenyl)-3,9-diazaspiro[5.5]undecan-3-yl)acetyl)-6'-oxo-3',4',6',8'-tetrahydro-7'H-spiro[azetidine-3,2'-pyrano[2,3-f]isoindol]-7'-yl)piperidine-2,6-dione | $^1$H NMR (600 MHz, DMSO-$d_6$)δ 10.97 (d, J = 1.8 Hz, 1H), 9.18 (s, 1H), 7.53 (s, 1H), 7.06 (d, J = 1.6 Hz, 1H), 6.56 (d, J = 13.6 Hz, 2H), 6.52 (d, J = 8.3 Hz, 1H), 6.49 (d, J = 2.3 Hz, 1H), 6.48 – 6.45 (m, 1H), 5.74 (t, J = 56.2 Hz, 1H), 5.07 (dt, J = 13.3, 4.4 Hz, 1H), 4.99 (s, 1H), 4.35 (dd, J = 2.3 Hz, 1H), 4.30 (d, J = 9.5 Hz, 1H), 4.28 – 4.20 (m, 1H), 4.15 (d, J = 10.6 Hz, 1H),4.11 – 3.98 (m, 4H), 3.40 – 3.27 (m, 3H), 3.25 – 3.10 (m, 7H), 3.01 (dd, J = 16.2, 5.1 Hz, 1H), 2.96 – 2.86 (m, 4H), 2.64 – 2.56 (m, 2H), 2.42 – 2.32 (m, 1H), 2.21 – 2.14 (m, 2H), 2.02 – 1.93 (m, 1H), 1.87 – 1.80 (m,2H), 1.71 – 1.59 (m, 4H), 1.48 – 1.42 (m, 2H), 0.99 (d, J = 6.5 Hz, 3H). | 873. 56 | General Synthesis Formula 65 |
| **202** | F6 | 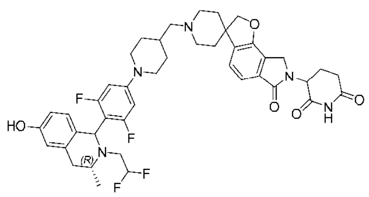 3-(1'-((1-(4-((3R)-2-(2,2-Difluoroethyl)-6-hydroxy-3-methyl-1,2,3,4-tetrahydroisoquinolin-1-yl)-3,5-difluorophenyl)piperidin-4-yl)methyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$)δ 10.99 (s, 1H), 9.09 (s, 1H), 7.37 (d, J = 7.6 Hz, 1H), 7.29 (d, J = 7.6 Hz, 1H), 6.58 (d, J = 13.6 Hz, 2H), 6.52 (d, J = 8.3 Hz, 1H), 6.49 (d, J = 2.4 Hz, 1H), 6.48 – 6.45 (m, 1H), 5.87 – 5.62 (m, 1H), 5.10 (dd, J = 13.3, 5.0 Hz, 1H), 4.98 (s, 1H), 4.71 – 4.64 (m, 2H), 4.40 (d, J = 17.1 Hz, 1H), 4.25 (d, J = 17.1 Hz, 1H), 3.84 – 3.77 (m, 2H), 3.65 – 3.55 (m, 2H), 3.39 – 3.30 (m, 2H), 3.13 – 3.05 (m, 3H), 3.01 (dd, J = 15.9, 4.8 Hz, 1H), 2.96-2.88 (m, 1H), 2.77 (t, J = 12.6 Hz, 2H), 2.64 – 2.58 (m, 1H), 2.46 – 2.38 (m, 2H), 2.28 – 2.18 (m, 2H), 2.03 – 1.93 (m, 4H), 1.85 – 1.79 (m, 2H), 1.33 – 1.22 (m, 4H), 0.98 (d, J = 6.5 Hz, 3H). | 790. 56 | General Synthesis Formula 1 |
| **203** | F7 | 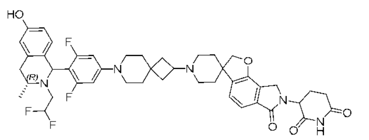 3-(1'-(7-(4-((3R)-2-(2,2-Difluoroethyl)-6-hydroxy-3-methyl-1,2,3,4-tetrahydroisoquinolin-1-yl)-3,5-difluorophenyl]-7- | $^1$H NMR (600 MHz, DMSO-$d_6$)δ 10.99 (s, 1H), 9.16 (s, 1H), 7.36 (d, J = 7.5 Hz, 1H), 7.30 (d, J = 7.7 Hz, 1H), 6.56 (d, J = 13.6 Hz, 2H), 6.52 (d, J = 8.3 Hz, 1H), 6.49 (s, 1H), 6.46 (d, J = 8.9 Hz, 1H), 5.74 (t, J = 55.4 Hz, 1H), 5.10 (dd, J = 13.3, 5.2 Hz, 1H), 4.98 (s, 1H), 4.70 – 4.63 (m, 2H), 4.40 (d, J = 17.1 Hz, 1H), 4.25 (d, J | 816. 56 | General Synthesis Formula 67 |

| | | | | | |
|---|---|---|---|---|---|
| | | azaspiro[3.5]nonan-2-yl)-6-oxo-6,8-dihydro-2H,7H-spiro[furo[2,3-e]isoindole-3,4'-piperidin]-7-yl)piperidine-2,6-dione | = 17.1 Hz, 1H), 3.77 – 3.70 (m, 2H), 3.48-3.42 (m, 2H), 3.35 (s, 2H), 3.28 – 3.20 (m, 2H), 3.15 (s, 2H), 3.01 (dd, J = 16.2, 4.9 Hz, 1H), 2.99 – 2.88 (m, 4H), 2.64 – 2.57 (m, 2H), 2.48 – 2.39 (m, 1H), 2.28-2.18 (m, 3H), 2.16 – 2.08 (m, 2H), 2.07 – 1.96 (m, 4H),1.68 – 1.58(m, 4H), 0.98 (d, J = 6.4 Hz, 3H). | | |
| **204** | F8 | 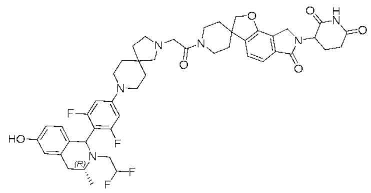 3-(1'-(2-(8-(4-((3R)-2-(2,2-Difluoroethyl)-6-hydroxy-3-methyl-1,2,3,4-tetrahydroisoquinolin-1-yl)-3,5-difluorophenyl)-2,8-diazaspiro[4.5]decan-2-yl)acetyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$)δ 10.98 (s, 1H), 9.16 (s, 1H),7.37 (d, J = 7.6 Hz, 1H), 7.32 (d, J = 7.6 Hz, 1H), 6.57 (dd, J = 13.2, 4.8 Hz, 2H), 6.52 (d, J = 8.3 Hz, 1H), 6.49 (d, J = 2.4 Hz, 1H), 6.46 (d, J = 9.0 Hz, 1H), 5.74 (t, J = 56.1 Hz, 1H), 5.12 – 5.07 (m, 1H), 4.98 (s, 1H), 4.73 – 4.63 (m, 3H), 4.52 (d, J = 15.7 Hz, 1H), 4.49 – 4.45 (m, 1H), 4.45 – 4.33 (m, 3H), 4.24 (dd, J = 17.0, 10.9 Hz, 1H), 3.40-3.33 (m, 1H),3.32 – 3.14 (m, 6H), 3.07 – 2.94 (m, 3H), 2.94 – 2.85 (m, 2H), 2.64 – 2.57 (m, 2H), 2.47 – 2.37 (m, 2H), 2.05-1.97 (m, 2H), 1.96 – 1.86 (m, 2H), 1.84 – 1.76 (m, 3H), 1.77 – 1.63 (m, 4H), 0.98 (d, J = 6.4 Hz, 3H). | 873.66 | General Synthesis Formula 65 |
| **205** | F9 | 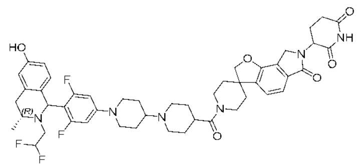 3-(1'-(1'-(4-((3R)-2-(2,2-Difluoroethyl)-6-hydroxy-3-methyl-1,2,3,4-tetrahydroisoquinolin-1-yl)-3,5-difluorophenyl)-[1,4'-bipiperidin]-4-carbonyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$)δ 10.99 (s, 1H), 9.19 (s, 1H), 7.43 (d, J = 7.6 Hz, 1H), 7.29 (d, J = 7.6 Hz, 1H), 6.61 (d, J = 13.1 Hz, 2H), 6.51 (d, J = 8.3 Hz, 1H), 6.49 (d, J = 2.2 Hz, 1H), 6.48 – 6.44 (m, 1H), 5.93 – 5.57 (m, 1H), 5.15 – 5.05 (m, 1H), 4.98 (s, 1H), 4.75-4.58 (m, 3H), 4.40 (dd, J = 17.1, 5.3 Hz, 2H), 4.23 (dd, J = 17.2, 9.2 Hz, 1H), 4.06-3.86 (m, 3H), 3.38 – 3.30 (m,2H), 3.26 – 3.18 (m, 1H), 3.15 – 2.86 (m, 7H), 2.82-2.70 (m, 3H), 2.69 – 2.55 (m, 2H), 2.45 – 2.29 (m, 1H), 2.11 – 1.62 (m, 13H), 0.98 (d, J = 6.4 Hz, 3H). | 887.66 | General Synthesis Formula 5 |
| **206** | F10 | 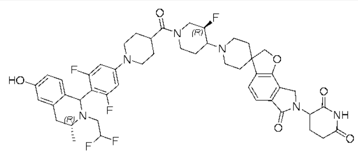 3-(1'-((3R)-1-(1-(4-((3R)-2-(2,2- | $^1$H NMR (600 MHz, DMSO-$d_6$)δ 10.99 (s, 1H), 9.16 (s, 1H), 7.36 (d, J = 7.6 Hz, 1H), 7.29 (d, J = 7.6 Hz, 1H), 6.57 – 6.51 (m, 3H), 6.49 (d, J = 2.4 Hz, 1H), 6.47 (dd, J = 8.0, 2.7 Hz, 1H), 5.74 (t, J = 55.9 | 905.75 | General Synthesis Formula 70 |

| | | | | | |
|---|---|---|---|---|---|
| | | Difluoroethyl)-6-hydroxy-3-methyl-1,2,3,4-tetrahydroisoquinolin-1-yl)-3,5-difluorophenyl)piperidine-4-carbonyl)-3-fluoropiperidin-4-yl)-6-oxo-6,8-dihydro-2H,7H-spiro[furo[2,3-e]isoindole-3,4'-piperidin]-7-yl)piperidine-2,6-dione | Hz, 1H), 5.42 (dd, J = 48.8, 14.5 Hz, 1H), 5.10 (dd, J = 13.2, 5.1 Hz, 1H), 4.98 (s, 1H), 4.73 – 4.63 (m, 2H), 4.59 (d, J = 12.7 Hz, 1H), 4.50 (s, 1H), 4.40 (d, J = 17.2 Hz, 1H), 4.25 (d, J = 17.1 Hz, 1H), 3.78 (s, 3H), 3.45 – 3.31 (d, J = 16.0 Hz, 2H), 3.22-3.12 (m, 3H), 3.04 – 2.97 (m, 2H), 2.96 – 2.88 (m, 2H), 2.86 – 2.76 (m, 3H), 2.68 – 2.57 (m, 3H), 2.46 – 2.38 (m, 1H), 2.29 – 2.13 (m, 4H), 2.08 – 1.96 (m, 3H), 1.72 – 1.65 (m, 2H), 1.65-1.55 (m, 2H), 0.99 (d, J = 6.5 Hz, 3H). | | |
| **207** | F11 | 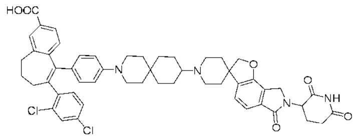 8-(2,4-Dichlorophenyl)-9-(4-(9-(7-(2,6-dioxopiperidin-3-yl)-6-oxo-7,8-dihydro-2H,6H-spiro[furo[2,3-e]isoindole-3,4'-piperidin]-1'-yl)-3-azaspiro[5.5]undecan-3-yl)phenyl)-6,7-dihydro-5H-benzo[7]annulene-3-carboxylic acid | $^1$H NMR (600 MHz, DMSO-$d_6$)δ 12.93 (s, 1H), 11.01 (s, 1H), 7.92 (d, J = 1.8 Hz, 1H), 7.76 (dd, J = 8.0, 1.8 Hz, 1H), 7.62 (d, J = 2.1 Hz, 1H), 7.36 (d, J = 7.6 Hz, 1H), 7.31 – 7.27 (m, 2H), 7.22 (d, J = 8.2 Hz, 1H), 6.88 (d, J = 8.0 Hz, 1H), 6.76 (s, 2H), 6.69 (d, J = 8.1 Hz, 2H), 5.10 (dd, J = 13.3, 5.1 Hz, 1H), 4.70 – 4.64 (m, 2H), 4.40 (d, J = 17.2 Hz, 1H), 4.24 (d, J = 17.1 Hz, 1H), 3.60 – 3.52 (m, 2H), 3.50 – 3.36 (m, 1H), 3.28 – 3.18 (m, 1H), 3.18 – 3.05 (m, 5H), 2.97 – 2.87 (m, 3H), 2.64 – 2.56 (m, 1H), 2.47 – 2.38 (m, 1H), 2.23 – 2.09 (m, 6H), 2.04 – 1.94 (m, 3H), 1.93 – 1.79 (m, 4H), 1.68 – 1.55 (m, 4H), 1.42 (s, 2H), 1.21 – 1.11 (m, 2H). | 913.65 | General Synthesis Formula 82 |
| **208** | G1 | 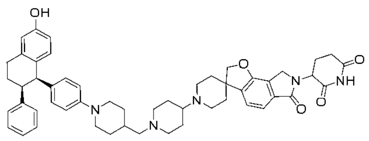 3-(1'-(1-(4-((1R,2S)-6-Hydroxy-2-phenyl-1,2,3,4-tetrahydronaphthalen-1-yl)phenyl)piperidin-4-yl)methyl)piperidin-4-yl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ10.99 (s, 1H), 9.33 (s, 1H), 7.36 (d, J = 7.6 Hz, 1H), 7.29 (d, J = 7.5 Hz, 1H), 7.14 (dt, J = 18.1, 5.7 Hz, 3H), 6.84 (d, J = 7.0 Hz, 2H), 6.69 – 6.56 (m, 4H), 6.49 (dd, J = 8.3, 2.3 Hz, 1H), 6.24 (d, J = 8.3 Hz, 2H), 5.37 – 5.29 (m, 1H), 5.10 (dd, J = 13.3, 5.0 Hz, 1H), 4.69 (dd, J = 15.4, 9.4 Hz, 2H), 4.40 (d, J = 17.2 Hz, 1H), 4.24 (d, J = 17.1 Hz, 1H), 4.16 (d, J = 4.7 Hz, 1H), 3.70 (s, 2H), 3.55 (s, 4H), 3.31 (d, J = 14.6 Hz, 3H), 3.14 (d, J = 35.2 Hz, 3H), 3.05 – 2.88 (m, 6H), 2.63 – 2.54 (m, 3H), 2.47 – 2.37 (m, 2H), 2.25 (d, J = 35.2 Hz, 4H), | 834.65 | General Synthesis Formula 2 |

| | | | | | |
|---|---|---|---|---|---|
| | | | 2.10 (dd, J = 12.8, 6.7 Hz, 1H), 2.04 – 1.96 (m, 4H), 1.75 (dd, J = 37.2, 8.2 Hz, 3H), 1.47 (dd, J = 14.1, 7.2 Hz, 1H). | | |
| **209** | G2 | 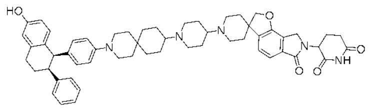 3-(1'-(1-(3-(4-((1R,2S)-6-Hydroxy-2-phenyl-1,2,3,4-tetrahydronaphthalen-1-yl)phenyl)-3-azaspiro[5.5]undecan-9-yl)piperidin-4-yl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ10.99 (s, 1H), 9.15 (s, 1H), 7.29 (dt, J = 37.6, 11.7 Hz, 3H), 7.17 – 7.06 (m, 4H), 6.84 (d, J = 7.2 Hz, 1H), 6.64 (dd, J = 19.4, 11.1 Hz, 4H), 6.49 (d, J = 8.1 Hz, 1H), 6.24 (d, J = 8.1 Hz, 1H), 5.33 (t, J = 4.7 Hz, 1H), 5.10 (dd, J = 13.2, 5.0 Hz, 1H), 4.68 (s, 2H), 4.40 (d, J = 17.3 Hz, 1H), 4.24 (d, J = 17.2 Hz, 1H), 4.15 (d, J = 4.5 Hz, 1H), 3.66 (d, J = 10.0 Hz, 2H), 3.50 (s, 3H), 2.96 (dt, J = 26.0, 20.7 Hz, 8H), 2.64 – 2.54 (m, 2H), 2.42 (dd, J = 20.0, 11.4 Hz, 2H), 2.28 (d, J = 39.3 Hz, 4H), 2.15 – 1.94 (m, 7H), 1.78 (dd, J = 43.6, 24.2 Hz, 5H), 1.59 (s, 3H), 1.52 (d, J = 7.0 Hz, 1H), 1.44 (dd, J = 26.1, 19.3 Hz, 3H), 1.19 – 1.10 (m, 2H) | 888.78 | General Synthesis Formula 7 |
| **210** | G3 | 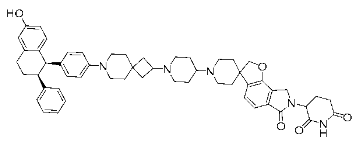 3-(1'-(1-(7-(4-((1R,2S)-6-Hydroxy-2-phenyl-1,2,3,4-tetrahydronaphthalen-1-yl)phenyl)-7-azaspiro[3.5]non-2-yl)piperidin-4-yl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ10.99 (s, 1H), 9.13 (s, 1H), 7.36 (d, J = 7.6 Hz, 1H), 7.29 (d, J = 7.4 Hz, 1H), 7.13 (dq, J = 14.1, 6.9 Hz, 3H), 6.84 (d, J = 7.0 Hz, 2H), 6.69 – 6.53 (m, 4H), 6.48 (dd, J = 8.3, 2.1 Hz, 1H), 6.23 (d, J = 7.5 Hz, 2H), 5.36 – 5.29 (m, 1H), 5.10 (dd, J = 13.2, 5.1 Hz, 1H), 4.69 (dd, J = 14.8, 9.2 Hz, 2H), 4.40 (d, J = 17.2 Hz, 1H), 4.24 (d, J = 17.2 Hz, 1H), 4.15 (d, J = 4.2 Hz, 1H), 3.57 (s, 3H), 3.15 (d, J = 32.1 Hz, 3H), 2.94 (ddd, J = 22.5, 21.1, 11.4 Hz, 7H), 2.77 (d, J = 29.0 Hz, 3H), 2.61 (d, J = 16.7 Hz, 1H), 2.47 – 2.40 (m, 1H), 2.37 – 2.30 (m, 2H), 2.18 (s, 4H), 2.13 – 2.06 (m, 1H), 2.04 – 1.95 (m, 6H), 1.89 (s, 2H), 1.71 (d, J = 5.8 Hz, 1H), 1.61 (d, J = 22.4 Hz, 3H). | 860.65 | General Synthesis Formula 7 |
| **211** | G4 | 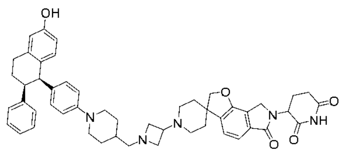 3-(1'-(1-(4-((1R,2S)-6-Hydroxy-2- | 1H NMR (600 MHz, DMSO-$d_6$) δ10.98 (s, 1H), 9.14 (s, 1H), 7.36 (s, 1H), 7.32 (d, J = 7.5 Hz, 1H), 7.19 – 7.09 (m, 3H), 6.84 (d, J = 7.0 Hz, 2H), 6.68 – 6.57 (m, 4H), 6.49 (dd, J = 8.2, 2.1 Hz, 1H), 6.25 (d, J = | 806.65 | General Synthesis Formula 2 |

| | | | | | |
|---|---|---|---|---|---|
| | | phenyl-1,2,3,4-tetrahydronaphthalen-1-yl)phenyl)piperidin-4-yl)methyl)azetidin-3-yl)-6-oxo-6,8-dihydro-2H,7H-spiro[furo[2,3-e]isoindole-3,4'-piperidin]-7-yl)piperidine-2,6-dione | 7.9 Hz, 2H), 5.09 (dd, J = 13.3, 5.0 Hz, 1H), 4.57 (dd, J = 15.4, 9.1 Hz, 2H), 4.38 (d, J = 17.1 Hz, 1H), 4.23 (d, J = 17.1 Hz, 1H), 4.16 (d, J = 4.4 Hz, 1H), 4.11 (s, 1H),3.15 (s, 2H), 3.65-3.50 (m, 4H),3.05-2.87 (m, 5H), 2.60 (d, J = 18.6 Hz, 1H), 2.45 – 2.37 (m, 1H), 2.09 (dd, J = 12.1, 5.8 Hz, 1H), 2.04 – 1.91 (m, 5H), 1.82 (s, 2H), 1.70 (s, 3H), 1.54-1.42 (m, 1H) | | |
| **212** | G6 | 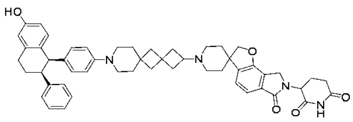 3-(1'-(9-(4-((1R,2S)-6-Hydroxy-2-phenyl-1,2,3,4-tetrahydronaphthalen-1-yl)phenyl)-9-azadispiro[3.1.1$^4$]dodecan-2-yl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ10.99 (s, 1H), 9.13 (s, 1H), 7.36 (d, J = 7.6 Hz, 1H), 7.28 (d, J = 7.6 Hz, 1H), 7.18 – 7.08 (m, 3H), 6.83 (d, J = 7.2 Hz, 2H), 6.63 (dd, J = 19.2, 10.8 Hz, 4H), 6.49 (d, J = 8.2 Hz, 1H), 6.25 (s, 2H), 5.09 (dd, J = 13.1, 5.1 Hz, 1H), 4.63 (dd, J = 14.4, 9.6 Hz, 2H), 4.39 (d, J = 17.2 Hz, 1H), 4.24 (d, J = 17.2 Hz, 1H), 4.15 (s, 1H), 3.62 – 3.55 (m, 1H), 3.17 (s, 1H), 3.00 – 2.85 (m, 8H), 2.60 (d, J = 18.3 Hz, 1H), 2.45 – 2.33 (m, 4H), 2.37 – 2.30 (m, 2H), 2.27 – 2.20 (m, 2H), 2.14 – 2.05 (m, 1H), 2.05 – 1.93 (m, 8H), 1.87 (s, 2H), 1.71 (s, 1H), 1.58 (s, 3H). | 817.66 | General Synthesis Formula 3 |
| **213** | G7 | 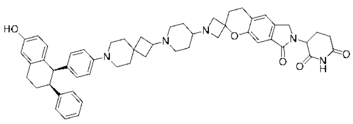 3-(1-(1-(7-(4-((1R,2S)-6-Hydroxy-2-phenyl-1,2,3,4-tetrahydronaphthalen-1-yl)phenyl)-7-azaspiro[3.5]nonan-2-yl)piperidin-4-yl)-8'-oxo-3',4',6',8'-tetrahydro-7'H-spiro[azetidine-3,2'-pyrano[2,3-f]isoindol]-7'-yl)piperidine-2,6-dione | $^1$H NMR (600 MHz, DMSO-$d_6$) δ10.96 (s, 1H), 9.10 (s, 1H), 7.32 (s, 1H), 7.18 – 7.09 (m, 3H), 7.03 (s, 1H), 6.83 (d, J = 7.1 Hz, 2H), 6.65 – 6.60 (m, 2H), 6.53 (d, J = 7.4 Hz, 2H), 6.48 (d, J = 8.1 Hz, 1H), 6.20 (d, J = 8.2 Hz, 2H), 5.07 (dd, J = 13.3, 4.9 Hz, 1H), 4.32 (d, J = 16.7 Hz, 1H), 4.20 (d, J = 16.4 Hz, 1H), 4.13 (d, J = 4.6 Hz, 1H), 3.10 (s, 4H), 3.02 – 2.85 (m, 8H), 2.63 – 2.54 (m, 1H), 2.43-2.32 (m, 1H), 2.15 – 1.92 (m, 10H), 1.77-1.67 (m, 3H), 1.59 (s, 4H). | 846.56 | General Synthesis Formula 7 |
| **214** | G8 | 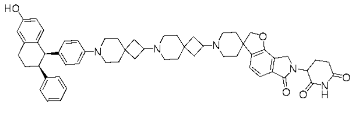 3-(1'-(7-(4-((1R,2S)-6-Hydroxy-2-phenyl-1,2,3,4-tetrahydronaphthalen-1-yl)phenyl)-7,7'-diaza[2,7'- | $^1$H NMR (600 MHz, DMSO-$d_6$) δ10.98 (s, 1H), 9.12 (s, 1H), 7.37 (d, J = 7.6 Hz, 1H), 7.31 (d, J = 7.6 Hz, 1H), 7.18 – 7.09 (m, 3H), 6.84 (d, J = 7.1 Hz, 2H), 6.61 (dd, J = 27.6, 15.4 Hz, 4H), 6.48 (d, J = 8.2 Hz, 1H), 6.23 (d, J = 8.3 Hz, 2H), 5.10 | 900.75 | General Synthesis Formula 7 |

| | | | | | |
|---|---|---|---|---|---|
| | | bipyrido[3.5]nonane]-2'-yl)-6-oxo-6,8-dihydro-2H,7H-spiro[furo[2,3-e]isoindole-3,4'-piperidin]-7-yl)piperidine-2,6-dione | (dd, J = 13.1, 4.9 Hz, 1H), 4.66 (t, J = 13.5 Hz, 2H), 4.40 (d, J = 17.2 Hz, 1H), 4.25 (d, J = 17.1 Hz, 1H), 4.15 (d, J = 4.6 Hz, 1H), 3.71 (d, J = 8.1 Hz, 2H), 2.95 (dd, J = 47.6, 15.1 Hz, 9H), 2.76 (d, J = 11.4 Hz, 2H), 2.63 (dd, J = 31.6, 12.4 Hz, 3H), 2.46-2.30 (m, 3H), 2.20 – 1.86 (m, 13H), 1.83 – 1.65 (m, 5H), 1.60 (d, J = 14.0 Hz, 4H). | | |
| **215** | G9 | 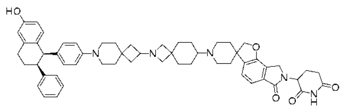 3-(1'-(2-(7-(4-(((1R,2S)-6-Hydroxy-2-phenyl-1,2,3,4-tetrahydronaphthalen-1-yl)phenyl)-7-azaspiro[3.5]nonan-2-yl)-2-azaspiro[1.5]nonan-7-yl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ10.98 (s, 1H), 9.49 (s, 1H), 7.35 (t, J = 7.7 Hz, 1H), 7.28 (d, J = 7.6 Hz, 1H), 7.17 – 7.09 (m, 3H), 6.83 (d, J = 7.1 Hz, 2H), 6.66 –.57 (m, 4H), 6.51 – 6.46 (m, 1H), 6.24 (d, J = 8.2 Hz, 2H), 5.10 (dd, J = 13.2, 4.9 Hz, 1H), 4.67 (q, J = 10.0 Hz, 2H), 4.39 (d, J = 17.2 Hz, 1H), 4.24 (d, J = 17.1 Hz, 1H), 4.15 (d, J = 4.7 Hz, 1H), 4.00 (dd, J = 16.1, 8.1 Hz, 1H), 3.94 (s, 1H), 3.82 (s, 1H), 3.73 – 3.65 (m, 2H), 3.19 (d, J = 18.5 Hz, 2H), 3.12 – 2.84 (m, 10H), 2.61 (d, J = 16.4 Hz, 1H), 2.46 – 2.37 (m, 1H), 2.25 – 1.90 (m, 12H), 1.85-1.75 (m, 2H), 1.75-1.70 (m, 1H), 1.65-1.45 (m, 8H). | 900.65 | General Synthesis Formula 7 |
| **216** | G10 | 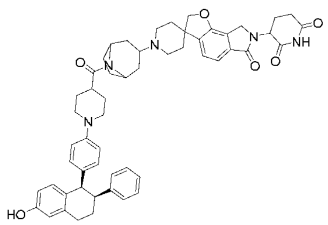 3-(1'-(8-(1-(4-(((1R,2S)-6-Hydroxy-2-phenyl-1,2,3,4-tetrahydronaphthalen-1-yl)phenyl)piperidine-4-carbonyl)-8-azabicyclo[3.2.1]octan-3-yl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ10.99 (s, 1H), 9.13 (s, 1H), 7.35 (d, J = 7.6 Hz, 1H), 7.28 (d, J = 7.6 Hz, 1H), 7.22 – 7.09 (m, 3H), 6.84 (d, J = 7.3 Hz, 2H), 6.68-6.55 (m, 4H), 6.49 (dd, J = 8.2, 2.3 Hz, 1H), 6.24 (d, J = 7.7 Hz, 2H), 5.10 (dd, J = 13.3, 5.2 Hz, 1H), 4.68 – 4.60 (m, 2H), 4.60 – 4.54 (m, 2H), 4.39 (d, J = 17.2 Hz, 1H), 4.24 (d, J = 17.1 Hz, 1H), 4.16 (d, J = 4.2 Hz, 1H), 3.57 (s, 4H), 3.08 – 2.88 (m, 7H), 2.70-2.55 (m, 2H), 2.45-2.40 (m, 1H), 2.17 – 1.94 (m, 10H), 1.80-1.60 (m, 9H). | 874.76 | General Synthesis Formula 15 |

| | | | | | |
|---|---|---|---|---|---|
| **217** | G11 | 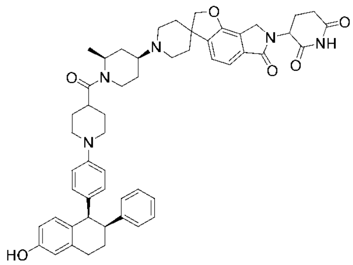 3-(1'-((2S,4S)-1-(1-(4-(((1R,2S)-6-Hydroxy-2-phenyl-1,2,3,4-tetrahydronaphthalen-1-yl)phenyl)piperidine-4-carbonyl)-2-methylpiperidin-4-yl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ10.99 (s, 1H), 9.13 (s, 1H), 7.36 (d, J = 7.5 Hz, 1H), 7.29 (dd, J = 7.7, 3.1 Hz, 1H), 7.22 – 7.09 (m, 3H), 6.84 (d, J = 7.2 Hz, 2H), 6.68 – 6.55 (m, 4H), 6.49 (d, J = 8.3 Hz, 1H), 6.24 (s, 2H), 5.10 (dd, J = 13.3, 5.1 Hz, 1H), 4.67 (s, 2H), 4.40 (d, J = 17.2 Hz, 1H), 4.24 (d, J = 17.1 Hz, 1H), 4.15 (s, 1H), 3.55 (s, 3H), 3.02 – 2.87 (m, 5H), 2.71 (d, J = 50.9 Hz, 3H), 2.61 (d, J = 17.5 Hz, 1H), 2.45 – 2.38 (m, 1H), 2.18 – 1.96 (m, 9H), 1.74 – 1.54 (m, 6H), 1.52 (d, J = 7.0 Hz, 3H),. | 862.66 | General Synthesis Formula 15 |
| **218** | G12 | 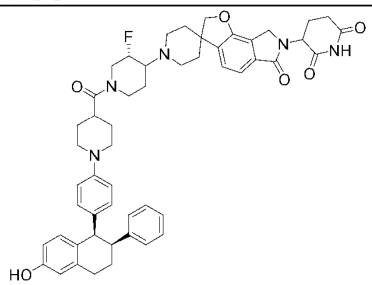 3-(1-(1R,2S)-6-hydroxy-2-phenyl-1,2,3,4-tetrahydronaphthalen-1-yl)phenyl)piperidine-4-carbonyl)piperidin-4-yl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ10.99 (s, 1H), 9.13 (s, 1H), 7.36 (d, J = 7.6 Hz, 1H), 7.28 (d, J = 7.6 Hz, 1H), 7.19 – 7.09 (m, 3H), 6.84 (d, J = 6.5 Hz, 2H), 6.64 (dd, J = 16.2, 5.3 Hz, 4H), 6.49 (dd, J = 8.3, 2.4 Hz, 1H), 6.27 (d, J = 7.4 Hz, 2H), 5.10 (dd, J = 13.2, 4.9 Hz, 1H), 4.68 (t, J = 14.3 Hz, 2H), 4.40 (d, J = 17.3 Hz, 1H), 4.24 (d, J = 17.3 Hz, 1H), 4.17 (d, J = 4.4 Hz, 1H), 3.54 (s, 4H), 3.32 (d, J = 13.2 Hz, 3H), 3.19 (s, 3H), 3.00 – 2.86 (m, 4H), 2.82 – 2.70 (m, 2H), 2.65 – 2.55 (m, 1H), 2.46 – 2.39 (m, 1H), 2.29 – 1.94 (m, 7H), 1.69 (s, 6H). | 866.76 | General Synthesis Formula 15 |
| **219** | G13 | 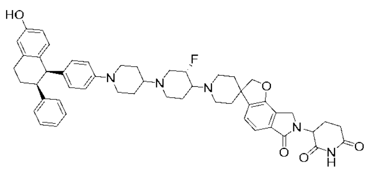 3-(1-(1R,2S)-6-hydroxy-2-phenyl-1,2,3,4-tetrahydronaphthalen-1-yl)phenyl)piperidine-4-carbonyl)piperidin-4-yl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ10.99 (s, 1H), 9.14 (s, 1H), 7.34 (s, 1H), 7.20 – 7.09 (m, 4H), 6.84 (d, J = 7.2 Hz, 2H), 6.69 – 6.55 (m, 4H), 6.49 (d, J = 7.6 Hz, 1H), 6.24 (d, J = 8.1 Hz, 2H), 5.10 (dd, J = 13.1, 5.0 Hz, 1H), 4.64 (s, 2H), 4.40 (d, J = 17.1 Hz, 1H), 4.24 (d, J = 17.2 Hz, 1H), 4.16 (d, J = 4.5 Hz, 1H), 3.70 (s, 2H), 3.17 (s, 5H), 3.04 – 2.86 (m, 5H), 2.63-2.53 (m, 2H), 2.46 – 2.37 (m, 1H), 2.20 – 1.86 (m, 9H), 1.76 – 1.52 (m, 4H). | 838.60 | General Synthesis Formula 4 |

| | | | | | |
|---|---|---|---|---|---|
| **220** | G14 | 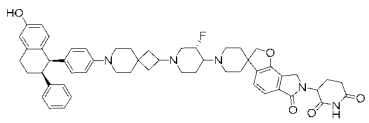 3-(3S)-3-Fluoro-1-(7-(4-(1R,2S)-6-hydroxy-2-phenyl-1,2,3,4-tetrahydronaphthalen-1-yl)phenyl)-7-azaspiro[3.5]nonan-2-yl)piperidin-4-yl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ10.99 (s, 1H), 9.13 (s, 1H), 7.34 (s, 2H), 7.17-7.10 (m, 3H), 6.84 (d, J = 7.4 Hz, 2H), 6.62 (dd, J = 25.8, 12.8 Hz, 4H), 6.49 (d, J = 8.1 Hz, 1H), 6.23 (d, J = 8.1 Hz, 2H), 5.10 (dd, J = 13.2, 4.9 Hz, 1H), 4.64 (s, 2H), 4.40 (d, J = 17.3 Hz, 1H), 4.24 (d, J = 17.2 Hz, 1H), 4.15 (d, J = 4.5 Hz, 1H), 3.80 – 3.60 (m, 3H), 3.02-2.90 (m, 10H), 2.60 (d, J = 17.0 Hz, 1H), 2.55-2.50 (m, 1H), 2.46 – 2.37 (m, 1H), 2.21 – 1.88 (m, 12H), 1.75 - 1.55 (m, 5H). | 878. 66 | General Synthesis Formula 7 |
| **221** | G15 | 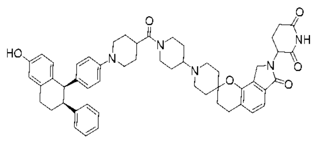 | $^1$H NMR (600 MHz, DMSO-$d_6$) δ11.03 (s, 1H), 9.14 (s, 1H), 7.29 (d, J = 7.7 Hz, 1H), 7.24 (d, J = 7.6 Hz, 1H), 7.14 (dt, J = 21.1, 6.9 Hz, 3H), 6.83 (d, J = 7.2 Hz, 2H), 6.63 (dd, J = 23.2, 5.0 Hz, 4H), 6.49 (dd, J = 8.2, 2.1 Hz, 1H), 6.27 (s, 2H), 5.12 (dd, J = 13.2, 5.1 Hz, 1H), 4.56 (d, J = 12.0 Hz, 1H), 4.42 (d, J = 17.3 Hz, 1H), 4.27 (d, J = 17.4 Hz, 1H), 4.16 (s, 2H), 3.58 – 3.50 (m, 2H), 3.35 – 3.28 (m, 2H), 3.18 (d, J = 12.4 Hz, 3H), 3.09 – 2.86 (m, 7H), 2.77 (s, 2H), 2.68 – 2.60 (m, 1H), 2.42-2.32 (m, J 1H), 2.20 – 1.97 (m, 7H), 1.91 (s, 4H), 1.70 (dd, J = 19.1, 13.1 Hz, 6H). | 862. 65 | General Synthesis Formula 15 |
| **222** | G16 | 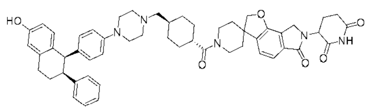 3-(4-(1R,2S)-6-Hydroxy-2-phenyl-1,2,3,4-tetrahydronaphthalen-1-yl)methyl)cyclohexane-1-carbonyl)-6-oxo-6,8-dihydro-2H,7H-spiro[isoindole-3,4'-piperidin]-7-yl)piperidine-2,6-dione | $^1$H NMR (600 MHz, DMSO-$d_6$) δ10.99 (s, 1H), 9.15 (s, 1H), 7.41 (d, J = 7.6 Hz, 1H), 7.28 (d, J = 7.6 Hz, 1H), 7.18 – 7.10 (m, 3H), 6.85 (d, J = 7.1 Hz, 2H), 6.68 – 6.60 (m, 4H), 6.49 (dd, J = 8.3, 2.3 Hz, 1H), 6.27 (d, J = 8.6 Hz, 2H), 5.12 – 5.06 (m, 1H), 4.69 – 4.60 (m, 2H), 4.39 (d, J = 11.4 Hz, 2H), 4.23 (dd, J = 17.0, 12.2 Hz, 1H), 4.18 (d, J = 4.9 Hz, 1H), 3.97 (d, J = 11.9 Hz, 1H),3.68- 3.60 (m, 2H), 3.55 (s, 2H), 3.23 – 3.16 (m, 2H), 3.12-2.86 (m, 9H), 2.75 – 2.57 (m, 3H), 2.45-2.40 (m, 1H), 2.14 – 1.95 (m, 3H), 1.87 – 1.68 (m, 9H), 1.52 – 1.35 (m, 4H). | 862. 65 | General Synthesis Formula 24 |

| | | | | | |
|---|---|---|---|---|---|
| **223** | G17 | 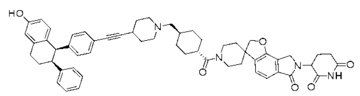 3-(4-(1R,2S)-6-Hydroxy-2-phenyl-1,2,3,4-tetrahydronaphthalen-1-yl)methyl)cyclohexane-1-carbonyl)-6-oxo-6,8-dihydro-2H,7H-spiro[isoindole-3,4'-piperidin]-7-yl)piperidine-2,6-dione | $^1$H NMR (600 MHz, DMSO-$d_6$) δ10.98 (s, 1H), 9.19 (s, 1H), 7.41 (d, J = 7.6 Hz, 1H), 7.29 (d, J = 7.6 Hz, 1H), 7.19 – 7.07 (m, 4H), 7.00 (d, J = 8.2 Hz, 1H), 6.86 (dd, J = 13.7, 7.1 Hz, 2H), 6.64 (d, J = 8.3 Hz, 2H), 6.50 (dd, J = 8.3, 2.4 Hz, 1H), 6.38 (dd, J = 7.9, 6.1 Hz, 2H), 5.12 – 5.06 (m, 1H), 4.69 – 4.60 (m, 2H), 4.40 (d, J = 12.0 Hz, 2H), 4.30-4.20 (m, 2H), 3.97 (d, J = 12.4 Hz, 1H), 3.51 (d, J = 11.9 Hz, 1H), 3.25 – 3.05 (m, 4H),3.05 – 2.87 (m, 6H), 2.82 – 2.75 (m, 1H), 2.75 – 2.67 (m, 1H), 2.67 – 2.57 (m, 2H), 2.41 (dd, J = 21.3, 7.6 Hz, 1H), 2.13 – 1.96 (m, 5H), 1.91 – 1.68 (m, 11H), 1.55 – 1.35 (m, 4H). | 885. 60 | General Synthesis Formula 24 |
| **224** | G18 | 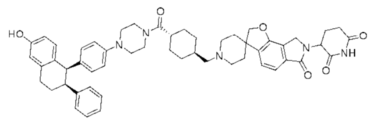 3-(1'-(((1R,4r)-4-(4-(4-(((1R,2S)-6-Hydroxy-2-phenyl-1,2,3,4-tetrahydronaphthalen-1-yl)phenyl)piperazine-1-carbonyl)cyclohexyl)methyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ10.99 (s, 1H), 9.13 (s, 1H), 7.36 (d, J = 7.6 Hz, 1H), 7.28 (d, J = 7.6 Hz, 1H), 7.18 – 7.10 (m, 3H), 6.84 (d, J = 7.1 Hz, 2H), 6.65 – 6.56 (m, 4H), 6.48 (dd, J = 8.3, 2.5 Hz, 1H), 6.24 (d, J = 8.7 Hz, 2H), 5.10 (dd, J = 13.1, 5.1 Hz, 1H), 4.65 (dd, J = 14.6, 9.4 Hz, 2H), 4.40 (d, J = 17.2 Hz, 1H), 4.24 (d, J = 17.2 Hz, 1H), 4.16 (d, J = 4.9 Hz, 1H), 3.65-3.50 (m, 5H), 3.10 – 2.86 (m, 11H), 2.65-2.57 (m, 2H), 2.47 – 2.41 (m, 1H),2.24 – 2.16 (m, 2H), 2.12-2.05 (m, 1H),2.04-1.90 (m, 5H), 1.84 – 1.77 (m, 2H), 1.75 – 1.67 (m, 3H),1.48-1.40 (m, 3H). | 862. 65 | General Synthesis Formula 26 |
| **225** | G19 | 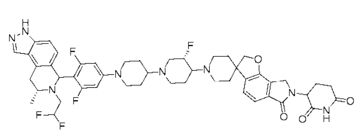 3-(1'-((3S)-1'-(4-((8R)-7-(2,2-Difluoroethyl)-8-methyl-6,7,8,9-tetrahydro-3H-pyrazolo[4,3-f]isoquinolin-6-yl)-3,5-difluorophenyl)-3-fluoro-[1,4'-bipiperidin]-4-yl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ13.00 (s, 1H), 10.99 (s, 1H), 8.08 (s, 1H), 7.34 (s, 2H), 7.24 (d, J = 8.6 Hz, 1H), 6.73 (d, J = 8.6 Hz, 1H), 6.60 (d, J = 13.0 Hz, 2H), 5.95-5.7 (m, 1H), 5.18 (s, 1H), 5.10 (dd, J = 13.2, 5.0 Hz, 1H), 4.63 (s, 2H), 4.40 (d, J = 17.2 Hz, 1H), 4.24 (d, J = 17.0 Hz, 1H), 3.91 (s, 2H), 3.50-3.45 (m, 1H), 3.22 – 3.15 (m, 2H), 3.09 – 2.99 (m, 2H), 2.96 – 2.87 (m, 2H), 2.80 – 2.57 (m, 4H), 2.47 – 2.37 (m, 1H), 2.27 – 1.83 (m, 9H), 1.75 – 1.58 (m, 2H), 1.06 (d, J = 6.5 Hz, 3H). | 901. 40 | General Synthesis Formula 48 |

| | | | | | |
|---|---|---|---|---|---|
| **226** | G20 | 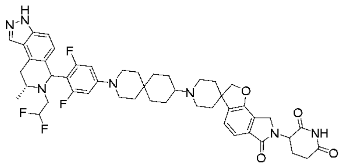<br>3-(1'-(3-(4-((8R)-7-(2,2-Difluoroethyl)-8-methyl-6,7,8,9-tetrahydro-3H-pyrazolo[4,3-f]isoquinolin-6-yl)-3,5-difluorophenyl)-3-azaspiro[5.5]undecan-9-yl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ10.99 (s, 1H), 8.07 (s, 1H), 7.36 (t, J = 5.8 Hz, 1H), 7.31 (d, J = 7.6 Hz, 1H), 7.23 (t, J = 8.4 Hz, 1H), 6.73 (d, J = 8.6 Hz, 1H), 6.54 (t, J = 13.5 Hz, 2H), 5.92-5.70 (m, 1H), 5.18 (s, 1H), 5.09 (dd, J = 13.3, 4.9 Hz, 1H), 4.67 (dt, J = 13.6, 6.8 Hz, 2H), 4.40 (d, J = 17.2 Hz, 1H), 4.24 (d, J = 17.2 Hz, 1H), 3.60-3.53 (m, 2H), 3.25-3.15 (m, 4H), 3.15 – 3.00 (m, 4H), 2.94 – 2.87 (m, 2H), 2.70-2.57 (m, 2H), 2.45 – 2.40 (m, 1H), 2.21 – 2.13 (m, 2H), 2.05 – 1.95 (m, 4H), 1.93-1.80 (m, 4H), 1.68 – 1.55 (m, 4H), 1.50-1.37 (m, 3H), 1.06 (d, J = 6.5 Hz, 3H). | 868.55 | General Synthesis Formula 47 |
| **227** | G21 | 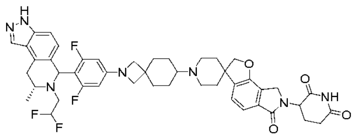<br>3-(1'-(2-(4-((8R)-7-(2,2-Difluoroethyl)-8-methyl-6,7,8,9-tetrahydro-3H-pyrazolo[4,3-f]isoquinolin-6-yl)-3,5-difluorophenyl)-2-azaspiro[3.5]nonan-7-yl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ13.00 (s, 1H), 10.99 (s, 1H), 8.07 (s, 1H), 7.36 (d, J = 7.6 Hz, 1H), 7.29 (d, J = 7.6 Hz, 1H), 7.23 (d, J = 8.5 Hz, 1H), 6.71 (d, J = 8.6 Hz, 1H), 6.01 (d, J = 11.2 Hz, 2H), 5.93-5.68 (m, 1H), 5.15 (s, 1H), 5.09 (dd, J = 13.3, 5.0 Hz, 1H), 4.67 (q, J = 9.6 Hz, 2H), 4.40 (d, J = 17.2 Hz, 1H), 4.24 (d, J = 17.2 Hz, 1H), 3.59 (s, 2H), 3.53 (s, 2H), 3.25 – 3.16 (m, 3H), 3.15-3.00 (m, 4H), 2.98 – 2.87 (m, 2H), 2.63 – 2.58 (m, 1H), 2.45 – 2.37 (m, 1H), 2.20 – 2.13 (m, 2H), 2.09 – 1.96 (m, 7H), 1.61 – 1.45 (m, 4H), 1.05 (d, J = 6.5 Hz, 3H). | 840.60 | General Synthesis Formula 47 |
| **228** | G22 | 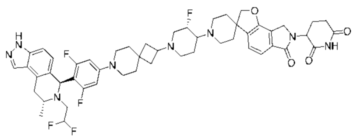<br>3-(1'-((3S)-1-(7-(4-((6S,8R)-7-(2,2-Difluoroethyl)-8-methyl-6,7,8,9-tetrahydro-3H-pyrazolo[4,3-f]isoquinolin-6-yl)-3,5-difluorophenyl)-7-azaspiro[3.5]nonan-2-yl)-3-fluoropiperidin-4-yl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ13.01 (s, 1H), 10.99 (s, 1H), 8.07 (s, 1H), 7.36 (d, J = 7.0 Hz, 2H), 7.23 (d, J = 8.6 Hz, 1H), 6.72 (d, J = 8.6 Hz, 1H), 6.55 (d, J = 13.7 Hz, 2H), 5.92-5.70 (m, 1H), 5.65-5.50 (m, 1H), 5.17 (s, 1H), 5.10 (dd, J = 13.2, 5.0 Hz, 1H), 4.70 – 4.62 (m, 2H), 4.40 (d, J = 17.2 Hz, 1H), 4.24 (d, J = 17.1 Hz, 1H), 3.76 (s, 2H), 3.24 – 2.98 (m, 10H), 2.91 (dd, J = 22.1, 9.4 Hz, 4H), 2.70 – 2.57 (m, 2H), 2.47-2.40 (m, 1H), 2.25 – 1.91 (m, 11H), 1.61 (d, J = 19.3 Hz, 4H), 1.06 (d, J = 6.5 Hz, 3H). | 941.60 | General Synthesis Formula 28 |

| | | | | | |
|---|---|---|---|---|---|
| **229** | G23 | 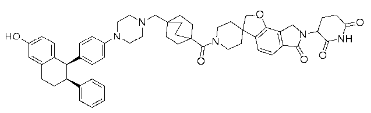<br>3-(1'-(4-(4-(((1R,2S)-6-Hydroxy-2-phenyl-1,2,3,4-tetrahydronaphthalen-1-yl)phenyl)piperazin-1-yl)methyl)bicyclo[2.2.2]octane-1-carbonyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ10.98 (s, 1H), 9.16 (s, 1H), 7.41 (d, J = 7.6 Hz, 1H), 7.28 (d, J = 7.6 Hz, 1H), 7.17 – 7.10 (m, 3H), 6.83 (d, J = 6.9 Hz, 2H), 6.68 – 6.57 (m, 4H), 6.49 (dd, J = 8.3, 2.4 Hz, 1H), 6.27 (d, J = 8.6 Hz, 2H), 5.08 (dd, J = 13.3, 5.0 Hz, 1H), 4.64 (q, J = 9.2 Hz, 2H), 4.39 (d, J = 17.1 Hz, 1H), 4.32 (d, J = 12.5 Hz, 2H), 4.23 (d, J = 17.1 Hz, 1H), 4.17 (d, J = 4.9 Hz, 1H), 3.57-3.50 (m, 2H), 3.22 – 3.14 (m, 2H), 3.06 – 2.87 (m, 9H), 2.63 – 2.58 (m, 1H), 2.45 – 2.37 (m, 1H), 2.13 – 1.95 (m, 3H), 1.90-1.82 (m, 4H), 1.80-1.70 (m, 4H), 1.62-1.53 (m, 4H). | 888.70 | General Synthesis Formula 24 |
| **230** | G24 | 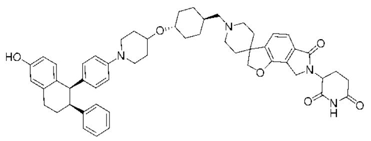<br>3-(1'-(((1R,4r)-4-((1-(4-(((1R,2S)-6-Hydroxy-2-phenyl-1,2,3,4-tetrahydronaphthalen-1-yl)phenyl)piperidin-4-yl)oxy)cyclohexyl)methyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ10.99 (s, 1H), 9.03 (s, 1H), 7.36 (d, J = 7.6 Hz, 1H), 7.27 (d, J = 7.6 Hz, 1H), 7.13 (ddd, J = 9.9, 8.1, 4.6 Hz, 3H), 6.83 (d, J = 7.0 Hz, 2H), 6.63 (dd, J = 19.3, 5.3 Hz, 4H), 6.49 (dd, J = 8.3, 2.4 Hz, 1H), 6.25 (d, J = 6.6 Hz, 2H), 5.10 (dd, J = 13.2, 5.0 Hz, 1H), 4.65 (q, J = 9.4 Hz, 2H), 4.40 (d, J = 17.2 Hz, 1H), 4.24 (d, J = 17.2 Hz, 1H), 4.16 (d, J = 4.2 Hz, 1H), 3.39 – 3.27 (m, 6H), 3.10-2.87 (m, 7H), 2.76 (s, 2H), 2.63–2.58 (m, 1H), 2.47 – 2.38 (m, 1H), 2.22 (t, J = 12.8 Hz, 2H), 2.13 – 2.07 (m, 1H), 2.02-1.91 (m, 5H), 1.87-1.68 (m, 6H), 1.53-1.43 (m, 2H), 1.24-1.15 (m, 2H), 1.10-1.00 (m, 2H),. | 849.76 | General Synthesis Formula 44 |
| **231** | G25 | 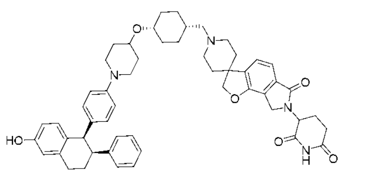<br>3-(1'-(((1S,4s)-4-((1-(4-(((1R,2S)-6-Hydroxy-2-phenyl-1,2,3,4-tetrahydronaphthalen-1-yl)phenyl)piperidin-4-yl)oxy)cyclohexyl)methyl)-6-oxo-6,8-dihydro-2H,7H-spiro[furo[2,3- | $^1$H NMR (600 MHz, DMSO-$d_6$) δ10.99 (s, 1H), 9.12 (s, 1H), 7.40 (d, J = 7.6 Hz, 1H), 7.26 (d, J = 7.6 Hz, 1H), 7.13 (dt, J = 23.1, 7.1 Hz, 3H), 6.84 (d, J = 7.2 Hz, 2H), 6.65 (d, J = 8.3 Hz, 1H), 6.60 (d, J = 2.2 Hz, 1H), 6.54 (d, J = 8.7 Hz, 2H), 6.48 (dd, J = 8.2, 2.4 Hz, 1H), 6.20 (d, J = 8.6 Hz, 2H), 5.09 (dd, J = 13.3, 5.1 Hz, 1H), 4.52 (q, J = 9.1 Hz, 2H), 4.38 (d, J = 17.1 Hz, 1H), 4.21 (d, J = 17.1 Hz, 1H), 4.13 (d, J = 5.0 Hz, 1H), 3.64 (s, 1H), 3.44 (d, | 849.76 | General Synthesis Formula 44 |

| | | | | | |
|---|---|---|---|---|---|
| | | e]isoindole-3,4'-piperidin]-7-yl)piperidine-2,6-dione | J = 8.5 Hz, 1H), 3.30 – 3.25 (m, 2H),3.01 – 2.78 (m, 6H), 2.76–2.69 (m, 2H), 2.62-2.56 (m, 1H), 2.53 - 2.51 (m, 1H), 2.46 – 2.37 (m, 1H), 2.18 – 2.06 (m, 3H), 2.00 - 1.87 (m, 4H), 1.79 (s, 2H), 1.72-1.55 (m, 5H), 1.52-1.37 (m, 6H). | | |
| **232** | G26 | 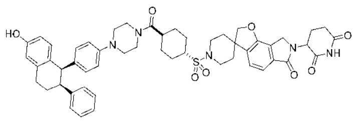 3-(1'-(((1R,4r)-4-(4-(4-(((1R,2S)-6-Hydroxy-2-phenyl-1,2,3,4-tetrahydronaphthalen-1-yl)phenyl)piperazine-1-carbonyl)cyclohexyl)sulfonyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ10.99 (s, 1H), 9.13 (s, 1H), 7.51 (d, J = 7.6 Hz, 1H), 7.29 (t, J = 7.3 Hz, 1H), 7.19 – 7.08 (m, 3H), 6.84 (d, J = 7.2 Hz, 2H), 6.64 (d, J = 8.3 Hz, 1H), 6.62 – 6.56 (m, 3H), 6.48 (d, J = 8.2 Hz, 1H), 6.24 (d, J = 8.5 Hz, 2H), 5.10 (dd, J = 13.2, 4.8 Hz, 1H), 4.60 (d, J = 12.0 Hz, 2H), 4.39 (d, J = 17.1 Hz, 1H), 4.23 (d, J = 17.0 Hz, 1H), 4.15 (d, J = 4.5 Hz, 1H), 3.65 (d, J = 11.9 Hz, 2H), 3.56 (s, 2H), 3.30 (d, J = 12.9 Hz, 2H), 3.19 (d, J = 18.9 Hz, 2H), 3.04 – 2.87 (m, 10H), 2.62-2.57 (m, 1H), 2.45 – 2.37 (m, 1H), 2.15 – 2.04 (m, 1H), 2.04 – 1.82 (m, 8H), 1.80-1.67 (m, 4H), 1.60-1.52 (m, 2H). | 912.75 | General Synthesis Formula 73 |
| **233** | G27 | 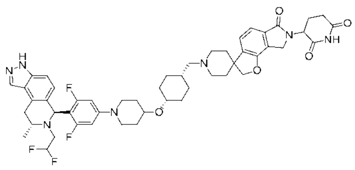 3-(1'-(((1R,4s)-4-((1-(4-((6S,8R)-7-(2,2-Difluoroethyl)-8-methyl-6,7,8,9-tetrahydro-3H-pyrazolo[4,3-f]isoquinolin-6-yl)-3,5-difluorophenyl)piperidin-4-yl)oxy)cyclohexyl)methyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO-$d_6$) δ12.99 (s, 1H), 10.99 (s, 1H), 8.07 (s, 1H), 7.39 (s, 1H), 7.25 (dd, J = 29.3, 7.7 Hz, 2H), 6.73 (d, J = 8.4 Hz, 1H), 6.54 (d, J = 13.4 Hz, 2H), 5.95-5.7 (m, 1H), 5.16 (s, 1H), 5.09 (d, J = 8.5 Hz, 1H), 4.54 (s, 2H), 4.38 (d, J = 16.9 Hz, 1H), 4.22 (d, J = 17.0 Hz, 1H), 3.67 (s, 1H), 3.54 (s, 4H), 3.19 (d, J = 16.1 Hz, 1H), 3.08 – 2.83 (m, 8H), 2.70-2.55 (m, 2H), 2.45 – 2.37 (m, 1H), 2.24 (s, 2H), 2.03 – 1.89 (m, 3H), 1.81 (s, 2H), 1.75-1.65 (m, 3H), 1.47 (d, J = 34.2 Hz, 6H), 1.35-1.27 (m, 2H), 1.05 (d, J = 6.1 Hz, 3H). | 912.65 | General Synthesis Formula 52 |
| **234** | H1 | 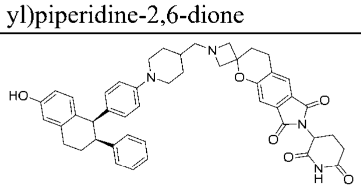 7'-(2,6-Dioxopiperidin-3-yl)-1-((1-(4-(((1R,2S)-6-hydroxy-2-phenyl-1,2,3,4- | $^1$H NMR (600 MHz, DMSO- $d_6$)δ 11.13 (s, 1H), 9.17 (s, 1H), 7.78 (s, 1H), 7.28 (s, 1H), 7.19 – 7.09 (m, 3H), 6.87 – 6.80 (m, 2H), 6.73 (s, 2H), 6.67 – 6.58 (m, 2H), 6.49 (dd, J = 8.3, 2.5 Hz, 1H), 6.29 (d, J = 8.1 Hz, 2H), 5.11 (dd, J = 12.9, 5.5 Hz, | 751.34 | General Synthesis Formula 2 |

| | | | | | |
|---|---|---|---|---|---|
| | | tetrahydronaphthalen-1-yl)phenyl)piperidin-4-yl)methyl)-3'-dihydro-6'-spiro[azetidine-3,2'-pyrano[2,3-f]isoindole]-6',8'(7'H)-dione | 1H), 4.56 – 4.27 (m, 6H), 4.18 (d, J = 5.0 Hz, 1H), 3.61 – 3.44 (m, 2H), 3.23 (s, 1H), 3.04 – 2.84 (m, 5H), 2.79 – 2.52 (m, 3H), 2.25 (d, J = 6.2 Hz, 2H), 2.13 – 1.95 (m, 2H), 1.76 (dd, J = 33.6, 9.7 Hz, 4H), 1.40 - 1.27 (m, 2H) | | |
| **235** | H2 | 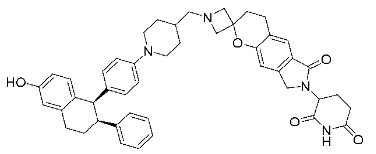 3-(1-((1-(4-((1R,2S)-6-Hydroxy-2-phenyl-1,2,3,4-tetrahydronaphthalen-1-yl)phenyl)piperidin-4-yl)methyl)-6'-oxo-3',4',6',8'-tetrahydro-7'H-spiro[pyridoazetidine-3,2'-pyrano[2,3-f]isoindol]-7'-yl)piperidine-2,6-dione | $^1$H NMR (600 MHz, DMSO-$d_6$)δ 10.99 (s, 1H), 9.16 (s, 1H), 7.52 (s, 1H), 7.14 (ddd, J = 12.7, 7.7, 5.9 Hz, 3H), 7.06 (d, J = 9.2 Hz, 1H), 6.87 – 6.80 (m, 2H), 6.73 (s, 2H), 6.67 – 6.60 (m, 2H), 6.49 (dd, J = 8.2, 2.5 Hz, 1H), 6.29 (d, J = 8.1 Hz, 2H), 5.07 (dd, J = 13.3, 5.1 Hz, 1H), 4.48 – 4.20 (m, 7H), 3.59 – 3.46 (m, 2H), 3.36 – 3.17 (m, 3H), 3.05 – 2.81 (m, 5H), 2.80 – 2.53 (m, 3H), 2.36 (qd, J = 13.1, 4.5 Hz, 1H), 2.22 (q, J = 6.6, 6.1 Hz, 2H), 2.08 (s, 1H), 2.02 – 1.94 (m, 1H), 1.76 (dd, J = 35.1, 9.6 Hz, 4H), 1.34 (s, 2H) | 737. 36 | General Synthesis Formula 2 |
| **236** | H3 | 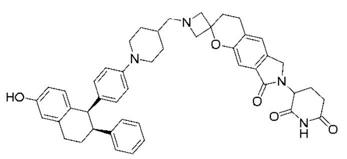 3-(1-((1-(4-((1R,2S)-6-Hydroxy-2-phenyl-1,2,3,4-tetrahydronaphthalen-1-yl)phenyl)piperidin-4-yl)methyl)-8'-oxo-3',4',6',8'-tetrahydro-7'H-spiro[pyridoazetidine-3,2'-pyrano[2,3-f]isoindol]-7'-yl)piperidine-2,6-dione | $^1$H NMR (600 MHz, DMSO- $d_6$)δ 10.99 (s, 1H), 9.17 (s, 1H), 7.38 (s, 1H), 7.19 – 7.09 (m, 4H), 6.87 – 6.80 (m, 2H), 6.71 (s, 2H), 6.66 – 6.59 (m, 2H), 6.49 (dd, J = 8.3, 2.6 Hz, 1H), 6.28 (d, J = 8.1 Hz, 2H), 5.08 (dd, J = 13.3, 5.1 Hz, 1H), 4.52 – 4.43 (m, 2H), 4.40 – 4.22 (m, 4H), 4.22 (d, J = 16.7 Hz,1H), 4.18 (d, J = 5.0 Hz,1H), 3.53 (dq, J = 8.2, 4.3 Hz, 2H), 3.22 (t, J = 6.2 Hz, 1H), 2.94 (ddt, J = 33.2, 19.6, 8.0 Hz, 5H), 2.81 – 2.54 (m, 3H), 2.45 – 2.31 (m, 1H), 2.27 – 2.16 (m, 2H), 2.16 – 1.94 (m, 2H), 1.84 – 1.67 (m, 4H), 1.43 – 1.27 (m, 2H) | 737. 36 | General Synthesis Formula 2 |
| **237** | H4 | 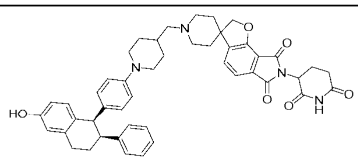 7-(2,6-Dioxopiperidin-3-yl)-1'-((1-(4-(((1R,2S)-6-hydroxy-2-phenyl-1,2,3,4-tetrahydronaphthalen-1-yl)phenyl)piperidin-4-yl)methyl)-7-hydro-2H,6H-spiro[furo[2,3- | $^1$H NMR (600 MHz, DMSO- $d_6$) δ 11.13 (s, 1H), 9.18 (s, 1H), 7.57 (d, J = 7.4 Hz, 1H), 7.50 (d, J = 7.3 Hz, 1H), 7.19 – 7.09 (m, 3H), 6.88 – 6.82 (m, 2H), 6.75 (s, 2H), 6.67 – 6.60 (m, 2H), 6.50 (dd, J = 8.3, 2.5 Hz, 1H), 6.31 (d, J = 8.1 Hz, 2H), 5.11 (dd, J = 12.9, 5.4 Hz, 1H), 4.81 (s, 2H), 4.19 (d, J = 5.0 Hz, 2H), 3.65 – 3.52 (m, 4H), 3.32 (ddd, J = 13.2, 5.1, 2.4 Hz, 2H), | 765. 36 | General Synthesis Formula 2 |

| | | | | | |
|---|---|---|---|---|---|
| | | e]isoindole-3,4'-piperidine]-6,8-dione | 3.08 (q, J = 8.2, 7.2 Hz, 3H), 3.03 – 2.85 (m, 3H), 2.76 (d, J = 25.1 Hz, 2H), 2.63 – 2.56 (m, 1H), 2.56 – 2.47 (m, 1H), 2.23 (dt, J = 14.5, 7.4 Hz, 2H), 2.15 – 1.92 (m, 5H), 1.85 (d, J = 12.9 Hz, 2H), 1.72 (dd, J = 12.6, 6.1 Hz, 1H), 1.38 (d, J = 11.9 Hz, 2H) | | |
| **238** | H5 | 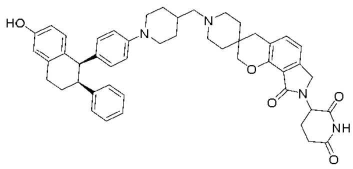 3-(1-((1-(4-((1R,2S)-6-Hydroxy-2-phenyl-1,2,3,4-tetrahydronaphthalen-1-yl)phenyl)piperidin-4-yl)methyl)-9'-oxo-7',8'-dihydro-2'H-spiro[pyridopiperidine-4,3'-pyrano[2,3-e]isoindol]-8'(4'H)-yl)piperidine-2,6-dione | $^1$H NMR (600 MHz, DMSO- $d_6$) δ 10.97 (s, 1H), 9.18 (s, 1H), 7.30 (dd, J = 21.8, 7.7 Hz, 1H), 7.20 – 7.10 (m, 3H), 7.05 (dd, J = 9.3, 7.7 Hz, 1H), 6.87 – 6.81 (m, 2H), 6.78 – 6.59 (m, 4H), 6.49 (dd, J = 8.3, 2.5 Hz, 1H), 6.28 (d, J = 8.1 Hz, 2H), 5.02 (d, J = 4.7 Hz, 1H), 4.32 (d, J = 17.3 Hz, 1H), 4.25 (q, J = 11.4 Hz, 1H), 4.19 (dd, J = 17.1,3.8 Hz, 1H),3.95 (s, 1H), 3.55 (td, J = 7.8, 3.7 Hz, 2H), 3.44 (t, J = 14.9 Hz, 2H), 3.36 – 3.28 (m, 1H), 3.22 – 3.03 (m, 4H), 3.01 – 2.86 (m, 4H), 2.77 – 2.56 (m, 4H), 2.40 – 2.30 (m, 1H), 2.14 – 2.04 (m, 1H), 1.95 (ddd, J = 10.1, 5.0, 2.4 Hz, 2H), 1.87 – 1.69 (m, 6H), 1.62 (s, 1H), 1.33 (s, 2H). | 765. 39 | General Synthesis Formula 2 |
| **239** | H6 | 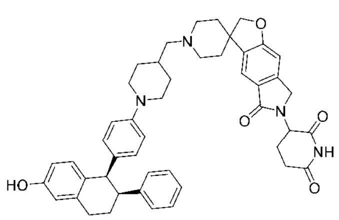 3-(1'-((1-(4-((1R,2S)-6-Hydroxy-2-phenyl-1,2,3,4-tetrahydronaphthalen-1-yl)phenyl)piperidin-4-yl)methyl)-5-oxo-5,7-dihydro-2H,6H-spiro[furo[2,3-f]isoindole-3,4'-piperidin]-6-yl)piperidine-2,6-dione | $^1$H NMR (600 MHz, DMSO- $d_6$) δ 10.98 (s, 1H), 9.17 (s, 1H), 7.41 (s, 1H), 7.20 – 7.10 (m, 3H), 7.04 (d, J = 9.7 Hz, 1H), 6.86 – 6.81 (m, 2H), 6.73 (s, 2H), 6.66 – 6.61 (m, 2H), 6.50 (dd, J = 8.2, 2.6 Hz, 1H), 6.30 (d, J = 8.1 Hz, 2H), 5.04 (dd, J = 13.3, 5.2 Hz, 1H), 4.69 – 4.55 (m, 2H), 4.36 (d, J = 17.1 Hz, 1H), 4.28 – 4.16 (m, 2H), 3.57 (q, J = 11.3, 9.2 Hz, 4H), 3.442 – 3.25 (m, 2H), 3.07 (q, J = 11.8, 8.8 Hz, 3H), 3.01 – 2.87 (m, 3H), 2.76 (s, 1H), 2.60 (dt, J = 16.9, 3.5 Hz, 1H), 2.43 – 2.33 (m, 1H), 2.24 (t, J = 13.5 Hz, 2H), 2.13 – 2.05 (m, 1H), 2.02 – 1.91 (m, 4H), 1.89 – 1.80 (m, 2H), 1.72 (dd, J = 12.3, 6.1 Hz, 1H), 1.37 (d, J = 12.6 Hz, 2H) | 751. 38 | General Synthesis Formula 2 |

| | | | | | |
|---|---|---|---|---|---|
| **240** | H7 | 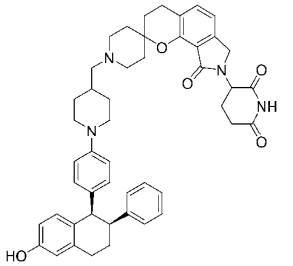 3-(1-((1-(4-((1R,2S)-6-Hydroxy-2-phenyl-1,2,3,4-tetrahydronaphthalen-1-yl)phenyl)piperidin-4-yl)methyl)-9'-oxo-3',4',7',9'-tetrahydro-8'H-spiro[pyridopiperidine-4,2'-pyrano[2,3-e]isoindol]-8'-yl)piperidine-2,6-dione | $^1$H NMR (600 MHz, DMSO- $d_6$) δ 11.06 – 10.92 (m, 1H), 9.17 (s, 1H), 7.35 (dd, J = 7.8, 3.5 Hz, 1H), 7.20 – 7.09 (m, 3H), 7.04 (dd, J = 7.6, 4.7 Hz, 1H), 6.86 – 6.81 (m, 2H), 6.69 (d, J = 24.2 Hz, 2H), 6.67 – 6.59 (m, 2H), 6.49 (dd, J = 8.4, 2.5 Hz, 1H), 6.29 (d, J = 8.1 Hz, 2H), 5.08 – 4.88 (m, 1H), 4.39 – 4.16 (m, 3H), 3.62 – 3.44 (m, 4H), 3.35 – 3.29 (m, 1H), 3.26 – 3.11 (m, 2H), 3.07 – 2.55 (m, 9H), 2.44 – 2.30 (m, 1H), 2.17 – 1.81 (m, 11H), 1.72 (dd, J = 12.6, 6.1 Hz, 1H), 1.44 – 1.25 (m, 2H) | 765.39 | General Synthesis Formula 2 |
| **241** | H8 | 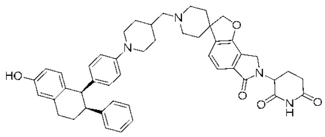 3-(1'-((1-(4-((1R,2S)-6-Hydroxy-2-phenyl-1,2,3,4-tetrahydronaphthalen-1-yl)phenyl)piperidin-4-yl)methyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO- $d_6$) δ 11.01 (s, 1H), 9.17 (s, 1H), 7.36 (d, J = 7.6 Hz, 1H), 7.29 (dd, J = 12.8, 7.7 Hz, 1H), 7.15 (dt, J = 12.9, 6.7 Hz, 3H), 6.87 – 6.81 (m, 2H), 6.75 (s, 2H), 6.69 – 6.60 (m, 2H), 6.50 (dd, J = 8.1, 2.7 Hz, 1H), 6.31 (d, J = 8.5 Hz, 2H), 5.10 (dd, J = 13.3, 5.0 Hz, 1H), 4.71 – 4.62 (m, 2H), 4.40 (dd, J = 17.2, 4.2 Hz, 1H), 4.30 – 4.15 (m, 2H), 3.57 (p, J = 8.1 Hz, 4H), 3.36 – 3.29 (m, 1H), 3.16 – 2.87 (m, 7H), 2.78 (s, 2H), 2.63 – 2.56 (m, 1H), 2.43 (dd, J = 13.3, 4.8 Hz, 1H), 2.24 (t, J = 14.0 Hz, 2H), 2.11 – 1.79 (m, 7H), 1.72 (dd, J = 12.8, 6.1 Hz, 1H), 1.37 (d, J = 15.1 Hz, 2H) | 751.38 | General Synthesis Formula 1 |
| **242** | H9 | 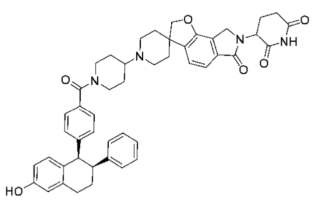 3-(1'-(1-(4-((1R,2S)-6-Hydroxy-2-phenyl-1,2,3,4-tetrahydronaphthalen-1-yl)benzoyl)piperidin-4-yl)-6-oxo-6,8-dihydro-2H,7H-spiro[furo[2,3-e]isoindole-3,4'-piperidin]-7-yl)piperidine-2,6-dione | $^1$H NMR (600 MHz, DMSO- $d_6$) δ 11.00 (s, 1H), 9.23 (s, 1H), 7.36 (d, J = 7.5 Hz, 1H), 7.29 (d, J = 7.6 Hz, 1H), 7.19 – 7.10 (m, 3H), 7.03 (d, J = 7.9 Hz, 2H), 6.83 (dd, J = 7.3, 2.2 Hz, 2H), 6.69 (d, J = 8.3 Hz, 1H), 6.65 (d, J = 2.5 Hz, 1H), 6.52 (dd, J = 8.3, 2.5 Hz, 1H), 6.47 (d, J = 7.8 Hz, 2H), 5.10 (dd, J = 13.3, 5.1 Hz, 1H), 4.72 – 4.63 (m, 2H), 4.58 (d, J = 6.8 Hz, 1H), 4.40 (d, J = 17.1 Hz, 1H), 4.32 (d, J = 5.4 Hz, 1H), 4.24 (d, J = 17.1 Hz, 1H), 3.58 – 3.51 (m, 3H), 3.41 (ddd, J = 13.3, 5.4, 2.4 Hz, 1H), 3.17 – 2.87 (m, 6H), 2.82 – 2.67 (m, 1H), 2.65 – 2.57 (m, 1H), | 765.36 | General Synthesis Formula 15 |

| | | | | | |
|---|---|---|---|---|---|
| | | | 2.43 (dd, J = 13.2, 4.7 Hz, 1H), 2.22 (t, J = 13.5 Hz, 2H), 2.12 (qd, J = 12.3, 5.6 Hz, 2H), 2.05 – 1.93 (m, 4H), 1.79 – 1.71 (m, 1H), 1.63 (dt, J = 13.3, 6.7 Hz, 2H), 1.24 (s, 1H) | | |
| **243** | H10 | 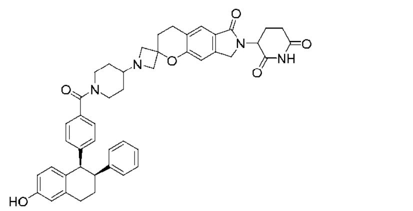 3-(1-(1-(4-(((1R,2S)-6-Hydroxy-2-phenyl-1,2,3,4-tetrahydronaphthalen-1-yl)benzoyl)piperidin-4-yl)-6'-oxo-3',4',6'-tetrahydro-7'H-spiro[pyridoazetidine-3,2'-pyrano[2,3-f]isoindol]-7'-yl)piperidine-2,6-dione | $^1$H NMR (600 MHz, DMSO- $d_6$) δ 10.98 (s, 1H), 9.23 (s, 1H), 7.53 (s, 1H), 7.12 (dd, J = 5.2, 1.9 Hz, 3H), 7.07 (d, J = 12.2 Hz, 1H), 6.98 (d, J = 7.7 Hz, 2H), 6.82 (dd, J = 6.6, 2.9 Hz, 2H), 6.72 – 6.62 (m, 2H), 6.55 – 6.43 (m, 3H), 5.07 (dd, J = 13.3, 5.1 Hz, 1H), 4.60 – 4.13 (m, 8H), 3.40 (ddd, J = 13.2, 5.4, 2.4 Hz, 1H), 3.05 – 2.84 (m, 6H), 2.79 – 2.66 (m, 1H), 2.64 – 2.55 (m, 1H), 2.37 (dd, J = 13.2, 4.7 Hz, 1H), 2.26 – 1.80 (m, 6H), 1.79 – 1.69 (m, 1H), 1.40 – 1.15 (m, 3H) | 751. 34 | General Synthesis Formula 15 |
| **244** | H11 | 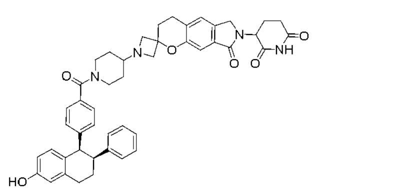 3-(1-(1-(4-(((1R,2S)-6-Hydroxy-2-phenyl-1,2,3,4-tetrahydronaphthalen-1-yl)benzoyl)piperidin-4-yl)-8'-oxo-3',4',6'-tetrahydro-7'H-spiro[pyridoazetidine-3,2'-pyrano[2,3-f]isoindol]-7'-yl)piperidine-2,6-dione | $^1$H NMR (600 MHz, DMSO- $d_6$) δ 10.99 (s, 1H), 9.22 (s, 1H), 7.39 (s, 1H), 7.12 (dt, J = 6.6, 3.1 Hz, 4H), 6.98 (d, J = 7.8 Hz, 2H), 6.82 (dd, J = 6.6, 2.9 Hz, 2H), 6.69 (d, J = 8.3 Hz, 1H), 6.65 (d, J = 2.5 Hz, 1H), 6.52 (dd, J = 8.3, 2.6 Hz, 1H), 6.46 (d, J = 7.9 Hz, 2H), 5.08 (dd, J = 13.3, 5.1 Hz, 1H), 4.59 – 4.16 (m, 8H), 3.40 (ddd, J = 13.2, 5.4, 2.4 Hz, 1H), 3.06 – 2.84 (m, 6H), 2.74 (d, J = 15.5 Hz, 1H), 2.64 – 2.55 (m, 1H), 2.39 (dd, J = 13.1, 4.7 Hz, 1H), 2.20 (dt, J = 12.7, 6.4 Hz, 2H), 2.11 (dt, J = 12.7, 6.2 Hz, 1H), 2.05 – 1.85 (m, 3H), 1.74 (dd, J = 12.9, 5.9 Hz, 1H), 1.38 – 1.12 (m, 3H) | 751. 34 | General Synthesis Formula 15 |
| **245** | H12 | 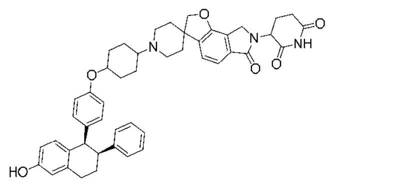 3-(1’-(4-((((1R,2S)-6-Hydroxy-2-phenyl-1,2,3,4-tetrahydronaphthalen-1-yl)phenyl)oxy)cyclohexyl)-6-oxo-6,8-dihydro-2H,7H-spiro[furo[2,3-e]isoindole-3,4'-piperidin]-7- | $^1$H NMR (600 MHz, DMSO- $d_6$) δ 11.00 (s, 1H), 9.16 (s, 1H), 7.35 (d, J = 7.6 Hz, 1H), 7.28 (d, J = 7.6 Hz, 1H), 7.14 (ddd, J = 13.9, 9.4, 5.4 Hz, 3H), 6.83 (t, J = 7.3 Hz, 2H), 6.66 (dd, J = 8.4, 4.7 Hz, 1H), 6.63 – 6.54 (m, 3H), 6.50 (dt, J = 8.3, 2.6 Hz, 1H), 6.28 (dd, J = 11.8, 8.3 Hz, 2H), 5.10 (dd, J = 13.3, 5.1 Hz, 1H), 4.72 – 4.63 (m, 2H), 4.40 (d, J = 17.2 Hz, 1H), 4.24 (d, J = 17.2 Hz, 1H), 4.19 (d, J = 5.1 | 752. 36 | General Synthesis Formula 76 |

| | | | | | |
|---|---|---|---|---|---|
| | | yl)piperidine-2,6-dione | Hz, 1H), 3.58 – 3.45 (m, 4H), 3.17 – 3.05 (m, 2H), 3.01 – 2.87 (m, 3H), 2.65 – 2.57 (m, 1H), 2.45 – 2.37 (m, 1H), 2.25 – 2.04 (m, 5H), 2.04 – 1.93 (m, 4H), 1.88 (d, J = 11.7 Hz, 1H), 1.82 – 1.68 (m, 2H), 1.58 (dq, J = 25.9, 12.8, 11.1 Hz, 3H), 1.40 – 1.31 (m, 1H). | | |
| **246** | H13 | 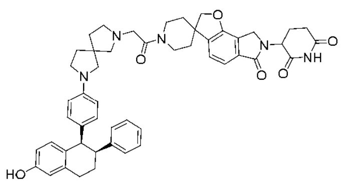 3-(1'-(2-(7-(4-(((1R,2S)-6-Hydroxy-2-phenyl-1,2,3,4-tetrahydronaphthalen-1-yl)phenyl)-2,7-diazaspiro[4.4]nonan-2-yl)acetyl)-6-oxo-6,8-dihydro-2H,7H-spiro[furo[2,3-e]isoindole-3,4'-piperidin]-7-yl)piperidine-2,6-dione | $^1$H NMR (400 MHz, DMSO- $d_6$) δ 11.00 (s, 1H), 9.12 (s, 1H), 7.42 – 7.28 (m, 2H), 7.22 – 7.09 (m, 3H), 6.87 (d, J = 7.2 Hz, 2H), 6.68 – 6.56 (m, 2H), 6.48 (dd, J = 8.3, 2.5 Hz, 1H), 6.26 – 6.09 (m, 4H), 5.10 (dt, J = 13.3, 4.4 Hz, 1H), 4.75 – 4.60 (m, 2H), 4.52 – 4.31 (m, 4H), 4.23 (dd, J = 17.2, 7.0 Hz, 1H), 4.13 (d, J = 18.4 Hz, 1H), 3.75 – 3.60 (m, 2H), 3.35 – 3.05 (m, 8H),3.37 – 3.05 (m, 8H), 3.04 – 2.79 (m, 4H), 2.66 – 2.56 (m, 1H), 2.45 – 2.35 (m, 1H),2.20 – 1.86 (m, 7H), 1.86 – 1.65 (m, 4H). | 820. 40 | General Synthesis Formula 10 |
| **247** | H14 | 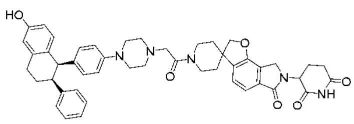 3-(1'-(2-(4-(4-(((1R,2S)-6-Hydroxy-2-phenyl-1,2,3,4-tetrahydronaphthalen-1-yl)phenyl)piperazin-1-yl)acetyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO- $d_6$) δ 11.00 (s, 1H), 9.16 (s, 1H), 7.37 (d, J = 7.5 Hz, 1H), 7.31 (d, J = 7.6 Hz, 1H), 7.20 – 7.10 (m, 3H), 6.89 – 6.83 (m, 2H), 6.66 – 6.60 (m, 4H), 6.49 (dd, J = 8.3, 2.6 Hz, 1H), 6.27 (d, J = 8.5 Hz, 2H), 5.10 (dt, J = 13.3, 5.2 Hz, 1H), 4.68 (ddd, J = 31.1, 14.3, 9.2 Hz, 2H), 4.49 – 4.33 (m, 4H), 4.27 – 4.16 (m, 2H), 3.70 – 3.60 (m, 3H),3.53 (s, 2H), 3.35-3.30 (m, 1H), 3.28 – 3.10 (m, 3H), 3.09 – 2.79 (m, 6H), 2.64 – 2.57 (m, 1H), 2.48 – 2.37 (m, 1H), 2.14 – 2.04 (m, 1H), 2.05 – 1.87 (m, 2H), 1.85 – 1.67 (m, 4H). | 780. 37 | General Synthesis Formula 10 |
| **248** | H15 | 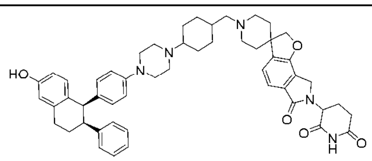 3-(1'-(4-(4-(((1R,2S)-6-Hydroxy-2-phenyl-1,2,3,4-tetrahydronaphthalen-1-yl)phenyl)piperazin-1-yl)cyclohexyl)methyl)-6-oxo-6,8- | $^1$H NMR (600 MHz, DMSO- $d_6$) δ 10.99 (s, 1H), 9.15 (s, 1H), 7.36 (d, J = 7.6 Hz, 1H), 7.28 (t, J = 6.4 Hz, 1H), 7.17-7.10 (m, 3H), 7.02 (s, 1H), 6.85 (d, J = 7.2 Hz, 2H), 6.67 – 6.59 (m, 4H), 6.49 (d, J = 8.2 Hz, 1H), 6.28 (dd, J = 8.6, 3.0 Hz, 2H), 5.10 (dd, J = 13.2, 5.1 Hz, 1H), 4.67 (q, J = 6.7, 4.9 Hz, 2H), 4.40 (d, J = 17.1 Hz, | 834. 45 | General Synthesis Formula 77 |

| | | | | | |
|---|---|---|---|---|---|
| | | dihydro-2H,7H-spiro[furo[2,3-e]isoindole-3,4'-piperidin]-7-yl)piperidine-2,6-dione | 1H), 4.24 (d, J = 17.1 Hz, 1H), 4.18 (d, J = 4.9 Hz, 1H),3.71 (s, 2H), 3.60-3.47 (m, 3H), 3.19 – 3.10 (m, 3H), 3.03 – 2.87 (m, 5H), 2.65 – 2.56 (m, 1H), 2.47 – 2.42 (m, 1H), 2.30-2.20 (m, 2H), 2.18 – 2.06 (m, 3H), 2.02-1.93 (m, 4H), 1.89 (s, 1H), 1.80 (d, J = 12.4 Hz, 2H), 1.72 (d, J = 8.5 Hz, 1H), 1.67 – 1.55 (m, 2H), 1.49 (d, J = 12.9 Hz, 2H). | | |
| **249** | H16 | 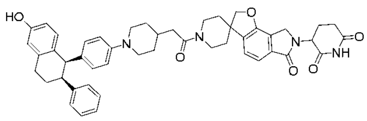 3-(1'-(2-(1-(4-(((1R,2S)-6-Hydroxy-2-phenyl-1,2,3,4-tetrahydronaphthalen-1-yl)phenyl)piperidin-4-yl)acetyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO- $d_6$) δ 10.99 (s, 1H), 9.19 (s, 1H), 7.41 (d, J = 7.6 Hz, 1H), 7.28 (d, J = 7.5 Hz, 1H), 7.19 – 7.11 (m, 3H), 6.87 – 6.79 (m, 2H), 6.64 (td, J = 7.6, 6.8, 3.6 Hz, 4H), 6.50 (dd, J = 8.3, 2.8 Hz, 1H), 6.43 (s, 2H),5.15 – 5.05 (m, 1H), 4.70 – 4.59 (m, 3H), 4.46 – 4.36 (m, 3H), 4.24 (dt, J = 17.1, 9.1 Hz, 3H), 3.92 (s, 1H), 3.50 (d, J = 11.9 Hz, 3H), 3.42-3.30 (m, 1H), 3.22-3.13 (m, 1H), 3.05 – 2.87 (m, 4H), 2.73 (s, 1H), 2.63 – 2.57 (m, 1H), 2.46 – 2.35 (m, 3H), 2.08 (dd, J = 12.2, 6.6 Hz, 1H), 2.03 – 1.95 (m, 2H), 1.95-1.80 (m, 3H), 1.79 – 1.69 (m, 4H), 1.60 – 1.40 (m, 2H), | 779. 37 | General Synthesis Formula 14 |
| **250** | H17 | 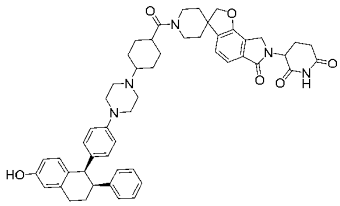 3-(1'-(4-(4-(((1R,2S)-6-Hydroxy-2-phenyl-1,2,3,4-tetrahydronaphthalen-1-yl)phenyl)piperazin-1-yl)cyclohexane-1-carbonyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO- $d_6$) δ 10.99 (s, 1H), 9.14 (s, 1H), 7.42 (d, J = 7.5 Hz, 1H), 7.29 (d, J = 7.5 Hz, 1H), 7.14 (dd, J = 13.0, 7.1 Hz, 3H), 6.84 (d, J = 7.1 Hz, 2H), 6.62 (d, J = 8.2 Hz, 4H), 6.49 (dd, J = 8.3, 2.5 Hz, 1H), 6.27 (d, J = 8.2 Hz, 2H), 5.09 (dt, J = 12.7, 5.9 Hz, 1H), 4.65 (dq, J = 20.2, 9.8 Hz, 2H), 4.40 (dd, J = 17.1, 6.7 Hz, 2H), 4.28 – 4.15 (m, 2H), 4.05 – 3.95 (m, 2H), 3.98 (d, J = 13.3 Hz, 1H), 3.71 (t, J = 10.3 Hz, 3H), 3.34 – 3.28 (m, 2H), 3.25-3.10 (m, 4H), 3.02 – 2.81 (m, 6H), 2.70 (q, J = 12.1 Hz, 2H), 2.63 – 2.57 (m, 1H), 2.45 – 2.38 (m, 1H), 2.17 – 2.04 (m, 3H), 2.02 – 1.95 (m, 1H), 1.91 – 1.66 (m, 7H), 1.55 (q, J = 13.5, 13.0 Hz, 3H), 1.24 (s, 1H). | 848. 43 | General Synthesis Formula 23 |

| | | | | | |
|---|---|---|---|---|---|
| **251** | H18 | 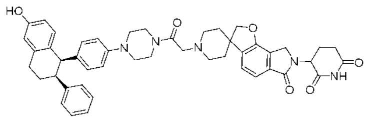 3-(1'-(2-(4-(((1R,2S)-6-Hydroxy-2-phenyl-1,2,3,4-tetrahydronaphthalen-1-yl)phenyl)piperazin-1-yl)-2-oxoethyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO- $d_6$) δ 10.99 (s, 1H), 9.13 (s, 1H), 7.36 (d, J = 7.4 Hz, 1H), 7.31 (d, J = 7.5 Hz, 1H), 7.14 (dq, J = 13.5, 7.3, 6.7 Hz, 3H), 6.85 (d, J = 7.3 Hz, 2H), 6.66 – 6.57 (m, 4H), 6.49 (dd, J = 8.3, 2.6 Hz, 1H), 6.26 (d, J = 7.9 Hz, 2H), 5.10 (dd, J = 13.4, 5.2 Hz, 1H), 4.72 – 4.60 (m, 2H), 4.45 – 4.34 (m, 3H), 4.24 (d, J = 17.2 Hz, 2H), 4.17 (d, J = 5.1 Hz, 2H), 3.63 (t, J = 6.4 Hz, 3H), 3.53 (d, J = 12.4 Hz, 2H), 3.46 (t, J = 5.2 Hz, 2H), 3.39 (s, 1H), 3.34 – 3.27 (m, 1H), 3.15 (q, J = 9.8 Hz, 2H), 3.09 (t, J = 5.2 Hz, 2H), 3.06 – 3.01 (m, 2H), 2.98-2.90 (m, 3H), 2.66 – 2.57 (m, 1H), 2.47 – 2.40 (m, 1H), 2.35-2.26 (m, 2H), 2.15-1.9 (m, J = 13.2, 6.9, 6.3 Hz, 1H), 2.03 – 1.90 (m, 3H), 1.76 – 1.68 (m, 1H). | 780.37 | General Synthesis Formula 11 |
| **252** | H19 | 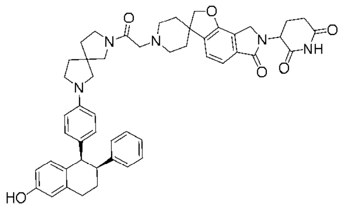 3-(1'-(2-(7-(4-(((1R,2S)-6-Hydroxy-2-phenyl-1,2,3,4-tetrahydronaphthalen-1-yl)phenyl)-2,7-diazaspiro[4.4]nonan-2-yl)-2-oxoethyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO- $d_6$) δ 10.98 (d, J = 6.3 Hz, 1H), 9.10 (s, 1H), 7.37 (d, J = 7.6 Hz, 1H), 7.30 (d, J = 7.6 Hz, 1H), 7.20 – 7.08 (m, 3H), 6.87 (t, J = 6.1 Hz, 2H), 6.66 – 6.58 (m, 2H), 6.48 (d, J = 8.2 Hz, 1H), 6.17 (dq, J = 22.1, 8.0, 6.3 Hz, 4H), 5.10 (dd, J = 13.3, 5.2 Hz, 1H), 4.64 (dd, J = 11.4, 4.5 Hz, 1H), 4.45 – 4.32 (m, 1H), 4.28 – 4.10 (m, 4H), 3.44-4.32(m, 2H), 3.28 (d, J = 13.3 Hz, 2H), 3.22 (t, J = 7.4 Hz, 3H), 3.17-3.05 (m, 4H), 3.05 – 2.86 (m, 4H), 2.63-2.58 (m, 1H), 2.47 – 2.37 (m, 2H), 2.29 (d, J = 9.8 Hz, 1H), 2.12 (s, 1H), 1.95 (ddt, J = 26.5, 18.0, 8.4 Hz, 7H), 1.72 (s, 1H),. | 820.40 | General Synthesis Formula 11 |
| **253** | H20 | 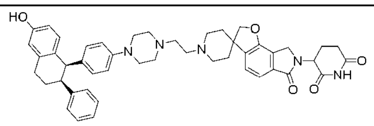 3-(1'-(2-(4-(((1R,2S)-6-Hydroxy-2-phenyl-1,2,3,4-tetrahydronaphthalen-1-yl)phenyl)piperazin-1-yl)ethyl)-6-oxo-6,8-dihydro-2H,7H-spiro[furo[2,3-e]isoindole-3,4'-piperidin]-7- | $^1$H NMR (600 MHz, DMSO- $d_6$) δ 10.99 (s, 1H), 9.13 (s, 1H), 7.35 (d, J = 6.1 Hz, 2H), 7.15 (dt, J = 14.8, 7.0 Hz, 3H), 6.85 (d, J = 7.3 Hz, 2H), 6.67 – 6.58 (m, 4H), 6.49 (dd, J = 8.3, 2.5 Hz, 1H), 6.26 (d, J = 8.2 Hz, 2H), 5.10 (dd, J = 13.3, 5.1 Hz, 1H), 4.63 (s, 2H), 4.39 (d, J = 17.2 Hz, 1H), 4.24 (d, J = 17.1 Hz, 1H), 4.17 (d, J | 766.39 | General Synthesis Formula 78 |

| | | | | | |
|---|---|---|---|---|---|
| | | yl)piperidine-2,6-dione | = 5.0 Hz, 1H), 3.09 (s, 5H), 3.02 – 2.84 (m, 7H), 2.83 – 2.72 (m, 2H), 2.65 – 2.58 (m, 2H), 2.46 – 2.35 (m, 2H), 2.23 – 2.05 (m, 3H), 2.04 – 1.84 (m, 2H), 1.76-1.67 (m, 1H). | | |
| **254** | H21 | 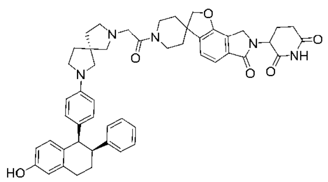 3-(1'-(2-((S)-7-(4-((1R,2S)-6-Hydroxy-2-phenyl-1,2,3,4-tetrahydronaphthalen-1-yl)phenyl)-2,7-diazaspiro[4.4]nonan-2-yl)acetyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO- $d_6$) δ 10.99 (s, 1H), 9.10 (s, 1H), 7.37 (dd, J = 8.3, 2.5 Hz, 1H), 7.34 – 7.29 (m, 1H), 7.19 – 7.10 (m, 3H), 6.91 – 6.84 (m, 2H), 6.62 (dt, J = 13.0, 4.1 Hz, 2H), 6.50 – 6.46 (m, 1H), 6.24 – 6.12 (m, 4H), 5.10 (dt, J = 13.3, 5.6 Hz, 1H), 4.74 – 4.60 (m, 3H), 4.48-4.32 (m, 2H), 4.27-4.21 (m, 2H), 4.13 (d, J = 4.9 Hz, 1H), 3.76 – 3.59 (m, 4H), 3.32 – 3.14 (m, 7H), 3.01 – 2.84 (m, 4H), 2.64 – 2.57 (m, 1H), 2.47 – 2.39 (m, 1H), 2.15 – 1.91 (m, 7H), 1.83 – 1.69 (m, 4H). | 820. 40 | General Synthesis Formula 10 |
| **255** | H22 | 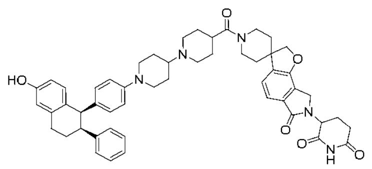 3-(2'-((1'-(4-((1R,2S)-6-Hydroxy-2-phenyl-1,2,3,4-tetrahydronaphthalen-1-yl)phenyl)-[1,4'-bipiperidin]-4-yl)carbonyl)-6-oxo-6,8-dihydro-2H,7H-spiro[furo[2,3-e]isoindole-3,4'-piperidin]-7-yl)piperidine-2,6-dione | $^1$H NMR (600 MHz, DMSO- $d_6$) δ 10.98 (s, 1H), 9.19 (d, J = 7.4 Hz, 1H), 7.42 (d, J = 7.5 Hz, 1H), 7.29 (d, J = 7.5 Hz, 1H), 7.14 (dt, J = 12.9, 6.8 Hz, 3H), 6.88 – 6.81 (m, 2H), 6.67 – 6.56 (m, 4H), 6.49 (dd, J = 8.3, 2.6 Hz, 1H), 6.24 (d, J = 7.9 Hz, 2H), 5.16 – 5.03 (m, 1H), 4.73-4.56 (m, 3H), 4.40 (dd, J = 16.9, 8.4 zHz, 2H), 4.23 (dd, J = 17.0, 14.3 Hz, 2H), 4.15 (d, J = 5.0 Hz, 1H), 3.73 – 3.67 (m, 2H), 3.50 (d, J = 11.6 Hz, 2H), 3.33 – 3.26 (m, 2H), 3.04 (dd, J = 19.4, 11.8 Hz, 3H), 2.94 (ddd, J = 29.1, 16.2, 8.6 Hz, 3H), 2.80 – 2.70 (m, 1H), 2.64 – 2.55 (m, 3H), 2.47 – 2.40 (m, 1H), 2.14 – 2.06 (m, 1H), 2.06 – 1.96 (m, 3H), 1.92 (d, J = 9.8 Hz, 2H), 1.85 (q, J = 19.2, 18.8 Hz, 3H), 1.81 – 1.66 (m, 6H) | 848. 43 | General Synthesis Formula 5 |
| **256** | H23 | 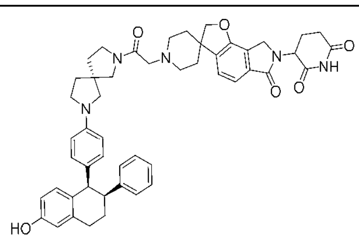 | $^1$H NMR (600 MHz, DMSO- $d_6$) δ 11.00 (s, 1H), 9.11 (s, 1H), 7.36 (d, J = 7.4 Hz, 1H), 7.31 (d, J = 7.6 Hz, 1H), 7.21 – 7.09 (m, 3H), 6.87 (dd, J = 7.5, 4.9 Hz, 2H), 6.66 – 6.58 (m, 2H), 6.48 (dt, J = 8.2, 2.6 Hz, 1H), 6.24 – 6.10 (m, 4H), 5.10 (dd, J | 820. 40 | General Synthesis Formula 11 |

| | | | | | |
|---|---|---|---|---|---|
| | | 3-(1'-(2-((S)-7-(4-(((1R,2S)-6-Hydroxy-2-phenyl-1,2,3,4-tetrahydronaphthalen-1-yl)phenyl)-2,7-diazaspiro[4.4]nonan-2-yl)-2-oxoethyl)-6-oxo-6,8-dihydro-2H,7H-spiro[furo[2,3-e]isoindole-3,4'-piperidin]-7-yl)piperidine-2,6-dione | = 13.3, 5.1 Hz, 1H), 4.65 (dd, J = 10.9, 4.3 Hz, 2H), 4.40 (dd, J = 17.3, 2.5 Hz, 1H), 4.24 (dd, J = 17.3, 3.0 Hz, 1H), 4.19 (d, J = 21.8 Hz, 1H), 4.13 (t, J = 4.9 Hz, 1H), 3.53 (dq, J = 14.4, 6.7, 5.7 Hz, 3H), 3.38 (p, J = 11.6 Hz, 3H), 3.30-3.25(m, 1H), 3.25-3.16 (m, 2H), 3.14 – 3.08 (m, 3H), 3.02 - 2.93 (m, 3H), 2.63 – 2.56 (m, 1H), 2.43 (qd, J = 13.4, 4.6 Hz, 1H), 2.30 (d, J = 11.0 Hz, 2H),j 2.12 (dd, J = 12.7, 6.2 Hz, 1H), 2.05 – 1.79 (m, 7H), 1.75 – 1.67 (m, 1H) | | |
| **257** | H24 | 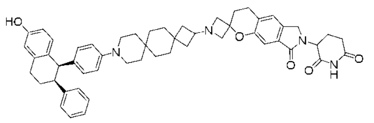 3-(1-(10-(4-((1R,2S)-6-Hydroxy-2-phenyl-1,2,3,4-tetrahydronaphthalen-1-yl)phenyl)-10-azadipyrido [3.2.7.2$^4$]tetradecan-2-yl)-8'-oxo-3',4',6'-tetrahydro-7'H-spiro[azetidine-3,2'-pyrano[2,3-f]isoindol]-7'-yl)piperidine-2,6-dione | $^1$H NMR (600 MHz, DMSO- $d_6$) δ 10.98 (s, 1H), 9.15 (s, 1H), 7.38 (s, 1H), 7.22 – 7.09 (m, 4H), 6.83 (d, J = 7.2 Hz, 3H), 6.80 – 6.56 (m, 3H), 6.49 (dd, J = 8.2, 2.5 Hz, 1H), 6.33 (s, 2H), 5.08 (dd, J = 14.4, 4.9 Hz, 1H), 4.35 (d, J = 16.5 Hz, 1H), 4.28 – 4.16 (m, 4H), 4.13 – 4.06 (m, 1H), 4.05-3.97 (m, 1H), 3.33 (d, J = 13.0 Hz, 1H), 3.11 (s, 3H), 3.02-2.85 (m, 5H), 2.65 – 2.57 (m, 1H), 2.38 (dt, J = 13.4, 6.8 Hz, 1H), 2.23 – 2.04 (m, 5H), 2.02 – 1.95 (m, 1H), 1.75 (d, J = 10.5 Hz, 3H), 1.64 – 1.42 (m, 8H), 1.40-1.25 (m, 4H) | 831.44 | General Synthesis Formula 3 |
| **258** | H25 | 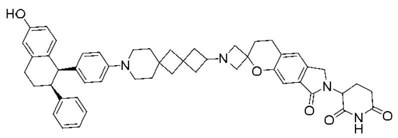 3-(1-(9-(4-((1R,2S)-6-Hydroxy-2-phenyl-1,2,3,4-tetrahydronaphthalen-1-yl)phenyl)-9-azadinitrile[3.1.1$^4$]dodecan-2-yl)-8'-oxo-3',4',6'-tetrahydro-7'H-spiro[azetidine-3,2'-pyrano[2,3-f]isoindol]-7'-yl)piperidine-2,6-dione | $^1$H NMR (600 MHz, DMSO- $d_6$) δ 10.98 (s, 1H), 9.14 (s, 1H), 7.37 (s, 1H), 7.17 – 7.11 (m, 4H), 6.83 (d, J = 7.3 Hz, 3H), 6.72 – 6.60 (m, 3H), 6.49 (dd, J = 8.5, 2.5 Hz, 1H), 6.30 (s, 2H), 5.08 (dd, J = 13.2, 5.1 Hz, 1H), 4.39 – 4.31 (m, 3H), 4.23 (d, J = 9.0 Hz, 2H), 4.12 – 4.06 (m, 2H), 4.01 – 3.93 (m, 1H), 3.32 (d, J = 9.5 Hz, 1H), 3.07 – 2.86 (m, 9H), 2.66 – 2.57 (m, 1H), 2.42 – 2.31 (m, 2H), 2.27 (s, 1H), 2.21 – 2.07 (m, 5H), 1.96 (dd, J = 27.3, 12.0 Hz, 3H), 1.87 (d, J = 10.1 Hz, 2H), 1.72 (d, J = 6.7 Hz, 1H), 1.61 (s, 4H) | 803.41 | General Synthesis Formula 3 |
| **259** | H26 | 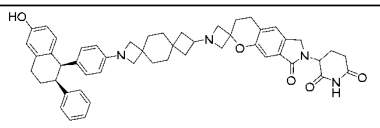 3-(1-(2-(4-(((1R,2S)-6-Hydroxy-2- | $^1$H NMR (600 MHz, DMSO- $d_6$) δ 10.97 (s, 1H), 9.10 (s, 1H), 7.38 (d, J = 2.6 Hz, 1H), 7.19 – 7.08 (m, 4H), 6.88 – 6.78 (m, 2H), 6.68 – 6.58 (m, 2H), 6.48 (dd, J = | 803.41 | General Synthesis Formula 3 |

| | | | | | |
|---|---|---|---|---|---|
| | | phenyl-1,2,3,4-tetrahydronaphthalen-1-yl)phenyl)-2-azadispiro[3.2.2$^{7}$.2$^{4}$]dodecan-9-yl)-8'-oxo-3',4',6'-tetrahydro-7'H-spiro[azetidine-3,2'-pyrano[2,3-f]isoindol]-7'-yl)piperidine-2,6-dione | 8.2, 2.6 Hz, 1H), 6.17 (d, J = 8.1 Hz, 2H), 6.03 (d, J = 8.1 Hz, 2H), 5.08 (dd, J = 13.3, 5.1 Hz, 1H), 4.35 (d, J = 16.9 Hz, 2H), 4.30 –4.15 (m, 4H), 4.13-4.05 (m, 2H), 3.36 (d, J = 5.1 Hz, 4H), 3.30-3.22 (m, 1H), 3.01 – 2.85 (m, 5H), 2.65 – 2.57 (m, 1H), 2.44 – 2.33 (m, 1H), 2.20 (d, J = 6.2 Hz, 2H), 2.11 (s, 3H), 2.01 – 1.96 (m, 1H), 1.77 (q, J = 10.1 Hz, 2H), 1.70 (dd, J = 12.5, 6.2 Hz, 1H), 1.60 (s, 2H), 1.53 (s, 2H), 1.50 – 1.40 (m, 4H). | | |
| **260** | H27 | 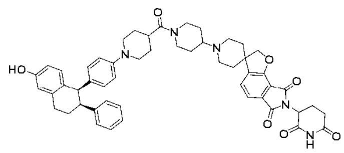 7-(2,6-Dioxopiperidin-3-yl)-1'-(1-(1-(4-(((1R,2S)-6-hydroxy-2-phenyl-1,2,3,4-tetrahydronaphthalen-1-yl)phenyl)piperidine-4-carbonyl)piperidin-4-yl)-7-hydro-2H,6H-spiro[furo[2,3-e]isoindole-3,4'-piperidine]-6,8-dione | $^{1}$H NMR (400 MHz, DMSO- $d_6$)) δ 11.12 (s, 1H), 9.15 (s, 1H), 7.62 – 7.47 (m, 2H), 7.22 – 7.07 (m, 3H), 6.91 – 6.79 (m, 2H), 6.77 – 6.56 (m, 4H), 6.49 (dd, J = 8.3, 2.5 Hz, 1H), 6.29 (s, 2H), 5.10 (s, 1H), 4.82 (s, 2H), 4.57 (d, J = 13.0 Hz, 1H), 4.24 – 4.08 (m, 2H), 3.56 (d, J = 11.9 Hz, 4H), 3.36 – 3.28 (m, 1H), 3.09 (d, J = 13.6 Hz, 3H), 3.00 – 2.91 (m, 2H), 2.89 (d, J = 2.6 Hz, 1H), 2.86 (s, 2H), 2.69 – 2.56 (m, 2H), 2.30 – 1.95 (m, 8H), 1.70 (s, 6H), 1.49 (d, J = 12.1 Hz, 1H). | 862.41 | General Synthesis Formula 15 |
| **261** | H28 | 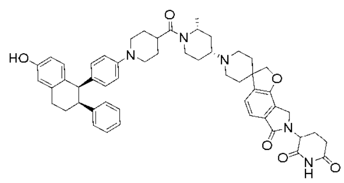 3-(1'-((2R,4R)-1-(1-(4-(((1R,2S)-6-hydroxy-2-phenyl-1,2,3,4-tetrahydronaphthalen-1-yl)phenyl)piperidine-4-carbonyl)-2-methylpiperidin-4-yl)-6-oxo-6,8-dihydro-2H,7H-spiro[furo[2,3-e]isoindole-3,4'-piperidine]-7-yl)piperidine-2,6-dione | $^{1}$H NMR (600 MHz, DMSO- $d_6$) δ 10.99 (s, 1H), 9.14 (s, 1H), 7.36 (d, J = 7.5 Hz, 1H), 7.31 – 7.26 (m, 1H), 7.15 (dt, J = 14.2, 6.9 Hz, 3H), 6.89 – 6.81 (m, 2H), 6.75 – 6.60 (m, 4H), 6.49 (dd, J = 8.3, 2.6 Hz, 1H), 6.29 (s, 2H), 5.10 (dd, J = 13.3, 5.1 Hz, 1H), 4.67 (t, J = 4.0 Hz, 2H), 4.40 (d, J = 17.2 Hz, 1H), 4.24 (d, J = 17.2 Hz, 1H),4.22 – 4.06 (m, 2H), 3.40 – 3.30 (m, 3H), 3.23 – 3.08 (m, 3H), 3.02-2.86 (m, 5H), 2.84 – 2.68 (m, 3H), 2.64 – 2.57 (m, 1H), 2.44 (td, J = 13.2, 4.6 Hz, 1H), 2.28 – 2.05 (m, 5H), 2.06 – 1.95 (m, 4H), 1.85 (s, 1H), 1.79 – 1.55 (m, 6H), 1.19 – 1.07 (m, 3H) | 862.45 | General Synthesis Formula 15 |
| **262** | H29 | 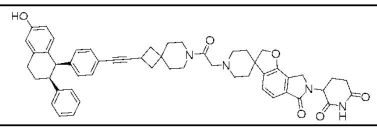 | $^{1}$H NMR (600 MHz, DMSO- $d_6$) δ 10.99 (s, 1H), 9.18 (s, 1H), 7.37 (d, J = 7.6 Hz, 1H), 7.30 (d, J = 7.6 Hz, 1H), 7.15 (dt, J | 843.40 | General Synthesis Formula |

| | | | | | |
|---|---|---|---|---|---|
| | | 3-(1'-(2-((4-(((1R,2S)-6-hydroxy-2-phenyl-1,2,3,4-tetrahydronaphthalen-1-yl)phenyl)ethynyl)-7-azaspiro[3.5]nonan-7-yl)-2-oxoethyl)-6-oxo-6,8-dihydro-2H,7H-spiro[furo[2,3-e]isoindole-3,4'-piperidine]-7-yl)piperidine-2,6-dione | = 14.7, 6.9 Hz, 3H), 7.01 – 6.95 (m, 2H), 6.88 – 6.81 (m, 2H), 6.68 – 6.60 (m, 2H), 6.50 (dd, J = 8.3, 2.6 Hz, 1H), 6.40 – 6.31 (m, 2H), 5.10 (dd, J = 13.3, 5.1 Hz, 1H), 4.65 (d, J = 4.7 Hz, 2H), 4.40 (d, J = 17.2 Hz, 1H), 4.34 – 4.20 (m, 3H), 3.38 (dd, J = 14.0, 5.4 Hz, 4H), 3.30 – 3.19 (m, 4H), 3.14 (d, J = 11.6 Hz, 2H), 3.06 – 2.87 (m, 3H), 2.66 – 2.56 (m, 1H), 2.46 – 2.38 (m, 1H), 2.35 – 2.18 (m, 4H), 2.07 (s, 1H), 2.02 – 1.87 (m, 5H), 1.78 – 1.71 (m, 1H), 1.64 (t, J = 5.7 Hz, 2H), 1.57 (d, J = 6.9 Hz, 2H). | | 39 |
| **263** | H30 | 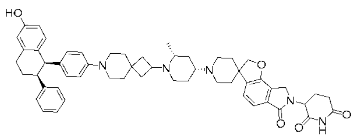 3-(1'-((2R,4R)-1-(7-(4-(((1R,2S)-6-hydroxy-2-phenyl-1,2,3,4-tetrahydronaphthalen-1-yl)phenyl)-7-azaspiro[3.5]nonan-2-yl)-2-methylpiperidin-4-yl)-6-oxo-6,8-dihydro-2H,7H-spiro[furo[2,3-e]isoindole-3,4'-piperidine]-7-yl)piperidine-2,6-dione | $^1$H NMR (600 MHz, DMSO- $d_6$) δ 10.99 (s, 1H), 9.13 (s, 1H), 7.36 (d, J = 7.5 Hz, 1H), 7.28 (d, J = 7.7 Hz, 1H), 7.14 (dt, J = 12.4, 6.6 Hz, 3H), 6.88 – 6.81 (m, 2H), 6.69 – 6.56 (m, 4H), 6.49 (dd, J = 8.2, 2.5 Hz, 1H), 6.24 (d, J = 8.1 Hz, 2H), 5.10 (dd, J = 13.2, 5.1 Hz, 1H), 4.75 – 4.64 (m, 2H), 4.40 (d, J = 17.2 Hz, 1H), 4.24 (d, J = 17.1 Hz, 1H), 4.15 (d, J = 5.0 Hz, 1H), 3.91 (s, 1H), 3.35 (d, J = 12.4 Hz, 2H), 3.34 – 3.27 (m, 2H), 3.09 – 2.88 (m, 9H), 2.62 (t, J = 2.5 Hz, 1H), 2.46 – 2.37 (m, 1H), 2.24 (d, J = 10.8 Hz, 4H), 2.18 – 1.92 (m, 10H), 1.76 – 1.55 (m, 5H), 1.25 (d, J = 7.2 Hz, 3H) | 874.48 | General Synthesis Formula 7 |
| **264** | H31 | 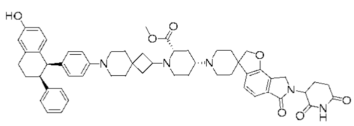 Methyl (2S,4R)-4-(7-(2,6-dioxopiperidin-3-yl)-6-oxo-7,8-dihydro-2H,6H-spiro[furo[2,3-e]isoindole-3,4'-piperidine]-1'-yl)-1-(7-(4-((1R,2S)-6-hydroxy-2-phenyl-1,2,3,4-tetrahydronaphthalen-1-yl)phenyl)-7-azaspiro[3.5]nonan-2-yl)piperidine-2-carboxylate | $^1$H NMR (600 MHz, DMSO- $d_6$) δ 10.99 (s, 1H), 9.13 (s, 1H), 7.36 (d, J = 7.6 Hz, 1H), 7.28 (d, J = 7.7 Hz, 1H), 7.13 (dq, J = 14.1, 7.0 Hz, 3H), 6.95 – 6.69 (m, 3H), 6.69 – 6.57 (m, 3H), 6.49 (dd, J = 8.2, 2.6 Hz, 1H), 6.27 (s, 2H), 5.10 (dd, J = 13.3, 5.1 Hz, 1H), 4.67 (d, J = 4.9 Hz, 2H), 4.40 (d, J = 17.2 Hz, 1H), 4.31 – 4.08 (m, 3H), 3.75 (s, 3H), 3.50 – 3.38 (m, 2H), 3.35 – 3.27 (m, 2H), 3.22 – 3.01 (m, 5H), 3.00 – 2.89 (m, 5H), 2.67 – 2.56 (m, 1H), 2.48 – 2.38 (m, 2H), 2.22 (s, 3H), 2.08 (td, J = 12.7, 6.2 Hz, 2H), 1.99 (d, J = 13.0 Hz, | 918.47 | General Synthesis Formula 9 |

| | | | | | |
|---|---|---|---|---|---|
| | | | 3H), 1.89 (s, 4H), 1.75 – 1.49 (m, 6H). | | |
| **265** | H32 | 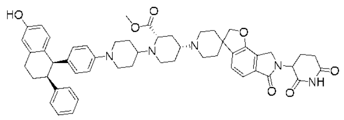 Methyl (2S,4R)-4-(7-(2,6-dioxopiperidin-3-yl)-6-oxo-7,8-dihydro-2H,6H-spiro[furo[2,3-e]isoindole-3,4'-piperidine]-1'-yl)-1-(4-((1R,2S)-6-hydroxy-2-phenyl-1,2,3,4-tetrahydronaphthalen-1-yl)phenyl)-[1,4'-bipiperidinyl]-2-carboxylate | $^1$H NMR (600 MHz, DMSO- $d_6$) δ 10.99 (s, 1H), 9.12 (s, 1H), 7.36 (d, J = 7.5 Hz, 1H), 7.28 (s, 1H), 7.17 – 7.10 (m, 3H), 6.84 (t, J = 8.2 Hz, 2H), 6.67 – 6.52 (m, 4H), 6.48 (dd, J = 8.2, 2.5 Hz, 1H), 6.23 (d, J = 8.5 Hz, 2H), 5.10 (dd, J = 13.4, 5.1 Hz, 1H), 4.66 (s, 2H), 4.40 (d, J = 17.2 Hz, 1H), 4.24 (d, J = 17.1 Hz, 1H), 4.15 (s, 1H), 3.72 (s, 1H), 3.63 (s, 2H), 3.55 – 3.45 (m, 2H), 3.14 – 3.06 (m, 4H), 3.03 – 2.87 (m, 5H), 2.65 – 2.57 (m, 2H), 2.45 – 2.40 (m, 2H), 2.32 – 2.15 (m, 6H), 2.14 – 2.06 (m, 2H), 1.99 (s, 4H), 1.72 (s, 3H). | 878. 44 | General Synthesis Formula 9 |
| **266** | H33 | 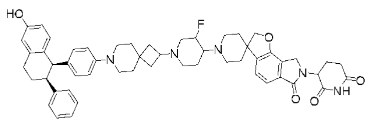 3-(1'-(3-fluoro-1-(7-(4-((1R,2S)-6-hydroxy-2-phenyl-1,2,3,4-tetrahydronaphthalen-1-yl)phenyl)-7-azaspiro[3.5]nonan-2-yl)piperidin-4-yl)-6-oxo-6,8-dihydro-2H,7H-spiro[furo[2,3-e]isoindole-3,4'-piperidine]-7-yl)piperidine-2,6-dione | $^1$H NMR (600 MHz, DMSO- $d_6$) δ 10.99 (s, 1H), 9.13 (s, 1H), 7.34 (t, J = 11.2 Hz, 2H), 7.19 – 7.09 (m, 3H), 6.87 – 6.81 (m, 2H), 6.69 – 6.54 (m, 4H), 6.49 (dd, J = 8.4, 2.5 Hz, 1H), 6.24 (d, J = 8.1 Hz, 2H), 5.10 (dd, J = 13.3, 5.1 Hz, 1H), 4.64 (d, J = 17.3 Hz, 2H), 4.40 (d, J = 17.2 Hz, 1H), 4.24 (d, J = 17.1 Hz, 1H), 4.15 (d, J = 5.0 Hz, 1H), 3.98 (s, 5H), 3.34 – 3.26 (m, 3H), 3.18 (s, 2H), 3.08 – 2.84 (m, 9H), 2.64 – 2.56 (m, 1H), 2.46 – 2.36 (m, 1H), 2.33 – 2.04 (m, 7H), 2.04 – 1.88 (m, 4H), 1.68 (dd, J = 61.1, 11.7 Hz, 5H). | 878. 46 | General Synthesis Formula 7 |
| **267** | H34 | 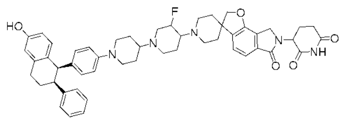 3-(4-(3-fluoro-1'-(4-((1R,2S)-6-hydroxy-2-phenyl-1,2,3,4-tetrahydronaphthalen-1-yl)phenyl)-[1,4'-bipiperidinyl]-4-yl)-6'-oxo-6',8'-dihydro-2'H,7'H-spiro[cyclohexane-1,3'-furo[2,3-e]isoindole]-7'-yl)piperidine-2,6-dione | $^1$H NMR (600 MHz, DMSO- $d_6$) δ 10.99 (s, 1H), 9.13 (s, 1H), 7.34 (t, J = 12.8 Hz, 2H), 7.14 (dq, J = 14.0, 7.1 Hz, 3H), 6.85 (d, J = 7.2 Hz, 2H), 6.67 – 6.55 (m, 4H), 6.49 (dd, J = 8.3, 2.5 Hz, 1H), 6.24 (d, J = 8.1 Hz, 2H), 5.10 (dd, J = 13.3, 5.1 Hz, 1H), 4.66 (s, 2H), 4.40 (d, J = 17.2 Hz, 1H), 4.24 (d, J = 17.2 Hz, 1H), 4.16 (d, J = 5.1 Hz, 1H), 3.71 (s, 3H), 3.63 (s, 2H), 3.35 – 3.27 (m, 3H), 3.16 (d, J = 20.3 Hz, 3H), 3.03 – 2.84 (m, 5H), 2.65 – 2.54 (m, 2H), 2.47 – 2.37 (m, 3H), 2.21 (s, 3H), 2.10 (qd, J = 13.1, 6.3 Hz, 3H), 2.04 – 1.82 (m, 4H), 1.80 – 1.58 (m, 3H). | 838. 43 | General Synthesis Formula 4 |

| | | | | | |
|---|---|---|---|---|---|
| **268** | H35 | 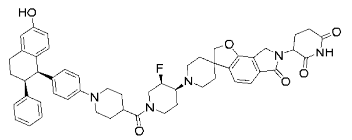 3-(1'-((3R,4S)-3-fluoro-1-(7-(4-(((1R,2S)-6-hydroxy-2-phenyl-1,2,3,4-tetrahydronaphthalen-1-yl)phenyl)-7-azaspiro[3.5]nonan-2-yl)piperidin-4-yl)-6-oxo-6,8-dihydro-2H,7H-spiro[furo[2,3-e]isoindole-3,4'-piperidine]-7-yl)piperidine-2,6-dione | $^1$H NMR (400 MHz, DMSO- $d_6$) δ 10.99 (s, 1H), 9.11 (s, 1H), 7.35 (s, 1H), 7.29 (s, 1H), 7.14 (dd, J = 9.6, 7.0 Hz, 3H), 6.87 – 6.81 (m, 2H), 6.65 (d, J = 8.0 Hz, 2H), 6.61 (d, J = 2.5 Hz, 2H), 6.50 (d, J = 2.5 Hz, 1H), 6.23 (d, J = 7.7 Hz, 2H), 5.33 (t, J = 4.8 Hz, 2H), 5.10 (dd, J = 13.1, 5.1 Hz, 1H), 4.67 (s, 2H), 4.40 (d, J = 17.1, 1H), 4.24 (d, J = 17.1, 1H), 4.15 (s, 1H), 3.70-3.50 (m, 4H), 2.94 (d, J = 17.4 Hz, 4H), 2.78 – 2.73 (m, 1H), 2.69 – 2.65 (m, 1H), 2.65-2.57 (m, 1H), 2.45 – 2.39 (m, 1H), 2.55 – 2.30 (m, 2H), 2.25-2.12 (m, 3H), 1.65 (s, 6H), 1.46 (t, J = 7.3 Hz, 4H). | 866. 42 | General Synthesis Formula 15 |
| **269** | H36 | 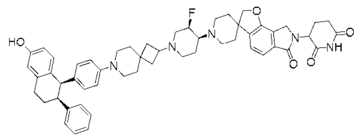 3-(1'-((3R,4S)-3-fluoro-1-(7-(4-(((1R,2S)-6-hydroxy-2-phenyl-1,2,3,4-tetrahydronaphthalen-1-yl)phenyl)-7-azaspiro[3.5]nonan-2-yl)piperidin-4-yl)-6-oxo-6,8-dihydro-2H,7H-spiro[furo[2,3-e]isoindole-3,4'-piperidine]-7-yl)piperidine-2,6-dione | $^1$H NMR (600 MHz, DMSO- $d_6$) δ 10.99 (s, 1H), 9.13 (s, 1H), 7.34 (t, J = 11.4 Hz, 2H), 7.13 (dq, J = 13.7, 7.1 Hz, 3H), 6.84 (d, J = 7.3 Hz, 2H), 6.70 – 6.53 (m, 4H), 6.49 (dd, J = 8.3, 2.5 Hz, 1H), 6.24 (d, J = 8.0 Hz, 2H), 5.10 (dd, J = 13.3, 5.1 Hz, 1H), 4.65 (s, 2H), 4.40 (d, J = 17.1 Hz, 2H), 4.24 (d, J = 17.1 Hz, 2H), 4.15 (d, J = 5.0 Hz, 2H), 3.90-3.70 (m, 2H), 3.34 – 3.27 (m, 2H), 3.20 (s, 3H), 3.06 – 2.81 (m, 7H), 2.66 – 2.56 (m, 1H), 2.48 – 2.36 (m, 2H), 2.29 – 2.04 (m, 7H), 2.03 – 1.81 (m, 4H), 1.72 (dd, J = 12.6, 5.9 Hz, 1H), 1.62 (s, 3H). | 878. 46 | General Synthesis Formula 7 |
| **270** | H37 | 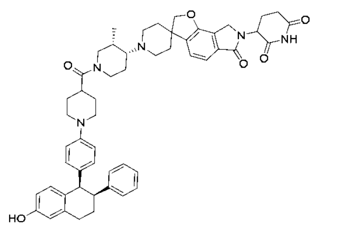 3-(1'-((3S,4R)-1-(1-(4-(((1R,2S)-6-hydroxy-2-phenyl-1,2,3,4-tetrahydronaphthalen-1-yl)phenyl)piperidine-4-carbonyl)-3-methylpiperidin-4-yl)-6-oxo-6,8-dihydro-2H,7H-spiro[furo[2,3-e]isoindole-3,4'-piperidine]-7- | $^1$H NMR (600 MHz, DMSO- $d_6$) δ 10.99 (s, 1H), 9.18 (s, 1H), 7.36 (d, J = 7.4 Hz, 1H), 7.28 (d, J = 7.8 Hz, 1H), 7.15 (dt, J = 14.4, 7.1 Hz, 3H), 6.84 (d, J = 7.3 Hz, 2H), 6.73 – 6.58 (m, 3H), 6.49 (dd, J = 8.4, 2.4 Hz, 1H), 6.28 (s, 2H), 5.17 – 5.05 (m, 1H), 4.66 (s, 2H), 4.40 (d, J = 17.1 Hz, 1H), 4.25 (d, J = 17.1 Hz, 1H), 4.18 (s, 1H), 4.00 (d, J = 13.7 Hz, 1H), 3.76 (s, 2H), 3.34 – 3.28 (m, 2H), 3.13 (dd, J = 33.2, 12.7 Hz, 2H), 3.04 – 2.86 (m, 5H), 2.86 – 2.69 (m, 2H), 2.61 (d, J = 16.3 Hz, 2H), 2.47-2.40 (m, 2H), 2.31-2.18 (m, | 862. 45 | General Synthesis Formula 15 |

| | | | | | |
|---|---|---|---|---|---|
| | | yl)piperidine-2,6-dione | 2H), 2.15-2.05 (m, 2H), 2.06 – 1.89 (m, 3H), 1.88 -1.51 (m, 7H), 1.05- 0.85 (m,3H) | | |
| **271** | H38 | 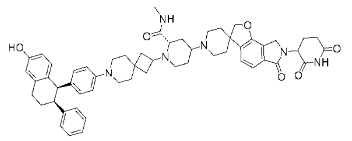 (2S)-4-(7-(2,6-Dioxopiperidin-3-yl)-6-oxo-7,8-dihydro-2H,6H-spiro[furo[2,3-e]isoindole-3,4'-piperidine]-1'-yl)-1-(7-(4-((1R,2S)-6-hydroxy-2-phenyl-1,2,3,4-tetrahydronaphthalen-1-yl)phenyl)-7-azaspiro[3.5]nonan-2-yl)-N-methylpiperidine-2-carboxamide | $^1$H NMR (600 MHz, DMSO- $d_6$) δ 10.99 (s, 1H), 9.13 (s, 1H), 7.36 (d, J = 7.5 Hz, 1H), 7.28 (d, J = 7.6 Hz, 1H), 7.16 – 7.13 (m, 3H), 6.84 (dd, J = 5.5, 3.3 Hz, 2H), 6.66 – 6.61 (m, 3H), 6.49 (dd, J = 8.2, 2.5 Hz, 2H), 6.29 (s, 2H), 5.10 (dd, J = 13.2, 5.1 Hz, 1H), 4.69 (p, J = 9.5 Hz, 2H), 4.40 (d, J = 17.2 Hz, 1H), 4.24 (d, J = 17.1 Hz, 1H), 4.18 (s, 1H), 3.43-3.36 (m, 2H), 3.35-3.27 (m, 1H), 3.16-3.09 (m, 4H), 3.06 – 2.90 (m, 10H), 2.89 (s, 3H), 2.85 – 2.80 (m, 1H), 2.64 – 2.57 (m, 1H), 2.47 – 2.41 (m, 1H), 2.36 (d, J = 11.8 Hz, 2H), 2.29 – 2.14 (m, 4H), 2.15-2.05 (m, 2H), 2.05-1.95 (m, 4H), 1.80 - 1.52 (m, 7H). | 917.49 | General Synthesis Formula 8 |
| **272** | H39 | 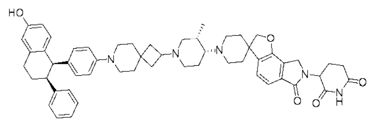 3-(1'-((3S,4R)-1-(7-(4-(((1R,2S)-6-hydroxy-2-phenyl-1,2,3,4-tetrahydronaphthalen-1-yl)phenyl)-7-azaspiro[3.5]nonan-2-yl)-3-methylpiperidin-4-yl)-6-oxo-6,8-dihydro-2H,7H-spiro[furo[2,3-e]isoindole-3,4'-piperidine]-7-yl)piperidine-2,6-dione | $^1$H NMR (600 MHz, DMSO- $d_6$) δ 10.99 (s, 1H), 9.14 (s, 1H), 7.36 (d, J = 7.7 Hz, 1H), 7.29 (d, J = 7.7 Hz, 1H), 7.20 – 7.09 (m, 3H), 6.88 – 6.81 (m, 2H), 6.69 – 6.56 (m, 4H), 6.49 (dd, J = 8.2, 2.6 Hz, 1H), 6.24 (d, J = 8.1 Hz, 2H), 5.10 (dd, J = 13.2, 5.1 Hz, 1H), 4.66 (q, J = 10.8, 7.9 Hz, 2H), 4.40 (d, J = 17.3 Hz, 1H), 4.24 (d, J = 17.1 Hz, 1H), 4.16 (d, J = 4.9 Hz, 1H), 3.38 (d, J = 12.3 Hz, 2H), 3.33 – 3.25 (m, 2H), 3.11 (s, 1H), 3.07 – 2.85 (m, 9H), 2.76 -2.65 (m, 2H), 2.64 – 2.56 (m, 1H), 2.46 – 2.34 (m, 2H), 2.28 (d, J = 14.3 Hz, 2H), 2.20 – 2.09 (m, 4H), 2.03 (d, J = 34.7 Hz, 6H), 1.72 (d, J = 6.1 Hz, 1H), 1.63 (s, 4H), 1.30 – 1.14 (m, 3H) | 874.48 | General Synthesis Formula 7 |
| **273** | H40 | 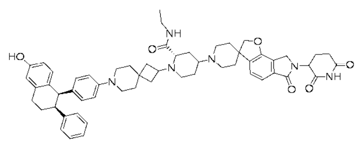 (2S)-4-(7-(2,6-Dioxopiperidin-3-yl)-6-oxo-7,8-dihydro-2H,6H-spiro[furo[2,3-e]isoindole-3,4'-piperidine]-1'-yl)-N-ethyl-1-(7-(4-((1R,2S)-6-hydroxy-2- | $^1$H NMR (600 MHz, DMSO- $d_6$) δ 10.99 (s, 1H), 9.13 (s, 1H), 8.77 (s, 1H), 7.36 (d, J = 7.6 Hz, 1H), 7.29 (d, J = 7.7 Hz, 1H), 7.17 – 7.08 (m, 3H), 6.89 – 6.79 (m, 2H), 6.66 – 6.51 (m, 4H), 6.48 (dd, J = 8.2, 2.5 Hz, 1H), 6.23 (d, J = 8.0 Hz, 2H), 5.10 (dd, J = 13.3, 5.1 Hz, 1H), 4.67 (d, J = 8.5 Hz, 2H), 4.40 (d, J = 17.1 Hz, 1H), 4.24 | 931.50 | General Synthesis Formula 8 |

| | | | | | |
|---|---|---|---|---|---|
| | | phenyl-1,2,3,4-tetrahydronaphthalen-1-yl)phenyl)-7-azaspiro[3.5]nonan-2-yl)piperidine-2-carboxamide | (d, J = 17.1 Hz, 1H), 4.15 (d, J = 5.0 Hz, 1H), 3.34 – 3.26 (m, 3H), 3.17 (p, J = 6.8 Hz, 4H), 3.09 (s, 1H), 3.05-2.85 (m, 9H), 2.65 – 2.56 (m, 1H), 2.45 – 2.38 (m, 1H), 2.35 – 2.14 (m, 4H), 2.13 – 1.88 (m, 10H), 1.71 (d, J = 5.4 Hz, 1H), 1.58 (d, J = 28.2 Hz, 4H), 1.09 (t, J = 7.2 Hz, 3H) | | |
| **274** | H41 | 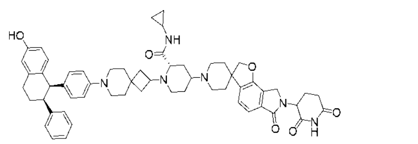 ((2S)-N-Cyclopropyl-4-(7-(2,6-Dioxopiperidin-3-yl)-6-oxo-7,8-dihydro-2H,6H-spiro[furo[2,3-e]isoindole-3,4'-piperidine]-1'-yl)-1-(7-(4-((1R,2S)-6-hydroxy-2-phenyl-1,2,3,4-tetrahydronaphthalen-1-yl)phenyl)-7-azaspiro[3.5]nonan-2-yl)piperidine-2-carboxamide | $^1$H NMR (600 MHz, DMSO- $d_6$) δ 10.99 (s, 1H), 9.13 (s, 1H), 8.88 (s, 1H), 7.36 (d, J = 7.6 Hz, 1H), 7.29 (d, J = 7.7 Hz, 1H), 7.18 – 7.08 (m, 3H), 6.88 – 6.79 (m, 2H), 6.68 – 6.51 (m, 4H), 6.48 (dd, J = 8.3, 2.6 Hz, 1H), 6.23 (d, J = 8.0 Hz, 2H), 5.10 (dd, J = 13.2, 5.1 Hz, 1H), 4.66 (q, J = 9.5 Hz, 2H), 4.40 (d, J = 17.1 Hz, 1H), 4.24 (d, J = 17.2 Hz, 1H), 4.15 (d, J = 4.9 Hz, 1H), 3.34 – 3.27 (m, 2H), 3.20-3.05 (m, 2H), 3.05-2.85 (m, 9H), 2.71 (dq, J = 7.3, 3.7 Hz, 1H), 2.64 – 2.57 (m, 1H), 2.46 – 2.38 (m, 1H), 2.37 – 2.14 (m, 4H), 2.14 – 1.86 (m, 10H), 1.72 (s, 1H), 1.58 (d, J = 26.8 Hz, 4H), 0.72 (d, J = 7.3 Hz, 2H), 0.48 (dd, J = 7.4, 3.5 Hz, 2H) | 943.50 | General Synthesis Formula 8 |
| **275** | H42 | 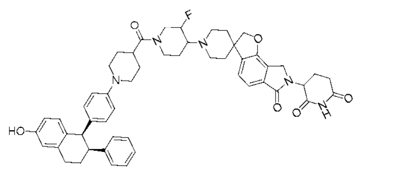 3-(1'-(3-fluoro-1-(1-(4-((1R,2S)-6-hydroxy-2-phenyl-1,2,3,4-tetrahydronaphthalen-1-yl)phenyl)piperidin-4-ylcarbonyl)piperidin-4-yl)-6-oxo-6,8-dihydro-2H,7H-spiro[furo[2,3-e]isoindole-3,4'-piperidine]-7-yl)piperidine-2,6-dione | $^1$H NMR (600 MHz, DMSO- $d_6$) δ 10.99 (s, 1H), 9.14 (s, 1H), 7.36 (d, J = 7.7 Hz, 1H), 7.28 (d, J = 7.6 Hz, 1H), 7.15 (dt, J = 14.2, 7.1 Hz, 3H), 6.84 (d, J = 7.3 Hz, 2H), 6.72 – 6.57 (m, 4H), 6.49 (dd, J = 8.3, 2.5 Hz, 1H), 6.28 (s, 2H), 5.56 – 5.30 (m, 3H), 5.10 (dd, J = 13.3, 5.1 Hz, 1H), 4.68 (q, J = 9.4 Hz, 2H), 4.40 (d, J = 17.1 Hz, 1H), 4.24 (d, J = 17.2 Hz, 1H), 4.18 (s, 1H), 3.60 – 3.45 (m, 4H),3.37 – 3.29 (m, 2H), 3.26 – 3.11 (m, 3H), 3.04 – 2.84 (m, 5H), 2.63 – 2.57 (m, 1H), 2.47 – 2.40 (m, 1H),2.30-2.20 (m, 2H), 2.15-2.05 (m, 1H), 2.04 – 1.90 (m, 4H), 1.80-1.60 (m, 6H). | 866.42 | General Synthesis Formula 15 |
| **276** | H43 | 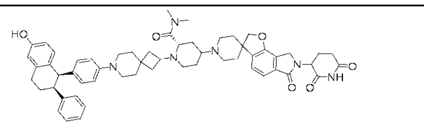 (2S)-4-(7-(2,6-Dioxopiperidin-3-yl)-6- | $^1$H NMR (600 MHz, DMSO- $d_6$) δ 10.99 (s, 1H), 9.14 (s, 1H), 7.36 (d, J = 7.5 Hz, 1H), 7.28 (d, J = 7.7 Hz, 1H), 7.14 (dt, J = 12.5, 6.6 Hz, 3H), 6.90 – 6.79 (m, 2H), | 931.50 | General Synthesis Formula 8 |

| | | | | | |
|---|---|---|---|---|---|
| | | oxo-7,8-dihydro-2H,6H-spiro[furo[2,3-e]isoindole-3,4'-piperidine]-1'-yl)-1-(7-(4-((1R,2S)-6-hydroxy-2-phenyl-1,2,3,4-tetrahydronaphthalen-1-yl)phenyl)-7-azaspiro[3.5]nonan-2-yl)-N,N-dimethylpiperidine-2-carboxamide | 6.71 – 6.54 (m, 4H), 6.49 (dd, J = 8.2, 2.6 Hz, 1H), 6.24 (d, J = 8.1 Hz, 2H), 5.10 (dd, J = 13.2, 5.1 Hz, 1H), 4.75 – 4.61 (m, 2H), 4.40 (d, J = 17.2 Hz, 2H), 4.24 (d, J = 17.1 Hz, 1H), 4.16 (d, J = 5.1 Hz, 1H), 3.78 (s, 2H), 3.42 – 3.27 (m, 4H), 3.15 (s, 7H), 3.01 – 2.84 (m, 10H), 2.66 – 2.57 (m, 1H), 2.47-2.35 (m, 2H), 2.24 (s, 2H), 2.19 – 2.07 (m, 2H), 2.06 – 1.91 (m, 7H), 1.86 (d, J = 12.3 Hz, 1H), 1.72 (d, J = 6.2 Hz, 1H), 1.59 (d, J = 33.8 Hz, 4H) | | |
| **277** | H44 | 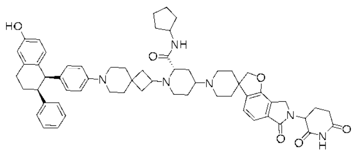 (2S)-N-Cyclopentyl-4-(7-(2,6-Dioxopiperidin-3-yl)-6-oxo-7,8-dihydro-2H,6H-spiro[furo[2,3-e]isoindole-3,4'-piperidine]-1'-yl)-1-(7-(4-((1R,2S)-6-hydroxy-2-phenyl-1,2,3,4-tetrahydronaphthalen-1-yl)phenyl)-7-azaspiro[3.5]nonan-2-yl)piperidine-2-carboxamide | $^1$H NMR (600 MHz, DMSO- $d_6$) δ 10.99 (s, 1H), 9.13 (s, 1H), 8.76 (s, 1H), 7.36 (d, J = 7.6 Hz, 1H), 7.29 (d, J = 7.6 Hz, 1H), 7.17 – 7.08 (m, 3H), 6.90 – 6.79 (m, 2H), 6.68 – 6.52 (m, 4H), 6.48 (dd, J = 8.3, 2.5 Hz, 1H), 6.23 (d, J = 8.1 Hz, 2H), 5.10 (dd, J = 13.2, 5.1 Hz, 1H), 4.67 (q, J = 9.4, 8.8 Hz, 2H), 4.40 (d, J = 17.1 Hz, 1H), 4.24 (d, J = 17.2 Hz, 1H), 4.15 (d, J = 4.9 Hz, 1H), 4.01 (q, J = 6.8 Hz, 1H), 3.33 – 3.25 (m, 2H), 3.22 – 3.08 (m, 2H), 3.04 – 2.81 (m, 9H), 2.67 – 2.57 (m, 1H), 2.47-2.40 (m, 1H), 2.29 (d, J = 43.6 Hz, 4H), 2.16 – 1.91 (m, 9H), 1.88 – 1.77 (m, 2H), 1.78 – 1.50 (m, 10H), 1.47-1.40 (m, 2H) | 971.54 | General Synthesis Formula 8 |
| **278** | H45 | 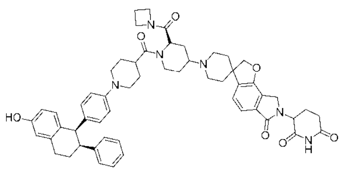 3-(1'-((2S)-2-(azetidine-1-carbonyl)-1-(1-(4-(((1R,2S)-6-hydroxy-2-phenyl-1,2,3,4-tetrahydronaphthalen-1-yl)phenyl)piperidin-4-ylcarbonyl)piperidin-4-yl)-6-oxo-6,8-dihydro-2H,7H-spiro[furo[2,3-e]isoindole-3,4'-piperidine]-7-yl)piperidine-2,6-dione | $^1$H NMR (600 MHz, DMSO- $d_6$) δ 10.99 (s, 1H), 9.14 (s, 1H), 7.36 (d, J = 7.6 Hz, 1H), 7.29 (d, J = 7.5 Hz, 1H), 7.20 – 7.10 (m, 3H), 6.88 – 6.81 (m, 2H), 6.81 – 6.54 (m, 4H), 6.50 (dd, J = 8.2, 2.6 Hz, 1H), 6.31 (s, 2H), 5.10 (dd, J = 13.3, 5.1 Hz, 1H), 4.76 – 4.56 (m, 3H), 4.40 (d, J = 17.0 Hz, 2H), 4.33 – 4.14 (m, 3H), 3.95-3.88 (m, 2H), 3.55-3.478 (m, 6H), 3.33 (d, J = 12.9 Hz, 1H), 3.18 (d, J = 11.3 Hz, 1H), 3.07 – 2.88 (m, 5H), 2.84 (s, 1H), 2.65 – 2.57 (m, 1H), 2.47 – 2.37 (m, 1H), 2.31 – 2.07 (m, 7H), 2.07 – 1.92 (m, 6H), 1.74 (d, J = 23.4 Hz, 4H). | 931.47 | General Synthesis Formula 16 |

| | | | | | |
|---|---|---|---|---|---|
| **279** | H46 | 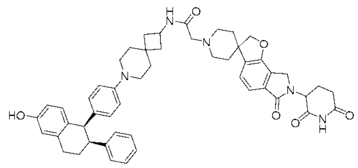 2-(7-(2,6-Dioxopiperidin-3-yl)-6-oxo-7,8-dihydro-2H,6H-spiro[furo[2,3-e]isoindole-3,4'-piperidine]-1'-yl)-N-(7-(4-((1R,2S)-6-hydroxy-2-phenyl-1,2,3,4-tetrahydronaphthalen-1-yl)phenyl)-7-azaspiro[3.5]nonan-2-yl)acetamide | $^1$H NMR (600 MHz, DMSO- $d_6$) δ 10.99 (s, 1H), 9.13 (s, 1H), 8.86 (d, J = 7.1 Hz, 1H), 7.36 (d, J = 7.6 Hz, 1H), 7.29 (d, J = 7.6 Hz, 1H), 7.20 – 7.07 (m, 3H), 6.89 – 6.79 (m, 2H), 6.79 – 6.52 (m, 4H), 6.49 (dd, J = 8.2, 2.6 Hz, 1H), 6.28 (s, 2H), 5.10 (dd, J = 13.3, 5.1 Hz, 1H), 4.71 – 4.60 (m, 2H), 4.54 (d, J = 6.7 Hz, 1H), 4.39 (d, J = 17.1 Hz, 1H), 4.34 – 4.26 (m, 1H), 4.30-4.23 (m, 2H), 4.18 (s, 1H), 3.49 (d, J = 11.7 Hz, 1H), 3.42 (s, 1H), 3.35 – 3.27 (m, 2H), 3.25-3.12 (m, 2H), 3.10 – 2.87 (m, 5H), 2.65 – 2.57 (m, 1H), 2.47 – 2.37 (m, 1H), 2.35 –2.17(m, 4H), 2.15-2.05 (m, 2H), 2.21 – 1.87 (m, 3H), 1.80 – 1.60 (m, 6H) | 834.42 | General Synthesis Formula 40 |
| **280** | H47 | 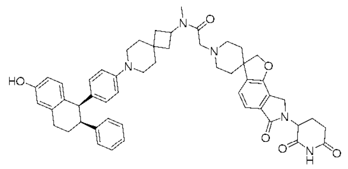 N-(7-(4-(((1R,2S)-6-hydroxy-2-phenyl-1,2,3,4-tetrahydronaphthalen-1-yl)phenyl)-7-azaspiro[3.5]nonan-2-yl)-N-methyl-2-(6-oxo-7-(2-oxopiperidin-3-yl)-7,8-dihydro-2H,6H-spiro[furo[2,3-e]isoindole-3,4'-piperidine]-1'-yl)acetamide | $^1$H NMR (600 MHz, DMSO- $d_6$) δ 10.99 (s, 1H), 9.14 (s, 1H), 7.37 (d, J = 7.5 Hz, 1H), 7.31 (d, J = 7.6 Hz, 1H), 7.19 – 7.10 (m, 3H), 6.84 (d, J = 7.3 Hz, 2H), 6.79 – 6.54 (m, 4H), 6.49 (dd, J = 8.4, 2.5 Hz, 1H), 6.26 (d, J = 8.8 Hz, 2H), 5.10 (dd, J = 13.3, 5.1 Hz, 1H), 4.70 – 4.61 (m, 2H), 4.39 (d, J = 17.2 Hz, 1H), 4.31 (d, J = 4.6 Hz, 1H), 4.33 – 4.26 (m, 2H), 4.24 (d, J = 17.1 Hz, 1H),4.17 (s, 1H), 3.50 (d, J = 12.0 Hz, 2H), 3.43-3.36 (m, 1H), 3.35 – 3.23 (m, 1H), 3.20-3.03 (m, 3H), 3.03-2.87 (m,8H), 2.65 – 2.55 (m, 1H), 2.47 – 2.37 (m, 1H), 2.34 – 2.13 (m, 2H), 2.20 – 1.87 (m, 9H), 1.80 – 1.60 (m, 4H). | 848.45 | General Synthesis Formula 40 |
| **281** | H48 | 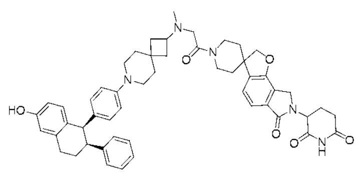 2-(7-(2,6-Dioxopiperidin-3-yl)-6-oxo-7,8-dihydro-2H,6H-spiro[furo[2,3-e]isoindole-3,4'-piperidine]-1'-yl)-N-(7-(4-((1R,2S)-6-hydroxy-2-phenyl-1,2,3,4-tetrahydronaphthalen-1-yl)phenyl)-7-azaspiro[3.5]nonan-2-yl)- | $^1$H NMR (600 MHz, DMSO- $d_6$) δ 10.99 (s, 1H), 9.15 (s, 1H), 7.39 (dd, J = 13.1, 7.6 Hz, 1H), 7.31 (d, J = 7.5 Hz, 1H), 7.14 (q, J = 6.9, 6.4 Hz, 3H), 6.90 – 6.80 (m, 2H), 6.78 – 6.59 (m, 4H), 6.49 (dd, J = 8.3, 2.5 Hz, 1H), 6.26 (d, J = 8.0 Hz, 2H), 5.18 – 5.04 (m, 1H), 4.73-4.61 (m, 2H), 4.45 – 4.32 (m, 1H), 4.28 – 4.12 (m, 2H), 3.84 – 3.74 (m, 1H),3.68 (d, J = 13.3 Hz, 1H), 3.36 – 3.28 (m, 1H), 3.20 (s, 1H), 3.12 – 2.83 (m, 7H), 2.72 (dd, J = 17.5, | 848.43 | General Synthesis Formula 40 |

| | | | | | |
|---|---|---|---|---|---|
| | | N-methylacetamide | 4.5 Hz, 3H), 2.64 – 2.56 (m, 1H), 2.17 (s, 3H),2.47 – 2.37 (m, 1H), 2.17 (s, 3H), 2.03 – 1.90 (m, 3H), 1.86 – 1.57 (m, 9H) | | |
| **282** | H49 | 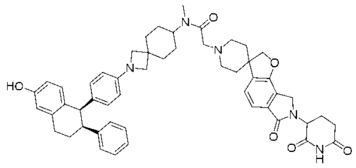 2-(7-(2,6-Dioxopiperidin-3-yl)-6-oxo-7,8-dihydro-2H,6H-spiro[furo[2,3-e]isoindole-3,4'-piperidine]-1'-yl)-N-(2-(4-((1R,2S)-6-hydroxy-2-phenyl-1,2,3,4-tetrahydronaphthalen-1-yl)phenyl)-2-azaspiro[3.5]nonan-7-yl)-N-methylacetamide | $^1$H NMR (600 MHz, DMSO- $d_6$) δ 10.99 (s, 1H), 9.11 (s, 1H), 7.37 (d, J = 7.5 Hz, 1H), 7.31 (d, J = 7.6 Hz, 1H), 7.18 – 7.10 (m, 3H), 6.87 – 6.81 (m, 2H), 6.67 – 6.57 (m, 2H), 6.48 (dd, J = 8.2, 2.5 Hz, 1H), 6.18 (d, J = 8.1 Hz, 2H), 6.11 – 5.98 (m, 2H), 5.10 (dd, J = 13.2, 5.1 Hz, 1H), 4.65 (t, J = 2.6 Hz, 2H), 4.42-4.32 (m, 1H), 4.30-4.18 (m, 1H), 4.12 (d, J = 4.9 Hz, 1H), 3.58 – 3.43 (m, 4H), 3.36 (d, J = 9.4 Hz, 3H), 3.32 – 3.24 (m, 1H), 3.22 – 3.06 (m, 2H), 2.98 – 2.84 (m, 3H), 2.79 (s, 3H), 2.65 – 2.56 (m, 1H), 2.47 – 2.40 (m, 1H), 2.37 – 2.24 (m, 2H), 2.10 (dd, J = 12.0, 6.3 Hz, 1H), 1.95 (t, J = 13.1 Hz, 5H), 1.72 (t, J = 14.3 Hz, 1H), 1.58 (d, J = 15.5 Hz, 5H), 1.47 (s, 1H). | 848.43 | General Synthesis Formula 40 |
| **283** | H50 | 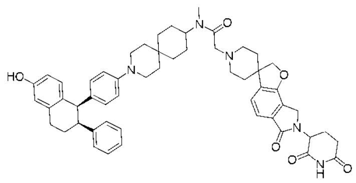 2-(7-(2,6-Dioxopiperidin-3-yl)-6-oxo-7,8-dihydro-2H,6H-spiro[furo[2,3-e]isoindole-3,4'-piperidine]-1'-yl)-N-(3-(4-((1R,2S)-6-hydroxy-2-phenyl-1,2,3,4-tetrahydronaphthalen-1-yl)phenyl)-3-azaspiro[5.5]undecan-9-yl)-N-methylacetamide | $^1$H NMR (600 MHz, DMSO- $d_6$) δ 11.00 (s, 1H), 9.16 (s, 1H), 7.37 (d, J = 7.5 Hz, 1H), 7.31 (d, J = 7.6 Hz, 1H), 7.15 (q, J = 6.8, 5.9 Hz, 3H), 6.92 – 6.76 (m, 3H), 6.68 – 6.58 (m, 3H), 6.49 (dd, J = 8.2, 3.1 Hz, 1H), 6.34 (s, 1H), 6.26 (d, J = 8.1 Hz, 1H), 5.10 (dd, J = 13.2, 5.1 Hz, 1H), 4.65 (s, 2H), 4.40 (d, J = 17.4Hz 1H), 4.29-4.15 (m, 3H), 3.52 (d, J = 11.6 Hz, 2H), 3.40 – 3.29 (m, 3H), 3.25-3.05 (m, 4H), 3.01 – 2.91 (m, 3H), 2.84 (s, 3H), 2.65-2.55 (m, 1H), 2.47 – 2.40 (m, 1H),2.37 – 2.26 (m, 2H), 2.15-2.05 (m, 1H), 2.05-1.90 (m, 4H), 1.85-1.65 (m, 5H), 1.64 – 1.33 (m, 7H). | 876.46 | General Synthesis Formula 40 |
| **284** | H51 | 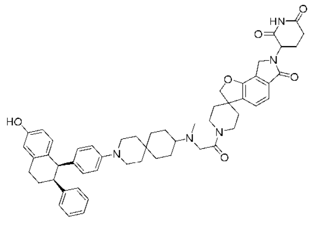 7-(2,6-Dioxopiperidin-3-yl)-N-(3-(4- | $^1$H NMR (600 MHz, DMSO- $d_6$) δ 10.99 (d, J = 2.2 Hz, 1H), 9.16 (s, 1H), 7.38 (d, J = 7.6 Hz, 1H), 7.32 (dd, J = 7.5, 3.0 Hz, 1H), 7.19 – 7.10 (m, 3H), 6.89 – 6.80 (m, 2H), 6.70 – 6.58 (m, 3H), 6.49 (dd, J = 8.3, 2.6 Hz, 1H), 6.32 (s, 2H), 5.17 – 5.03 (m, 1H), 4.77 – 4.60 (m, 2H), 4.40 (dd, J | 876.45 | General Synthesis Formula 41 |

| | | | | | |
|---|---|---|---|---|---|
| | | ((1R,2S)-6-hydroxy-2-phenyl-1,2,3,4-tetrahydronaphthalen-1-yl)phenyl)-3-azaspiro[5.5]undecan-9-yl)-N-methyl-6-oxo-7,8-dihydro-2H,6H-spiro[furo[2,3-e]isoindole-3,4'-piperidine]-1'-carboxamide | = 17.1, 8.8 Hz, 3H), 4.25 – 4.17 (m, 2H), 3.33 (d, J = 13.1 Hz, 1H), 3.26 – 3.04 (m, 6H), 3.04 – 2.84 (m, 5H), 2.78 (dd, J = 21.1, 4.9 Hz, 3H), 2.64 – 2.56 (m, 1H), 2.47 – 2.38 (m, 1H), 2.14 - 2.05 (m, 1H), 2.01 – 1.95 (m, 2H), 1.95 – 1.87 (m, 2H), 1.87 – 1.76 (m, 5H),1.73 (d, J = 8.7 Hz, 2H), 1.67-1.57 (m, 3H), 1.55 – 1.39 (m, 2H). | | |
| **285** | H52 | 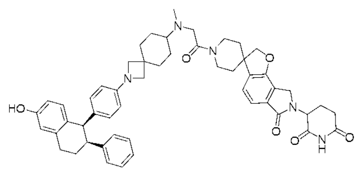 7-(2,6-Dioxopiperidin-3-yl)-N-(2-(4-(((1R,2S)-6-hydroxy-2-phenyl-1,2,3,4-tetrahydronaphthalen-1-yl)phenyl)-2-azaspiro[3.5]nonan-7-yl)-N-methyl-6-oxo-7,8-dihydro-2H,6H-spiro[furo[2,3-e]isoindole-3,4'-piperidine]-1'-carboxamide | $^1$H NMR (600 MHz, DMSO- $d_6$) δ 10.99 (s, 1H), 9.20 (s, 1H),δ 7.38 (dd, J = 7.5, 5.0 Hz, 1H), 7.32 (dd, J = 7.5, 1.8 Hz, 1H), 7.17 – 7.09 (m, 3H), 6.86 – 6.81 (m, 2H), 6.65 – 6.58 (m, 2H), 6.48 (dd, J = 8.3, 2.5 Hz, 1H), 6.18 (d, J = 8.2 Hz, 2H), 6.06 – 6.01 (m, 2H), 5.10 (dt, J = 12.1, 5.7 Hz, 1H), 4.75 – 4.60 (m, 2H), 4.43 – 4.30 (m, 3H), 4.24 (dd, J = 17.1, 12.3 Hz, 1H), 4.20 – 4.13 (m, 1H), 4.12 (d, J = 5.0 Hz, 1H), 3.49 – 3.41 (m, 2H), 3.35 (d, J = 7.7 Hz, 2H), 3.31 – 3.25 (m, 1H), 3.24 – 3.14 (m, 2H), 3.01 – 2.85 (m, 2H), 2.76 (dd, J = 20.5, 4.9 Hz, 2H), 2.64 – 2.57 (m, 2H), 2.47 – 2.37 (m, 1H), 2.14 – 2.05 (m, 1H), 2.04 - 1.90 (m, 6H), 1.85 – 1.75 (m, 3H), 1.74 – 1.66 (m,1H), 1.56 – 1.42 (m, 4H). | 848. 42 | General Synthesis Formula 41 |
| **286** | H53 | 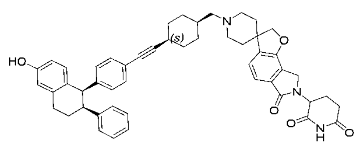 3-(1'-(((1S,4s)-4-((4-(((1R,2S)-6-hydroxy-2-phenyl-1,2,3,4-tetrahydronaphthalen-1-yl)phenyl)ethynyl)cyclohexyl)methyl)-6-oxo-6,8-dihydro-2H,7H-spiro[furo[2,3-e]isoindole-3,4'-piperidine]-7-yl)piperidine-2,6-dione | $^1$H NMR (600 MHz, DMSO- $d_6$) δ 11.00 (s, 1H), 9.19 (s, 1H), 7.36 (d, J = 7.6 Hz, 1H), 7.27 (d, J = 7.6 Hz, 1H), 7.20 – 7.10 (m, 3H), 6.96 (d, J = 8.1 Hz, 2H), 6.87 – 6.82 (m, 2H), 6.67 – 6.59 (m, 2H), 6.50 (dd, J = 8.4, 2.6 Hz, 1H), 6.35 (d, J = 8.1 Hz, 2H), 5.10 (dd, J = 13.3, 5.1 Hz, 1H), 4.70 – 4.62 (m, 2H), 4.40 (dd, J = 17.2, 3.2 Hz, 1H), 4.24 (d, J = 17.2,1H),3.60– 3.52 (m, 2H),3.22 (s, 1H), 3.14 – 3.01 (m, 2H), 3.01 – 2.89 (m, 5H), 2.65 – 2.57 (m, 1H), 2.48 – 2.38 (m, 2H), 2.21 (t, J = 13.7 Hz, 2H), 2.15 – 2.04 (m, 1H), 2.03 – 1.90 (m, 5H), 1.80 (d, J = 12.5 Hz, 3H), 1.74 (dd, J = 11.9, 6.3 Hz, 1H), 1.41 (q, J = 12.0 Hz, 2H), 1.10 - 1.01 (m, 2H) | 774. 38 | General Synthesis Formula 79 |

| | | | | | |
|---|---|---|---|---|---|
| **287** | H54 | 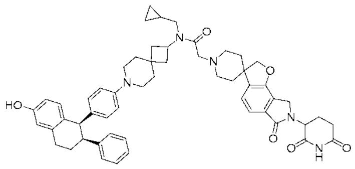 N-(Cyclopropylmethyl)-2-(7-(2,6-dioxopiperidin-3-yl)-6-oxo-7,8-dihydro-2H,6H-spiro[furo[2,3-e]isoindole-3,4'-piperidine]-1'-yl)-N-(7-(4-((1R,2S)-6-hydroxy-2-phenyl-1,2,3,4-tetrahydronaphthalen-1-yl)phenyl)-7-azaspiro[3.5]nonan-2-yl)acetamide | $^1$H NMR (600 MHz, DMSO- $d_6$) δ 11.01 (s, 1H), 9.16 (s, 1H), 7.37 (d, J = 7.6 Hz, 1H), 7.30 (d, J = 7.6 Hz, 1H), 7.14 (q, J = 7.0, 6.4 Hz, 3H), 6.83 (dd, J = 7.6, 2.0 Hz, 2H), 6.75 – 6.58 (m, 4H), 6.49 (dd, J = 8.2, 2.4 Hz, 1H), 6.27 (s, 2H), 5.10 (dd, J = 13.2, 5.1 Hz, 1H), 4.70 – 4.60 (m, z2H), 4.42 (s, 1H), 4.39 (d, J = 17.3 Hz, 1H), 4.30 (d, J = 4.6 Hz, 1H), 4.23 (d, J = 17.3 Hz, 1H), 4.17 (s, 2H), 3.50 (d, J = 11.5 Hz, 2H), 3.37-3.25 (m, 3H), 3.24 – 2.85 (m, 10H), 2.70 – 2.54 (m, 1H), 2.47 – 2.37 (m, 1H), 2.37 – 2.19 (m, 3H), 2.19 – 2.04 (m, 2H), 2.03 – 1.88 (m, 4H), 1.85 – 1.60 (m, 4H), 0.96 (s, 1H), 0.60 – 0.50 (m, 1H), 0.46 (dd, J = 8.1, 1.9 Hz, 1H), 0.38 – 0.27 (m, 2H) | 888. 46 | General Synthesis Formula 40 |
| **288** | H55 | 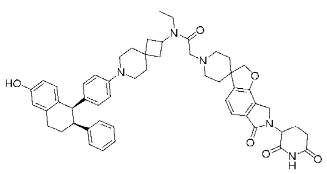 2-(7-(2,6-Dioxopiperidin-3-yl)-6-oxo-7,8-dihydro-2H,6H-spiro[furo[2,3-e]isoindole-3,4'-piperidine]-1'-yl)-N-ethyl-N-(7-(4-((1R,2S)-6-hydroxy-2-phenyl-1,2,3,4-tetrahydronaphthalen-1-yl)phenyl)-7-azaspiro[3.5]nonan-2-yl)acetamide | $^1$H NMR (600 MHz, DMSO- $d_6$) δ 11.00 (s, 1H), 9.15 (s, 1H), 7.37 (d, J = 7.5 Hz, 1H), 7.30 (d, J = 7.6 Hz, 1H), 7.19 – 7.08 (m, 3H), 6.86 – 6.80 (m, 2H), 6.76 – 6.56 (m, 4H), 6.48 (dd, J = 8.3, 2.5 Hz, 1H), 6.27 (s, 2H), 5.10 (dd, J = 13.3, 5.1 Hz, 1H), 4.70 – 4.59 (m, 2H), 4.38 (d, J = 17.2Hz, 1H), 4.35-4.15 (m, 5H), 3.55-3.35 (m, 4H), 3.31 (d, J = 10.5 Hz, 2H), 3.24 – 2.83 (m, 10H), 2.65 – 2.55 (m, 1H), 2.45 – 2.38 (m, 1H),2.39 – 2.24 (m, 2H), 2.24 – 2.04 (m, 3H), 2.04 – 1.87 (m, 4H), 1.72 (s, 4H), 1.11 (dt, J = 22.7, 6.9 Hz, 3H) | 862. 45 | General Synthesis Formula 40 |
| **289** | H56 | 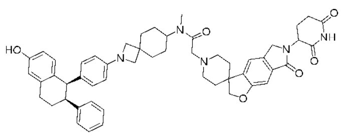 2-(6-(2,6-Dioxopiperidin-3-yl)-7-oxo-6,7-dihydro-2H,5H-spiro[furo[2,3-f]isoindole-3,4'-piperidine]-1'-yl)-N-(2-(4-((1R,2S)-6-hydroxy-2-phenyl-1,2,3,4-tetrahydronaphthalen-1-yl)phenyl)-2-azaspiro[3.5]nonan-7-yl)-N-methylacetamide | $^1$H NMR (600 MHz, DMSO- $d_6$)δ 11.00 (s, 1H), 9.12 (s, 1H), 7.35 (s, 1H), 7.19 – 7.07 (m,4H), 6.87 – 6.80 (m, 2H), 6.67 – 6.57 (m, 2H), 6.51 – 6.44 (m, 1H), 6.17 (t, J = 6.1 Hz, 2H), 6.04 (dd, J = 8.7, 2.6 Hz, 2H), 5.10 (dd, J = 13.2, 5.1 Hz, 1H), 4.59 (d, J = 2.6 Hz, 2H), 4.39 (d, J = 17.2 Hz, 1H), 4.31 – 4.24 (m, 1H), 4.11 (d, J = 4.9 Hz, 1H), 3.57 – 3.43 (m, 5H), 3.43 – 3.33 (m, 4H), 3.31 – 3.24 (m, 1H), 3.20 – 3.07 (m, 2H), 3.03 – 2.83 (m,3H), 2.79 (s, 3H), | 848. 43 | General Synthesis Formula 40 |

| | | | | | |
|---|---|---|---|---|---|
| | | | 2.65-2.55 (m, 1H), 2.43 – 2.23 (m, 3H), 2.05 - 1.85 (m, 4H), 1.80 – 1.65 (m, 2H), 1.66 – 1.39 (m, 6H). | | |
| **290** | H57 | 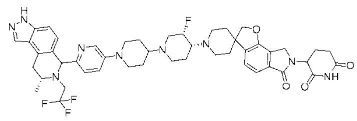 3-(1'-((3S,4R)-3-fluoro-1'-(6-((8R)-8-methyl-7-(2,2,2-trifluoroethyl)-6,7,8,9-tetrahydro-3H-pyrazolo[4,3-f]isoquinolin-6-yl)pyridin-3-yl)-[1,4'-bipiperidinyl]-4-yl)-6-oxo-6,8-dihydro-2H,7H-spiro[furo[2,3-e]isoindole-3,4'-piperidine]-7-yl)piperidine-2,6-dione | $^1$H NMR (600 MHz, DMSO- $d_6$) δ13.04 (s, 1H), 11.00 (s, 1H), 8.19 (d, J = 4.9 Hz, 1H), 8.09 (s, 1H), 7.57 – 7.17 (m, 5H), 6.84 (d, J = 8.6 Hz, 1H), 5.14 – 5.03 (m, 2H), 4.67 (s, 2H), 4.40 (d, J = 17.1 Hz, 1H), 4.24 (d, J = 17.2 Hz, 2H), 3.93 (s, 4H), 3.59 (dd, J = 16.0, 9.4 Hz, 4H), 3.47 (q, J = 6.6 Hz, 3H), 3.18 – 3.07 (m, 3H), 3.01 (dd, J = 16.4, 9.1 Hz, 2H), 2.95 – 2.86 (m, 2H), 2.82 – 2.71 (m, 3H), 2.66 – 2.58 (m, 2H), 2.06 – 1.94 (m, 3H), 1.75 (d, J = 55.3 Hz, 2H), 1.41 (d, J = 4.2 Hz, 1H), 1.24 (s, 1H), 1.10 (dd, J = 6.8, 2.8 Hz, 3H). | 884. 42 | General Synthesis Formula 63 |
| **291** | H58 | 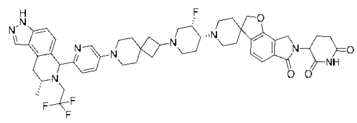 3-(1'-((3S,4R)-3-fluoro-1-(7-(6-((8R)-8-methyl-7-(2,2,2-trifluoroethyl)-6,7,8,9-tetrahydro-3H-pyrazolo[4,3-f]isoquinolin-6-yl)pyridin-3-yl)-7-azaspiro[3.5]nonan-2-yl)piperidin-4-yl)-6-oxo-6,8-dihydro-2H,7H-spiro[furo[2,3-e]isoindole-3,4'-piperidine]-7-yl)piperidine-2,6-dione | $^1$H NMR (600 MHz, DMSO- $d_6$) δ 13.05 (s, 1H), 11.00 (s, 1H), 8.12 (d, J = 29.3 Hz, 2H), 7.50 (s, 1H), 7.41 – 7.17 (m, 4H), 6.82 (d, J = 8.7 Hz, 1H), 5.11 (dd, J = 13.4, 5.1 Hz, 2H), 4.66 (s, 2H), 4.40 (d, J = 17.2 Hz, 1H), 4.24 (d, J = 17.1 Hz, 1H), 3.89 – 3.68 (m, 5H), 3.66 – 3.50 (m, 5H), 3.47 (td, J = 7.0, 4.6 Hz, 2H), 3.27 – 3.09 (m, 7H), 3.07 – 2.98 (m, 1H), 2.97 – 2.86 (m, 3H), 2.62 (d, J = 1.9 Hz, 1H), 2.48 – 2.39 (m, 1H), 2.17 (s, 5H), 2.09 – 1.84 (m, 4H), 1.74 – 1.58 (m, 4H), 1.10 (dd, J = 6.7, 2.0 Hz, 3H) | 924. 45 | General Synthesis Formula 62 |
| **292** | H59 | 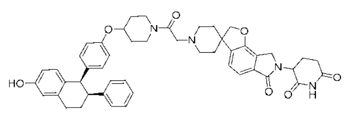 3-(1'-(2-(4-(((1R,2S)-6-hydroxy-2-phenyl-1,2,3,4-tetrahydronaphthalen-1-yl)phenoxy)piperidin-1-yl)-2-oxoethyl)-6-oxo-6,8-dihydro-2H,7H-spiro[furo[2,3-e]isoindole-3,4'-piperidine]-7-yl)piperidine-2,6-dione | $^1$H NMR (600 MHz, DMSO- $d_6$) δ 11.00 (s, 1H), 9.16 (s, 1H), 7.37 (d, J = 7.6 Hz, 1H), 7.31 (d, J = 7.7 Hz, 1H), 7.20 – 7.10 (m, 3H), 6.87 – 6.79 (m, 2H), 6.67 (d, J = 8.4 Hz, 1H), 6.64 – 6.58 (m, 3H), 6.50 (d, J = 8.4 Hz, 1H), 6.33 – 6.25 (m, 2H), 5.10 (dd, J = 13.3, 5.1 Hz, 1H), 4.65 (d, J = 4.8 Hz, 1H), 4.54 – 4.47 (m, 1H), 4.43 – 4.29 (m, 3H), 4.28 – 4.16 (m, 2H), 3.86 (t, J = 9.1 Hz, 1H), 3.65 (s, 2H), 3.40 – 3.23 (m, 5H), 3.14 (q, J = 11.2 Hz, 2H), 3.05 – 2.87 (m, 3H), 2.64 – 2.56 (m, 1H), 2.47 – 2.38 | 795. 37 | General Synthesis Formula 80 |

| | | | | | |
|---|---|---|---|---|---|
| | | | (m, 1H), 2.30 (td, J = 14.3, 4.3 Hz, 2H), 2.09 (qd, J = 12.6, 6.0 Hz, 1H), 1.96 (d, J = 14.4 Hz, 5H), 1.72 (dd, J = 12.5, 6.0 Hz, 1H), 1.66 – 1.56 (m, 1H), 1.55 – 1.45 (m, 1H). | | |
| **293** | H60 | 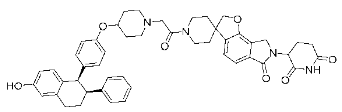 3-(1'-(2-(4-(((1R,2S)-6-hydroxy-2-phenyl-1,2,3,4-tetrahydronaphthalen-1-yl)phenoxy)piperidin-1-yl)acetyl)-6-oxo-6,8-dihydro-2H,7H-spiro[furo[2,3-e]isoindole-3,4'-piperidine]-7-yl)piperidine-2,6-dione | $^1$H NMR (600 MHz, DMSO- $d_6$) δ 11.00 (s, 1H), 9.17 (s, 1H), 7.37 (d, J = 7.6 Hz, 1H), 7.31 (dd, J = 7.6, 1.8 Hz, 1H), 7.14 (ddp, J = 10.5, 6.7, 3.7 Hz, 3H), 6.87 – 6.78 (m, 2H), 6.64 (ddd, J = 17.9, 8.4, 3.7 Hz, 4H), 6.50 (dd, J = 8.3, 2.6 Hz, 1H), 6.30 (dd, J = 8.5, 6.7 Hz, 2H), 5.10 (dt, J = 13.2, 5.1 Hz, 1H), 4.74 – 4.56 (m, 3H), 4.46 – 4.32 (m, 4H), 4.26 – 4.18 (m, 2H), 3.54 (s, 2H), 3.39 – 3.29 (m, 3H), 3.21 (q, J = 16.6, 15.0 Hz, 2H), 3.08 (s, 1H), 3.01 – 2.85 (m, 4H), 2.63 – 2.57 (m, 1H), 2.46 – 2.38 (m, 1H), 2.18 (d, J = 11.0 Hz, 1H), 2.07 (dp, J = 12.4, 6.7 Hz, 2H), 2.01 – 1.94 (m, 2H), 1.86 – 1.75 (m, 4H), 1.75 – 1.67 (m, 1H) | 795.37 | General Synthesis Formula 81 |
| **294** | H61 | 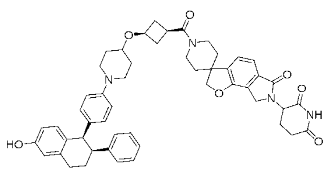 3-(1'-((1S,3s)-3-((1-(4-(((1R,2S)-6-hydroxy-2-phenyl-1,2,3,4-tetrahydronaphthalen-1-yl)phenyl)piperidin-4-yl)oxy)cyclobutane-1-carbonyl)-6-oxo-6,8-dihydro-2H,7H-spiro[furo[2,3-e]isoindole-3,4'-piperidine]-7-yl)piperidine-2,6-dione | $^1$H NMR (600 MHz, DMSO- $d_6$) δ 11.00 (d, J = 2.0 Hz, 1H), 9.20 (s, 1H), 7.42 (d, J = 7.5 Hz, 1H), 7.27 (d, J = 7.6 Hz, 1H), 7.23 – 7.06 (m, 4H), 6.99 (d, J = 33.6 Hz, 1H), 6.86 – 6.79 (m, 2H), 6.70 – 6.58 (m, 2H), 6.50 (dd, J = 8.3, 2.6 Hz, 1H), 6.40 (s, 2H), 5.10 (dt, J = 13.4, 5.3 Hz, 1H), 4.64 (h, J = 8.8 Hz, 3H), 4.37 (ddd, J = 18.0, 15.2, 4.7 Hz, 3H), 4.22 (dd, J = 17.2, 9.4 Hz, 2H), 4.00 (p, J = 7.5 Hz, 1H), 3.77 (d, J = 13.5 Hz, 1H), 3.55 (s, 1H), 3.44 (d, J = 12.4 Hz, 2H), 3.40 – 3.31 (m, 1H), 3.14 – 3.06 (m, 1H), 2.99 – 2.87 (m, 4H), 2.77 – 2.68 (m, 1H), 2.63 – 2.55 (m, 1H), 2.46 – 2.37 (m, 3H), 2.16 – 1.88 (m, 6H), 1.79 – 1.61 (m, 6H). | 835.04 | General Synthesis Formula 43 |
| **295** | H62 | 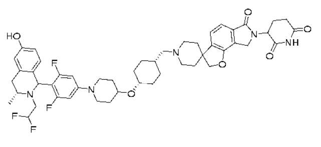 3-(1'-(((1S,4s)-4-((1-(4-((3R)-2-(2,2- | $^1$H NMR (600 MHz, DMSO- $d_6$) δ 11.00 (s, 1H), 9.05 (s, 1H), 7.36 (d, J = 7.6 Hz, 1H), 7.28 (d, J = 7.6 Hz, 1H), 6.57 – 6.51 (m, 3H), 6.49 (d, J = 2.4 Hz, 1H), 6.46 (dd, J = 8.2, 2.6 Hz, 1H), 5.75 (t, J = 55.9 | 888.29 | General Synthesis Formula 72 |

| | | | | | |
|---|---|---|---|---|---|
| | | difluoroethyl)-6-hydroxy-3-methyl-1,2,3,4-tetrahydroisoquinolin-1-yl)-3,5-difluorophenyl)piperidin-4-yl)oxy)cyclohexyl)methyl)-6-oxo-6,8-dihydro-2H,7H-spiro[furo[2,3-e]isoindole-3,4'-piperidine]-7-yl)piperidine-2,6-dione | Hz, 1H), 5.10 (dd, J = 13.3, 5.0 Hz, 1H), 4.98 (s, 1H), 4.68 – 4.63 (m, 2H), 4.40 (d, J = 17.2 Hz, 1H), 4.24 (d, J = 17.1 Hz, 1H), 3.60-3.48 (m, 6H), 3.38 – 3.31 (m, 1H), 3.12 – 2.96 (m, 8H), 2.96 – 2.88 (m, 1H), 2.63 – 2.58 (m, 2H), 2.46 – 2.38 (m, 1H), 2.23 (t, J = 13.5 Hz, 2H), 2.02 – 1.87 (m, 4H), 1.87 – 1.80 (m, 2H), 1.79 – 1.71 (m, 2H), 1.57 – 1.51 (m, 2H), 1.50-1.44 (m, 4H), 1.42 – 1.33 (m, 2H) , 0.97 (d, J = 6.4 Hz, 3H). | | |
| **296** | I1 | 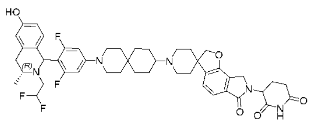 3-(1'-(3-(4-((3R)-2-(2,2-difluoroethyl)-6-hydroxy-3-methyl-1,2,3,4-tetrahydroisoquinolin-1-yl)-3,5-difluorophenyl)-3-azaspiro[5.5]undecan-9-yl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.99 (s, 1H), 9.17 (s, 1H), 7.36 (d, J = 7.6 Hz, 1H), 7.29 (d, J = 7.6 Hz, 1H), 6.56 – 6.51 (m, 3H), 6.49 (d, J = 2.4 Hz, 1H), 6.48 – 6.45 (m, 1H), 5.85 – 5.62 (m, 1H), 5.10 (dd, J = 13.3, 5.1 Hz, 1H), 4.98 (s, 1H), 4.71 – 4.65 (m, 2H), 4.40 (d, J = 17.2 Hz, 1H), 4.25 (d, J = 17.1 Hz, 1H), 3.60 – 3.55 (m, 2H), 3.36 (d, J = 5.9 Hz, 2H), 3.20 (q, J = 9.8, 6.1 Hz, 4H), 3.16 – 3.07 (m, 2H), 3.00 (td, J = 15.3, 14.7, 4.2 Hz, 2H), 2.92 (ddd, J = 18.1, 13.7, 5.3 Hz, 2H), 2.64 – 2.58 (m, 1H), 2.47 – 2.39 (m, 1H), 2.19 (td, J = 14.3, 4.1 Hz, 2H), 2.05-1.95 (m, 3H), 1.88 (dd, J = 33.9, 12.2 Hz, 4H), 1.65 (q, J = 12.6 Hz, 2H), 1.59 (t, J = 5.7 Hz, 2H), 1.41 (q, J = 5.7 Hz, 2H), 1.23-1.15 (m, 2H),0.98 (d, J = 6.5 Hz, 3H). | 844.55 | General Synthesis Formula 67 |
| **297** | I2 | 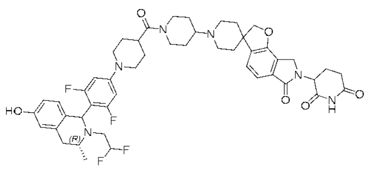 3-(1'-(1-(4-((1S,3R)-2-(2,2-difluoroethyl)-6-hydroxy-3-methyl-1,2,3,4-tetrahydroisoquinolin-1-yl)-3,5-difluorophenyl)piperidin-4-carbonyl)piperidin-4-yl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.99 (s, 1H), 9.18 (s, 1H), 7.36 (d, J = 7.5 Hz, 1H), 7.30 (d, J = 7.6 Hz, 1H), 6.56 – 6.52 (m, 3H), 6.49 – 6.45 (m, 2H), 5.74 (t, J = 56.3 Hz, 1H), 5.10 (dd, J = 13.3, 5.1 Hz, 1H), 4.98 (s, 1H), 4.68 (s, 2H), 4.57 (d, J = 13.1 Hz, 1H), 4.40 (d, J = 17.2 Hz, 1H), 4.24 (d, J = 17.2 Hz, 1H), 4.20 (d, J = 13.0 Hz, 1H), 3.82 – 3.74 (m, 2H), 3.56 (d, J = 16.4 Hz, 3H), 3.10 (qd, J = 7.3, 4.7 Hz, 4H), 3.03 – 2.98 (m, 2H), 2.95 – 2.87 (m, 3H), 2.82 (t, J = 12.5 Hz, 3H), 2.62 (dt, J = 3.7, 1.9 Hz, 1H), 2.59 (d, J = 3.6 Hz, | 887.56 | General Synthesis Formula 70 |

| | | | | | |
|---|---|---|---|---|---|
| | | | 1H), 2.45 – 2.38 (m, 1H), 2.24 – 2.06 (m, 3H), 2.06 – 1.96 (m, 4H), 1.68 (d, J = 12.5 Hz, 2H), 1.61 (s, 2H), 1.53 – 1.43 (m, 2H), 0.98 (d, J = 6.5 Hz, 3H). | | |
| **298** | I3 | 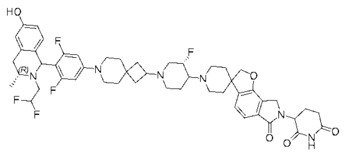 3-(1'-((3S)-1-(7-(4-((3R)-2-(2,2-difluoroethyl)-6-hydroxy-3-methyl-1,2,3,4-tetrahydroisoquinolin-1-yl)-3,5-difluorophenyl)-7-azaspiro[3.5]nonan-2-yl)-3-fluoropiperidin-4-yl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ10.99 (s, 1H), 9.18 (s, 1H), 7.40 – 7.27 (m, 2H), 6.55 (d, J = 13.5 Hz, 2H), 6.51 (d, J = 8.4 Hz, 2H), 6.49 (d, J = 2.4 Hz, 1H), 6.46 (dd, J = 8.3, 2.6 Hz, 1H), 5.75 – 5.53 (m, 1H), 5.05 (dd, J = 13.3, 5.1 Hz, 1H), 4.97 (s, 1H), 4.65 (q, J = 9.2, 7.6 Hz, 2H), 4.40 (d, J = 17.3 Hz, 1H), 4.25 (d, J = 17.2 Hz, 1H), 3.85 – 3.70 (m,2H), 3.36 – 3.30 (m, 1H), 3.23 – 3.07 (m, 6H), 2.93 – 2.83 (m, 2H), 2.62 (dd, J = 3.7, 2.1 Hz, 1H), 2.62 – 2.54 (m, 1H), 2.45 – 2.37 (m, 1H), 2.25 – 2.15 (m, 4H), 2.15 – 2.05 (m, 1H), 2.04 – 1.90 (m, 6H), 1.66 – 1.55 (m, 4H), 0.97 (d, J = 6.4 Hz, 2H). | 917.65 | General Synthesis Formula 68 |
| **299** | I4 | 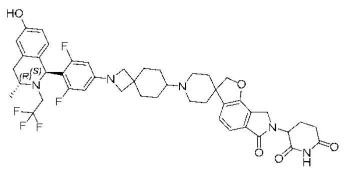 3-(1'-(2-(3,5-difluoro-4-((1S,3R)-6-hydroxy-3-methyl-2-(2,2,2-trifluoroethyl)-1,2,3,4-tetrahydroisoquinolin-1-yl)phenyl)-2-azaspiro[3.5]nonan-7-yl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.99 (s, 1H), 9.17 (s, 1H), 7.36 (d, J = 7.5 Hz, 1H), 7.29 (d, J = 7.6 Hz, 1H), 6.51 (d, J = 8.3 Hz, 1H), 6.49 (d, J = 2.4 Hz, 1H), 6.47 (d, J = 2.5 Hz, 1H), 6.45 (d, J = 2.6 Hz, 1H), 6.00 (d, J = 11.6 Hz, 2H), 5.10 (dd, J = 13.3, 5.1 Hz, 1H), 5.01 (s, 1H), 4.72 – 4.65 (m, 2H), 4.40 (d, J = 17.2 Hz, 1H), 4.24 (d, J = 17.1 Hz, 1H), 3.59 (s, 2H), 3.53 (s, 4H), 3.29-3.22 (m, 2H), 3.16-3.06 (dd, m, 1H), 2.96 – 2.88 (m, 1H), 2.88 – 2.81 (m, 1H), 2.62-2.58 (m, 1H), 2.43 - 2.38 (m, 1H), 2.18 (t, J = 13.7 Hz, 3H), 2.10-1.95 (m, 7H), 1.58 (t, J = 12.6 Hz, 2H), 1.50 (d, J = 12.9 Hz, 2H), 1.00 (d, J = 6.4 Hz, 3H). | 834.55 | General Synthesis Formula 67 |
| **300** | I5 | 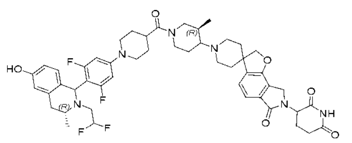 3-(1'-((3R)-1-(1-(4-((3R)-2-(2,2-difluoroethyl)-6-hydroxy-3-methyl- | $^1$H NMR (600 MHz, DMSO-$d_6$) δ 10.99 (s, 1H), 9.17 (s, 1H), 7.36 (d, J = 7.4 Hz, 1H), 7.28 (d, J = 7.6 Hz, 1H), 6.59 – 6.51 (m, 3H), 6.51 – 6.43 (m, 3H), 5.74 (t, J = 56.2 Hz, 1H), 5.10 (dd, J = 13.2, 5.3 Hz, 2H), 4.98 (s, 1H), 4.70 – 4.63 (m, 2H), | 901.55 | General Synthesis Formula 70 |

| | | | | | |
|---|---|---|---|---|---|
| | | 1,2,3,4-tetrahydroisoquinolin-1-yl)-3,5-difluorophenyl)piperidin-4-carbonyl)-3-methylpiperidin-4-yl)-6-oxo-6,8-dihydro-2H,7H-spiro[furo[2,3-e]isoindole-3,4'-piperidine]-7-yl)piperidine-2,6-dione | 4.63 – 4.57 (m, 1H), 4.47 – 4.41 (m, 1H), 4.39 (d, J = 6.7 Hz, 1H), 4.28 – 4.18 (m, 2H), 4.04 (d, J = 13.9 Hz, 1H), 3.79 (s, 3H), 3.62 (s, 1H), 3.20 – 3.06 (m, 2H), 3.06 – 2.77 (m, 8H), 2.65 – 2.57 (m, 1H), 2.47 – 2.40 (m, 1H), 2.27 – 2.10 (m, 3H), 2.07 – 1.95 (m, 3H), 1.76 – 1.56 (m, 6H), 1.05 - 0.85 (m, J 6H). | | |
| **301** | I6 | 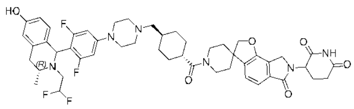 3-(1'-((1S,4r)-4-((4-(4-((3R)-2-(2,2-difluoroethyl)-6-hydroxy-3-methyl-1,2,3,4-tetrahydroisoquinolin-1-yl)-3,5-difluorophenyl)piperazin-1-yl)methyl)cyclohexane-1-carbonyl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.99 (s, 1H), 9.17 (s, 1H), 7.41 (d, J = 7.6 Hz, 1H), 7.28 (d, J = 7.5 Hz, 1H), 6.67 (d, J = 13.0 Hz, 2H), 6.52 – 6.48 (m, 2H), 6.46 (dd, J = 8.3, 2.5 Hz, 1H), 5.76 (tt, J = 56.2, 4.4 Hz, 1H), 5.12 – 5.06 (m,1H), 4.99 (s, 1H), 4.69 – 4.59 (m, 2H), 4.43 – 4.36 (m, 1H), 4.26 – 4.17 (m, 1H), 4.00 - 3.94 (m, 1H), 3.89 (d, J = 10.8 Hz, 2H), 3.58 (d, J = 10.3 Hz, 2H), 3.36 – 3.31 (m, 2H), 3.19 (t, J = 13.5 Hz, 2H), 3.14 – 3.05 (m, 4H), 3.04 – 2.96 (m, 4H), 2.91 (td, J = 17.6, 15.4, 5.3 Hz, 2H), 2.74 – 2.55 (m, 3H), 2.47 – 2.39 (m, 1H), 2.13 - 1.94 (m, 1H), 1.88 – 1.68 (m, 9H), 1.56 – 1.36 (m, 2H), 1.12 – 1.03 (m, 2H), 0.97 (d, J = 6.5 Hz, 3H). | 901.65 | General Synthesis Formula 24 |
| **302** | J1 | 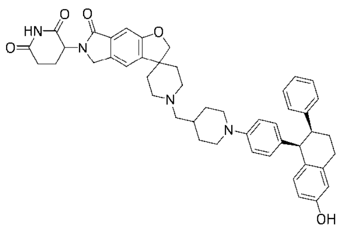 3-(1'-((1-(4-((1R,2S)-6-hydroxy-2-phenyl-1,2,3,4-tetrahydronaphthalen-1-yl)phenyl)piperidin-4-yl)methyl)-7-oxo-5,7-dihydro-2H,6H-spiro[furo[2,3-f]isoindole-3,4'-piperidine]-6-yl)piperidine-2,6-dione | $^1$H NMR (600 MHz, DMSO-$d_6$) δ 10.99 (d, *J* = 4.8 Hz, 1H), 9.14 (s, 1H), 7.31 (s, 1H), 7.18 – 7.10 (m, 3H), 7.08 (s, 1H), 6.84 (d, *J* = 7.2 Hz, 2H), 6.67 – 6.60 (m, 4H), 6.49 (dd, *J* = 8.3, 2.5 Hz, 1H), 6.25 (d, *J* = 8.1 Hz, 2H), 5.10 (dd, *J* = 13.3, 5.1 Hz, 1H), 4.63 – 4.56 (m, 2H), 4.42 – 4.35 (m, 1H), 4.27 (d, *J* = 16.9 Hz, 1H), 4.16 (d, *J* = 5.0 Hz, 1H), 3.57 (s, 3H), 3.33-3.25 (m, 2H), 3.10-3.03 (m, 3H), 2.99 – 2.88 (m, 3H), 2.66 – 2.57 (m, 2H), 2.42 – 2.32 (m, 1H), 2.24-2.15 (m, 2H), 2.10 (qd, *J* = 12.7, 6.3 Hz, 1H), 2.03 – 1.92 (m, 4H), 1.80 (d, *J* = 12.8 Hz, 2H), 1.72 (dd, *J* = 12.6, 6.2 Hz, 1H), 1.30 (q, *J* = 12.0 Hz, 2H). | 751.47 | General Synthesis Formula 1 |

| | | | | | |
|---|---|---|---|---|---|
| **303** | J2 | 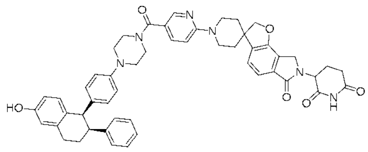 3-(1'-(5-(4-(4-((1R,2S)-6-hydroxy-2-phenyl-1,2,3,4-tetrahydronaphthalen-1-yl)phenyl)piperazin-1-carbonyl)pyridin-2-yl)-6-oxo-6,8-dihydro-2H,7H-spiro[furo[2,3-e]isoindole-3,4'-piperidine]-7-yl)piperidine-2,6-dione | $^{1}$H NMR (600 MHz, DMSO-$d_6$) δ 10.99 (s, 1H), 9.13 (s, 1H), 8.22 (d, J = 2.3 Hz, 1H), 7.66 (dd, J = 8.9, 2.4 Hz, 1H), 7.41 (d, J = 7.6 Hz, 1H), 7.27 (d, J = 7.5 Hz, 1H), 7.16 (t, J = 7.3 Hz, 2H), 7.12 (t, J = 7.1 Hz, 1H), 6.99 (d, J = 9.1 Hz, 1H), 6.85 (d, J = 7.4 Hz, 2H), 6.65 (d, J = 8.3 Hz, 1H), 6.60 (dd, J = 8.2, 5.4 Hz, 3H), 6.49 (dd, J = 8.4, 2.5 Hz, 1H), 6.25 (d, J = 8.3 Hz, 2H), 5.10 (dd, J = 13.3, 5.1 Hz, 1H), 4.73 – 4.66 (m, 2H), 4.40 (dd, J = 15.9, 9.5 Hz, 3H), 4.24 (d, J = 17.0 Hz, 1H), 4.16 (d, J = 5.0 Hz, 1H), 3.61 (s, 2H), 3.34 – 3.27 (m, 1H),3.13 – 3.03 (m, 6H), 3.03-2.87 (m, 4H), 2.63-2.58 (m, 1H), 2.47-2.40 (m, 1H), 2.15-2.05 (m, 1H), 2.04 – 1.90 (m, 3H), 1.81 (t, J = 13.8 Hz, 2H), 1.72 (dd, J = 12.4, 6.2 Hz, 1H). | 843.56 | General Synthesis Formula 74 |
| **304** | J3 | 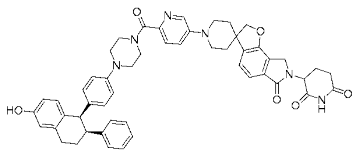 3-(1'-(6-(4-(4-((1R,2S)-6-hydroxy-2-phenyl-1,2,3,4-tetrahydronaphthalen-1-yl)phenyl)piperazin-1-carbonyl)pyridin-3-yl)-6-oxo-6,8-dihydro-2H,7H-spiro[furo[2,3-e]isoindole-3,4'-piperidine]-7-yl)piperidine-2,6-dione | $^{1}$H NMR (600 MHz, DMSO-$d_6$) δ 10.99 (s, 1H), 9.11 (s, 1H), 8.35 (d, J = 2.8 Hz, 1H), 7.57 (d, J = 8.7 Hz, 1H), 7.49 (dd, J = 8.9, 2.8 Hz, 1H), 7.43 (d, J = 7.6 Hz, 1H), 7.28 (d, J = 7.5 Hz, 1H), 7.18 – 7.09 (m, 3H), 6.84 (d, J = 7.4 Hz, 2H), 6.65 (d, J = 8.3 Hz, 1H), 6.60 (d, J = 8.3 Hz, 3H), 6.49 (dd, J = 8.3, 2.5 Hz, 1H), 6.25 (d, J = 8.3 Hz, 2H), 5.10 (dd, J = 13.3, 5.1 Hz, 1H), 4.70 – 4.63 (m, 2H), 4.41 (d, J = 17.1 Hz, 1H) – 4.24 (d, J = 17.0 Hz, 1H), 4.16 (d, J = 5.0 Hz, 1H), 3.97-3.90 (m, 2H), 3.74 (d, J = 16.5 Hz, 2H), 3.32 – 3.27 (m, 1H), 3.09 (s, 1H), 3.03 – 2.96 (m, 4H), 2.96-2.87 (M, 3H), 2.60 (d, J = 14.3 Hz, 1H), 2.47 – 2.40 (m, 1H), 2.13 – 1.96 (m, 4H), 1.83 (t, J = 14.1 Hz, 2H), 1.71 (dd, J = 12.4, 6.2 Hz, 1H). | 843.60 | General Synthesis Formula 74 |
| **305** | J4 | 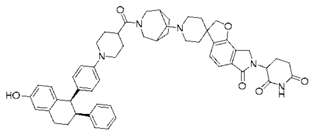 3-(1'-(3-(1-(4-((1R,2S)-6-hydroxy-2-phenyl-1,2,3,4-tetrahydronaphthalen-1- | $^{1}$H NMR (600 MHz, DMSO-$d_6$) δ 10.99 (s, 1H), 9.13 (s, 1H), 7.37 (d, *J* = 7.6 Hz, 1H), 7.29 (d, *J* = 7.6 Hz, 1H), 7.22 – 7.10 (m, 3H), 6.86 – 6.82 (m, 2H), 6.75 – 6.60 (m, 4H), 6.49 (dd, *J* = 8.3, 2.6 Hz, 1H), 6.28 (s, 2H), 5.10 (dd, *J* = 13.4, 5.3 Hz, | 874.76 | General Synthesis Formula 15 |

| | | | | | |
|---|---|---|---|---|---|
| | | yl)phenyl)piperidin-4-carbonyl)-3-azabicyclo[3.2.1]octan-8-yl)-6-oxo-6,8-dihydro-2H,7H-spiro[furo[2,3-e]isoindole-3,4'-piperidine]-7-yl)piperidine-2,6-dione | 1H), 4.67 (q, $J$ = 9.1, 7.4 Hz, 2H), 4.40 (d, $J$ = 17.1 Hz, 1H), 4.25 (dd, $J$ = 17.2, 2.6 Hz, 1H), 4.18 (s, 1H), 3.99 (d, $J$ = 13.6 Hz, 2H), 3.85-3.68 (m, 3H), 3.57 (s, 1H), 3.35 – 3.30 (m, 2H), 3.25-3.15 (m, 1H), 3.02 – 2.96 (m, 2H), 2.96-2.88 (m, 3H), 2.79 (s, 1H), 2.65 - 2.58 (m, 1H), 2.47-2.42 (m,1H), 2.37-2.35 (m, 2H), 2.15 – 2.05 (m, 1H), 2.03-1.93 (m,4H), 1.80 – 1.60 (m, 9H), 1.75 – 1.69 (m, 3H), 1.50-1.40 (m, 1H). | | |
| **306** | J5 | 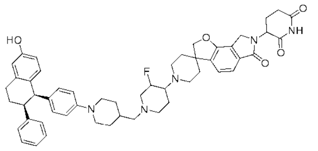 3-(1'-(3-fluoro-1-((1-(4-((1R,2S)-6-hydroxy-2-phenyl-1,2,3,4-tetrahydronaphthalen-1-yl)phenyl)piperidin-4-yl)methyl)piperidin-4-yl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.99 (s, 1H), 9.12 (s, 1H), 7.35 (d, $J$ = 7.4 Hz, 1H), 7.31 (s, 1H), 7.18 -7.10 (m, 3H), 6.84 (d, $J$ = 7.2 Hz, 2H), 6.67 – 6.55 (m, 4H), 6.49 (dd, $J$ = 8.3, 2.6 Hz, 1H), 6.25 (s, 2H), 5.10 (dd, $J$ = 13.3, 5.1 Hz, 2H), 4.65 (s, 2H), 4.40 (d, $J$ = 17.1 Hz, 1H), 4.24 (d, $J$ = 17.1 Hz, 1H), 4.16 (s, 1H), 3.65 – 3.50 (m, 4H), 3.00 – 2.88 (m, 5H), 2.62-2.57 (m, 1H), 2.47 - 2.40 (m, 1H), 2.25 - 2.15 (m, 2H), 2.15 – 2.05 (m, 1H), 2.04 – 1.90 (m, 6H), 1.80 – 1.70 (m, 4H). | 852. 60 | General Synthesis Formula 13 |
| **307** | J6 | 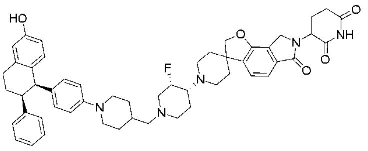 3-(1'-((3S,4R)-3-fluoro-1-((1-(4-((1R,2S)-6-hydroxy-2-phenyl-1,2,3,4-tetrahydronaphthalen-1-yl)phenyl)piperidin-4-yl)methyl)piperidin-4-yl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.99 (s, 1H), 9.14 (s, 1H), 7.36 (d, $J$ = 7.5 Hz, 1H), 7.31 (s, 1H), 7.20 - 7.10 (m, 3H), 6.87 – 6.82 (m, 2H), 6.67 – 6.60 (m, 4H), 6.49 (dd, $J$ = 8.3, 2.6 Hz, 1H), 6.27 (s, 2H), 5.53 (s, 1H), 5.10 (dd, $J$ = 13.3, 5.1 Hz, 1H), 4.67 (s, 2H), 4.40 (d, $J$ = 17.2 Hz, 1H), 4.24 (d, $J$ = 17.1 Hz, 1H), 4.17 (s, 1H), 3.34 – 3.28 (m, 3H), 3.13 (s, 1H), 3.03 – 2.88 (m, 5H), 2.65-2.55 (m, 1H), 2.47-2.40 (m, 1H), 2.22 (s, 3H), 2.15 - 2.05 (m, 1H), 2.05 – 1.86 (m, 6H), 1.85 – 1.70 (m, 4H),. | 852. 76 | General Synthesis Formula 13 |
| **308** | J7 | 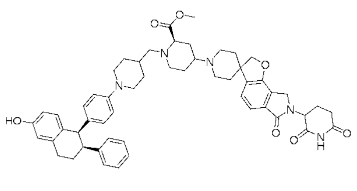 (2R)-4-(7-(2,6-dioxopiperidin-3-yl)-6- | $^1$H NMR (600 MHz, DMSO-$d_6$) δ 10.99 (s, 1H), 9.14 (s, 1H), 7.36 (d, $J$ = 7.6 Hz, 1H), 7.28 (d, $J$ = 7.6 Hz, 1H), 7.20 – 7.08 (m, 3H), 6.86 – 6.82 (m, 2H), 6.67 – 6.58 (m, 4H), 6.49 (dd, $J$ = 8.2, 2.6 Hz, 1H), 6.29 (s, 2H), 5.10 (dd, $J$ = 13.3, 5.1 Hz, | 892. 60 | General Synthesis Formula 9 |

| | | | | | |
|---|---|---|---|---|---|
| | | oxo-7,8-dihydro-2H,6H-spiro[furo[2,3-e]isoindole-3,4'-piperidine]-1'-yl)-1-((1-(4-((1R,2S)-6-hydroxy-2-phenyl-1,2,3,4-tetrahydronaphthalen-1-yl)phenyl)piperidin-4-yl)methyl)piperidine-2-carboxylate | 1H), 4.70 – 4.63 (m, 2H), 4.40 (d, *J* = 17.2 Hz, 1H), 4.24 (d, *J* = 17.2 Hz, 1H), 4.19 (s, 1H), 3.71 (s, 3H), 3.35 - 3.25 (m, 3H), 3.20 - 3.05 (m, 3H), 3.00 - 2.85 (m, 6H), 2.62 - 2.57 (m, 1H), 2.47 - 2.40 (m, 1H), 2.20 – 1.90 (m, 10H), 1.85-1.60 (m, 6H). | | |
| **309** | J8 | 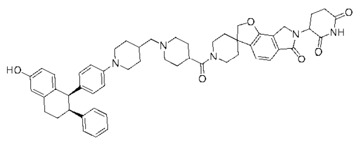 3-(1'-(1-((1-(4-((1R,2S)-6-hydroxy-2-phenyl-1,2,3,4-tetrahydronaphthalen-1-yl)phenyl)piperidin-4-yl)methyl)piperidin-4-carbonyl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.98 (d, *J* = 2.2 Hz, 1H), 8.91 (s, 1H), 7.41 (d, *J* = 7.6 Hz, 1H), 7.29 (d, *J* = 7.6 Hz, 1H), 7.18 – 7.10 (m, 3H), 6.86 – 6.81 (m, 2H), 6.67 (d, *J* = 7.8 Hz, 2H), 6.65 – 6.60 (m, 2H), 6.49 (dd, *J* = 8.3, 2.6 Hz, 1H), 6.28 (d, *J* = 8.2 Hz, 2H), 5.11 - 5.04 (m, 1H), 4.71 – 4.60 (m, 2H), 4.43 – 4.36 (m, 2H), 4.23 (dd, *J* = 17.1, 13.2 Hz, 1H), 4.17 (d, *J* = 5.1 Hz, 1H), 4.00 (d, *J* = 13.7 Hz, 1H), 3.65 – 3.52 (m, 2H), 3.34 – 3.30 (m, 1H), 3.25 – 3.05 (m, 3H), 3.03 - 2.85 (m, 8H), 2.78 - 2.64 (m, 2H), 2.63 - 2.57 (m, 1H), 2.47 – 2.40 (m, 1H), 2.14 – 2.04 (m, 1H), 2.00 – 1.70 (m, 13H), 1.35 – 1.27 (m, 2H). | 862.60 | General Synthesis Formula 32 |
| **310** | J9 | 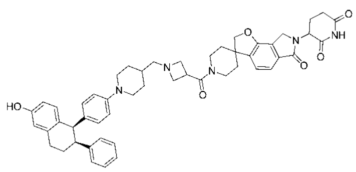 3-(1'-(1-((1-(4-((1R,2S)-6-hydroxy-2-phenyl-1,2,3,4-tetrahydronaphthalen-1-yl)phenyl)piperidin-4-yl)methyl)azetidin-3-carbonyl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.98 (d, *J* = 2.4 Hz, 1H), 9.13 (s, 1H), 7.40 (dd, *J* = 7.6, 5.8 Hz, 1H), 7.31 (dd, *J* = 7.6, 3.0 Hz, 1H), 7.14 (dq, *J* = 14.0, 6.9 Hz, 3H), 6.86 – 6.82 (m, 2H), 6.70 – 6.59 (m, 4H), 6.49 (dd, *J* = 8.3, 2.5 Hz, 1H), 6.26 (d, *J* = 8.1 Hz, 2H), 5.13 – 5.06 (m, 1H), 4.69 – 4.62 (m, 2H), 4.45 – 4.30 (m, 4H), 4.23 (dd, *J* = 17.1, 11.4 Hz, 2H), 4.18-4.15 (m, 2H), 3.92 (q, *J* = 8.9 Hz, 1H), 3.34 – 3.28 (m, 2H), 3.22 – 3.09 (m, 4H), 2.99 (dd, *J* = 16.9, 6.0 Hz, 1H), 2.96 – 2.88 (m, 2H), 2.86 – 2.80 (m, 1H), 2.63 – 2.57 (m, 1H), 2.45 – 2.41 (m, 1H), 2.12 – 2.06 (m, 1H), 2.02 - 1.95 (m, 1H), 1.90 – 1.65 (m, 8H), 1.34 – 1.26 (m, 2H). | 834.55 | |
| **311** | J10 | 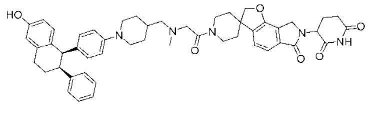 3-(1'-(N-((1-(4-((1R,2S)-6-hydroxy-2- | $^1$H NMR (600 MHz, DMSO-$d_6$) δ 10.99 (d, *J* = 1.9 Hz, 1H), 9.13 (s, 1H), 7.40 – 7.27 (m, 2H), 7.18-7.10 (m, 3H), 6.84 (d, *J* = 7.2 Hz, 2H), 6.67 – 6.60 (m, 4H), 6.49 | 822.66 | General Synthesis Formula 75 |

| | | | | | |
|---|---|---|---|---|---|
| | | phenyl-1,2,3,4-tetrahydronaphthalen-1-yl)phenyl)piperidin-4-yl)methyl)-N-methylglycyl)-6-oxo-6,8-dihydro-2H,7H-spiro[furo[2,3-e]isoindole-3,4'-piperidine]-7-yl)piperidine-2,6-dione | (dd, *J* = 8.3, 2.5 Hz, 1H), 6.26 (d, *J* = 8.1 Hz, 2H), 5.09 (dt, *J* = 13.2, 5.6 Hz, 1H), 4.75 – 4.61 (m, 2H), 4.43-4.34 (m, 3H), 4.24 (dd, *J* = 17.0, 12.7 Hz, 1H), 4.16 (d, *J* = 5.0 Hz, 1H), 3.68 – 3.62 (m, 1H), 3.60 – 3.50 (m, 2H), 3.25 – 3.20 (m, 2H), 3.10 – 3.05 (m, 2H), 3.02 – 2.84 (m, 7H), 2.67 – 2.57 (m, 2H), 2.47 – 2.40 (m, *1*H), 2.15 – 2.05 (m, 1H), 2.03 – 1.87 (m, 4H), 1.85 – 1.68 (m, 5H), 1.35 – 1.27 (m, 2H). | | |
| **312** | J11 | 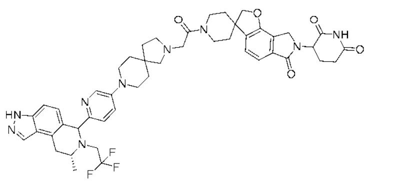 3-(1'-(2-(8-(6-((8R)-8-methyl-7-(2,2,2-trifluoroethyl)-6,7,8,9-tetrahydro-3H-pyrazolo[4,3-f]isoquinolin-6-yl)pyridin-3-yl)-2,8-diazaspiro[4.5]decane-2-yl)acetyl)-6-oxo-6,8-dihydro-2H,7H-spiro[furo[2,3-e]isoindole-3,4'-piperidine]-7-yl)piperidine-2,6-dione | $^1$H NMR (400 MHz, DMSO-*d*$_6$) δ 13.05 (s, 1H), 10.99 (s, 1H), 10.12 (s, 1H), 8.16 (t, *J* = 3.2 Hz, 1H), 8.10 (s, 1H), 7.52 (s, 1H), 7.40 – 7.25 (m, 4H), 6.82 (d, *J* = 8.6 Hz, 1H), 5.12 – 5.05 (m, *2*H), 4.75 – 4.60 (m, 2H), 4.53 – 4.45 (m, 1H), 4.45 – 4.32 (m, 3H), 4.24 (dd, *J* = 17.2, 7.3 Hz, 2H), 3.65 – 3.55 (m, 3H), 3.30 – 3.10 (m, *6*H), 3.05 – 2.85 (m, *5*H), 2.65 – 2.56 (m, 1H), 2.47 – 2.40 (d, *J* = 13.0 Hz, 1H), 2.05 – 1.65 (m, 11H), 1.10 (d, *J* = 6.5 Hz, 3H). | 880.66 | General Synthesis Formula 59 |
| **313** | J12 | 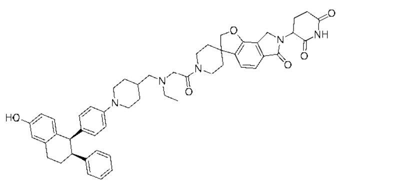 3-(1'-(N-ethyl-N-((1-(4-((1R,2S)-6-hydroxy-2-phenyl-1,2,3,4-tetrahydronaphthalen-1-yl)phenyl)piperidin-4-yl)methyl)glycyl)-6-oxo-6,8-dihydro-2H,7H-spiro[furo[2,3-e]isoindole-3,4'-piperidine]-7-yl)piperidine-2,6-dione | $^1$H NMR (400 MHz, DMSO-*d*$_6$) δ 10.99 (s, 1H), 9.12 (s, 1H), 7.37 – 7.25 (m, 2H), 7.19 – 7.10 (m, 3H), 6.87 – 6.81 (m, 2H), 6.68 – 6.58 (m, 4H), 6.49 (d, *J* = 8.3 Hz, 1H), 6.27 – 6.20 (m, 2H), 5.09 (dd, *J* = 11.5, 6.9 Hz, 2H), 4.75 – 4.60 (m, *2*H), 4.45 – 4.29 (m, 4H), 4.23 (dd, *J* = 17.3, 8.3 Hz, 1H), 4.15 (s, 1H), 3.75(d, 2H), 3.60 - 3.50 (m, 2H), 3.30 - 3.20 (m, 3H) , 3.05 - 2.85 (m, *5*H), 2.65 – 2.55 (m, 1H), 2.47 – 2.40 (m, 1H), 2.15 – 1.65 (m, 12H), 1.31 – 1.22 (m, 3H). | 836.66 | General Synthesis Formula 75 |
| **314** | J13 | 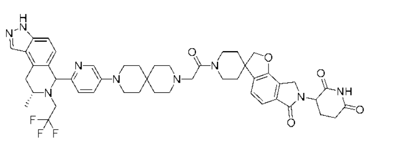 3-(1'-(2-(9-(6-((8R)-8-methyl-7-(2,2,2-trifluoroethyl)-6,7,8,9-tetrahydro-3H-pyrazolo[4,3-f]isoquinolin-6- | $^1$H NMR (600 MHz, DMSO-*d*$_6$) δ 13.02 (s, 1H), 10.99 (s, 1H), 8.15 (d, *J* = 2.9 Hz, 1H), 8.11 (s, 1H), 7.56 (s, 1H), 7.37 (d, *J* = 7.7 Hz, 1H), 7.35 – 7.25 (m, 3H), 6.82 (d, *J* = 8.6 Hz, 1H), 5.15 – 5.07 (m, 2H), 4.74 – 4.63 (m, 3H), 4.44 – 4.36 (m, 3H), 4.32 (dd, *J* = 16.0,11.7 Hz, 1H), 4.24 (dd, | 894.56 | General Synthesis Formula 59 |

| | | | | | |
|---|---|---|---|---|---|
| | | yl)pyridin-3-yl)-3,9-diazaspiro[5.5]undecane-3-yl)acetyl)-6-oxo-6,8-dihydro-2H,7H-spiro[furo[2,3-e]isoindole-3,4'-piperidine]-7-yl)piperidine-2,6-dione | $J$ = 17.1,4.9 Hz, 1H), 3.67 (d, $J$ = 14.0 Hz, 2H), 3.37 (s, 2H), 3.26 (s, 4H), 3.20 – 3.10 (m, 4H), 3.07 – 3.00 (m, 2H), 2.95 – 2.88 (m, 3H), 2.63 – 2.57 (m, 1H), 2.47 – 2.40 (m, 1H), 2.01 – 1.96 (m, 1H), 1.94 – 1.75 (m, 6H), 1.75 – 1.65 (m, 4H), 1.55 - 1.50 (m, 2H), 1.10 (d, $J$ = 6.6 Hz, 3H). | | |
| **315** | J14 | 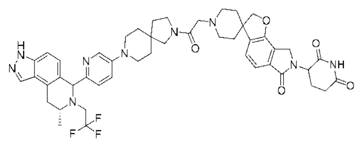 3-(1'-(2-(8-(6-((8R)-8-methyl-7-(2,2,2-trifluoroethyl)-6,7,8,9-tetrahydro-3H-pyrazolo[4,3-f]isoquinolin-6-yl)pyridin-3-yl)-2,8-diazaspiro[4.5]decane-2-yl)-2-oxoethyl)-6-oxo-6,8-dihydro-2H,7H-spiro[furo[2,3-e]isoindole-3,4'-piperidine]-7-yl)piperidine-2,6-dione | $^1$H NMR (400 MHz, DMSO-$d_6$) δ 13.02 (s, 1H), 11.00 (s, 1H), 8.20 – 8.15 (m, 1H), 8.10 (s, 1H), 7.53 (s, 1H), 7.37 (d, $J$ = 7.6 Hz, 1H), 7.35 - 7.25 (m, 3H), 6.82 (dd, $J$ = 8.7, 4.5 Hz, 1H), 5.15 – 5.07 (m, 2H), 4.65 (s, 2H), 4.40 (d, $J$ = 17.2 Hz, 1H), 4.24 (d, 1H), 4.19 (s,1H), 3.39 (s, 1H), 3.33 (s, 2H), 3.26 – 3.16 (m, 6H), 3.07 – 2.85 (m, 5H), 2.65 – 2.55 (m, 1H), 2.58 (s, 1H), 2.47 – 2.40 (m, 1H), 2.30 – 2.23 (m, 2H), 2.02 – 1.86 (m, 4H),1.85 – 1.77 (m, 1H), 1.70 1.60 (m, 4H), 1.10 (d, $J$ = 6.5 Hz, 3H). | 880.60 | General Synthesis Formula 60 |
| **316** | J15 | 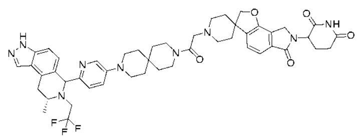 3-(1'-(2-(9-(6-((8R)-8-methyl-7-(2,2,2-trifluoroethyl)-6,7,8,9-tetrahydro-3H-pyrazolo[4,3-f]isoquinolin-6-yl)pyridin-3-yl)-3,9-diazaspiro[5.5]undecane-3-yl)-2-oxoethyl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 13.02 (s, 1H), 11.01 (s, 1H), 8.14 (d, $J$ = 3.0 Hz, 1H), 8.11 (s, 1H), 7.53 (s, 1H), 7.37 (d, $J$ = 7.6 Hz, 2H), 7.35 - 7.25 (m, 3H), 6.82 (d, $J$ = 8.7 Hz, 1H), 5.15 – 5.08 (m, 2H), 4.68 – 4.63 (m, 2H), 4.52 (d, $J$ = 4.7 Hz, 1H), 4.40 (d, $J$ = 17.2, 1H), 4.33 (d, $J$ = 4.8 Hz, 2H), 4.24 (d, $J$ = 17.2 Hz, 1H), 3.50 – 3.40 (m, 2H), 3.34 (s, 2H), 3.27 (s, 5H), 3.20 – 3.10 (m, 4H), 3.08 – 2.97 (m, 3H), 2.95 – 2.87 (m, 3H), 2.65 – 2.57 (m, 1H), 2.47 – 2.40 (m, 1H), 2.34 – 2.27 (m, 2H), 2.04 – 1.91 (m, 3H), 1.66 – 1.56 (m, 4H), 1.56 – 1.52 (m, 2H), 1.50 - 1.43 (m, 2H), 1.10 (d, $J$ = 6.6 Hz, 3H). | 894.66 | General Synthesis Formula 60 |
| **317** | J16 | 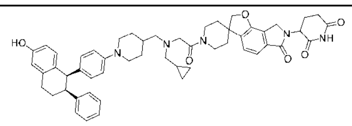 3-(1'-(N-(cyclopropylmethyl)-N-((1-(4-((1R,2S)-6-hydroxy-2-phenyl-1,2,3,4-tetrahydronaphthalen-1-yl)phenyl)piperidin-4- | $^1$H NMR (400 MHz, DMSO-$d_6$) δ 11.00 (s, 1H), 9.02 (s, 1H), 7.39 – 7.25 (m, 2H), 7.20 – 7.08 (m, 3H), 6.84 (d, $J$ = 7.0 Hz, 2H), 6.71 – 6.55 (m, 4H), 6.48 (dd, $J$ = 8.3, 2.5 Hz, 1H), 6.25 (d, $J$ = 8.0 Hz, 2H), 5.10 (dt, $J$ = 13.3, 4.6 Hz, 1H), 4.76 – 4.61 (m, 2H), 4.50 – 4.33 (m, 3H), 4.23 (dd, $J$ | 862.65 | General Synthesis Formula 75 |

| | | | | | |
|---|---|---|---|---|---|
| | | yl)methyl)glycyl)-6-oxo-6,8-dihydro-2H,7H-spiro[furo[2,3-e]isoindole-3,4'-piperidine]-7-yl)piperidine-2,6-dione | = 17.2, 7.8 Hz, 1H), 4.16 (d, *J* = 4.9 Hz, 1H), 3.36 – 3.20 (m, 3H), 3.19 – 3.04 (m, 3H), 3.03 – 2.85 (m, 4H), 2.65 - 2.57 (m, *1*H), 2.47 – 2.40 (m, 1H), 2.14 – 2.06 (m, 1H), 2.02 – 1.86 (m, 2H), 1.85 - 1.65 (m, 5H), 1.40 -1.10 (m, 5H), 0.65 (s, 2H), 0.49 – 0.37 (m, 2H). | | |
| **318** | K1 | 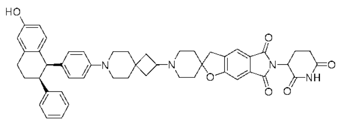 6-(2,6-dioxopiperidin-3-yl)-1'-(7-(4-((1R,2S)-6-hydroxy-2-phenyl-1,2,3,4-tetrahydronaphthalen-1-yl)phenyl)-7-azaspiro[3.5]nonane-2-yl)spiro[furo[2,3-f]isoindole-2,4'-piperidine]-5,7(3H,6H)-dione | $^1$H NMR (600 MHz, DMSO-$d_6$) δ 11.00 (s, 1H), 9.93 (s, 1H), 7.86 (dd, J = 9.0, 2.4 Hz, 4H), 7.39 (d, J = 2.2 Hz, 2H), 7.29 (d, J = 7.7 Hz, 2H), 7.25 (d, J = 7.6 Hz, 2H), 7.14 (dd, J = 8.8, 2.2 Hz, 2H), 6.90 (d, J = 9.3 Hz, 2H), 5.15 (dd, J = 13.3, 5.1 Hz, 2H), 4.43 (d, J = 17.4 Hz, 2H), 2.67-2.63 (m, 6H), 2.40-2.32 (m, 1H), 2.14-2.08 (m, 5H), 2.07-2.04 (m, 4H), 2.01 (s, 2H), 1.93-1.88 (m, 8H), 1.87-1.81 (m, 4H). | 790. 37 | General Synthesis Formula 3 |
| **319** | K2 | 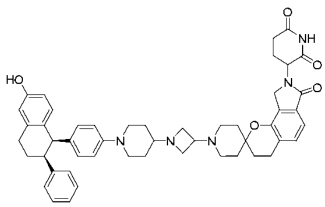 3-(1-(1-(1-(4-((1R,2S)-6-hydroxy-2-phenyl-1,2,3,4-tetrahydronaphthalen-1-yl)phenyl)piperidin-4-yl)azetidin-3-yl)-7'-oxo-3',4',7',9'-tetrahydro-8'H-spiro[piperidine-4,2'-pyrano[2,3-e]isoindole]-8'-yl)piperidine-2,6-dione | $^1$H NMR (600 MHz, DMSO-$d_6$) δ 11.02 (s, 1H), 7.28 (d, J = 7.7 Hz, 1H), 7.23 (d, J = 7.6 Hz, 1H), 7.14 (dq, J = 14.2, 7.1 Hz, 3H), 6.84 (d, J = 7.4 Hz, 2H), 6.68 – 6.56 (m, 4H), 6.49 (d, J = 8.2 Hz, 1H), 6.23 (d, J = 8.4 Hz, 2H), 5.13 (dd, J = 13.2, 5.1 Hz, 1H), 4.48 – 4.29 (m, 6H), 4.19 (dd, J = 50.2, 11.0 Hz, 7H), 3.67 (d, J = 9.9 Hz, 3H), 3.29 (d, J = 15.1 Hz, 2H), 3.01 – 2.85 (m, 6H), 2.40 – 2.32 (m, 1H), 2.12-2.00 (m, 2H), 1.97 – 1.65 (m, 9H), 1.42-1.36 (m, 2H). | 805. 42 | General Synthesis Formula 23 |
| **320** | K3 | 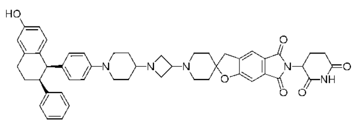 6-(2,6-dioxopiperidin-3-yl)-1'-(1-(1-(4-((1R,2S)-6-hydroxy-2-phenyl-1,2,3,4-tetrahydronaphthalen-1-yl)phenyl)piperidin-4-yl)azetidin-3-yl)spiro[furo[2,3-f]isoindole-2,4'-piperidine]-5,7(3H,6H)-dione | $^1$H NMR (600 MHz, DMSO-$d_6$) δ 11.12 (s, 1H), 7.80 (s, 1H), 7.22 (s, 1H), 7.19 – 7.10 (m, 3H), 6.84 (d, J = 7.2 Hz, 2H), 6.62 (dd, J = 15.1, 8.3 Hz, 4H), 6.49 (dd, J = 8.3, 2.1 Hz, 1H), 6.24 (d, J = 8.5 Hz, 2H), 5.10 (dd, J = 12.9, 5.4 Hz, 1H), 4.35 (s, 4H), 4.15 (d, J = 4.8 Hz, 1H), 3.72 – 3.59 (m, 2H), 3.37 – 3.24 (m, 4H), 3.05 – 2.84 (m, 6H), 2.65 – 2.51 (m, 6H), 2.20-1.98 (m, 6H), 1.96-1.92 (m, 2H), 1.75-1.68 (m, 1H), 1.47-1.33 (m, 2H). | 805. 38 | General Synthesis Formula 23 |

| | | | | | |
|---|---|---|---|---|---|
| **321** | K4 | 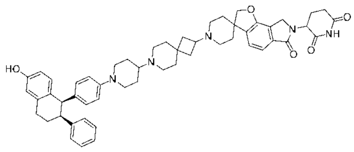 3-(1'-((1R,2S)-6-hydroxy-2-phenyl-1,2,3,4-tetrahydronaphthalen-1-yl)phenyl)piperidin-4-yl)-7-azaspiro[3.5]nonane-2-yl)-6-oxo-6,8-dihydro-2H,7H-spiro[furo[2,3-e]isoindole-3,4'-piperidine]-7-yl)piperidine-2,6-dione | $^1$H NMR (400 MHz, DMSO-$d_6$) δ 11.00 (s, 1H), 9.32 (s, 1H), 7.33 (dd, J = 25.0, 7.6 Hz, 2H), 7.20 – 7.08 (m, 3H), 6.84 (d, J = 6.7 Hz, 2H), 6.67 – 6.54 (m, 4H), 6.48 (dd, J = 8.3, 2.2 Hz, 1H), 6.23 (d, J = 8.5 Hz, 2H), 5.10 (dd, J = 13.2, 5.0 Hz, 1H), 4.66 (s, 1H), 4.40 (d, J = 17.3 Hz, 1H), 4.24 (d, J = 17.2 Hz, 1H), 4.15 (d, J = 4.5 Hz, 1H), 3.62 (s, 13H), 3.30 (d, J = 10.2 Hz, 3H), 3.03 – 2.83 (m, 6H), 2.61 – 2.53 (m, 2H), 2.23 – 2.10 (m, 4H), 2.02 (d, J = 24.7 Hz, 6H), 1.85 – 1.57 (m, 6H). | 860.70 | General Synthesis Formula 37 |
| **322** | K5 | 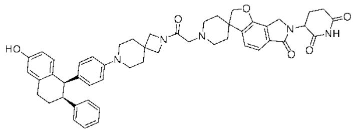 3-(1R,2S)-6-hydroxy-2-phenyl-1,2,3,4-tetrahydronaphthalen-1-yl)phenyl)-2,7-diazaspiro[3.5]nonane-2-yl)-2-oxoethyl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.99 (s, 1H), 7.39 – 7.21 (m, 2H), 7.19 – 7.09 (m, 3H), 6.84 (d, J = 7.1 Hz, 2H), 6.63 (dd, J = 16.1, 5.0 Hz, 4H), 6.49 (dd, J = 8.2, 2.1 Hz, 1H), 6.25 (d, J = 8.1 Hz, 2H), 5.10 (dd, J = 13.2, 5.0 Hz, 1H), 4.64 (d, J = 3.8 Hz, 1H), 4.40 (d, J = 17.2 Hz, 1H), 4.24 (d, J = 17.2 Hz, 1H), 4.16 (d, J = 4.5 Hz, 1H), 4.01 (s, 1H), 3.90 (s, 1H), 3.72 (s, 2H), 3.48 – 3.37 (m, 5H), 3.31 (s, 2H), 3.17 (d, J = 6.3 Hz, 2H), 3.07 – 2.86 (m, 7H), 2.60 (d, J = 17.5 Hz, 1H), 2.46 – 2.37 (m, 1H), 2.28 (t, J = 12.2 Hz, 1H), 2.15 – 2.05 (m, 1H), 2.04 – 1.88 (m, 3H), 1.80 (s, 4H), 1.72 (d, J = 6.1 Hz, 1H). | 820.60 | General Synthesis Formula 11 |
| **323** | K6 | 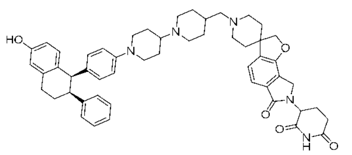 3-(1,4-(1R,2S)-6-hydroxy-2-phenyl-1,2,3,4-tetrahydronaphthalen-1-yl)phenyl)-[1,4'-bipiperidin]-4-yl)methyl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 11.00 (s, 1H), 9.49 (s, 1H), 7.36 (d, J = 7.6 Hz, 1H), 7.28 (d, J = 7.6 Hz, 1H), 7.14 (dq, J = 14.1, 6.9 Hz, 3H), 6.84 (d, J = 7.1 Hz, 2H), 6.61 (dt, J = 24.2, 8.2 Hz, 4H), 6.49 (dd, J = 8.3, 2.0 Hz, 1H), 6.24 (d, J = 8.5 Hz, 2H), 5.10 (dd, J = 13.2, 5.0 Hz, 1H), 4.67 (d, J = 5.9 Hz, 1H), 4.40 (d, J = 17.2 Hz, 1H), 4.24 (d, J = 17.1 Hz, 1H), 4.15 (d, J = 4.6 Hz, 1H), 3.79-3.70 (m, 6H), 3.54 (d, J = 10.9 Hz, 2H), 3.30 (d, J = 13.1 Hz, 3H), 3.07 (s, 3H), 3.01 – 2.85 (m, 5H), 2.58 (dd, J = 27.5, 15.5 Hz, 3H), 2.43 (dd, J = 13.1, 4.6 Hz, 1H), 2.31 – 1.90 (m, 10H), 1.69 (dd, J = 21.9, 9.5 Hz, | 834.65 | General Synthesis Formula 4 |

| | | | | | |
|---|---|---|---|---|---|
| | | | 3H), 1.48 (dd, J = 23.7, 11.4 Hz, 2H). | | |
| **324** | K7 | 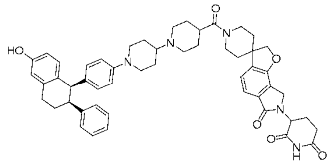 3-(1R,2S)-6-hydroxy-2-phenyl-1,2,3,4-tetrahydronaphthalen-1-yl)phenyl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.98 (s, 1H), 9.24 (s, 1H), 7.42 (d, J = 7.5 Hz, 1H), 7.29 (d, J = 7.5 Hz, 1H), 7.17 – 7.11 (m, 3H), 6.84 (d, J = 7.2 Hz, 2H), 6.65 – 6.57 (m, 4H), 6.49 (d, J = 8.2 Hz, 1H), 6.23 (d, J = 8.4 Hz, 2H), 5.14 – 5.03 (m, 1H), 4.66 (dd, J = 25.3, 12.5 Hz, 2H), 4.38 (d, J = 7.9 Hz, 2H), 4.25 (d, J = 13.8 Hz, 1H), 4.15 (d, J = 4.7 Hz, 1H), 4.01 (d, J = 13.3 Hz, 1H), 3.66 – 3.61 (m, 1H), 3.21 (d, J = 12.2 Hz, 1H), 3.16 (dd, J = 11.3, 7.0 Hz, 1H), 3.10 – 2.82 (m, 7H), 2.74 (t, J = 11.7 Hz, 1H), 2.58 (dd, J = 26.7, 14.7 Hz, 3H), 2.46 – 2.36 (m, 1H), 2.15 – 1.97 (m, 4H), 1.95 – 1.78 (m, 6H), 1.77 – 1.59 (m, 5H), 1.26 (dt, J = 14.5, 7.2 Hz, 3H). | 848.60 | General Synthesis Formula 32 |
| **325** | K8 | 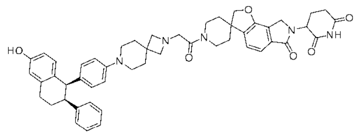 3-(1R,2S)-6-hydroxy-2-phenyl-1,2,3,4-tetrahydronaphthalen-1-yl)phenyl)-2,7-diazaspiro[3.5]nonane-2-yl)-2-oxoethyl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.99 (s, 1H), 10.46 (s, 1H), 7.36 (d, J = 7.6 Hz, 1H), 7.31 (d, J = 7.5 Hz, 1H), 7.14 (dd, J = 13.9, 7.1 Hz, 3H), 6.84 (d, J = 7.1 Hz, 2H), 6.68 – 6.56 (m, 4H), 6.49 (dd, J = 8.2, 1.8 Hz, 1H), 6.23 (d, J = 8.3 Hz, 2H), 5.09 (dt, J = 12.8, 4.8 Hz, 1H), 4.67 (td, J = 22.2, 9.3 Hz, 2H), 4.50 (ddd, J = 21.3, 16.1, 4.7 Hz, 2H), 4.40 (dd, J = 17.1, 4.7 Hz, 1H), 4.31 (d, J = 13.3 Hz, 1H), 4.25 (d, J = 8.9 Hz, 1H), 4.15 (d, J = 4.6 Hz, 1H), 4.03 (d, J = 6.6 Hz, 3H), 3.34 – 3.16 (m, 3H), 3.14 – 2.99 (m, 4H), 2.97 – 2.79 (m, 5H), 2.60 (d, J = 17.5 Hz, 1H), 2.09 (dd, J = 12.1, 5.8 Hz, 1H), 1.91 (t, J = 41.7 Hz, 6H), 1.80 – 1.71 (m, 3H), 1.18 (t, J = 7.3 Hz, 2H). | 820.65 | General Synthesis Formula 11 |
| **326** | K9 | 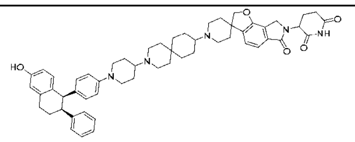 3-(1-(1-(1R,2S)-6-hydroxy-2-phenyl-1,2,3,4-tetrahydronaphthalen-1-yl)phenyl)piperidin-4-yl)-3-azaspiro[5.5]undecane-9-yl)-6-oxo- | $^1$H NMR (600 MHz, DMSO-$d_6$) δ 10.99 (s, 1H), 9.79 (s, 1H), 7.36 (d, J = 7.6 Hz, 1H), 7.30 (d, J = 7.6 Hz, 1H), 7.14 (dq, J = 14.0, 7.0 Hz, 3H), 6.84 (d, J = 7.2 Hz, 2H), 6.66 – 6.57 (m, 4H), 6.49 (dd, J = 8.2, 1.8 Hz, 1H), 6.24 (d, J = 8.4 Hz, 2H), 5.10 (dd, J = 13.1, 5.0 Hz, 1H), 4.72 – | 888.65 | General Synthesis Formula 39 |

| | | | | | |
|---|---|---|---|---|---|
| | | 6,8-dihydro-2H,7H-spiro[furo[2,3-e]isoindole-3,4'-piperidine]-7-yl)piperidine-2,6-dione | 4.62 (m, 1H), 4.40 (d, J = 17.2 Hz, 1H), 4.27 – 4.21 (m, 1H), 4.15 (d, J = 4.7 Hz, 1H), 3.69 (s, 3H), 3.40 – 3.16 (m, 7H), 3.14 – 2.96 (m, 5H), 2.92 (dd, J = 15.1, 10.8 Hz, 2H), 2.65 – 2.51 (m, 4H), 2.43 (dd, J = 26.6, 13.5 Hz, 1H), 2.28 – 2.20 (m, 2H), 2.09 (d, J = 5.4 Hz, 4H), 2.04 – 1.85 (m, 5H), 1.75 – 1.54 (m, 6H), 1.52 – 1.32 (m, 4H), 1.12 – 0.98 (m, 1H) | | |
| **327** | K10 | 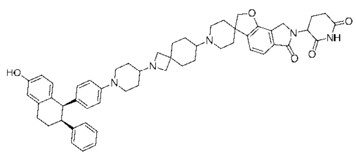 3-(1-(1-(1-(1R,2S)-6-hydroxy-2-phenyl-1,2,3,4-tetrahydronaphthalen-1-yl)phenyl)piperidin-4-yl)-2-azaspiro[3.5]nonane-7-yl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.99 (s, 1H), 10.20 (s, 1H), 7.35 (d, J = 7.5 Hz, 1H), 7.29 (t, J = 6.6 Hz, 1H), 7.20 – 7.08 (m, 3H), 6.85 (d, J = 7.2 Hz, 2H), 6.67 – 6.56 (m, 4H), 6.49 (dd, J = 8.2, 1.8 Hz, 1H), 6.23 (d, J = 8.3 Hz, 2H), 5.10 (dd, J = 13.2, 4.9 Hz, 1H), 4.71 – 4.63 (m, 1H), 4.39 (d, J = 17.2 Hz, 1H), 4.24 (d, J = 17.1 Hz, 1H), 4.15 (d, J = 4.4 Hz, 1H), 4.01 – 3.85 (m, 3H), 3.81 (s, 1H), 3.68 (s, 3H), 3.30 (d, J = 10.1 Hz, 3H), 3.21 (s, 1H), 3.14 – 3.02 (m, 2H), 2.95 (dt, J = 26.5, 13.0 Hz, 4H), 2.57 (dd, J = 38.8, 13.8 Hz, 3H), 2.43 (dd, J = 28.7, 15.7 Hz, 1H), 2.29 – 2.15 (m, 2H), 2.13 – 1.94 (m, 7H), 1.81 (dd, J = 113.6, 6.2 Hz, 3H), 1.52 (dd, J = 27.4, 16.8 Hz, 3H), 1.36 (d, J = 10.4 Hz, 3H), 1.19 (dd, J = 25.3, 18.1 Hz, 1H) | 860.70 | General Synthesis Formula 39 |
| **328** | K11 | 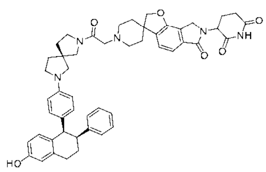 3-(1,2S)-6-hydroxy-2-phenyl-1,2,3,4-tetrahydronaphthalen-1-phenyl)-2,7-diazaspiro[4.4]nonane-2-yl)-2-oxoethyl)-6-oxo-6,8-dihydro-2H,7H-spiro[2,3-e]isoindole-3,4'-piperidine]-7-yl)piperidine-2,6-dione | $^1$H NMR (600 MHz, DMSO-$d_6$) δ 11.00 (s, 1H), 9.91 (s, 1H), 7.36 (d, J = 7.5 Hz, 1H), 7.30 (d, J = 7.5 Hz, 1H), 7.14 (dt, J = 7.1, 4.9 Hz, 3H), 6.86 (t, J = 6.9 Hz, 2H), 6.65 – 6.57 (m, 2H), 6.48 (d, J = 8.2 Hz, 1H), 6.25 – 6.12 (m, 4H), 5.10 (dd, J = 13.2, 4.9 Hz, 1H), 4.70 – 4.61 (m, 2H), 4.15 – 4.11 (m, 2H), 3.58 – 3.45 (m, 4H), 3.42 – 3.28 (m, 3H), 3.28 – 3.02 (m, 7H), 3.00 – 2.86 (m, 3H), 2.60 (d, J = 16.6 Hz, 1H), 2.43 (ddd, J = 26.5, 13.2, 3.9 Hz, 1H), 2.36 – 2.07 (m, 3H), 2.05 – 1.66 (m, 9H), 1.24 (ddd, J = 34.6, 15.1, 7.0 Hz, 1H) | 820.55 | General Synthesis Formula 11 |

| | | | | | |
|---|---|---|---|---|---|
| **329** | K12 | 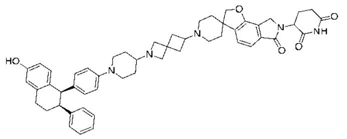 3-(1-(1-(1R,2S)-6-hydroxy-2-phenyl-1,2,3,4-tetrahydronaphthalen-1-yl)phenyl)piperidin-4-yl)-2-azaspiro[3.3]heptane-6-yl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.99 (s, 1H), 7.36 (d, J = 7.5 Hz, 1H), 7.29 (d, J = 7.6 Hz, 1H), 7.18 – 7.08 (m, 3H), 6.84 (d, J = 7.1 Hz, 2H), 6.67 – 6.54 (m, 4H), 6.49 (dd, J = 8.2, 2.2 Hz, 1H), 6.23 (d, J = 8.5 Hz, 2H), 5.10 (dd, J = 13.2, 5.0 Hz, 1H), 4.65 (q, J = 9.9 Hz, 1H), 4.40 (d, J = 17.2 Hz, 1H), 4.27 – 4.18 (m, 3H), 4.15 (d, J = 4.6 Hz, 1H), 4.06 (s, 1H), 3.63 (s, 3H), 3.26 (dd, J = 51.3, 29.6 Hz, 4H), 3.04 – 2.82 (m, 5H), 2.69 (s, 1H), 2.60 (d, J = 16.6 Hz, 2H), 2.54 (d, J = 9.2 Hz, 4H), 2.47 – 2.37 (m, 2H), 2.17 – 2.04 (m, 3H), 2.00 (dd, J = 17.0, 10.1 Hz, 3H), 1.90 (d, J = 14.2 Hz, 2H), 1.72 (d, J = 5.7 Hz, 1H), 1.28 (dd, J = 79.7, 22.8 Hz, 3H) | 832.60 | General Synthesis Formula 13 |
| **330** | K13 | 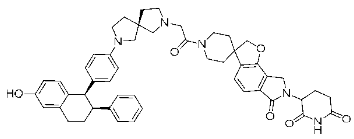 3-(1,2)-(R)-7-(4-hydroxy-2-phenyl-1,2,3,4-tetrahydronaphthalen-1-yl)phenyl)-2,7-diazaspiro[4.4]nonane-2-yl)acetyl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.99 (s, 1H), 9.12 (s, 1H), 7.36 (t, J = 8.7 Hz, 1H), 7.29 (t, J = 8.4 Hz, 1H), 7.19 – 7.09 (m, 3H), 6.86 (d, J = 7.5 Hz, 2H), 6.65 – 6.58 (m, 2H), 6.48 (dd, J = 8.2, 2.0 Hz, 1H), 6.16 (dd, J = 32.4, 8.1 Hz, 4H), 5.17 – 5.00 (m, 1H), 4.76 – 4.57 (m, 2H), 4.44 – 4.31 (m, 2H), 4.24 (dd, J = 17.0, 5.5 Hz, 1H), 4.12 (s, 1H), 3.30 – 3.24 (m, 2H), 3.23 – 3.13 (m, 5H), 3.11 (d, J = 7.3 Hz, 6H), 3.02 – 2.87 (m, 4H), 2.81 (s, 1H), 2.60 (d, J = 17.3 Hz, 1H), 2.43 (dd, J = 26.2, 13.2 Hz, 1H), 2.17 – 2.07 (m, 1H), 1.99 (d, J = 5.4 Hz, 2H), 1.91 (d, J = 4.8 Hz, 3H), 1.74 (dt, J = 12.0, 10.8 Hz, 4H) | 820.65 | General Synthesis Formula 41 |
| **331** | K14 | 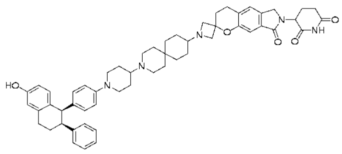 3-(1-(1-(1R,2S)-6-hydroxy-2-phenyl-1,2,3,4-tetrahydronaphthalen-1-yl)phenyl)piperidin-4-yl)-3-azaspiro[5.5]undecane-9-yl)-8-oxo-3,4,6,8-tetrahydro-7-spiro-7-azabicyclo[2.2.2]octane[2,3-f]isoindole]-7-yl)piperidine-2,6-dione | $^1$H NMR (600 MHz, DMSO-$d_6$) δ 10.96 (s, 1H), 9.29 (s, 1H), 7.53 (s, 1H), 7.14 (dq, J = 14.1, 7.0 Hz, 3H), 7.06 (d, J = 13.1 Hz, 1H), 6.84 (d, J = 7.2 Hz, 2H), 6.66 – 6.57 (m, 4H), 6.49 (dd, J = 8.2, 1.9 Hz, 1H), 6.24 (d, J = 8.5 Hz, 2H), 5.07 (dd, J = 13.2, 5.0 Hz, 1H), 4.41 (s, 1H), 4.39 – 4.31 (m, 3H), 4.24 (d, J = 15.4 Hz, 2H), 4.15 (d, J = 4.8 Hz, 2H), 3.70 (d, J = 4.8 Hz, 2H), 3.31 (ddd, J = 36.4, 26.0, 12.6 Hz, 5H), 3.07 – 2.84 (m, 7H), 2.65 – | 874.65 | General Synthesis Formula 4 |

| | | | | | |
|---|---|---|---|---|---|
| | | | 2.53 (m, 3H), 2.37 (dd, J = 24.2, 11.2 Hz, 1H), 2.26 – 2.03 (m, 6H), 2.01 – 1.89 (m, 2H), 1.88 – 1.61 (m, 6H), 1.52 (s, 1H), 1.46 – 1.33 (m, 3H), 1.32 – 1.22 (m, 2H) | | |
| **332** | K15 | 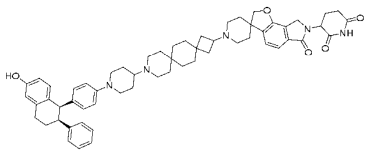 3-(1-(1R,2S)-6-hydroxy-2-phenyl-1,2,3,4-tetrahydronaphthalen-1-yl)phenyl)piperidin-4-yl)-10-azaspiro[3.2.5$^7$.2$^4$]tetradecane-2-yl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.98 (s, 1H), 9.24 (s, 1H), 7.35 (t, J = 9.4 Hz, 1H), 7.32 – 7.27 (m, 1H), 7.14 (dt, J = 13.5, 6.7 Hz, 3H), 6.84 (d, J = 7.2 Hz, 2H), 6.65 – 6.57 (m, 4H), 6.49 (d, J = 6.3 Hz, 1H), 6.24 (d, J = 8.2 Hz, 2H), 5.16 – 5.02 (m, 1H), 4.65 (s, 1H), 4.40 (d, J = 17.2 Hz, 1H), 4.29 – 4.21 (m, 1H), 4.15 (d, J = 4.4 Hz, 1H), 3.68 (s, 4H), 3.30 (d, J = 12.8 Hz, 6H), 3.09 – 2.96 (m, 4H), 2.95 – 2.80 (m, 4H), 2.58 (dd, J = 25.9, 15.7 Hz, 3H), 2.11 (dd, J = 25.3, 13.8 Hz, 7H), 1.98 (d, J = 12.4 Hz, 4H), 1.81 – 1.60 (m, 5H), 1.58 – 1.36 (m, 8H), 1.30 – 1.12 (m, 3H) | 928.75 | General Synthesis Formula 4 |
| **333** | K16 | 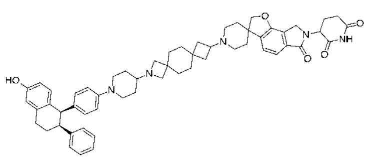 3-(1-(1R,2S)-6-hydroxy-2-phenyl-1,2,3,4-tetrahydronaphthalen-1-yl)phenyl)piperidin-4-yl)-2-azadispiro[3.2.3$^7$.2$^4$]dodecane-9-yl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.99 (s, 1H), 10.10 (s, 1H), 7.35 (d, J = 7.6 Hz, 1H), 7.29 (d, J = 7.6 Hz, 1H), 7.14 (dd, J = 15.2, 7.2 Hz, 3H), 6.84 (d, J = 7.3 Hz, 2H), 6.64 – 6.57 (m, 4H), 6.49 (dd, J = 8.1, 1.9 Hz, 1H), 6.23 (d, J = 8.4 Hz, 2H), 5.10 (dd, J = 13.2, 5.0 Hz, 1H), 4.65 (s, 1H), 4.40 (d, J = 17.2 Hz, 1H), 4.24 (d, J = 17.3 Hz, 1H), 4.15 (d, J = 4.7 Hz, 1H), 3.84 (s, 5H), 3.41 (d, J = 9.6 Hz, 2H), 3.30 (d, J = 10.3 Hz, 3H), 3.05 – 2.81 (m, 6H), 2.60 (d, J = 16.4 Hz, 1H), 2.43 (ddd, J = 17.9, 13.2, 4.2 Hz, 1H), 2.25 – 2.04 (m, 6H), 1.96 (dt, J = 44.1, 10.2 Hz, 8H), 1.67 (dd, J = 80.0, 37.5 Hz, 6H), 1.39 (dd, J = 56.6, 36.7 Hz, 6H) | 900.70 | General Synthesis Formula 4 |
| **334** | K17 | 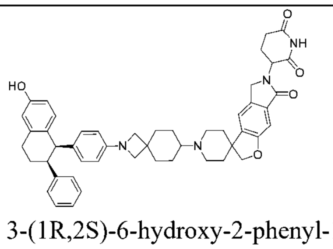 3-(1R,2S)-6-hydroxy-2-phenyl-1,2,3,4-tetrahydronaphthalen-1-yl)phenyl)-2- | $^1$H NMR (600 MHz, DMSO-$d_6$) δ 10.99 (s, 1H), 9.47 (s, 1H), 7.32 (s, 1H), 7.13 (dq, J = 14.2, 7.1 Hz, 3H), 7.08 (s, 1H), 6.84 (d, J = 7.4 Hz, 2H), 6.62 (dd, J = 15.0, 5.0 Hz, 2H), 6.48 (dd, J = 8.3, 2.1 Hz, 1H), 6.19 (d, J = 8.3 Hz, 2H), 6.05 (d, J = 8.2 Hz, 2H), 5.10 (dd, J = 13.2, 5.0 Hz, | 777.60 | General Synthesis Formula 6 |

| | | | | | |
|---|---|---|---|---|---|
| | | azaspiro[3.5]nonane-7-yl)-7-oxo-5,7-dihydro-2H,6H-spirofuro[2,3-f]isoindole-3,4'-piperidine-6-yl)piperidine-2,6-dione | 1H), 4.64 – 4.57 (m, 2H), 4.39 (d, J = 17.0 Hz, 1H), 4.27 (dd, J = 16.8, 7.9 Hz, 1H), 4.12 (d, J = 4.7 Hz, 1H), 3.54 – 3.41 (m, 5H), 3.27 (dd, J = 22.1, 7.8 Hz, 2H), 3.10 (d, J = 10.4 Hz, 2H), 2.94 (tdd, J = 22.8, 17.3, 5.6 Hz, 3H), 2.60 (d, J = 16.7 Hz, 1H), 2.43 – 2.32 (m, 1H), 2.25 – 2.07 (m, 3H), 1.99 (t, J = 15.8 Hz, 7H), 1.80 – 1.66 (m, 2H), 1.62 – 1.41 (m, 4H) | | |
| **335** | K18 | 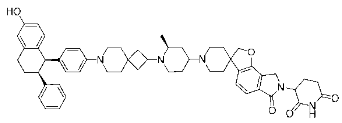 3-(1R,2S)-6-hydroxy-2-phenyl-1,2,3,4-tetrahydronaphthalen-1-yl)phenyl)-7-azaspiro[3.5]nonane-2-yl)-2-methylpiperidin-4-yl)-6-oxo-6,8-dihydro-2H,7H-spiro[2,3-e]isoindole-3,4'-piperidine]-7-yl)piperidine-2,6-dione | $^1$H NMR (600 MHz, DMSO-$d_6$) δ 10.99 (s, 1H), 7.36 (d, J = 7.6 Hz, 1H), 7.28 (d, J = 7.5 Hz, 1H), 7.13 (dq, J = 14.0, 6.9 Hz, 3H), 6.83 (d, J = 7.1 Hz, 2H), 6.70 (s, 2H), 6.66 – 6.60 (m, 2H), 6.49 (dd, J = 8.2, 2.0 Hz, 1H), 6.28 (d, J = 7.5 Hz, 2H), 5.10 (dd, J = 13.2, 5.0 Hz, 1H), 4.68 (s, 2H), 4.40 (d, J = 17.1 Hz, 1H), 4.25 (d, J = 17.3 Hz, 1H), 4.17 (d, J = 3.8 Hz, 1H), 3.84 (dd, J = 33.5, 28.1 Hz, 2H), 3.33 (dd, J = 25.6, 11.1 Hz, 4H), 3.05 (dd, J = 66.5, 25.8 Hz, 7H), 2.92 (dd, J = 15.0, 10.9 Hz, 3H), 2.61 (d, J = 16.4 Hz, 1H), 2.54 (d, J = 10.8 Hz, 1H), 2.47 – 2.37 (m, 1H), 2.24 (s, 4H), 2.12 (ddd, J = 21.7, 18.2, 6.9 Hz, 4H), 2.06 – 1.90 (m, 6H), 1.71 (s, 3H), 1.63 (s, 2H), 1.46 – 1.32 (m, 1H), 1.26 (d, J = 6.3 Hz, 2H) | 874.65 | General Synthesis Formula 7 |
| **336** | K19 | 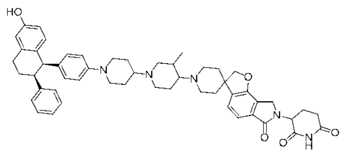 3-(1R,2S)-6-hydroxy-2-phenyl-1,2,3,4-tetrahydronaphthalen-1-yl)phenyl)-3-methyl-[1,4'-bipiperidin]-4-yl)-6-oxo-6,8-dihydro-2H,7H-spirofuro[2,3-e]isoindole-3,4'-piperidine]-7-yl)piperidine-2,6-dione | $^1$H NMR (600 MHz, DMSO-$d_6$) δ 10.99 (s, 1H), 9.92 (s, 1H), 7.36 (d, J = 7.5 Hz, 1H), 7.29 (d, J = 6.3 Hz, 1H), 7.14 (dq, J = 14.2, 6.9 Hz, 3H), 6.83 (d, J = 7.0 Hz, 4H), 6.67 – 6.60 (m, 2H), 6.53 – 6.47 (m, 1H), 6.34 (d, J = 8.0 Hz, 2H), 5.10 (dd, J = 13.2, 4.9 Hz, 1H), 4.67 (s, 2H), 4.40 (d, J = 17.2 Hz, 2H), 3.72 – 3.47 (m, 4H), 3.40 – 3.29 (m, 2H), 3.06 (dd, J = 57.7, 24.6 Hz, 6H), 2.92 (dt, J = 17.2, 9.1 Hz, 3H), 2.78 (s, 1H), 2.70 (s, 1H), 2.61 (d, J = 15.9 Hz, 1H), 2.43 (dt, J = 12.8, 9.0 Hz, 2H), 2.31 (s, 1H), 2.19 (d, J = 21.2 Hz, 3H), 2.12 – 1.92 (m, 6H), 1.72 (d, J = 12.9 Hz, 5H), 1.22 (d, J = 4.9 Hz, 3H) | 834.65 | General Synthesis Formula 7 |

| | | | | | |
|---|---|---|---|---|---|
| **337** | K20 | 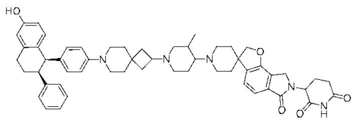 3-(1-(7-(4-(1R,2S)-6-hydroxy-2-phenyl-1,2,3,4-tetrahydronaphthalen-1-yl)phenyl)-7-azaspiro[3.5]nonane-2-yl)-3-methylpiperidin-4-yl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.99 (s, 1H), 9.29 (s, 1H), 7.36 (d, J = 7.6 Hz, 1H), 7.29 (d, J = 7.2 Hz, 1H), 7.15 (dt, J = 14.1, 6.9 Hz, 3H), 6.85 (d, J = 7.3 Hz, 2H), 6.66 – 6.59 (m, 4H), 6.49 (dd, J = 8.3, 2.2 Hz, 1H), 6.24 (d, J = 8.2 Hz, 2H), 5.10 (dd, J = 13.2, 5.0 Hz, 1H), 4.67 (s, 2H), 4.40 (d, J = 17.2 Hz, 1H), 4.25 (d, J = 17.2 Hz, 1H), 4.16 (d, J = 4.7 Hz, 1H), 3.86 (s, 3H), 3.71 (s, 7H), 3.30 (d, J = 10.1 Hz, 3H), 3.09 (s, 2H), 3.04 – 2.87 (m, 5H), 2.70 (s, 1H), 2.60 (d, J = 16.9 Hz, 3H), 2.47 – 2.38 (m, 2H), 2.33 (s, 1H), 2.09 (ddd, J = 25.9, 15.4, 9.7 Hz, 4H), 2.02 – 1.91 (m, 3H), 1.80 – 1.62 (m, 3H), 1.29 – 1.18 (m, 4H) | 874. 65 | General Synthesis Formula 7 |
| **338** | K21 | 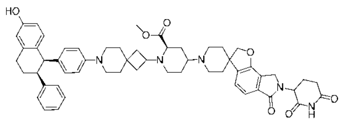 (2R)-4-(7-(2,6-dioxopiperidin-3-yl)-6-oxo-7,8-dihydro-2H,6H-spirofuro[2,3-e]isoindole-3,4'-piperidine]-1-yl)-1-(7-(4-((1R,2S)-6-hydroxy-2-phenyl-1,2,3,4-tetrahydronaphthalen-1-yl)phenyl)-7-azaspiro[3.5]nonane-2-yl)piperidine-2-carboxylate | $^1$H NMR (600 MHz, DMSO-$d_6$) δ 10.99 (s, 1H), 7.36 (d, J = 7.6 Hz, 1H), 7.29 (d, J = 6.6 Hz, 1H), 7.17 – 7.10 (m, 3H), 6.83 (d, J = 7.1 Hz, 2H), 6.75 – 6.58 (m, 4H), 6.49 (dd, J = 8.2, 2.2 Hz, 1H), 6.27 (d, J = 7.1 Hz, 2H), 5.10 (dd, J = 13.2, 5.0 Hz, 1H), 4.66 (s, 2H), 4.40 (d, J = 17.2 Hz, 1H), 4.24 (d, J = 17.2 Hz, 1H), 4.17 (d, J = 4.2 Hz, 1H), 3.74 (s, 3H), 3.31 (d, J = 13.6 Hz, 8H), 3.11 – 2.99 (m, 5H), 2.98 – 2.86 (m, 5H), 2.61 (d, J = 17.1 Hz, 1H), 2.45 – 2.37 (m, 2H), 2.18 (s, 3H), 2.12 – 2.05 (m, 2H), 2.00 (dd, J = 17.0, 11.3 Hz, 3H), 1.90 (d, J = 15.8 Hz, 2H), 1.72 (d, J = 6.0 Hz, 2H), 1.64 (s, 2H), 1.56 (s, 2H), 1.24 (s, 1H) | 103 2.17 | General Synthesis Formula 7 |
| **339** | K22 | 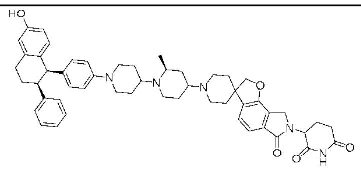 3-(1R,2S)-6-hydroxy-2-phenyl-1,2,3,4-tetrahydronaphthalen-1-yl)phenyl)-2-methyl-[1,4'-bipiperidin]-4-yl)-6-oxo-6,8-dihydro-2H,7H-spirofuro[2,3-e]isoindole-3,4'-piperidine]-7-yl)piperidine-2,6-dione | $^1$H NMR (600 MHz, DMSO-$d_6$) δ 10.99 (s, 1H), 7.36 (d, *J* = 7.6 Hz, 1H), 7.28 (d, *J* = 7.2 Hz, 1H), 7.19 – 7.10 (m, 3H), 6.88 – 6.81 (m, 2H), 6.66 – 6.56 (m, 4H), 6.49 (dd, *J* = 8.0, 2.0 Hz, 1H), 6.24 (t, *J* = 8.0 Hz, 2H), 5.10 (dd, *J* = 13.3, 5.0 Hz, 1H), 4.67 (s, 1H), 4.40 (d, *J* = 17.1 Hz, 1H), 4.24 (d, *J* = 17.2 Hz, 1H), 4.16 (d, *J* = 4.9 Hz, 1H), 3.77 – 3.54 (m, 6H), 3.30 – 3.20 (m, 3H), 3.14 – 2.88 (m, 8H), 2.67 – 2.56 | 834. 70 | General Synthesis Formula 7 |

| | | | | | |
|---|---|---|---|---|---|
| | | | (m, 3H), 2.48 – 2.37 (m, 1H), 2.35 – 2.22 (m, 2H), 2.19 – 1.94 (m, 8H), 1.75 – 1.69 (m, 1H), 1.60 (d, *J* = 9.4 Hz, 1H), 1.55 – 1.46 (m, 1H), 1.30 (d, *J* = 5.3 Hz, 2H), 1.24 (s, 1H), 1.18 (t, *J* = 7.2 Hz, 1H) | | |
| **340** | K23 | 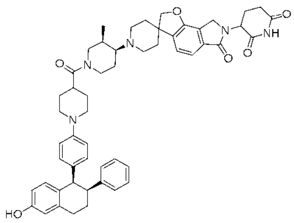 3-(1-(1-(1-(1R,2S)-6-hydroxy-2-phenyl-1,2,3,4-tetrahydronaphthalen-1-yl)phenyl)piperidin-4-carbonyl)-3-dimethylpiperidin-4-yl)-6-oxo-6,8-dihydro-2H,7H-spirofuro[2,3-e]isoindole-3,4'-piperidine]-7-yl)piperidine-2,6-dione | $^1$H NMR (600 MHz, DMSO-$d_6$) δ 10.98 (s, 1H), 7.36 (t, J = 6.6 Hz, 1H), 7.29 (d, J = 7.4 Hz, 1H), 7.15 (dd, J = 15.3, 8.2 Hz, 4H), 7.04 – 6.78 (m, 3H), 6.70 – 6.62 (m, 2H), 6.51 (d, J = 7.6 Hz, 1H), 6.42 (s, 2H), 5.17 – 5.04 (m, 1H), 4.73 – 4.51 (m, 3H), 4.40 (d, J = 17.0 Hz, 2H), 4.24 (d, J = 16.8 Hz, 2H), 4.19 – 4.07 (m, 1H), 3.97 (d, J = 12.5 Hz, 1H), 3.74 (s, 1H), 3.27 (s, 2H), 3.19 – 2.98 (m, 7H), 2.92 (dd, J = 26.8, 11.9 Hz, 3H), 2.60 (d, J = 16.1 Hz, 2H), 2.48 – 2.36 (m, 3H), 2.08 (d, J = 11.8 Hz, 3H), 1.94 (dd, J = 34.1, 17.2 Hz, 5H), 1.75 (d, J = 13.7 Hz, 4H), 1.21 (dd, J = 19.1, 11.9 Hz, 1H), 1.08 (t, J = 9.0 Hz, 1H), 0.97 (d, J = 5.6 Hz, 1H) | 862.60 | General Synthesis Formula 18 |
| **341** | K24 | 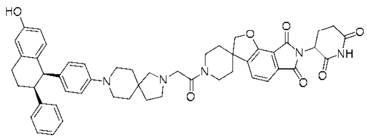 7-(2,6-Dioxopiperidin-3-yl)-1'-(2-(8-(4-(1R,2S)-6-hydroxy-2-phenyl-1,2,3,4-tetrahydronaphthalen-1-yl)phenyl)-2,8-diazaspiro[4.5]decane-2-yl)acetyl)-7H,2H-spiro[isoindole-3,4'-piperidine]-6,8-dione | $^1$H NMR (600 MHz, DMSO-$d_6$) δ 11.11 (s, 1H), 10.11 (s, 1H), 7.68 (d, J = 7.4 Hz, 1H), 7.46 (d, J = 7.3 Hz, 1H), 7.18 – 7.11 (m, 3H), 6.84 (d, J = 7.1 Hz, 2H), 6.67 – 6.59 (m, 4H), 6.49 (d, J = 8.2 Hz, 1H), 6.25 (d, J = 8.2 Hz, 2H), 5.10 (dd, J = 12.9, 5.4 Hz, 1H), 4.82 (dd, J = 26.5, 9.3 Hz, 2H), 4.53 – 4.41 (m, 2H), 4.37 (d, J = 13.1 Hz, 1H), 4.16 (d, J = 4.4 Hz, 1H), 3.65 (d, J = 14.2 Hz, 3H), 3.31 – 3.17 (m, 4H), 3.00 (dd, J = 26.1, 14.2 Hz, 6H), 2.94 – 2.81 (m, 3H), 2.60 (d, J = 18.1 Hz, 1H), 2.09 (dt, J = 18.6, 9.7 Hz, 1H), 2.05 – 1.96 (m, 2H), 1.88 (ddd, J = 30.5, 24.9, 13.4 Hz, 4H), 1.73 (d, J = 34.0 Hz, 5H), 1.24 (s, 1H) | 848.55 | General Synthesis Formula 41 |
| **342** | K25 | 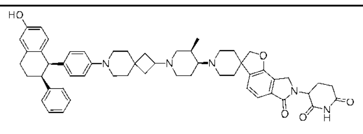 3-(7-(4-(1R,2S)-6-hydroxy-2-phenyl-1,2,3,4-tetrahydronaphthalen-1- | $^1$H NMR (600 MHz, DMSO-$d_6$) δ 11.00 (s, 1H), 9.75 (s, 1H), 7.36 (d, J = 7.5 Hz, 1H), 7.29 (d, J = 7.2 Hz, 1H), 7.18 – 7.10 (m, 3H), 6.83 (d, J = 7.1 Hz, 2H), 6.75 (s, 2H), 6.66 – 6.60 (m, 2H), 6.49 (d, J = 8.2 | 874.65 | General Synthesis Formula 7 |

| | | | | | |
|---|---|---|---|---|---|
| | | yl)phenyl)-7-azaspiro[3.5]nonane-2-yl)-3-methylpiperidin-4-yl)-6-oxo-6,8-dihydro-2H,7H-spiro[furo[2,3-e]isoindole-3,4'-piperidine]-7-yl)piperidine-2,6-dione | Hz, 1H), 6.30 (d, J = 7.7 Hz, 2H), 5.10 (dd, J = 13.2, 4.8 Hz, 1H), 4.67 (s, 2H), 4.40 (d, J = 17.2 Hz, 1H), 4.25 (d, J = 17.2 Hz, 1H), 4.19 (d, J = 4.0 Hz, 1H), 3.48 (s, 3H), 3.35 (dd, J = 32.1, 11.0 Hz, 3H), 3.23 – 3.07 (m, 4H), 3.06 – 2.88 (m, 7H), 2.73 (d, J = 43.6 Hz, 2H), 2.60 (d, J = 16.1 Hz, 1H), 2.43 (dt, J = 13.0, 9.3 Hz, 2H), 2.27 (d, J = 12.2 Hz, 2H), 2.17 (d, J = 24.9 Hz, 3H), 2.10 – 1.92 (m, 6H), 1.72 (d, J = 54.0 Hz, 5H), 1.27 – 1.17 (m, 3H) | | |
| **343** | K26 | 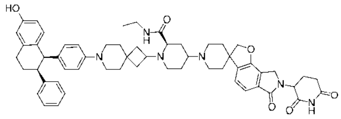 (2R)-4-(7-(2,6-Dioxopiperidin-3-yl)-6-oxo-7,8-dihydro-2H,6H-spiro[furo[2,3-e]isoindole-3,4'-piperidine]-1'-yl)-N-ethyl-1-(7-(4-((1R,2S)-6-hydroxy-2-phenyl-1,2,3,4-tetrahydronaphthalen-1-yl)phenyl)-7-azaspiro[3.5]nonane-2-yl)piperidine-2-carboxamide | $^1$H NMR (600 MHz, DMSO-$d_6$) δ 10.99 (s, 1H), 9.13 (s, 1H), 7.36 (d, J = 7.6 Hz, 1H), 7.30 (d, J = 6.5 Hz, 1H), 7.13 (dd, J = 12.6, 7.1 Hz, 3H), 6.83 (d, J = 7.0 Hz, 2H), 6.67 – 6.54 (m, 4H), 6.48 (dd, J = 8.3, 2.4 Hz, 1H), 6.23 (d, J = 8.4 Hz, 2H), 5.10 (dd, J = 13.2, 5.1 Hz, 1H), 4.73 – 4.60 (m, 2H), 4.40 (d, J = 17.2 Hz, 1H), 4.24 (d, J = 17.2 Hz, 1H), 4.15 (d, J = 4.8 Hz, 1H), 3.61 (s, 5H), 3.30 (d, J = 13.1 Hz, 5H), 2.98 (d, J = 10.6 Hz, 4H), 2.92 (t, J = 12.9 Hz, 5H), 2.71 (ddd, J = 11.1, 7.4, 3.8 Hz, 1H), 2.64 – 2.57 (m, 1H), 2.45 – 2.37 (m, 2H), 2.28 (d, J = 11.9 Hz, 1H), 2.25 – 2.07 (m, 5H), 2.05 – 1.96 (m, 5H), 1.72 (d, J = 5.8 Hz, 1H), 1.58 (d, J = 27.1 Hz, 4H), 1.25 (d, J = 13.0 Hz, 1H), 0.72 (d, J = 7.4 Hz, 2H), 0.49 (d, J = 2.8 Hz, 2H) | 931. 65 | General Synthesis Formula 8 |
| **344** | K27 | 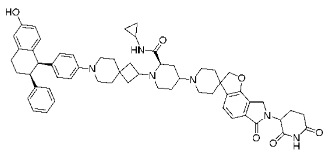 (2R)-N-Cyclopropyl-4-(7-(2,6-Dioxopiperidin-3-yl)-6-oxo-7,8-dihydro-2H,6H-spiro[furo[2,3-e]isoindole-3,4'-piperidine]-1-yl)-1-(7-(4-((1R,2S)-6-hydroxy-2-phenyl-1,2,3,4-tetrahydronaphthalen-1-yl)phenyl)-7-azaspiro[3.5]nonane-2-yl)piperidine-2-carboxamide | $^1$H NMR (600 MHz, DMSO-$d_6$) δ 10.99 (s, 1H), 7.36 (d, J = 7.6 Hz, 1H), 7.30 (d, J = 6.5 Hz, 1H), 7.13 (dd, J = 12.6, 7.1 Hz, 3H), 6.83 (d, J = 7.0 Hz, 2H), 6.67 – 6.54 (m, 4H), 6.48 (dd, J = 8.3, 2.4 Hz, 1H), 6.23 (d, J = 8.4 Hz, 2H), 5.10 (dd, J = 13.2, 5.1 Hz, 1H), 4.73 – 4.60 (m, 2H), 4.40 (d, J = 17.2 Hz, 1H), 4.24 (d, J = 17.2 Hz, 1H), 4.15 (d, J = 4.8 Hz, 1H), 3.61 (s, 5H), 3.29 (s, 5H), 2.98 (d, J = 10.6 Hz, 4H), 2.92 (t, J = 12.9 Hz, 5H), 2.71 (ddd, J = 11.1, 7.4, 3.8 Hz, 1H), 2.64 – 2.57 (m, | 943. 65 | General Synthesis Formula 16 |

| | | | | | |
|---|---|---|---|---|---|
| | | | 1H), 2.45 – 2.37 (m, 2H), 2.28 (d, J = 11.9 Hz, 1H), 2.20 (s, 3H), 2.05 – 1.96 (m, 5H), 1.92 (d, J = 10.9 Hz, 2H), 1.72 (d, J = 5.8 Hz, 1H), 1.58 (d, J = 27.1 Hz, 4H), 1.25 (d, J = 13.0 Hz, 1H), 0.72 (d, J = 7.4 Hz, 2H), 0.49 (d, J = 2.8 Hz, 2H) | | |
| **345** | K28 | 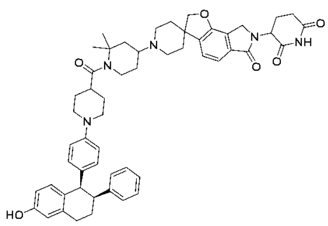 3-(1-(1-(4-(1R,2S)-6-hydroxy-2-phenyl-1,2,3,4-tetrahydronaphthalen-1-yl)phenyl)piperidin-4-carbonyl)-2,2-dimethylpiperidin-4-yl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.99 (s, 1H), 10.04 (s, 1H), 7.36 (d, J = 7.6 Hz, 1H), 7.29 (d, J = 7.6 Hz, 1H), 7.17 – 7.12 (m, 3H), 6.83 (d, J = 7.2 Hz, 2H), 6.73 – 6.59 (m, 4H), 6.49 (dd, J = 8.3, 2.3 Hz, 1H), 6.28 (d, J = 5.9 Hz, 2H), 5.10 (dd, J = 13.3, 5.0 Hz, 1H), 4.68 (q, J = 10.5 Hz, 2H), 4.40 (d, J = 17.2 Hz, 1H), 4.24 (d, J = 17.2 Hz, 1H), 4.18 (d, J = 4.0 Hz, 1H), 3.59 (d, J = 8.8 Hz, 4H), 3.52 (s, 4H), 3.32 (d, J = 13.6 Hz, 3H), 3.02 – 2.87 (m, 4H), 2.61 (d, J = 17.3 Hz, 1H), 2.44 – 2.39 (m, 1H), 2.26 (t, J = 12.5 Hz, 2H), 2.18 (d, J = 7.5 Hz, 1H), 2.09 (dt, J = 18.9, 6.2 Hz, 1H), 2.00 (t, J = 14.6 Hz, 4H), 1.91 (dd, J = 27.0, 12.2 Hz, 3H), 1.70 (dd, J = 18.5, 12.5 Hz, 5H), 1.49 (s, 3H), 1.40 (s, 3H) | 876.60 | General Synthesis Formula 15 |
| **346** | K29 | 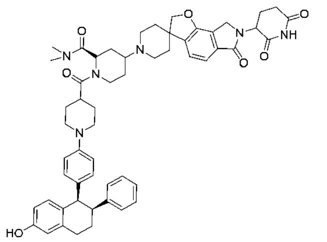 (2S)-4-(7-(2,6-Dioxopiperidin-3-yl)-6-oxo-7,8-dihydro-2H,6H-spiro[furo[2,3-e]isoindole-3,4'-piperidine]-1'-yl)-1-(1-(4-((1R,2S)-6-hydroxy-2-phenyl-1,2,3,4-tetrahydronaphthalen-1-yl)phenyl)piperidin-4-carbonyl)-N,N-dimethylpiperidine-2-carboxamide | $^1$H NMR (600 MHz, DMSO-$d_6$) δ 10.99 (s, 1H), 10.11 (s, 1H), 7.36 (d, J = 7.5 Hz, 1H), 7.30 (d, J = 7.6 Hz, 1H), 7.17 – 7.12 (m, 3H), 6.83 (d, J = 7.1 Hz, 4H), 6.67 – 6.61 (m, 2H), 6.50 (dd, J = 8.2, 1.9 Hz, 1H), 6.35 (d, J = 6.9 Hz, 2H), 5.09 (dd, J = 13.2, 5.0 Hz, 1H), 4.69 (t, J = 6.6 Hz, 2H), 4.40 (d, J = 17.2 Hz, 1H), 4.23 (dd, J = 17.3, 10.8 Hz, 2H), 3.07 (s, 4H), 3.02 – 2.96 (m, 3H), 2.96 – 2.88 (m, 4H), 2.83 (s, 4H), 2.61 (d, J = 16.7 Hz, 1H), 2.51 (s, 6H), 2.47 – 2.30 (m, 3H), 2.21 (d, J = 13.0 Hz, 3H), 2.09 (ddd, J = 18.8, 12.6, 6.4 Hz, 1H), 2.00 (dd, J = 20.3, 10.0 Hz, 4H), 1.91 (d, J = 11.8 Hz, 1H), 1.70 (d, J = 46.7 Hz, 6H) | 919.60 | General Synthesis Formula 16 |

| | | | | | |
|---|---|---|---|---|---|
| **347** | K30 | 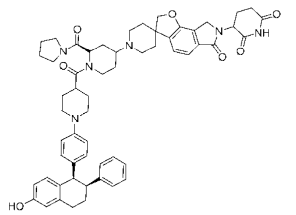 3-(1R,2S)-6-hydroxy-2-phenyl-1,2,3,4-tetrahydronaphthalen-1-yl)phenyl)piperidin-4-carbonyl)-2-(pyrrolidin-1-carbonyl)piperidin-4-yl)-6-oxo-6,8-dihydro-2H,7H-spiro[2,3-e]isoindole-3,4'-piperidine]-7-yl)piperidine-2,6-dione | $^1$H NMR (600 MHz, DMSO-$d_6$) δ 10.99 (s, 1H), 10.02 (s, 1H), 7.36 (d, J = 7.6 Hz, 1H), 7.30 (d, J = 7.6 Hz, 1H), 7.18 – 7.11 (m, 3H), 6.81 (t, J = 23.0 Hz, 4H), 6.66 (d, J = 8.3 Hz, 1H), 6.62 (d, J = 2.1 Hz, 1H), 6.50 (dd, J = 8.3, 2.3 Hz, 1H), 6.32 (d, J = 6.7 Hz, 2H), 5.10 (dd, J = 13.2, 5.0 Hz, 1H), 4.68 (q, J = 10.3 Hz, 2H), 4.58 (s, 1H), 4.40 (d, J = 17.2 Hz, 1H), 4.24 (d, J = 17.2 Hz, 1H), 4.20 (d, J = 4.4 Hz, 1H), 3.75 (d, J = 122.2 Hz, 6H), 3.37 – 3.31 (m, 3H), 3.28 – 3.14 (m, 3H), 3.05 – 2.82 (m, 7H), 2.61 (d, J = 16.9 Hz, 1H), 2.47 – 2.39 (m, 1H), 2.34 (s, 1H), 2.25 – 2.13 (m, 3H), 2.09 (dt, J = 12.6, 7.0 Hz, 1H), 2.01 (dd, J = 18.8, 10.6 Hz, 4H), 1.97 – 1.85 (m, 4H), 1.81 – 1.69 (m, 6H), 1.64 (s, 1H) | 945.60 | General Synthesis Formula 16 |
| **348** | K31 | 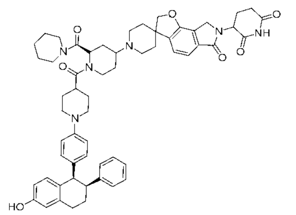 3-(1,2S)-1-(1-(4-(1R,2S)-6-hydroxy-2-phenyl-1,2,3,4-tetrahydronaphthalen-1-yl)phenyl)piperidin-4-carbonyl)-2-(piperidin-1-carbonyl)piperidin-4-yl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.99 (s, 1H), 10.08 (s, 1H), 7.36 (d, J = 7.6 Hz, 1H), 7.30 (d, J = 7.6 Hz, 1H), 7.18 – 7.12 (m, 3H), 6.83 (d, J = 7.1 Hz, 4H), 6.66 (d, J = 8.3 Hz, 1H), 6.63 (d, J = 1.7 Hz, 1H), 6.50 (dd, J = 8.2, 2.2 Hz, 1H), 6.35 (s, 2H), 5.10 (dd, J = 13.2, 5.0 Hz, 1H), 4.72 – 4.64 (m, 2H), 4.40 (d, J = 17.2 Hz, 1H), 4.28 – 4.20 (m, 2H), 3.83 (d, J = 22.9 Hz, 2H), 3.47 – 3.41 (m, 5H), 3.34 (d, J = 11.8 Hz, 3H), 3.16 (d, J = 13.1 Hz, 2H), 3.11 – 2.97 (m, 4H), 2.95 – 2.81 (m, 4H), 2.60 (d, J = 17.0 Hz, 1H), 2.46 – 2.38 (m, 1H), 2.30 – 2.16 (m, 4H), 2.12 – 2.06 (m, 1H), 2.02 – 1.88 (m, 5H), 1.71 (d, J = 37.6 Hz, 6H), 1.56 (d, J = 39.0 Hz, 4H), 1.44 (s, 2H) | 959.65 | General Synthesis Formula 16 |
| **349** | K32 | 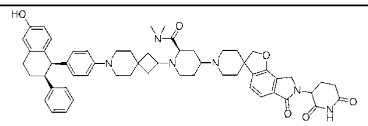 (2R)-4-(7-(2,6-Dioxopiperidin-3-yl)-6-oxo-7,8-dihydro-2H,6H-spiro[furo[2,3-e]isoindole-3,4'-piperidine]-1'-yl)-1-(7-(4-((1R,2S)-6-hydroxy-2-phenyl-1,2,3,4-tetrahydronaphthalen-1- | $^1$H NMR (600 MHz, DMSO-$d_6$) δ 10.99 (s, 1H), 9.14 (s, 1H), 7.36 (d, J = 7.6 Hz, 1H), 7.30 (s, 1H), 7.17 – 7.10 (m, 3H), 6.83 (d, J = 7.0 Hz, 2H), 6.67 – 6.53 (m, 4H), 6.48 (dd, J = 8.3, 2.3 Hz, 1H), 6.23 (d, J = 8.3 Hz, 2H), 5.10 (dd, J = 13.2, 5.0 Hz, 1H), 4.67 (s, 2H), 4.40 (d, J = 17.2 Hz, 1H), 4.24 (d, J = 17.2 Hz, 1H), 4.15 | 931.65 | General Synthesis Formula 16 |

| | | | | | |
|---|---|---|---|---|---|
| | | yl)phenyl)-7-azaspiro[3.5]nonane-2-yl)-N,N-dimethylpiperidine-2-carboxamide | (d, J = 4.8 Hz, 1H), 3.77 (s, 1H), 3.52 (d, J = 10.4 Hz, 4H), 3.30 (d, J = 13.8 Hz, 4H), 3.14 (s, 3H), 3.01 (d, J = 12.8 Hz, 1H), 2.99 – 2.94 (m, 3H), 2.92 (s, 4H), 2.66 – 2.56 (m, 2H), 2.46 – 2.35 (m, 2H), 2.32 (s, 1H), 2.18 (s, 3H), 2.09 (dd, J = 11.4, 5.2 Hz, 1H), 2.04 – 1.96 (m, 5H), 1.88 (dd, J = 50.7, 11.2 Hz, 3H), 1.79 – 1.64 (m, 2H), 1.57 (d, J = 34.7 Hz, 4H), 1.24 (s, 1H) | | |
| **350** | K33 | 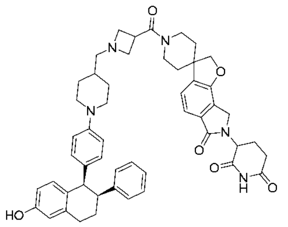 3-(1-(1-(1-(1R,2S)-6-hydroxy-2-phenyl-1,2,3,4-tetrahydronaphthalen-1-yl)phenyl)piperidin-4-yl)methyl)azetidin-3-carbonyl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.98 (s, 1H), 7.43 – 7.37 (m, 1H), 7.31 (d, J = 7.5 Hz, 1H), 7.17 – 7.11 (m, 3H), 6.83 (d, J = 7.0 Hz, 2H), 6.70 – 6.56 (m, 4H), 6.49 (d, J = 8.2 Hz, 1H), 6.27 (d, J = 7.5 Hz, 2H), 5.14 – 5.02 (m, 1H), 4.65 (q, J = 9.4 Hz, 2H), 4.39 (dt, J = 20.6, 10.3 Hz, 4H), 4.26 – 4.14 (m, 4H), 3.94 (dd, J = 34.4, 25.7 Hz, 2H), 3.31 (d, J = 12.6 Hz, 2H), 3.24 – 3.08 (m, 4H), 3.05 – 2.87 (m, 4H), 2.81 (dd, J = 26.0, 13.1 Hz, 1H), 2.62 – 2.58 (m, 2H), 2.46 – 2.37 (m, 1H), 2.13 – 2.04 (m, 1H), 2.00 – 1.92 (m, 1H), 1.76 (s, 3H), 1.72 (d, J = 11.7 Hz, 5H), 1.29 (d, J = 10.5 Hz, 2H) | 834.86 | General Synthesis Formula 32 |
| **351** | K34 | 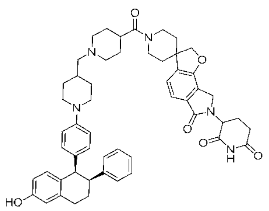 3-(1-(1-(4-(1R,2S)-6-hydroxy-2-phenyl-1,2,3,4-tetrahydronaphthalen-1-yl)phenyl)piperidin-4-methyl)piperidin-4-carbonyl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.99 (s, 1H), 9.05 (s, 1H), 7.42 (d, J = 7.6 Hz, 1H), 7.29 (d, J = 7.6 Hz, 1H), 7.18 – 7.12 (m, 3H), 6.84 (d, J = 7.0 Hz, 2H), 6.74 (s, 2H), 6.63 (dd, J = 14.0, 5.2 Hz, 2H), 6.49 (dd, J = 8.3, 2.3 Hz, 1H), 6.31 (d, J = 8.0 Hz, 2H), // 5.13 – 5.04 (m, 1H), 4.66 (dt, J = 21.0, 9.4 Hz, 2H), 4.40 (dd, J = 16.8, 7.4 Hz, 2H), 4.29 – 4.22 (m, 1H), 4.19 (d, J = 4.6 Hz, 1H), 4.01 (d, J = 12.4 Hz, 1H), 3.32 (dd, J = 13.8, 3.6 Hz, 3H), 3.22 (t, J = 12.5 Hz, 2H), 3.16 – 3.07 (m, 1H), 2.97 (d, J = 16.1 Hz, 6H), 2.94 – 2.87 (m, 2H), 2.76 (d, J = 8.8 Hz, 3H), 2.60 (d, J = 17.6 Hz, 1H), 2.45 – 2.38 (m, 1H), 2.14 – 2.05 (m, 1H), 1.98 (dd, J = 14.0, 8.9 Hz, 3H), 1.92 – 1.81 (m, 6H), 1.74 (dd, J = 16.4, | 862.60 | General Synthesis Formula 32 |

| | | | | | |
|---|---|---|---|---|---|
| | | | 8.3 Hz, 4H), 1.35 (d, J = 10.7 Hz, 2H) | | |
| **352** | K35 | 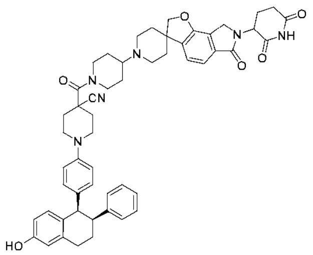 4-(4-(7-(2,6-dioxopiperidin-3-yl)-6-oxo-7,8-dihydro-2H,6H-spiro[furo[2,3-e]isoindole-3,4'-piperidine]-1'-yl)piperidin-1-carbonyl)-1-(4-((1R,2S)-6-hydroxy-2-phenyl-1,2,3,4-tetrahydronaphthalen-1-yl)phenyl)piperidin-4-carbonitrile | $^1$H NMR (600 MHz, DMSO-$d_6$) δ 11.00 (s, 1H), 9.58 (s, 1H), 7.36 (d, J = 7.6 Hz, 1H), 7.29 (d, J = 7.6 Hz, 1H), 7.19 – 7.11 (m, 3H), 6.84 (d, J = 7.1 Hz, 2H), 6.67 – 6.58 (m, 4H), 6.49 (dd, J = 8.3, 2.3 Hz, 1H), 6.25 (d, J = 8.6 Hz, 2H), 5.09 (dd, J = 13.3, 5.0 Hz, 1H), 4.68 (q, J = 9.5 Hz, 2H), 4.40 (d, J = 17.2 Hz, 1H), 4.24 (d, J = 17.2 Hz, 1H), 4.16 (d, J = 4.9 Hz, 1H), 3.59 (dd, J = 25.7, 10.8 Hz, 6H), 3.30 (dd, J = 13.8, 3.7 Hz, 3H), 3.12 (d, J = 8.5 Hz, 2H), 2.94 (ddt, J = 17.7, 13.3, 5.7 Hz, 4H), 2.78 (dd, J = 31.5, 19.5 Hz, 3H), 2.60 (d, J = 17.8 Hz, 1H), 2.47 – 2.34 (m, 1H), 2.17 (d, J = 11.6 Hz, 4H), 2.13 – 2.05 (m, 3H), 2.00 (dd, J = 8.6, 5.4 Hz, 4H), 1.70 (dd, J = 22.5, 17.4 Hz, 3H) | 873.66 | General Synthesis Formula 15 |
| **353** | K36 | 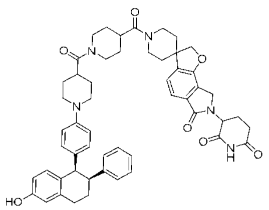 4-(4-(7-(2,6-dioxopiperidin-3-yl)-6-oxo-7,8-dihydro-2H,6H-spiro[furo[2,3-e]isoindole-3,4'-piperidine]-1'-yl)piperidin-1-carbonyl)-1-(4-((1R,2S)-6-hydroxy-2-phenyl-1,2,3,4-tetrahydronaphthalen-1-yl)phenyl)piperidin-4-carbonitrile | $^1$H NMR (600 MHz, DMSO-$d_6$) δ 10.98 (s, 1H), 7.41 (dd, J = 19.3, 7.2 Hz, 1H), 7.29 (d, J = 7.5 Hz, 1H), 7.15 (dd, J = 15.6, 8.3 Hz, 3H), 6.82 (d, J = 7.0 Hz, 2H), 6.67 – 6.63 (m, 2H), 6.53 – 6.49 (m, 1H), 6.45 (d, J = 7.1 Hz, 2H), 5.09 (d, J = 13.0 Hz, 1H), 4.72 – 4.61 (m, 2H), 4.44 – 4.35 (m, 3H), 4.29 – 4.20 (m, 2H), 4.10 – 3.93 (m, 3H), 3.37 (d, J = 13.0 Hz, 3H), 3.17 (dd, J = 34.2, 19.3 Hz, 5H), 2.97 (ddd, J = 33.1, 21.8, 16.2 Hz, 6H), 2.78 – 2.57 (m, 4H), 2.43 (dd, J = 25.7, 13.2 Hz, 1H), 2.13 – 2.03 (m, 1H), 2.01 – 1.94 (m, 1H), 1.83 (d, J = 14.0 Hz, 5H), 1.71 (d, J = 32.3 Hz, 4H) | 876.60 | General Synthesis Formula 15 |
| **354** | K37 | 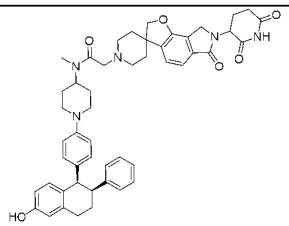 2-(7-(2,6-dioxopiperidin-3-yl)-6-oxo-7,8-dihydro-2H,6H-spiro[furo[2,3-e]isoindole-3,4'-piperidine]-1'-yl)-N-(1-(4-((1R,2S)-6-hydroxy-2-phenyl- | $^1$H NMR (600 MHz, DMSO-$d_6$) δ 10.99 (s, 1H), 9.68 (s, 1H), 7.40 – 7.34 (m, 1H), 7.32 (s, 1H), 7.14 (dq, J = 14.2, 7.0 Hz, 3H), 6.83 (d, J = 7.0 Hz, 2H), 6.65 (ddd, J = 16.4, 10.5, 4.8 Hz, 4H), 6.49 (d, J = 8.2 Hz, 1H), 6.27 (dd, J = 8.1, 4.7 Hz, 2H), 5.09 (dd, J = 13.3, 5.0 Hz, 1H), 4.65 (q, J = 9.6 Hz, 2H), 4.40 (d, J = 17.0 Hz, 2H), 4.30 – 4.21 (m, 2H), 4.17 (t, J = 4.7 Hz, | 808.60 | General Synthesis Formula 40 |

| | | | | | |
|---|---|---|---|---|---|
| | | 1,2,3,4-tetrahydronaphthalen-1-yl)phenyl)piperidin-4-yl)-N-methylacetamide | 1H), 3.69 – 3.60 (m, 3H), 3.31 (d, J = 10.0 Hz, 2H), 3.17 (d, J = 8.9 Hz, 2H), 3.03 – 2.85 (m, 4H), 2.81 (s, 3H), 2.69 (dd, J = 34.8, 23.0 Hz, 2H), 2.60 (d, J = 16.6 Hz, 1H), 2.43 (ddd, J = 26.6, 13.3, 4.5 Hz, 1H), 2.31 (dd, J = 12.2, 8.7 Hz, 2H), 2.15 – 2.05 (m, 1H), 2.04 – 1.91 (m, 3H), 1.89 – 1.79 (m, 2H), 1.72 (d, J = 6.2 Hz, 2H), 1.59 (d, J = 9.2 Hz, 1H) | | |
| **355** | K38 | 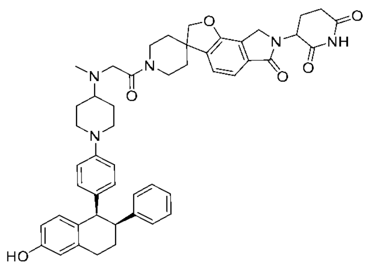 3-(1-(1R,2S)-6-hydroxy-2-phenyl-1,2,3,4-tetrahydronaphthalen-1-yl)phenyl)piperidin-4-yl)-N-methylglycyl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.99 (s, 1H), 9.39 (s, 1H), 7.38 (t, J = 5.9 Hz, 1H), 7.31 (d, J = 5.9 Hz, 1H), 7.19 – 7.09 (m, 3H), 6.83 (d, J = 7.0 Hz, 2H), 6.67 – 6.57 (m, 4H), 6.49 (dd, *J* = 8.2, 1.6 Hz, 1H), 6.24 (d, J = 7.8 Hz, 2H), 5.09 (dt, J = 10.7, 5.1 Hz, 1H), 4.74 – 4.61 (m, 2H), 4.40 (dd, J = 17.1, 6.7 Hz, 3H), 4.29 – 4.17 (m, 2H), 4.15 (d, J = 4.5 Hz, 1H), 3.75 (s, 2H), 3.32 (d, J = 10.6 Hz, 3H), 3.19 (t, J = 12.5 Hz, 1H), 3.03 – 2.86 (m, 4H), 2.79 (d, J = 14.4 Hz, 3H), 2.58 (dd, J = 27.9, 14.3 Hz, 3H), 2.47 – 2.37 (m, 1H), 2.15 – 2.02 (m, 3H), 1.98 (dd, J = 15.7, 10.3 Hz, 2H), 1.75 (dd, J = 36.9, 10.9 Hz, 6H) | 898.66 | General Synthesis Formula 41 |
| **356** | K39 | 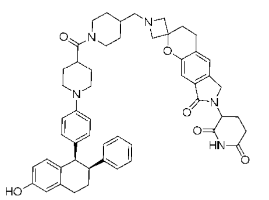 3-(1-((1-(1-(4-((1R,2S)-6-hydroxy-2-phenyl-1,2,3,4-tetrahydronaphthalen-1-yl)phenyl)piperidin-4-carbonyl)piperidin-4-yl)methyl)-8'-oxo-3',4',6',8'-tetrahydro-7'H-spiro[azetidine-3,2'-pyrano[2,3-f]isoindole]-7'-yl)piperidine-2,6-dione | $^1$H NMR (600 MHz, DMSO-$d_6$) δ 10.99 (s, 1H), 7.38 (s, 1H), 7.19 – 7.10 (m, 4H), 6.81 (t, J = 18.3 Hz, 4H), 6.64 (dd, J = 18.2, 5.2 Hz, 2H), 6.49 (dd, J = 8.3, 2.3 Hz, 1H), 6.33 (d, J = 6.4 Hz, 2H), 5.07 (dd, J = 13.2, 5.0 Hz, 1H), 4.45 (d, J = 10.0 Hz, 1H), 4.40 – 4.28 (m, 4H), 4.23 (dd, J = 28.3, 11.1 Hz, 3H), 3.97 (d, J = 10.9 Hz, 1H), 3.31 – 3.16 (m, 5H), 3.04 – 2.92 (m, 5H), 2.87 (dd, J = 26.5, 21.0 Hz, 4H), 2.60 (d, J = 17.8 Hz, 1H), 2.39 (dd, J = 17.9, 8.4 Hz, 1H), 2.27 – 2.17 (m, 2H), 2.09 (ddd, J = 12.4, 11.5, 6.4 Hz, 1H), 2.01 – 1.95 (m, 1H), 1.92 – 1.81 (m, 1H), 1.78 – 1.63 (m, 7H), 1.29 – 1.22 (m, 1H), 1.20 – 1.01 (m, 2H) | 848.65 | General Synthesis Formula 15 |

| | | | | | |
|---|---|---|---|---|---|
| **357** | K40 | 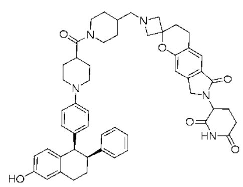 3-(1-((1-(1-(4-((1R,2S)-6-hydroxy-2-phenyl-1,2,3,4-tetrahydronaphthalen-1-yl)phenyl)piperidin-4-carbonyl)piperidin-4-yl)methyl)-6'-oxo-3',4',6',8'-tetrahydro-7'H-spiro[azetidine-3,2'-pyrano[2,3-f]isoindole]-7'-yl)piperidine-2,6-dione | $^1$H NMR (600 MHz, DMSO-$d_6$) δ 10.98 (s, 1H), 7.52 (s, 1H), 7.18 – 7.11 (m, 3H), 7.07 (d, J = 6.8 Hz, 1H), 6.82 (d, J = 7.0 Hz, 4H), 6.67 – 6.61 (m, 2H), 6.50 (dd, J = 8.2, 2.0 Hz, 1H), 6.36 (s, 2H), 5.06 (dd, J = 13.3, 5.0 Hz, 1H), 4.48 (d, J = 9.8 Hz, 1H), 4.36 (d, J = 17.2 Hz, 4H), 4.29 – 4.20 (m, 3H), 3.97 (d, J = 10.7 Hz, 2H), 3.38 – 3.18 (m, 5H), 3.06 – 2.97 (m, 3H), 2.92 (ddd, J = 31.7, 18.3, 6.1 Hz, 6H), 2.60 (d, J = 16.8 Hz, 1H), 2.41 – 2.31 (m, 1H), 2.22 (d, J = 5.1 Hz, 2H), 2.08 (qd, J = 12.8, 6.2 Hz, 1H), 2.01 – 1.95 (m, 1H), 1.89 (d, J = 32.3 Hz, 1H), 1.80 – 1.66 (m, 7H), 1.18 – 0.94 (m, 2H) | 848.60 | General Synthesis Formula 15 |
| **358** | K41 | 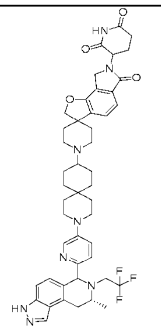 3-(1'-(3-(6-((8R)-8-methyl-7-(2,2,2-trifluoroethyl)-6,7,8,9-tetrahydro-3H-pyrazolo[4,3-f]isoquinolin-6-yl)pyridin-3-yl)-3-azaspiro[5.5]undecan-9-yl)-6-oxo-6,8-dihydro-2H,7H-spiro[furo[2,3-e]isoindole-3,4'-piperidine]-7-yl)piperidine-2,6-dione | $^1$H NMR (400 MHz, DMSO-$d_6$) δ 10.99 (s, 1H), 9.93 (s, 1H), 8.17 – 8.12 (m, 1H), 8.10 (s, 1H), 7.57 (d, *J* = 6.6 Hz, 1H), 7.38 – 7.25 (m, 4H), 6.82 (d, J = 8.7 Hz, 1H), 5.14 (s, 1H), 5.10 (dd, *J* = 13.3, 5.0 Hz, 1H), 4.74 – 4.62 (m, 2H), 4.40 (d, J = 17.3 Hz, 1H), 4.24 (d, J = 17.3 Hz, 1H), 3.24 (s, 6H), 3.19 – 3.01 (m, 5H), 2.91 (dd, J = 15.7, 7.7 Hz, 3H), 2.60 (d, J = 16.0 Hz, 1H), 2.46 – 2.38 (m, 1H), 2.30 (t, J = 12.0 Hz, 2H), 1.97 (dd, J = 15.2, 8.4 Hz, 5H), 1.85 (d, J = 12.4 Hz, 2H), 1.72 – 1.60 (m, 4H), 1.42 (d, J = 23.0 Hz, 2H), 1.28 – 1.14 (m, 3H), 1.10 (d, J = 6.5 Hz, 3H) | 851.76 | General Synthesis Formula 61 |
| **359** | K42 | 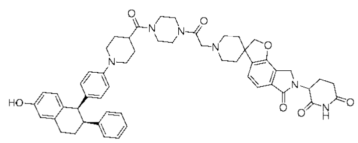 3-(1'-(2-(4-(1-(4-((1R,2S)-6-hydroxy-2-phenyl-1,2,3,4-tetrahydronaphthalen-1-yl)phenyl)piperidin-4-carbonyl)piperazin-1-yl)-2-oxoethyl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 11.01 (s, 1H), 7.37 (d, J = 7.5 Hz, 1H), 7.31 (d, J = 7.5 Hz, 1H), 7.14 (dt, J = 13.7, 6.8 Hz, 3H), 6.79 (t, J = 39.1 Hz, 4H), 6.66 – 6.60 (m, 2H), 6.49 (d, J = 6.9 Hz, 1H), 6.30 (s, 2H), 5.11 (dd, J = 13.2, 5.0 Hz, 1H), 4.66 (q, J = 9.5 Hz, 1H), 4.45 – 4.30 (m, 3H), 4.29 – 4.12 (m, 4H), 3.67 – 3.61 (m, 2H), 3.54 (dd, J = 28.4, 16.8 Hz, 8H), 3.36 (dd, J = 43.9, 11.6 Hz, 4H), 3.24 – 3.10 (m, 3H), 3.04 – 2.87 (m, 4H), 2.79 – 2.72 (m, | 891.66 | General Synthesis Formula 40 |

| | | | | | |
|---|---|---|---|---|---|
| | | | 1H), 2.63 – 2.54 (m, 1H), 2.46 – 2.38 (m, 1H), 2.31 (d, J = 13.0 Hz, 1H), 2.09 (dd, J = 12.2, 5.9 Hz, 1H), 2.02 – 1.92 (m, 2H), 1.26 (dt, J = 15.1, 7.6 Hz, 5H) | | |
| **360** | K43 | 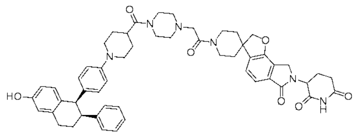 3-(1'-(2-(4-(1-(4-((1R,2S)-6-hydroxy-2-phenyl-1,2,3,4-tetrahydronaphthalen-1-yl)phenyl)piperidin-4-carbonyl)piperazin-1-yl)acetyl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 11.01 (s, 1H), 9.18 (s, 1H), 7.36 (s, 1H), 7.32 (d, J = 7.6 Hz, 1H), 7.18 – 7.12 (m, 3H), 6.83 (d, J = 7.0 Hz, 2H), 6.78 – 6.58 (m, 4H), 6.49 (dd, J = 8.2, 2.2 Hz, 1H), 6.30 (s, 2H), 5.10 (dt, J = 13.0, 5.0 Hz, 1H), 4.68 (ddd, J = 29.2, 14.1, 9.2 Hz, 2H), 4.40 (dt, J = 11.6, 10.5 Hz, 4H), 4.29 – 4.06 (m, 4H), 3.76 (s, 3H), 3.32 (d, J = 10.0 Hz, 4H), 3.22 (dd, J = 32.8, 19.7 Hz, 4H), 3.03 – 2.87 (m, 5H), 2.81 (d, J = 22.3 Hz, 3H), 2.60 (d, J = 17.4 Hz, 1H), 2.46 – 2.35 (m, 1H), 2.09 (dd, J = 12.4, 5.9 Hz, 1H), 2.02 – 1.95 (m, 1H), 1.92 (s, 1H), 1.82 (d, J = 11.9 Hz, 2H), 1.74 (d, J = 23.5 Hz, 5H) | 891.60 | General Synthesis Formula 41 |
| **361** | K44 | 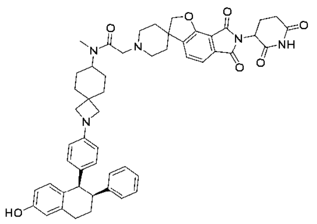 2-(7-(2,6-dioxopiperidin-3-yl)-6,8-dioxo-7,8-dihydro-2H,6H-spiro[furo[2,3-e]isoindole-3,4'-piperidine]-1'-yl)-N-(2-(4-((1R,2S)-6-hydroxy-2-phenyl-1,2,3,4-tetrahydronaphthalen-1-yl)phenyl)-2-azaspiro[3.5]nonan-7-yl)-N-methylacetamide | $^1$H NMR (600 MHz, DMSO-$d_6$) δ 11.12 (s, 1H), 7.60 (d, J = 7.4 Hz, 1H), 7.53 – 7.46 (m, 1H), 7.13 (dq, J = 13.7, 6.8 Hz, 3H), 6.84 (d, J = 7.2 Hz, 2H), 6.66 – 6.56 (m, 2H), 6.48 (d, J = 8.2 Hz, 1H), 6.18 (d, J = 7.8 Hz, 2H), 6.08 – 5.95 (m, 2H), 5.11 (dd, J = 12.9, 5.4 Hz, 1H), 4.80 (d, J = 3.6 Hz, 2H), 4.74 (d, J = 12.0 Hz, 1H), 4.48 (d, J = 24.0 Hz, 1H), 4.37 (s, 1H), 4.27 (s, 2H), 4.12 (d, J = 4.6 Hz, 1H), 3.49 (ddd, J = 20.7, 15.0, 8.9 Hz, 4H), 3.27 (s, 1H), 3.17 (s, 2H), 3.00 – 2.85 (m, 3H), 2.81 – 2.72 (m, 3H), 2.63 – 2.56 (m, 1H), 2.28 (d, J = 13.5 Hz, 2H), 2.11 (s, 1H), 2.03 – 1.89 (m, 5H), 1.71 (dd, J = 27.1, 21.9 Hz, 1H), 1.55 (t, J = 40.2 Hz, 7H) | 862.60 | General Synthesis Formula 40 |
| **362** | K45 | 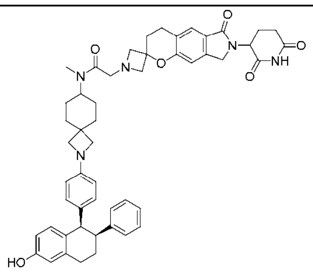 2-(7'-(2,6-dioxopiperidin-3-yl)-6'-oxo- | $^1$H NMR (600 MHz, DMSO-$d_6$) δ 10.98 (s, 1H), 7.52 (d, J = 1.9 Hz, 1H), 7.19 – 7.01 (m, 4H), 6.83 (d, J = 7.5 Hz, 2H), 6.67 – 6.56 (m, 2H), 6.48 (d, J = 8.2 Hz, 1H), 6.22 – 6.15 (m, 2H), 6.04 (dd, J = 8.1, 4.3 Hz, 2H), 5.07 (dd, J = 13.2, 4.9 Hz, 1H), 4.52 (dd, J = 66.2, 32.5 Hz, 3H), | 834.65 | General Synthesis Formula 40 |

| | | | | | |
|---|---|---|---|---|---|
| | | 4',6',7',8'-tetrahydro-3'H-spiro[azetidine-3,2'-pyrano[2,3-f]isoindole]-1-yl)-N-(2-(4-((1R,2S)-6-hydroxy-2-phenyl-1,2,3,4-tetrahydronaphthalen-1-yl)phenyl)-2-azaspiro[3.5]nonan-7-yl)-N-methylacetamide | 4.36 (dd, J = 17.5, 9.4 Hz, 3H), 4.26 (dd, J = 23.7, 15.1 Hz, 5H), 3.47 (dt, J = 20.1, 6.6 Hz, 2H), 3.36 (d, J = 18.8 Hz, 2H), 3.01 – 2.83 (m, 5H), 2.74 (d, J = 12.0 Hz, 3H), 2.60 (d, J = 16.5 Hz, 1H), 2.41 – 2.31 (m, 1H), 2.23 (s, 2H), 2.10 (dd, J = 13.3, 6.9 Hz, 1H), 2.02 – 1.88 (m, 3H), 1.70 (d, J = 5.4 Hz, 1H), 1.56 (dd, J = 35.6, 11.1 Hz, 5H), 1.43 (s, 1H) | | |
| **363** | K46 | 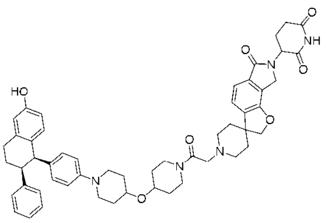 3-(1'-(2-(4-((1-(4-((1R,2S)-6-hydroxy-2-phenyl-1,2,3,4-tetrahydronaphthalen-1-yl)phenyl)piperidin-4-yl)oxy)piperidin-1-yl)-2-oxoethyl)-6-oxo-6,8-dihydro-2H,7H-spiro[furo[2,3-e]isoindole-3,4'-piperidine]-7-yl)piperidine-2,6-dione | $^1$H NMR (400 MHz, DMSO-$d_6$) δ 11.00 (s, 1H), 7.36 (d, J = 7.6 Hz, 1H), 7.30 (d, J = 7.6 Hz, 1H), 7.19 – 7.11 (m, 3H), 6.83 (d, J = 6.8 Hz, 2H), 6.78 – 6.58 (m, 4H), 6.49 (dd, J = 8.2, 2.2 Hz, 1H), 6.29 (d, J = 6.6 Hz, 2H), 5.10 (dd, J = 13.2, 5.0 Hz, 1H), 4.70 – 4.61 (m, 1H), 4.39 (d, J = 17.3 Hz, 1H), 4.32 (s, 1H), 4.21 (dd, J = 20.4, 10.7 Hz, 2H), 3.51 (d, J = 9.3 Hz, 3H), 3.43 – 3.30 (m, 5H), 3.27 – 3.07 (m, 4H), 2.97 (t, J = 11.2 Hz, 3H), 2.91 – 2.81 (m, 5H), 2.62 (t, J = 19.1 Hz, 1H), 2.47 – 2.22 (m, 3H), 2.15 – 2.03 (m, 1H), 1.93 (dd, J = 23.3, 10.9 Hz, 6H), 1.76 – 1.67 (m, 1H), 1.55 (s, 4H), 1.40 (d, J = 10.6 Hz, 1H) | 878.66 | General Synthesis Formula 40 |
| **364** | K47 | 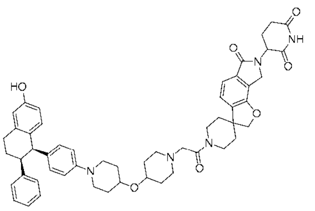 3-(1'-(2-(4-((1-(4-((1R,2S)-6-hydroxy-2-phenyl-1,2,3,4-tetrahydronaphthalen-1-yl)phenyl)piperidin-4-yl)oxy)piperidin-1-yl)acetyl)-6-oxo-6,8-dihydro-2H,7H-spiro[furo[2,3-e]isoindole-3,4'-piperidine]-7-yl)piperidine-2,6-dione | $^1$H NMR (400 MHz, DMSO-$d_6$) δ 11.00 (s, 1H), 7.36 (d, J = 7.6 Hz, 1H), 7.31 (d, J = 7.6 Hz, 1H), 7.19 – 7.10 (m, 3H), 6.81 (dd, J = 26.9, 20.4 Hz, 4H), 6.66 – 6.59 (m, 2H), 6.49 (d, J = 8.2 Hz, 1H), 6.29 (d, J = 6.1 Hz, 2H), 5.09 (dt, J = 13.1, 4.2 Hz, 1H), 4.76 – 4.59 (m, 2H), 4.47 – 4.32 (m, 4H), 4.27 – 4.15 (m, 3H), 3.75 – 3.55 (m, 4H), 3.44 (d, J = 42.7 Hz, 4H), 3.34 – 3.11 (m, 5H), 2.98 – 2.85 (m, 5H), 2.60 (d, J = 16.8 Hz, 1H), 2.39 (dd, J = 29.8, 16.8 Hz, 1H), 2.18 – 2.03 (m, 2H), 1.96 (dd, J = 17.8, 12.5 Hz, 6H), 1.75 (dd, J = 16.3, 10.4 Hz, 5H) | 878.60 | General Synthesis Formula 41 |
| **365** | K48 | 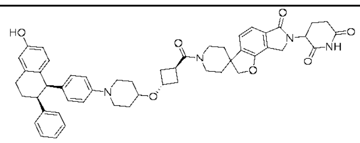 3-(1'-((1R,3R)-3-((1-(4-((1R,2S)-6- | $^1$H NMR (400 MHz, DMSO-$d_6$) δ 11.00 (s, 1H), 7.36 (d, J = 7.6 Hz, 1H), 7.30 (d, J = 7.6 Hz, 1H), 7.19 – 7.11 (m, 3H), 6.83 (d, J = 6.8 Hz, 2H), 6.78 – 6.58 (m, 4H), | 835.65 | General Synthesis Formula 43 |

| | | | | | |
|---|---|---|---|---|---|
| | | hydroxy-2-phenyl-1,2,3,4-tetrahydronaphthalen-1-yl)phenyl)piperidin-4-yl)oxy)cyclobutane-1-carbonyl)-6-oxo-6,8-dihydro-2H,7H-spiro[furo[2,3-e]isoindole-3,4'-piperidine]-7-yl)piperidine-2,6-dione | 6.49 (dd, J = 8.2, 2.2 Hz, 1H), 6.29 (d, J = 6.6 Hz, 2H), 5.10 (dd, J = 13.2, 5.0 Hz, 1H), 4.70 – 4.61 (m, 1H), 4.39 (d, J = 17.3 Hz, 1H), 4.32 (s, 1H), 4.21 (dd, J = 20.4, 10.7 Hz, 2H), 3.51 (d, J = 9.3 Hz, 3H), 3.43 – 3.30 (m, 5H), 3.27 – 3.07 (m, 4H), 2.97 (t, J = 11.2 Hz, 3H), 2.91 – 2.81 (m, 5H), 2.62 (t, J = 19.1 Hz, 1H), 2.47 – 2.22 (m, 3H), 2.15 – 2.03 (m, 1H), 1.93 (dd, J = 23.3, 10.9 Hz, 6H), 1.76 – 1.67 (m, 1H), 1.55 (s, 4H), 1.40 (d, J = 10.6 Hz, 1H) | | |
| **366** | K50 | 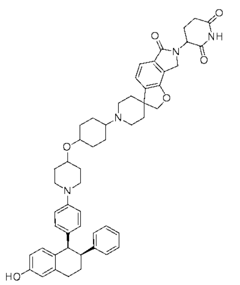 3-(1'-(4-((1-(4-((1R,2S)-6-hydroxy-2-phenyl-1,2,3,4-tetrahydronaphthalen-1-yl)phenyl)piperidin-4-yl)oxy)cyclohexyl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 11.00 (s, 1H), 7.35 (t, J = 8.5 Hz, 1H), 7.27 (d, J = 7.5 Hz, 1H), 7.13 (dq, J = 14.3, 7.1 Hz, 3H), 6.84 (d, J = 7.0 Hz, 2H), 6.70 – 6.54 (m, 4H), 6.48 (dd, J = 8.2, 1.9 Hz, 1H), 6.25 (d, J = 6.0 Hz, 2H), 5.10 (dd, J = 13.3, 4.9 Hz, 1H), 4.67 (q, J = 9.5 Hz, 2H), 4.40 (d, J = 17.2 Hz, 1H), 4.24 (d, J = 17.2 Hz, 1H), 4.15 (s, 1H), 3.31 (d, J = 13.7 Hz, 3H), 3.26 – 3.16 (m, 2H), 3.09 (d, J = 11.6 Hz, 2H), 3.02 – 2.86 (m, 4H), 2.79 (dd, J = 28.9, 24.1 Hz, 2H), 2.60 (d, J = 17.2 Hz, 1H), 2.42 (dd, J = 15.7, 11.3 Hz, 1H), 2.15 (d, J = 13.5 Hz, 2H), 2.08 (dd, J = 21.6, 8.7 Hz, 3H), 2.03 – 1.89 (m, 5H), 1.85 (s, 3H), 1.78 – 1.68 (m, 2H), 1.57 – 1.41 (m, 4H), 1.28 – 1.19 (m, 2H) | 835.65 | General Synthesis Formula 44 |
| **367** | K51 | 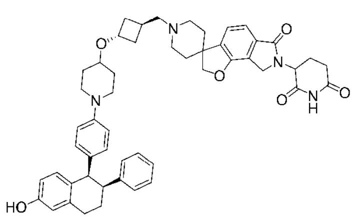 3-(1'-(((1R,3R)-3-((1-(4-((1R,2S)-6-hydroxy-2-phenyl-1,2,3,4-tetrahydronaphthalen-1-yl)phenyl)piperidin-4-yl)oxy)cyclobutyl)methyl)-6-oxo-6,8-dihydro-2H,7H-spiro[furo[2,3-e]isoindole-3,4'-piperidine]-7-yl)piperidine-2,6-dione | $^1$H NMR (400 MHz, DMSO-$d_6$) δ 11.01 (s, 1H), 7.34 (t, J = 5.9 Hz, 1H), 7.27 (d, J = 7.6 Hz, 1H), 7.19 – 7.09 (m, 3H), 6.82 (d, J = 6.3 Hz, 4H), 6.63 (dd, J = 8.1, 5.3 Hz, 2H), 6.49 (dd, J = 8.3, 2.3 Hz, 1H), 6.33 (d, J = 8.0 Hz, 2H), 5.10 (dd, J = 13.2, 5.0 Hz, 1H), 4.72 – 4.59 (m, 2H), 4.39 (d, J = 17.3 Hz, 1H), 4.22 (t, J = 10.9 Hz, 2H), 4.04 – 3.84 (m, 1H), 3.39 (ddd, J = 35.0, 16.9, 11.4 Hz, 6H), 3.22 (d, J = 27.1 Hz, 2H), 3.12 – 2.88 (m, 6H), 2.59 (d, J = 16.6 Hz, 1H), 2.49 – 2.34 (m, 3H), 2.29 – 2.03 (m, 5H), 2.01 – 1.83 (m, 5H), 1.80 – 1.48 (m, 5H) | 821.56 | General Synthesis Formula 44 |

| | | | | | |
|---|---|---|---|---|---|
| **368** | K52 | 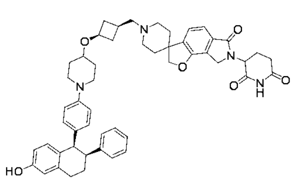 3-(1'-(((1R,3R)-3-((1-(4-((1R,2S)-6-hydroxy-2-phenyl-1,2,3,4-tetrahydronaphthalen-1-yl)phenyl)piperidin-4-yl)oxy)cyclobutyl)methyl)-6-oxo-6,8-dihydro-2H,7H-spiro[furo[2,3-e]isoindole-3,4'-piperidine]-7-yl)piperidine-2,6-dione | $^1$H NMR (400 MHz, DMSO-$d_6$) δ11.01 (s, 1H), 7.34 (t, *J* = 6.3 Hz, 1H), 7.27 (d, *J* = 7.6 Hz, 1H), 7.19 – 7.09 (m, 3H), 6.82 (d, *J* = 6.4 Hz, 2H), 6.73 (s, 2H), 6.63 (dd, *J* = 10.1, 5.3 Hz, 2H), 6.49 (dd, *J* = 8.3, 2.4 Hz, 1H), 6.29 (d, *J* = 7.9 Hz, 2H), 5.10 (dd, *J* = 13.3, 5.0 Hz, 1H), 4.69 – 4.60 (m, 2H), 4.39 (d, *J* = 17.3 Hz, 1H), 4.23 (d, *J* = 17.3 Hz, 1H), 4.18 (d, *J* = 4.7 Hz, 1H), 3.99 – 3.91 (m, 1H), 3.35 (ddd, *J* = 28.0, 23.8, 13.1 Hz, 6H), 3.19 (s, 2H), 3.10 – 2.80 (m, 6H), 2.59 (d, *J* = 16.5 Hz, 1H), 2.47 – 2.33 (m, 3H), 2.25 – 2.03 (m, 5H), 2.01 – 1.81 (m, 5H), 1.76 – 1.63 (m, 3H), 1.54 (s, 2H) | 821.56 | General Synthesis Formula 44 |
| **369** | L1 | 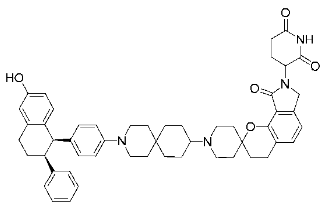 3-(1-(3-(4-((1R,2S)-6-hydroxy-2-phenyl-1,2,3,4-tetrahydronaphthalen-1-yl)phenyl)-3-azaspiro[5.5]undecan-9-yl)-9'-oxo-3',4',7'-tetrahydro-8'-H-spiro[piperidine-4,2'-pyrano[2,3-e]isoindole]-8'-yl)piperidine-2,6-dione | $^1$H NMR (600 MHz, DMSO-$d_6$) δ 10.98 (s, 1H), 8.99 (s, 1H)),7.44 (d, J = 8.1 Hz, 1H), 7.41 – 7.32 (m, 2H), 7.18 – 7.10 (m, 3H), 7.04 (d, J = 7.6 Hz, 1H), 6.84 (d, J = 7.4 Hz, 2H), 6.66 – 6.60 (m, 2H), 6.49 (dd, J = 8.3, 2.5 Hz, 1H), 6.31 (d, J = 8.3 Hz, 2H), 5.03 (dd, J = 13.2, 5.2 Hz, 1H), 4.93 (q, J = 7.8, 7.4 Hz, 1H), 4.55 – 4.39 (m, 1H), 4.38 – 4.27 (m, 1H), 4.26 – 4.17 (m, 2H), 3.25 – 3.04 (m, 7H), 3.01 – 2.79 (m, 5H), 2.41 – 2.31 (m, 1H), 2.13 – 1.99 (m, 4H), 1.99 – 1.77 (m, 8H), 1.76 – 1.59 (m, 5H), 1.51 – 1.43 (m, 2H), 1.38 (d, J = 7.0 Hz, 1H), 1.21 (dt, J = 25.8, 6.3 Hz, 3H) | 819.44 | General Synthesis Formula 6 |
| **370** | L2 | 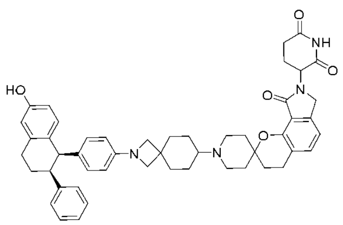 3-(1-(2-(4-((1R,2S)-6-hydroxy-2-phenyl-1,2,3,4-tetrahydronaphthalen-1-yl)phenyl)-2-azaspiro[3.5]nonan-7-yl)-9'-oxo-3',4',7'-tetrahydro-8'-H-spiro[piperidine-4,2'-pyrano[2,3-e]isoindole]-8'-yl)piperidine-2,6-dione | $^1$H NMR (600 MHz, DMSO-$d_6$) δ 10.97 (s, 1H), 9.12 (s, 1H), 7.35 (d, J = 7.7 Hz, 1H), 7.15 (dd, J = 8.0, 6.2 Hz, 2H), 7.13 – 7.10 (m, 1H), 7.04 (d, J = 7.6 Hz, 1H), 6.86 – 6.81 (m, 2H), 6.63 (d, J = 8.3 Hz, 1H), 6.60 (d, J = 2.5 Hz, 1H), 6.48 (dd, J = 8.3, 2.5 Hz, 1H), 6.18 (d, J = 8.2 Hz, 2H), 6.03 (d, J = 8.3 Hz, 2H), 5.04 (dd, J = 13.2, 5.2 Hz, 1H), 4.33 (d, J = 17.2 Hz, 1H), 4.21 (d, J = 17.3 Hz, 1H), 4.12 (d, J = 5.0 Hz, 1H), 3.02 – 2.81 (m, 6H), 2.59 (dt, J = 16.1, 3.3 Hz, 1H), 2.35 (tt, J = | 791.41 | General Synthesis Formula 6 |

| | | | | | |
|---|---|---|---|---|---|
| | | | 13.9, 6.7 Hz, 2H), 2.15 – 1.85 (m, 14H), 1.61 – 1.43 (m, 5H), 1.24 (d, J = 5.4 Hz, 6H) | | |
| **371** | L3 | 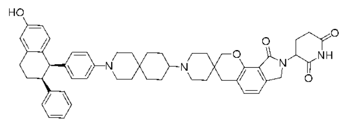 3-(1-(3-(4-((1R,2S)-6-hydroxy-2-phenyl-1,2,3,4-tetrahydronaphthalen-1-yl)phenyl)-3-azaspiro[5.5]undecan-9-yl)-9'-oxo-7',9'-dihydro-2'-H-spiro[piperidine-4,3'-pyrano[2,3-e]isoindole]-8'-(4'H)-yl)piperidine-2,6-dione | $^1$H NMR (600 MHz, DMSO-$d_6$) δ 10.98 (s, 1H), 9.07 (s, 1H), 7.30 (dd, J = 12.9, 7.7 Hz, 1H), 7.14 (dd, J = 11.7, 7.0 Hz, 3H), 7.05 (t, J = 8.2 Hz, 1H), 6.84 (d, J = 7.3 Hz, 4H), 6.66 – 6.61 (m, 2H), 6.51 – 6.47 (m, 1H), 6.35 (s, 2H), 5.02 (dd, J = 11.8, 5.4 Hz, 1H), 4.32 (d, J = 17.3 Hz, 1H), 4.23 (dd, J = 19.4, 8.0 Hz, 2H), 3.95 (s, 1H), 3.26 – 3.07 (m, 7H), 2.94-2,97 (m, 6H), 2.68 – 2.55 (m, 3H), 2.34 (td, J = 13.3, 4.7 Hz, 1H), 2.08 (q, J = 7.2, 5.9 Hz, 1H), 1.94 (ddt, J = 34.1, 13.4, 7.0 Hz, 4H), 1.60-1.80 (m, 10H), 1.48 (d, J = 22.9 Hz, 2H), 1.25 – 1.12 (m, 3H) | 819. 44 | General Synthesis Formula 6 |
| **372** | L4 | 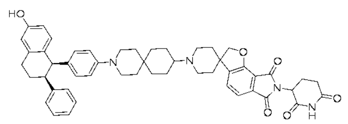 7-(2,6-dioxopiperidin-3-yl)-1'-((3-(4-(((1R,2S)-6-hydroxy-2-phenyl-1,2,3,4-tetrahydronaphthalen-1-yl)phenyl)-3-azaspiro[5.5]undecan-9-yl)-7-hydrogen-2H,6H-spiro[furo[2,3-e]isoindole-3,4'-piperidine]-6,8-dione | $^1$H NMR (600 MHz, DMSO-$d_6$) δ 11.14 (s, 1H), 9.43 (s, 1H), 7.58 (d, J = 7.4 Hz, 1H), 7.50 (d, J = 7.3 Hz, 1H), 7.14 (dq, J = 14.0, 7.2 Hz, 3H), 6.84 (d, J = 7.2 Hz, 4H), 6.66 – 6.60 (m, 2H), 6.49 (dd, J = 8.3, 2.5 Hz, 1H), 6.31 (s, 2H), 5.11 (dd, J = 12.9, 5.5 Hz, 1H), 4.81 (s, 2H), 4.20 (d, J = 5.0 Hz, 1H), 3.22 (s, 1H), 3.11-3.15 (m, 7H), 3.03 – 2.84 (m, 4H), 2.63 – 2.57 (m, 1H), 2.17 (dt, J = 14.3, 7.2 Hz, 2H), 2.11 – 1.98 (m, 4H), 1.94 – 1.82 (m, 3H), 1.72 (dd, J = 12.4, 6.2 Hz, 1H), 1.69 – 1.58 (m, 3H), 1.46 (dd, J = 14.1, 7.2 Hz, 2H), 1.24 (d, J = 5.2 Hz, 2H), 1.18 (t, J = 7.3 Hz, 4H) | 819. 40 | General Synthesis Formula 6 |
| **373** | L5 | 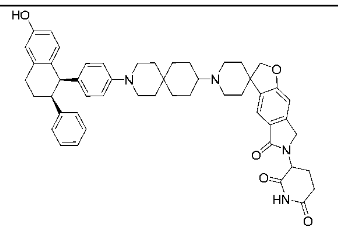 3-(1'-(3-(4-((1R,2S)-6-hydroxy-2-phenyl-1,2,3,4-tetrahydronaphthalen-1-yl)phenyl)-3-azaspiro[5.5]undecan-9-yl)-5-oxo-5,7-dihydro-2H,6H-spiro[furo[2,3-f]isoindole-3,4'- | $^1$H NMR (600 MHz, DMSO-$d_6$) δ 10.99 (s, 1H), 9.39 (s, 1H), 7.44 (s, 1H), 7.14 (dd, J = 12.0, 6.9 Hz, 4H), 7.04 (d, J = 6.5 Hz, 1H), 6.84 (d, J = 7.3 Hz, 3H), 6.66 – 6.61 (m, 2H), 6.49 (d, J = 8.3 Hz, 1H), 6.34 (d, J = 8.2 Hz, 2H), 5.04 (dd, J = 13.3, 5.2 Hz, 1H), 4.68 – 4.61 (m, 2H), 4.36 (d, J = 17.3 Hz, 1H), 4.23 (dd, J = 17.6, 5.6 Hz, 2H), 3.23 – 3.05 (m, 8H), 3.01 – 2.86 (m, 4H), 2.59 (d, J = 17.0 Hz, | 805. 43 | General Synthesis Formula 6 |

| | | | | | |
|---|---|---|---|---|---|
| | | piperidine]-6-yl)piperidine-2,6-dione | 1H), 2.37 (td, J = 13.2, 4.6 Hz, 1H), 2.18 (d, J = 13.8 Hz, 2H), 2.08 (dq, J = 13.1, 6.7 Hz, 1H), 1.99 (ddd, J = 18.3, 13.6, 6.6 Hz, 4H), 1.94 – 1.80 (m, 4H), 1.78 – 1.58 (m, 6H), 1.56 – 1.43 (m, 3H) | | |
| **374** | L6 | 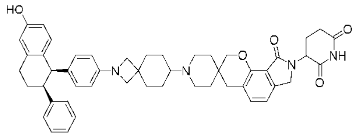 3-(1-(2-(4-((1R,2S)-6-hydroxy-2-phenyl-1,2,3,4-tetrahydronaphthalen-1-yl)phenyl)-2-azaspiro[3.5]nonan-7-yl)-9'-oxo-7',9'-dihydro-2'-H-spiro[piperidine-4,3'-pyrano[2,3-e]isoindole]-8'-(4'H)-yl)piperidine-2,6-dione | $^1$H NMR (600 MHz, DMSO-$d_6$) δ 10.98 (s, 1H), 9.15 (s, 1H), 7.30 (s, 1H), 7.19 – 7.09 (m, 3H), 7.05 (s, 1H), 6.86 – 6.80 (m, 2H), 6.65 – 6.57 (m, 2H), 6.48 (dd, J = 8.3, 2.5 Hz, 1H), 6.17 (d, J = 8.1 Hz, 2H), 6.03 (d, J = 8.1 Hz, 2H), 5.02 (dd, J = 13.3, 5.2 Hz, 1H), 4.32 (d, J = 17.3 Hz, 1H), 4.23 (s, 1H), 4.18 (d, J = 17.2 Hz, 1H), 4.11 (d, J = 4.9 Hz, 1H), 3.94 (s, 1H), 3.02 – 2.86 (m, 5H), 2.66 – 2.56 (m, 3H), 2.55 (s, 2H), 2.35 (dd, J = 13.3, 4.7 Hz, 1H), 2.09 (dd, J = 11.8, 5.5 Hz, 1H), 2.06 – 1.92 (m, 6H), 1.68-1.72 (m, 6H), 1.55 – 1.41 (m, 4H), 1.24 (s, 4H) | 791.41 | General Synthesis Formula 6 |
| **375** | L7 | 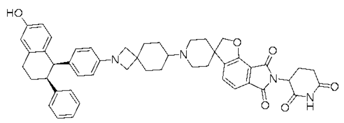 7-(2,6-dioxopiperidin-3-yl)-1'-((2-(4-(((1R,2S)-6-hydroxy-2-phenyl-1,2,3,4-tetrahydronaphthalen-1-yl)phenyl)-2-azaspiro[3.5]nonan-7-yl)-7-hydrogen-2H,6H-spiro[furo[2,3-e]isoindole-3,4'-piperidine]-6,8-dione | $^1$H NMR (600 MHz, DMSO-$d_6$) δ 11.14 (s, 1H), 9.14 (s, 1H), 7.57 (d, J = 7.4 Hz, 1H), 7.50 (d, J = 7.3 Hz, 1H), 7.13 (dq, J = 14.3, 7.3 Hz, 3H), 6.83 (d, J = 7.4 Hz, 2H), 6.65 – 6.58 (m, 2H), 6.48 (dd, J = 8.3, 2.5 Hz, 1H), 6.18 (d, J = 8.1 Hz, 2H), 6.04 (d, J = 8.2 Hz, 2H), 5.11 (dd, J = 12.9, 5.4 Hz, 1H), 4.81 (s, 1H), 4.12 (d, J = 5.0 Hz, 1H), 3.37 (s, 4H), 3.25 (s, 3H), 3.11 (d, J = 11.9 Hz, 2H), 3.01 – 2.84 (m, 3H), 2.64 – 2.57 (m, 1H), 2.20 – 1.96 (m, 10H), 1.70 (dd, J = 12.4, 6.1 Hz, 1H), 1.59 – 1.42 (m, 4H), 1.24 (d, J = 5.4 Hz, 3H) | 791.37 | General Synthesis Formula 6 |
| **376** | L8 | 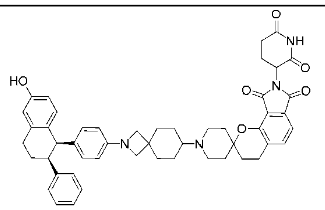 8'-(2,6-dioxopiperidin-3-yl)-1-(2-(4-((1R,2S)-6-hydroxy-2-phenyl-1,2,3,4-tetrahydronaphthalen-1-yl)phenyl)-2-azaspiro[3.5]nonan-7-yl)-3',4'-dihydro-7-pyrido[4,2'-pyrano[2,3-e]isoindole]- | $^1$H NMR (600 MHz, DMSO-$d_6$) δ 11.12 (s, 1H), 9.16 (s, 1H), 7.62 (d, J = 7.5 Hz, 1H), 7.39 (d, J = 7.4 Hz, 1H), 7.14 (dt, J = 16.1, 7.2 Hz, 3H), 6.83 (d, J = 7.4 Hz, 2H), 6.65 – 6.58 (m, 2H), 6.48 (dd, J = 8.4, 2.5 Hz, 1H), 6.17 (d, J = 8.0 Hz, 2H), 6.03 (d, J = 8.2 Hz, 2H), 5.09 (dd, J = 12.8, 5.5 Hz, 1H), 4.11 (d, J = 5.0 Hz, 1H), 3.23 (s, 3H), 2.88-2.92 (m, 7H), 2.15 – 1.92 (m, 14H), 1.70 (dd, J = 12.6, 6.1 | 805.39 | General Synthesis Formula 6 |

| | | | | | |
|---|---|---|---|---|---|
| | | 7',9'-dione | Hz, 1H), 1.57 – 1.50 (m, 4H), 1.34 – 1.19 (m, 5H) | | |
| **377** | L9 | 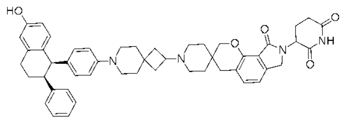 3-(1-(7-(4-((1R,2S)-6-hydroxy-2-phenyl-1,2,3,4-tetrahydronaphthalen-1-yl)phenyl)-7-azaspiro[3.5]nonan-2-yl)-9'-oxo-7',9'-dihydro-2'H-spiro[piperidine-4,3'-pyrano[2,3-e]isoindole]-8'-(4'H)-yl)piperidine-2,6-dione | $^1$H NMR (400 MHz, DMSO-$d_6$) δ 10.99 (s, 1H), 9.17 (s, 1H), 8.25 – 8.03 (m, 1H), 7.92 – 7.72 (m, 1H), 7.34 – 7.26 (m, 1H), 7.18 – 7.09 (m, 2H), 7.08 – 7.01 (m, 1H), 6.88 – 6.78 (m, 2H), 6.69 (s, 1H), 6.66 – 6.57 (m, 2H), 6.48 (dd, J = 8.3, 2.5 Hz, 1H), 6.27 (d, J = 8.0 Hz, 2H), 5.02 (dd, J = 12.9, 5.1 Hz, 1H), 4.39 – 3.87 (m, 5H), 3.87 – 3.60 (m, 4H), 3.54 – 3.41 (m, 2H), 3.41 – 3.21 (m, 3H), 3.18 – 2.84 (m, 8H), 2.71 – 2.52 (m, 2H), 2.45 – 2.29 (m, 1H), 2.19 (s, 2H), 2.07 – 1.89 (m, 3H), 1.85 – 1.56 (m, 4H), 1.39 (s, 1H), 1.33 – 1.14 (m, 2H) | 791.41 | General Synthesis Formula 6 |
| **378** | L10 | 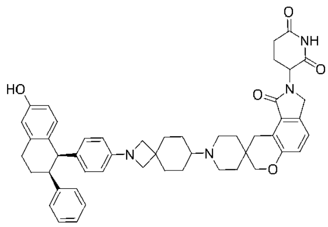 3-(1-(2-(4-(((1R,2S)-6-hydroxy-2-phenyl-1,2,3,4-tetrahydronaphthalen-1-yl)phenyl)-2-azaspiro[3.5]nonan-7-yl)-1'-oxo-1',9'-dihydro-7'H-pyrido[piperidine-4,8"-pyrano[3,2-e]isoindole]-2'(3'H)-yl)piperidine-2,6-dione | $^1$H NMR (600 MHz, DMSO-$d_6$) δ 11.02 (s, 1H), 9.05 (s, 1H), 7.33 (dd, J = 8.3, 4.7 Hz, 1H), 7.13 (dq, J = 14.3, 7.3 Hz, 3H), 7.06 (dd, J = 8.4, 2.3 Hz, 1H), 6.83 (d, J = 7.3 Hz, 2H), 6.65 – 6.58 (m, 2H), 6.48 (dd, J = 8.2, 2.5 Hz, 1H), 6.18 (dd, J = 8.4, 3.3 Hz, 2H), 6.04 (dd, J = 8.3, 4.6 Hz, 2H), 5.01 (dd, J = 12.9, 5.1 Hz, 1H), 4.35 – 4.07 (m, 5H), 3.89 (s, 1H), 3.42 (t, J = 7.9 Hz, 4H), 3.34 – 3.20 (m, 3H), 3.22 – 3.11 (m, 2H), 3.10 – 2.80 (m, 5H), 2.60 (dd, J = 17.5, 3.5 Hz, 1H), 2.45 – 2.34 (m, 1H), 2.15 – 1.92 (m, 6H), 1.83 – 1.65 (m, 5H), 1.58 – 1.38 (m, 4H) | 791.41 | General Synthesis Formula 6 |
| **379** | L11 | 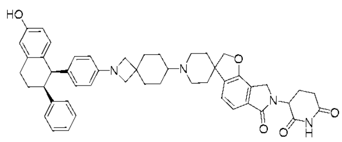 3-(1'-(2-(4-((1R,2S)-6-hydroxy-2-phenyl-1,2,3,4-tetrahydronaphthalen-1-yl)phenyl)-2-azaspiro[3.5]nonan-7-yl)-6-oxo-6,8-dihydro-2H,7H-spiro[furo[2,3-e]isoindole-3,4'-piperidine]-7-yl)piperidine-2,6-dione | $^1$H NMR (400 MHz, DMSO-$d_6$) δ 11.00 (s, 1H), 9.31 (s, 1H), 7.35 (d, J = 7.6 Hz, 1H), 7.28 (d, J = 7.6 Hz, 1H), 7.19 – 7.09 (m, 3H), 6.87 – 6.80 (m, 2H), 6.66 – 6.58 (m, 2H), 6.48 (dd, J = 8.3, 2.5 Hz, 1H), 6.18 (d, J = 8.1 Hz, 2H), 6.04 (d, J = 8.1 Hz, 2H), 5.10 (dd, J = 13.3, 5.1 Hz, 1H), 4.67 (s, 1H), 4.40 (d, J = 17.3 Hz, 1H), 4.23 (d, J = 17.2 Hz, 1H), 4.12 (d, J = 4.9 Hz, 1H), 3.37 (s, 2H), 3.10 (d, J = 12.1 Hz, 2H), 3.01 – 2.85 (m, 4H), 2.69 – 2.54 (m, 2H), 2.43 (dd, J = 13.2, 4.7 Hz, 1H), | 777.39 | General Synthesis Formula 6 |

| | | | | | |
|---|---|---|---|---|---|
| | | | 2.23 – 2.06 (m, 3H), 1.98-2.02 (m, 9H), 1.70 (dd, J = 12.4, 5.5 Hz, 1H), 1.49 (dd, J = 25.1, 12.6 Hz, 4H), 1.24 (s, 3H) | | |
| **380** | L12 | 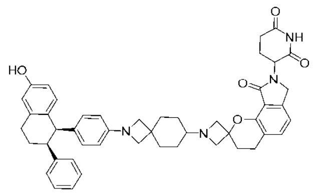 3-(1-(2-(4-((1R,2S)-6-hydroxy-2-phenyl-1,2,3,4-tetrahydronaphthalen-1-yl)phenyl)-2-azaspiro[3.5]nonan-7-yl)-9'-oxo-3',4',7'-tetrahydro-8'H-spiro[azetidine-3,2'-pyrano[2,3-e]isoindole]-8'-yl)piperidine-2,6-dione | $^1$H NMR (600 MHz, DMSO-$d_6$) δ 10.98 (d, J = 3.4 Hz, 1H), 9.09 (s, 1H), 7.36 (dd, J = 7.8, 4.4 Hz, 1H), 7.17 – 7.07 (m, 4H), 6.85 – 6.81 (m, 2H), 6.64 – 6.58 (m, 2H), 6.48 (dd, J = 8.3, 2.5 Hz, 1H), 6.17 (d, J = 8.3 Hz, 2H), 6.04 (d, J = 8.3 Hz, 2H), 4.97 (dd, J = 13.3, 5.1 Hz, 1H), 4.43 (dt, J = 10.8, 4.7 Hz, 1H), 4.39 – 4.18 (m, 5H), 4.11 (d, J = 5.0 Hz, 1H), 3.08-3.12 (m, 6H), 3.00 – 2.79 (m, 5H), 2.63 – 2.57 (m, 1H), 2.43 – 2.34 (m, 2H), 2.20 (dt, J = 18.3, 7.3 Hz, 2H), 2.14 – 2.05 (m, 2H), 2.02 – 1.91 (m, 4H), 1.85 (d, J = 11.7 Hz, 1H), 1.70 (dd, J = 12.4, 6.2 Hz, 1H), 1.45 (dq, J = 30.0, 16.9, 14.8 Hz, 2H) | 763.38 | General Synthesis Formula 6 |
| **381** | L13 | 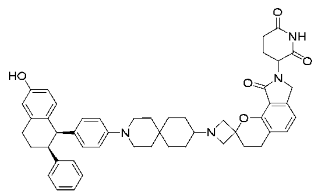 3-(1-(3-(4-((1R,2S)-6-hydroxy-2-phenyl-1,2,3,4-tetrahydronaphthalen-1-yl)phenyl)-3-azaspiro[5.5]undecan-9-yl)-9'-oxo-3',4',7'-tetrahydro-8'H-spiro[azetidine-3,2'-pyrano[2,3-e]isoindole]-8'-yl)piperidine-2,6-dione | $^1$H NMR (600 MHz, DMSO-$d_6$) δ 10.98 (s, 1H), 9.15 (s, 1H), 7.36 (dd, J = 7.9, 3.2 Hz, 1H), 7.17 (d, J = 11.5 Hz, 1H), 7.16 – 7.12 (m, 2H), 7.10 (dt, J = 7.7, 4.7 Hz, 1H), 6.83 (d, J = 7.3 Hz, 2H), 6.70 (d, J = 40.3 Hz, 1H), 6.65 – 6.59 (m, 3H), 6.48 (dd, J = 8.3, 2.5 Hz, 1H), 6.26 (s, 2H), 4.96 (dd, J = 13.3, 5.1 Hz, 1H), 4.43 (dd, J = 12.5, 5.9 Hz, 1H), 4.35 (dt, J = 19.9, 10.0 Hz, 2H), 4.28 – 4.14 (m, 3H), 3.11 – 2.79 (m, 8H), 2.60 (d, J = 14.8 Hz, 1H), 2.43 – 2.36 (m, 2H), 2.25 – 2.14 (m, 2H), 2.09 (h, J = 11.7 Hz, 1H), 2.03 – 1.91 (m, 2H), 1.85 (s, 1H), 1.74 (d, J = 13.7 Hz, 3H), 1.54 (s, 2H), 1.41 (d, J = 23.4 Hz, 3H), 1.37 – 1.21 (m, 4H), 1.18 – 1.02 (m, 2H) | 791.41 | General Synthesis Formula 6 |
| **382** | L14 | 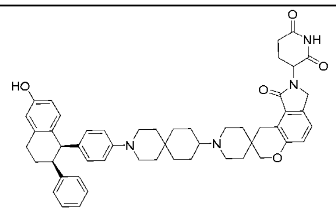 3-(1-(3-(4-((1R,2S)-6-hydroxy-2-phenyl-1,2,3,4-tetrahydronaphthalen-1- | $^1$H NMR (600 MHz, DMSO-$d_6$) δ 11.00 (d, J = 3.4 Hz, 1H), 9.17 (s, 1H), 7.33 (dd, J = 8.3, 4.6 Hz, 1H), 7.14 (dt, J = 12.5, 6.7 Hz, 2H), 7.06 (dd, J = 8.2, 3.0 Hz, 1H), 6.84 (d, J = 7.3 Hz, 1H), 6.74 (s, 2H), 6.66 – 6.60 (m, 5H), 6.49 (dd, J = 8.3, 2.5 Hz, 1H), 6.29 (d, J = 8.4 Hz, 1H), 5.01 (dd, J | 819.44 | General Synthesis Formula 6 |

| | | | | | |
|---|---|---|---|---|---|
| | | yl)phenyl)-3-azaspiro[5.5]undecan-9-yl)-1'-oxo-1',9'-dihydro-7'H-spiro[piperidine-4,8'-pyrano[3,2-e]isoindole]-2'(3'H)-yl)piperidine-2,6-dione | = 13.3, 5.1 Hz, 1H), 4.32 (dd, J = 17.0, 6.3 Hz, 1H), 4.25 – 4.13 (m, 3H), 3.90 (d, J = 3.2 Hz, 1H), 3.25 (s, 1H), 3.17 (d, J = 12.5 Hz, 2H), 3.06 (d, J = 16.0 Hz, 3H), 2.99 (dd, J = 18.2, 7.7 Hz, 2H), 2.96 – 2.85 (m, 2H), 2.64 – 2.58 (m, 2H), 2.43 – 2.33 (m, 2H), 2.14 – 2.03 (m, 2H), 2.01 – 1.95 (m, 2H), 1.93 – 1.87 (m, 3H), 1.85 – 1.76 (m, 3H), 1.70 (qt, J = 14.2, 7.2 Hz, 3H), 1.59 (q, J = 14.4, 12.1 Hz, 3H), 1.49 – 1.40 (m, 2H), 1.23 (q, J = 6.3 Hz, 2H), 1.21 – 1.04 (m, 2H) | | |
| **383** | L15 | 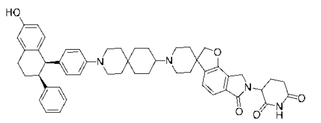 3-(1'-(3-(4-((1R,2S)-6-hydroxy-2-phenyl-1,2,3,4-tetrahydronaphthalen-1-yl)phenyl)-3-azaspiro[5.5]undecan-9-yl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 11.00 (s, 1H), 9.17 (s, 1H), 7.35 (d, J = 7.5 Hz, 1H), 7.32 – 7.25 (m, 1H), 7.14 (qd, J = 14.9, 13.3, 7.7 Hz, 3H), 6.84 (d, J = 7.3 Hz, 2H), 6.64 (d, J = 8.4 Hz, 1H), 6.63 – 6.53 (m, 3H), 6.48 (dd, J = 8.3, 2.5 Hz, 1H), 6.22 (d, J = 8.1 Hz, 2H), 5.10 (dd, J = 13.3, 5.1 Hz, 1H), 4.70 – 4.63 (m, 2H), 4.40 (d, J = 17.2 Hz, 1H), 4.24 (d, J = 17.2 Hz, 1H), 4.14 (d, J = 5.0 Hz, 1H), 3.09 (d, J = 11.9 Hz, 2H), 3.05 – 2.87 (m, 6H), 2.64 – 2.56 (m, 1H), 2.43 (dd, J = 13.2, 4.7 Hz, 1H), 2.28 (td, J = 14.1, 4.1 Hz, 2H), 2.10 (ddt, J = 18.7, 12.4, 7.4 Hz, 1H), 2.05 – 1.87 (m, 6H), 1.86 – 1.75 (m, 2H), 1.74 – 1.53 (m, 5H), 1.48 – 1.36 (m, 3H), 1.32 – 1.20 (m, 3H), 1.20 – 1.10 (m, 2H) | 805.43 | General Synthesis Formula 6 |
| **384** | L16 | 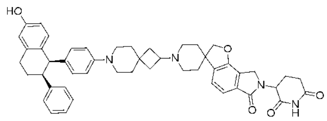 3-(1-(7-(4-((1R,2S)-6-hydroxy-2-phenyl-1,2,3,4-tetrahydronaphthalen-1-yl)phenyl)-7-azaspiro[3.5]nonan-2-yl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 11.00 (s, 1H), 9.17 (s, 1H), 7.37 – 7.24 (m, 2H), 7.19 – 7.08 (m, 3H), 6.86 – 6.81 (m, 2H), 6.64 (d, J = 8.4 Hz, 1H), 6.61 (d, J = 2.6 Hz, 1H), 6.58 – 6.52 (m, 2H), 6.48 (dd, J = 8.3, 2.6 Hz, 1H), 6.24 – 6.17 (m, 2H), 5.10 (dd, J = 13.3, 5.2 Hz, 1H), 4.62 (s, 2H), 4.39 (d, J = 17.1 Hz, 1H), 4.23 (d, J = 17.1 Hz, 1H), 4.13 (d, J = 5.0 Hz, 1H), 2.94 (dq, J = 41.6, 6.8, 6.3 Hz, 8H), 2.63 – 2.56 (m, 3H), 2.44 (td, J = 13.2, 4.6 Hz, 1H), 2.27 – 1.81 (m, 11H), 1.75 – 1.56 (m, 5H), 1.24 (d, J = 5.0 Hz, 2H) | 777.39 | General Synthesis Formula 6 |

| | | | | | |
|---|---|---|---|---|---|
| **385** | L17 | 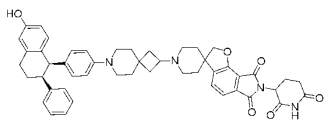 7-(2,6-dioxopiperidin-3-yl)-1'-(7-(4-(((1R,2S)-6-hydroxy-2-phenyl-1,2,3,4-tetrahydronaphthalen-1-yl)phenyl)-7-azaspiro[3.5]nonan-2-yl)-7-hydrogen-2H,6H-spiro[furo[2,3-e]isoindole-3,4'-piperidine]-6,8-dione | $^1$H NMR (600 MHz, DMSO-$d_6$) δ 11.12 (s, 1H), 9.16 (s, 1H), 7.47 (s, 2H), 7.19 – 7.09 (m, 3H), 6.87 – 6.81 (m, 2H), 6.64 (d, J = 8.4 Hz, 1H), 6.60 (d, J = 2.5 Hz, 1H), 6.54 (d, J = 8.3 Hz, 2H), 6.48 (dd, J = 8.3, 2.6 Hz, 1H), 6.20 (d, J = 8.3 Hz, 2H), 5.10 (dd, J = 12.9, 5.4 Hz, 1H), 4.73 (s, 2H), 4.13 (d, J = 5.0 Hz, 1H), 3.02 – 2.83 (m, 7H), 2.63 – 2.57 (m, 4H), 2.19 – 1.93 (m, 7H), 1.70 (d, J = 5.6 Hz, 2H), 1.60 (s, 4H), 1.32 – 1.21 (m, 4H), 1.18 (t, J = 7.3 Hz, 1H), 0.86 (t, J = 7.0 Hz, 1H) | 791. 37 | General Synthesis Formula 6 |
| **386** | L18 | 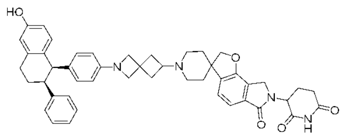 3-(1'-(2-(4-((1R,2S)-6-hydroxy-2-phenyl-1,2,3,4-tetrahydronaphthalen-1-yl)phenyl)-2-azaspiro[3.3]heptan-6-yl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 11.00 (s, 1H), 9.15 (s, 1H), 7.36 (d, J = 7.6 Hz, 1H), 7.32 – 7.27 (m, 1H), 7.18 – 7.10 (m, 3H), 6.85 – 6.78 (m, 2H), 6.64 – 6.58 (m, 2H), 6.48 (dd, J = 8.3, 2.5 Hz, 1H), 6.18 (d, J = 8.0 Hz, 2H), 6.07 (t, J = 6.4 Hz, 2H), 5.10 (dd, J = 12.9, 5.4 Hz, 1H), 4.68 – 4.61 (m, 1H), 4.40 (d, J = 17.1 Hz, 1H), 4.24 (d, J = 17.1 Hz, 1H), 4.12 (d, J = 4.9 Hz, 1H), 3.74 (d, J = 4.1 Hz, 2H), 3.66 (d, J = 2.7 Hz, 3H), 3.03 – 2.84 (m, 3H), 2.63 – 2.57 (m, 4H), 2.46 – 2.38 (m, 3H), 2.14 – 1.93 (m, 6H), 1.70 (dd, J = 12.8, 6.1 Hz, 1H), 1.49 – 1.42 (m, 1H), 1.32 – 1.20 (m, 3H), 0.86 (t, J = 6.9 Hz, 1H) | 749. 36 | General Synthesis Formula 6 |
| **387** | L19 | 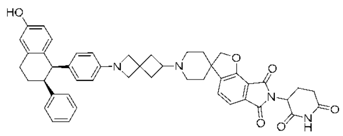 7-(2,6-dioxopiperidin-3-yl)-1'-((2-(4-(((1R,2S)-6-hydroxy-2-phenyl-1,2,3,4-tetrahydronaphthalen-1-yl)phenyl)-2-azaspiro[3.3]heptan-6-yl)-7-hydrogen-2H,6H-spiro[furo[2,3-e]isoindole-3,4'-piperidine]-6,8-dione | $^1$H NMR (600 MHz, DMSO-$d_6$) δ 11.12 (s, 1H), 9.14 (s, 1H), 7.57 (d, J = 7.4 Hz, 1H), 7.50 (d, J = 7.4 Hz, 1H), 7.19 – 7.10 (m, 4H), 6.84 (dd, J = 16.7, 7.5 Hz, 2H), 6.67 – 6.58 (m, 2H), 6.48 (dd, J = 8.3, 2.6 Hz, 1H), 6.18 (d, J = 8.0 Hz, 1H), 6.14 – 6.03 (m, 2H), 5.11 (dd, J = 12.9, 5.4 Hz, 1H), 4.78 (d, J = 6.1 Hz, 1H), 4.10 (dd, J = 22.8, 5.2 Hz, 2H), 3.79 – 3.56 (m, 7H), 3.45 – 3.39 (m, 2H), 3.28 (d, J = 13.7 Hz, 2H), 3.04 – 2.83 (m, 5H), 2.63 – 2.57 (m, 1H), 2.26 – 1.94 (m, 6H), 1.70 (dd, J = 13.1, 6.2 Hz, 1H), 1.24 (d, J = 6.0 Hz, 2H) | 763. 34 | General Synthesis Formula 6 |

| | | | | | |
|---|---|---|---|---|---|
| **388** | L20 | 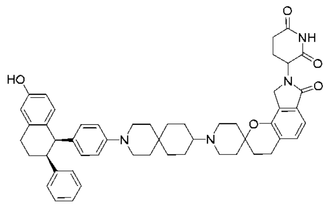 3-(1-(3-(4-((1R,2S)-6-hydroxy-2-phenyl-1,2,3,4-tetrahydronaphthalen-1-yl)phenyl)-3-azaspiro[5.5]undecan-9-yl)-7'-oxo-3',4',7'-tetrahydro-8'H-spiro[piperidine-4,2'-pyrano[2,3-e]isoindole]-8'-yl)piperidine-2,6-dione | $^1$H NMR (600 MHz, DMSO-$d_6$) δ 11.03 (s, 1H), 9.17 (s, 1H), 7.29 (d, J = 7.7 Hz, 1H), 7.24 (d, J = 7.6 Hz, 1H), 7.18 – 7.10 (m, 3H), 6.86 – 6.81 (m, 3H), 6.68 (s, 1H), 6.66 – 6.59 (m, 2H), 6.49 (dd, J = 8.3, 2.5 Hz, 1H), 6.29 (s, 2H), 5.11 (dd, J = 13.3, 5.2 Hz, 1H), 4.42 (d, J = 17.3 Hz, 1H), 4.27 (d, J = 17.4 Hz, 1H), 4.22 – 4.16 (m, 1H), 3.47 (t, J = 12.0 Hz, 2H), 3.32 (d, J = 13.7 Hz, 2H), 3.19 (t, J = 13.8 Hz, 3H), 3.08 (s, 3H), 3.02 – 2.85 (m, 5H), 2.62 (d, J = 16.8 Hz, 1H), 2.43 – 2.35 (m, 1H), 2.14 – 2.06 (m, 1H), 2.06 – 1.97 (m, 3H), 1.98 – 1.86 (m, 6H), 1.86 – 1.77 (m, 2H), 1.77 – 1.70 (m, 1H), 1.63 (s, 4H), 1.54 – 1.42 (m, 2H), 1.28 – 1.09 (m, 2H) | 819.44 | General Synthesis Formula 6 |
| **389** | L21 | 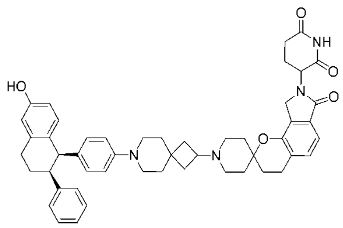 3-(1-(7-(4-((1R,2S)-6-hydroxy-2-phenyl-1,2,3,4-tetrahydronaphthalen-1-yl)phenyl)-7-azaspiro[3.5]nonan-2-yl)-7'-oxo-3',4'-,7'-tetrahydro-8'H-spiro[piperidine-4,2'-pyrano[2,3-e]isoindole]-8'-yl)piperidine-2,6-dione | $^1$H NMR (600 MHz, DMSO-$d_6$) δ 11.05 (s, 1H), 9.16 (s, 1H), 7.29 (d, J = 7.7 Hz, 1H), 7.24 (d, J = 7.6 Hz, 1H), 7.18 – 7.10 (m, 3H), 6.86 – 6.81 (m, 2H), 6.73 – 6.59 (m, 4H), 6.48 (dd, J = 8.3, 2.5 Hz, 1H), 6.26 (s, 2H), 5.14 (dd, J = 13.4, 5.2 Hz, 1H), 4.42 (d, J = 17.4 Hz, 1H), 4.28 (s, 1H), 4.17 (d, J = 5.1 Hz, 2H), 3.81 (h, J = 8.4 Hz, 3H), 3.11 – 2.85 (m, 7H), 2.69 – 2.58 (m, 1H), 2.34 (qd, J = 13.1, 4.3 Hz, 2H), 2.20 (t, J = 7.1 Hz, 2H), 2.14 – 1.79 (m, 8H), 1.68 (dd, J = 36.5, 18.1 Hz, 5H), 1.45 (dd, J = 15.1, 8.3 Hz, 1H), 1.39 (d, J = 1.6 Hz, 1H), 1.24 (d, J = 5.2 Hz, 3H) | 791.41 | General Synthesis Formula 6 |
| **390** | L22 | 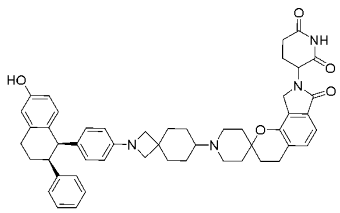 3-(1-(2-(4-((1R,2S)-6-hydroxy-2-phenyl-1,2,3,4-tetrahydronaphthalen-1-yl)phenyl)-2-azaspiro[3.5]nonan-7-yl)-7'-oxo-3',4',7'-tetrahydro-8'H-spiro[piperidine-4,2'-pyrano[2,3-e]isoindole]-8'-yl)piperidine-2,6-dione | $^1$H NMR (600 MHz, DMSO-$d_6$) δ 11.03 (s, 1H), 9.19 (s, 1H), 7.29 (d, J = 7.7 Hz, 1H), 7.24 (d, J = 7.7 Hz, 1H), 7.19 – 7.10 (m, 3H), 6.86 – 6.81 (m, 2H), 6.65 – 6.58 (m, 2H), 6.48 (dd, J = 8.3, 2.5 Hz, 1H), 6.18 (d, J = 8.2 Hz, 2H), 6.04 (d, J = 8.0 Hz, 2H), 5.11 (dd, J = 13.3, 5.2 Hz, 1H), 4.41 (d, J = 17.4 Hz, 1H), 4.27 (d, J = 17.4 Hz, 1H), 4.12 (d, J = 5.0 Hz, 1H), 3.48 – 3.40 (m, 3H), 3.37 (s, 2H), 3.31 – 3.13 (m, 4H), 3.00 – 2.84 (m, 4H), 2.62 (dd, J = 17.2, 3.8 Hz, 1H), 2.41 – 2.34 (m, 1H), | 791.41 | General Synthesis Formula 6 |

| | | | | | |
|---|---|---|---|---|---|
| | | | 2.14 – 1.96 (m, 7H), 1.96 – 1.84 (m, 4H), 1.70 (dd, J = 12.2, 6.1 Hz, 1H), 1.58 – 1.40 (m, 5H), 1.24 (d, J = 4.6 Hz, 2H) | | |
| **391** | L23 | 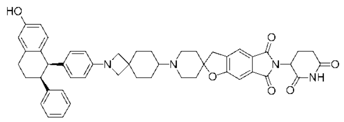 6-(2,6-dioxopiperidin-3-yl)-1'-(2-(4-(((1R,2S)-6-hydroxy-2-phenyl-1,2,3,4-tetrahydronaphthalen-1-yl)phenyl)-2-azaspiro[3.5]nonan-7-yl)spiro[furo[2,3-f]isoindole-2,4'-piperidine]-5,7(3H,6H)-dione | $^1$H NMR (600 MHz, DMSO-$d_6$) δ 11.11 (s, 1H), 9.10 (s, 1H),7.81 (s, 1H), 7.26 (d, J = 43.5 Hz, 1H), 7.18 – 7.10 (m, 3H), 6.87 – 6.81 (m, 2H), 6.65 – 6.58 (m, 2H), 6.48 (dd, J = 8.3, 2.5 Hz, 1H), 6.19 (d, J = 8.1 Hz, 2H), 6.04 (dd, J = 8.4, 4.0 Hz, 2H), 5.10 (dd, J = 13.3, 5.2 Hz, 1H), 4.12 (d, J = 5.0 Hz, 1H), 3.55 (d, J = 11.8 Hz, 2H), 3.52 – 3.42 (m, 4H), 3.37 (s, 3H), 3.28 (d, J = 14.8 Hz, 3H), 3.26 – 3.16 (m, 3H), 3.01 – 2.85 (m, 3H), 2.64 – 2.54 (m, 1H), 2.24 – 1.96 (m, 8H), 1.71 (d, J = 5.9 Hz, 1H), 1.52 (d, J = 9.7 Hz, 4H) | 791.37 | General Synthesis Formula 6 |
| **392** | L24 | 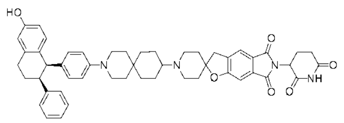 6-(2,6-dioxopiperidin-3-yl)-1'-(3-(4-(((1R,2S)-6-hydroxy-2-phenyl-1,2,3,4-tetrahydronaphthalen-1-yl)phenyl)-3-azaspiro[5.5]undecan-9-yl)spiro[furo[2,3-f]isoindole-2,4'-piperidine]-5,7(3H,6H)-dione | $^1$H NMR (600 MHz, DMSO-$d_6$) δ 11.11 (s, 1H), 9.14 (s, 1H), 7.81 (s, 1H), 7.30 (s, 1H), 7.22 (s, 1H), 7.19 – 7.10 (m, 3H), 6.87 – 6.81 (m, 2H), 6.71 (s, 1H), 6.67 – 6.60 (m, 2H), 6.49 (dd, J = 8.2, 2.6 Hz, 1H), 6.29 (s, 2H), 5.10 (dd, J = 13.3, 5.2 Hz, 1H), 4.19 (s, 1H), 3.37 (s, 1H), 3.36 – 3.17 (m, 7H), 3.16 – 2.85 (m, 8H), 2.60 (d, J = 14.1 Hz, 1H), 2.26 – 2.00 (m, 7H), 1.94 – 1.86 (m, 2H), 1.73 (d, J = 5.8 Hz, 1H), 1.69 – 1.55 (m, 4H), 1.45 (s, 2H), 1.27 – 1.12 (m, 3H) | 819.40 | General Synthesis Formula 6 |
| **393** | L25 | 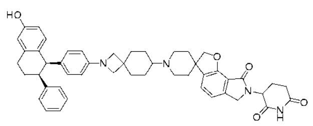 3-(1'-(2-(4-((1R,2S)-6-hydroxy-2-phenyl-1,2,3,4-tetrahydronaphthalen-1-yl)phenyl)-2-azaspiro[3.5]nonan-7-yl)-8-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.98 (s, 1H), 9.12 (s, 1H), 7.32 (d, J = 7.5 Hz, 1H), 7.20 – 7.06 (m, 4H), 6.84 (d, J = 7.4 Hz, 2H), 6.66 – 6.58 (m, 2H), 6.48 (dd, J = 8.2, 2.5 Hz, 1H), 6.19 (d, J = 8.1 Hz, 2H), 6.04 (d, J = 8.1 Hz, 2H), 5.05 (dd, J = 13.3, 5.1 Hz, 1H), 4.67 (s, 2H), 4.41 (d, J = 17.4 Hz, 1H), 4.27 (d, J = 17.3 Hz, 1H), 4.12 (d, J = 5.0 Hz, 1H), 3.50 (d, J = 12.0 Hz, 2H), 3.48 – 3.42 (m, 2H), 3.37 (s, 2H), 3.31 – 3.22 (m, 2H), 3.11 (d, J = 11.8 Hz, 2H), 3.02 – 2.87 (m, 3H), 2.59 (dd, J = 17.9, 4.2 Hz, 1H), 2.35 (td, J = 13.2, 4.6 Hz, 1H), 2.21 – 2.06 (m, 4H), | 777.39 | General Synthesis Formula 6 |

| | | | | | |
|---|---|---|---|---|---|
| | | | 2.06 – 1.91 (m, 6H), 1.74 – 1.67 (m, 1H), 1.59 – 1.43 (m, 4H) | | |
| **394** | L26 | 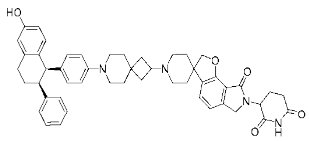 3-(1'-(7-(4-((1R,2S)-6-hydroxy-2-phenyl-1,2,3,4-tetrahydronaphthalen-1-yl)phenyl)-7-azaspiro[3.5]nonan-2-yl)-8-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.98 (s, 1H), 9.14 (s, 1H), 7.34 (d, J = 7.6 Hz, 1H), 7.22 – 7.05 (m, 4H), 6.88 – 6.81 (m, 2H), 6.67 (s, 2H), 6.64 (d, J = 8.4 Hz, 1H), 6.62 (d, J = 2.5 Hz, 1H), 6.49 (dd, J = 8.3, 2.5 Hz, 1H), 6.32 – 6.21 (m, 2H), 5.06 (dd, J = 13.3, 5.1 Hz, 1H), 4.63 (d, J = 36.3 Hz, 2H), 4.41 (d, J = 17.5 Hz, 1H), 4.27 (d, J = 17.3 Hz, 1H), 4.17 (d, J = 5.0 Hz, 1H), 3.72 (q, J = 8.4 Hz, 2H), 3.47 – 3.40 (m, 2H), 3.36 – 3.28 (m, 2H), 3.20 – 2.86 (m, 9H), 2.64 – 2.56 (m, 1H), 2.43 – 2.32 (m, 2H), 2.21 (s, 2H), 2.11 – 1.95 (m, 6H), 1.77 – 1.61 (m, 4H) | 777. 39 | General Synthesis Formula 6 |
| **395** | L27 | 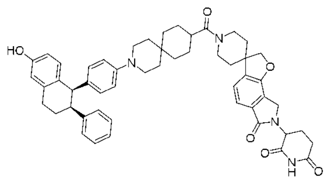 3-(1'-((3-(((1R,2S)-6-hydroxy-2-phenyl-1,2,3,4-tetrahydronaphthalen-1-yl)phenyl)-3-azaspiro[5.5]undecan-9-carbonyl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.99 (s, 1H), 9.18 (s, 1H), 7.41 (d, J = 7.6 Hz, 1H), 7.28 (d, J = 7.6 Hz, 1H), 7.22 – 7.08 (m, 4H), 6.86 – 6.81 (m, 2H), 6.68 – 6.61 (m, 2H), 6.50 (dd, J = 8.2, 2.6 Hz, 1H), 6.39 (s, 3H), 5.09 (dd, J = 13.3, 5.1 Hz, 1H), 4.64 (dt, J = 26.5, 9.6 Hz, 2H), 4.43 – 4.36 (m, 2H), 4.23 (dd, J = 17.2, 7.3 Hz, 2H), 3.96 (d, J = 14.1 Hz, 1H), 3.37 (s, 2H), 3.19 (t, J = 11.3 Hz, 2H), 3.10 – 2.88 (m, 6H), 2.76 – 2.56 (m, 4H), 2.43 (d, J = 13.1 Hz, 1H), 2.12 – 2.06 (m, 1H), 1.99 (td, J = 11.4, 6.7 Hz, 2H), 1.73 (s, 4H), 1.65 – 1.43 (m, 7H), 1.27 – 1.23 (m, 3H) | 833. 42 | General Synthesis Formula 20 |
| **396** | M1 | 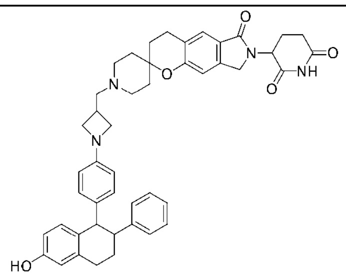 3-(1-((1-(4-(6-hydroxy-2-phenyl-1,2,3,4-tetrahydronaphthalen-1-yl)phenyl)azetidin-3-yl)methyl)-6'-oxo-3',4',6',8'-tetrahydro-7'-pyrido[4,2'-pyrano[2,3-f]isoindole]-7'- | $^1$H NMR (600 MHz, DMSO-$d_6$) δ 10.97 (s, 1H), 9.48 (s, 1H), 7.50 (s, 1H), 7.13 (dt, J = 13.8, 6.9 Hz, 3H), 7.04 (s, 1H), 6.82 (d, J = 7.2 Hz, 2H), 6.61 (dd, J = 13.4, 5.2 Hz, 2H), 6.47 (dd, J = 8.3, 2.3 Hz, 1H), 6.19 (d, J = 8.3 Hz, 2H), 6.06 (d, J = 8.3 Hz, 2H), 5.06 (dd, J = 13.2, 5.1 Hz, 1H), 4.34 (d, J = 16.9 Hz, 1H), 4.21 (d, J = 16.8 Hz, 1H), 4.12 (d, J = 4.8 Hz, 1H), 3.88 (q, J = 7.5 Hz, 2H), 3.37 – 3.23 (m, 6H), 3.20 – 3.06 (m, 3H), 2.99 – 2.85 (m, 6H), 2.59 (d, J = 16.8 Hz, 1H), 2.36 (ddd, | 737. 60 | General Synthesis Formula 1 |

| | | | | | |
|---|---|---|---|---|---|
| | | yl)piperidine-2,6-dione | J = 26.2, 13.1, 4.3 Hz, 1H), 2.12 – 2.03 (m, 2H), 1.98 – 1.93 (m, 3H), 1.90 – 1.83 (m, 3H), 1.71 – 1.66 (m, 1H) | | |
| **397** | M2 | 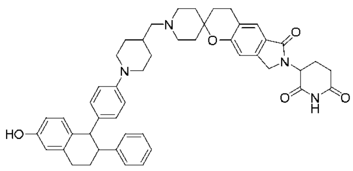 3-(1-((1-(4-(6-hydroxy-2-phenyl-1,2,3,4-tetrahydronaphthalen-1-yl)phenyl)piperidin-4-yl)methyl)-6'-oxo-3',4',6',8'-tetrahydro-7'-pyran[piperidin4,2'-pyrano[2,3-f]isoindole]-7'-yl)piperidine-2,6-dione | $^1$H NMR (600 MHz, DMSO-$d_6$) δ 10.96 (s, 1H), 9.12 (s, 1H), 7.50 (d, J = 6.1 Hz, 1H), 7.17 – 7.09 (m, 3H), 7.02 (d, J = 16.8 Hz, 1H), 6.83 (d, J = 7.2 Hz, 2H), 6.62 (dd, J = 17.0, 5.0 Hz, 4H), 6.48 (dd, J = 8.3, 2.1 Hz, 1H), 6.26 (d, J = 7.3 Hz, 2H), 5.06 (dd, J = 13.3, 5.0 Hz, 1H), 4.34 (dd, J = 16.8, 10.0 Hz, 1H), 4.21 (dd, J = 16.9, 7.5 Hz, 1H), 4.16 (d, J = 4.4 Hz, 1H), 3.30 (d, J = 10.3 Hz, 2H), 3.19 – 3.08 (m, 5H), 3.01 – 2.86 (m, 6H), 2.66 – 2.57 (m, 4H), 2.39 – 2.32 (m, 2H), 2.13 – 2.04 (m, 2H), 2.00 – 1.91 (m, 5H), 1.87 (t, J = 6.7 Hz, 1H), 1.80 (d, J = 11.3 Hz, 2H), 1.73 – 1.70 (m, 1H), 1.30 (d, J = 10.8 Hz, 2H) | 767.18 | General Synthesis Formula 1 |
| **398** | M3 | 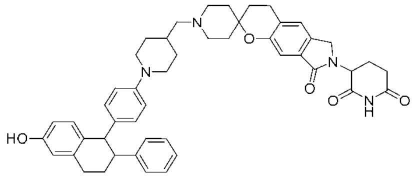 3-(1-((1-(4-(6-hydroxy-2-phenyl-1,2,3,4-tetrahydronaphthalen-1-yl)phenyl)piperidin-4-yl)methyl)-8'-oxo-3',4',6',8'-tetrahydro-7'-pyrido[4,2'-pyrano[2,3-f]isoindole]-7'-yl)piperidine-2,6-dione | $^1$H NMR (600 MHz, DMSO-$d_6$) δ 10.98 (s, 1H), 8.96 (s, 1H), 7.36 (s, 1H), 7.24 (s, 1H), 7.14 (dt, J = 20.7, 7.8 Hz, 3H), 6.84 (d, J = 7.1 Hz, 3H), 6.64 (dd, J = 12.8, 5.0 Hz, 3H), 6.50 (dd, J = 8.2, 2.2 Hz, 1H), 6.34 (s, 2H), 5.06 (dd, J = 13.3, 5.1 Hz, 1H), 4.35 (d, J = 16.7 Hz, 1H), 4.25 – 4.19 (m, 2H), 3.44 (d, J = 10.8 Hz, 2H), 3.34 (d, J = 9.8 Hz, 1H), 3.26 – 3.18 (m, 2H), 3.17 – 3.10 (m, 2H), 3.03 – 2.94 (m, 3H), 2.93 – 2.88 (m, 5H), 2.60 (d, J = 17.3 Hz, 1H), 2.39 (ddd, J = 26.1, 13.1, 4.2 Hz, 1H), 2.12 – 2.01 (m, 4H), 2.00 – 1.93 (m, 7H), 1.92 – 1.87 (m, 2H), 1.75 – 1.71 (m, 1H) | 765.72 | General Synthesis Formula 1 |
| **399** | M4 | 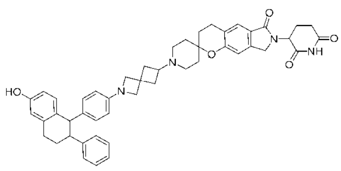 3-(1-(2-(4-(6-hydroxy-2-phenyl-1,2,3,4-tetrahydronaphthalen-1-yl)phenyl)-2-azaspiro[3.3]heptan-6-yl)-6-oxo-3',4',6',8'-tetrahydro-7'- | $^1$H NMR (600 MHz, DMSO-$d_6$) δ 10.97 (s, 1H), 9.11 (s, 1H), 7.51 (d, J = 6.1 Hz, 1H), 7.13 (dt, J = 19.4, 6.3 Hz, 3H), 7.02 (d, J = 8.1 Hz, 1H), 6.82 (d, J = 7.4 Hz, 2H), 6.64 – 6.59 (m, 2H), 6.49 – 6.46 (m, 1H), 6.18 (d, J = 8.2 Hz, 2H), 6.06 (d, J = 8.2 Hz, 2H), 5.07 (dd, J = 13.3, 5.0 Hz, 1H), 4.34 (t, J = 13.2 Hz, 1H), 4.22 (dd, J = 16.9, 7.3 Hz, 1H), 4.12 (d, J = 4.8 Hz, | 763.70 | General Synthesis Formula 6 |

| | | | | | |
|---|---|---|---|---|---|
| | | spiro[piperidine-4,2'-pyrano[2,3-f]isoindole]-7'-yl)piperidine-2,6-dione | 1H), 3.35 – 3.26 (m, 4H), 3.04 – 2.87 (m, 8H), 2.60 (d, J = 17.2 Hz, 1H), 2.45 – 2.33 (m, 5H), 2.12 – 2.05 (m, 1H), 2.03 – 1.95 (m, 5H), 1.89 (t, J = 6.7 Hz, 2H), 1.80 (d, J = 13.2 Hz, 2H), 1.70 (d, J = 5.8 Hz, 1H) | | |
| **400** | M5 | 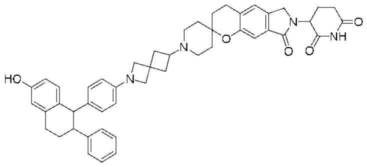 3-(1-(2-(4-(6-hydroxy-2-phenyl-1,2,3,4-tetrahydronaphthalen-1-yl)phenyl)-2-azaspiro[3.3]heptan-6-yl)-8'-oxo-3',4',6',8'-tetrahydro-7'-spiro[piperidine-4,2'-pyrano[2,3-f]isoindole]-7'-yl)piperidine-2,6-dione | $^1$H NMR (600 MHz, DMSO-$d_6$) δ 10.98 (s, 1H), 9.11 (s, 1H), 7.36 (s, 1H), 7.21 (s, 1H), 7.13 (dt, J = 13.6, 6.9 Hz, 3H), 6.82 (d, J = 7.3 Hz, 2H), 6.61 (dd, J = 12.5, 5.1 Hz, 2H), 6.48 (dd, J = 8.2, 2.2 Hz, 1H), 6.18 (d, J = 8.3 Hz, 2H), 6.06 (d, J = 8.3 Hz, 2H), 5.07 (dd, J = 13.3, 5.1 Hz, 1H), 4.34 (d, J = 16.6 Hz, 1H), 4.22 (d, J = 16.6 Hz, 1H), 4.12 (d, J = 4.8 Hz, 1H), 3.78 – 3.72 (m, 2H), 3.65 (t, J = 9.2 Hz, 2H), 3.28 (d, J = 11.3 Hz, 2H), 3.04 (d, J = 10.4 Hz, 2H), 2.97 – 2.88 (m, 6H), 2.60 (d, J = 18.4 Hz, 1H), 2.43 – 2.36 (m, 4H), 2.12 – 2.05 (m, 2H), 2.02 – 1.96 (m, 4H), 1.89 (t, J = 6.7 Hz, 2H), 1.80 – 1.74 (m, 2H), 1.70 (d, J = 5.9 Hz, 1H) | 763.74 | General Synthesis Formula 6 |
| **401** | M6 | 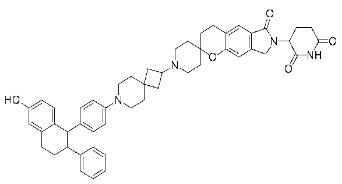 3-(1-(7-(4-(6-hydroxy-2-phenyl-1,2,3,4-tetrahydronaphthalen-1-yl)phenyl)-7-azaspiro[3.5]nonan-2-yl)-6'-oxo-3',4',6',8'-tetrahydro-7'-spiro[piperidine-4,2'-pyrano[2,3-f]isoindole]-7'-yl)piperidine-2,6-dione | $^1$H NMR (600 MHz, DMSO-$d_6$) δ 10.97 (s, 1H), 9.14 (s, 1H), 7.51 (d, J = 7.1 Hz, 1H), 7.21 (s, 1H), 7.16 – 7.11 (m, 2H), 7.03 (d, J = 11.9 Hz, 3H), 6.83 (d, J = 7.2 Hz, 2H), 6.64 (d, J = 8.3 Hz, 1H), 6.61 (d, J = 1.9 Hz, 2H), 6.48 (dd, J = 8.2, 2.2 Hz, 1H), 6.23 (d, J = 8.0 Hz, 2H), 5.07 (dd, J = 13.2, 5.1 Hz, 1H), 4.35 (d, J = 16.9 Hz, 1H), 4.24 – 4.19 (m, 1H), 4.15 (d, J = 4.6 Hz, 1H), 3.83 (dd, J = 16.6, 8.4 Hz, 1H), 3.30 (d, J = 12.4 Hz, 3H), 3.04 – 2.87 (m, 10H), 2.60 (d, J = 16.5 Hz, 1H), 2.40 – 2.33 (m, 2H), 2.20 – 2.16 (m, 3H), 2.12 – 2.06 (m, 1H), 2.03 – 1.95 (m, 5H), 1.89 (t, J = 6.8 Hz, 2H), 1.83 (d, J = 13.0 Hz, 1H), 1.71 (d, J = 6.1 Hz, 1H), 1.62 (d, J = 21.1 Hz, 3H) | 791.75 | General Synthesis Formula 6 |
| **402** | M7 | 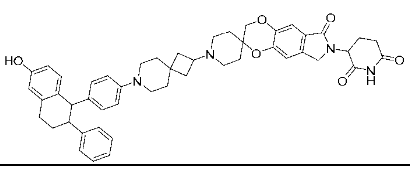 | $^1$H NMR (400 MHz, DMSO-$d_6$) δ 10.95 (s, 1H), 9.12 (s, 1H), 7.23 (s, 1H), 7.12 (t, J = 7.2 Hz, 4H), 6.83 (d, J = 6.4 Hz, 2H), 6.62 (dd, J = 10.1, 5.2 Hz, 4H), 6.48 (dd, | 793.70 | General Synthesis Formula 6 |

| | | | | | |
|---|---|---|---|---|---|
| | | 3-(1-(7-(4-(6-hydroxy-2-phenyl-1,2,3,4-tetrahydronaphthalen-1-yl)phenyl)-7-azaspiro[3.5]nonan-2-yl)-6'-oxo-6',8'-dihydro-3'H,7'H-spiro[piperidine-4,2'-[1,4]dioxin[2,3-f]isoindole]-7'-yl)piperidine-2,6-dione | J = 8.3, 2.3 Hz, 1H), 6.26 (d, J = 8.1 Hz, 2H), 5.06 (dd, J = 13.2, 4.9 Hz, 1H), 4.33 (d, J = 17.0 Hz, 1H), 4.23 – 4.12 (m, 4H), 3.40 – 3.26 (m, 3H), 3.10 – 2.85 (m, 8H), 2.59 (d, J = 16.7 Hz, 1H), 2.41 – 2.30 (m, 1H), 2.18 (d, J = 7.8 Hz, 2H), 2.12 – 2.04 (m, 1H), 2.00 (d, J = 9.4 Hz, 4H), 1.88 (t, J = 12.1 Hz, 2H), 1.64 (d, J = 8.5 Hz, 4H) | | |
| **403** | M8 | 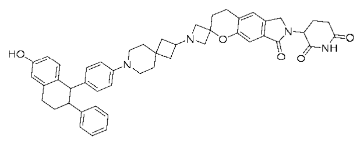 3-(1-(7-(4-(6-hydroxy-2-phenyl-1,2,3,4-tetrahydronaphthalen-1-yl)phenyl)-7-azaspiro[3.5]nonan-2-yl)-8'-oxo-3',4',6',8'-tetrahydro-7'H-spiro[azetidine-3,2'-pyrano[2,3-f]isoindole]-7'-yl)piperidine-2,6-dione | $^1$H NMR (400 MHz, DMSO-$d_6$) δ 10.96 (s, 1H), 9.11 (s, 1H), 7.37 (s, 1H), 7.17 – 7.08 (m, 4H), 6.83 (d, J = 6.5 Hz, 2H), 6.62 (dd, J = 10.0, 5.2 Hz, 4H), 6.48 (dd, J = 8.3, 2.2 Hz, 1H), 6.24 (d, J = 7.1 Hz, 2H), 5.07 (dd, J = 13.2, 5.0 Hz, 1H), 4.35 (d, J = 16.8 Hz, 2H), 4.28 – 4.09 (m, 5H), 3.33 – 3.27 (m, 1H), 3.07 – 2.86 (m, 9H), 2.59 (d, J = 16.9 Hz, 1H), 2.43 – 2.32 (m, 1H), 2.24 – 2.09 (m, 5H), 2.04 – 1.94 (m, 3H), 1.88 – 1.80 (m, 2H), 1.72 – 1.60 (m, 4H) | 763.65 | General Synthesis Formula 6 |
| **404** | M9 | 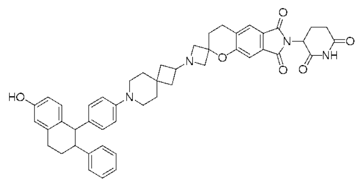 7'-(2,6-dioxopiperidin-3-yl)-1-(7-(4-(6-hydroxy-2-phenyl-1,2,3,4-tetrahydronaphthalen-1-yl)phenyl)-7-azaspiro[3.5]nonan-2-yl)-3'-dihydro-6'-azetidine-3,2'-pyrano[2,3-f]isoindole]-6',8'-dione | $^1$H NMR (400 MHz, DMSO-$d_6$) δ 11.10 (s, 1H), 9.11 (s, 1H), 7.77 (s, 1H), 7.30 (d, J = 6.2 Hz, 1H), 7.17 – 7.08 (m, 3H), 6.83 (d, J = 6.4 Hz, 2H), 6.62 (dd, J = 10.0, 5.2 Hz, 4H), 6.48 (dd, J = 8.3, 2.3 Hz, 1H), 6.24 (d, J = 7.8 Hz, 2H), 5.10 (dd, J = 12.8, 5.3 Hz, 1H), 4.45 – 4.29 (m, 2H), 4.27 – 4.04 (m, 5H), 3.33 – 3.27 (m, 1H), 2.93 (ddd, J = 30.8, 20.4, 15.3 Hz, 9H), 2.57 (dd, J = 21.8, 11.1 Hz, 2H), 2.22 (t, J = 6.2 Hz, 2H), 2.07 (ddd, J = 15.4, 11.2, 5.8 Hz, 3H), 1.83 (s, 2H), 1.73 – 1.59 (m, 5H) | 777.65 | General Synthesis Formula 6 |
| **405** | M10 | 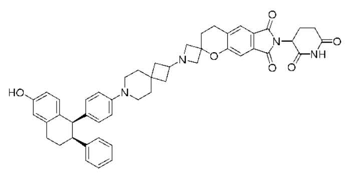 7'-(2,6-dioxopiperidin-3-yl)-1-(7-(4-(((1R,2S)-6-hydroxy-2-phenyl-1,2,3,4-tetrahydronaphthalen-1-yl)phenyl)-7-azaspiro[3.5]nonan-2-yl)-3',4'-dihydro- | $^1$H NMR (600 MHz, DMSO-$d_6$) δ 11.11 (s, 1H), 9.14 (s, 1H), 7.78 (s, 1H), 7.31 (d, J = 8.2 Hz, 1H), 7.13 (dt, J = 18.5, 6.4 Hz, 3H), 6.83 (d, J = 7.3 Hz, 2H), 6.67 – 6.60 (m, 2H), 6.51 – 6.47 (m, 1H), 6.26 (s, 2H), 5.76 (s, 2H), 5.11 (dd, J = 12.8, 5.5 Hz, 1H), 4.41 (d, J = 10.1 Hz, 1H), 4.33 (s, 1H), 4.25 (s, 1H), 4.16 (s, 2H), 3.31 (d, J = 13.5 Hz, 2H), 3.06 – 2.85 (m, 9H), 2.60 | 777.65 | General Synthesis Formula 6 |

| | | | | | |
|---|---|---|---|---|---|
| | | 6'-pyranazetidine-3,2'-pyrano[2,3-f]isoindole]-6',8'-dione | (d, J = 17.9 Hz, 1H), 2.54 (d, J = 4.4 Hz, 1H), 2.22 (t, J = 6.0 Hz, 2H), 2.08 (ddd, J = 27.4, 14.2, 7.6 Hz, 4H), 1.84 (d, J = 8.5 Hz, 2H), 1.72 (d, J = 6.2 Hz, 1H), 1.68 – 1.61 (m, 4H) | | |
| **406** | M11 | 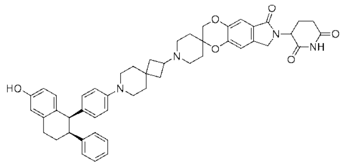 3-(1-(7-(4-((1R,2S)-6-hydroxy-2-phenyl-1,2,3,4-tetrahydronaphthalen-1-yl)phenyl)-7-azaspiro[3.5]nonan-2-yl)-6'-oxo-6',8'-dihydro-3'H,7'H-spiro[piperidine-4,2'-[1,4]dioxin[2,3-f]isoindole]-7'-yl)piperidine-2,6-dione | $^1$H NMR (600 MHz, DMSO-$d_6$) δ 10.97 (s, 1H), 9.14 (s, 1H), 7.24 (s, 1H), 7.17 – 7.11 (m, 4H), 6.83 (d, J = 7.2 Hz, 2H), 6.62 (dd, J = 16.1, 5.1 Hz, 4H), 6.48 (dd, J = 8.3, 2.2 Hz, 1H), 6.24 (d, J = 8.0 Hz, 2H), 5.07 (dd, J = 13.3, 4.9 Hz, 1H), 4.35 – 4.30 (m, 2H), 4.30 – 4.19 (m, 3H), 4.16 (dd, J = 12.7, 7.2 Hz, 2H), 3.84 (dd, J = 16.4, 8.1 Hz, 1H), 3.08 – 2.88 (m, 10H), 2.60 (d, J = 16.8 Hz, 1H), 2.40 – 2.33 (m, 3H), 2.19 (s, 2H), 2.09 (dd, J = 11.7, 5.2 Hz, 2H), 1.98 (d, J = 11.5 Hz, 3H), 1.87 (t, J = 13.7 Hz, 2H), 1.71 (d, J = 5.7 Hz, 1H), 1.63 (d, J = 18.2 Hz, 3H) | 793.70 | General Synthesis Formula 6 |
| **407** | M12 | 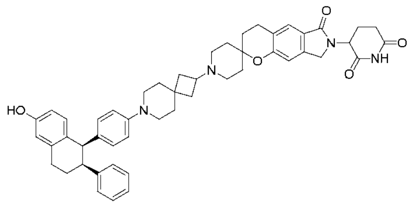 3-(1-(7-(4-((1R,2S)-6-hydroxy-2-phenyl-1,2,3,4-tetrahydronaphthalen-1-yl)phenyl)-7-azaspiro[3.5]nonan-2-yl)-6'-oxo-3',4',6'-tetrahydro-7'H-spiro[piperidine-4,2'-pyrano[2,3-f]isoindole]-7'-yl)piperidine-2,6-dione | $^1$H NMR (600 MHz, DMSO-$d_6$) δ 10.97 (s, 1H), 9.18 (s, 1H), 7.51 (d, J = 6.7 Hz, 1H), 7.13 (dq, J = 14.1, 6.9 Hz, 3H), 7.02 (s, 1H), 6.83 (d, J = 7.1 Hz, 2H), 6.78 (s, 2H), 6.64 (d, J = 8.4 Hz, 1H), 6.62 (d, J = 2.0 Hz, 1H), 6.49 (dd, J = 8.3, 2.3 Hz, 1H), 6.31 (d, J = 8.1 Hz, 2H), 5.07 (dd, J = 13.2, 5.1 Hz, 1H), 4.34 (dd, J = 16.8, 8.4 Hz, 1H), 4.23 (d, J = 6.4 Hz, 1H), 4.22 – 4.18 (m, 1H), 3.32 (d, J = 11.4 Hz, 3H), 3.13 (s, 2H), 3.07 – 2.96 (m, 5H), 2.95 – 2.85 (m, 4H), 2.60 (d, J = 16.7 Hz, 1H), 2.36 (dd, J = 13.2, 4.6 Hz, 1H), 2.22 (s, 2H), 2.10 – 1.97 (m, 6H), 1.88 (dd, J = 15.7, 8.8 Hz, 3H), 1.70 (d, J = 23.7 Hz, 5H) | 791.80 | General Synthesis Formula 6 |
| **408** | M13 | 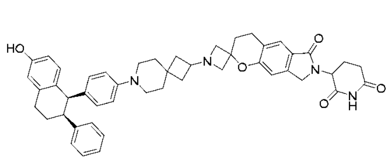 3-(1-(7-(4-((1R,2S)-6-hydroxy-2-phenyl-1,2,3,4-tetrahydronaphthalen-1- | $^1$H NMR (600 MHz, DMSO-$d_6$) δ 10.97 (s, 1H), 9.16 (s, 1H), 7.53 (s, 1H), 7.19 – 7.10 (m, 3H), 7.08 (d, J = 7.8 Hz, 1H), 6.83 (d, J = 7.1 Hz, 2H), 6.69 – 6.60 (m, 4H), 6.48 (dd, J = 8.3, 2.3 Hz, 1H), 6.26 (d, J = 7.5 Hz, 2H), 5.06 (dd, J = 13.2, 5.0 Hz, 1H), 4.37 (dd, J = 23.1, 6.9 Hz, 2H), | 763.76 | General Synthesis Formula 6 |

| | | | | | |
|---|---|---|---|---|---|
| | | yl)phenyl)-7-azaspiro[3.5]nonan-2-yl)-6'-oxo-3',4',6'-tetrahydro-7'H-spiro[azetidine-3,2'-pyrano[2,3-f]isoindole]-7'-yl)piperidine-2,6-dione | 4.25 (dd, J = 18.6, 12.8 Hz, 3H), 4.18 – 4.07 (m, 4H), 3.31 (d, J = 14.1 Hz, 1H), 3.04 (s, 2H), 2.99 – 2.85 (m, 7H), 2.60 (d, J = 17.2 Hz, 1H), 2.41 – 2.32 (m, 1H), 2.19 (s, 2H), 2.16 – 2.06 (m, 3H), 1.99 (d, J = 7.0 Hz, 1H), 1.84 (d, J = 8.4 Hz, 2H), 1.72 (d, J = 5.9 Hz, 1H), 1.65 (s, 3H) | | |
| **409** | M14 | 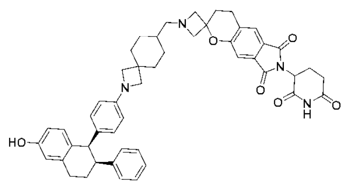 7'-(2,6-dioxopiperidin-3-yl)-1-((2-(4-(((1R,2S)-6-hydroxy-2-phenyl-1,2,3,4-tetrahydronaphthalen-1-yl)phenyl)-2-azaspiro[3.5]nonan-7-yl)methyl)-3'-dihydro-6'-azetidine-3,2'-pyrano[2,3-f]isoindole]-6',8'-dione | $^1$H NMR (600 MHz, DMSO-$d_6$) δ 11.12 (s, 1H), 7.77 (s, 1H), 7.29 (d, *J* = 3.4 Hz, 1H), 7.17 – 7.09 (m, 3H), 6.83 (d, *J* = 7.1 Hz, 2H), 6.62 (d, *J* = 8.4 Hz, 1H), 6.60 (d, *J* = 2.3 Hz, 1H), 6.48 (dd, *J* = 8.3, 2.5 Hz, 1H), 6.17 (d, *J* = 8.5 Hz, 2H), 6.03 (d, *J* = 8.4 Hz, 2H), 5.11 (dd, *J* = 12.9, 5.4 Hz, 1H), 4.51 – 4.39 (m, 2H), 4.36 – 4.26 (m, 2H), 4.11 (d, *J* = 4.8 Hz, 1H), 3.41 – 3.36 (m, 3H), 3.34 (s, 2H), 3.29 – 3.25 (m, 1H), 3.22 (s, 1H), 3.16 – 3.13 (m, 1H), 3.02 – 2.85 (m, 5H), 2.63 – 2.58 (m, 1H), 2.57 – 2.53 (m, 1H), 2.24 (q, *J* = 6.5 Hz, 2H), 2.13 – 2.01 (m, 2H), 1.85 (d, *J* = 8.9 Hz, 2H), 1.70 (d, *J* = 5.7 Hz, 1H), 1.63 (d, *J* = 12.0 Hz, 2H), 1.41 (dd, *J* = 21.9, 9.3 Hz, 2H), 1.07 – 0.97 (m, 2H) | 791.24 | General Synthesis Formula 12 |
| **410** | M15 | 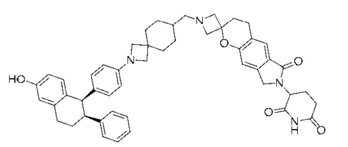 3-(1-((2-(4-((1R,2S)-6-hydroxy-2-phenyl-1,2,3,4-tetrahydronaphthalen-1-yl)phenyl)-2-azaspiro[3.5]nonan-7-yl)methyl)-6-oxo-3',4',6',8'-tetrahydro-7'H-spiro[azetidine-3,2'-pyrano[2,3-f]isoindole]-7'-yl)piperidine-2,6-dione | $^1$H NMR (600 MHz, DMSO-$d_6$) δ 10.98 (s, 1H), 7.52 (s, 1H), 7.17 – 7.09 (m, 3H), 7.07 (d, *J* = 14.3 Hz, 1H), 6.83 (d, *J* = 7.1 Hz, 2H), 6.62 (d, *J* = 8.4 Hz, 1H), 6.60 (d, *J* = 2.4 Hz, 1H), 6.48 (dd, *J* = 8.3, 2.5 Hz, 1H), 6.17 (d, *J* = 8.5 Hz, 2H), 6.03 (d, *J* = 8.4 Hz, 2H), 5.06 (dd, *J* = 13.0, 4.5 Hz, 1H), 4.45 (d, *J* = 6.0 Hz, 1H), 4.39 – 4.20 (m, 5H), 4.11 (d, *J* = 4.9 Hz, 1H), 3.41 – 3.36 (m, 2H), 3.34 (s, 2H), 3.30 – 3.25 (m, 1H), 3.23 – 3.18 (m, 1H), 3.16 – 3.12 (m, 1H), 3.01 – 2.84 (m, 5H), 2.60 (d, *J* = 17.1 Hz, 1H), 2.41 – 2.31 (m, 1H), 2.20 (dt, *J* = 13.2, 6.6 Hz, 2H), 2.09 (dt, *J* = 12.6, 7.0 Hz, 1H), 2.02 – 1.94 (m, 1H), 1.89 – 1.82 (m, 2H), 1.70 (dd, *J* = 11.2, 5.2 Hz, 1H), 1.63 (d, *J* = 12.0 Hz, 3H), 1.41 (t, *J* = 12.8 Hz, 2H), 1.08 – 0.95 (m, 2H) | 777.15 | General Synthesis Formula 12 |

| | | | | | |
|---|---|---|---|---|---|
| **411** | M16 | 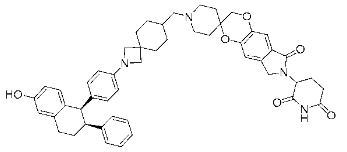<br>3-(1-((2-(4-((1R,2S)-6-hydroxy-2-phenyl-1,2,3,4-tetrahydronaphthalen-1-yl)phenyl)-2-azaspiro[3.5]nonan-7-yl)methyl)-6'-oxo-6',8'-dihydro-3'H,7'H-spiro[piperidine-4,2'-[1,4]dioxin[2,3-f]isoindole]-7'-yl)piperidine-2,6-dione | $^1$H NMR (600 MHz, DMSO-$d_6$) δ 10.98 (s, 1H), 7.23 (s, 1H), 7.17 – 7.10 (m, 4H), 6.83 (d, *J* = 7.1 Hz, 2H), 6.63 (d, *J* = 8.4 Hz, 1H), 6.60 (d, *J* = 2.3 Hz, 1H), 6.48 (dd, *J* = 8.3, 2.4 Hz, 1H), 6.18 (d, *J* = 8.4 Hz, 2H), 6.04 (d, *J* = 8.5 Hz, 2H), 5.07 (dd, *J* = 13.3, 5.1 Hz, 1H), 4.36 – 4.09 (m, 6H), 3.43 – 3.39 (m, 2H), 3.38 – 3.33 (m, 2H), 3.30 – 3.25 (m, 1H), 3.24 – 3.10 (m, 2H), 3.09 – 3.01 (m, 2H), 3.00 – 2.86 (m, 3H), 2.59 (d, *J* = 16.6 Hz, 1H), 2.41 – 2.31 (m, 1H), 2.15 – 2.05 (m, 2H), 2.03 – 1.91 (m, 5H), 1.87 (d, *J* = 12.4 Hz, 2H), 1.79 (s, 1H), 1.70 (d, *J* = 11.0 Hz, 3H), 1.47 (t, *J* = 11.2 Hz, 2H), 1.09 – 0.98 (m, 2H) | 807.37 | General Synthesis Formula 12 |
| **412** | M17 | 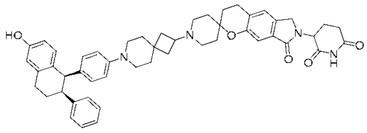<br>3-(1-(7-(4-((1R,2S)-6-hydroxy-2-phenyl-1,2,3,4-tetrahydronaphthalen-1-yl)phenyl)-7-azaspiro[3.5]nonan-2-yl)-8'-oxo-3',4',6'-tetrahydro-7'H-spiro[piperidine-4,2'-pyrano[2,3-f]isoindole]-7'-yl)piperidine-2,6-dione | $^1$H NMR (600 MHz, DMSO-$d_6$) δ 10.98 (s, 1H), 7.36 (s, 1H), 7.20 (s, 1H), 7.17 – 7.10 (m, 3H), 6.83 (d, *J* = 7.0 Hz, 2H), 6.66 (s, 1H), 6.64 (d, *J* = 8.4 Hz, 2H), 6.61 (d, *J* = 2.1 Hz, 1H), 6.48 (dd, *J* = 8.2, 2.3 Hz, 1H), 6.26 (d, *J* = 7.8 Hz, 2H), 5.06 (dd, *J* = 13.3, 5.0 Hz, 1H), 4.34 (d, *J* = 16.7 Hz, 1H), 4.22 (d, *J* = 16.7 Hz, 1H), 4.17 (d, *J* = 4.6 Hz, 1H), 3.84 (td, *J* = 16.5, 8.2 Hz, 1H), 3.30 (dd, *J* = 20.0, 7.3 Hz, 3H), 3.09 – 2.96 (m, 7H), 2.95 – 2.84 (m, 4H), 2.60 (d, *J* = 17.2 Hz, 1H), 2.39 (qd, *J* = 13.2, 4.3 Hz, 1H), 2.20 (t, *J* = 7.6 Hz, 2H), 2.09 (ddd, *J* = 25.2, 12.8, 6.3 Hz, 1H), 2.03 – 1.94 (m, 5H), 1.90 (t, *J* = 6.8 Hz, 2H), 1.85 – 1.76 (m, 2H), 1.74 – 1.69 (m, 1H), 1.65 (d, *J* = 19.3 Hz, 4H) | 791.62 | General Synthesis Formula 6 |
| **413** | N1 | 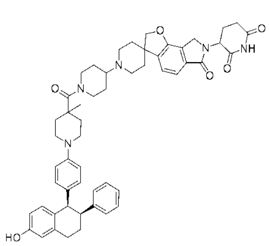<br>3-(1'-(1-(1-(4-((1R,2S)-6-hydroxy-2-phenyl-1,2,3,4-tetrahydronaphthalen-1-yl)phenyl)piperidin-4-yl)carbonyl)piperidin-4-yl)-6-oxo-6,8- | $^1$H NMR (600 MHz, DMSO-$d_6$) δ 10.99 (s, 1H), 9.55 (s, 1H), 9.15 (s, 1H), 7.35 (d, J = 7.6 Hz, 1H), 7.28 (d, J = 7.6 Hz, 1H), 7.13 (dq, J = 14.2, 7.0 Hz, 3H), 6.83 (d, J = 7.1 Hz, 2H), 6.73 – 6.53 (m, 4H), 6.48 (dd, J = 8.3, 2.3 Hz, 1H), 6.27 (d, J = 6.3 Hz, 2H), 5.09 (dd, J = 13.2, 5.0 Hz, 1H), 4.66 (q, J = 9.5 Hz, 2H), 4.42 (dd, J = 33.0, 14.3 Hz, 3H), 4.20 (dd, J = 39.8, 10.5 Hz, 3H), 3.55 (d, J = 11.3 Hz, 3H), | 862.45 | General Synthesis Formula 15 |

| | | | | | |
|---|---|---|---|---|---|
| | | dihydro-2H,7H-spiro[furo[2,3-e]isoindole-3,4'-piperidine]-7-yl)piperidine-2,6-dione | 3.30 (d, J = 10.1 Hz, 1H), 3.21 (s, 2H), 3.09 (d, J = 11.4 Hz, 2H), 2.98 – 2.90 (m, 4H), 2.81 (s, 2H), 2.63 – 2.52 (m, 1H), 2.44 – 2.34 (m, 1H), 2.22 – 2.04 (m, 7H), 1.99 (dd, J = 18.7, 10.6 Hz, 3H), 1.73 – 1.48 (m, 5H), 1.26 (s, 3H). | | |
| **414** | N2 | 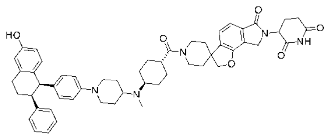 3-(1'-((1R,4r)-4-((1-(4-((1R,2S)-6-hydroxy-2-phenyl-1,2,3,4-tetrahydronaphthalen-1-yl)phenyl)piperidin-4-yl)(methyl)amino)cyclohexane-1-carbonyl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.98 (s, 1H), 9.09 (s, 2H), 7.42 (d, J = 7.6 Hz, 1H), 7.29 (d, J = 7.6 Hz, 1H), 7.14 (dq, J = 14.1, 6.9 Hz, 3H), 6.84 (d, J = 7.2 Hz, 2H), 6.65 – 6.58 (m, 4H), 6.49 (dd, J = 8.3, 2.2 Hz, 1H), 6.24 (d, J = 8.5 Hz, 2H), 5.09 (dt, J = 12.5, 6.2 Hz, 1H), 4.65 (dt, J = 20.8, 9.8 Hz, 2H), 4.40 (dd, J = 16.8, 7.4 Hz, 2H), 4.29 – 4.19 (m, 1H), 4.16 (d, J = 4.8 Hz, 1H), 3.96 (d, J = 12.2 Hz, 3H), 3.44 (d, J = 9.9 Hz, 3H), 3.32 – 3.28 (m, 1H), 3.21 (t, J = 12.3 Hz, 1H), 3.03 – 2.87 (m, 3H), 2.63 (dd, J = 38.2, 9.3 Hz, 6H), 2.43 (dd, J = 26.8, 13.8 Hz, 1H), 2.15 – 1.94 (m, 7H), 1.89 – 1.63 (m, 11H), 1.57 (d, J = 7.8 Hz, 2H). | 876.46 | General Synthesis Formula 34 |
| **415** | N3 | 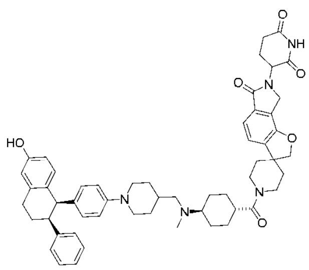 3-(1'-((1R,4r)-4-((1-(4-((1R,2S)-6-hydroxy-2-phenyl-1,2,3,4-tetrahydronaphthalen-1-yl)phenyl)piperidin-4-yl)methyl)(methyl)amino)cyclohexane-1-carbonyl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.98 (s, 1H), 9.14 (s, 1H), 8.98 (s, 1H), 7.42 (d, J = 7.4 Hz, 1H), 7.29 (t, J = 7.9 Hz, 1H), 7.17 – 7.12 (m, 4H), 6.84 (d, J = 7.1 Hz, 3H), 6.64 (d, J = 8.4 Hz, 1H), 6.62 (d, J = 2.1 Hz, 1H), 6.49 (dd, J = 8.3, 2.3 Hz, 1H), 6.27 (d, J = 7.6 Hz, 2H), 5.25 – 4.96 (m, 3H), 4.65 (dd, J = 22.1, 10.8 Hz, 2H), 4.39 (d, J = 6.2 Hz, 2H), 4.17 (d, J = 4.6 Hz, 2H), 3.96 (d, J = 12.4 Hz, 2H), 3.31 (d, J = 12.9 Hz, 3H), 3.21 (d, J = 10.7 Hz, 1H), 3.09 (d, J = 9.4 Hz, 2H), 2.98 (d, J = 5.6 Hz, 1H), 2.96 – 2.90 (m, 3H), 2.74 (d, J = 4.5 Hz, 3H), 2.69 (s, 2H), 2.60 (d, J = 17.8 Hz, 1H), 2.41 (d, J = 16.1 Hz, 1H), 2.09 (d, J = 6.2 Hz, 1H), 2.03 (s, 1H), 1.98 (d, J = 6.8 Hz, 1H), 1.91 (s, 2H), 1.85 (s, 2H), 1.79 – 1.71 (m, 6H), 1.59 (d, J = 12.3 Hz, 3H), 1.46 (s, 1H), 1.31 (d, J = 12.6 Hz, 2H). | 890.48 | General Synthesis Formula 36 |

| | | | | | |
|---|---|---|---|---|---|
| **416** | N4 | 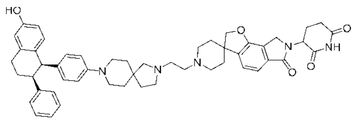 3-(1'-(2-(8-(4-((1R,2S)-6-hydroxy-2-phenyl-1,2,3,4-tetrahydronaphthalen-1-yl)phenyl)-2,8-diazaspiro[4.5]decane-2-yl)ethyl)-6-oxo-6,8-dihydro-2H,7H-spiro[furo[2,3-e]isoindole-3,4'-piperidine]-7-yl)piperidine-2,6-dione | $^1$H NMR (400 MHz, DMSO-$d_6$) δ 11.02 – 10.89 (m, 1H), 9.10 (s, 1H), 8.19 (s, 1H), 7.37 (d, J = 7.6 Hz, 1H), 7.26 (d, J = 7.6 Hz, 1H), 7.13 (t, J = 8.0 Hz, 3H), 6.83 (d, J = 6.6 Hz, 2H), 6.64 (d, J = 8.4 Hz, 1H), 6.60 (d, J = 1.9 Hz, 1H), 6.53 (d, J = 8.6 Hz, 2H), 6.47 (dd, J = 8.3, 2.4 Hz, 1H), 4.55 – 4.46 (m, 2H), 4.37 (d, J = 17.2 Hz, 1H), 4.21 (d, J = 17.1 Hz, 1H), 4.12 (d, J = 4.7 Hz, 1H), 2.99 – 2.85 (m, 10H), 2.57 (dd, J = 14.4, 8.0 Hz, 4H), 2.47 – 2.42 (m, 3H), 2.40 (s, 2H), 2.12 – 2.00 (m, 3H), 1.99 – 1.95 (m, 1H), 1.89 (t, J = 11.0 Hz, 2H), 1.70 (dt, J = 21.7, 9.0 Hz, 4H), 1.56 (dd, J = 14.3, 7.7 Hz, 6H) | 820.44 | General Synthesis Formula 12 |
| **417** | N5 | 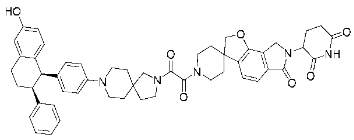 3-(1'-(2-(8-(4-((1R,2S)-6-hydroxy-2-phenyl-1,2,3,4-tetrahydronaphthalen-1-yl)phenyl)-2,8-diazaspiro[4.5]decane-2-yl)-2-oxoacetyl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.99 (s, 1H), 9.16 (s, 1H), 7.49 – 7.35 (m, 1H), 7.31 – 7.19 (m, 1H), 7.18 – 7.10 (m, 3H), 7.08 – 6.77 (m, 4H), 6.70 – 6.61 (m, 2H), 6.53 – 6.48 (m, 1H), 6.39 (s, 2H), 5.10 (dt, J = 13.1, 4.8 Hz, 1H), 4.65 (dq, J = 15.1, 9.6 Hz, 2H), 4.38 (dt, J = 44.6, 22.3 Hz, 2H), 4.33 – 4.18 (m, 5H), 3.57 (d, J = 12.7 Hz, 1H), 3.53 – 3.48 (m, 1H), 3.46 (s, 1H), 3.34 (s, 3H), 3.29 (d, J = 15.5 Hz, 3H), 3.24 – 3.19 (m, 1H), 3.01 (ddd, J = 20.2, 13.3, 6.9 Hz, 1H), 2.97 – 2.88 (m, 3H), 2.60 (d, J = 16.6 Hz, 1H), 2.46 – 2.38 (m, 1H), 2.08 (qd, J = 12.0, 5.9 Hz, 1H), 2.02 – 1.94 (m, 1H), 1.91 (d, J = 4.8 Hz, 1H), 1.87 – 1.81 (m, 2H), 1.80 (s, 1H), 1.77 (s, 1H), 1.75 (s, 4H) | 848.01 | General Synthesis Formula 10 |
| **418** | N6 | 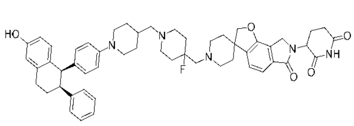 3-(1'-((4-fluoro-1-((1-(4-((1R,2S)-6-hydroxy-2-phenyl-1,2,3,4-tetrahydronaphthalen-1-yl)phenyl)piperidin-4-yl)methyl)piperidin-4-yl)methyl)-6-oxo-6,8-dihydro-2H,7H-spiro[furo[2,3-e]isoindole-3,4'-piperidine]-7- | $^1$H NMR (600 MHz, DMSO-$d_6$) δ 10.99 (s, 1H), 9.32 (s, 1H), 9.13 (s, 1H), 7.38 – 7.26 (m, 2H), 7.20 – 7.09 (m, 3H), 6.84 (d, J = 7.0 Hz, 2H), 6.65 (d, J = 8.4 Hz, 2H), 6.62 (d, J = 2.2 Hz, 2H), 6.49 (dd, J = 8.3, 2.4 Hz, 1H), 6.26 (d, J = 8.1 Hz, 2H), 5.10 (dd, J = 13.2, 5.1 Hz, 1H), 4.66 (s, 3H), 4.40 (d, J = 17.2 Hz, 2H), 4.20 (dd, J = 44.9, 10.9 Hz, 3H), 3.31 (d, J = 10.2 Hz, 4H), 2.95 (tdd, J = 17.8, 15.0, 5.7 | 866.46 | General Synthesis Formula 37 |

| | | | | | |
|---|---|---|---|---|---|
| | | yl)piperidine-2,6-dione | Hz, 4H), 2.61 (d, J = 16.8 Hz, 3H), 2.48 – 2.36 (m, 2H), 2.27 (d, J = 14.3 Hz, 4H), 2.13 – 2.05 (m, 2H), 1.97 (dd, J = 27.3, 22.1 Hz, 5H), 1.84 – 1.68 (m, 4H), 1.35 – 1.22 (m, 3H) | | |
| **419** | N7 | 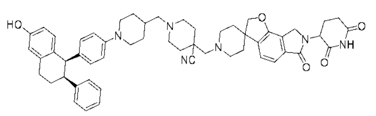 4-((7-(2,6-dioxopiperidin-3-yl)-6-oxo-7,8-dihydro-2H,6H-spiro[furo[2,3-e]isoindole-3,4'-piperidine]-1'-yl)methyl)-1-((1-(4-((1R,2S)-6-hydroxy-2-phenyl-1,2,3,4-tetrahydronaphthalen-1-yl)phenyl)piperidin-4-yl)cyanide) | $^1$H NMR (600 MHz, DMSO-$d_6$) δ 10.98 (s, 1H), 9.15 (s, 2H), 7.36 (s, 1H), 7.31 (d, J = 7.4 Hz, 1H), 7.19 – 7.10 (m, 3H), 6.84 (d, J = 7.0 Hz, 2H), 6.63 (dd, J = 16.5, 5.2 Hz, 4H), 6.49 (dd, J = 8.3, 2.4 Hz, 1H), 6.27 (s, 2H), 5.09 (dd, J = 13.3, 5.1 Hz, 1H), 4.58 (s, 2H), 4.39 (d, J = 17.1 Hz, 1H), 4.27 – 4.14 (m, 3H), 3.55 (s, 3H), 3.31 (d, J = 10.5 Hz, 3H), 3.15 (s, 2H), 3.06 – 2.88 (m, 6H), 2.68 – 2.54 (m, 3H), 2.42 (ddd, J = 13.2, 8.7, 5.2 Hz, 2H), 2.29 (s, 2H), 2.18 (s, 1H), 2.14 – 2.06 (m, 2H), 2.04 – 1.94 (m, 3H), 1.91 (d, J = 9.1 Hz, 2H), 1.80 (s, 3H), 1.76 – 1.68 (m, 2H), 1.28 (dd, J = 33.4, 8.7 Hz, 3H) | 873.46 | General Synthesis Formula 37 |
| **420** | N8 | 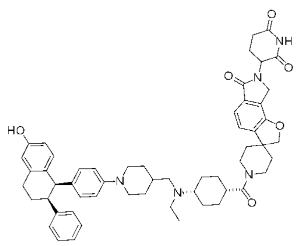 3-(1'-((1S,4s)-4-(ethyl((1-(4-((1R,2S)-6-hydroxy-2-phenyl-1,2,3,4-tetrahydronaphthalen-1-yl)phenyl)piperidin-4-yl)methyl)amino)cyclohexane-1-carbonyl)-6-oxo-6,8-dihydro-2H,7H-spiro[furo[2,3-e]isoindole-3,4'-piperidine]-7-yl)piperidine-2,6-dione | $^1$H NMR (600 MHz, DMSO-$d_6$) δ 10.99 (s, 1H), 9.15 (s, 1H), 8.48 (s, 1H), 7.41 (d, J = 7.6 Hz, 1H), 7.29 (d, J = 7.6 Hz, 1H), 7.20 – 7.07 (m, 3H), 6.84 (d, J = 7.1 Hz, 2H), 6.74 – 6.58 (m, 4H), 6.49 (dd, J = 8.2, 2.2 Hz, 1H), 6.26 (d, J = 7.2 Hz, 2H), 5.10 (dd, J = 13.2, 4.9 Hz, 1H), 4.69 – 4.60 (m, 2H), 4.40 (d, J = 17.2 Hz, 2H), 4.20 (dd, J = 37.8, 10.5 Hz, 3H), 3.89 (d, J = 12.3 Hz, 2H), 3.31 (d, J = 12.6 Hz, 2H), 3.23 – 3.13 (m, 3H), 2.94 (ddd, J = 27.3, 22.7, 10.9 Hz, 6H), 2.75 – 2.54 (m, 4H), 2.47 – 2.37 (m, 2H), 2.15 – 2.04 (m, 1H), 2.04 – 1.92 (m, 3H), 1.88 (s, 3H), 1.80 (d, J = 29.1 Hz, 4H), 1.73 (s, 4H), 1.63 (s, 2H), 1.32 (d, J = 11.1 Hz, 2H), 1.24 (t, J = 7.1 Hz, 3H) | 904.49 | General Synthesis Formula 35 |
| **421** | N9 | 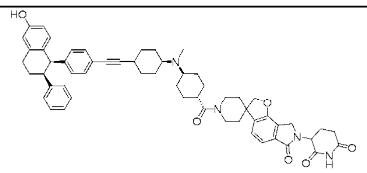 3-(1'-((1R,4r)-4-((4-((4-((1R,2S)-6- | 1H NMR (600 MHz, DMSO-$d_6$) δ 10.99 (s, 1H), 9.19 (s, 1H), 8.85 (s, 1H), 7.42 (d, J = 7.6 Hz, 1H), 7.29 (d, J = 7.6 Hz, 1H), 7.14 (dd, J = 14.5, 7.2 Hz, 3H), 6.96 (d, J = 8.2 Hz, 2H), 6.84 (d, J = 7.1 Hz, 2H), | 899.47 | General Synthesis Formula 38 |

| | | | | | |
|---|---|---|---|---|---|
| | | hydroxy-2-phenyl-1,2,3,4-tetrahydronaphthalen-1-yl)phenyl)ethynyl)cyclohexyl)(methyl)amino)cyclohexane-1-carbonyl)-6-oxo-6,8-dihydro-2H,7H-spiro[furo[2,3-e]isoindole-3,4'-piperidine]-7-yl)piperidine-2,6-dione | 6.64 (d, J = 8.6 Hz, 2H), 6.50 (dd, J = 8.3, 2.4 Hz, 1H), 6.35 (d, J = 8.2 Hz, 2H), 5.10 (dd, J = 12.3, 6.1 Hz, 1H), 4.70 – 4.61 (m, 3H), 4.40 (dd, J = 16.7, 7.9 Hz, 2H), 4.24 (dd, J = 24.2, 10.8 Hz, 3H), 3.97 (d, J = 12.5 Hz, 1H), 3.38 (s, 2H), 3.36 (s, 3H), 3.24 – 3.17 (m, 2H), 3.00 (dd, J = 16.5, 5.8 Hz, 2H), 2.96 (dd, J = 12.0, 6.1 Hz, 1H), 2.90 (d, J = 12.1 Hz, 1H), 2.70 (d, J = 14.2 Hz, 2H), 2.65 (d, J = 4.7 Hz, 2H), 2.60 (d, J = 18.4 Hz, 1H), 2.47 – 2.41 (m, 2H), 2.05 (d, J = 12.2 Hz, 3H), 2.01 (s, 4H), 1.84 (s, 1H), 1.78 (s, 1H), 1.74 (s, 3H), 1.58 (d, J = 11.2 Hz, 1H), 1.57 – 1.51 (m, 3H), 1.49 (d, J = 10.8 Hz, 2H) | | |
| **422** | N10 | 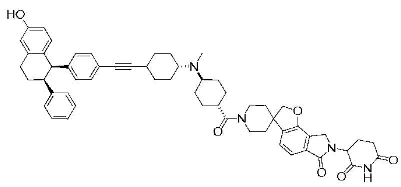 3-(1'-((1R,4r)-4-((4-((4-((1R,2S)-6-hydroxy-2-phenyl-1,2,2,3,4-tetrahydronaphthalen-1-yl)phenyl phenylphenyl)-ethinyl-ethinylcyclohexene-1-cyclohexene-4-dione | $^1$H NMR (600 MHz, DMSO-$d_6$) δ 10.99 (s, 1H), 7.42 (d, *J* = 7.5 Hz, 1H), 7.29 (d, *J* = 7.6 Hz, 1H), 7.20 – 7.11 (m, 3H), 7.04 (d, *J* = 8.2 Hz, 2H), 6.87 (d, *J* = 7.1 Hz, 2H), 6.63 (dd, *J* = 5.3, 2.9 Hz, 2H), 6.50 (dd, *J* = 8.3, 2.4 Hz, 1H), 6.38 (d, *J* = 8.2 Hz, 2H), 5.12 – 5.06 (m, 1H), 4.70 – 4.61 (m, 2H), 4.40 (dd, *J* = 16.8, 7.9 Hz, 2H), 4.28 (d, *J* = 5.2 Hz, 1H), 4.23 (dd, *J* = 16.6, 14.2 Hz, 1H), 3.97 (d, *J* = 12.7 Hz, 1H), 3.24 – 3.18 (m, 2H), 3.03 – 2.88 (m, 5H), 2.66 (d, *J* = 3.8 Hz, 3H), 2.62 – 2.58 (m, 1H), 2.47 – 2.38 (m, 2H), 2.08 (qd, *J* = 13.0, 6.4 Hz, 1H), 2.02 – 1.94 (m, 5H), 1.89 – 1.82 (m, 4H), 1.81 – 1.70 (m, 7H), 1.69 – 1.42 (m, 8H) | 899.47 | General Synthesis Formula 38 |
| **423** | N11 | 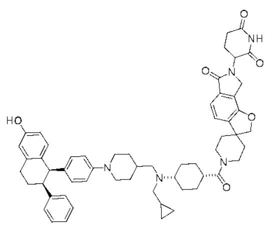 3-(1'-((1S,4s)-4-((cyclopropylmethyl)((1-(4-((1R,2S)-6-hydroxy-2-phenyl-1,2,3,4-tetrahydronaphthalen-1-yl)phenyl)piperidin-4- | 1H NMR (600 MHz, DMSO-$d_6$) δ 10.99 (s, 1H), 9.15 (s, 1H), 8.58 (s, 1H), 7.41 (d, J = 7.6 Hz, 1H), 7.29 (d, J = 7.6 Hz, 1H), 7.17 – 7.09 (m, 3H), 6.83 (t, J = 7.0 Hz, 2H), 6.72 – 6.60 (m, 4H), 6.49 (dt, J = 8.3, 4.0 Hz, 1H), 6.26 (d, J = 6.5 Hz, 2H), 5.10 (dd, J = 13.1, 4.8 Hz, 1H), 4.64 (dt, J = 19.0, 9.6 Hz, 2H), 4.39 (d, J = 17.1 Hz, 2H), 4.20 (dd, J = 36.5, 10.4 Hz, 3H), 3.87 (s, 1H), 3.57 (s, 3H), 3.41 – 3.29 (m, 3H), 3.23 – 3.16 (m, 1H), 3.07 – 3.00 (m, 4H), | 930.51 | General Synthesis Formula 35 |

| | | | | | |
|---|---|---|---|---|---|
| | | yl)methyl)amino)cyclohexane-1-carbonyl)-6-oxo-6,8-dihydro-2H,7H-spiro[furo[2,3-e]isoindole-3,4'-piperidine]-7-yl)piperidine-2,6-dione | 2.98 – 2.87 (m, 3H), 2.69 (d, J = 24.3 Hz, 2H), 2.64 – 2.53 (m, 2H), 2.46 – 2.37 (m, 2H), 2.12 – 2.05 (m, 1H), 1.97 (dd, J = 11.5, 6.5 Hz, 2H), 1.93 – 1.78 (m, 7H), 1.73 (s, 3H), 1.62 (s, 1H), 1.39 – 1.23 (m, 3H), 1.10 (d, J = 7.1 Hz, 1H), 0.69 – 0.63 (m, 2H), 0.48 – 0.37 (m, 2H) | | |
| **424** | O1 | 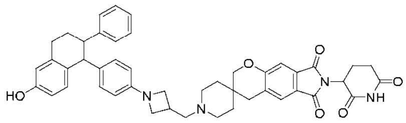 7'-(2,6-dioxopiperidin-3-yl)-1-((1-(4-(6-hydroxy-2-phenyl-1,2,3,4-tetrahydronaphthalen-1-yl)phenyl)azetidin-3-yl)methyl)-2'H-pyrido[piperidine-4,3'-pyrano[2,3-f]isoindole]-6',8'(4'H,7'H)-dione | $^1$H NMR (400 MHz, DMSO-$d_6$) δ 11.09 (s, 1H), 7.70 (s, 1H), 7.60 (s, 1H), 7.29 (d, *J* = 1.8 Hz, 1H), 7.17 – 7.09 (m, 3H), 6.82 (d, *J* = 6.5 Hz, 2H), 6.62 (d, *J* = 8.4 Hz, 1H), 6.60 (d, *J* = 2.0 Hz, 1H), 6.48 (dd, *J* = 8.2, 2.3 Hz, 1H), 6.20 (d, *J* = 8.3 Hz, 2H), 6.06 (d, *J* = 7.7 Hz, 2H), 5.09 (dd, *J* = 12.8, 5.3 Hz, 1H), 4.30 (s, 1H), 4.12 (d, *J* = 4.7 Hz, 1H), 4.02 (s, 1H), 3.50 – 3.23 (m, 8H), 3.18 – 3.03 (m, 4H), 3.02 – 2.82 (m, 4H), 2.78 (s, 1H), 2.69 – 2,53 (m, 1H), 2.15 – 1.94 (m, 3H), 1.77 – 1.56 (dd, *J* = 31.8, 17.1 Hz, 5H) | 751.63 | General Synthesis Formula 1 |
| **425** | O2 | 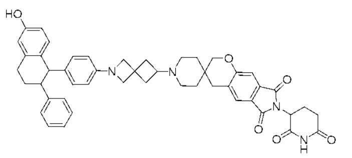 7'-(2,6-dioxopiperidin-3-yl)-1-(2-(4-(6-hydroxy-2-phenyl-1,2,3,4-tetrahydronaphthalen-1-yl)phenyl)-2-azaspiro[3.3]heptan-6-yl)-2'-pyrido[piperidine-4,3'-pyrano[2,3-f]isoindole]-6'-8'-(4H',7H')-dione | $^1$H NMR (600 MHz, DMSO-$d_6$) δ 11.10 (s, 1H), 7.70 (s, 1H), 7.60 (s, 1H), 7.29 (d, *J* = 4.2 Hz, 1H), 7.12 (dt, *J* = 20.9, 7.1 Hz, 3H), 6.81 (d, *J* = 7.3 Hz, 2H), 6.61 (d, *J* = 8.3 Hz, 1H), 6.59 (d, *J* = 1.7 Hz, 1H), 6.47 (d, *J* = 8.2 Hz, 1H), 6.17 (d, *J* = 8.3 Hz, 2H), 6.05 (d, *J* = 8.3 Hz, 2H), 5.09 (dd, *J* = 12.8, 5.4 Hz, 1H), 4.29 (s, 1H), 4.11 (d, *J* = 4.9 Hz, 1H), 4.04 (s, 1H), 3.73 (d, *J* = 3.6 Hz, 1H), 3.63 (s, 2H), 3.31 – 3.25 (m, 3H), 3.04 (s, 1H), 2.91 (ddd, *J* = 30.8, 20.5, 7.8 Hz, 4H), 2.79 (s, 1H), 2.62 – 2.57 (m, 1H), 2.39 (t, *J* = 10.0 Hz, 2H), 2.11 – 2.06 (m, 1H), 2.04 – 1.95 (m, 4H), 1. 79 – 1.67 (dd, *J* = 30.6, 8.3 Hz, 3H), 1.66 – 1.54 (m, 3H), 1.46 (dd, *J* = 14.2, 7.1 Hz, 1H), 1.33 – 1.27 (m, 1H) | 777.73 | General Synthesis Formula 6 |
| **426** | O3 | 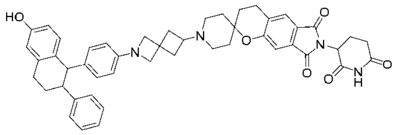 7'-(2,6-dioxopiperidin-3-yl)-1-(2-(4-(6-hydroxy-2-phenyl-1,2,3,4- | 1H NMR (600 MHz, DMSO-$d_6$) δ 11.11 (s, 1H), 7.75 (m, 1H), 7.37 (s, 1H), 7.16 – 7.10 (m, 3H), 6.82 (d, J = 7.3 Hz, 2H), 6.63 – 6.59 (m, 2H), 6.48 (m, 2H), 6.18 (d, J = 8.1 Hz, 2H), 6.06 (d, J = 8.1 Hz, | 777.67 | General Synthesis Formula 6 |

| | | | | | |
|---|---|---|---|---|---|
| | | tetrahydronaphthalen-1-yl)phenyl)-2-azaspiro[3.3]heptan-6-yl)-3'-dihydro-6'-pyrido[piperidine-4,2'-pyrano[2,3-f]isoindole]-6',8'-dione | 1H), 5.10 (dd, J = 13.0, 5.3 Hz, 1H), 4.12 (d, J = 5.0 Hz, 1H), 3.74 (t, J = 8.8 Hz, 2H), 3.28 (m, 3H), 3.05 (t, J = 11.9 Hz, 2H), 2.99 – 2.84 (m, 6H), 2.59 (dd, J = 9.9, 6.7 Hz, 1H), 2.42 (m, 2H), 2.11 – 1.97 (m, 7H), 1.93 (t, J = 6.8 Hz, 2H), 1.86 – 1.75 (m, 2H), 1.70 (m, 1H), 1.45 (m, 1H) | | |

Example 427

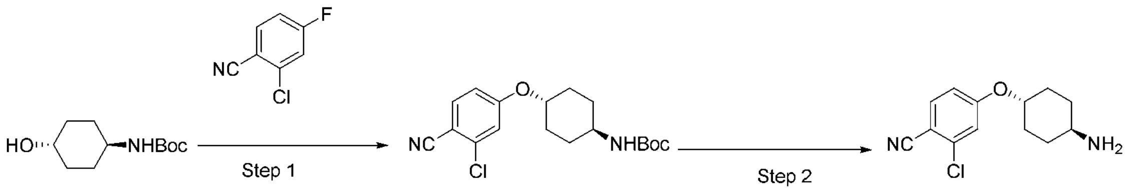


2-18a 2-18b 2-18c

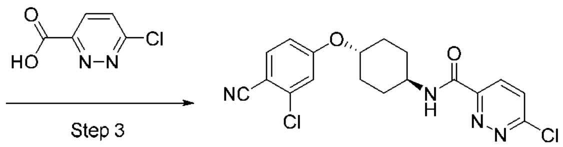


Intermediate 2-18

Step 1: The Intermediate 2-18a (20 g, 92.9 mmol) was dissolved in tetrahydrofuran (100 mL) in a single-necked flask, cooled to 0 °C, and sodium hydride (8.36 g, 209.02 mmol) was added slowly. The mixture was stirred at 0 °C for 1 h. A solution of 2-chloro-4-fluorobenzonitrile (20 g, 92.9 mmol) in tetrahydrofuran (100 mL) was added to the flask, and the reaction was stirred at 0 °C for 0.5 h. The mixture was then allowed to warm to room temperature and stirred for an additional 3 h. After completion, the reaction was cooled to 0 °C, quenched with ice water, and extracted three times with ethyl acetate (100 mL each). The organic phase was washed with saturated brine, dried over anhydrous sodium sulfate, filtered, and concentrated to afford the crude product. The crude product was purified by column chromatography (0-25% ethyl acetate) to yield a white solid intermediate 2-18b (21.92 g).

$^1$H NMR (600 MHz, $CD_3OD$) δ 7.69 (d, *J* = 8.8 Hz, 1H), 7.18 (d, *J* = 2.4 Hz, 1H), 7.03 (dd, *J* = 8.8, 2.4 Hz, 1H), 4.43 (tt, *J* = 10.2, 4.1 Hz, 1H), 3.42 (dq, *J* = 10.9, 5.8 Hz, 1H), 2.18 - 2.10 (m, 2H), 2.07 - 1.97 (m, 2H), 1.61 - 1.51 (m, 2H), 1.46 (s, 9H).

Step 2: Intermediate 2-18b (21.92 g, 62.48 mmol) was added to a single-necked flask containing dioxane (25 mL). A solution of hydrochloric acid in dioxane (25 mL) was added to the reaction flask. After completion, the mixture was stirred at room temperature for 2 h. After completion, the reaction was concentrated directly to afford a white solid crude product, intermediate 2-18c (20.05 g).

$^1$H NMR (600 MHz, DMSO-$d_6$) δ 8.18 (s, 3H), 7.86 (d, *J* = 8.7 Hz, 1H), 7.42 (dd, *J* = 2.5, 0.9 Hz, 1H), 7.14 (ddd, *J* = 8.8, 2.4, 1.0 Hz, 1H), 4.52 (tt, *J* = 9.6, 4.2 Hz, 1H), 3.10 - 3.01 (m, 1H), 2.10 (dd, *J* = 12.6, 4.4 Hz, 1H), 2.00 (d, *J* = 11.8 Hz, 1H), 1.58 - 1.41 (m, 1H).

Step 3: The intermediate 2-18c (20.05 g, 79.97 mmol), triethylamine (32.37 g, 319.87 mmol), propylphosphonic anhydride (12.2 g, 96 mmol), and dichloromethane (200 mL) were added to a single-necked flask sequentially. The mixture was stirred at room temperature for 5 min. 6-Chloropyridazine-3-carboxylic acid (16.48 g, 103.96 mmol) was added slowly to the flask, and the temperature was raised to 30 °C. The reaction was stirred for an additional 2.5 h. After completion, the mixture was concentrated and purified by trituration (petroleum ether: ethyl acetate = 1:1) to afford a brown solid intermediate 2-18 (13.45 g).

$^1$H NMR (600 MHz, DMSO-$d_6$) δ 9.17 (d, *J* = 8.2 Hz, 1H), 8.23 (d, *J* = 8.9 Hz, 1H), 8.11 (d, *J* = 8.9 Hz, 1H), 7.87 (d, *J*

= 8.7 Hz, 1H), 7.41 (d, *J* = 2.4 Hz, 1H), 7.15 (dd, *J* = 8.8, 2.4 Hz, 1H), 4.54 (td, *J* = 10.5, 5.1 Hz, 1H), 3.91 (dtd, *J* = 11.5, 7.7, 4.1 Hz, 1H), 2.13 (d, *J* = 12.3 Hz, 2H), 1.90 (s, 2H), 1.71 (q, *J* = 13.4 Hz, 2H), 1.57 - 1.48 (m, 2H).

Example 428

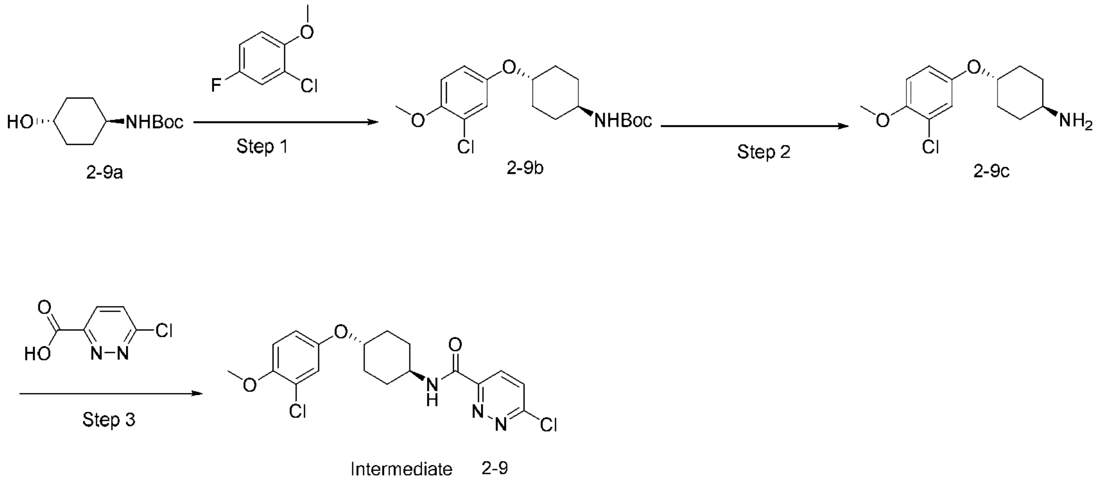


2-9a 2-9b 2-9c

Intermediate 2-9

Step 1: Prepared according to Step 1 of Example 427.
Step 2: Prepared according to Step 2 of Example 427.
Step 3: Prepared according to Step 3 of Example 427.
$^1$H NMR (600 MHz, DMSO-$d_6$) δ 9.14 (d, *J* = 8.2 Hz, 1H), 8.23 (d, *J* = 8.9 Hz, 1H), 8.11 (d, *J* = 8.8 Hz, 1H), 7.64 - 7.59 (m, 1H), 6.73 (d, *J* = 8.1 Hz, 2H), 4.49 (tt, *J* = 10.5, 4.2 Hz, 1H), 3.90 (s, 4H), 2.19 - 2.10 (m, 2H), 1.95 - 1.88 (m, 2H), 1.67-1.74 (m, 2H), 1.49-1.55 (m, 2H).

Example 429

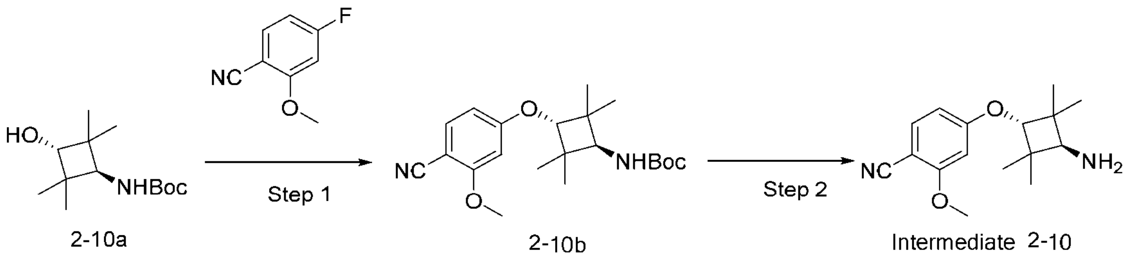


2-10a 2-10b Intermediate 2-10

Step 1: Prepared according to Step 1 of Example 427.

LC-MS: [M+H] $^+$ = 375.49.

Step 2: Prepared according to Step 2 of Example 427.

LC-MS: [M+H] $^+$ = 275.37.
$^1$H NMR (600 MHz, DMSO-$d_6$) δ 8.48 (d, *J* = 6.0 Hz, 2H), 7.64 (d, *J* = 8.6 Hz, 1H), 6.63 (d, *J* = 2.2 Hz, 1H), 6.53 (dd, *J* = 8.6, 2.2 Hz, 1H), 4.31 (s, 1H), 3.90 (s, 3H), 3.07 (d, *J* = 5.4 Hz, 1H), 1.32 (s, 6H), 1.11 (s, 6H).

Example 430

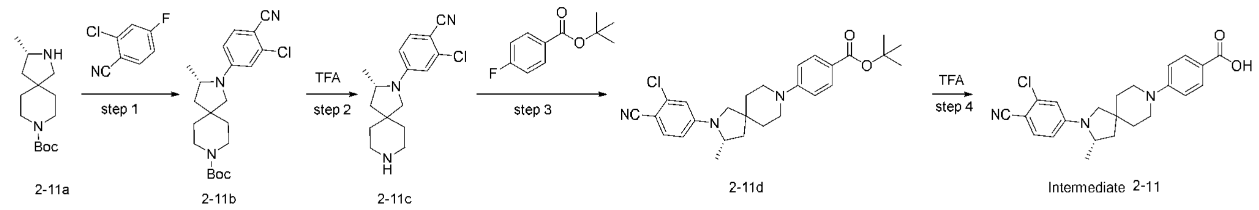


Step 1: The (S)-3-methyl-2,8-diazaspiro[4.5]decane-8-carboxylic acid tert-butyl ester (4 g, 15.72 mmol), 2-chloro-4-fluorobenzonitrile (4.89 g, 31.45 mmol), DIEA (6.1 g, 47.16 mmol) and DMSO (40 mL) were added sequentially to a reaction flask. Under nitrogen atmosphere, the mixture was stirred at 100 °C for 4 h. After completion, the reaction was cooled to 0 °C, and water and ethyl acetate were added for extraction and phase separation. The organic phase was concentrated under vacuum, and the residue was purified by normal-phase chromatography (PE: EA = 4:1) to afford a yellow solid intermediate 2-11b (4.3 g, 70.13%).

LC-MS: $[M\text{-}t\text{-}Bu+H]^+$=334.39.

Step 2: (Intermediate 2-11c) Prepared according to Step 2 of Example 432.

LC-MS: $[M+H]^+$ = 290.38.

Step 3: (Intermediate 2-11d)

The compound 2-11c (1 g, 3.45 mmol), tert-butyl 4-fluorobenzoate (1.02 g, 5.18 mmol), $K_2CO_3$ (2.4 g, 17.25 mmol) and DMSO (10 mL) were added sequentially to a reaction flask. Under nitrogen atmosphere, the mixture was stirred at 100 °C for 12 h. After completion, the reaction was cooled to 0 °C, and water and ethyl acetate were added for extraction and phase separation. The organic phase was concentrated under vacuum, and the crude product was purified by normal-phase chromatography (PE: EA = 4:1) to afford a yellow solid intermediate 2-11d (660 mg, 41.04%).
LC-MS: $[M+H]^+$ = 466.39

Step 4: (Intermediate 2-11) Prepared according to Step 2 of Example 432.

$^1$H NMR (600 MHz, DMSO-$d_6$) δ 7.74 (d, $J$ = 8.9 Hz, 2H), 7.58 (d, $J$ = 8.8 Hz, 1H), 6.98 - 6.91 (m, 2H), 6.78 (d, $J$ = 2.4 Hz, 1H), 6.64 (dd, $J$ = 8.9, 2.4 Hz, 1H), 4.02 (m, 1H), 3.44 (s, 2H), 3.31 (d, $J$ = 10.6 Hz, 4H), 2.22 (dd, $J$ = 12.8, 7.7 Hz, 1H), 1.77 - 1.63 (m, 2H), 1.56 (dd, $J$ = 12.8, 6.5 Hz, 1H), 1.46 (m, 2H), 1.19 (d, $J$ = 6.1 Hz, 3H).

LC-MS: $[M+H]^+$=410.30.

Example 431

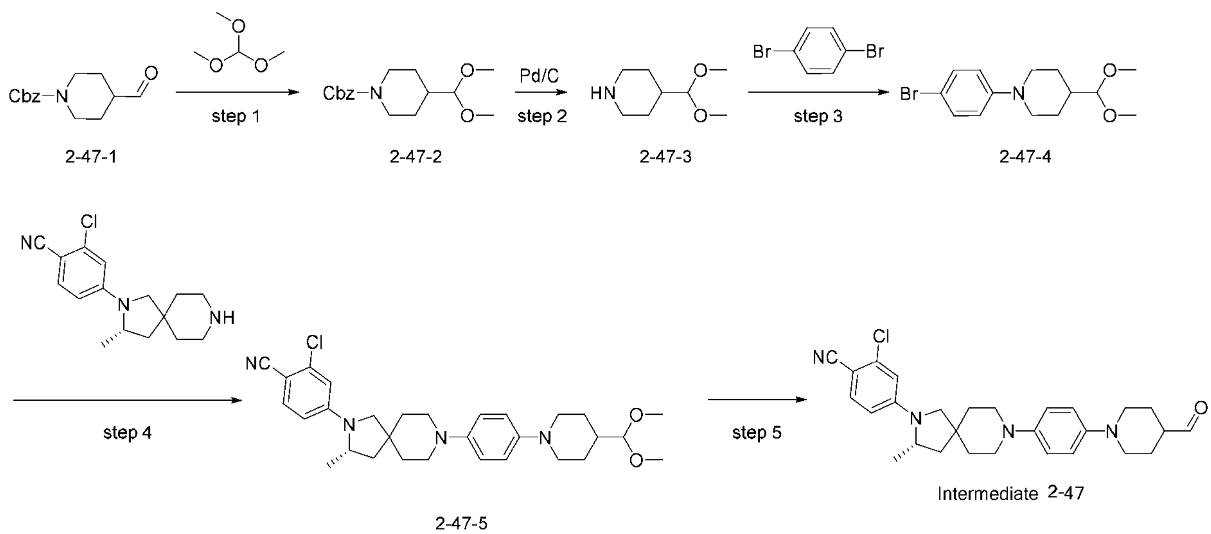

Step 1: (Compound 2-47-2)

The trimethyl orthoformate (27 g, 255 μmol) and hydrochloric acid (0.2 mL) were added sequentially to a solution of 4-formyl-N-Cbz-piperidine (21 g, 85 mmol) in methanol (210 mL). Under nitrogen atmosphere, the mixture was stirred at 70 °C for 2 h. After completion, the reaction was concentrated to afford a colorless oily compound 47-2 (22 g, 88.31%), which was used directly in the next step.

Step 2: (Compound 2-47-3)

Pd/C (500 mg, 10%) was added to a solution of compound 2-47-2 (5 g, 17 mmol) in methanol (210 mL). The mixture was purged with hydrogen three times and stirred at 40 °C for 12 h. After completion, the reaction was filtered, and the filtrate was concentrated to afford a white solid compound 2-47-3 (2.5 g, 92.12%), which was used directly in the next step.

Step 3: (Compound 2-47-4)

The compound 2-47-3 (3 g, 18.84 mmol), 1,4-dibromobenzene (13.33 g, 56.52 mmol), $Pd(OAc)_2$ (426 mg, 1.884 mmol), BINAP (2.3 g, 3.768 mmol), and $Cs_2CO_3$ (18.4 g, 56.52 mmol) was added sequentially to a glass vial, and then dioxane (30 mL) was added. Under nitrogen atmosphere, the mixture was stirred at 110 °C for 3 h. After completion, the reaction was filtered, and the filtrate was purified by normal-phase chromatography (PE: EA = 4:1) to afford a yellow solid compound 2-47-4 (1.7 g, 28.72%).

LC-MS: $[M+H]^+$ = 316.38.

Step 4: (Compound 2-47-5)

The 11c (4.7 g, 16.22 mmol), compound 2-47-4 (6.12 g, 19.46 mmol), $Pd_2(dba)_3$ (1.5 g, 1.622 mmol), X-phos (1.5 g, 3.244 mmol), and $Cs_2CO_3$ (15.8 g, 48.66 mmol) were added sequentially to a glass vial, and then dioxane (47 mL) was added. Under nitrogen atmosphere, the mixture was stirred at 110 °C for 3 h. After completion, the reaction was filtered, and the filtrate was purified by normal-phase chromatography (PE: EA = 3:1) to afford a yellow solid compound 2-47-5 (4 g, 47.15%).

LC-MS: $[M+H]^+$ = 523.49.

Step 5: (Intermediate 2-47)

The compound 2-47-5 (4 g, 7.65 mmol) was added to a reaction flask in a mixture of DCM/$H_2O$ (40/2 mL). TFA (8 mL) was added dropwise, and the mixture was stirred at room temperature for 2 h. After completion, the reaction was concentrated, and the crude product was purified by normal-phase chromatography (DCM: MeOH = 20:1) to afford a green solid intermediate 2-47 (2 g, 54.83%).

LC-MS: $[M+H]^+$ = 477.40.

$^1$H NMR (400 MHz, DMSO-$d_6$) δ 9.65 (s, 1H), 7.62 (d, *J* = 8.8 Hz, 1H), 7.12 (s, 4H), 6.81 (d, *J* = 2.3 Hz, 1H), 6.67 (dd, *J* = 8.9, 2.4 Hz, 1H), 4.05 (m, 1H), 3.58 (s, 5H), 3.17 (s, 2H), 2.58 (s, 1H), 2.25 (s, 1H), 2.08 (s, 1H), 1.99 (s, 2H), 1.87 (s, 2H), 1.63 (s, 6H), 1.21 (d, *J* = 6.0 Hz, 4H).

Example 432

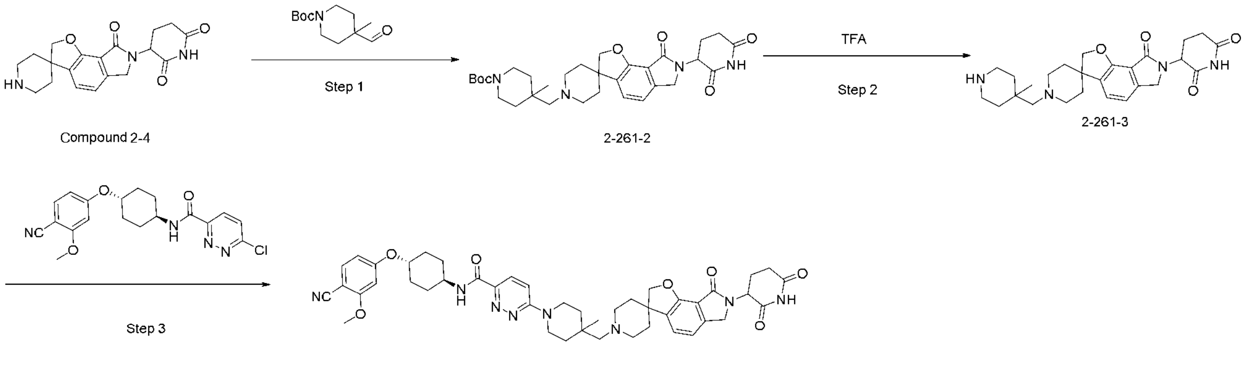


Compound 2-4

2-261-2

2-261-3

Compound 2-261

Step 1: The compound 4 (100.0 mg, 281.38 μmol) and tert-butyl 4-formyl-4-methylpiperidine-1-carboxylate (76.75 mg, 337.65 μmol) were added sequentially to a reaction flask, and then THF (2 mL) and $Ti(O\text{-}iPr)_4$ (159.95 mg, 562.76 μmol) were added, and the mixture was stirred at 60 °C for 1 h. $NaBH_3CN$ (53.05 mg, 844.13 μmol) was then added, and the reaction was stirred at 60 °C for an additional 1 h. After completion, the mixture was concentrated and purified by preparative thin-layer chromatography (DCM:MeOH = 10:1) to afford a white solid compound 2-261-2 (50 mg, 31.36%).

LC-MS: $[M+H]^+$ = 567.58.

Step 2: The compound 2-261-2 (40.0 mg, 70.58 μmol) was added to a reaction flask, and DCM (1 mL) and TFA (1 mL) were added, and the mixture was stirred at room temperature for 1 h. After completion, the reaction was concentrated and purified by reversed-phase column chromatography (MeCN in water = 30%) to afford a white solid compound 2-261-3 (30 mg, 91.09%).

LC-MS: $[M+H]^+$ = 467.49.

Step 3: The compound 2-261-3 (20.0 mg, 42.86 μmol) and intermediate 10 (19.90 mg, 51.44 μmol) were added sequentially to a reaction flask, and DMSO (1 mL) and DIEA (16.62 mg, 128.59 μmol) were added, and the mixture was stirred at 80 °C for 2 h. After completion, the reaction was concentrated and purified by preparative HPLC to afford a white solid compound 2-261 (4 mg, 11.42%).

$^1$H NMR (600 MHz, DMSO-$d_6$) δ 10.98 (s, 1H), 8.60 (d, *J* = 8.2 Hz, 1H), 7.85 (d, *J* = 9.5 Hz, 1H), 7.62 (d, *J* = 8.8 Hz, 1H), 7.39 (d, *J* = 9.6 Hz, 1H), 7.33 (d, *J* = 7.5 Hz, 1H), 7.12 (d, *J* = 3.3 Hz, 1H), 6.73 (d, *J* = 7.7 Hz, 2H), 5.05 (dd, *J* = 13.3, 5.1 Hz, 1H), 4.67 (d, *J*= 6.7 Hz, 2H), 4.50 (tt, *J* = 9.8, 4.3 Hz, 1H), 4.41 (d, *J* = 17.3 Hz, 1H), 4.27 (d, *J* = 17.3 Hz, 1H), 4.19 - 4.06 (m, 2H), 3.89 (s, 3H), 3.87 - 3.83 (m, 1H), 3.59 (s, 2H), 3.34 (s, 2H), 3.27 (d, *J* = 11.5 Hz, 2H), 3.17 (s, 1H), 2.91 (ddd, *J* = 18.1, 13.5, 5.5 Hz, 1H), 2.63 - 2.57 (m, 1H), 2.36 (qd, *J* = 13.2, 4.5 Hz, 3H), 2.26 (s, 1H), 2.15 - 2.12 (m, 2H), 1.98 - 1.88 (m, 5H), 1.64 (qd, *J* = 13.1, 12.1, 5.8 Hz, 6H), 1.56 - 1.48 (m, 2H), 1.28 (d, *J*= 12.2 Hz, 3H).

LC-MS: $[M+H]^+$=817.66.

Example 433

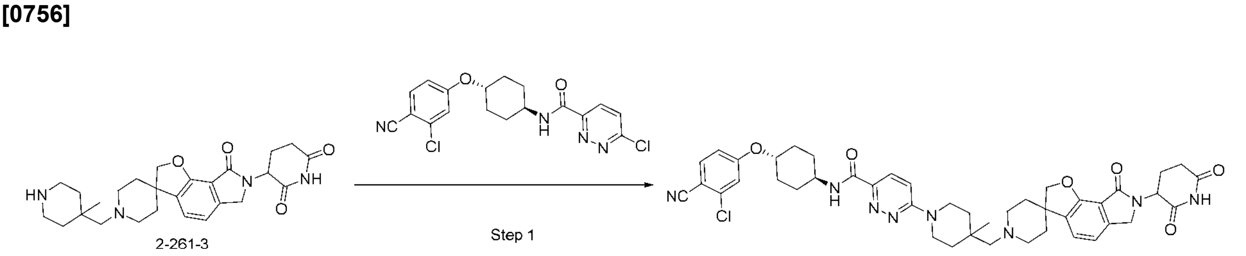


2-261-3

Compound 2-262

Step 1: (Compound 2-262) Prepared according to Step 3 of Example 432.

$^1$H NMR (600 MHz, DMSO-$d_6$) δ 10.97 (s, 1H), 8.60 (d, *J*= 8.2 Hz, 1H), 7.86 (dd, *J* = 9.1, 5.2 Hz, 2H), 7.43 - 7.36 (m, 2H), 7.33 (d, *J* = 7.5 Hz, 1H), 7.13 (ddd, *J*= 16.7, 8.3, 2.7 Hz, 2H), 5.06 (dd, *J* = 13.3, 5.0 Hz, 1H), 4.67 (d, *J* = 8.0 Hz, 2H), 4.55 - 4.53 (m, 1H), 4.41 (d, *J* = 17.3 Hz, 1H), 4.26 (s, 1H), 4.12 (dt, *J* = 13.9, 4.7 Hz, 2H), 3.87 (dq, *J* = 11.3, 3.6 Hz, 1H), 3.59 (d, *J* = 12.1 Hz, 2H), 3.47 (ddd, *J* = 13.7, 9.6, 3.9 Hz, 2H), 3.44 - 3.20 (m, 4H), 3.17 (d, *J* = 4.0 Hz, 2H), 2.95 - 2.88 (m, 1H), 2.63 - 2.57 (m, 1H), 2.35 (td, *J* = 13.3, 4.4 Hz, 3H), 2.31 - 2.18 (m, 1H), 2.16 - 2.08 (m, 2H), 1.97 (qd, *J* = 6.2, 5.7, 2.9 Hz, 1H), 1.90 (t, *J* = 7.1 Hz, 4H), 1.72 - 1.57 (m, 6H), 1.56 - 1.48 (m, 2H).

LC-MS: $[M+H]^+$=821.56.

Example 434

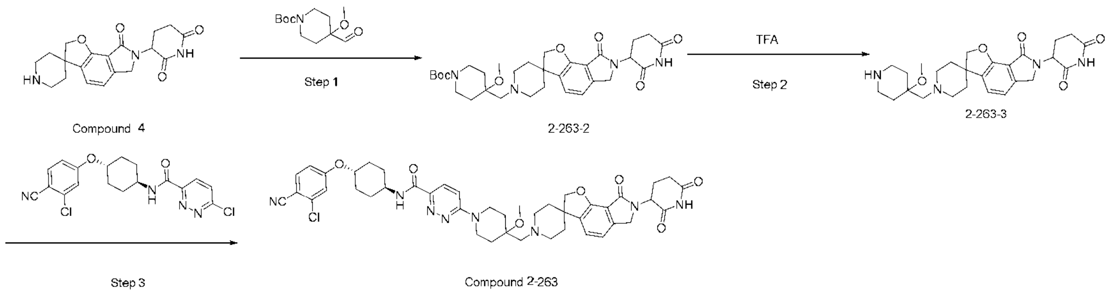


Compound 4

2-263-2

2-263-3

Compound 2-263

Step 1: (Compound 2-263-2) Prepared according to Step 1 of Example 432.

LC-MS: $[M+H]^+$ =583.48.

Step 2: (Compound 2-263-3) Prepared according to Step 2 of Example 432.

LC-MS: $[M+H]^+$=483.39.

Step 3: (Compound 2-263) Prepared according to Step 3 of Example 432.

$^1$H NMR (600 MHz, DMSO-$d_6$) δ 10.98 (s, 1H), 8.63 (d, *J* = 8.1 Hz, 1H), 7.86 (dd, *J* = 7.3, 4.4 Hz, 2H), 7.44 (d, *J* = 9.3 Hz, 1H), 7.39 (d, *J* = 2.7 Hz, 1H), 7.31 (dd, *J* = 7.6, 2.5 Hz, 1H), 7.13 (ddd, *J* = 15.0, 8.4, 2.6 Hz, 2H), 5.06 (dd, *J* = 13.3, 5.1 Hz, 1H), 4.68 (s, 2H), 4.54 (dd, *J* = 9.8, 5.3 Hz, 1H), 4.41 (d, *J* = 17.3 Hz, 1H), 4.29 (s, 1H), 4.26 (s, 2H), 3.87 (d, *J* = 6.7 Hz, 1H), 3.63 (s, 2H), 3.33 (d, *J* = 11.6 Hz, 2H), 3.30 (s, 3H), 2.92 (td, *J* = 13.0, 6.8 Hz, 1H), 2.63 - 2.57 (m, 1H), 2.45 - 2.28 (m, 4H), 2.14 - 2.09 (m, 2H), 2.05 - 1.93 (m, 4H), 1.90 (d, *J* = 12.5 Hz, 4H), 1.65 (q, *J* = 12.2, 11.3 Hz, 5H), 1.52 (dt, *J* = 15.6, 10.8 Hz, 3H).

LC-MS: $[M+H]^+$=837.66.

Example 435

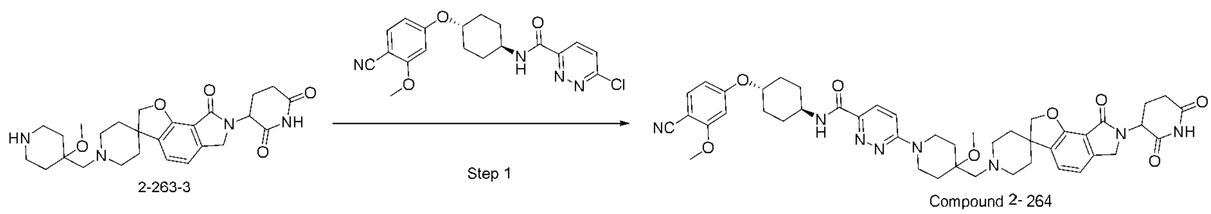


2-263-3

Compound 2- 264

Step 3: (Compound 2-264) Prepared according to Step 3 of Example 432.

$^1$H NMR (600 MHz, DMSO-$d_6$) δ 10.98 (s, 1H), 8.62 (d, *J* = 8.2 Hz, 1H), 7.87 (d, *J* = 9.4 Hz, 1H), 7.62 (d, *J* = 8.6 Hz, 1H), 7.44 (d, *J* = 9.6 Hz, 1H), 7.31 (d, *J* = 7.4 Hz, 1H), 7.12 (d, *J* = 7.6 Hz, 1H), 6.73 (d, *J* = 7.3 Hz, 2H), 5.06 (dd, *J*= 13.3, 5.1 Hz, 1H), 4.68 (s, 2H), 4.49 (dq, *J* = 9.8, 5.0, 4.2 Hz, 1H), 4.41 (d, *J* = 17.3 Hz, 1H), 4.27 (dd, *J*= 17.1, 7.5 Hz, 3H), 3.90 (s, 3H), 3.88 - 3.86 (m, 1H), 3.65 - 3.62 (m, 2H), 3.40 - 3.36 (m, 2H), 3.32 (d, *J* = 22.0 Hz, 6H), 2.91 (ddd, *J*= 18.0, 13.5, 5.5 Hz, 1H), 2.63 - 2.57 (m, 1H), 2.55 (s, 1H), 2.43 - 2.26 (m, 3H), 2.17 - 2.08 (m, 2H), 2.08 - 1.81 (m, 7H), 1.65 (td, *J*= 14.1, 7.0 Hz, 4H), 1.52 (dt, *J*= 13.9, 10.1 Hz, 2H).

LC-MS: $[M+H]^+$=833.66.

Example 436

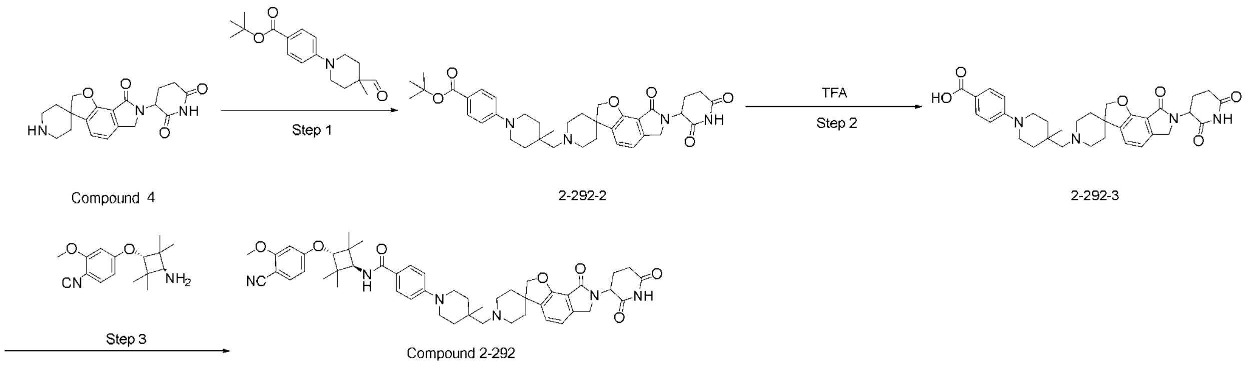


Compound 4

2-292-2

2-292-3

Compound 2-292

Step 1: (Compound 2-292-2) Prepared according to Step 1 of Example 432.

LC-MS: $[M+H]^+$=643.58.

Step 2: (Compound 2-292-3) Prepared according to Step 2 of Example 432.

LC-MS: $[M+H]^+$=587.48.

Step 3: (Compound 2-292) Prepared according to Step 3 of Example 427.

$^1$H NMR (600 MHz, DMSO-$d_6$) δ 10.97 (s, 1H), 7.78 (d, *J* = 8.7 Hz, 2H), 7.66 (d, *J*= 8.6 Hz, 1H), 7.52 (d, *J* = 9.2 Hz, 1H), 7.33 (d, *J* = 7.5 Hz, 1H), 7.12 (d, *J* = 7.7 Hz, 1H), 7.01 (d, *J* = 8.8 Hz, 2H), 6.65 (d, *J* = 1.9 Hz, 1H), 6.55 (dd, *J* = 8.6, 2.0 Hz, 1H), 5.06 (dd, *J* = 13.3, 4.9 Hz, 1H), 4.68 (d, *J* = 7.0 Hz, 2H), 4.41 (d, *J* = 17.4 Hz, 1H), 4.27 (d, *J* = 15.7 Hz, 2H), 4.06 (d, *J* = 9.2 Hz, 1H), 3.92 (s, 3H), 3.57 (d, *J* = 13.0 Hz, 4H), 3.32 (dd, *J* = 51.3, 10.8 Hz, 3H), 3.20 - 3.12 (m, 4H), 2.91 (ddd, *J* = 22.6, 12.1, 5.3 Hz, 1H), 2.60 (d, *J* = 17.0 Hz, 1H), 2.43 - 2.32 (m, 3H), 2.00 - 1.86 (m, 3H), 1.66 (dd, *J* = 41.4, 11.3 Hz, 4H), 1.28 - 1.20 (m, 8H), 1.16 (s, 6H).

LC-MS: $[M+H]^+$=843.76.

Example 437

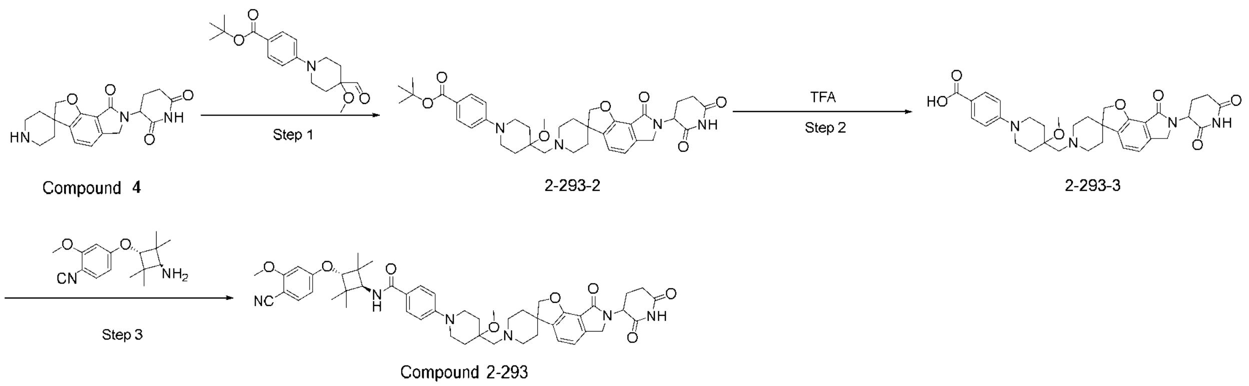


Compound 4

2-293-2

2-293-3

Compound 2-293

Step 1: (Compound 2-293-2) Prepared according to Step 1 of Example 432.

LC-MS: $[M+H]^+$ =659.57.

Step 2: (Compound 2-293-3) Prepared according to Step 2 of Example 432.

LC-MS: $[M+H]^+$=603.40.

Step 3: (Compound 2-293) Prepared according to Step 3 of Example 427.

$^1$H NMR (600 MHz, DMSO-$d_6$) δ 10.97 (s, 1H), 7.79 (d, *J* = 8.7 Hz, 2H), 7.66 (d, *J* = 8.6 Hz, 1H), 7.53 (d, *J* = 9.2 Hz, 1H), 7.32 (d, *J* = 7.5 Hz, 1H), 7.12 (d, *J* = 7.6 Hz, 1H), 7.02 (d, *J* = 8.9 Hz, 2H), 6.65 (d, *J* = 2.1 Hz, 1H), 6.55 (dd, *J* = 8.7, 2.1 Hz, 1H), 5.06 (dd, *J* = 13.3, 5.1 Hz, 1H), 4.69 (s, 2H), 4.41 (d, *J* = 17.5 Hz, 1H), 4.27 (d, *J* = 16.8 Hz, 2H), 4.07 (d, *J* = *9.2* Hz, 1H), 3.92 (s, 3H), 3.73 - 3.65 (m, 4H), 3.38 (s, 2H), 3.29 (d, *J* = 16.2 Hz, 5H), 3.09 (t, *J* = 11.3 Hz, 2H), 2.91 (ddd, *J* = 22.6, 12.5, 5.3 Hz, 1H), 2.63 - 2.57 (m, 1H), 2.40 - 2.31 (m, 3H), 2.02 - 1.86 (m, 5H), 1.70 (t, *J* = 10.4 Hz, 2H), 1.23 (s, 6H), 1.16 (s, 6H).

LC-MS: $[M+H]^+$=859.66.

Example 438

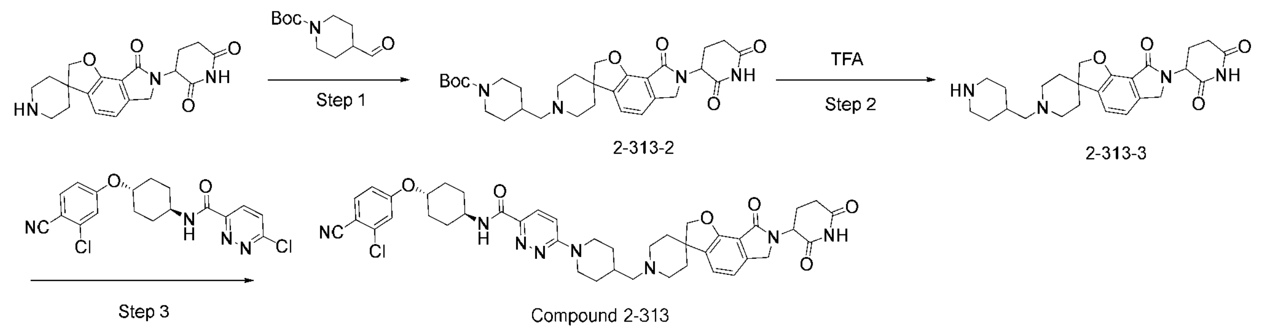

2-313-2
2-313-3
Compound 2-313

Step 1: (Compound 313-2)

In a reaction flask, compound 4 (50.0 mg, 140.69 μmol) and tert-butyl 4-formylpiperidine-1-carboxylate (59.44 mg, 281.38 μmol) were sequentially added, followed by the addition of THF (1.6 mL) as solvent. The reaction mixture was stirred at room temperature for 1 hour, and then $NaBH_3CN$ (89.45 mg, 422.07 μmol) was added. The mixture was stirred at room temperature for an additional 1 hour. After completion, the mixture was concentrated under reduced pressure and purified by preparative thin-layer chromatography (DCM: MeOH = 10:1) to afford a white solid compound 2-313-2 (70 mg, 45.01%).

LC-MS: $[M+H]^+$ =553.49.

Step 2: (Compound 2-313-3) Prepared according to Step 2 of Example 432.

LC-MS: $[M+H]^+$=453.40.

Step 3: (Compound 2-313) Prepared according to Step 3 of Example 432.

$^1$H NMR (600 MHz, DMSO-$d_6$) δ 10.97 (s, 1H), 8.58 (d, *J* = 8.1 Hz, 1H), 7.85 (t, *J* = 9.2 Hz, 2H), 7.43 - 7.36 (m, 2H), 7.33 (d, *J*= *7.5* Hz, 1H), 7.15 - 7.08 (m, 2H), 5.05 (dd, *J* = 13.3, 5.1 Hz, 1H), 4.67 (s, 2H), 4.60 - 4.48 (m, *J*= 11.9, 11.2, 5.6 Hz, 3H), 4.41 (d, *J* = 17.3 Hz, 1H), 4.27 (d, *J* = 17.4 Hz, 1H), 3.93 - 3.80 (m, *J* = 11.8, 8.3, 4.0 Hz, 2H), 3.15 - 3.01 (m, 6H), 2.96 - 2.85 (m, *J* = 17.9, 13.5, 5.4 Hz, 1H), 2.63 - 2.55 (m, 1H), 2.41 - 2.30 (m, 1H), 2.20 (d, *J* = 15.0 Hz, 3H), 2.14 - 2.07 (m, 2H), 2.00 - 1.83 (m, 8H), 1.69 - 1.59 (m, *J* = 13.2, 3.2 Hz, 2H), 1.57 - 1.46 (m, *J* = 12.9, 9.3 Hz, 2H), 1.33 - 1.20 (m, *J* = 13.6, 4.7 Hz, 2H).

LC-MS: $[M+H]^+$=807.55.

Example 439

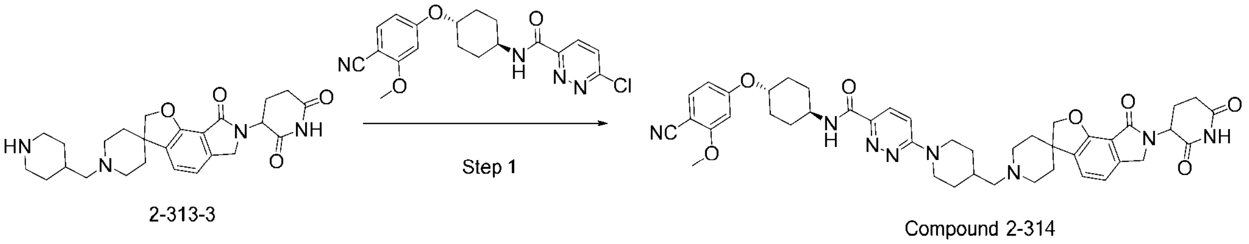


2-313-3

Compound 2-314

Step 3: (Compound 2-314) Prepared according to Step 3 of Example 432.

$^1H$ NMR (600 MHz, DMSO-$d_6$) δ 10.91 (s, 1H), 8.52 (d, *J* = 8.1 Hz, 1H), 7.79 (d, *J* = 9.5 Hz, 1H), 7.58 - 7.54 (m, 1H), 7.35 (d, *J* = 9.6 Hz, 1H), 7.28 (d, *J* = 7.5 Hz, 1H), 7.06 (d, *J* = 7.6 Hz, 1H), 6.67 (d, *J* = 7.3 Hz, 2H), 5.00 (dd, *J* = 13.3, 5.1 Hz, 1H), 4.62 (s, 2H), 4.50 - 4.41 (m, 3H), 4.36 (d, *J*= 17.4 Hz, 1H), 4.22 (d, *J* = 17.3 Hz, 1H), 3.84 (s, 3H), 3.82 - 3.78 (m, 1H), 3.09 - 2.98 (m, *J* = 14.7, 10.2, 5.6 Hz, 6H), 2.90 - 2.80 (m, *J* = 17.3, 13.6, 5.5 Hz, 1H), 2.54 (d, *J* = 14.1 Hz, 1H), 2.36 - 2.26 (m, 1H), 2.22 - 2.03 (m, 5H), 1.97 - 1.76 (m, 8H), 1.65 - 1.53 (m, *J* = 13.2, 3.2 Hz, 2H), 1.52 - 1.40 (m, 2H), 1.28 - 1.14 (m, *J* = 13.5, 5.4, 4.4 Hz, 3H).

LC-MS: $[M+H]^+$=803.56.

Example 440

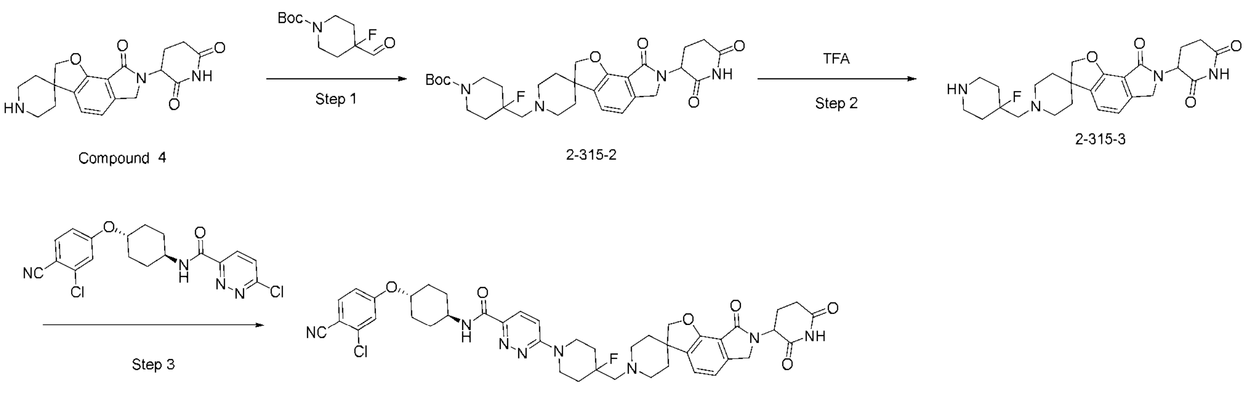


Compound 4

2-315-2

2-315-3

Compound 2-315

Step 1: (Compound 2-315-2)

In a reaction vessel, compound 4 (50.0 mg, 140.69 μmol) and *tert*-butyl 4-fluoro-4-formylpiperidine-1-carboxylate (59.44 mg, 281.38 μmol) were successively added, followed by the addition of THF (1.6 mL) as solvent. The reaction mixture was stirred at room temperature for 1 h, and then $NaBH_3CN$ (89.45 mg, 422.07 μmol) was added. The mixture was stirred at room temperature for an additional 1 h. After completion, the mixture was concentrated and purified by preparative thin-layer chromatography (DCM: MeOH = 10:1) to afford a white solid compound 2-315-2 (40 mg, 25.72%).

LC-MS: $[M+H]^+$ =571.45.

Step 2: (Compound 2-315-3) Prepared according to Step 2 of Example 432.

LC-MS: $[M+H]^+$=471.40.

Step 3: (Compound 2-315) Prepared according to Step 3 of Example 432.

$^1H$ NMR (600 MHz, DMSO-$d_6$) δ 10.98 (s, 1H), 8.63 (d, *J* = 8.3 Hz, 1H), 7.90 (d, *J* = 9.5 Hz, 1H), 7.88 - 7.84 (m, 1H), 7.49 (d, *J* = 9.7 Hz, 1H), 7.39 (d, *J* = 2.5 Hz, 1H), 7.33 (d, *J*=7.6 Hz, 1H), 7.15 - 7.10 (m, 2H), 5.05 (dd, *J* = 13.4, 5.2 Hz, 1H), 4.68 (s, 2H), 4.74 - 4.61 (m, *J* = 10.3, 5.6 Hz, 1H), 4.41 (d, *J* = 17.5 Hz, 3H), 4.27 (d, *J* = 17.4 Hz, 1H), 3.93 - 3.82 (m, 2H), 3.33 (s, 5H), 2.96 - 2.85 (m, 1H), 2.59 (s, 1H), 2.41 - 2.32 (m, 1H), 2.27 (s, 2H), 2.09 (d, *J* = 16.4 Hz, 4H), 2.03 - 1.79 (m, 8H), 1.71 - 1.59 (m, *J* = 12.3, 11.7 Hz, 2H), 1.57 - 1.48 (q, *J* = 10.9, 10.1 Hz, 2H), 1.24 (s, 1H).

LC-MS: $[M+H]^+$=825.56.

Example 441

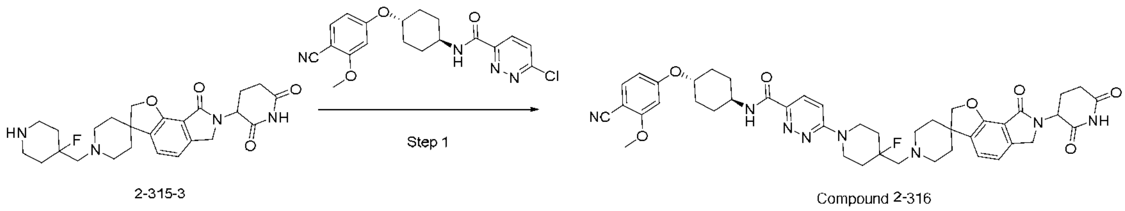


2-315-3

Compound 2-316

Step 1: (Compound 2-316) Prepared according to Step 3 of Example 432.

$^1$H NMR (400 MHz, DMSO-$d_6$) δ 10.96 (s, 1H), 8.62 (d, *J* = 8.2 Hz, 1H), 7.85 (d, *J* = 10.3 Hz, 1H), 7.68 - 7.58 (m, 1H), 7.43 (d, *J* = 13.5 Hz, 2H), 7.16 - 6.97 (m, 1H), 6.78 - 6.69 (m, 2H), 5.05 (dd, *J* = 13.2, 5.1 Hz, 1H), 4.79 - 4.45 (m, 3H), 4.45 - 4.20 (m, 4H), 3.90 (s, 4H), 3.59 (s, 1H), 3.40 (d, *J* = 12.3 Hz, 2H), 2.99 - 2.83 (m, *J* = 10.1, 7.8 Hz, 2H), 2.59 (d, *J* = 19.3 Hz, 2H), 2.55 (s, 1H), 2.43 - 2.05 (m, 6H), 2.04 - 1.77 (m, 8H), 1.74 - 1.59 (m, 4H), 1.59 - 1.45 (m, *J* = 11.0 Hz, 2H).
LC-MS: $[M+H]^+$=821.66.

Example 442

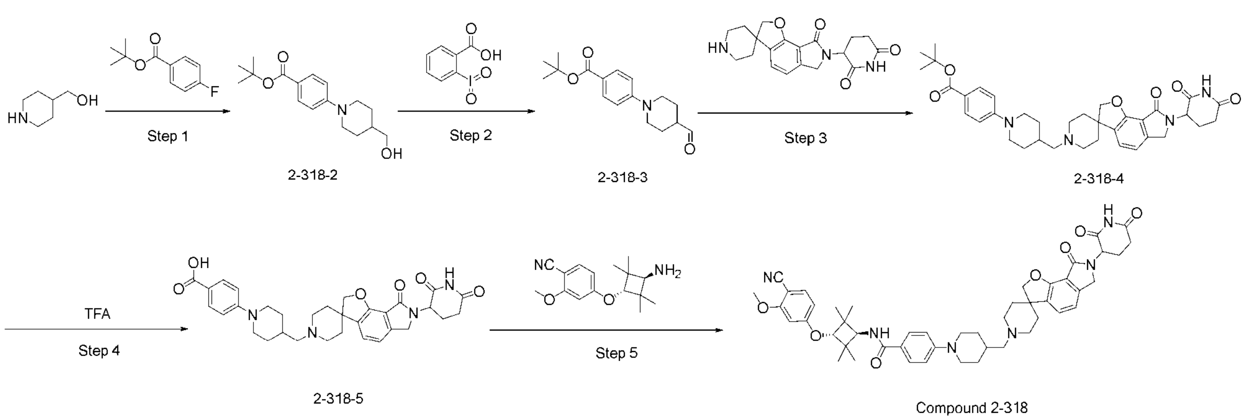


2-318-2

2-318-3

2-318-4

2-318-5

Compound 2-318

Step 1: (Compound 2-318-2)

In a reaction vessel, piperidin-4-ylmethanol (3.0 g, 26.05 mmol) and tert-butyl 4-fluorobenzoate (6.13 g, 31.38 mmol) were successively added, followed by the addition of DMSO (20 mL) as solvent. The reaction mixture was stirred at 120°C for 2 h. After completion, the mixture was extracted with ethyl acetate (EA, 30 mL × 3) and water (50 mL × 2). The organic layer was concentrated and purified by column chromatography (PE: EA = 1:1) to afford compound 2-318-2 as a white solid (4.26 g, 56.13%).
LC-MS: $[M+H]^+$ = 292.38.

Step 2: (Compound 2-318-3)

In a reaction vessel, compound 2-318-2 (0.50 g, 1.72 mmol) and 2-iodoxybenzoic acid (0.72 g, 2.57 mmol) were successively added, followed by the addition of $CH_3CN$ (20 mL) as solvent. The reaction mixture was stirred at 80°C for 2 h. After completion, the mixture was cooled to room temperature, filtered through diatomaceous earth, and the filtrate was concentrated. Then the obtained mixture was purified by normal-phase column chromatography (PE: EA = 3:1) to afford a yellow solid compound 2-318-3 (0.36 g, 73.39%).
LC-MS: $[M+H]^+$ = 290.28.

Step 3: (Compound 2-318-4)

In a reaction vessel, compound 4 (100.0 mg, 281.38 μmol), compound 2-318-3 (81.42 mg, 281.38 μmol), and titanium(IV) isopropoxide (159.95 mg, 562.76 μmol) were successively added, followed by the addition of a mixed solvent of THF: DMSO = 3:1 (4 mL). The reaction mixture was stirred at 60°C for 1 h, and then $NaBH_3CN$ (35.36 mg, 562.76 μmol) was added. The mixture was stirred at 60°C for an additional 1 h. After completion, the mixture was concentrated and purified by column chromatography (DCM: MeOH = 10:1) to afford a white solid compound 2-318-4 (82.30 mg, 46.52%).
LC-MS: $[M+H]^+$ = 629.58.

Step 4: (Compound 2-318-5)

In a reaction vessel, compound 2-318-4 (147.0 mg, 233.79 μmol) was dissolved in dichloromethane (DCM, 2 mL), followed by the addition of trifluoroacetic acid (TFA, 2 mL). The reaction mixture was stirred at room temperature for 2 h. After completion, the mixture was concentrated and used directly in the next step without further purification.
LC-MS: $[M+H]^+$ = 573.58.

Step 5: (Compound 2-318)

In a reaction vessel, compound 2-318-5 (100.0 mg, 174.62 μmol), intermediate 10 (57.49 mg, 209.55 μmol), EDCI (50.21 mg, 261.93 μmol), HOBt (35.39 mg, 261.93 μmol), and DIEA (67.71 mg, 523.87 μmol) were successively added, followed by the addition of DMF (2 mL) as solvent. The reaction mixture was stirred at room temperature for 3 h. After completion, the obtained mixture was purified by preparative HPLC to afford a white solid compound 2-318 (38.10 mg, 26.32%).
$^1$H NMR (600 MHz, DMSO-$d_6$) δ 10.97 (s, 1H), 7.77 (d, *J* = 8.5 Hz, 2H), 7.66 (d, *J* = 8.6 Hz, 1H), 7.52 (dd, *J* = 9.2, 2.3 Hz, 1H), 7.34 (d, *J* = 7.5 Hz, 1H), 7.12 (d, *J*= 7.5 Hz, 1H), 7.00 (d, *J* = 8.6 Hz, 2H), 6.65 (d, *J* = 2.2 Hz, 1H), 6.55 (dd, *J* = 8.6, 2.2 Hz, 1H), 5.06 (dd, *J* = 13.3, 5.1 Hz, 1H), 4.68 (s, 2H), 4.41 (d, *J* = 17.4 Hz, 1H), 4.33 - 4.24 (m, 2H), 4.06 (d, *J* = 9.2 Hz, 1H), 3.92 (s, 5H), 3.62 - 3.58 (m, 2H), 3.15 - 3.04 (m, 4H), 2.95 - 2.80 (m, 3H), 2.67 - 2.56 (m, 1H), 2.37 (qd, *J* = 13.1, 4.4 Hz, 1H), 2.22 (d, *J* = 13.0 Hz, 2H), 2.09 (d, *J* = 13.3 Hz, 1H), 2.02 - 1.89 (m, 3H), 1.85 (d, *J* = 12.9 Hz, 2H), 1.33 (ddt, *J* = 19.1, 12.1, 6.2 Hz, 2H), 1.23 (s, 6H), 1.16 (s, 6H).
LC-MS: $[M+H]^+$ = 829.66.

Example 443

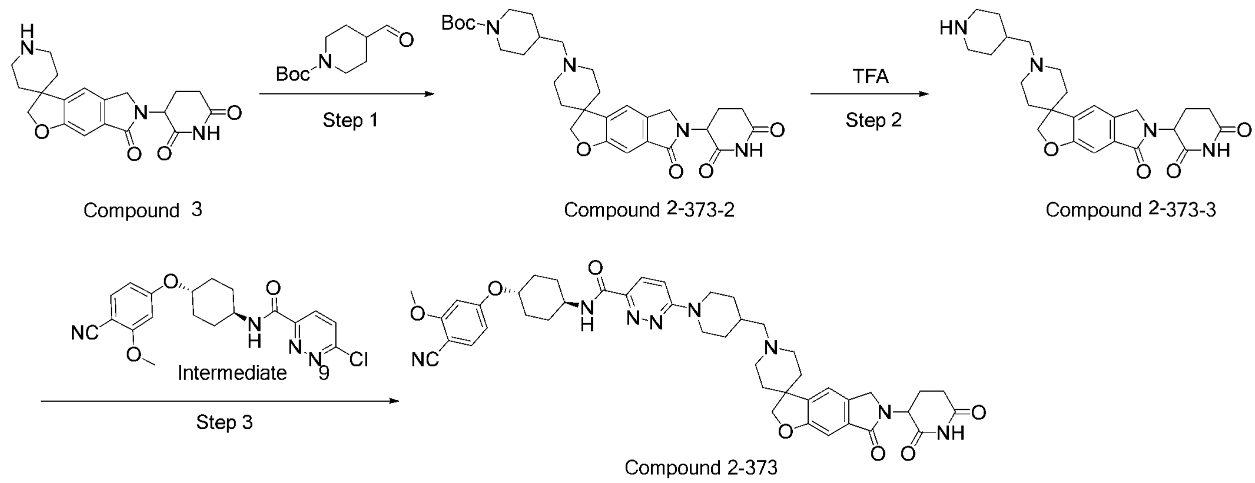


Compound 3

Compound 2-373-2

Compound 2-373-3

Compound 2-373

Step 1: (Compound 2-373-2) Prepared according to Step 1 of Example 432.

LC-MS: $[M+H]^+$ = 553.48.

Step 2: (Compound 2-373-3) Prepared according to Step 2 of Example 432.

LC-MS: $[M+H]^+$ = 453.39.

Step 3: (Compound 2-373) Prepared according to Step 3 of Example 432.

$^1H$ NMR (400 MHz, DMSO-$d_6$) δ 10.99 (d, *J* = 4.7 Hz, 1H), 8.59 (d, *J*= 8.2 Hz, 1H), 7.85 (d, *J* = 9.5 Hz, 1H), 7.62 (d, *J*= 9.2 Hz, 1H), 7.41 (d, *J* = 9.7 Hz, 1H), 7.29 (d, *J* = 20.9 Hz, 1H), 7.07 (d, *J* = 16.8 Hz, 1H), 6.73 (dd, *J* = 7.0, 2.0 Hz, 2H), 5.11 (dd, *J*= 13.2, 5.2 Hz, 1H), 4.60 (d, *J* = 13.7 Hz, 2H), 4.57 - 4.47 (m, 3H), 4.39 (dd, *J*= 17.1, 7.4 Hz, 1H), 4.26 (dd, *J*= 17.0, 8.3 Hz, 1H), 3.90 (s, 3H), 3.85 (d, *J* = 11.2 Hz, 1H), 3.17 - 3.01 (m, 6H), 2.98 - 2.85 (m, 1H), 2.60 (d, *J* = 15.5 Hz, 2H), 2.41 - 2.31 (m, 1H), 2.23 (t, *J* = 12.1 Hz, 2H), 2.13 (d, *J* = 10.4 Hz, 2H), 2.04 - 1.96 (m, 3H), 1.87 (dd, *J* = 35.1, 20.7 Hz, 5H), 1.65 (dd, *J* = 24.0, 11.0 Hz, 2H), 1.52 (dd, *J* = 23.3, 10.1 Hz, 2H), 1.26 (d, *J* = 13.7 Hz, 3H).

LC-MS: $[M+H]^+$ = 803.56.

Example 444

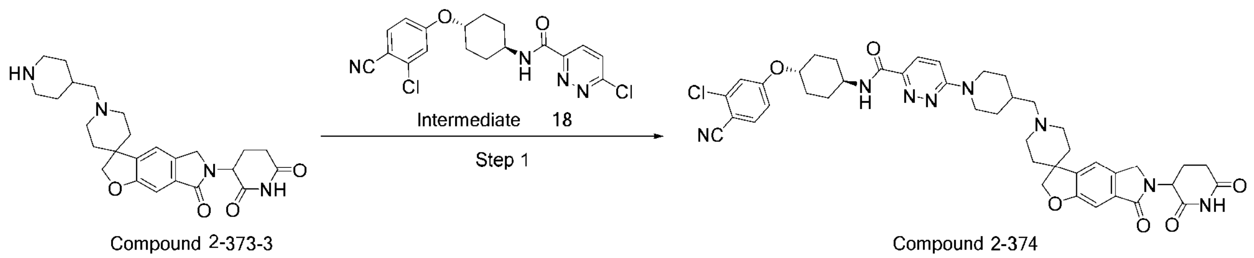


Compound 2-373-3

Compound 2-374

Step 1: (Compound 2-374) Prepared according to Step 3 of Example 432.

$^1H$ NMR (400 MHz, DMSO-$d_6$) δ 11.00 (s, 1H), 8.60 (d, *J*= 8.2 Hz, 1H), 7.86 (t, *J* = 8.6 Hz, 2H), 7.44 - 7.37 (m, 2H), 7.32 (s, 1H), 7.14 (dd, *J* = 8.8, 2.4 Hz, 1H), 7.09 (s, 1H), 5.11 (dd, *J* = 13.2, 5.0 Hz, 1H), 4.62 (s, 1H), 4.53 (d, *J* = 10.6 Hz, 3H), 4.34 (dd, *J* = 51.3, 17.1 Hz, 2H), 3.92 - 3.79 (m, 1H), 3.60 (d, *J* = 11.5 Hz, 2H), 3.09 (d, *J* = 12.5 Hz, 5H), 2.98 - 2.86 (m, 1H), 2.61 (d, *J* = 16.0 Hz, 1H), 2.40 - 2.31 (m, 1H), 2.24 (t, *J* = 12.0 Hz, 3H), 2.15 - 2.08 (m, 2H), 1.98 (t, *J* = 12.0 Hz, 3H), 1.89 (t, *J* = 11.2 Hz, 4H), 1.65 (dd, *J*= 23.9, 11.2 Hz, 2H), 1.52 (dd, *J* = 22.9, 10.2 Hz, 2H), 1.26 (d, *J* = 14.0 Hz, 4H).

LC-MS: $[M+H]^+$ = 807.56.

Example 445

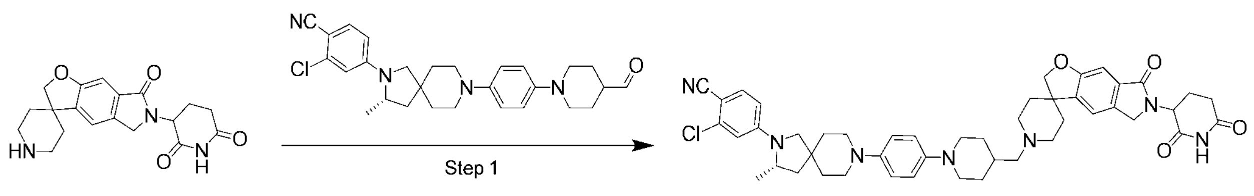


Compound 2- 376

Step 1: (Compound 2-376) Prepared according to Step 1 of Example 432.

$^1H$ NMR (400 MHz, DMSO-$d_6$) δ 10.99 (s, 1H), 7.63 (d, *J* = 8.8 Hz, 1H), 7.30 (d, *J* = 18.1 Hz, 3H), 7.26 - 7.12 (m, 2H), 7.09 (s, 1H), 6.82 (d, *J* = 1.8 Hz, 1H), 6.68 (dd, *J* = *8.9,* 1.9 Hz, 1H), 5.10 (dd, *J* = 13.2, 4.9 Hz, 1H), 4.70 - 4.56 (m, 2H), 4.33 (dd, *J* = 52.9, 17.1 Hz, 3H), 4.06 (dd, *J* = 13.0, 6.4 Hz, 2H), 3.60 (s, 2H), 3.39 (d, *J*= *9.7* Hz, 7H), 3.11 (s, 4H), 2.97 - 2.86 (m, 2H), 2.60 (d, *J* = 16.4 Hz, 1H), 2.44 - 2.31 (m, 2H), 2.26 (t, *J* = 12.5 Hz, 2H), 2.13 (s, 1H), 2.03 - 1.88 (m, 6H), 1.73 - 1.58 (m, 3H), 1.46 (s, 2H), 1.24 (s, 1H), 1.22 (d, *J* = 6.0 Hz, 3H).

LC-MS: $[M+H]^+$ = 816.56.

Example 446

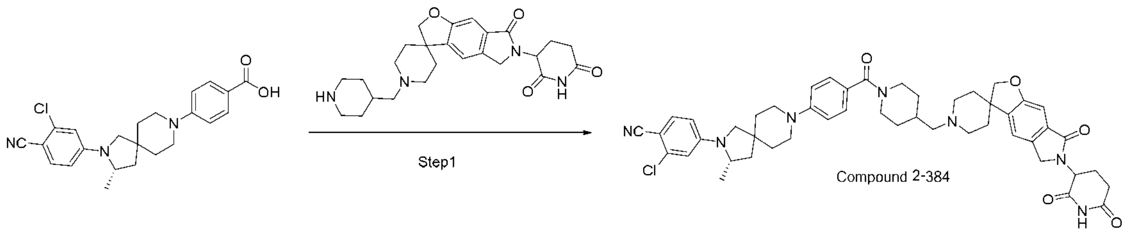


Step 3: (Compound 2-384) Prepared according to Step 3 of Example 427.

$^1$H NMR (600 MHz, DMSO-$d_6$) δ 10.99 (s, 1H), 7.61 (d, *J* = 8.9 Hz, 1H), 7.31 (s, 1H), 7.27 (d, *J* = 8.4 Hz, 2H), 7.09 (s, 1H), 6.98 (d, *J* = 8.5 Hz, 2H), 6.81 (d, *J* = 2.3 Hz, 1H), 6.67 (dd, *J = 8.9,* 2.3 Hz, 1H), 5.10 (dd, *J* = 13.2, 5.1 Hz, 1H), 4.67 - 4.55 (m, 2H), 4.39 (d, *J* = 17.1 Hz, 1H), 4.27 (d, *J* = 17.1 Hz, 1H), 4.05 (q, *J* = 6.5 Hz, 1H), 3.59 (d, *J* = 14.0 Hz, 2H), 3.49 - 3.30 (m, 6H), 3.23 (ddt, *J* = 14.4, 11.0, 5.2 Hz, 2H), 3.07 (q, *J* = 7.6, 6.6 Hz, 4H), 2.93 (td, *J* = 13.0, 12.5, 6.8 Hz, 2H), 2.66 - 2.57 (m, 1H), 2.42 - 2.32 (m, 1H), 2.23 (td, *J* = 18.1, 15.4, 10.3 Hz, 4H), 2.05 - 1.90 (m, 3H), 1.86 - 1.68 (m, 4H), 1.59 (dd, *J* = 12.8, 6.5 Hz, 1H), 1.50 (h, *J* = 5.0, 4.4 Hz, 2H), 1.30 - 1.12 (m, 6H).

LC-MS: $[M+H]^+$ = 844.76.

Example 447

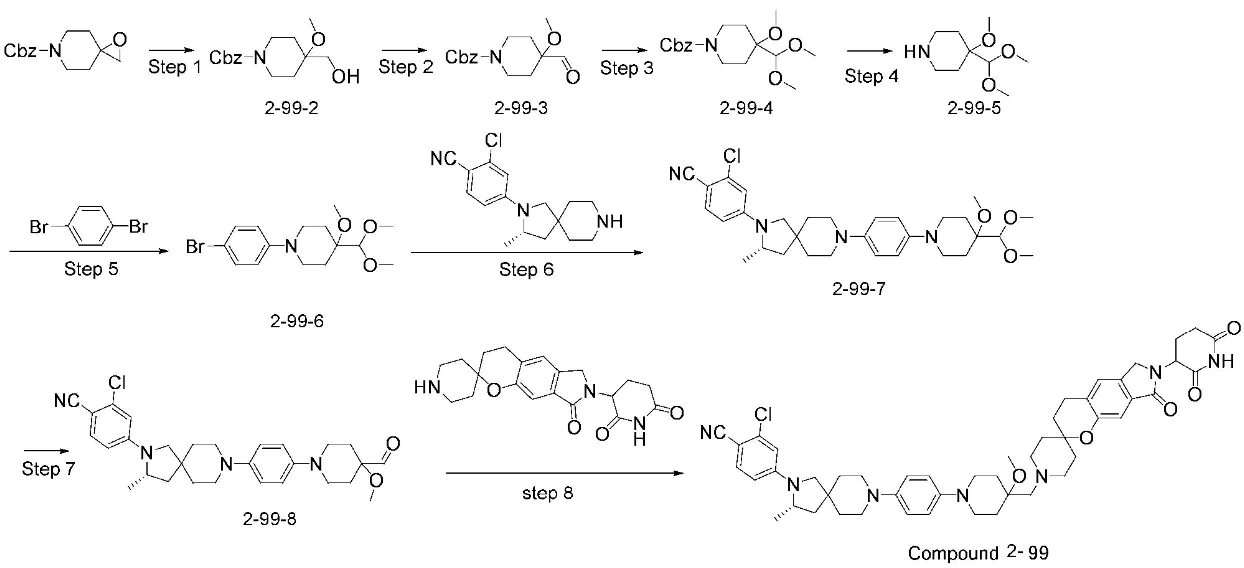


Step 1: (Compound 2-99-2)

In a pear-shaped flask, benzyl 1-oxa-6-azaspiro[2.5]octane-6-carboxylate (10 g, 40.5 mmol) and p-toluenesulfonic acid (208.98 mg, 1.22 mmol) were successively added, followed by the addition of MeOH (10 mL) as the solvent. The reaction mixture was stirred at room temperature for 12 h. After completion, the mixture was concentrated and purified by preparative thin-layer column chromatography (EA:PE = 1:1) to afford a colorless oily compound 2-99-2 (5.2 g, 46.06%).

Step 2: (Compound 2-99-3)

In a pear-shaped flask, intermediate 2-99-2 (5.2 g, 18.63 mmol) and 2-iodoxybenzoic acid (7.8 g, 27.95 mmol) were successively added, followed by the addition of ACN (60 mL) as the solvent. The reaction mixture was stirred at room

temperature for 2 h. After completion, the mixture was concentrated and purified by preparative thin-layer column chromatography (EA:PE = 1:2) to afford a colorless oily compound 2-99-3 (3.8 g, 73.64%).

Step 3: (Compound 2-99-4)

In a pear-shaped flask, compound 2-99-3 (3.8 g, 13.72 mmol) and trimethyl orthoformate (4.36 g, 41.16 mmol) were successively added, followed by the addition of MeOH (20 mL) as the solvent. After dropwise addition of hydrochloric acid (0.1 mL), the reaction mixture was stirred at 70°C for 2 h. After completion, the mixture was concentrated and purified by preparative thin-layer column chromatography (EA:PE = 1:3) to afford a colorless oily compound 2-99-4 (2 g, 63.2%).

Step 4: (Compound 2-293-5)

In a pear-shaped flask, compound 2-99-4 (2 g, 6.19 mmol) and $Pd(OH)_2/C$ (400 mg) were successively added, followed by the addition of MeOH (20 mL) as the solvent. under a hydrogen atmosphere, the reaction mixture was stirred at room temperature for 3 h. After completion, the mixture was filtered, concentrated, and dried under reduced pressure to afford a yellow oily compound 2-99-5 (800 mg, 68.37%).

Step 5: (Compound 2-293-6)

LC-MS: [M+H]+ = 344.25.
In a pear-shaped flask, compound 2-99-5 (800 mg, 4.23 mmol), 1,4-dibromobenzene (2.97 g, 12.69 mmol), $Pd(OAc)_2$ (95.17 mg, 0.423 mmol), BINAP (526.21 mg, 0.846 mmol), and $Cs_2CO_3$ (4.12 g, 12.69 mmol) were successively added, followed by the addition of dioxane (8 mL) as the solvent. Under nitrogen atmosphere, the reaction mixture was stirred at 110°C for 2 h. After completion, the mixture was filtered, concentrated, and purified by preparative thin-layer column chromatography (EA:PE = 1:5) to afford a yellow solid compound 2-99-6 (730 mg, 50.31%).

Step 6: (Compound 2-99-7)

LC-MS: [M+H]+ = 553.14.
In a pear-shaped flask, compound 2-99-6 (730 mg, 2.13 mmol), (S)-2-chloro-4-(3-methyl-2,8-diazaspiro[4.5]decan-2-yl)benzonitrile (738 mg, 2.55 mmol), $Pd_2(dba)_3$ (233.33 mg, 0.255 mmol), BINAP (317.22 mg, 0.51 mmol), and tBuOK (715.68 mg, 6.39 mmol) were successively added, followed by the addition of toluene (8 mL) as the solvent. Under nitrogen atmosphere, the reaction mixture was stirred at 110°C for 2 h. After completion, the mixture was filtered, concentrated, and purified by preparative thin-layer column chromatography (EA:PE = 1:1) to afford a yellow solid compound 2-99-7 (170 mg, 14.46%).

Step 7: (Compound 2-99-8)

In a pear-shaped flask, compound 2-99-7 (170 mg, 0.308 mmol), TFA (0.4 mL), and $H_2O$ (0.1 mL) were successively added, followed by the addition of DCM (0.2 mL) as the solvent. The reaction mixture was stirred at room temperature for 1 h. After completion, the mixture was concentrated and dried under reduced pressure to afford a crude blue solid compound 2-99-8 (190 mg).

Step 8: (Compound 2-99) Prepared according to Step 1 of Example 12.

$^1$H NMR (600 MHz, Methanol-*d$_4$*) δ 7.53 (d, *J* = 8.8 Hz, 1H), 7.51 - 7.45 (m, 2H), 7.37 (s, 1H), 7.35 (s, 1H), 7.19 (s, 2H), 6.83 (s, 1H), 6.71 (d, *J* = 8.8 Hz, 1H), 5.15 (dd, *J* = 13.4, 5.1 Hz, 1H), 4.50 - 4.34 (m, 3H), 4.17 - 4.07 (m, 1H), 3.75 - 3.64 (m, 4H), 3.62 - 3.55 (m, 4H), 3.49 - 3.43 (m, 2H), 3.39 (s, 3H), 3.25 - 3.18 (m, 2H), 3.02 (s, 2H), 2.96 - 2.87 (m, 3H), 2.84 - 2.77 (m, 2H), 2.56 - 2.46 (m, 2H), 2.24 - 2.16 (m, 2H), 2.15 - 2.08 (m, 4H), 2.08 - 2.03 (m, 2H), 1.99 (s, 2H), 1.94 - 1.83 (m, 4H), 1.78 (dd, *J*= 12.9, 6.9 Hz, 2H), 1.33 (d, *J*= 6.1 Hz, 3H).
LC-MS: $[M+H]^+$=860.76.

Example 448

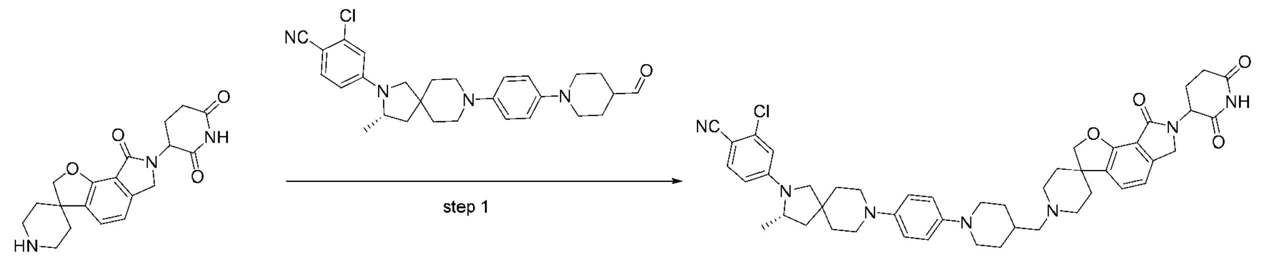


Compound 2-103

Step 1: (Compound 2-103) Prepared according to Step 1 of Example 432.

$^1$H NMR (600 MHz, DMSO-$d_6$) δ 10.98 (s, 1H), 7.62 (d, *J* = 8.8 Hz, 1H), 7.33 (d, *J* = 7.5 Hz, 1H), 7.26 (s, 1H), 7.11 (d, *J* = 8.2 Hz, 3H), 7.00 (s, 1H), 6.81 (d, *J* = 2.2 Hz, 1H), 6.68 (dd, *J* = 8.9, 2.4 Hz, 1H), 5.06 (dd, *J* = 13.3, 5.1 Hz, 1H), 4.68 (s, 2H), 4.41 (d, *J* = 17.5 Hz, 1H), 4.27 (d, *J* = 17.5 Hz, 1H), 4.05 (q, *J* = 6.4 Hz, 1H), 3.72 - 3.56 (m, 5H), 3.17 - 3.06 (m, 6H), 2.91 (m, 2H), 2.64 - 2.56 (m, 1H), 2.37 (dd, *J* = 13.4, 4.7 Hz, 1H), 2.23 (t, *J* = 14.0 Hz, 3H), 1.94 (d, *J* = 14.4 Hz, 4H), 1.76 (s, 1H), 1.61 (s, 3H), 1.44 (s, 3H), 1.28 - 1.17 (m, 9H).

LC-MS: $[M+H]^+$=816.76.

Example 449

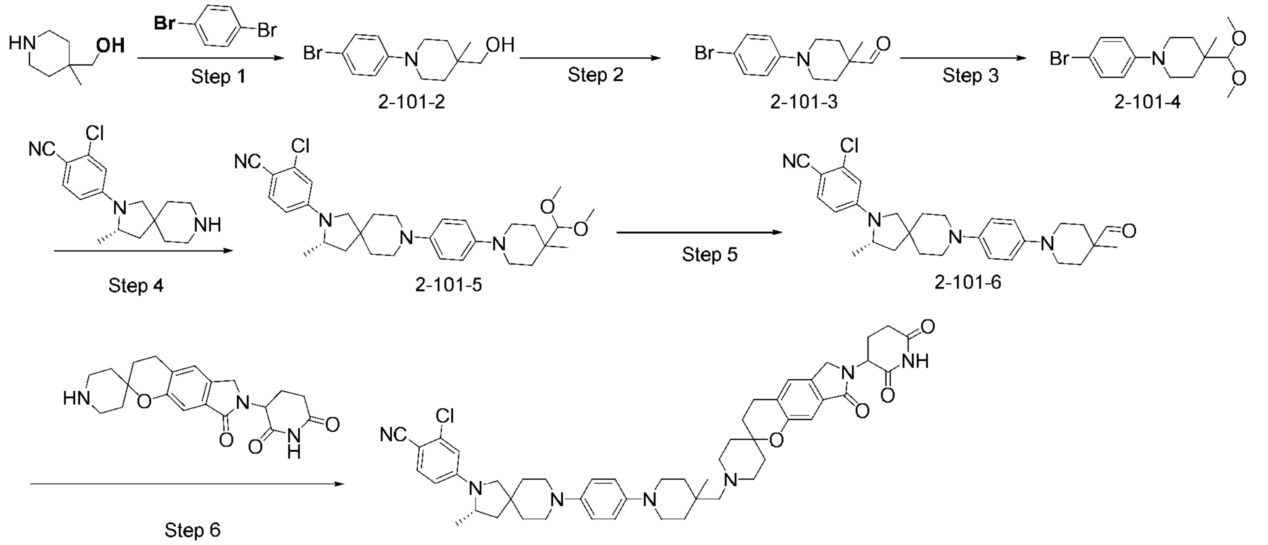


Compound 2-101

Step 1: (Compound 2-101-2)

LC-MS: $[M+H]^+$ =284.20.

In a 100 mL round-bottom flask, (4-methylpiperidin-4-yl)methanol (2 g, 15.48 mmol) and 1,4-dibromobenzene (10.96 g, 46.44 mmol) were successively added, followed by the addition of 1,4-dioxane (50 mL) as the solvent. Under nitrogen atmosphere, palladium(II) acetate (0.1 equiv), 1,1'-binaphthyl-2,2'-diylbis(diphenylphosphine) (BINAP, 0.2 equiv), and cesium carbonate (3 equiv) were added. The reaction mixture was stirred at 80°C for 4 h. After completion, the mixture was concentrated and purified by column chromatography (PE:EA = 5:1) to afford compound 2-101-2 as a white solid (1.28 g, 29.10%).

Step 2: (Compound 2-101-3)

In a 100 mL round-bottom flask, the compound 2-101-2 (1 g, 15.48 mmol) and 2-iodoxybenzoic acid (1.5 equiv) were successively added, followed by the addition of acetonitrile (50 mL) as the solvent. The reaction mixture was stirred at 80°C for 1.5 h. After completion, the mixture was concentrated and purified by column chromatography (PE:EA = 5:1) to

afford a white solid compound 2-101-3 (800 mg, 80.57%).

LC-MS: $[M+H]^+$ = 282.18.

Step 3: (Compound 2-101-4)

In a 100 mL round-bottom flask, the compound 2-101-3 (500 mg, 1.77 mmol) and trimethyl orthoformate (3eq) were successively added, followed by the addition of methanol (50 mL) as the solvent. Concentrated hydrochloric acid (1 mL) was added, and the reaction mixture was stirred at 70°C for 3 h. After completion, the mixture was concentrated and purified by column chromatography (PE:EA =5:1) to afford a white solid compound 2-101-4 (300 mg, 51.58%).

LC-MS: $[M+H]^+$=328.25.

Step 4: (Compound 2-101-5) Prepared according to Step 6 of Example 447
Step 5: (Compound 2-101-6) Prepared according to Step 7 of Example 447
Step 6: (Compound 2-101) Prepared according to Step 1 of Example 432.

LC-MS: $[M+H]^+$=830.47.

$^1$H NMR (600 MHz, Methanol-$d_4$) δ 7.53 (d, *J* = 8.8 Hz, 1H), 7.46 (d, *J* = 8.4 Hz, 2H), 7.37 (s, 1H), 7.34 (s, 1H), 7.25 (d, *J* = 8.0 Hz, 2H), 6.83 (d, *J* = 2.4 Hz, 1H), 6.71 (dd, *J* = 8.9, 2.4 Hz, 1H), 5.15 (dd, *J* = 13.4, 5.1 Hz, 1H), 4.47 - 4.36 (m, 2H), 4.12 (q, 1H), 3.70 (s, 1H), 3.66 - 3.53 (m, 9H), 3.48 (d, *J* = 10.5 Hz, 1H), 3.38 - 3.34 (m, 4H), 3.01 (t, *J* = 6.8 Hz, 2H), 2.96 - 2.86 (m, 2H), 2.83 - 2.76 (m, 1H), 2.56 - 2.41 (m, 2H), 2.25 - 2.15 (m, 4H), 2.13 - 2.04 (m, 4H), 1.99 (d, *J* = 6.9 Hz, 2H), 1.93 - 1.84 (m, 2H), 1.84 - 1.73 (m, 4H), 1.35 (s, 3H), 1.33 (d, *J* = 6.1 Hz, 3H).

LC-MS: $[M+H]^+$=844.66.

Example 450

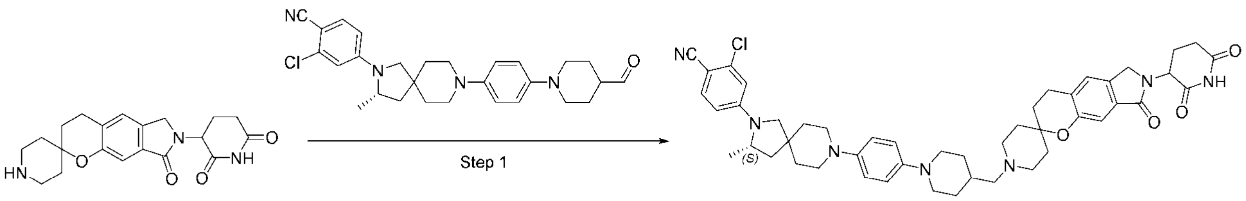


Compound 2-102

Step 1: (Compound 2-102) Prepared according to Step 1 of Example 432.

$^1$H NMR (400 MHz, Methanol-$d_4$) δ 7.52 (d, *J* = 8.9 Hz, 1H), 7.42 (d, *J* = 8.6 Hz, 2H), 7.35 (d, *J* = 13.1 Hz, 2H), 7.23 (d, *J* = 8.7 Hz, 2H), 6.82 (d, *J* = 2.3 Hz, 1H), 6.70 (dd, *J* = 8.9, 2.4 Hz, 1H), 5.14 (dd, *J* = 13.3, 5.1 Hz, 1H), 4.51 - 4.35 (m, 2H), 4.17 - 4.06 (m, 1H), 3.84 (d, *J* = 12.4 Hz, 2H), 3.61 (s, 2H), 3.59 - 3.50 (m, 4H), 3.47 (d, *J* = 10.6 Hz, 2H), 3.44 - 3.36 (m, 2H), 3.23 (d, *J* = 6.9 Hz, 2H), 3.17 - 3.06 (m, 2H), 3.01 (t, *J* = 6.8 Hz, 2H), 2.92 (ddd, *J* = 17.6, 13.5, 5.4 Hz, 1H), 2.80 (ddd, *J* = 17.6, 4.7, 2.4 Hz, 1H), 2.57 - 2.41 (m, 2H), 2.25 - 2.12 (m, 4H), 2.11 - 2.04 (m, 4H), 2.04 - 1.95 (m, 4H), 1.90 - 1.82 (m, 2H), 1.76 (dd, *J* = 13.1, 7.0 Hz, 1H), 1.59 (q, *J* = 12.5, 12.1 Hz, 2H), 1.32 (d, *J* = 6.1 Hz, 3H).

LC-MS: $[M+H]^+$=829.75.

Example 451

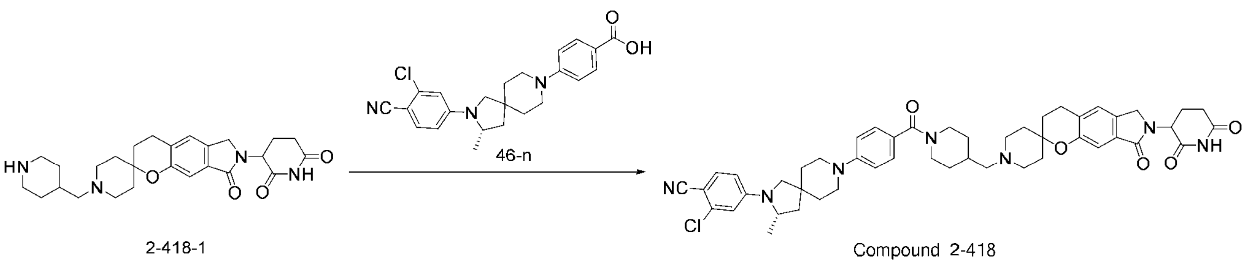


2-418-1

Compound 2-418

Step 1: (Compound 2-418) Prepared according to Step 3 of Example 427.

$^1$H NMR (400 MHz, DMSO-$d_6$) δ 10.99 (s, 1H), 7.60 (d, *J* = 8.9 Hz, 1H), 7.36 (s, 1H), 7.28 - 7.22 (m, 3H), 6.97 (d, *J* = 8.3 Hz, 2H), 6.80 (d, *J* = 2.3 Hz, 1H), 6.66 (dd, *J* = 8.9, 2.4 Hz, 1H), 5.07 (dt, *J* = 13.3, 4.4 Hz, 1H), 4.34 (d, *J* = 16.8 Hz, 1H), 4.22 (d, *J* = 16.8 Hz, 1H), 4.04 (q, *J* = 6.6 Hz, 1H), 3.34 (t, *J* = 9.8 Hz, 4H), 3.27 - 3.15 (m, 5H), 3.12 (d, *J* = 6.1 Hz, 2H), 2.98 - 2.84 (m, 5H), 2.60 (d, *J*= 17.5 Hz, 1H), 2.39 (dd, *J* = 13.2, 4.6 Hz, 2H), 2.24 (dd, *J* = 12.8, 7.7 Hz, 1H), 2.13 (s, 1H), 2.03 - 1.92 (m, 4H), 1.89 (q, *J* = 6.6, 5.9 Hz, 3H), 1.82 - 1.69 (m, 4H), 1.58 (dd, *J* = 12.8, 6.6 Hz, 1H), 1.50 (t, *J* = 4.8 Hz, 2H), 1.24 (d, *J= 3.6* Hz, 2H), 1.21 (d, *J* = 6.0 Hz, 4H), 1.17 (d, *J* = 3.7 Hz, 1H).

LC-MS: $[M+H]^+$=858.56.

Test Example 1: Binding Activity of E3 Ligase Inhibitors to E3 Ubiquitin Ligase CRBN

The binding activity of the E3 ligase inhibitor disclosed in the present disclosure and positive control samples lenalidomide and pomalidomide to the E3 ubiquitin ligase CRBN is determined by the results that the E3 ligase inhibitor disclosed in the present disclosure and lenalidomide and pomalidomide competed with thalidomide labeled with XL665 for binding to the CRBN protein, thereby preventing fluorescence resonance energy transfer (FRET) from occurring, thus determining the binding ability of the compound to the CRBN protein.

CRBN Binding Activity Test Kit (CEREBLON BINDING KITS (PE, 64BDCRBNPEG).

The assay is based on homogeneous time-resolved fluorescence (HTRF) technology. When a donor and an acceptor are in close proximity, the donor can transfer energy to the acceptor, resulting in excitation and emission at 665nm.

The donor used in this kit is Europium-labeled GST Antibody (GST Eu Cryptate Antibody). The acceptor is thalidomide labeled with XL665 (Thalidomide-Red reagent). The donor binds to the CRBN protein with the GST tag, and the E3 ligase inhibitor disclosed by the present disclosure, as well as lenalidomide and pomalidomide, compete with thalidomide for binding to the CRBN protein, and the binding activity of the compound is determined based on the fluorescence value emitted by the acceptor at 665 nm. The stronger the binding ability of the compound, the weaker the signal.

The donor and acceptor were each diluted 50-fold with the buffer (PROTAC binding buffer 1) in the kit, and the CRBN protein was diluted 45-fold. The 8mM solution of the compound disclosed in the present disclosure was diluted 10-fold with the diluent (1 X diluent) in the kit, followed by a 5-fold gradient dilutions for seven times sequentially.

To the wells of a white 3 84-well plate (PE, Part number: 6008280) were sequentially added 5μl of the compound, 5μl of the diluted CRBN protein and 10μl of the donor and the receptor in equal volume mixture. The plate was incubated at room temperature for 3 hours.

Calculate the ratio of emission signals of receptor and donor for each individual well.

$$\text{Ratio} = \text{signal at 665 nm / signal at 620 nm} *10^4$$

$$\text{Calculate the coefficient of variation (CV)} = \text{standard deviation (SD)/ mean Ratio} *100$$

The $IC_{50}$ values were calculated using GraphPad Prism based on the concentration of the compound, Ratio and coefficient of variation.

Table 1 shows the $IC_{50}$ values of the E3 ligase inhibitor disclosed in the present disclosure, lenalidomide and pomalidomide.

| Compound | $IC_{50}$ (μM) | Compound | $IC_{50}$ (μM) |
|---|---|---|---|
| lenalidomide | 3.1 | E3 ligase inhibitor 16 | 1.2 |
| pomalidomide | 1.3 | E3 ligase inhibitor 17 | 15.2 |
| E3 ligase inhibitor 1 | 14.2 | E3 ligase inhibitor 18 | 8.4 |
| E3 ligase inhibitor 2 | 14.0 | E3 ligase inhibitor 19 | 9.3 |
| E3 ligase inhibitor 3 | 5.7 | E3 ligase inhibitor 20 | 7.9 |
| E3 ligase inhibitor 4 | 12.7 | E3 ligase inhibitor 21 | 1.0 |

(continued)

| Compound | $IC_{50}$ (μM) | Compound | $IC_{50}$ (μM) |
|---|---|---|---|
| E3 ligase inhibitor 5 | 30.5 | E3 ligase inhibitor 22 | 0.8 |
| E3 ligase inhibitor 6 | 0.8 | E3 ligase inhibitor 23 | 15.2 |
| E3 ligase inhibitor 7 | 9.9 | E3 ligase inhibitor 24 | 7.0 |
| E3 ligase inhibitor 8 | 2.1 | E3 ligase inhibitor 25 | 10.4 |
| E3 ligase inhibitor 9 | 8.6 | E3 ligase inhibitor 26 | 5.0 |
| E3 ligase inhibitor 10 | 27.3 | E3 ligase inhibitor 27 | 36.4 |
| E3 ligase inhibitor 11 | 13.9 | E3 ligase inhibitor 28 | 8.9 |
| E3 ligase inhibitor 12 | 3.0 | Compound 2-1 | 0.78 |
| E3 ligase inhibitor 13 | 3.9 | Compound 2-2 | 35.94 |
| E3 ligase inhibitor 14 | 3.5 | Compound 2-3 | 1.92 |
| E3 ligase inhibitor 15 | 8.6 | Compound 2-4 | 4.58 |
| Compound 2-5 | 3.62 | Compound 2-8 | 3.02 |
| Compound 2-6 | 1.49 | Compound 2-7 | 0.32 |

Conclusion: The E3 ligase inhibitor disclosed in the present disclosure is capable of binding to the CRBN protein well, and some of the E3 ligase inhibitors show comparable or even superior binding ability to CRBN compared with positive control samples, lenalidomide and pomalidomide.

**Test Example 2: Inhibitory effect of the PROTAC compounds provided in the present disclosure on cell proliferation in T-47D cells (source ATCC)**

The complete medium required for T-47D cells was 1640 ( ATCC, Product number: A10491-01) basal medium containing 10% fetal bovine serum. T-47D cells were inoculated in 96-well plates at a density of 3,000 cells/well, and different concentrations of the PROTAC compounds 1-55 provided in the examples of the present disclosure and a positive control sample ARV-471 (source: Innochem) were prepared to treat cells, and the cells were treated at 37°C in a cell culture incubator with 5% $CO_2$ for 6 days. Cell viability was detected using a CCK-8 kit (Beyotime, Product number: C0040) according to the manufacturer's instructions. The $IC_{50}$ values of each compound after 6 days of treatment were calculated using GraphPadPrism software based on the inhibition rate, and the results are shown in Tables 2 and 3.

Table 2 $IC_{50}$ values for inhibition of the proliferation of T-47D cells by the PROTAC compounds provided by the present disclosure and the positive control sample

| Compound | $IC_{50}$(T-47D) [μM] | Compound | $IC_{50}$(T-47D) [μM] | Compound | $IC_{50}$(T-47D) [μM] |
|---|---|---|---|---|---|
| ARV-471 | 0.0017 | Compound A8 | 0.1040 | Compound M3 | 0.1000 |
| Compound A7 | 0.1670 | Compound A18 | 0.0680 | Compound B2 | 0.2290 |
| Compound A4 | 0.2000 | Compound A19 | 0.0200 | Compound M5 | 0.3130 |
| Compound A6 | 0.1770 | Compound A20 | 0.0250 | Compound A27 | 0.0150 |
| Compound A9 | 0.0240 | Compound B1 | 0.0380 | Compound A26 | 0.0210 |
| Compound A1 | 0.0080 | Compound B3 | 0.1000 | Compound M7 | 0.0170 |
| Compound A12 | 0.1270 | Compound B5 | 0.0820 | Compound M11 | 0.0010 |
| Compound A10 | 0.0350 | Compound A21 | 0.0190 | Compound A24 | 0.0100 |
| Compound A11 | 0.0720 | Compound M1 | 0.1170 | Compound A33 | 0.0092 |
| Compound O3 | 0.2020 | Compound M2 | 0.0460 | Compound M13 | 0.0016 |
| Compound A2 | 0.0004 | Compound B4 | 0.0569 | Compound A30 | 0.0118 |
| Compound A17 | 0.0280 | Compound A28 | 0.0306 | Compound M8 | 0.0220 |

(continued)

| Compound | $IC_{50}$(T-47D) [μM] | Compound | $IC_{50}$(T-47D) [μM] | Compound | $IC_{50}$(T-47D) [μM] |
|---|---|---|---|---|---|
| Compound A15 | 0.0630 | Compound A29 | 0.2150 | Compound B8 | 0.0029 |
| Compound A13 | 0.0310 | Compound M4 | 0.1540 | Compound A25 | 0.0200 |
| CompoundA14 | 0.0440 | Compound M6 | 0.0110 | Compound M9 | 0.1010 |
| Compound O1 | 0.1300 | Compound M12 | 0.0020 | Compound M10 | 0.0140 |
| Compound O2 | 0.1800 | Compound A22 | 0.0570 | Compound A32 | 0.0560 |
| Compound A16 | 0.0910 | Compound A23 | 0.0090 | Compound B7 | 1.0000 |
| Compound B10 | 0.0066 | Compound L1 | 0.0052 | | |

Table 3 $IC_{50}$ values for inhibition of the proliferation of T-47D cells by the PROTAC compounds provided by the present disclosure

| Compound | $IC_{50}$ (T-47D) [nM] | Compound | $IC_{50}$ (T-47 D) [n M] | Compound | $IC_{50}$ (T-47 D) [n M] | Compound | $IC_{50}$ (T-47D ) [nM ] |
|---|---|---|---|---|---|---|---|
| Compound A3 | 2 | Compound A55 | 8.8 3 | Compound G7 | 10. 75 | Compound H15 | 17.3 6 |
| Compound A5 | 7 | Compound A58 | 2.1 9 | Compound G8 | 11.5 6 | Compound H16 | 5.70 |
| Compound A8 | 104 | Compound A59 | 8.8 2 | Compound G9 | 16. 18 | Compound H17 | 1.02 |
| Compound A12 | 127 | Compound A60 | 18. 26 | Compound G10 | 1.1 9 | Compound H18 | 1.55 |
| Compound A28 | 30.6 | Compound A61 | 26. 96 | Compound G11 | 1.8 3 | Compound H19 | 1.80 |
| Compound A31 | 2 | Compound A62 | 2.1 2 | Compound G12 | 2.8 4 | Compound H20 | 1.22 |
| Compound A32 | 56 | Compound A63 | 1.6 8 | Compound G13 | 1.1 6 | Compound H21 | 1.02 |
| Compound A34 | 68 | Compound A64 | 6.9 6 | Compound G15 | 2.7 3 | Compound H22 | 1.57 |
| Compound A35 | 0.23 | Compound A65 | 7.5 4 | Compound G16 | 0.9 2 | Compound H23 | 1.58 |
| Compound A36 | 21 | Compound A66 | 1.9 3 | Compound G17 | 1.7 5 | Compound H24 | 14.3 5 |
| Compound A37 | 7 | Compound A67 | 1.7 6 | Compound G18 | 2.4 0 | Compound H25 | 6.02 |
| Compound A3 8 | 15 | Compound A68 | 7.0 3 | Compound G20 | 3.1 6 | Compound H26 | 10.4 8 |
| Compound A39 | 41 | Compound A69 | 3.53 | Compound G21 | 25.06 | Compound H27 | 4.65 |
| Compound A40 | 13 | Compound A70 | 4.1 7 | Compound G22 | 5.4 0 | Compound H28 | 1.01 |
| Compound A41 | 23 | Compound A71 | 3.3 8 | Compound G23 | 3.1 0 | Compound H29 | 5.47 |
| Compound A42 | 60 | Compound A72 | 4.4 9 | Compound G25 | 3.3 1 | Compound H30 | 5.78 |

(continued)

| Compound | $IC_{50}$ (T-47D) [nM] | Compound | $IC_{50}$ (T-47 D) [n M] | Compound | $IC_{50}$ (T-47 D) [n M] | Compound | $IC_{50}$ (T-47D ) [nM ] |
|---|---|---|---|---|---|---|---|
| Compound A43 | 41 | Compound A73 | 2.2 6 | Compound G26 | 1.2 6 | Compound H31 | 1.80 |
| Compound A44 | 5 | Compound A74 | 1.6 4 | Compound H1 | 100 00 | Compound H32 | 1.44 |
| Compound A45 | 79 | Compound A75 | 2.8 3 | Compound H2 | 100 00 | Compound H33 | 1.37 |
| Compound A46 | 10.61 | Compound A76 | 1.2 7 | Compound H3 | 200 | Compound H34 | 1.33 |
| Compound A47 | 11.71 | Compound A77 | 1.0 3 | Compound H4 | 227 5 | Compound H35 | 0.99 |
| Compound A48 | 12.30 | Compound A78 | 1.1 8 | Compound H5 | 22. 36 | Compound H36 | 1.06 |
| Compound A49 | 9.63 | Compound A79 | 1.0 2 | Compound H6 | 4.4 2 | Compound H38 | 2.58 |
| Compound A50 | 4.16 | Compound A80 | 5.7 5 | Compound H7 | 10. 39 | Compound H39 | 3.38 |
| Compound A51 | 12.21 | Compound A81 | 5.8 9 | Compound H8 | 31. 87 | Compound H40 | 1.42 |
| Compound A52 | 10.87 | Compound A82 | 1.8 6 | Compound H9 | 594 | Compound H41 | 1.88 |
| Compound A53 | 3.87 | Compound A83 | 5.5 5 | Compound H10 | 11.5 2 | Compound H42 | 1.32 |
| Compound A54 | 6.16 | Compound A84 | 1.2 0 | Compound H11 | 124 | Compound H44 | 3.74 |
| Compound A85 | 1.12 | Compound A118 | 2.4 5 | Compound H12 | 15. 87 | Compound H45 | 1.47 |
| Compound A86 | 1.64 | Compound A119 | 1.1 8 | Compound H46 | 1.4 0 | Compound J11 | 12.3 0 |
| Compound A88 | 1.18 | Compound A120 | 1.3 4 | Compound H47 | 1.1 0 | Compound J12 | 1.10 |
| Compound A89 | 2.16 | Compound A121 | 1.3 6 | Compound H48 | 1.0 8 | Compound J13 | 13.4 4 |
| Compound A90 | 1.78 | Compound A122 | 1.1 8 | Compound H49 | 1.1 8 | Compound J14 | 11.4 7 |
| Compound A91 | 1.11 | Compound A123 | 1.34 | Compound H50 | 3.59 | Compound J15 | 3.49 |
| Compound A92 | 1.34 | Compound A124 | 2.3 9 | Compound H51 | 1.5 2 | Compound J16 | 1.58 |
| Compound A93 | 1.85 | Compound B7 | >1 00 00 | Compound H52 | 1.7 4 | Compound K1 | 38.4 3 |
| Compound A94 | 2.74 | Compound B8 | 31 | Compound H53 | 7.5 6 | Compound K2 | 27.2 8 |
| Compound A95 | 1.49 | Compound B9 | 7 | Compound H54 | 4.5 9 | Compound K3 | 4.13 |

(continued)

| Compound | $IC_{50}$ (T-47D) [nM] | Compound | $IC_{50}$ (T-47 D) [n M] | Compound | $IC_{50}$ (T-47 D) [n M] | Compound | $IC_{50}$ (T-47D ) [nM ] |
|---|---|---|---|---|---|---|---|
| Compound A96 | 3.38 | Compound B10 | 3 | Compound H55 | 1.3 9 | Compound K4 | 32.4 1 |
| Compound A97 | 2.33 | Compound B11 | 55 | Compound H56 | 2.9 9 | Compound K5 | 1.61 |
| Compound A98 | 1.19 | Compound B12 | 33 | Compound H57 | 26. 90 | Compound K6 | 39.6 0 |
| Compound A99 | 2.07 | Compound B13 | 18 | Compound H58 | 7.7 9 | Compound K7 | 1.41 |
| Compound A100 | 0.84 | Compound B14 | 62. 97 | Compound H59 | 0.5 7 | Compound K8 | 2.07 |
| Compound A101 | 0.91 | Compound B15 | 31. 69 | Compound H60 | 0.7 6 | Compound K11 | 1.06 |
| Compound A102 | 20.16 | Compound B16 | 6.4 1 | Compound H61 | 1.3 7 | Compound K12 | 28.2 0 |
| Compound A103 | 2.99 | Compound B17 | 2.4 3 | Compound I2 | 4.6 9 | Compound K13 | 1.19 |
| Compound A104 | 17.08 | Compound B18 | 3.9 1 | Compound I3 | 1.2 3 | Compound K14 | 35.9 0 |
| Compound A105 | 1.30 | Compound B19 | 2.2 6 | Compound I4 | 12. 74 | Compound K15 | 76.8 3 |
| Compound A106 | 1.20 | Compound B20 | 3.3 8 | Compound I5 | 4.2 6 | Compound K16 | 34.3 6 |
| Compound A107 | 1.52 | Compound B21 | 2.9 6 | Compound I6 | 1.6 9 | Compound K17 | 1.54 |
| Compound A108 | 1.80 | Compound B22 | 1.3 0 | Compound J1 | 16. 68 | Compound K18 | $< 10^{-4}$ |
| Compound A109 | 2.93 | Compound B23 | 10. 25 | Compound J2 | 2.0 5 | Compound K19 | 5.34 |
| Compound A111 | 1.20 | Compound B29 | 5.3 7 | Compound J3 | 1.3 6 | Compound K20 | 2.59 |
| Compound A112 | 2.01 | Compound B30 | 2.3 0 | Compound J4 | 6.5 0 | Compound K21 | 1.12 |
| Compound A113 | 4.61 | Compound B31 | 2.4 9 | Compound J5 | 1.1 8 | Compound K22 | 8.52 |
| Compound A114 | 1.18 | Compound B32 | 9.9 8 | Compound J6 | 1.4 1 | Compound K23 | 1.04 |
| Compound A115 | 10.86 | Compound B33 | 2.1 0 | Compound J7 | 1.1 2 | Compound K24 | 1.44 |
| Compound A116 | 1.61 | Compound B34 | 2.1 6 | Compound J8 | 1.0 7 | Compound K25 | 3.71 |
| Compound A117 | 10.05 | Compound B35 | 2.9 2 | Compound J9 | 0.7 9 | Compound K26 | 1.12 |
| Compound B36 | 1.03 | Compound C19 | 14. 71 | Compound J10 | 0.8 3 | Compound K27 | 1.05 |

(continued)

| Compound | $IC_{50}$ (T-47D) [nM] | Compound | $IC_{50}$ (T-47 D) [n M] | Compound | $IC_{50}$ (T-47 D) [n M] | Compound | $IC_{50}$ (T-47D ) [nM ] |
|---|---|---|---|---|---|---|---|
| Compound B37 | 8.54 | Compound C22 | 6.6 0 | Compound K28 | 1.2 0 | Compound L11 | 4 |
| Compound B38 | 1.23 | Compound C23 | 6.5 8 | Compound K29 | 1.0 9 | Compound L12 | 49.1 5 |
| Compound B39 | 1.83 | Compound C24 | 1.4 2 | Compound K30 | 1.1 4 | Compound L13 | 48.8 0 |
| Compound B40 | 1.00 | Compound D1 | 1.5 5 | Compound K31 | 1.1 2 | Compound L14 | 63.7 6 |
| Compound B41 | 0.75 | Compound D2 | 1.1 7 | Compound K32 | 3.1 2 | Compound L15 | 8.02 |
| Compound B42 | 2459 | Compound D3 | 8.6 5 | Compound K33 | 0.8 6 | Compound L16 | 7.09 |
| Compound B43 | 4.04 | Compound D4 | 1.0 7 | Compound K34 | 1.6 3 | Compound L18 | 44.9 7 |
| Compound B44 | 4.68 | Compound D5 | 0.9 0 | Compound K35 | 1.0 7 | Compound L19 | 61.9 5 |
| Compound B45 | 3.07 | Compound D6 | 1.2 6 | Compound K36 | 1.1 9 | Compound L20 | 63.2 3 |
| Compound B46 | >100 00 | Compound D7 | 1.8 8 | Compound K37 | 1.2 1 | Compound L21 | 26.5 5 |
| Compound B47 | 1.29 | Compound D8 | 6.8 1 | Compound K38 | 1.1 0 | Compound L22 | 18.5 8 |
| Compound B48 | 4.35 | Compound D9 | 0.2 7 | Compound K39 | 1.3 9 | Compound L23 | 48.3 6 |
| Compound B49 | 316 | Compound D10 | 1.5 5 | Compound K40 | 1.3 3 | Compound L24 | 46.0 3 |
| Compound C1 | 1.15 | Compound D11 | 1.5 9 | Compound K41 | 40. 98 | Compound L25 | 7.97 |
| Compound C2 | 1.68 | Compound D12 | 1.1 0 | Compound K42 | 1.1 6 | Compound L26 | 6.52 |
| Compound C3 | 10.19 | Compound D13 | 1.1 8 | Compound K43 | 1.5 3 | Compound L27 | 8.51 |
| Compound C4 | 1.17 | Compound E1 | 1.1 3 | Compound K44 | 9.7 5 | Compound M12 | 2 |
| Compound C5 | 1.30 | Compound E2 | 7.1 4 | Compound K45 | 2.3 6 | Compound M14 | 30 |
| Compound C6 | 2.93 | Compound F1 | 1.8 2 | Compound K46 | 1.0 5 | Compound M15 | 6 |
| Compound C7 | 1.75 | Compound F2 | 2.4 8 | Compound K47 | 1.0 8 | Compound K16 | 3 |
| Compound C8 | 0.99 | Compound F4 | 1.1 6 | Compound K48 | 6.3 0 | Compound M17 | 8 |
| Compound C9 | 1.14 | Compound F5 | 1.3 0 | Compound K50 | 2.7 2 | Compound N1 | 1.18 |

(continued)

| Compound | $IC_{50}$ (T-47D) [nM] | Compound | $IC_{50}$ (T-47 D) [n M] | Compound | $IC_{50}$ (T-47 D) [n M] | Compound | $IC_{50}$ (T-47D ) [nM ] |
|---|---|---|---|---|---|---|---|
| Compound C10 | 0.50 | Compound F6 | 18. 17 | Compound L2 | 0.0 3 | Compound N2 | 3.63 |
| Compound C11 | 0.66 | Compound F7 | 1.6 8 | Compound L3 | 5 | Compound N3 | 4.92 |
| Compound C12 | 37.40 | Compound F8 | 1.8 1 | Compound L4 | 9.2 3 | Compound N4 | 3.00 |
| Compound C13 | 0.61 | Compound F9 | 3.1 7 | Compound L5 | 0.4 5 | Compound N5 | 1.79 |
| Compound C14 | 0.96 | Compound F10 | >1 00 0 | Compound L6 | 0.0 01 | Compound N6 | 0.87 |
| Compound C15 | 9.07 | Compound F11 | 10. 39 | Compound L7 | 3.5 5 | Compound N7 | 1.72 |
| Compound C17 | 3.58 | Compound G1 | 6.0 1 | Compound L8 | 13. 49 | Compound N8 | 6.60 |
| Compound C18 | 11.14 | Compound G3 | 5.2 6 | Compound L9 | 0.0 1 | Compound N9 | 5.55 |
| Compound G4 | 0.95 | Compound H13 | 1.6 8 | Compound L10 | 17. 89 | Compound N10 | 3.42 |
| Compound G6 | 4.21 | Compound H14 | 1.7 3 | Compound N11 | 1.5 1 | Compound N12 | 1.69 |
| Compound N13 | 2.53 | | | | | | |

Conclusion: All of the PROTAC compounds provided by the present disclosure are capable of effectively inhibiting the proliferation of T-47D cells, and the inhibitory effect of some of the compounds is comparable to, or even superior to, that of the positive control sample.

**Test Example 3 Inhibitory effect of the PROTAC compounds provided in the present disclosure on cell proliferation of MCF-7 cells**

The complete medium required for MCF-7 cells (source ATCC) was DMEM (gibco, Product number :11995-065) basal medium containing 10% fetal bovine serum. MCF-7 cells were inoculated in 96-well plates at a density of 800 cells/well, and different concentrations of the PROTAC compounds 1-55 provided in the examples of the present disclosure and a positive control sample ARV-471 (source: Innochem) were prepared to treat the cells, and the cells were treated at 37°C in a cell culture incubator with 5% $CO_2$ for 6 days. Cell viability was detected using a CCK-8 kit (Beyotime, Product number: C0040) according to the manufacturer's instructions. The $IC_{50}$ values of each PROTAC compound after 6 days of treatment were calculated using GraphPadPrism software based on the inhibition rate, and the results are shown in Table 4.

Table 4 $IC_{50}$ values for inhibition of the proliferation of MCF-7 cells by the PROTAC compounds of the present disclosure and positive control sample

| Compound | $IC_{50}$(MCF-7) [μM] | Compound | $IC_{50}$(MCF-7) [μM] |
|---|---|---|---|
| ARV-471 | 17.91 | Compound B3 | 100 |
| Compound A7 | 99.92 | Compound B5 | 100 |
| Compound A4 | 5.27 | Compound A21 | 2.52 |
| Compound A6 | 48.16 | Compound M1 | 44.08 |

(continued)

| Compound | $IC_{50}$(MCF-7) [μM] | Compound | $IC_{50}$(MCF-7) [μM] |
|---|---|---|---|
| Compound A9 | 100.00 | Compound M2 | 4.98 |
| Compound A1 | 49.49 | Compound B4 | 10.01 |
| Compound A12 | 4.60 | Compound A29 | 5.48 |
| Compound A10 | 37.21 | Compound M4 | 19.28 |
| Compound A11 | 4.22 | Compound M6 | 4.38 |
| Compound O3 | 86.75 | Compound A22 | 0.94 |
| Compound A2 | 34.04 | Compound M3 | 4.99 |
| Compound A17 | 4.44 | Compound B2 | 10.18 |
| Compound A15 | 1.19 | Compound M5 | 5.66 |
| Compound A13 | 24.50 | Compound A27 | 4.36 |
| Compound A14 | 4.82 | Compound A26 | 3.78 |
| Compound O1 | 53.55 | Compound M7 | 13.63 |
| Compound O2 | 100 | Compound A24 | 12.27 |
| Compound A16 | 100 | Compound A30 | 30.18 |
| Compound A8 | 19.00 | Compound M8 | 51.71 |
| Compound A19 | 100 | Compound A25 | 23.45 |
| Compound A20 | 11.57 | Compound M9 | 100 |
| Compound B1 | 100 | Compound M10 | 100 |
| Compound B7 | 100 | | |

Test Example 4 Inhibitory effect of the PROTAC compounds disclosed in the present disclosure on cell proliferation of MCF-7 cells

The complete medium required for MCF-7 cells (source ATCC) was DMEM (gibco, Product number :11995-065) basal medium containing 10% fetal bovine serum. MCF-7 cells were inoculated in 96-well plates at a density of 800 cells/well, and different concentrations of the PROTAC compounds 1-55 provided in the embodiments of the present disclosure and a positive control sample ARV-471 (source: Innochem) were prepared to treat cells, and cells were treated at 37°C in a cell culture incubator with 5% $CO_2$ for 9 days. Cell viability was detected using a CCK -8 kit (Beyotime , Product number: C0040) according to the manufacturer's instruction. The $IC_{50}$ values of each PROTAC compound after 9 days of treatment were calculated using GraphPadPrism software based on the inhibition rate, and the results are shown in Tables 5 and 6.

Table 5 $IC_{50}$ values for inhibition of the proliferation of MCF-7 cells by the PROTAC compounds of the present disclosure and positive control sample

| Compound | $IC_{50}$(MCF-7) [μM] |
|---|---|
| ARV-471 | 0.07 |
| Compound A18 | 0.17 |
| Compound A28 | 0.06 |
| Compound M12 | 0.01 |
| Compound A23 | 0.01 |
| Compound M11 | 0.02 |
| Compound A33 | 0.03 |
| Compound M13 | 0.07 |

(continued)

| Compound | $IC_{50}$(MCF-7) [μM] |
|---|---|
| Compound B8 | 0.09 |
| Compound A32 | 0.04 |
| Compound B10 | 0.04 |
| Compound L1 | 0.13 |

Table 6 $IC_{50}$ values for inhibition of the proliferation of MCF-7 cells by the PROTAC compounds of the present disclosure

| Compound | $IC_{50}$ (MCF-7) [nM] | Compound | $IC_{50}$ (MCF-7) [nM] | Compound | $IC_{50}$ (MCF-7) [nM] |
|---|---|---|---|---|---|
| Compound A3 | 44.91 | Compound A49 | 11073 | Compound H2 | 5897 |
| Compound A5 | >100 | Compound A50 | 4077 | Compound H3 | 2412 |
| Compound A8 | 19.00 | Compound A51 | 2925 | Compound H4 | 7740 |
| Compound A12 | 4.60 | Compound A52 | 5680 | Compound H5 | 2261 |
| Compound A28 | 66 | Compound A53 | < 0.256 | Compound H6 | 4170 |
| Compound A31 | >100 | Compound A54 | < 0.256 | Compound H7 | 330 |
| Compound A32 | >100 | Compound A55 | < 0.256 | Compound H8 | 27168 |
| Compound A34 | 174 | Compound A56 | 2792 | Compound H9 | 5020 |
| Compound A35 | >100 | Compound A57 | 2792 | Compound H10 | 9280 |
| Compound A36 | 270 | Compound A58 | 12.84 | Compound H11 | 24090 |
| Compound A37 | 37 | Compound A59 | 905.49 | Compound H12 | 35.53 |
| Compound A38 | 240 | Compound A60 | 824.86 | Compound H13 | 3.24 |
| Compound A39 | 30 | Compound A61 | 836.63 | Compound H14 | > 100000 |
| Compound A40 | 76 | Compound A62 | 15.15 | Compound H15 | 43.45 |
| Compound A41 | 33 | Compound A63 | 3.24 | Compound H16 | 9771 |
| Compound A42 | 38 | Compound A64 | 206 | Compound H17 | 62.70 |
| Compound A43 | 62 | Compound A65 | 125 | Compound H18 | 7375 |
| Compound A44 | 2770 | Compound A66 | 1.39 | Compound H19 | 151 |
| Compound A45 | 160 | Compound A67 | 3.96 | Compound H20 | 1324 |
| Compound A46 | 1250 | Compound A68 | 19.14 | Compound H21 | 163 |
| Compound A47 | 871 | Compound A69 | 90.76 | Compound H22 | 3.50 |
| Compound A48 | 7441 | Compound A70 | 41.91 | Compound H23 | 9.19 |
| Compound A107 | 12.8 | Compound B7 | >100 | Compound H24 | 1176 |
| Compound A108 | 7394 | Compound B8 | 6.57 | Compound K1 | 511 |
| Compound A109 | 42.82 | Compound B9 | 86 | Compound K2 | 1990 |
| Compound A111 | 93.59 | Compound B10 | 37 | Compound K3 | 4297 |
| Compound A112 | 59.36 | Compound B11 | 4610 | Compound K4 | < 0.256 |
| Compound A113 | 21.64 | Compound B12 | 950 | Compound K5 | 132 |
| Compound A114 | 10.28 | Compound B13 | 310 | Compound K6 | 724 |
| Compound A115 | 60.56 | Compound B14 | <0.256 | Compound K7 | 10.58 |
| Compound A116 | 25234 | Compound B15 | 48.30 | Compound K8 | 10.50 |
| Compound A117 | 63.28 | Compound B16 | 740 | Compound K11 | 97.16 |

(continued)

| Compound | $IC_{50}$ (MCF-7) [nM] | Compound | $IC_{50}$ (MCF-7) [nM] | Compound | $IC_{50}$ (MCF-7) [nM] |
|---|---|---|---|---|---|
| Compound A118 | 16.26 | Compound B17 | 16.59 | Compound K12 | 30 |
| Compound A119 | 19.36 | Compound B18 | 15.59 | Compound K13 | 1238 |
| Compound A120 | 12.36 | Compound B19 | 4170 | Compound K14 | 220 |
| Compound A121 | 22.45 | Compound B20 | 1627 | Compound K15 | 196 |
| Compound A122 | 14830 | Compound B21 | 312 | Compound K16 | 214 |
| Compound A123 | 164 | Compound B22 | 11.78 | Compound K17 | 70.03 |
| Compound A124 | 56369 | Compound B23 | 417 | Compound K18 | 12.61 |
| Compound A71 | 14.49 | Compound B29 | 224 | Compound K19 | 48.58 |
| Compound A72 | 6841 | Compound B30 | 9.92 | Compound K20 | 19.64 |
| Compound A73 | 4170 | Compound B31 | 19.81 | Compound K21 | 71.87 |
| Compound A74 | >1000 00 | Compound B32 | 528 | Compound K22 | 24.27 |
| Compound A75 | 7.71 | Compound B33 | 28.11 | Compound K23 | 5.67 |
| Compound A76 | 19259 | Compound B34 | 179 | Compound K24 | 32.45 |
| Compound A77 | 52112 | Compound B35 | 49.70 | Compound K25 | 2158 |
| Compound A78 | 3.35 | Compound B36 | 21717 | Compound K26 | 48.18 |
| Compound A79 | 2.59 | Compound B37 | 2797 | Compound K27 | 33.13 |
| Compound A80 | >1000 00 | Compound B38 | 90.45 | Compound K28 | 8.18 |
| Compound A81 | 22.32 | Compound B39 | 25111 | Compound K29 | 19.48 |
| Compound A82 | 448 | Compound B40 | 45.32 | Compound K30 | 7.34 |
| Compound A83 | 39.00 | Compound B41 | 29187 | Compound K31 | 1.53 |
| Compound H41 | 5.05 | Compound B42 | 90899 | Compound K32 | 0.779 |
| Compound A84 | 5.26 | Compound B43 | 232 | Compound K33 | 17.51 |
| Compound A85 | 9880 | Compound B44 | 35.45 | Compound K34 | 10.51 |
| Compound A86 | 84.96 | Compound B45 | 186 | Compound K35 | 32.75 |
| Compound A88 | 66.80 | Compound B46 | 7.91 | Compound K48 | 9213 |
| Compound A89 | 110 | Compound B47 | 27104 | Compound K50 | 83.3 |
| Compound A90 | 52.75 | Compound B48 | 33.52 | Compound N8 | 25350 |
| Compound A91 | 325 | Compound B49 | 7148 | Compound N9 | 127783 |
| Compound A92 | 11.57 | Compound C1 | 8.05 | Compound N10 | 792 |
| Compound A93 | 10.54 | Compound C2 | 31.08 | Compound N11 | 4.29 |
| Compound A94 | 44.98 | Compound C3 | 98.74 | Compound J5 | 4.63 |
| Compound A95 | 201 | Compound C4 | 66.96 | Compound J6 | 9.31 |
| Compound A96 | 10.99 | Compound C5 | 6.70 | Compound J7 | 486 |
| Compound A97 | 217 | Compound C6 | 92.5 | Compound J8 | 4.89 |
| Compound A98 | 5.12 | Compound C7 | 84.69 | Compound J9 | 5.64 |
| Compound A99 | 4.19 | Compound C8 | 2731 | Compound J10 | 6.12 |
| Compound A100 | 2.27 | Compound C9 | 63652 | Compound J11 | 4374 |
| Compound A101 | 850 | Compound C10 | 54.83 | Compound J12 | 1943 |
| Compound A102 | 107 | Compound C11 | 5968 | Compound J13 | 440 |

(continued)

| Compound | $IC_{50}$ (MCF-7) [nM] | Compound | $IC_{50}$ (MCF-7) [nM] | Compound | $IC_{50}$ (MCF-7) [nM] |
|---|---|---|---|---|---|
| Compound A103 | 13.64 | Compound C12 | 180 | Compound J14 | 9664 |
| Compound A104 | 30.30 | Compound C13 | 3.24 | Compound J15 | 22036 |
| Compound A105 | 26556 | Compound C14 | 15.03 | Compound J16 | 2250 |
| Compound A106 | 190.4 | Compound C15 | 406 | Compound L5 | 830 |
| Compound D6 | 32950 | Compound C17 | 1142 | Compound L6 | 930 |
| Compound D7 | 2345 | Compound C18 | 11408 | Compound L7 | 510 |
| Compound D8 | 11419 | Compound C19 | 23123 | Compound L8 | 990 |
| Compound D9 | 10.84 | Compound C22 | 564 | Compound L9 | 1420 |
| Compound D10 | 12.54 | Compound D1 | 9.05 | Compound L10 | 925 |
| Compound D11 | 19.20 | Compound D2 | 3623 | Compound L11 | 5 |
| Compound D13 | 186 | Compound D3 | 6.58 | Compound L12 | 9633 |
| Compound E1 | 12.59 | Compound D4 | 3581 | Compound L13 | 2951 |
| Compound E2 | 34.77 | Compound D5 | 180 | Compound L14 | 1003 |
| Compound F1 | 25.34 | Compound H25 | 1715 | Compound L15 | 19 |
| Compound F2 | 5128 | Compound H26 | 1256 | Compound L16 | 39 |
| Compound F4 | 0.103 | Compound H27 | 800 | Compound L18 | 544 |
| Compound F5 | 1.21 | Compound H28 | 8.41 | Compound L19 | 86 |
| Compound F6 | 4223 | Compound H29 | 10.92 | Compound L20 | 2186 |
| Compound F7 | 3781 | Compound H30 | 0.115 | Compound L21 | 91 |
| Compound F8 | 203 | Compound H31 | 43.31 | Compound L22 | 111 |
| Compound F9 | 4364 | Compound H32 | 4023 | Compound L23 | 447 |
| Compound F10 | 3.81 | Compound H33 | 12.46 | Compound L24 | 159 |
| Compound H42 | 9.28 | Compound H34 | 10.94 | Compound L25 | 757 |
| Compound H44 | 5.06 | Compound H35 | 34.39 | Compound L26 | 828 |
| Compound H45 | 16.23 | Compound H36 | 72.44 | Compound L27 | >100000 |
| Compound H46 | 140 | Compound H38 | 31.83 | Compound M12 | 12 |
| Compound H47 | 363 | Compound H39 | 65.22 | Compound M14 | 23 |
| Compound H48 | 1263 | Compound H40 | 57.85 | Compound M15 | 48 |
| Compound F11 | 103 | Compound H49 | 5.08 | Compound K16 | 320 |
| Compound G1 | 4.13 | Compound H50 | 9562 | Compound M17 | 30 |
| Compound G2 | 20.59 | Compound H51 | 153 | Compound N 1 | 14.81 |
| Compound G4 | 3.77 | Compound H52 | 5322 | Compound N2 | 247 |
| Compound G6 | 25353 | Compound H53 | 41.94 | Compound N3 | 195 |
| Compound G7 | 217 | Compound H54 | 260 | Compound N4 | 132 |
| Compound G8 | 749 | Compound H55 | 199 | Compound N5 | 34.10 |
| Compound G9 | 20.67 | Compound H56 | 2254 | Compound K36 | 15.17 |
| Compound G10 | 18.21 | Compound H57 | 7366 | Compound K37 | 2513 |
| Compound G11 | 890 | Compound H58 | 2320 | Compound K38 | 4803 |
| Compound G12 | 9.27 | Compound H59 | 7361 | Compound K39 | 58.98 |

(continued)

| Compound | $IC_{50}$ (MCF-7) [nM] | Compound | $IC_{50}$ (MCF-7) [nM] | Compound | $IC_{50}$ (MCF-7) [nM] |
|---|---|---|---|---|---|
| Compound G13 | 6.82 | Compound H60 | 106 | Compound K40 | 2350 |
| Compound G15 | 10.16 | Compound H61 | 4025 | Compound K41 | 590 |
| Compound G16 | 3.07 | Compound I2 | 11.68 | Compound K42 | 14.62 |
| Compound G17 | 626 | Compound I3 | 14.90 | Compound K43 | 3074 |
| Compound G18 | 129 | Compound I4 | 119.05 | Compound K44 | 936 |
| Compound G20 | 49.72 | Compound I5 | 10.46 | Compound K45 | 75.16 |
| Compound G21 | 8.60 | Compound I6 | 6.57 | Compound K46 | 85.05 |
| Compound G22 | 5.08 | Compound J1 | 857 | Compound K47 | 80.19 |
| Compound G23 | 445 | Compound J2 | 303468 | Compound N12 | 167 |
| Compound G25 | 8.97 | Compound J3 | 8.23 | Compound N13 | 8.18 |
| Compound G26 | 32428 | Compound J4 | 22.62 | Compound L2 | 730 |
| Compound L4 | 970 | Compound L3 | 130 | | |

**Test Example 5 Degradation of ERα by the PROTAC compounds disclosed in the present disclosure in MCF-7 cells**

MCF-7 (ATCC) cells were cultured in DMEM medium (11995-065, Gibco) containing 10% FBS (10099141C, Gibco) at 37°C, 5% $CO_2$ until reaching the logarithmic growth phase. MCF-7 cells were inoculated into 6-well plates at a density of $3\times10^5$ cells/well and cultured at 37°C, 5% $CO_2$ for 24h. After the cells were attached to the wall, 1μL of the compound solution in dimethyl sulfoxide (final concentration 0.15-1000nM) was added and continued to be cultured in incubator at 37°C with 5% $CO_2$ for 4 h. After 4 h, the culture medium was discarded, and each well was washed once with pre-cooled 1×DPBS, and the cells were lysed with a mixture of 50 μL of RIPA lysis buffer (P0013B, Beyotime) and 1× protease inhibitor (P1005, Beyotime), and then centrifuged at 14,000 g for 30 min at 4°C after resting on ice for 20 min, and the supernatant was carefully collected to detect the protein level of ERα by protein immunoblotting (Immunoblotting).

Protein immunoblotting: The total protein concentration in the collected cell lysate supernatant was determined by BCA Protein Concentration Assay Kit (Enhanced) (P0009, Beyotime). According to the total protein concentration detected by BCA, the protein was adjusted to 1 μg/μL with RIPA lysis buffer and 5× SDS-PAGE protein loading buffer (MB01015, Genscript), and the protein was denatured by water bath at 95°C for 5 min, and then centrifuged after denaturation to enrich the condensate on the wall of the centrifuge tubes as a loading sample. 20 μL of the sample (20 μg of total protein) was loaded into to the wells of a 12% precast gel (M00669, Genscript), and electrophoresis was carried out at 100 V for 20 min and then switched to 150 V for 40 min. At the end of electrophoresis, the proteins in the SDS-PAGE gels were transferred to a PVDF membrane at a constant voltage of 100 V for 60 min. After that, the PVDF membrane corresponding to ERα protein and internal reference protein was cut out and incubated in 1×QμickBlock blocking solution (P0252, Beyotime) for 30 min at room temperature on a shaker. After blocking, ERα primary antibody (1:1000, 8644s, CST) was added and incubated overnight at 4°C. At the end of the incubation, the PVDF membrane was washed three times with 1× TBST buffer for 5 min each time; Rabbit IgG (H+L) secondary antibody (1:7500, SA5-35571, Invitrogen) was added and incubated for 1h at room temperature on the shaker, and the PVDF membrane was again washed three times with 1× TBST buffer for 5 min each time; finally, the scanning imaging was conducted in a dual-color infrared laser imaging system ( Odyssey DLx, LI-COR). Protein profiles were analyzed by Image Studio Lite analysis software for grayscale values. Grayscale correction values were calculated for each sample using the Formula: corrected grayscale value = grayscale value of the target protein /corresponding grayscale value of the internal reference. The degradation rate for the compounds was then calculated by comparing the corrected grayscale values of the compound group to the control group.

Table 7 Degradation rate (%) of ERα by the PROTAC compounds of the present disclosure and positive control sample in MCF-7 cells

| Degradation rate (%) of ERα in MCF-7 cells | | | | | |
|---|---|---|---|---|---|
| Compound | 10nM | 1nM | Compound | 10nM | 1nM |
| ARV-471 | 76 | 38 | Compound A107 | 58 | 37 |

(continued)

| Degradation rate (%) of ERα in MCF-7 cells | | | | | |
|---|---|---|---|---|---|
| Compound | 10nM | 1nM | Compound | 10nM | 1nM |
| Compound A1 | -17 | | Compound A108 | 46 | 22 |
| Compound A2 | -22 | | Compound A109 | 54 | 10 |
| Compound A3 | 50 | | Compound A111 | 74 | 39 |
| Compound A4 | -17 | | Compound A112 | 63 | 15 |
| Compound A5 | -6 | | Compound A113 | 52 | 4 |
| Compound A6 | -17 | | Compound A114 | 62 | 46 |
| Compound A7 | -29 | | Compound A115 | 16 | 0 |
| Compound A8 | -42 | | Compound A116 | 4 | 25 |
| Compound A9 | -24 | | Compound A117 | 47 | 48 |
| Compound A10 | -3 | | Compound A118 | 55 | -7 |
| Compound A11 | -17 | | Compound A119 | 66 | 12 |
| Compound A12 | 4 | | Compound A120 | 74 | 52 |
| Compound A13 | -9 | | Compound A121 | 78 | 52 |
| Compound A14 | 5 | | Compound A122 | 51 | 40 |
| Compound A15 | -2 | | Compound A123 | 61 | 29 |
| CompoundA16 | -5 | | Compound A124 | 20 | 18 |
| Compound A17 | -2 | | Compound B1 | -35 | |
| Compound A18 | 20 | | Compound B2 | -26 | |
| Compound A19 | 18 | | Compound B3 | -14 | |
| Compound A20 | 18 | | Compound B4 | -34 | |
| Compound A21 | 4 | | Compound B5 | 4 | |
| Compound A22 | 13 | | Compound B7 | -6 | |
| Compound A23 | 14 | | Compound B8 | 16 | |
| Compound A24 | 32 | | Compound B9 | 17 | 0 |
| Compound A25 | 8 | | Compound B10 | 23 | 3 |
| Compound A26 | 13 | | Compound B11 | -16 | -40 |
| Compound A27 | 4 | | Compound B12 | -11 | -8 |
| Compound A29 | 8 | | Compound B13 | -4 | -9 |
| Compound A30 | 23 | | Compound B14 | 73 | 12 |
| Compound A31 | 49 | | Compound B15 | -18 | -13 |
| Compound A32 | -6 | | Compound B16 | 16 | -36 |
| Compound A33 | 26 | -7 | Compound B17 | 61 | 37 |
| Compound A34 | 13 | 4 | Compound B18 | 64 | 36 |
| Compound A35 | 54 | | Compound B19 | 72 | 26 |
| Compound A36 | -14 | 11 | Compound B20 | 64 | 42 |
| Compound A37 | 8 | -12 | Compound B21 | 55 | 38 |
| Compound A38 | -24 | -1 | Compound B22 | 70 | 38 |
| Compound A39 | -27 | -16 | Compound B23 | 40 | 17 |

(continued)

| Degradation rate (%) of ERα in MCF-7 cells | | | | | |
|---|---|---|---|---|---|
| Compound | 10nM | 1nM | Compound | 10nM | 1nM |
| Compound A40 | 35 | 15 | Compound B29 | 16 | 7 |
| Compound A41 | -18 | -9 | Compound B30 | 40 | 19 |
| Compound A42 | -36 | -49 | Compound B31 | 71 | 38 |
| Compound A43 | -8 | -2 | Compound B32 | 6 | -7 |
| Compound A44 | 22 | 8 | Compound B33 | 46 | 28 |
| Compound A45 | -15 | -3 | Compound B34 | 71 | 64 |
| Compound A46 | -26 | -22 | Compound B35 | 72 | 60 |
| Compound A47 | 14 | 9 | Compound B36 | 55 | 55 |
| Compound A48 | 11 | 4 | Compound B37 | 41 | 11 |
| Compound A49 | 37 | 9 | Compound B38 | 57 | 63 |
| Compound A50 | 27 | 11 | Compound B39 | 49 | 41 |
| Compound A51 | 15 | 5 | Compound B40 | 61 | 54 |
| Compound A52 | -33 | -22 | Compound B41 | 53 | 59 |
| Compound A53 | 45 | 10 | Compound B42 | 26 | 17 |
| Compound A54 | 65 | 40 | Compound B43 | 62 | 54 |
| Compound A55 | 42 | 22 | Compound B44 | 62 | 34 |
| Compound A58 | 69 | 42 | Compound B45 | 49 | 15 |
| Compound A59 | 11 | 11 | Compound B46 | 50 | 40 |
| Compound A60 | -14 | -31 | Compound B47 | 33 | 28 |
| Compound A61 | -19 | -4 | Compound B48 | 74 | 63 |
| Compound A62 | 82 | 52 | Compound B49 | 43 | 24 |
| Compound A63 | 68 | 69 | Compound B50 | 64 | 61 |
| Compound A64 | 47 | 13 | Compound C1 | 49 | 18 |
| Compound A65 | 23 | 23 | Compound C2 | 63 | 42 |
| Compound A66 | 54 | 3 | Compound C3 | 63 | 10 |
| Compound A67 | 67 | 27 | Compound C4 | 75 | 38 |
| Compound A68 | 40 | 11 | Compound C5 | 66 | 43 |
| Compound A69 | 35 | 21 | Compound C6 | 42 | 27 |
| Compound A70 | 71 | 18 | Compound C7 | 63 | 38 |
| Compound A71 | 73 | 32 | Compound C8 | 40 | 29 |
| Compound A72 | 64 | 33 | Compound C9 | 41 | 39 |
| Compound A73 | 72 | 26 | Compound C10 | 65 | 53 |
| Compound A74 | 62 | 28 | Compound C11 | 69 | 60 |
| Compound A75 | 14 | 16 | Compound C12 | 22 | -34 |
| Compound A76 | 12 | -6 | Compound C13 | 62 | 41 |
| Compound A77 | 61 | 29 | Compound C14 | 55 | 41 |
| Compound A78 | 78 | 64 | Compound C15 | 45 | 18 |
| Compound A79 | 47 | 45 | Compound C17 | 31 | 16 |

(continued)

| Degradation rate (%) of ERα in MCF-7 cells | | | | | |
|---|---|---|---|---|---|
| Compound | 10nM | 1nM | Compound | 10nM | 1nM |
| Compound A80 | 44 | 29 | Compound C18 | 29 | 10 |
| Compound A81 | 20 | -39 | Compound C19 | 45 | 15 |
| Compound A82 | 51 | 16 | Compound C22 | 25 | 10 |
| Compound A83 | 46 | 23 | Compound C23 | 55 | 39 |
| Compound A84 | 74 | 63 | Compound C24 | 56 | 43 |
| Compound A85 | 65 | 45 | Compound D1 | 68 | 19 |
| Compound A86 | 32 | 5 | Compound D2 | 29 | 31 |
| Compound A88 | 54 | 57 | Compound D3 | 60 | 38 |
| Compound A89 | 72 | 48 | Compound D4 | 69 | 41 |
| Compound A90 | 72 | 50 | Compound D5 | 58 | 61 |
| Compound A91 | 33 | 2 | Compound D6 | 59 | 38 |
| Compound A92 | 66 | 36 | Compound D7 | 64 | 36 |
| Compound A93 | 69 | 31 | Compound D8 | 62 | 9 |
| Compound A94 | 48 | 22 | Compound D9 | 55 | 22 |
| Compound A95 | 48 | 42 | Compound D10 | 58 | 3 |
| Compound A96 | 66 | 40 | Compound D11 | 66 | 6 |
| Compound A97 | 75 | 50 | Compound D12 | 61 | 39 |
| Compound A98 | 90 | 48 | Compound D13 | 68 | 42 |
| Compound A99 | 77 | 35 | Compound E1 | 75 | 39 |
| Compound A100 | 73 | 65 | Compound E2 | 39 | 9 |
| Compound A101 | 61 | 52 | Compound H25 | -50 | -9 |
| Compound A102 | 73 | 36 | Compound H26 | -11 | -9 |
| Compound A103 | 70 | 37 | Compound H27 | 46 | 11 |
| Compound A104 | 70 | 15 | Compound H28 | 65 | 36 |
| Compound A105 | 43 | 7 | Compound H29 | 42 | 12 |
| Compound A106 | 62 | 19 | Compound H30 | 62 | -1 |
| Compound F1 | 66 | 59 | Compound H31 | 83 | 46 |
| Compound F2 | -2 | -5 | Compound H32 | 58 | 35 |
| Compound F4 | 63 | 64 | Compound H33 | 76 | 32 |
| Compound F5 | 23 | 20 | Compound H34 | 65 | 42 |
| Compound F6 | -32 | -35 | Compound H35 | 67 | 26 |
| Compound F7 | 59 | 35 | Compound H36 | 70 | 24 |
| Compound F8 | 63 | 63 | Compound H38 | 71 | 49 |
| Compound F9 | 45 | 46 | Compound H39 | 71 | 20 |
| Compound F10 | 43 | 53 | Compound H40 | 84 | 69 |
| Compound F11 | 43 | 14 | Compound H41 | 80 | 63 |
| Compound G1 | 35 | 25 | Compound H42 | 77 | 49 |
| Compound G3 | 79 | 47 | Compound H44 | 73 | 36 |

(continued)

| Degradation rate (%) of ERα in MCF-7 cells | | | | | |
|---|---|---|---|---|---|
| Compound | 10nM | 1nM | Compound | 10nM | 1nM |
| Compound G4 | 14 | 33 | Compound H45 | 61 | 25 |
| Compound G6 | 46 | 8 | Compound H46 | 77 | 53 |
| Compound G7 | 54 | 15 | Compound H47 | 76 | 39 |
| Compound G8 | 73 | 29 | Compound H48 | 71 | 38 |
| Compound G9 | 84 | 40 | Compound H49 | 71 | 50 |
| Compound G10 | 66 | 45 | Compound H50 | 71 | 32 |
| Compound G11 | 56 | 38 | Compound H51 | 75 | 46 |
| Compound G12 | 57 | 24 | Compound H52 | 61 | 53 |
| Compound G13 | 71 | 30 | Compound H53 | 14 | 10 |
| Compound G15 | 20 | -19 | Compound H54 | 44 | 11 |
| Compound G16 | 70 | 54 | Compound H55 | 62 | 28 |
| Compound G17 | 55 | 52 | Compound H56 | 21 | -5 |
| Compound G18 | 77 | 63 | Compound H57 | 60 | 38 |
| Compound G20 | 45 | 22 | Compound H58 | 70 | 50 |
| Compound G21 | 57 | 45 | Compound H59 | 34 | 40 |
| Compound G22 | 65 | 50 | Compound H60 | 35 | 38 |
| Compound G23 | 43 | 1 | Compound H61 | 40 | 37 |
| Compound G25 | 77 | 10 | Compound I2 | 61 | 63 |
| Compound G26 | 66 | 37 | Compound I3 | 67 | 40 |
| Compound H1 | -16 | -13 | Compound I4 | 54 | 39 |
| Compound H2 | -70 | -77 | Compound I5 | 56 | 60 |
| Compound H3 | -37 | -20 | Compound I6 | 45 | 57 |
| Compound H4 | -7 | -32 | Compound J1 | -14 | 9 |
| Compound H5 | -12 | -3 | Compound J2 | 41 | 21 |
| Compound H6 | 7 | -6 | Compound J3 | 49 | 34 |
| Compound H7 | -2 | 12 | Compound J4 | 60 | 32 |
| Compound H8 | -48 | -23 | Compound J5 | 78 | 40 |
| Compound H9 | -54 | -39 | Compound J6 | 68 | 52 |
| Compound H10 | -4 | -28 | Compound J7 | 75 | 29 |
| Compound H11 | -41 | -39 | Compound J8 | 85 | 78 |
| Compound H12 | 28 | 4 | Compound J9 | 69 | 63 |
| Compound H13 | 62 | 39 | Compound J10 | 73 | 50 |
| Compound H14 | -13 | 8 | Compound J11 | 75 | 40 |
| Compound H15 | 51 | 29 | Compound J12 | 53 | 60 |
| Compound H16 | 37 | 9 | Compound J13 | 71 | 37 |
| Compound H17 | 54 | 47 | Compound J14 | 39 | 5 |
| Compound H18 | 58 | 18 | Compound J15 | 66 | 33 |
| Compound H19 | 21 | 27 | Compound J16 | 47 | 17 |

(continued)

| Degradation rate (%) of ERα in MCF-7 cells | | | | | |
|---|---|---|---|---|---|
| Compound | 10nM | 1nM | Compound | 10nM | 1nM |
| Compound H20 | -14 | -18 | Compound L5 | 4 | 6 |
| Compound H21 | 37 | 18 | Compound L6 | 45 | 14 |
| Compound H22 | 64 | 32 | Compound L7 | -4 | 4 |
| Compound H23 | 51 | 44 | Compound L8 | 27 | 27 |
| Compound H24 | 11 | -7 | Compound L9 | 29 | 10 |
| Compound K1 | -3 | -2 | Compound L10 | -6 | -11 |
| Compound K2 | 3 | -10 | Compound L11 | 60 | 37 |
| Compound K3 | -25 | 1 | Compound L12 | -14 | -27 |
| Compound K4 | 53 | 48 | Compound L13 | -13 | -6 |
| Compound K5 | 58 | 30 | Compound L14 | -21 | -4 |
| Compound K6 | 62 | 17 | Compound L15 | 57 | 18 |
| Compound K7 | 64 | 55 | Compound L16 | 51 | 29 |
| Compound K8 | 64 | 44 | Compound L17 | 10 | -2 |
| Compound K11 | 25 | 13 | Compound L18 | 5 | -6 |
| Compound K12 | 44 | 3 | Compound L19 | 14 | 9 |
| Compound K13 | 57 | 39 | Compound L20 | 6 | -12 |
| Compound K14 | 14 | -3 | Compound L21 | -17 | -11 |
| Compound K15 | 42 | 16 | Compound L22 | 18 | 3 |
| Compound K16 | 62 | 32 | Compound L23 | 2 | -20 |
| Compound K17 | 35 | 14 | Compound L24 | 7 | 10 |
| Compound K18 | 80 | 43 | Compound L25 | 6 | -20 |
| Compound K19 | 63 | 32 | Compound L26 | 18 | 10 |
| Compound K20 | 70 | 37 | Compound L27 | 52 | 24 |
| Compound K21 | 75 | 35 | Compound M15 | -24 | -23 |
| Compound K22 | 35 | 16 | Compound K16 | -19 | -15 |
| Compound K23 | 78 | 57 | Compound M17 | 0 | -9 |
| Compound K24 | 46 | 31 | Compound N1 | 68 | 54 |
| Compound K25 | 70 | 25 | Compound N2 | -8 | -7 |
| Compound K26 | 88 | 67 | Compound N3 | 1 | -4 |
| Compound K27 | 86 | 71 | Compound N4 | 73 | 54 |
| Compound K28 | 86 | 66 | Compound N5 | 69 | 58 |
| Compound K29 | 66 | 43 | Compound N6 | 55 | 34 |
| Compound K30 | 55 | 48 | Compound N7 | 13 | 2 |
| Compound K31 | 65 | 61 | Compound N8 | 31 | -2 |
| Compound K32 | 88 | 66 | Compound N9 | 39 | 21 |
| Compound K33 | 62 | 57 | Compound N10 | 48 | 57 |
| Compound K34 | 76 | 75 | Compound N11 | 64 | 35 |
| Compound K35 | 64 | 46 | Compound N12 | 64 | 39 |

(continued)

| Degradation rate (%) of ERα in MCF-7 cells | | | | | |
|---|---|---|---|---|---|
| Compound | 10nM | 1nM | Compound | 10nM | 1nM |
| Compound K36 | 61 | 20 | Compound N13 | 46 | 29 |
| Compound K37 | 23 | 20 | Compound K46 | 85 | 54 |
| Compound K38 | 35 | 23 | Compound K47 | 80 | 57 |
| Compound K39 | 56 | 30 | Compound K48 | 42 | 20 |
| Compound K40 | 45 | 14 | Compound K50 | 72 | 19 |
| Compound K41 | 64 | -3 | Compound L1 | -29 | -2 |
| Compound K42 | 83 | 68 | Compound L2 | -27 | -31 |
| Compound K43 | 69 | 23 | Compound L3 | 44 | 23 |
| Compound K44 | 56 | 1 | Compound L4 | 48 | 9 |
| Compound K45 | 59 | 21 | | | |

Test Example 6 Degradation of ERα by the PROTAC compounds disclosed in the present disclosure in T-47D cells
T-47D (ATCC) cells were cultured in PRMI-1640 medium (30-2001, ATCC) containing 10% FBS (10099141C, Gibco) at 37°C, 5% $CO_2$ until reaching the logarithmic growth phase. T-47D cells were inoculated into 6-well plates at a density of $3\times10^5$ cells/well and cultured at 37°C, 5% $CO_2$ for 24 h. After the cells were attached to the wall, 1μL of the compound solution in dimethyl sulfoxide (final concentration 0.15-1000 nM) was added and continued to be cultured in the incubator with 5% $CO_2$ at 37°C for 4 h. After 4 h, the culture medium was discarded, and each well was washed once with pre-cooled 1×DPBS, and the cells were lysed with a mixture of 50 μL of RIPA lysis buffer (P0013B, Beyotime) and 1× protease inhibitor (P1005, Beyotime), and then centrifuged at 14,000 g for 30 min at 4°C after resting on ice for 20 min, and the supernatant was carefully collected to detect the protein level of ERα by protein immunoblotting (Immunoblotting).

Table 8 Degradation rate (%) of ERα by the PROTAC compounds of the present disclosure and positive control sample in T-47D cells

| Degradation rate (%) of ERα in T-47D cells | | | | | |
|---|---|---|---|---|---|
| Compound | 10nM | 1nM | Compound | 10nM | 1nM |
| ARV-471 | 85 | 61 | Compound A107 | 78 | 54 |
| Compound A1 | -11 | | Compound A108 | 73 | 41 |
| Compound A2 | -17 | | Compound A109 | 75 | 31 |
| Compound A3 | 66 | | Compound A111 | 82 | 49 |
| Compound A4 | -1 | | Compound A112 | 78 | 40 |
| Compound A5 | 34 | | Compound A113 | 64 | 26 |
| Compound A6 | -23 | | Compound A114 | 73 | 48 |
| Compound A7 | -14 | | Compound A115 | 43 | 17 |
| Compound A8 | -22 | | Compound A116 | 23 | 28 |
| Compound A9 | -25 | | Compound A117 | 77 | 66 |
| Compound A10 | -37 | | Compound A118 | 79 | 38 |
| Compound A11 | -3 | | Compound A119 | 73 | 51 |
| Compound A12 | -1 | | Compound A120 | 72 | 39 |
| Compound A13 | 12 | | Compound A121 | 74 | 58 |
| Compound A14 | 4 | | Compound A122 | 65 | 56 |
| Compound A15 | 16 | | Compound A123 | 84 | 66 |

(continued)

| Degradation rate (%) of ERα in T-47D cells | | | | | |
|---|---|---|---|---|---|
| Compound | 10nM | 1nM | Compound | 10nM | 1nM |
| Compound A16 | -22 | | Compound A124 | 52 | 52 |
| Compound A17 | -16 | | Compound B1 | -15 | |
| Compound A18 | 14 | | Compound B2 | -92 | |
| CompoundA19 | 18 | | Compound B3 | -18 | |
| Compound A20 | 36 | | Compound B4 | -9 | |
| Compound A21 | -15 | | Compound B5 | -7 | |
| Compound A22 | 19 | | Compound B7 | 19 | |
| Compound A23 | 53 | | Compound B8 | 33 | |
| Compound A24 | 13 | | Compound B9 | 45 | 16 |
| Compound A25 | -1 | | Compound B10 | 46 | 5 |
| Compound A26 | 40 | | Compound B11 | -38 | -34 |
| Compound A27 | -15 | | Compound B12 | -4 | -17 |
| Compound A28 | 36 | 11 | Compound B13 | -7 | -1 |
| Compound A29 | -2 | | Compound B14 | 58 | -4 |
| Compound A30 | 46 | | Compound B15 | 3 | -4 |
| Compound A31 | 77 | | Compound B16 | 60 | 24 |
| Compound A32 | 9 | | Compound B17 | 76 | 52 |
| Compound A33 | 56 | 12 | Compound B18 | 79 | 54 |
| Compound A34 | 28 | 5 | Compound B19 | 73 | 55 |
| Compound A35 | 86 | | Compound B20 | 73 | 47 |
| Compound A36 | -23 | 2 | Compound B21 | 83 | 66 |
| Compound A37 | 35 | 15 | Compound B22 | 83 | 47 |
| Compound A3 8 | 6 | -12 | Compound B23 | 54 | 5 |
| Compound A39 | 6 | 11 | Compound B29 | 14 | -11 |
| Compound A40 | 25 | 6 | Compound B30 | 46 | 15 |
| Compound A41 | 17 | 8 | Compound B31 | 65 | 38 |
| Compound A42 | 30 | 41 | Compound B32 | 64 | 29 |
| Compound A43 | 26 | 7 | Compound B33 | 80 | 51 |
| Compound A44 | 48 | 5 | Compound B34 | 83 | 71 |
| Compound A45 | -28 | -6 | Compound B35 | 88 | 74 |
| Compound A46 | 33 | 7 | Compound B36 | 68 | 54 |
| Compound A47 | 37 | -10 | Compound B37 | 36 | 12 |
| Compound A48 | 23 | -37 | Compound B38 | 63 | 57 |
| Compound A49 | 48 | 17 | Compound B39 | 66 | 47 |
| Compound A50 | 53 | 24 | Compound B40 | 68 | 64 |
| Compound A51 | 3 | -10 | Compound B41 | 74 | 66 |
| Compound A52 | 2 | 3 | Compound B42 | 13 | 3 |
| Compound A53 | 66 | 37 | Compound B43 | 80 | 57 |

(continued)

| Degradation rate (%) of ERα in T-47D cells | | | | | |
|---|---|---|---|---|---|
| Compound | 10nM | 1nM | Compound | 10nM | 1nM |
| Compound A54 | 62 | 15 | Compound B44 | 64 | 23 |
| Compound A55 | 51 | 12 | Compound B45 | 54 | 43 |
| Compound A56 | 38 | 38 | Compound B46 | 76 | 69 |
| Compound A57 | 43 | 34 | Compound B47 | 65 | 59 |
| Compound A58 | 71 | 35 | Compound B48 | 80 | 61 |
| Compound A59 | 50 | 42 | Compound B49 | 68 | 42 |
| Compound A60 | -1 | 11 | Compound B50 | 83 | 62 |
| Compound A61 | -2 | 9 | Compound C1 | 72 | 50 |
| Compound A62 | 89 | 73 | Compound C2 | 81 | 49 |
| Compound A63 | 80 | 67 | Compound C3 | 55 | 25 |
| Compound A64 | 61 | 12 | Compound C4 | 85 | 54 |
| Compound A65 | 50 | 14 | Compound C5 | 77 | 52 |
| Compound A66 | 75 | 22 | Compound C6 | 49 | 38 |
| Compound A67 | 76 | 26 | Compound C7 | 74 | 45 |
| Compound A68 | 77 | 35 | Compound C8 | 78 | 60 |
| Compound A69 | 67 | 19 | Compound C9 | 63 | 48 |
| Compound A70 | 75 | 40 | Compound C10 | 80 | 65 |
| Compound A71 | 86 | 52 | Compound C11 | 68 | 56 |
| Compound A72 | 51 | 39 | Compound C12 | 47 | 27 |
| Compound A73 | 73 | 55 | Compound C13 | 70 | 46 |
| Compound A74 | 87 | 73 | Compound C14 | 66 | 50 |
| Compound A75 | 76 | 53 | Compound C15 | 71 | 41 |
| Compound A76 | 68 | 40 | Compound C17 | 73 | 47 |
| Compound A77 | 78 | 54 | Compound C18 | 50 | 24 |
| Compound A78 | 89 | 79 | Compound C19 | 65 | 32 |
| Compound A79 | 60 | 40 | Compound C22 | 63 | 32 |
| Compound A80 | 55 | 25 | Compound C23 | 76 | 53 |
| Compound A81 | 45 | -33 | Compound C24 | 70 | 66 |
| Compound A82 | 78 | 51 | Compound D1 | 83 | 34 |
| Compound A83 | 62 | 40 | Compound D2 | 68 | 61 |
| Compound A84 | 77 | 56 | Compound D3 | 73 | 34 |
| Compound A85 | 88 | 68 | Compound D4 | 79 | 54 |
| Compound A86 | 74 | 46 | Compound D5 | 80 | 68 |
| Compound A88 | 72 | 65 | Compound D6 | 84 | 59 |
| Compound A89 | 70 | 40 | Compound D7 | 81 | 42 |
| Compound A90 | 79 | 42 | Compound D8 | 75 | 33 |
| Compound A91 | 49 | -1 | Compound D9 | 82 | 52 |
| Compound A92 | 66 | 48 | Compound D10 | 77 | 24 |

(continued)

| Degradation rate (%) of ERα in T-47D cells | | | | | |
|---|---|---|---|---|---|
| Compound | 10nM | 1nM | Compound | 10nM | 1nM |
| Compound A93 | 71 | 26 | Compound D11 | 82 | 52 |
| Compound A94 | 80 | 54 | Compound D12 | 81 | 55 |
| Compound A95 | 68 | 52 | Compound D13 | 82 | 54 |
| Compound A96 | 78 | 31 | Compound E1 | 82 | 57 |
| Compound A97 | 78 | 45 | Compound E2 | 72 | 23 |
| Compound A98 | 83 | 51 | Compound H25 | 48 | 23 |
| Compound A99 | 82 | 51 | Compound H26 | 2 | 1 |
| Compound A100 | 84 | 77 | Compound H27 | 78 | 36 |
| Compound A101 | 45 | 44 | Compound H28 | 83 | 62 |
| Compound A102 | 51 | 16 | Compound H29 | 44 | 8 |
| Compound A103 | 68 | 29 | Compound H30 | 74 | 37 |
| Compound A104 | 72 | 35 | Compound H31 | 85 | 55 |
| Compound A105 | 73 | 38 | Compound H32 | 77 | 52 |
| Compound A106 | 74 | 43 | Compound H33 | 72 | 27 |
| Compound F1 | 71 | 51 | Compound H34 | 77 | 30 |
| Compound F2 | 39 | 24 | Compound H35 | 79 | 62 |
| Compound F4 | 79 | 62 | Compound H36 | 75 | 21 |
| Compound F5 | 63 | 51 | Compound H38 | 75 | 34 |
| Compound F6 | -59 | -7 | Compound H39 | 64 | 15 |
| Compound F7 | 48 | 31 | Compound H40 | 86 | 56 |
| Compound F8 | 68 | 61 | Compound H41 | 83 | 59 |
| Compound F9 | 38 | 42 | Compound H42 | 73 | 37 |
| Compound F10 | 65 | 59 | Compound H44 | 85 | 53 |
| Compound F11 | 57 | 20 | Compound H45 | 78 | 58 |
| Compound G1 | 53 | 22 | Compound H46 | 81 | 50 |
| Compound G3 | 82 | 42 | Compound H47 | 82 | 49 |
| Compound G4 | 65 | 65 | Compound H48 | 78 | 53 |
| Compound G6 | 71 | 33 | Compound H49 | 77 | 48 |
| Compound G7 | 68 | 18 | Compound H50 | 63 | 11 |
| Compound G8 | 81 | 45 | Compound H51 | 79 | 49 |
| Compound G9 | 86 | 44 | Compound H52 | 74 | 49 |
| Compound G10 | 85 | 62 | Compound H53 | 35 | 5 |
| Compound G11 | 80 | 55 | Compound H54 | 60 | 24 |
| Compound G12 | 81 | 47 | Compound H55 | 83 | 56 |
| Compound G13 | 80 | 43 | Compound H56 | 47 | 24 |
| Compound G15 | 72 | 39 | Compound H57 | 71 | 38 |
| Compound G16 | 81 | 68 | Compound H58 | 84 | 53 |
| Compound G17 | 44 | 39 | Compound H59 | 46 | 61 |

(continued)

| Degradation rate (%) of ERα in T-47D cells | | | | | |
|---|---|---|---|---|---|
| Compound | 10nM | 1nM | Compound | 10nM | 1nM |
| Compound G18 | 78 | 49 | Compound H60 | 37 | 41 |
| Compound G20 | 69 | 51 | Compound H61 | 76 | 63 |
| Compound G21 | 63 | 34 | Compound I2 | 55 | 56 |
| Compound G22 | 63 | 34 | Compound I3 | 68 | 41 |
| Compound G23 | 62 | 28 | Compound I4 | 62 | 41 |
| Compound G24 | 81 | 47 | Compound I5 | 68 | 55 |
| Compound G25 | 79 | 49 | Compound I6 | 65 | 56 |
| Compound H1 | -15 | -6 | Compound J1 | -10 | -6 |
| Compound H2 | -23 | -11 | Compound J2 | 67 | 37 |
| Compound H3 | -56 | -43 | Compound J3 | 63 | 42 |
| Compound H4 | -48 | -41 | Compound J4 | 51 | 8 |
| Compound H5 | 12 | 18 | Compound J5 | 81 | 46 |
| Compound H6 | -32 | -35 | Compound J6 | 81 | 39 |
| Compound H7 | -15 | -11 | Compound J7 | 67 | 16 |
| Compound H8 | -37 | -20 | Compound J8 | 81 | 79 |
| Compound H9 | -31 | -3 | Compound J9 | 83 | 78 |
| Compound H10 | 36 | 0 | Compound J10 | 88 | 70 |
| Compound H11 | 8 | -16 | Compound J11 | 82 | 52 |
| CompoundH12 | 11 | -1 | Compound J12 | 86 | 68 |
| Compound H13 | 71 | 48 | Compound J13 | 92 | 60 |
| Compound H14 | 27 | 31 | Compound J14 | 74 | 55 |
| Compound H15 | 56 | 5 | Compound J15 | 80 | 49 |
| Compound H16 | 69 | 48 | Compound J16 | 68 | 35 |
| Compound H17 | 68 | 61 | Compound L5 | 45 | 12 |
| Compound H18 | 57 | 69 | Compound L6 | 68 | 29 |
| Compound H19 | 55 | 39 | Compound L7 | 34 | 11 |
| Compound H20 | -14 | 13 | Compound L8 | 21 | 32 |
| Compound H21 | 65 | 35 | Compound L9 | 49 | 15 |
| Compound H22 | 73 | 56 | Compound L10 | 29 | 14 |
| Compound H23 | 68 | 50 | Compound L11 | 69 | 14 |
| Compound H24 | 21 | 16 | Compound L12 | -21 | -26 |
| Compound K1 | 27 | 7 | Compound L13 | -8 | -7 |
| Compound K2 | 16 | 8 | Compound L14 | -19 | -23 |
| Compound K3 | 52 | 28 | Compound L15 | 71 | 23 |
| Compound K4 | 72 | 42 | Compound L16 | 64 | 29 |
| Compound K5 | 85 | 73 | Compound L17 | 23 | 24 |
| Compound K6 | 58 | 36 | Compound L18 | 8 | 13 |
| Compound K7 | 79 | 63 | Compound L19 | 9 | 12 |

(continued)

| Degradation rate (%) of ERα in T-47D cells | | | | | |
|---|---|---|---|---|---|
| Compound | 10nM | 1nM | Compound | 10nM | 1nM |
| Compound K8 | 85 | 67 | Compound L20 | 41 | 28 |
| Compound K11 | 47 | 44 | Compound L21 | 21 | 26 |
| Compound K12 | 60 | 18 | Compound L22 | 9 | -7 |
| Compound K13 | 68 | 35 | Compound L23 | 37 | 24 |
| Compound K14 | 67 | 20 | Compound L24 | 31 | 21 |
| Compound K15 | 35 | 18 | Compound L25 | 17 | -1 |
| Compound K16 | 72 | 31 | Compound L26 | 30 | -11 |
| Compound K17 | 66 | 23 | Compound L27 | 66 | 12 |
| Compound K18 | 79 | 42 | Compound M6 | 54 | 22 |
| Compound K19 | 56 | 21 | Compound M7 | 56 | 17 |
| Compound K20 | 44 | 4 | Compound M10 | 54 | 22 |
| Compound K21 | 75 | 11 | Compound M11 | 56 | 17 |
| Compound K22 | 19 | -3 | Compound M13 | 27 | 13 |
| Compound K23 | 86 | 61 | Compound M14 | -17 | -29 |
| Compound K24 | 72 | 48 | Compound M15 | 13 | -15 |
| Compound K25 | 48 | -12 | Compound K16 | 11 | 1 |
| Compound K26 | 87 | 56 | Compound M17 | 20 | 10 |
| Compound K27 | 88 | 69 | Compound N1 | 79 | 58 |
| Compound K28 | 86 | 62 | Compound N2 | 42 | 10 |
| Compound K29 | 79 | 46 | Compound N3 | 70 | 30 |
| Compound K30 | 73 | 63 | Compound N4 | 85 | 64 |
| Compound K31 | 79 | 73 | Compound N5 | 82 | 63 |
| Compound K32 | 90 | 67 | Compound N6 | 62 | 35 |
| Compound K33 | 70 | 58 | Compound N7 | 67 | 56 |
| Compound K34 | 86 | 76 | Compound N8 | 55 | 21 |
| Compound K35 | 72 | 60 | Compound N9 | 62 | 29 |
| Compound K36 | 76 | 54 | Compound N10 | 76 | 65 |
| Compound K37 | 56 | 48 | Compound N11 | 57 | 53 |
| Compound K38 | 74 | 61 | Compound N12 | 71 | 50 |
| Compound K39 | 69 | 15 | Compound N13 | 66 | 47 |
| Compound K40 | 77 | 40 | Compound K48 | 80 | 47 |
| Compound K41 | 84 | 54 | Compound K50 | 84 | 52 |
| Compound K42 | 88 | 74 | Compound L1 | -45 | -9 |
| Compound K43 | 84 | 66 | Compound L2 | -23 | -20 |
| Compound K44 | 66 | 36 | Compound L3 | 54 | 25 |
| Compound K45 | 74 | 37 | Compound L4 | 44 | 9 |
| Compound K46 | 90 | 73 | | | |
| Compound K47 | 89 | 74 | | | |

Test Example 7 Inhibitory activity of the compounds disclosed in the present disclosure on the proliferation of VCaP cells

VCaP cells (ATCC, CRL-2876) were cultured in DMEM (Gibco, 11995-065) complete medium containing 10% fetal bovine serum (Hyclone, SH30406.05). On the first day of the experiment, VCaP cells were inoculated in 96-well plates at a density of 15,000 cells/well using DMEM medium containing 10% fetal bovine serum, with 100μL of cell suspension per well, and placed in a cell culture incubator with 5% $CO_2$ at 37°C overnight. On the second day, 100μL of different concentrations of the test compound of the present disclosure and the positive control sample ARV-110 (source: Energy chemical) were added to each well respectively. The compound concentrations were prepared in complete DMEM medium and serially diluted 5-fold starting from 10 μM, resulting in a total of nine concentration points. Wells receiving 100 μL of DMEM medium containing 10% FBS only were used as blank controls. The plates were incubated at 37°C in a 5% $CO_2$ incubator for 168 hours. On the ninth day, 96-well cell culture plates were taken out and 20μL of WST-8 (Beytime, C0040) reagent was added to each well, and the absorbance at 450 nm was detected by using a microplate reader ((TECAN, F50), and the 50% proliferation inhibitory concentration ($IC_{50}$ value) was calculated based on the concentration of the compounds and the absorbance values by logistic regression using Graphpad Prism software.

The $IC_{50}$ values of the compounds disclosed in the present disclosure for inhibiting the proliferation of VCAP cells are shown in Table 6 below.

Table 6 The $IC_{50}$ values of the compounds disclosed in the present disclosure for inhibiting the proliferation of VCAP cells

| Compound | $IC_{50}$ (μM) |
|---|---|
| ARV-110 | 0.19 |
| Compound 2-261 | 0.012 |
| Compound 2-262 | 0.011 |
| Compound 2-263 | 0.011 |
| Compound 2-264 | 0.003 |
| Compound 2-292 | 0.003 |
| Compound 2-103 | 0.036 |
| Compound 2-99 | 0.007 |
| Compound 2-101 | 0.010 |
| Compound 2-293 | 0.005 |
| Compound 2-315 | 0.007 |
| Compound 2-316 | 0.002 |
| Compound 2-318 | 0.001 |
| Conclusion: the compounds disclosed in the present disclosure are capable of inhibiting the proliferation of VCAP cells effectively, and some of the compounds show significantly superior inhibitory ability on the proliferation of VCAP cells compared with the positive control sample. | |

Test Example 8 Inhibitory activity of the compounds disclosed in the present disclosure on LNCAP cell proliferation

LNCAP FGC cells (ATCC, CLR-1740) were cultured in RPMI 1640 (ATCC, 30-2001) complete medium containing 10% fetal bovine serum (Hyclone, SH30406.05). On the first day of the experiment, LNCaP FGC cells were inoculated in 96-well plates at a density of 5,000 cells/well using RPMI 1640 medium containing 10% fetal bovine serum, with 100μL of cell suspension per well, and placed in a cell culture incubator with 5% $CO_2$ at 37°C overnight. On the second day, 100μL of different concentrations of the test compounds of the disclosure and the positive control sample ARV-110 (source: Energy chemical) were added to each well respectively. The compound concentrations were prepared in complete DMEM medium and serially diluted 5-fold starting from 10 μM, resulting in a total of nine concentration points. Wells receiving 100 μL of RPMI 1640 medium containing 10% FBS only were used as controls. The plates were incubated at 37°C in a 5% $CO_2$ incubator for 96 hours. On the sixth day, 96-well cell culture plates were taken out and 20 μL of WST-8 (Beytime, C0040) reagent was added to each well, and the absorbance at 450 nm was detected by using a microplate reader ((TECAN, F50), and the 50% proliferation inhibitory concentration ($IC_{50}$ value) was calculated based on the

concentration of the compounds and the absorbance values by Logistic regression using Graphpad Prism software.

The $IC_{50}$ values of the compounds disclosed in the present disclosure for inhibiting the proliferation of LNCAP cells are shown in Table 7 below.

Table 7 The $IC_{50}$ values of the compounds disclosed in the present disclosure for inhibiting the proliferation of LNCAP cells

| Compound | $IC_{50}$ (μM) |
|---|---|
| ARV-110 | 0.32 |
| Compound 2-261 | 49.060 |
| Compound 2-262 | 21.255 |
| Compound 2-263 | 6.295 |
| Compound 2-264 | 33.650 |
| Compound 2-292 | 68.470 |
| Compound 2-103 | 0.850 |
| Compound 2-99 | 2.710 |
| Compound 2-101 | 5.075 |
| Compound 2-293 | 88.130 |
| Compound 2-313 | 34.310 |
| Compound 2-314 | 42.370 |
| Compound 2-315 | 2.450 |
| Compound 2-316 | 68.230 |
| Compound 2-318 | 9.850 |
| Conclusion: The compounds disclosed in the present disclosure are capable of inhibiting the proliferation of LNCAP cells effectively, and the inhibitory capabilities of some of the compounds to the proliferation of LNCAP cells are comparable to, or even superior than, that of the positive control sample. | |

Test Example 9 Degradation activity of the compounds disclosed in the present disclosure against AR protein in VCAP cells (cell source: ATCC)

AR-positive human prostate cancer cells, VCaP, were inoculated in six-well microplates (Thermo Catalog No. 140675) in DMEM (GiBco Catalog No. 11995-065) containing 10% FBS (Hyclone Catalog No. SH30406.05) at a density of $3 \times 10^5$ cells/well. After overnight incubation in a carbon dioxide incubator (ESCO), different concentrations of the compounds of the present disclosure and the positive control ARV-110 (Energy chemical) were added at a volume of 1 μL/well to reach a final concentration of 5 nM and 0.5 nM of the compounds and the positive control sample, and the corresponding solvent control was set, and the cells were cultured for 8 h. After 8 h, the culture medium was discarded, and cells were washed with PBS, then RIPA lysis buffer containing 1% Protease Inhibitor Cocktail was added, and the total protein extract was obtained after lysis and centrifugation, and the protein concentration in the extract was detected by BCA method. Protein electrophoresis was carried out by SDS-PAGE, after which the protein was transferred to PVDF (Millipore Sigma catalog number PSRP010R5) membrane by 100V constant-voltage electrotransfer for 60 min, and the PVDF membrane was placed in 1×TBST solution with 5% milk and blocked at room temperature for 1 h. The primary antibody solution (Anti-Androgen receptor, Cell Signaling Technology Catalog No. 5153S; 1: 2000 dilution) was prepared and incubated overnight at 4°C. The primary antibody solution was discarded, and the PVDF membrane was washed three times with 1× TBST for 5 min each. The secondary antibody solution (Thermo catalog number SA5-35571; 1:7500 dilution) was prepared, and the secondary antibody dilution was incubated at room temperature for 1h. The secondary antibody solution was discarded, and the PVDF membrane was washed three times with 1×TBST for 5 min each. Western membrane imaging was performed using a dual-color infrared laser imaging system (Licor catalog number Odyssey CLX). Bands were analyzed in grayscale using the software Image Studio. For each sample, GAPDH protein bands were detected as internal reference. The degradation rates of the AR protein for the compounds and positive control sample were calculated based on the grayscale of the protein bands.

The degradation rates of the AR protein in VCAP cells for the compounds disclosed in the present disclosure are

shown in Table 8 below.

Table 8 Degradation rates of the AR protein in VCAP cells induced by the compounds disclosed in the present disclosure

| Compound | 5 nM | 0.5 nM |
|---|---|---|
| ARV-110 | 71% | 10% |
| Compound 2-261 | 82% | 44% |
| Compound 2-262 | 75% | 34% |
| Compound 2-263 | 88% | 65% |
| Compound 2-292 | 72% | 30% |
| Compound 2-103 | 47% | 0% |
| Compound 2-293 | 89% | 72% |
| Compound 2-313 | 89% | 78% |
| Compound 2-314 | 91% | 86% |
| Compound 2-315 | 71% | 31% |
| Compound 2-316 | 61% | 35% |
| Compound 2-318 | 78% | 55% |
| Conclusion: The compounds disclosed in the present disclosure are capable of degrading AR proteins effectively, and the capabilities of some of the compounds to degrade AR proteins are comparable to, or even superior to, that of the positive control sample. | | |

Test Example 10 Degradation activity of the compounds disclosed in the present disclosure against AR protein in LNCAP cells (cell source: ATCC)

AR-positive human prostate cancer cells, LNCaP FGC, were inoculated in six-well microplates (Thermo Catalog No. 140675) in RPMI 1640 (ATCC Catalog No. 30-2001) containing 10% FBS (Hyclone Catalog No. SH30406.05) at a density of 1.5 × $10^5$ cells/well. After the cells were cultured in a carbon dioxide incubator (ESCO) overnight, different concentrations of the compounds of the disclosure and the positive control ARV-110 (Energy chemical) were added at a volume of 1 μL/well to reach a final concentration of 100nM and 10 nM respectively, and the corresponding solvent control was set, and the cells were cultured for 8 h. After 8 h, the culture medium was discarded, and the cells were washed with PBS. After the cells were washed with PBS, RIPA lysis buffer containing 1% Protease Inhibitor Cocktail was added, and the total protein extract was obtained after lysis and centrifugation, and the protein concentration in the extract was detected by BCA method. Protein electrophoresis was carried out by SDS-PAGE, after which the proteins were transferred to PVDF (Millipore Sigma Catalog No. PSRP010R5) membrane by 100V constant voltage electrotransfer for 60 min, and the PVDF membrane was placed in 1×TBST solution with 5% milk and blocked at room temperature for 1h. The primary antibody solution was prepared (Anti-Androgen receptor, Cell Signaling Technology Catalog No. 5153S; 1: 2000 dilution) and incubated overnight at 4°C. The primary antibody solution was discarded, and the PVDF membrane was washed three times with 1× TBST for 5 min each. The secondary antibody solution (Thermo catalog number SA5-35571; 1:7500 dilution) was prepared, and the secondary antibody dilution was incubated at room temperature for 1h. The secondary antibody solution was discarded, and the PVDF membrane was washed three times with 1× TBST for 5 min each. Western membrane imaging was performed using a dual-color infrared laser imaging system (Licor catalog number Odyssey CLX). Bands were analyzed in grayscale using the software Image Studio. For each sample, GAPDH protein bands were detected as internal reference. The degradation rates of the AR protein for the compounds and positive control sample were calculated based on the grayscale of the protein bands.

The degradation rates of the AR protein in LNCAP cells for the compounds disclosed in the present disclosure are shown in Table 9 below.

Table 9 Degradation rates of the AR protein in LNCAP cells induced by the compounds disclosed in the present disclosure

| Compound | 100 nM (%) | 10 nM (%) |
|---|---|---|
| ARV-110 | 62% | 11% |

(continued)

| Compound | 100 nM (%) | 10 nM (%) |
|---|---|---|
| Compound 2-261 | 70% | 53% |
| Compound 2-262 | 68% | 47% |
| Compound 2-263 | 69% | 38% |
| Compound 2-264 | 71% | 52% |
| Compound 2-292 | 78% | 43% |
| Compound 2-103 | 79% | 55% |
| Compound 2-99 | 69% | 40% |
| Compound 2-101 | 67% | 32% |
| Compound 2-293 | 82% | 62% |
| Compound 2-313 | 79% | 75% |
| Compound 2-314 | 76% | 69% |
| Compound 2-315 | 44% | 33% |
| Compound 2-316 | 61% | 49% |
| Compound 2-318 | 73% | 58% |
| Compound 2-376 | 70% | 20% |
| Compound 2-384 | 63% | 48% |
| Conclusion: The compounds disclosed in the present disclosure are capable of degrading AR proteins effectively, and the capabilities of some of the compounds to degrade AR proteins are comparable to, or even superior to, that of the positive control sample. | | |

The present disclosure has been illustrated by the above-described embodiments, but it should be understood that the above-described embodiments are only used for the purpose of example and illustration and are not intended to limit the present disclosure to the scope of the described embodiments. Furthermore, it is understood by those skilled in the art that the present disclosure is not limited to the above-described embodiments, and that a greater variety of variations and modifications may be made in accordance with the teachings of the present disclosure, all of which fall within the scope of the claimed protection of the present disclosure. The scope of protection of the present disclosure is defined by the appended claims and their equivalents.

## Claims

**1.** A compound, wherein the compound is a compound having the following chemical structure:

CLM-L-PTM,

or an isomer, an isotopic derivative, a polymorph, a prodrug, or a pharmaceutically acceptable salt or a solvate thereof,
wherein:

PTM is a binding moiety that targets the estrogen receptor protein, and has the following structure:

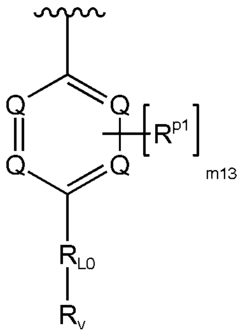

;

$R_{L0}$ is selected from a single bond, -O-, alkylene, and -C(=O)-; preferably, $R_{L0}$ is selected from a single bond, -O-, $C_{1-3}$ alkylene, and -C(=O)-;

$R_v$ is selected from

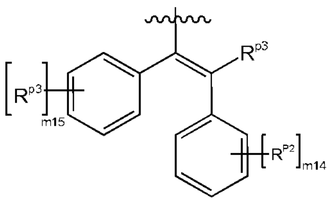, 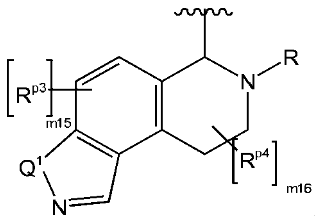, 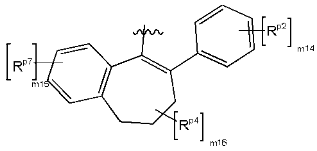,

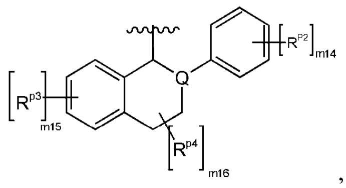, 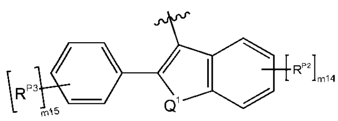, 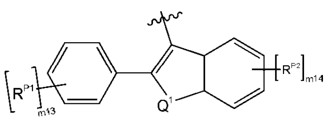,

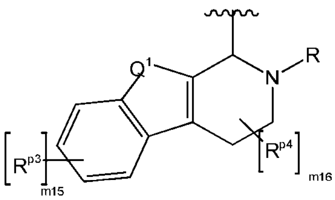, 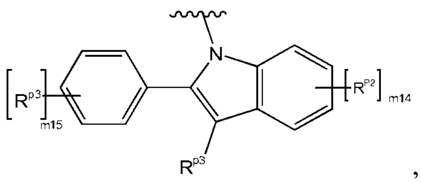, 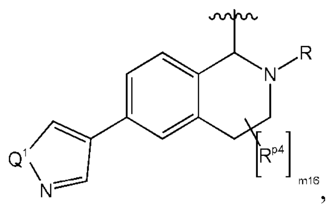,

and

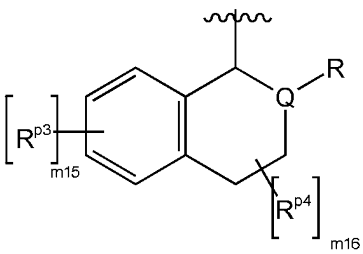, 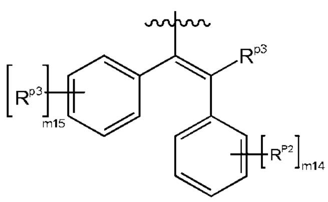, 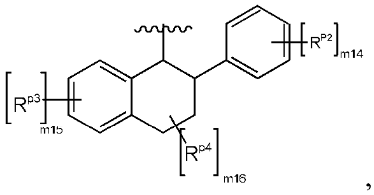,

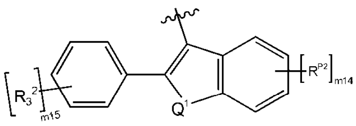, 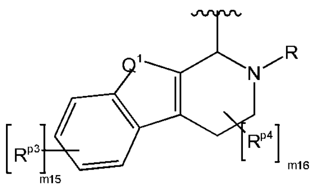, 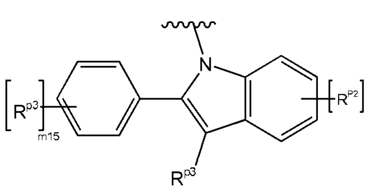,

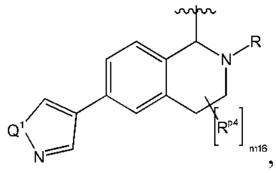, and 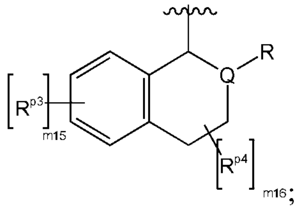;

R is

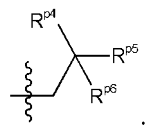;

each occurrence of Q is each independently selected from $CR^x$ and N;
each occurrence of $Q^1$ is O, S, or $NR^x$;
each occurrence of $R^{P1}$, $R^{P2}$, $R^{P3}$, $R^{P4}$, $R^{P5}$, $R^{P6}$, $R^x$, and $R^{P7}$ are each independently selected from H, carboxyl, deuterium atom, halogen, alkyl, deuterated alkyl, heteroalkyl, alkenyl, alkynyl, alkoxy, haloalkyl, haloalkoxy,hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkoxy, haloalkyl,hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl;
preferably, each occurrence of $R^{P1}$, $R^{P2}$, $R^{P3}$, $R^{P4}$, $R^{P5}$, $R^{P6}$, $R^x$ , and $R^{P7}$ are each independently selected from H, carboxyl, deuterium atom, halogen, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy,hydroxyl, $C_1$-$C_6$ hydroxyalkyl, nitro, cyano, amino, $C_1$-$C_6$ alkylamino, $C_1$-$C_6$ alkylcarbonyl, $C_1$-$C_6$ alkoxycarbonyl, $C_1$-$C_6$ alkylaminocarbonyl, $C_3$-$C_8$ cycloalkyl, 4-10-membered heterocyclyl, $C_5$-$C_{10}$ heterocyclyl, $C_6$-$C_{10}$ aryl, $C_5$-$C_{10}$ heteroaryl, and 5-10-membered heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, 4-10-membered heterocyclyl, $C_5$-$C_{10}$ heterocyclyl, $C_6$-$C_{10}$ aryl, $C_5$-$C_{10}$ heteroaryl, and 5-10-membered heteroaryl are each independently optionally substituted with one or more substituents selected from halogen, $C_1$-$C_6$ alkyl, $C_1$-$C_6$ heteroalkyl, $C_1$-$C_6$ alkoxy, $C_1$-$C_6$ haloalkyl,hydroxyl, $C_1$-$C_6$ hydroxyalkyl, cyano, amino, nitro, $C_3$-$C_8$ cycloalkyl, $C_5$-$C_{10}$ heterocyclyl, 4-10-membered heterocyclyl, $C_2$-$C_6$ alkenyl, $C_2$-$C_6$ alkynyl, $C_1$-$C_6$ alkylamino, $C_1$-$C_6$ alkylcarbonyl, $C_1$-$C_6$ alkoxycarbonyl, $C_1$-$C_6$ alkylaminocarbonyl, $C_6$-$C_{10}$ aryl, $C_5$-$C_{10}$ heteroaryl, and 5-10-membered heteroaryl; preferably, each occurrence of $R^{P1}$, $R^{P2}$, $R^{P3}$, $R^{P4}$, $R^{P5}$, $R^{P6}$, $R^x$ , and $R^{P7}$ are each independently selected from H, carboxyl, deuterium atom, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy,hydroxyl, nitro, cyano, amino, $C_3$-$C_8$ cycloalkyl, 4-10-membered heterocyclyl, $C_5$-$C_{10}$ heterocyclyl, $C_6$-$C_{10}$ aryl, $C_5$-$C_{10}$ heteroaryl, and 5-10-membered heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, 4-10-membered heterocyclyl, $C_5$-$C_{10}$ heterocyclyl, $C_6$-$C_{10}$ aryl, $C_5$-$C_{10}$ heteroaryl, and 5-10-membered heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_5$-$C_{10}$ heterocyclyl, 4-10-membered heterocyclyl, $C_6$-$C_{10}$ aryl, $C_5$-$C_{10}$ heteroaryl, and 5-10-membered heteroaryl; preferably, each occurrence of $R^{P1}$, $R^{P2}$, $R^{P3}$, $R^{P4}$, $R^{P5}$, $R^{P6}$, $R^x$, and $R^{P7}$ are each independently selected from H, carboxyl, hydroxyl, deuterium atom, F, Cl, Br, I, $C_1$-$C_3$ alkyl, and $C_1$-$C_3$ alkoxy;
m13 is 0, 1, 2, 3, 4 or 5;
m14 is 0, 1, 2, 3, 4 or 5;
m15 is 0, 1, 2, 3, 4 or 5;
m16 is 0, 1, 2, 3, 4 or 5;
preferably, the PTM is:

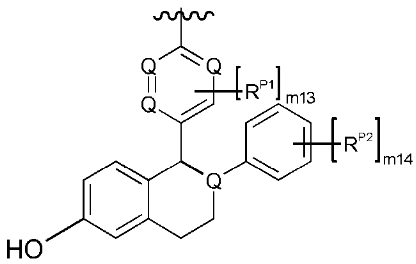, 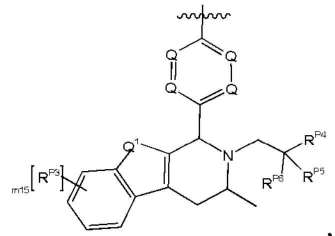, 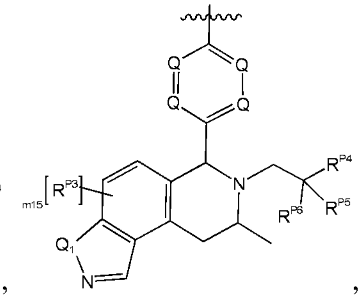,

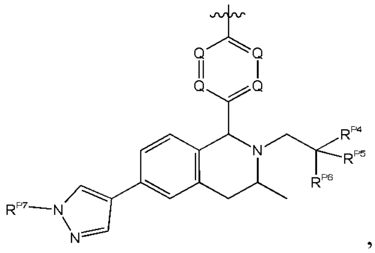, 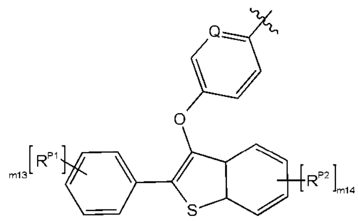, 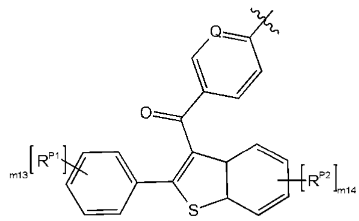,

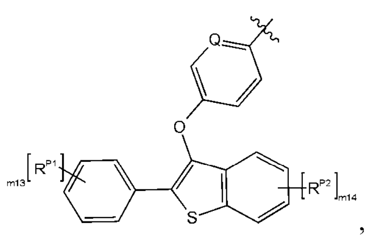, 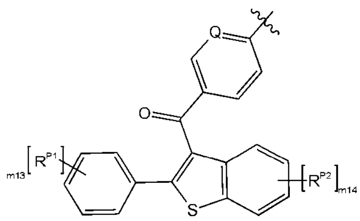, 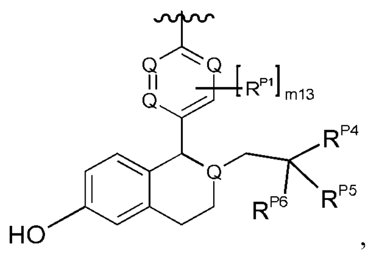,

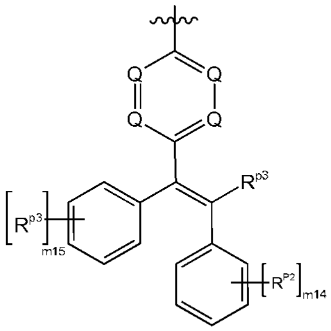 or 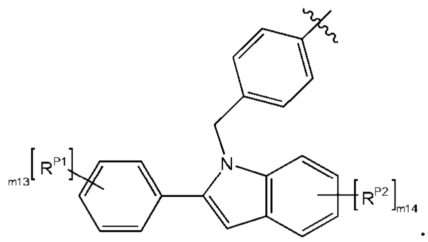;

L is a bond or a chemical linker moiety that covalently connects CLM and the PTM, and the CLM is a cereblon E3 ubiquitin ligase binding moiety selected from the following structures:

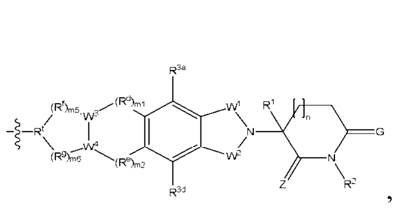, 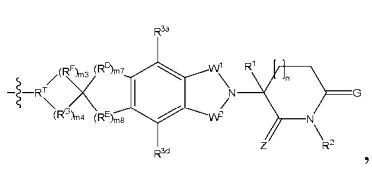, 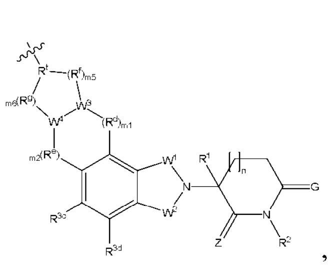,

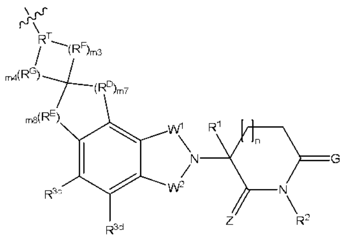, 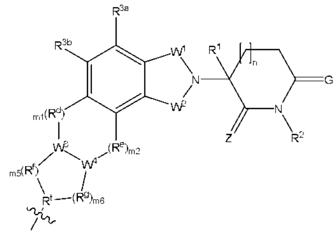, and 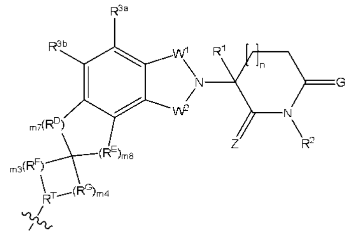;

wherein:

$W^1$ and $W^2$ are identical or different and are each independently $CR^aR^b$ or C(=O), and at least one of $W^1$ and $W^2$ is C(=O);

G and Z are identical or different and are each independently selected from O, S, and Se;

$R^{3a}$, $R^{3b}$, $R^{3c}$, and $R^{3d}$ are each independently selected from H, deuterium atom, halogen, alkyl, deuterated alkyl, heteroalkyl, alkenyl, alkynyl, alkoxy, haloalkyl, haloalkoxy,hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkoxy, haloalkyl,hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl;

each occurrence of $R^d$, $R^e$, $R^f$, $R^g$, $R^D$, $R^E$, $R^F$, and $R^G$ are each independently $C(R^m)_2$, $NR^m$, C(=O), O, or S;

each occurrence of $W^3$ and $W^4$ are each independently $CR^m$ or N;

each occurrence of $R^t$ and $R^T$ are each independently N or $CR^{2h}$, and when $R^D$, $R^E$, $R^F$, and $R^G$ are all $C(R^m)_2$, $R^T$ is $CR^{2h}$;

each occurrence of m1 and m2 are each independently an integer of 0, 1,2, 3, 4, 5 or 6, and $m1 + m2 \leq 6$;

each occurrence of m3 is each independently an integer of 0, 1,2, 3, 4, 5, 6 or 7, and m4 is an integer of 1, 2, 3, 4, 5, 6, 7 or 8; and $m3 + m4 \leq 8$;

each occurrence of m5 and m6 are each independently an integer of 0, 1, 2, 3, 4, 5, 6 or 7, and $m5 + m6 \leq 7$;

each occurrence of m7 and m8 are each independently an integer of 0, 1, 2, 3, 4, 5, 6 or 7, and $m7 + m8 \leq 7$;

each occurrence of $R^m$ is each independently selected from H, deuterium atom, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl;

$R^{2h}$ is selected from H, deuterium atom, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylcarbonyl, alkoxycarbonyl, alkylaminocarbonyl, cycloalkyl, heterocyclyl, aryl, heteroaryl, alkenyl, and alkynyl, wherein the alkyl, heteroalkyl, alkoxy, cycloalkyl, heterocyclyl, aryl, and heteroaryl are each independently optionally substituted with one or more substituents selected from halogen, alkyl, heteroalkyl, alkenyl, alkynyl, alkoxy, haloalkyl, hydroxyl, hydroxyalkyl, cyano, amino, nitro, cycloalkyl, heterocyclyl, alkylamino, alkylcarbonyl, alkoxycarbonyl, alkylaminocarbonyl, aryl, and heteroaryl;

$R^1$ is selected from H, halogen, deuterium atom, $C_1$-$C_6$ alkyl, $C_1$-$C_6$ alkoxy, hydroxyl, cycloalkyl, $C_1$-$C_6$ haloalkyl, and hydroxyalkyl;

$R^2$, $R^a$, and $R^b$ are each independently selected from H, $C_1$-$C_3$ alkyl, $C_3$-$C_6$ cycloalkyl, and $C_1$-$C_6$ alkoxy; and

n is 0, 1, 2 or 3;

preferably, the compound is not:

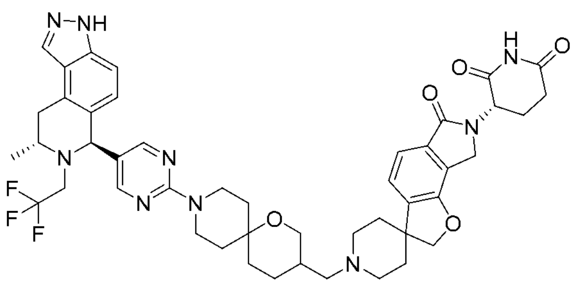,

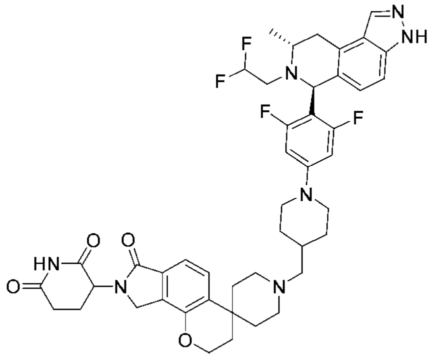,

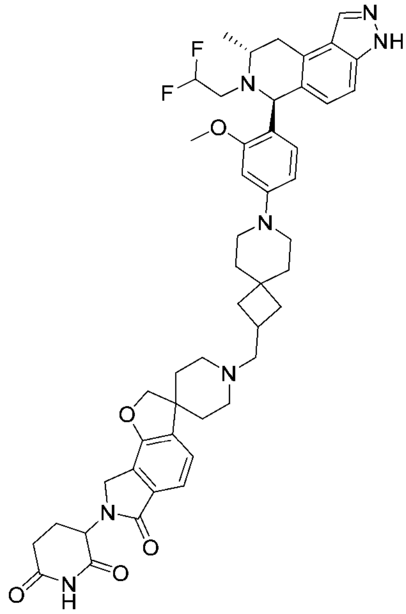,

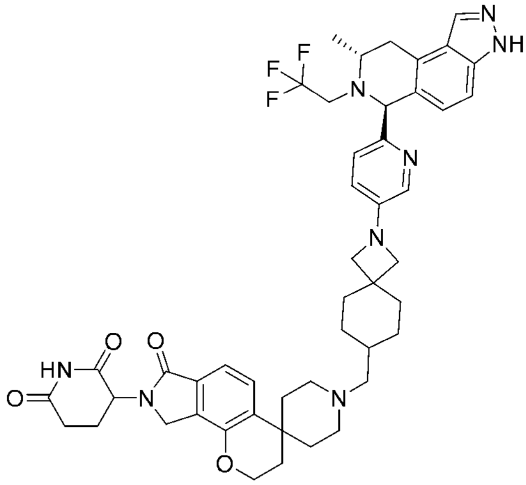,

or

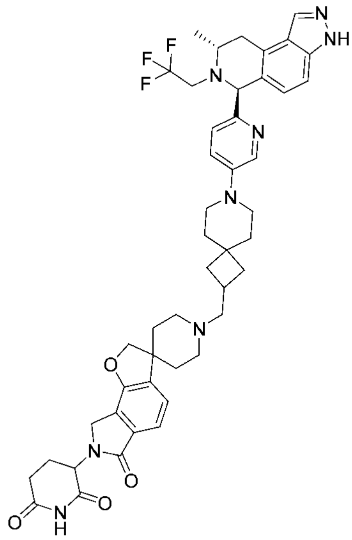.

**2.** A compound, wherein the compound is a compound having the following chemical structure:

CLM-L-PTM,

or an isomer, an isotopic derivative, a polymorph, a prodrug, or a pharmaceutically acceptable salt or a solvate thereof,
wherein:

PTM is a binding moiety that targets the estrogen receptor protein, and has the following structure:

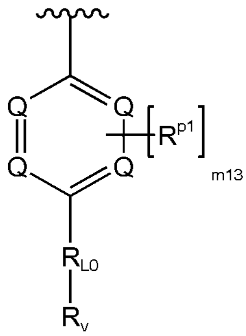
;

$R_{L0}$ is selected from a single bond, -O-, alkylene, and -C(=O)-; preferably, $R_{L0}$ is selected from a single bond, -O-, $C_{1-3}$ alkylene, and -C(=O)-;
$R_v$ is selected from

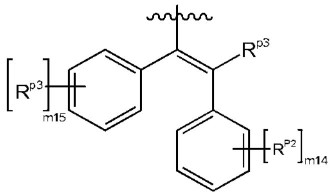
,
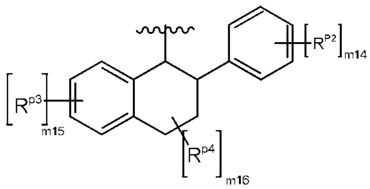
,
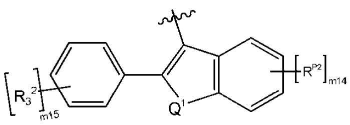
,
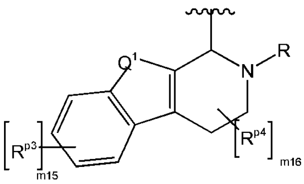
,
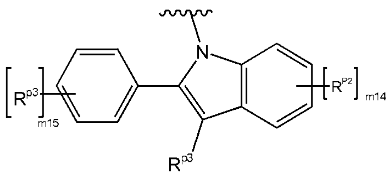
,
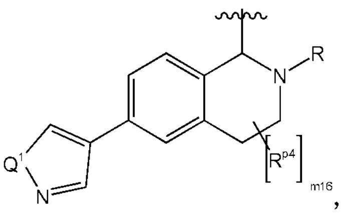
, and
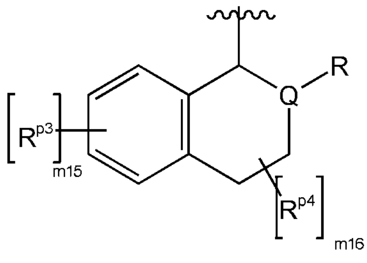
;

R is

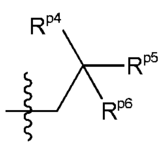
;

each occurrence of Q is each independently selected from $CR^x$ and N;
$Q^1$ is O, S or $NR^x$;
each occurrence of $R^{P1}$, $R^{P2}$, $R^{P3}$, $R^{P4}$, $R^{P5}$, $R^{P6}$ and $R^x$ are each independently selected from H, deuterium atom, halogen, alkyl, deuterated alkyl, heteroalkyl, alkenyl, alkynyl, alkoxy, haloalkyl, haloalkoxy,hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl, wherein the alkyl, heteroalkyl, alkenyl, alkynyl, alkoxy, cycloalkyl, heterocyclyl, aryl, and heteroaryl are each optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl;
m13 is 0, 1, 2, 3, 4 or 5;
m14 is 0, 1, 2, 3, 4 or 5;
m15 is 0, 1, 2, 3, 4 or 5;
m16 is 0, 1, 2, 3, 4 or 5;
preferably, the PTM is:

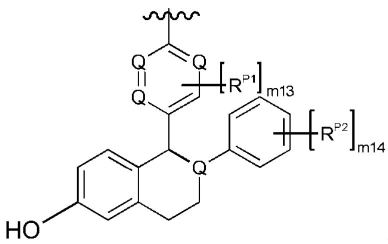
,
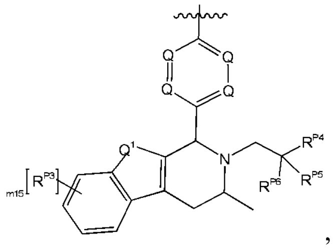
,
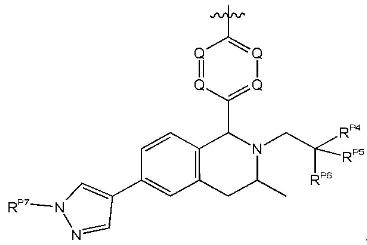
,
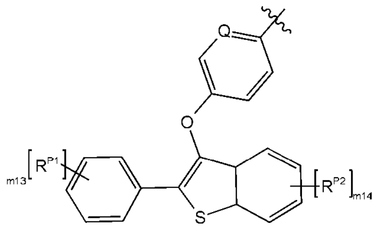
,
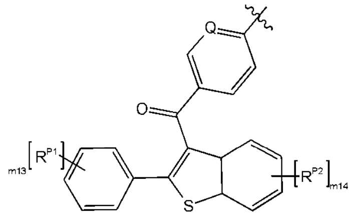
,
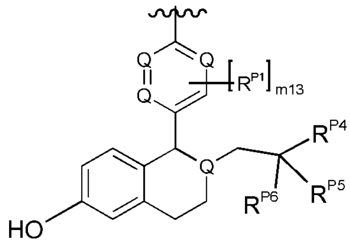
,
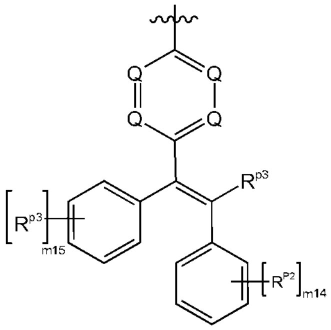
or
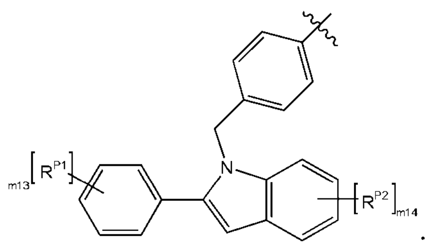
;

L is a bond or a chemical linker moiety that covalently connects CLM and the PTM, and
the CLM is a cereblon E3 ubiquitin ligase binding moiety selected from the following structures:

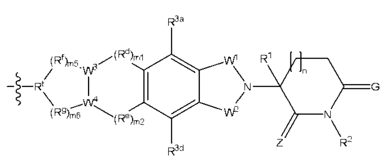
,
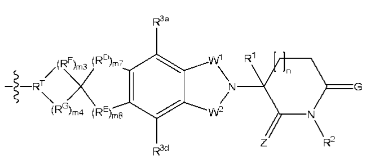
,
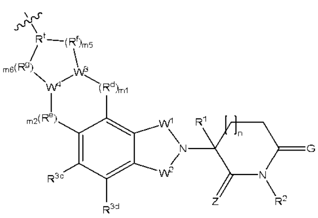
,

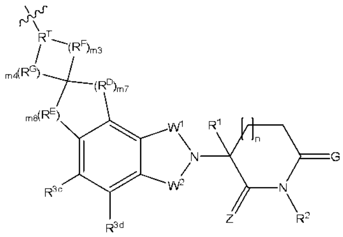 , 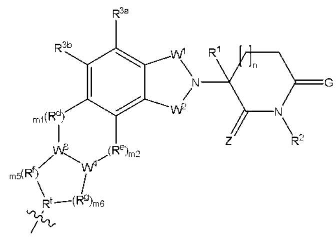 , and 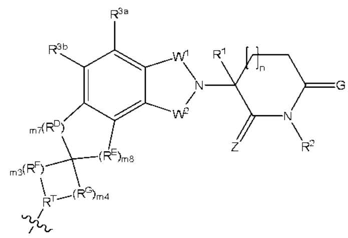 ;

wherein:

$W^1$ and $W^2$ are identical or different and are each independently $CR^aR^b$ or C(=O), and at least one of $W^1$ and $W^2$ is C(=O);

G and Z are identical or different and are each independently selected from O, S, and Se;

$R^{3a}$, $R^{3b}$, $R^{3c}$, and $R^{3d}$ are each independently selected from H, deuterium atom, halogen, alkyl, deuterated alkyl, heteroalkyl, alkenyl, alkynyl, alkoxy, haloalkyl, haloalkoxy,hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkoxy, haloalkyl,hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl;

each occurrence of $R^d$, $R^e$, $R^f$, $R^g$, $R^D$, $R^E$, $R^F$, and $R^G$ are each independently $C(R^m)_2$, $NR^m$, C(=O), O, or S;

each occurrence of $W^3$ and $W^4$ are each independently $CR^m$ or N;

each occurrence of $R^t$ and $R^T$ are each independently N or $CR^{2h}$, and when $R^D$, $R^E$, $R^F$, and $R^G$ are all $C(R^m)_2$, $R^T$ is $CR^{2h}$;

each occurrence of m1 and m2 are each independently an integer of 0, 1, 2, 3, 4, 5, or 6, and $m1 + m2 \leq 6$;

each occurrence of m3 is independently an integer of 0, 1, 2, 3, 4, 5, 6, or 7; m4 is an integer of 1, 2, 3, 4, 5, 6, 7, or 8, and $m3 + m4 \leq 8$;

each occurrence of m5 and m6 are each independently an integer of 0, 1, 2, 3, 4, 5, 6, or 7, and $m5 + m6 \leq 7$;

each occurrence of m7 and m8 are each independently an integer of 0, 1, 2, 3, 4, 5, 6, or 7, and $m7 + m8 \leq 7$;

each occurrence of $R^m$ is independently selected from H, deuterium atom, halogen, alkyl, deuterated alkyl, heteroalkyl, alkenyl, alkynyl, alkoxy, haloalkyl, haloalkoxy,hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkoxy, haloalkyl,hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl;

$R^{2h}$ is selected from H, deuterium atom, halogen, alkyl, deuterated alkyl, heteroalkyl, alkenyl, alkynyl, alkoxy, haloalkyl, haloalkoxy,hydroxyl, hydroxyalkyl, nitro, cyano, amino, alkylamino, alkylcarbonyl, alkoxycarbonyl, alkylaminocarbonyl, cycloalkyl, heterocyclyl, aryl, heteroaryl, alkenyl and alkynyl, wherein the alkyl, heteroalkyl, alkoxy, cycloalkyl, heterocyclyl, aryl, and heteroaryl are each independently optionally substituted with one or more substituents selected from halogen, alkyl, heteroalkyl, alkenyl, alkynyl, alkoxy, haloalkyl,hydroxyl, hydroxyalkyl, cyano, amino, nitro, cycloalkyl, heterocyclyl, alkylamino, alkylcarbonyl, alkoxycarbonyl, alkylaminocarbonyl, aryl, and heteroaryl;

$R^1$ is selected from H, halogen, deuterium atom, $C_1$-$C_6$ alkyl, $C_1$-$C_6$ alkoxy, hydroxyl, cycloalkyl, $C_1$-$C_6$ haloalkyl, and hydroxyalkyl;

$R^2$, $R^a$, and $R^b$ are each independently selected from H, $C_1$-$C_3$ alkyl, $C_3$-$C_6$ cycloalkyl, and $C_1$-$C_6$ alkoxy; and

n is 0, 1, 2, or 3.

**3.** A compound, wherein the compound is a compound having the following chemical structure:

CLM-L-PTM,

or an isomer, an isotopic derivative, a polymorph, a prodrug, or a pharmaceutically acceptable salt or a solvate thereof,
wherein:

PTM is a binding moiety that targets the estrogen receptor protein, and is selected from the following structures:

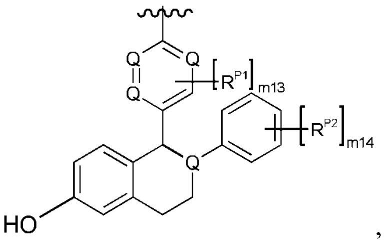
,
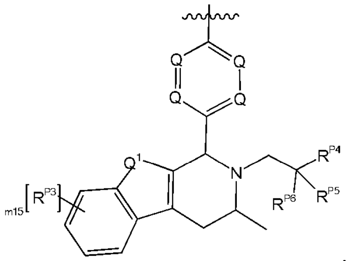
,
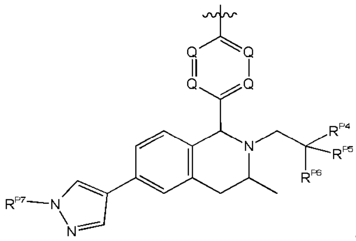
,
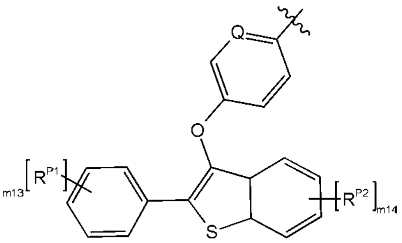
and
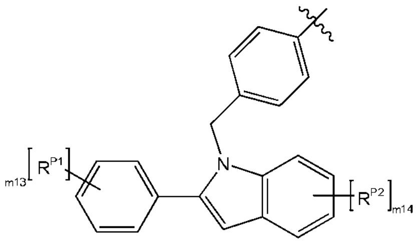
;

L is a bond or a chemical linker moiety that covalently connects CLM and the PTM; and
the CLM is a cereblon E3 ubiquitin ligase binding moiety selected from the following structures:

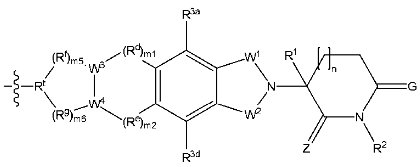
,
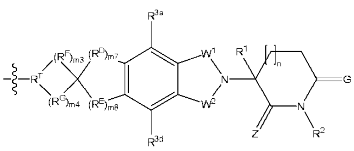
,
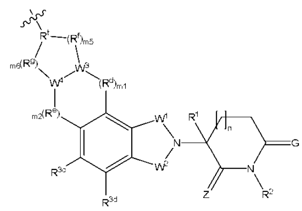
,
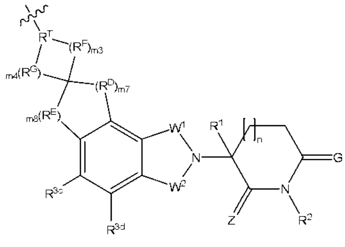
,
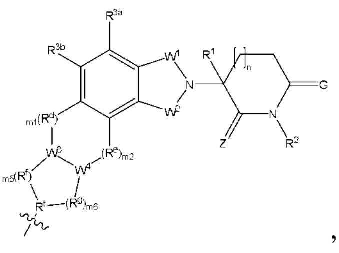
, and
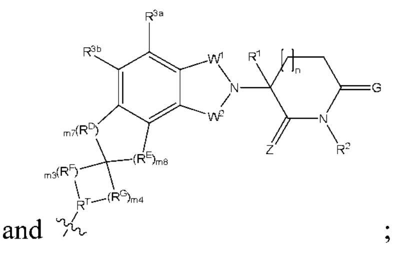
;

wherein:

each occurrence of Q is each independently selected from $CR^x$ and N;
$Q^1$ is O, S, or NH;
each occurrence of $R^{P1}$, $R^{P2}$, $R^{P3}$, $R^{P4}$, $R^{P5}$, $R^{P6}$, and $R^x$ are each independently selected from H, deuterium atom, halogen, alkyl, deuterated alkyl, heteroalkyl, alkenyl, alkynyl, alkoxy, haloalkyl, haloalkoxy,hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkoxy, haloalkyl,hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl;

m13 is 0, 1, 2, 3, 4, or 5;

m14 is 0, 1, 2, 3, 4, or 5;

m15 is 0, 1, 2, 3, 4, or 5;

$W^1$ and $W^2$ are identical or different and are each independently $CR^aR^b$ or C(=O), and at least one of $W^1$ and $W^2$ is C(=O);

G and Z are identical or different and are each independently selected from O, S, and Se;

$R^{3a}$, $R^{3b}$, $R^{3c}$, and $R^{3d}$ are each independently selected from H, deuterium atom, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl;

each occurrence of $R^d$, $R^e$, $R^f$, $R^g$, $R^D$, $R^E$, $R^F$, and $R^G$ are each independently $C(R^m)_2$, $NR^m$, C(=O), O, or S;

each occurrence of $W^3$ and $W^4$ are each independently $CR^m$ or N;

each occurrence of $R^t$ and $R^T$ are each independently N or $CR^{2h}$, and when $R^D$, $R^E$, $R^F$, and $R^G$ are all $C(R^m)_2$, $R^T$ is $CR^{2h}$;

each occurrence of m1 and m2 are each independently an integer of 0, 1, 2, 3, 4, 5 or 6, and $m1 + m2 \leq 6$;

each occurrence of m3 is each independently an integer of 0, 1, 2, 3, 4, 5, 6 or 7, and m4 is an integer of 1, 2, 3, 4, 5, 6, 7 or 8; and $m3 + m4 \leq 8$;

each occurrence of m5 and m6 are each independently an integer of 0, 1, 2, 3, 4, 5, 6 or 7, and $m5 + m6 \leq 7$;

each occurrence of m7 and m8 are each independently an integer of 0, 1, 2, 3, 4, 5, 6 or 7, and $m7 + m8 \leq 7$;

each occurrence of $R^m$ is independently selected from H, deuterium atom, halogen, alkyl, deuterated alkyl, heteroalkyl, alkenyl, alkynyl, alkoxy, haloalkyl, haloalkoxy,hydroxyl, hydroxyalkyl, nitro, cyano, amino, alkylamino, alkylcarbonyl, alkoxycarbonyl, alkylaminocarbonyl, cycloalkyl, heterocyclyl, aryl, and heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylcarbonyl, alkoxycarbonyl, alkylaminocarbonyl, aryl, and heteroaryl;

$R^{2h}$ is selected from H, deuterium atom, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylcarbonyl, alkoxycarbonyl, alkylaminocarbonyl, cycloalkyl, heterocyclyl, aryl, , heteroaryl, alkenyl, and alkynyl,wherein the alkyl, heteroalkyl, alkoxy, cycloalkyl, heterocyclyl, aryl, and heteroaryl are each independently optionally substituted with one or more substituents selected from halogen, alkyl, heteroalkyl, alkenyl, alkynyl, alkoxy, haloalkyl, hydroxyl, hydroxyalkyl, cyano, amino, nitro, cycloalkyl, heterocyclyl, alkylamino, alkylcarbonyl, alkoxycarbonyl, alkylaminocarbonyl, aryl, and heteroaryl;

$R^1$ is selected from H, halogen, deuterium atom, $C_1$-$C_6$ alkyl, $C_1$-$C_6$ alkoxy, hydroxyl, cycloalkyl, $C_1$-$C_6$ haloalkyl, and hydroxyalkyl;

$R^2$, $R^a$, and $R^b$ are independently selected from H, $C_1$-$C_3$ alkyl, $C_3$-$C_6$ cycloalkyl, and $C_1$-$C_6$ alkoxy; and n is 0, 1, 2, or 3.

**4.** The compound according to any one of claims 1 to 3, wherein:

$W^1$ and $W^2$ are identical or different and are each independently $CH_2$ or C(=O), and at least one of $W^1$ and $W^2$ is C(=O); and/or

$R^{P1}$, $R^{P2}$, $R^{P3}$, $R^{P4}$, $R^{P5}$, $R^{P6}$, $R^x$, $R^{3a}$, $R^{3b}$, $R^{3c}$, and $R^{3d}$ are each independently selected from H, deuterium atom, halogen, $C_1$-$C_6$ alkyl, heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted with one or

more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; preferably, $R^{P1}$, $R^{P2}$, $R^{P3}$, $R^{P4}$, $R^{P5}$, $R^{P6}$, $R^x$, $R^{3a}$, $R^{3b}$, $R^{3c}$, and $R^{3d}$ are each independently selected from H, deuterium atom, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; preferably, $R^{P1}$, $R^{P2}$, $R^{P3}$, $R^{P4}$, $R^{P5}$, $R^{P6}$, $R^x$, $R^{3a}$, $R^{3b}$, $R^{3c}$, and $R^{3d}$ are each independently selected from H, hydroxyl, deuterium atom, halogen, $C_1$-$C_3$ alkyl, and $C_1$-$C_3$ alkoxy; preferably, $R^{P1}$, $R^{P2}$, $R^{P3}$, $R^{P4}$, $R^{P5}$, $R^{P6}$, $R^x$, $R^{3a}$, $R^{3b}$, $R^{3c}$, and $R^{3d}$ are each independently selected from H, deuterium atom, halogen, $C_1$-$C_3$ alkyl, and $C_1$-$C_3$ alkoxy; preferably, $R^{P1}$, $R^{P2}$, $R^{P3}$, $R^{P4}$, $R^{P5}$, $R^{P6}$, $R^x$, $R^{3a}$, $R^{3b}$, $R^{3c}$, and $R^{3d}$ are each independently selected from H, deuterium atom, F, Cl, Br, I, $C_1$-$C_3$ alkyl, and $C_1$-$C_3$ alkoxy; and/or

each occurrence of $R^d$, $R^e$, $R^f$ and $R^g$ are each independently $C(R^m)_2$ or O; and/or

each occurrence of $R^D$, $R^E$, $R^F$, and $R^G$ are each independently $C(R^m)_2$ or O; and/or

$W^3$ and $W^4$ are CH; and/or

$R^{2h}$ is selected from H, deuterium atom, halogen, $C_1$-$C_6$ alkyl, $C_1$-$C_6$ deuterated alkyl, $C_1$-$C_6$ heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted with one or more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; preferably, $R^{2h}$ is selected from H, deuterium atom, F, Cl, Br, I, $C_1$-$C_6$ alkyl, $C_1$-$C_6$ deuterated alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; preferably, $R^{2h}$ is selected from H, deuterium atom, halogen, $C_1$-$C_3$ alkyl, and $C_1$-$C_3$ alkoxy; preferably, $R^{2h}$ is selected from H, deuterium atom, F, Cl, Br, I, $C_1$-$C_3$ alkyl, $C_1$-$C_3$ haloalkyl, $C_1$-$C_3$ alkoxy, and $C_1$-$C_3$ haloalkoxy; and/or

each occurrence of m1 and m2 are each independently an integer of 0, 1, 2 or 3, and $m1 + m2 \leq 3$; preferably $m1 + m2 = 1$ or $m1 + m2 = 2$; and/or

each occurrence of m3 is each independently an integer of 0, 1, 2, 3 or 4, and m4 is an integer of 1,2, 3, 4, or 5; and $m3 + m4 \leq 5$; preferably $m3 + m4 = 2$, $m3 + m4 = 3$, or $m3 + m4 = 4$; and/or

each occurrence of m5 and m6 are each independently an integer of 0, 1, 2, 3, or 4, and $m5 + m6 \leq 4$, preferably $m5 + m6 = 2$ or $m5 + m6 = 3$; and/or

each occurrence of m7 and m8 are each independently an integer of 0, 1, 2, 3 or 4, and $m7 + m8 \leq 4$; preferably $m7 + m8 = 2$ or $m7 + m8 = 3$; and/or

each occurrence of $R^m$ is independently selected from H, deuterium atom, halogen, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, aryl, and heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted with one or more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; preferably, each occurrence of $R^m$ is independently selected from H, deuterium atom, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; preferably, each occurrence of $R^m$ is independently selected from H, deuterium atom, halogen, $C_1$-$C_3$ alkyl, and $C_1$-$C_3$ alkoxy; and/or

$R^1$ is selected from H, halogen, $C_1$-$C_3$ alkyl, and hydroxyl; preferably, $R^1$ is selected from H, F, Cl, Br, I, $C_1$-$C_3$ alkyl, and hydroxyl; and/or

$R^2$ is selected from H and $C_1$-$C_3$ alkyl; and/or
n is 0 or 1.

**5.** The compound according to any one of claims 1 to 4, wherein the CLM is selected from the following structures:

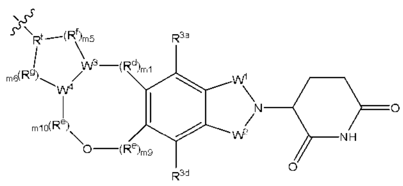,
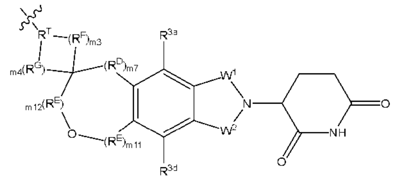,
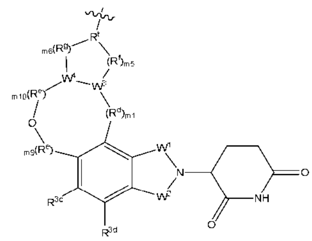,
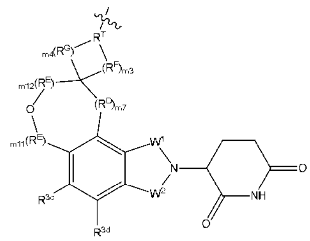,
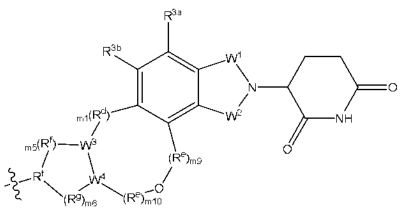, and
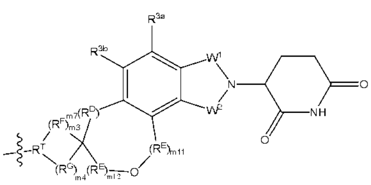;

$W^1$, $W^2$, $W^3$, $W^4$, $R^{3a}$, $R^{3b}$ $R^{3c}$, $R^{3d}$, $R^d$, $R^e$, $R^f$, $R^t$, $R^g$, $R^D$, $R^E$, $R^F$, $R^T$, $R^G$, m3, m4, m5, and m6 are as defined in claim 1, 2, 3, or 4;
preferably,
$R^{3a}$, $R^{3b}$, $R^{3c}$, and $R^{3d}$ are each independently selected from H, deuterium atom, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl;
each occurrence of m1, m9, and m10 are each independently an integer of 0, 1, 2, 3, 4, or 5, and $m1 + m9 + m10 \leq 5$; preferably, each occurrence of m1, m9, and m10 are each independently an integer of 0, 1, or 2, and $m1 + m9 + m10 \leq 2$; preferably, $m1 + m9 + m10 = 1$ or $m1 + m9 + m10 = 0$; and
each occurrence of m7, m11, and m12 are each independently an integer of 0, 1, 2, 3, 4, 5, or 6, and $m7 + m11 + m12 \leq 6$; preferably, each occurrence of m7, m11, and m12 are each independently an integer of 0, 1, 2, or 3, and $m7 + m11 + m12 \leq 3$; preferably, $m7 + m11 + m12 = 2$ or $m7 + m11 + m12 = 1$.

**6.** The compound according to any one of claims 1 to 5, wherein the CLM is selected from the following structures:

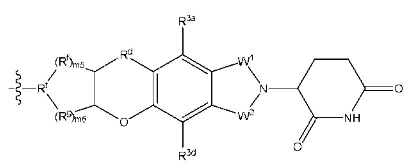,
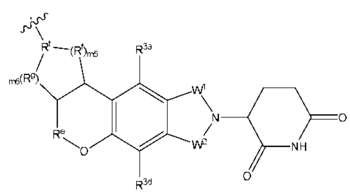,
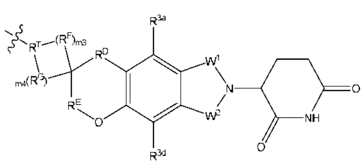,
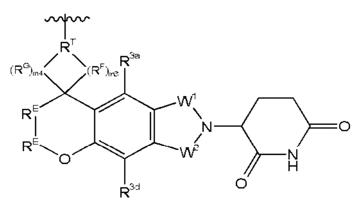,
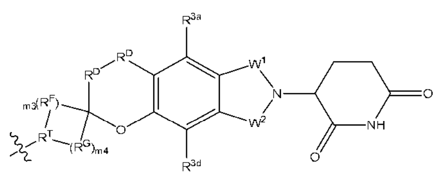,
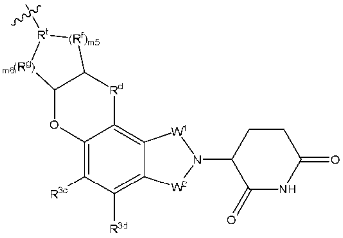,

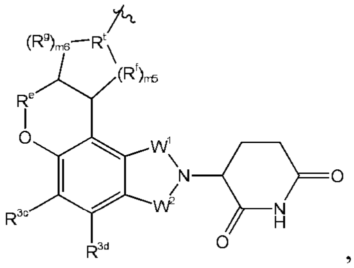, 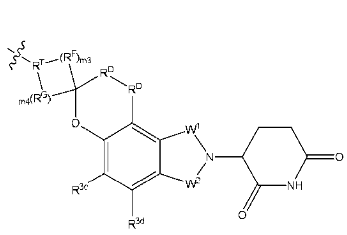, 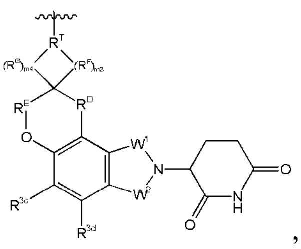,

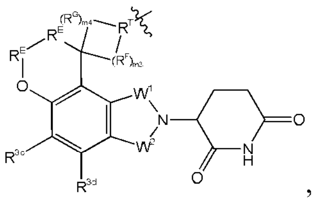, 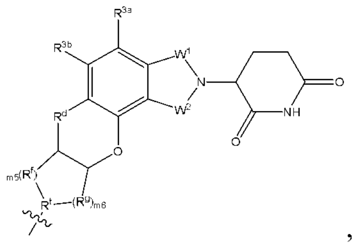, 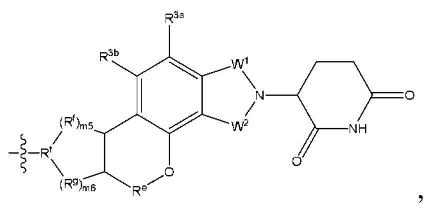,

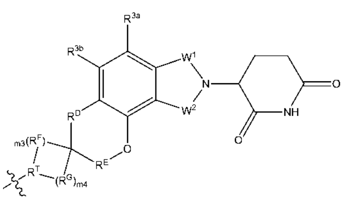, 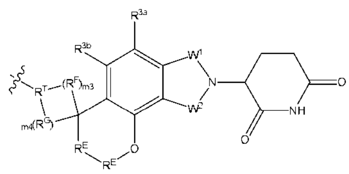, 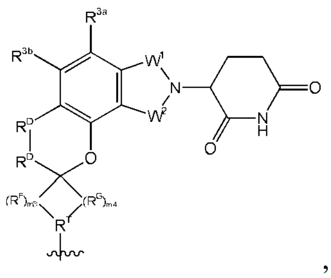,

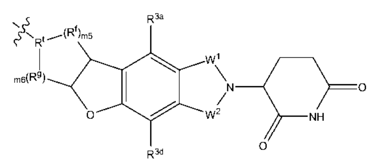, 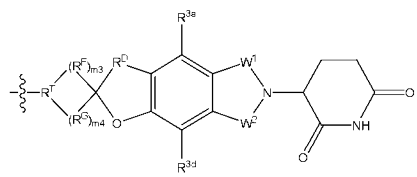, 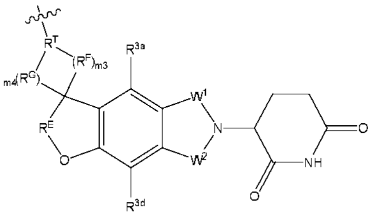,

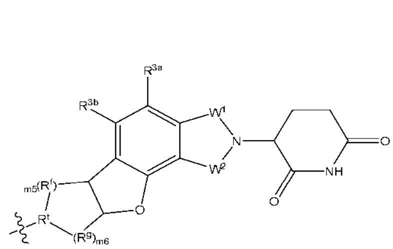, 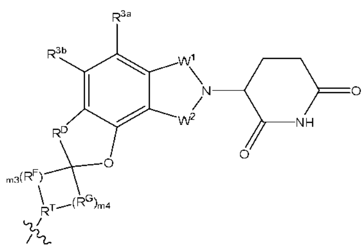, 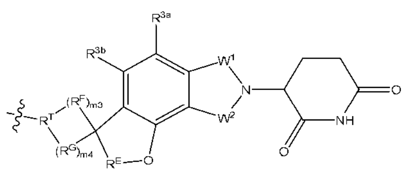,

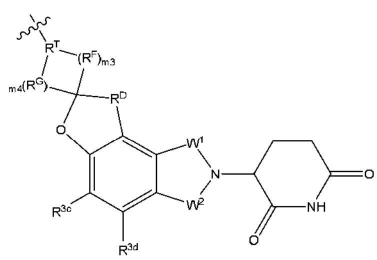, 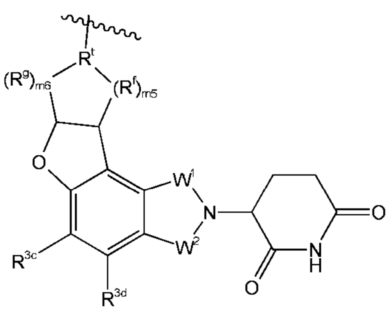 and 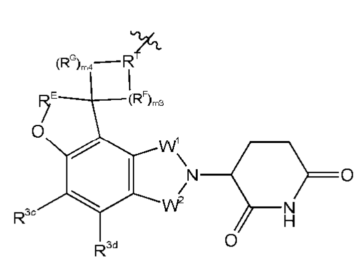;

wherein:

$W^1$, $W^2$, $R^{3a}$, $R^{3b}$, $R^{3c}$, $R^{3a}$, $R^d$, $R^e$, $R^f$, $R^t$, $R^g$, $R^D$, $R^E$, $R^F$, $R^T$, $R^G$, m3, m4, m5, and m6 are as defined in claim 1, 2, 3, or 4;

preferably:

$R^{3a}$, $R^{3b}$, $R^{3c}$, and $R^{3d}$ are each independently selected from H, deuterium atom, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; and/or
each occurrence of m3 is each independently an integer of 0, 1, 2, 3, or 4, and m4 is an integer of 1, 2, 3, 4, or 5, and m3 + m4 $\leq$ 5, preferably m3 + m4 = 2, m3 + m4 = 3, or m3 + m4 = 4; and/or
each occurrence of m5 and m6 are each independently an integer of 0, 1, 2, 3, or 4, and m5 + m6 $\leq$ 4; preferably m5 + m6 = 2 or m5 + m6 = 3; and/or
$W^1$ and $W^2$ are identical or different and are each independently $CH_2$ or C(=O), and at least one of $W^1$ and $W^2$ is C(=O); and/or
each occurrence of $R^d$, $R^e$, $R^f$, $R^g$, $R^D$, $R^E$, $R^F$, and $R^G$ are each independently $C(R^m)_2$ or O, each occurrence of $R^m$ is each independently selected from H, deuterium atom, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy,hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl,hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; and/or
each occurrence of $R^t$ and $R^T$ are each independently N or $CR^{2h}$, wherein $R^{2h}$ is selected from H, deuterium atom, F, Cl, Br, I, $C_1$-$C_6$ alkyl, $C_1$-$C_6$ deuterated alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl.

**7.** The compound according to any one of claims 1 to 6, wherein the CLM is selected from the following structures:

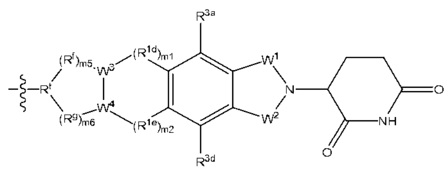,
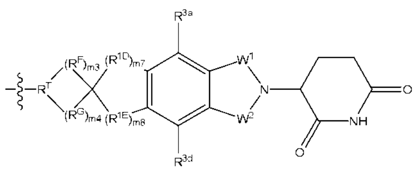,
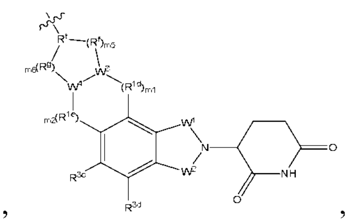,
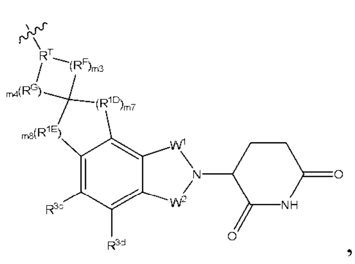,
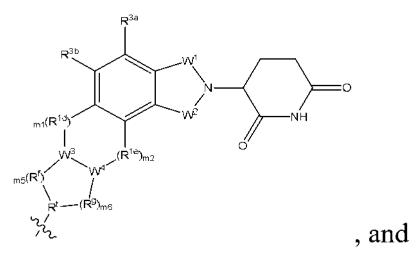, and
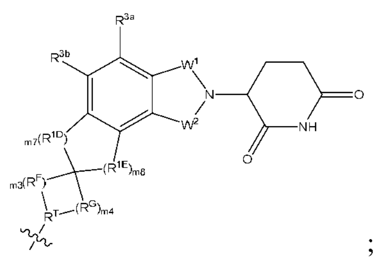;

wherein:

$W^1$, $W^2$, $W^3$, $W^4$, $R^{3a}$, $R^{3b}$, $R^{3c}$, $R^{3d}$, $R^f$, $R^t$, $R^g$, $R^F$, $R^G$, m1, m2, m3, m4, m5, m6, m7, and m8 are as defined in claim 1, 2, 3, or 4;
preferably,
$R^{3a}$, $R^{3b}$, $R^{3c}$, and $R^{3d}$ are each independently selected from H, deuterium atom, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy,hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl;
each occurrence of $R^{1d}$, $R^{1e}$, $R^{1D}$, and $R^{1E}$ are each independently $C(R^m)_2$;
$R^T$ is N or $CR^{2h}$, and when both $R^F$ and $R^G$ are $C(R^m)_2$, $R^T$ is $CR^{2h}$; and
$R^{2h}$ and $R^m$ are as defined in claim 1, 2, 3, or 4.

**8.** The compound according to any one of claims 1 to 7, wherein the CLM is selected from the following structures:

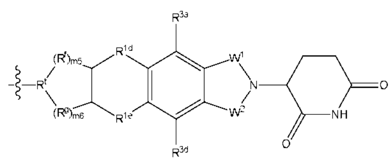,
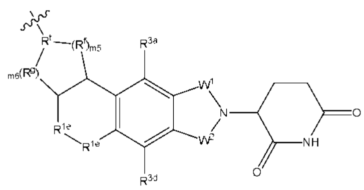,
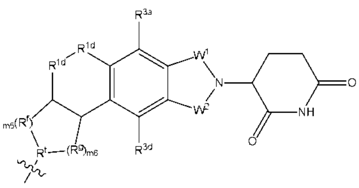,
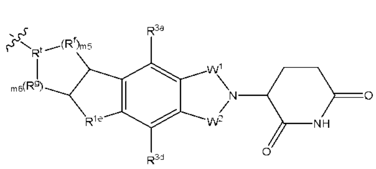,
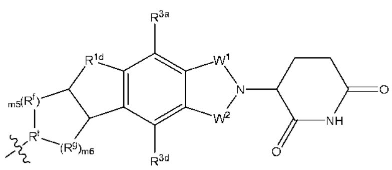,
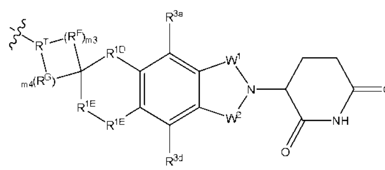,
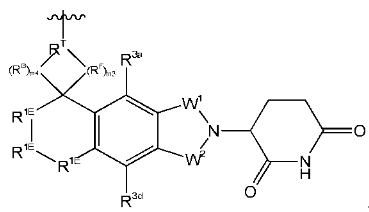,
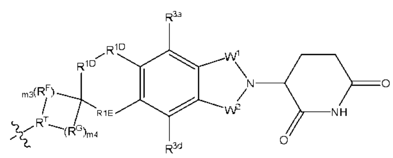,
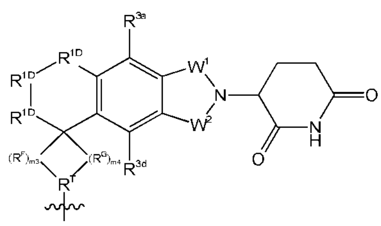,
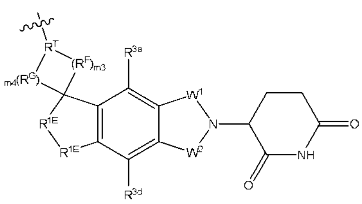,
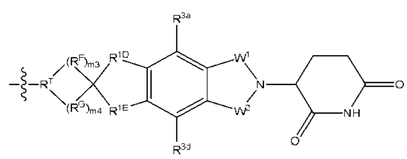,
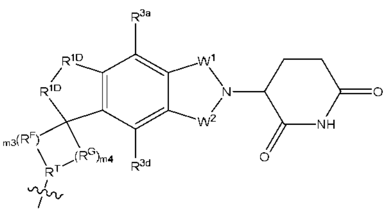,
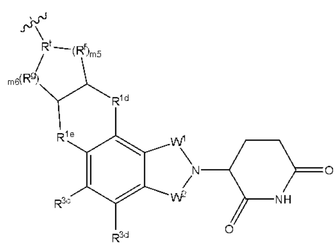,
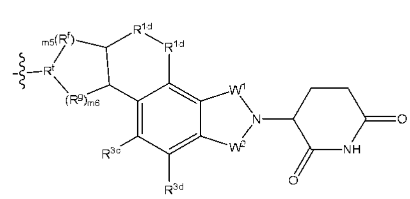,
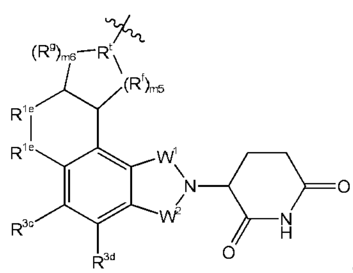,

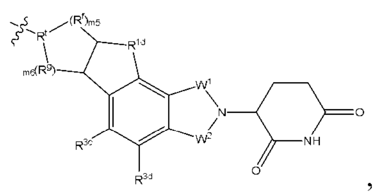,
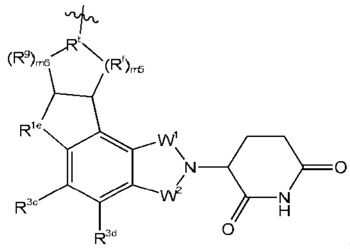,
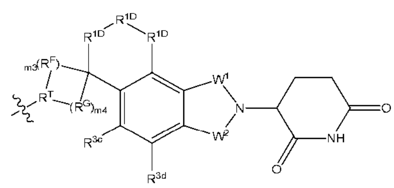,
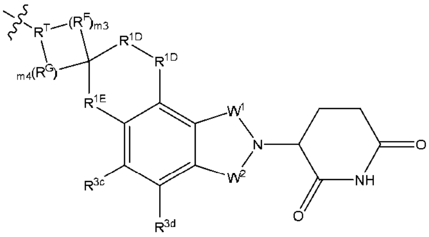,
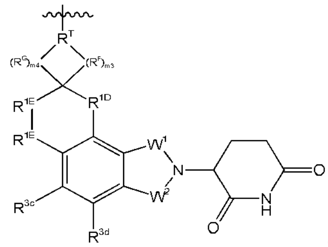,
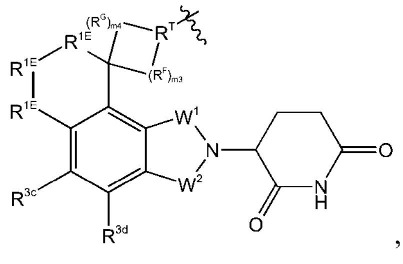,
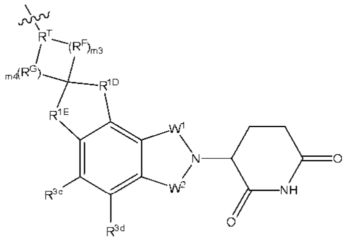,
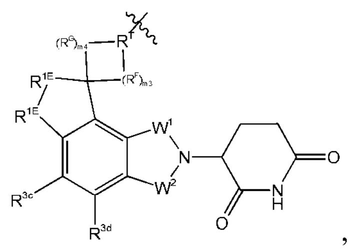,
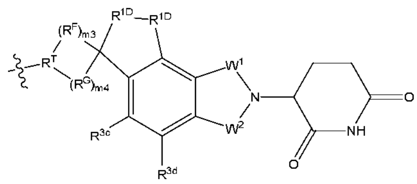,
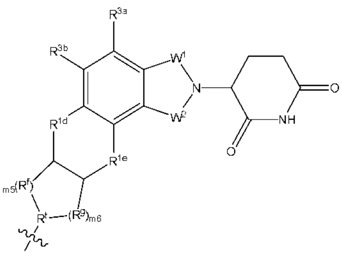,
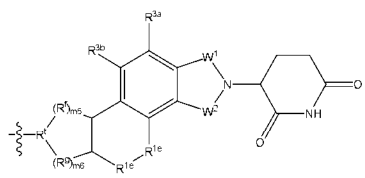,
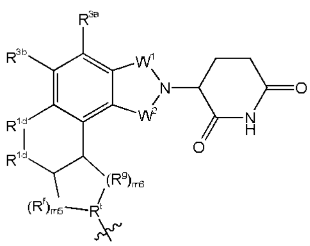,
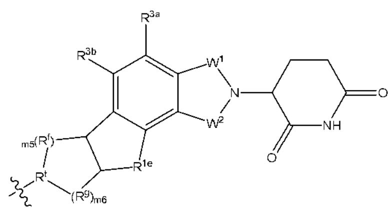,
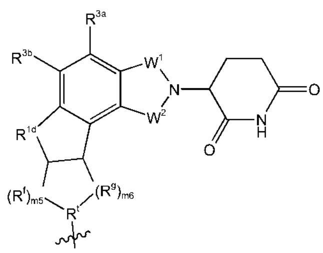,
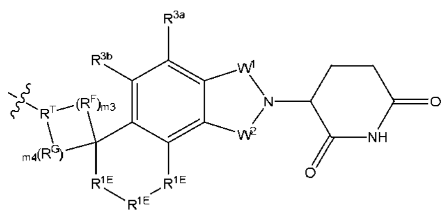,
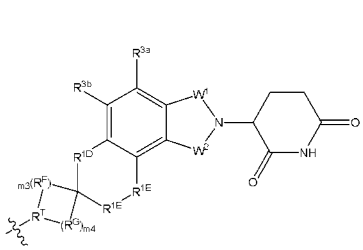,
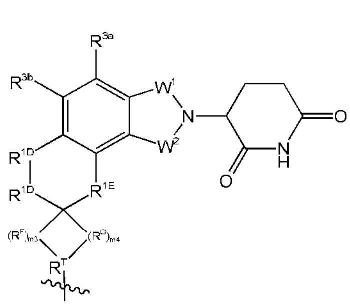,
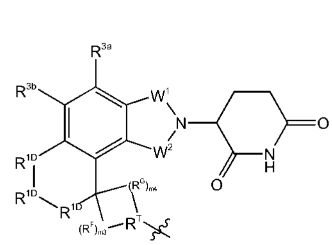,

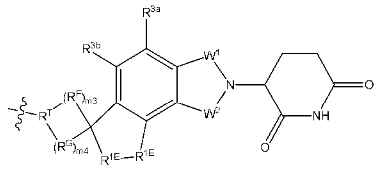, 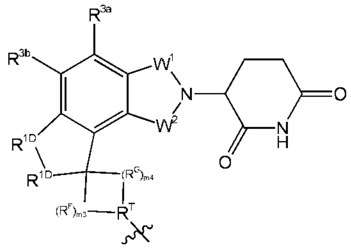, and 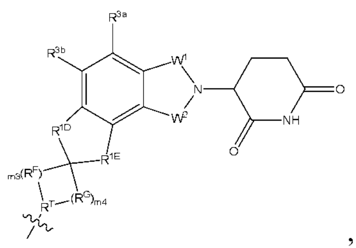,

wherein, $W^1$, $W^2$, $R^{3a}$, $R^{3b}$, $R^{3c}$, $R^{3a}$, $R^f$, $R^t$, $R^g$, $R^F$, $R^G$, m3, m4, m5, and m6 are as defined in claim 1, 2, 3, or 4; each occurrence of $R^{1d}$, $R^{1e}$, $R^{1D}$, $R^{1E}$ are each independently $C(R^m)_2$; $R^T$ is N or $CR^{2h}$, and when both $R^F$ and $R^G$ are $C(R^m)_2$, $R^T$ is $CR^{2h}$; and $R^{2h}$ and $R^m$ are as defined in claims 1, 2, 3, or 4;
preferably:

$R^{3a}$, $R^{3b}$, $R^{3c}$, and $R^{3d}$ are each independently selected from H, deuterium atom, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; and/or
each occurrence of m3 is independently an integer of 0, 1, 2, 3, or 4, m4 is an integer of 1, 2, 3, 4, or 5; and $m3 + m4 \leq 5$; preferably, m3 + m4 = 2, m3 + m4 =3, or m3 + m4 =4; and/or
each occurrence of m5 and m6 are each independently 0, 1, 2, 3, or 4, and $m5 + m6 \leq 4$, preferably m5 + m6 = 2 or m5 + m6 =3; and/or
$W^1$ and $W^2$ are identical or different and are each independently $CH_2$ or C(=O), and at least one of $W^1$ and $W^2$ is C(=O); and/or
each occurrence of $R^{1d}$, $R^{1e}$, $R^{1D}$, $R^{1E}$, $R^f$, $R^g$, $R^F$, and $R^G$ are each independently $C(R^m)_2$, and each occurrence of $R^m$ is each independently selected from H, deuterium atom, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; and/or
each occurrence of $R^t$ is each independently N or $CR^{2h}$, each occurrence of $R^T$ is each independently $CR^{2h}$, and $R^{1h}$, $R^{2h}$, and $R^{3h}$ are each independently selected from H, deuterium atom, F, Cl, Br, I, $C_1$-$C_6$ alkyl, $C_1$-$C_6$ deuterated alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl,hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl.

**9.** The compound according to any one of claims 1 to 8, wherein the CLM is selected from the following structures:

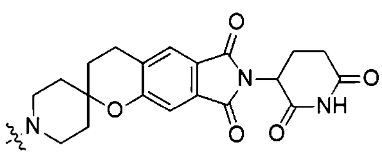, 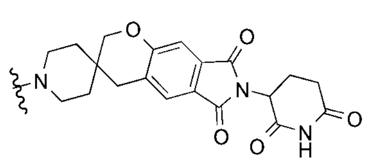, 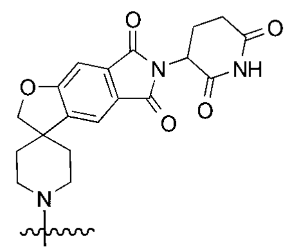,

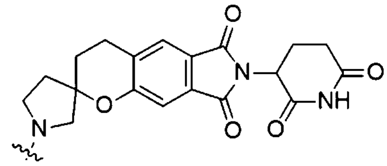,
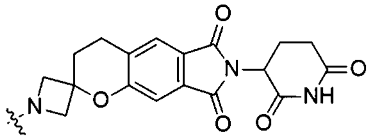,
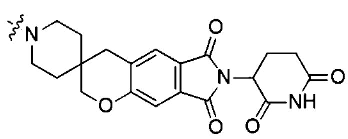,

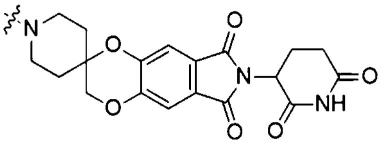,
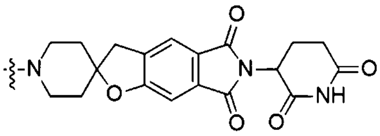,
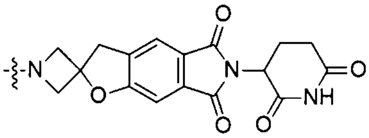,

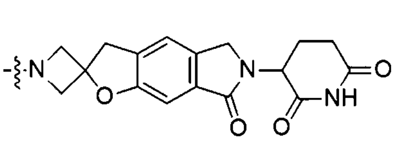,
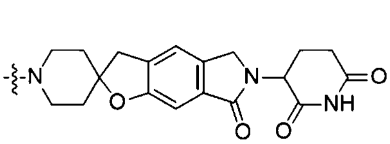,
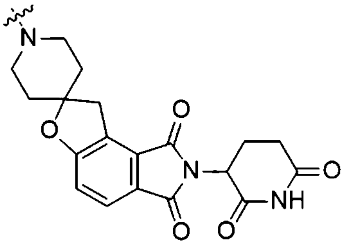,

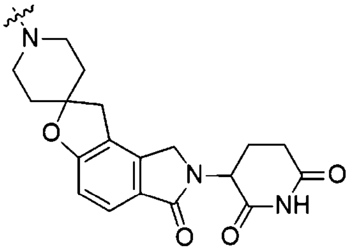,
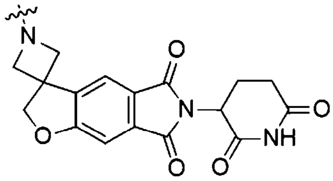,
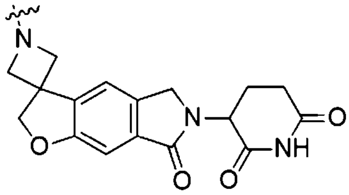,

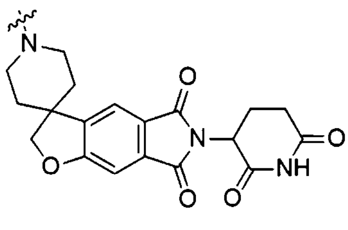,
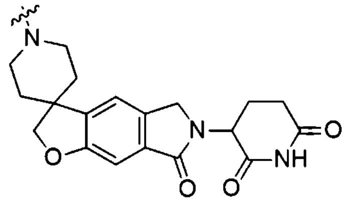,
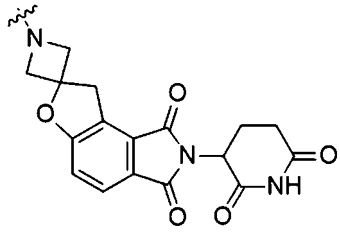,

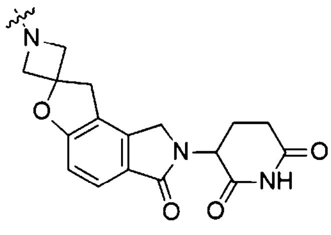,
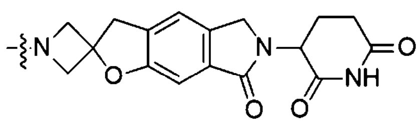,
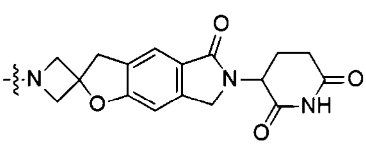,

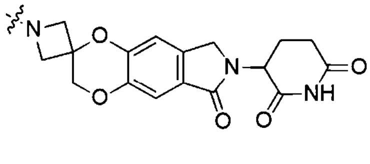,
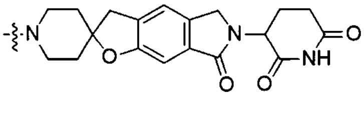,
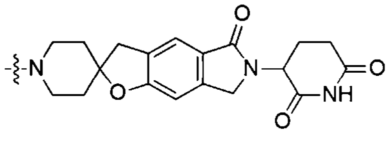,

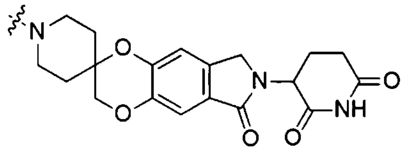 , 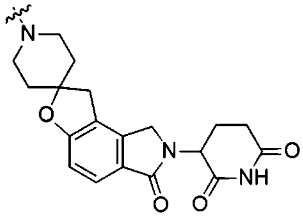 , 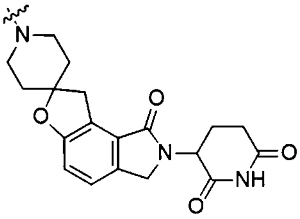 ,

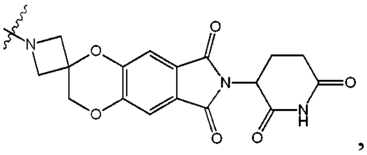 , 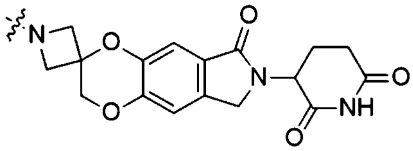 , 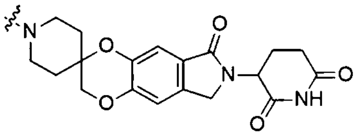 ,

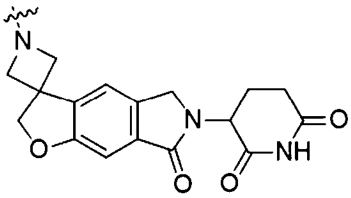 , 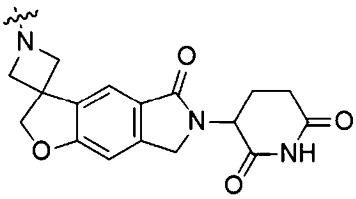 , 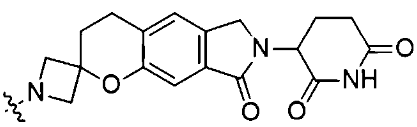 ,

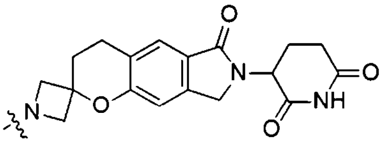 , 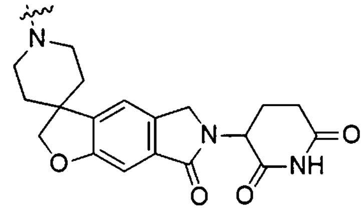 , 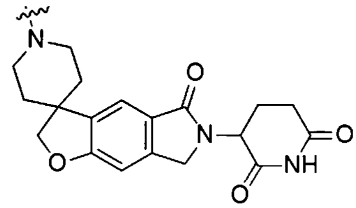 ,

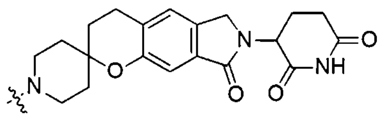 , 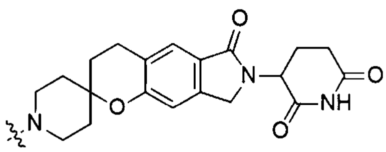 , 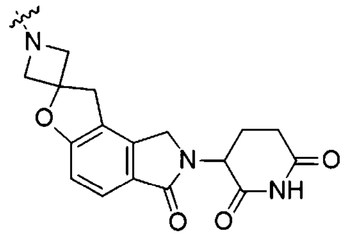 ,

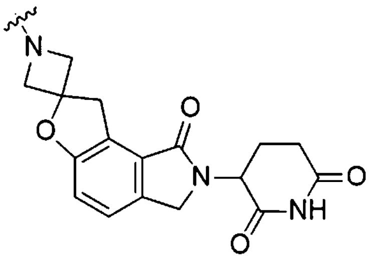 , 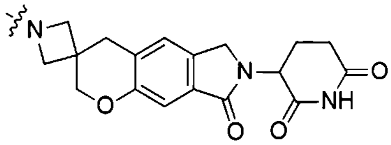 , 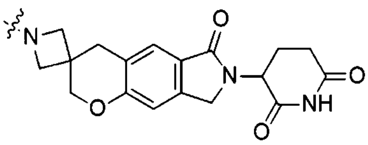 ,

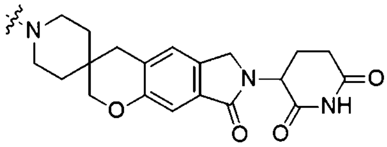 , 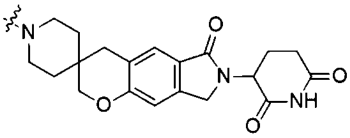 , 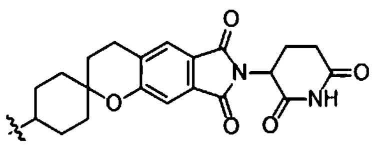 ,

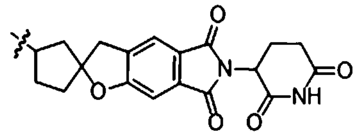 , 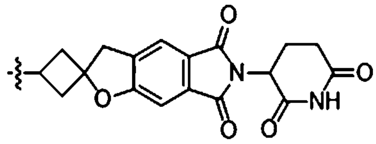 , 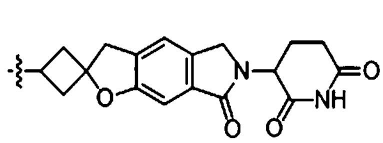 ,

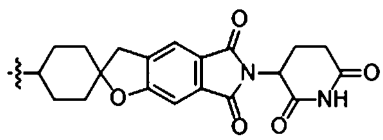 , 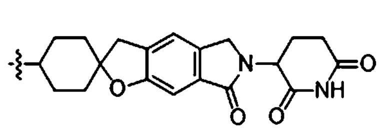 , 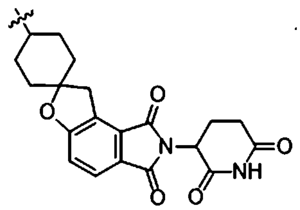 ,

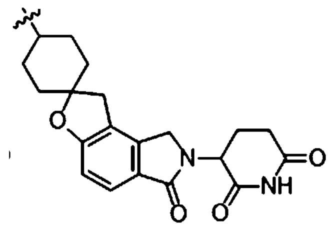 , 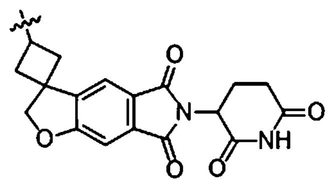 , 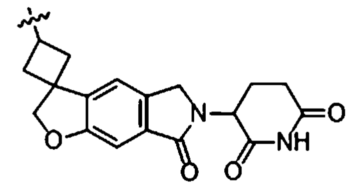 ,

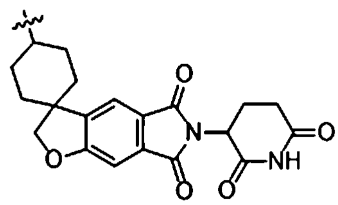 , 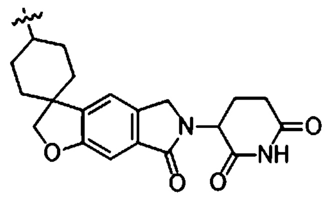 , 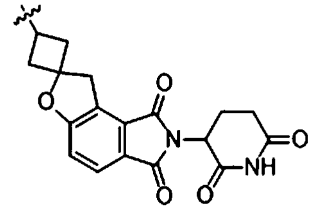 ,

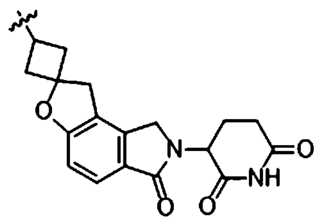 , 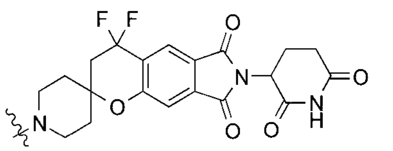 , 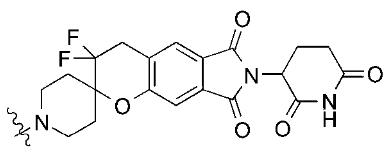 ,

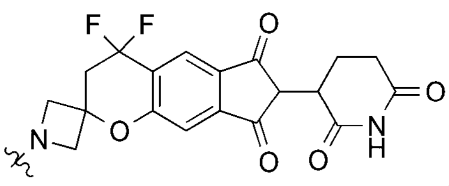 , 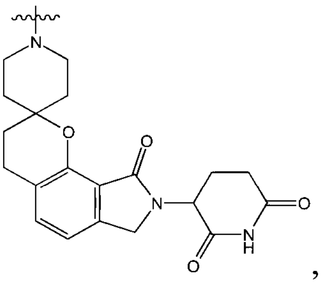 , 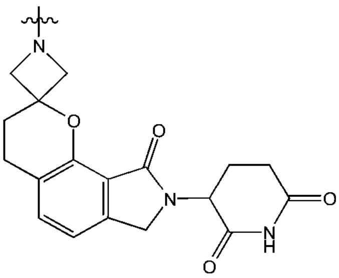 ,

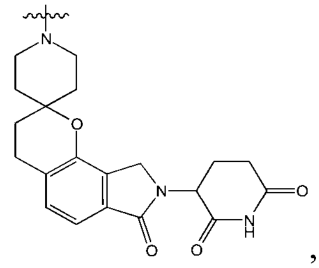,
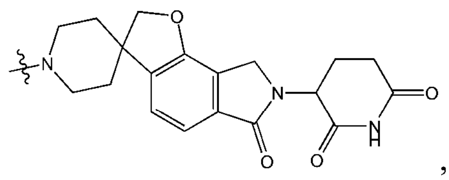,
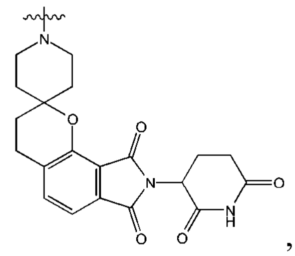,

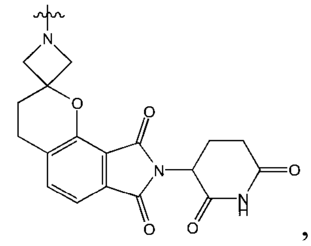,
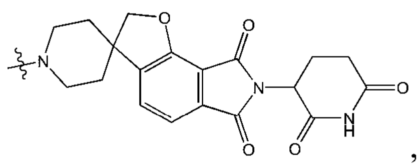,
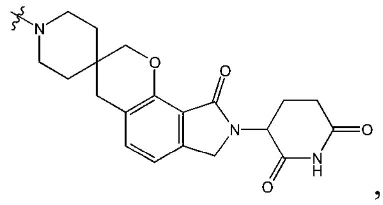,

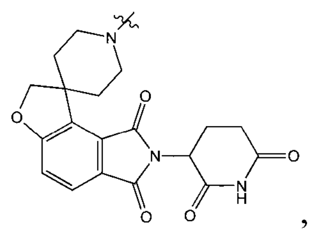,
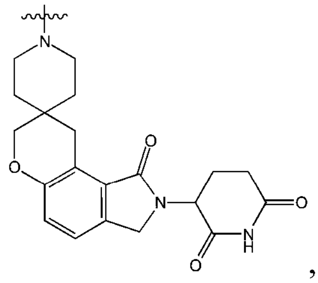,
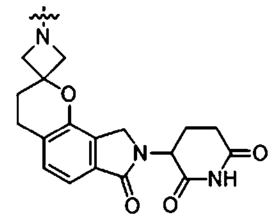,
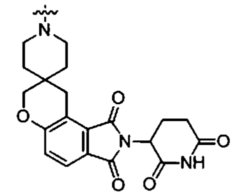,

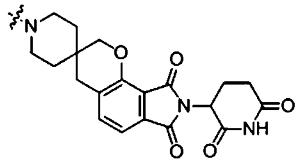,
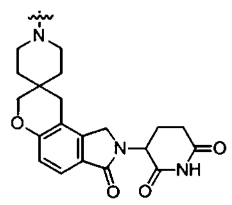,
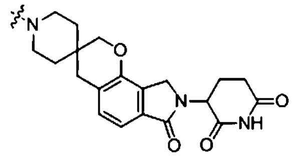,
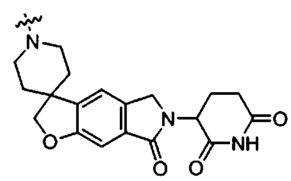,

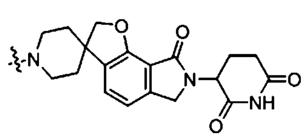,
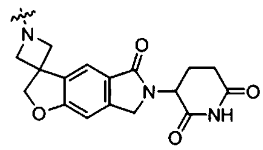,
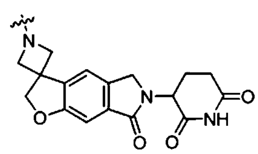,
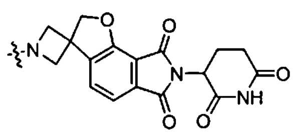,

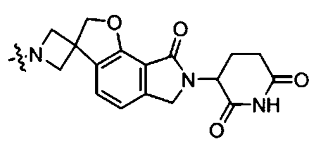,
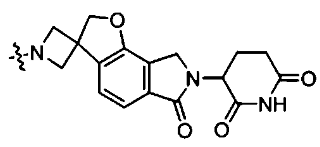,
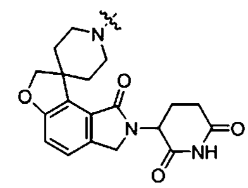,
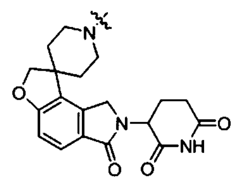,

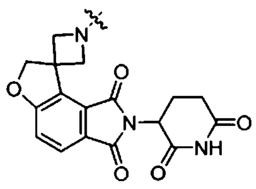, 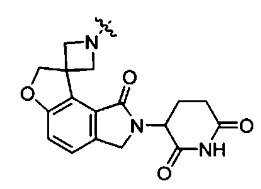, 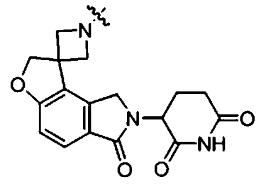, 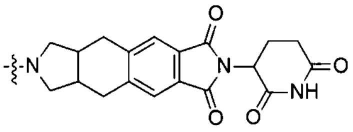,

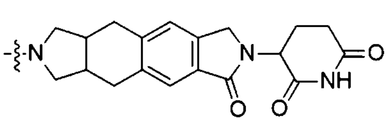, 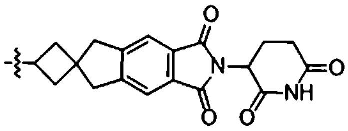, 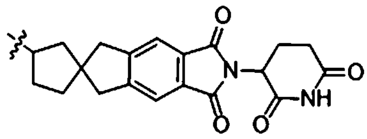,

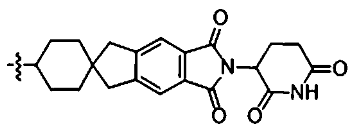, 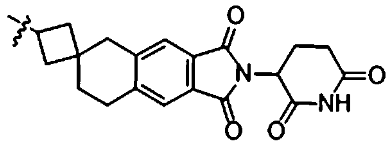, 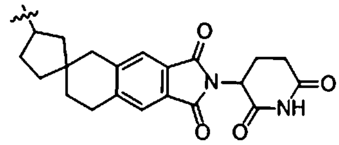,

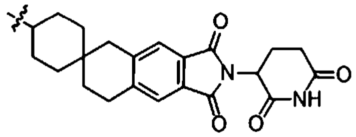, 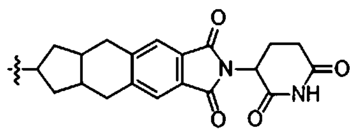, 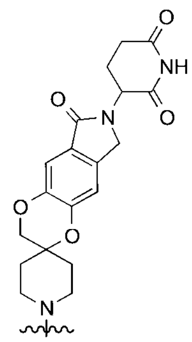,

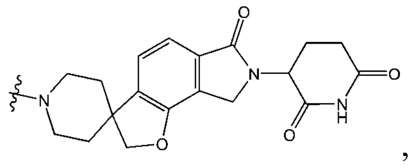, 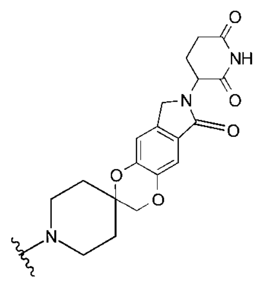, 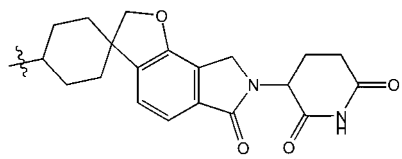,

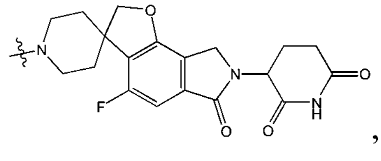, and 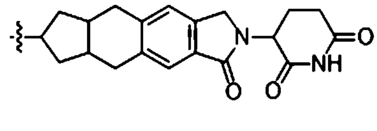.

**10.** The compound according to any one of claims 1 to 9, wherein L is a bond or $-(B^L)_q-$: each occurrence of $B^L$ is identical or different and is each independently selected from: $CR^{L1}R^{L2}$, O, S, SO, $SO_2$, $NR^{L3}$, $SO_2NR^{L3}$, $SONR^{L3}$, $CONR^{L3}$, $NR^{L3}CONR^{L4}$, $NR^{L3}SO_2NR^{L4}$, CO, $CR^{L1}=CR^{L2}$, C≡C, $SiR^{L1}R^{L2}$, $P(O)R^{L1}$, $P(O)OR^{L1}$, $NR^{L3}C(=NCN)NR^{L4}$, $NR^{L3}C(=NCN)$, $NR^{L3}C(=CNO_2)NR^{L4}$, cycloalkylene, heterocyclylene, arylene, or heteroarylene, wherein the cycloalkylene, heterocyclylene, arylene, and heteroarylene are optionally substituted with 0-6 $R^{L1}$ and/or $R^{L2}$ groups; preferably, each occurrence of $B^L$ is each independently selected from: $CR^{L1}R^{L2}$, O, S, SO, $SO_2$, $NR^{L3}$, CO, C≡C, 3-16-membered cycloalkylene , 3-16-membered heterocyclylene, 6-10-membered arylene, or 5-10-membered heteroarylene, wherein the 3-16-membered cycloalkylene, 3-16-membered heterocyclylene, 6-10-membered arylene, or 5-10-membered heteroarylene are optionally substituted with 0, 1, 2, or 3 $R^{L1}$ and/or $R^{L2}$ groups;

each occurrence of $R^{L1}$, $R^{L2}$, $R^{L3}$, and $R^{L4}$ are each independently selected from: H, halogen, $C_{1-8}$ alkyl, O-$C_{1-8}$ alkyl, S-$C_{1-8}$ alkyl, NH-$C_{1-8}$ alkyl, N($C_{1-8}$ alkyl)$_2$, $C_{3-11}$ cycloalkyl, aryl, heteroaryl, $C_{3-11}$ heterocyclyl, O-$C_{3-8}$ cycloalkyl, O-$C_{3-11}$ heterocyclyl, CO-$C_{3-8}$ cycloalkyl, CO-$C_{3-11}$ heterocyclyl, O-aryl, O-heteroaryl, S-$C_{3-8}$ cycloalkyl, NH-$C_{3-8}$ cycloalkyl, N($C_{3-8}$ cycloalkyl)$_2$, N($C_{3-8}$ cycloalkyl)($C_{1-8}$ alkyl), N($C_{1-8}$ alkylene)($C_{3-8}$ cycloalkyl), NH-$C_{3-8}$ heterocyclyl, N($C_{3-8}$ heterocyclyl)$_2$, N($C_{3-8}$ heterocyclyl)($C_{1-8}$ alkyl), NH-aryl, N(aryl)($C_{1-8}$ alkyl), NH-heteroaryl, N(heteroaryl)($C_{1-8}$ alkyl), OH, $NH_2$, SH, $SO_2$ P(O)(O-$C_{1-8}$ alkyl)($C_{1-8}$ alkyl), P(O)(O-$C_{1-8}$ alkyl)$_2$, C≡C-$C_{1-8}$ alkyl, C≡CH, CH=CH-($C_{1-8}$ alkyl), C($C_{1-8}$ alkyl)=CH-($C_{1-8}$ alkyl), C($C_{1-8}$ alkyl)=C($C_{1-8}$ alkyl)$_2$, Si(OH)$_3$, Si($C_{1-8}$ alkyl)$_3$, Si(OH)($C_{1-8}$ alkyl)$_2$, CO-$C_{1-8}$ alkyl, COO-$C_{1-8}$ alkyl, $CO_2H$, CN, $CF_3$, $CHF_2$, $CH_2F$, $NO_2$, $SF_5$, $SO_2$NH-$C_{1-8}$ alkyl, $SO_2$N($C_{1-8}$ alkyl)$_2$, SONH-$C_{1-8}$ alkyl, SON($C_{1-8}$ alkyl)$_2$, CONH-$C_{1-8}$ alkyl, CONH-$C_{3-8}$ cycloalkyl, CONH-$C_{3-11}$ heterocyclyl, CON($C_{1-8}$ alkyl)$_2$, N($C_{1-8}$ alkyl)CONH($C_{1-8}$ alkyl), N($C_{1-8}$ alkyl)CON($C_{1-8}$ alkyl)$_2$, NHCONH($C_{1-8}$ alkyl), NHCON($C_{1-8}$ alkyl)$_2$, $NHCONH_2$, N($C_{1-8}$ alkyl)$SO_2$NH($C_{1-8}$ alkyl), N($C_{1-8}$ alkyl) $SO_2$N($C_{1-8}$ alkyl)$_2$, $NHSO_2$NH($C_{1-8}$ alkyl), $NHSO_2$N($C_{1-8}$ alkyl)$_2$, and $NHSO_2NH_2$, optionally, the $C_{1-8}$ alkyl, $C_{3-11}$ cycloalkyl, $C_{3-8}$ cycloalkyl, $C_{3-11}$ heterocyclyl, $C_{6-10}$ aryl, and 5-10 membered heteroaryl are each independently substituted with one or more substituents selected from: halogen, alkyl, heteroalkyl, alkenyl, alkynyl, alkoxy, hydroxyl, haloalkyl, hydroxyalkyl, cyano, amino, nitro, cycloalkyl, heterocyclyl, halocycloalkyl, haloheteroalkyl, alkylamino, aryl, heteroaryl, haloaryl, and haloheteroaryl; preferably, the $C_{1-8}$ alkyl, $C_{3-11}$ cycloalkyl, $C_{3-8}$ cycloalkyl, $C_{3-11}$ heterocyclyl, $C_{6-10}$ aryl, and 5-10 membered heteroaryl are each independently substituted with one or more substituents selected from F, Cl, Br, I, $C_{1-6}$ alkyl, methoxy, ethoxy; and

q is an integer greater than or equal to 1; preferably, q is 1, 2, 3, 4, 5, 6, 7, 8, 9, 10, 11, 12, 13, 14, 15, 16, 17, 18, 19, or 20;

preferably,

each occurrence of $B^L$ is independently identical or different and is selected from: $CR^{L1}R^{L2}$, O, S, SO, $SO_2$, $NR^{L3}$, $SO_2NR^{L3}$, $SONR^{L3}$, $CONR^{L3}$, $NR^{L3}CONR^{L4}$, $NR^{L3}SO_2NR^{L4}$, CO, $CR^{L1}$=$CR^{L2}$, C≡C, $SiR^{L1}R^{L2}$, P(O)$R^{L1}$, P(O)O$R^{L1}$, $NR^{L3}$C(=NCN)$NR^{L4}$, $NR^{L3}$C(=NCN), $NR^{L3}$C(=$CNO_2$)$NR^{L4}$, cycloalkylene, heterocyclylene, arylene, or heteroarylene, wherein the cycloalkylene, heterocyclylene, arylene, and heteroarylene are optionally substituted with 0-6 $R^{L1}$ and/or $R^{L2}$ groups;

each occurrence of $R^{L1}$, $R^{L2}$, $R^{L3}$, and $R^{L4}$ are each independently selected from H, halogen, $C_{1-8}$ alkyl, O-$C_{1-8}$ alkyl, S-$C_{1-8}$ alkyl, NH-$C_{1-8}$ alkyl, N($C_{1-8}$ alkyl)$_2$, $C_{3-11}$ cycloalkyl, aryl, heteroaryl, $C_{3-11}$ heterocyclyl, O-$C_{3-8}$ cycloalkyl, O-$C_{3-11}$ heterocyclyl, O-aryl, O-heteroaryl, S-$C_{3-8}$ cycloalkyl, NH-$C_{3-8}$ cycloalkyl, N($C_{3-8}$ cycloalkyl)$_2$, N($C_{3-8}$ cycloalkyl)($C_{1-8}$ alkyl), NH-$C_{3-8}$ heterocyclyl, N($C_{3-8}$ heterocyclyl)$_2$, N($C_{3-8}$ heterocyclyl)($C_{1-8}$ alkyl), NH-aryl, N(aryl)($C_{1-8}$ alkyl), NH-heteroaryl, N(heteroaryl)($C_{1-8}$ alkyl), OH, $NH_2$, SH, $SO_2$ P(O)(O-$C_{1-8}$ alkyl)($C_{1-8}$ alkyl), P(O)(O-$C_{1-8}$ alkyl)$_2$, C≡C-$C_{1-8}$ alkyl, C≡CH, CH=CH-($C_{1-8}$ alkyl), C($C_{1-8}$ alkyl)=CH-($C_{1-8}$ alkyl), C($C_{1-8}$ alkyl)=C($C_{1-8}$ alkyl)$_2$, Si(OH)$_3$, Si($C_{1-8}$ alkyl)$_3$, Si(OH)($C_{1-8}$ alkyl)$_2$, CO-$C_{1-8}$ alkyl, $CO_2H$ CN, $CF_3$, $CHF_2$, $CH_2F$, $NO_2$, $SF_5$, $SO_2$NH-$C_{1-8}$ alkyl, $SO_2$N($C_{1-8}$ alkyl)$_2$, SONH-$C_{1-8}$ alkyl, SON($C_{1-8}$ alkyl)$_2$, CONH-$C_{1-8}$ alkyl, CON($C_{1-8}$ alkyl)$_2$, N($C_{1-8}$ alkyl)CONH($C_{1-8}$ alkyl), N($C_{1-8}$ alkyl)CON($C_{1-8}$ alkyl)$_2$, NHCONH($C_{1-8}$ alkyl), NHCON($C_{1-8}$ alkyl)$_2$, $NHCONH_2$, N($C_{1-8}$ alkyl)$SO_2$NH($C_{1-8}$ alkyl), N($C_{1-8}$ alkyl)$SO_2$N($C_{1-8}$ alkyl)$_2$, $NHSO_2$NH($C_{1-8}$ alkyl), $NHSO_2$N($C_{1-8}$ alkyl)$_2$, and $NHSO_2NH_2$, optionally, the $C_{1-8}$ alkyl, $C_{3-11}$ cycloalkyl, $C_{3-11}$ heterocyclyl, $C_{6-10}$ aryl, and $C_{5-10}$ heteroaryl are each independently substituted with one or more substituents selected from halogen, alkyl, heteroalkyl, alkenyl, alkynyl, alkoxy, hydroxyl, haloalkyl, hydroxyalkyl, cyano, amino, nitro, cycloalkyl, heterocyclyl, halocycloalkyl, haloheteroalkyl, alkylamino, aryl, heteroaryl, haloaryl, and haloheteroaryl; and

q is an integer greater than or equal to 1; preferably, q is 1, 2, 3, 4, 5, 6, 7, 8, 9, 10, 11, 12, 13, 14, 15, 16, 17, 18, 19, or 20.

**11.** The compound according to claim 10, wherein $B^L$ is selected from one or more of the following structures: -O-, -S-, -SO-, -$SO_2$-, -$CH_2$ -, -CO-, -NH-, -CH($CH_3$)-, -CH($CH_2CH_3$)-, -C($CH_3$)$_2$-, -N($CH_3$)-, - N($CH_2CH_3$)-,

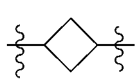 , 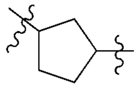 , 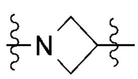 , 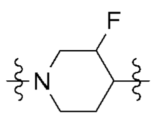 , 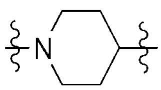 ,

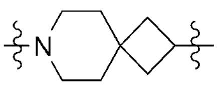 , 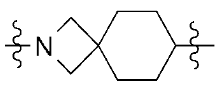 , 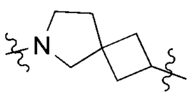 , 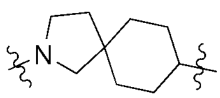 , 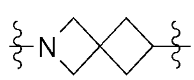 ,

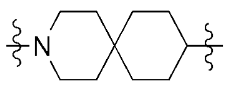 , 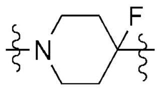 , 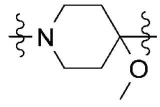 , 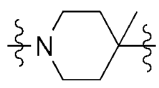 , 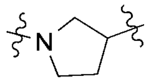 , 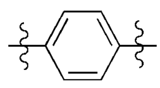 ,

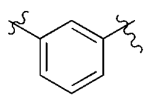 , 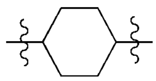 , 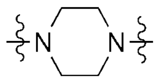 , 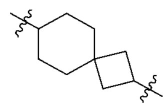 , 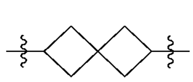 , 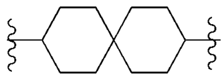 ,

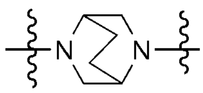 , 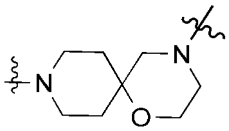 , 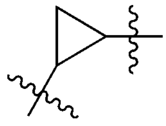 , 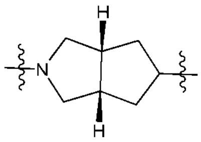 , 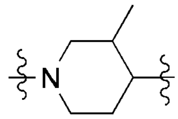 ,

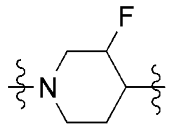 , 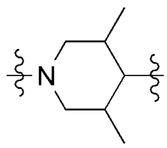 , 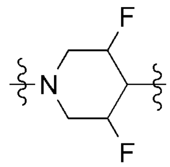 , 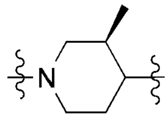 , 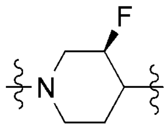 , 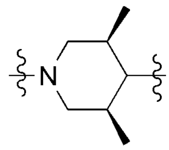 ,

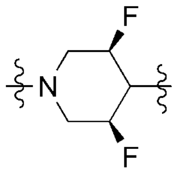 , 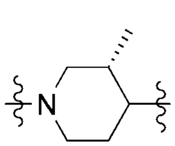 , 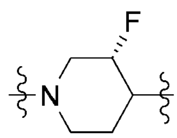 , 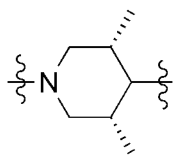 , 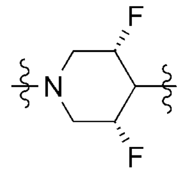 ,

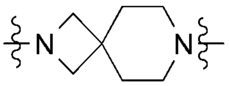 , 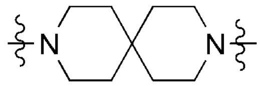 , 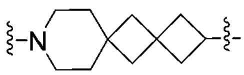 , 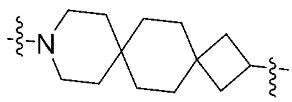 ,

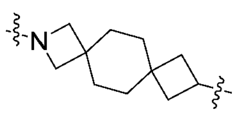 , 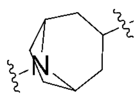 , 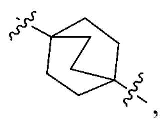 , 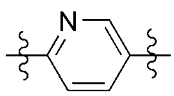 , 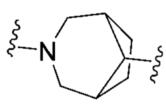 , 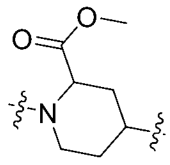 ,

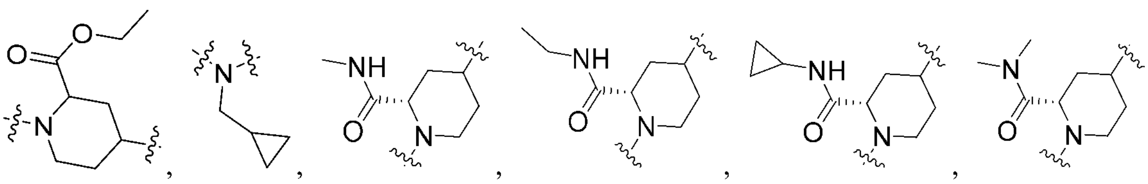 , , , , , ,

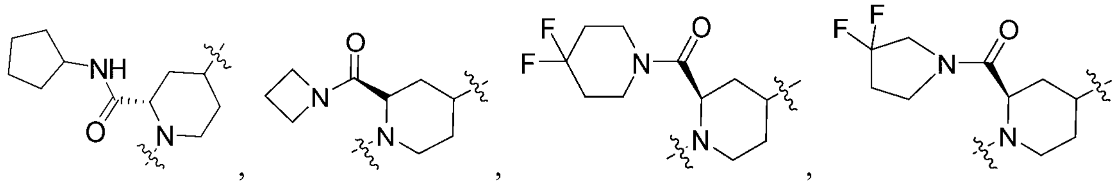 , , , ,

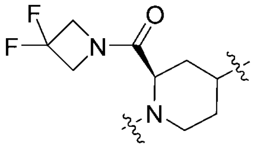, 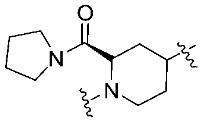, 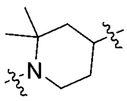, 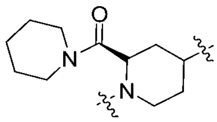, 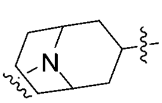, 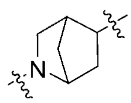,

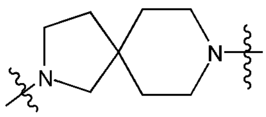, 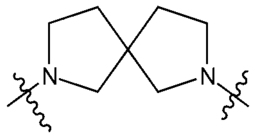, 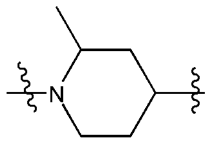, 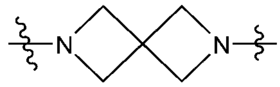,

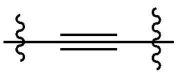, 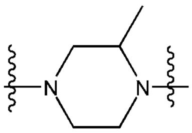, 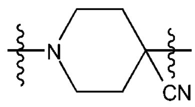, 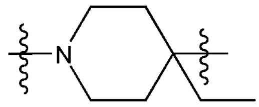 and 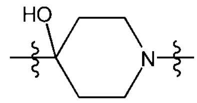,

wherein

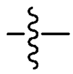

is the point of attachment.

**12.** The compound according to any one of claims 1 to 11, wherein L is selected from the following structures:

a covalent bond, $-(CH_2)_j-$, $-(CH_2)_p-NH-(CH_2)_s-$, $-(CH_2)_y-NH-(CH_2)_j-NH-(CH_2)_s-$, $-(CH_2)_p-CO-(CH_2)_s-$, $-(CH_2)_p-O-(CH_2)_s-$, $-(CH_2)_y-CO-(CH_2)_j-CO-(CH_2)_s-$, $-(CH_2)_y-O-(CH_2)_j-O-(CH_2)_s-$, $-(CH_2)_y-O-(CH_2)_j-CO-(CH_2)_s-$, $-(CH_2)_p-NH-(CH_2)_y-O-(CH_2)_j-CO-(CH_2)_s-$,

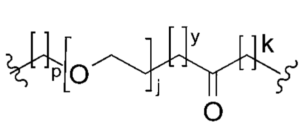, 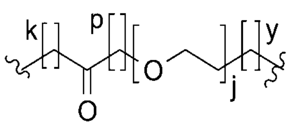,

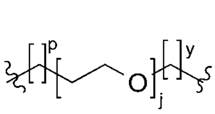, 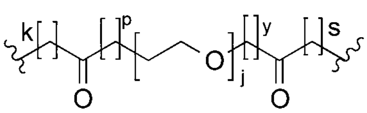, 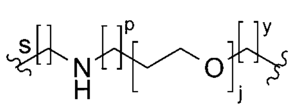,

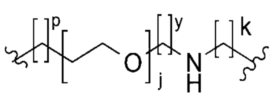, 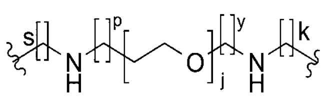, 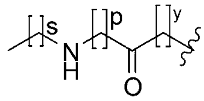, 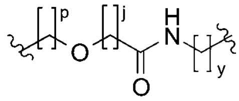,

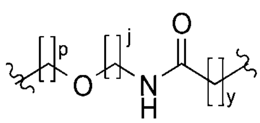, 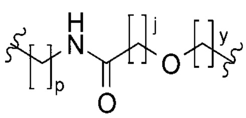, 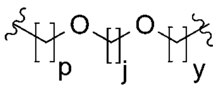, 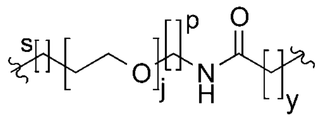,

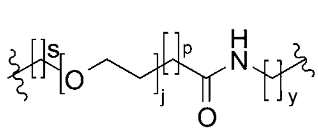, 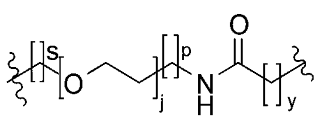, 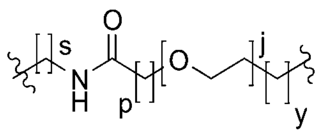,

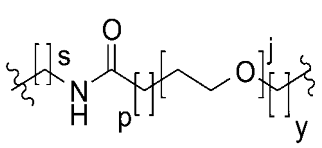, 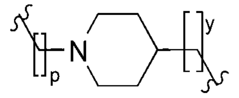, 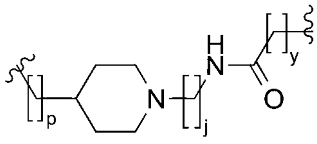,

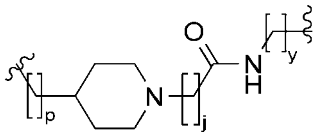, 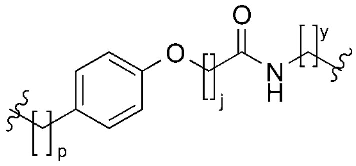, 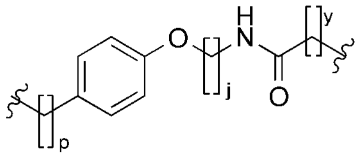,

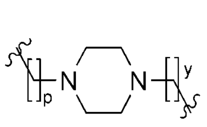, 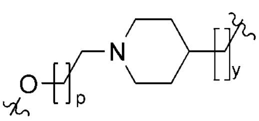, 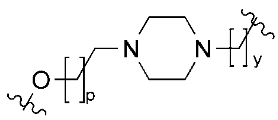, 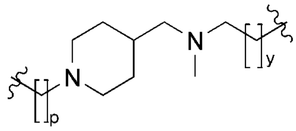,

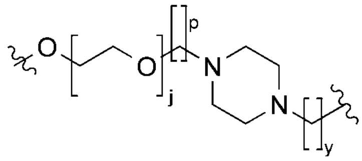, 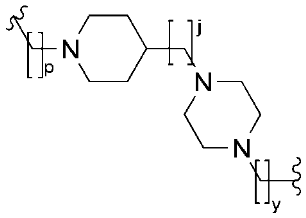, 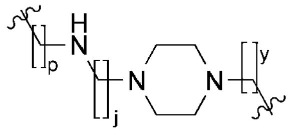, ,

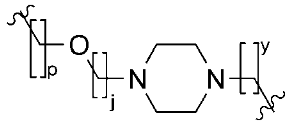, 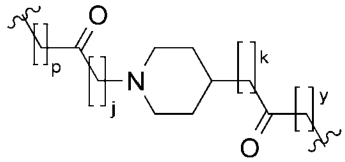, 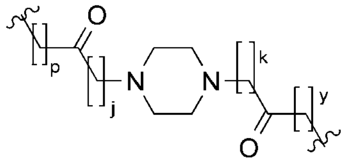,

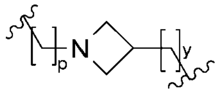, 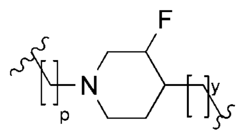, 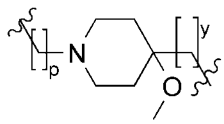, 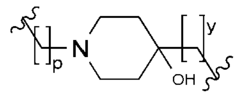,

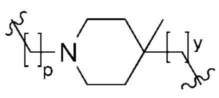, 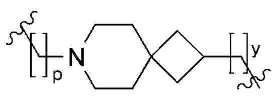, 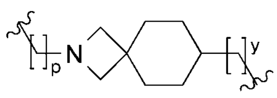, 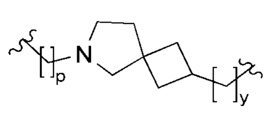,

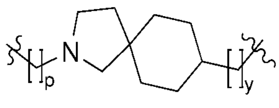, 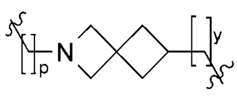, 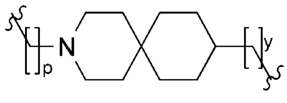, 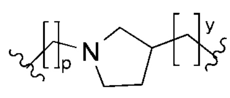,

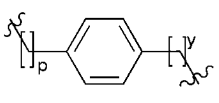, 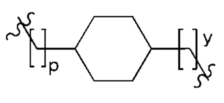, 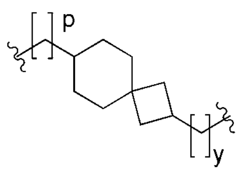, 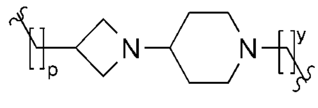,

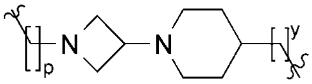, 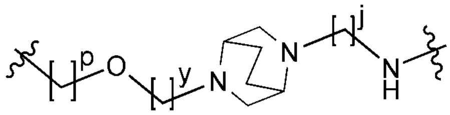,

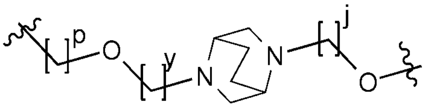, 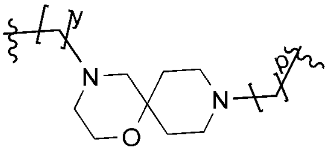, 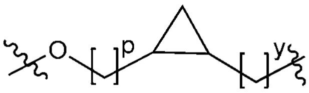,

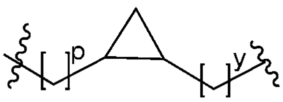, 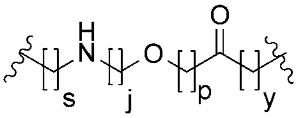, 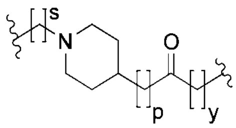, 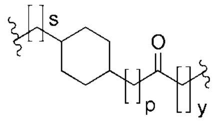,

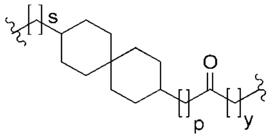, 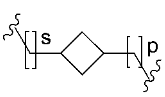, 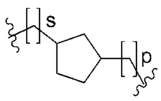, 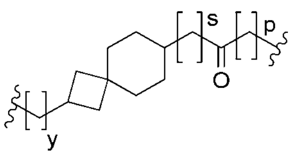,

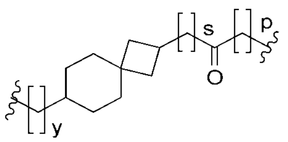, 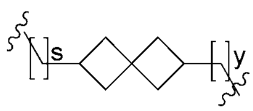, 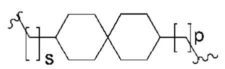, 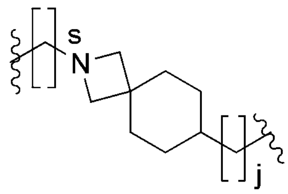,

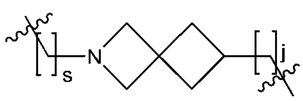, 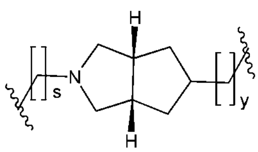, 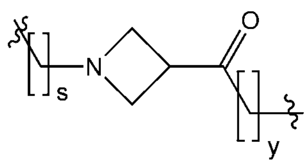,

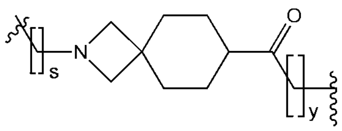, 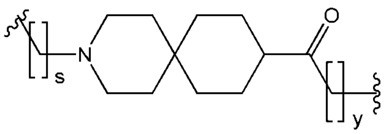, 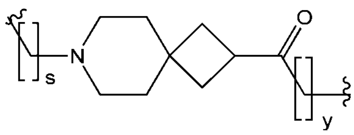,

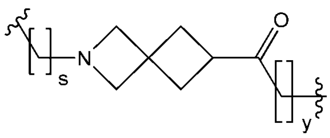, 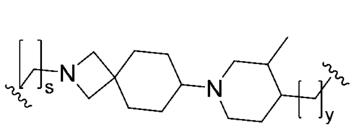, 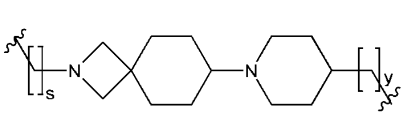,

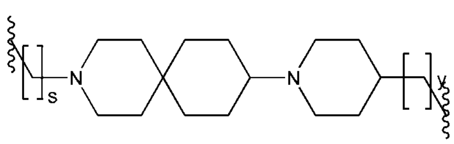, 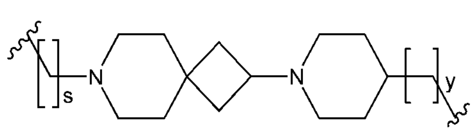,

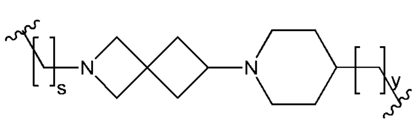, 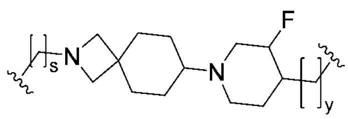, 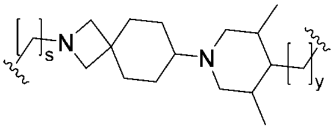,

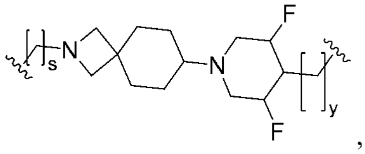, 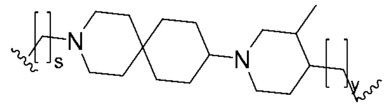, 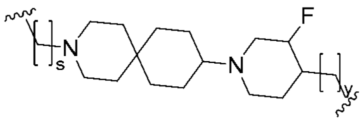,

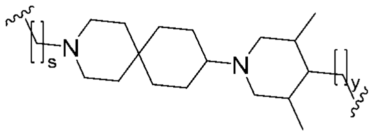, 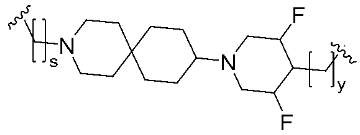, 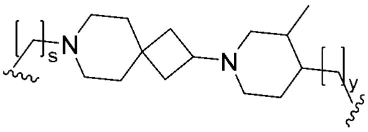,

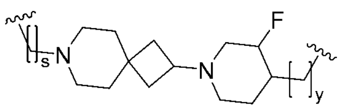, 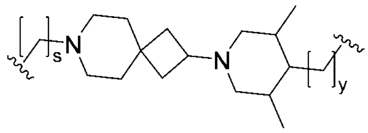, 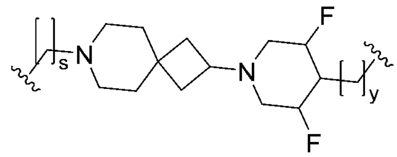,

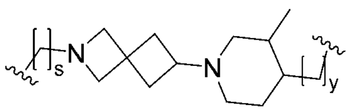, 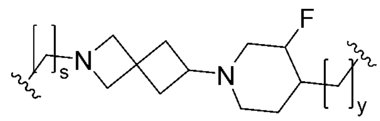, 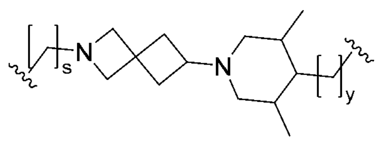,

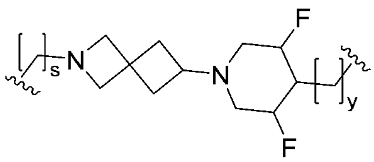, 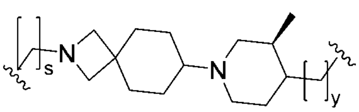, 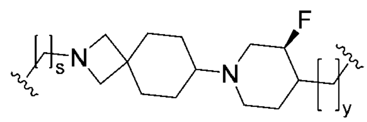,

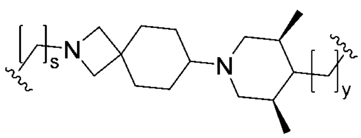, 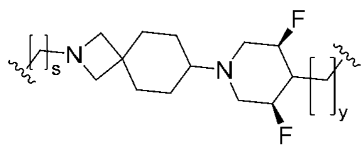, 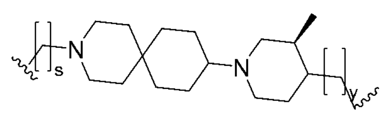,

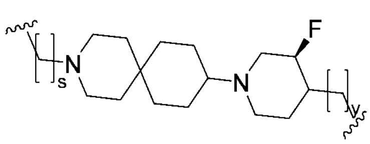, 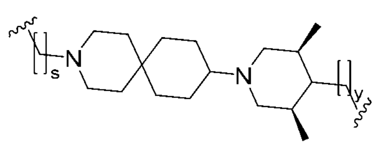, 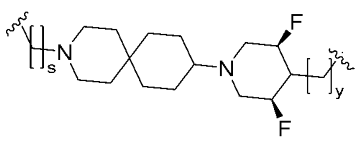,

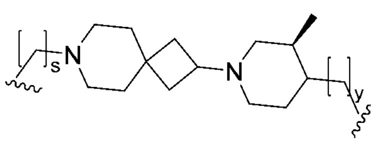, 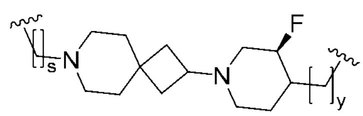, 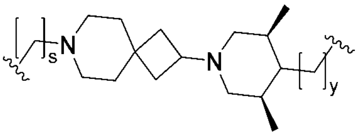,

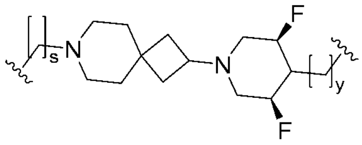, 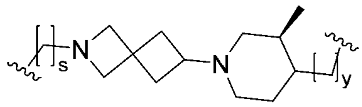, 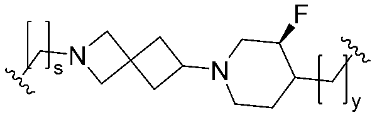,

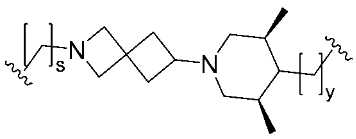, 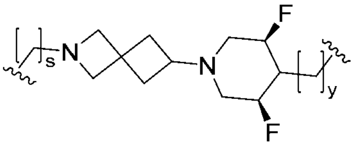, 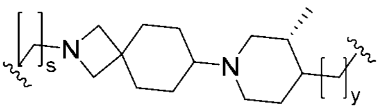,

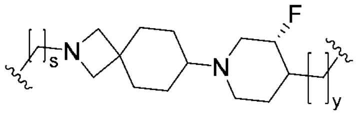, 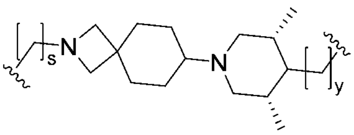, 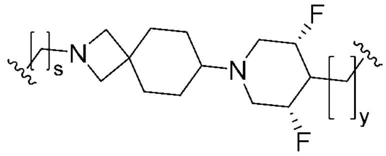,

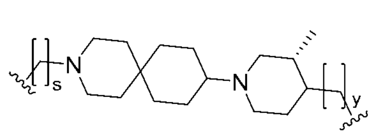, 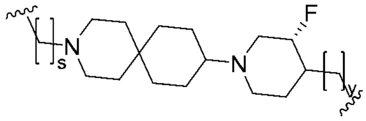, 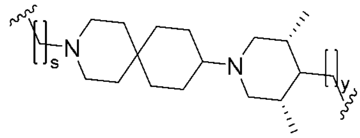,

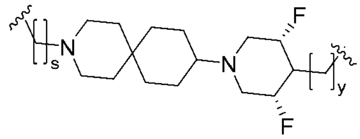, 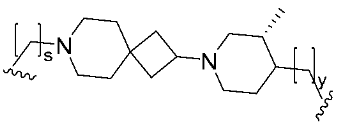, 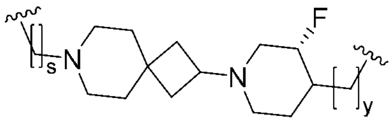,

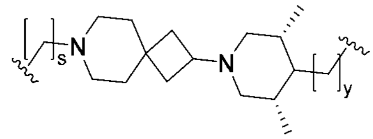, 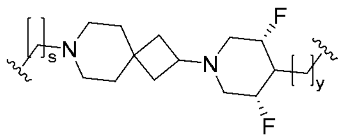, 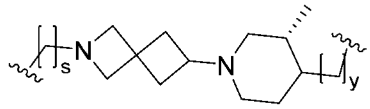,

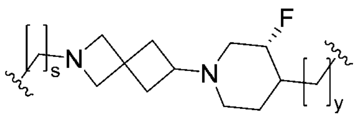, 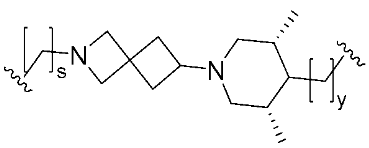, 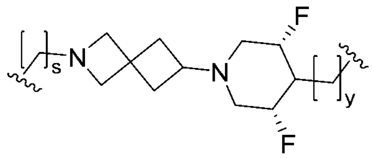,

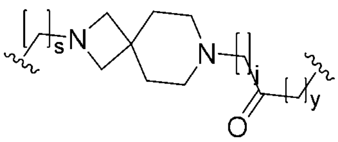, 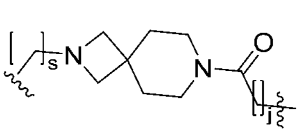, 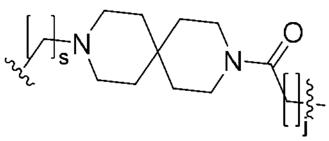,

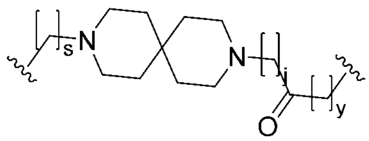, 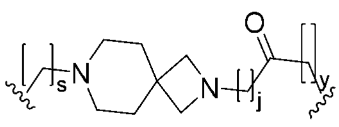, 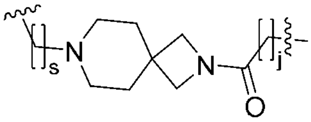,

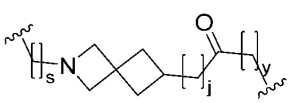, 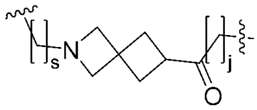, 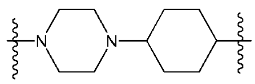, 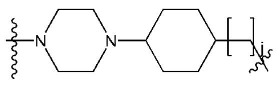,

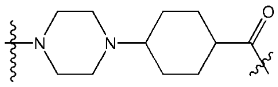, 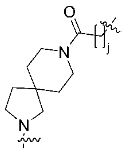, 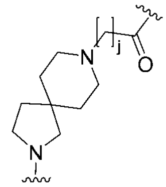, 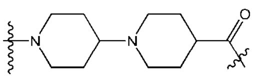, 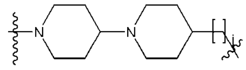,

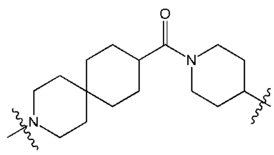, 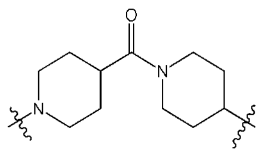, 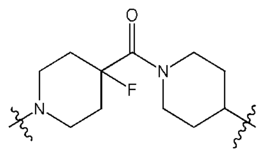, 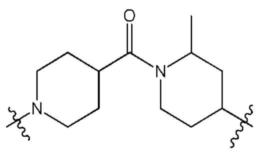,

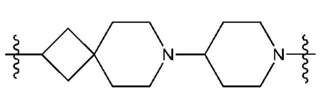, 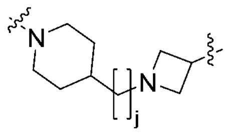, 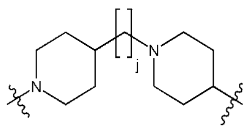, 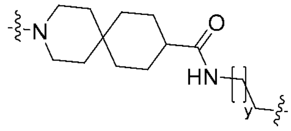,

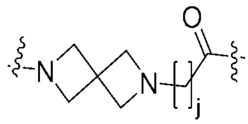, 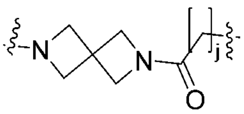, 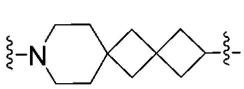, 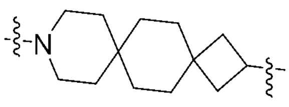,

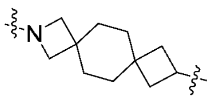 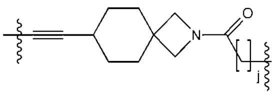, 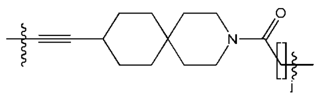, 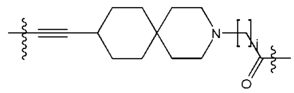,

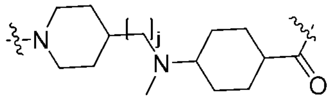, 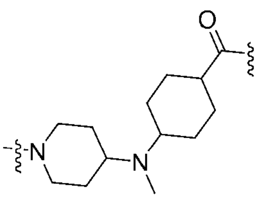, 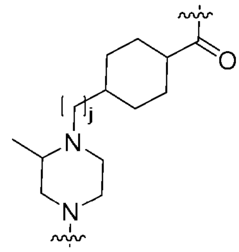, 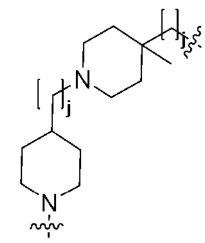,

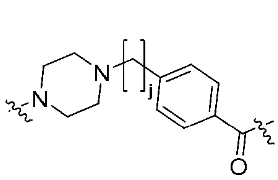, 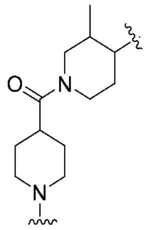, 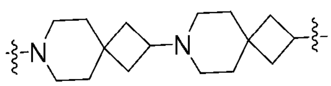, 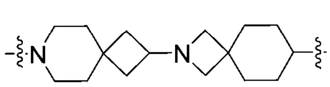,

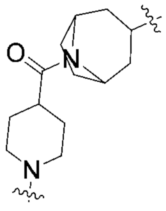, 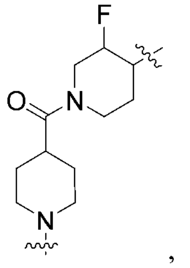, 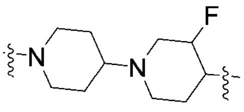, 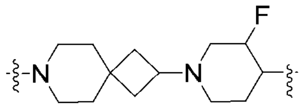,

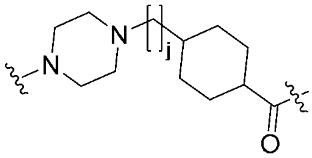, 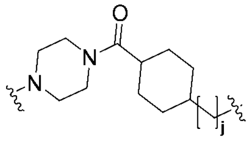, 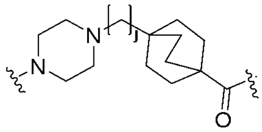, 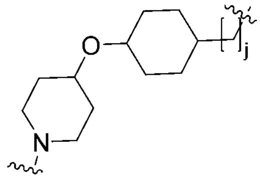,

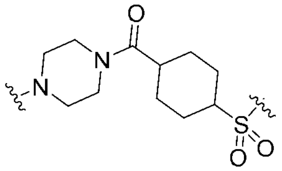, 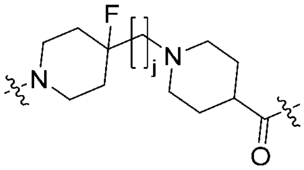, 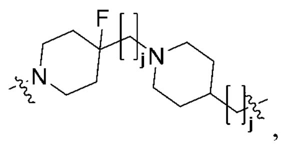, 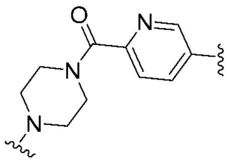,

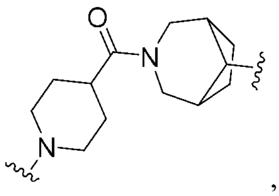, 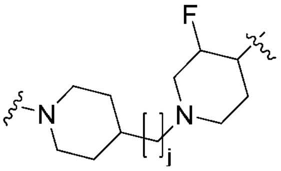, 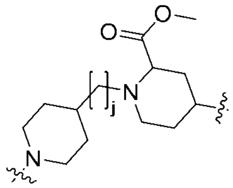, 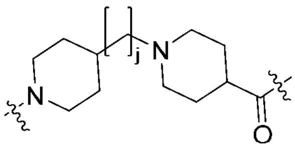,

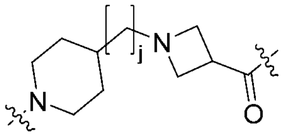, 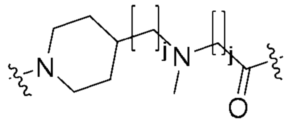, 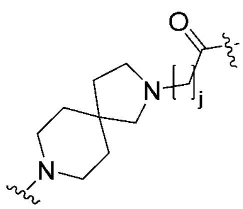, 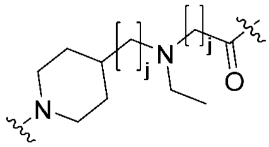,

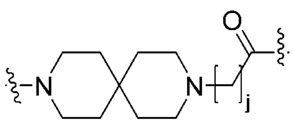, 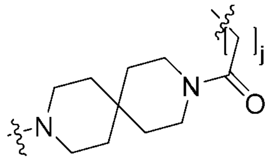, 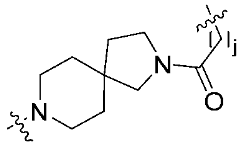, 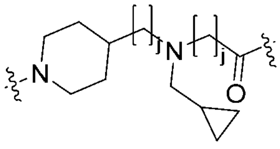,

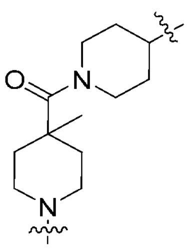, 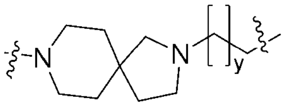, 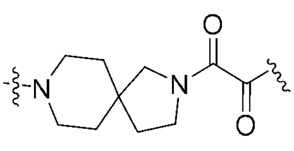, 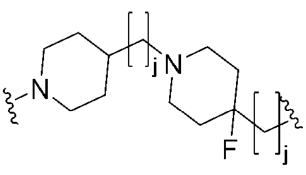,

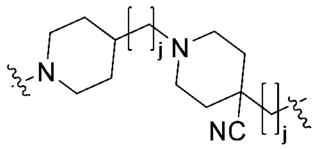 , 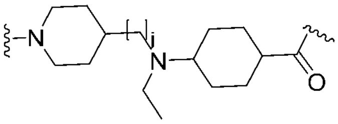 , 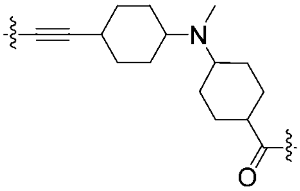 ,

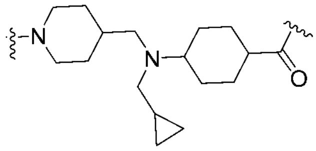 , 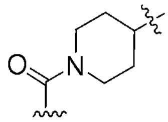 , 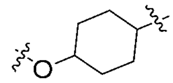 , 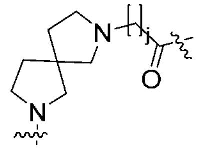 , 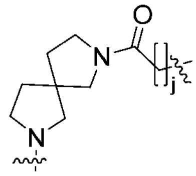 ,

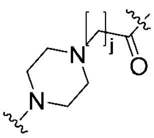 , 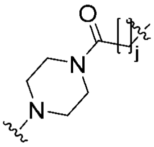 , 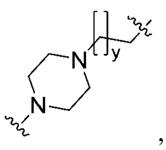 , 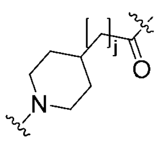 , 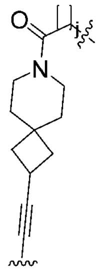 , 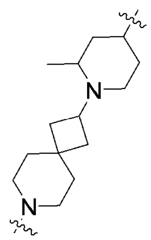 , 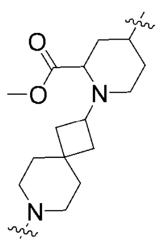 ,

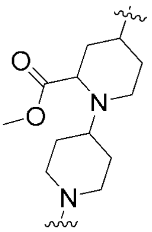 , 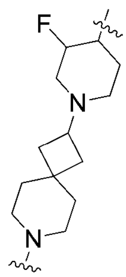 , 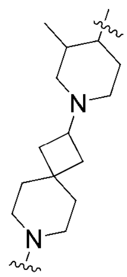 , 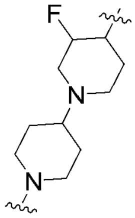 , 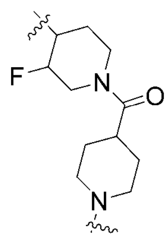 , 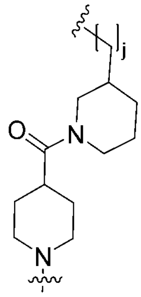 , 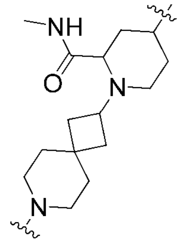 ,

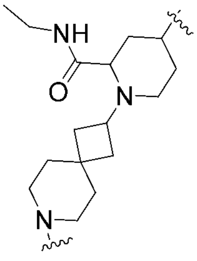 , 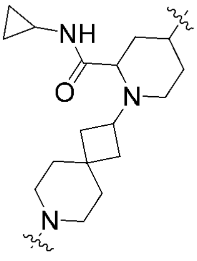 , 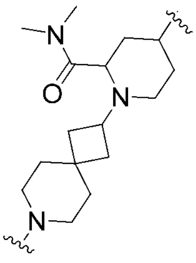 , 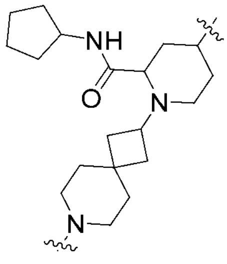 , 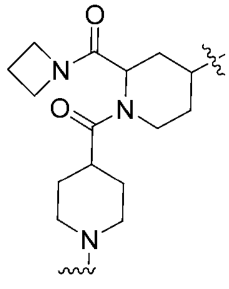 ,

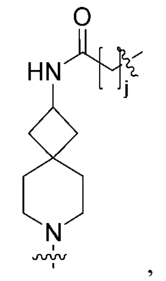 , 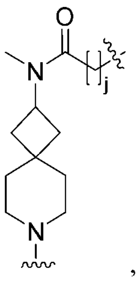 , 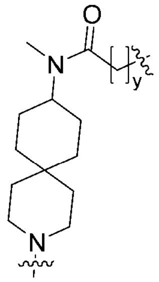 , 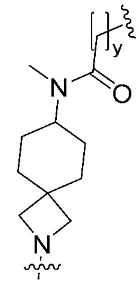 , 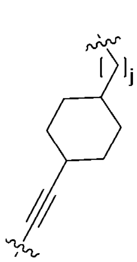 , 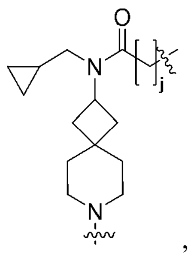 , 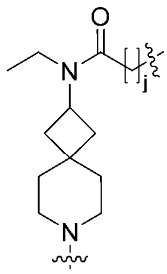 ,

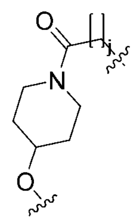, 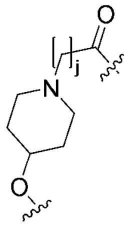, 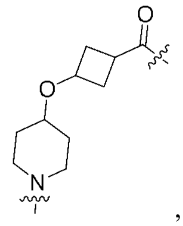, 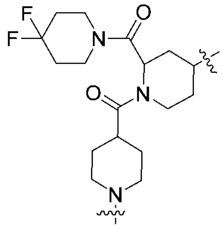, 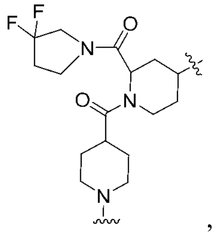, 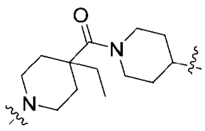,

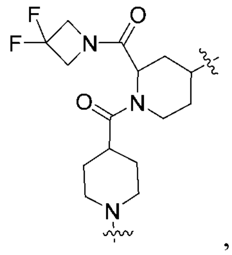, 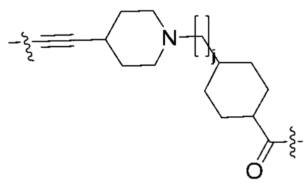, 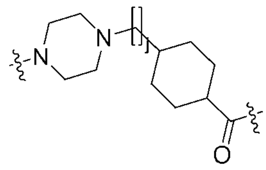, 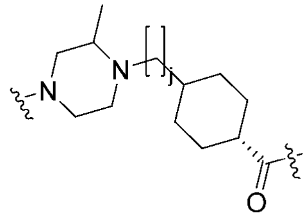,

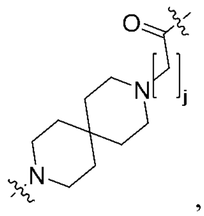, 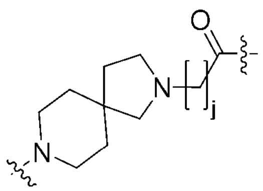, 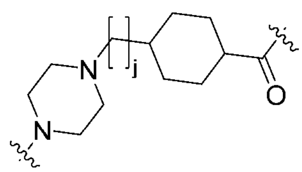, 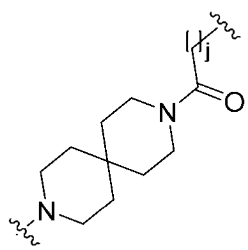,

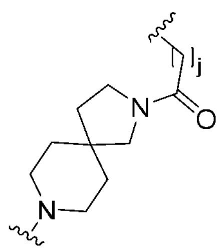, 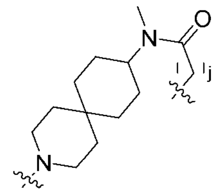, 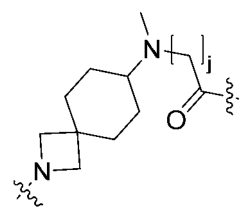, 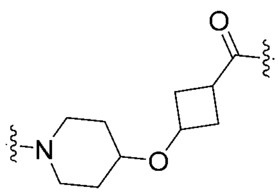,

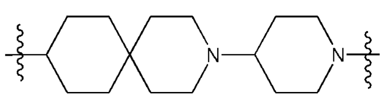, 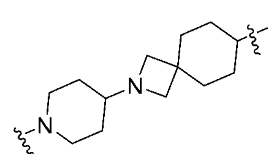, 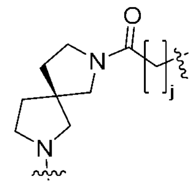, 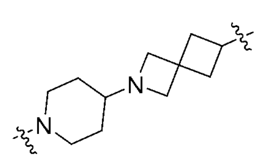,

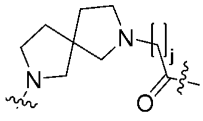, 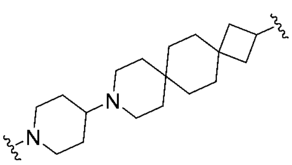, 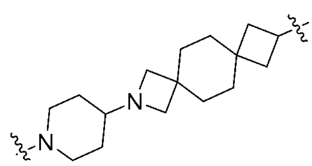, 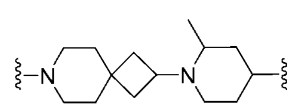,

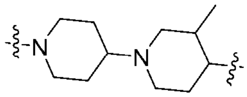, 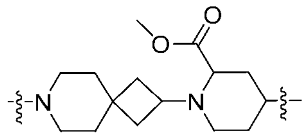, 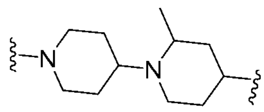, 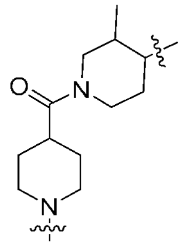,

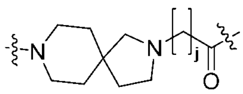, 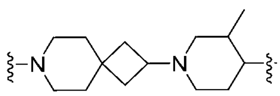, 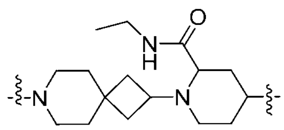, 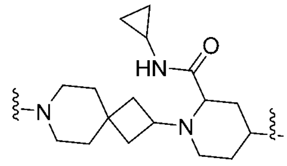,

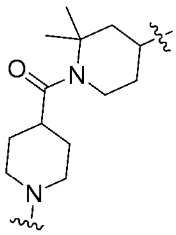, 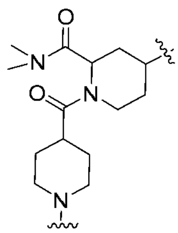, 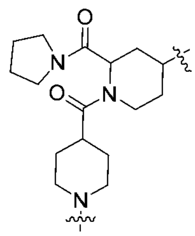, 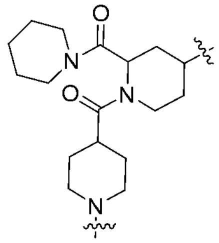, 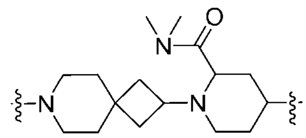,

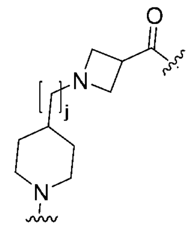, 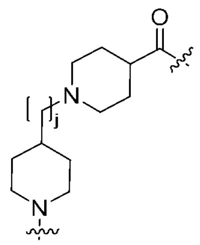, 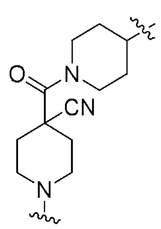, 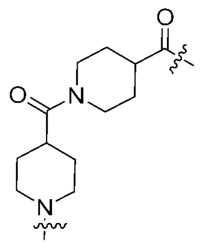, 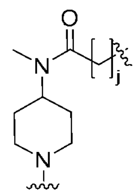, 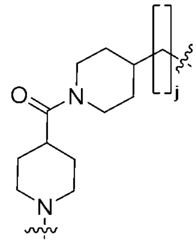,

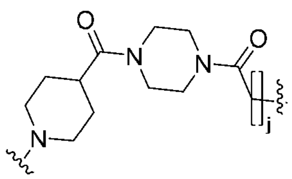, 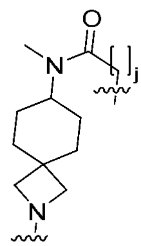, 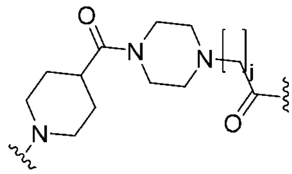, 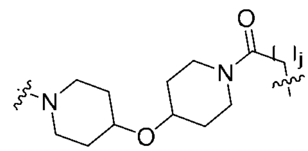,

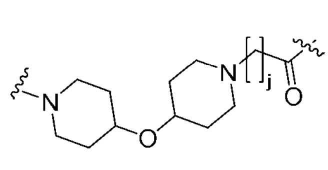, 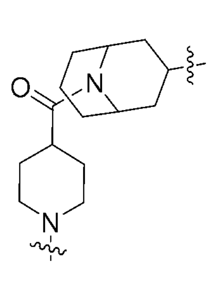, 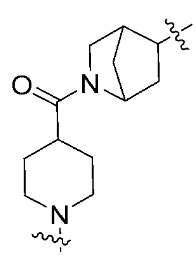, 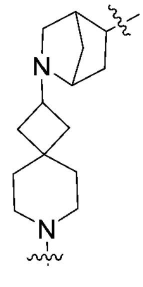, 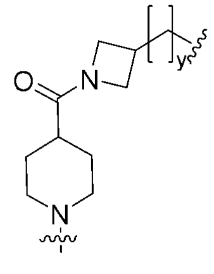,

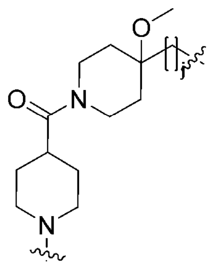 , 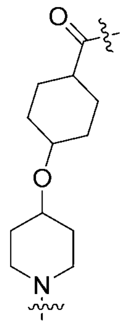 , 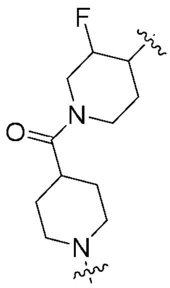 , 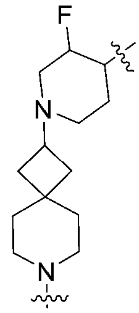 , 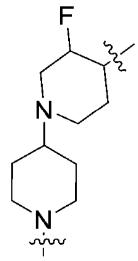 , 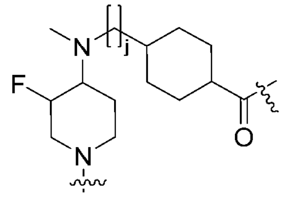 ,

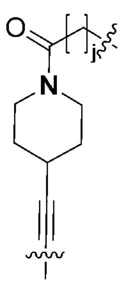 , 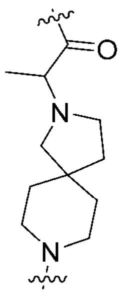 , 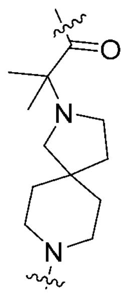 , 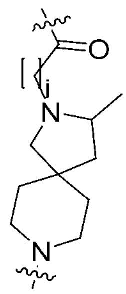 , 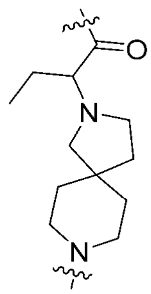 , 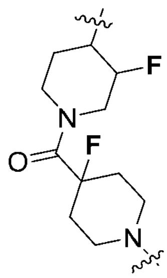 , 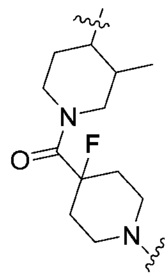 ,

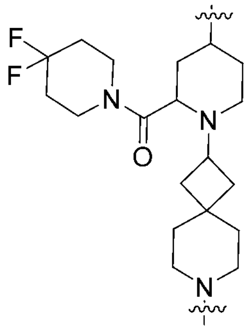 , 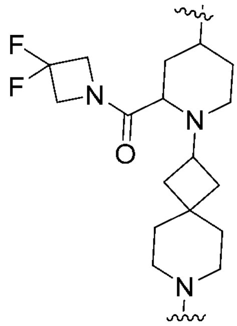 , 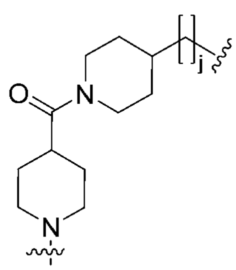 , 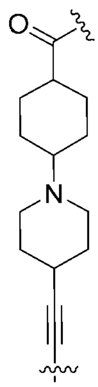 , 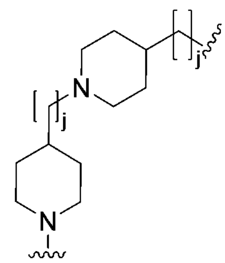 ,

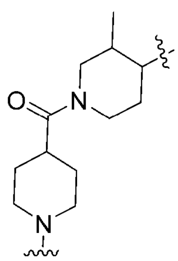 , 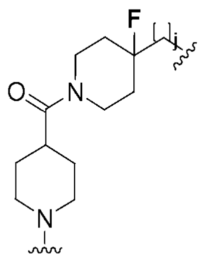 , 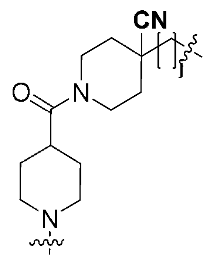 , 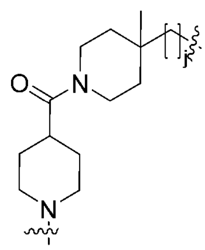 , 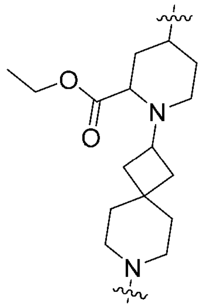 , 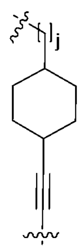 ,

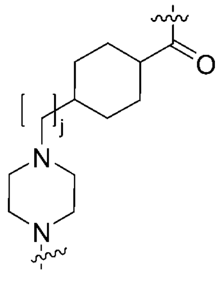 , 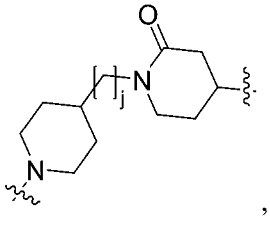 , 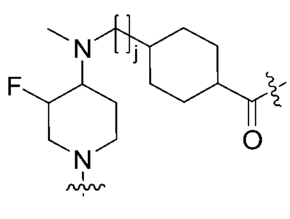 , 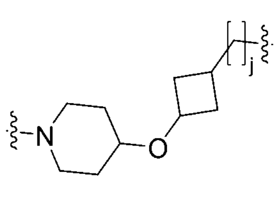 ,

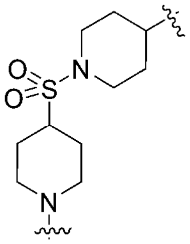 , 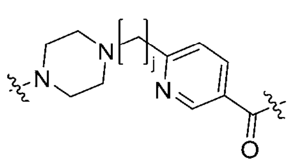 , 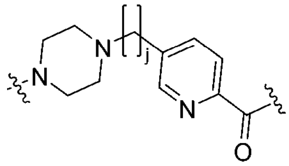 , 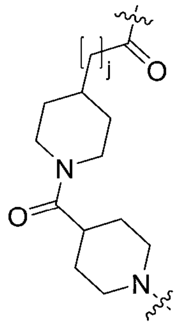 ,

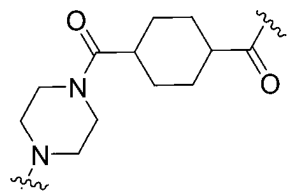 , 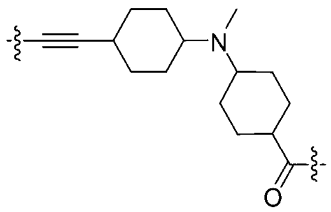 , 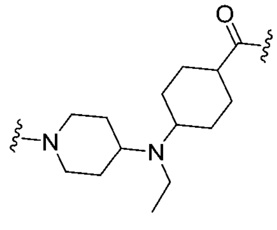 , 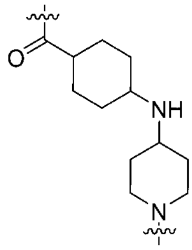 ,

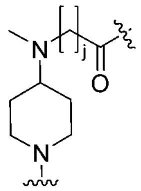 and 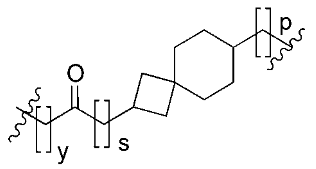 ;

wherein each occurrence of j is each independently 1, 2, 3, 4, 5, 6, 7, 8, 9, or 10;
k, s, p, and y are each independently 0, 1, 2, 3, 4, 5, 6, 7, 8, 9, or 10;

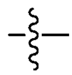

is a point of attachment to CLM or PTM;
L is preferably selected from a covalent bond, -$CH_2$-, -$(CH_2)_2$-, -$(CH_2)_3$-, -$(CH_2)_4$-, -$(CH_2)_5$-, -$(CH_2)_6$-, - $(CH_2)_7$-, -$(CH_2)_8$-, -NH-$CH_2$-, -NH-$(CH_2)_2$-, -NH-$(CH_2)_3$-, -NH-$(CH_2)_4$-, -NH-$(CH_2)_5$-, -NH-$(CH_2)_6$-, -NH-$(CH_2)_7$-, -NH-$(CH_2)_8$-, -CO-NH-$CH_2$-, -CO-NH-$(CH_2)_2$-, -CO-NH-$(CH_2)_3$-, -CO-NH-$(CH_2)_4$-, -CO-NH-$(CH_2)_5$-, -CO-NH-$(CH_2)_6$-, -CO-NH-$(CH_2)_7$-, -CO-NH-$(CH_2)_8$-, -$CH_2$-NH-, -$(CH_2)_2$-NH-, -$(CH_2)_3$-NH-, -$(CH_2)_4$-NH-, -$(CH_2)_5$-NH-, - $(CH_2)_6$-NH-, -$(CH_2)_7$-NH-, -$(CH_2)_8$-NH-, -NH-$CH_2$-NH-, -NH-$(CH_2)_2$-NH-, -NH-$(CH_2)_3$-NH-, -NH-$(CH_2)_4$-NH-, - NH-$(CH_2)_5$-NH-, -NH-$(CH_2)_6$-NH-, -NH-$(CH_2)_7$-NH-, -NH-$(CH_2)_8$-NH-, -($CH_2$-$CH_2$-O)-$CH_2$-$CH_2$-, -($CH_2$-$CH_2$-$O)_2$-$CH_2$-$CH_2$-, -($CH_2$-$CH_2$-$O)_3$-$CH_2$-$CH_2$-, -NH-($CH_2$-$CH_2$-O)-$CH_2$-$CH_2$-, -NH-($CH_2$-$CH_2$-$O)_2$-$CH_2$-$CH_2$-, -NH-($CH_2$-$CH_2$-$O)_3$-$CH_2$-$CH_2$-, -CO-NH-($CH_2$-$CH_2$-O)-$CH_2$-$CH_2$-, -CO-NH-($CH_2$-$CH_2$-$O)_2$-$CH_2$-$CH_2$-, -CO-NH-($CH_2$-$CH_2$-$O)_3$-$CH_2$-$CH_2$-, -($CH_2$-$CH_2$-O)-$CH_2$-$CH_2$-NH-, -($CH_2$-$CH_2$-$O)_2$-$CH_2$-$CH_2$-NH-, -($CH_2$-$CH_2$-$O)_3$-$CH_2$-$CH_2$-NH-, -NH-($CH_2$-$CH_2$-O)-$CH_2$-$CH_2$-NH-, -NH-($CH_2$-$CH_2$-$O)_2$-$CH_2$-$CH_2$-NH-, -NH-($CH_2$-$CH_2$-$O)_3$-$CH_2$-$CH_2$-NH-, - CO-NH-($CH_2$-$CH_2$-O)-$CH_2$-$CH_2$-NH-, -CO-NH-($CH_2$-$CH_2$-$O)_2$-$CH_2$-$CH_2$-NH-, -CO-NH-($CH_2$-$CH_2$-$O)_3$-$CH_2$-$CH_2$-NH-, -$CH_2$-$CH_2$-(O-$CH_2$-$CH_2$)-, -$CH_2$-$CH_2$-(O-$CH_2$-$CH_2)_2$-, -$CH_2$-$CH_2$-(O-$CH_2$-$CH_2)_3$-, -NH-$CH_2$-$CH_2$-(O-$CH_2$-$CH_2$)-, -NH-$CH_2$-$CH_2$-(O-$CH_2$-$CH_2)_2$-, -NH-$CH_2$-$CH_2$-(O-$CH_2$-$CH_2)_3$-, -CO-NH-$CH_2$-$CH_2$-(O-$CH_2$-$CH_2$)-, -CO-NH-$CH_2$-$CH_2$-(O-$CH_2$-$CH_2)_2$-, -CO-NH-$CH_2$-$CH_2$-(O-$CH_2$-$CH_2)_3$-, -$CH_2$-$CH_2$-(O-$CH_2$-$CH_2$)-NH-, -$CH_2$-$CH_2$-(O-$CH_2$-$CH_2)_2$-NH-, -$CH_2$-$CH_2$-(O-$CH_2$-$CH_2)_3$-NH-, -NH-$CH_2$-$CH_2$-(O-$CH_2$-$CH_2$)-NH-, -NH-$CH_2$-$CH_2$-(O-$CH_2$-$CH_2)_2$-NH-, -NH-$CH_2$-$CH_2$-(O-$CH_2$-$CH_2)_3$-NH-, -NH-$CH_2$-$CH_2$-O-$CH_2$-$CH_2$-CO-, -CO-$CH_2$-$CH_2$-O-$CH_2$-$CH_2$-NH-, -NH-$(CH_2)_4$-CO-, -NH-$(CH_2)_5$-CO-, -NH-$(CH_2)_6$-CO-, -CO-$(CH_2)_4$-NH-, -CO-$(CH_2)_5$-NH-, -CO-$(CH_2)_6$-NH-, -NH-($CH_2$-$CH_2$-O)-$(CH_2)_3$-, -NH-($CH_2$-$CH_2$-O)-$(CH_2)_4$-, -NH-($CH_2$-$CH_2$-O)-$(CH_2)_5$-, -NH-($CH_2$-$CH_2$-O)-$(CH_2)_6$-, - $(CH_2)_3$-(O-$CH_2$-$CH_2$)-NH-, -$(CH_2)_4$-(O-$CH_2$-$CH_2$)-NH-, -$(CH_2)_5$-(O-$CH_2$-$CH_2$)-NH-, -$(CH_2)_6$-(O-$CH_2$-$CH_2$)-NH-, - $CH_2$-$CH_2$-O-$(CH_2)_2$-CO-, -$CH_2$-$CH_2$-O-$(CH_2)_3$-CO-, -$CH_2$-$CH_2$-O-$(CH_2)_4$-CO-, -CO-$(CH_2)_2$-O-$CH_2$-$CH_2$-,

$-CO-(CH_2)_3-O-CH_2-CH_2-$, $-CO-(CH_2)_4-O-CH_2-CH_2-$, $-CO-(CH_2)_2-$, $-CO-(CH_2)_3-$, $-CO-(CH_2)_4-$, $-CO-(CH_2)_5-$, $-CO-(CH_2)_6-$, $-(CH_2)_2-CO-$, $-(CH_2)_3-CO-$, $-(CH_2)_4-CO-$, $-(CH_2)_5-CO-$, $-(CH_2)_6-CO-$, $-CO-(CH_2)_2-CO-$, $-CO-(CH_2)_3-CO-$, $-CO-(CH_2)_4-CO-$, $-CO-(CH_2)_5-CO-$, $-CO-(CH_2)_6-CO-$, $-CH_2-CO-CH_2-$, $-CH_2-CO-(CH_2)_2-$, $-CH_2-CO-(CH_2)_3-$, $-CH_2-CO-(CH_2)_4-$, $-(CH_2)_2-CO-CH_2-$, $-(CH_2)_2-CO-(CH_2)_2-$, $-(CH_2)_2-CO-(CH_2)_3-$, $-(CH_2)_2-CO-(CH_2)_4-$, $-(CH_2)_3-CO-CH_2-$, $-(CH_2)_3-CO-(CH_2)_2-$, $-(CH_2)_3-CO-(CH_2)_3-$, $-(CH_2)_3-CO-(CH_2)_4-$, $-(CH_2)_4-CO-CH_2-$, $-(CH_2)_4-CO-(CH_2)_2-$, $-(CH_2)_4-CO-(CH_2)_3-$, $-(CH_2)_4-CO-(CH_2)_4-$, $-CH_2-O-CH_2-$, $-CH_2-O-(CH_2)_2-$, $-CH_2-O-(CH_2)_3-$, $-CH_2-O-(CH_2)_4-$, $-(CH_2)_2-O-CH_2-$, $-(CH_2)_2-O-(CH_2)_2-$, $-(CH_2)_2-O-(CH_2)_3-$, $-(CH_2)_2-O-(CH_2)_4-$, $-(CH_2)_3-O-CH_2-$, $-(CH_2)_3-O-(CH_2)_2-$, $-(CH_2)_3-O-(CH_2)_3-$, $-(CH_2)_3-O-(CH_2)_4-$, $-(CH_2)_4-O-CH_2-$, $-(CH_2)_4-O-(CH_2)_2-$, $-(CH_2)_4-O-(CH_2)_3-$, $-(CH_2)_4-O-(CH_2)_4-$,

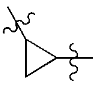, 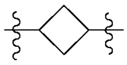, 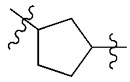, 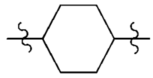, 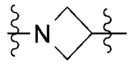, 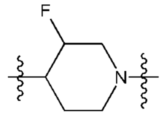, 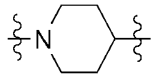,

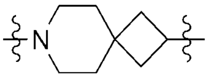, 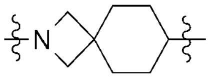, 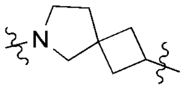, 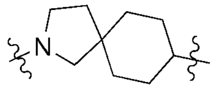, 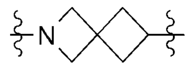,

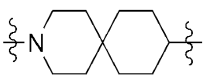, 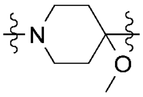, 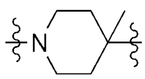, 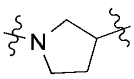, 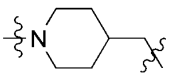, 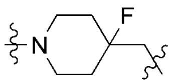,

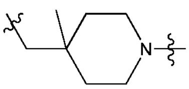, 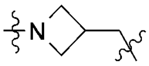, 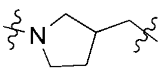, 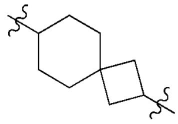, 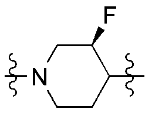, 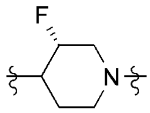,

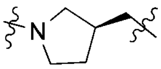, 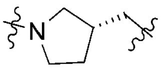, 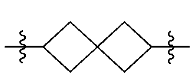, 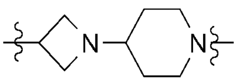, 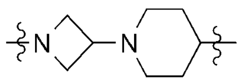,

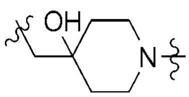, 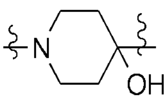, 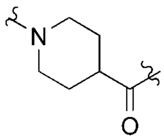, 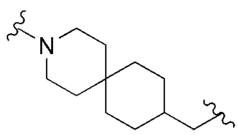, 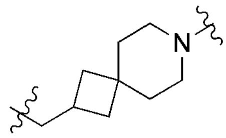,

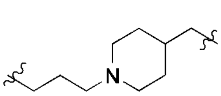, 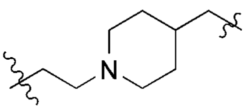, 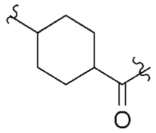, 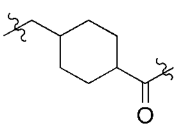, 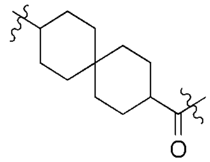,

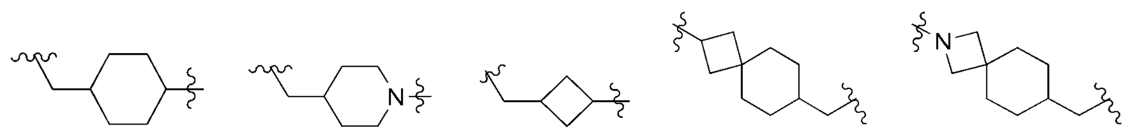, , , , ,

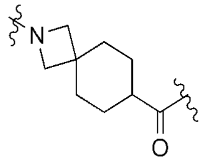 , 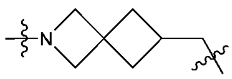 , 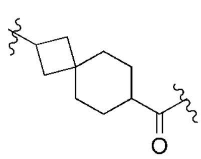 , 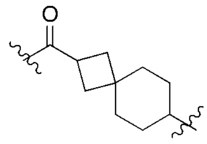 , 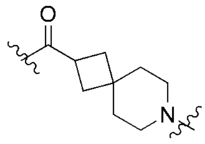 ,

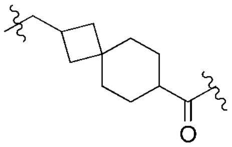 , 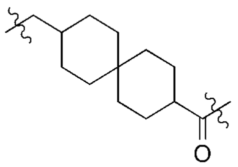 , 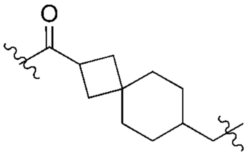 , 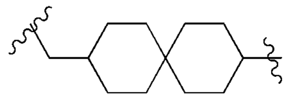 ,

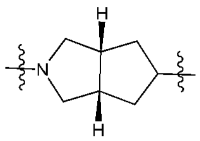 , 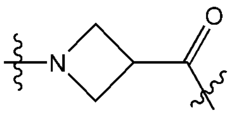 , 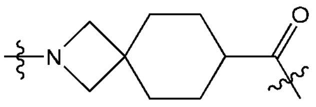 , 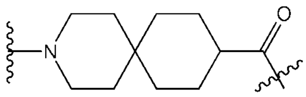 ,

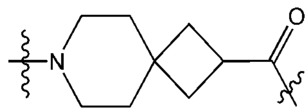 , 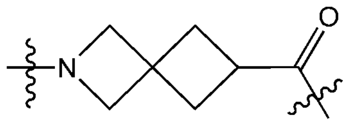 , 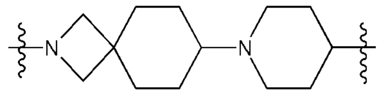 ,

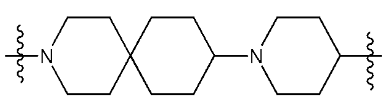 , 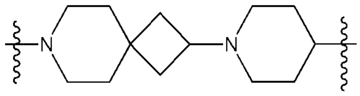 , 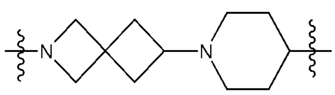 ,

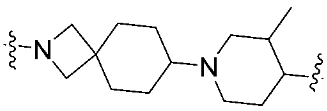 , 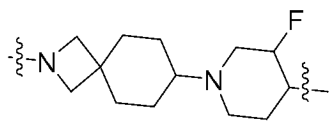 , 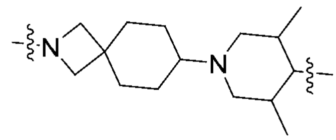 ,

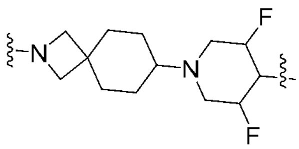 , 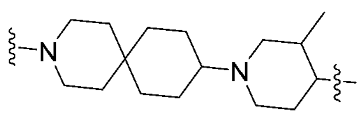 , 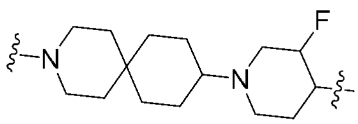 ,

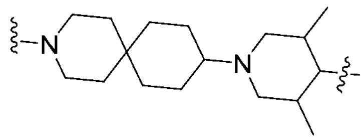 , 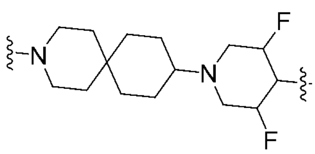 , 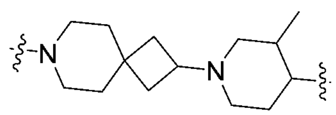 ,

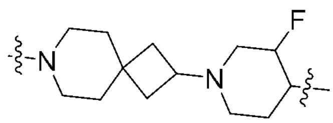 , 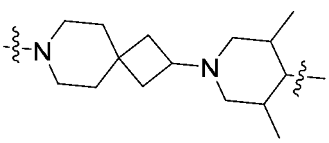 , 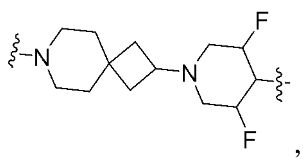 ,

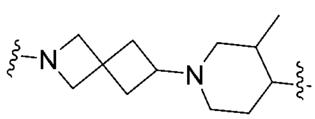, 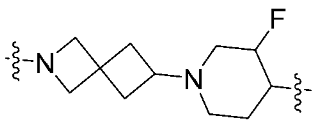, 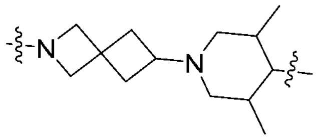,

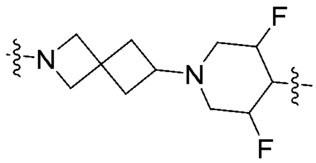, 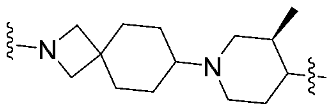, 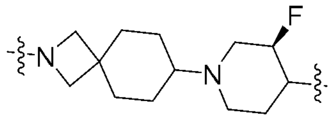,

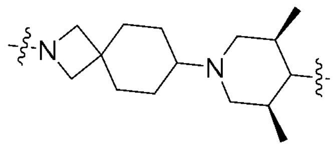, 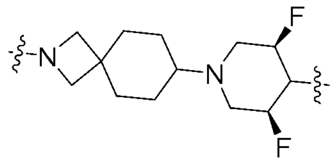, 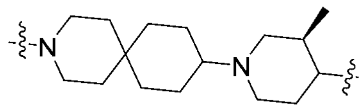,

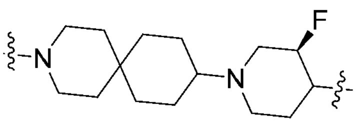, 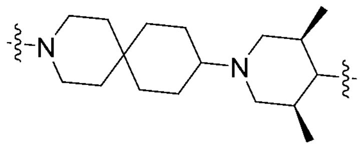, 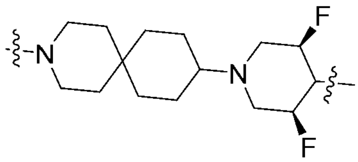,

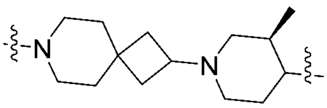, 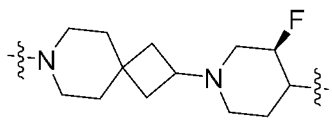, 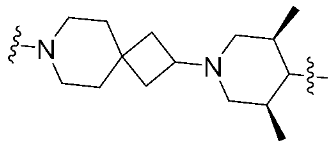,

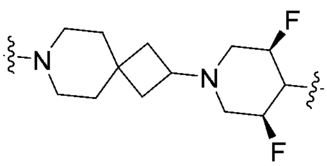, 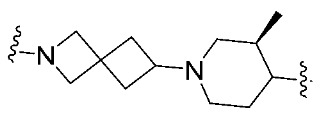, 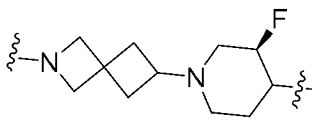,

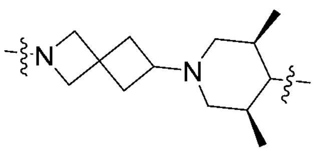, 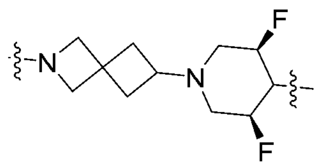, 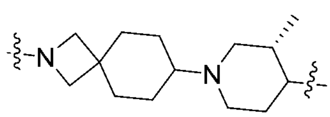,

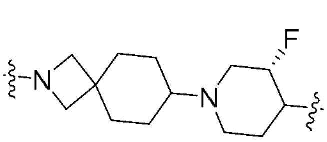, 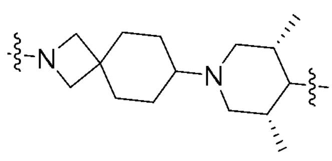, 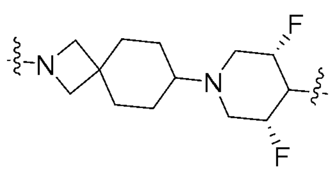,

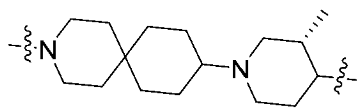, 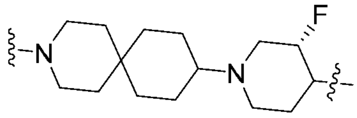, 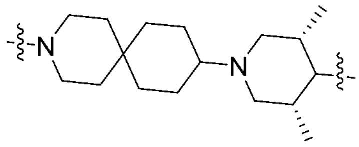,

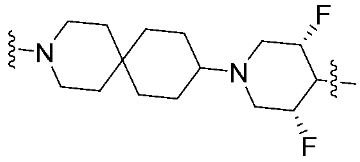, 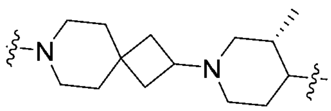, 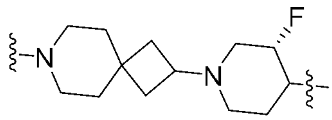,

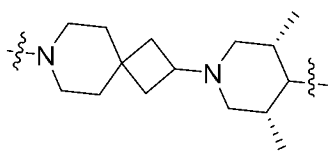, 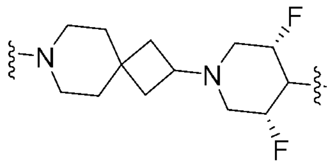, 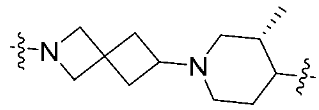,

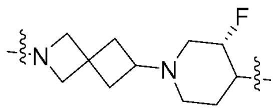, 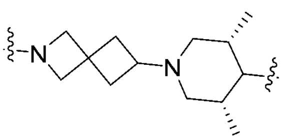, 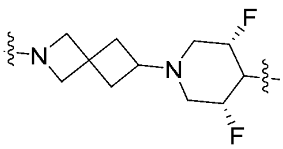, 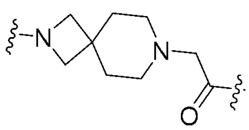,

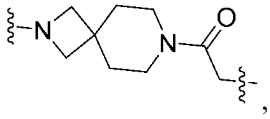, 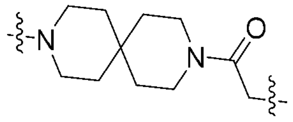, 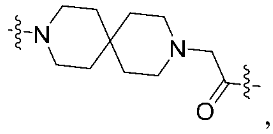, 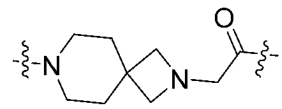,

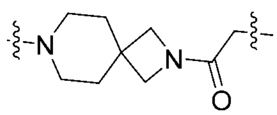, 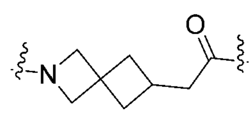, 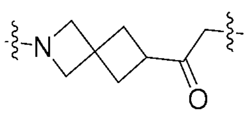, 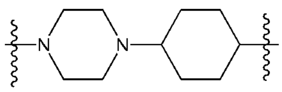,

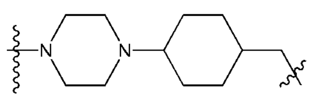, 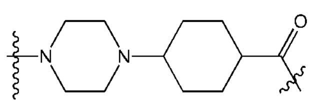, 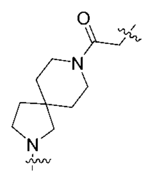, 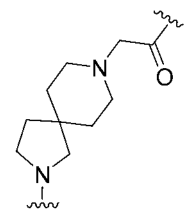,

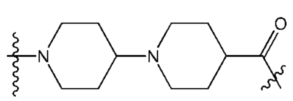, 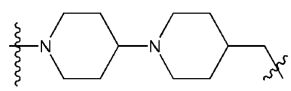, 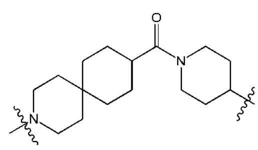, 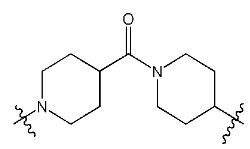,

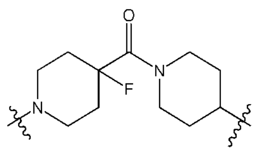, 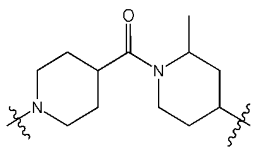, 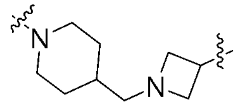, 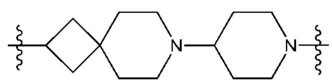,

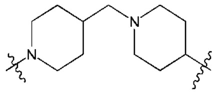, 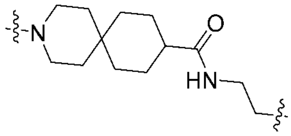, 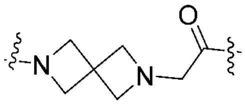, 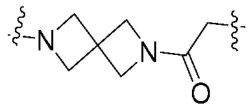,

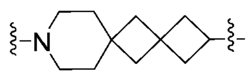 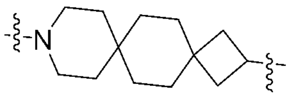, 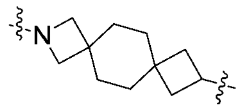 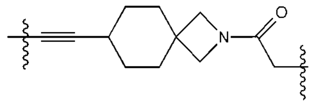,

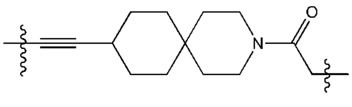, 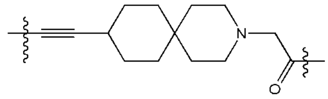, 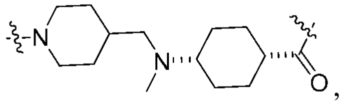,

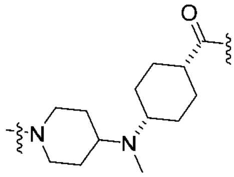, 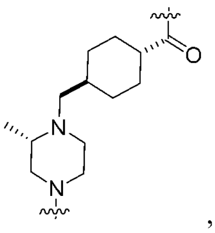, 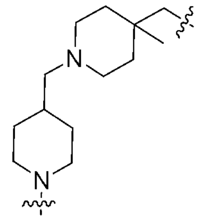, 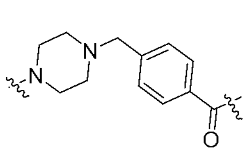, 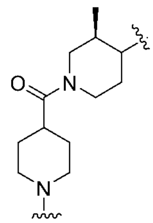,

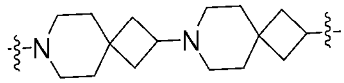, 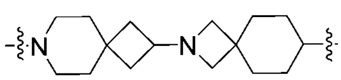, 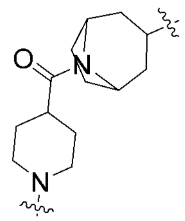, 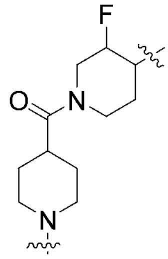,

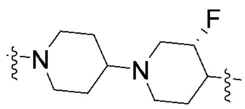, 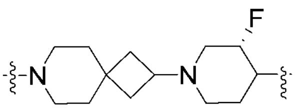, 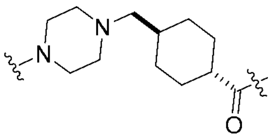, 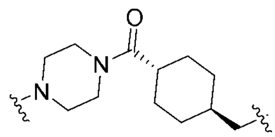,

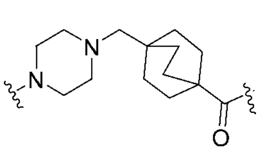, 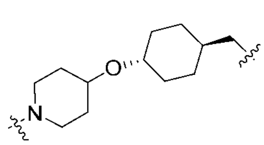, 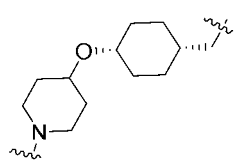, 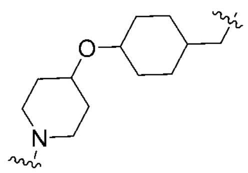,

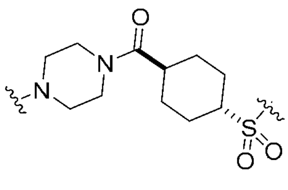, 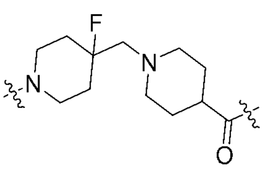, 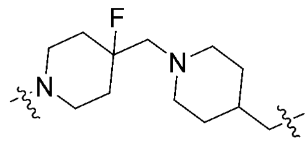, 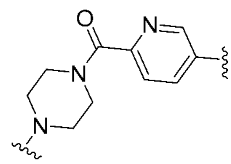,

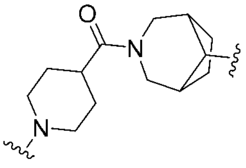, 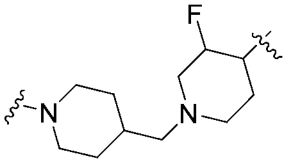, 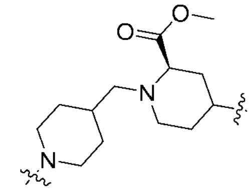, 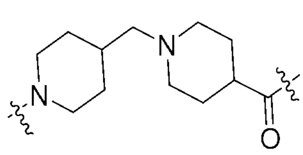,

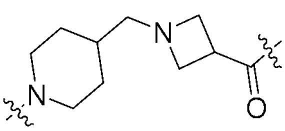, 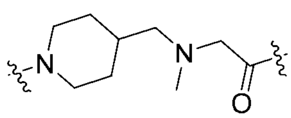, 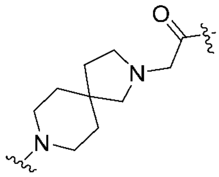 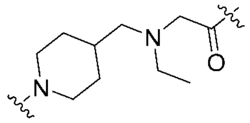

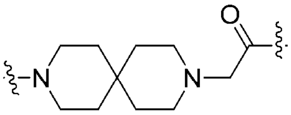 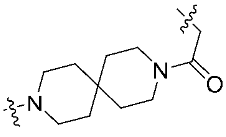 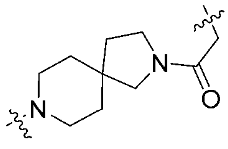 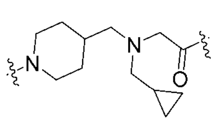, 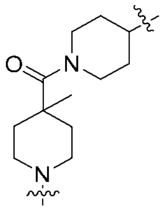,

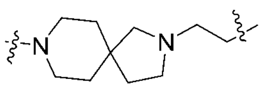, 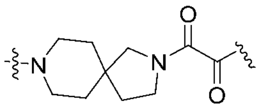, 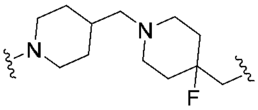, 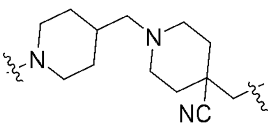,

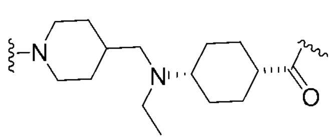 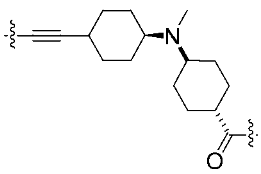, 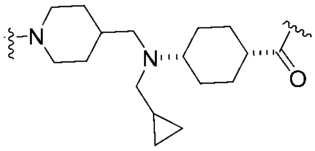, 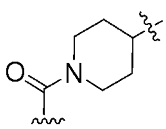,

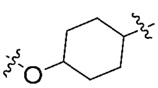, 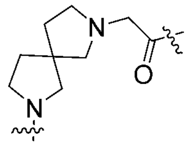, 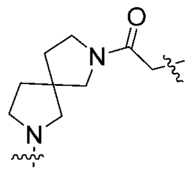, 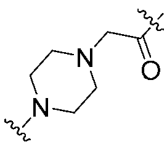, 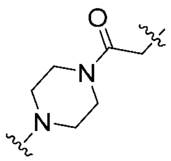, 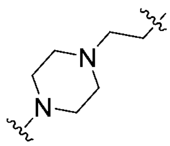,

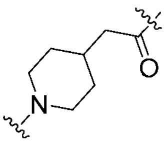, 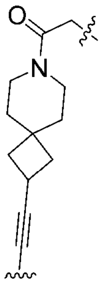, 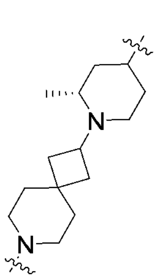, 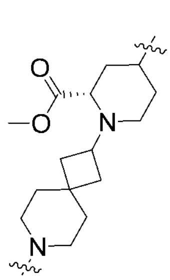, 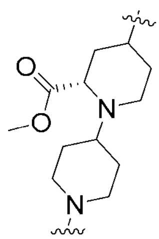, 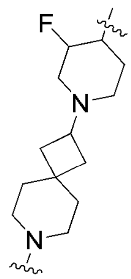, 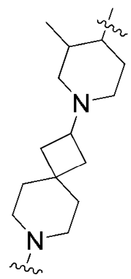,

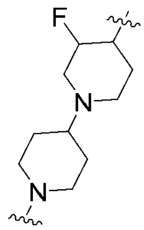, 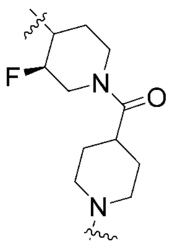, 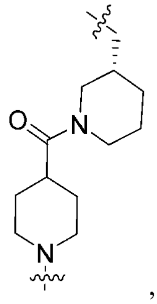, 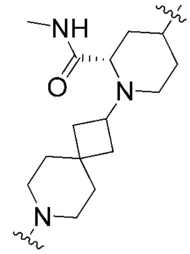, 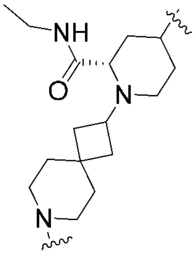, 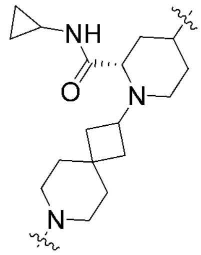,

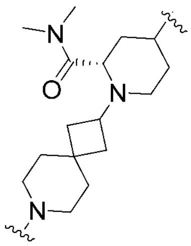, 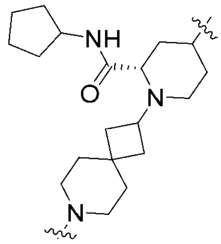, 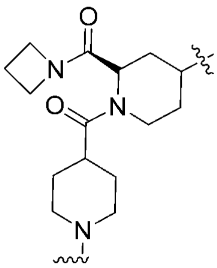, 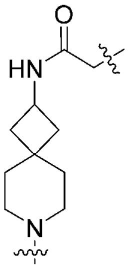, 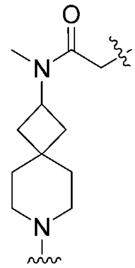, 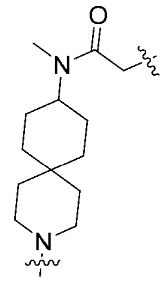,

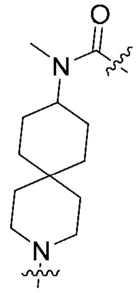, 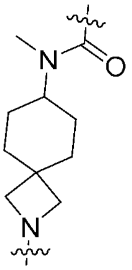, 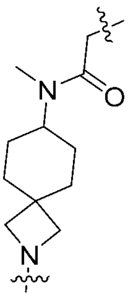, 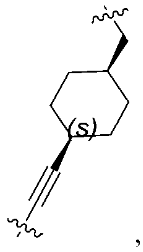, 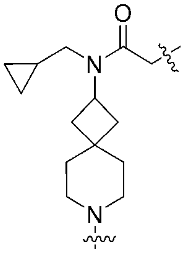, 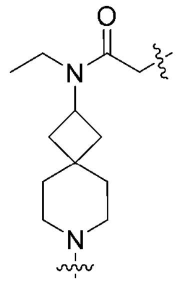, 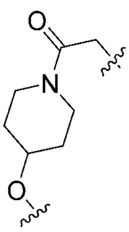,

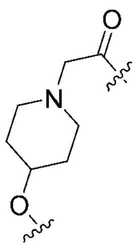, 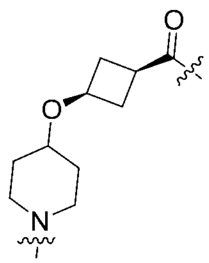, 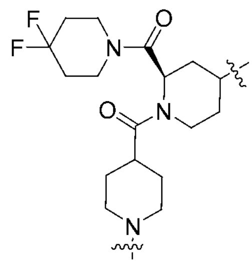, 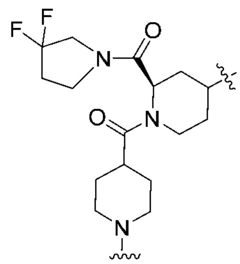, 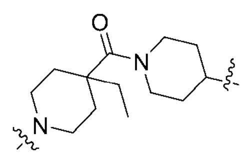,

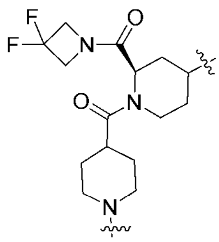, 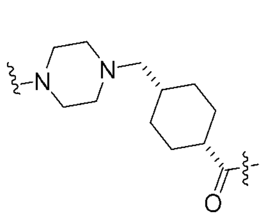, 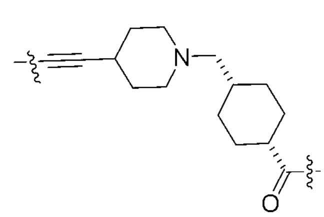, 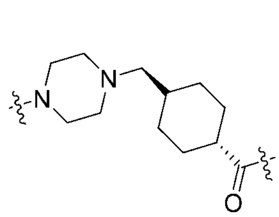,

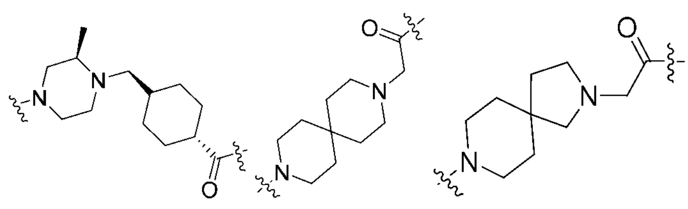 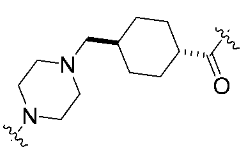 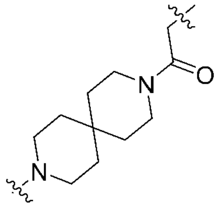

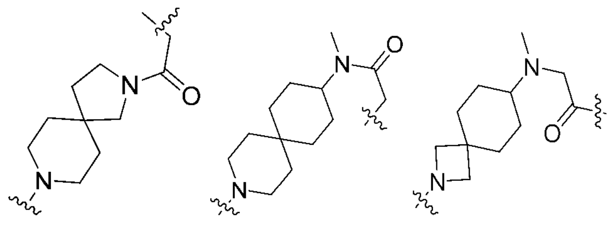, 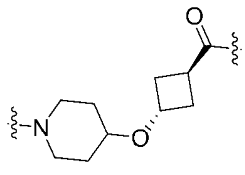, 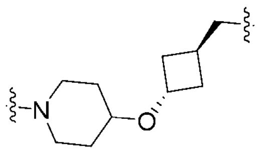,

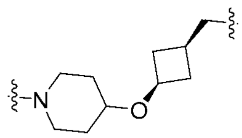, 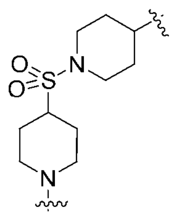, 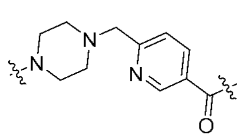, 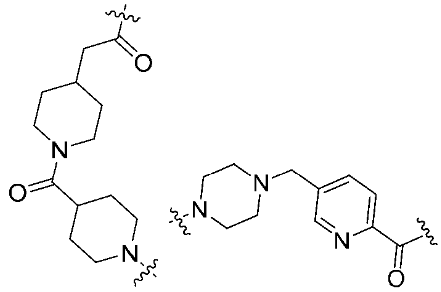,

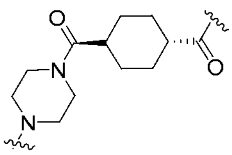, 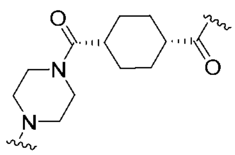, 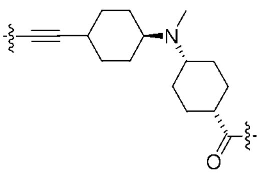, 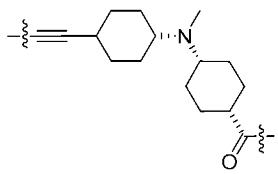,

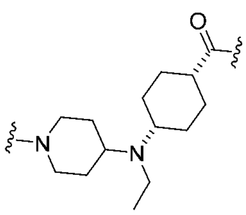, 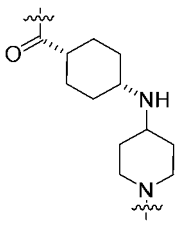, 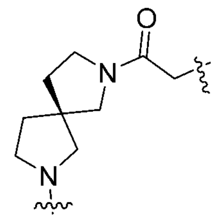, 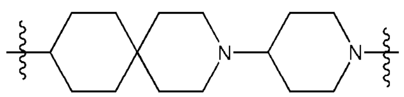,

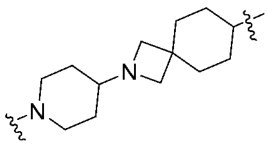, , 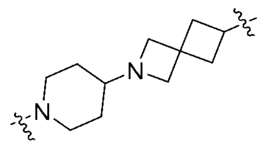, 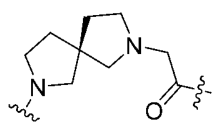, 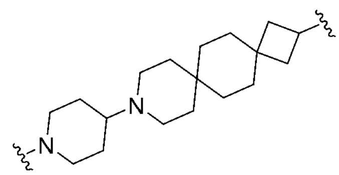,

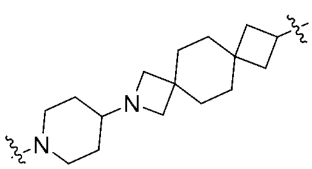, 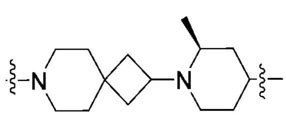, 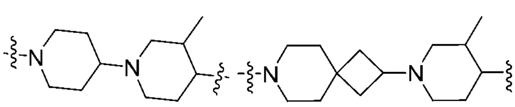

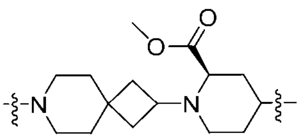 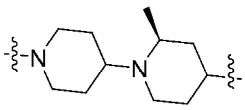, 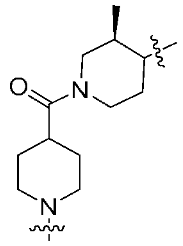, 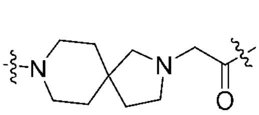,

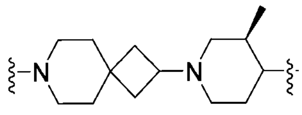, 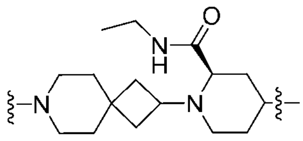, 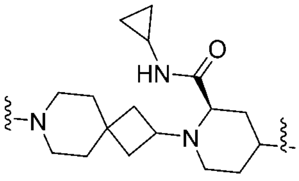, 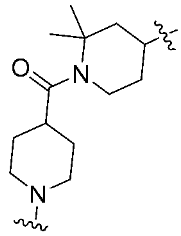,

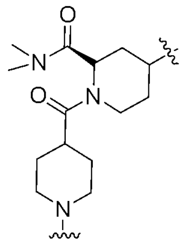, 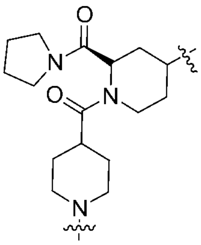, 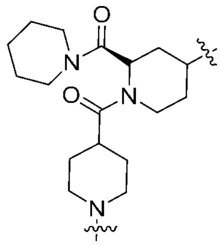, 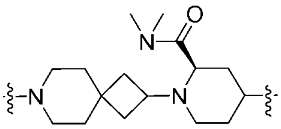, 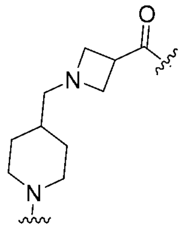,

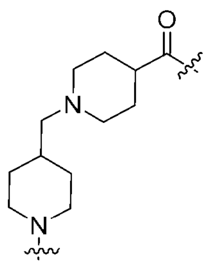, 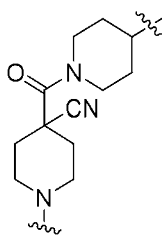, 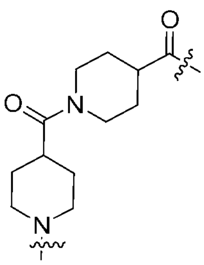, 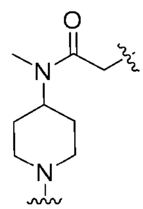, 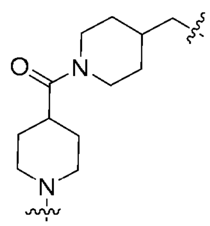,

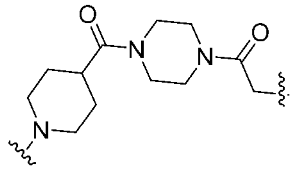, 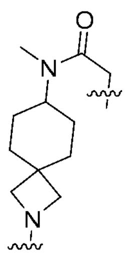, 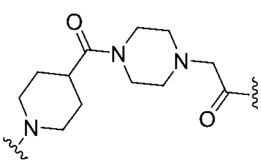, 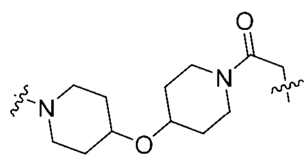,

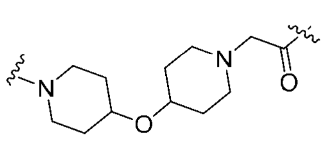, 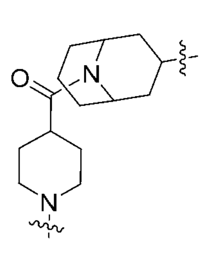, 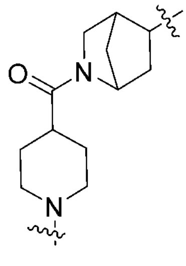, 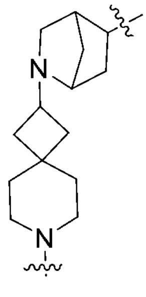, 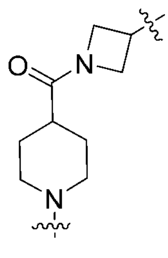,

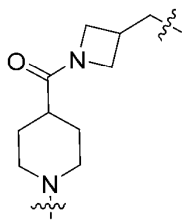, 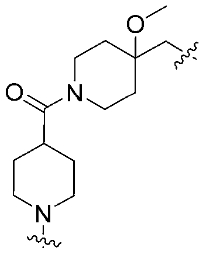, 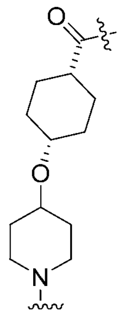, 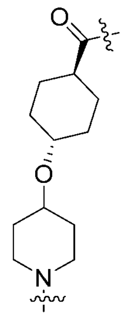, 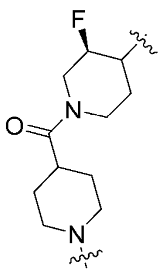, 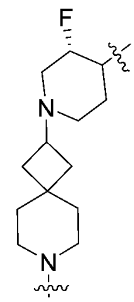, 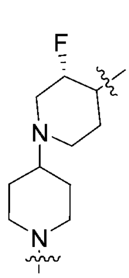,

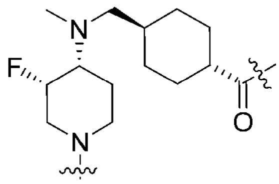, 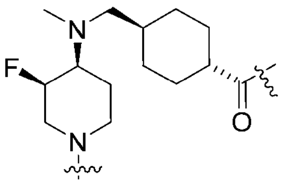, 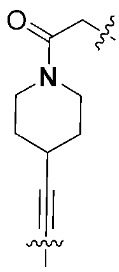, 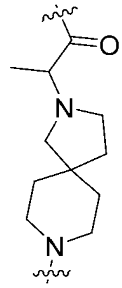, 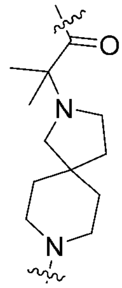, 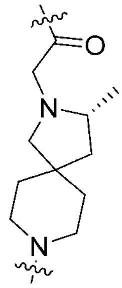,

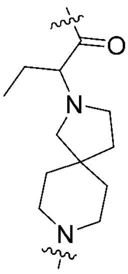, 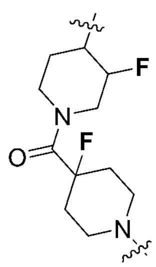, 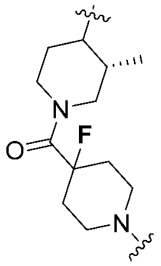, 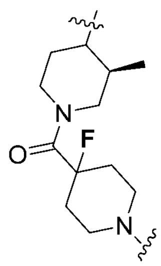, 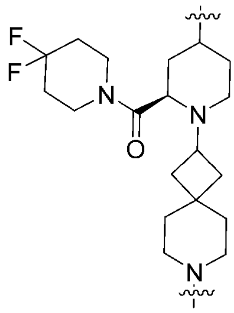, 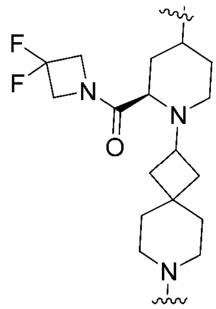,

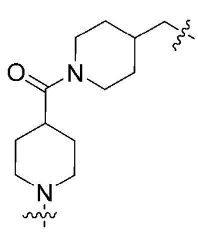, 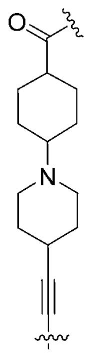, 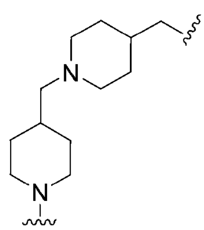, 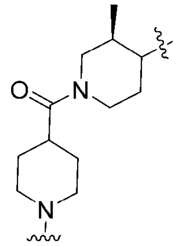, 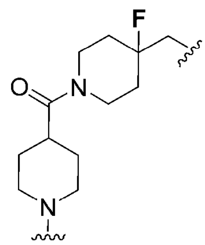, 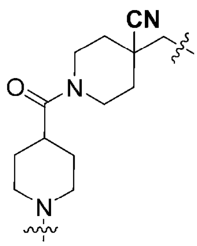,

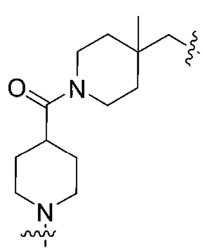, 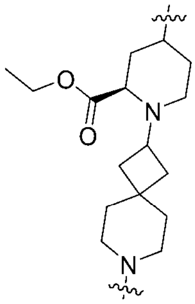, 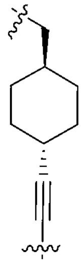, 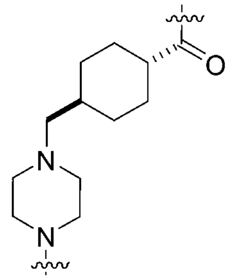, 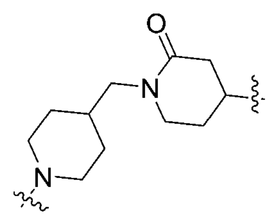,

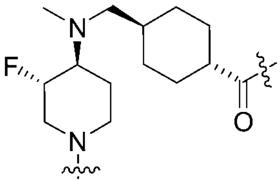, 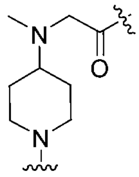 and 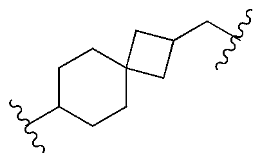.

**13.** The compound according to any one of claims 1 to 12, wherein the PTM is selected from the following structures:

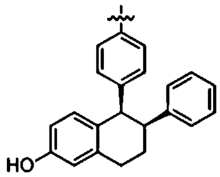, 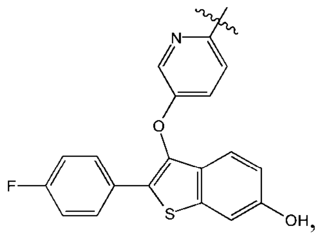, 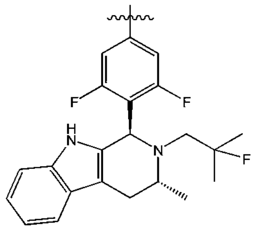, 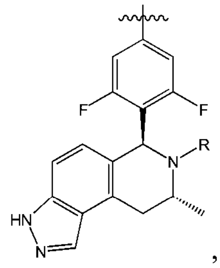, 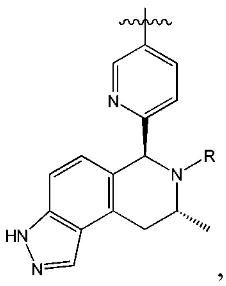, 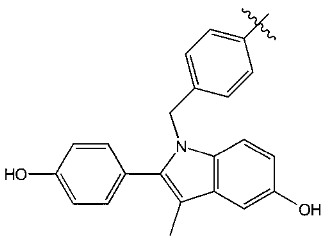, 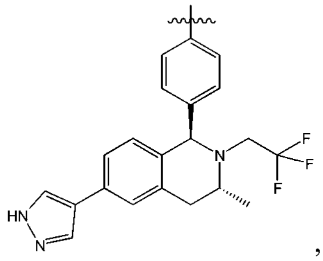, 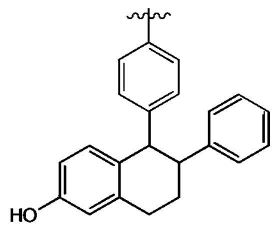, 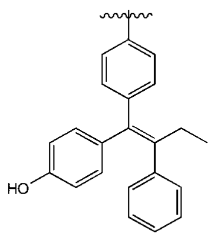, 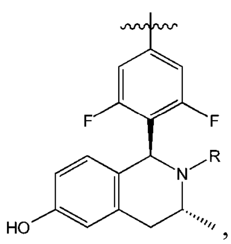, 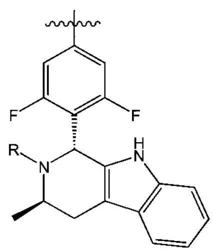, 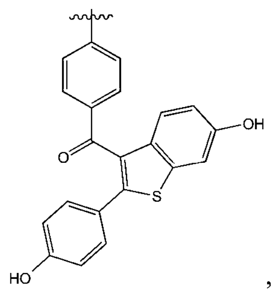, 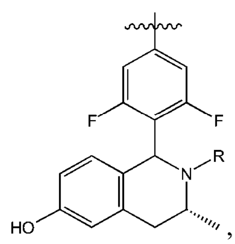,

and

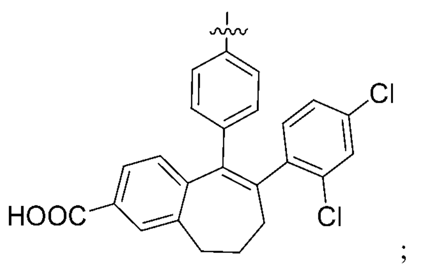;

R is selected from

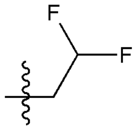, 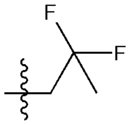, 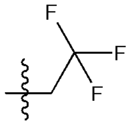 and 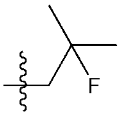.

**14.** The compound according to any one of claims 1 to 13, wherein the compound is selected from the following compounds:

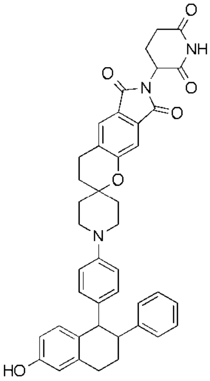 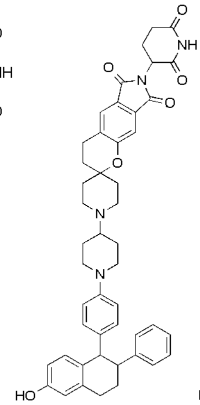 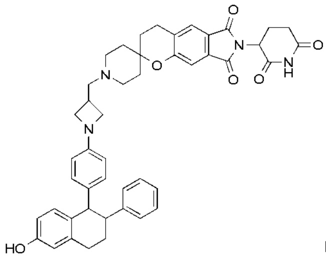 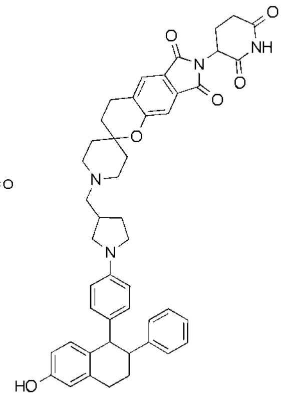

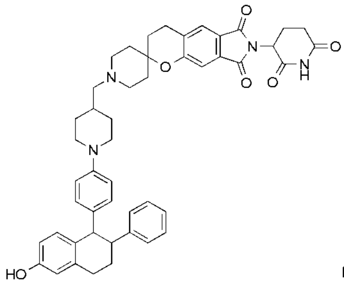 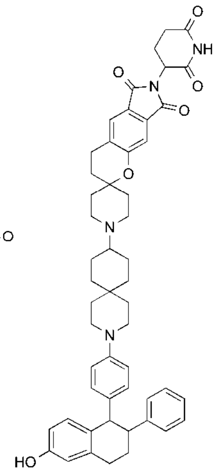 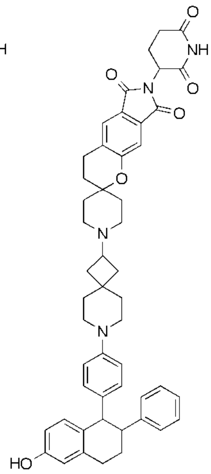 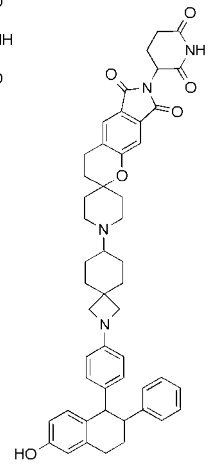 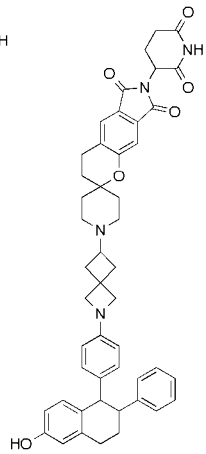

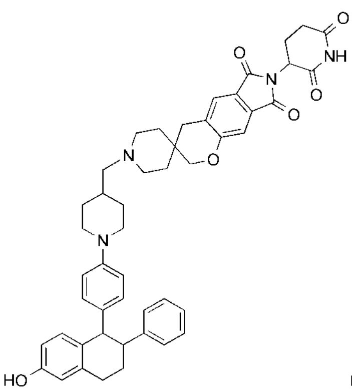 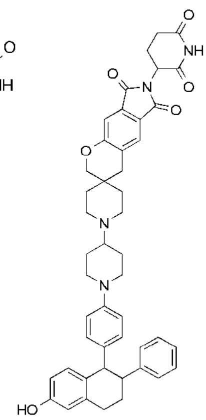 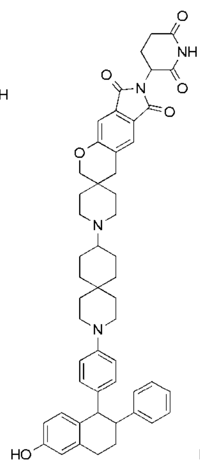 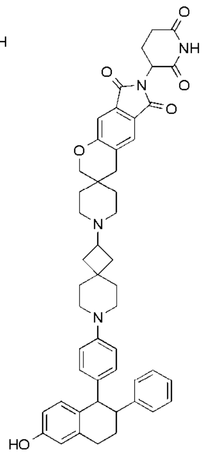 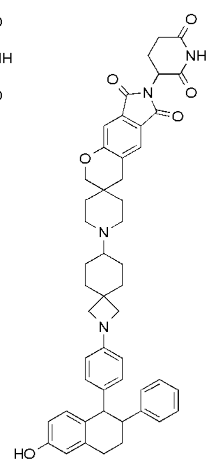

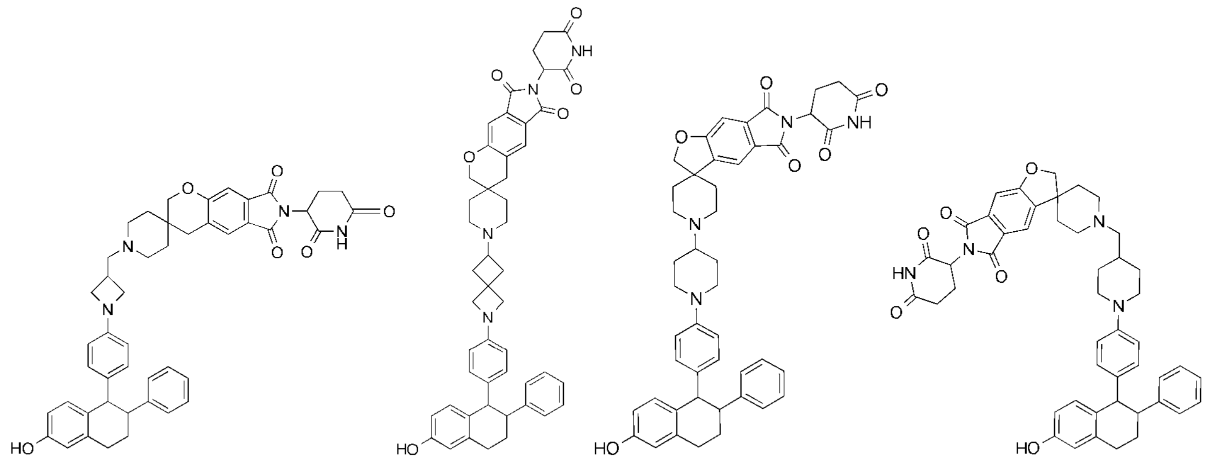

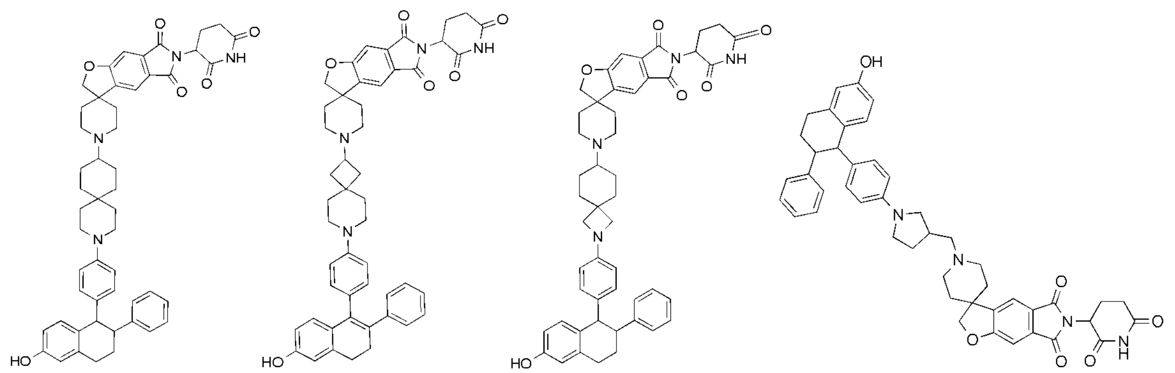

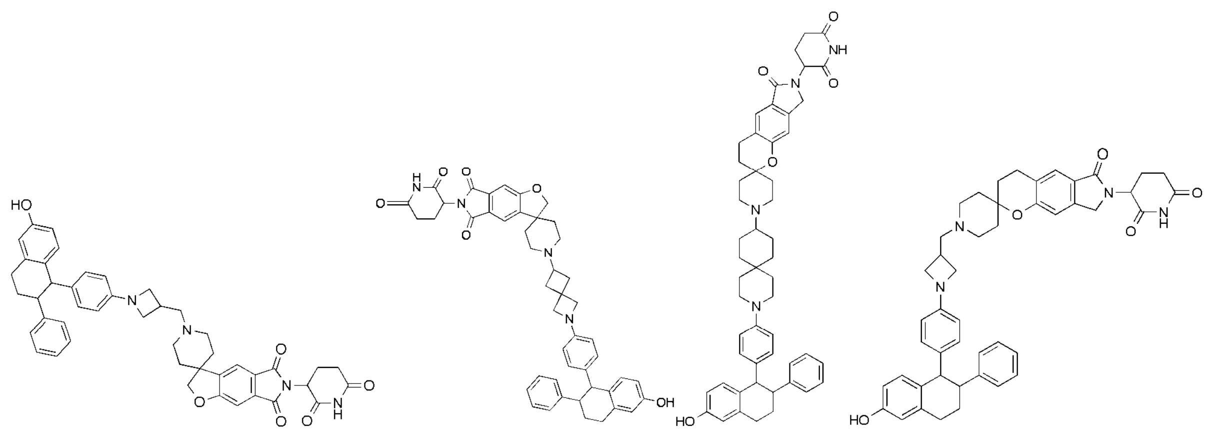

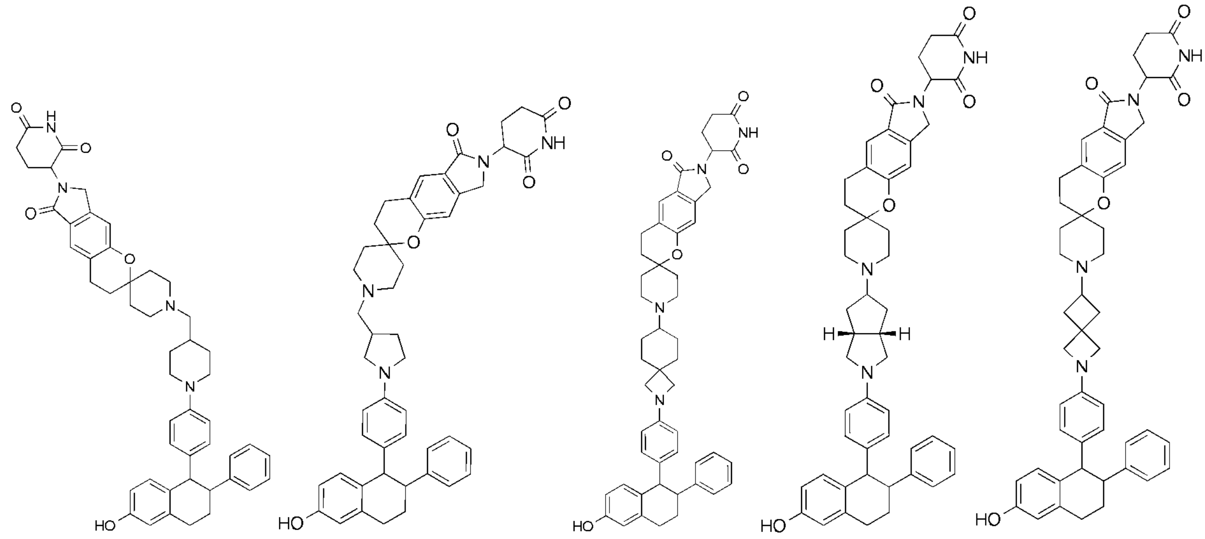

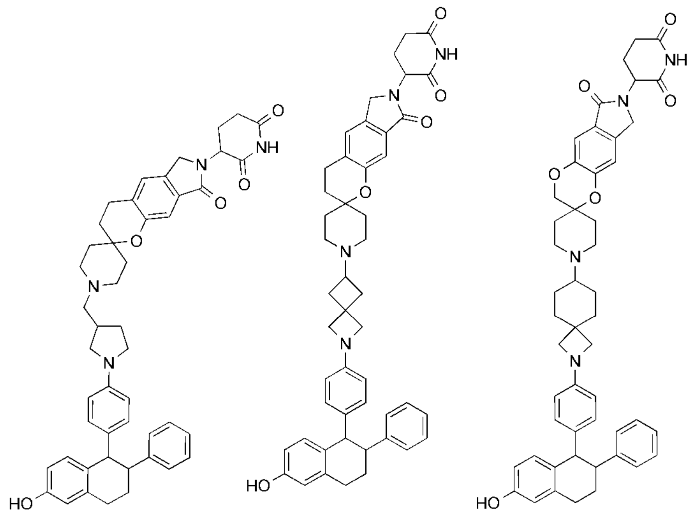

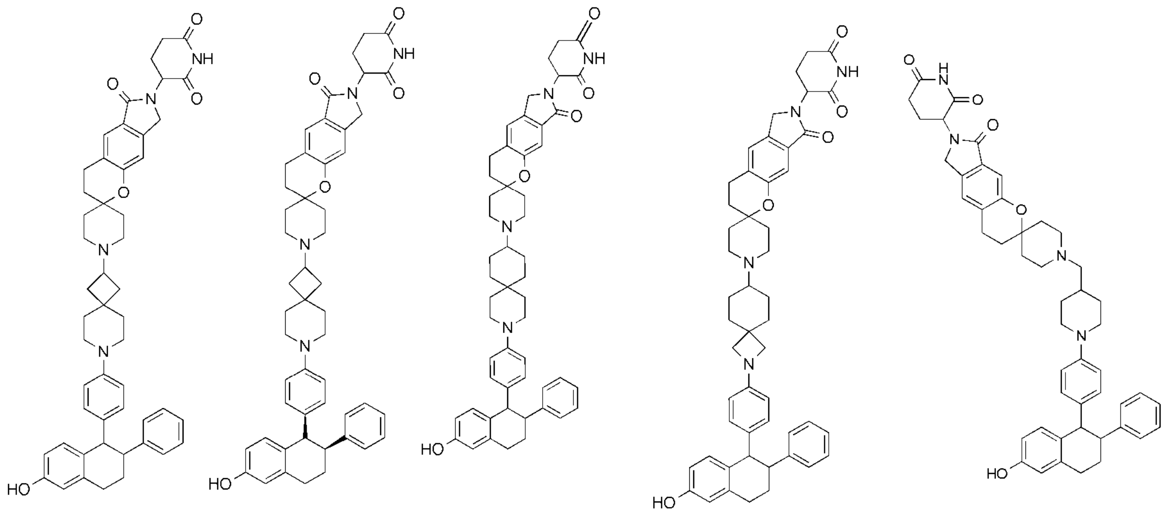

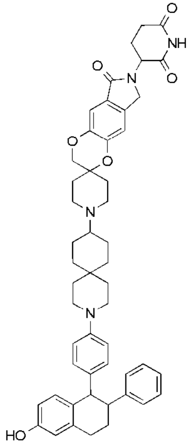
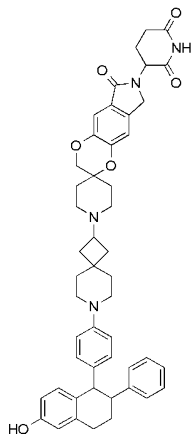
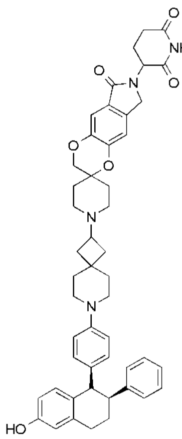
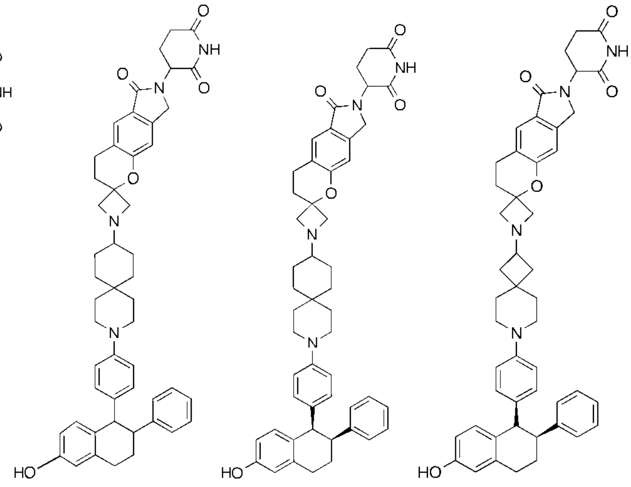

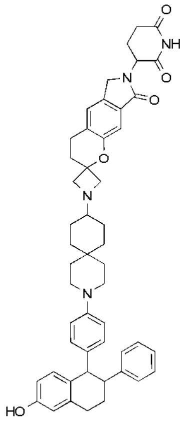
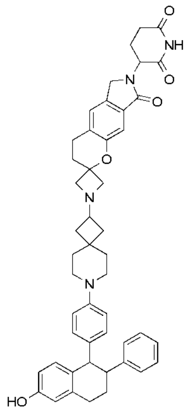
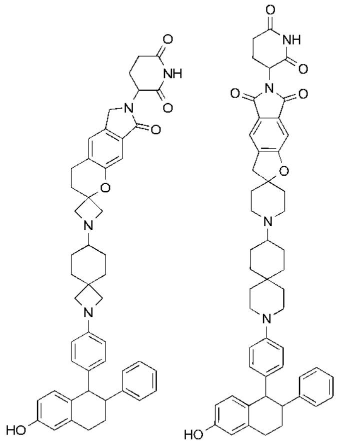
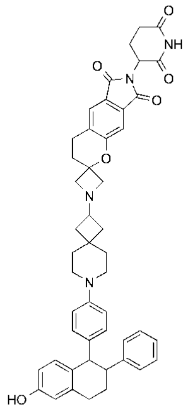
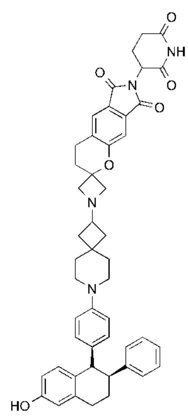

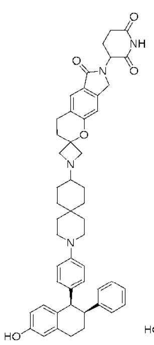
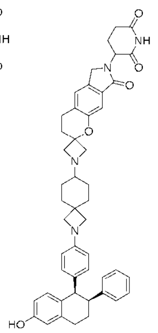
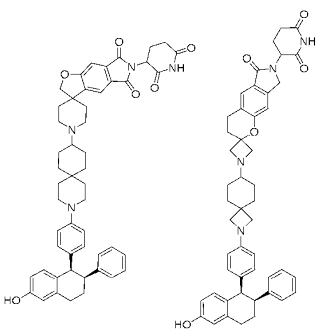
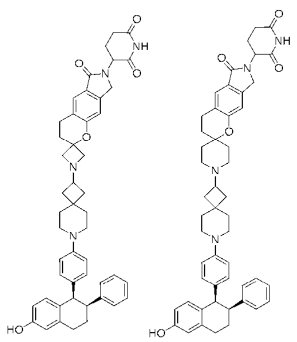
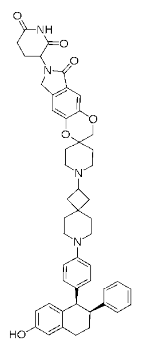
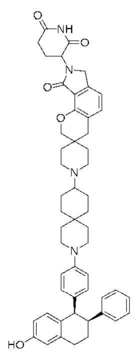

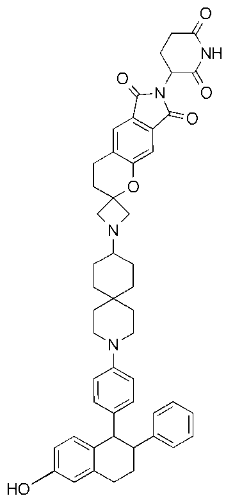 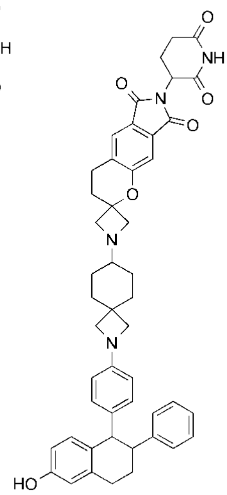 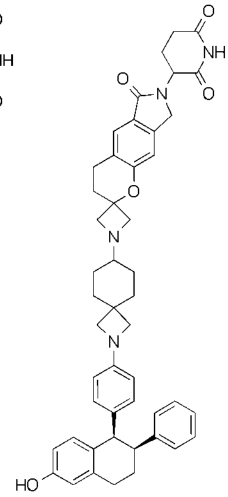 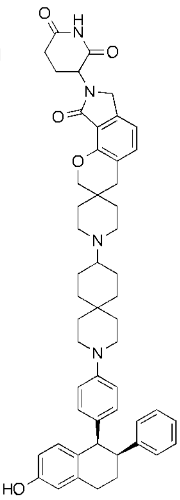 ,

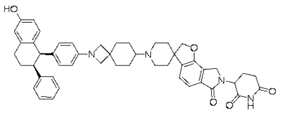 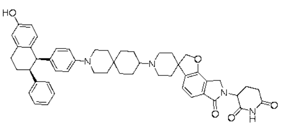 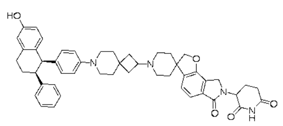 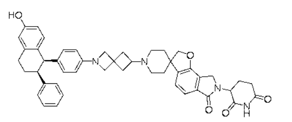

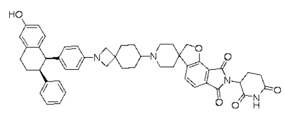 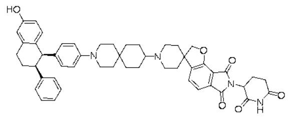 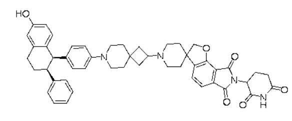 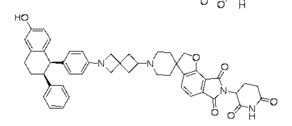

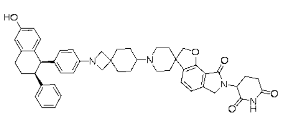 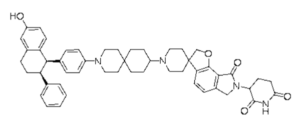 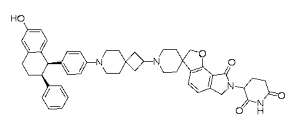 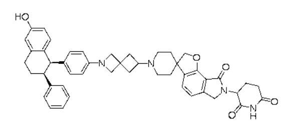

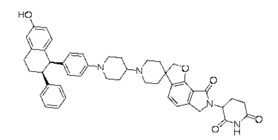 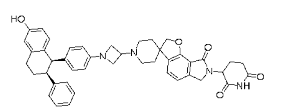

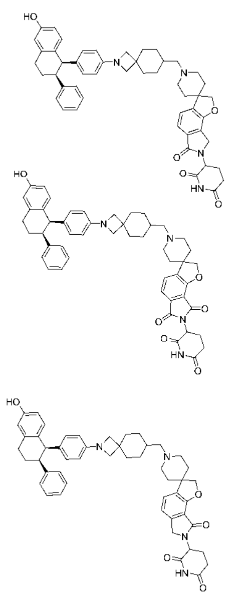 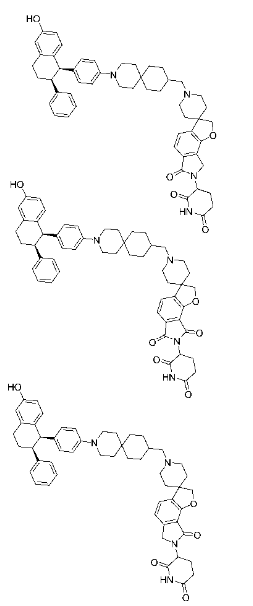 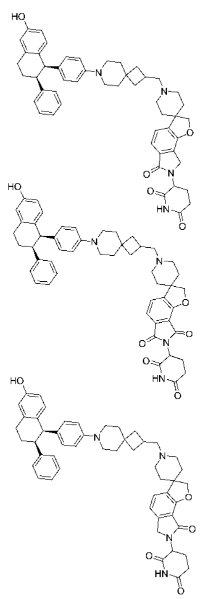 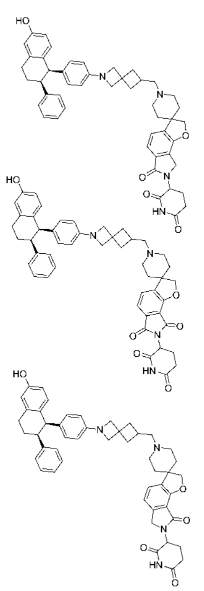

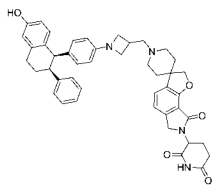

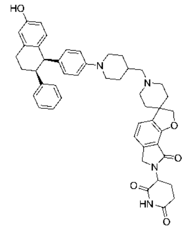

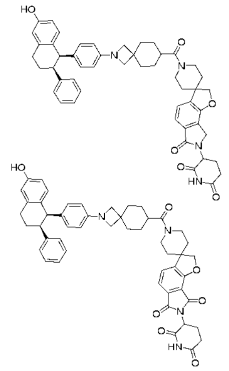

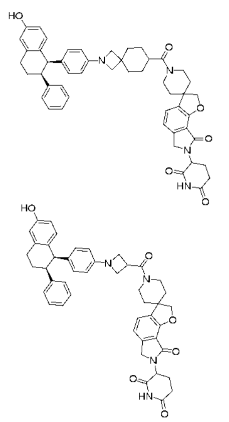

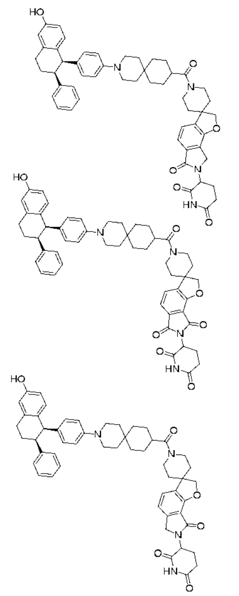

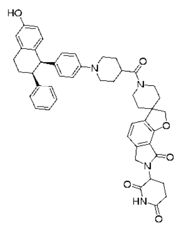

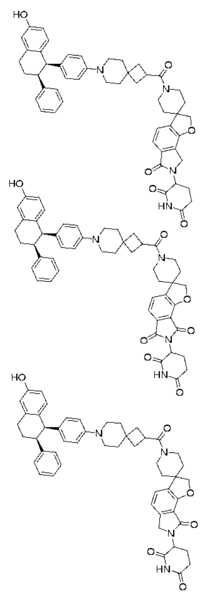

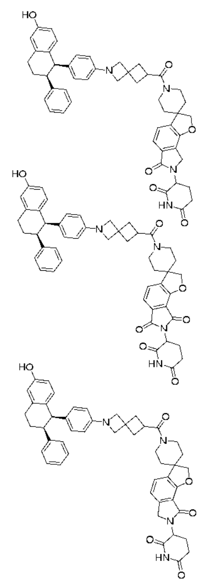

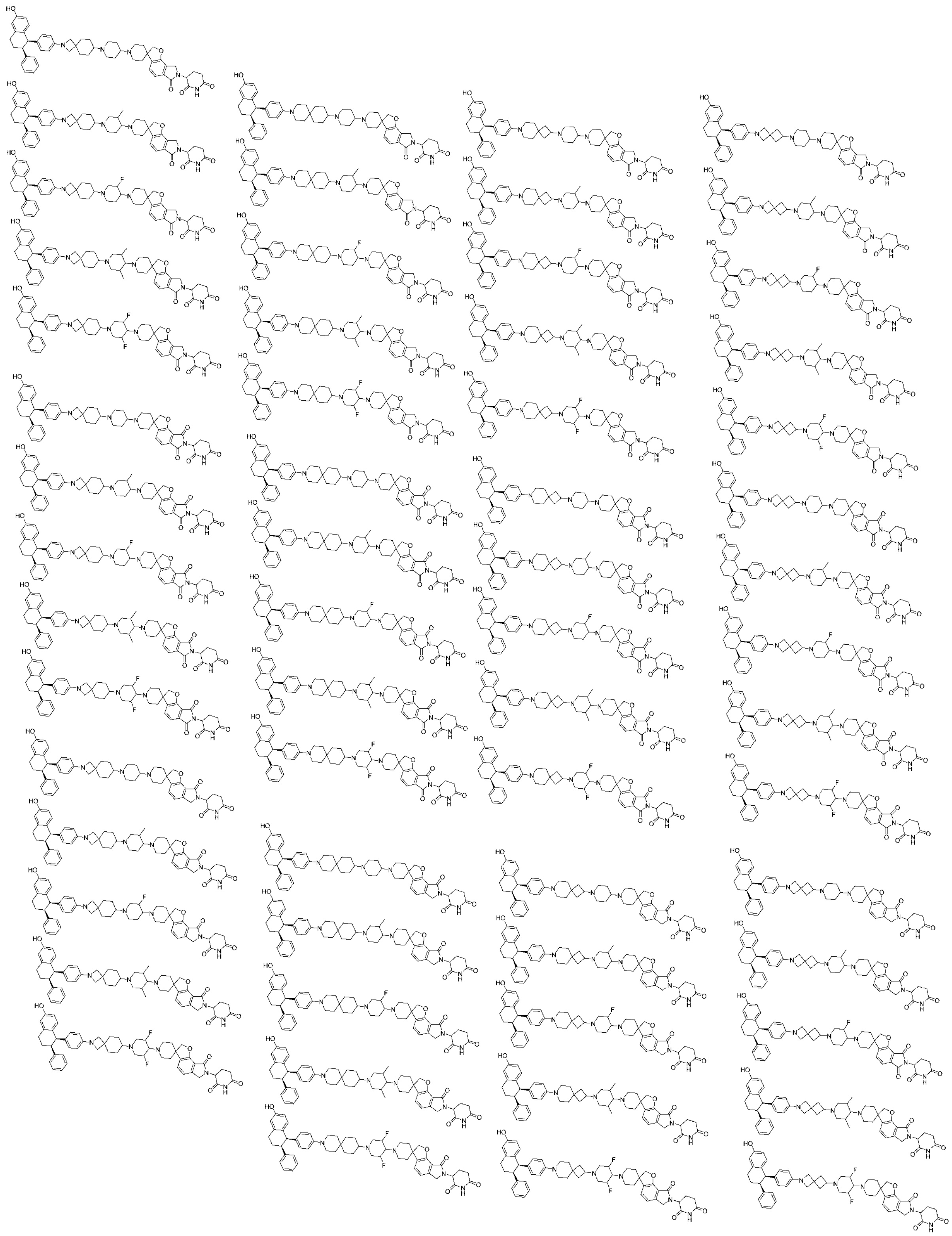

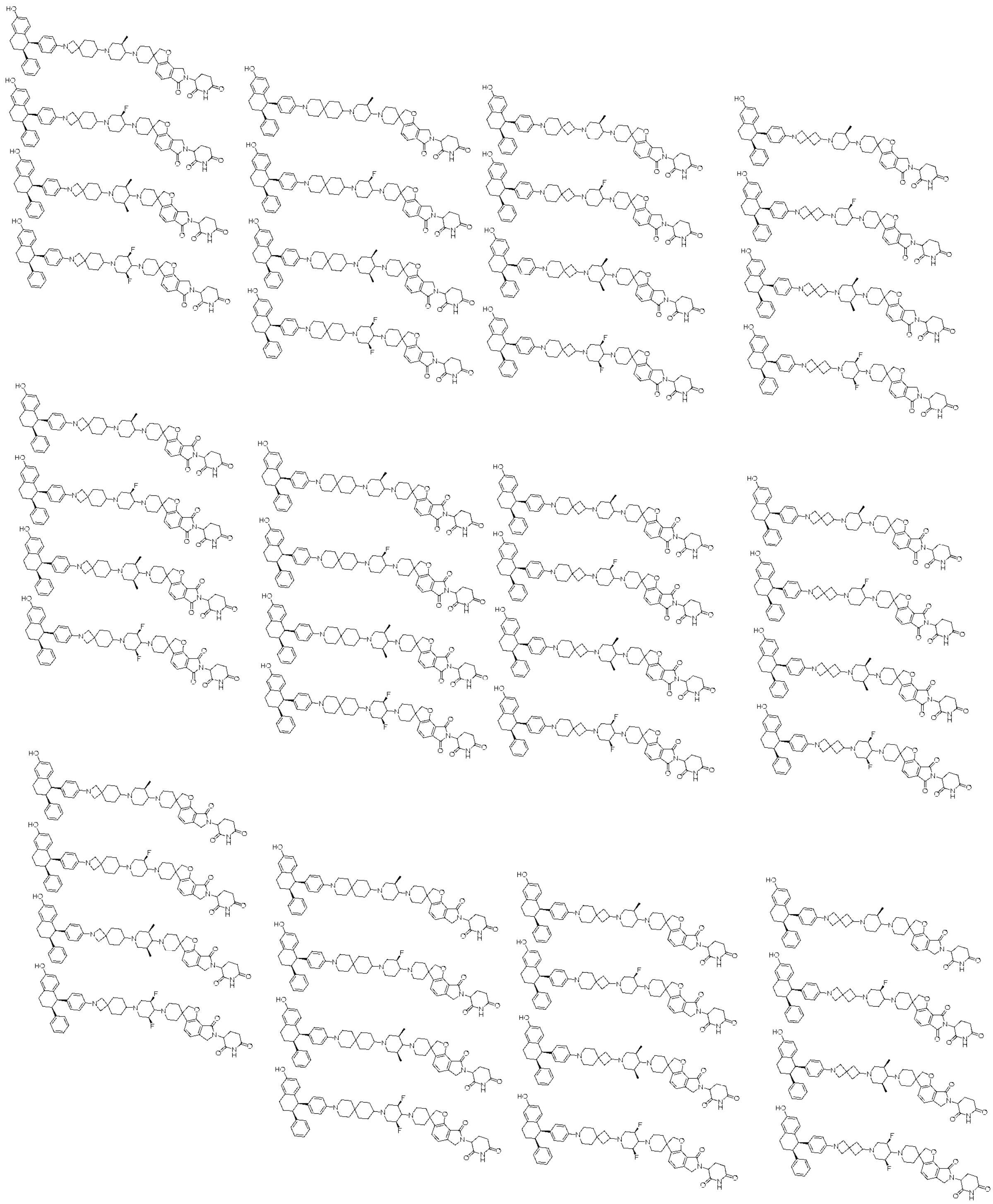

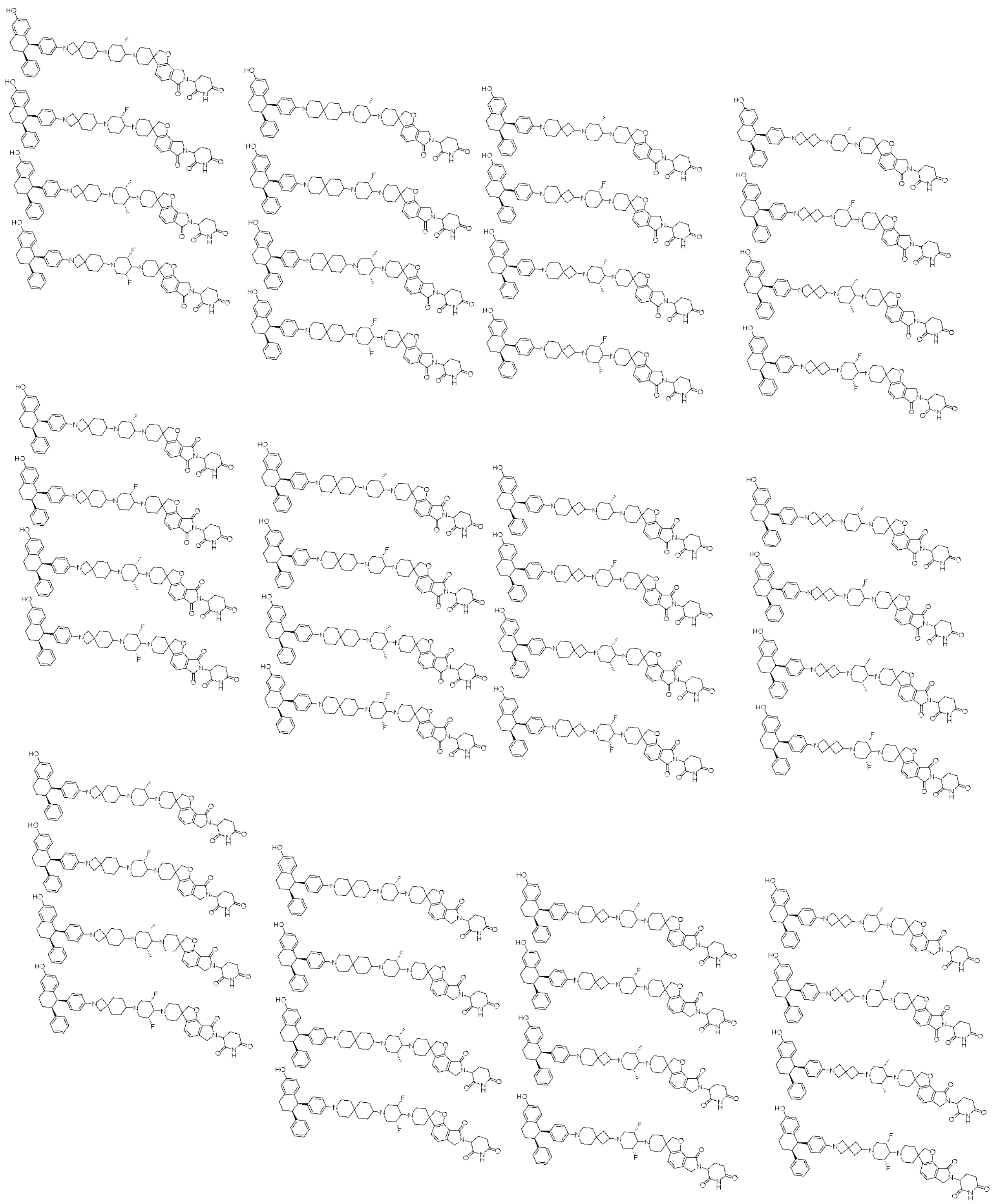

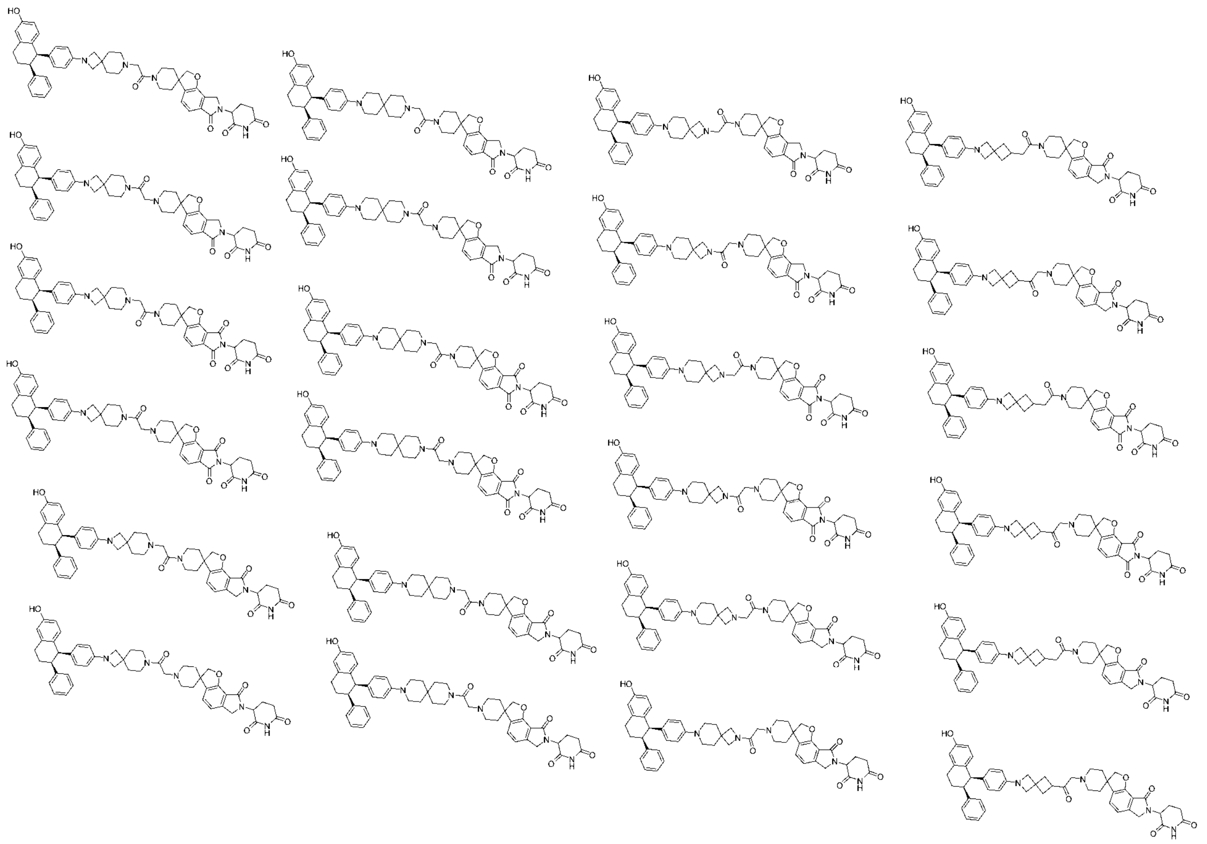

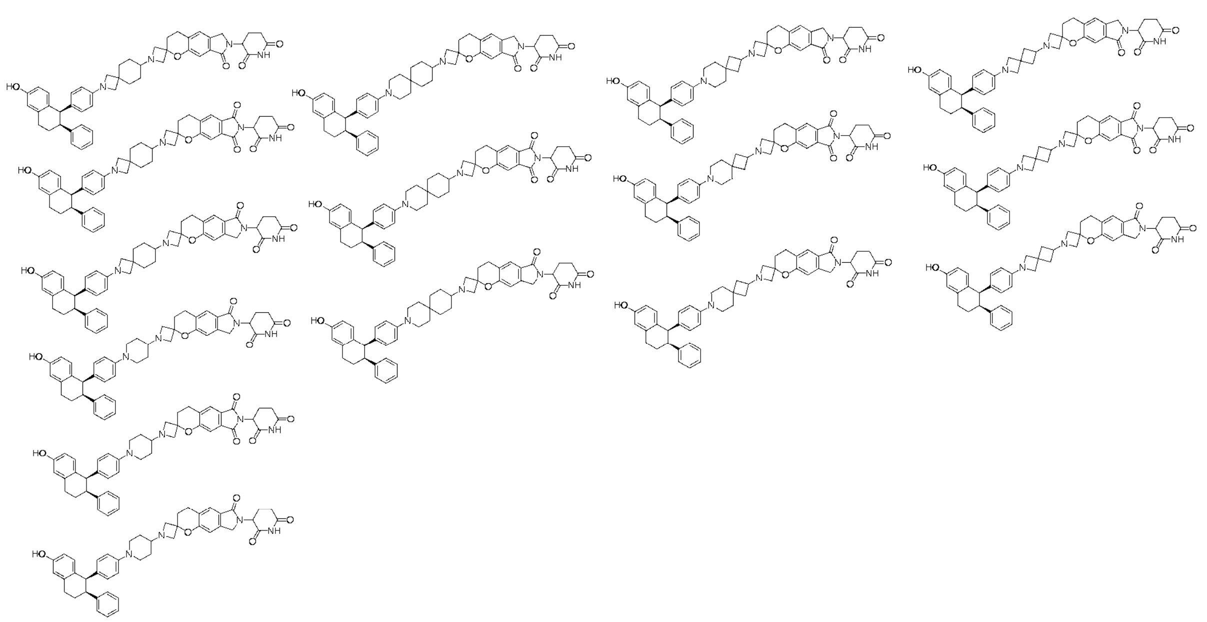

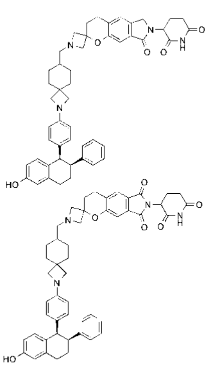
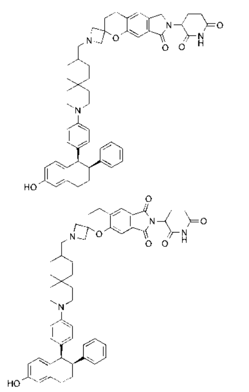
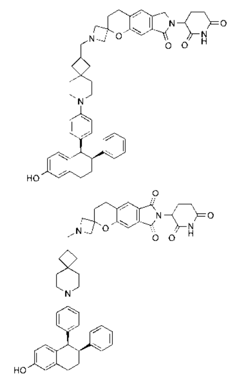
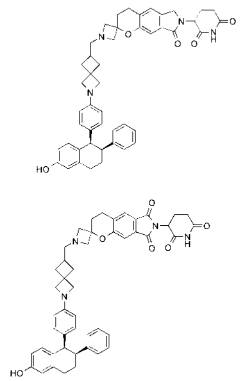
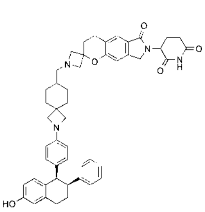
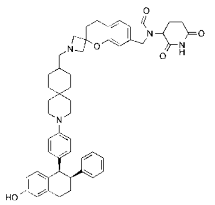
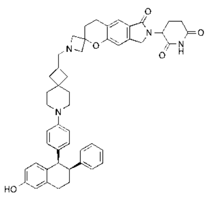
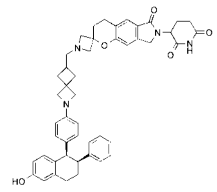
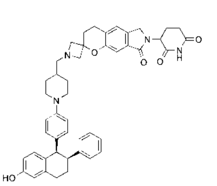
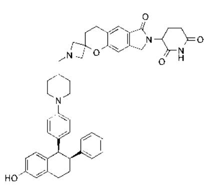
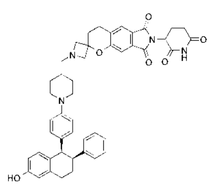
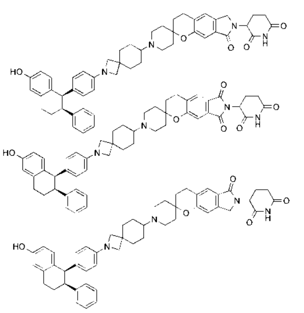
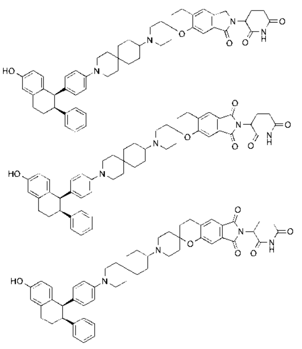
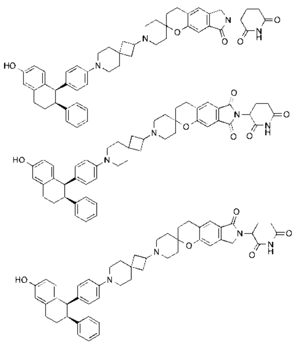
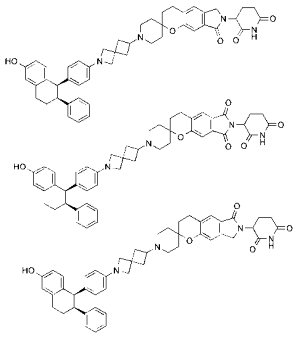
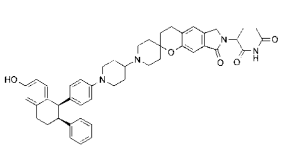
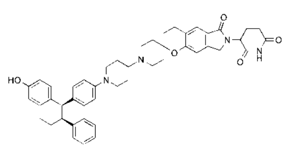
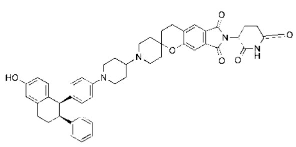

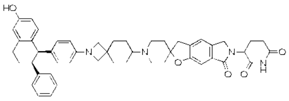
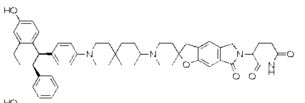
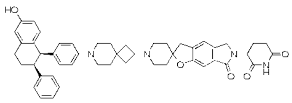
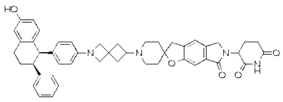
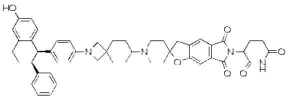
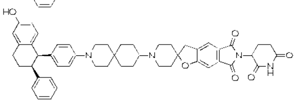
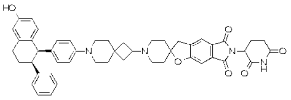
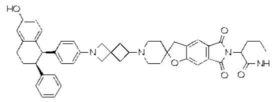
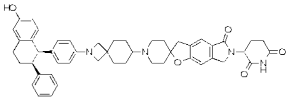
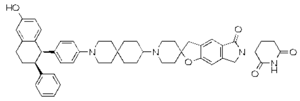
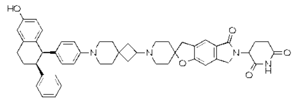
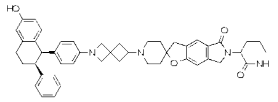
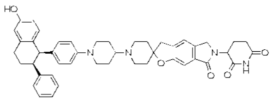
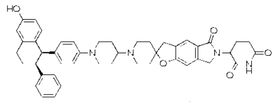
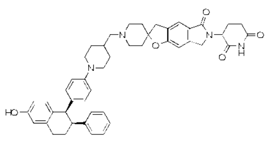
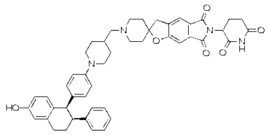
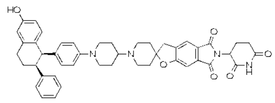
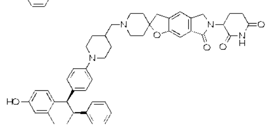

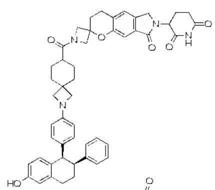
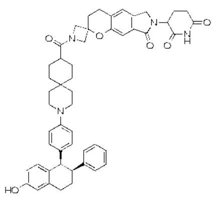
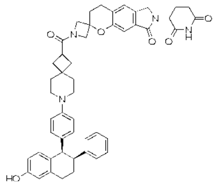
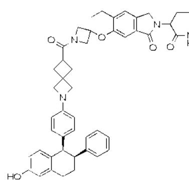
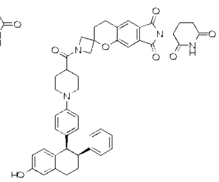
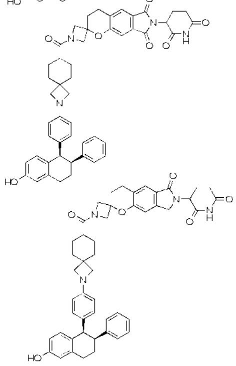
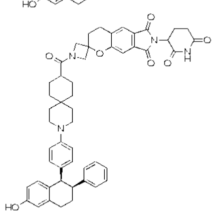
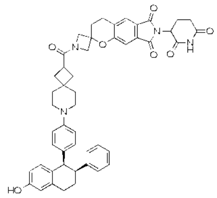
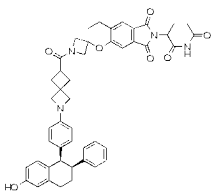
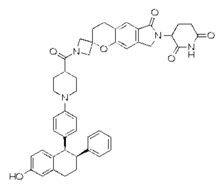
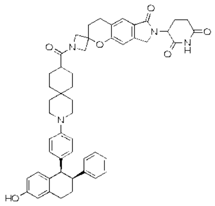
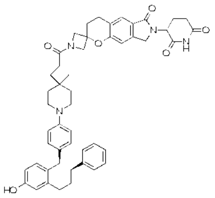
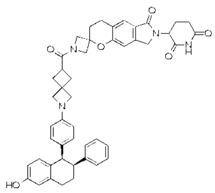
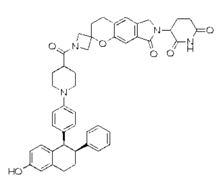

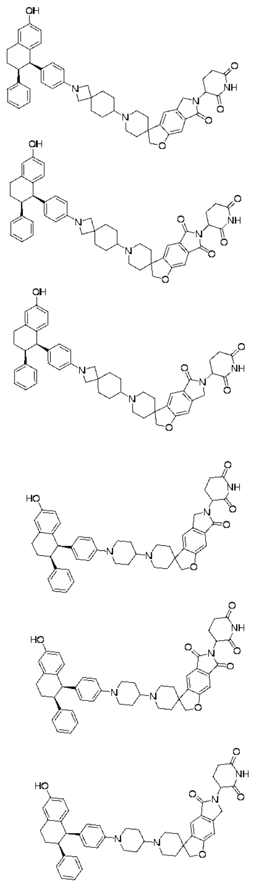
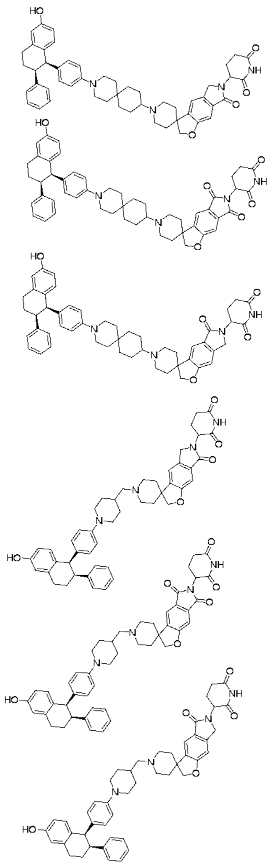
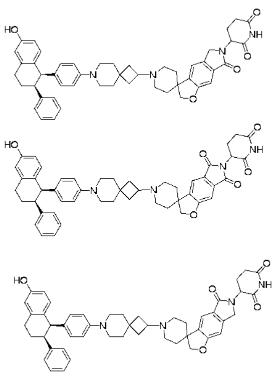
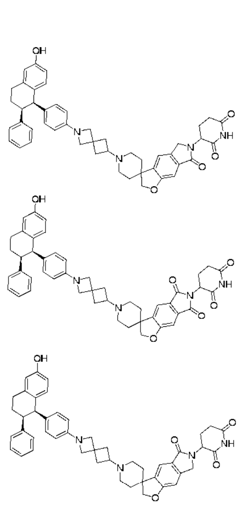

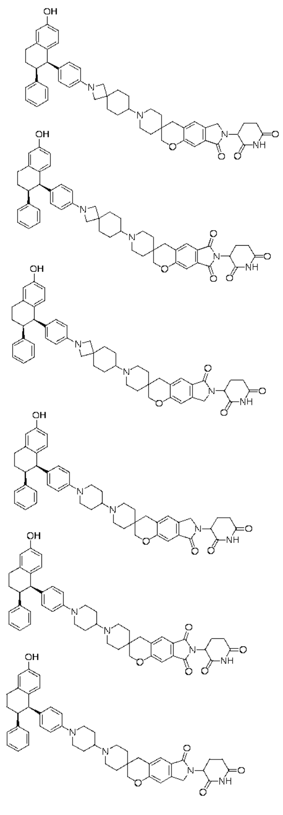
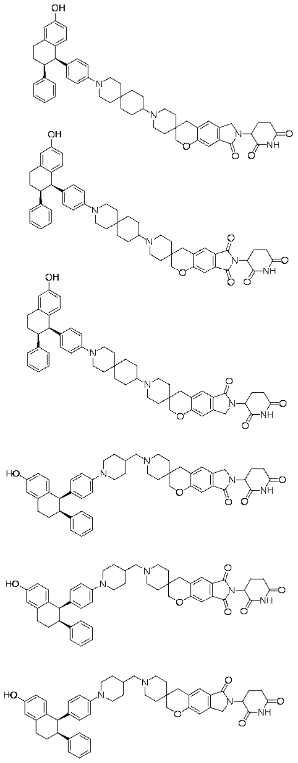
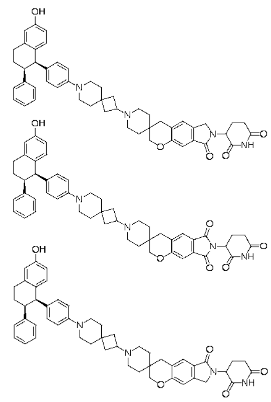
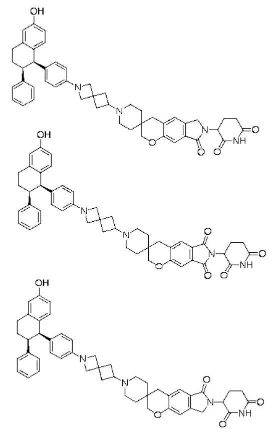

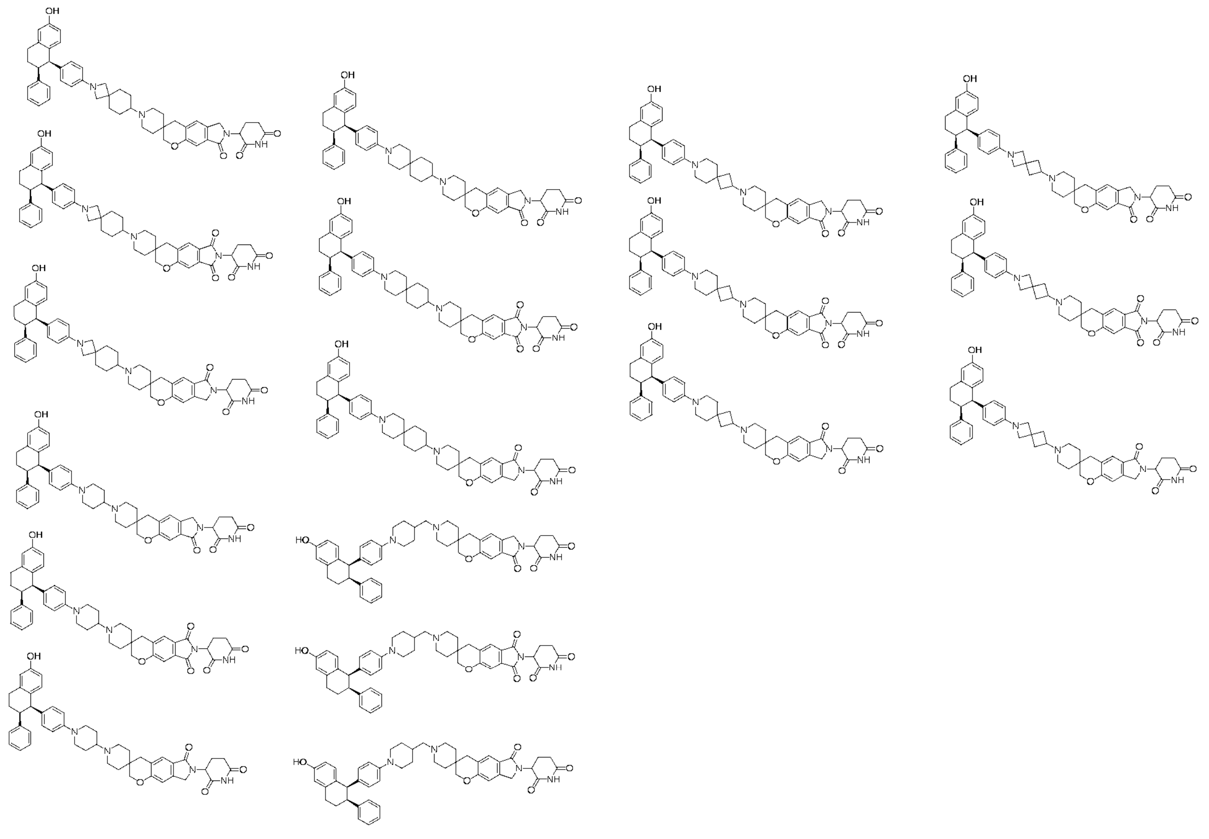

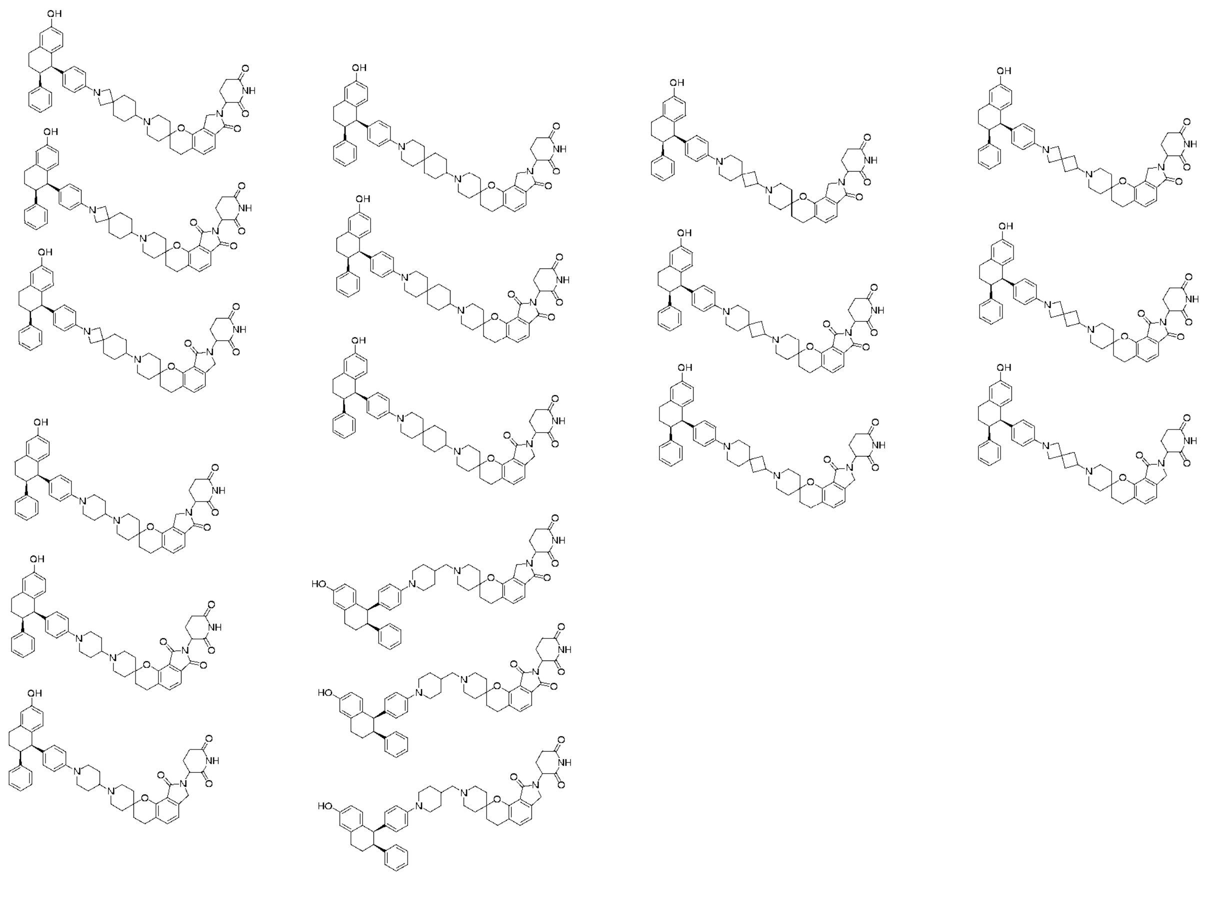

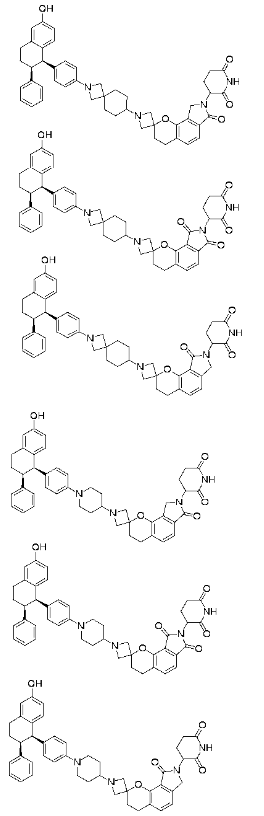

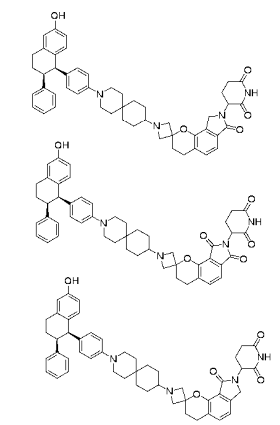

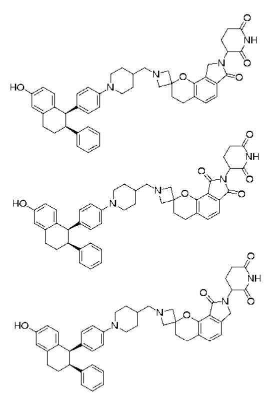

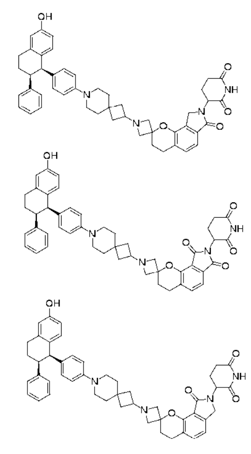

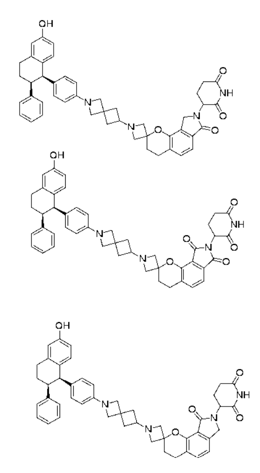

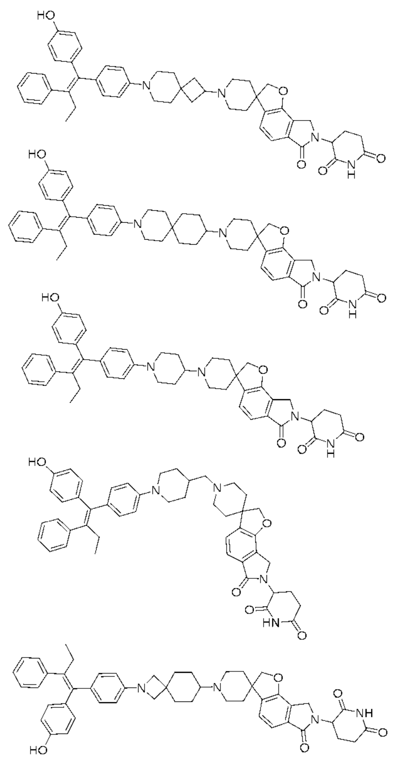

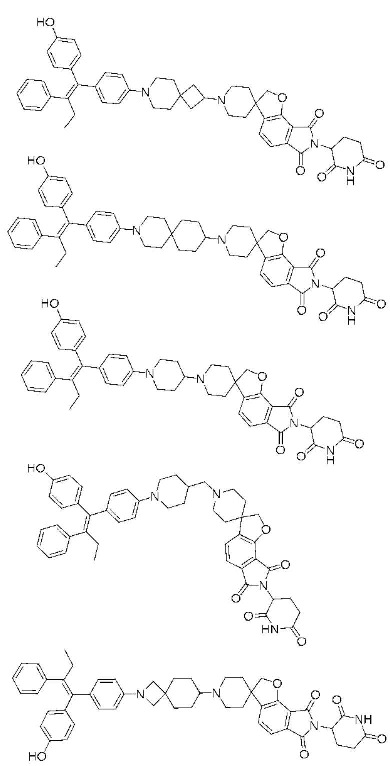

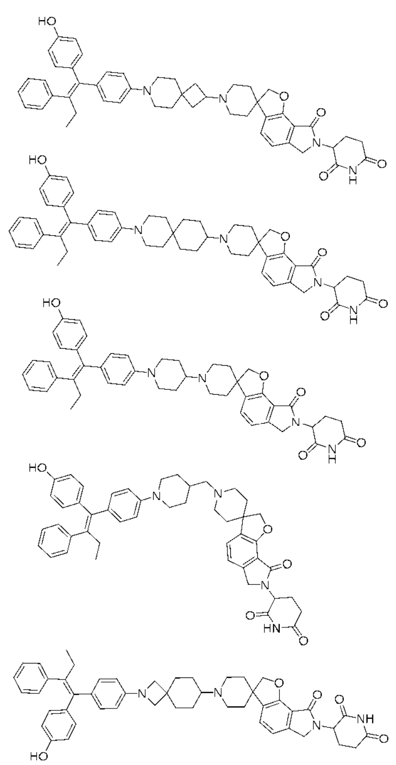

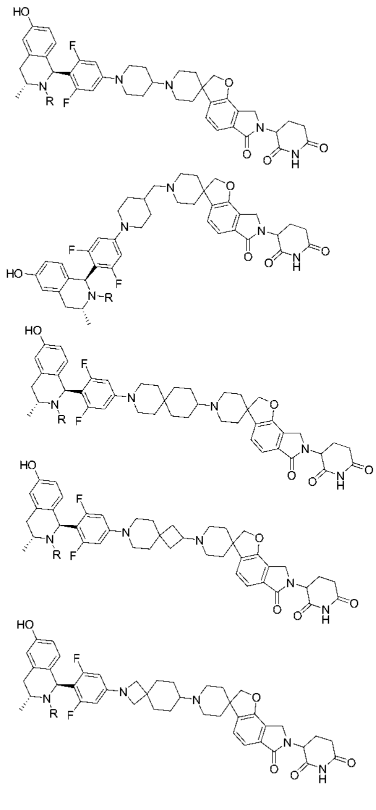
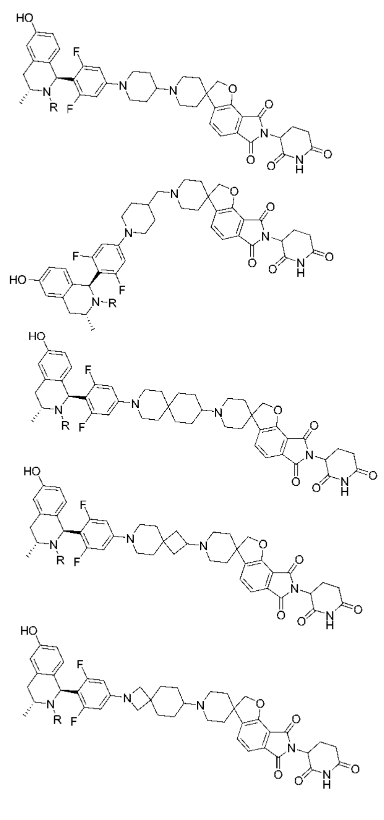
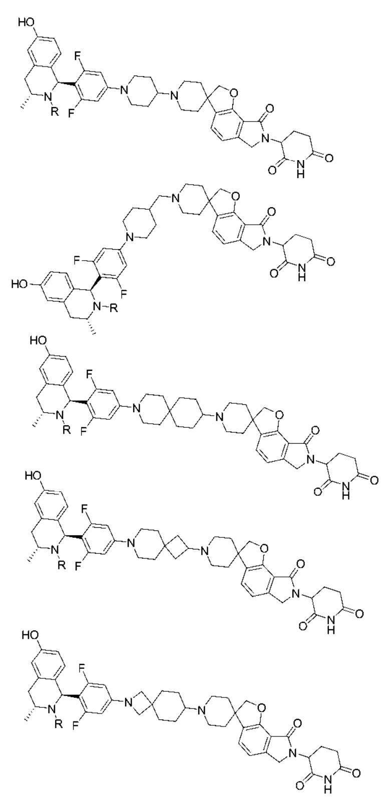

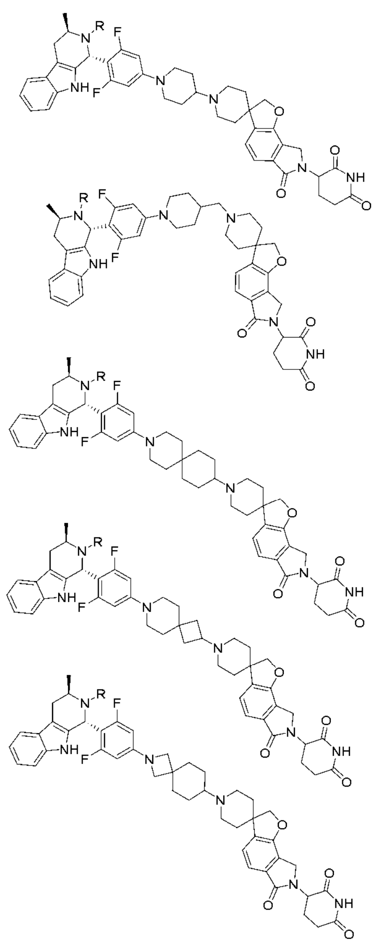
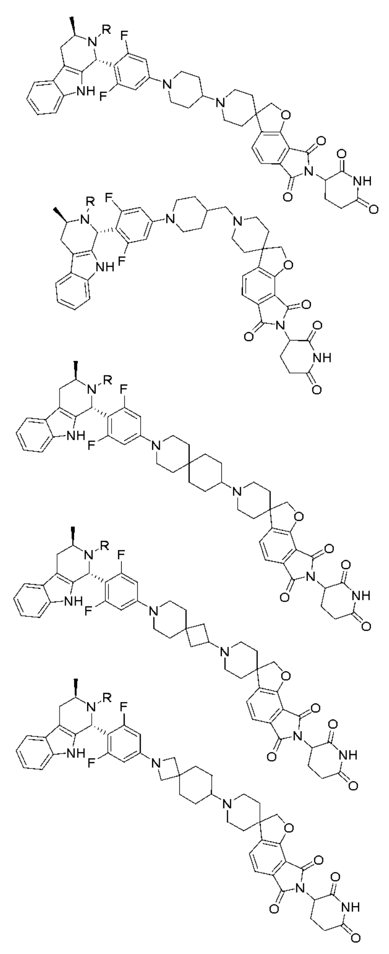
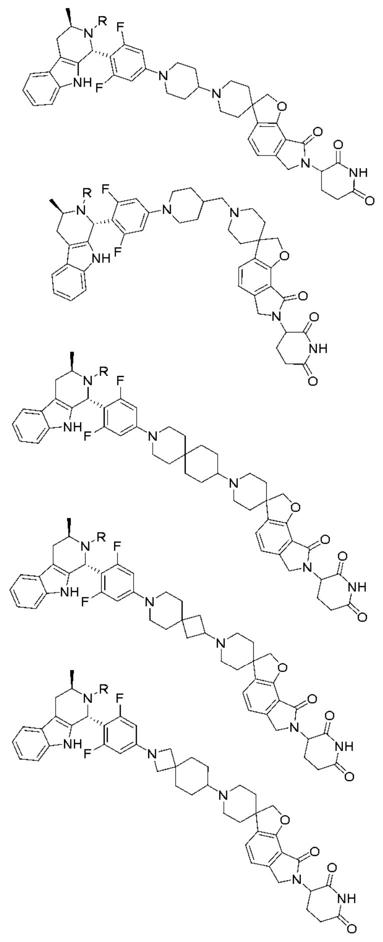

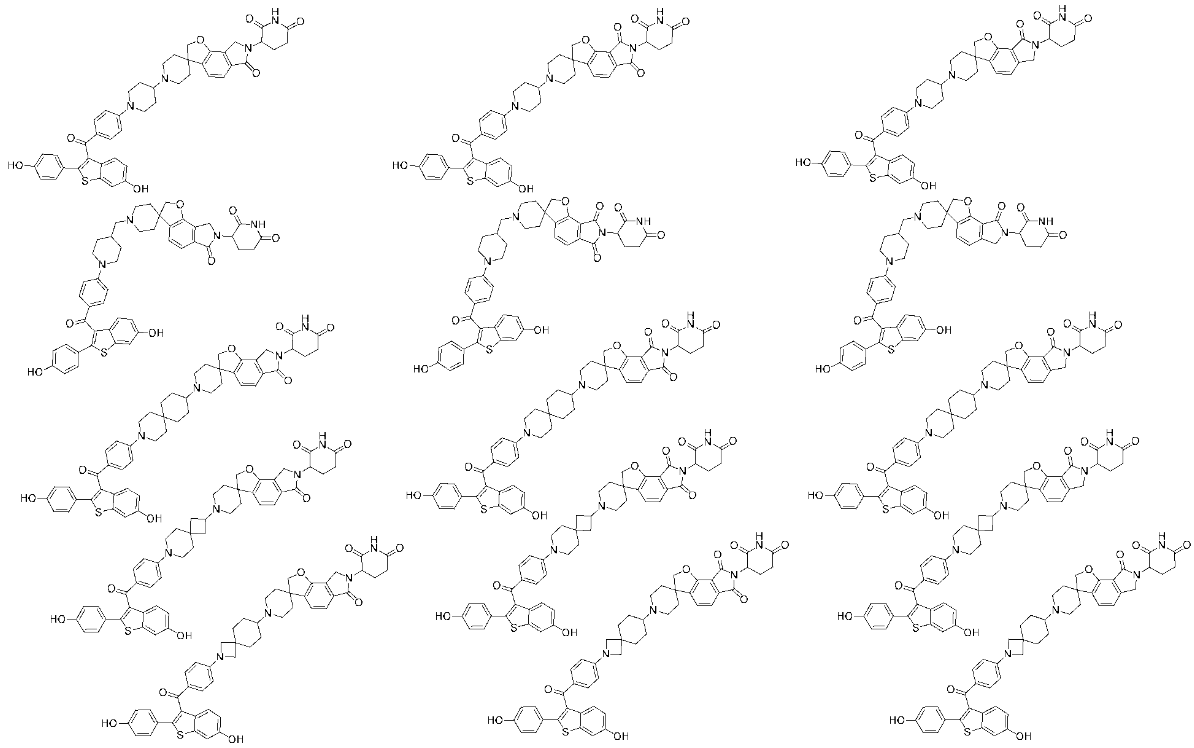

;

R is selected from

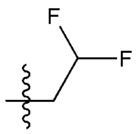, 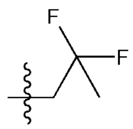, 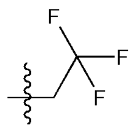, and 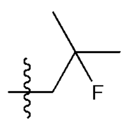.

**15.** The compound according to any one of claims 1 to 13, wherein the compound is selected from the following compounds:

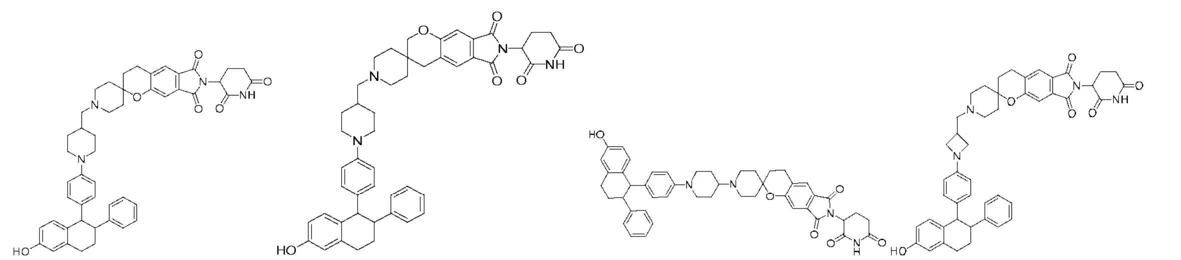

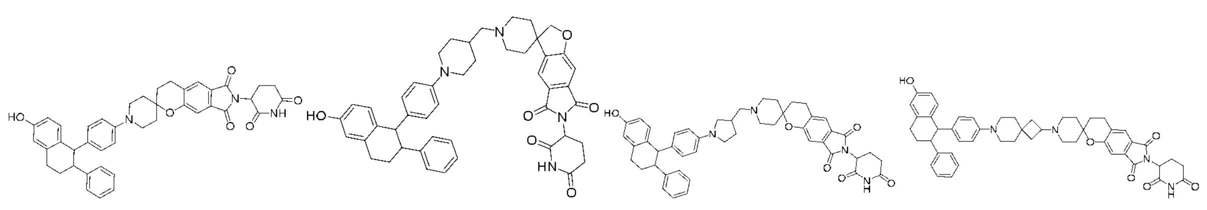

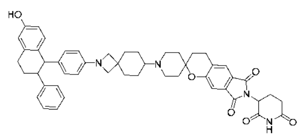

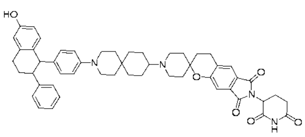

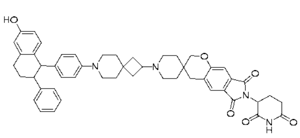

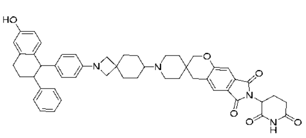

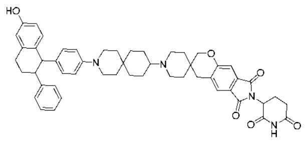
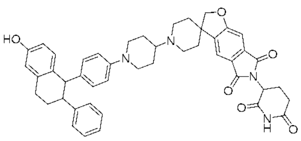
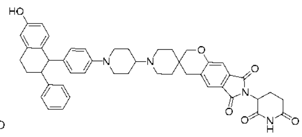
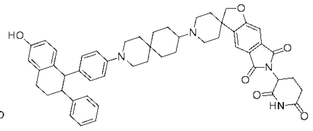

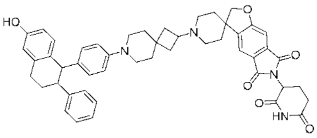
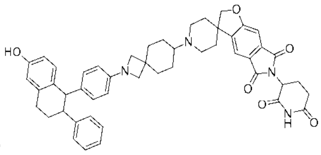
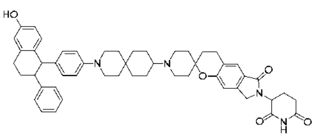
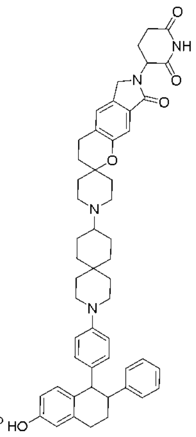

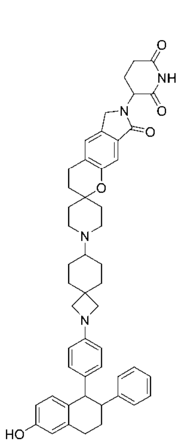
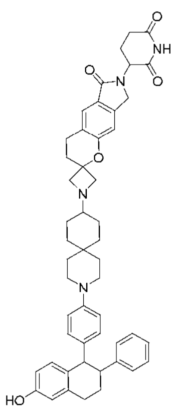
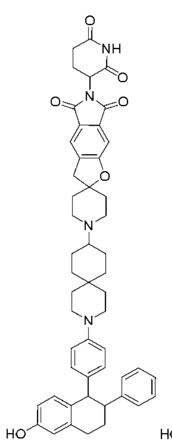
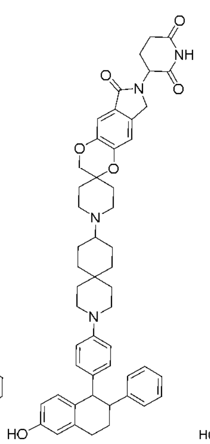
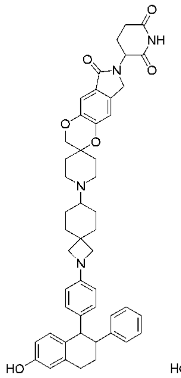
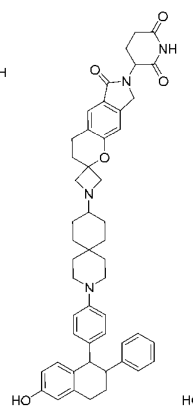
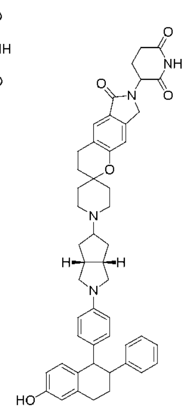

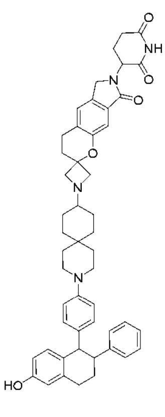
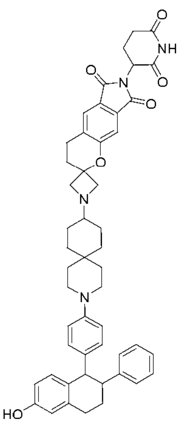
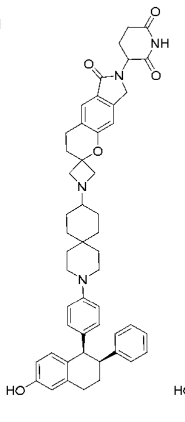
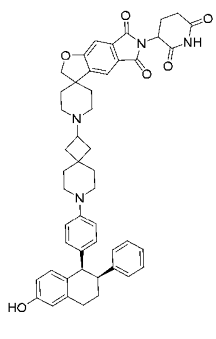
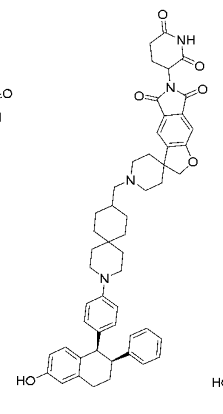
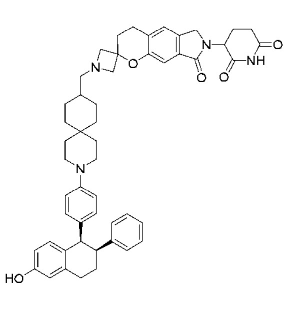

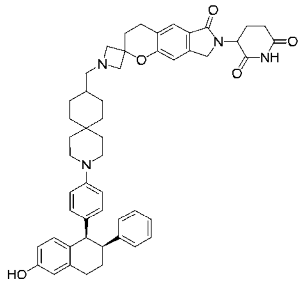

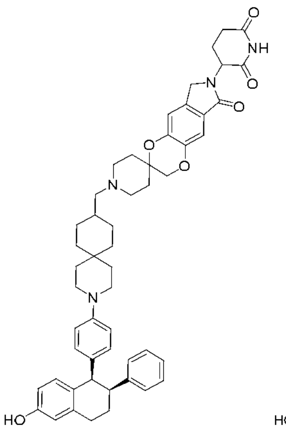

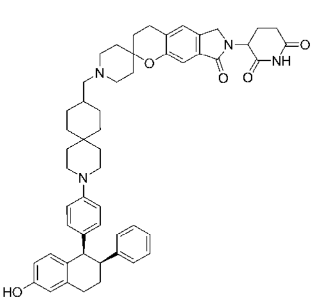

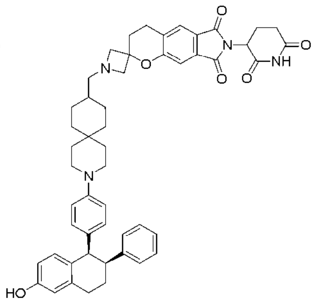

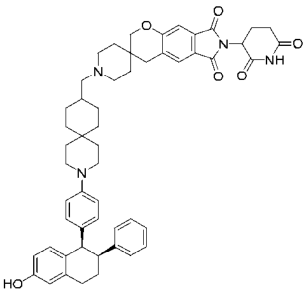

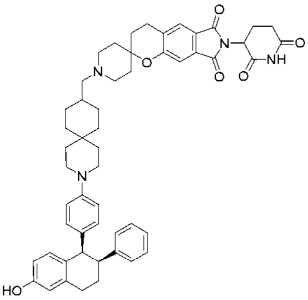

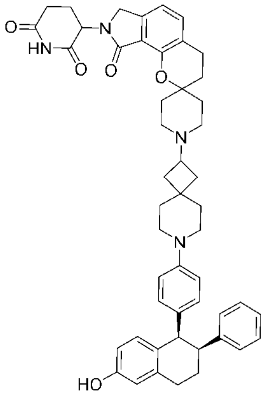

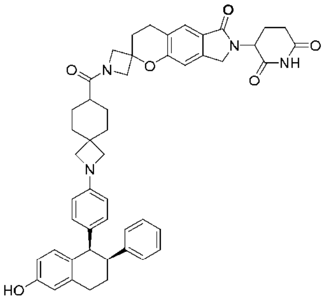

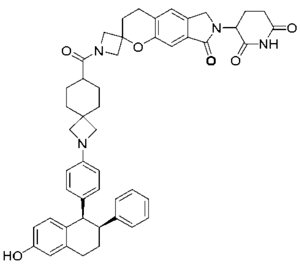

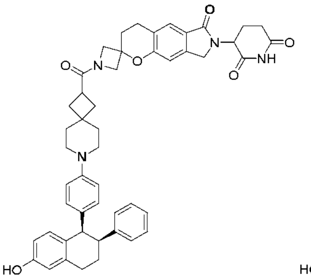

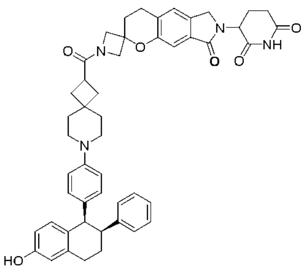

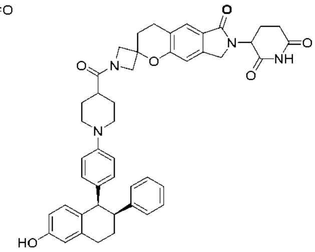

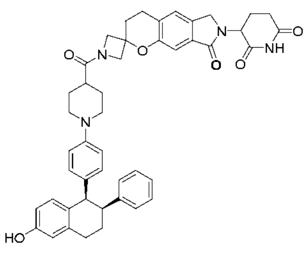

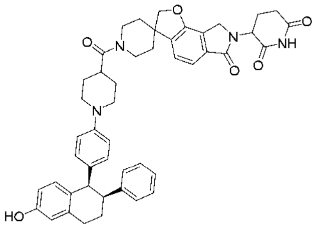

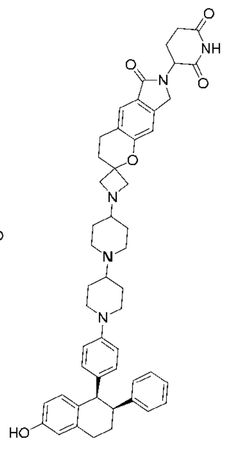

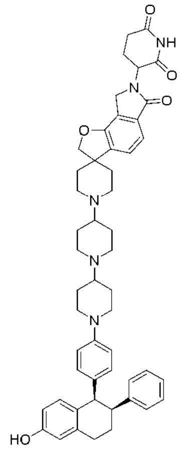

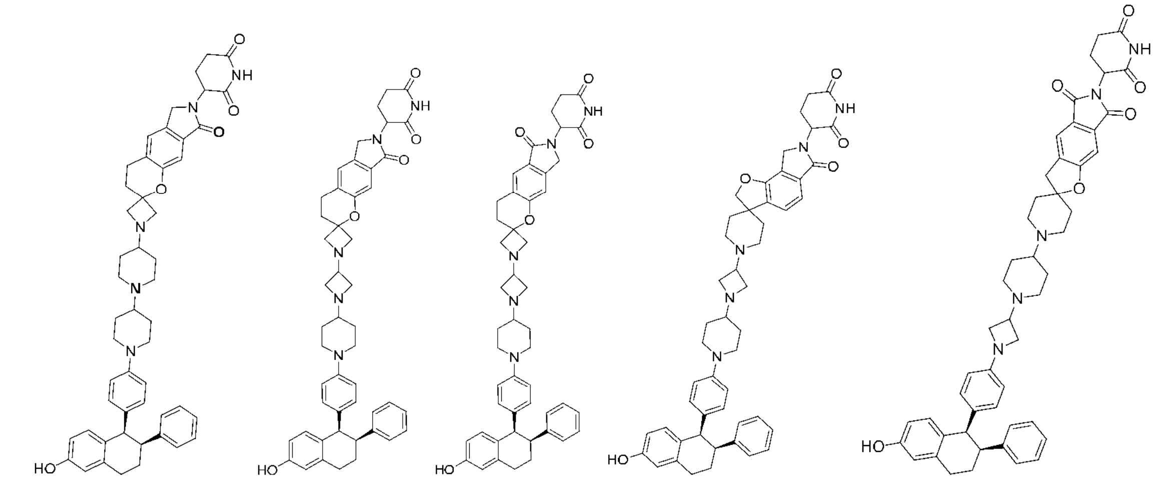

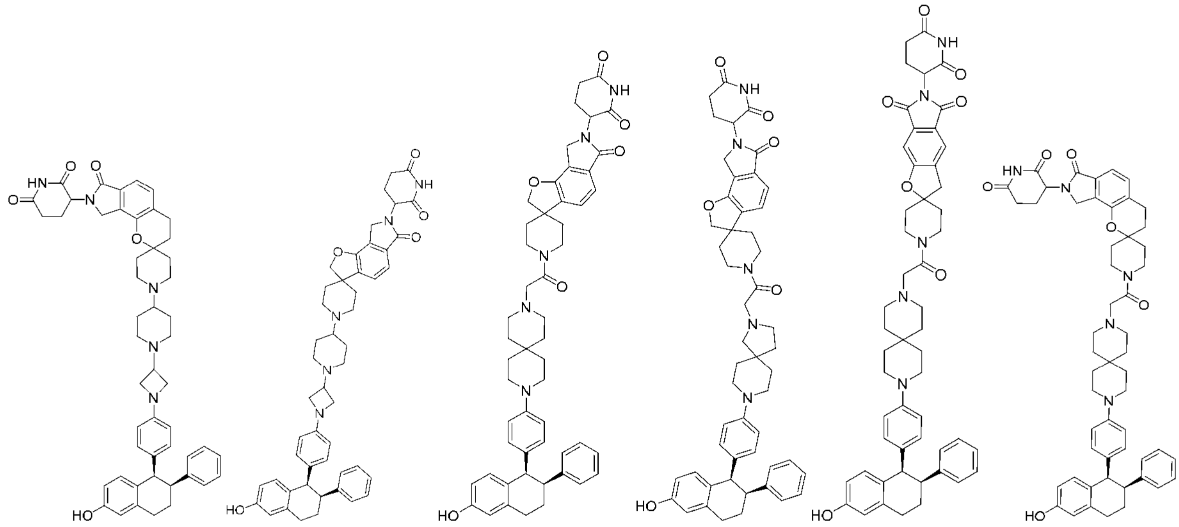

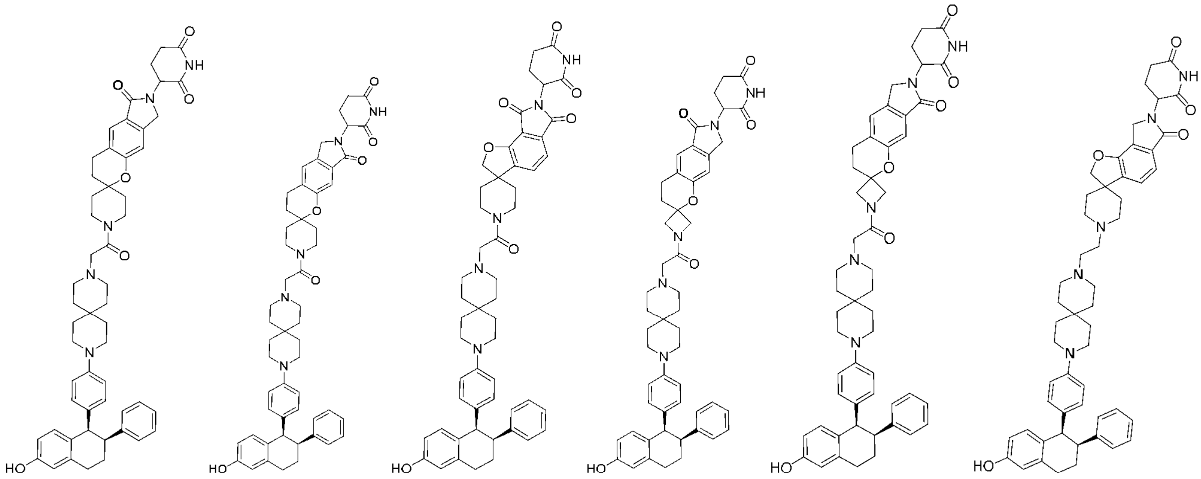

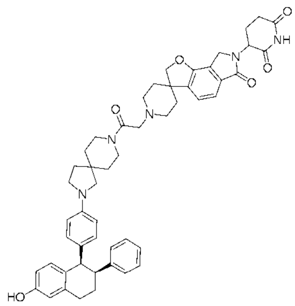
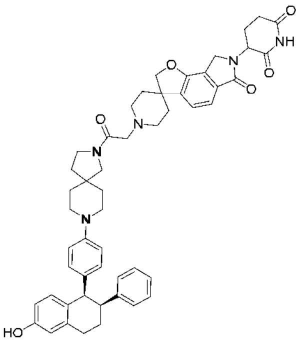
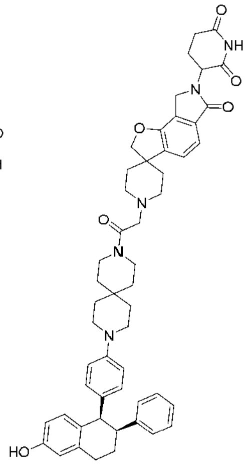
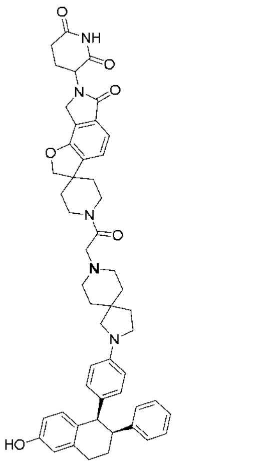

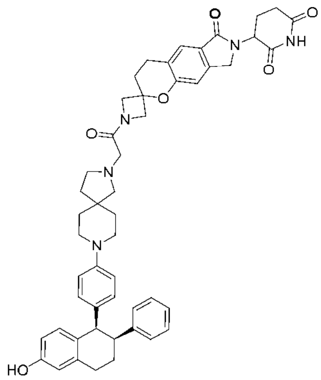
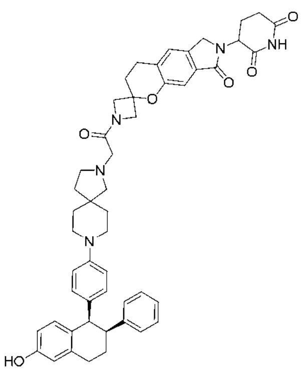
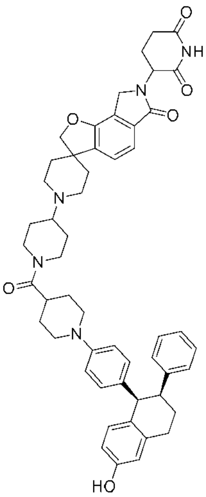
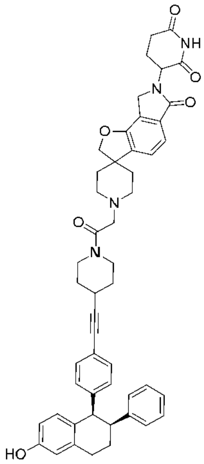
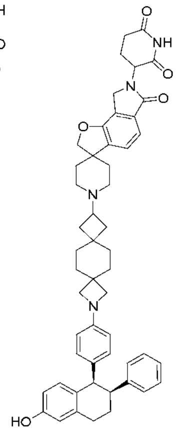

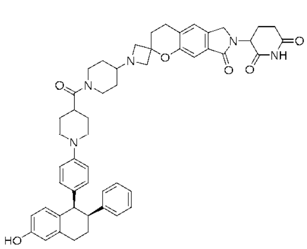
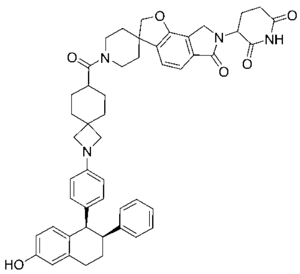
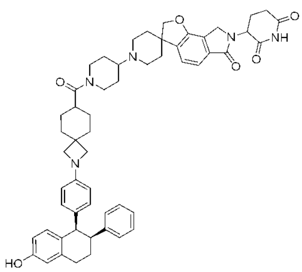
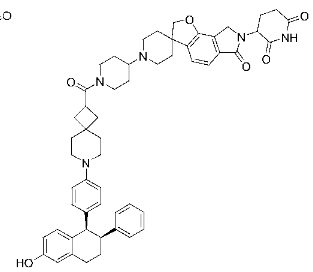

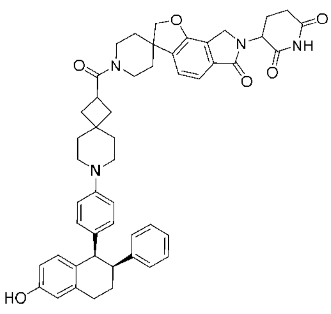
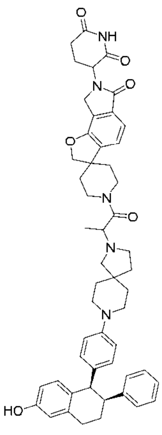
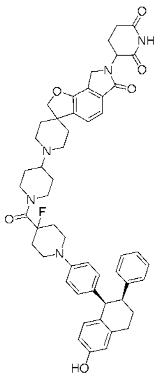
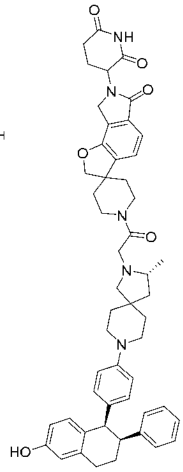
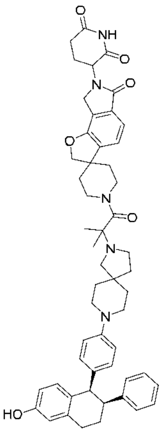
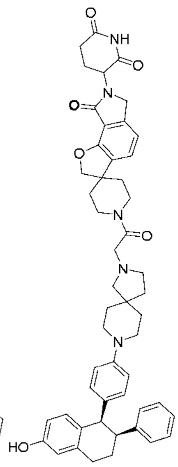
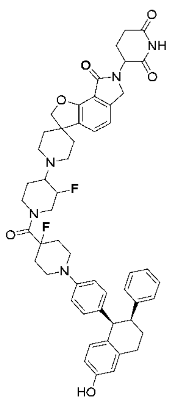
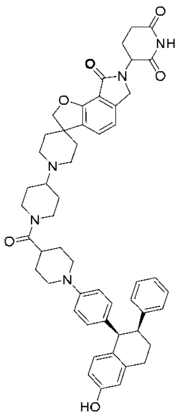
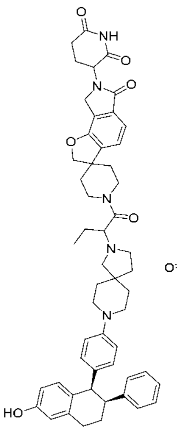
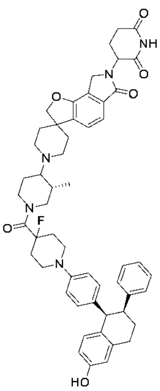
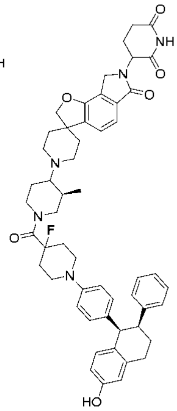
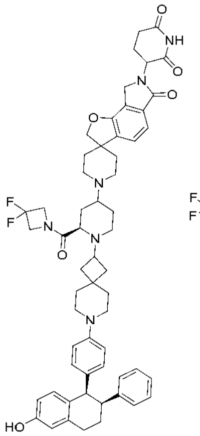
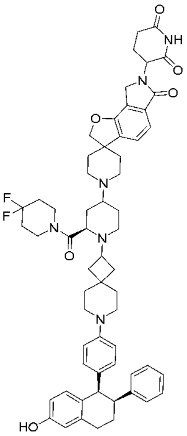
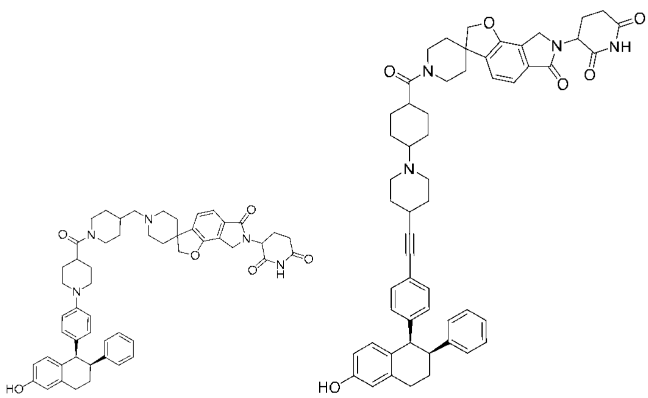
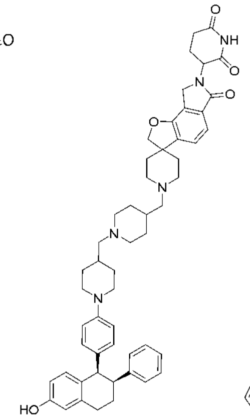
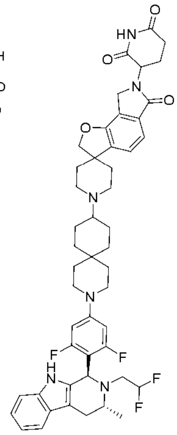
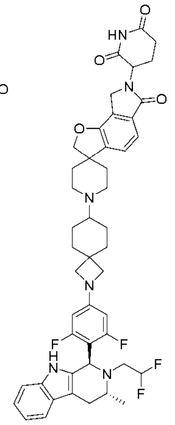
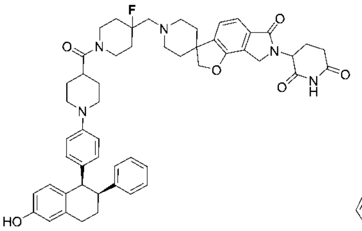
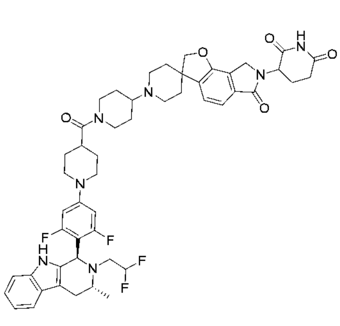
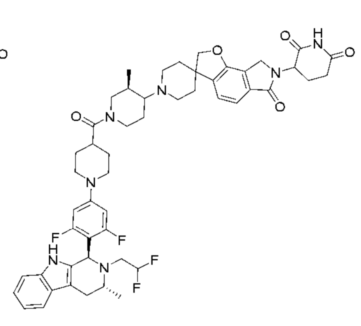

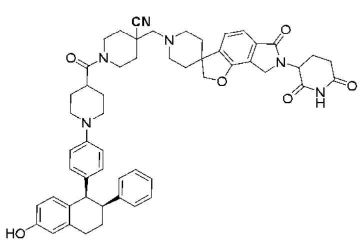
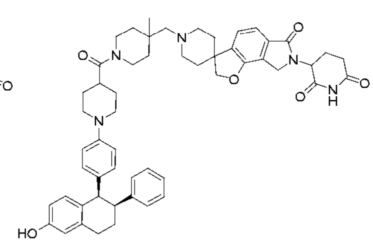
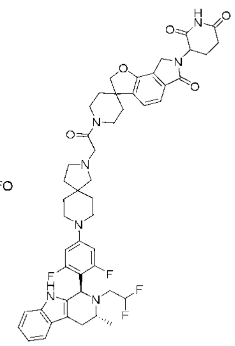
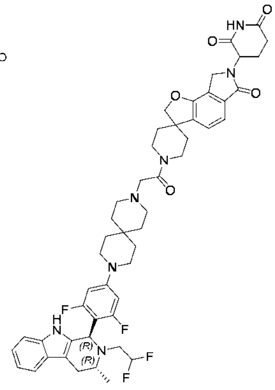

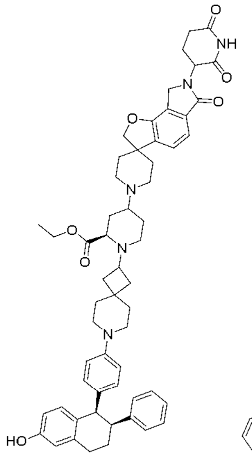
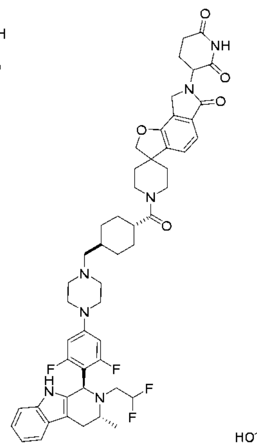
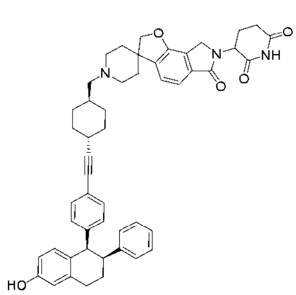
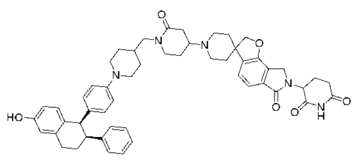

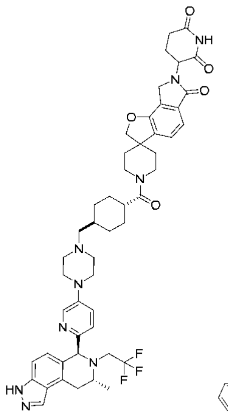
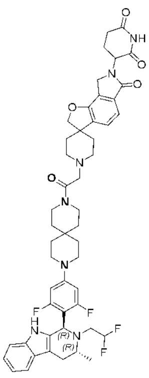
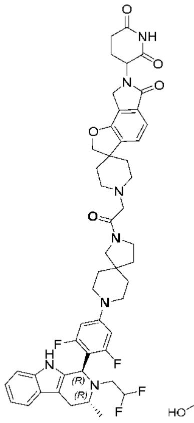
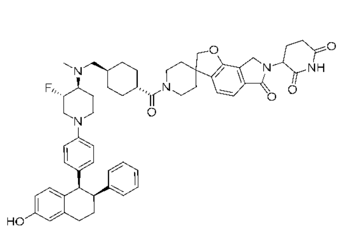
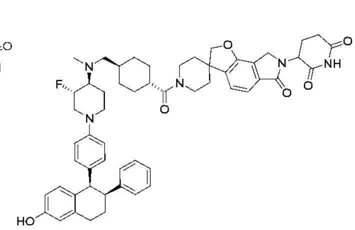

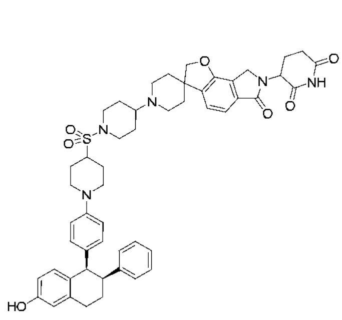
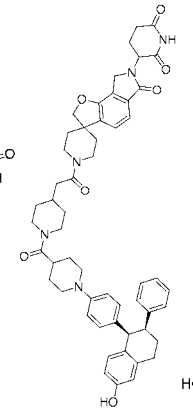
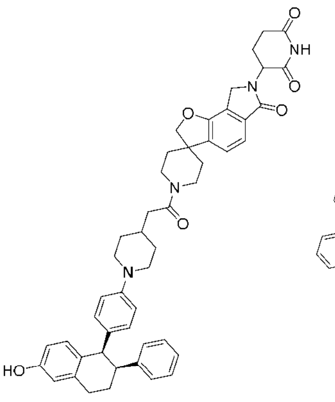
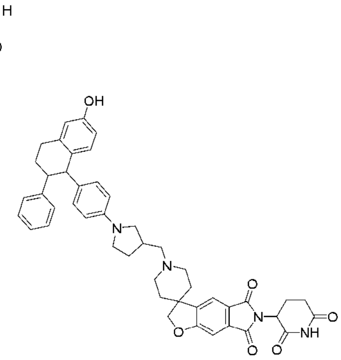

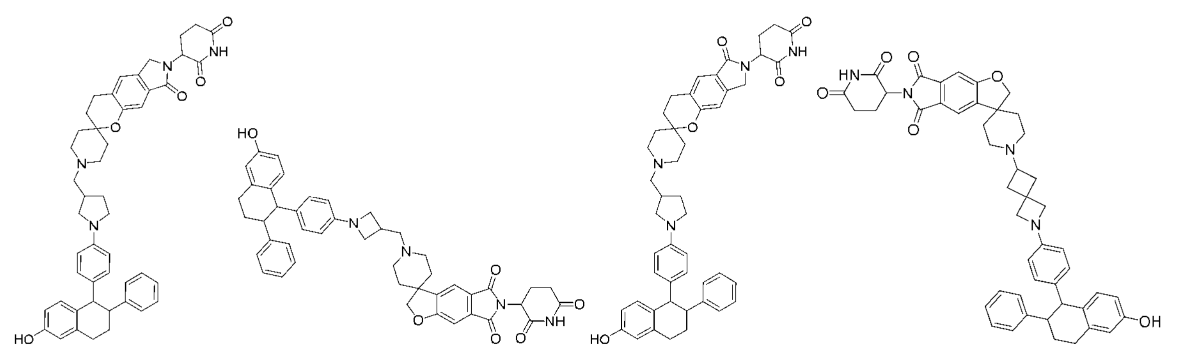

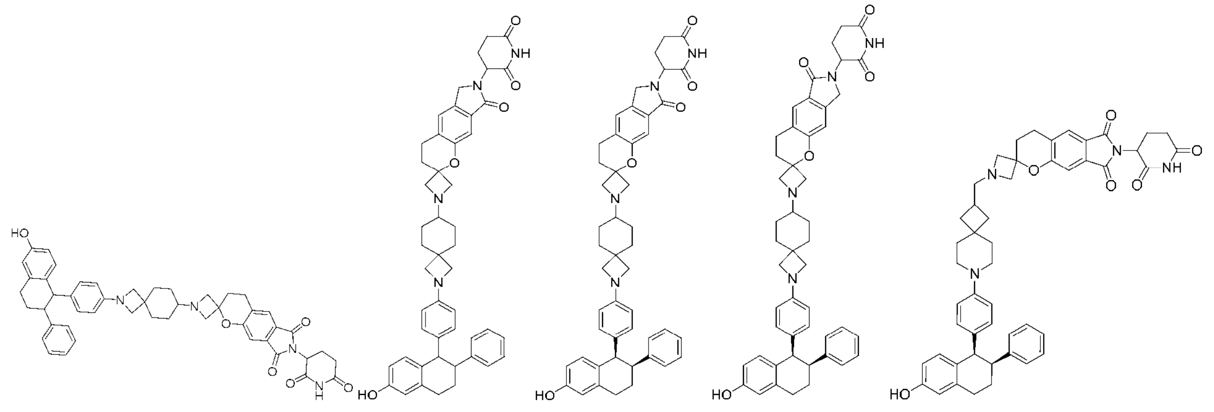

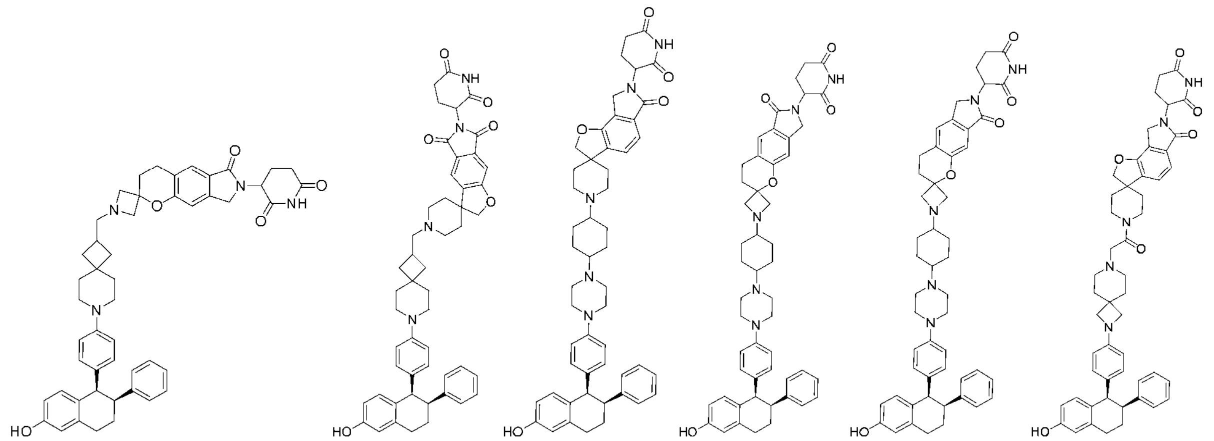

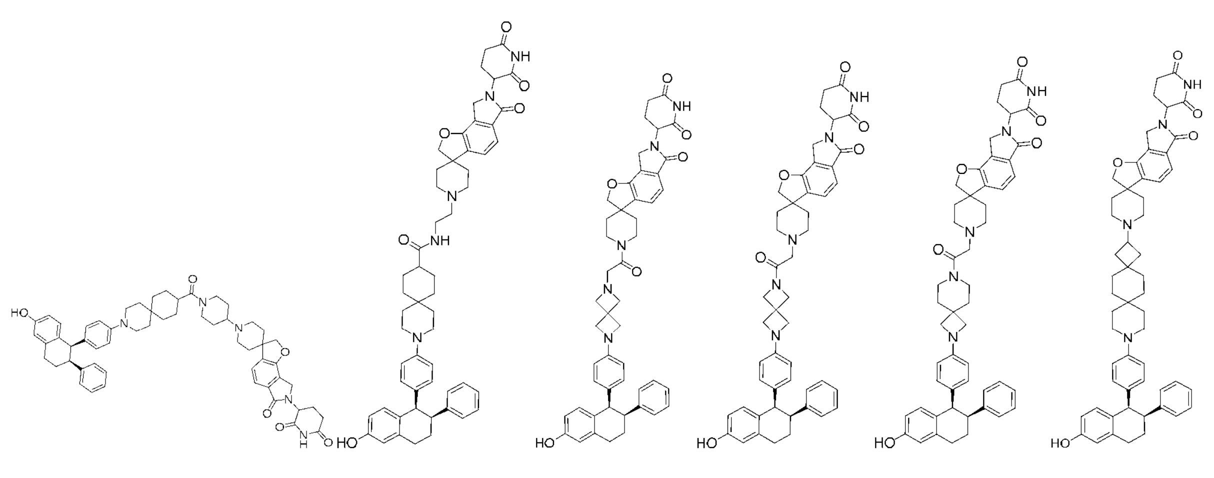

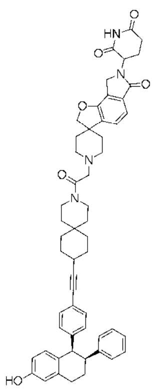
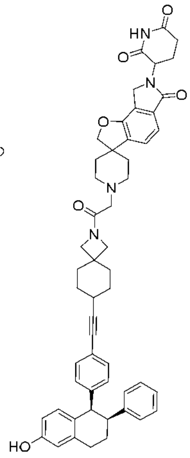
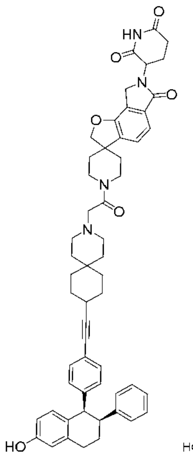
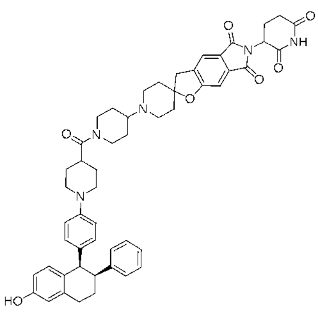
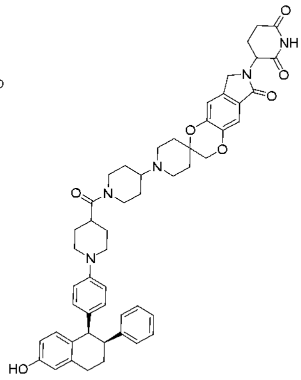
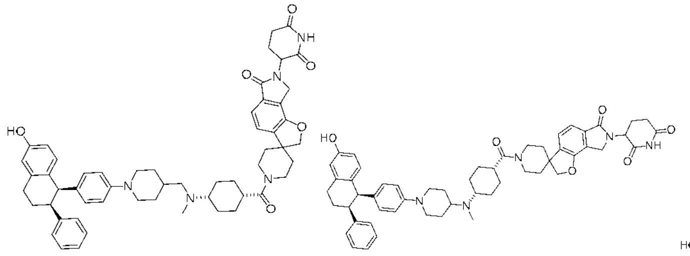
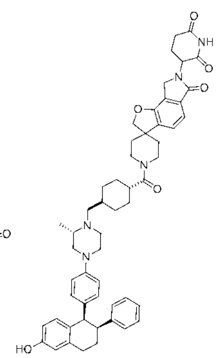
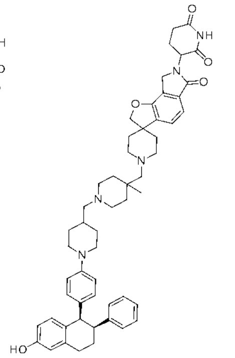
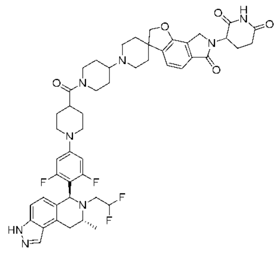
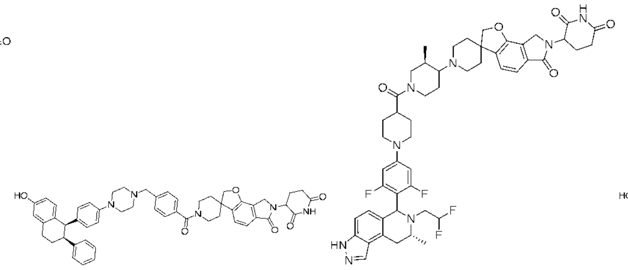
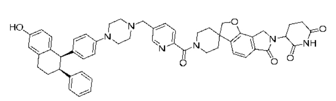
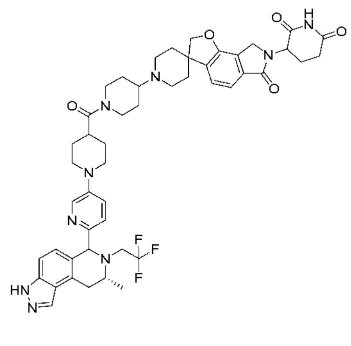
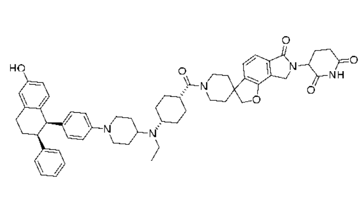
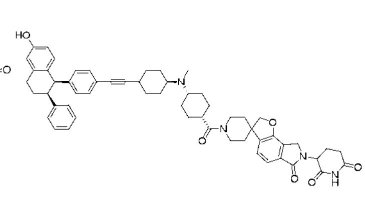

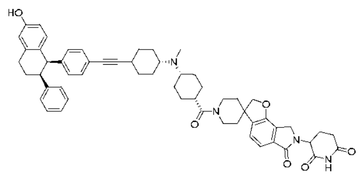
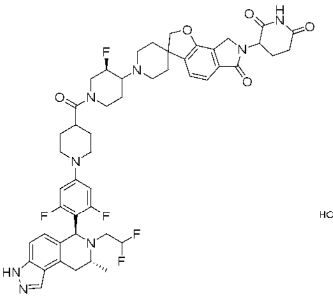
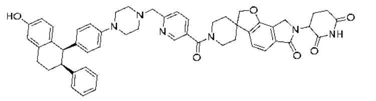

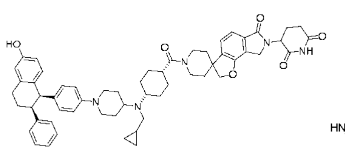
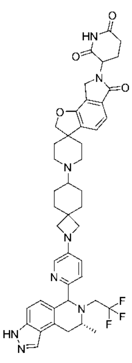
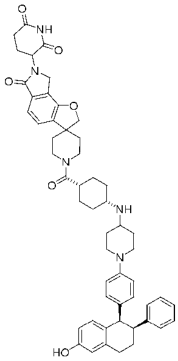
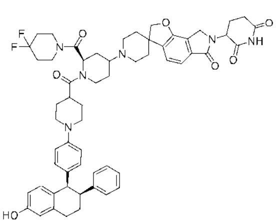
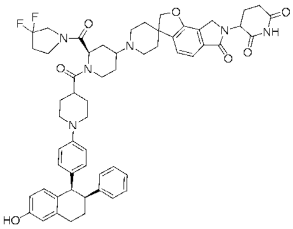

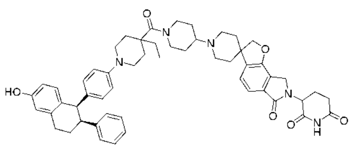
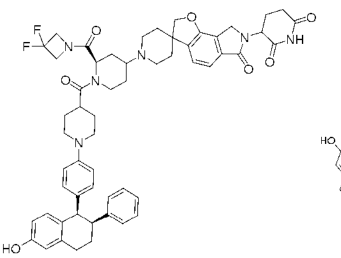
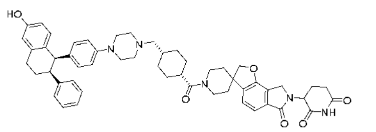

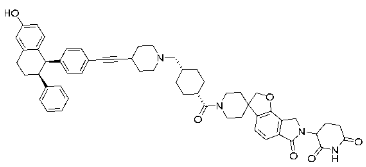
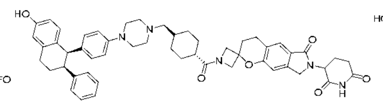
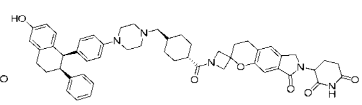

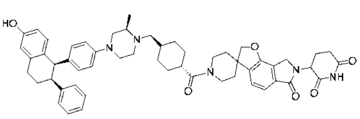
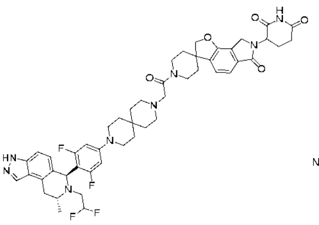
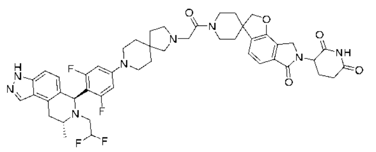

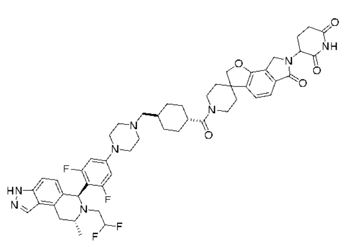
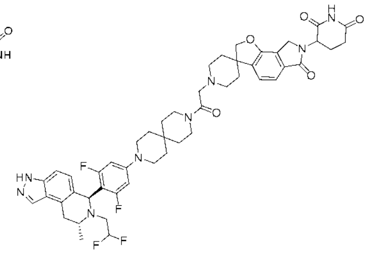
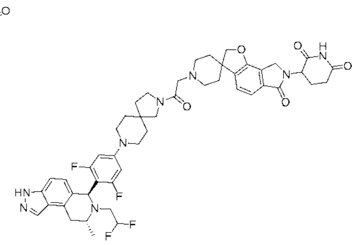

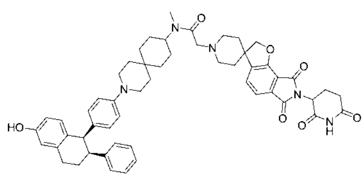

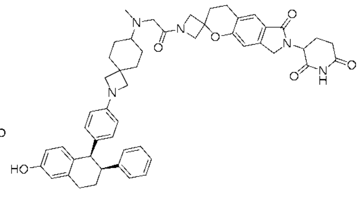

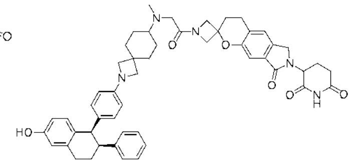

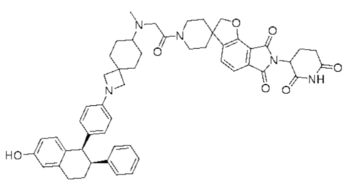

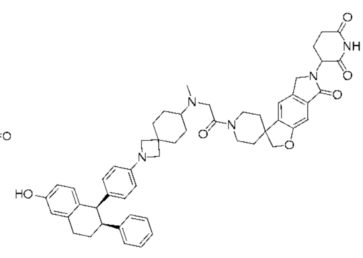

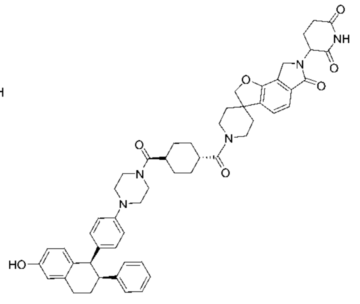

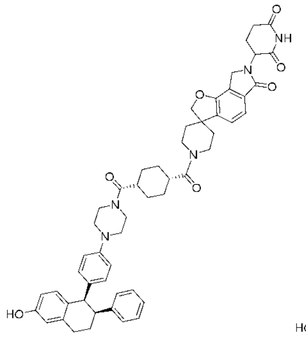

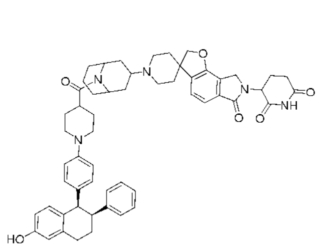

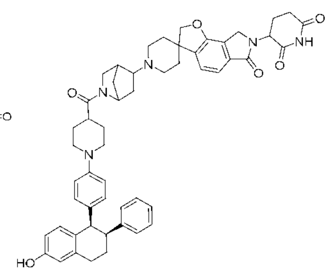

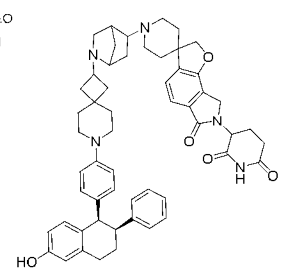

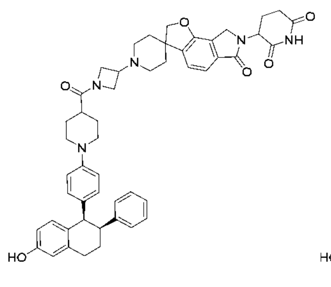

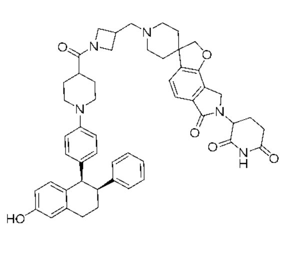

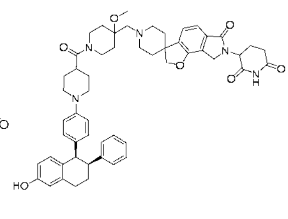

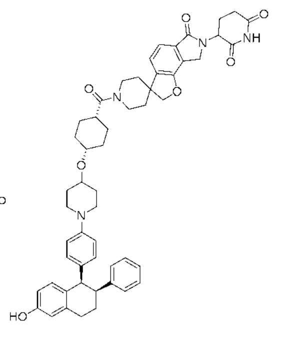

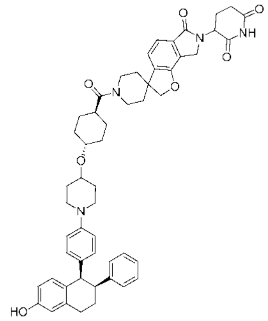

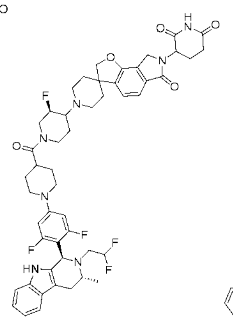

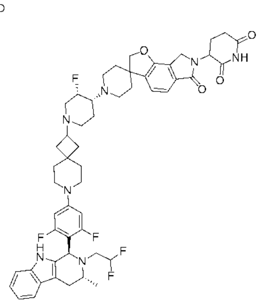

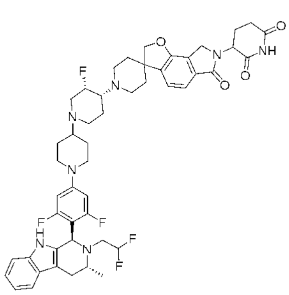

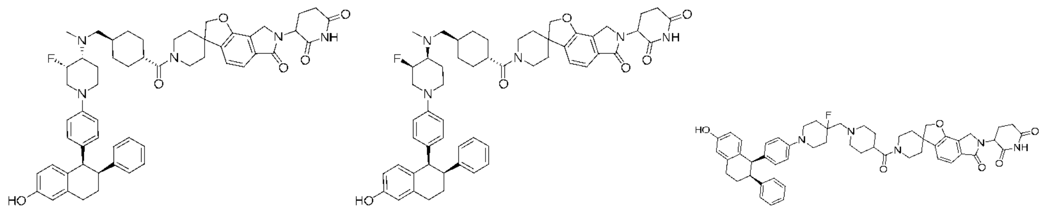

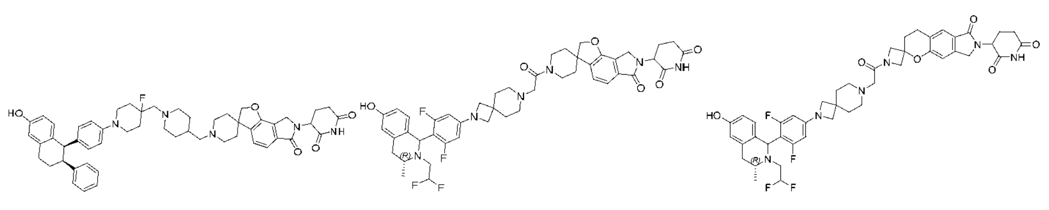

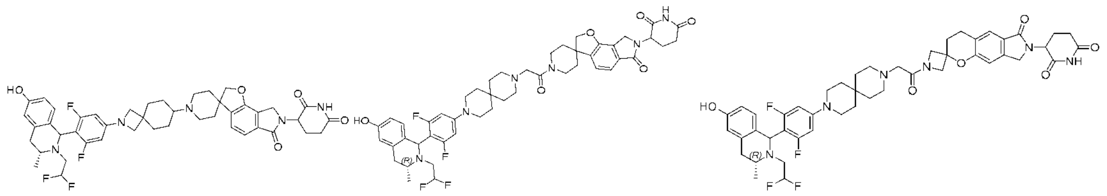

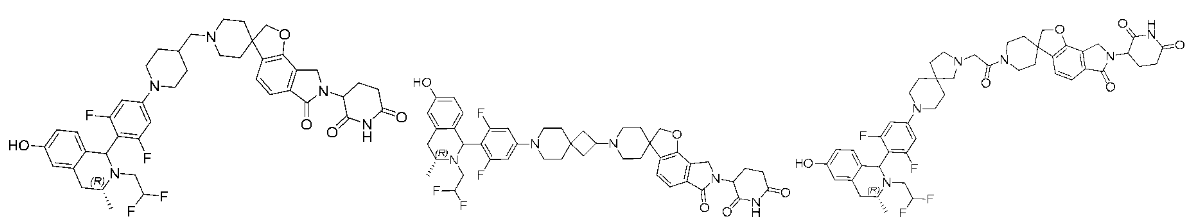

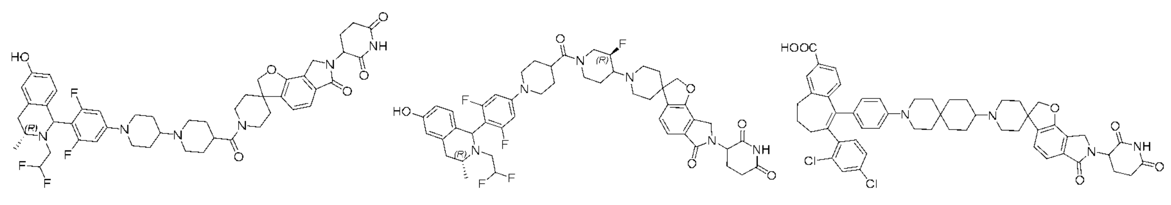

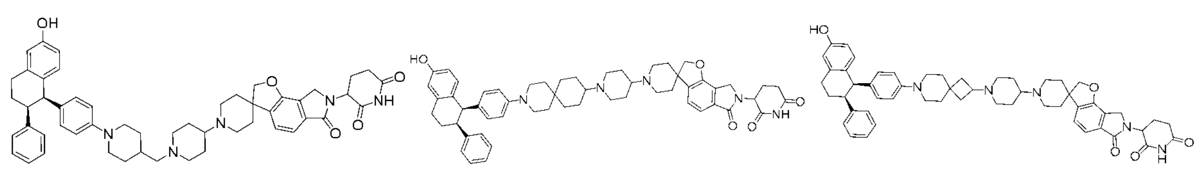

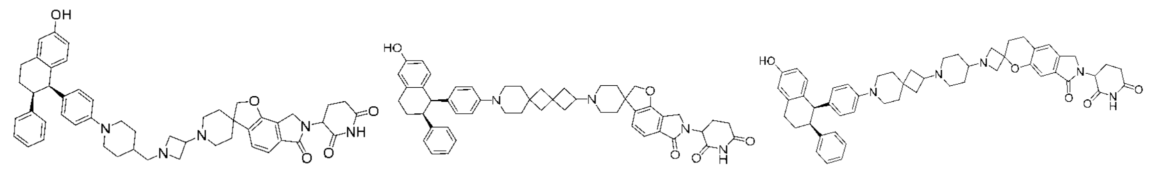

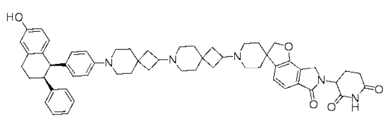
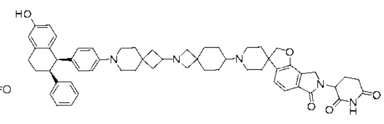
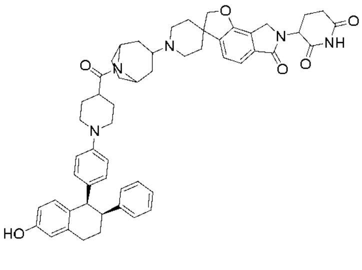

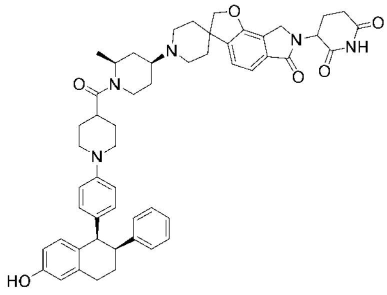
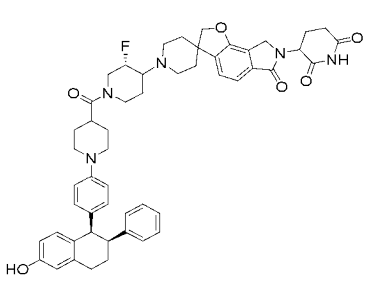
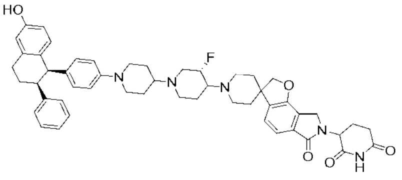

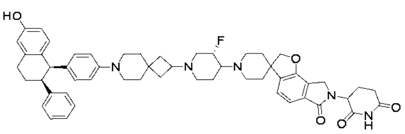
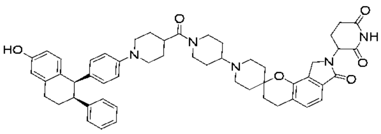
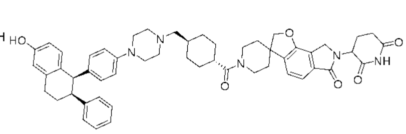

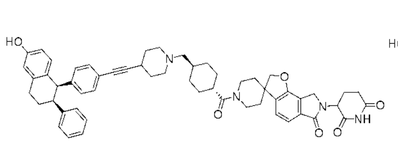
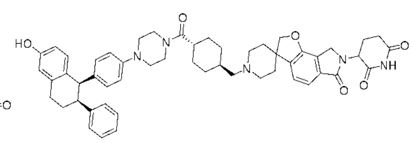
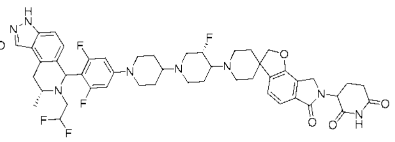

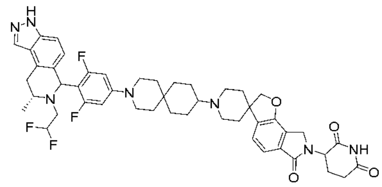
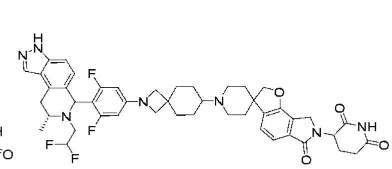
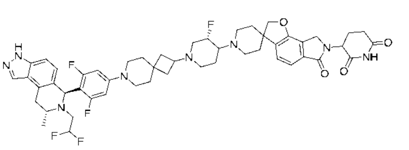

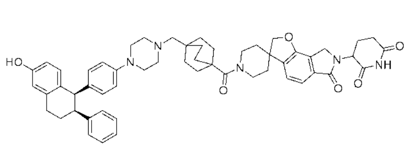
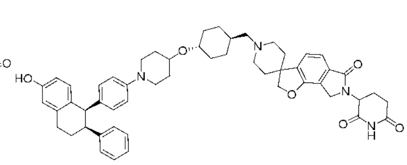
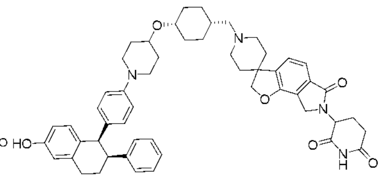

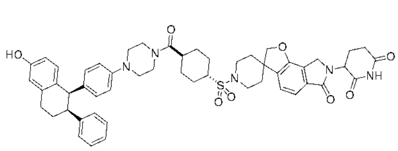
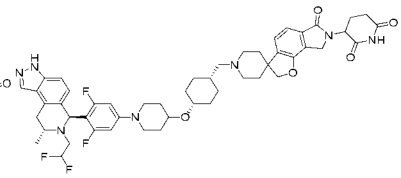
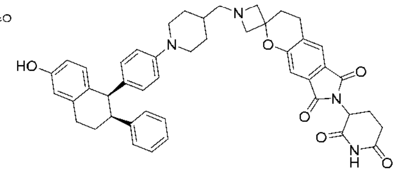

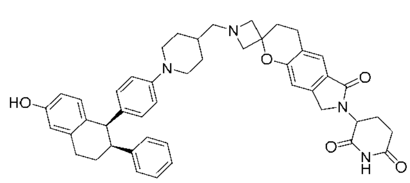
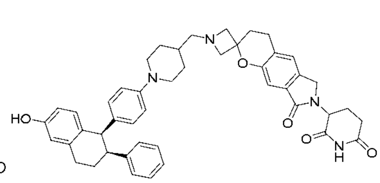
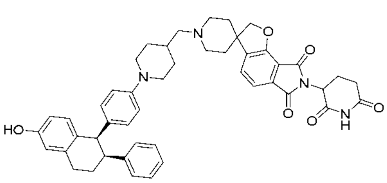

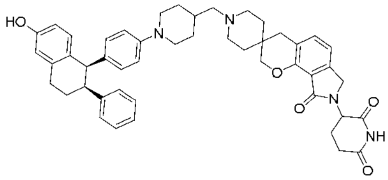
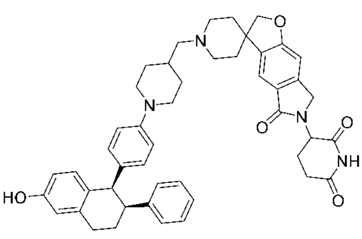
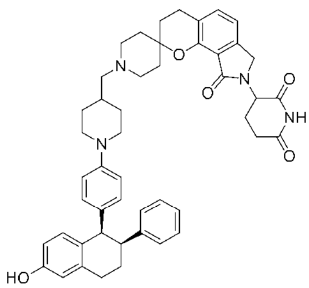
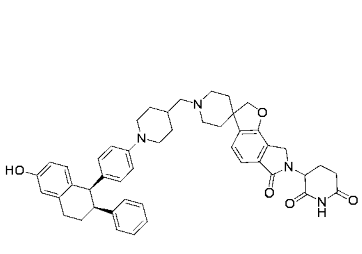
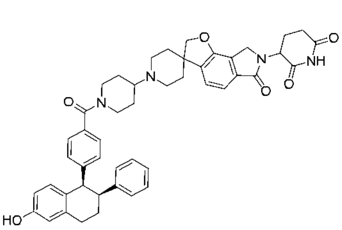
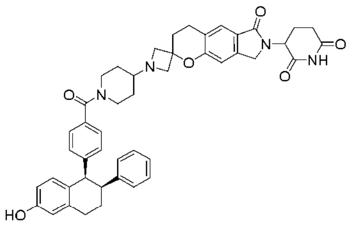
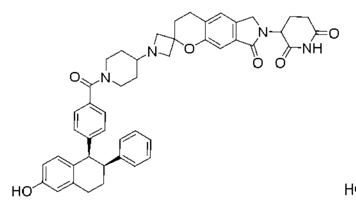
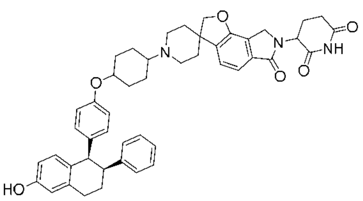
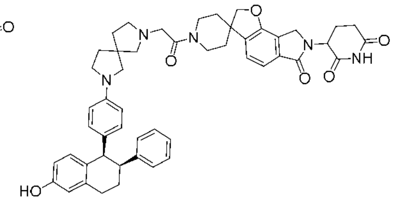
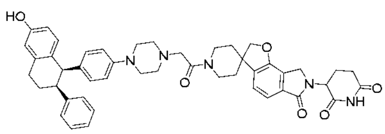
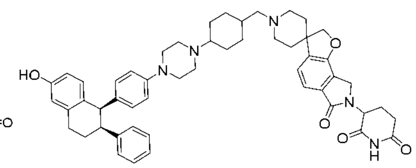
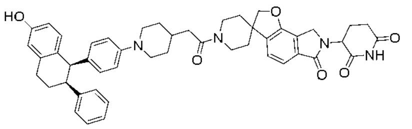
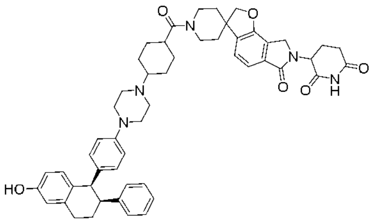
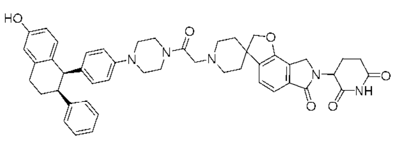
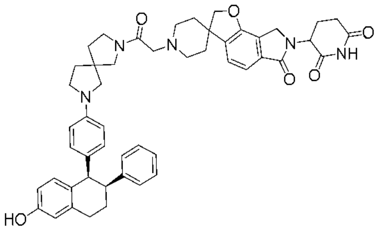
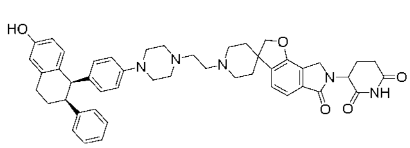
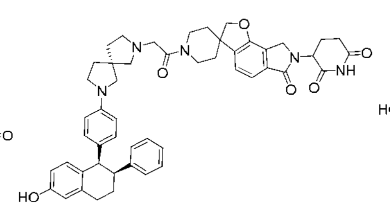
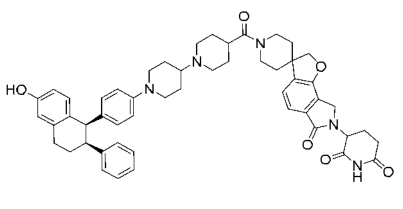
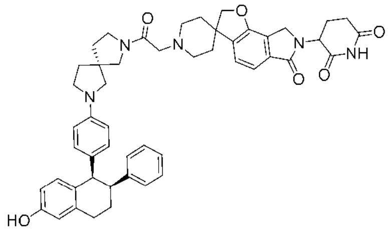
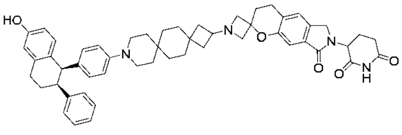
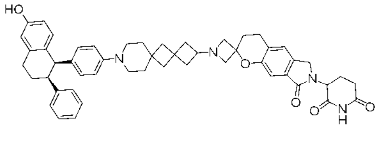

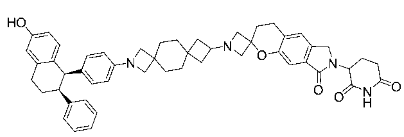
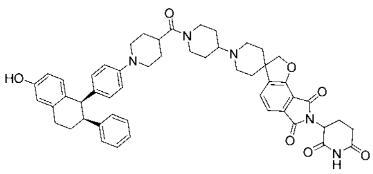
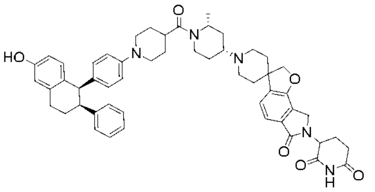

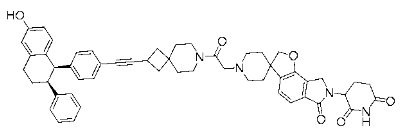
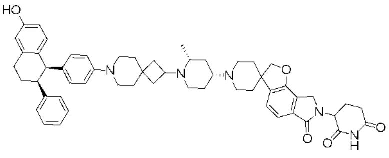
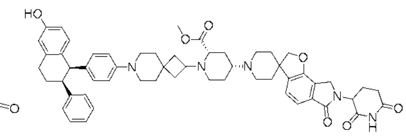

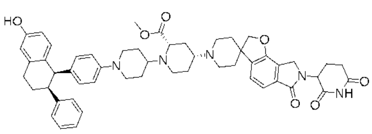
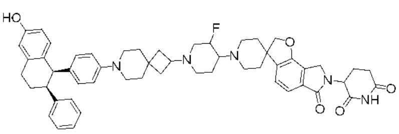
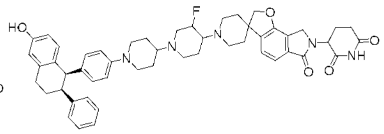

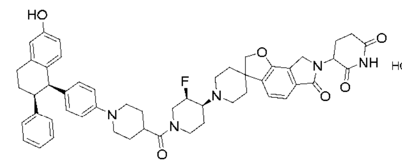
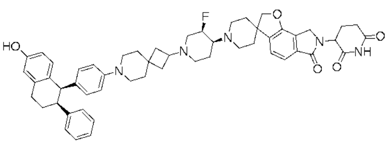
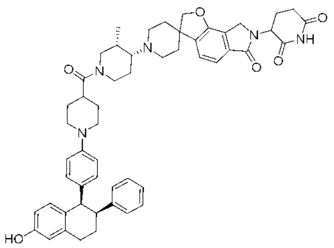

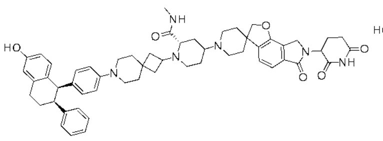
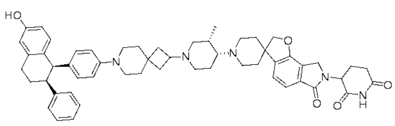
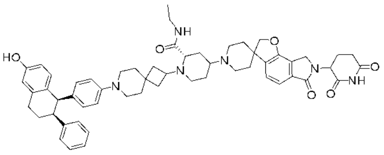

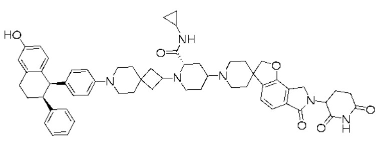
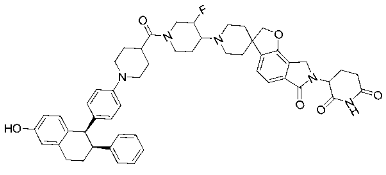
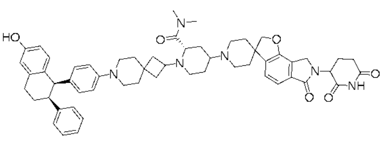

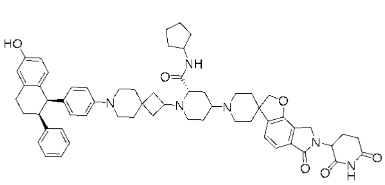
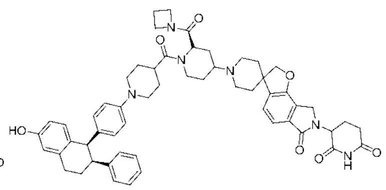
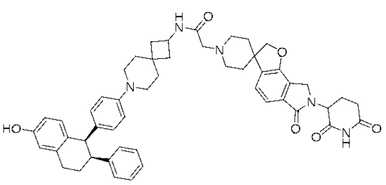

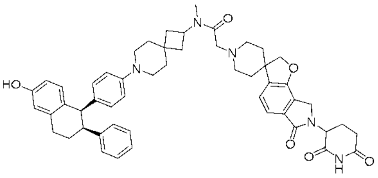
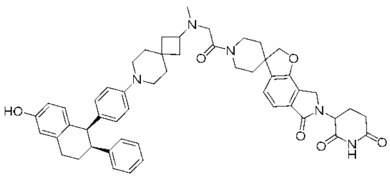
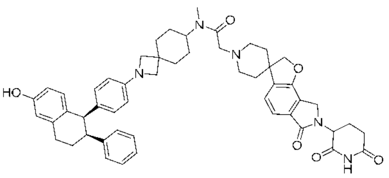

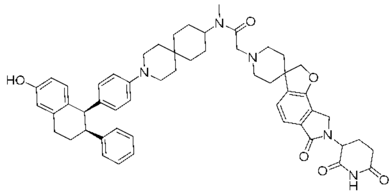
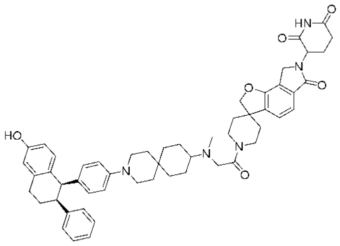
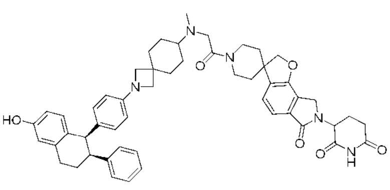

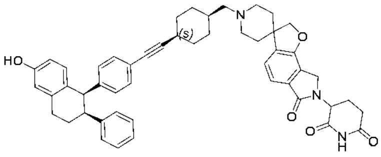
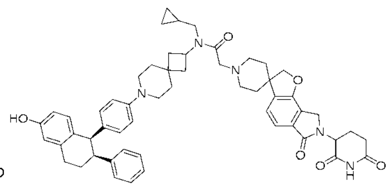
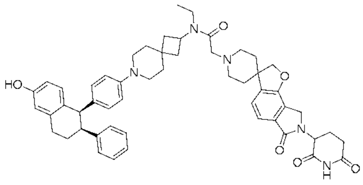

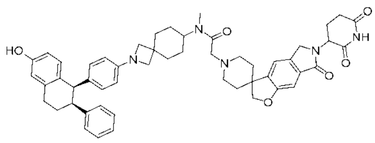
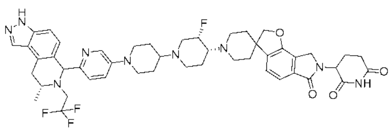
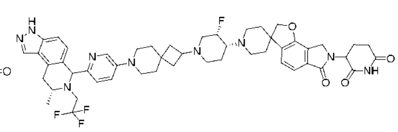

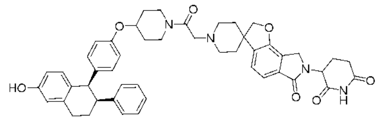
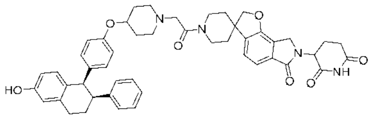
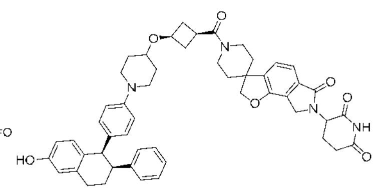

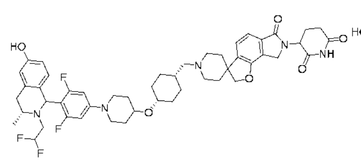
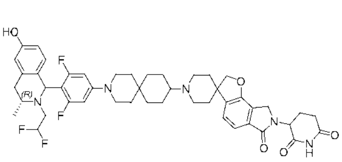
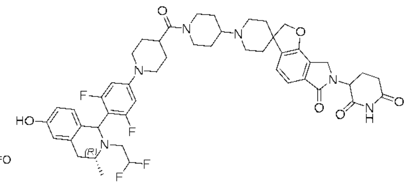

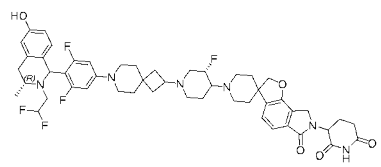
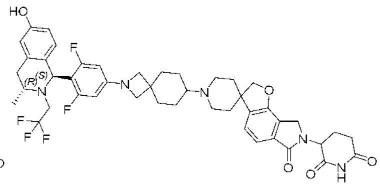
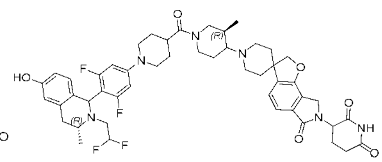

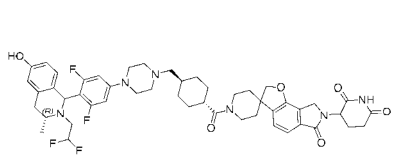
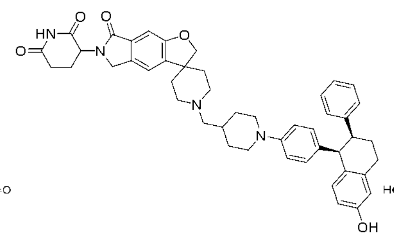
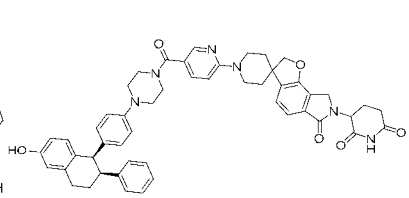

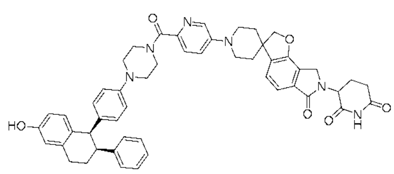
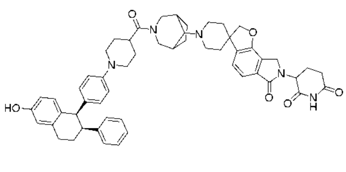
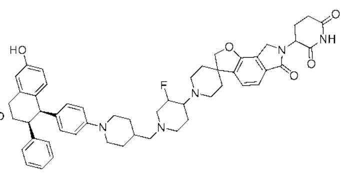
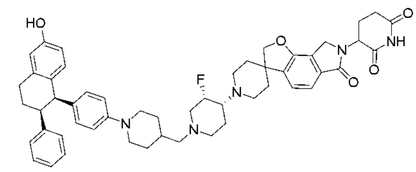
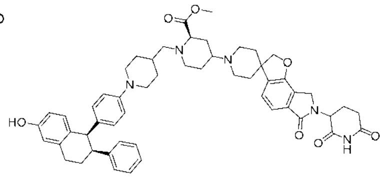
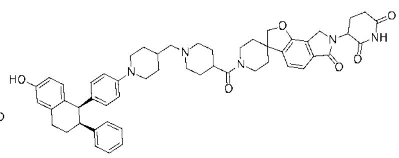
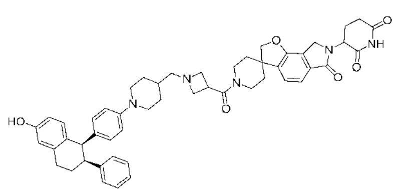
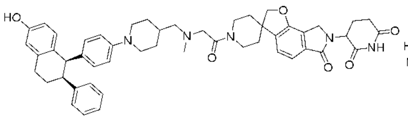
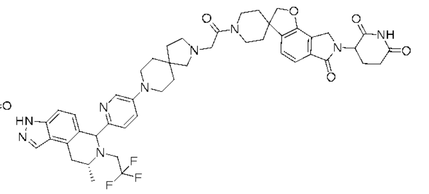
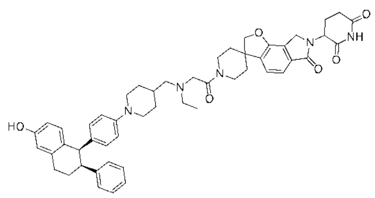
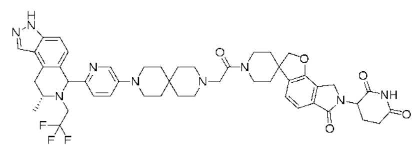
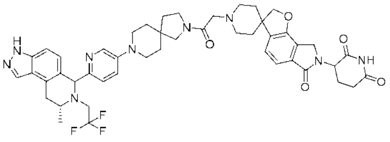
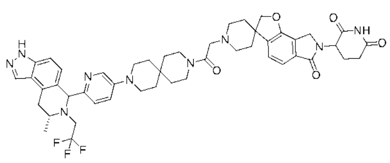
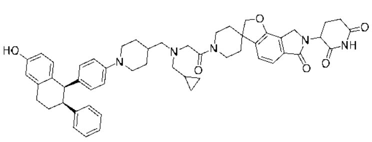
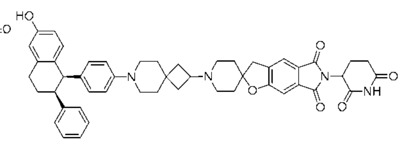
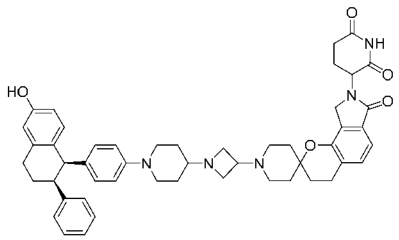
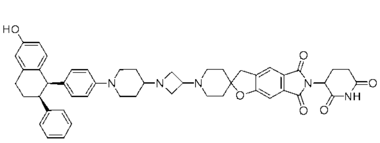
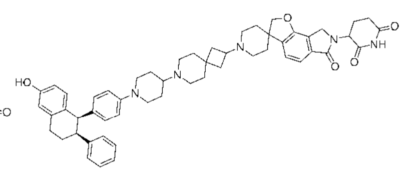
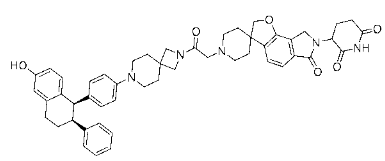
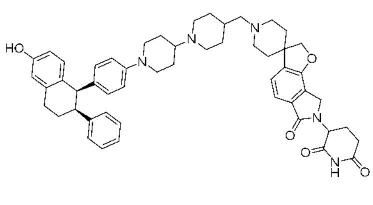
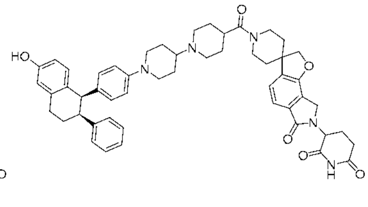
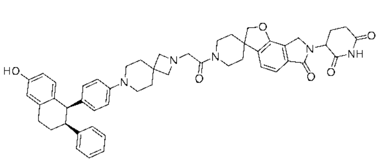
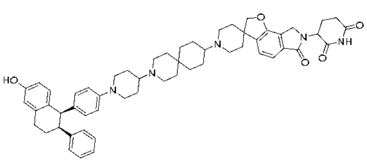
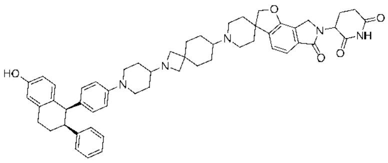

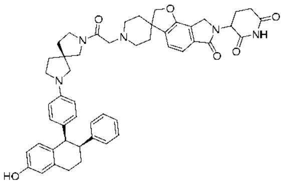
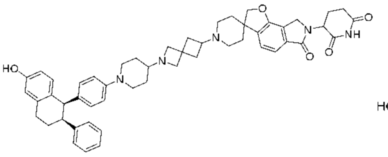
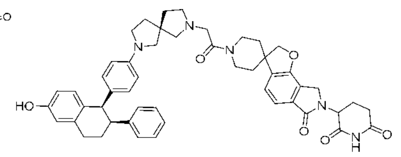

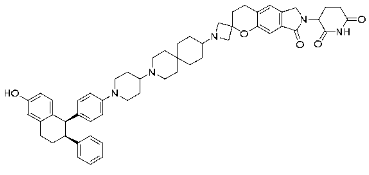
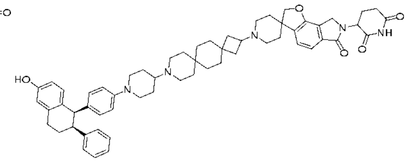
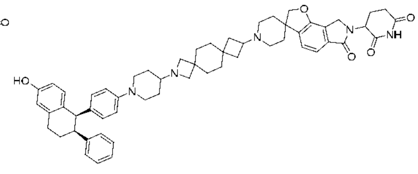

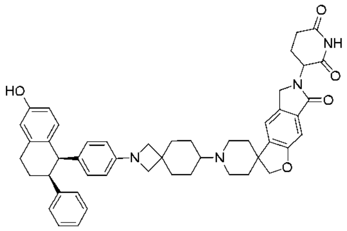
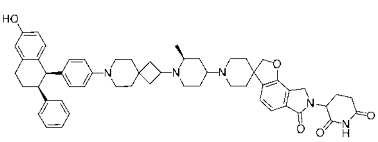
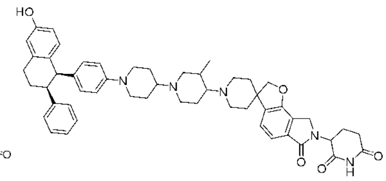

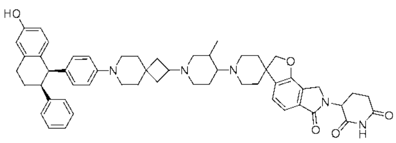
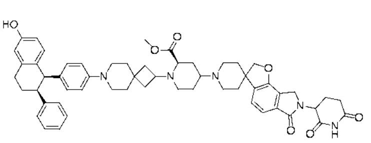
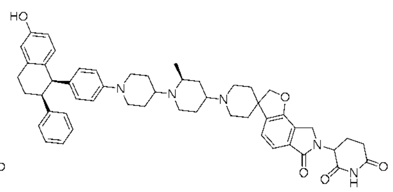

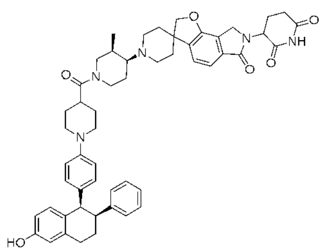
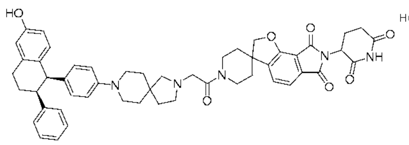
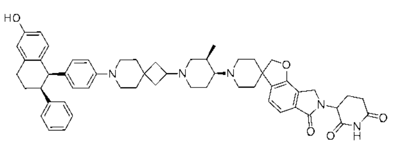

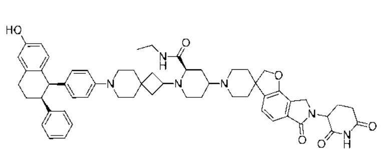
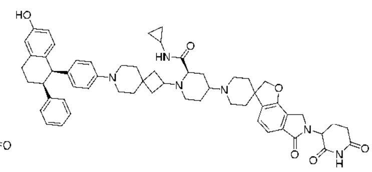
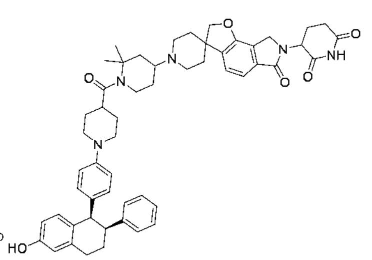

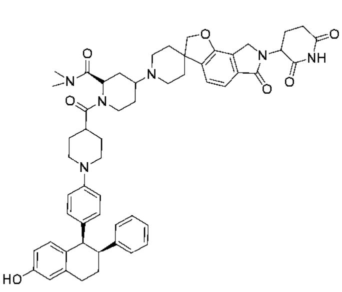
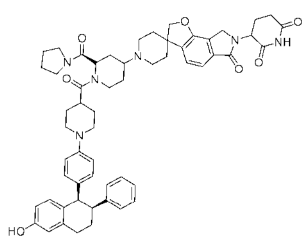
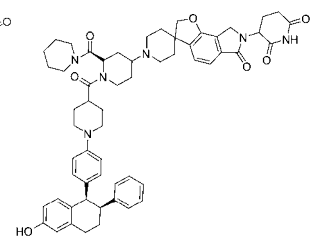

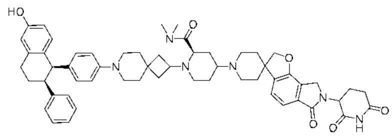
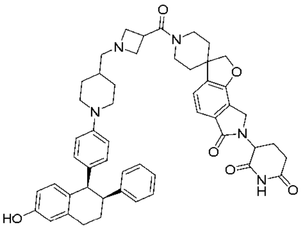
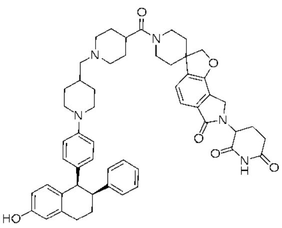

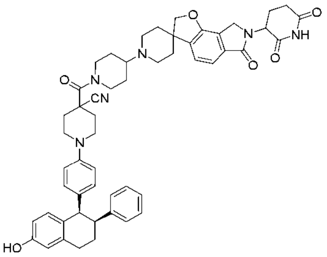
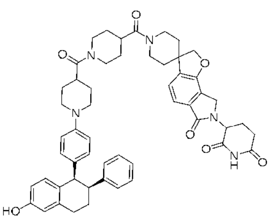
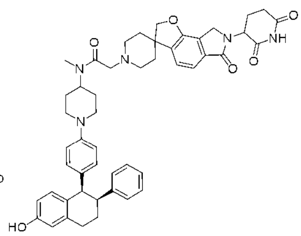
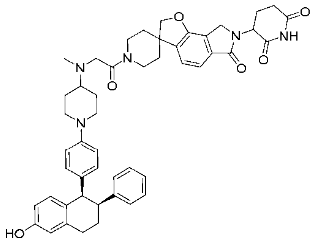

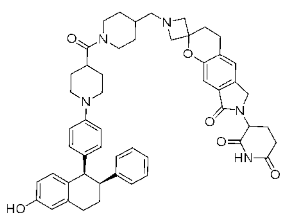
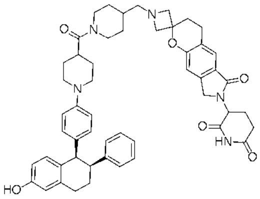
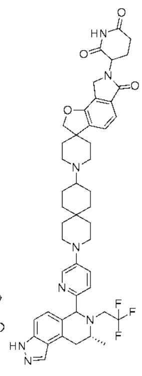
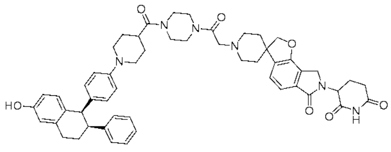

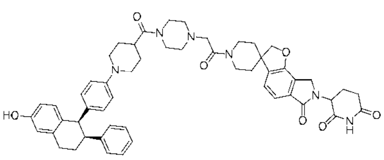
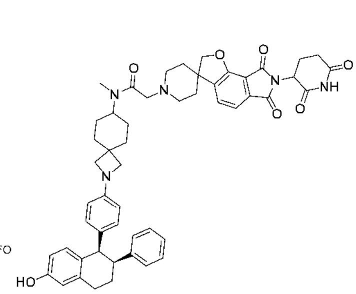
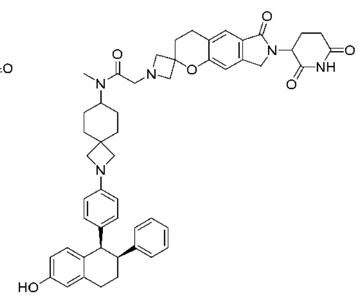

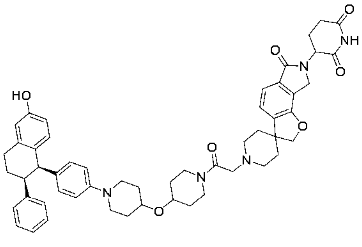
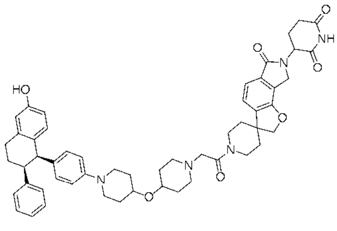
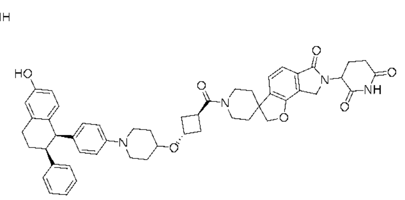

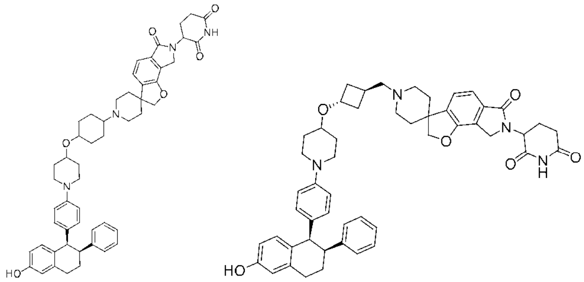
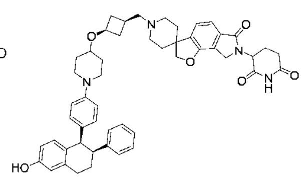
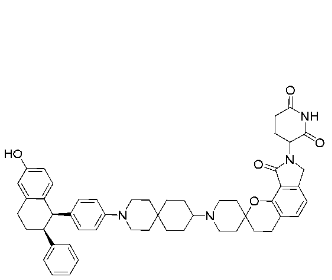

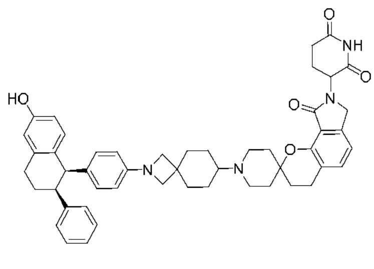
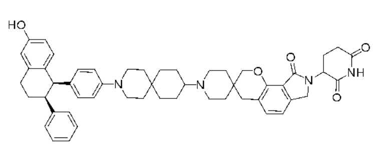
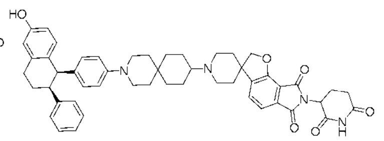

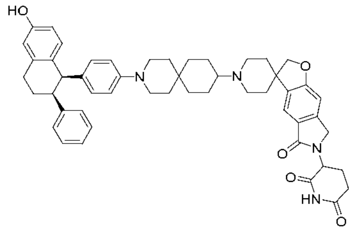
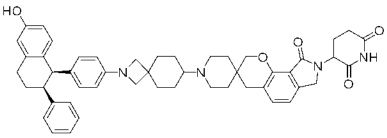
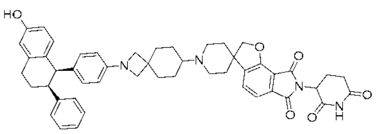

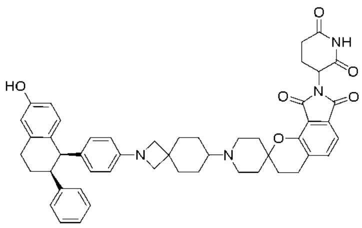
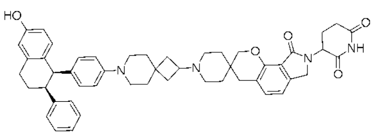
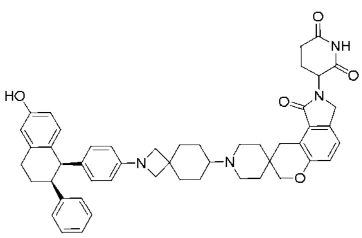

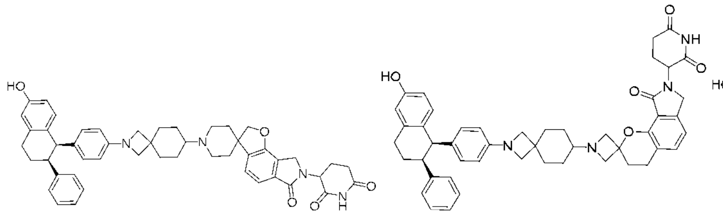
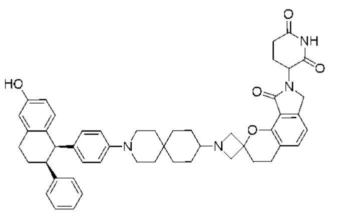

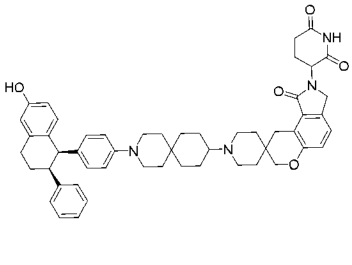
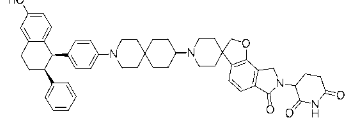
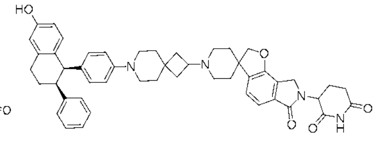

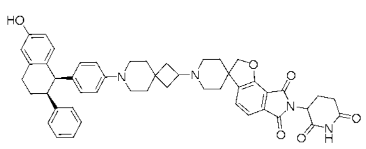
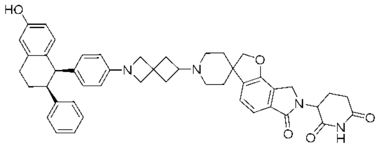
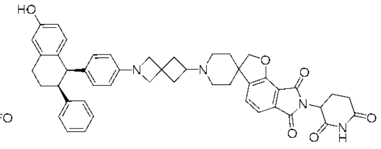

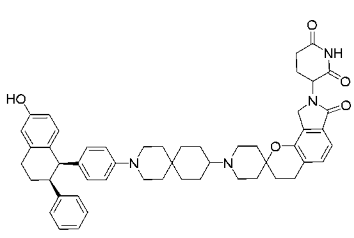
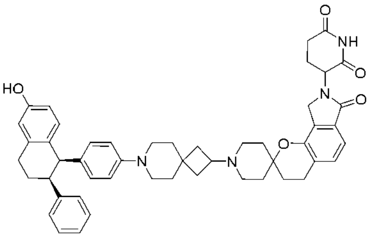
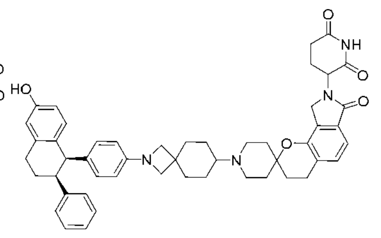

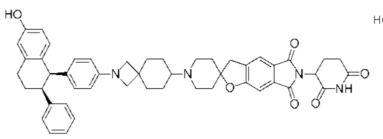
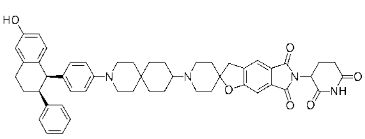
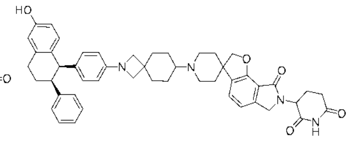

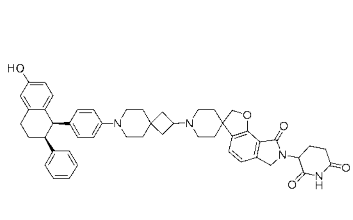
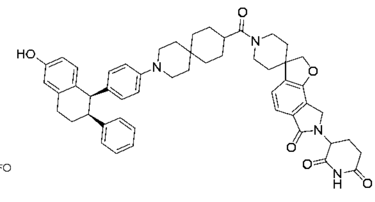
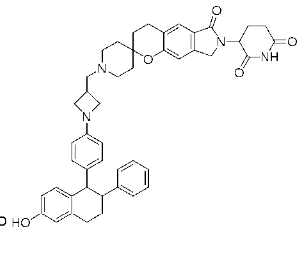

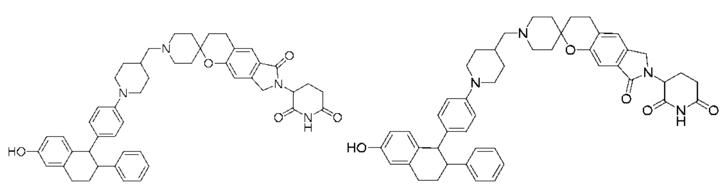
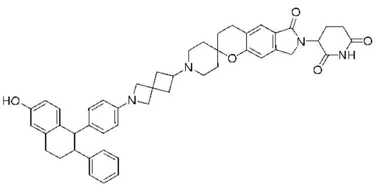

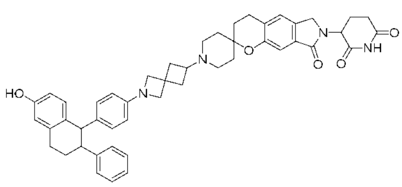
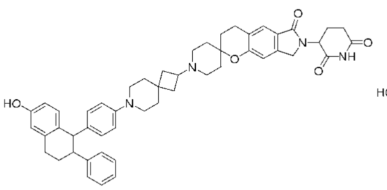
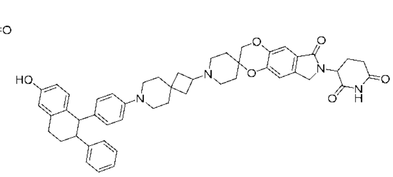

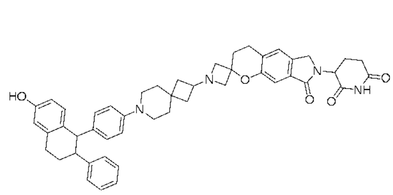
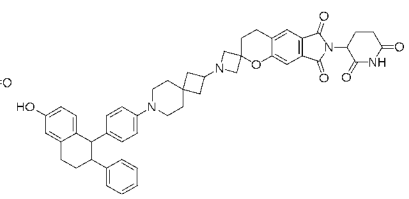
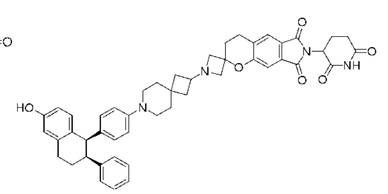

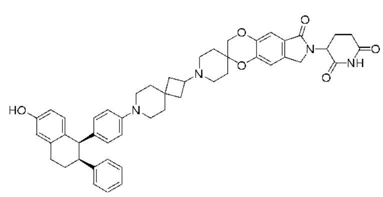
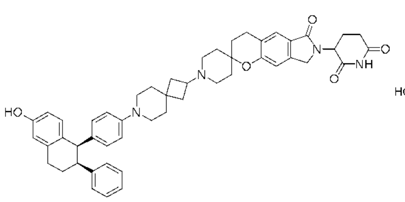
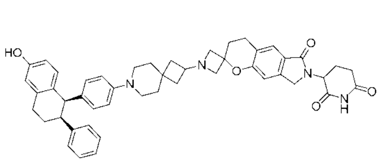

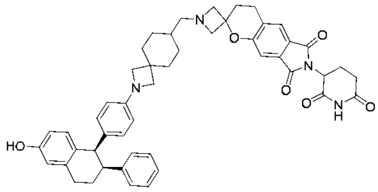
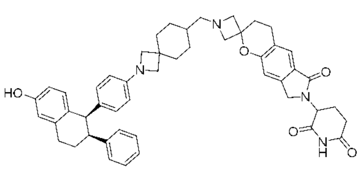
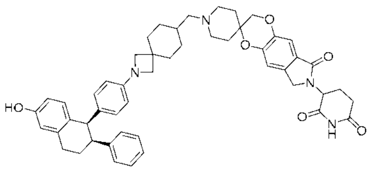

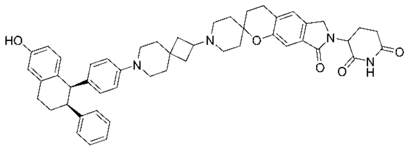
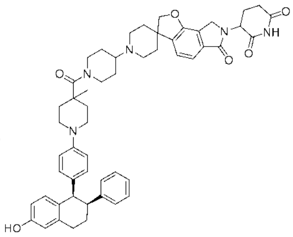
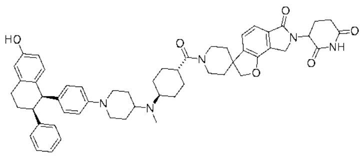

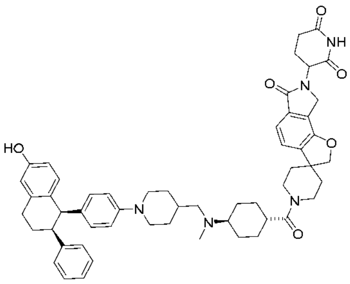
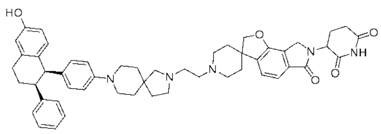
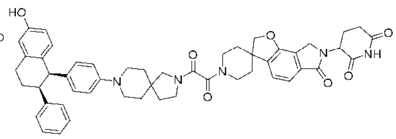

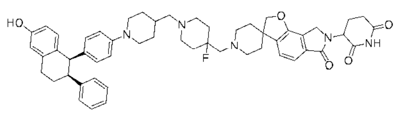
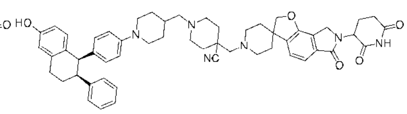
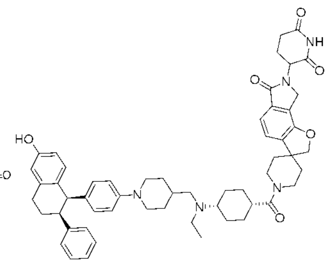

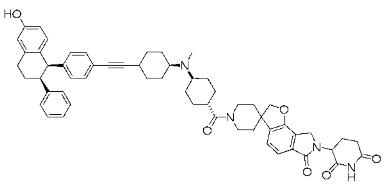
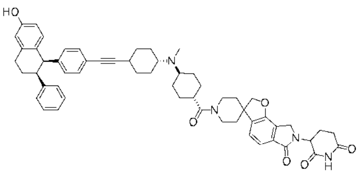
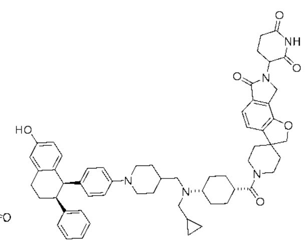

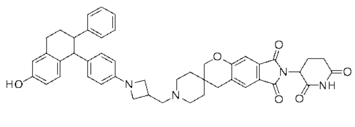
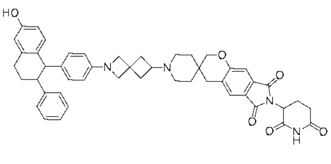
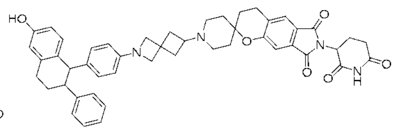

.

**16.** A pharmaceutical composition, wherein the pharmaceutical composition comprises the compound according to any one of claims 1 to 15.

**17.** Use of the compound according to any one of claims 1 to 15, or the pharmaceutical composition according to claim 16, in the manufacture of a medicament for treating or preventing a disease treated by degradation of estrogen receptor protein.

**18.** Use of the compound according to any one of claims 1 to 15, or the pharmaceutical composition according to claim 16, in the manufacture of a medicament for treating or preventing a disease treated by binding to cerebellar proteins in vivo.

**19.** Use of the compound according to any one of claims 1 to 15, or the pharmaceutical composition according to claim 16, in the treatment or prevention of a disease associated with accumulation and/or aggregation of a target protein, wherein the target protein is an estrogen receptor.

**20.** The use according to any one of claims 17 to 19, wherein the disease is a tumor or cancer, preferably the tumor or cancer is breast cancer, ductal breast carcinoma, prostate cancer, mantle cell lymphoma, chronic myeloid leukemia, acute myeloid leukemia, myelomonocytic leukemia, non-small cell lung cancer, lung adenocarcinoma, and/or cervical cancer.

**21.** The compound according to any one of claims 1 to 15, or the pharmaceutical composition according to claim 16, for use in the treatment or prevention of a disease treated by degradation of estrogen receptor protein.

**22.** The compound according to any one of claims 1 to 145, or the pharmaceutical composition according to claim 16, for use in the treatment or prevention of a disease treated by binding to cerebellar proteins in vivo.

**23.** The compound according to any one of claims 1 to 15, or the pharmaceutical composition according to claim 14, for use in the treatment or prevention of a disease associated with accumulation and/or aggregation of a target protein, wherein the target protein is an estrogen receptor.

**24.** The compound or the pharmaceutical composition for use according to any one of claims 21 to 23, wherein the disease is a tumor or cancer, preferably the tumor or cancer is breast cancer, ductal breast carcinomat, prostate cancer, mantle cell lymphoma, chronic myeloid leukemia, acute myeloid leukemia, myelomonocytic leukemia, non-small cell lung cancer, lung adenocarcinoma, and/or cervical cancer.

**25.** A method for treating or preventing a disease treateded by degradation of estrogen receptor protein, comprising administering to a subject in need thereof a therapeutically effective amount of the compound according to any one of claims 1 to 15, or the pharmaceutical composition according to claim 16.

**26.** A method for treating or preventing a disease treateded by binding to cerebellar proteins in vivo, comprising administering to a subject in need thereof a therapeutically effective amount of the compound according to any one of claims 1 to 15, or the pharmaceutical composition according to claim 16.

**27.** A method for treating or preventing a disease associated with accumulation and/or aggregation of a target protein, comprising administering to a subject in need thereof a therapeutically effective amount of the compound according to any one of claims 1 to 15, or the pharmaceutical composition according to claim 16, wherein the target protein is an estrogen receptor.

**28.** The method according to any one of claims 25 to 27, wherein the disease is a tumor or cancer, preferably the tumor or cancer is breast cancer, ductal breast carcinoma, prostate cancer, mantle cell lymphoma, chronic myeloid leukemia, acute myeloid leukemia, myelomonocytic leukemia, non-small cell lung cancer, lung adenocarcinoma, and/or cervical cancer.

**29.** A compound or an isomer, an isotopic derivative, a polymorph, a prodrug, or a pharmaceutically acceptable salt or a solvate thereof, wherein the compound is selected from any one of the following structures:

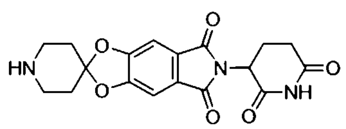 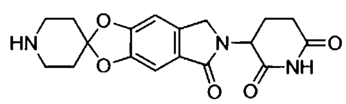 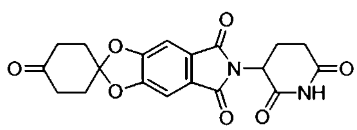

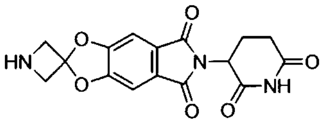
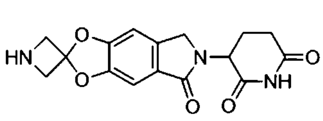
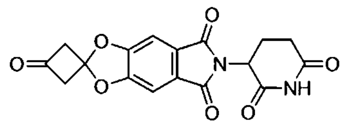

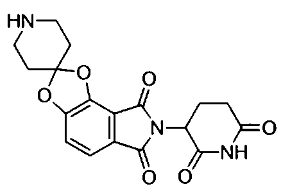
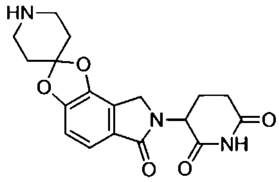
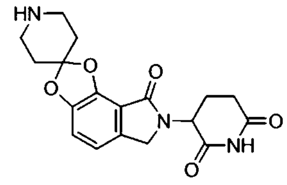

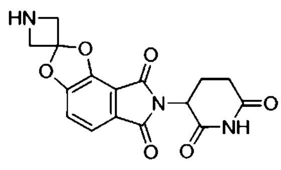
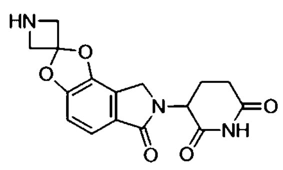
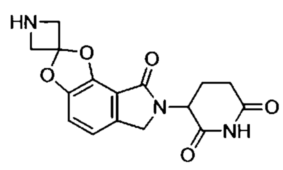

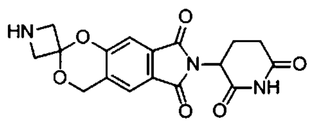
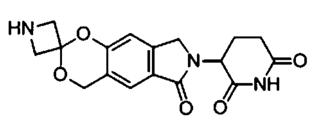
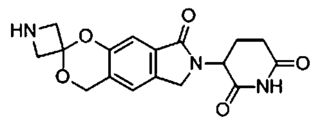

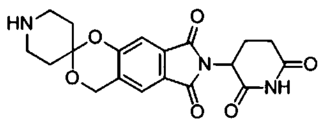
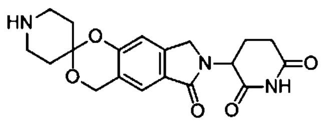
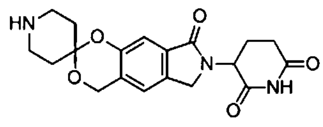

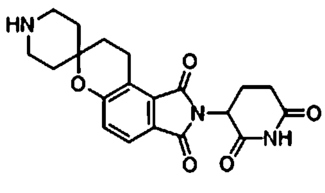
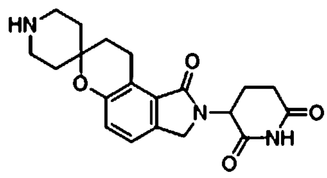
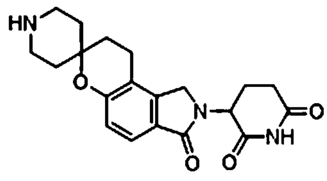

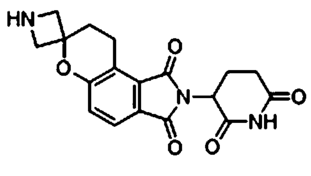
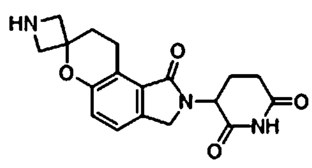
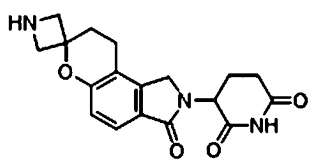

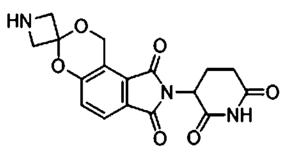
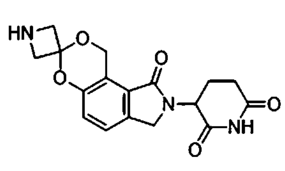
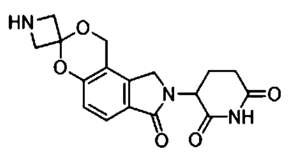

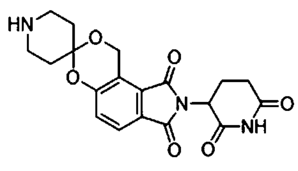
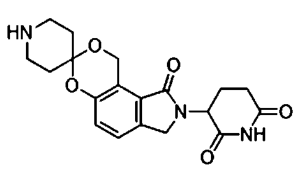
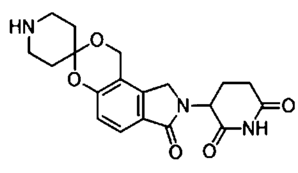

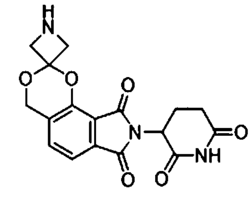
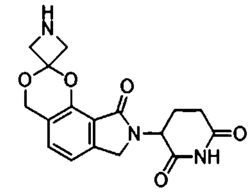
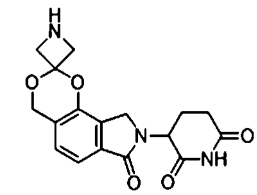

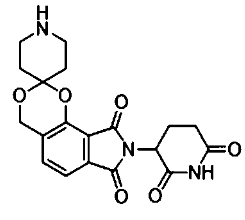
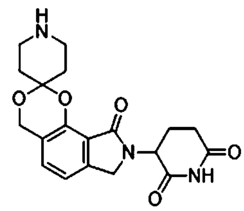
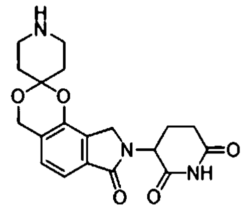

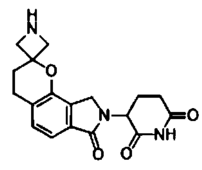
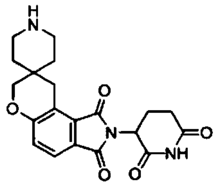
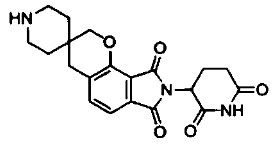

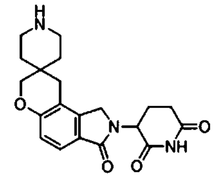
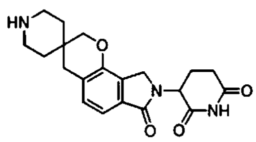
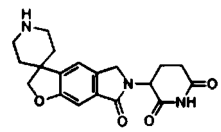
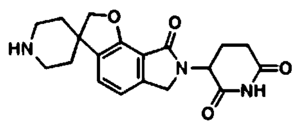

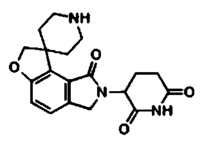
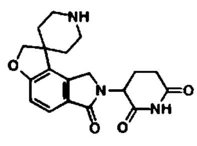
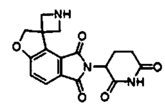
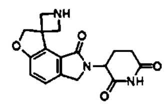
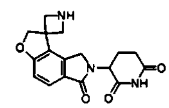

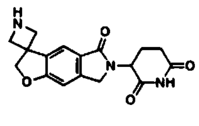
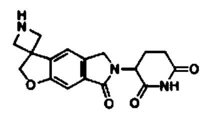
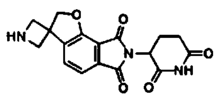
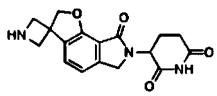
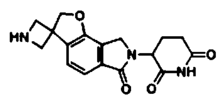

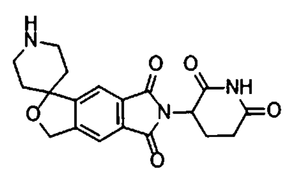
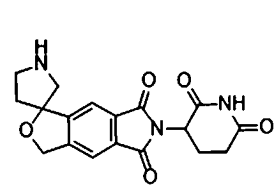
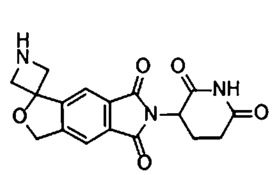

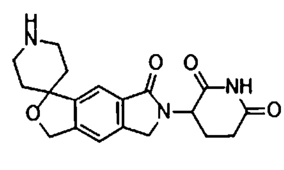
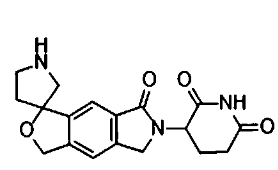
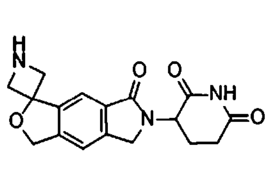

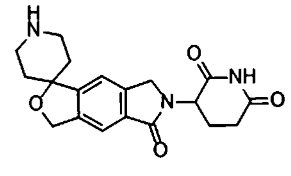 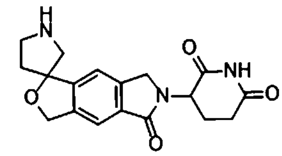 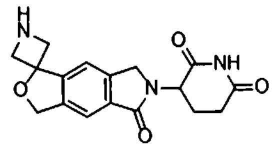

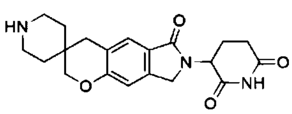, 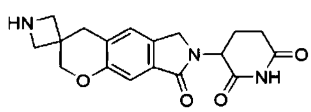, 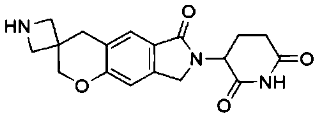,

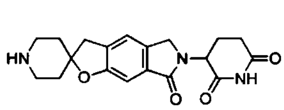, 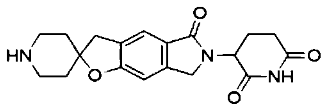, 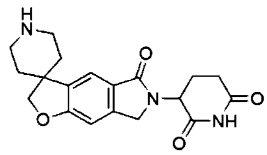,

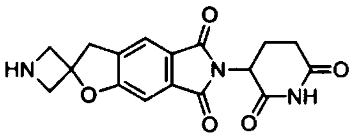, 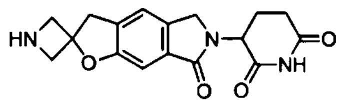, 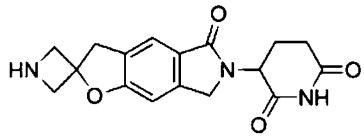,

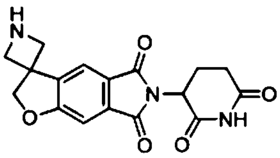, 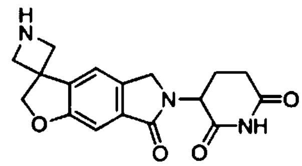, 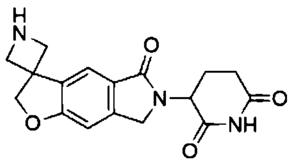,

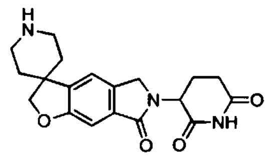, 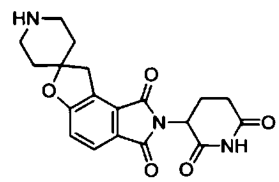, 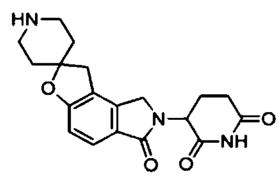,

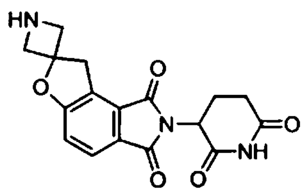, 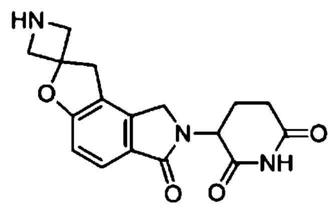, 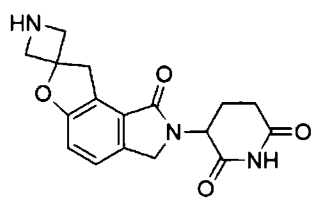,

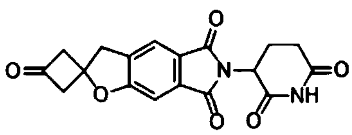, 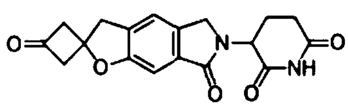, 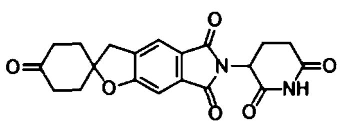,

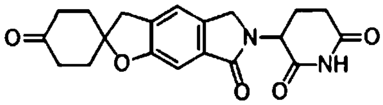 , 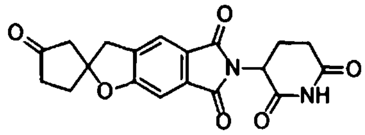 , 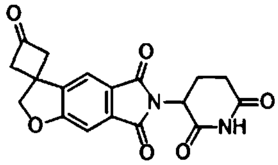 ,

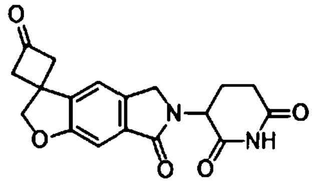 , 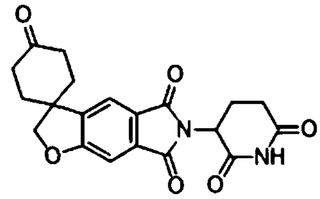 , 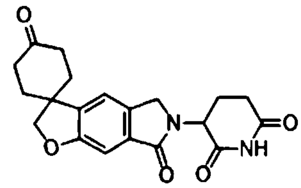 ,

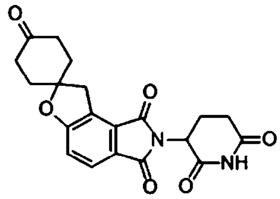 , 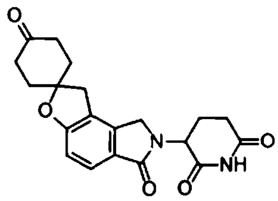 , 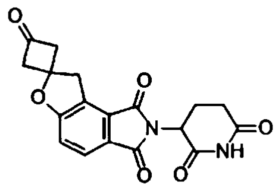 , 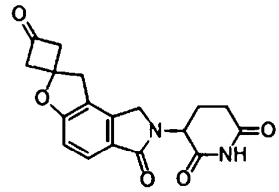 ,

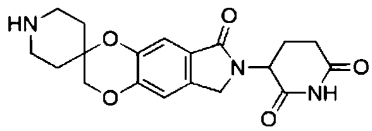 , 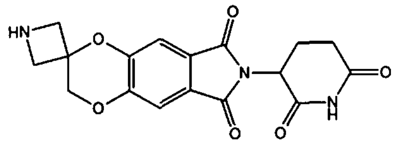 , 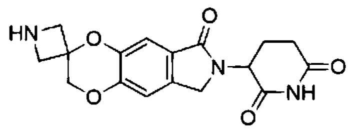 ,

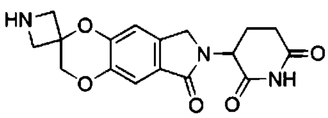 , 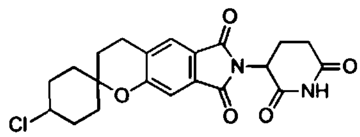 , 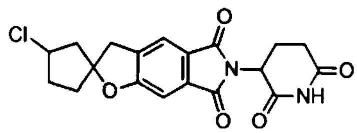 ,

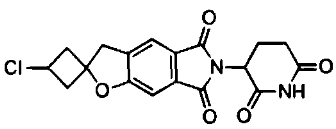 , 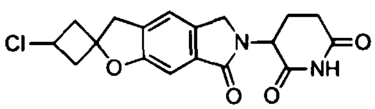 , 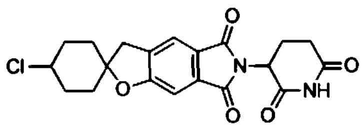 ,

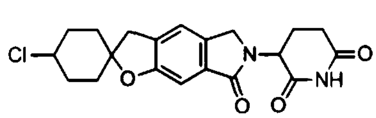 , 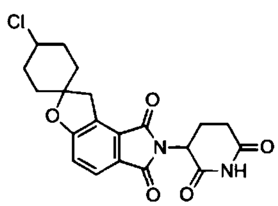 , 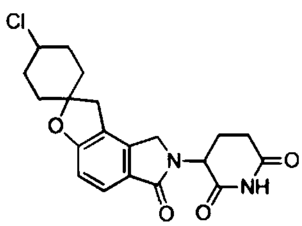 ,

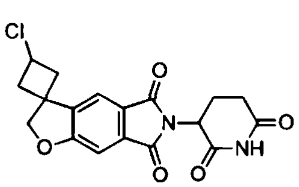 , 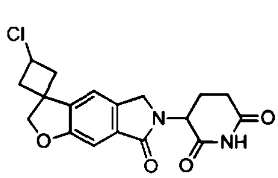 , 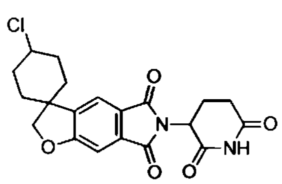 , 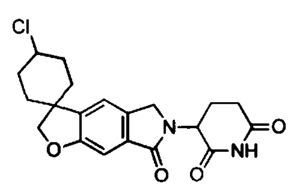 ,

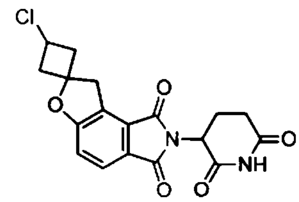,
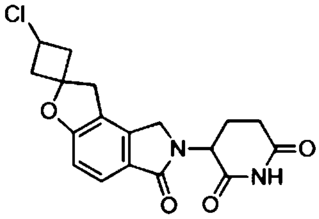,
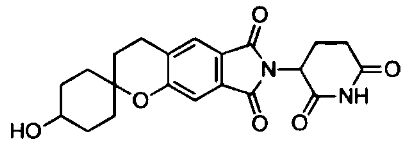,
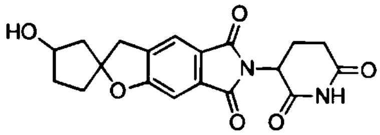,
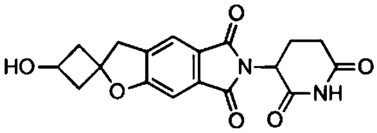,
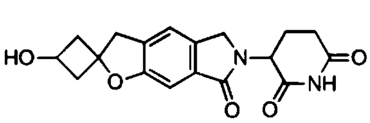,
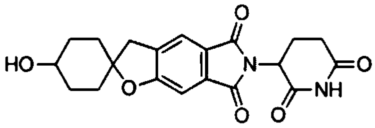,
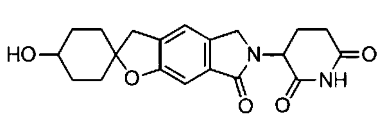,
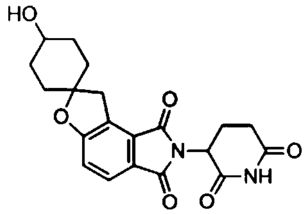,
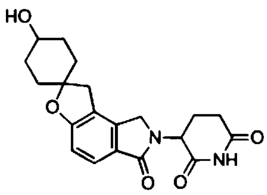,
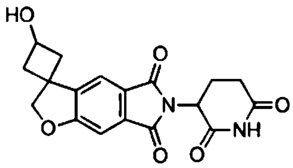,
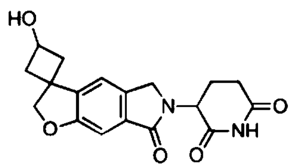,
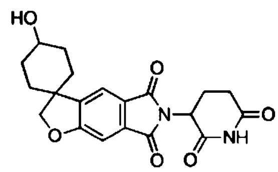,
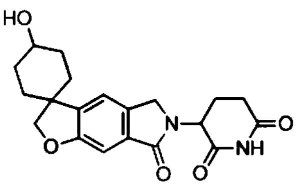,
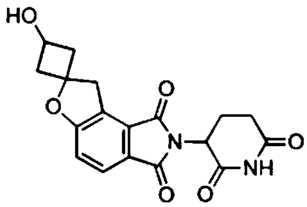,
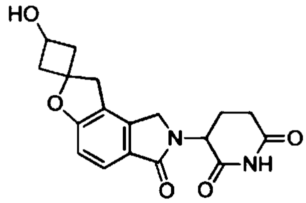,
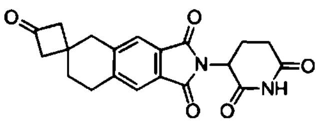,
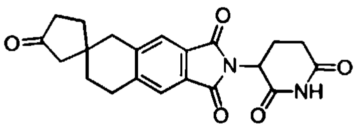,
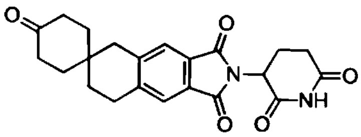,
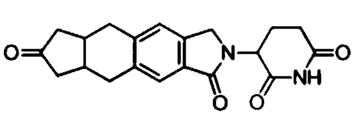,
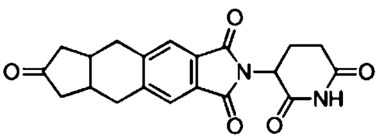,
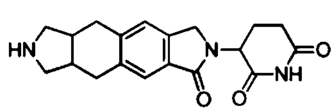,
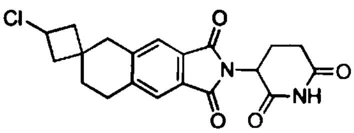,
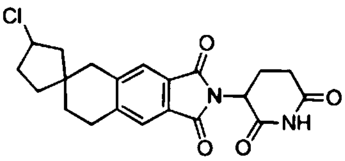,
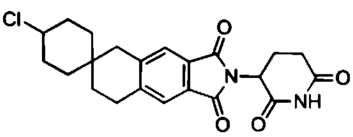,

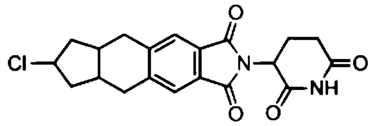, 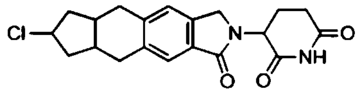, 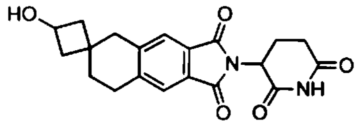,

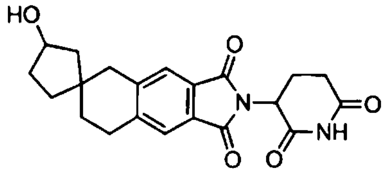, 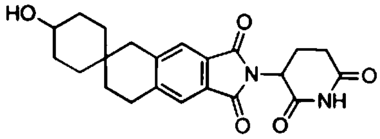, 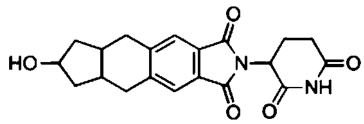,

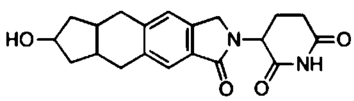, 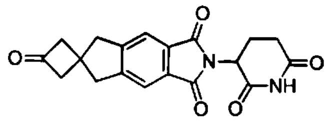, 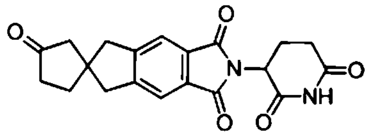,

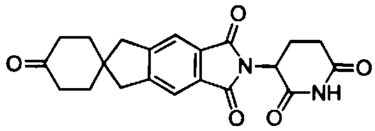, 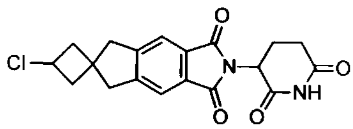, 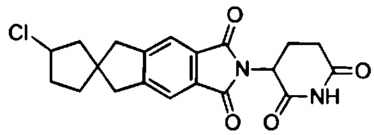,

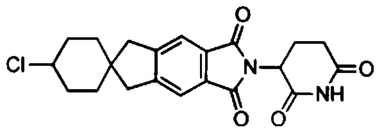, 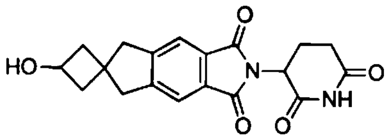, 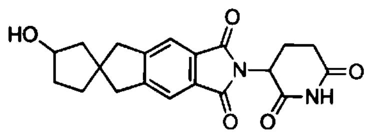,

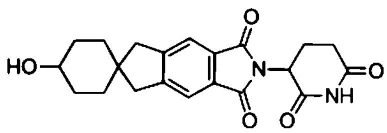, 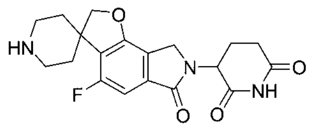, 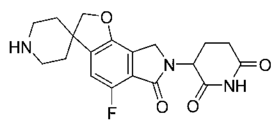,

and

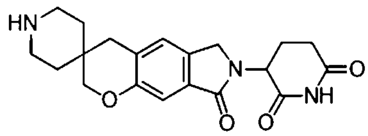.

**30.** A compound, wherein the compound is a compound having the following chemical structure:

$CLM_a$-$L_a$-$PTM_a$,

or an isomer, an isotopic derivative, a polymorph, a prodrug, or a pharmaceutically acceptable salt or a solvate thereof,
wherein:

$PTM_a$ is a target protein binding moiety; preferably, the target protein is selected from B7.1,B7, TNFR2, NADPH oxidase, Bcl/Bax and other adaptor in the apoptosis pathway, C5a receptor, HMG-CoA reductase,

PDE V phosphodiesterase, PDE IV phosphodiesterase (type 4), PDE I I, PDE I II, PDE III phosphodiesterase, squalene epoxidase, CXCR1, CXCR2, nitric oxide (NO) synthase, cyclooxygenase-1, cyclooxygenase-2, 5-HT receptor, dopamine receptor, G protein (i.e., Gq), histamine receptor, 5-lipoxygenase, trypsin-like serine protease, thymidylate synthase, purine nucleoside phosphorylase, GAPDH trypanosome, glycogen phosphorylase, carbonic anhydrase, chemokine receptor, JAK-STAT, RXR and an analogue thereof, HIV-1 protease, HIV-1 integrase, influenza neuraminidase, hepatitis B reverse transcriptase, sodium channel, multidrug-resistant bacteria, P-glycoprotein, tyrosine kinase, CD23, CD124, tyrosine kinase p561ck, CD4, CD5, IL-2 receptor, IL-1 receptor, TNF-$\alpha$ receptor, ICAM-1, Cat+ channel, VCAM, VLA-4 integrin, selectin, CD40/CD40L, inosine monophosphate dehydrogenase, p38 MAP kinase, Ras/Raf/MEK/ERK pathway, interleukin-1 converting enzyme, caspase, HCV,

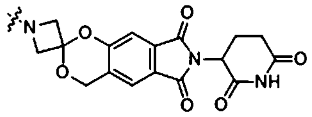 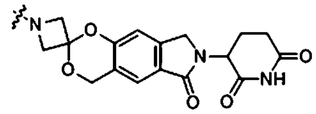 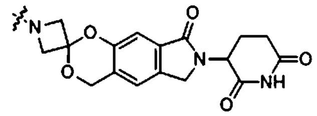

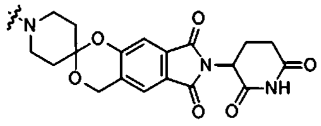 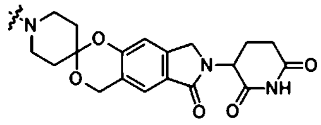 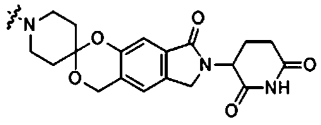

NS3 protease, HCV NS3 RNA helicase, glycinamide rbonucleotide transformylase, rhinovirus, 3C protease, herpes simplex virus type 1 (HSV-1) protease, cytomegalovirus (CMV) protease, poly(ADP-ribose) polymerase, cyclin-dependent kinase 4/6, vascular endothelial growth factor, oxytocin receptor, microsomal transfer protein inhibitor, bile acid transport inhibitor, 5$\alpha$-reductase inhibitor, angiotensin II, glycine receptor, norepinephrine reuptake receptor, endothelin receptor, neuropeptide Y and a receptor thereof, adenosine receptor, adenosine kinase and AMP deaminase, purinergic receptors (P2Y1, P2Y2, P2Y4, P2Y6, P2X1-7), farnesyltransferase, geranylgeranyltransferase, NGF TrkA receptor, $\beta$-amyloid protein, tyrosine kinase Flk-II KDR, vitronectin receptor, integrin receptor, Her-21 nerve sheath, telomerase inhibitors, cytosolic phospholipase A2, EGF receptor tyrosine kinase, ecdysone 20-monooxygenase, GABA-gated chloride ion channel, acetylcholinesterase, voltage-sensitive sodium channel protein, calcium release channel and chloride ion channel, acetyl-CoA carboxylase, adenylosuccinate synthase, protoporphyrinogen oxidase, enolpyruvylshikimate phosphate synthase, MYC protein, androgen receptor, estrogen receptor, aurora kinase A, Bcr-Ab1 protein, epidermal growth factor receptor, anaplastic lymphoma kinase, Bruton's tyrosine kinase, or bromodomain-containing protein 4;

$L_a$ is a bond or a chemical linker moiety that covalently connects $CLM_a$ and the $PTM_a$; and

the $CLM_a$ is a cerebellar protein E3 ubiquitin ligase binding moiety and selected from any one of the following structures:

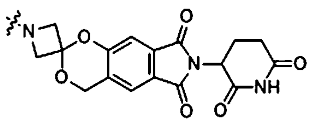 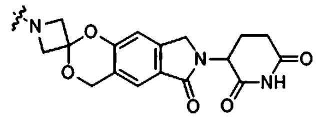 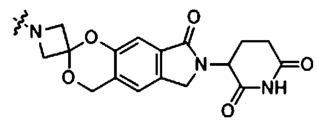

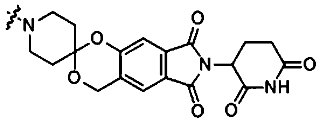 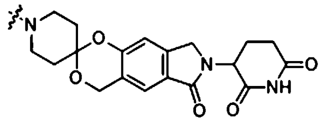 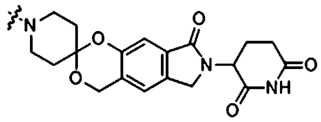

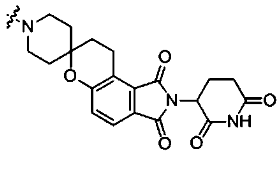 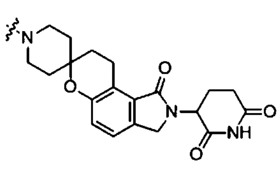 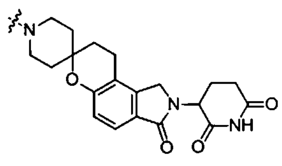

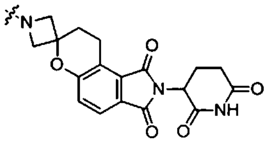 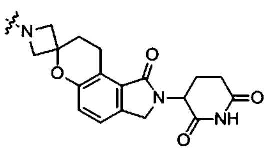 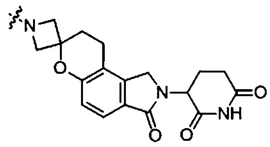

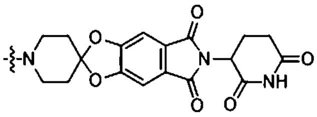 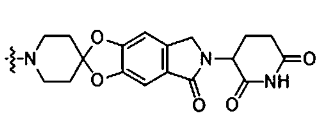 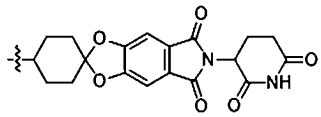

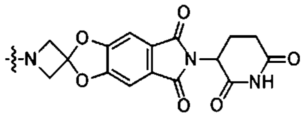 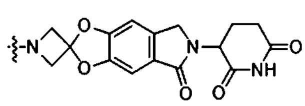 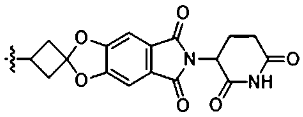

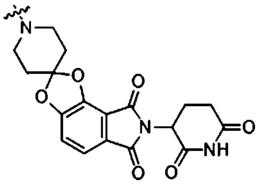 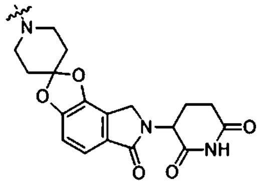 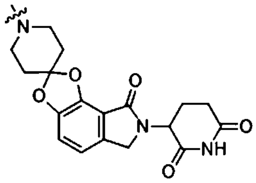

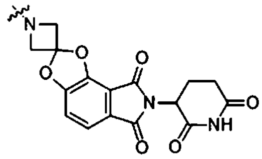 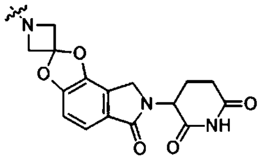 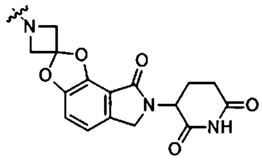

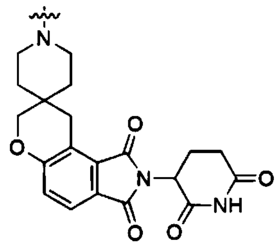 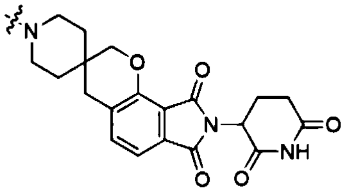 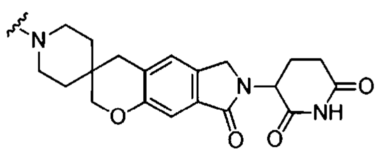

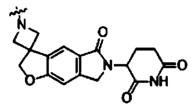 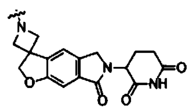 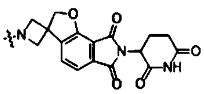 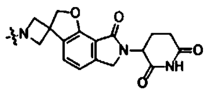 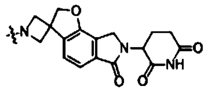

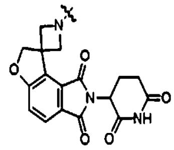 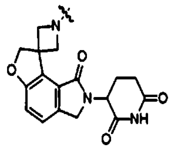 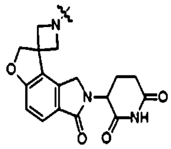 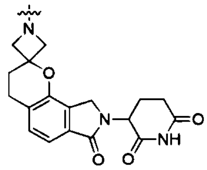 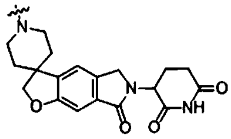

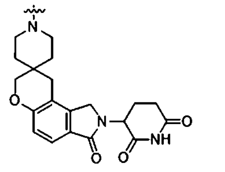
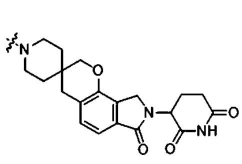
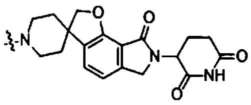
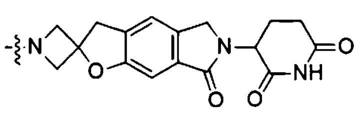

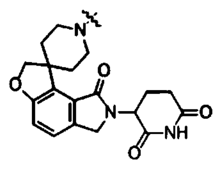
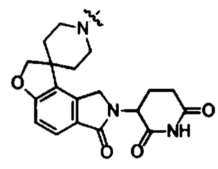
,
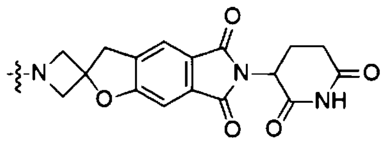
,

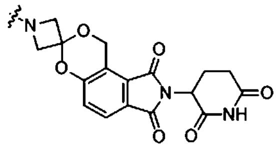
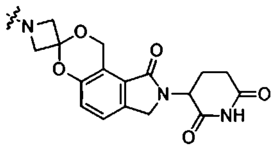
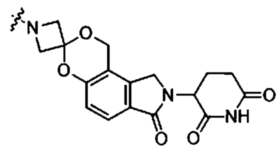

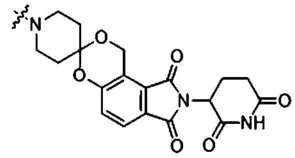
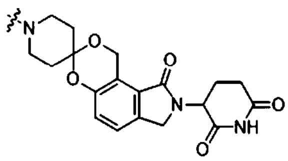
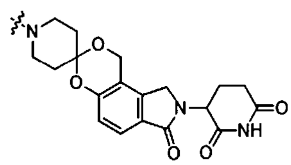

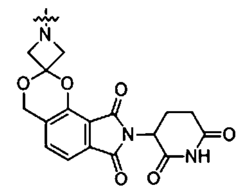
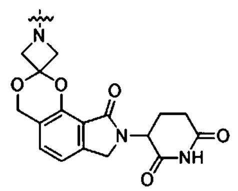
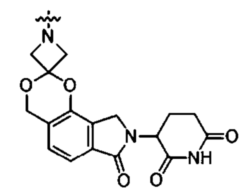

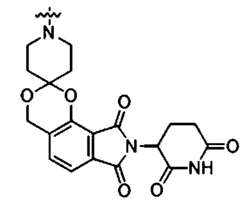
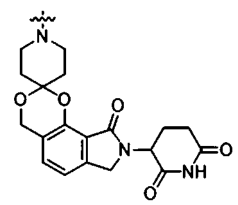
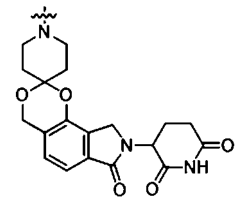

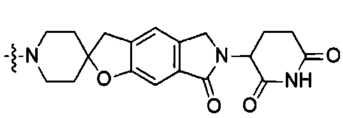
,
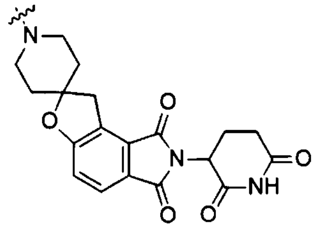
,
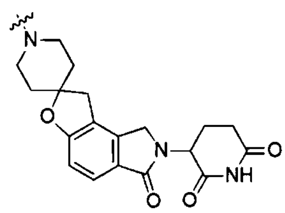
,

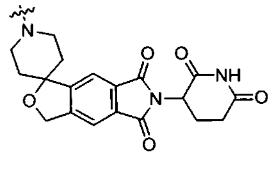
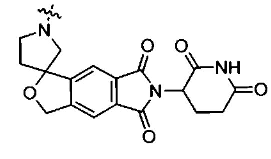
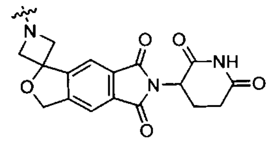

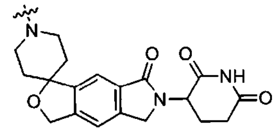
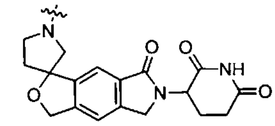
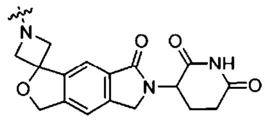

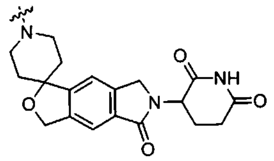
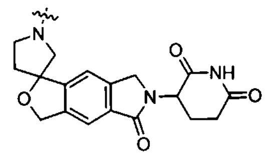
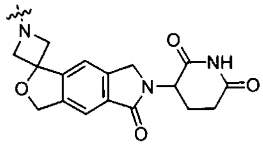
,

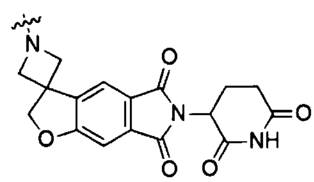
,
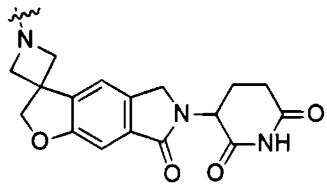
,
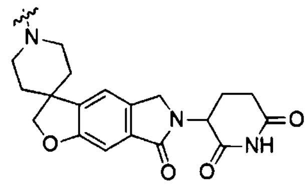
,

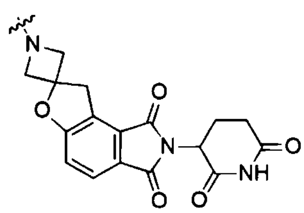
,
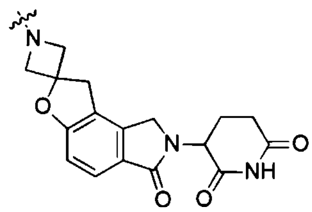
,
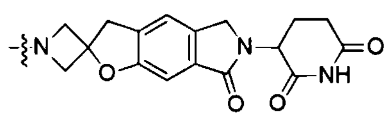
,

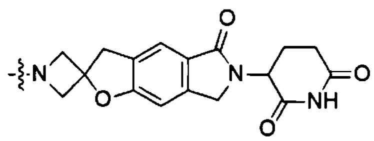
,
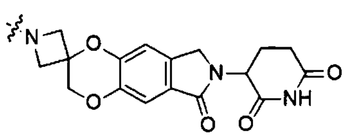
,
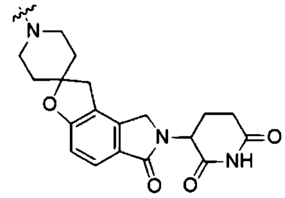

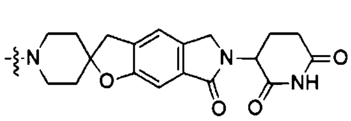
,
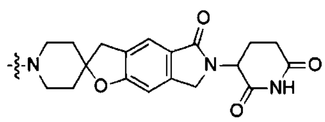
,
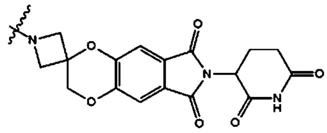
,

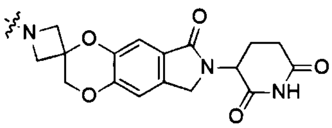
,
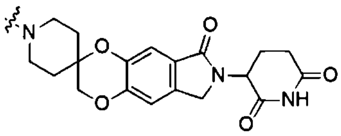
,
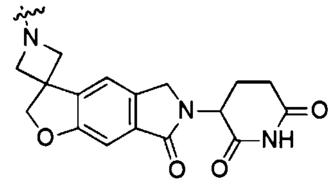
,

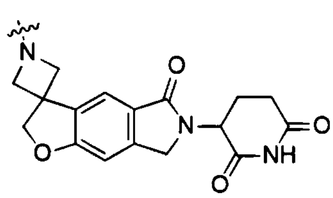
,
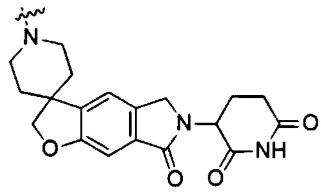
,
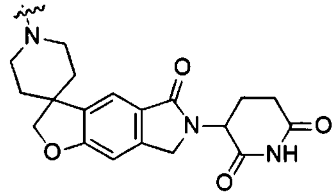
,

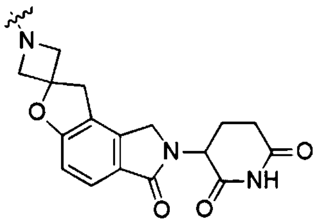
,
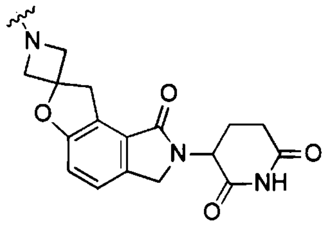
,
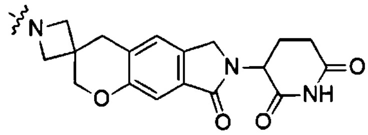
,

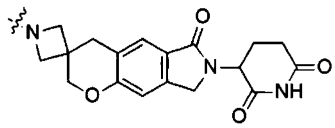
,
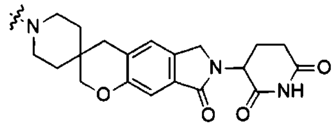
,
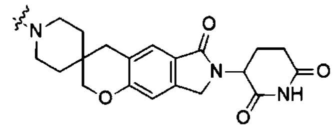
,

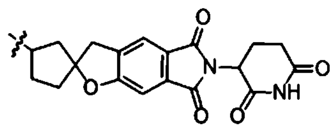
,
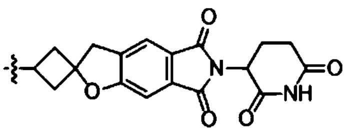
,
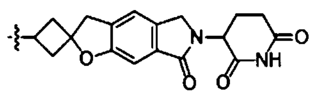
,

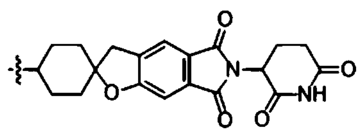
,
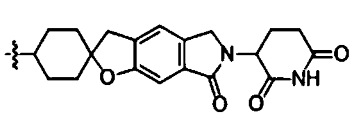
,
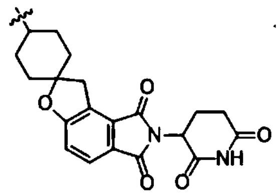
,

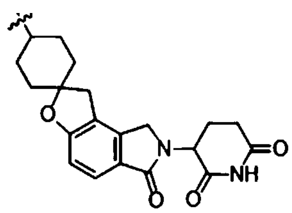
,
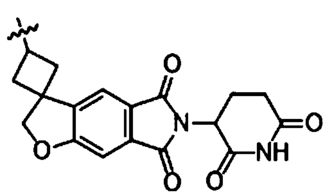
,
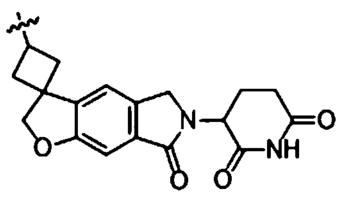
,

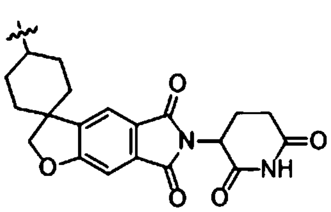
,
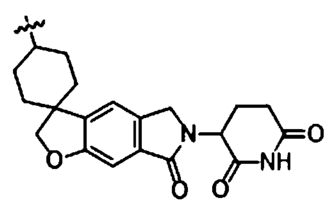
,
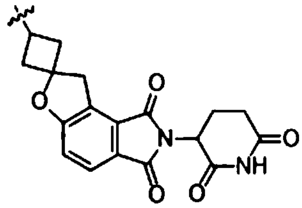
,

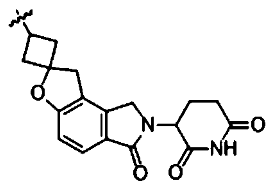, 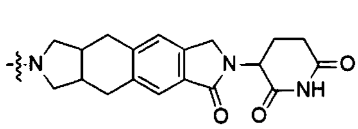, 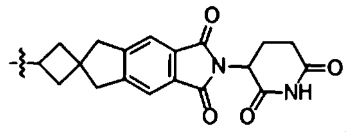,

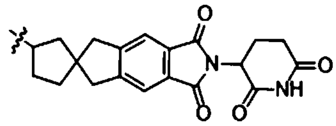, 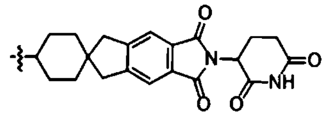, 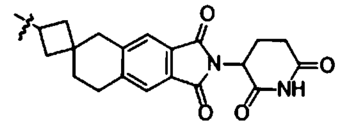,

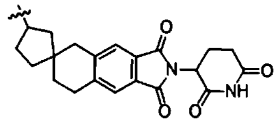, 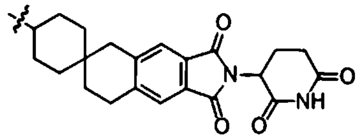, 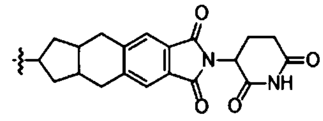,

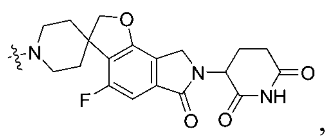, 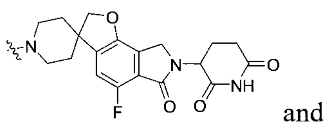 and 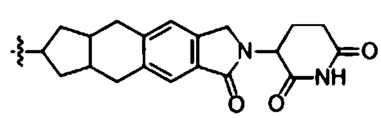.

**31.** The compound according to claim 30, wherein:

$PTM_a$ is a binding moiety that targets the estrogen receptor protein,

$L_a$ has the structure of $-(B^{La})_{q1}-$,

each occurrence of $B^{La}$ is identical or different and is each independently selected from: a covalent bond, $CR^{La}R^{Lb}$, O, S, SO, $SO_2$, CO, C(=NCN), C(=$CNO_2$), $C_2$-$C_6$ alkenylene, $C_2$-$C_6$ alkynylene, $C_3$-$C_{11}$ cycloalkylene optionally substituted with 0 to 6 $R^{La}$ and/or $R^{Lb}$ groups, 3-11-membered heterocyclylene optionally substituted with 0 to 6 $R^{La}$ and/or $R^{Lb}$ groups, arylene optionally substituted with 0 to 6 $R^{La}$ and/or $R^{Lb}$ groups, heteroarylene optionally substituted with 0 to 6 $R^{La}$ and/or $R^{Lb}$ groups, $C_6$-$C_{16}$ spirocyclylene optionally substituted with 0 to 6 $R^{La}$ and/or $R^{Lb}$ groups, and 6-16-membered heterospirocyclylene optionally substituted with 0 to 6 $R^{La}$ and/or $R^{Lb}$ groups;

$R^{La}$ and $R^{Lb}$ are each independently selected from H, halogen, alkyl, haloalkyl, alkoxy, haloalkoxy, alkenyl, haloalkenyl, alkynyl, haloalkynyl, cycloalkyl, aryl, heteroaryl, heterocyclyl, OH, CN, and $NO_2$; and

q1 is an integer greater than or equal to 1.

**32.** The compound according to claim 30 or 31, wherein each occurrence of $B^{La}$ is identical or different and is independently selected from: a covalent bond, $CR^{La}R^{Lb}$, $C_3$-$C_{11}$ cycloalkylene optionally substituted with 0 to 6 $R^{La}$ and/or $R^{Lb}$ groups, 3-11-membered heterocyclylene optionally substituted with 0 to 6 $R^{La}$ and/or $R^{Lb}$ groups;

each occurrence of $R^{La}$ and $R^{Lb}$ are each independently selected from H, F, Cl, Br, I, $C_{1-8}$ alkyl, $C_1$-8 alkoxy, - OH, $-NH_2$, $-CO_2H$, -CN, $-CF_3$, $-CHF_2$, $-CH_2F$, and-$NO_2$; and/or

q1 is an integer greater than or equal to 1, preferably 1, 2, 3, 4, 5, 6, 7, 8, 9, 10, 11, 12, 13, 14, 15, 16, 17, 18, 19 or 20, preferably 1, 2, 3, 4, or 5;

preferably, $B^{La}$ is selected from one or more of the following structures: covalent bond, $-(CH_2)_{k1}-$,

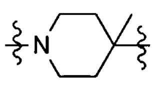, 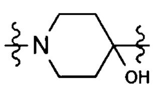, 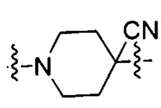, 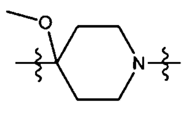, 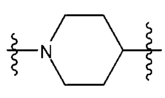 and 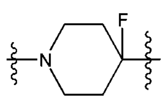;

wherein k1 is 1, 2, 3, 4, 5, 6, 7, 8, 9 or 10.

**33.** The compound according to any one of claims 30 to 32,wherein $L_a$ is selected from the following structures::

a covalent bond, $-(CH_2)_{j1}-$,

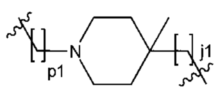 , 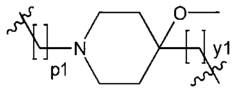 , 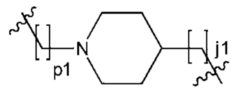 ,

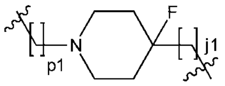 ;

wherein, j1 is 1, 2, 3, 4, 5, 6, 7, 8, 9 or 10;
p1 and y1 are 0, 1, 2, 3, 4, 5, 6, 7, 8, 9 or 10;
preferably, $L_a$ is selected from

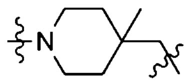 , 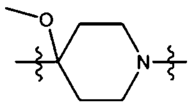 , 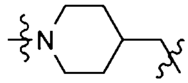 ,

and

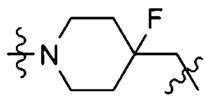 .

**34.** The compound according to any one of claims 20 to 33, wherein the $PTM_a$ is selected from the following structures:

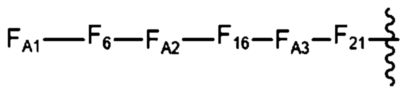 ;

wherein each occurrence of $F_6$, $F_{16}$, and $F_{21}$ are each independently selected from a single bond, NH, SO, S, O, $SO_2$, alkylene, haloalkylene, heteroalkylene, alkoxyalkylene, heteroalkoxyalkylene, alkenylene, alkynylene, C(=O), OC(=O), C(=O)O, C(=O)NH, and NHC(=O), or a combination thereof; wherein the alkylene, alkoxyalkylene, and alkenylene are optionally substituted with 0, 1, 2, 3, 4, 5, or 6 $R_c$; preferably, each occurrence of $F_6$, $F_{16}$, and $F_{21}$ are each independently selected from a single bond, NH, O, C(=O), C(=O)O, and NHC(=O);
each occurrence of $F_{A1}$ is independently an aryl or heteroaryl, wherein the aryl or heteroaryl is optionally substituted with 0, 1, 2, 3, 4, 5, or 6 $R_A$;
each occurrence of $F_{A3}$ is independently an arylene or heteroarylene, wherein the arylene or heteroarylene group is optionally substituted with 0, 1, 2, 3, 4, 5, or 6 $R_c$, preferably substituted with 0, 1, 2, or 3 $R_c$;
$F_{A2}$ is a cycloalkylene, spirocycloalkylene, heterocycloalkylene, or spiroheterocycloalkylene, optionally substituted with 0, 1, 2, 3, 4, 5, or 6 $R_A$;
each occurrence of Rc is independently selected from H, halogen, $C_1$-$_6$ alkyl, $C_1$-$_6$ haloalkyl, $C_1$-$_6$ heteroalkyl, $C_1$-$_6$ alkoxy, $C_1$-$_6$ heteroalkoxy, $C_3$-$_7$ cycloalkyl, $C_3$-$_7$ heterocyclyl, $C_6$-$_8$ aryl, 5-10 membered heteroaryl, OH, $NH_2$, CN, and $NO_2$, preferably H, F, Cl, Br, I, $CH_3$, $OCH_3$, $CF_3$, OH, $NH_2$, CN, or $NO_2$;
each occurrence of $R_A$ is independently selected from H, halogen, $C_1$-$_6$ alkyl, $C_1$-$_6$ haloalkyl, $C_1$-$_6$ heteroalkyl, $C_1$-$_6$ alkoxy, $C_1$-$_6$ heteroalkoxy, $C_3$-$_7$ cycloalkyl, $C_3$-$_7$ heterocyclyl, $C_6$-$_8$ aryl, 5-10 membered heteroaryl, oxo (=O), thio (=S), OH, $NH_2$, CN, and NO2, preferably oxo (=O), thio (=S), H, F, Cl, Br, I, $CH_3$, $OCH_3$, $CF_3$, OH, $NH_2$, CN, or $NO_2$.

**35.** The compound according to claim 34, wherein:

$F_{A1}$ is a phenyl group substituted with 0, 1, 2, or 3 $R_c$; and/or
$F_{A2}$ is selected from

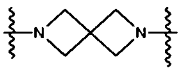, 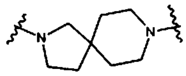, 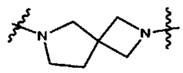, 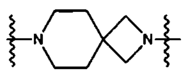,

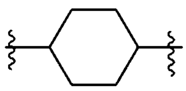, 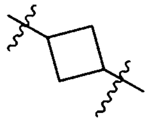 and 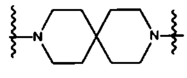,

optionally substituted with 0, 1, 2, 3 or 4 $R_A$; and/or each occurrence of $F_{A3}$ is independently a 6-10 membered arylene or a 5- 13 membered heteroarylene containing 1, 2, 3, 4, or 5 heteroatoms selected from N, O, and/or S; the arylene or heteroarylene is optionally substituted with 0, 1, 2, 3, 4, 5, or 6 $R_c$; preferably, $F_{A3}$ is selected from the following groups, each of which is optionally substituted with 0, 1, 2, 3, 4, 5, or 6 $R_c$ substituents:

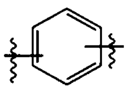, 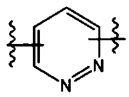, 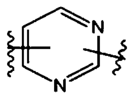, 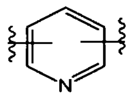, and 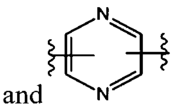,

wherein

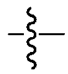

denotes a point of attachment, when the

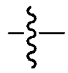

on the $F_{A3}$ group is not fixed, it indicates that

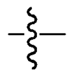

may be attached to any connectable atom of the $F_{A3}$ group that can be connected.

**36.** The compound according to any one of claims 30 to 35, wherein $PTM_a$ is selected from:

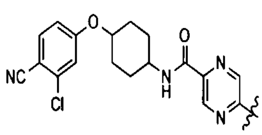, 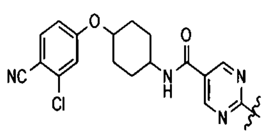, 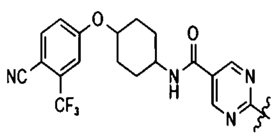, 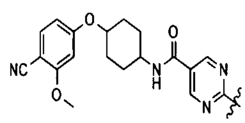,

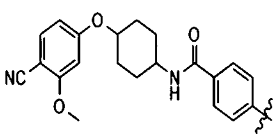, 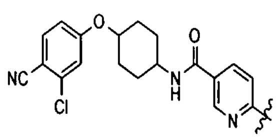, 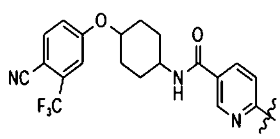, 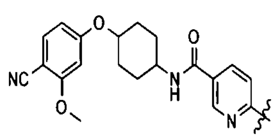,

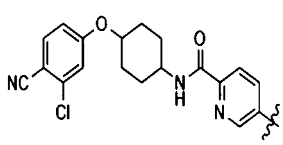, 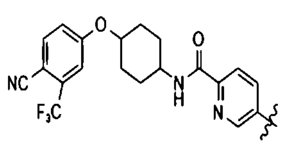, 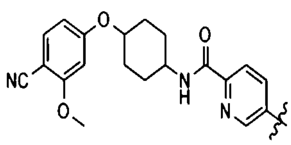, 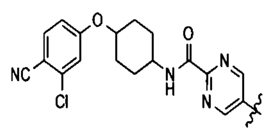,

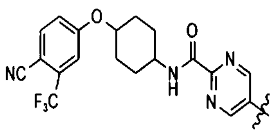, 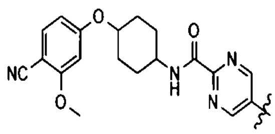, 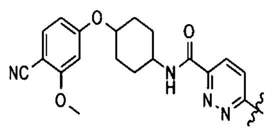, 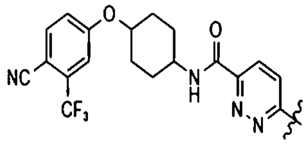,

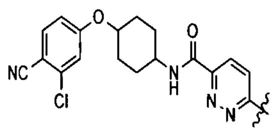, 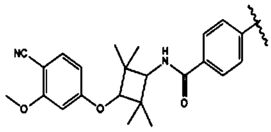, 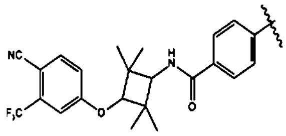, 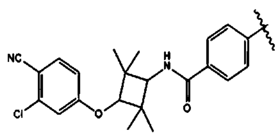,

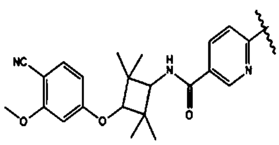, 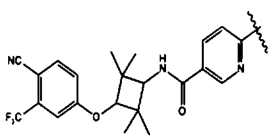, 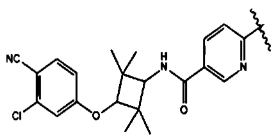, 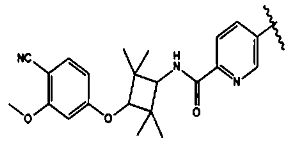,

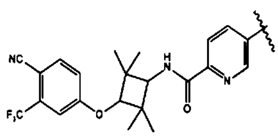, 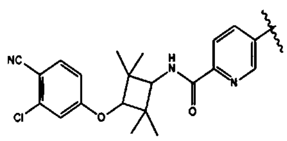, 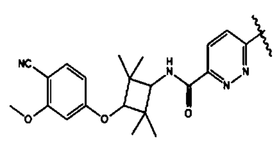, 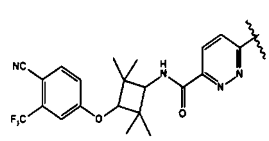,

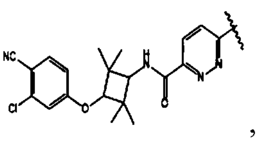, 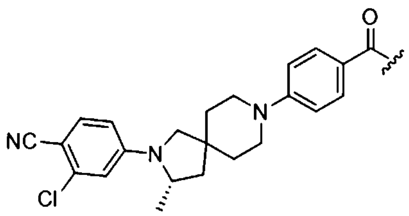 and 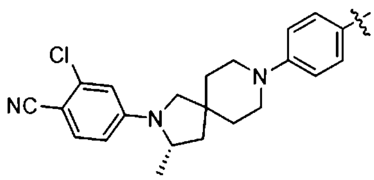.

**37.** The compound according to any one of claims 30 to 36, wherein the compound is selected from:

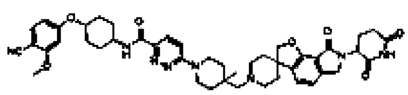 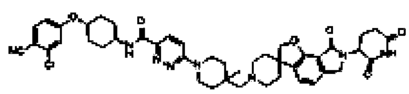

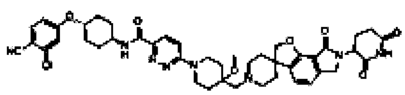 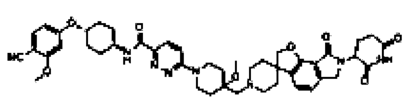

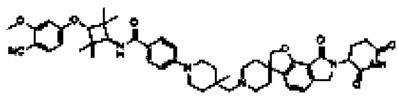 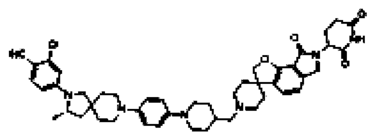

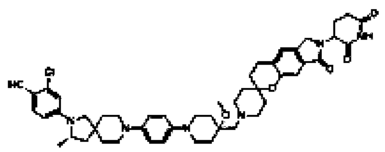 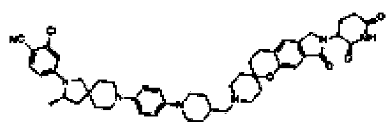

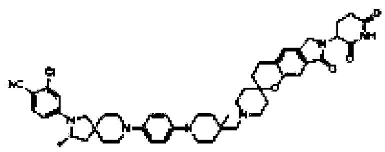

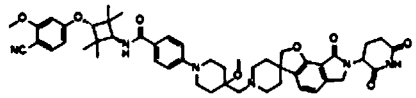

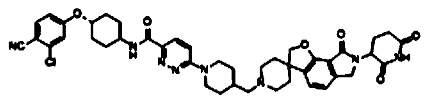

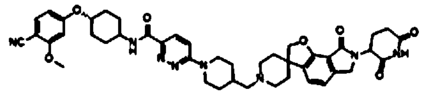

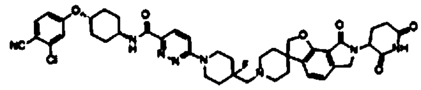

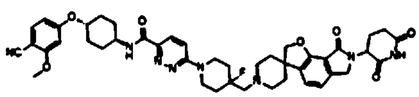

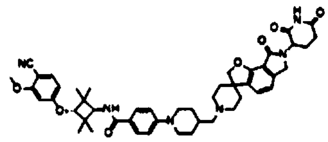

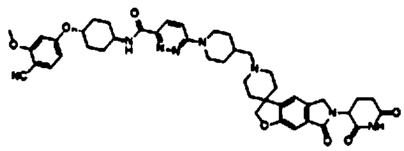

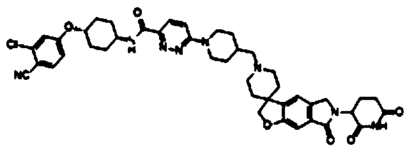

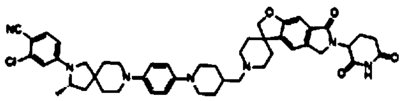

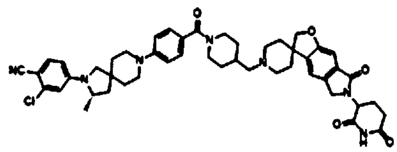

**38.** A pharmaceutical composition, wherein the pharmaceutical composition comprises the compound according to any one of claims 30 to 37.

**39.** The compound according to any one of claims 30 to 37, or the pharmaceutical composition according to claim 38, for use as a medicament.

**40.** The compound according to any one of claims 30 to 37, or the pharmaceutical composition according to claim 38, for use as a medicament in the treatment of prostate cancer.

**41.** Use of the compound according to any one of claims 30 to 37, or the pharmaceutical composition according to claim 38, in the manufacture of a medicament for the treatment of cancer, preferably, the cancer is prostate cancer.

**42.** A method for treating or preventing cancer, comprising administering to a subject in need thereof a therapeutically effective amount of the compound according to any one of claims 30-37, or the pharmaceutical composition according to claim 38; preferably, the cancer is prostate cancer.

## INTERNATIONAL SEARCH REPORT

International application No.
**PCT/CN2024/074559**

**A. CLASSIFICATION OF SUBJECT MATTER**

C07D491/20(2006.01)i; C07D491/107(2006.01)i; C07D491/113(2006.01)i; C07D487/04(2006.01)i; C07D487/00(2006.01)i; A61K31/438(2006.01)i; A61K31/397(2006.01)i; A61K31/4035(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

CNTXT, ENTXT, DWPI, CNKI, STN(REG, CAPLUS), 甘李药业, 徐福明, 陈文敏, 周冠, 王苑郦, 王康华, 李晓光, 小脑蛋白, E3泛素连接酶, 雌激素, 雄激素, 蛋白降解靶向嵌合体, 螺环, PROTAC? , cereblon? , estrogen? , androgen? , spiro? , degrad? , 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| PX | WO 2024015412 A1 (UNIV. MICHIGAN et al.) 18 January 2024 (2024-01-18)<br>see claims 1 and 58-67, and description, paragraphs 0536 and 0631 | 1-30 |
| PX | WO 2023183607 A1 (UNIV. MICHIGAN et al.) 28 September 2023 (2023-09-28)<br>see descriptions, paragraphs 0017-0019, 0039-0070, 0072, 0122, 0138, 0154, 0169-0170, 0180-0186, 0477-0478, 0482, 0491 and 0515-0525, tables 2 and E13, and claim 25 | 1-42 |
| PX | WO 2024015408 A1 (REGENTS OF THE UNIVESITY OF MICHIGAN et al.) 18 January 2024 (2024-01-18)<br>see claims 1 and 66-74, and description, paragraphs 0376 and 0402, and table 1 | 1-30 |
| PX | WO 2024015406 A1 (UNIV. MICHIGAN et al.) 18 January 2024 (2024-01-18)<br>see claims 1 and 67-75, and description, paragraphs 0423 and 0439, and table 1 | 1-30 |
| PX | WO 2023143589 A1 (GAN & LEE PHARMACEUTICALS CO., LTD.) 03 August 2023 (2023-08-03)<br>see claims 1-25 and 47-54 | 1-42 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 April 2024** | **22 April 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/074559**

**C. DOCUMENTS CONSIDERED TO BE RELEVANT**

| Category* | Citation of document, with indication, where appropriate, of the relevant passages | Relevant to claim No. |
|---|---|---|
| PA | WO 2023201012 A1 (UNIV. MICHIGAN et al.) 19 October 2023 (2023-10-19)<br>see claims 1-40 | 1-42 |
| X | WO 2022187588 A1 (UNIV MICHIGAN REGENTS) 09 September 2022 (2022-09-09)<br>see claims 1-35, and description, table 1 | 1-4, 7-8, 10-13, 16-28 |
| A | WO 2021143822 A1 (JIANGSU HENGRUI MEDICINE CO., LTD.) 22 July 2021 (2021-07-22)<br>see claims 1-31, description, pages 32-33, and abstract | 1-42 |
| A | CN 114641337 A (THE REGENTS OF THE UNIVERSITY OF MICHIGAN) 17 June 2022 (2022-06-17)<br>see claims 1-37 and 63-73, and description, tables 1-7, compounds | 1-42 |
| A | CN 115348872 A (ORIONIS BIOSCIENCES, INC.) 15 November 2022 (2022-11-15)<br>see claims 1-24 | 1-42 |
| A | CN 115023267 A (THE REGENTS OF THE UNIVERSITY OF MICHIGAN) 06 September 2022 (2022-09-06)<br>see claims 1-120 | 29-42 |
| A | WO 2021231927 A1 (UNIV MICHIGAN REGENTS) 18 November 2021 (2021-11-18)<br>see claims 1-49 | 29-42 |
| A | WO 2022187423 A1 (UNIV MICHIGAN REGENTS) 09 September 2022 (2022-09-09)<br>see claims 1-33 | 1-42 |

**INTERNATIONAL SEARCH REPORT**

International application No.
**PCT/CN2024/074559**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **19-20, 25-28, 42**
because they relate to subject matter not required to be searched by this Authority, namely:

Claims 19-20, 25-28 and 42 relate to a method for treating a human/animal body. In the present search report, a search is performed on the basis of the corresponding pharmaceutical use of a compound or composition as claimed.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2024/074559**

| Patent document cited in search report | Publication date (day/month/year) | Patent family member(s) | Publication date (day/month/year) |
|---|---|---|---|
| WO 2024015412 A1 | 18 January 2024 | None | |
| WO 2023183607 A1 | 28 September 2023 | None | |
| WO 2024015408 A1 | 18 January 2024 | None | |
| WO 2024015406 A1 | 18 January 2024 | None | |
| WO 2023143589 A1 | 03 August 2023 | None | |
| WO 2023201012 A1 | 19 October 2023 | None | |
| WO 2022187588 A1 | 09 September 2022 | None | |
| WO 2021143822 A1 | 22 July 2021 | TW 202140448 A | 01 November 2021 |
| CN 114641337 A | 17 June 2022 | CA 3151824 A1 | 04 March 2021 |
| | | WO 2021041664 A1 | 04 March 2021 |
| | | AU 2020336381 A1 | 03 March 2022 |
| | | EP 4021580 A1 | 06 July 2022 |
| | | US 2022388978 A1 | 08 December 2022 |
| | | JP 2022545735 A | 28 October 2022 |
| CN 115348872 A | 15 November 2022 | WO 2021126974 A1 | 24 June 2021 |
| | | JP 2023508891 A | 06 March 2023 |
| | | CA 3162266 A1 | 24 June 2021 |
| | | AU 2020408333 A1 | 07 July 2022 |
| | | US 2023099031 A1 | 30 March 2023 |
| | | EP 4076530 A1 | 26 October 2022 |
| CN 115023267 A | 06 September 2022 | EP 4031241 A1 | 27 July 2022 |
| | | JP 2022548775 A | 21 November 2022 |
| | | WO 2021055756 A1 | 25 March 2021 |
| | | AU 2020348849 A1 | 07 April 2022 |
| | | CA 3155010 A1 | 25 March 2021 |
| | | US 2022380368 A1 | 01 December 2022 |
| WO 2021231927 A1 | 18 November 2021 | US 2023357249 A1 | 09 November 2023 |
| WO 2022187423 A1 | 09 September 2022 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Proc. Natl. Acad. Sci. USA*, 2001, vol. 98, 8584
- *Cell Biochem Funct.*, 2019, vol. 37, 21-30
- *Cell Chem. Biol.*, 2018, vol. 25, 67-77
- *Science*, 2010, vol. 327, 1345
- *Science*, 2014
- *Science*, 2014, vol. 343, 301
- *Science*, 2014, vol. 343, 305
- Anti-Androgen receptor. *Cell Signaling Technology Catalog No. 5153S*, vol. 1, 2000